# EUROPEAN PATENT SPECIFICATION

(11) **EP 4 433 457 B1**
(45) Date of publication and mention of the grant of the patent: **08.07.2026**
(21) Application number: 22840846.4
(22) Date of filing: 18.11.2022
(51) Int. Cl.: C07D 231/56, C07D 471/04, C07D 487/04, A61P 25/00, A61K 31/519

(54) **HETEROCYCLIC AMIDES AND METHODS OF USING THE SAME**
HETEROCYCLISCHE AMIDE UND VERFAHREN ZU IHRER VERWENDUNG
AMIDES HÉTÉROCYCLIQUES ET LEURS PROCÉDÉS D'UTILISATION

(30) Priority: 18.11.2021 US 202163280939 P
(43) Date of publication of application: 25.09.2024
(73) Proprietor: Rgenta Therapeutics, Inc., Woburn, Massachusetts 01801 (US)
(72) Inventor: WAGER, Travis T., Brookline, Massachusetts 02446 (US); WENG, Zhiping, Brookline, Massachusetts 02445 (US); XI, Hualin Simon, Lexington, Massachusetts 02420 (US)
(74) Representative: Heller, Benjamin Henry
(86) International application number: PCT/US2022/080182
(87) International publication number: WO 2023/092098

(56) References cited:
- WO-A1-2019/028440
- US-A1- 2020 390 765

## Description

### CROSS REFERENCE TO RELATED APPLICATIONS

### BACKGROUND

More than 90% of human genes produce multiple mature transcripts via alternative splicing. This process is essential for generating different transcripts in different cell and tissue types, during the developmental process, and in response to internal and external signals. Alternative splicing are prevalent not only for protein-coding genes but also for most other kinds of genes including microRNA genes and long noncoding genes. Splicing is carried out by the spliceosome. Small nuclear RNAs (snRNAs) are key components of the spliceosome. The major spliceosome comprises the U1, U2, U4, U5, and U6 snRNAs, and it catalyzes the removal of -95% of human introns, while the remaining introns (called the U12-type of introns) are removed by the minor spliceosome, comprising the U11, U12, U4atac, U5, and U6atac snRNAs. These snRNAs are in complex with their respective protein partners to form the functional unit of small nuclear ribonucleoproteins (snRNPs).

Splicing is a highly regulated process, with the regulation exerted by both cis-elements and trans-factors. The cis-elements that are recognized by the snRNAs include the 5'-splice site, 3'-splice site, and the branchpoint, each of these associating with a sequence motif that is recognized by a component of the spliceosome. In addition, there are intronic splicing enhancers (ISE), intronic splicing silencer (ISS), exonic splicing enhancer (ESE), and exonic splicing enhancer (ESS), which are recognized by a myriad of trans-factors commonly known as RNA-binding proteins (RBPs). Some of these RBPs directly bind to the cis-elements in a sequencing-specific way, while other RBPs recognize RNA structures (e.g., RNA duplex or unpaired loop region), yet others function via protein-protein interaction. There are ~1600 RBPs annotated in the human genome, and they are expressed in a cell-type-specific manner and form an extensive regulatory network for splicing regulation.

Dysregulation of splicing is implicated in roughly half of human diseases. Some diseases are caused by mutations in the spliceosome components or RBPs, while others by mutations in the cis-elements such as splice sites, branchpoint, or the various splicing enhancers and silencers. Although current approaches to treating these diseases, such as CRISPR-based genome editing, virus-aided gene therapy, or a variety of oligonucleotide-based technologies, continue to improve, they still suffer major technical and clinical challenges. In particular, oligonucleotide-based therapeutics show unfavorable pharmacokinetics, can not be orally administered, and can not be delivered effectively to many tissues, especially the brain. Small-molecule drugs have excellent pharmacokinetics, effective delivery, and bioavailability, and have only recently become available for modulating RNA splicing. Yet, the currently molecules come from a few limited chemical series. Thus, there is a great need to develop additional small molecule splicing modulators (SMSMs).

Almost 50 inherited disorders in humans result from an increase in the number of copies of single repeats in genomic DNA. These DNA repeats appear to be predisposed to such expansion because they have unusual structural characteristics, which disrupt cellular replication, repair, and recombination machinery. The presence of expanding DNA repeats alters gene expression in human cells, leading to disease.

One of these inherited disorders is Huntington's disease (HD). HD is a deadly neurodegenerative disorder with no cure associated with cognitive impairment, dementia, and loss of motor coordination. It is characterized by the progressive and hereditary increase in the length of the CAG trinucleotide repeats that encode a stretch of polyglutamine, in the Huntington gene (HTT) coding region. These repeats can increase in number from one generation to the next. The normal allele of the HTT gene contains fewer than 36 CAG repeats, while the mutant allele contains more than 36 repeats. Most HD patients carry one normal allele and one mutant allele that causes the disease. Functionally, the aberrant accumulation of CAG repeats is believed to confer a toxic gain of function on the mutant HD protein, causing it to aggregate, form protein deposits (ie, inclusion bodies), and induce cell death. The severity of the disease generally reflects the extent of repeat expansion in the mutant HTT protein.

Myotonic dystrophy type 1 (DM1) and type 2 (DM2) are associated with long repeats of polyCUG and polyCCUG in the 3'-UTR and intron 1 regions of the transcription of myotonic dystrophy protein kinase (DMPK) and protein 9 of zinc finger (ZNF9), respectively. While normal individuals have up to 30 CTG repeats, DMI patients have a higher number of repeats ranging from 50 to thousands. The severity of the disease and the age of onset correlate with the number of repetitions. Adult-onset patients show milder symptoms and have fewer than 100 repeats, juvenile-onset DM1 patients have up to 500 repeats, and congenital cases typically have around 1,000 CTG repeats. Expanded transcripts containing CUG repeats form a secondary structure, accumulate in the nucleus as nuclear foci, and sequester RNA-binding proteins (RNA-BP).

Besides the extra copies of repeats inherited at birth, for many repeat expansion diseases, repeats are highly unstable and their repeat numbers continue to expand throughout the life time of patients. This repeat instability has been shown experimentally to be mediated by proteins in DNA mismatch repair (MMR) processes including PMS1, MLH1, MSH3. Human genetics data from genome-wide association studies has indicated that variants in MMR proteins are associated with clinically relevant HD symptomatology including age at motor onset, rate of progression and somatic instability. Knocking down and knocking out MMR genes have been shown to stall or slow the somatic repeat expansion in various preclinical models of repeat expansion diseases. Thus, there is a need for splicing modulators of MMR genes as potential therapeutic agents to treat a variety of repeat expansion diseases.

### SUMMARY

The references to methods of treatment in the summary and detailed description of the invention in this description are to be interpreted as references to compounds, pharmaceutical compositions and medicaments of the present invention for use in a method of treatment of the human (or animal) body by therapy. Here we describe a series of novel small molecule splicing modulators (SMSMs), which can be used to treat a wide variety of diseases, including neurodegenerative and repeat expansion diseases. These SMSMs target regions of a primary RNA transcript that are cis-elements, such as splice sites, branch points, splicing enhancers, or splicing silencers. These regions may contain unpaired nucleotides in an RNA duplex, called bulges. The bulges may be naturally occurring or caused by diseases. When the SMSMs come into contact with the RNA transcript, it may be bound by the spliceosome or the other trans-factors, most notably RNA-binding proteins (RBPs). The SMSMs reported herein may cause an alteration in the sequence or abundance of the mature transcript, which may, in turn, cause a difference in the sequence or abundance of the functional protein should the transcript be protein-coding or the sequence or abundance of the functional RNA should the transcript be non-coding.

In some aspects, the present disclosure provides, *inter alia,* a compound of Formula (I): 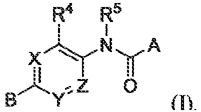 or a pharmaceutically acceptable salt thereof, wherein:
X=N or CR¹; Y=N or CR²; Z=N or CR³;
R¹, R², R³ and R⁴ are each independently selected from the group consisting of H, halogen, hydroxy, cyano, -COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₁-C₆cycloalkyloxy, C₃-C₈cycloalkyl, NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy, are optionally substituted with one or more halogen, hydroxyl, methoxy, C₃-C₈cycloalkyl, or NH₂;
R⁵ is H, C₁-C₆alkyl, or C₁-C₆haloalkyl;
A is selected from the group consisting of 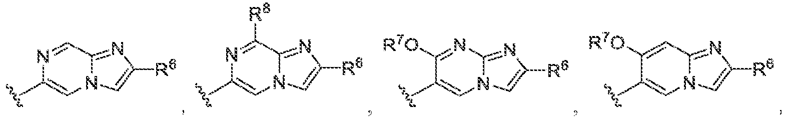 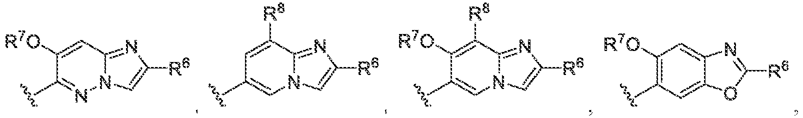 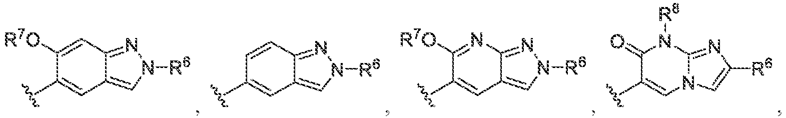 and 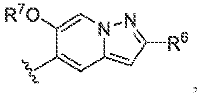
wherein A is optionally substituted with 1-4 R⁹;
R⁶ is C₁-C₃alkyl or C₁-C₃haloalkyl;
R⁷ is H, C₁-C₆alkyl, C₁-C₆haloalkyl, C₁-C₆cycloalkyl, or heterocycloalkyl, wherein heterocycloalkyl is optionally substituted with 1-3 substituents independently selected from halogen and C₁-C₆alkyl;
R⁸ is halogen, C₁-C₆alkyl, C₁-C₆alkoxy, C₁-C₆haloalkyl, or C₁-C₆cycloalkyl;
each R⁹ is independently selected from the group consisting of halogen, hydroxy, cyano, - COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₃-C₈cycloalkyloxy, C₃-C₈cycloalkyl, NH₂, NH(C₁-C₆alkyl), N(C₁-C₆alkyl)₂, - NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl, methoxy, C₃-C₈cycloalkyl, or NH₂;
B is heterocycloalkyl not linked to formula (I) by a nitrogen atom, optionally substituted with 1 to 6 R¹²; or
B is NR¹⁰R¹¹, wherein
R¹⁰ is -(CH₂)₀₋₃aryl, -(CH₂)₀₋₃heteroaryl, -(CH₂)₀₋₃heterocycloalkyl comprising at least 1 nitrogen ring atom, or C₁-C₈heteroalkyl comprising at least one nitrogen atom, each R¹⁰ optionally substituted with 1 to 6 R¹²; and
R¹¹ is hydrogen, C₁₋₇alkyl, C₁₋₇haloalkyl, or C₃₋₈cycloalkyl; or
R¹⁰ and R¹¹ are taken together with the nitrogen atom to which they are attached to form a heterocycloalkyl comprising 1, 2 or 3 total nitrogen ring atoms and 0 or 1 additional ring heteroatoms selected from O and S, and the heterocycloalkyl is optionally substituted with 1 to 6 R¹²;
   each R¹² is independently selected from the group consisting of halogen, hydroxy, cyano, -COOH, -C(O)-C₁-C₆alkyl, -C(O)O-C₁-C₆alkyl, C₁-C₆alkyl, C₁-C₆heteroalkyl, C₂-C₆alkenyl, C₂-C₆alkynyl, C₁-C₆alkoxy, -(CH₂)₀₋₂-C₃-C₈cycloalkyl, -(CH₂)₀₋₂-SO₂-C₁-C₆alkyl, C₁-C₆heteroalkylene-C₃-C₈cycloalkyl, -O-C₃-C₈cycloalkyl, 4-7-membered monocyclic heterocycloalkyl, C₁-C₆heteroalkylene-(4-7-membered monocyclic heterocycloalkyl), -O-(4-7-membered monocyclic heterocycloalkyl), -(CH₂)₀₋₂-(4-7-membered monocyclic heterocycloalkyl), NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl or NH₂, and wherein the cycloalkyl and heterocycloalkyl are optionally substituted with one or more halogen, hydroxyl, C₁-C₆alkyl, C₁-C₆heteroalkyl, C₁-C₆alkoxy, or NH₂; or
two R¹² on the same carbon can be taken together as keto (=O).

In some aspects, the present disclosure provides a compound obtainable by, or obtained by, a method for preparing a compound as described here (e.g., a method comprising one or more steps described in herein).

In some aspects, the present disclosure provides a pharmaceutical composition comprising a compound of the present disclosure, or a pharmaceutically acceptable salt, solvate, or prodrug thereof, and a pharmaceutically acceptable diluent or carrier.

In some aspects, the present disclosure provides an intermediate as described herein, being suitable for use in a method for preparing a compound as described herein (e.g., the intermediate is selected from the intermediates described herein).

In some aspects, the present disclosure provides a method of treating or preventing a disease or disorder disclosed herein in a subject in need thereof, comprising administering to the subject a therapeutically effective amount of a compound of the present disclosure or a pharmaceutically acceptable salt thereof, or a pharmaceutical composition of the present disclosure.

In some aspects, the present disclosure provides a method of treating a disease or disorder disclosed herein in a subject in need thereof, comprising administering to the subject a therapeutically effective amount of a compound of the present disclosure or a pharmaceutically acceptable salt thereof, or a pharmaceutical composition of the present disclosure.

In some aspects, the present disclosure provides a compound of the present disclosure or a pharmaceutically acceptable salt thereof for use in treating or preventing a disease or disorder disclosed herein.

In some aspects, the present disclosure provides a compound of the present disclosure or a pharmaceutically acceptable salt thereof for use in treating a disease or disorder disclosed herein.

In some aspects, the present disclosure provides use of a compound of the present disclosure or a pharmaceutically acceptable salt thereof for treating or preventing a disease or disorder disclosed herein.

In some aspects, the present disclosure provides use of a compound of the present disclosure or a pharmaceutically acceptable salt thereof for treating a disease or disorder disclosed herein.

In some aspects, the present disclosure provides use of a compound of the present disclosure or a pharmaceutically acceptable salt thereof in the manufacture of a medicament for treating or preventing a disease or disorder disclosed herein.

In some aspects, the present disclosure provides use of a compound of the present disclosure or a pharmaceutically acceptable salt thereof in the manufacture of a medicament for treating a disease or disorder disclosed herein.

In some aspects, the present disclosure provides a method of preparing a compound of the present disclosure.

In some aspects, the present disclosure provides a method of preparing a compound, comprising one or more steps described herein.

Unless otherwise defined, all technical and scientific terms used herein have the same meaning as commonly understood by one of ordinary skill in the art to which this disclosure belongs. Although methods and materials similar or equivalent to those described herein can be used in the practice or testing of the present disclosure, suitable methods and materials are described below. In the case of conflict, the present specification, including definitions, will control. In addition, the materials, methods, and examples are illustrative only and are not intended to be limiting.

Other features and advantages of the disclosure will be apparent from the following detailed description and claims.

### DETAILED DESCRIPTION

Compounds described herein are generally designed to treat diseases and disorders disclosed herein.

### Definitions

Unless otherwise stated, the following terms used in the specification and claims have the following meanings set out below.

As used herein, "alkyl", "C₁, C₂, C₃, C₄, C₅, C₆, or C₇ alkyl" or "C₁-C₇ alkyl" is intended to include C₁, C₂, C₃, C₄, C₅,C₆, or C₇ straight chain (linear) saturated aliphatic hydrocarbon groups and C₃, C₄, C₅,C₆, or C₇ branched saturated aliphatic hydrocarbon groups. For example, C₃-C₇ alkyl is intended to include C₁, C₂, C₃, C₄, C₅, C₆, and C₇ alkyl groups. Examples of alkyl include, moieties having from one to six carbon atoms, such as, but not limited to, methyl, ethyl, n-propyl, i-propyl, n-butyl, s-butyl, t-butyl, n-pentyl, i-pentyl, or n-hexyl. In some embodiments, a straight chain or branched alkyl has six or fewer carbon atoms (*e.g.,* C₁-C₆ for straight chain, C₃-C₆ for branched chain), and in another embodiment, a straight chain or branched alkyl has four or fewer carbon atoms.

As used herein, "alkenyl" is intended to include straight-chain or branched hydrocarbon groups having from 2 to 6 carbon atoms, one or more carbon-carbon double bonds, and no triple bonds ("C₂-C₆alkenyl"). The one or more carbon-carbon double bonds can be internal (such as in 2-butenyl) or terminal (such as in 1-butenyl). Examples of C₂-C₆alkenyl groups include ethenyl (C₂), 1-propenyl (C₃), 2-propenyl (C₃), 1- butenyl (C₄), 2-butenyl (C₄), butadienyl (C₄), and the like.

As used herein, "alkynyl" is intended to include straight-chain or branched hydrocarbon groups having from 2 to 6 carbon atoms, one or more carbon-carbon triple bonds, and optionally one or more double bonds ("C₂-C₆ alkynyl"). The one or more carbon-carbon triple bonds can be internal (such as in 2-butynyl) or terminal (such as in 1-butynyl). Examples of C₂-C₄alkynyl groups include, without limitation, ethynyl (C₂), 1-propynyl (C₃), 2- propynyl (C₃), 1-butynyl (C₄), 2-butynyl (C₄), and the like.

As used herein, the term "optionally substituted alkyl" refers to unsubstituted alkyl or alkyl having designated substituents replacing one or more hydrogen atoms on one or more carbons of the hydrocarbon backbone. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulphhydryl, alkylthio, arylthio, thiocarboxylate, sulphates, alkylsulphinyl, sulphonato, sulphamoyl, sulphonamido, nitro, trifluoromethyl, cyano, azido, heterocycloalkyl, alkylaryl, or an aromatic or heteroaromatic moiety.

Other optionally substituted moieties (such as optionally substituted cycloalkyl, heterocycloalkyl, aryl, or heteroaryl) include both the unsubstituted moieties and the moieties having one or more of the designated substituents. For example, substituted heterocycloalkyl includes those substituted with one or more alkyl groups, such as 2,2,6,6-tetramethyl-piperidinyl and 2,2,6,6-tetramethyl-1,2,3,6-tetrahydropyridinyl.

As used herein, "heteroalkyl", "C₁, C₂, C₃, C₄, C₅, C₆, C₇, or Cs heteroalkyl" or "C₁-C₈ heteroalkyl" is intended to include C₁, C₂, C₃, C₄, C₅,C₆, C₇, or C₈ straight chain (linear) saturated aliphatic hydrocarbon groups and C₃, C₄, C₅C₆, C₇, or Cs branched saturated aliphatic hydrocarbon groups, in which at least one of the carbons has been replaced with N, O, or S. The heteroatom will be bonded to any required hydrogens to complete the heteroatom's valence (e.g., a CH₂ may be replaced with an "O" or a "NH", a CH may be replaced with an N, etc.)). Such substituents can include, for example, -O-CH(CH₃)₂, -CH₂-N(CH₃)-CH₂CH₂OCH₃, -S-CH₂CH₂-O-CH₂CH₃, and so forth.

As used herein, "heteroalkylene" is a bivalent heteroalkyl group with two open valences. Such substituents can include, for example, -CH₂-O-CH₂-, -O-CH₂CH(CH₃)-NH-CH₂-, -CH₂-O-CH₂CH₂-S-CH₂-, etc.

As used herein, the term "alkoxy" refers to the group -OR where R is alkyl. Particular alkoxy groups are methoxy, ethoxy, n-propoxy, isopropoxy, n-butoxy, tert-butoxy, sec-butoxy, n-pentoxy, n-hexoxy, and 1,2- dimethylbutoxy. Particular alkoxy groups are lower alkoxy, i.e., with between 1 and 6 carbon atoms.

As used herein, the term "cycloalkyl" refers to a saturated or partially unsaturated hydrocarbon monocyclic or polycyclic (e.g., fused, bridged, or spiro rings) system having 3 to 30 carbon atoms (e.g., C₃-C₁₂, C₃-C₁₀, or C₃-C₈). Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, 1,2,3,4-tetrahydronaphthalenyl, and adamantyl. In the case of polycyclic cycloalkyl, only one of the rings in the cycloalkyl needs to be non-aromatic.

As used herein, the term "heterocycloalkyl" or "heterocyclyl" refers to a saturated or partially unsaturated 3-8 membered monocyclic, 7-12 membered bicyclic (fused, bridged, or spiro rings), or 11-14 membered tricyclic ring system (fused, bridged, or spiro rings) having one or more heteroatoms (such as O, N, S, P, or Se), e.g., 1 or 1-2 or 1-3 or 1-4 or 1-5 or 1-6 heteroatoms, *or e.g.,* 1, 2, 3, 4, 5, or 6 heteroatoms, independently selected from the group consisting of nitrogen, oxygen and sulphur, unless specified otherwise. Examples of heterocycloalkyl groups include, but are not limited to, piperidinyl, piperazinyl, pyrrolidinyl, dioxanyl, tetrahydrofuranyl, isoindolinyl, indolinyl, imidazolidinyl, pyrazolidinyl, oxazolidinyl, isoxazolidinyl, triazolidinyl, oxiranyl, azetidinyl, oxetanyl, thietanyl, 1,2,3,6-tetrahydropyridinyl, tetrahydropyranyl, dihydropyranyl, pyranyl, morpholinyl, tetrahydrothiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, 1,4-dioxa-8-azaspiro[4.5]decanyl, 1,4-dioxaspiro[4.5]decanyl, 1-oxaspiro[4.5]decanyl, 1-azaspiro[4.5]decanyl, 3'H-spiro[cyclohexane-1,1'-isobenzofuran]-yl, 7'H-spiro[cyclohexane-1,5'-furo[3,4-b]pyridin]-yl, 3'H-spiro[cyclohexane-1,1'-furo[3,4-c]pyridin]-yl, 3-azabicyclo[3.1.0]hexanyl, 3-azabicyclo[3.1.0]hexan-3-yl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like. In the case of multicyclic heterocycloalkyl, only one of the rings in the heterocycloalkyl needs to be non-aromatic (e.g., 4,5,6,7-tetrahydrobenzo[c]isoxazolyl).

As used herein, the term "cycloalkyloxy" refers to a -O-cycloalkyl group in which cycloalkyl is as defined herein. Preferably the cycloalkyloxy is a C₁-C₆cycloalkyloxy. Examples include, but are not limited to, cyclopropanoxy and cyclobutanoxy.

As used herein, the term "aryl" refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system. Examples of aryl groups include, but are not limited to, phenyl, naphthyl and the like. Conveniently, an aryl is phenyl.

As used herein, the term "heteroaryl" is intended to include a stable 5-, 6-, or 7-membered monocyclic or 7-, 8-, 9-, 10-, 11- or 12-membered bicyclic aromatic heterocyclic ring which consists of carbon atoms and one or more heteroatoms, e.g., 1 or 1-2 or 1-3 or 1-4 or 1-5 or 1-6 heteroatoms, *or e.g.,* 1, 2, 3, 4, 5, or 6 heteroatoms, independently selected from the group consisting of nitrogen, oxygen and sulphur. The nitrogen atom may be substituted or unsubstituted *(i.e.,* N or NR wherein R is H or other substituents, as defined). The nitrogen and sulphur heteroatoms may optionally be oxidised (*i.e.,* N→O and S(O)ₚ, where p = 1 or 2). It is to be noted that total number of S and O atoms in the aromatic heterocycle is not more than 1. Examples of heteroaryl groups include pyrrole, furan, thiophene, thiazole, isothiazole, imidazole, triazole, tetrazole, pyrazole, oxazole, isoxazole, pyridine, pyrazine, pyridazine, pyrimidine, and the like. Heteroaryl groups can also be fused or bridged with alicyclic or heterocyclic rings, which are not aromatic so as to form a multicyclic system (e.g., 4,5,6,7-tetrahydrobenzo[c]isoxazolyl).

Furthermore, the terms "aryl" and "heteroaryl" include multicyclic aryl and heteroaryl groups, *e.g.,* tricyclic, bicyclic, *e.g.,* naphthalene, benzoxazole, benzodioxazole, benzothiazole, benzoimidazole, benzothiophene, quinoline, isoquinoline, naphthrydine, indole, benzofuran, purine, benzofuran, deazapurine, indolizine.

The cycloalkyl, heterocycloalkyl, aryl, or heteroaryl ring can be substituted at one or more ring positions (e.g., the ring-forming carbon or heteroatom such as N) with such substituents as described above, for example, alkyl, alkenyl, alkynyl, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulphhydryl, alkylthio, arylthio, thiocarboxylate, sulphates, alkylsulphinyl, sulphonato, sulphamoyl, sulphonamido, nitro, trifluoromethyl, cyano, azido, heterocycloalkyl, alkylaryl, or an aromatic or heteroaromatic moiety. Aryl and heteroaryl groups can also be fused or bridged with alicyclic or heterocyclic rings, which are not aromatic so as to form a multicyclic system (e.g., tetralin, methylenedioxyphenyl such as benzo[d][1,3]dioxole-5-yl).

As used herein, the term "substituted," means that any one or more hydrogen atoms on the designated atom is replaced with a selection from the indicated groups, provided that the designated atom's normal valency is not exceeded, and that the substitution results in a stable compound. When a substituent is oxo or keto *(i.e.,* =O), then 2 hydrogen atoms on the atom are replaced. Keto substituents are not present on aromatic moieties. Ring double bonds, as used herein, are double bonds that are formed between two adjacent ring atoms (e.g., C=C, C=N or N=N). "Stable compound" and "stable structure" are meant to indicate a compound that is sufficiently robust to survive isolation to a useful degree of purity from a reaction mixture, and formulation into an efficacious therapeutic agent.

When a bond to a substituent is shown to cross a bond connecting two atoms in a ring, then such substituent may be bonded to any atom in the ring. When a substituent is listed without indicating the atom via which such substituent is bonded to the rest of the compound of a given formula, then such substituent may be bonded via any atom in such formula. Combinations of substituents and/or variables are permissible, but only if such combinations result in stable compounds.

When any variable (e.g., R) occurs more than one time in any constituent or formula for a compound, its definition at each occurrence is independent of its definition at every other occurrence. Thus, for example, if a group is shown to be substituted with 0-2 R moieties, then the group may optionally be substituted with up to two R moieties and R at each occurrence is selected independently from the definition of R. Also, combinations of substituents and/or variables are permissible, but only if such combinations result in stable compounds.

As used herein, the term "hydroxy" or "hydroxyl" includes groups with an -OH or - O⁻.

As used herein, the term "cyano" refers to the group -CN.

As used herein, the term "halo" or "halogen" refers to fluoro, chloro, bromo and iodo.

As used herein, the term "haloalkyl" refers to a branched or unbranched alkyl substituted with one or more halogens. For example, a C₁₋₇haloalkyl is an alkyl group of from one to seven cabons wherein at least one H is substituted by a halogen. Examples of haloalkyl include but are not limited to CFH₂, CF₂H, CF₃, CH₂CF₃, CF₂CF₃, C(F)(CH₃)₂, CH₂CH₂Br, CH(I)CH₂F, and CH₂Cl.

As used herein, the term "optionally substituted haloalkyl" refers to unsubstituted haloalkyl having designated substituents replacing one or more hydrogen atoms on one or more hydrocarbon backbone carbon atoms. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), am idino, imino, sulphhydryl, alkylthio, arylthio, thiocarboxylate, sulphates, alkylsulphinyl, sulphonato, sulphamoyl, sulphonamido, nitro, trifluoromethyl, cyano, azido, heterocycloalkyl, alkylaryl, or an aromatic or heteroaromatic moiety.

As used herein, the expressions "one or more of A, B, or C," "one or more A, B, or C," "one or more of A, B, and C," "one or more A, B, and C," "selected from the group consisting of A, B, and C", "selected from A, B, and C", and the like are used interchangeably and all refer to a selection from a group consisting of A, B, and/or C, i.e., one or more As, one or more Bs, one or more Cs, or any combination thereof, unless indicated otherwise.

It is to be understood that the present disclosure provides methods for the synthesis of the compounds of any of the Formulae described herein. The present disclosure also provides detailed methods for the synthesis of various disclosed compounds of the present disclosure according to the following schemes as well as those shown in the Examples.

It is to be understood that, throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

It is to be understood that the synthetic processes of the disclosure can tolerate a wide variety of functional groups, therefore various substituted starting materials can be used. The processes generally provide the desired final compound at or near the end of the overall process, although it may be desirable in certain instances to further convert the compound to a pharmaceutically acceptable salt thereof.

It is to be understood that compounds of the present disclosure can be prepared in a variety of ways using commercially available starting materials, compounds known in the literature, or from readily prepared intermediates, by employing standard synthetic methods and procedures either known to those skilled in the art, or which will be apparent to the skilled artisan in light of the teachings herein. Standard synthetic methods and procedures for the preparation of organic molecules and functional group transformations and manipulations can be obtained from the relevant scientific literature or from standard textbooks in the field. Although not limited to any one or several sources, classic texts such as Smith, M. B., March, J., March's Advanced Organic Chemistry: Reactions, Mechanisms, and Structure, 5th edition, John Wiley & Sons: New York, 2001; Greene, T.W., Wuts, P.G. M., Protective Groups in Organic Synthesis, 3rd edition, John Wiley & Sons: New York, 1999; R. Larock, Comprehensive Organic Transformations, VCH Publishers (1989); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), are useful and recognised reference textbooks of organic synthesis known to those in the art

One of ordinary skill in the art will note that, during the reaction sequences and synthetic schemes described herein, the order of certain steps may be changed, such as the introduction and removal of protecting groups. One of ordinary skill in the art will recognise that certain groups may require protection from the reaction conditions via the use of protecting groups. Protecting groups may also be used to differentiate similar functional groups in molecules. A list of protecting groups and how to introduce and remove these groups can be found in Greene, T.W., Wuts, P.G. M., Protective Groups in Organic Synthesis, 3rd edition, John Wiley & Sons: New York, 1999.

It is to be understood that, unless otherwise stated, any description of a method of treatment includes use of the compounds to provide such treatment or prophylaxis as is described herein, as well as use of the compounds to prepare a medicament to treat or prevent such condition. It is to be understood that, unless otherwise stated, any description of a method of treatment includes use of the compounds to provide such treatment or prophylaxis as is described herein, as well as use of the compounds to prepare a medicament to treat such condition. The treatment includes treatment of human or non-human animals including rodents and other disease models.

As used herein, the term "subject" is interchangeable with the term "subject in need thereof", both of which refer to a subject having a disease or having an increased risk of developing the disease. A "subject" includes a mammal. The mammal can be *e.g.,* a human or appropriate non-human mammal, such as primate, mouse, rat, dog, cat, cow, horse, goat, camel, sheep or a pig. The subject can also be a bird or fowl. In one embodiment, the mammal is a human. A subject in need thereof can be one who has been previously diagnosed or identified as having a disease or disorder disclosed herein. A subject in need thereof can also be one who is suffering from a disease or disorder disclosed herein. Alternatively, a subject in need thereof can be one who has an increased risk of developing such disease or disorder relative to the population at large (i.e., a subject who is predisposed to developing such disorder relative to the population at large). A subject in need thereof can have a refractory or resistant a disease or disorder disclosed herein (i.e., a disease or disorder disclosed herein that does not respond or has not yet responded to treatment). The subject may be resistant at start of treatment or may become resistant during treatment. In some embodiments, the subject in need thereof received and failed all known effective therapies for a disease or disorder disclosed herein. In some embodiments, the subject in need thereof received at least one prior therapy.

As used herein, the term "treating" or "treat" describes the management and care of a subject for the purpose of combating a disease, condition, or disorder and includes the administration of a compound of the present disclosure, or a pharmaceutically acceptable salt thereof, to alleviate the symptoms or complications of a disease, condition or disorder, or to eliminate the disease, condition or disorder. The term "treat" can also include treatment of a cell *in vitro* or an animal model.

It is to be appreciated that references to "treating" or "treatment" include the alleviation of established symptoms of a condition. "Treating" or "treatment" of a state, disorder or condition therefore includes: (1) delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

It is to be understood that a compound of the present disclosure, or a pharmaceutically acceptable salt thereof, can or may also be used to prevent a relevant disease, condition or disorder, or used to identify suitable candidates for such purposes.

As used herein, the term "preventing," "prevent," or "protecting against" describes reducing or eliminating the onset of the symptoms or complications of such disease, condition or disorder.

It is to be understood that one skilled in the art may refer to general reference texts for detailed descriptions of known techniques discussed herein or equivalent techniques. These texts include Ausubel et al., Current Protocols in Molecular Biology, John Wiley and Sons, Inc. (2005); Sambrook et al., Molecular Cloning, A Laboratory Manual (3rd edition), Cold Spring Harbor Press, Cold Spring Harbor, New York (2000); Coligan et al., Current Protocols in Immunology, John Wiley & Sons, N.Y.; Enna et al., Current Protocols in Pharmacology, John Wiley & Sons, N.Y.; Fingl et al., The Pharmacological Basis of Therapeutics (1975), Remington's Pharmaceutical Sciences, Mack Publishing Co., Easton, PA, 18th edition (1990). These texts can, of course, also be referred to in making or using an aspect of the disclosure.

It is to be understood that the present disclosure also provides pharmaceutical compositions comprising any compound described herein in combination with at least one pharmaceutically acceptable excipient or carrier.

As used herein, the term "pharmaceutical composition" is a formulation containing the compounds of the present disclosure in a form suitable for administration to a subject. In one embodiment, the pharmaceutical composition is in bulk or in unit dosage form. The unit dosage form is any of a variety of forms, including, for example, a capsule, an IV bag, a tablet, a single pump on an aerosol inhaler or a vial. The quantity of active ingredient (e.g., a formulation of the disclosed compound or salt, hydrate, solvate or isomer thereof) in a unit dose of composition is an effective amount and is varied according to the particular treatment involved. One skilled in the art will appreciate that it is sometimes necessary to make routine variations to the dosage depending on the age and condition of the subject. The dosage will also depend on the route of administration. A variety of routes are contemplated, including oral, pulmonary, rectal, parenteral, transdermal, subcutaneous, intravenous, intramuscular, intraperitoneal, inhalational, buccal, sublingual, intrapleural, intrathecal, intranasal, and the like. Dosage forms for the topical or transdermal administration of a compound of this disclosure include powders, sprays, ointments, pastes, creams, lotions, gels, solutions, patches and inhalants. In one embodiment, the active compound is mixed under sterile conditions with a pharmaceutically acceptable carrier, and with any preservatives, buffers, or propellants that are required.

As used herein, the term "pharmaceutically acceptable" refers to those compounds, anions, cations, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

It is to be understood that a pharmaceutical composition of the disclosure is formulated to be compatible with its intended route of administration. Examples of routes of administration include parenteral, *e.g.,* intravenous, intradermal, subcutaneous, oral *(e.g.,* ingestion), inhalation, transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulphite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

It is to be understood that a compound or pharmaceutical composition of the disclosure can be administered to a subject in many of the well-known methods currently used for chemotherapeutic treatment. For example, a compound of the disclosure may be injected into the blood stream or body cavities or taken orally or applied through the skin with patches. The dose chosen should be sufficient to constitute effective treatment but not so high as to cause unacceptable side effects. The state of the disease condition (e.g., a disease or disorder disclosed herein) and the health of the subject should preferably be closely monitored during and for a reasonable period after treatment.

As used herein, the term "therapeutically effective amount", refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician.

A "therapeutically effective amount" means the amount of a compound that, when administered to a subject for treating a disease, is sufficient to effect such treatment for the disease. The "therapeutically effective amount" will vary depending on the compound, the disease and its severity and the age, weight, etc., of the subject to be treated.

It is to be understood that, for any compound, the therapeutically effective amount can be estimated initially either in cell culture assays, e.g., of neoplastic cells, or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between toxic and therapeutic effects is the therapeutic index, and it can be expressed as the ratio, LD₅₀/ED₅₀. Pharmaceutical compositions that exhibit large therapeutic indices are preferred. The dosage may vary within this range depending upon the dosage form employed, sensitivity of the subject, and the route of administration.

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy.

The pharmaceutical compositions containing active compounds of the present disclosure may be manufactured in a manner that is generally known, e.g., by means of conventional mixing, dissolving, granulating, dragee-making, levigating, emulsifying, encapsulating, entrapping, or lyophilising processes. Pharmaceutical compositions may be formulated in a conventional manner using one or more pharmaceutically acceptable carriers comprising excipients and/or auxiliaries that facilitate processing of the active compounds into preparations that can be used pharmaceutically. Of course, the appropriate formulation is dependent upon the route of administration chosen.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol and sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilisation. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible pharmaceutically acceptable carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or com starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser, which contains a suitable propellant, *e.g.,* a gas such as carbon dioxide, or a nebuliser.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The active compounds can be prepared with pharmaceutically acceptable carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the particular therapeutic effect to be achieved.

In therapeutic applications, the dosages of the pharmaceutical compositions used in accordance with the disclosure vary depending on the agent, the age, weight, and clinical condition of the recipient subject, and the experience and judgment of the clinician or practitioner administering the therapy, among other factors affecting the selected dosage. Generally, the dose should be sufficient to result in slowing, and preferably regressing, the symptoms of the disease or disorder disclosed herein and also preferably causing complete regression of the disease or disorder. An effecti ve amount of a pharmaceutical agent is that which provides an objectively identifiable improvement as noted by the clinician or other qualified observer. Improvement in survival and growth indicates regression. As used herein, the term "dosage effective manner" refers to amount of an active compound to produce the desired biological effect in a subject or cell.

It is to be understood that the pharmaceutical compositions can be included in a container, pack, or dispenser together with instructions for administration.

It is to be understood that, for the compounds of the present disclosure being capable of further forming salts, all of these forms are also contemplated within the scope of the claimed disclosure.

As used herein, the term "pharmaceutically acceptable salts" refer to derivatives of the compounds of the present disclosure wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulphonic, acetic, ascorbic, benzene sulphonic, benzoic, bicarbonic, carbonic, citric, edetic, ethane disulphonic, 1,2-ethane sulphonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulphonic, maleic, malic, mandelic, methane sulphonic, napsylic, nitric, oxalic, pamoic, pantothenic, phenylacetic, phosphoric, polygalacturonic, propionic, salicylic, stearic, subacetic, succinic, sulphamic, sulphanilic, sulphuric, tannic, tartaric, toluene sulphonic, and the commonly occurring amine acids, e.g., glycine, alanine, phenylalanine, arginine, etc.

In some embodiments, the pharmaceutically acceptable salt is a sodium salt, a potassium salt, a calcium salt, a magnesium salt, a diethylamine salt, a choline salt, a meglumine salt, a benzathine salt, a tromethamine salt, an ammonia salt, an arginine salt, or a lysine salt.

Other examples of pharmaceutically acceptable salts include hexanoic acid, cyclopentane propionic acid, pyruvic acid, malonic acid, 3-(4-hydroxybenzoyl)benzoic acid, cinnamic acid, 4-chlorobenzenesulphonic acid, 2-naphthalenesulphonic acid, 4-toluenesulphonic acid, camphorsulphonic acid, 4-methylbicyclo-[2.2.2]-oct-2-ene-1-carboxylic acid, 3-phenylpropionic acid, trimethylacetic acid, tertiary butylacetic acid, muconic acid, and the like. The present disclosure also encompasses salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, N-methylglucamine, and the like. In the salt form, it is understood that the ratio of the compound to the cation or anion of the salt can be 1:1, or any ratio other than 1:1, e.g., 3:1, 2:1, 1:2, or 1:3.

The compounds, or pharmaceutically acceptable salts thereof, are administered orally, nasally, transdermally, pulmonary, inhalationally, buccally, sublingually, intraperitoneally, subcutaneously, intramuscularly, intravenously, rectally, intrapleurally, intrathecally and parenterally. In one embodiment, the compound is administered orally. One skilled in the art will recognise the advantages of certain routes of administration.

A salt, for example, can be formed between an anion and a positively charged group (e.g., amino) on a substituted compound disclosed herein. Suitable anions include chloride, bromide, iodide, sulphate, bisulphate, sulphamate, nitrate, phosphate, citrate, methanesulphonate, trifluoroacetate, glutamate, glucuronate, glutarate, malate, maleate, succinate, fumarate, tartrate, tosylate, salicylate, lactate, naphthalenesulphonate, and acetate (e.g., trifluoroacetate).

As used herein, the term "pharmaceutically acceptable anion" refers to an anion suitable for forming a pharmaceutically acceptable salt. Likewise, a salt can also be formed between a cation and a negatively charged group (e.g., carboxylate) on a substituted compound disclosed herein. Suitable cations include sodium ion, potassium ion, magnesium ion, calcium ion, and an ammonium cation such as tetramethylammonium ion or diethylamine ion. The substituted compounds disclosed herein also include those salts containing quaternary nitrogen atoms.

It is to be understood that the compounds of the present disclosure, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

As used herein, the term "solvate" means solvent addition forms that contain either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as H₂O.

Reference to Formula (I) can include subformulas of Formula (I), e.g., Formulas (Ix), (Ia), (Ib), (Ic), (Id), (Ie), (If), (Ig), (Ih), (Ii), (Ij), (Ik), (Im), (In), (Io), (Ip), and (Iq).

Compounds of any one of the Formulae disclosed herein may exist in a number of different tautomeric forms and references to compounds of Formula (I) include all such forms. For the avoidance of doubt, where a compound can exist in one of several tautomeric forms, and only one is specifically described or shown, all others are nevertheless embraced by Formula (I). Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

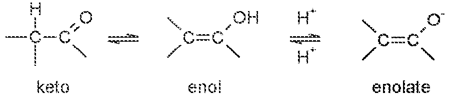

Compounds of any one of the Formulae disclosed herein containing an amine function may also form N-oxides. A reference herein to a compound of Formula (I) that contains an amine function also includes the N-oxide. Where a compound contains several amine functions, one or more than one nitrogen atom may be oxidised to form an N-oxide. Particular examples of N-oxides are the N-oxides of a tertiary amine or a nitrogen atom of a nitrogen-containing heterocycle. N-oxides can be formed by treatment of the corresponding amine with an oxidising agent such as hydrogen peroxide or a peracid (e.g. a peroxycarboxylic acid), see for example Advanced Organic Chemistry, by Jerry March, 4th Edition, Wiley Interscience, pages. More particularly, N-oxides can be made by the procedure of L. W. Deady (Syn. Comm. 1977, 7, 509-514) in which the amine compound is reacted with meta-chloroperoxybenzoic acid (mCPBA), for example, in an inert solvent such as dichloromethane.

As used herein, the term "isomerism" means compounds that have identical molecular formulae but differ in the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images of each other are termed "enantiomers" or sometimes optical isomers. A mixture containing equal amounts of individual enantiomeric forms of opposite chirality is termed a "racemic mixture."

As used herein, the term "chiral center" refers to a carbon atom bonded to four nonidentical substituents.

As used herein, the term "chiral isomer" means a compound with at least one chiral center. Compounds with more than one chiral center may exist either as an individual diastereomer or as a mixture of diastereomers, termed "diastereomeric mixture." When one chiral center is present, a stereoisomer may be characterised by the absolute configuration (R or S) of that chiral center. Absolute configuration refers to the arrangement in space of the substituents attached to the chiral center. The substituents attached to the chiral center under consideration are ranked in accordance with the *Sequence Rule* of Cahn, Ingold and Prelog. (Cahn et al., Angew. Chem. Inter. Edit. 1966, 5, 385; errata 511; Cahn et al., Angew. Chem. 1966, 78, 413; Cahn and Ingold, J Chem. Soc. 1951 (London), 612; Cahn et al., Experientia 1956, 12, 81; Cahn, J Chem. Educ. 1964, 41, 116).

As used herein, the term "geometric isomer" means the diastereomers that owe their existence to hindered rotation about double bonds or a cycloalkyl linker (e.g., 1,3-cyclobutyl). These configurations are differentiated in their names by the prefixes cis and trans, or Z and E, which indicate that the groups are on the same or opposite side of the double bond in the molecule according to the Cahn-Ingold-Prelog rules.

It is to be understood that the compounds of the present disclosure may be depicted as different chiral isomers or geometric isomers. It is also to be understood that when compounds have chiral isomeric or geometric isomeric forms, all isomeric forms are intended to be included in the scope of the present disclosure, and the naming of the compounds does not exclude any isomeric forms, it being understood that not all isomers may have the same level of activity.

It is to be understood that the structures and other compounds discussed in this disclosure include all atropic isomers thereof. It is also to be understood that not all atropic isomers may have the same level of activity.

As used herein, the term "atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

As used herein, the term "tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerisation is possible, a chemical equilibrium of the tautomers will be reached. The exact ratio of the tautomers depends on several factors, including temperature, solvent and pH. The concept of tautomers that are interconvertible by tautomerisations is called tautomerism. Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

It is to be understood that the compounds of the present disclosure may be depicted as different tautomers. It should also be understood that when compounds have tautomeric forms, all tautomeric forms are intended to be included in the scope of the present disclosure, and the naming of the compounds does not exclude any tautomer form. It will be understood that certain tautomers may have a higher level of activity than others.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterised by the absolute configuration of its asymmetric center and is described by the R- and S-sequencing rules of Cahn and Prelog, or by the manner in which the molecule rotates the plane of polarised light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The compounds of this disclosure may possess one or more asymmetric centers; such compounds can therefore be produced as individual (R)- or (S)-stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the disclosure may have geometric isomeric centers (E- and Z- isomers).

Accordingly, the present disclosure includes those compounds of any one of the Formulae disclosed herein as defined hereinbefore when made available by organic synthesis and when made available within the human or animal body by way of cleavage of a prodrug thereof. Accordingly, the present disclosure includes those compounds of any one of the Formulae disclosed herein that are produced by organic synthetic means and also such compounds that are produced in the human or animal body by way of metabolism of a precursor compound, that is a compound of any one of the Formulae disclosed herein may be a synthetically-produced compound or a metabolically-produced compound.

The dosage regimen utilising the compounds is selected in accordance with a variety of factors including type, species, age, weight, sex and medical condition of the subject; the severity of the condition to be treated; the route of administration; the renal and hepatic function of the subject; and the particular compound or salt thereof employed. An ordinarily skilled physician or veterinarian can readily determine and prescribe the effective amount of the drug required to prevent, counter, or arrest the progress of the condition. An ordinarily skilled physician or veterinarian can readily determine and prescribe the effective amount of the drug required to counter or arrest the progress of the condition.

Techniques for formulation and administration of the disclosed compounds of the disclosure can be found in Remington: the Science and Practice of Pharmacy, 19th edition, Mack Publishing Co., Easton, PA (1995). In an embodiment, the compounds described herein, and the pharmaceutically acceptable salts thereof, are used in pharmaceutical preparations in combination with a pharmaceutically acceptable carrier or diluent. Suitable pharmaceutically acceptable carriers include inert solid fillers or diluents and sterile aqueous or organic solutions. The compounds will be present in such pharmaceutical compositions in amounts sufficient to provide the desired dosage amount in the range described herein.

All percentages and ratios used herein, unless otherwise indicated, are by weight. Other features and advantages of the present disclosure are apparent from the different examples. The provided examples illustrate different components and methodology useful in practicing the present disclosure. The examples do not limit the claimed disclosure. Based on the present disclosure the skilled artisan can identify and employ other components and methodology useful for practicing the present disclosure.

In the synthetic schemes described herein, compounds may be drawn with one particular configuration for simplicity. Such particular configurations are not to be construed as limiting the disclosure to one or another isomer, tautomer, regioisomer or stereoisomer, nor does it exclude mixtures of isomers, tautomers, regioisomers or stereoisomers; however, it will be understood that a given isomer, tautomer, regioisomer or stereoisomer may have a higher level of activity than another isomer, tautomer, regioisomer or stereoisomer.

The invention having now been described by way of written description, those of skill in the art will recognize that the invention can be practiced in a variety of embodiments and that the foregoing description and examples below are for purposes of illustration and not limitation of the claims that follow.

As use herein, the phrase "compound of the disclosure" refers to those compounds which are disclosed herein, both generically and specifically.

### Compounds of the Present Disclosure

In some aspects, the present disclosure provides, inter alia, a compound of formula (I): 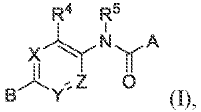 or a pharmaceutically acceptable salt thereof, wherein:
X=N or CR¹; Y=N or CR²; Z=N or CR³;
R¹, R², R³and R⁴ are each independently selected from the group consisting of H, halogen, hydroxy, cyano, -COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₁-C₆cycloalkyloxy, C₃-C₈cycloalkyl, NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy, are optionally substituted with one or more halogen, hydroxyl, methoxy, C₃-C₈cycloalkyl, or NH₂;
R⁵ is H, C₁-C₆alkyl, or C₁-C₆haloalkyl;
A is selected from the group consisting of 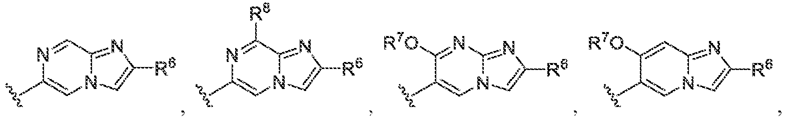 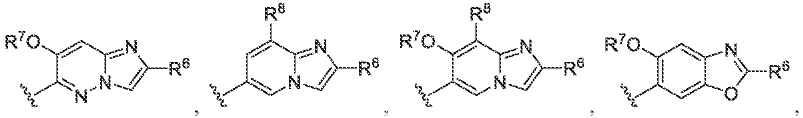 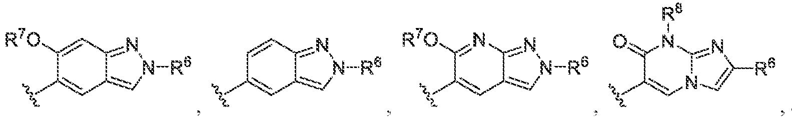 and 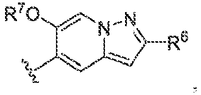
wherein A is optionally substituted with 1-4 R⁹;
R⁶ is C₁-C₃alkyl or C₁-C₃haloalkyl;
R⁷ is H, C₁-C₆alkyl, C₁-C₆haloalkyl, C₁-C₆cycloalkyl, or heterocycloalkyl, wherein heterocycloalkyl is optionally substituted with 1-3 substituents independently selected from halogen and C₁-C₆alkyl;
R⁸ is halogen, C₁-C₆alkyl, C₁-C₆alkoxy, C₁-C₆haloalkyl, or C₁-C₆cycloalkyl;
each R⁹ is independently selected from the group consisting of halogen, hydroxy, cyano, - COOH, -C(O)-C₁-C₆alkyl, -C(O)O-C₁-C₆alkyl. C₁-C₆alkyl, C₂-C₆alkenyl, C₂-C₆alkynyl, C₁-C₆alkoxy, C₃-C₈cycloalkyloxy, C₃-C₈cycloalkyl, NH₂, NH(C₁-C₆alkyl), N(C₁-C₆alkyl)₂, - NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl, methoxy, C₃-C₈cycloalkyl, or NH₂;
B is heterocycloalkyl not linked to formula (I) by a nitrogen atom, optionally substituted with 1 to 6 R¹²; or
B is NR¹⁰R¹¹, wherein
R¹⁰ is -(CH₂)₀₋₃aryl, -(CH₂)₀₋₃heteroaryl, -(CH₂)₀₋₃heterocycloalkyl comprising at least 1 nitrogen ring atom, or C₁-C₈heteroalkyl comprising at least one nitrogen atom, each R¹⁰ optionally substituted with 1 to 6 R¹²; and
R¹¹ is hydrogen, C₁₋₇alkyl, C₁₋₇haloalkyl, or C₃₋₈cycloalkyl; or
R¹⁰ and R¹¹ are taken together with the nitrogen atom to which they are attached to form a heterocycloalkyl comprising 1, 2 or 3 total nitrogen ring atoms and 0 or 1 additional ring heteroatoms selected from O and S, and the heterocycloalkyl is optionally substituted with 1 to 6 R¹²;
each R¹² is independently selected from the group consisting of halogen, hydroxy, cyano, - COOH, -C(O)-C₁-C₆alkyl, -C(O)O-C₁-C₆alkyl, C₁-C₆alkyl, C₁-C₆heteroalkyl, C₂-C₆alkenyl, C₂-C₆alkynyl, C₁-C₆alkoxy, -(CH₂)₀₋₂-C₃-C₈cycloalkyl, -(CH₂)₀₋₂-SO₂-C₁-C₆alkyl, C₁-C₆heteroalkylene-C₃-C₈cycloalkyl, -O-C₃-C₈cycloalkyl, -4-7-membered monocyclic heterocycloalkyl, C₁-C₆heteroalkylene-(4-7-membered monocyclic heterocycloalkyl), -O-(4-7-membered monocyclic heterocycloalkyl), -(CH₂)₀₋₂-(4-7-membered monocyclic heterocycloalkyl), NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl or NH₂, and wherein the cycloalkyl and heterocycloalkyl are optionally substituted with one or more halogen, hydroxyl, C₁-C₆alkyl, C₁-C₆heteroalkyl, C₁-C₆alkoxy, or NH₂; or
two R¹² on the same carbon can be taken together as keto (=O).

In some aspects, the present disclosure provides, inter alia, a compound of formula (I): 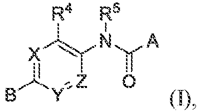 or a pharmaceutically acceptable salt thereof, wherein:
X=N or CR¹; Y=N or CR²; Z=N or CR³;
R¹, R², R³ and R⁴ are each independently selected from the group consisting of H, halogen, hydroxy, cyano, -COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₁-C₆cycloalkyloxy, C₃-C₈cycloalkyl, NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy, are optionally substituted with one or more halogen, hydroxyl, methoxy, C₃-C₈cycloalkyl, or NH₂;
R⁵ is H, C₁-C₆alkyl, or C₁-C₆haloalkyl;
A is selected from the group consisting of 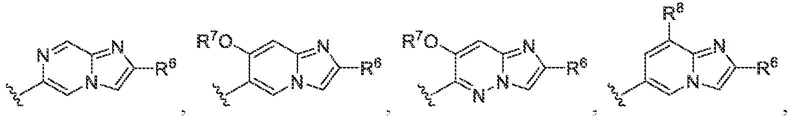 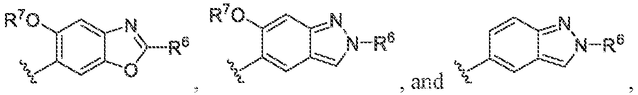
wherein A is optionally substituted with 1-4 R⁹;
R⁶ is C₁-C₃alkyl or C₁-C₃haloalkyl;
R⁷ is H, C₁-C₆alkyl, C₁-C₆haloalkyl, C₁-C₆cycloalkyl, or heterocycloalkyl, wherein heterocycloalkyl is optionally substituted with 1-3 substituents independently selected from halogen and C₁-C₆alkyl;
R⁸ is halogen, C₁-C₆alkyl, C₁-C₆alkoxy, C₁-C₆haloalkyl, or C₁-C₆cycloalkyl;
each R⁹ is independently selected from the group consisting of halogen, hydroxy, cyano, - COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₃-C₈cycloalkyloxy, C₃-C₈cycloalkyl, NH₂, NH(C₁-C₆alkyl), N(C₁-C₆alkyl)₂, - NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl, methoxy, C₃-C₈cycloalkyl, or NH₂;
B is NR¹⁰R¹¹, wherein
R¹⁰ is -(CH₂)₀₋₁heterocycloalkyl comprising 1 nitrogen ring atom, optionally substituted with 1 to 6 R¹²; and
R¹¹ is hydrogen, C₁₋₇alkyl, C₁₋₇haloalkyl, or C₃₋₈cycloalkyl; or
R¹⁰ and R¹¹ are taken together with the nitrogen atom to which they are attached to form a heterocycloalkyl comprising 1, 2 or 3 total nitrogen ring atoms and 0 or 1 additional ring heteroatoms selected from O and S, and the heterocycloalkyl is optionally substituted with 1 to 6 R¹²;
   each R¹² is independently selected from the group consisting of halogen, hydroxy, cyano, -COOH, -C(O)-C₁-C₆alkyl, -C(O)O-C₁-C₆alkyl, C₁-C₆alkyl, C₁-C₆heteroalkyl, C₂-C₆alkenyl, C₂-C₆alkynyl, C₁-C₆alkoxy, -(CH₂)₀₋₂-C₃-C₈cycloalkyl, C₁-C₆heteroalkylene-C₃-C₈cycloalkyl, -O-C₃-C₈cycloalkyl, 4-7-membered monocyclic heterocycloalkyl, C₁-C₆heteroalkylene-(4-7-membered monocyclic heterocycloalkyl), -O-(4-7-membered monocyclic heterocycloalkyl), -(CH₂)₀₋₂-(4-7-membered monocyclic heterocycloalkyl), NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, - C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl or NH₂, and wherein the cycloalkyl and heterocycloalkyl are optionally substituted with one or more halogen, hydroxyl, C₁-C₆alkyl, C₁-C₆heteroalkyl, C₁-C₆alkoxy, or NH₂; or two R¹² on the same carbon can be taken together as keto (=O).

In some aspects, the present disclosure provides, *inter alia,* a compound of Formula (I): 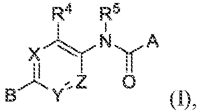 or a pharmaceutically acceptable salt thereof, wherein:
X=N or CR¹; Y=N or CR²; Z=N or CR³;
R¹, R², R³ and R⁴ are each independently selected from the group consisting of H, halogen, hydroxy, cyano, -COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₁-C₆cycloalkyloxy, C₃-C₈cycloalkyl, NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy, are optionally substituted with one or more halogen, hydroxyl, methoxy, C₃-C₈cycloalkyl, or NH₂;
R⁵ is H, C₁-C₆alkyl, or C₁-C₆haloalkyl;
A is selected from the group consisting of 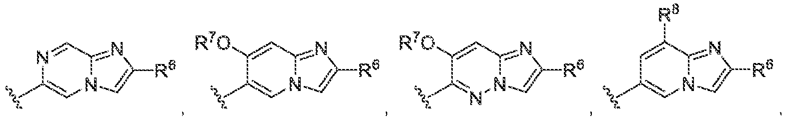 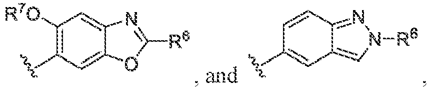
wherein A is optionally substituted with 1-4 R⁹;
R⁶ is C₁-C₃alkyl or C₁-C₃haloalkyl;
R⁷ is H, C₁-C₆alkyl, C₁-C₆haloalkyl, C₁-C₆cycloalkyl, or heterocycloalkyl;
R⁸ is halogen, C₁-C₆alkyl, C₁-C₆alkoxy, C₁-C₆haloalkyl, or C₁-C₆cycloalkyl;
each R⁹ is independently selected from the group consisting of halogen, hydroxy, cyano, - COOH, -C(O)-C₁-C₆alkyl, -C(O)O-C₁-C₆alkyl. C₁-C₆alkyl, C₂-C₆alkenyl, C₂-C₆alkynyl, C₁-C₆alkoxy, C₃-C₈cycloalkyloxy, C₃-C₈cycloalkyl, NH₂, NH(C₁-C₆alkyl), N(C₁-C₆alkyl)₂, - NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl, methoxy, C₃-C₈cycloalkyl, or NH₂;
B is NR¹⁰R¹¹, wherein
R¹⁰ is -(CH₂)₀₋₁heterocycloalkyl comprising 1 nitrogen ring atom, optionally substituted with 1 to 6 R¹²; and
R¹¹ is hydrogen, C₁₋₇alkyl, C₁₋₇haloalkyl, or C₃₋₈cycloalkyl; or
R¹⁰ and R¹¹ are taken together with the nitrogen atom to which they are attached to form a heterocycloalkyl comprising 1, 2 or 3 total nitrogen ring atoms and 0 or 1 additional ring heteroatoms selected from O and S, and the heterocycloalkyl is optionally substituted with 1 to 6 R¹²;
   each R¹² is independently selected from the group consisting of halogen, hydroxy, cyano, -COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, -(CH₂)₀₋₂-C₃-C₈cycloalkyl, 5-6-membered monocyclic heterocycloalkyl, NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl or NH₂, and wherein the cycloalkyl and heterocycloalkyl are optionally substituted with one or more halogen, hydroxyl, C₁-C₆alkyl, C₁-C₆alkoxy, or NH₂.

In some aspects, the present disclosure provides, inter alia, a compound of formula (Ix): 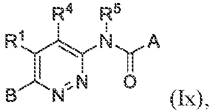 or a pharmaceutically acceptable salt thereof, wherein:
R¹ and R⁴ are each independently selected from the group consisting of H, halogen, hydroxy, cyano, -COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₁-C₆cycloalkyloxy, C₃-C₈cycloalkyl, NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy, are optionally substituted with one or more halogen, hydroxyl, methoxy, C₃-C₈cycloalkyl, or NH₂;
R⁵ is H, C₁-C₆alkyl, or C₁-C₆haloalkyl;
A is selected from the group consisting of 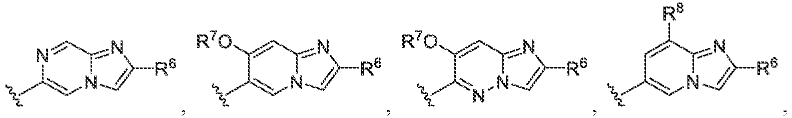 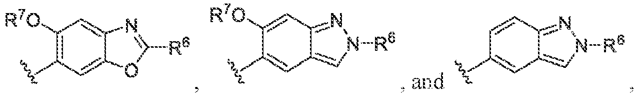
wherein A is optionally substituted with 1-4 R⁹;
R⁶ is C₁-C₃alkyl or C₁-C₃haloalkyl;
R⁷ is H, C₁-C₆alkyl, C₁-C₆haloalkyl, C₁-C₆cycloalkyl, or heterocycloalkyl, wherein heterocycloalkyl is optionally substituted with 1-3 substituents independently selected from halogen and C₁-C₆alkyl;
R⁸ is halogen, C₁-C₆alkyl, C₁-C₆alkoxy, C₁-C₆haloalkyl, or C₁-C₆cycloalkyl;
each R⁹ is independently selected from the group consisting of halogen, hydroxy, cyano, - COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₃-C₈cycloalkyloxy, C₃-C₈cycloalkyl, NH₂, NH(C₁-C₆alkyl), N(C₁-C₆alkyl)₂, - NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl, methoxy, C₃-C₈cycloalkyl, or NH₂;
B is NR¹⁰R¹¹, wherein
R¹⁰ is -(CH₂)₀₋₁heterocycloalkyl comprising 1 nitrogen ring atom, optionally substituted with 1 to 6 R¹²; and
R¹¹ is hydrogen, C₁₋₇alkyl, C₁₋₇haloalkyl, or C₃₋₈cycloalkyl; or
R¹⁰ and R¹¹ are taken together with the nitrogen atom to which they are attached to form a monocyclic or bicyclic heterocycloalkyl comprising 1, 2 or 3 total nitrogen ring atoms and 0 or 1 additional ring heteroatoms selected from O and S, and the heterocycloalkyl is optionally substituted with 1 to 6 R¹²;
   each R¹² is independently selected from the group consisting of halogen, hydroxy, cyano, -COOH, -C(O)-C₁-C₆alkyl, -C(O)O-C₁-C₆alkyl, C₁-C₆alkyl, C₁-C₆heteroalkyl, C₂-C₆alkenyl, C₂-C₆alkynyl, C₁-C₆alkoxy, -(CH₂)₀₋₂-C₃-C₈cycloalkyl, C₁-C₆heteroalkylene-C₃-C₈cycloalkyl, -O-C₃-C₈cycloalkyl, 4-7-membered monocyclic heterocycloalkyl, C₁-C₆heteroalkylene-(4-7-membered monocyclic heterocycloalkyl), -O-(4-7-membered monocyclic heterocycloalkyl), -(CH₂)₀₋₂-(4-7-membered monocyclic heterocycloalkyl), NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, - C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl or NH₂, and wherein the cycloalkyl and heterocycloalkyl are optionally substituted with one or more halogen, hydroxyl, C₁-C₆alkyl, C₁-C₆heteroalkyl, C₁-C₆alkoxy, or NH₂; or two R¹² on the same carbon can be taken together as keto (=O).

In some embodiments, 0, 1, or 2 of X, Y, and Z are N.

In some embodiments, the compound is of Formula (Ia), 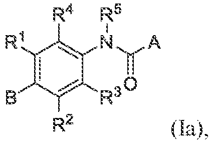 or a pharmaceutically acceptable salt thereof.

In some emboidments, the compound is of Formula (Ib), 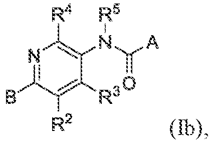 or a pharmaceutically acceptable salt thereof.

In some embodiments, the compound is of Formula (Ic), 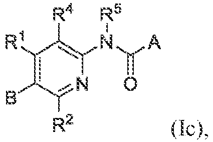 or a pharmaceutically acceptable salt thereof.

In some embodiments, the compound is of Formula (Id), 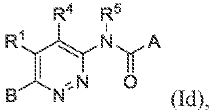 or a pharmaceutically acceptable salt thereof.

In some embodiments, the compound is of Formula (Ie), 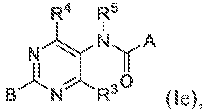 or a pharmaceutically acceptable salt thereof.

In some embodiments, the compound is of Formula (If), 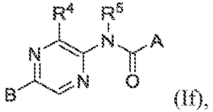 or a pharmaceutically acceptable salt thereof.

In some embodiments, R¹, R², R³ and R⁴ are each independently selected from the group consisting of H, halogen, C₁-C₆alkyl, C₁-C₆haloalkyl, C₁-C₆alkoxy, and C₁-C₆cycloalkyloxy. In some embodiments, R¹, R², R³ and R⁴ are each independently selected from the group consisting of H, halogen, C₁-C₆alkoxy, and C₁-C₆alkyl. In some embodiments, R¹, R², R³ and R⁴ are each H.

In some embodiments, R⁵ is H.

In some embodiments, A is selected from the group consisting of 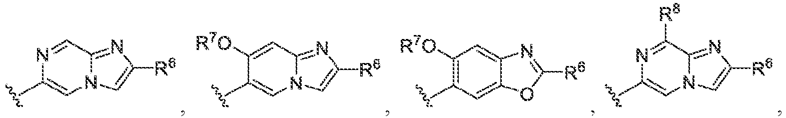 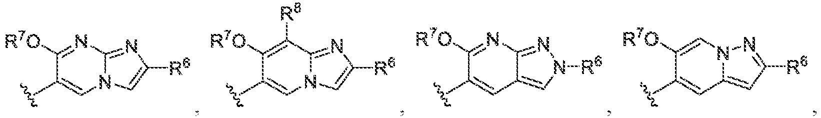 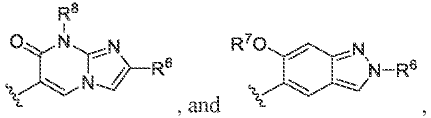 wherein A is optionally substituted with 1-3 R⁹.

In some embodiments, A is selected from the group consisting of 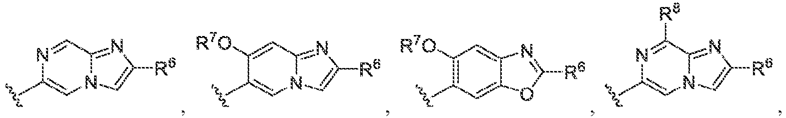 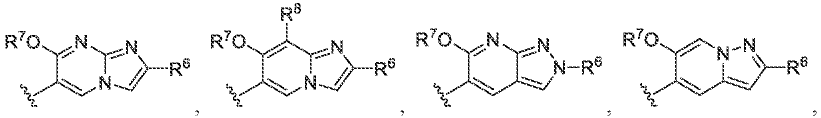 and 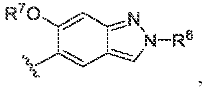 wherein A is optionally substituted with 1-3 R⁹.

In some embodiments, A is selected from the group consisting of 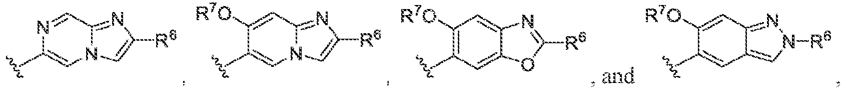 wherein A is optionally substituted with 1-3 R⁹.

In some embodiments, A is substituted by one R⁹ selected from the group consisting of halogen and C₁-C₆alkyl. In some embodiments, A is not substituted by R⁹.

In some embodiments, R⁶ is Me.

In some embodiments, R⁷ is C₁-C₆alkyl, C₁-C₆cycloalkyl, or heterocycloalkyl. In some embodiments, R⁷ is Me, Et, isopropyl, or cyclobutyl.

**In** some embodiments, A is selected from the group consisting of

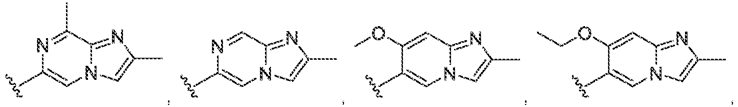

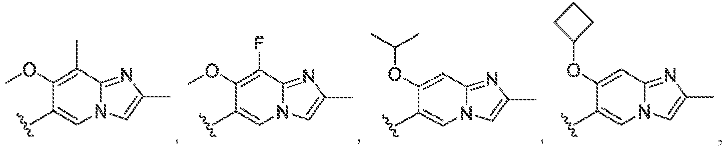

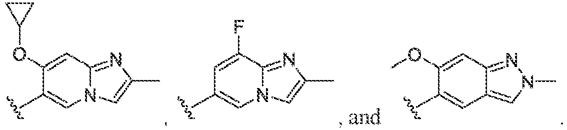

In some embodiments, A is selected from the group consisting of

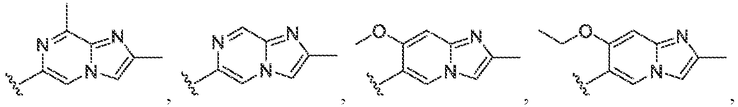

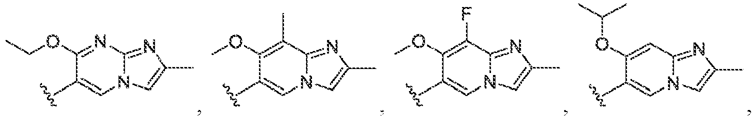

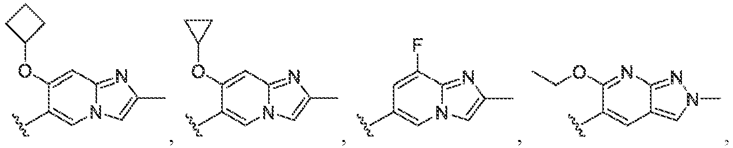

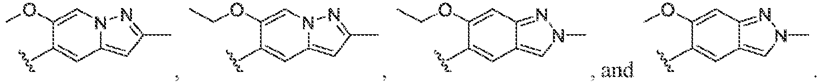

In some embodiments, A is

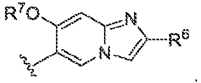

In some embodiments, A is

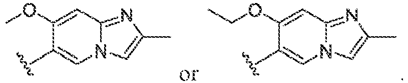

In some embodiments, A is

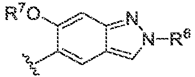

In some embodiments, A is

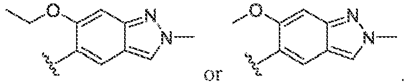

In some embodiments, A is

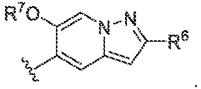

In some embodiments, A is

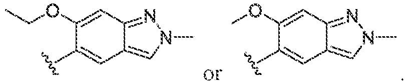

In some embodiments, B is NR¹⁰R¹¹, wherein
R¹⁰ and R¹¹ are taken together with the nitrogen atom to which they are attached to form a heterocycloalkyl comprising 1, 2 or 3 total nitrogen ring atoms and 0 or 1 additional ring heteroatoms selected from O and S, and the heterocycloalkyl is optionally substituted with 1, 2, 3, or 4 R¹².

In some embodiments, R¹⁰ and R¹¹ are taken together with the nitrogen atom to which they are attached to form a monocyclic heterocycloalkyl of 4-7 ring atoms with 1 or 2 total nitrogen ring atoms and 0 or 1 additional ring heteroatoms selected from O and S, and the heterocycloalkyl is optionally substituted with 1, 2, 3, or 4 R¹².
In some embodiments, B is 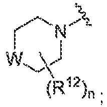
W is NR¹³ or CR¹⁴R¹⁴;
R¹³ is selected from the group consisting of hydrogen, C₁-C₆alkyl, C₂-C₆alkenyl, C₂-C₆alkynyl, and -(CH₂)₀₋₂-C₃-C₈cycloalkyl, wherein alkyl, alkenyl, alkynyl, are optionally substituted with one or more halogen, hydroxyl, methoxy, or NH₂, and wherein the cycloalkyl is optionally substituted with one or more halogen, hydroxyl, C₁-C₆alkyl, C₁-C₆alkoxy, or NH₂;
each R¹⁴ is independently H or R¹²; and
n = 0, 1, 2, 3, or 4.

In some embodiments, each R¹² is independently selected from the group consisting of halogen, hydroxy, 4-7-membered monocyclic heterocycloalkyl, C₁-C₆heteroalkyl, and C₁-C₆alkyl, wherein alkyl is optionally substituted with one or more halogen, hydroxyl, methoxy, C₃-C₈cycloalkyl, or NH₂, and heterocycloalkyl is optionally substituted with one or more halogen, hydroxyl, methoxy, or C₁-C₆alkyl; and
R¹¹ is H or unsubstituted C₁-C₆alkyl.

In some embodiments, each R¹² is independently selected from the group consisting of halogen, hydroxy, and C₁-C₆alkyl, wherein alkyl is optionally substituted with one or more halogen, hydroxyl, methoxy, C₃-C₈cycloalkyl, or NH₂; and
R¹³ is H or unsubstituted C₁-C₆alkyl.

In some embodiments, each R¹² is independently C₁-C₆alkyl.

In some embodiments, B is

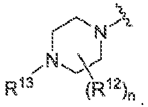

In some embodiments, R¹³ is H or unsubstituted C₁-C₆alkyl.

In some embodiments, each R¹² is independently C₁-C₆alkyl.

In some embodiments, B is a bicyclic 6-14 membered heterocycloalkyl comprising 1, 2 or 3 total nitrogen ring atoms and 0 or 1 additional ring heteroatoms selected from O and S, and the heterocycloalkyl is optionally substituted with 1, 2, 3, or 4 R¹².

In some embodiments, each R¹² is independently C₁-C₆alkyl.

In some embodiments, B is NR¹⁰R¹¹ and R¹¹ is hydrogen, C₁₋₇alkyl, C₁₋₇haloalkyl, or C₃₋₈cycloalkyl.

In some embodiments, R¹¹ is H or C₁₋₇alkyl, and R¹⁰ is C₁-C₈heteroalkyl comprising at least one nitrogen atom.

In some embodiments, R¹⁰ is -(CH₂)₀₋₃heterocycloalkyl comprising at least 1 nitrogen ring atom, each R¹⁰ optionally substituted with 1 to 6 R¹².

In some embodiments, the compound is of formula (Ig) 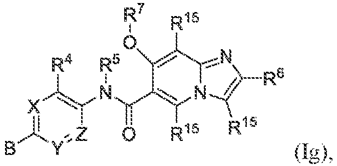 or a pharmaceutically acceptable salt thereof,
wherein each R¹⁵ is independently H, halogen, C₁-C₆alkyl, or C₁-C₆haloalkyl.

In some embodiments, the compound is of formula (Ih) 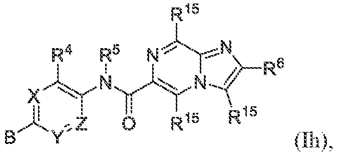 or a pharmaceutically acceptable salt thereof,
wherein each R¹⁵ is independently H, halogen, C₁-C₆alkyl, or C₁-C₆haloalkyl.

In some embodiments, the compound is of formula (Ii) 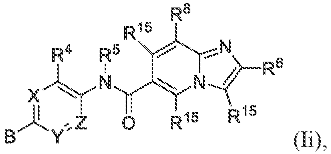 or a pharmaceutically acceptable salt thereof,
wherein each R¹⁵ is independently H, halogen, C₁-C₆alkyl, or C₁-C₆haloalkyl.

In some embodiments, the compound is of formula (Ij) 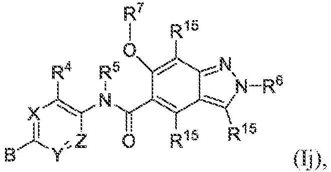 or a pharmaceutically acceptable salt thereof,
wherein each R¹⁵ is independently H, halogen, C₁-C₆alkyl, or C₁-C₆haloalkyl.

In some embodiments, the compound is of formula (Ik) 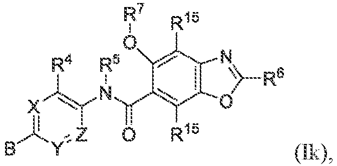 or a pharmaceutically acceptable salt thereof,
wherein each R¹⁵ is independently H, halogen, C₁-C₆alkyl, or C₁-C₆haloalkyl.

In some embodiments, the compound is of formula (Im) 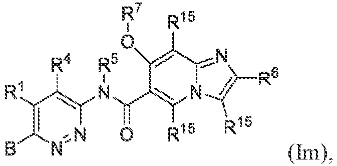 or a pharmaceutically acceptable salt thereof,
wherein each R¹⁵ is independently H, halogen, C₁-C₆alkyl, or C₁-C₆haloalkyl.

In some embodiments, the compound is of formula (In) 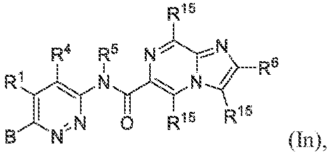 or a pharmaceutically acceptable salt thereof,
wherein each R¹⁵ is independently H, halogen, C₁-C₆alkyl, or C₁-C₆haloalkyl.

In some embodiments, the compound is of formula (Io) 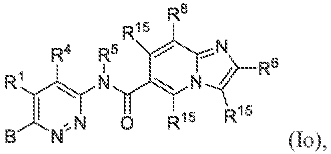 or a pharmaceutically acceptable salt thereof,
wherein each R¹⁵ is independently H, halogen, C₁-C₆alkyl, or C₁-C₆haloalkyl.

In some embodiments, the compound is of formula (Ip) 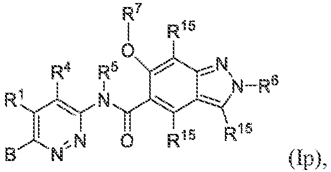 or a pharmaceutically acceptable salt thereof,
wherein each R¹⁵ is independently H, halogen, C₁-C₆alkyl, or C₁-C₆haloalkyl.

In some embodiments, the compound is of formula (Iq) 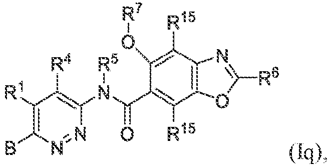 or a pharmaceutically acceptable salt thereof,
wherein each R¹⁵ is independently H, halogen, C₁-C₆alkyl, or C₁-C₆haloalkyl.

In some embodiments, the compound is of formula (Ir) 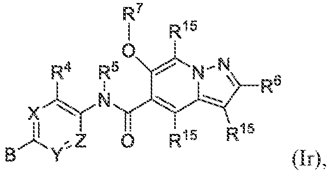 or a pharmaceutically acceptable salt thereof,
wherein each R¹⁵ is independently H, halogen, C₁-C₆alkyl, or C₁-C₆haloalkyl.

In some embodiments, the compound is of formula (Is) 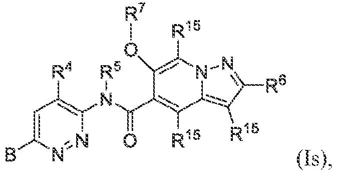 or a pharmaceutically acceptable salt thereof,
wherein each R¹⁵ is independently H, halogen, C₁-C₆alkyl, or C₁-C₆haloalkyl.

In some embodiments, the compond is of formula (It) 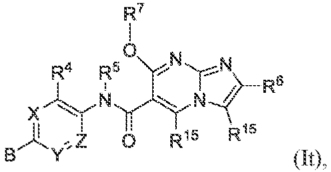 or a pharmaceutically acceptable salt thereof,
wherein each R¹⁵ is independently H, halogen, C₁-C₆alkyl, or C₁-C₆haloallcyl.

In some embodiments, the compound is of formula (Iu) 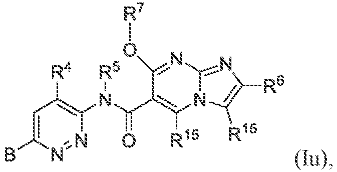 or a pharmaceutically acceptable salt thereof,
wherein each R¹⁵ is independently H, halogen, C₁-C₆alkyl, or C₁-C₆haloalkyl.

In some embodiments, the compound is selected from a compound of Table 1.

In some aspects, the disclosure provides a pharmaceutical composition comprising a compound of the present disclosure, or a pharmaceutically acceptable salt thereof and one or more pharmaceutically acceptable excipients.

In some aspects, the present disclosure provides use of a compound of the present disclosure or a pharmaceutically acceptable salt thereof in the manufacture of a medicament for treating or preventing a disease or disorder disclosed herein.

In some aspects, the present disclosure provides a method of preparing a compound of the present disclosure.

In some aspects, the present disclosure provides a method of preparing a compound, comprising one or more steps described herein

In some embodiments, the compound is selected from the compounds described in Table 1 and pharmaceutically acceptable salts thereof.

In some embodiments, the compound is selected from the compounds described in Table 1.

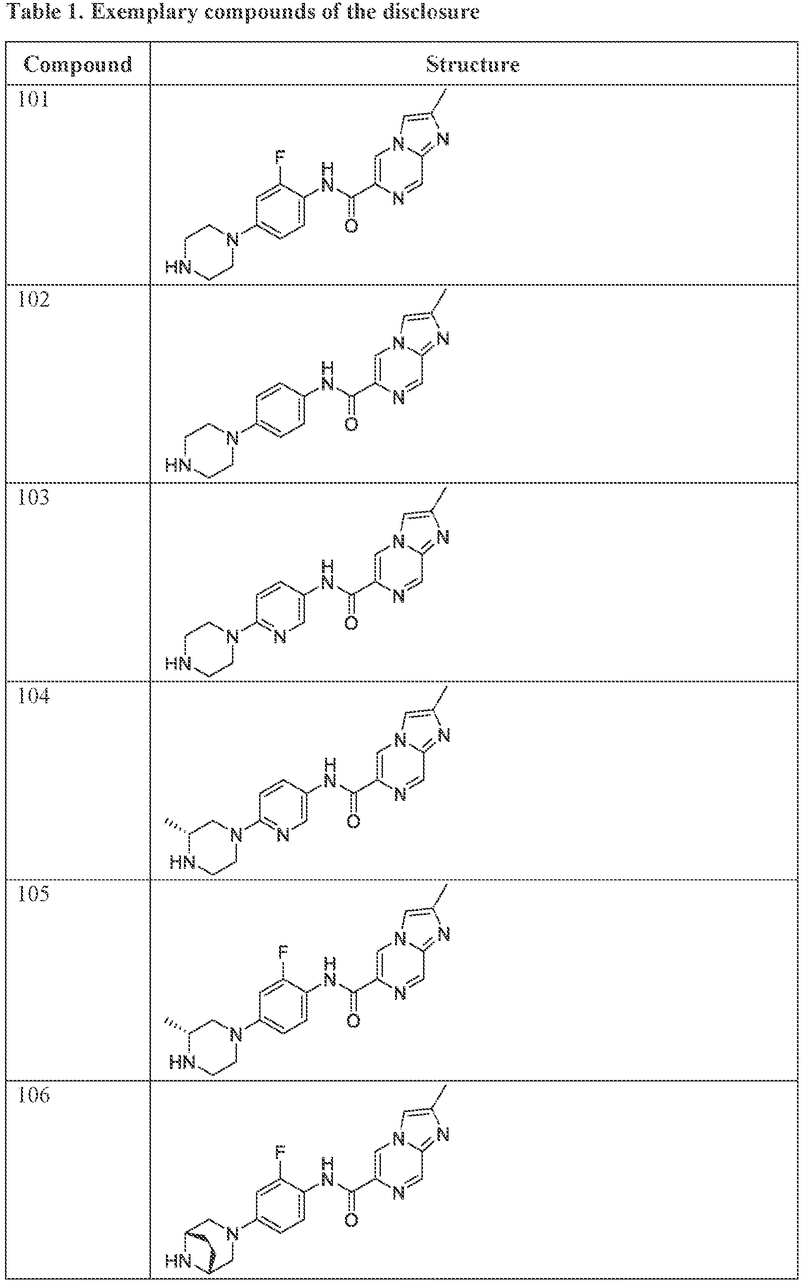

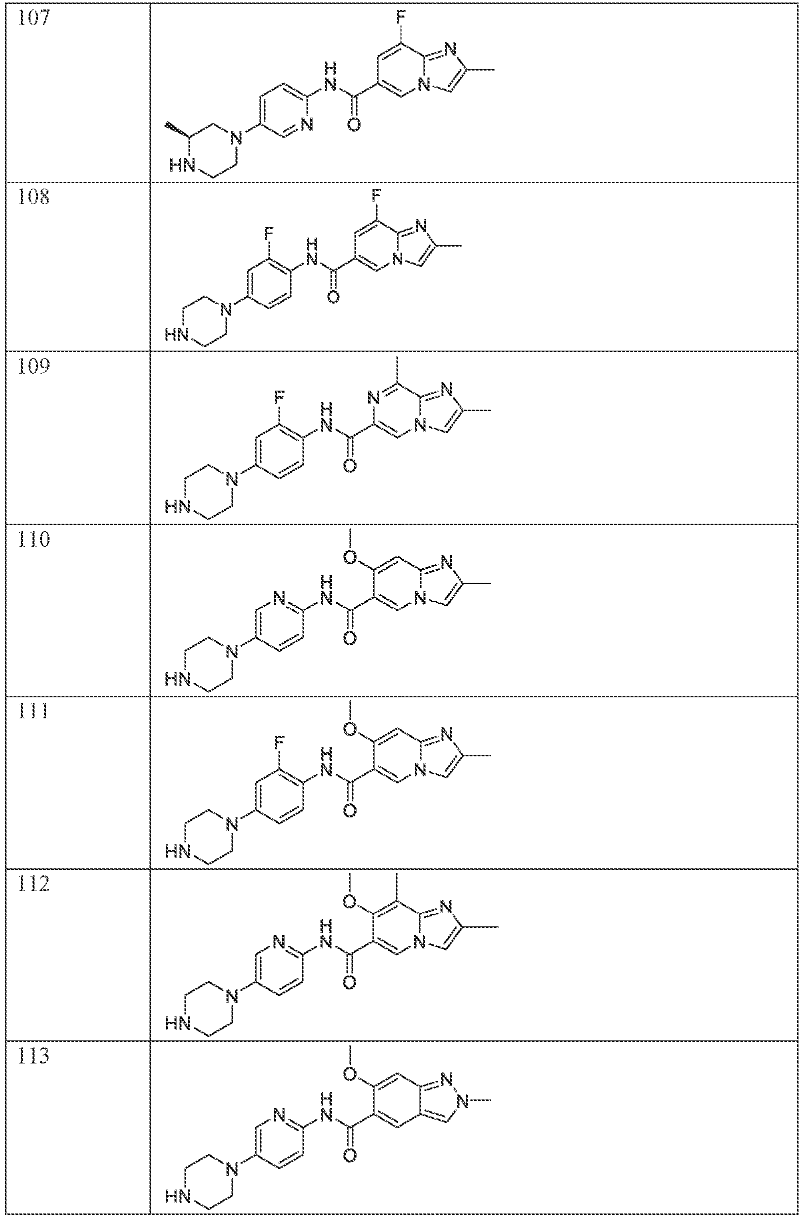

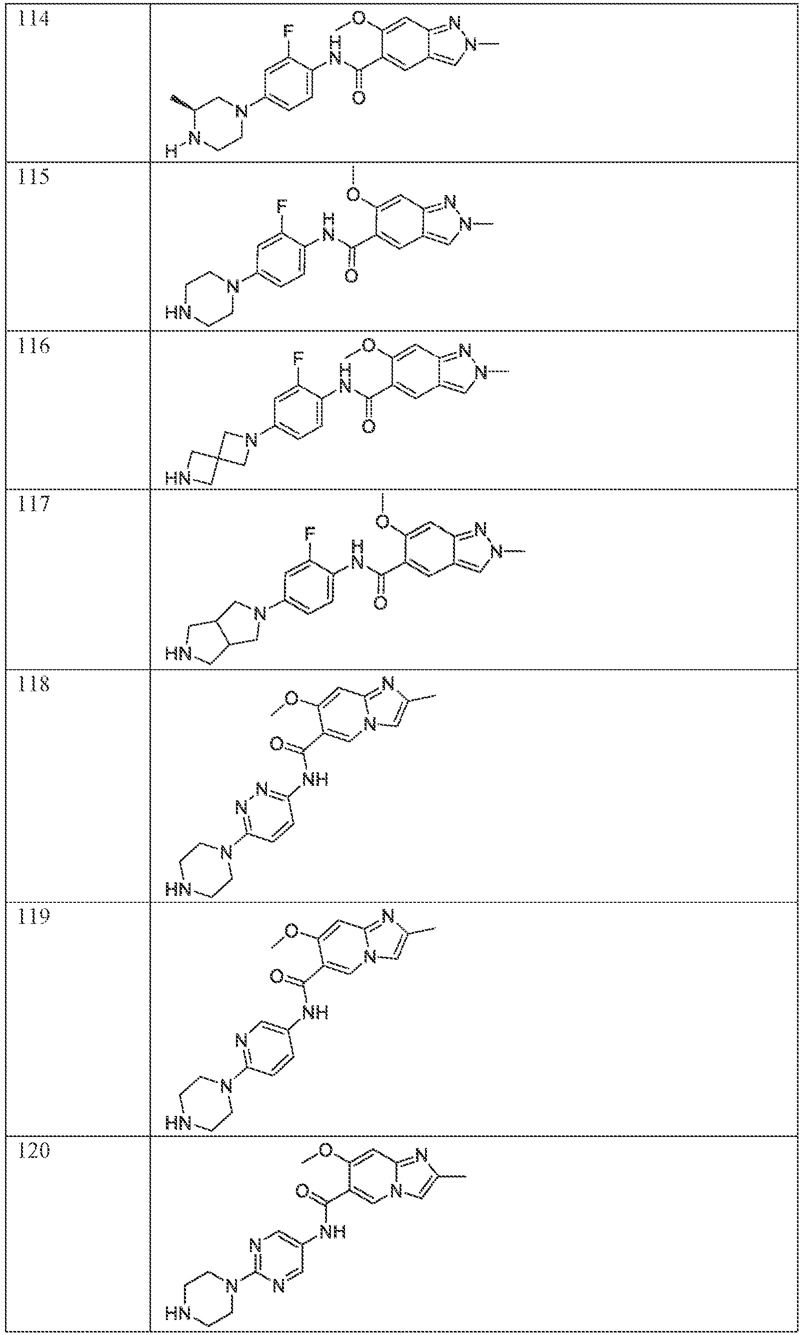

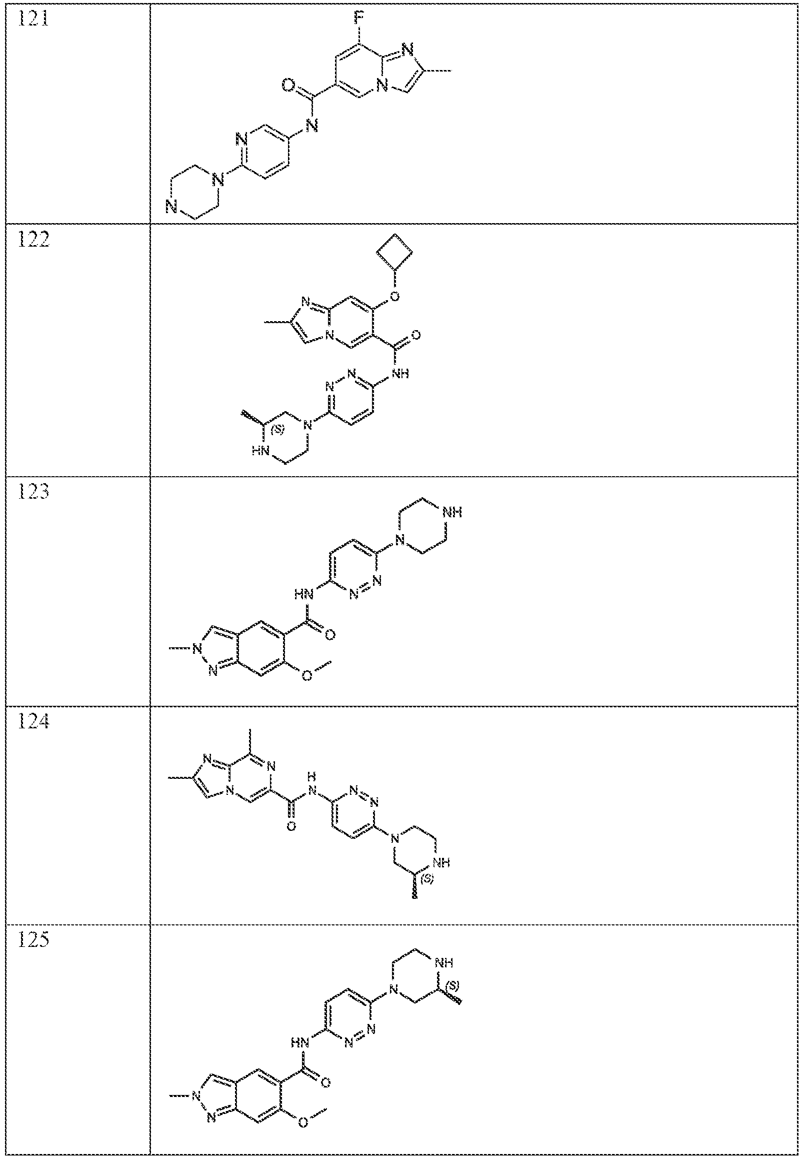

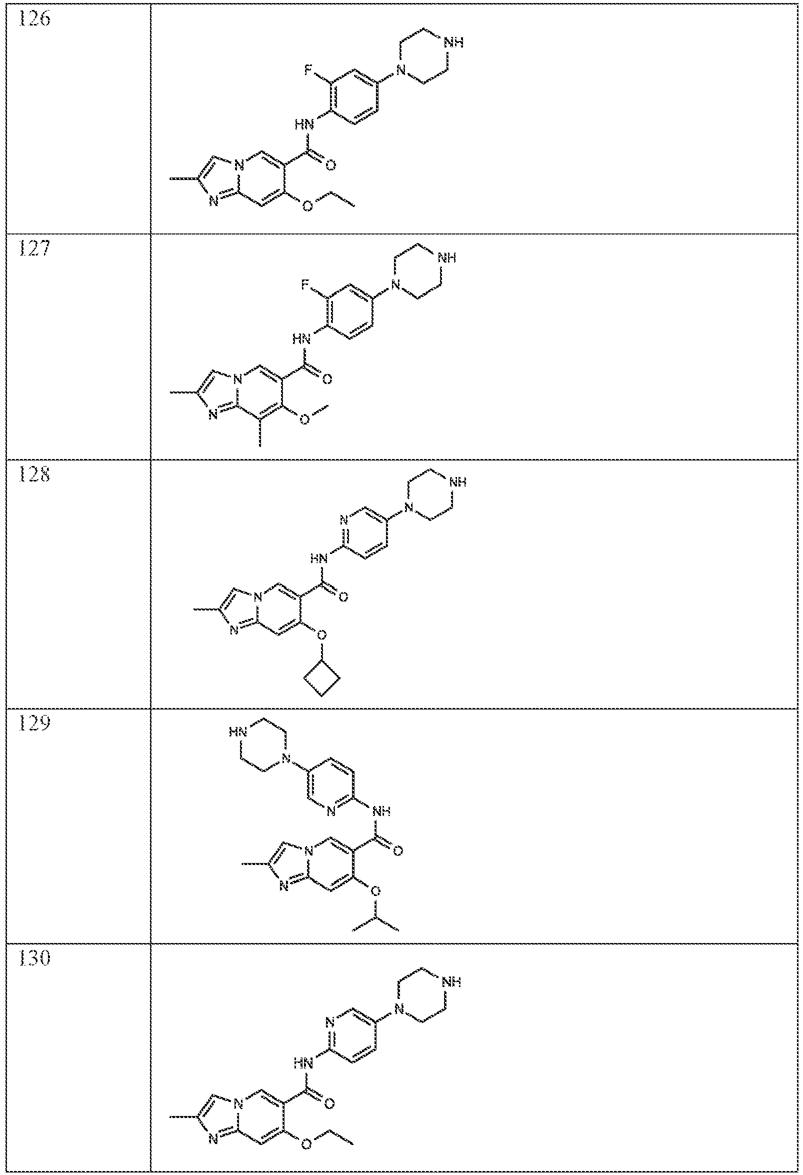

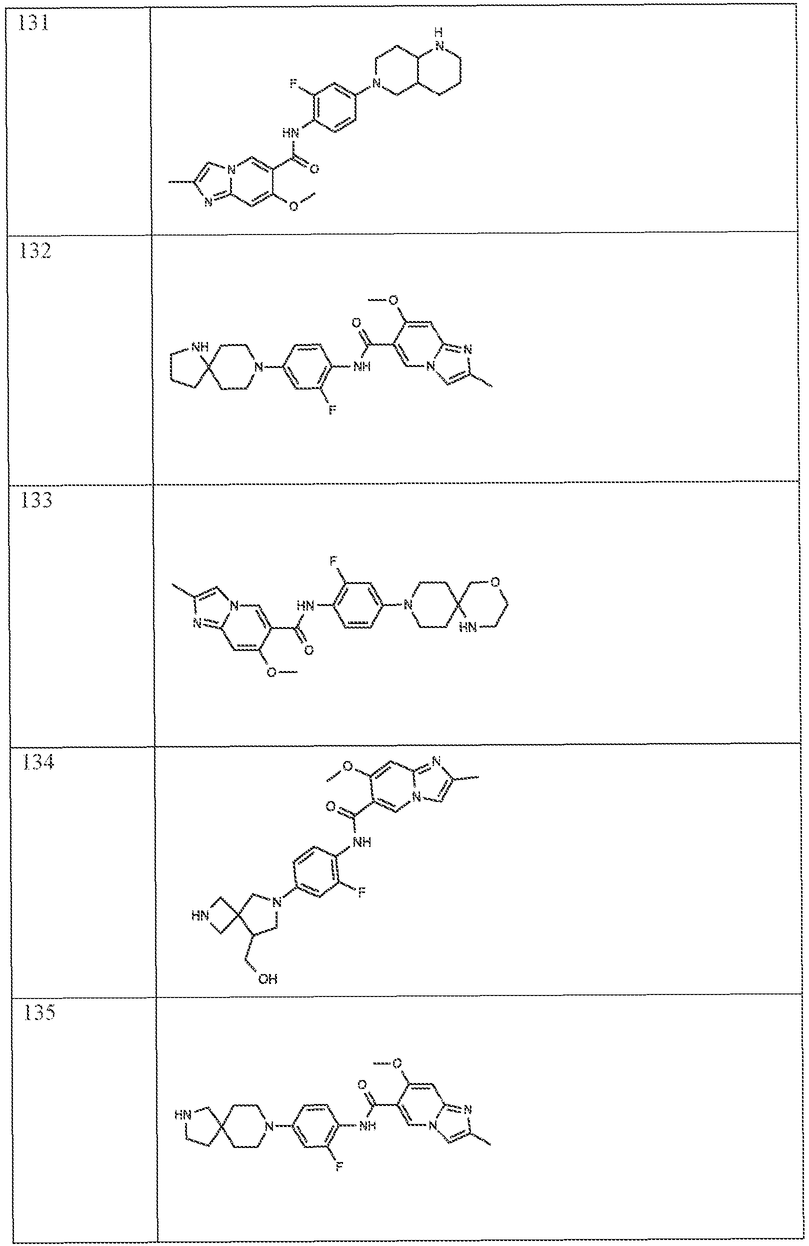

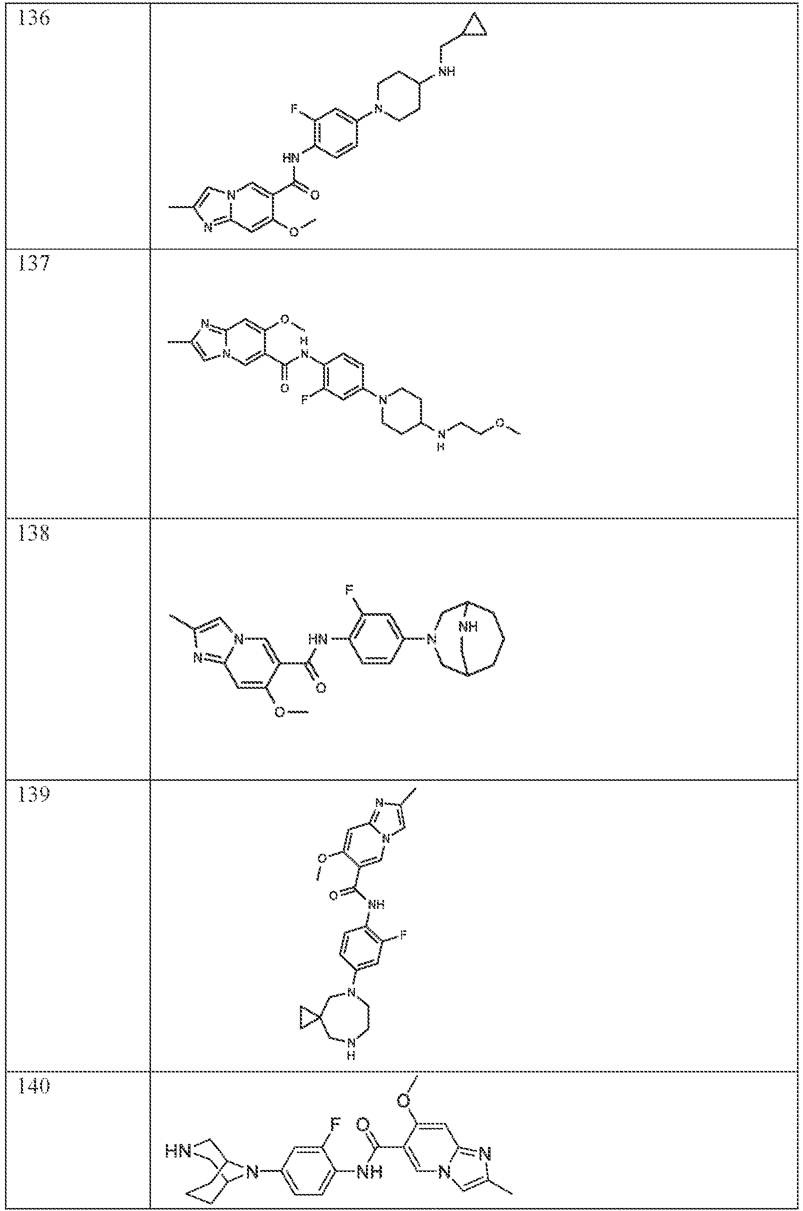

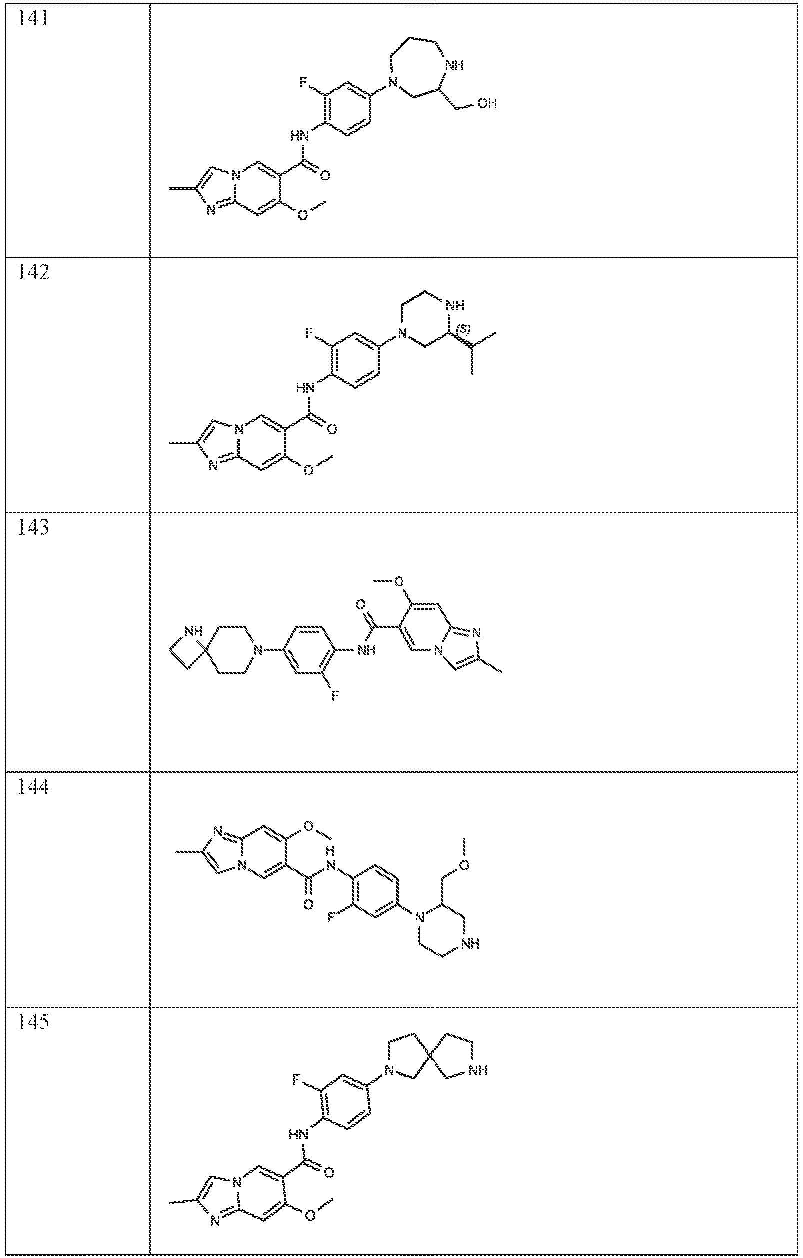

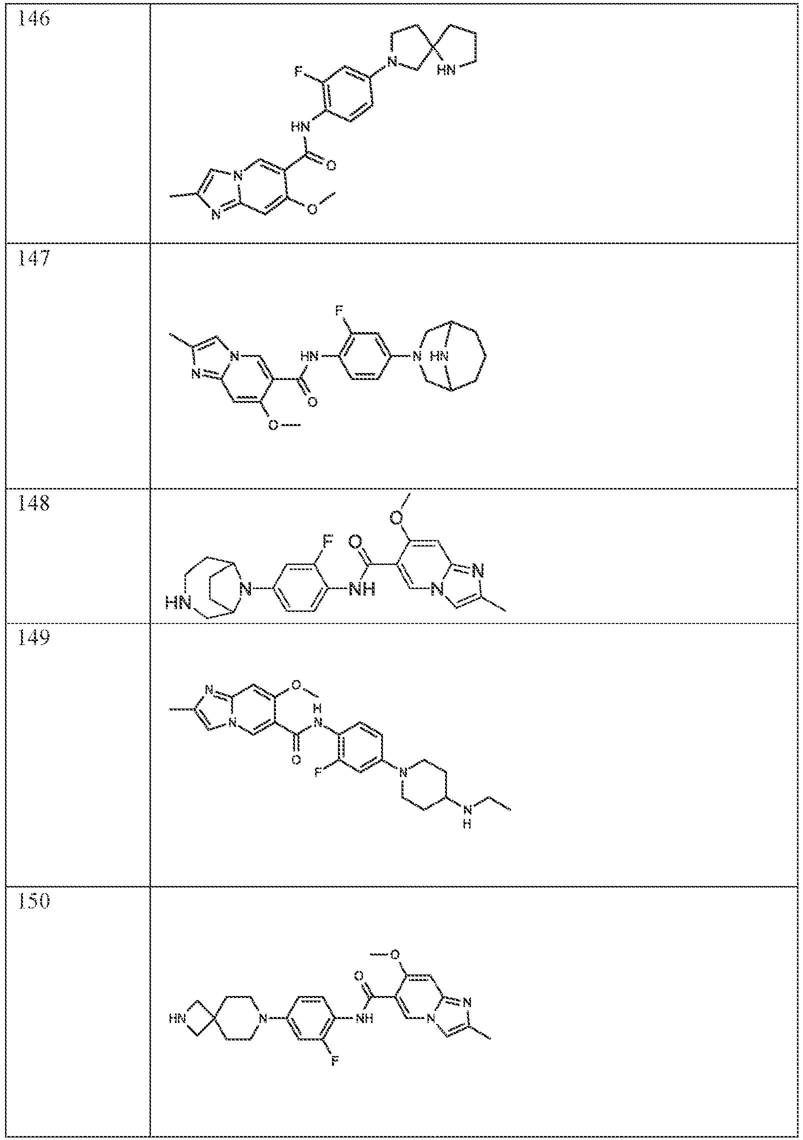

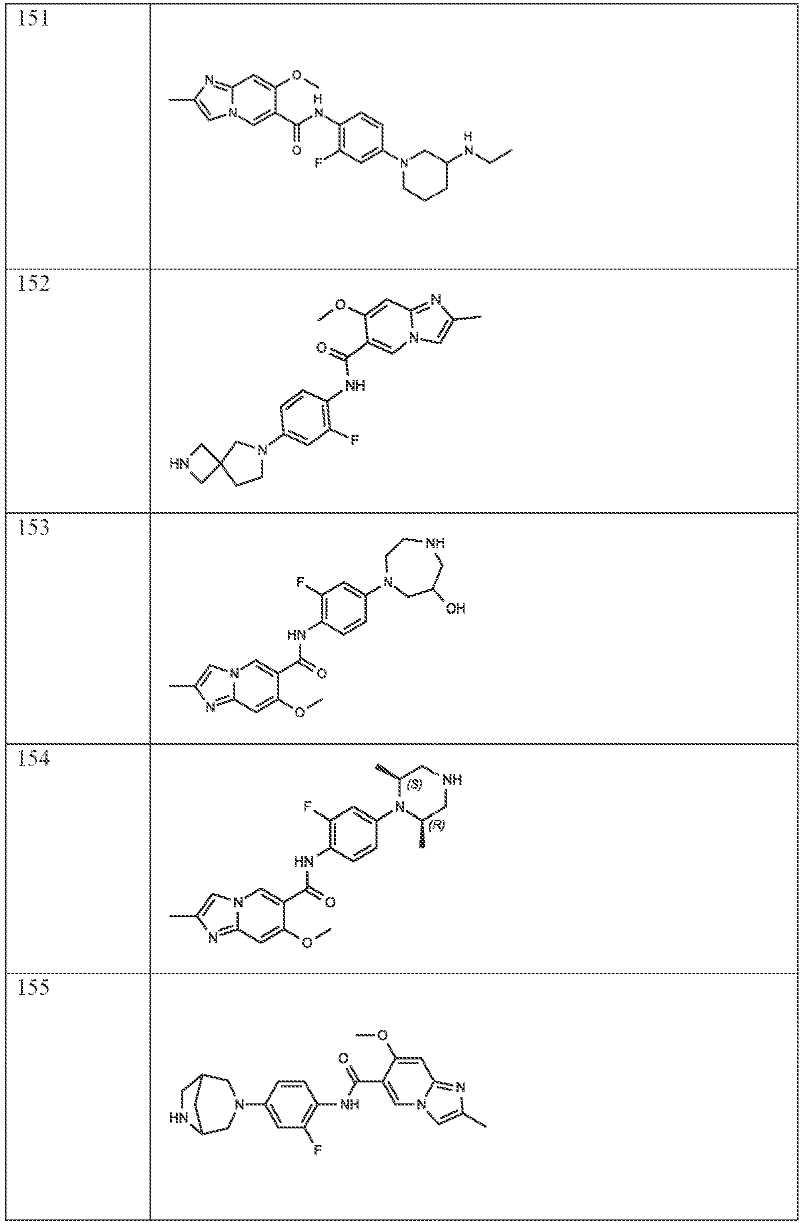

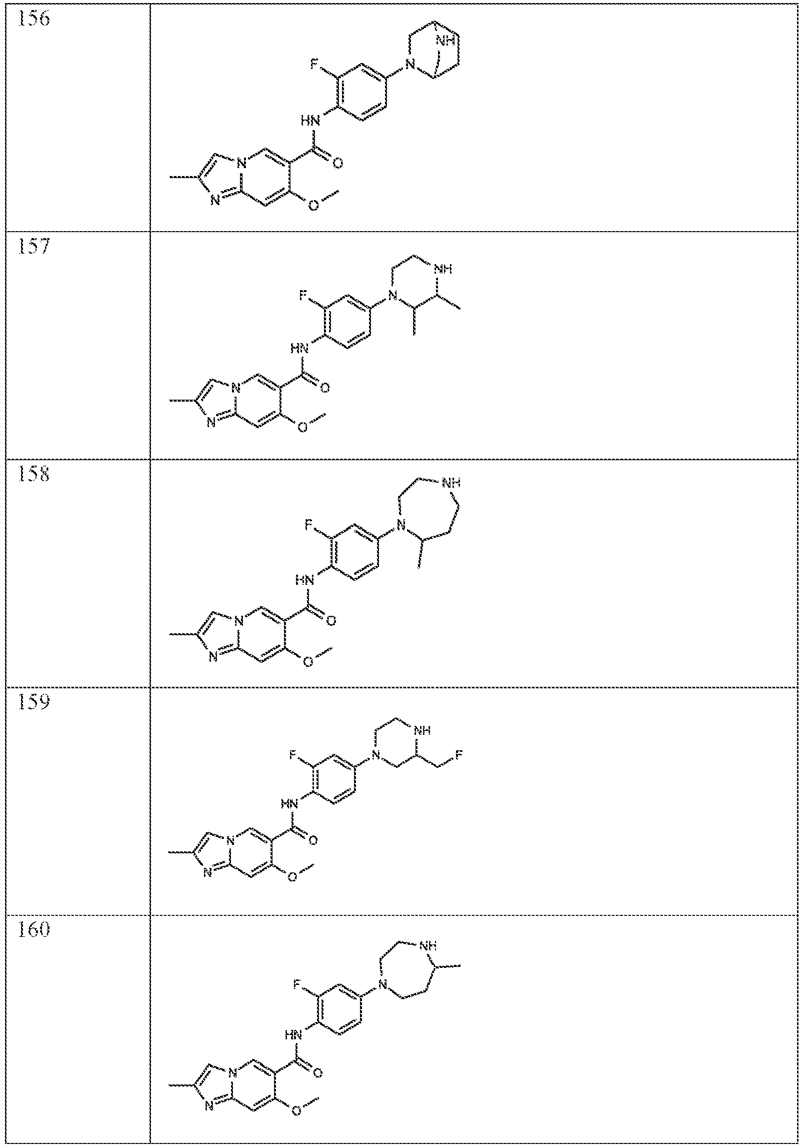

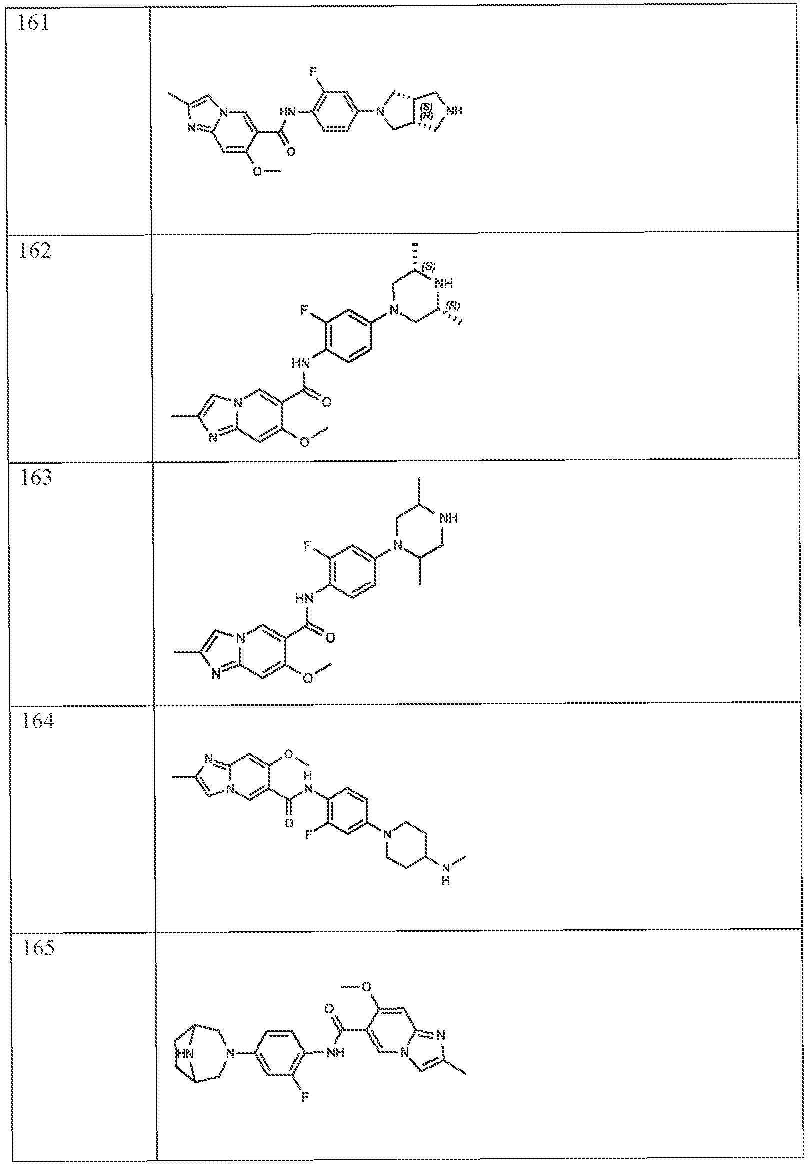

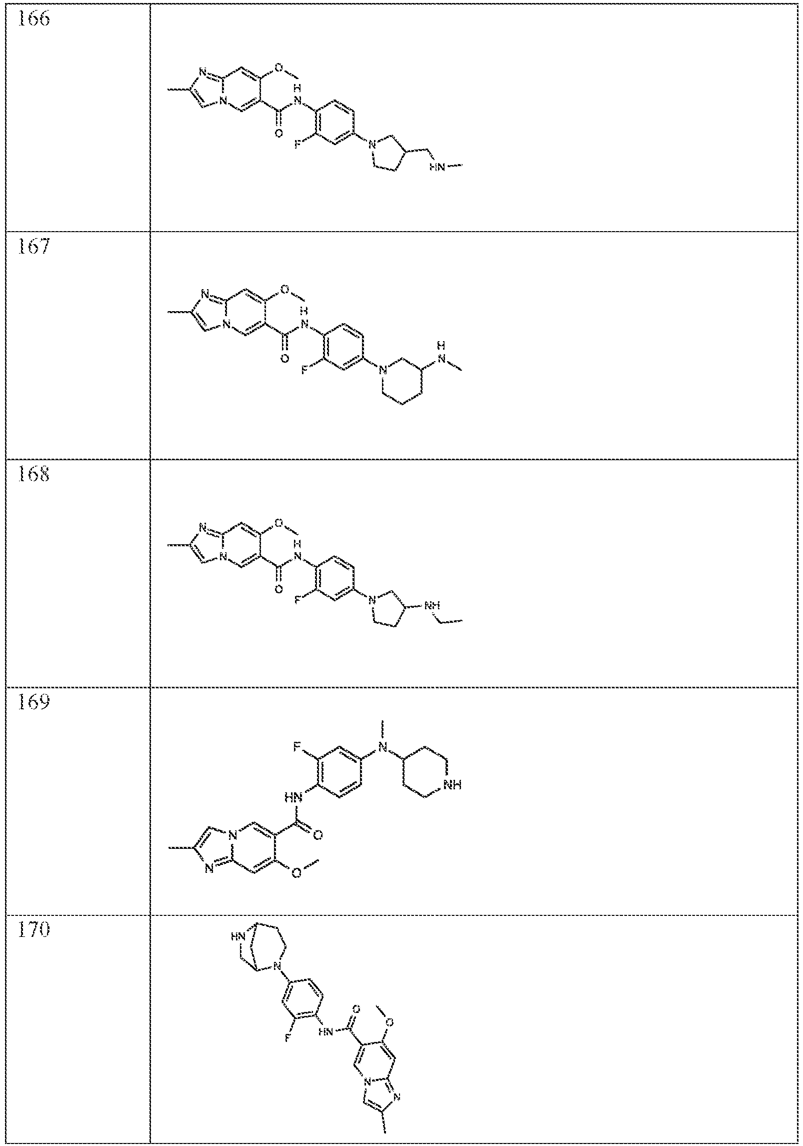

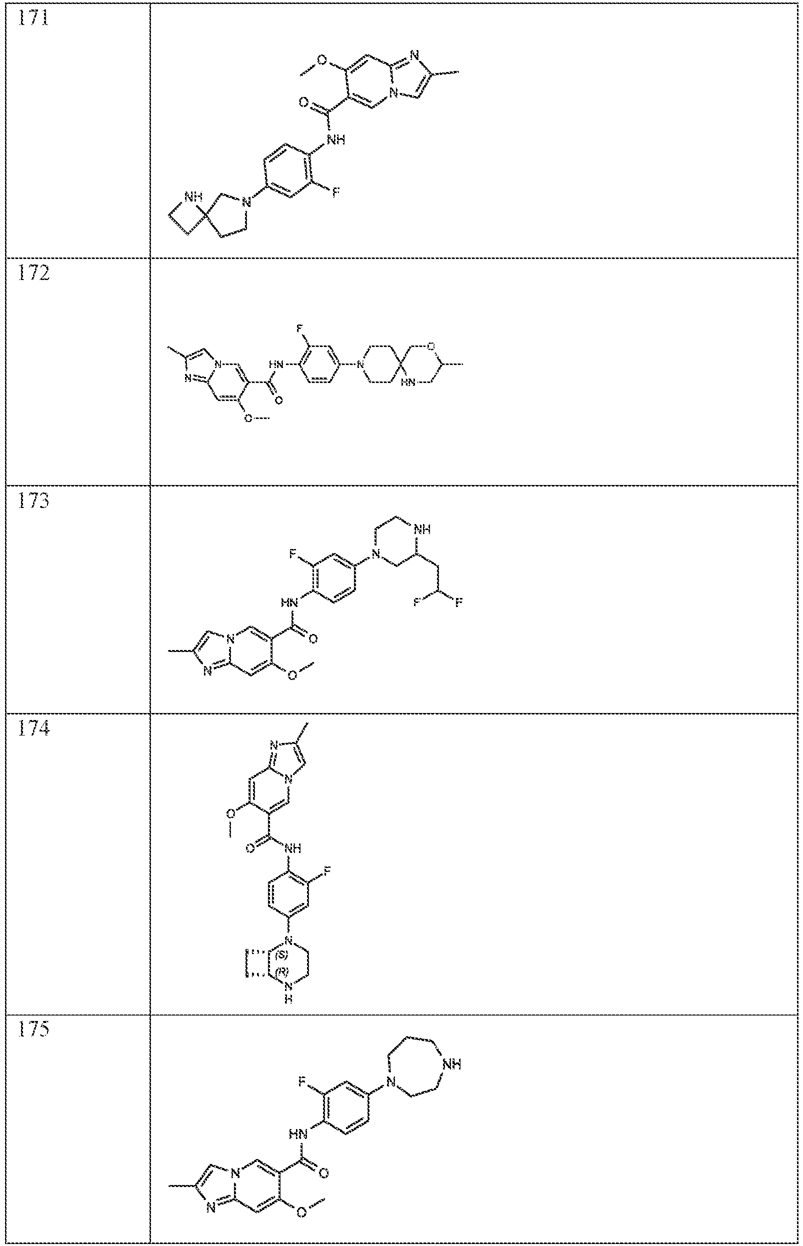

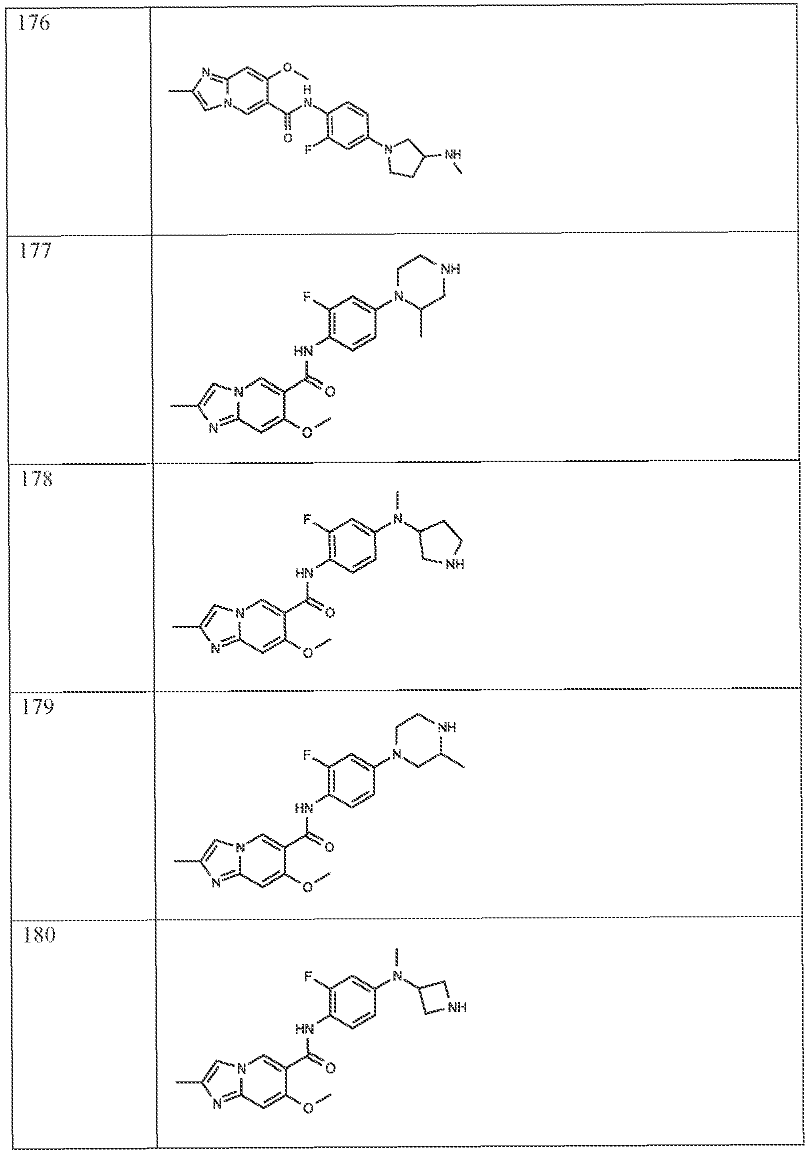

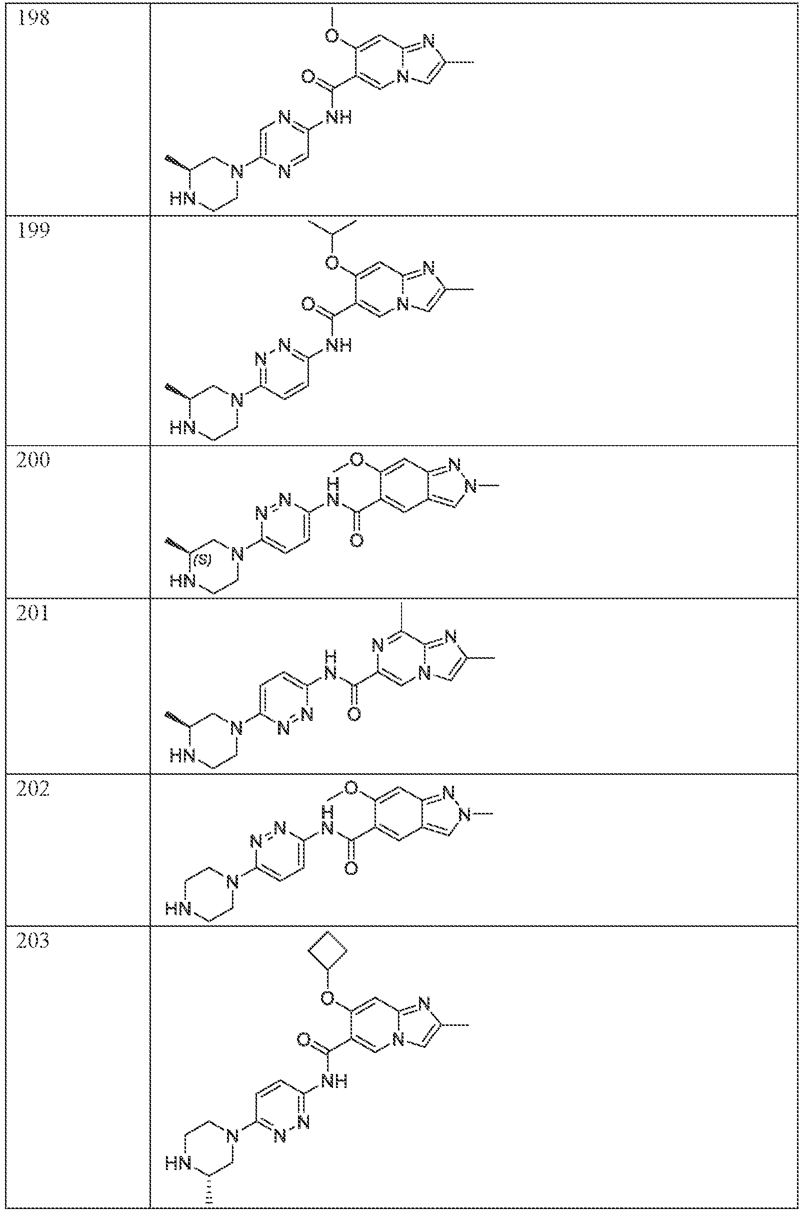

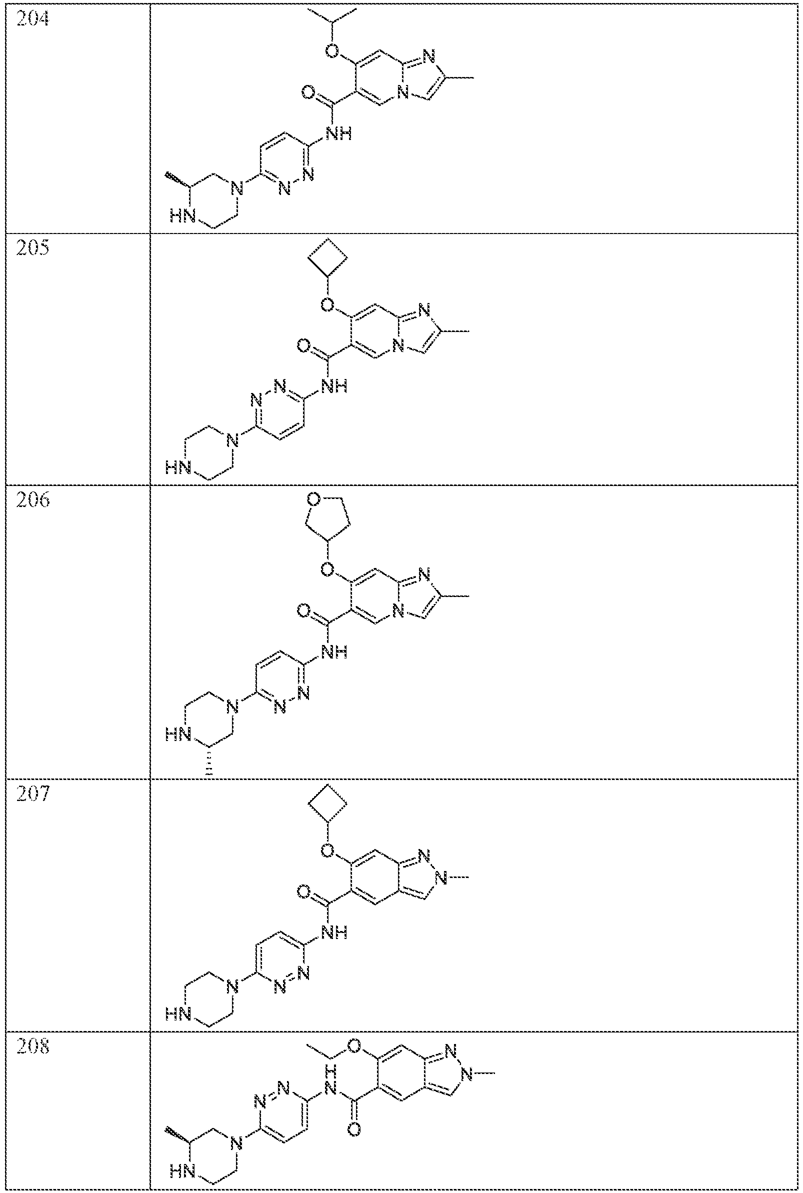

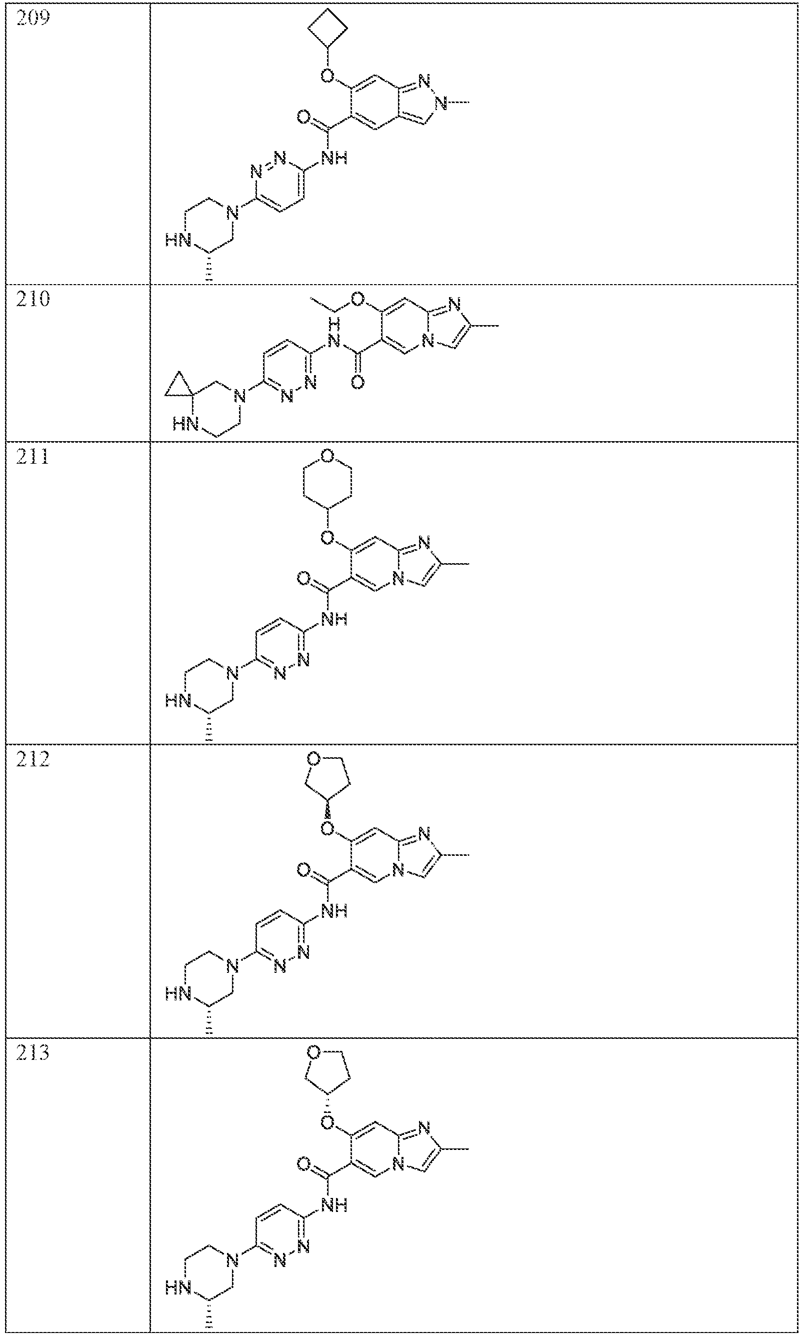

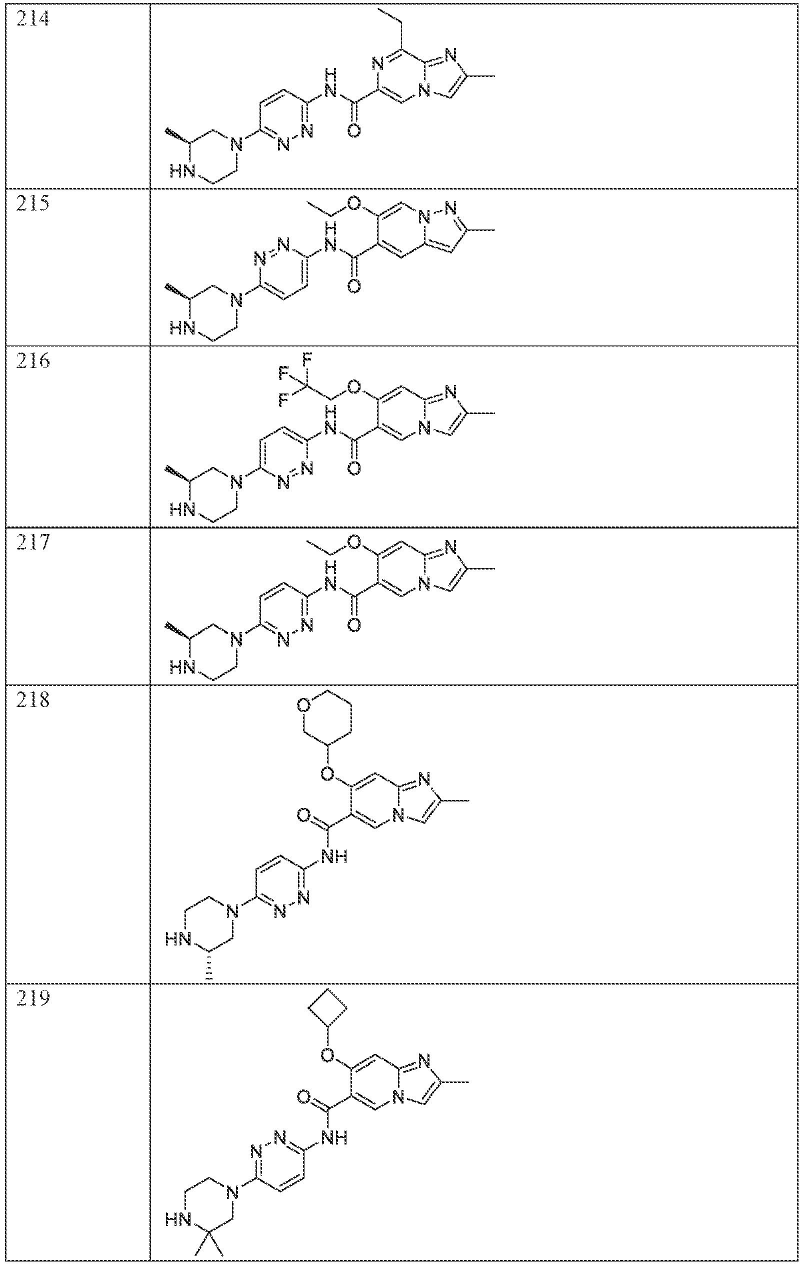

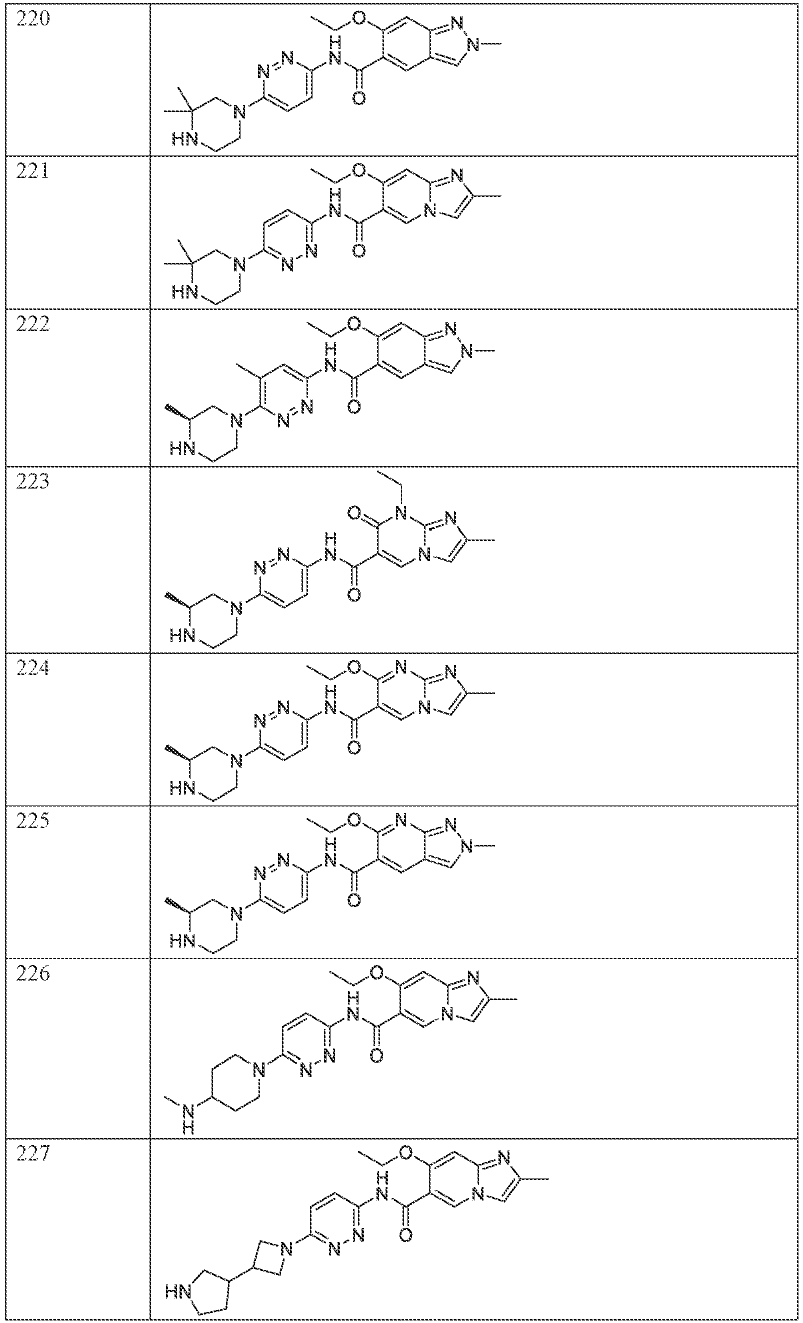

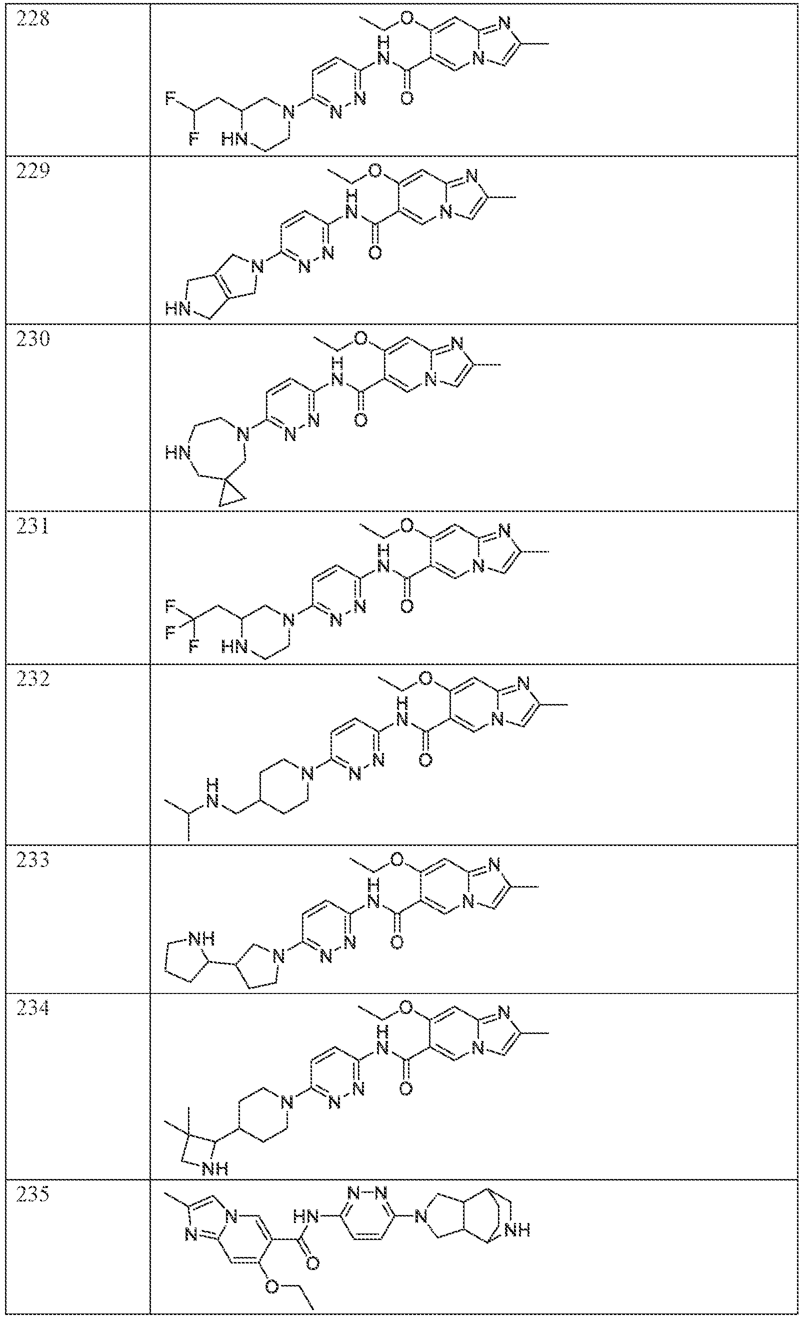

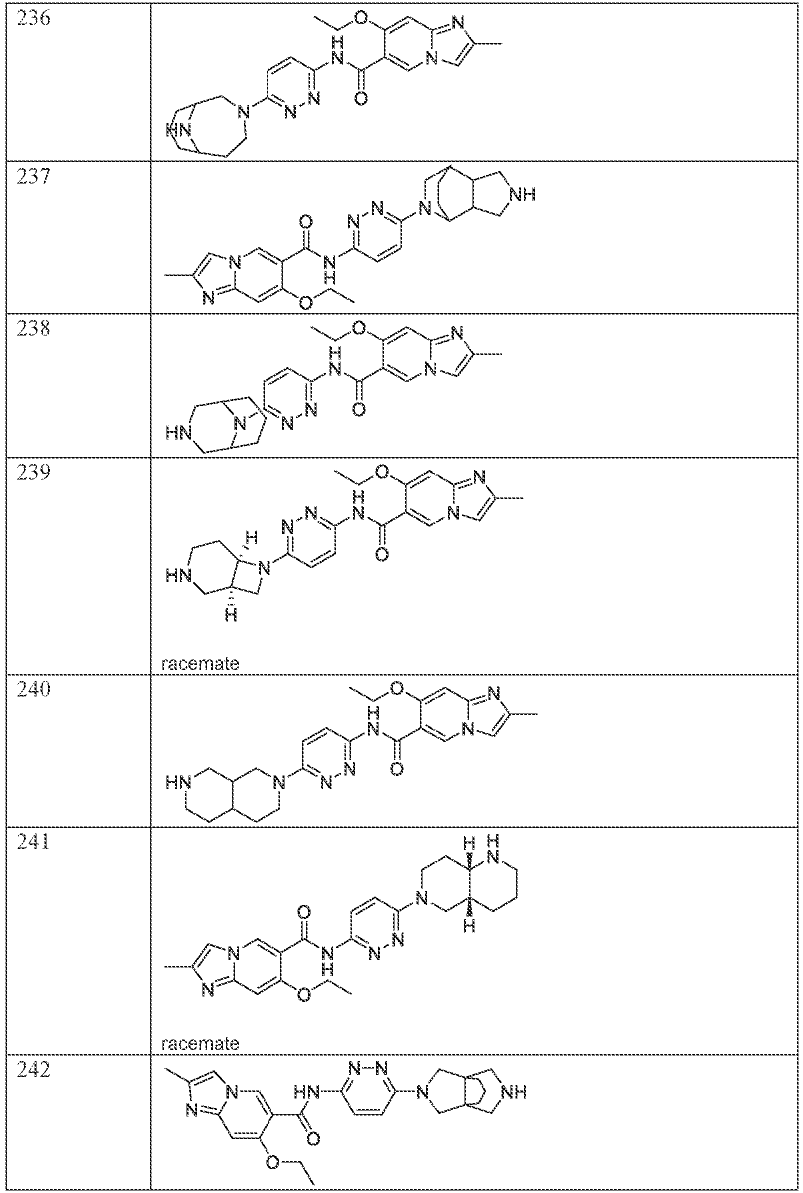

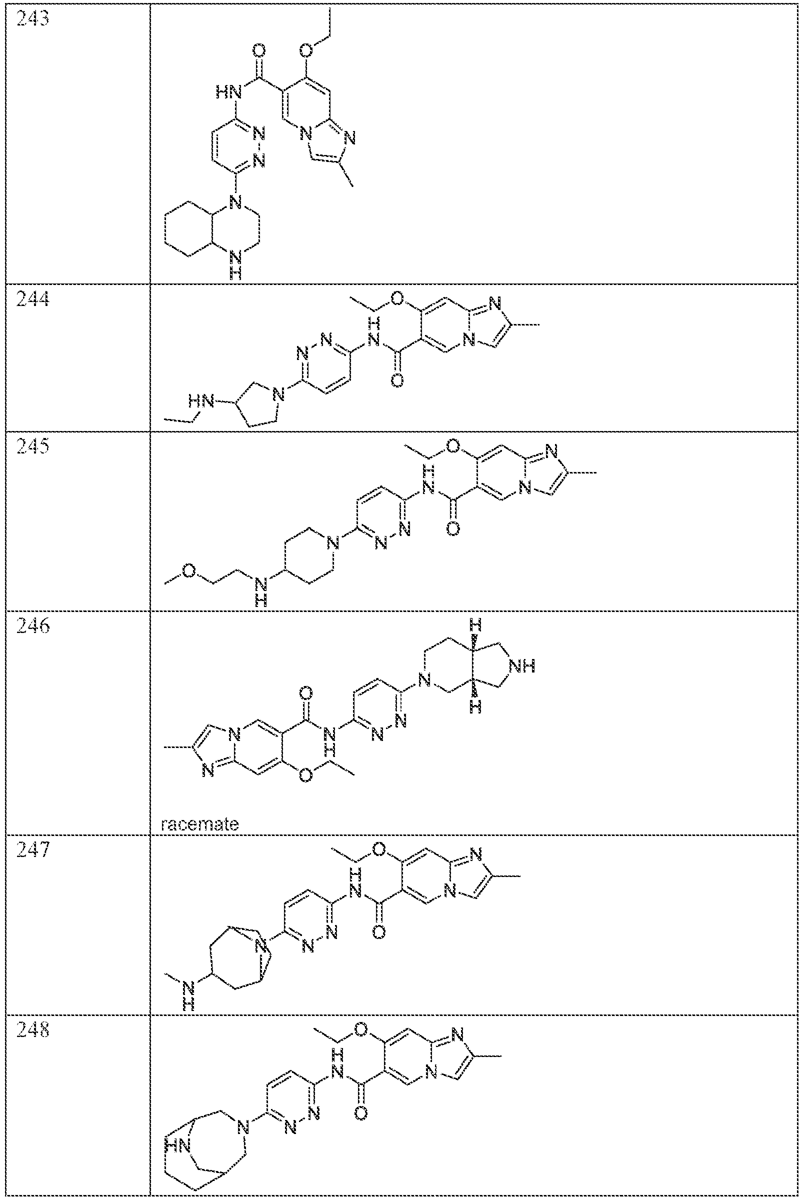

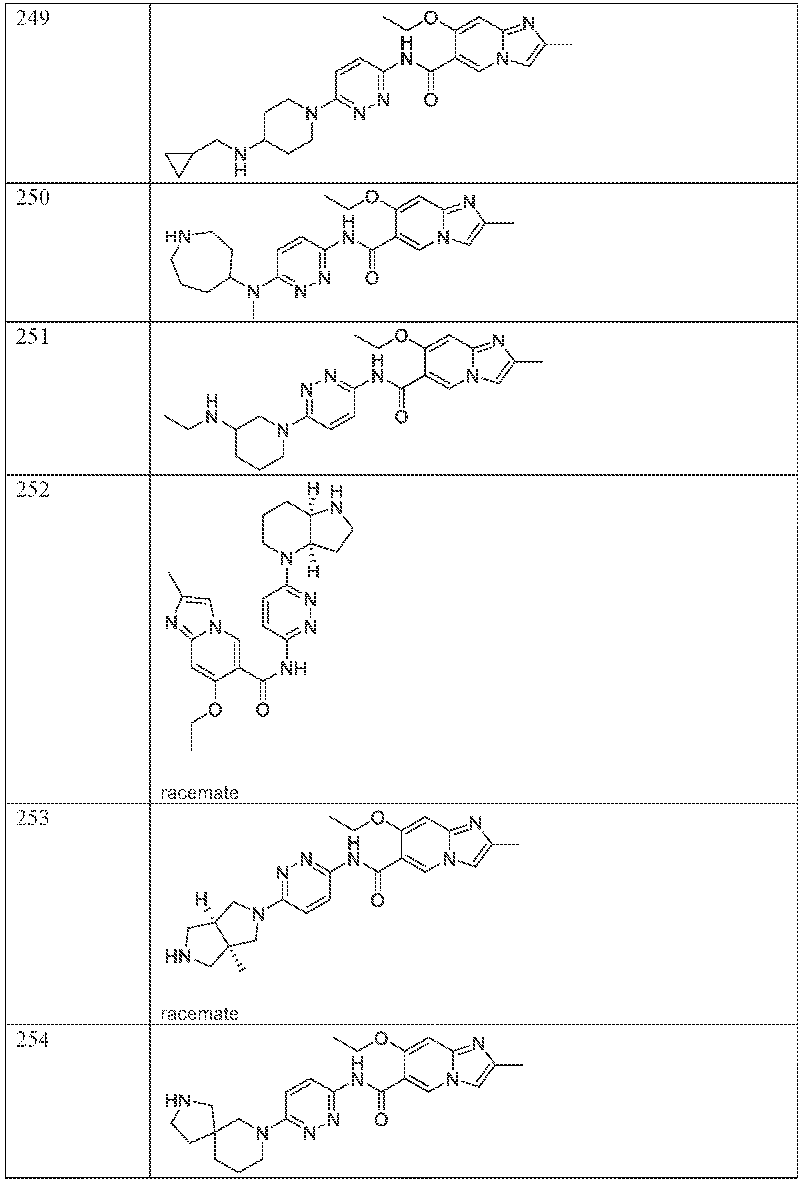

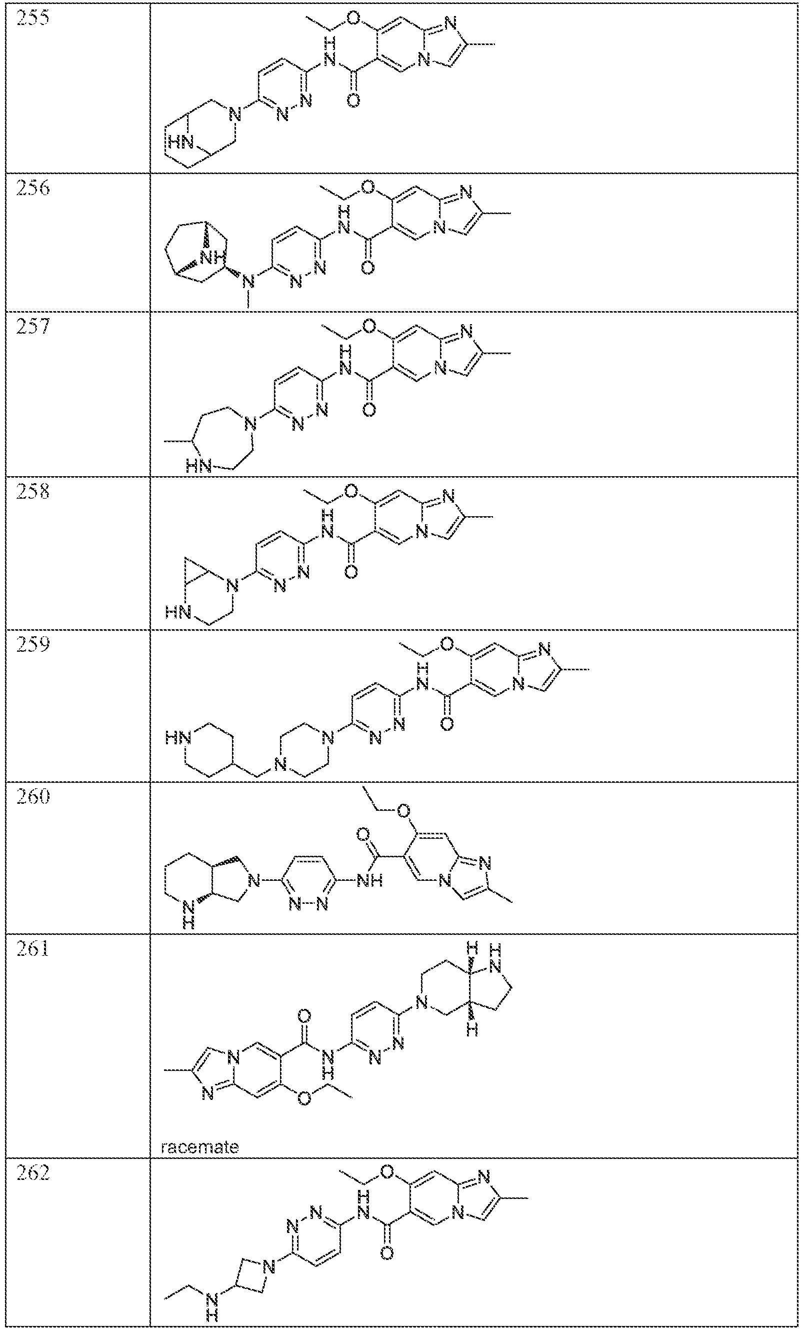

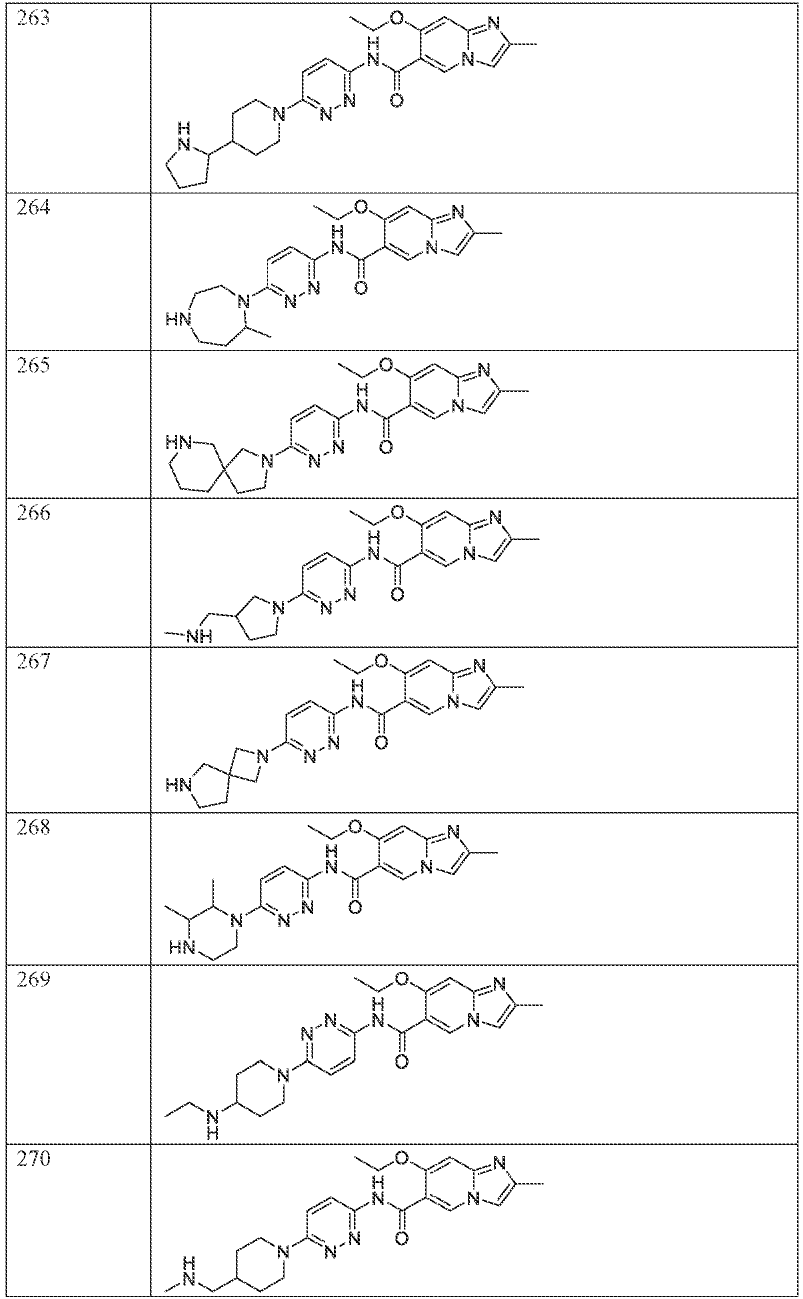

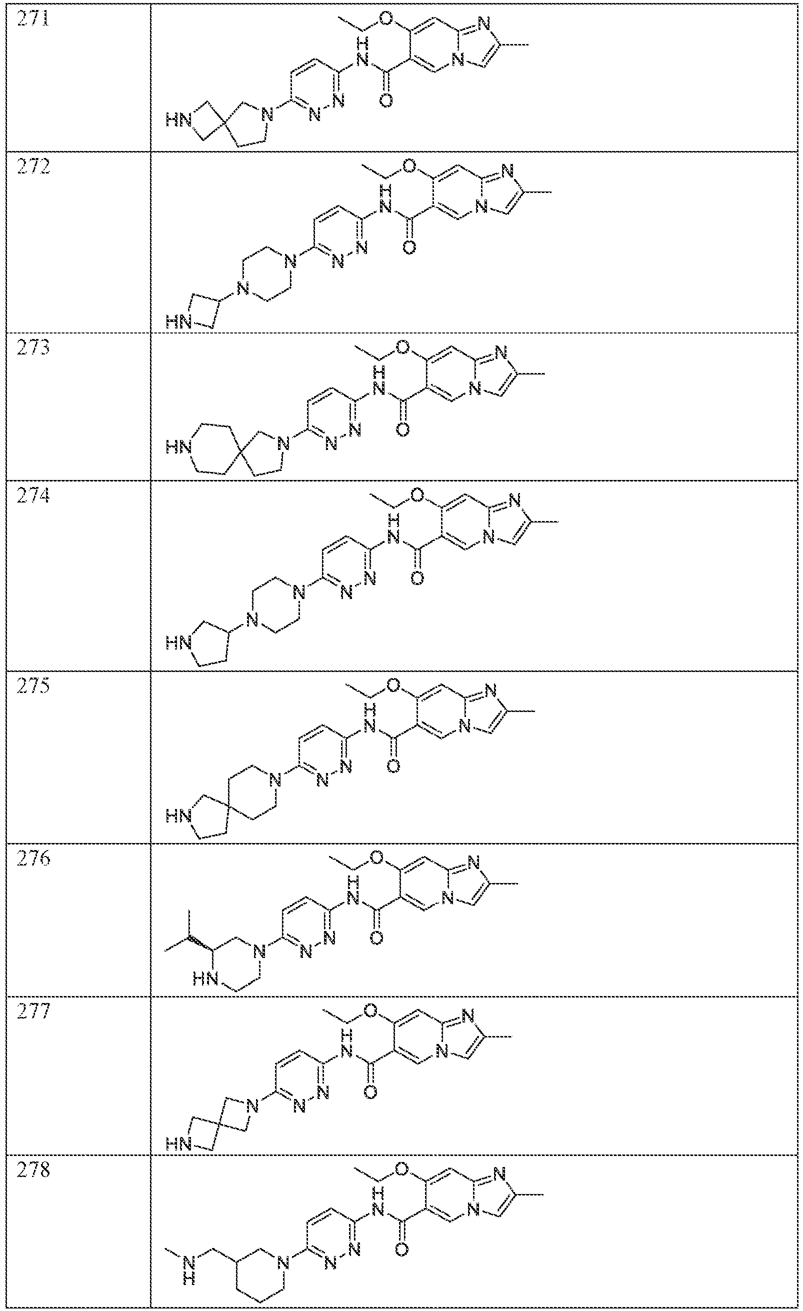

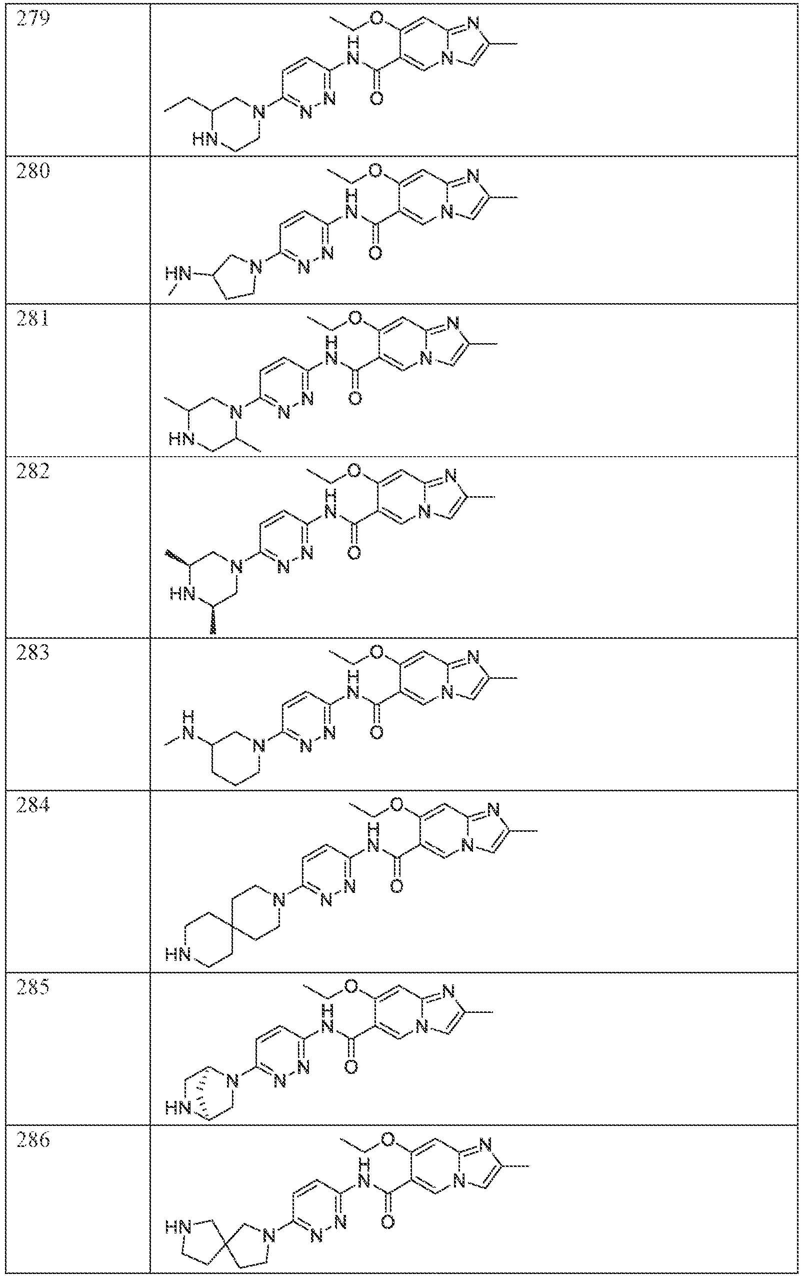

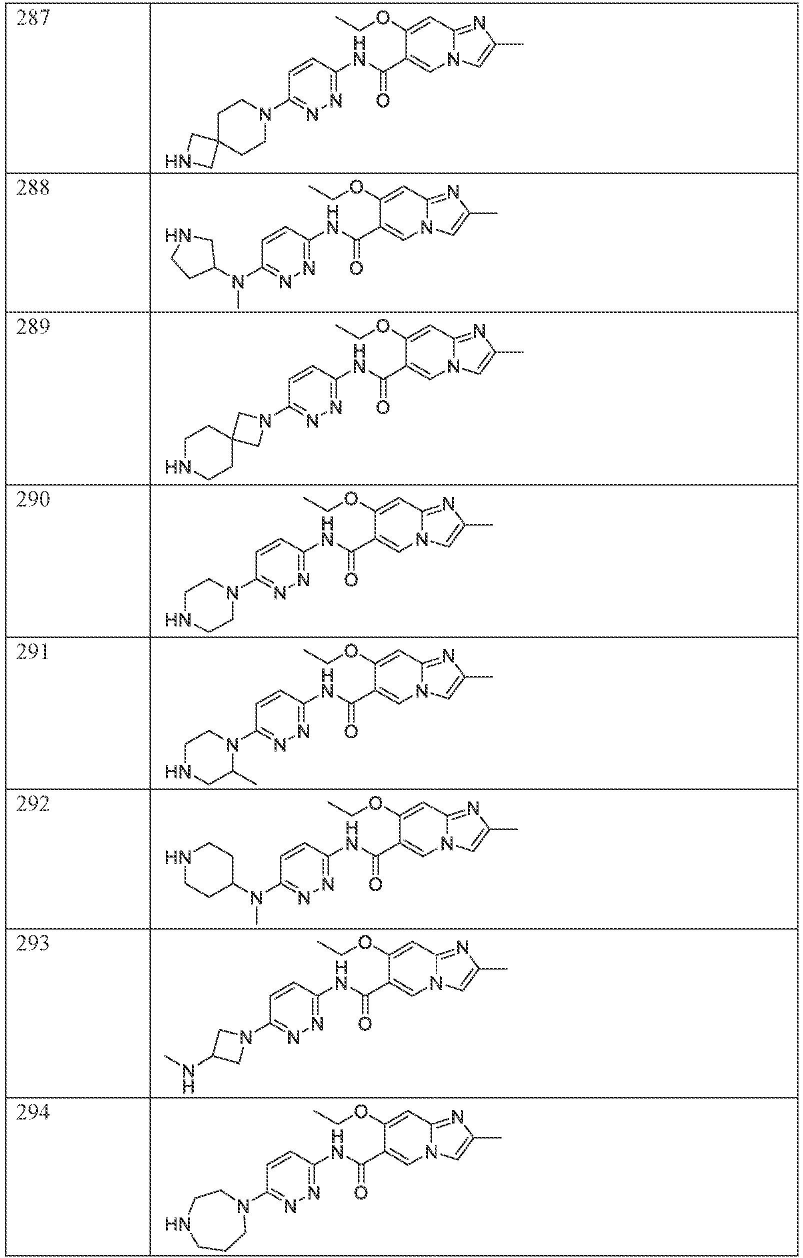

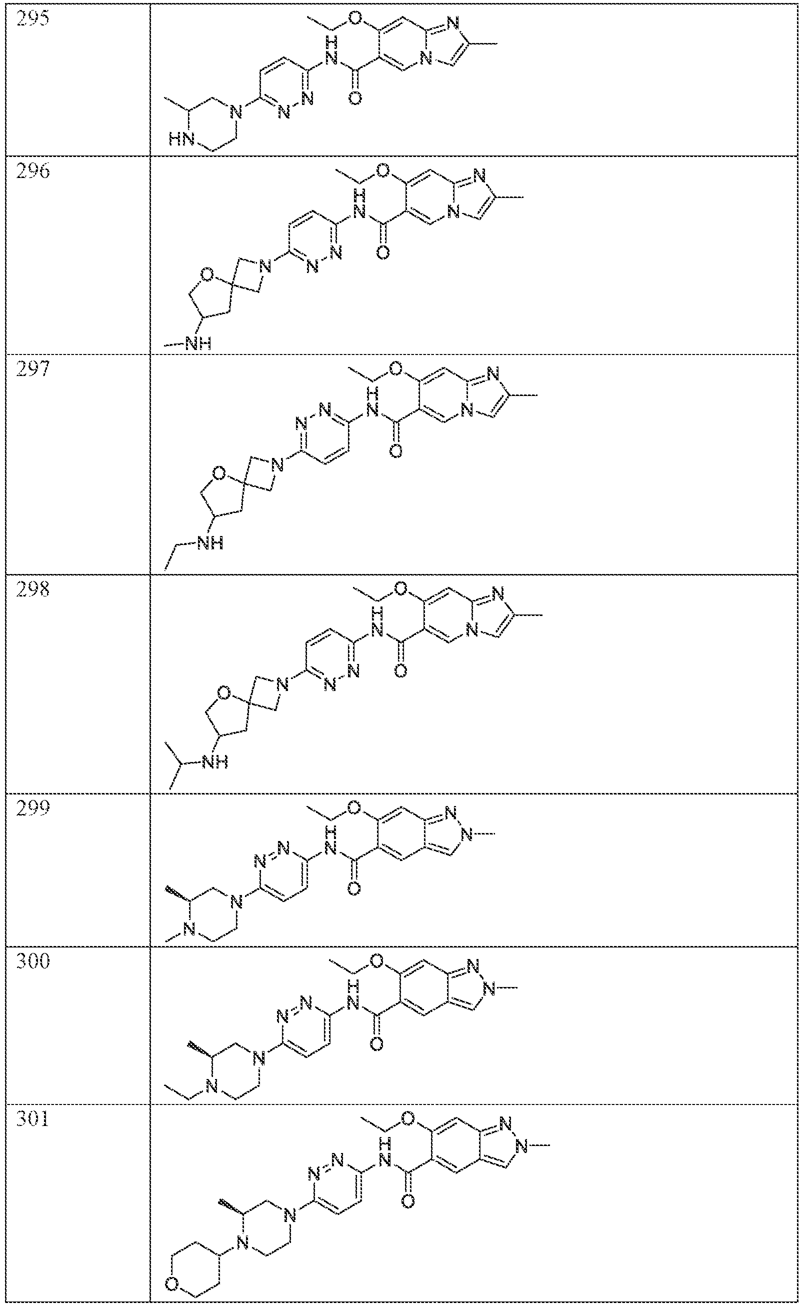

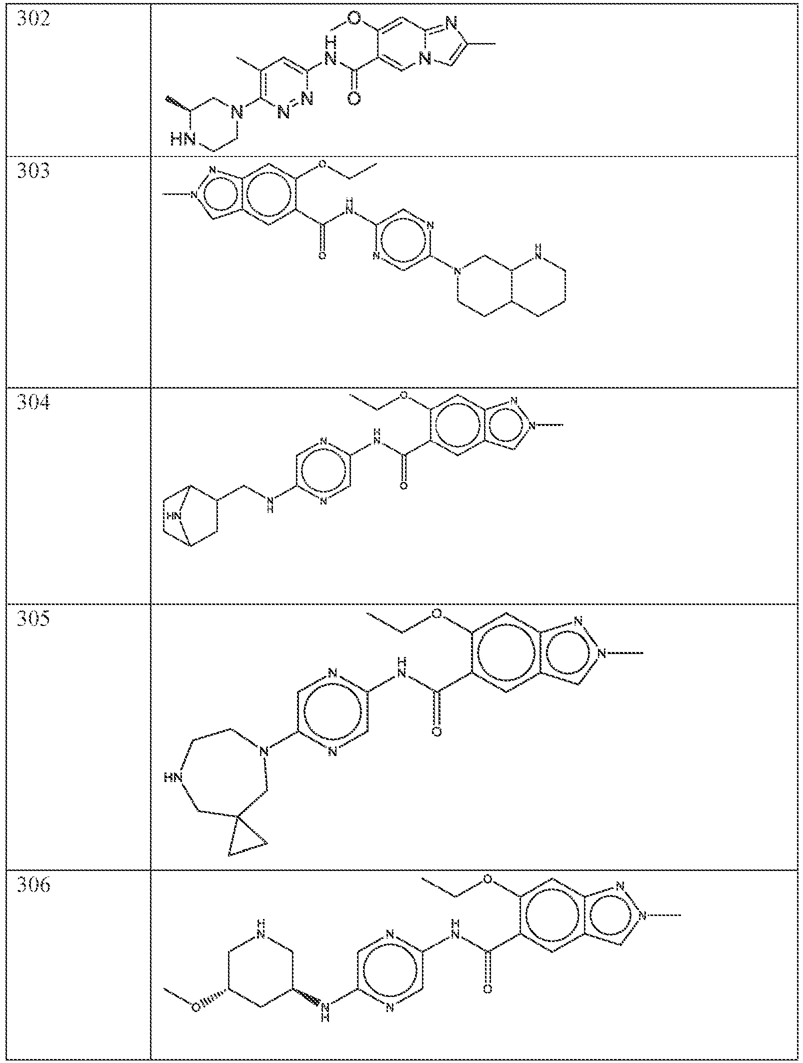

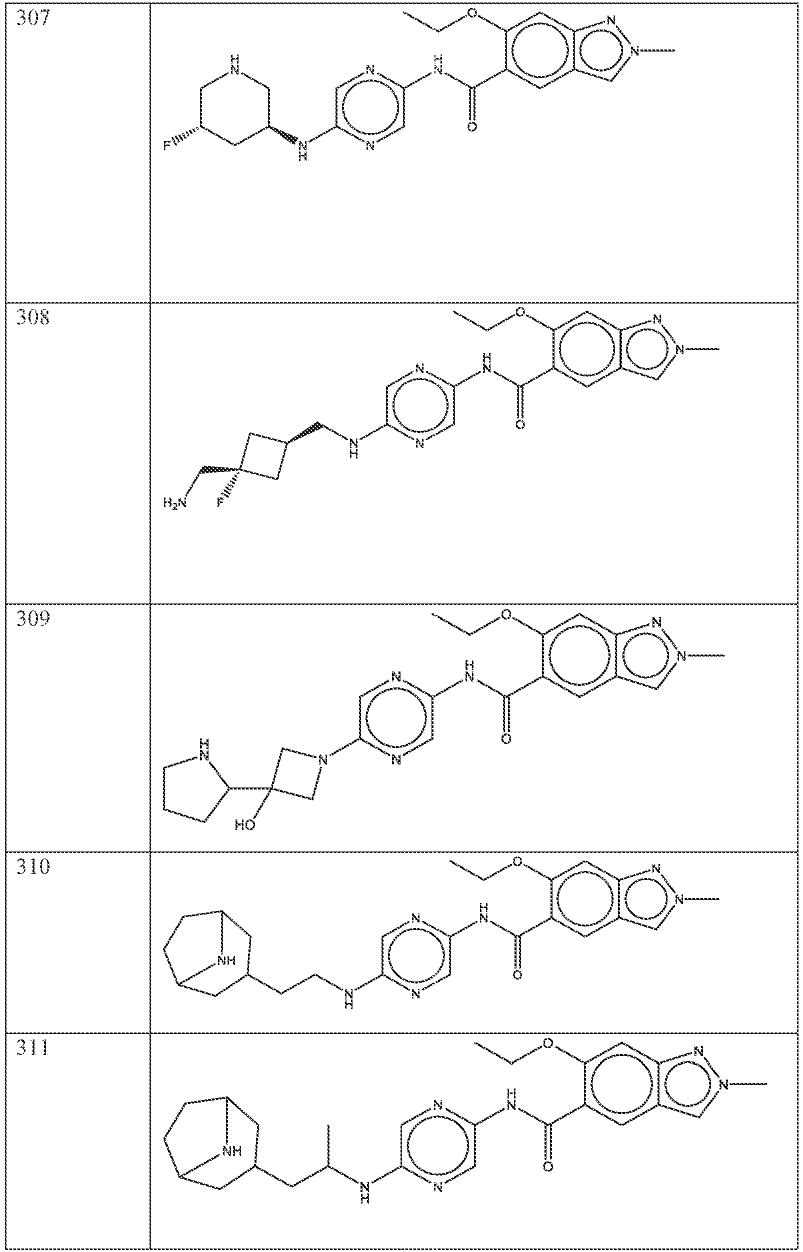

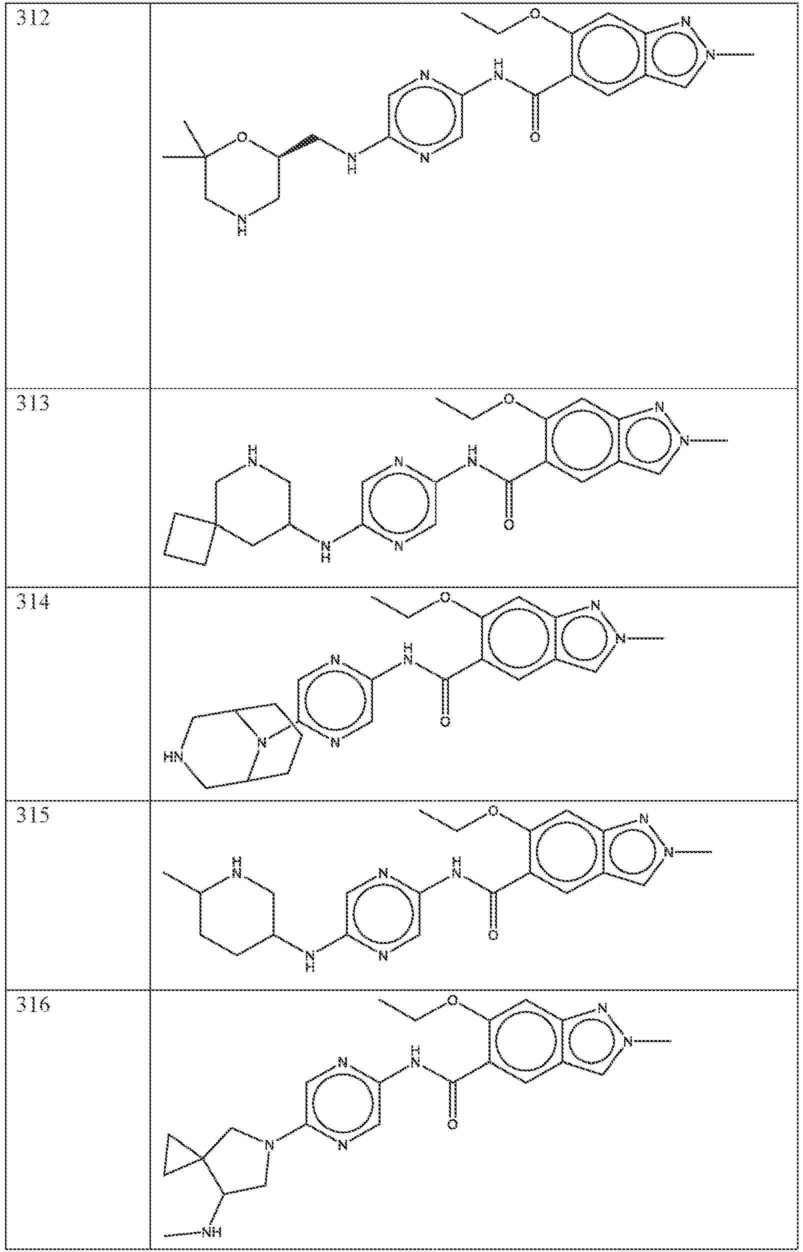

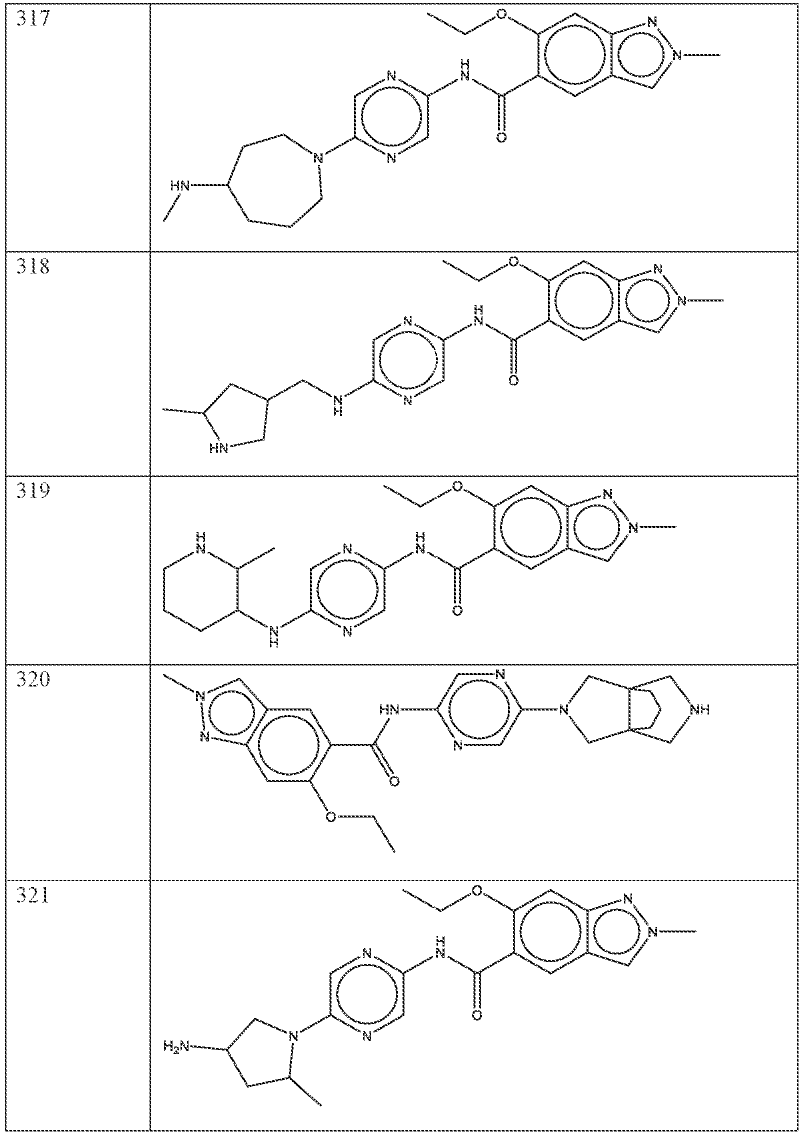

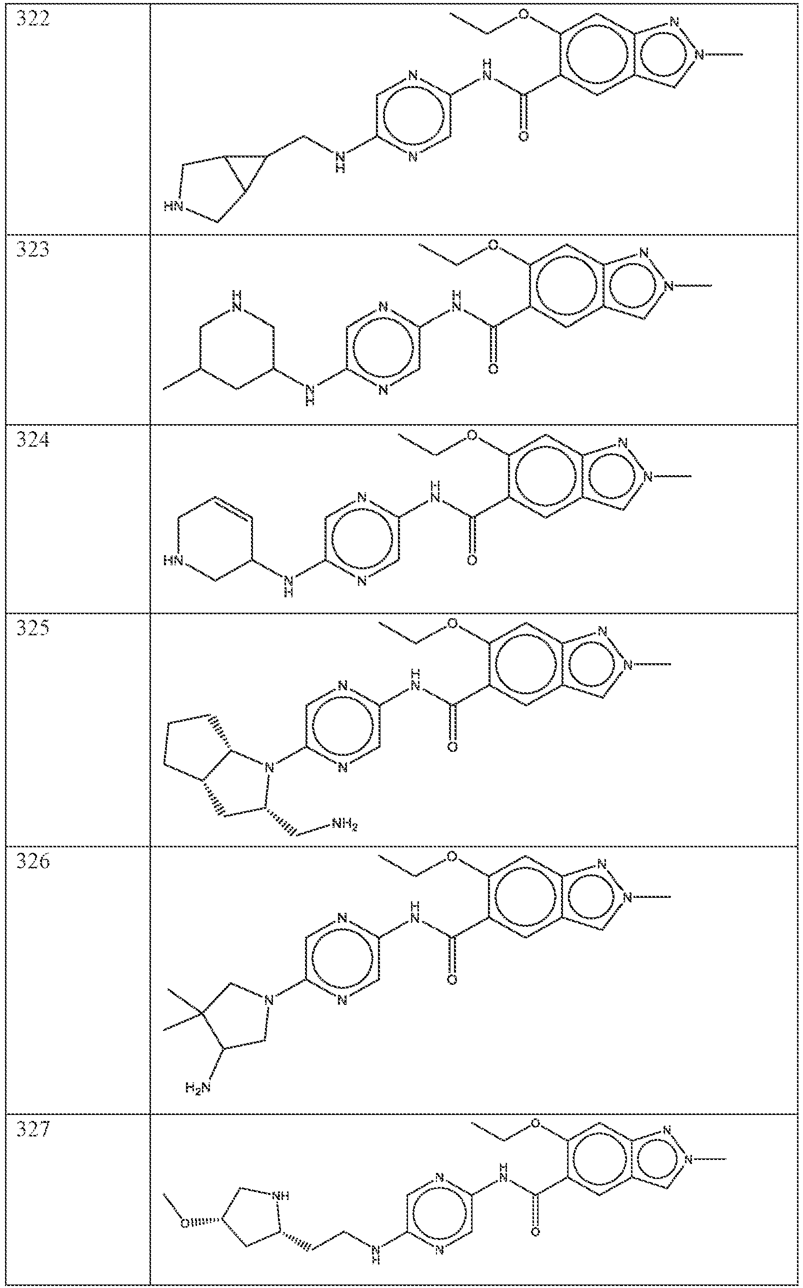

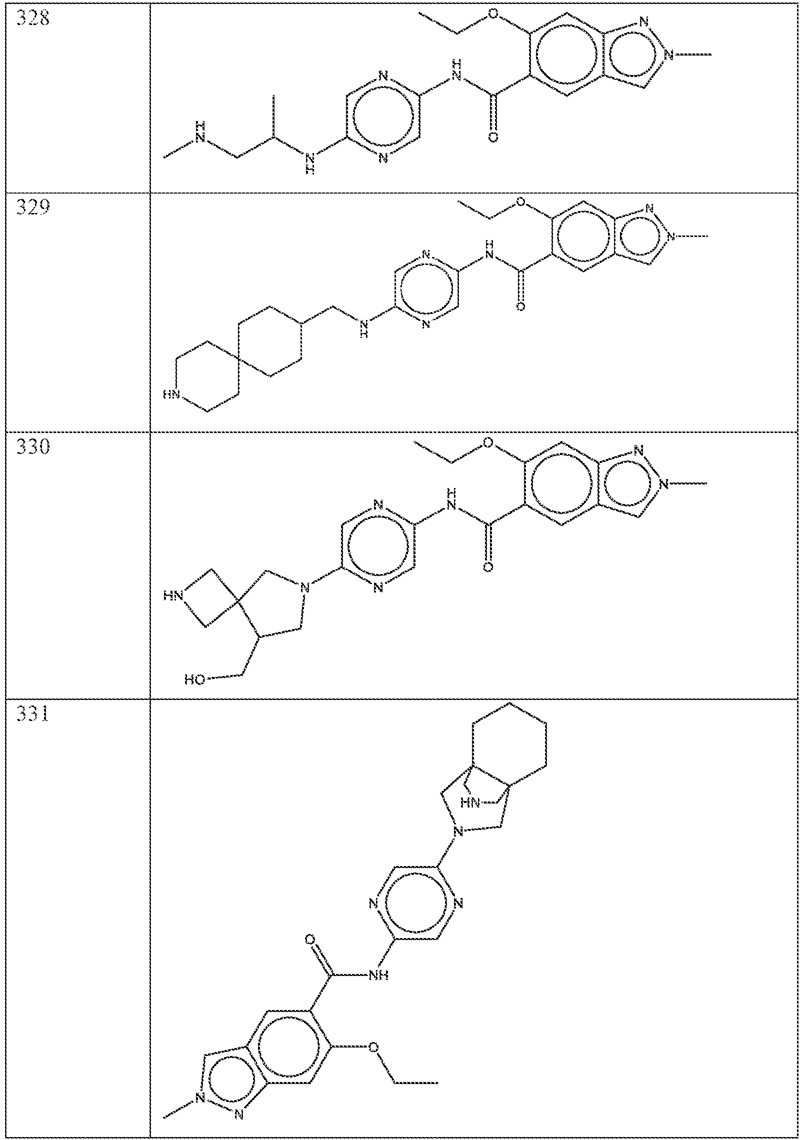

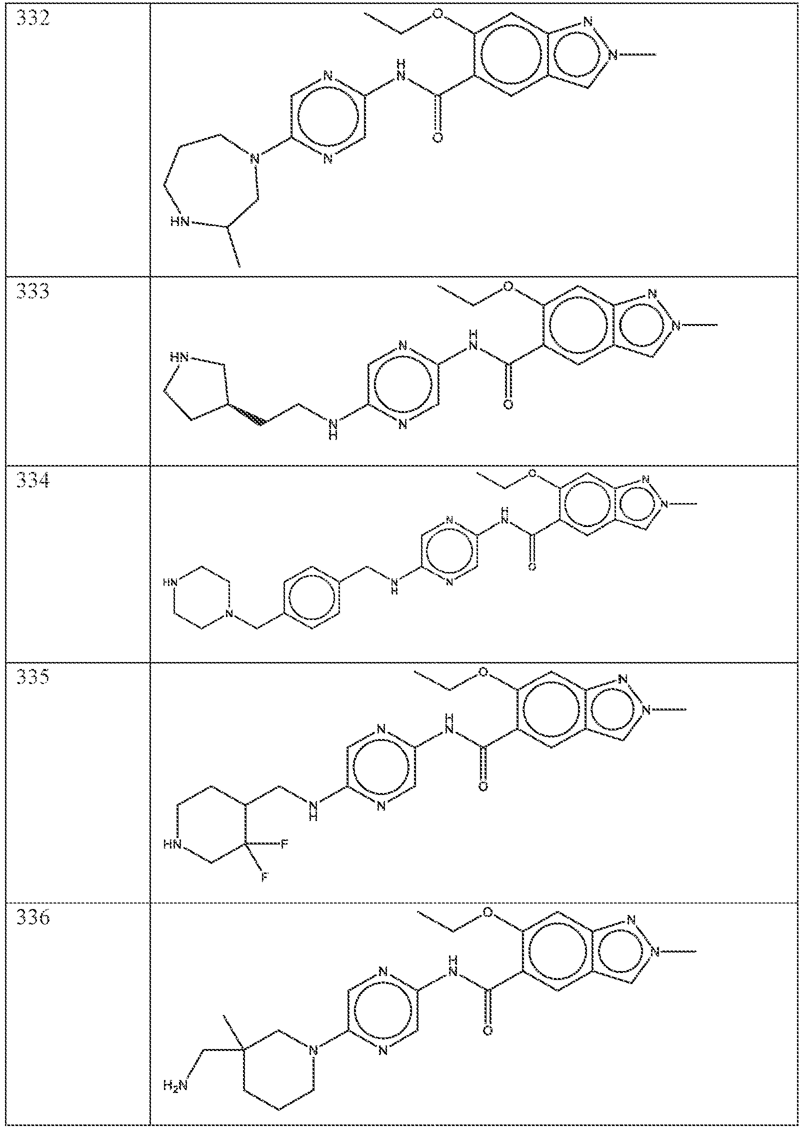

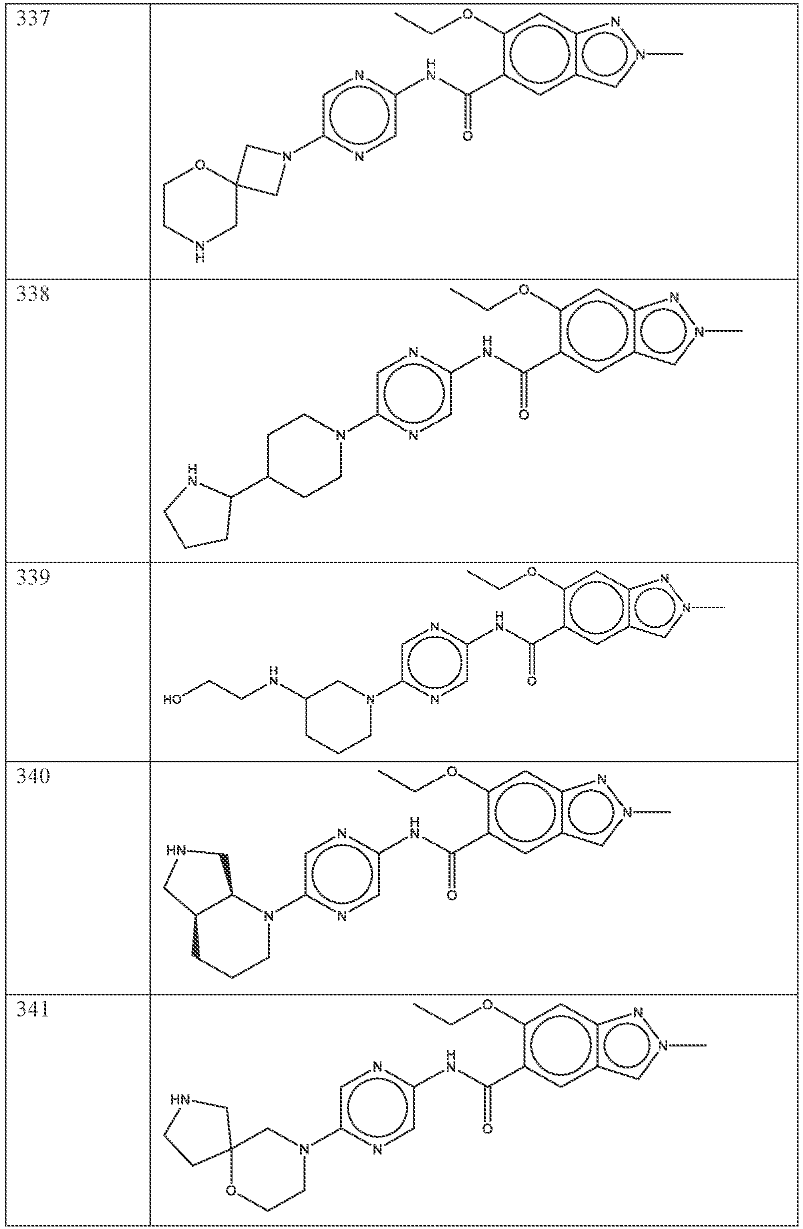

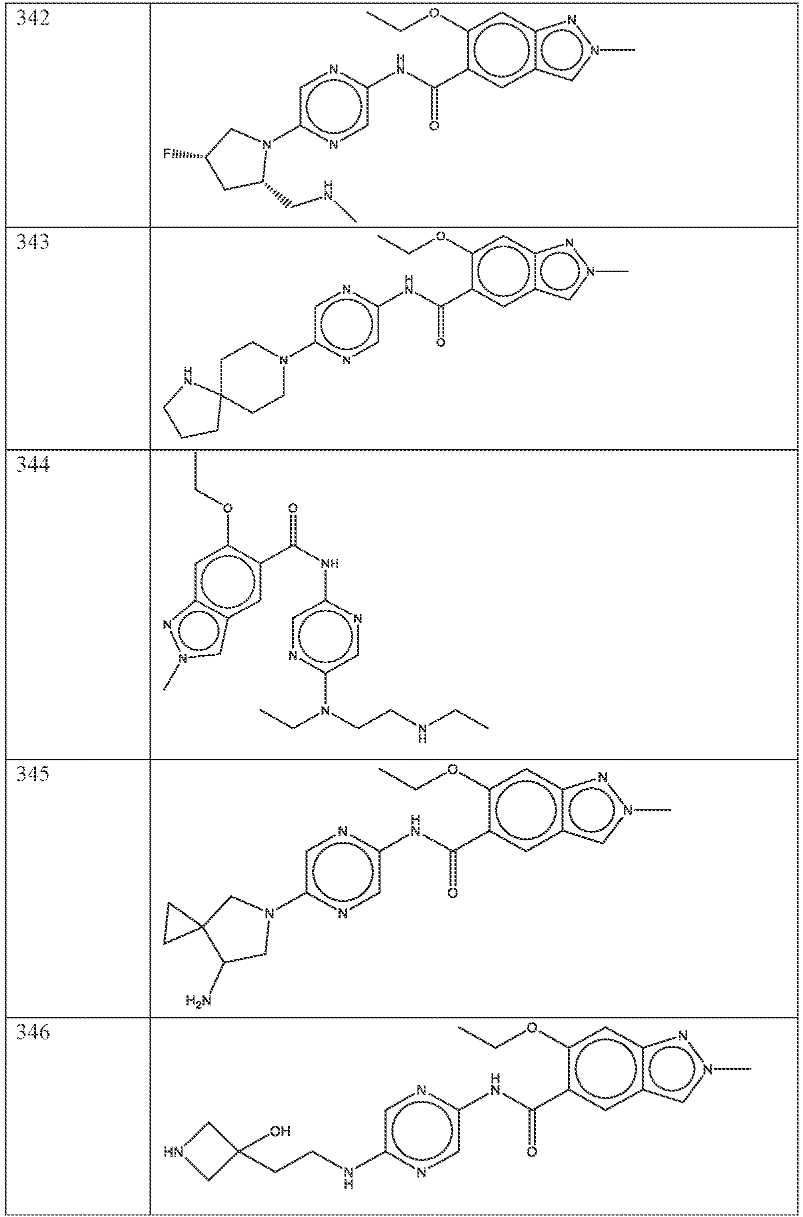

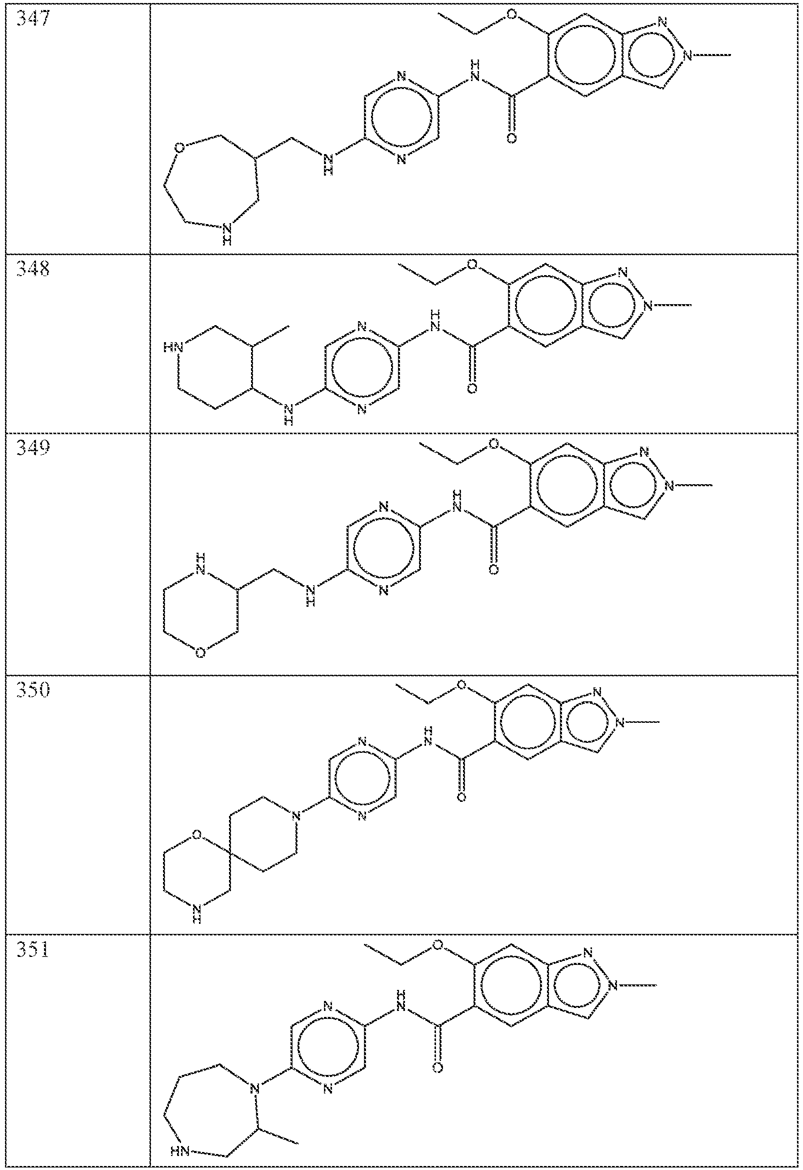

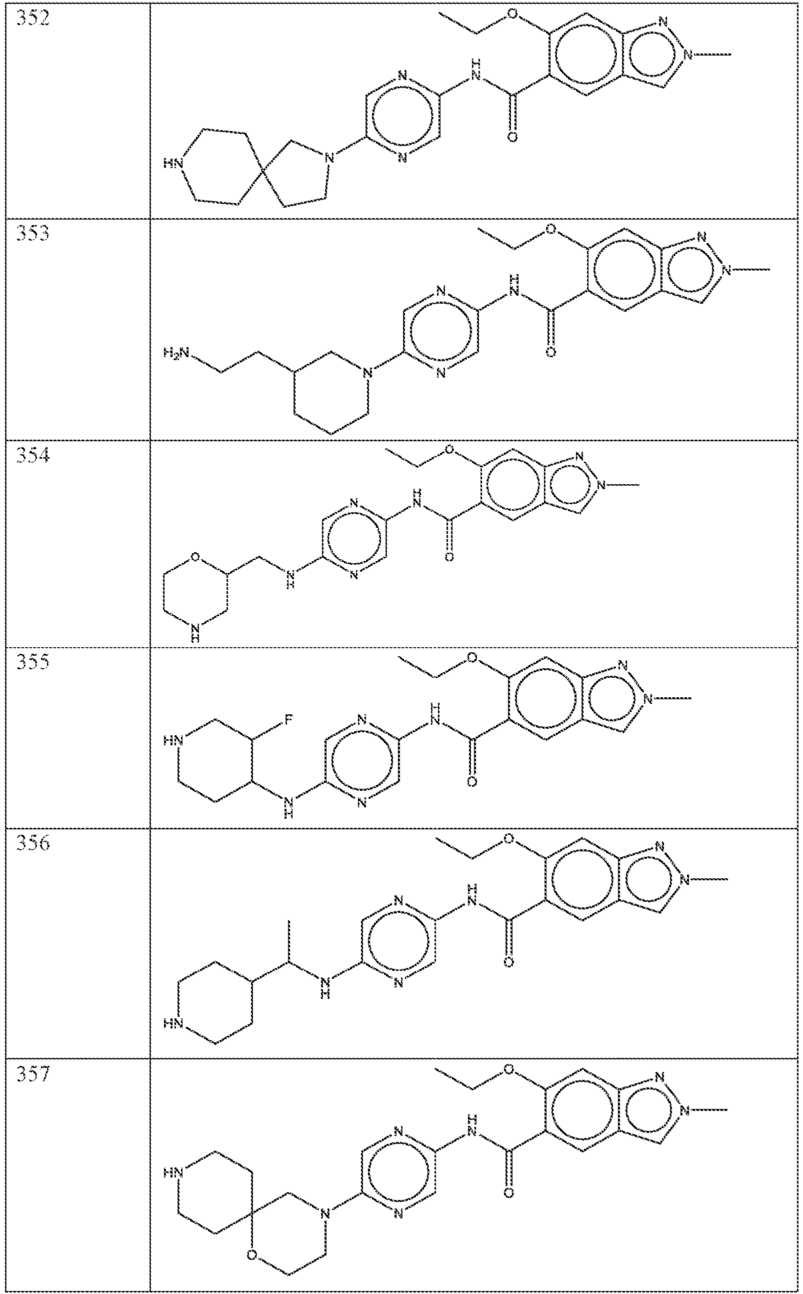

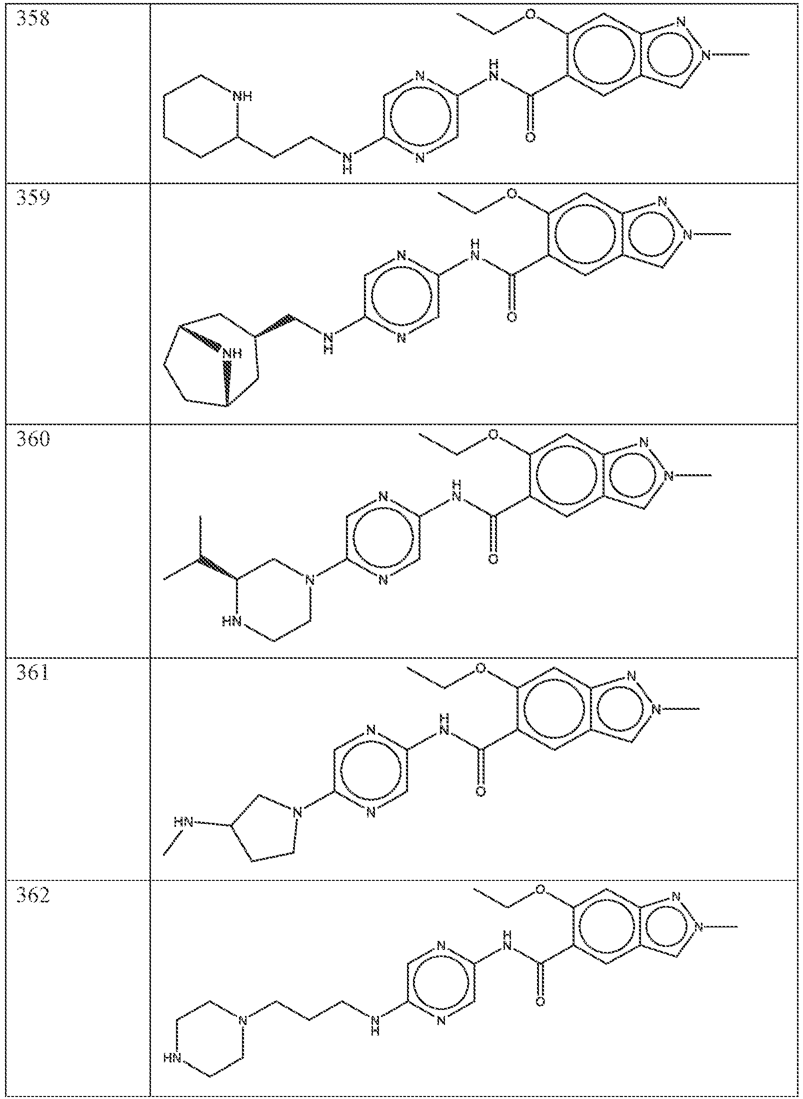

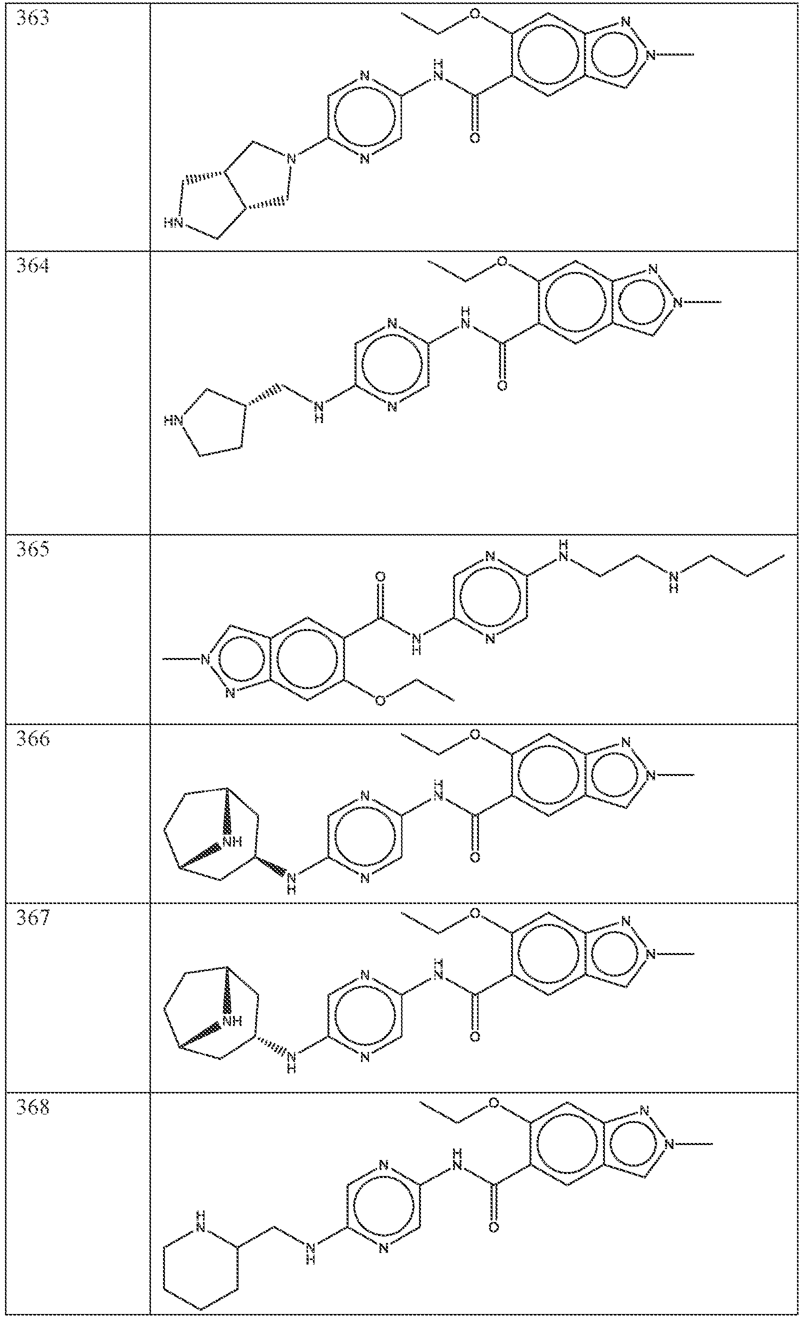

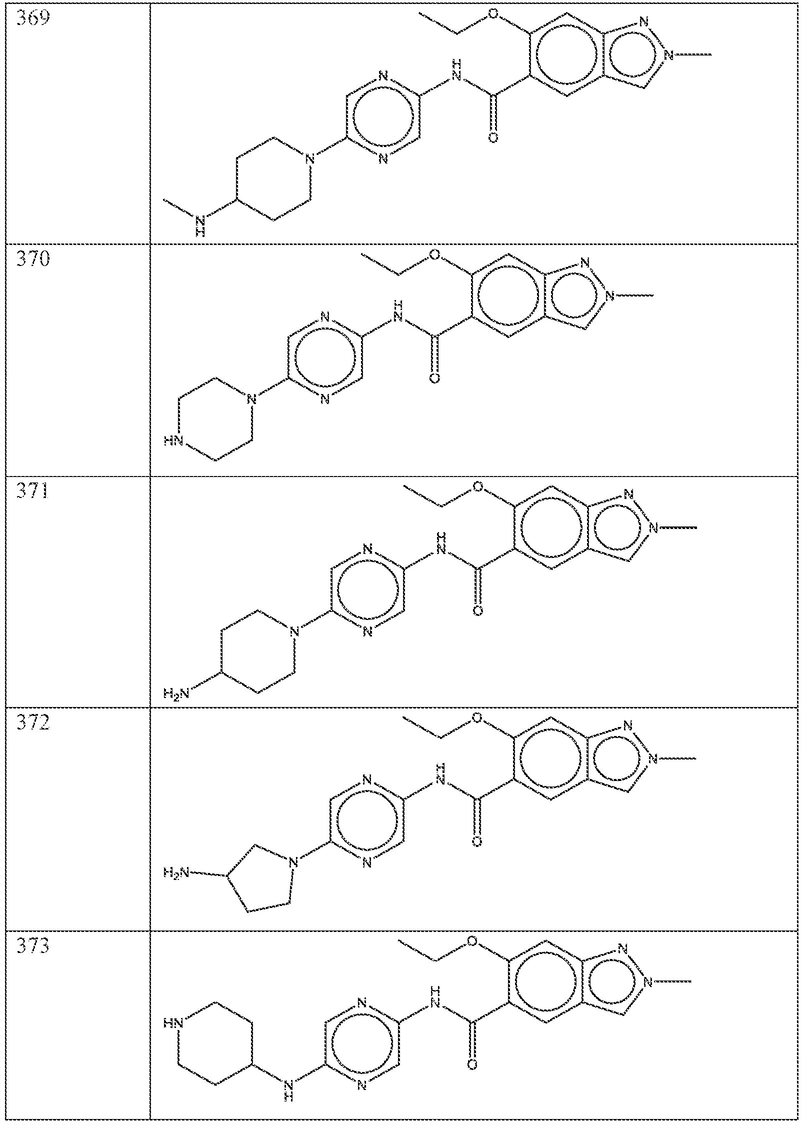

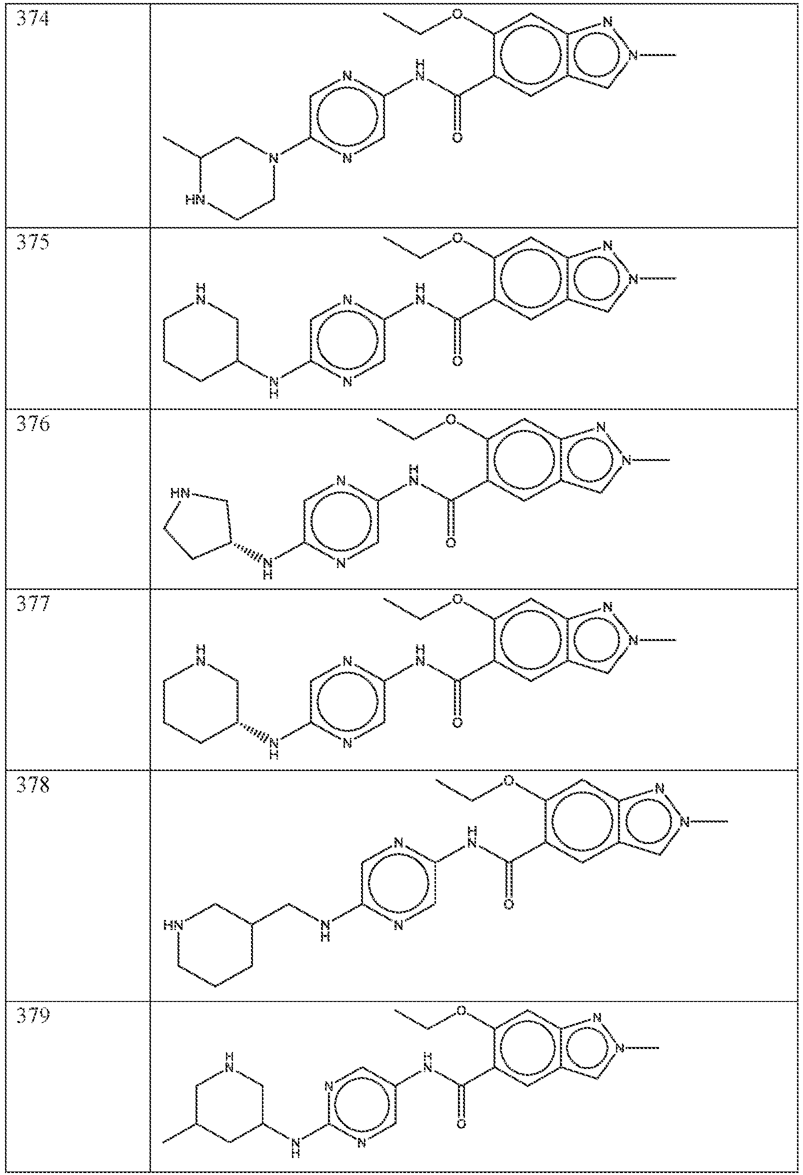

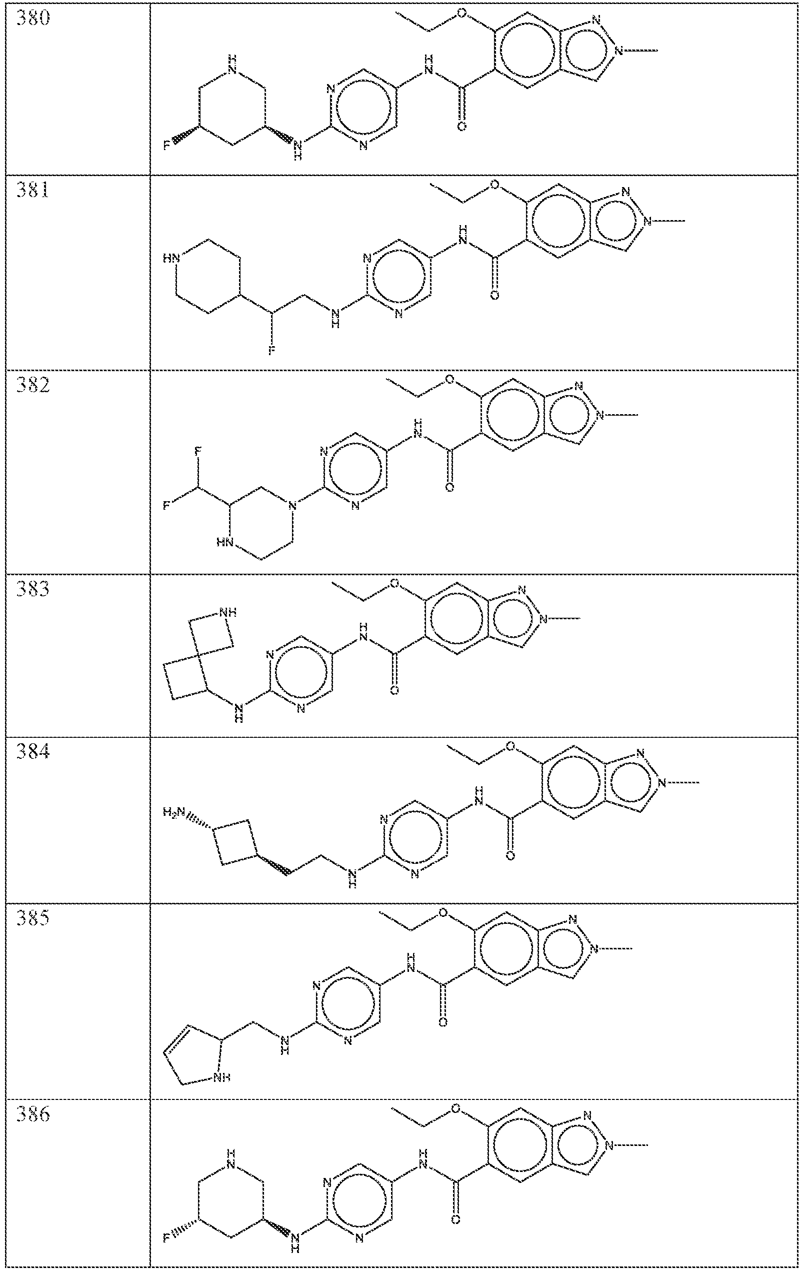

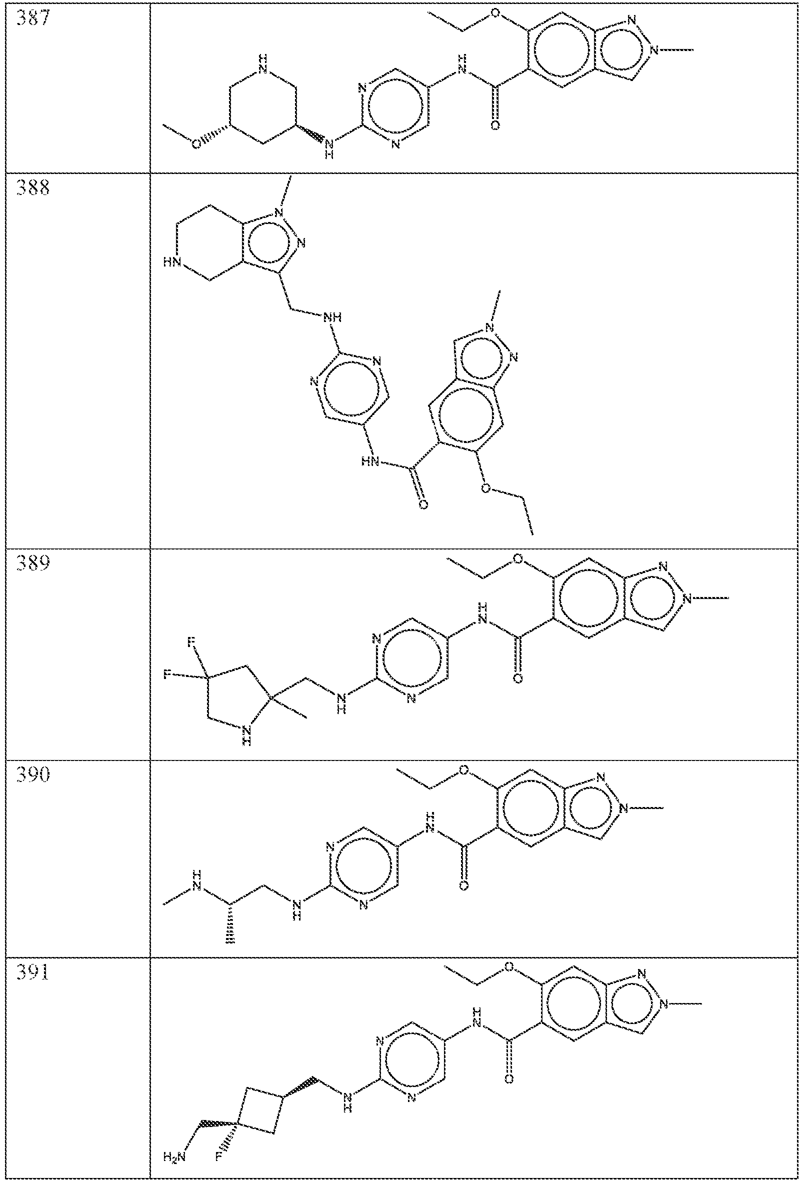

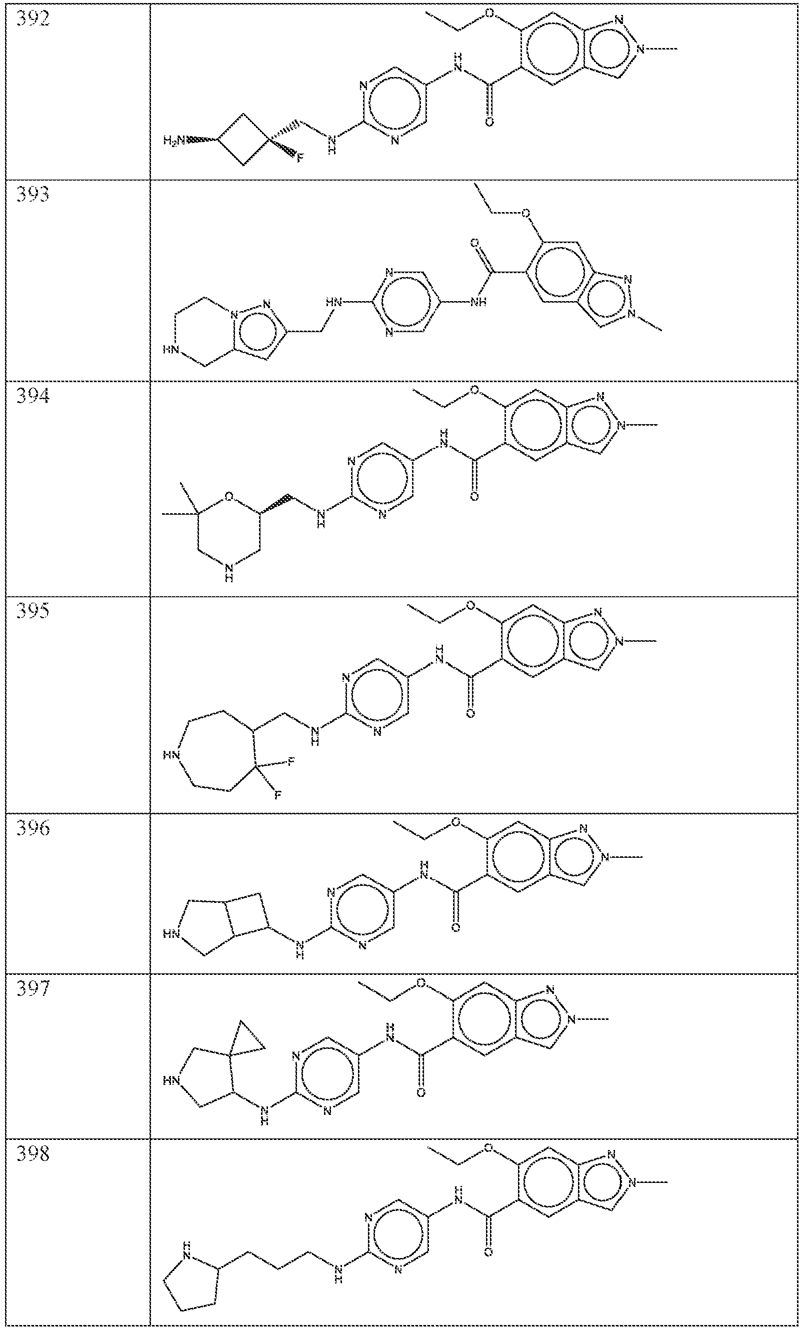

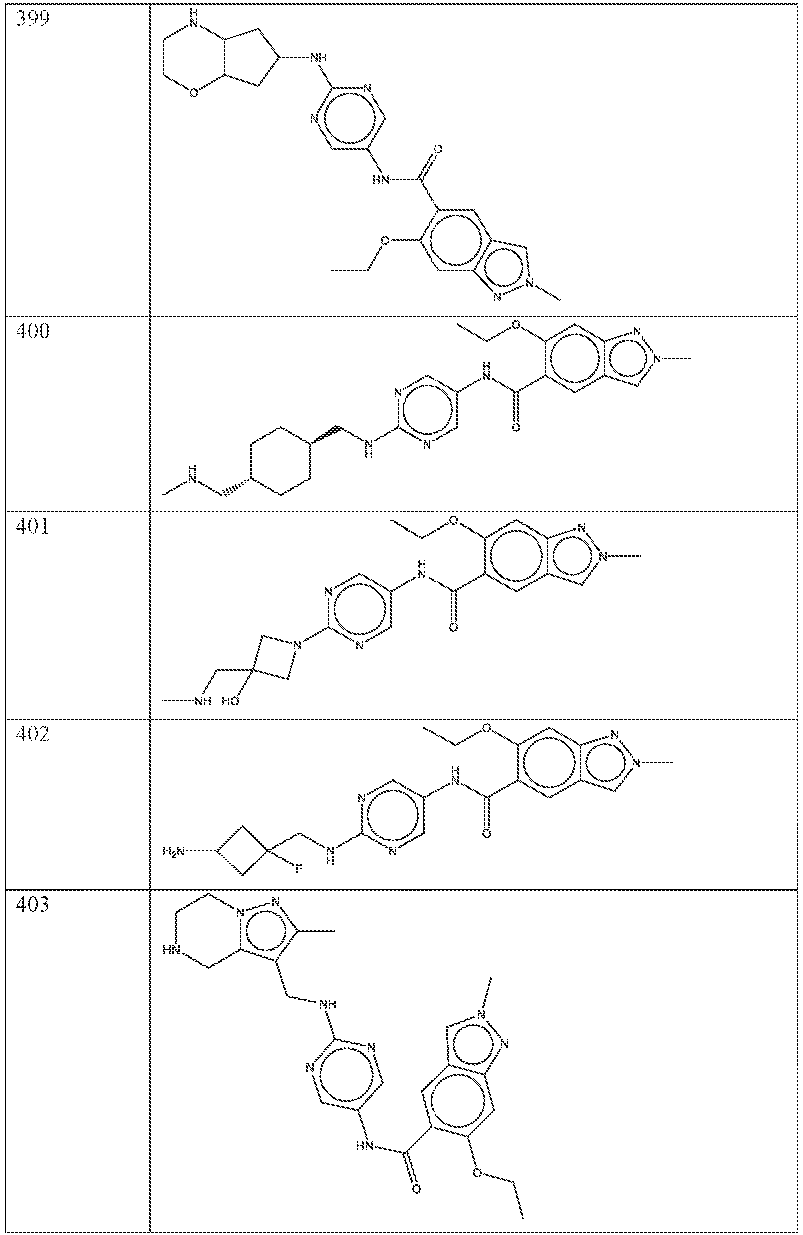

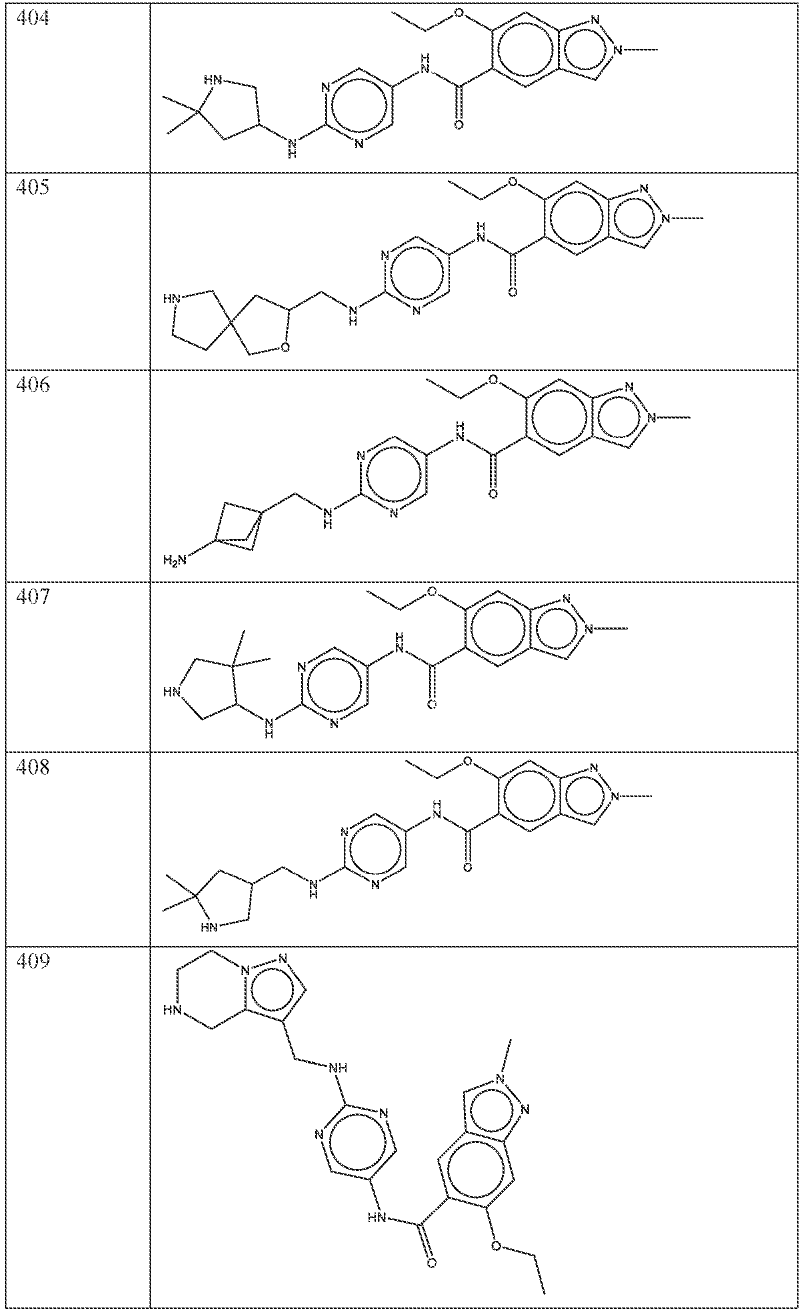

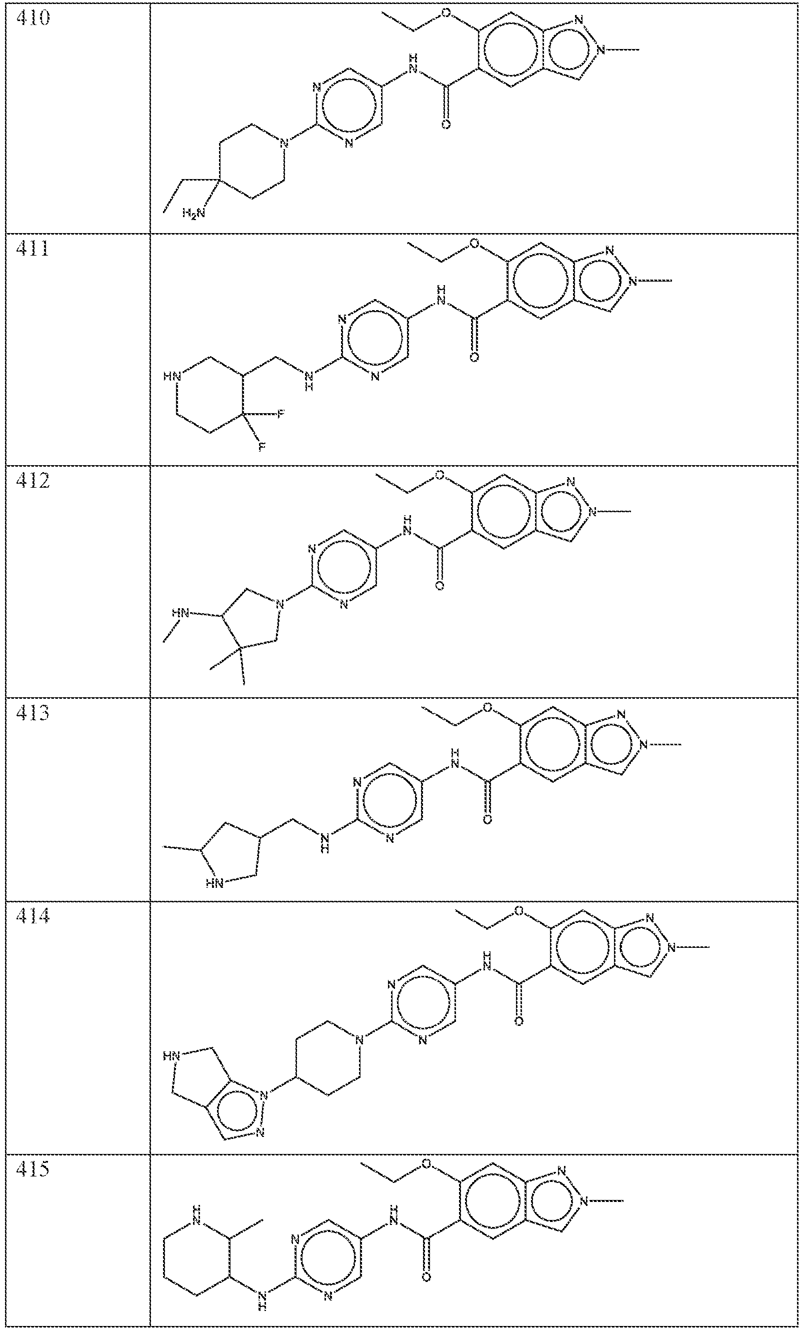

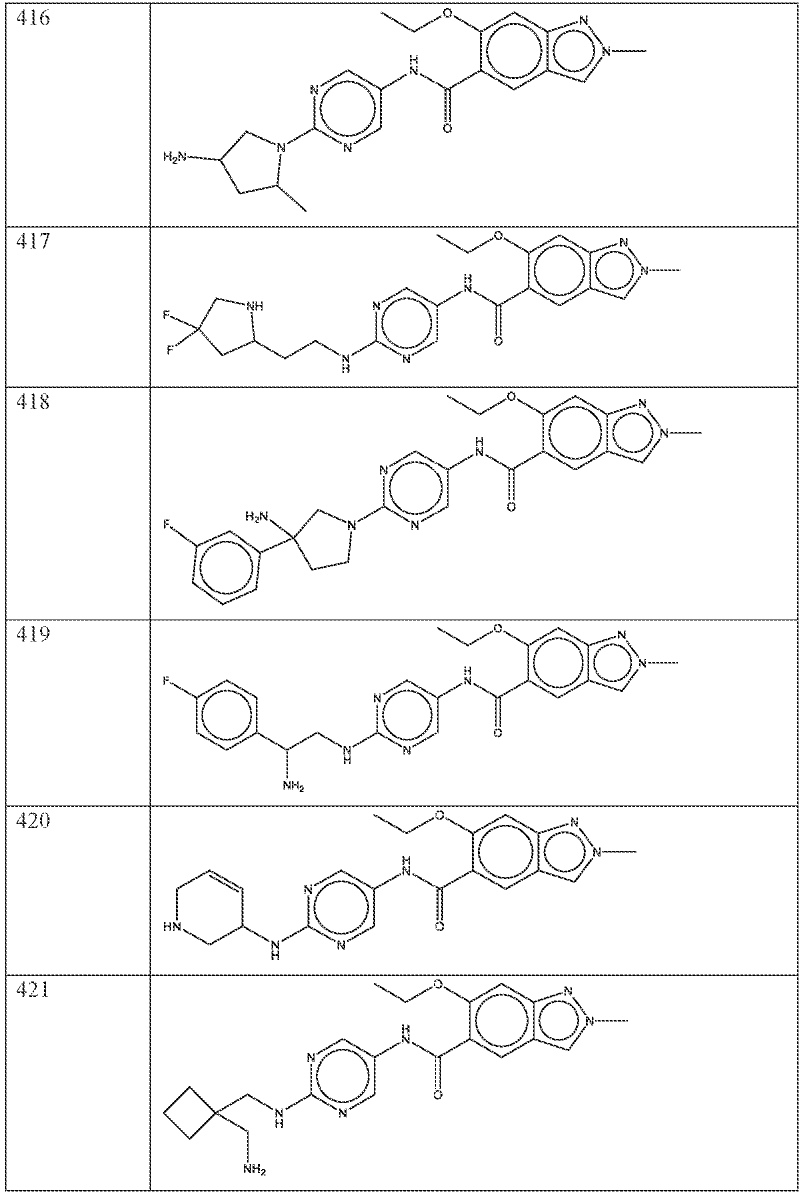

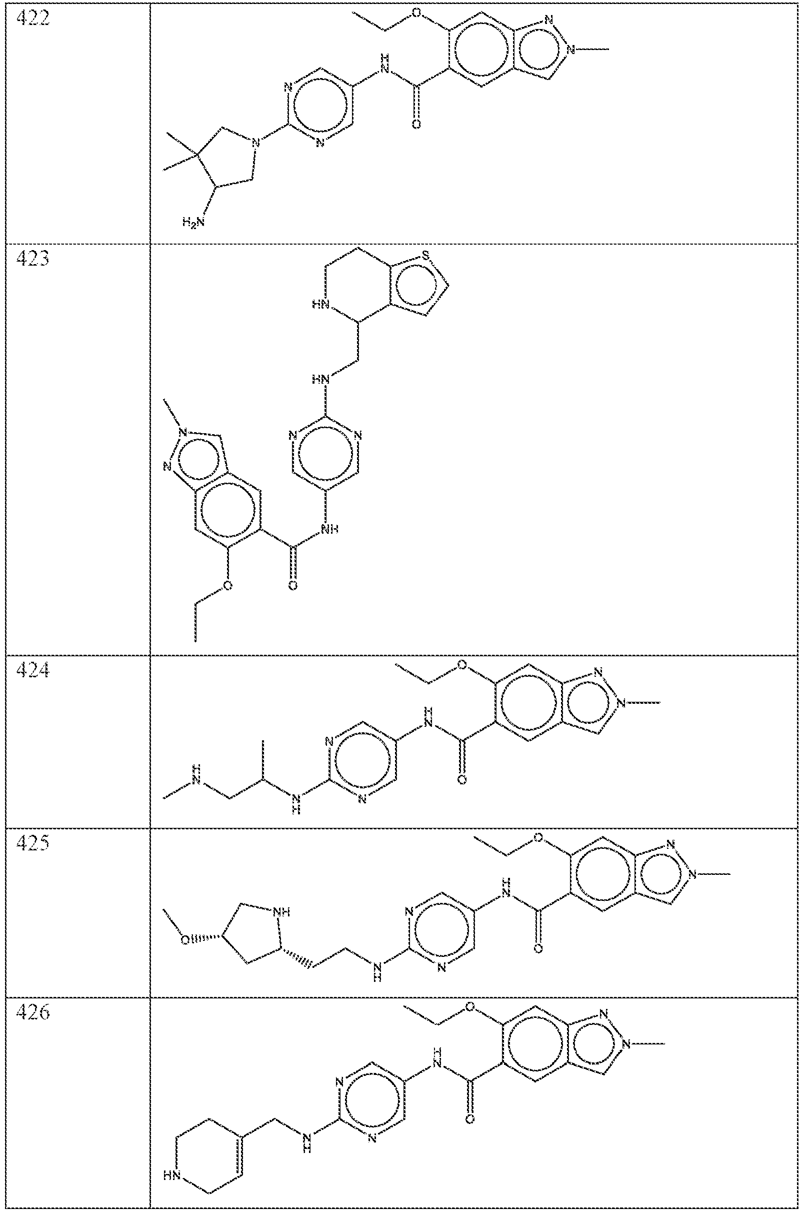

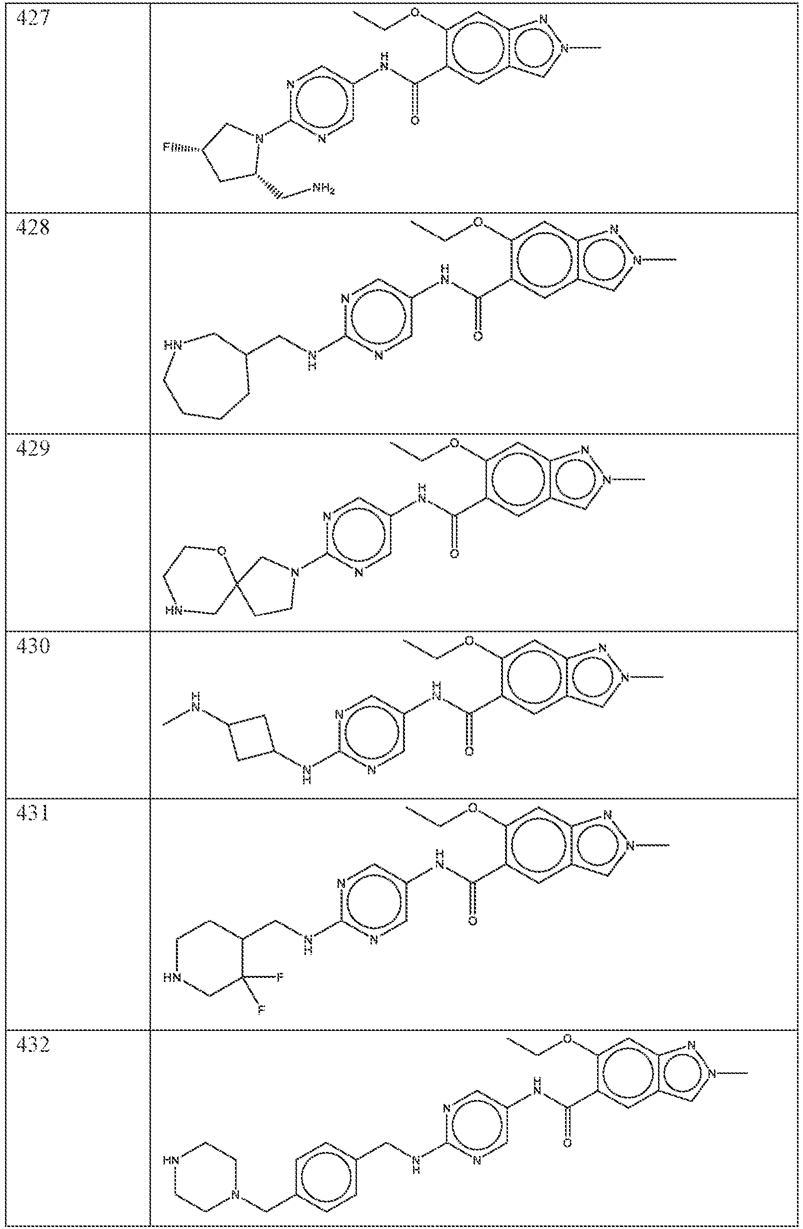

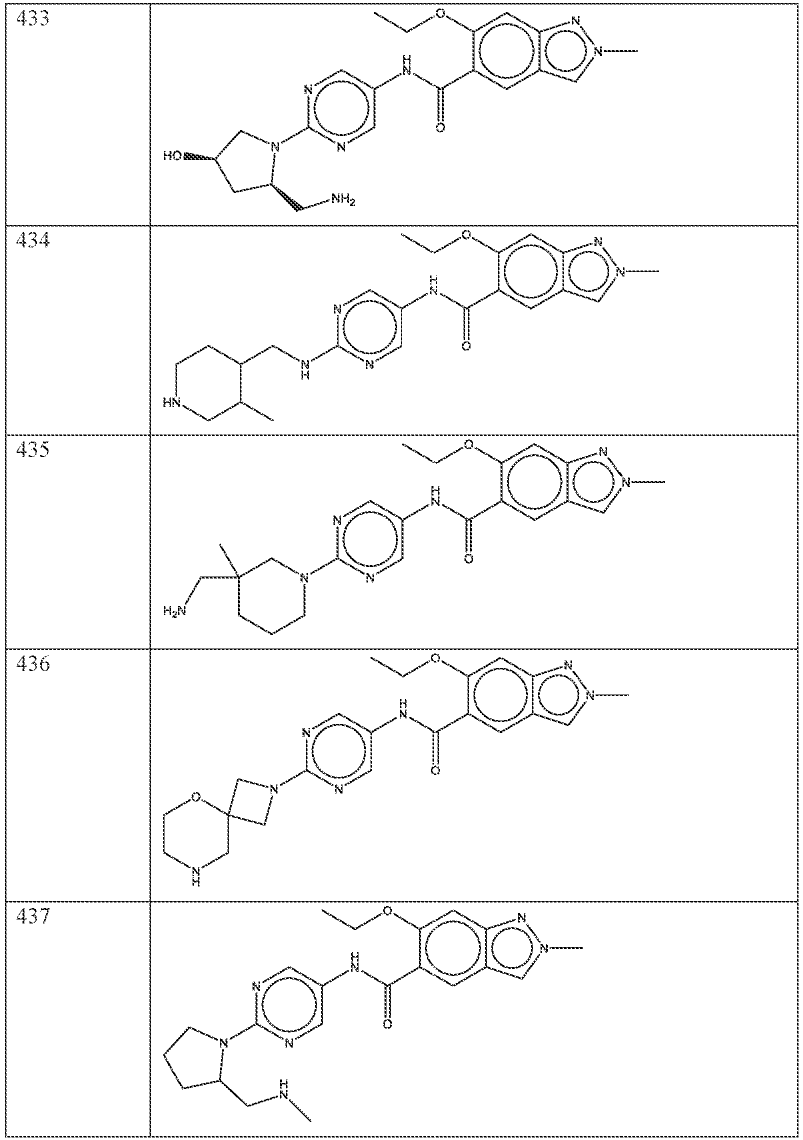

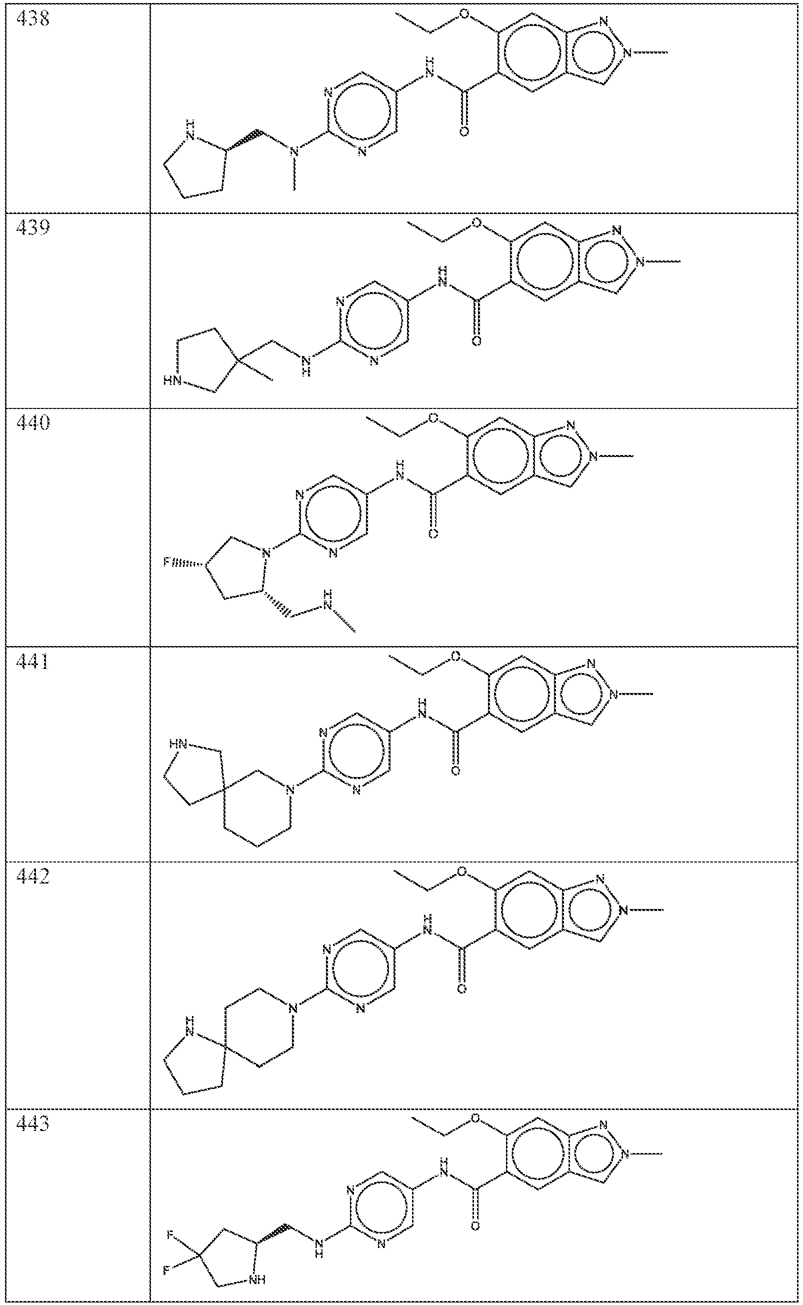

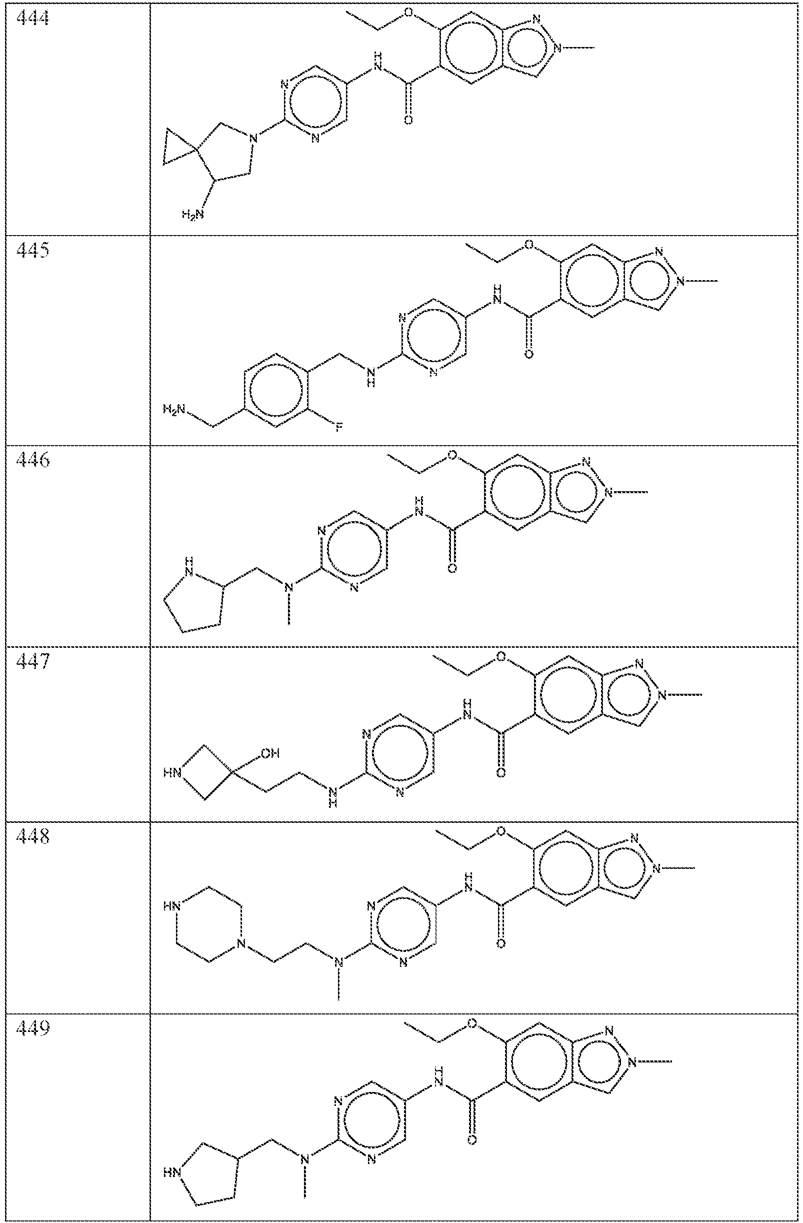

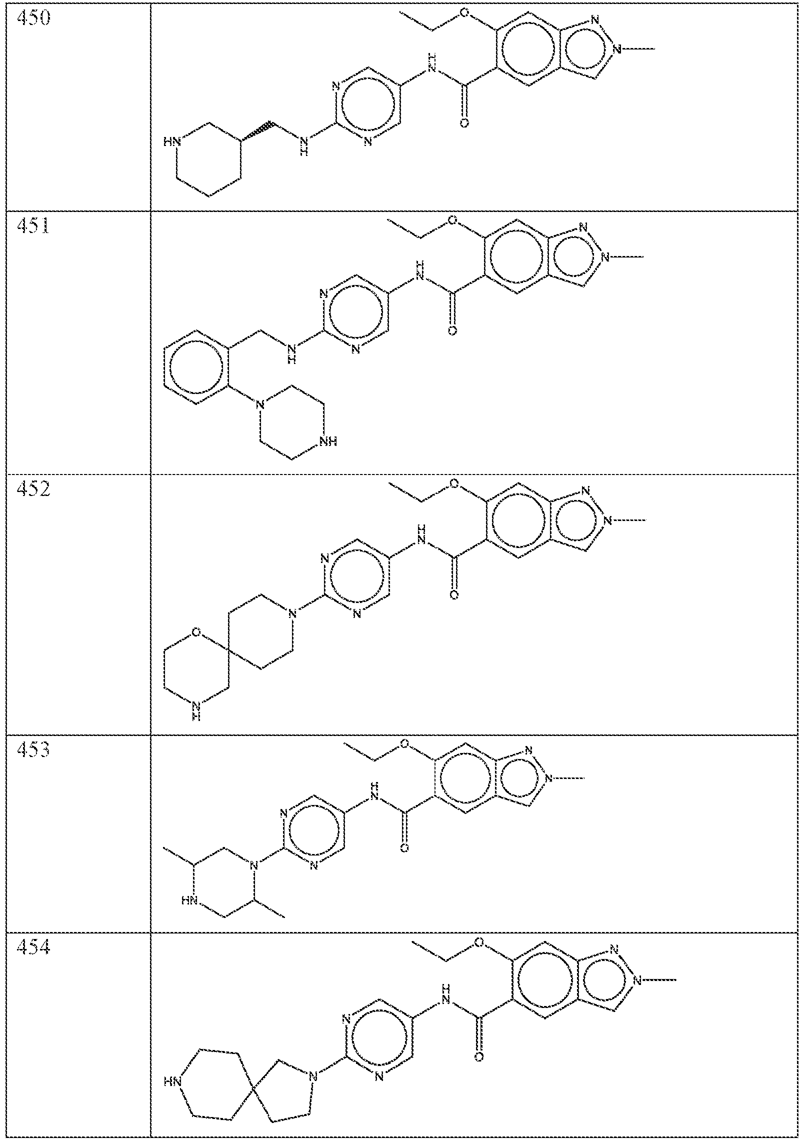

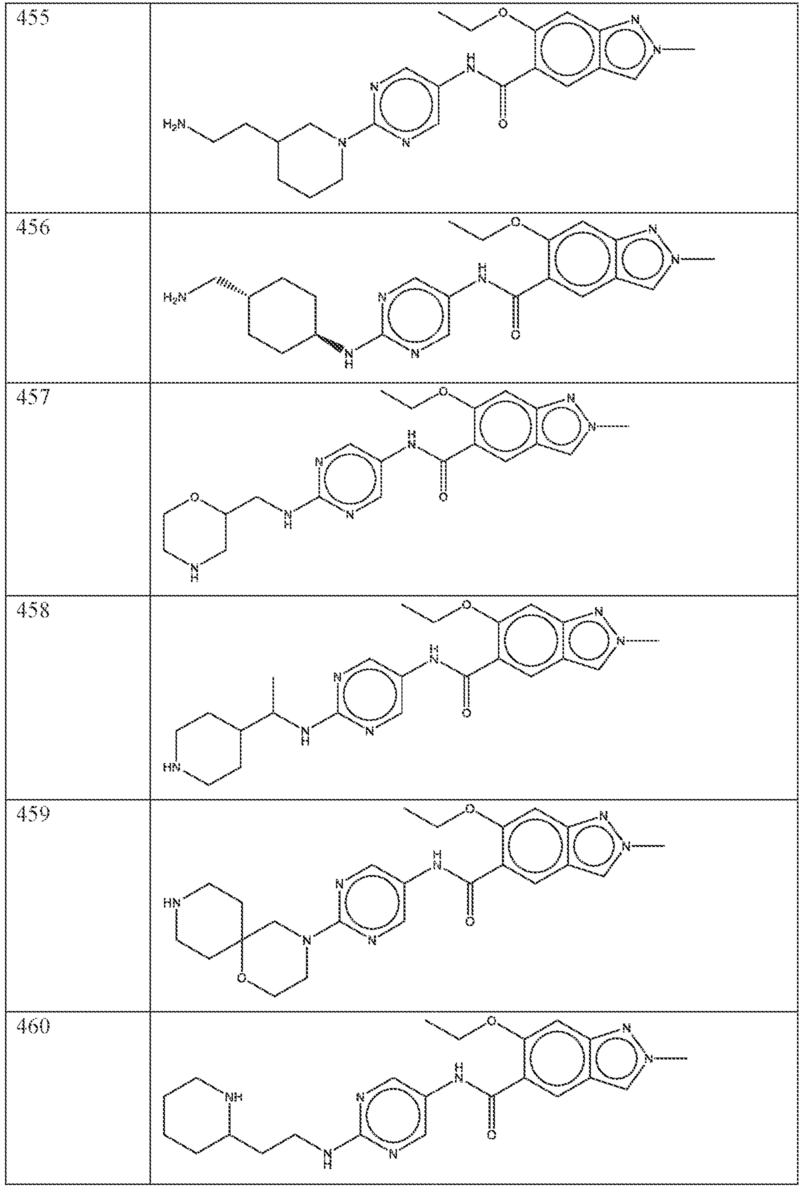

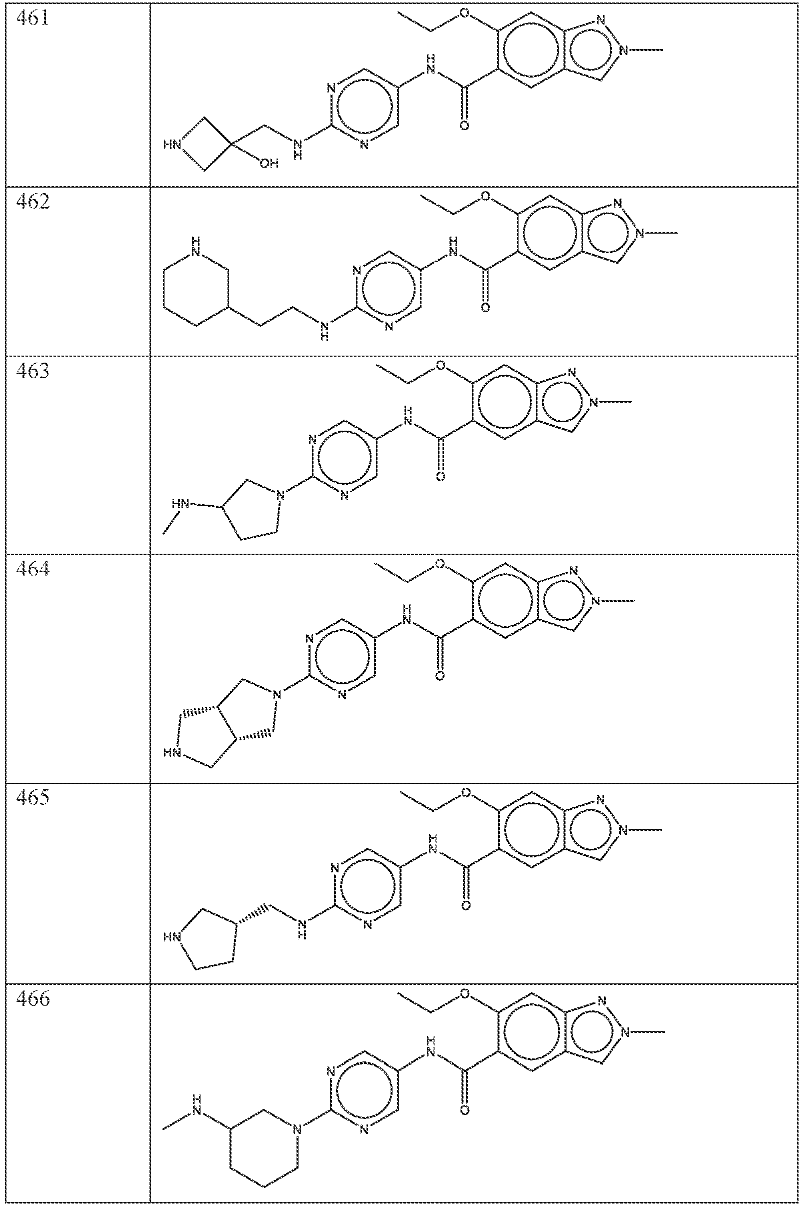

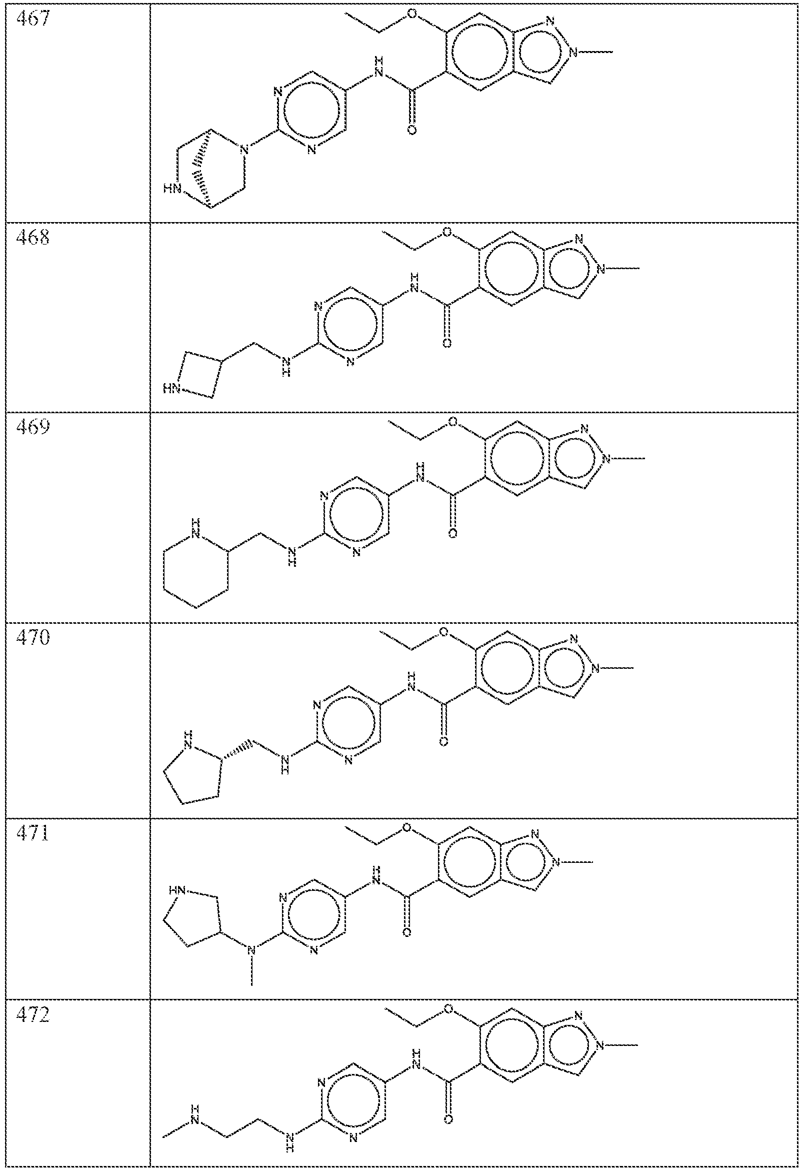

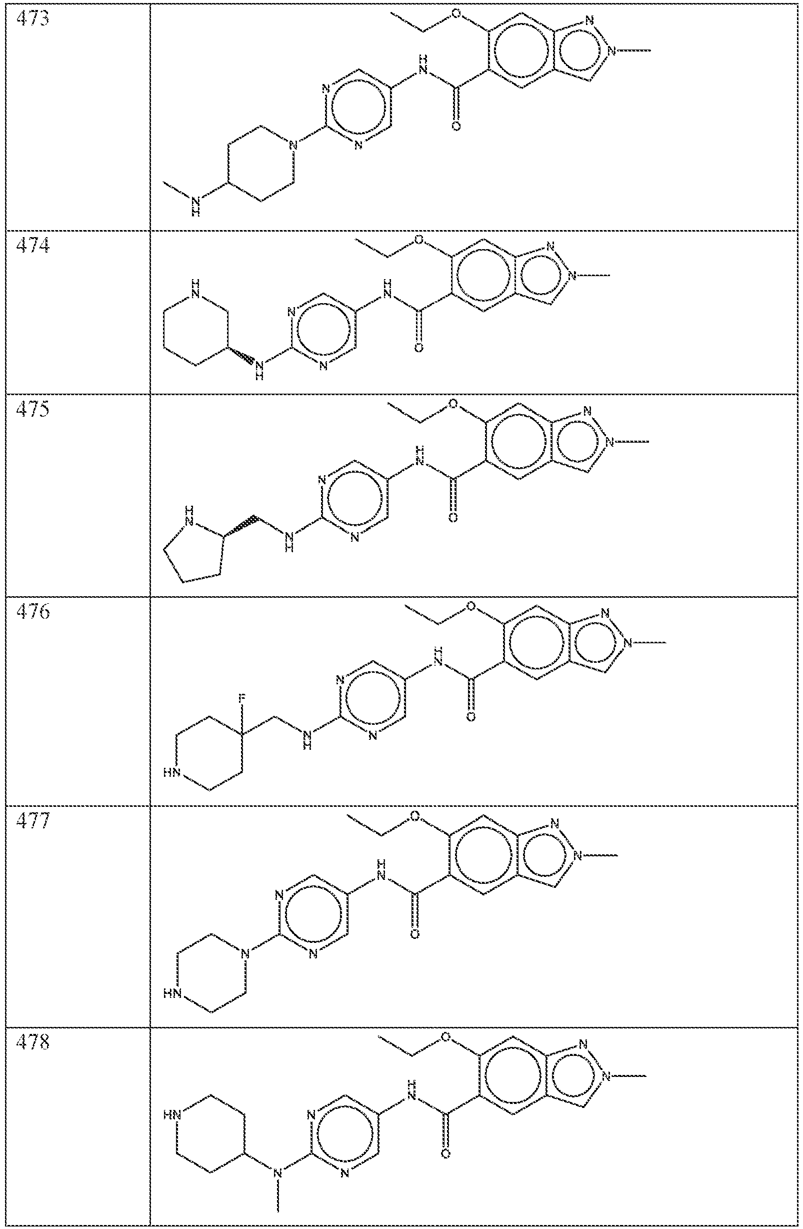

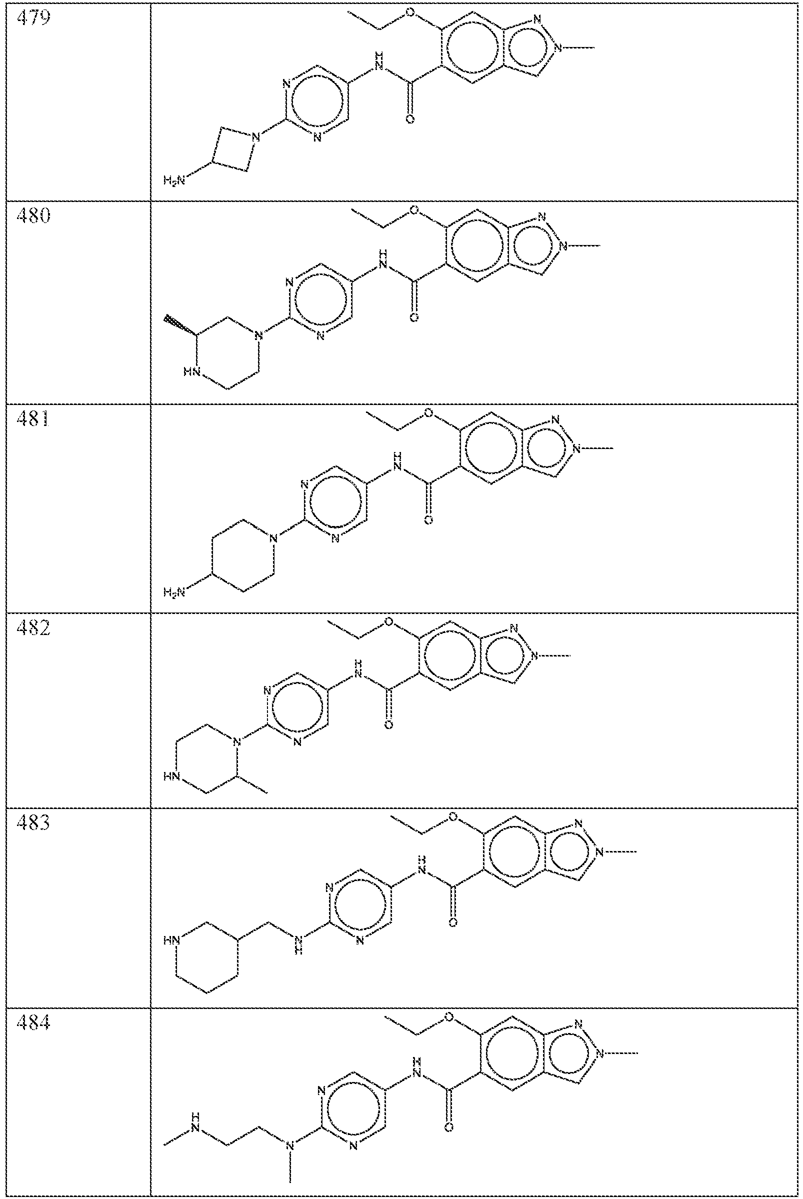

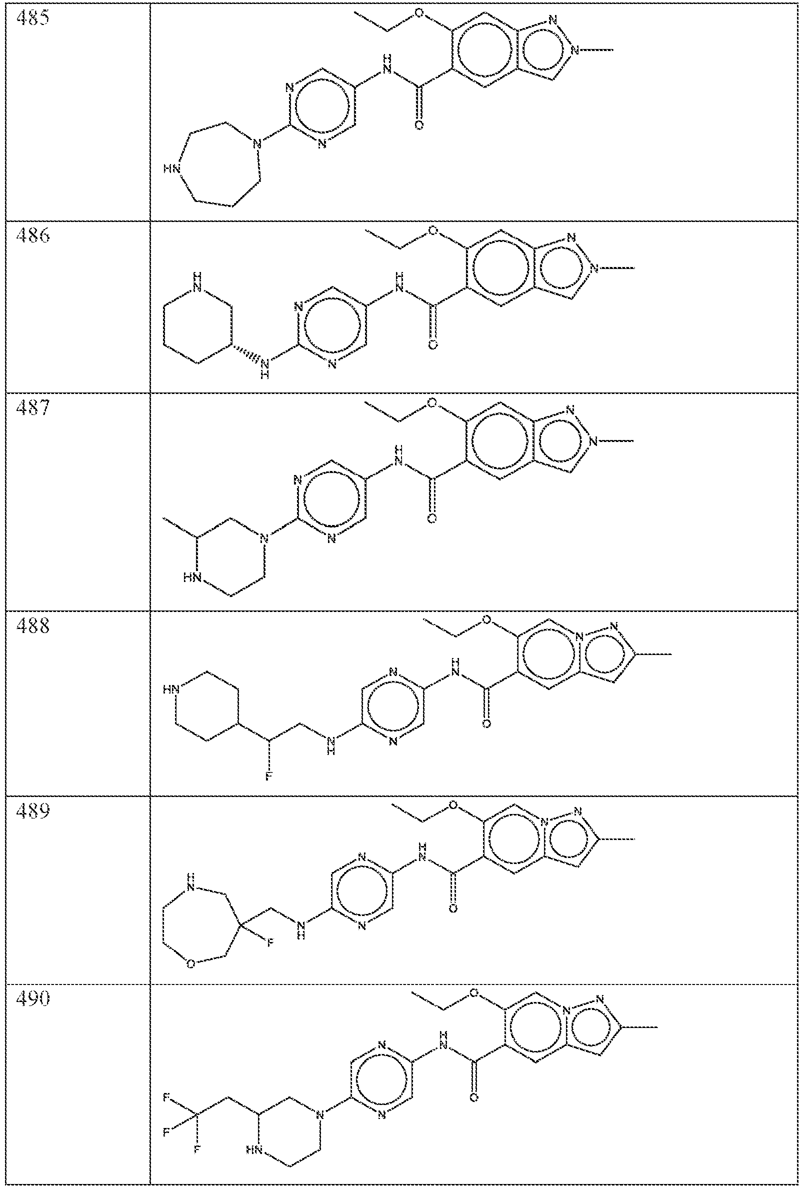

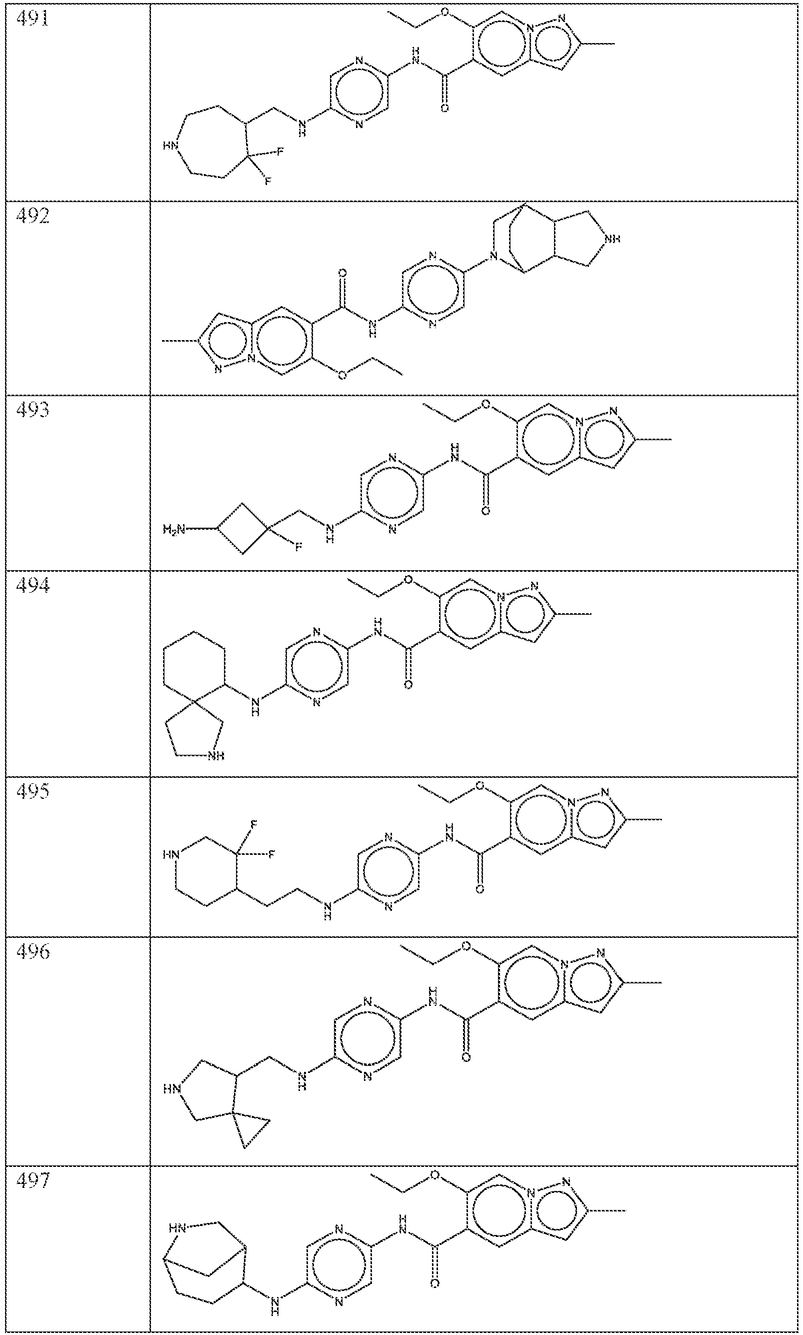

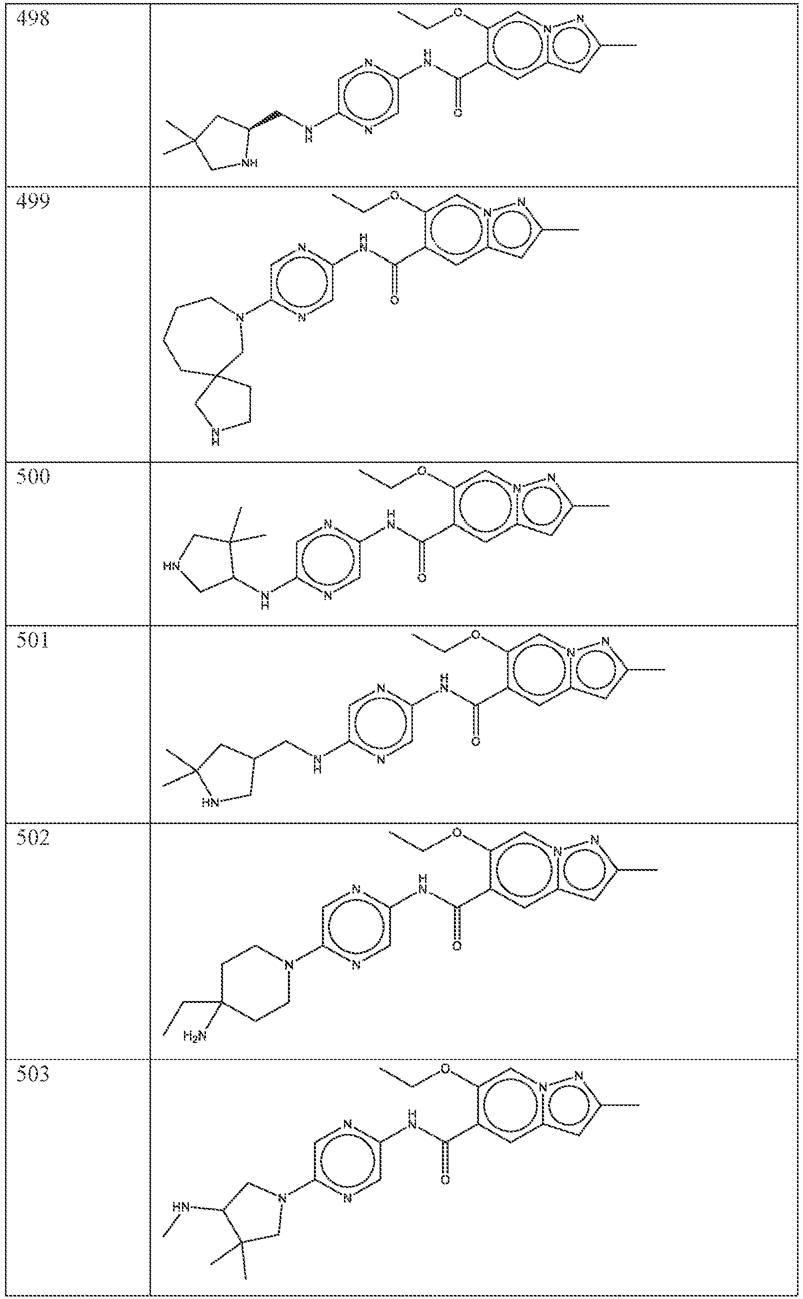

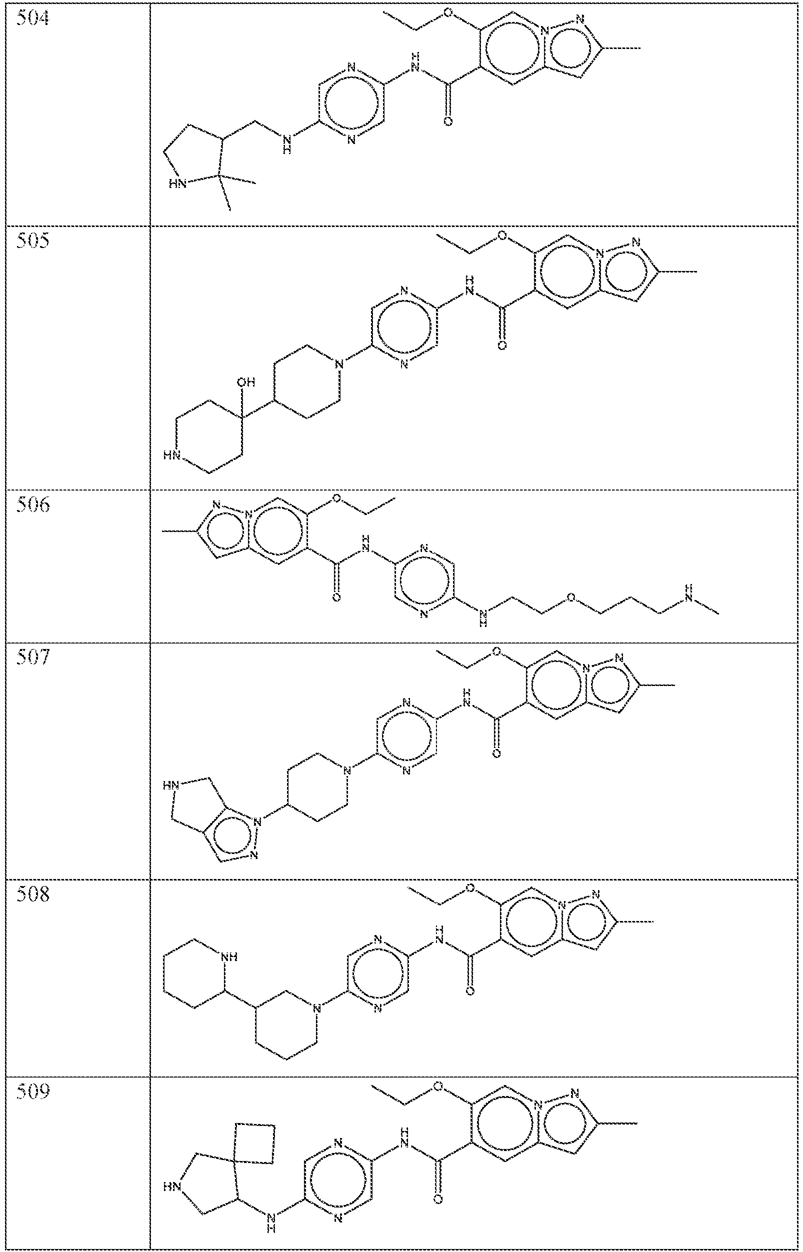

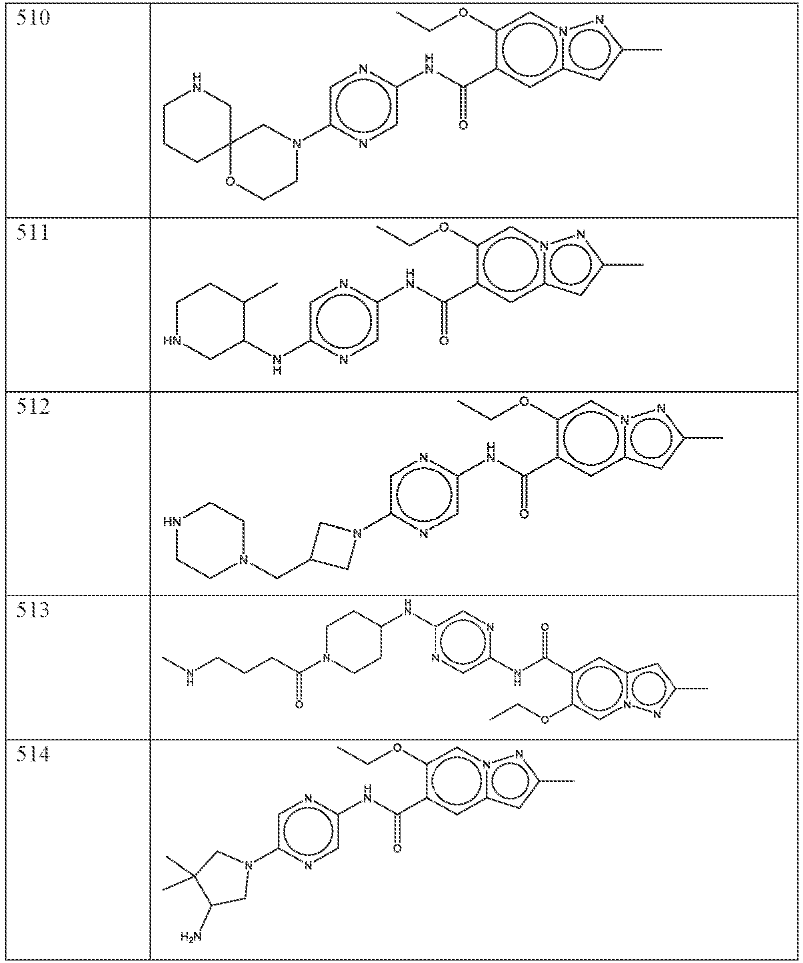

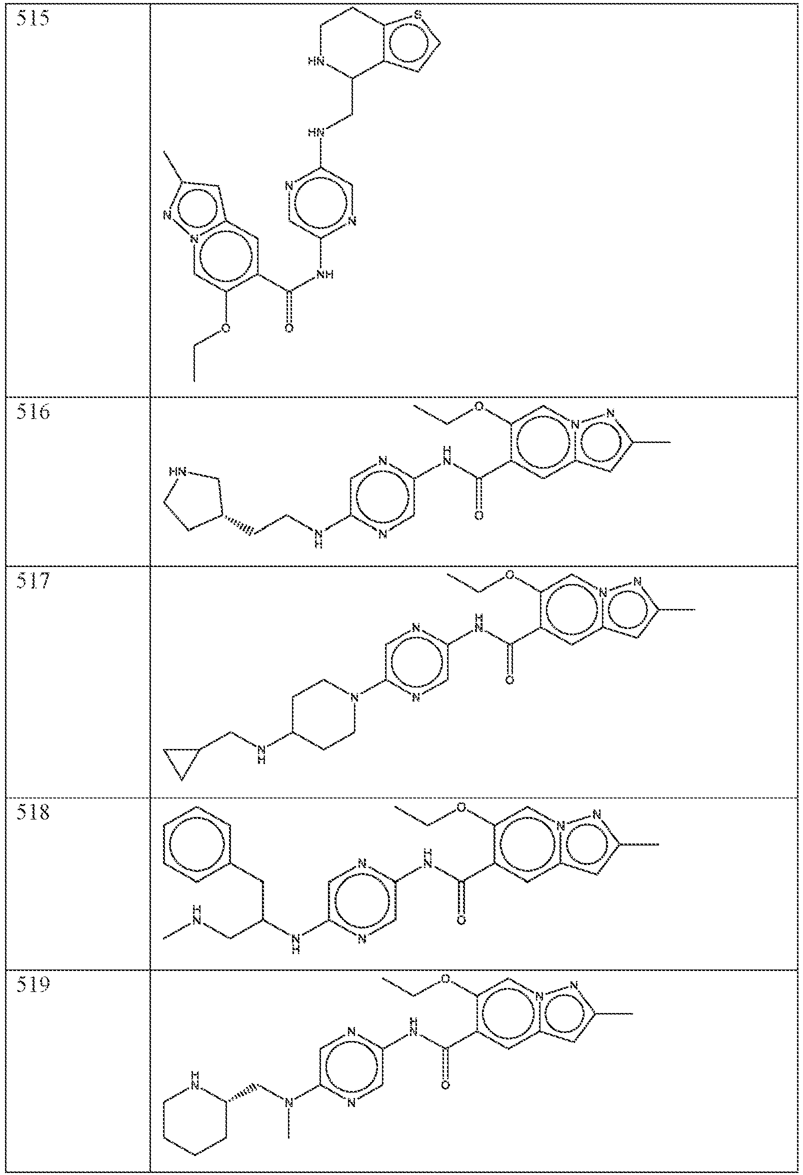

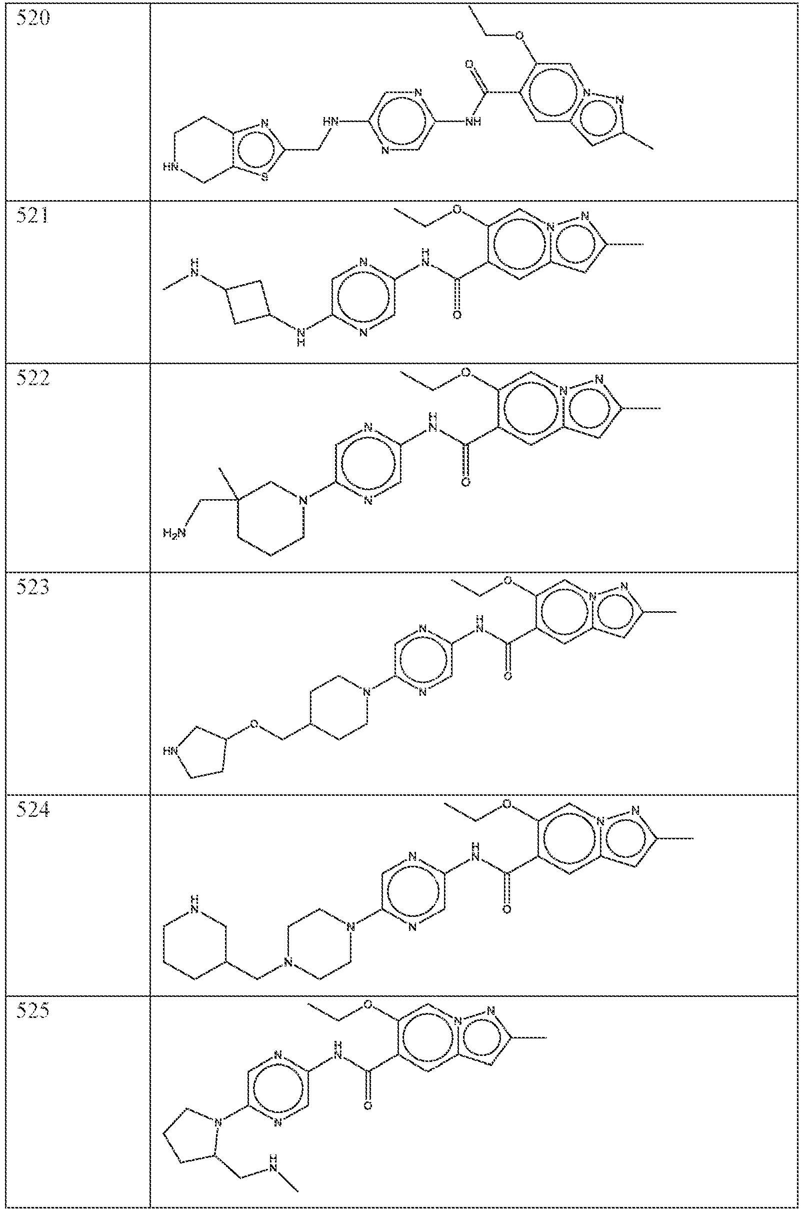

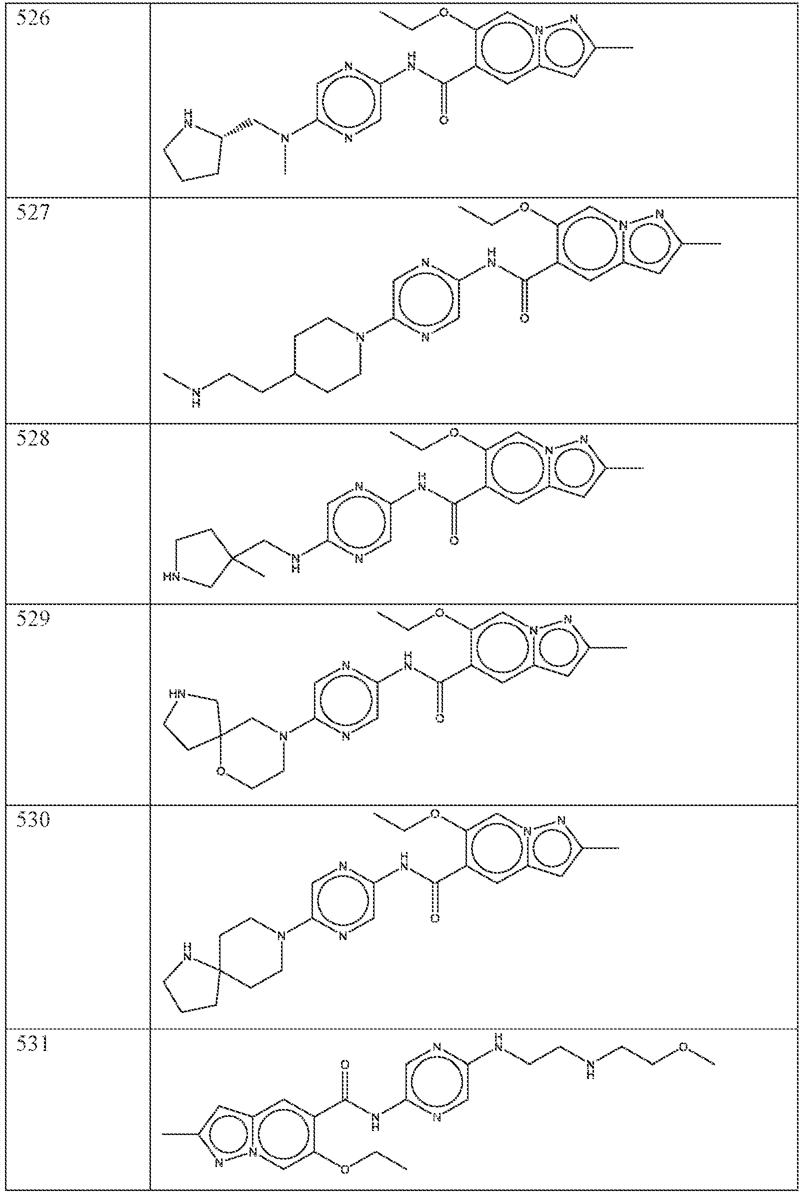

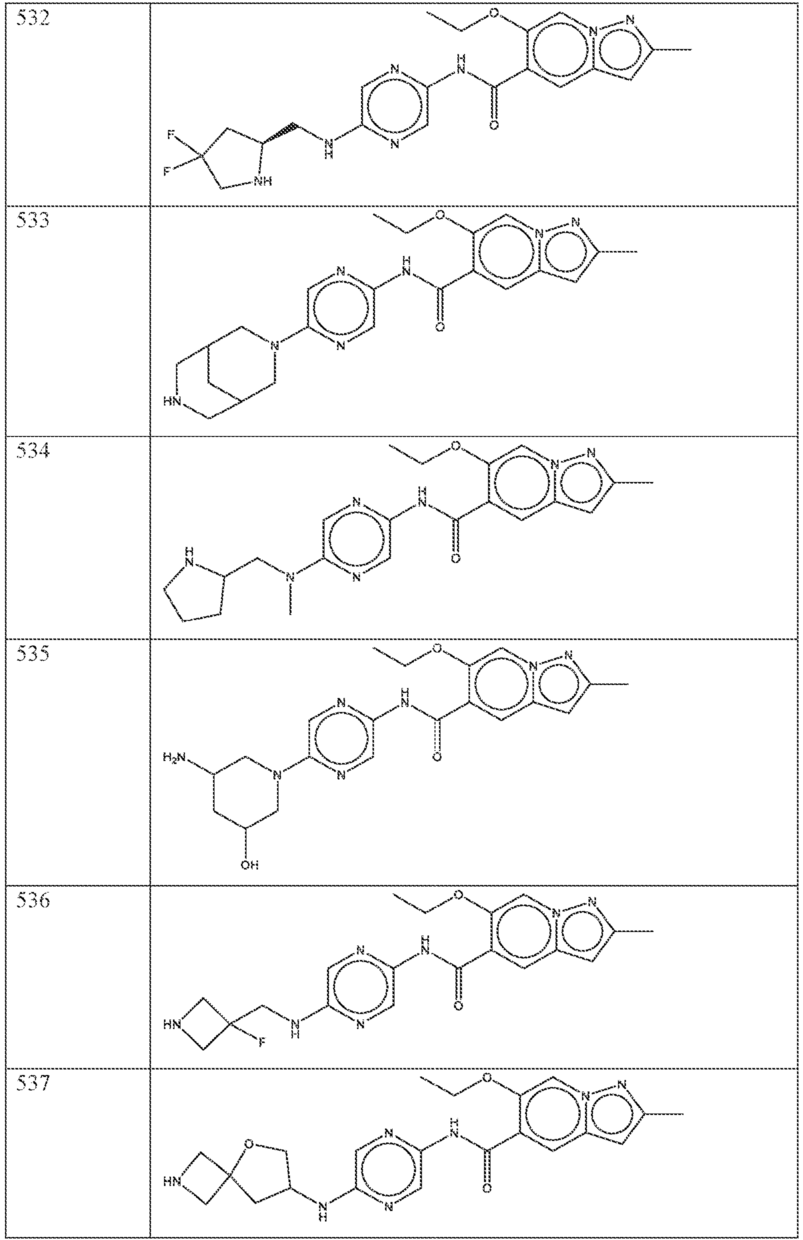

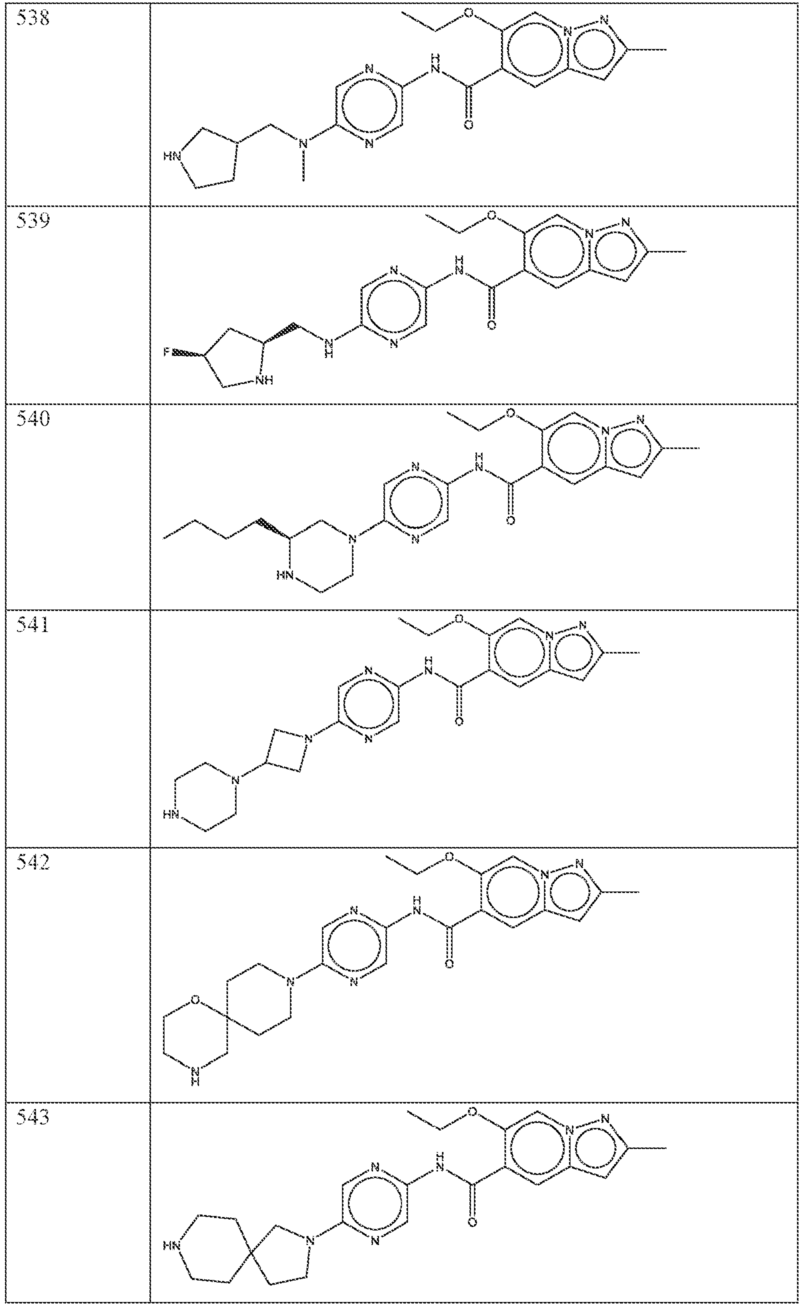

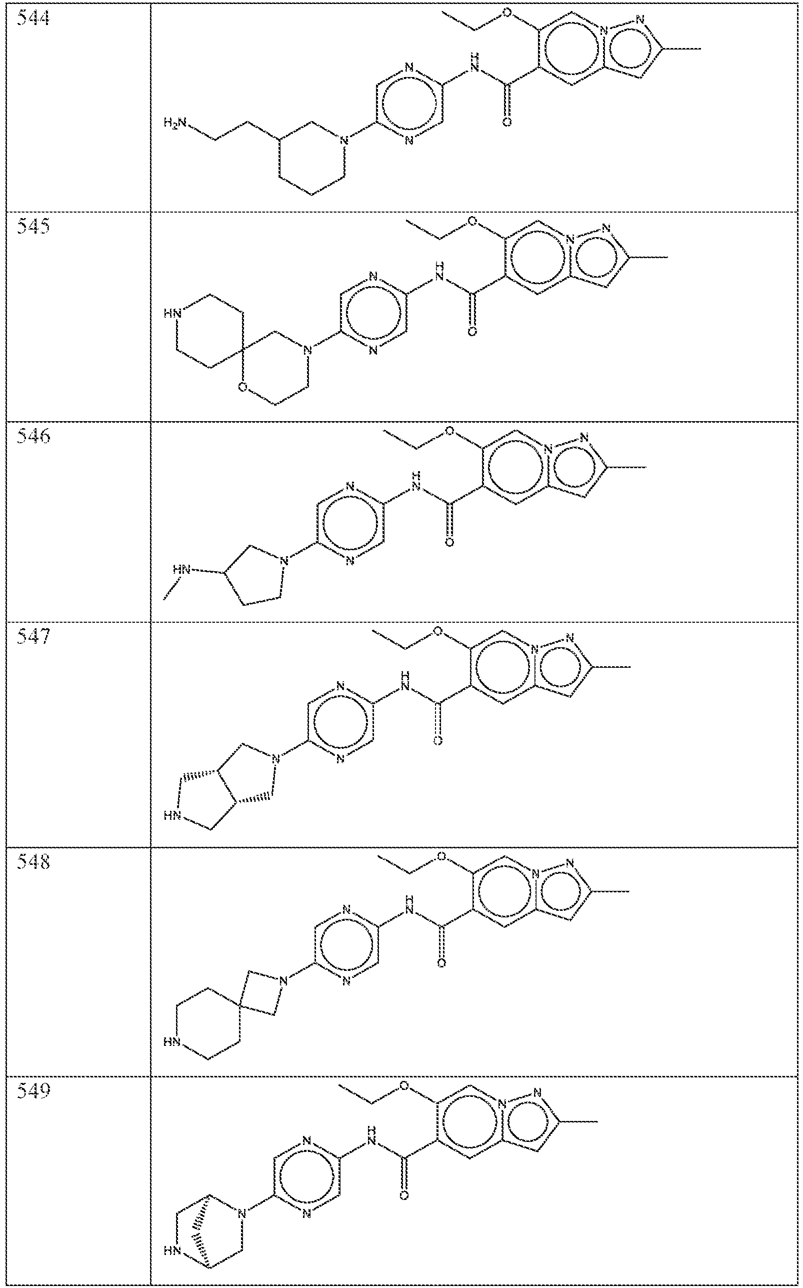

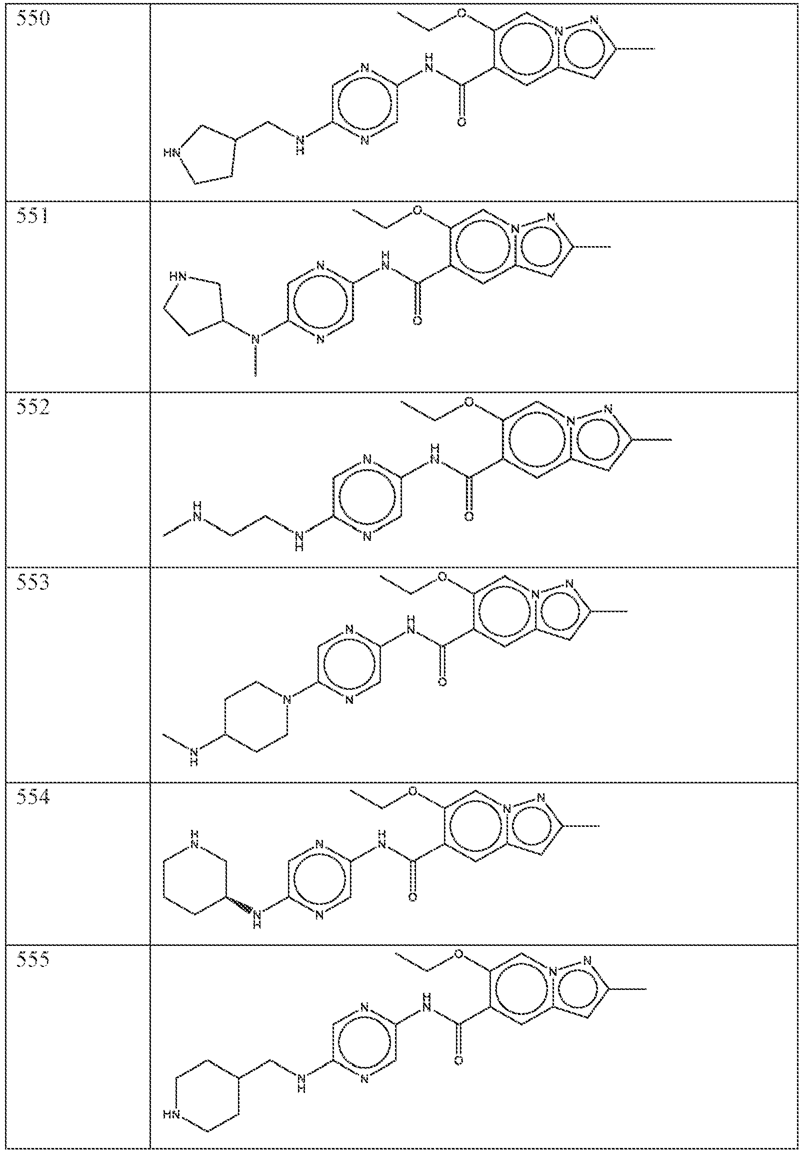

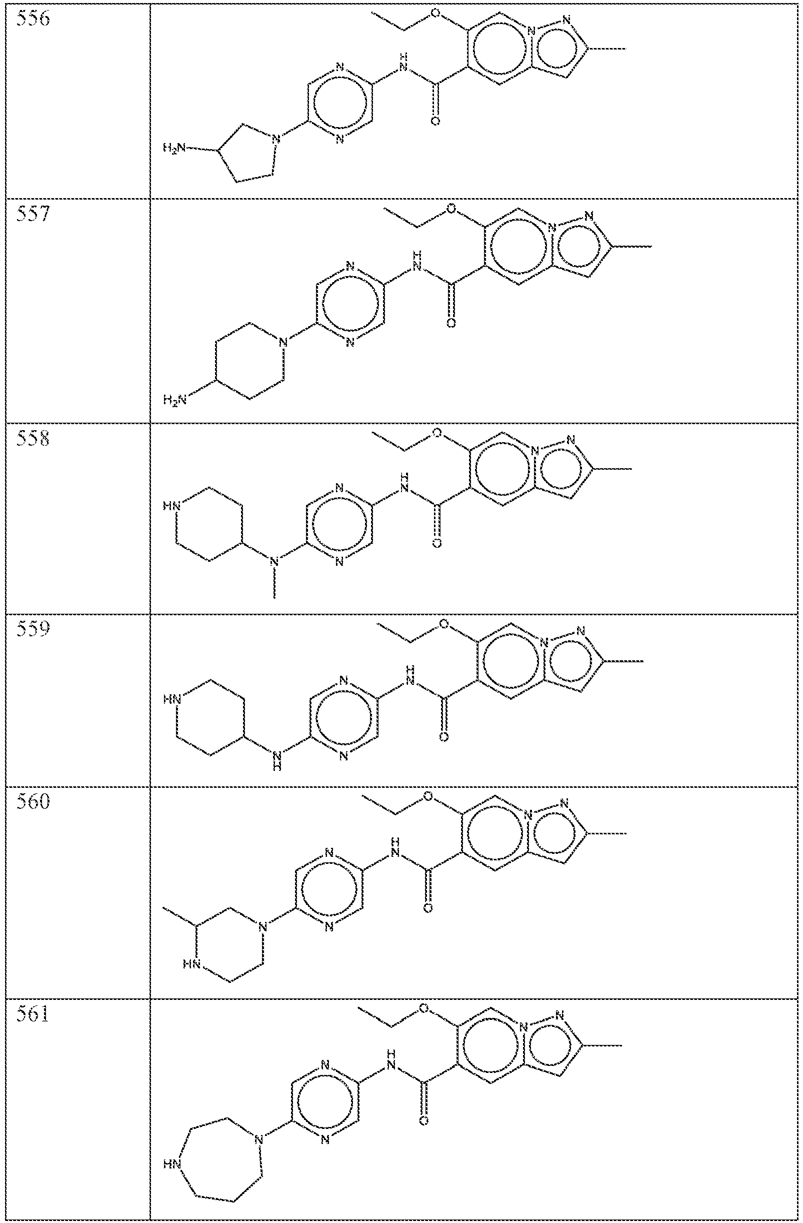

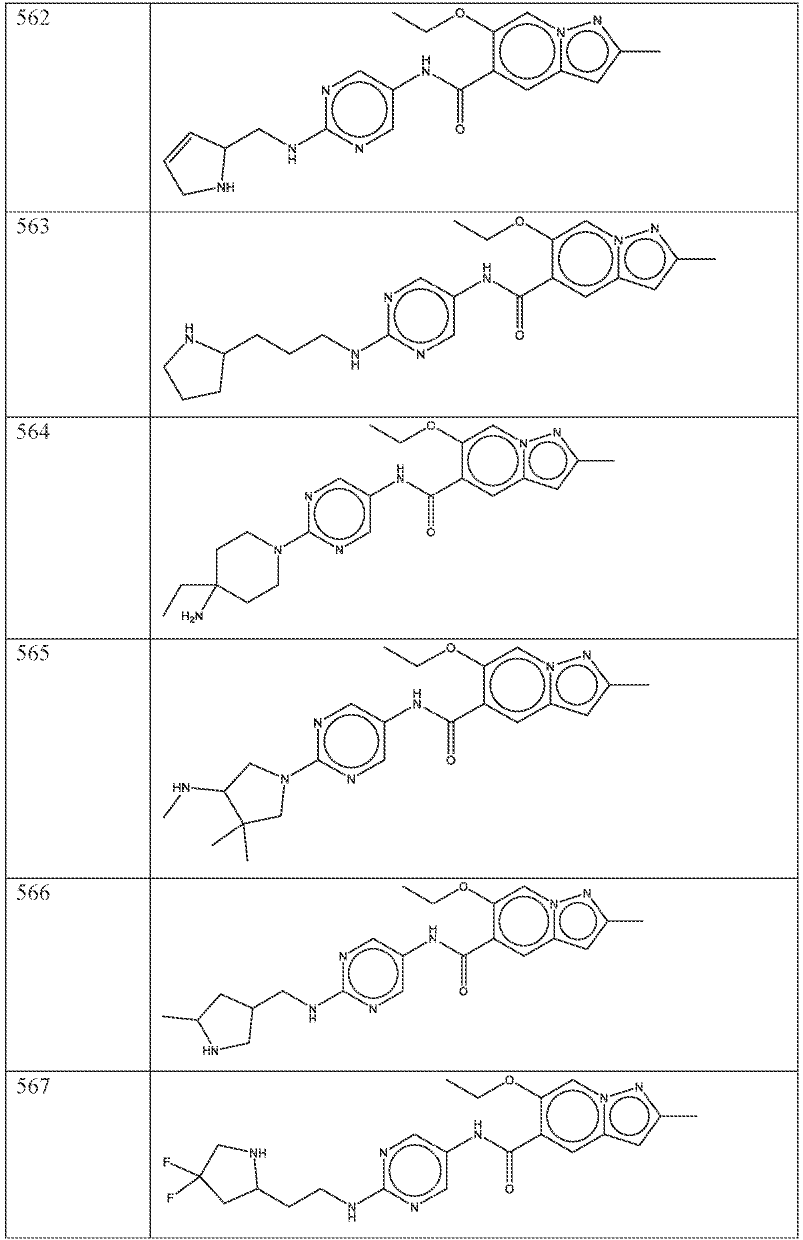

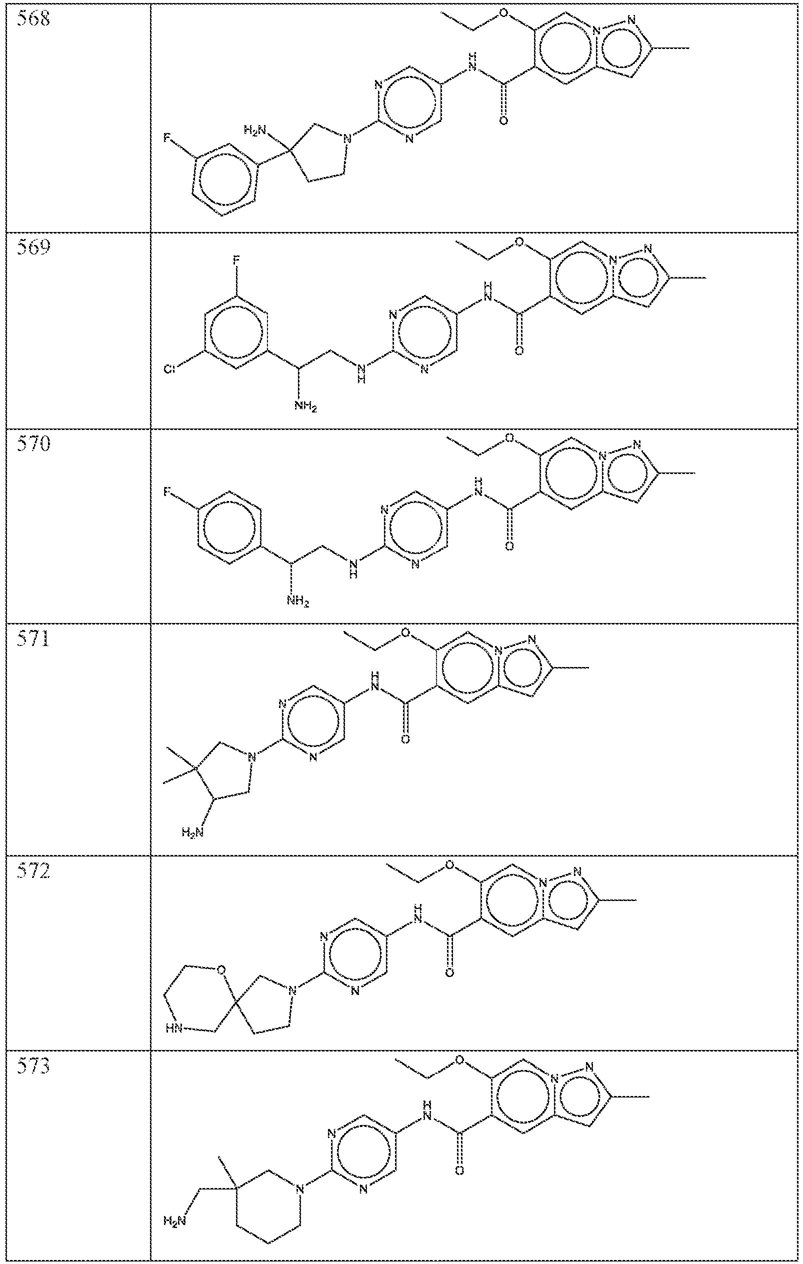

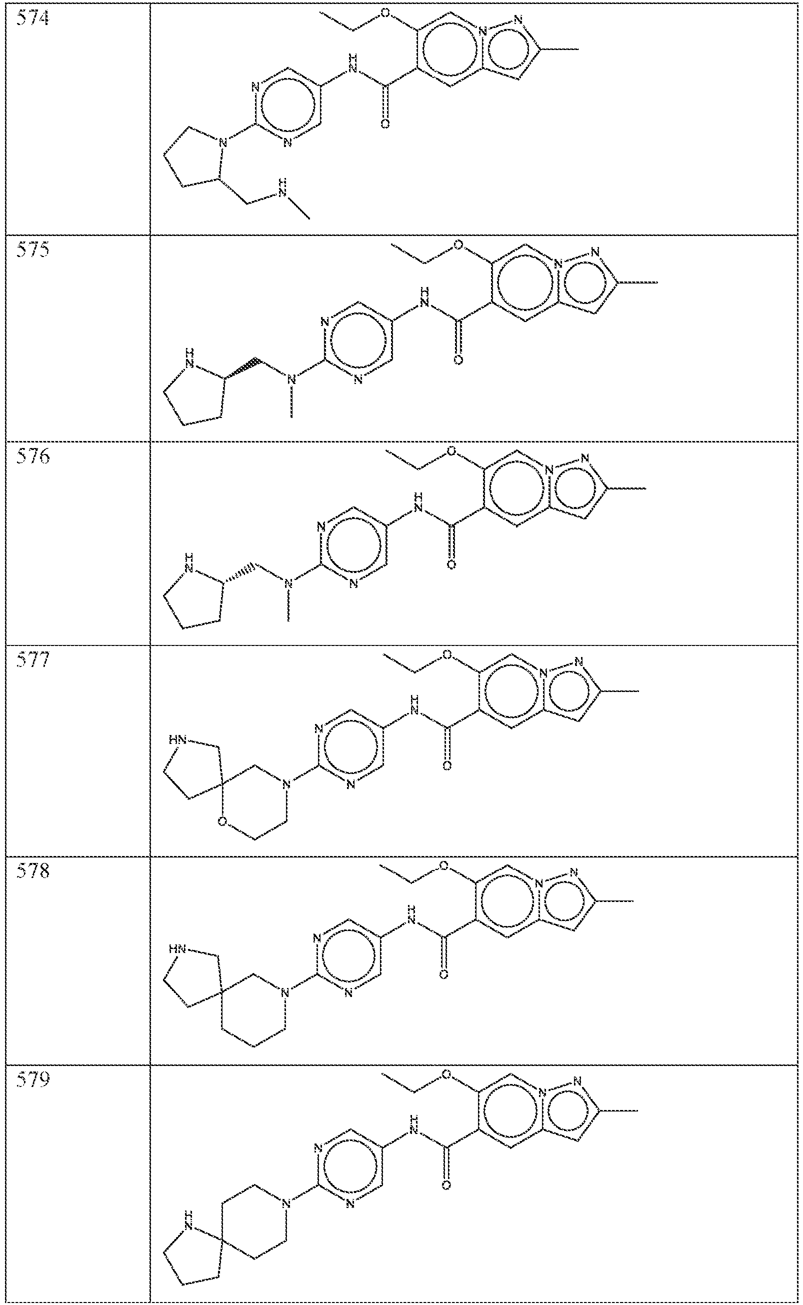

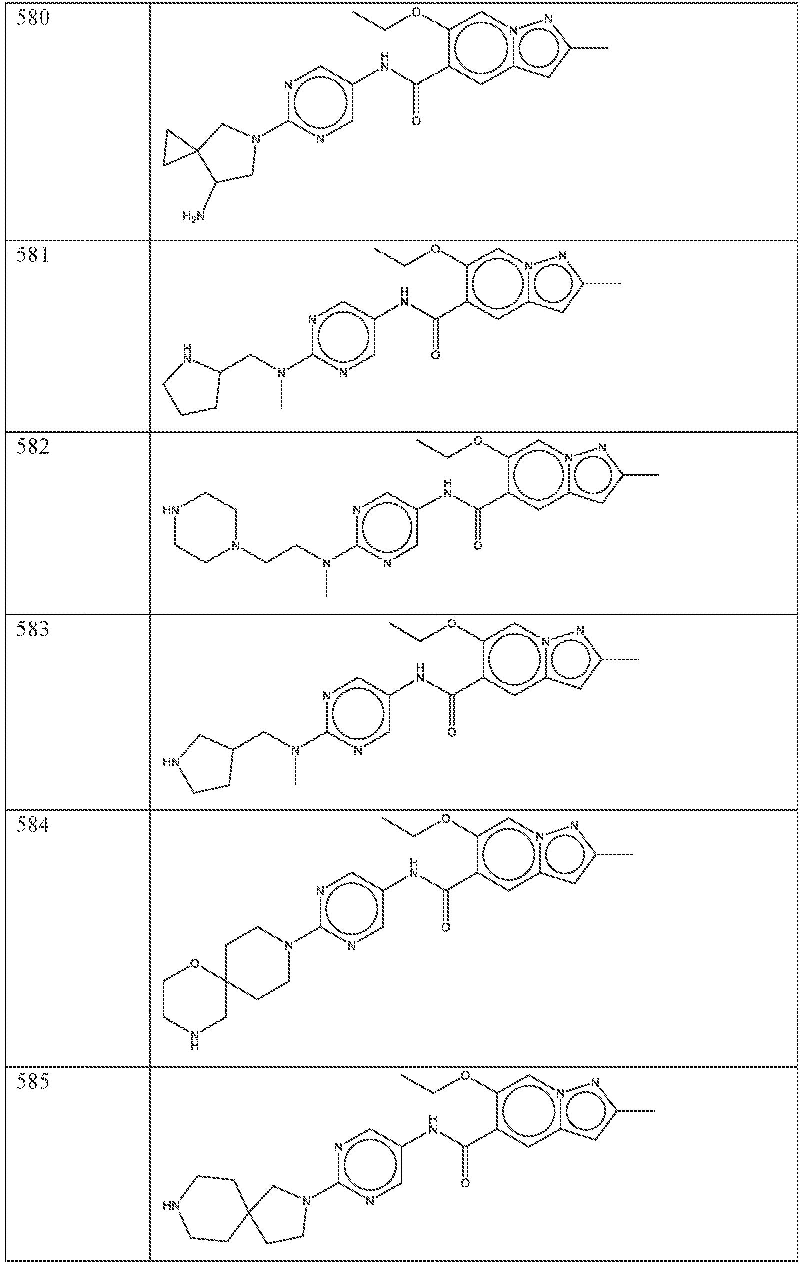

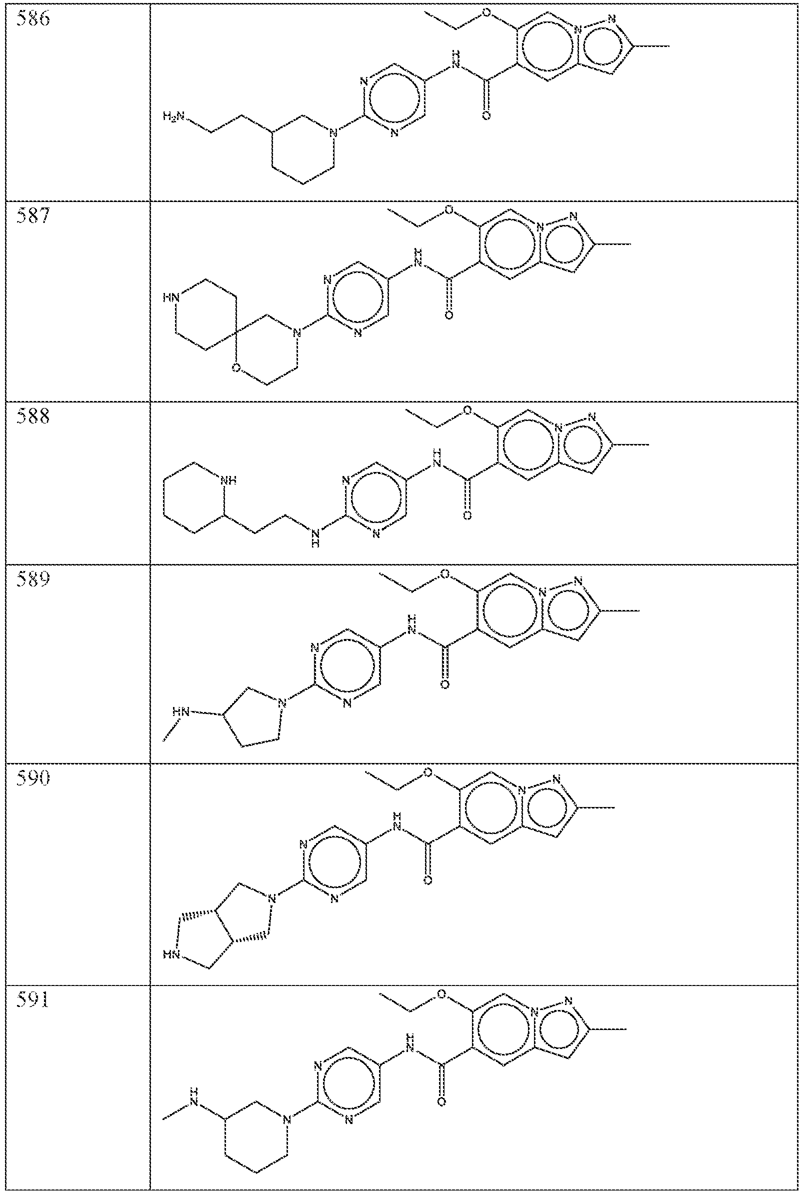

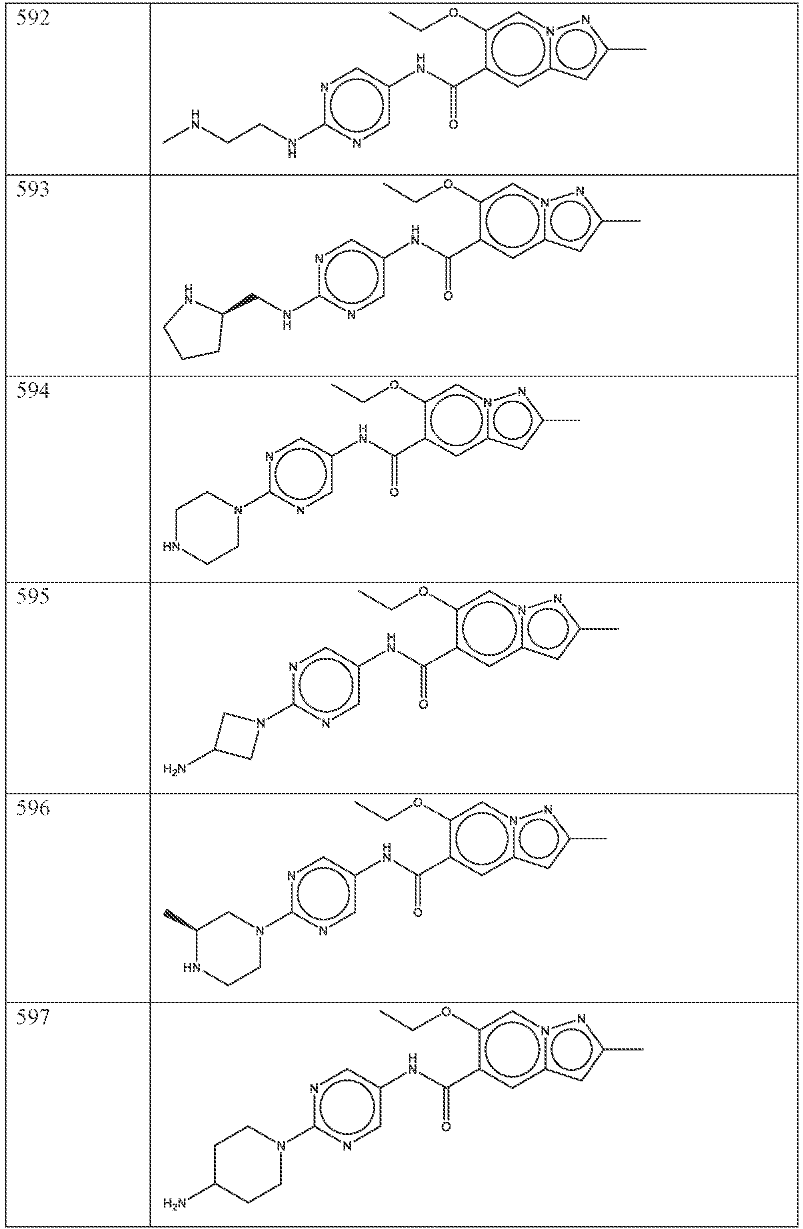

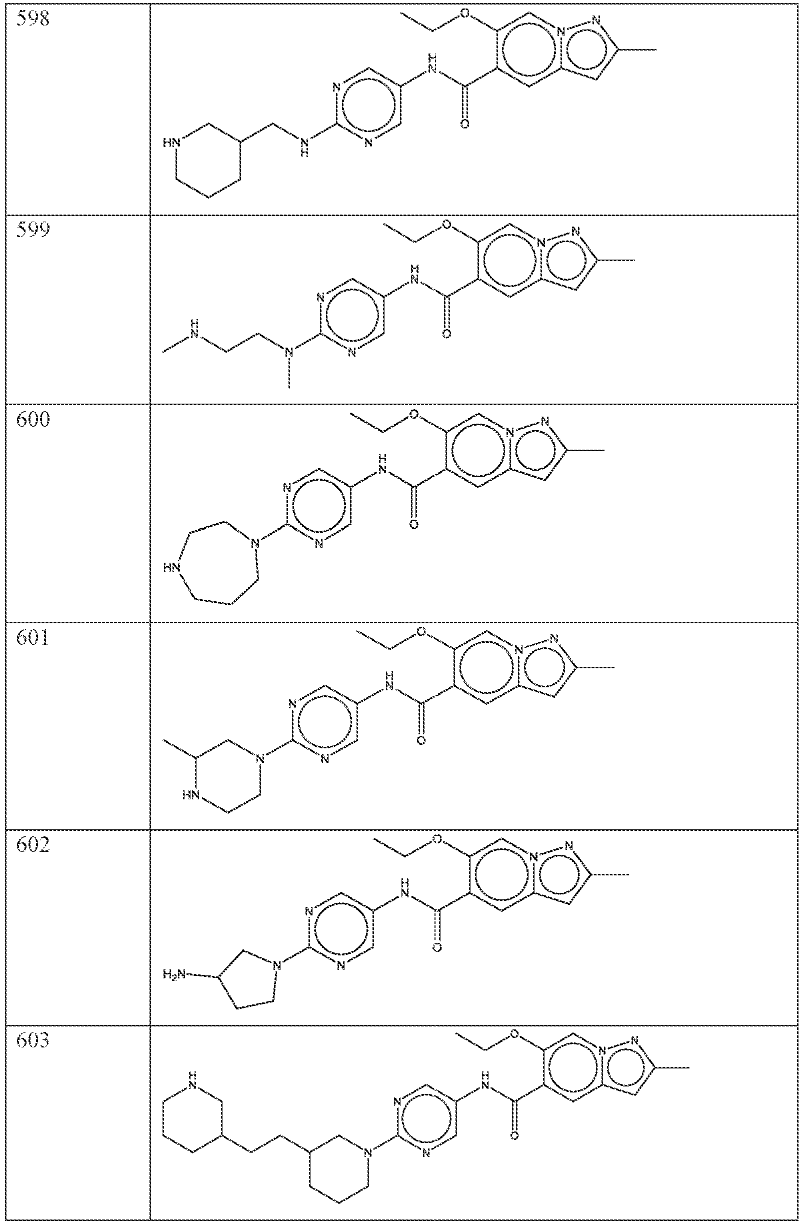

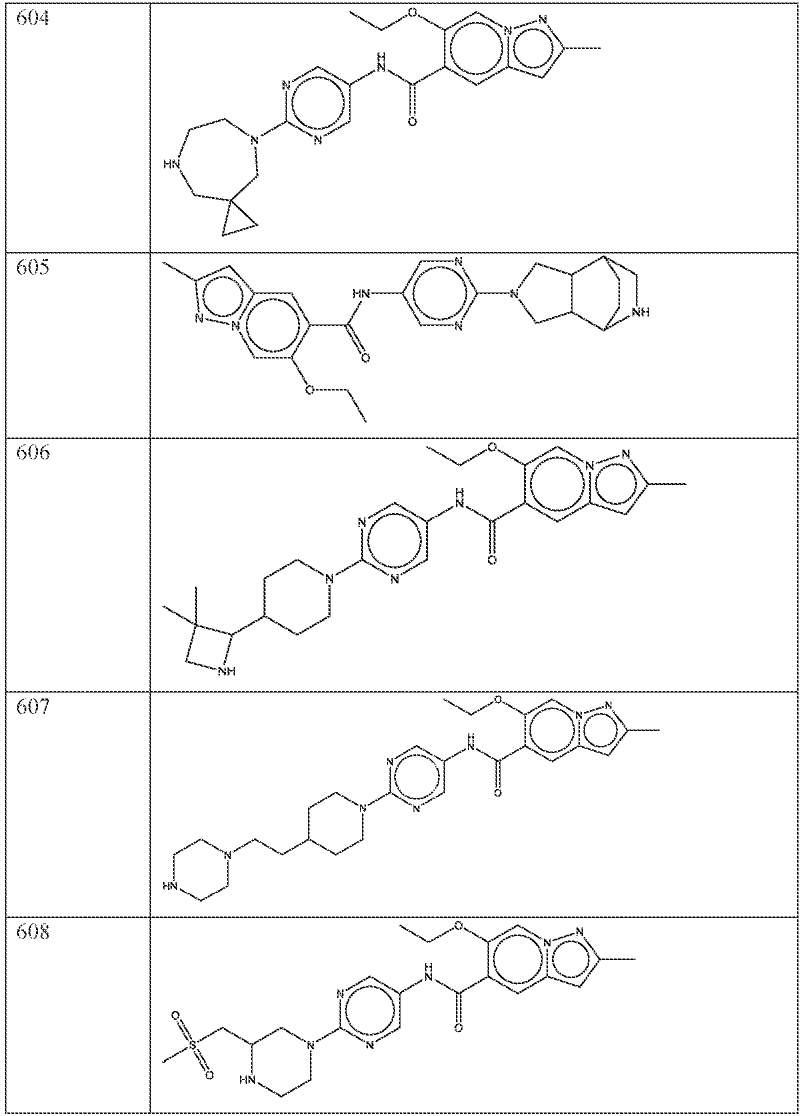

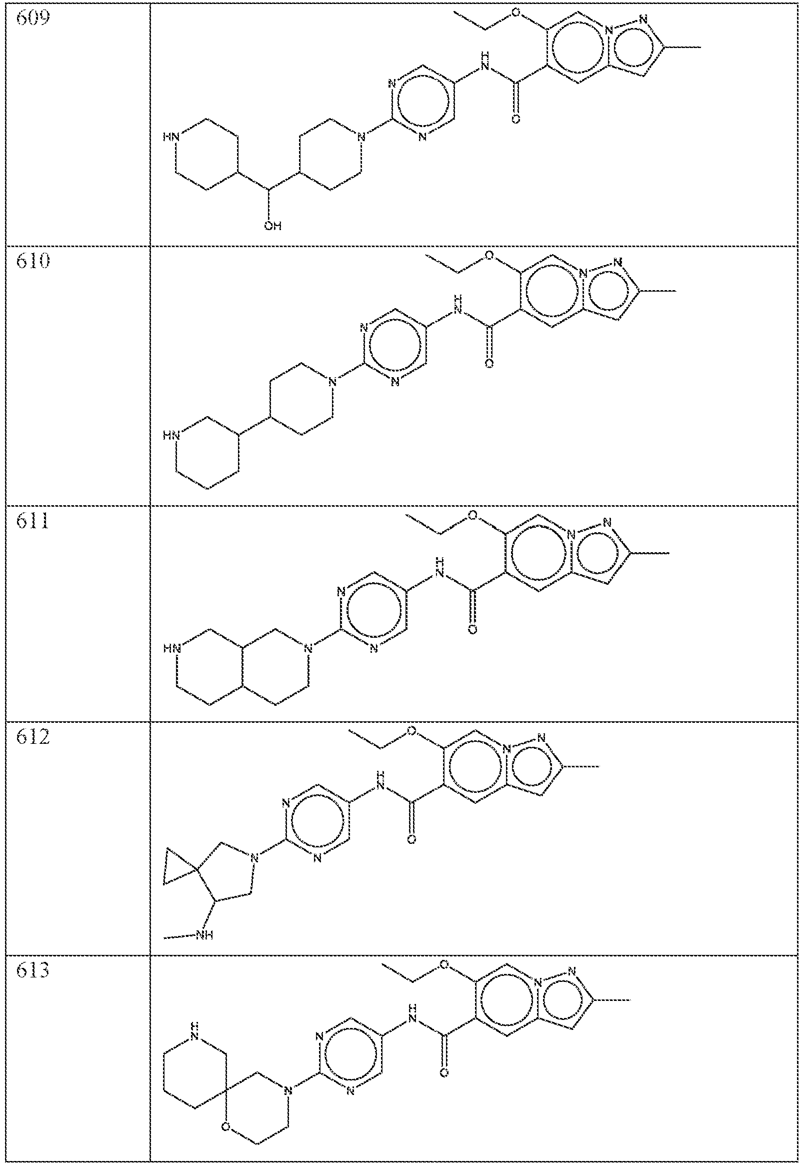

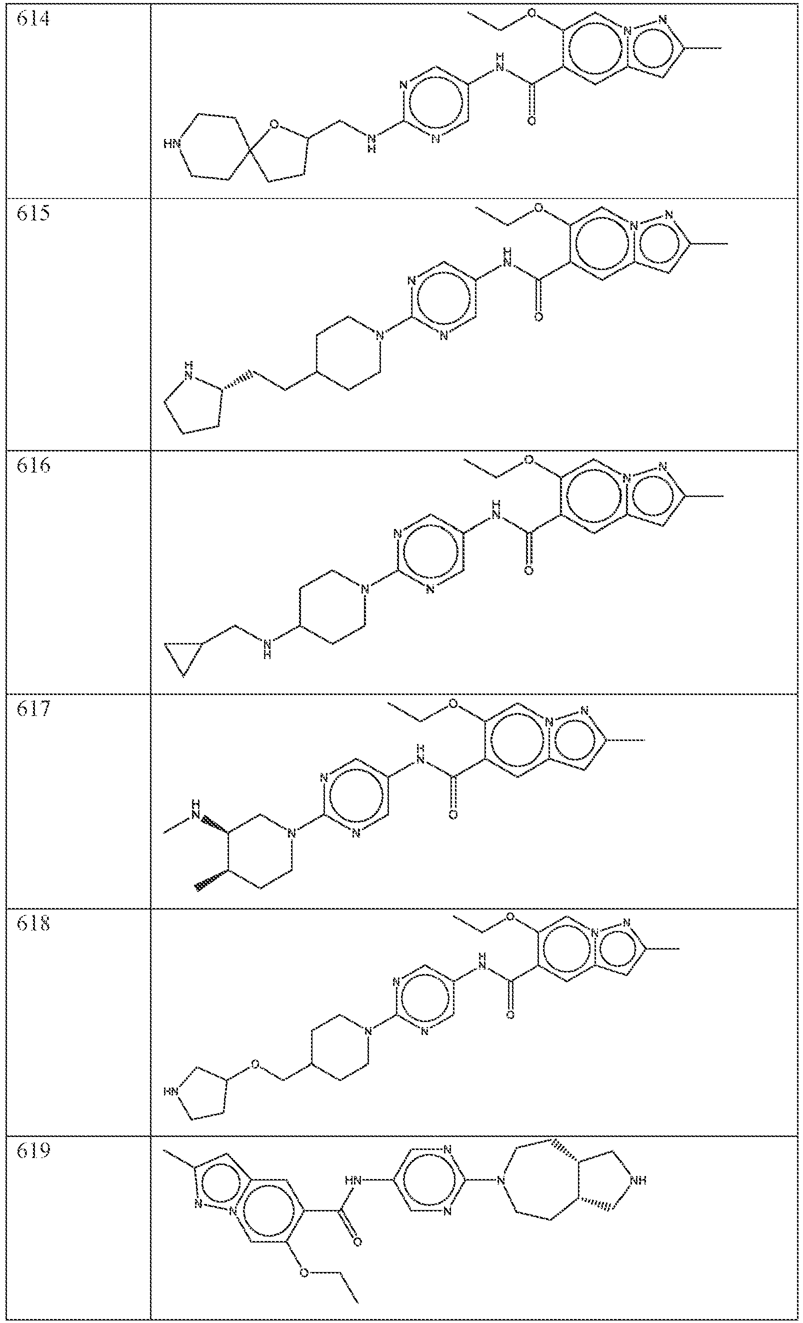

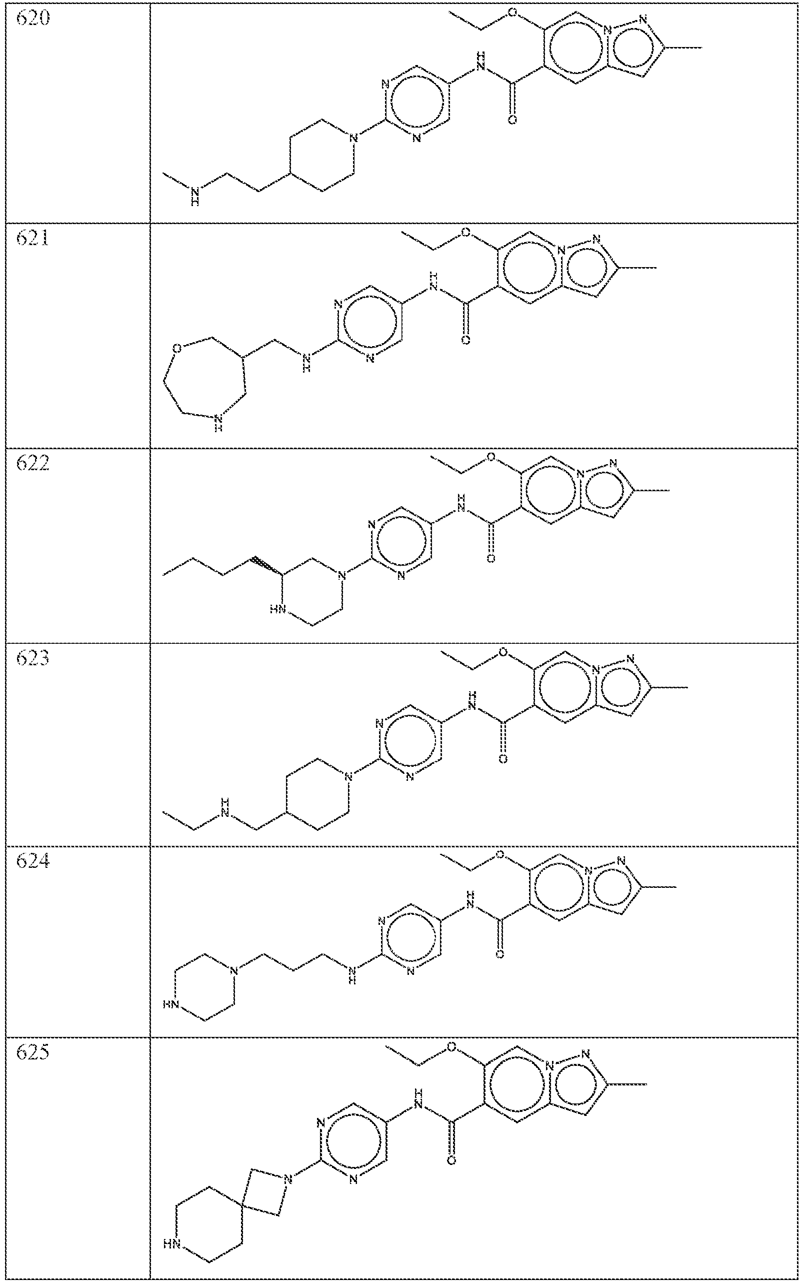

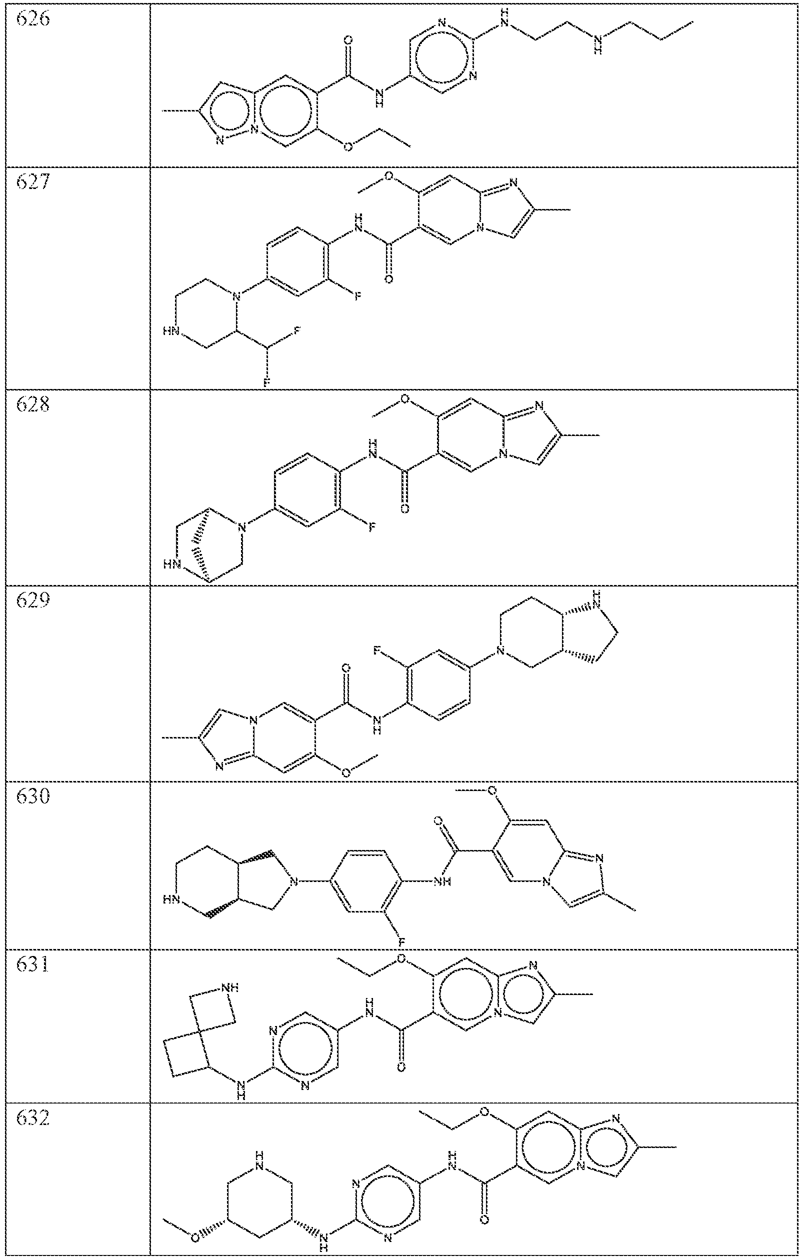

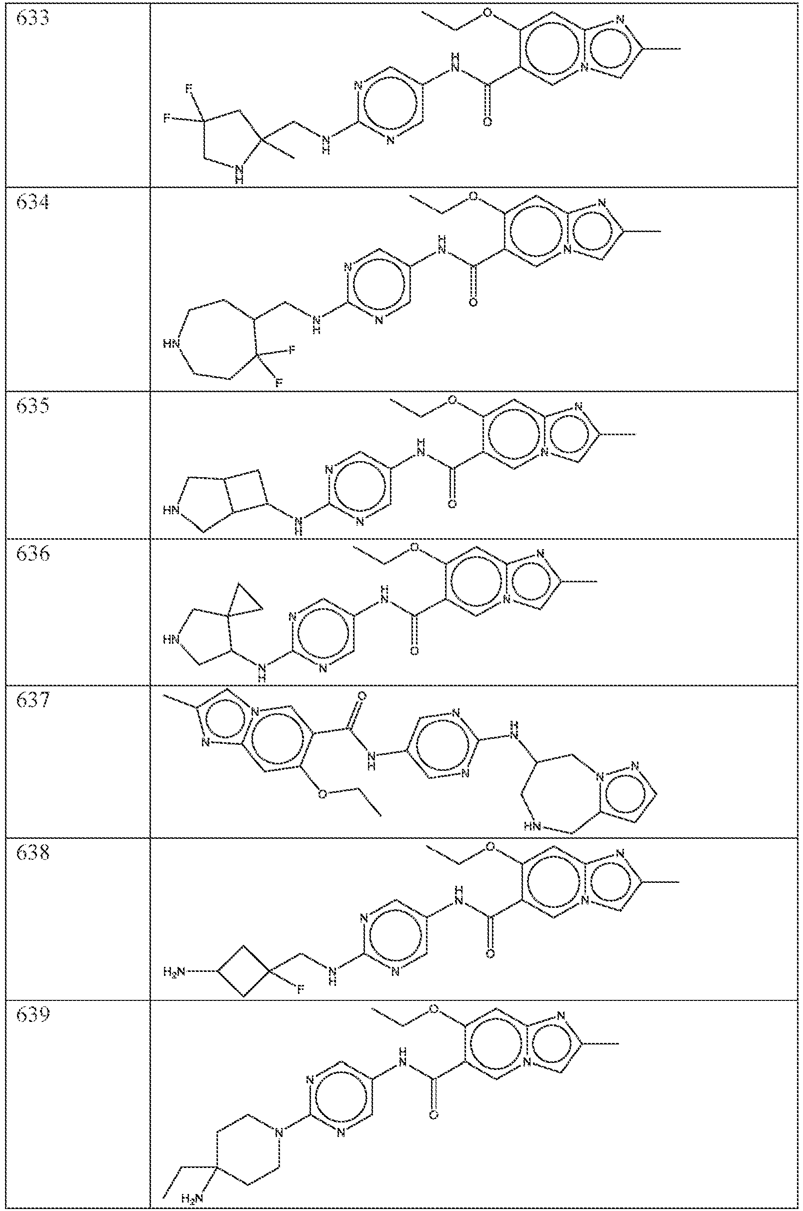

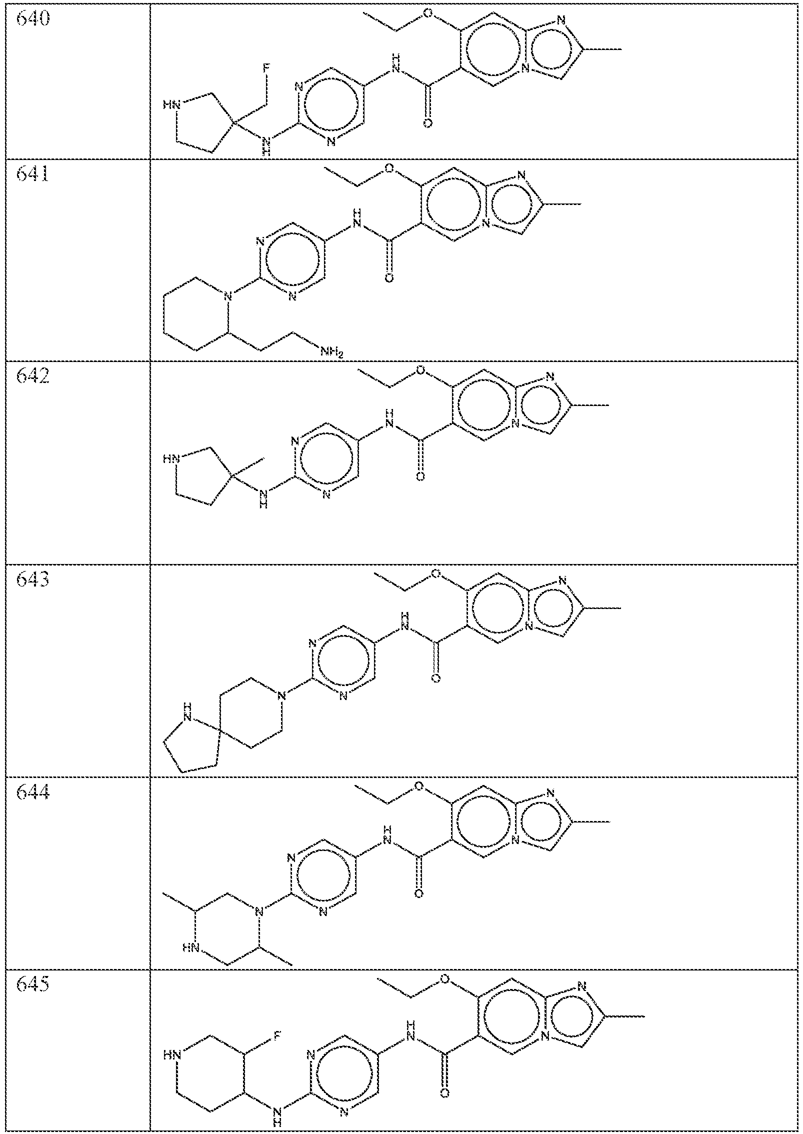

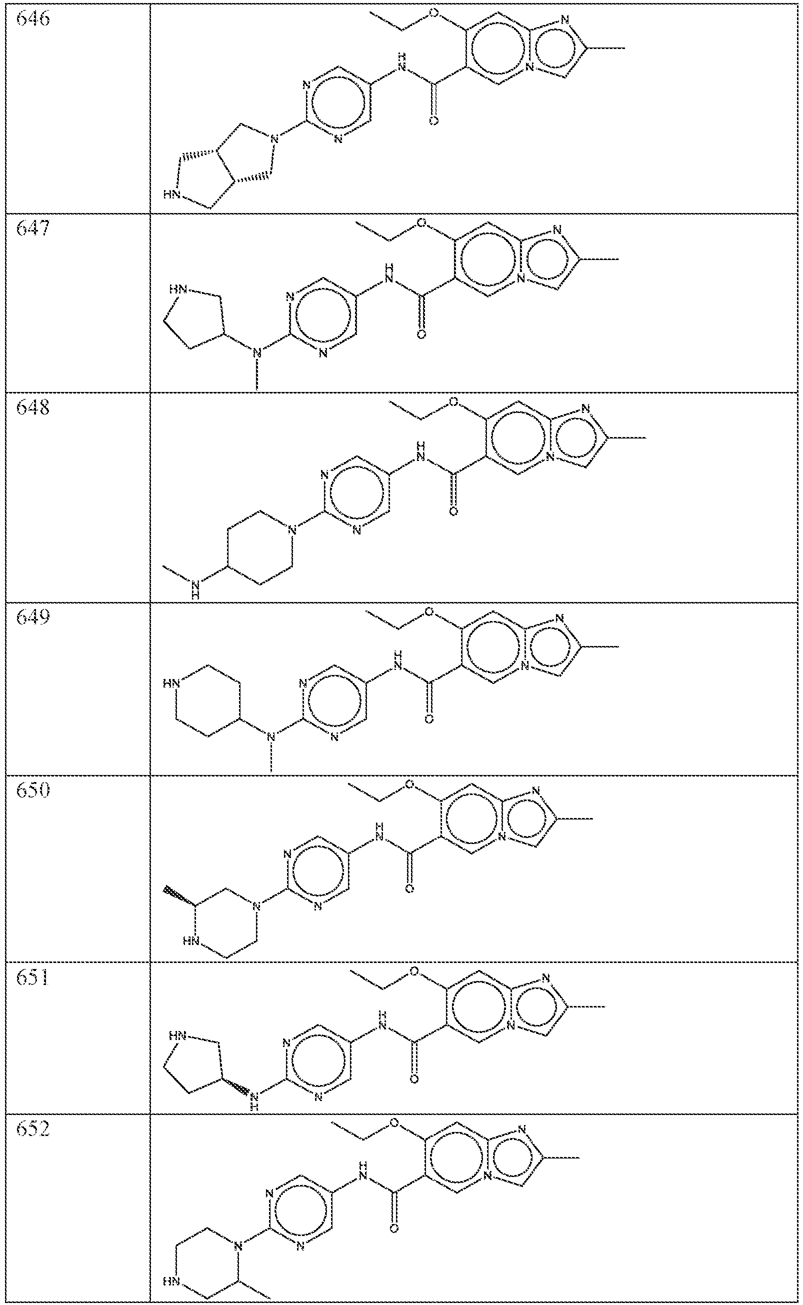

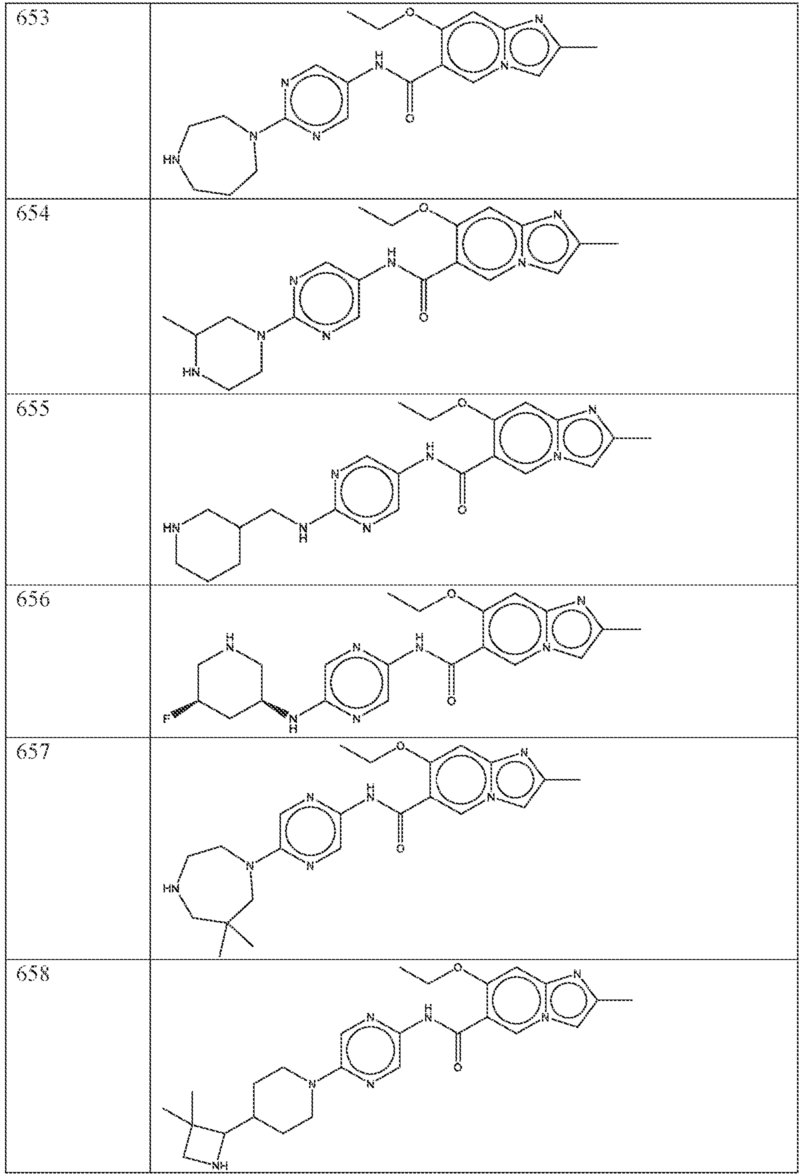

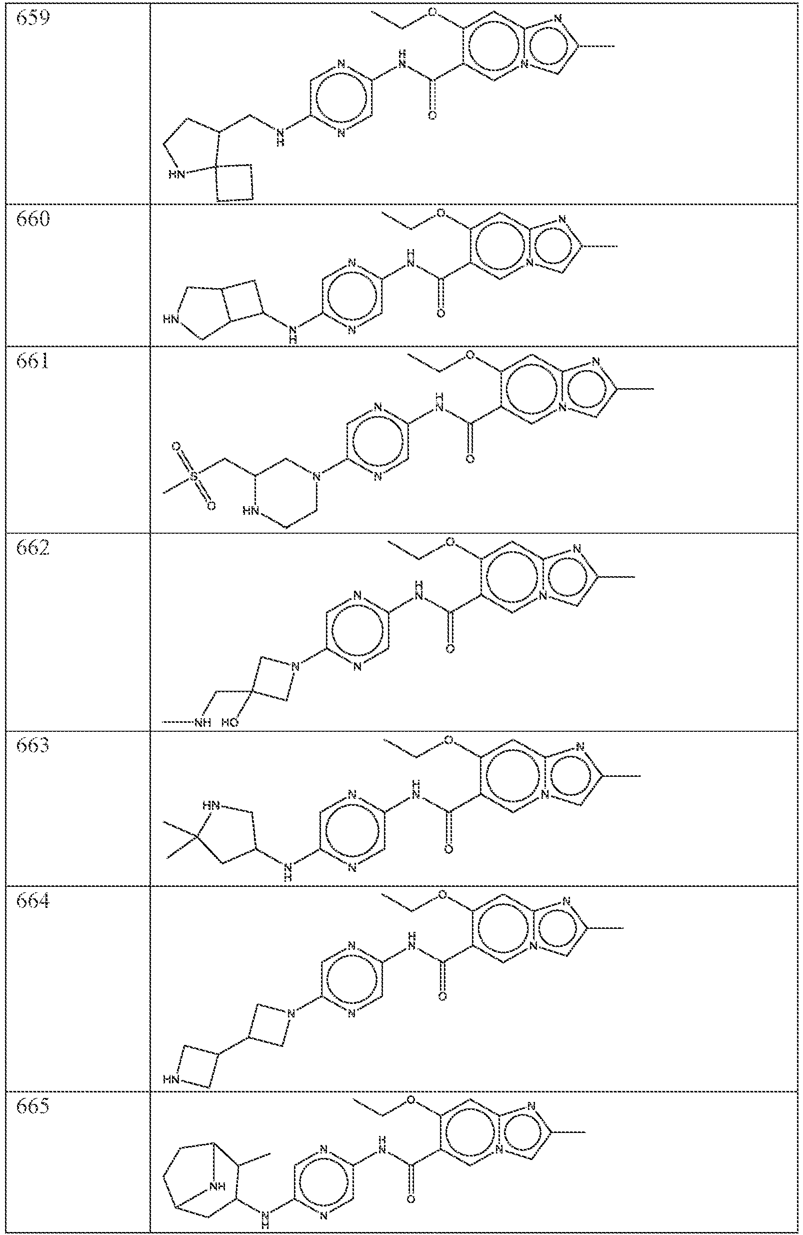

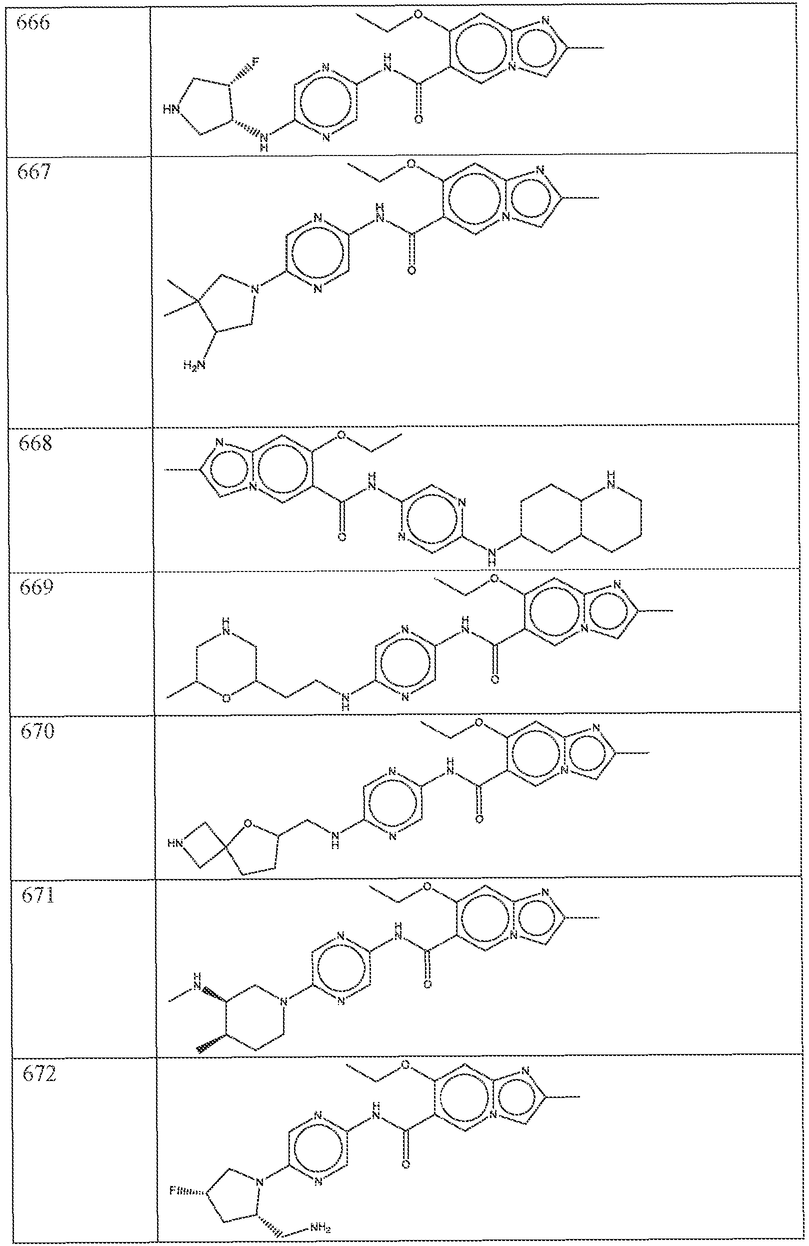

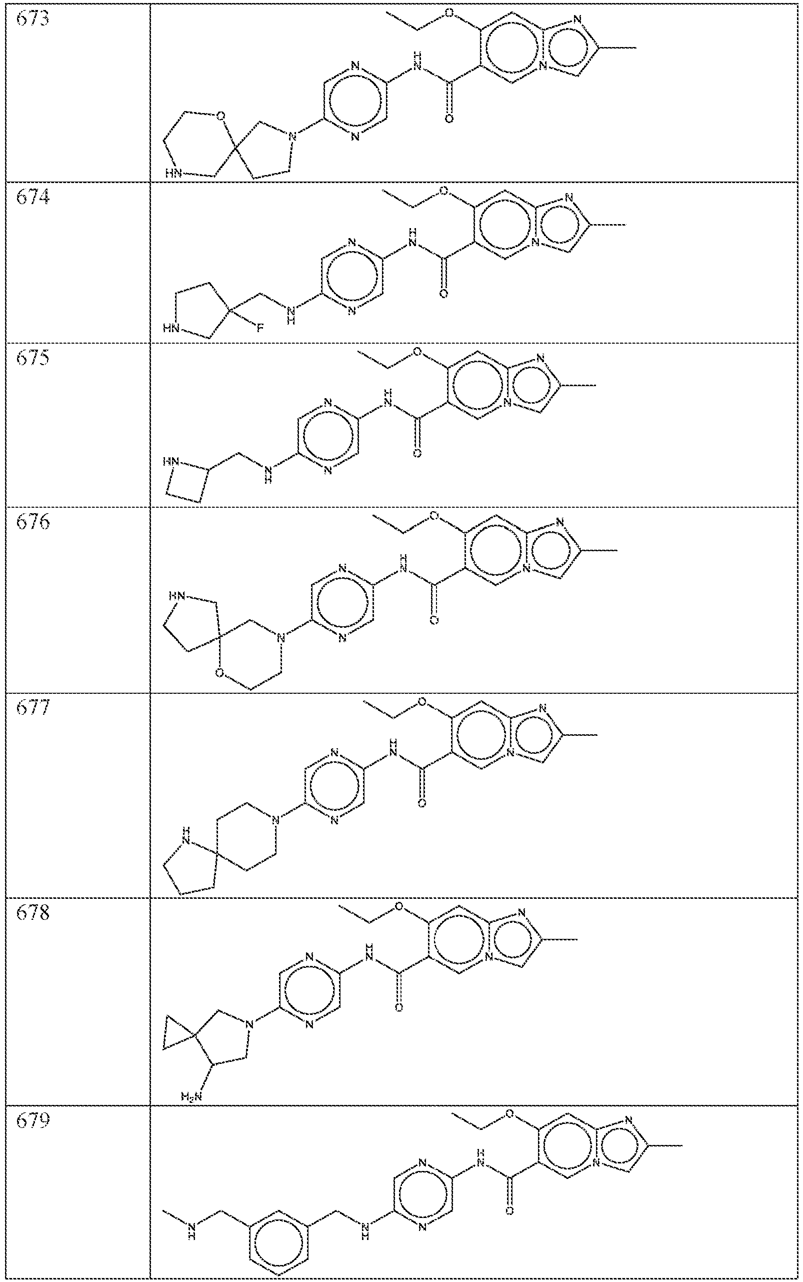

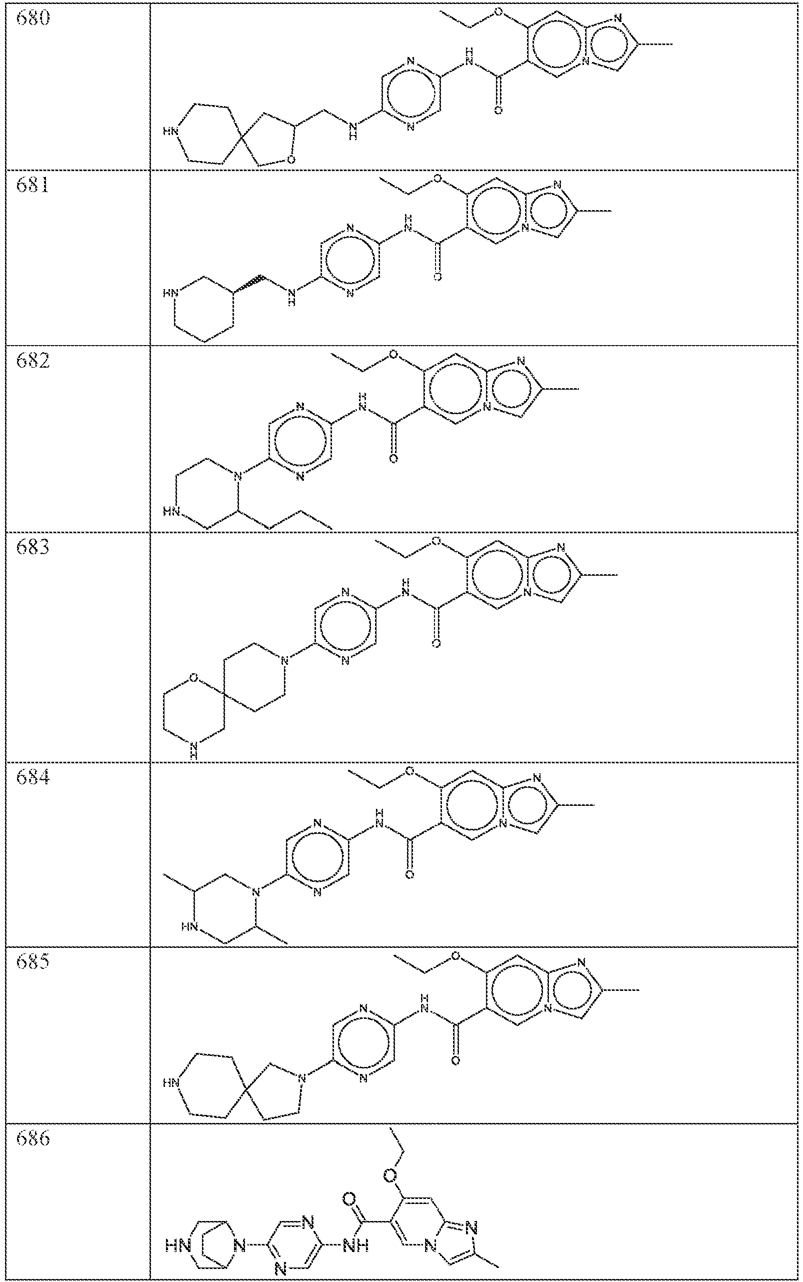

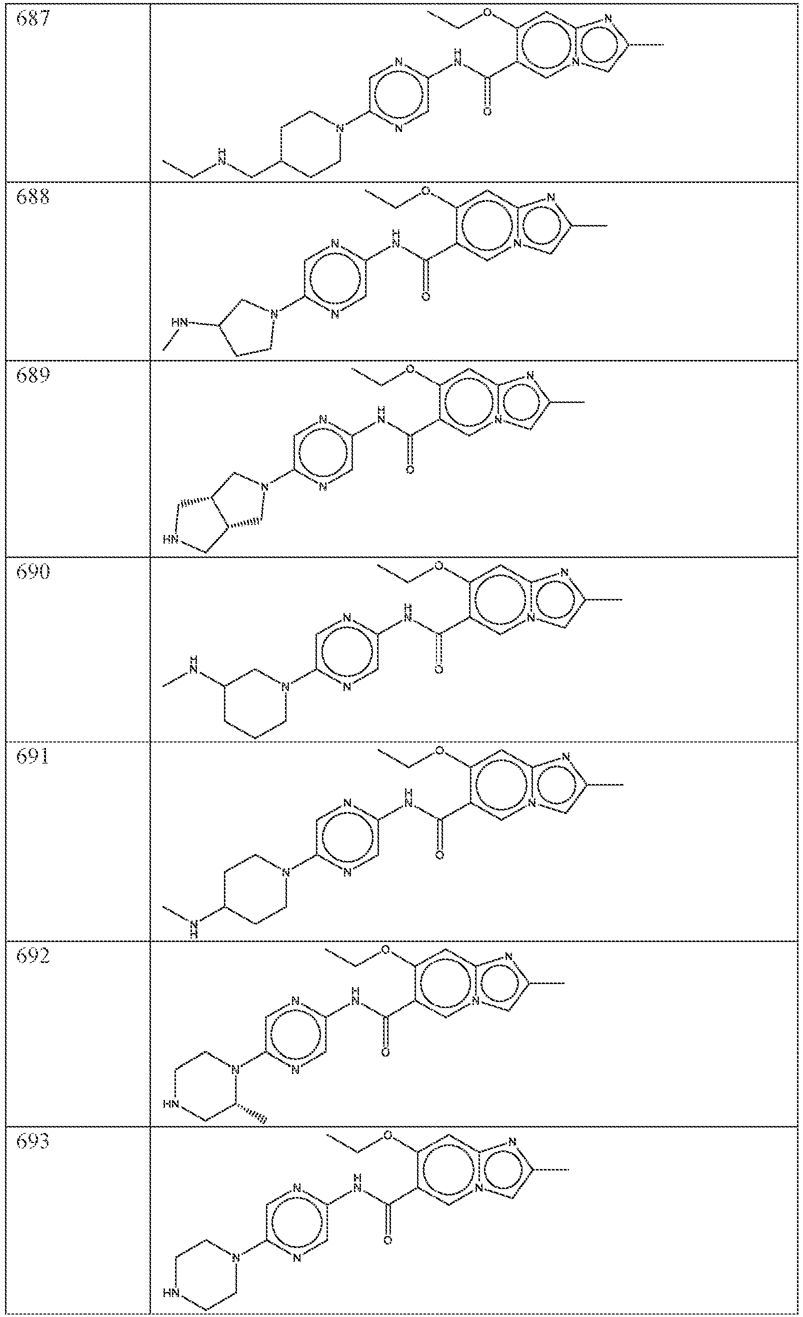

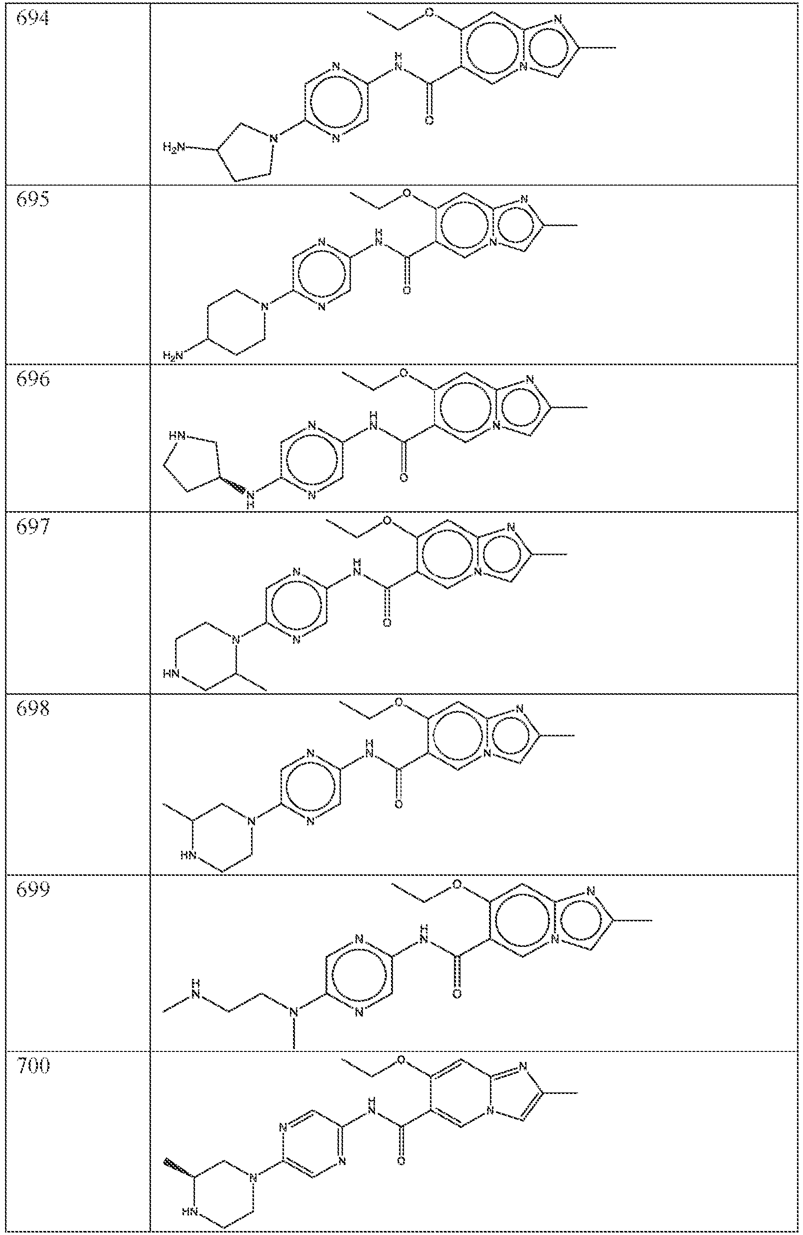

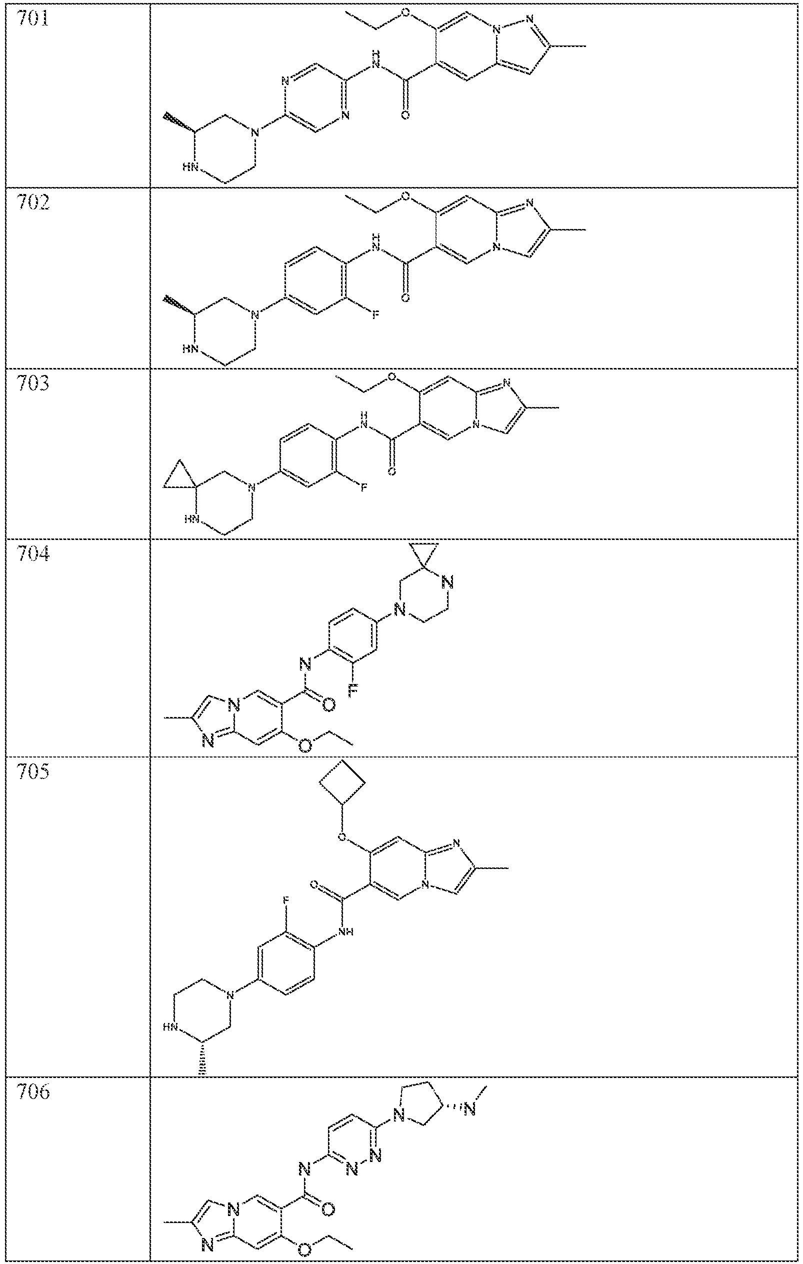

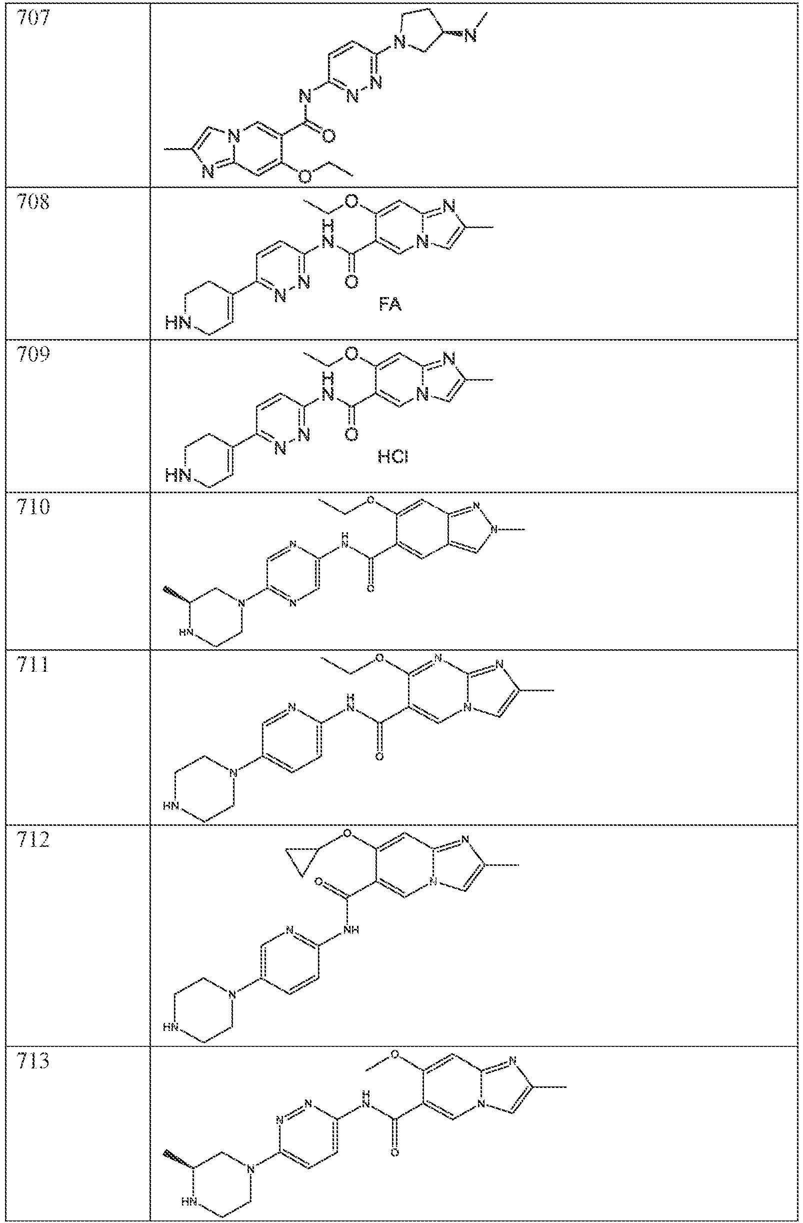

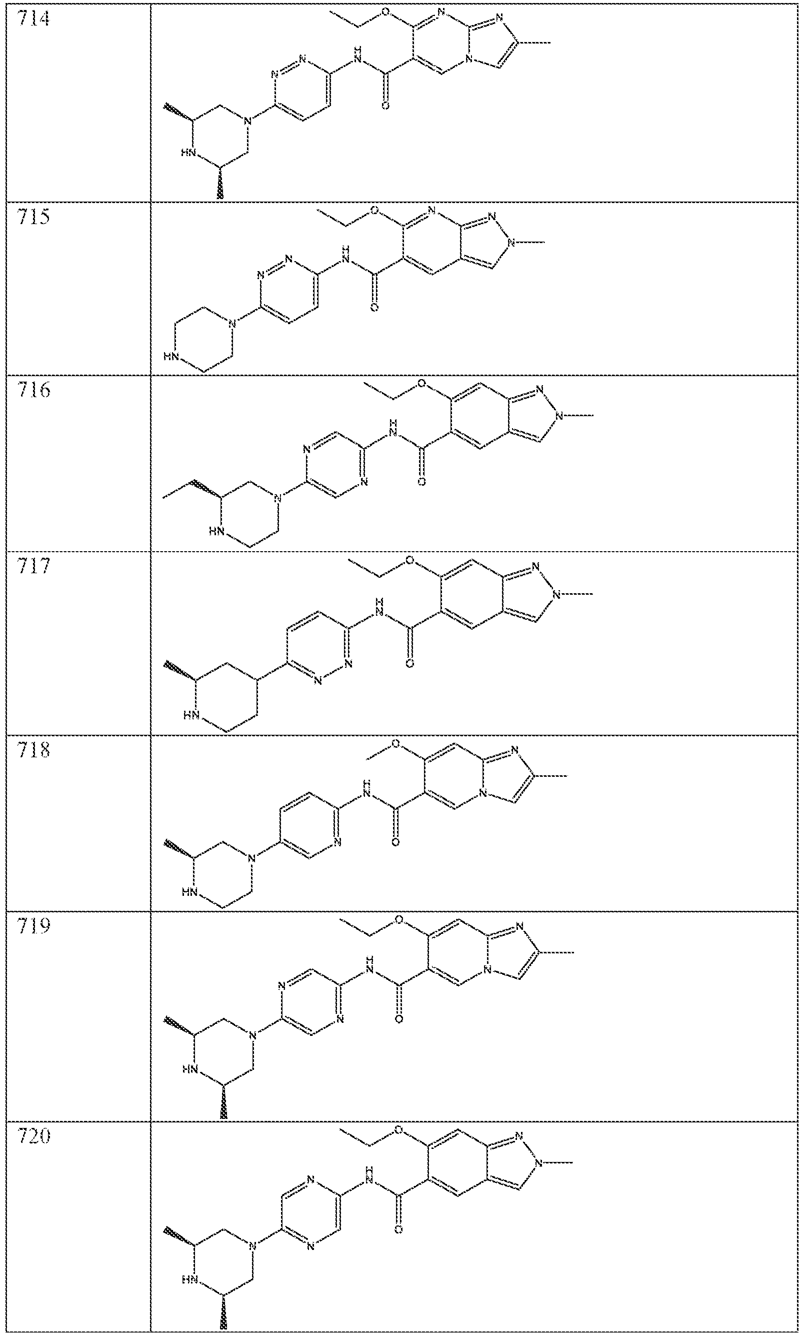

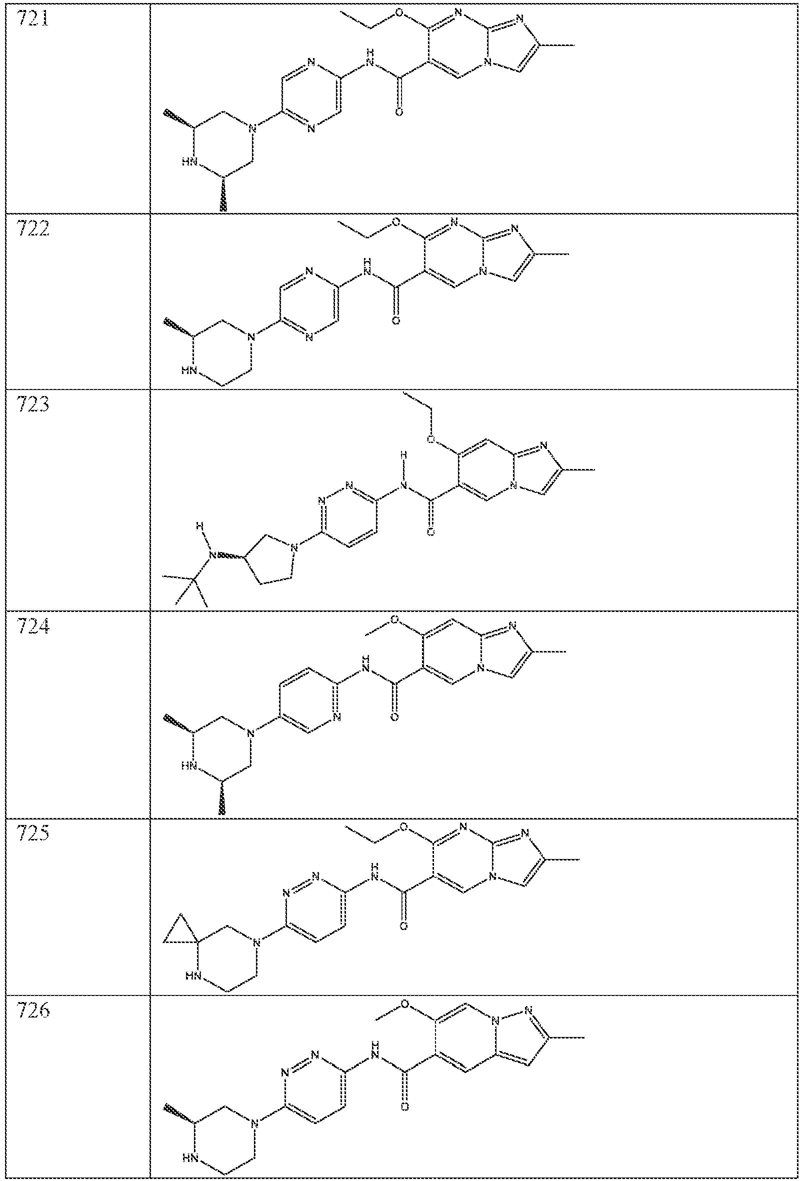

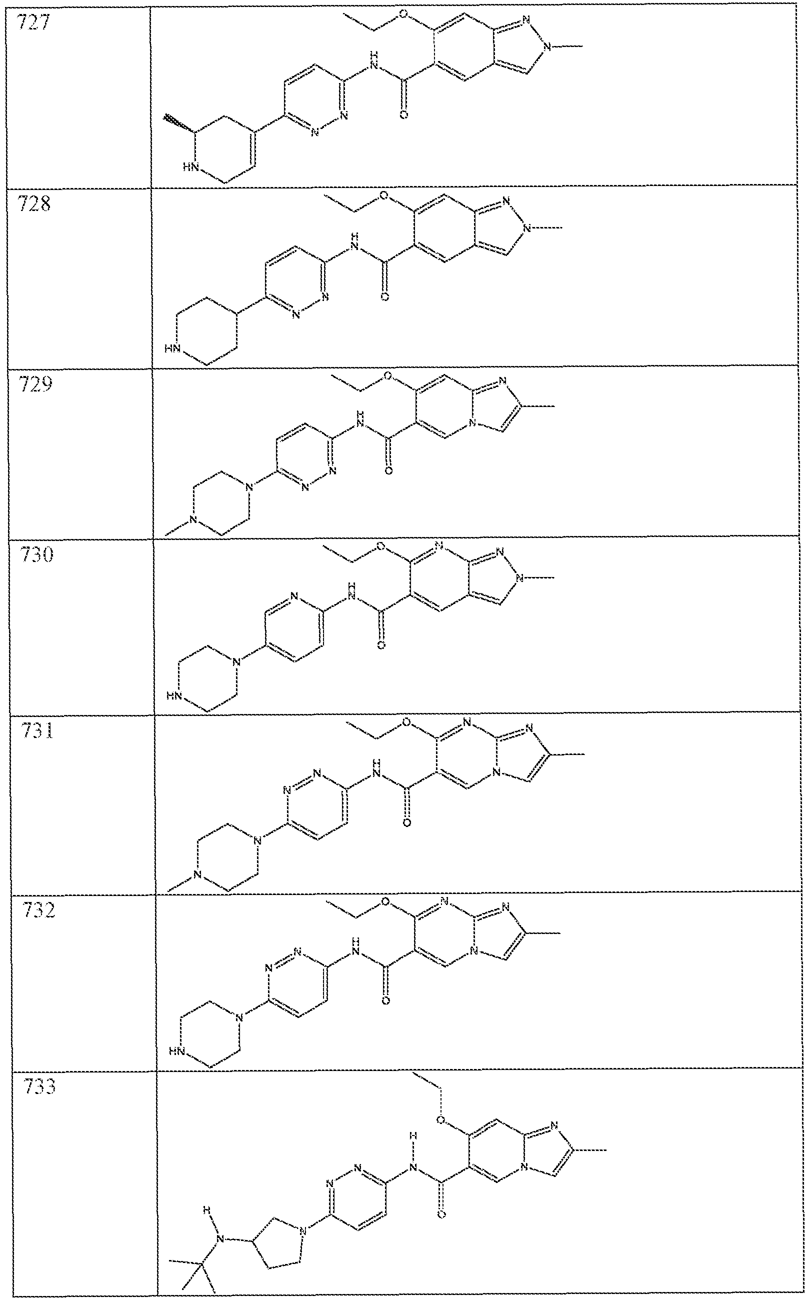

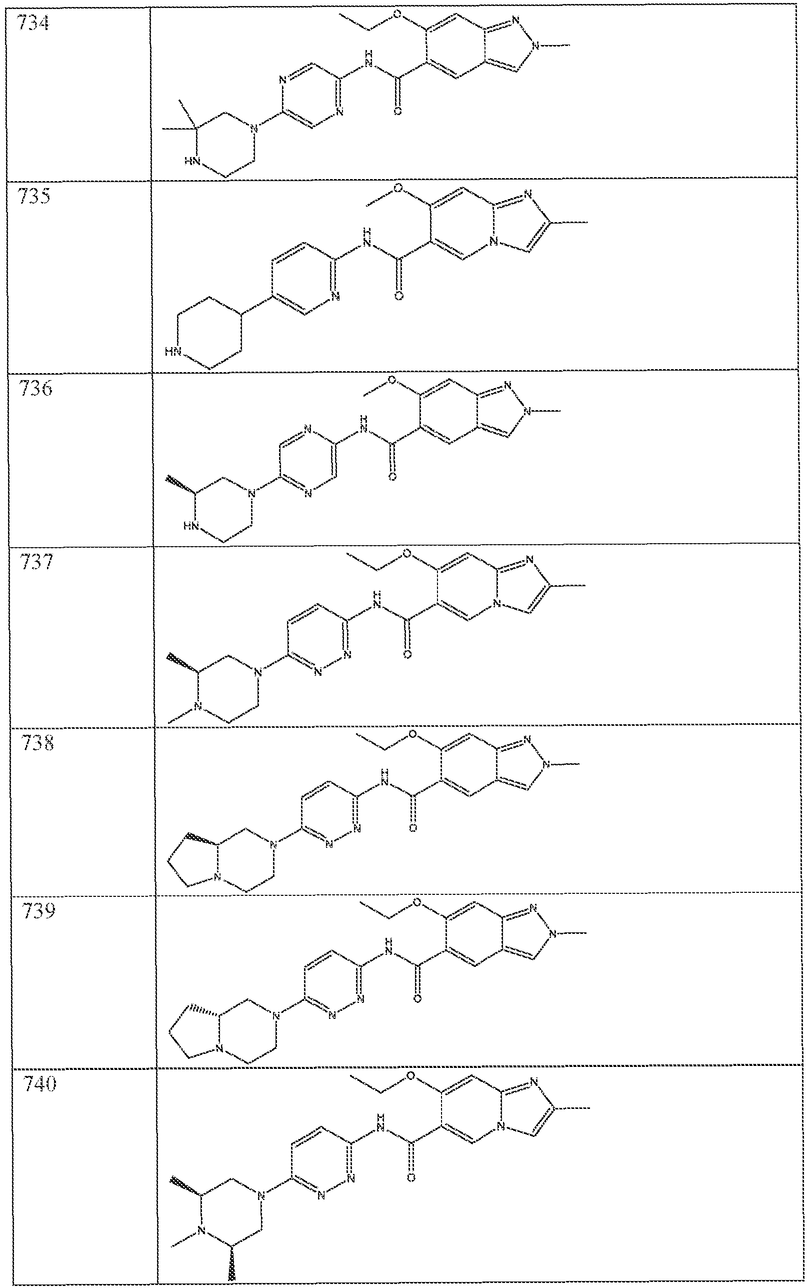

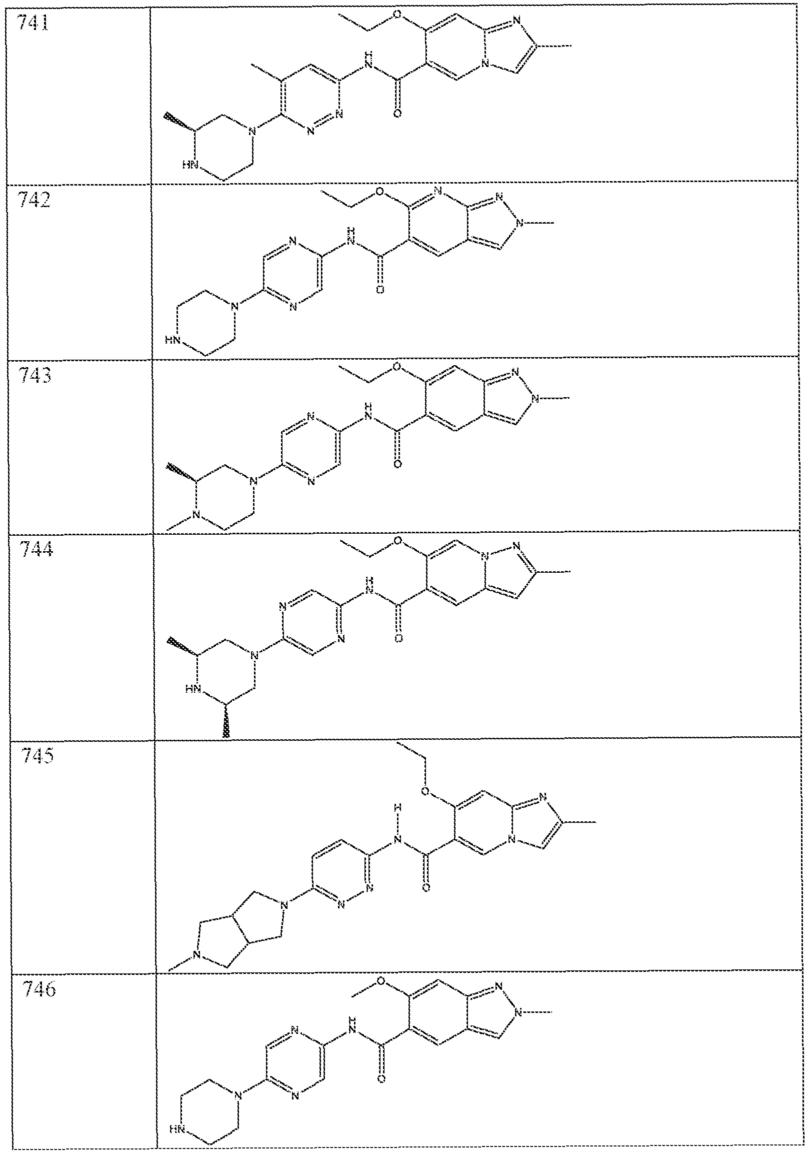

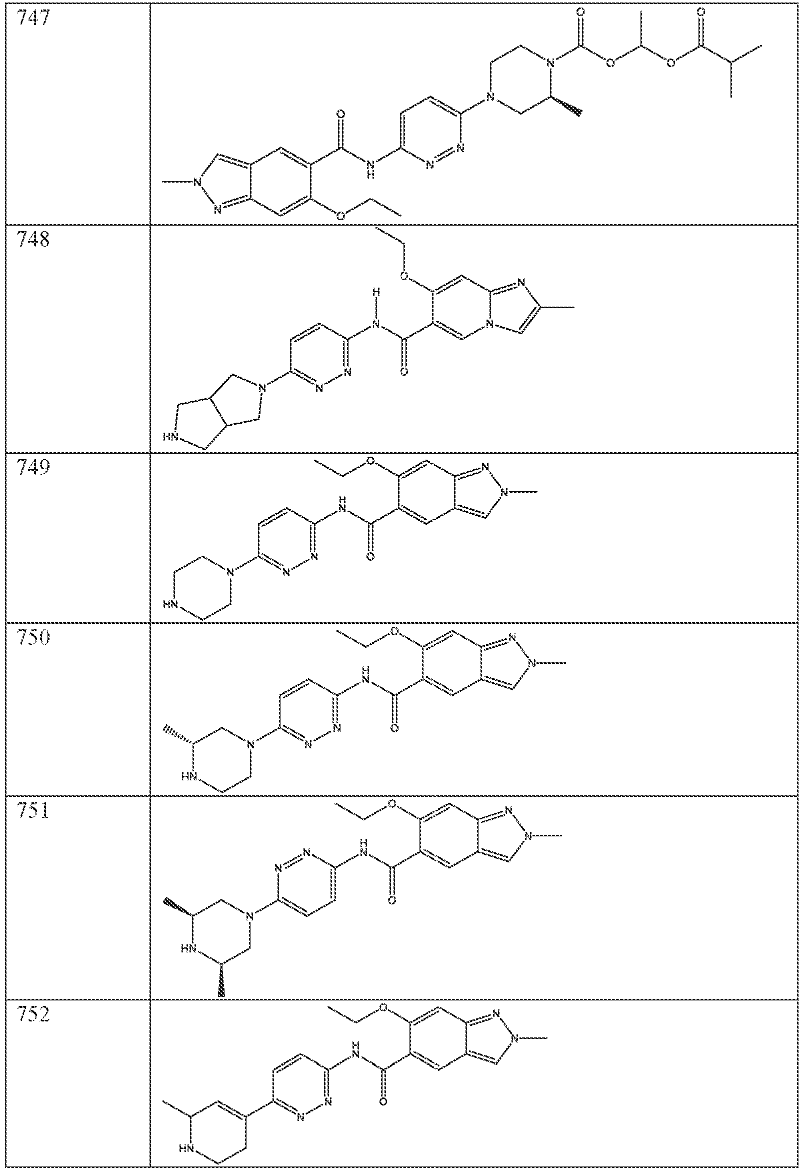

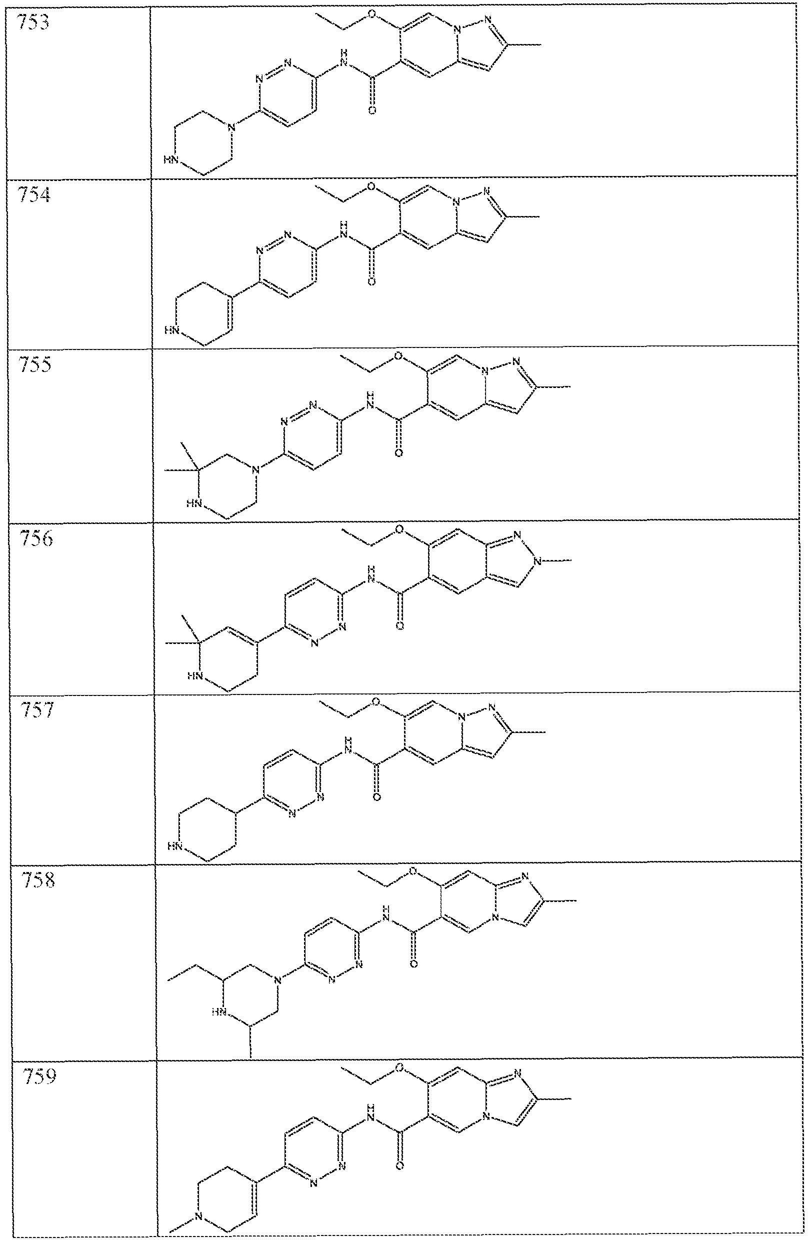

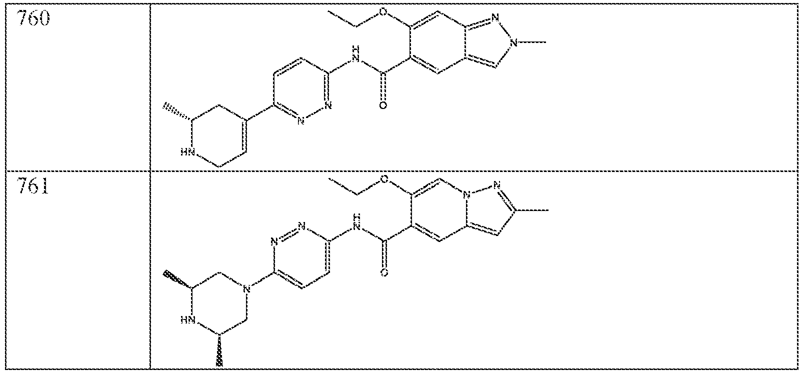

or a pharmaceutically acceptable salt thereof.

For the avoidance of doubt it is to be understood that, where in this specification a group is qualified by "described herein", the said group encompasses the first occurring and broadest definition as well as each and all of the particular definitions for that group.

The various functional groups and substituents making up the compounds of the Formula (I) are typically chosen such that the molecular weight of the compound does not exceed 1000 daltons. More usually, the molecular weight of the compound will be less than 900, for example less than 800, or less than 750, or less than 700, or less than 650 daltons. More conveniently, the molecular weight is less than 600 and, for example, is 550 daltons or less.

It will be understood that the compounds of any one of the Formulae disclosed herein and any pharmaceutically acceptable salts thereof, comprise stereoisomers and mixtures of stereoisomers of said compounds.

It is to be understood that the compounds of any Formula described herein include the compounds themselves, as well as their salts, and their solvates, if applicable.

The *in vivo* effects of a compound of any one of the Formulae disclosed herein may be exerted in part by one or more metabolites that are formed within the human or animal body after administration of a compound of any one of the Formulae disclosed herein. As stated hereinbefore, the *in vivo* effects of a compound of any one of the Formulae disclosed herein may also be exerted by way of metabolism of a precursor compound (a prodrug).

Suitably, the present disclosure excludes any individual compounds not possessing the biological activity defined herein.

### Methods of Synthesis

By way of example only, provided is a scheme for preparing the small molecule splicing modulators (SMSMs) described herein.

In some embodiments, a scheme for preparing an SMSM is described herein in

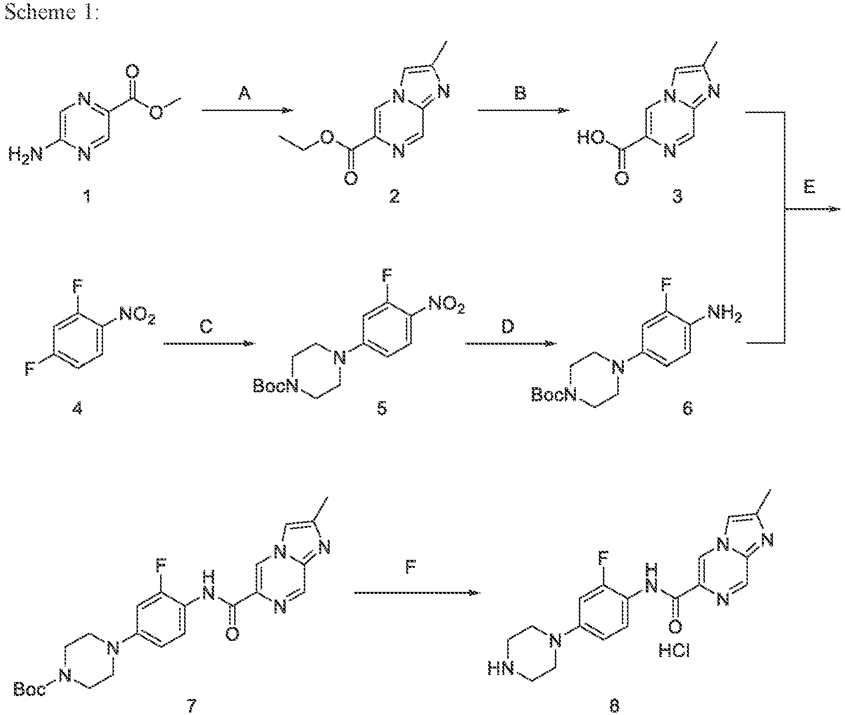

In some aspects, the present disclosure provides a method of preparing a compound of the present disclosure.

In some aspects, the present disclosure provides a method of preparing a compound, comprising one or more steps as described herein.

In some aspects, the present disclosure provides a compound obtainable by, or obtained by, or directly obtained by a method for preparing a compound as described herein.

In some aspects, the present disclosure provides an intermediate as described herein, being suitable for use in a method for preparing a compound as described herein.

The compounds of the present disclosure can be prepared by any suitable technique known in the art. Particular processes for the preparation of these compounds are described further in the accompanying examples.

In the description of the synthetic methods described herein and in any referenced synthetic methods that are used to prepare the starting materials, it is to be understood that all proposed reaction conditions, including choice of solvent, reaction atmosphere, reaction temperature, duration of the experiment and workup procedures, can be selected by a person skilled in the art.

It is understood by one skilled in the art of organic synthesis that the functionality present on various portions of the molecule must be compatible with the reagents and reaction conditions utilized.

It will be appreciated that during the synthesis of the compounds of the disclosure in the processes defined herein, or during the synthesis of certain starting materials, it may be desirable to protect certain substituent groups to prevent their undesired reaction. The skilled chemist will appreciate when such protection is required, and how such protecting groups may be put in place, and later removed. For examples of protecting groups see one of the many general texts on the subject, for example, 'Protective Groups in Organic Synthesis' by Theodora Green (publisher: John Wiley & Sons). Protecting groups may be removed by any convenient method described in the literature or known to the skilled chemist as appropriate for the removal of the protecting group in question, such methods being chosen so as to effect removal of the protecting group with the minimum disturbance of groups elsewhere in the molecule. Thus, if reactants include, for example, groups such as amino, carboxy or hydroxy it may be desirable to protect the group in some of the reactions mentioned herein.

By way of example, a suitable protecting group for an amino or alkylamino group is, for example, an acyl group, for example an alkanoyl group such as acetyl, an alkoxycarbonyl group, for example a methoxycarbonyl, ethoxycarbonyl, or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed by, for example, hydrolysis with a suitable base such as an alkali metal hydroxide, for example lithium or sodium hydroxide. Alternatively an acyl group such as a tert-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium on carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium, sodium hydroxide or ammonia. Alternatively an arylmethyl group such as a benzyl group may be removed, for example, by hydrogenation over a catalyst such as palladium on carbon.

A suitable protecting group for a carboxy group is, for example, an esterifying group, for example a methyl or an ethyl group which may be removed, for example, by hydrolysis with a base such as sodium hydroxide, or for example a tert-butyl group which may be removed, for example, by treatment with an acid, for example an organic acid such as trifluoroacetic acid, or for example a benzyl group which may be removed, for example, by hydrogenation over a catalyst such as palladium on carbon.

Once a compound of Formula (I) has been synthesised by any one of the processes defined herein, the processes may then further comprise the additional steps of: (i) removing any protecting groups present; (ii) converting the compound of Formula (I) into another compound of Formula (I); and/or (iii) forming a pharmaceutically acceptable salt, hydrate or solvate thereof.

The resultant compounds of Formula (I) can be isolated and purified using techniques well known in the art.

Conveniently, the reaction of the compounds is carried out in the presence of a suitable solvent, which is preferably inert under the respective reaction conditions. Examples of suitable solvents comprise but are not limited to hydrocarbons, such as hexane, petroleum ether, benzene, toluene or xylene; chlorinated hydrocarbons, such as trichlorethylene, 1,2-dichloroethane, tetrachloromethane, chloroform or dichloromethane; alcohols, such as methanol, ethanol, isopropanol, n-propanol, n-butanol or tert-butanol; ethers, such as diethyl ether, diisopropyl ether, tetrahydrofuran (THF), 2-methyltetrahydrofuran, cyclopentylmethyl ether (CPME), methyl tert-butyl ether (MTBE) or dioxane; glycol ethers, such as ethylene glycol monomethyl or monoethyl ether or ethylene glycol dimethyl ether (diglyme); ketones, such as acetone, methylisobutylketone (MIBK) or butanone; amides, such as acetamide, dimethylacetamide, dimethylformamide (DMF) or N-methylpyrrolidinone (NMP); nitriles, such as acetonitrile; sulphoxides, such as dimethyl sulphoxide (DMSO); nitro compounds, such as nitromethane or nitrobenzene; esters, such as ethyl acetate or methyl acetate, or mixtures of the said solvents or mixtures with water.

The reaction temperature is suitably between about -100 °C and 300 °C, depending on the reaction step and the conditions used.

Reaction times are generally in the range between a fraction of a minute and several days, depending on the reactivity of the respective compounds and the respective reaction conditions. Suitable reaction times are readily determinable by methods known in the art, for example reaction monitoring. Based on the reaction temperatures given above, suitable reaction times generally lie in the range between 10 minutes and 48 hours.

Moreover, by utilising the procedures described herein, in conjunction with ordinary skills in the art, additional compounds of the present disclosure can be readily prepared. Those skilled in the art will readily understand that known variations of the conditions and processes of the following preparative procedures can be used to prepare these compounds. As will be understood by the person skilled in the art of organic synthesis, compounds of the present disclosure are readily accessible by various synthetic routes, some of which are exemplified in the accompanying examples. The skilled person will easily recognise which kind of reagents and reactions conditions are to be used and how they are to be applied and adapted in any particular instance - wherever necessary or useful - in order to obtain the compounds of the present disclosure. Furthermore, some of the compounds of the present disclosure can readily be synthesised by reacting other compounds of the present disclosure under suitable conditions, for instance, by converting one particular functional group being present in a compound of the present disclosure, or a suitable precursor molecule thereof, into another one by applying standard synthetic methods, like reduction, oxidation, addition or substitution reactions; those methods are well known to the skilled person. Likewise, the skilled person will apply - whenever necessary or useful - synthetic protecting (or protective) groups; suitable protecting groups as well as methods for introducing and removing them are well-known to the person skilled in the art of chemical synthesis and are described, in more detail, in, e.g., P.G.M. Wuts, T.W. Greene, "Greene's Protective Groups in Organic Synthesis", 4th edition (2006) (John Wiley & Sons).

General routes for the preparation of a compound of the application are described herein.

### Biological Assays

Compounds designed, selected and/or optimised by methods described above, once produced, can be characterised using a variety of assays known to those skilled in the art to determine whether the compounds have biological activity. For example, the molecules can be characterised by conventional assays, including but not limited to those assays described below, to determine whether they have a predicted activity, binding activity and/or binding specificity.

### Pharmaceutical Compositions

In some aspects, the present disclosure provides a pharmaceutical composition comprising a compound of the present disclosure as an active ingredient. In some embodiments, the present disclosure provides a pharmaceutical composition comprising at least one compound of each of the formulae described herein, or a pharmaceutically acceptable salt or solvate thereof, and one or more pharmaceutically acceptable carriers or excipients. In some embodiments, the present disclosure provides a pharmaceutical composition comprising at least one compound selected from Table 1.

As used herein, the term "composition" is intended to encompass a product comprising the specified ingredients in the specified amounts, as well as any product which results, directly or indirectly, from combination of the specified ingredients in the specified amounts.
The compounds of present disclosure can be formulated for oral administration in forms such as tablets, capsules (each of which includes sustained release or timed release formulations), pills, powders, granules, elixirs, tinctures, suspensions, syrups and emulsions. The compounds of present disclosure on can also be formulated for intravenous (bolus or infusion), intraperitoneal, topical, subcutaneous, intramuscular or transdermal (e.g., patch) administration, all using forms well known to those of ordinary skill in the pharmaceutical arts.

The formulation of the present disclosure may be in the form of an aqueous solution comprising an aqueous vehicle. The aqueous vehicle component may comprise water and at least one pharmaceutically acceptable excipient. Suitable acceptable excipients include those selected from the group consisting of a solubility enhancing agent, chelating agent, preservative, tonicity agent, viscosity/suspending agent, buffer, and pH modifying agent, and a mixture thereof.

Any suitable solubility enhancing agent can be used. Examples of a solubility enhancing agent include cyclodextrin, such as those selected from the group consisting of hydroxypropyl-β-cyclodextrin, methyl-β-cyclodextrin, randomly methylated-β-cyclodextrin, ethylated-β-cyclodextrin, triacetyl-β-cyclodextrin, peracetylated-β-cyclodextrin, carboxymethyl-β-cyclodextrin, hydroxyethyl-β-cyclodextrin, 2-hydroxy-3-(trimethylammonio)propyl-β-cyclodextrin, glucosyl-β-cyclodextrin, sulphated β-cyclodextrin (S-β-CD), maltosyl-β-cyclodextrin, β-cyclodextrin sulphobutyl ether, branched-β-cyclodextrin, hydroxypropyl-γ-cyclodextrin, randomly methylated-γ-cyclodextrin, and trimethyl-γ-cyclodextrin, and mixtures thereof.

Any suitable chelating agent can be used. Examples of a suitable chelating agent include those selected from the group consisting of ethylenediaminetetraacetic acid and metal salts thereof, disodium edetate, trisodium edetate, and tetrasodium edetate, and mixtures thereof.
Any suitable preservative can be used. Examples of a preservative include those selected from the group consisting of quaternary ammonium salts such as benzalkonium halides (preferably benzalkonium chloride), chlorhexidine gluconate, benzethonium chloride, cetyl pyridinium chloride, benzyl bromide, phenylmercury nitrate, phenylmercury acetate, phenylmercury neodecanoate, merthiolate, methylparaben, propylparaben, sorbic acid, potassium sorbate, sodium benzoate, sodium propionate, ethyl p-hydroxybenzoate, propylaminopropyl biguanide, and butyl-p-hydroxybenzoate, and sorbic acid, and mixtures thereof.

The aqueous vehicle may also include a tonicity agent to adjust the tonicity (osmotic pressure). The tonicity agent can be selected from the group consisting of a glycol (such as propylene glycol, diethylene glycol, triethylene glycol), glycerol, dextrose, glycerin, mannitol, potassium chloride, and sodium chloride, and a mixture thereof.

The aqueous vehicle may also contain a viscosity/suspending agent. Suitable viscosity/suspending agents include those selected from the group consisting of cellulose derivatives, such as methyl cellulose, ethyl cellulose, hydroxyethylcellulose, polyethylene glycols (such as polyethylene glycol 300, polyethylene glycol 400), carboxymethyl cellulose, hydroxypropylmethyl cellulose, and cross-linked acrylic acid polymers (carbomers), such as polymers of acrylic acid cross-linked with polyalkenyl ethers or divinyl glycol (Carbopols - such as Carbopol 934, Carbopol 934P, Carbopol 971, Carbopol 974 and Carbopol 974P), and a mixture thereof.

In order to adjust the formulation to an acceptable pH (typically a pH range of about 5.0 to about 9.0, more preferably about 5.5 to about 8.5, particularly about 6.0 to about 8.5, about 7.0 to about 8.5, about 7.2 to about 7.7, about 7.1 to about 7.9, or about 7.5 to about 8.0), the formulation may contain a pH modifying agent. The pH modifying agent is typically a mineral acid or metal hydroxide base, selected from potassium hydroxide, sodium hydroxide, and hydrochloric acid, and mixtures thereof, and preferably sodium hydroxide and/or hydrochloric acid. These acidic and/or basic pH modifying agents are added to adjust the formulation to the target acceptable pH range. Hence it may not be necessary to use both acid and base - depending on the formulation, the addition of one of the acid or base may be sufficient to bring the mixture to the desired pH range.

The aqueous vehicle may also contain a buffering agent to stabilise the pH. When used, the buffer is selected from the group consisting of a phosphate buffer (such as sodium dihydrogen phosphate and disodium hydrogen phosphate), a borate buffer (such as boric acid, or salts thereof including disodium tetraborate), a citrate buffer (such as citric acid, or salts thereof including sodium citrate), and e-aminocaproic acid, and mixtures thereof.

The formulation may further comprise a wetting agent. Suitable classes of wetting agents include those selected from the group consisting of polyoxypropylene-polyoxyethylene block copolymers (poloxamers), polyethoxylated ethers of castor oils, polyoxyethylenated sorbitan esters (polysorbates), polymers of oxyethylated octyl phenol (Tyloxapol), polyoxyl 40 stearate, fatty acid glycol esters, fatty acid glyceryl esters, sucrose fatty esters, and polyoxyethylene fatty esters, and mixtures thereof.

According to a further aspect of the disclosure there is provided a pharmaceutical composition which comprises a compound of the disclosure as defined hereinbefore, or a pharmaceutically acceptable salt, hydrate or solvate thereof, in association with a pharmaceutically acceptable diluent or carrier.

The compositions of the disclosure may be in a form suitable for oral use (for example as tablets, lozenges, hard or soft capsules, aqueous or oily suspensions, emulsions, dispersible powders or granules, syrups or elixi rs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

The compositions of the disclosure may be obtained by conventional procedures using conventional pharmaceutical excipients, well known in the art. Thus, compositions intended for oral use may contain, for example, one or more colouring, sweetening, flavouring and/or preservative agents.

An effective amount of a compound of the present disclosure for use in therapy is an amount sufficient to treat or prevent a disease or disorder referred to herein, slow its progression and/or reduce the symptoms associated with the condition.

An effective amount of a compound of the present disclosure for use in therapy is an amount sufficient to treat a disease or disorder referred to herein, slow its progression and/or reduce the symptoms associated with the condition.

The size of the dose for therapeutic or prophylactic purposes of a compound of Formula (I) will naturally vary according to the nature and severity of the conditions, the age and sex of the animal or subject and the route of administration, according to well-known principles of medicine.

### Methods of Use

Provided herein is a method of treating a disease in a subject in need thereof, the method comprising administering to the subject a therapeutically effective amount of a compound or a pharmaceutical composition described herein, wherein the disease is selected from the group consisting of Dentatorubropallidoluysian atrophy, Huntington's disease, Spinal and bulbar muscular atrophy, SCA1 (Spinocerebellar ataxia Type 1), SCA2 (Spinocerebellar ataxia Type 2), SCA3 (Spinocerebellar ataxia Type 3 or Machado-Joseph disease), SCA6 (Spinocerebellar ataxia Type 6), SCA7 (Spinocerebellar ataxia Type 7), SCA12 (Spinocerebellar ataxia Type 12), SCA17 (Spinocerebellar ataxia Type 17), FRAXA (Fragile X syndrome), FXTAS (Fragile X-associated tremor/ataxia syndrome), FRAXE (Fragile XE mental retardation), Baratela-Scott syndrome, FRDA (Friedreich's ataxia), DM1 (Myotonic dystrophy Type 1), DM2 (Myotonic dystrophy Type 2) SCAS (Spinocerebellar ataxia Type 8), Fuchs endothelial corneal dystrophy, Desbuquois dysplasia, amyotrophic lateral sclerosis, frontotemporal dementia..

In some embodiments, the disease is Huntington's disease.

Provided herein is a method of treating a disease in a subject in need thereof, the method comprising administering to the subject a therapeutically effective amount of a compound or a pharmaceutical composition disclosed herein, wherein the disease is Huntington's disease.

In some embodiments, the disease is myotonic dystrophy 1.

Provided herein is a method of treating a disease in a subject in need thereof, the method comprising administering to the subject a therapeutically effective amount of a compound or a pharmaceutical composition disclosed herein, wherein the disease is myotonic dystrophy 1.

In some embodiments, the disease is selected from the group consisting of FRAXA (Fragile X syndrome), FXTAS (Fragile X-associated tremor/ataxia syndrome), FRAXE (Fragile XE mental retardation).

Provided herein is a method of treating a disease in a subject in need thereof, the method comprising administering to the subject a therapeutically effective amount of a compound or a pharmaceutical composition disclosed herein, wherein the disease is selected from the group consisting of FRAXA (Fragile X syndrome), FXTAS (Fragile X-associated tremor/ataxia syndrome), FRAXE (Fragile XE mental retardation).

### Routes of Administration

The compounds of the disclosure or pharmaceutical compositions comprising these compounds may be administered to a subject by any convenient route of administration, whether systemically/ peripherally or topically (i.e., at the site of desired action).

Routes of administration include, but are not limited to, oral (e.g. by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intra-arterial, intracardiac, intrathecal, intraspinal, intracapsular, subcapsular, intraorbital, intraperitoneal, intratracheal, subcuticular, intraarticular, subarachnoid, and intrastemal; by implant of a depot or reservoir, for example, subcutaneously or intramuscularly.

### EXAMPLES

For exemplary purpose, neutral compounds of Formula (I) are synthesized and tested in the examples. It is understood that the neutral compounds of Formula (I) may be converted to the corresponding pharmaceutically acceptable salts of the compounds using routine techniques in the art (e.g., by saponification of an ester to the carboxylic acid salt, or by hydrolyzing an amide to form a corresponding carboxylic acid and then converting the carboxylic acid to a carboxylic acid salt).

Nuclear magnetic resonance (NMR) spectra were recorded at 400 MHz or 300 MHz as stated; the chemical shifts (δ) are reported in parts per million (ppm). Spectra were recorded using a Bruker or Varian instrument with 8, 16 or 32 scans.

LC-MS chromatograms and spectra were recorded using an Agilent 1200 or Shimadzu LC-20 AD&MS 2020 instrument using a C-18 column such as C18 2.1 x 30 mm, unless otherwise stated. Injection volumes were 0.7 - 8.0 µl and the flow rates were typically 0.8 or 1.2 ml/min. Detection methods were diode array (DAD) or evaporative light scattering (ELSD) as well as positive ion electrospray ionisation. MS range was 100 - 1000 Da. Solvents were gradients of water and acetonitrile both containing a modifier (typically 0.01 - 0.04 %) such as trifluoroacetic acid or ammonium carbonate.

Abbreviations:
- ACN: Acetonitrile
- AcOH: Acetic acid
- Boc: *tert*-Butyloxycarbonyl
- BINAP: (2,2'-bis(diphenylphosphino)-1,1'-binaphthyl)
- CDCl₃: Chloroform-*d*
- DAD: Diode-Array Detection
- DCM: Dichloromethane
- DIPEA: N,N-Diisopropylethylamine
- DIEA: N,N-Diisopropylethylamine
- DMF: N,N-Dimethylformamide
- DMSO: Dimethylsulphoxide
- DMSO-*d*₆: Hexadeuterodimethylsulphoxide
- DMT: Dimercaptotriazine
- ESI: Electrospray ionisation
- EA: Ethyl acetate
- EtOAc: Ethyl acetate
- EtOH: Ethanol
- FA: Formic acid
- h: Hour(s)
- HATU: 1-[Bis(dimethylamino)methylene]-1H-1,2,3-triazolo[4,5-b]pyridinium 3-oxide hexafluorophosphate
- HMPA: Hexamethylphosphoramide
- ¹H NMR: Proton nuclear magnetic resonance spectroscopy
- HPLC: High performance liquid chromatography
- i-PrOH: Isopropyl alcohol
- LCMS: Liquid chromatography-mass spectrometry
- MeOD: Methanol-*d*₄
- MeOH: Methanol
- MeCN: Acetonitrile
- MS: Mass Spectrometry
- MTBE: Methyl tert-butyl ether
- min: Minute(s)
- n-BuLi: n-Butyllithium
- NMI: 1-Methylimidazole
- NMP: N-Methyl-2-Pyrrolidone
- NBS: N-Bromosuccinimide
- Pd(dppf)Cl₂: [1,1'-Bis(diphenylphosphino)ferrocene]dichloropalladium(II)
- PE: Petroleum ether
- PPTS: Pyridinium p-toluenesulfonate
- RuPhos: 2-Dicyclohexylphosphino-2',6'-diisopropoxybiphenyl
- rt or r.t.: Room temperature
- TCFH: N,N,N',N'-Tetramethylchloroformamidinium hexafluorophosphate
- TEA: Triethylamine
- TFA: Trifluoroacetic acid
- THF: Tetrahydrofuran
- TLC: Thin layer chromatography
- Y: Yield

### Example 1. 2-methylimidazo[1,2-a]pyrazine-6-carboxylic acid.

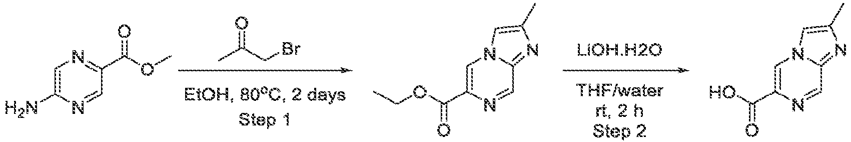

### Step 1: Preparation of ethyl 2-methylimidazo[1,2-a]pyrazine-6-carboxylate.

To a mixture of methyl 5-aminopyrazine-2-carboxylate (5 g, 32.7 mmol) in EtOH (50 mL) was added 1-bromopropan-2-one (9 g, 63.4 mmol). The mixtre was stirred at 80 °C for 2 days. After cooling to rt, the reaction mixture was concentrated. The crude was purified by silica gel column (DCM/MeOH=10:1) to give title product (3.5 g, Y: 52%) as a grey solid. ESI-MS (M+H)⁺: 206.1. ¹H NMR (400 MHz, DMSO-d₆) δ 9.58 (d*, J =* 0.9 Hz, 1H), 9.26 (s, 1H), 8.28 (s, 1H), 4.41 (q, *J* = 7.1 Hz, 2H), 2.55 (s, 3H), 1.37 (t, *J* = 7.1 Hz, 4H).

### Step 2 : Preparation of 2-methylimidazo[1,2-a]pyrazine-6-carboxylic acid.

To a mixture of ethyl 2-methylimidazo[1,2-a]pyrazine-6-carboxylate (400 mg, 1.94 mmol) in THF/water (12 mL, 5:1) was added LiOH.H₂O (388 mg, 9.71 mmol). The mixtre was stirred at rt for 2 h. After concentration, the residue was adjusted to pH = 5 with 1M HCl. The cured was purified by reverse phase column to give title product (200 mg, Y: 58%) as a grey solid. ESI-MS (M+H)⁺: 178.1.

### Example 2. 8-fluoro-2-methylimidazo[1,2-a]pyridine-6-carboxylic acid.

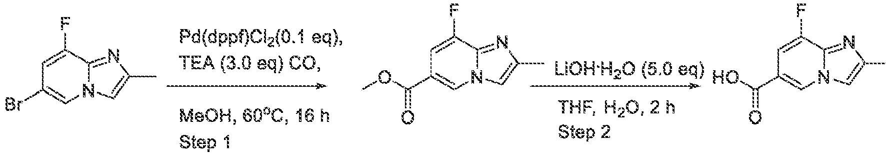

### Step 1: Preparation of methyl 8-fluoro-2-methylimidazo[1,2-a]pyridine-6-carboxylate.

To a mixture of 6-bromo-8-fluoro-2-methylimidazo[1,2-a]pyridine (1 g, 4.37 mmol) in MeOH (20 mL) was added TEA (1.3 g, 13.11 mmol) and Pd(dppf)Cl₂ (322 mg, 0.44 mmol). The resulting mixture was stirred overnight at 60 °C under CO (balloon). The mixture was allowed to cooling down to room temperature and concentrated. The residue was purified by silica gel column chromatography (PE:EA=5:1) to give title product (800 mg, 88%) as a yellow solid. ESI-MS (M+H)⁺: 209.0 ¹H NMR (400 MHz, DMSO-d₆) δ 9.15 (d, *J* = 1.0 Hz, 1H), 7.97 (d, *J =* 3.0 Hz, 1H), 7.38 (dd, *J =* 11.5, 1.0 Hz, 1H), 3.89 (s, 3H), 2.38 (s, 3H).

### Step 2: Preparation of 8-fluoro-2-methylimidazo[1,2-a]pyridine-6-carboxylic acid.

To a mixture of methyl 8-fluoro-2-methylimidazo[1,2-a]pyridine-6-carboxylate (800 mg, 3.85 mmol) in THF (20 mL) was added H₂O (4 mL) and LiOH·H₂O (809 mg, 19.23 mmol). The reaction mixture was stirred for 2 h at room temperature. After concentration, the residue was diluted with water and the mixture was adjusted to pH to 5 with HCl (2M). The solid was formed and collected by filtration and the cake was washed with water. The solid was dried under vaccum at 55°C to give title product (670 mg, 90%) as a white solid. ESI-MS (M+H)⁺: 195.0 ¹H NMR (400 MHz, DMSO-d6) δ 9.32 (d, *J =* 1.1 Hz, 1H), 8.18 *(d, J=* 1.4 Hz, 1H), 7.84 (d*, J =* 10.8 Hz, 1H), 2.48 (d, *J* = 0.6 Hz, 3H).

### Example 3. 2,8-dimethylimidazo[1,2-a]pyrazine-6-carboxylic acid

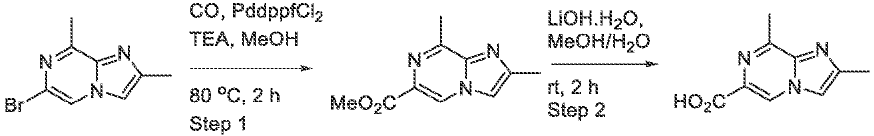

### Step 1: Preparation of methyl 2,8-dimethylimidazo[1,2-a]pyrazine-6-carboxylate.

To a mixture of 6-bromo-2,8-dimethylimidazo[1,2-a]pyrazine (500 mg, 2.21 mmol) in MeOH (20 mL) was added TEA (670 mg, 6.64 mmol) and Pd(dppf)Cl₂ (162 mg, 0.22 mmol). The resulting mixture was stirred at 80 °C overnight under CO. The mixture was allowed to cooling down to room temperature and concentrated. The residue was purified by silica gel column chromatography (PE:EA=5:1) to give methyl 2,8-dimethylimidazo[1,2-a]pyrazine-6-carboxylate (420 mg, 92.6%) as a yellow solid. ESI-MS (M+H)⁺: 206.

### Step2: Preparation of 2,8-dimethylimidazo[1,2-a]pyrazine-6-carboxylic acid.

To a mixture of methyl 2,8-dimethylimidazo[1,2-a]pyrazine-6-carboxylate (420 mg, 2.04 mmol) in MeOH (10 mL) and H₂O (1 mL) was added LiOH·H₂O (150 mg, 6.12 mmol). The reaction mixture was stirred for 2 h at room temperature. The mixture was adjust pH to 5 with HCl (2M). The reaction was concentrated to give 2,8-dimethylimidazo [1,2-a]pyrazine-6-carboxylic acid (450 mg, crude, containing some inorganic salt) as a yellow solid, which was used to next step without further purification. ESI-MS (M+H)⁺: 192.1.

### Example 4. 7-methoxy-2-methylimidazo[1,2-a]pyridine-6-carboxylic acid.

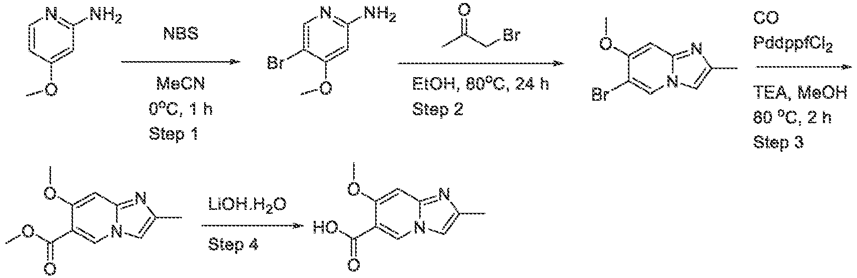

### Step 1: Preparation of 5-bromo-4-methoxypyridin-2-amine.

To a solution of 4-methoxypyridin-2-amine (1.00 g, 8.07 mmol) in MeCN (10 mL) was added NBS (1.45 g, 8.07 mmol) portionwise at 0 °C and stirred at 0 °C for 1 h. The reaction mixture was diluted with ethyl acetate (80 mL) and washed with water (80 mL) and brine (80 mL). The organic layer was dried over sodium sulfate, filterted and concentrated in vacuo to give 5-bromo-4-methoxypyridin-2-amine (1.5 g, crude) as an off-white solid, which was used to next step without further purification. ESI-MS (M+H)⁺: 203.0.

### Step 2: Preparation of 6-bromo-7-methoxy-2-methylimidazo[1,2-a]pyridine.

To a solution of 5-bromo-4-methoxypyridin-2-amine (1.50 g, 7.40 mmol) in EtOH (30 mL) was added 1-bromopropan-2-one (2.00 g, 14.90 mmol). The mixtre was stirred at 80 °C for 24 h. After cooling to rt, the reaction mixture was concentrated. The residue was dissolved with EA and the orangic phase was stirred with sat. aqueous sodium carbante for 30 min. The two phases were separated. The organic pahse was concentrated. The crude was purified by silica gel column (DCM/MeOH=10:1) to give title product (0.8 g, Y: 47%) as a grey solid. ESI-MS (M+H)⁺: 241.0. ¹H NMR (400 MHz, DMSO-d₆) δ 8.78 (s, 1H), 7.45 (s, 1H), 6.95 (s, 1H), 3.89 (s, 3H), 2.26 (s, 3H).

### Step 3: Preparation of methyl 7-methoxy-2-methylimdazo[1,2-a]pyridine-6-carboxylate.

To a mixture of 6-bromo-7-methoxy-2-methylimidazo[1,2-a]pyridine (0.80 g, 3.32 mmol) in MeOH (20 mL) was added TEA(1.00 g, 9.96 mmol) and Pd(dppf)Cl₂ ( 243 mg, 0.33 mmol. The mixture was charged with CO for three times and stirred at 80 °C for 16 h. The reaction mixture was diluted with ethyl acetate (100 mL) and washed with water (50 mL) and brine (50 mL). The organic layer was dried over sodium sulfate, filtered and concentrated in vacuo to give methyl-7-methoxy-2-methylimidazo [1,2-a]pyridine-6-carboxylate (0.5 g, crude) as a gray solid. ESI-MS (M+H)⁺: 221.2.

### Step 4 : Preparation of 7-methoxy-2-methylimidazo[1,2-a]pyridine-6-carboxylic acid.

To a mixture of methyl 7-methoxy-2-methylimidazo[1,2-a]pyridine-6-carboxylate (250 mg, 1.14 mmol) in THF/water (6 mL, 5:1) was added LiOH·H₂O (100 mg, 3.41 mmol). The mixture was stirred at rt for 2 h. After concentration, the residue was adjusted to pH = 5 with 1M HCl. The cured was purified by reverse phase column to give 7-methoxy-2-methylimidazo[1,2-a]pyridine-6-carboxylic acid (200 mg, Y: 85%) as a gray solid. ESI-MS (M+H)⁺: 207.1.

### Example 5. 7-methoxy-2-methylimidazo[1,2-a]pyridine-6-carboxylic acid.

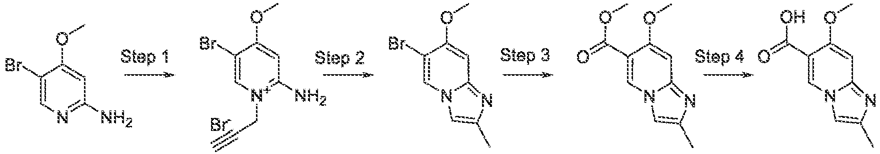

### Step 1: Preparation of 2-amino-5-bromo-4-methoxy-1-(prop-2-yn-1 -yl)pyridin-1-ium bromide.

5-Bromo-4-methoxypyridin-2-amine (30.2 g, 148.74 mmol), 3-bromoprop-1-yne (21.23 g, 178.49 mmol) and 2-propanol (350 mL) were added to a small Schlenk flask and stirred vigorously at 80 °C overnight. After that, the mixture was allowed to cool to room temperature and the excess of solvent and propargyl bromide was removed under high vacuum. The resulting crude residue of 2-amino-5-bromo-4-methoxy-1-(prop-2-yn-1-yl)pyridin-1-ium bromide (47.0 g, 90.0% purity, 131.37 mmol, 88.3% yield) was used in the next step without purification. ¹H NMR (400 MHz, DMSO-d₆) δ: 8.6 (br s, 2H), 8.57 (s, 1H), 6.75 (s, 1H), 5.06 (s, 2H), 3.97 (s, 3H).

### Step 2: Preparation of 6-bromo-7-methoxy-2-methylimidazo[1,2-a]pyridine.

To a stirring solution of sodium hydroxide (5.89 g, 147.36 mmol, 5.89 mL, 1.01 equiv.) in deionised H₂O (300 mL) was added 2-amino-5-bromo-4-methoxy-1-(prop-2-yn-1-yl)pyridin-1-ium bromide (46.98 g, 145.9 mmol) via powder addition funnel over a period of 30 minutes. Immediately upon addition, the solution phase turned yellow and yellow oil became dispersed as a distinct separate phase. The oil product was extracted with EtOAc (2 × 150 mL), dried over anhydrous MgSO₄, filtered and concentrated under reduced pressure to afford 6-bromo-7-methoxy-2-methylimidazo[1,2-a]pyridine (22.5 g, 90.0% purity, 84.0 mmol, 57.6% yield) as a pale yellow solid. ¹H NMR (400 MHz, DMSO-d₆) δ: 8.76 (s, 1H), 7.43 (s, 1H), 6.94 (s, 1H), 3.87 (s, 3H), 2.55 (s, 3H).

### Step 3: Preparation of methyl 7-methoxy-2-methylimidazo[1,2-a]pyridine-6-carboxylate.

6-Bromo-7-methoxy-2-methylimidazo[1,2-a]pyridine (30.68 g, 127.26 mmol) was dissolved in MeOH (250 mL) and [1,1'-Bis(diphenylphosphino)ferrocene]dichloropalladium(II), complex with dichloromethane (2.08 g, 2.55 mmol) was added followed by triethylamine (28.33 g, 279.98 mmol). The resulting mixture was transferred into autoclave and stirred at 130 °C under 40 bar pressure of CO overnight. Then the MeOH was evaporated and the residue was partitioned between water (150 mL) and EtOAc (300 mL). The organic layer was separated, dried over Na₂SO₄ and evaporated under reduced pressure to give crude methyl 7-methoxy-2-methylimidazo[1,2-a]pyridine-6-carboxylate (19.15 g, 86.96 mmol, 68.3% yield), which was used in the next step without purification. ESI-MS (M+H)⁺: 221.2.

### Step 4: Preparation of 7-methoxy-2-methylimidazo[1,2-a]pyridine-6-carboxylic acid.

A mixture of methyl 7-methoxy-2-methylimidazo[1,2-a]pyridine-6-carboxylate (19.15 g, 86.96 mmol) and potassium hydroxide (7.32 g, 130.43 mmol) was stirred in methanol (150 mL) and H₂O (50 mL) overnight. The reaction mixture was concentrated under reduced pressure to remove methanol, and the resulting aqueous solution was neutralized with 1 N HCl to pH=5 to precipitate the carboxylic acid. The solid 7-methoxy-2-methylimidazo[1,2-a]pyridine-6-carboxylic acid (14.0 g, 95.0% purity, 64.5 mmol, 74.2% yield) was isolated by filtration, washed with H₂O (20 mL) and MeCN (20 mL), dried and used directly in the next step without further purification. ¹H NMR (400 MHz, DMSO-d₆) δ: 9.23 (s, 1H), 7.88 (s, 1H), 7.21 (s, 1H), 3.97 (s, 3H), 2.39 (s, 3H).

### Example 6. 7-methoxy-2,8-dimethylimidazo[1,2-a]pyridine-6-carboxylic acid.

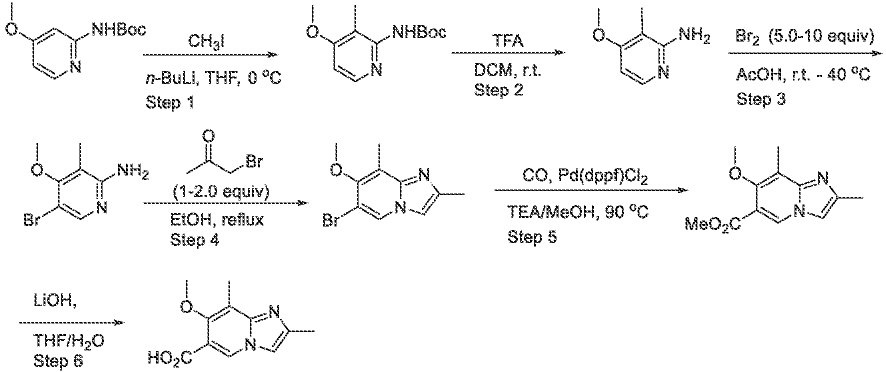

### Step 1: Preparation of tert-butyl (4-methoxy-3-methylpyridin-2-yl)carbamate.

To a stirred mixture of tert-butyl (4-methoxypyridin-2-yl)carbamate (4.47 g, 19.93 mmol) in dry THF (100 mL) was added n-BuLi (2.5 M, 19.9 mL) under N₂ atmosphere with ice-water bathing, the reaction was stirred for 1 h at 0 °C, then CH₃I was added by dropwise within 30 min, the mixture was stirred for 2 h at room temperature. The mixture was quenched with H₂O (50 mL) and extracted with EtOAc (50 mL x 3). The organic phase was washed with saturated NaCl (20 mL) and dried with Na₂SO₄, The mixture was concentrated under vacuum to afford crude product, the crude product was purified by silica gel column (PE/EA= 1:1) to afford title product (4.3 g , Y: 86.1%) as light yellow solid. ESI-MS (M+H)⁺: 239.1.

### Step 2: Preparation of 4-methoxy-3-methylpyridin-2-amine.

To a stirred mixture of tert-butyl (4-methoxy-3-methylpyridin-2-yl)carbamate (4.0 g, 16.78 mmol) in DCM (50 mL) was added TFA (10 mL) by dropwise under N₂ atmosphere at room temperature. the reaction was stirred for 4 h at room temperature. The mixture was concentrated under under vacuum to afford title product TFA salt (4.1 g Y: 99%) as light yellow solid, the crude mixture was directedly used for the next step without further purification. ESI-MS (M+H)⁺: 139.1.

### Step 3: Preparation of 5-bromo-4-methoxy-3-methylpyridin-2-amine.

To a stirred mixture of 4-methoxy-3-methylpyridin-2-amine (2.70 g, 19.55 mmol) in AcOH (20 mL) was added Br₂ (15.62 g, 97.75 mmol) by dropwise at 40 °C within 30 min. the mixture was stirred for 1 h at 40 °C. The mixture was quenched with H₂O (10 mL) and neutralized with NH₄HCO₃, extracted with i-PrOH/CHCl₃ (1:3, v/v, 50 mL x 3). The organic phase was washed with saturated NaCl (20 mL) and dried with Na₂SO₄. The mixture was concentrated under under vacuum to afford crude product, the crude product was purified by silica gel column (DCM/MeOH = 10:1) to afford title product (2.9 g , Y: 68.3%) as white solid. ESI-MS (M+H)⁺: 217.1. ¹H NMR (400 MHz, DMSO-d₆) δ 7.98 (s, 1H),5.97 (s, 2H), 3.71 (s, 3H), 1.99 (s, 3H).

### Step 4: Preparation of 6-bromo-7-methoxy-2,8-dimethylimidazo[1,2-a]pyridine.

To a stirred mixture of 5-bromo-4-methoxy-3-methylpyridin-2-amine (2.0 g, 9.21 mmol) in EtOH (10 mL) was added 1-bromopropan-2-one (2.52 g, 18.42 mmol) by portion at room temperature, the mixture was stirred for 8 h at 80 °C in a sealed tube. The mixture was quenched with H₂O (10 mL) and neutralized with Na₂CO₃, extracted with EtOAc (50 mL x 3). The organic phase was washed with saturated NaCl (20 mL) and dried with Na₂SO₄. After concentration, the crude product was purified by silica gel column (DCM/MeOH= 10:1) to afford title product (1.3 g , Y: 55.3%) as white solid. ESI-MS (M+H)⁺: 255.0.

### Step 5: Preparation of methyl 7-methoxy-2,8-dimethylimidazo[1,2-a]pyridine-6-carboxylate.

To a solution of 6-bromo-7-methoxy-2,8-dimethylimidazo[1,2-a]pyridine (500 mg, 1.96 mmol) in MeOH (10 mL) were added TEA (595 mg, 5.88 mmol) and Pd(dppf)Cl₂ (143.28 mg, 0.196 mmol) at room temperature under CO atmosphere (balloon). The mixture was stirred for 4 h at 90 °C. After concentration, the residue was purified by silica gel column (DCM/MeOH = 10:1) to afford title product (245 mg , Y: 53.3%) as light yellow solid. ESI-MS (M+H)⁺: 235.1.

### Step 6: Preparation of 7-methoxy-2,8-dimethylimidazo[1,2-a]pyridine-6-carboxylic acid.

To a solution of compound methyl 7-methoxy-2,8-dimethylimidazo[1,2-a]pyridine-6- carboxylate (200 mg, 0.85 mmol) in THF (4.0 mL) were added H₂O (1 mL) and LiOH.H2O (59 mg, 2.46 mmol). The reaction mixture was stirred at room temperature for 1 h. The organic solvent was concentrated under reduced pressure and extracted with CH₂Cl₂ (50 mL×3). Then the aqueous solution was acidified to pH = 2 with 1M HCl. Then the aqueous solution was extracted with EA (50 mLx3). The combined organic layer was washed with water and brine, dried over anhydrous Na₂SO₄, filtered and concentrated under reduced pressure to give title product (100 mg, yield: 53.4%) as a yellow solid. ESI-MS (M+H)⁺: 221.0.

### Example 7. 6-methoxy-2-methyl-2H-indazole-5-carboxylic acid

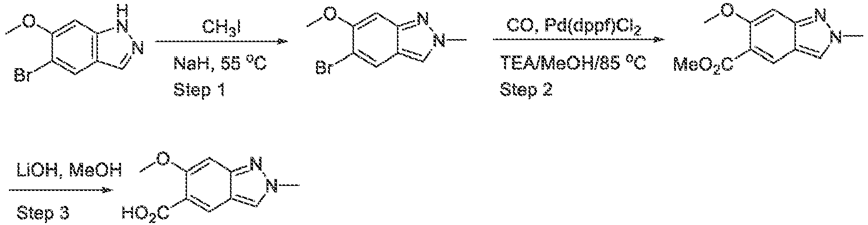

### Step 1: Preparation of 5-bromo-6-metlioxy-2-methyl-2H-indazole.

To a solution of 5-bromo-6-methoxy-1H-indazole (3.2 g, 14.1 mmol) in anhydrous THF (30 mL) was added sodium hydride (1.13 g, 28.2 mmol) and the mixture was stirred under nitrogen for 0.5 h. Then iodomethane(4 g, 28.2 mmol) was added and the resulting mixture was stirred at 55 °C for 3 h. The mixture was carefully diluted with water and extracted with EA. The organic phase was washed with brine (50 mL), dried over Na₂SO₄ and concentrated to dryness. The crude was purified by column chromatography (EA:PE=2: 1, v/v) to give title product (780 mg, Y: 23%) as a yellow solid. ¹H NMR (400 MHz, CDCl₃) δ 7.86 (s, 1H), 7.77 (s, 1H), 7.02 (s, 1H),4.17 (s, 3H), 3.95 (s, 3H). ESI-MS (M+H)⁺: 240.8, MS (M+2+H)⁺: 242.8.

### Step 2: Preparation of methyl 6-methoxy-2-methyl-2H-indazole-5-carboxylate.

A mixture of 5-bromo-6-methoxy-2-methyl-2H-indazole (1.10 g, 4.60 mmol), TEA (1.39 g, 13.8 mmol) and Pd(dppf)Cl₂ (341 mg, 0.46 mmol) in MeOH (30 mL) was purged with carbon monoxide for three times at rt. The mixture was stirred for at 85 °C under carbon monoxide overnight. The catalyst was filtered off and the filtrate was concentrated to give crude product, which was purfied by column chromatography (EA: PE=4: 1, v/v) to give methyl 6-methoxy-2-methyl-2H-indazole-5-carboxylate (770 mg, Y: 76%) as an orange solid. ESI-MS (M+H)⁺: 221.

### Step 3: Preparation of 6-methoxy-2-methyl-2H-indazole-5-carboxylic acid.

To a mixture of methyl 6-methoxy-2-methyl-2*H*-indazole-5-carboxylate (770 mg, 3.21 mmol) in MeOH (10 mL) and H₂O (5 mL) was added LiOH.H₂O (1.28 g, 32.10 mmol). The reaction mixture was stirred for 2 h at room temperature. After concentration, the residue was diluted with water and adjusted to pH = 6 with 1M HCl. The precipitate was collected by filtration and dried under vacuum to give 6-methoxy-2-methyl-2H-indazole-5-carboxylic acid (450 mg, crude) as a yellow solid, which was used to next step without further purification. ESI-MS (M+H)⁺: 207.1.

### Example 8. tert-butyl 4-(6-aminopyridazin-3-yl)piperazine-1-carboxylate.

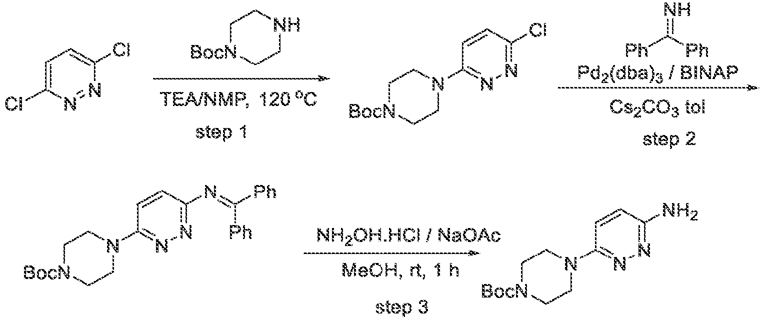

### Step 1: Preparation of tert-butyl 4-(6-chloropyridazin-3-yl)pipermine-1-carboxylate

To a suspension of 3,6-dichloropyridazine (1.0 g, 6.75 mmol) and tert-butyl piperazine-1-carboxylate (1.3 g, 6.75 mmol) in NMP (20 mL) was added TEA (2.0 g, 20.2 mmol), and the reaction mixture was stirred for 2 h at 120 °C. After cooling to rt, the mixture was diluted with water 150 mL and 150 mL of EA. The EA layer was separated, washed with brine and water, dried over Na₂SO₄ and concentrated to dryness. The crude was purified by silica gel column (10~50% EA in PE). The product was obtained as off-white solid (1.2 g, Y: 60%). ¹H NMR (400 MHz, CDCl₃) δ 6.96 (d, *J* = 9.6 Hz, 1H), 6.77 (d*, J =* 9.6 Hz, 1H), 3.55 (dd, *J =* 6.3, 4.0 Hz, 4H), 3.42 (d, *J =* 5.2 Hz, 4H), 1.48 (s, 9H).

### Step 2: Preparation of tert-butyl 4-(6-((diphenylmethylene)amino)pyridazin-3-yl)piperazine-1-carboxylate

A mixture of tert-butyl 4-(6-chloropyridazin-3-yl)piperazine-1-carboxylate (1.5 g, 5.01 mmol), diphenylmethanimine (1.4 g, 7.55 mmol), Pd₂(dba)₃ (289 mg, 0.25 mmol), BINAP (144 mg, 0.25 mmol) and Cs₂CO₃ (489 mg, 15.0 mmol) in toluene (15 mL) was purged with N₂ for three times at room temperature. Then the reaction mixture was stirred at 100 °C for 16 h. After cooling to rt, the mixture was diluted with water (150 mL) and EA (150 mL). The EA layer was separated, washed with brine and water, dried over Na₂SO₄, concentrated to dryness. The crude was purified by silica gel column (10~50% EA in PE). The product was obtained as off-white solid (1.25 g, yield: 56 %.) ESI-MS: [M+H] ⁺ 480.1

### Step 3: Preparation of tert-butyl 4-(6-aminopyridazin-3-yl)piperazine-1-carboxylate

A mixture of tert-butyl 4-(6-((diphenylmethylene)amino)pyridazin-3-yl)piperazine-1-carboxylate (1.2 g, 2.71 mmol), NH₂OH.HCl (378 mg, 5.42 mmol) and NaOAc (1.1 g, 13.5 mmol) in MeOH (15 mL) was stirred for 1 h at rt. After diluting with water, the mixture was extracted with EA (30 mL x 2). The combined organics was washed with brine, dried over Na₂SO₄. After concentration, the crude was used to next step without further purification. ¹H NMR (400 MHz, CDCl₃) δ 7.23 (d, *J* = 9.5 Hz, 1H), 6.90 (d, *J =* 9.5 Hz, 1H), 3.62 (br s, 4H), 3.59 - 3.55 (m, 4H), 1.49 (s, 9H).

### Example 9. 8-fluoro-2-methylimidazo[1,2-a]pyridine-6-carboxylic acid.

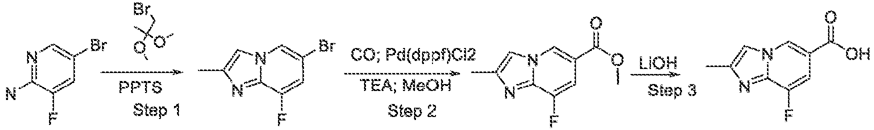

### Step 1: Preparation of 6-bromo-8-fluoro-2-methylimidazo[1,2-a]pyridine HBr.

A mixture of 5-bromo-3-fluoropyridin-2-amine (50 g; 262 mmol), 1-bromo-2,2-dimethoxypropane (57.5 g; 315 mmol) and PPTS (6.6 g; 26.2 mmol) in *i*-PrOH (300 mL) was stirred at 70 °C for 16 h. Next, the reaction mixture was filtered and the obtained solid was rinsed with cold i-PrOH (2 × 100 mL), dried in vacuo to give 6-bromo-8-fluoro-2-methylimidazo[1,2-a]pyridine as HBr salt (35 g; 113 mmol; 43 % yield). ESI-MS: 230.1 (M+H)⁺.

### Step 2: Preparation of methyl 8-fluoro-2-methylimidazo[1,2- a]pyridine-6-carboxylate.

A mixture of 6-bromo-8-fluoro-2-methylimidazo[1,2- a]pyridine HBr (35 g; 113 mmol), Pd(dppf)Cl₂ (9.2 g; 11.3 mmol) and TEA (63 mL; 452 mmol) in MeOH (300 mL) was stirred at 50 °C in autoclave (under CO atmosphere, 10 atm.) for 14 h. Next, the reaction mixture was concentrated in vacuo and purified by column chromatography to afford the title compound (9 g; 43 mmol; 38% yield). ESI-MS: 209.2 (M+H)⁺.

### Step 3: Preparation of 8-fluoro-2-methylimidazo[1,2-a]pyridine-6-carboxylic acid.

Lithium hydroxide monohydrate (2.4 g, 100 mmol) was added to a stirred solution of methyl 8-fluoro-2-methylimidazo[1,2- alpyridine-6-carboxylate (9 g; 43 mmol) in THF/water (100 mL/50 mL) and the obtained solution was stirred at r.t. for 14 h. Next, the reaction mixture was concentrated in vacuo, acidified to pH=6 and extracted with EtOAc. Organics were washed with water, dried with Na₂SO₄ and evaporated to give 8-fluoro-2-methylimidazo[1,2-a]pyridine-6-carboxylic acid (7.2 g; 37 mmol; 86% yield). ESI-MS: 195.2 (M+H)⁺.

### Example 10. N-(2-fluoro-4-(piperazin-1-yl)phenyl)-2-methylimidazo[1,2-a]pyrazine-6-carboxamide HCl salt (Compound 101).

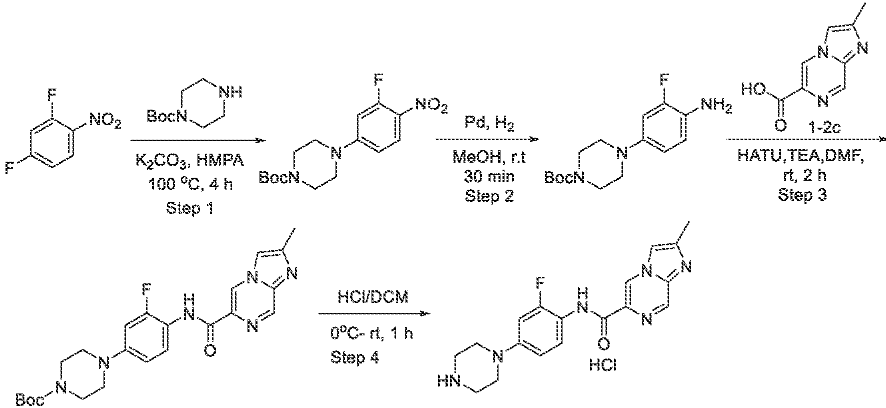

### Step 1: Preparation of tert-butyl 4-(3-fluoro-4-nitrophenyl)piperazine-1-carboxylate.

A mixture of 2,4-difluoro-1-nitrobenzene (100 mg, 0.63 mmol), K₂CO₃ (217 mg, 1.57 mmol) and tert-butyl piperazine-1-carboxylate (98 mg, 0.52 mmol) in HMPA (1 mL) was heated to 100 °C for 4 h. After cooling to rt, the reaction mixture was diluted with ethyl acetate (30 mL) and washed with water (30 mL) and brine (30 mL). The organic layer was dried over Na₂SO₄, concentrated to dryness. The crude was purified by pre-TLC (EA:PE=1:5, v/v) to give title product (140 mg, Y: 82.8%) as a yellow solid. ESI-MS (M+H)⁺: 326.1. ¹H NMR (400 MHz, CDCl₃) δ 8.07 - 8.02 (m, 1H), 6.61 - 6.50 (m, 2H), 3.62 - 3.48 (m, 4H), 3.47 - 3.36 (m, 4H), 1.49 (s, 9H).

### Step 2: Preparation of tert-butyl 4-(4-amino-3-fluorophenyl)piperazine-1-carboxylate.

A mixture of tert-butyl 4-(3-fluoro-4-nitrophenyl)piperazine-1-carboxylate (140 mg, 0.43 mmol) and Pd/C (14 mg, 10% wt/wt) in MeOH (10 mL) was purged with hydrogen for three times at rt. The mixture was stirred for 30 min at rt under hydrogen. The catalyst was filtered off and the filtrate was concentrated to give title product, which was used in next step without further purification. ESI-MS (M+H)⁺: 296.1.

### Step 3: Preparation of tert-butyl 4-(3-fluoro-4-(2-methylimidazo[1,2-a]pyrazine-6-carboxamido)phenyl)piperazine-1-carboxylate.

To a stirred solution of 2-methylimidazo[1,2-a]pyrazine-6-carboxylic acid (100 mg, 0.56 mmol), tert-butyl 4-(4-amino-3-fluorophenyl)piperazine-1-carboxylate (110 mg, 0.373 mmol) and DIPEA (240 mg, 1.865 mmol) in DMF (6 mL) was added HATU (284 mg, 0.746 mmol) at rt. After addition was completed, the mixture was stirred for 2 h. The reaction mixture was treated with EA/Water (100 mL, 1:1). The organic phase was separated and the aqueous phase was extracted with EA (50 mLx1). The combined organic layers was washed with brine and dried over sodium sulfate. After concentration under reduced pressure, the crude was sluury with EA/PE (1:10, v/v, 5 mL) for 30 min at rt. Then the solid was collected by filtration and washed with EA/PE (1:10, v/v, 3 mL x 3). The product was obtained (64 mg, Y: 37.9%) as a light brown solid, which was used to next step without further purification. ESI-MS (M+H)⁺: 455.1.

### Step 4: Preparation of N-(2-fluoro-4-(piperazin-1-yl)phenyl)-2-methylimidazo[1,2-a]pyrazine-6-carboxamide HCl salt.

To a solution of tert-butyl 4-(3-fluoro-4-(2-methylimidazo[1,2-a]pyrazine-6-carboxamido)phenyl)piperazine-1-carboxylate (64 mg, 0.141 mmol) in DCM (5 mL) was added HCl-dioxane (4 M, 0.36 mL, 1.41 mmol) at 0 °C and the mixture was allowed to warm to rt and stirred for 1 h. After concentration, the residue was treated with DCM (10 mL) and EA (10 mL). The solid was collected by filtration and washed EA three times (3 mL x 3). The solid was dried under vacuum at 55 °C to provide title product as light grey solid (35.3 mg, yield: 60%). ESI-MS (M+H)⁺: 355.1. ¹H NMR (400 MHz, DMSO-d₆) δ 10.20 (s, 1H), 9.47 (s, 3H), 9.19 (s, 1H), 8.24 (s, 1H), 7.80 - 7.74 (m, 1H), 7.01 (dd, *J =* 13.9, 2.3 Hz, 1H), 6.87 (d, *J* = 8.7 Hz, 1H), 3.48 - 3.42 (m, 4H), 3.20 (s, 4H), 2.53 (s, 3H).

### Example 11. 2-methyl-N-(4-(piperazin-1-yl)phenyl)imidazo|1,2-a]pyrazine-6-carboxamide (Compound 102).

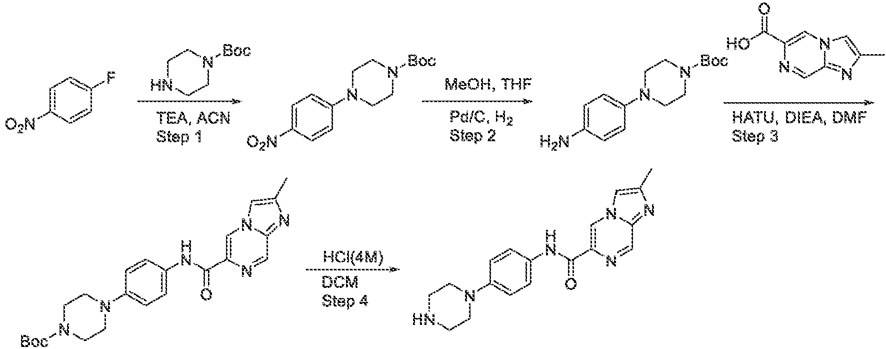

### Step 1: Preparation of tert-butyl 4-(4-nitrophenyl)piperazine-1-carboxylate.

A mixture of 1-fluoro-4-nitrobenzene (500 mg, 3.55 mmol), tert-butyl piperazine-1-carboxylate (989 mg, 5.32 mmol) and triethyl amine (1.1 g, 10.65 mmol) in MeCN (10 mL) was stirred at 80 °C for 4 h. After cooling to rt, the mixture was concentrated. The crude was purified by silica gel column (PE:EA=10:1,) to give title product (490 mg, Y: 45%) as a yellow solid. ESI-MS (M+H)⁺: 308.2.

### Step 2: Preparation of tert-butyl 4-(4-aminophenyl)piperazine-1-carboxylate.

A mixture of tert-butyl 4-(4-nitrophenyl)piperazine-1-carboxylate (490 mg, 1.6 mmol) and Pd/C (50 mg, 10% wt/wt) in MeOH (10 mL)/THF (2 mL) was purged with hydrogen for three times at rt. The mixture was stirred for 1 h at rt under hydrogen. The catalyst was filtered off and the filtrate was concentrated to give title product (400 mg, Y: 90%), which was used in next step without further purification. ESI-MS (M+H)⁺: 278.1. ¹H NMR (400 MHz, DMSO-d₆) δ 6.70 (d, *J =* 8.8 Hz, 2H), 6.49 (d, *J =* 8.8 Hz, 2H), 4.60 (s, 2H), 3.46 - 3.38 (m, 4H), 2.87 - 2.78 (m, 4H), 1.41 (s, 9H).

### Step 3: Preparation of tert-butyl 4-(4-(2-methylimidazo[1,2-a]pyrazine-6-carboxamido)phenyl)piperazine-1-carboxylate.

To a stirred solution of 2-methylimidazo[1,2-a]pyrazine-6-carboxylic acid (150 mg, 0.85 mmol), tert-butyl 4-(4-aminophenyl)piperazine-1-carboxylate (196 mg, 0.71 mmol) and DIPEA (275 mg, 2.13 mmol) in DMF (3 mL) was added HATU (405 mg, 1.07 mmol) at rt. After addition was completed, the mixture was stirred for 1 h. After diluting with water, the precipitate was collected by filtration. The solid was washed with MeCN and dried to give title proudct (160 mg, Y: 52%) as a yellow solid. ESI-MS (M+H)⁺: 437.2. ¹H NMR (400 MHz, DMSO-d₆) δ 10.35 (s, 1H), 9.29 (s, 1H), 8.99 (s, 1H), 8.09 (s, 1H), 7.76 (d, *J =* 7.7 Hz, 2H), 6.96 (d, *J* = 7.8 Hz, 2H), 3.46 (br s, 4H), 3.07 (br s, 4H), 2.46 (s, 3H), 1.42 (s, 9H).

### Step 4: Preparation of 2-methyl-N-(4-(piperazin-1-yl)phenyl)imidazo[1,2-a]pyrazine-6-carboxamide hydrochloride.

To a solution of tert-butyl 4-(4-(2-methylimidazo[1,2-a]pyrazine-6-carboxamido)phenyl)piperazine-1-carboxylate (80 mg, 0.18 mmol) in DCM (2 mL) was added HCl-dioxane (4 M, 0.5 mL, 2 mmol) at 0 °C and the mixture was allowed to warm to rt and stirred for 2 h. After concentration, the residue was treated with EA (5 mL). The solid was collected by filtration and washed EA three times (5 mL). The solid was dried under vacuum to provide title product as yellow solid (60 mg, yield: 90%). ESI-MS (M+H)⁺: 337.1. ¹H NMR (400 MHz, DMSO-d₆) δ 10.55 (s, 1H), 9.47 (s, 1H), 9.37 (br s, 2H), 9.19 (s, 1H), 8.26 (s, 1H), 7.81 (d, *J =* 9.1 Hz, 2H), 7.05 (d, *J* = 9.1 Hz, 2H), 3.45 - 3.33 (m, 4H), 3.24 - 3.21 (m, 4H), 2.54 (s, 3H).

### Example 12. 2-methyl-N-(6-(piperazin-1-yl)pyridin-3-yl)imidazo|1,2-a]pyrazine-6-carboxamide (Compound 103).

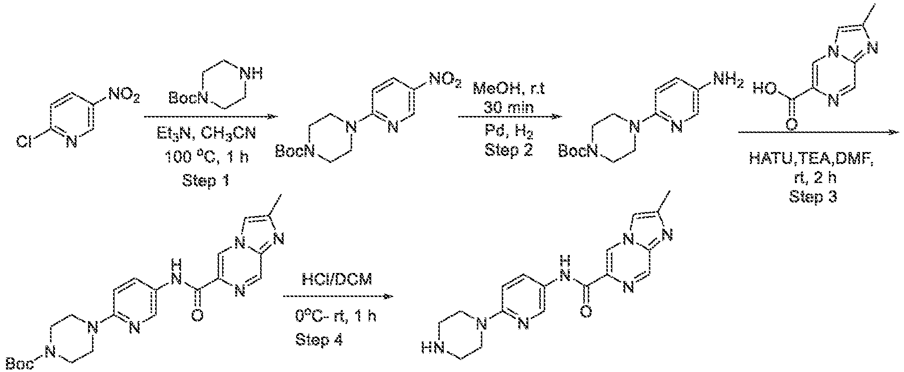

### Step 1: Preparation of tert-butyl 4-(5-nitropyridin-2-yl)piperazine-1-carboxylate.

A mixture of 2-chloro-5-nitropyridine (1.0 g, 6.3 mmol), Et₃N (1.92 g, 18.9 mmol) and tert-butyl piperazine-1-carboxylate (2.35 g, 12.6 mmol) in CH₃CN (10 mL) was stirred at 100 °C for 1 h. After cooing to rt, the reaction mixture was diluted with ethyl acetate (30 mL) and washed with water once (30 mL), brine (30 mL). The organic layer was dried over Na₂SO₄, concentrated to dryness. The crude was purified by silica gel column (EA:PE=1:5, v/v) to give title produt (1.8 g, Y: 92%) as a yellow solid. ESI-MS (M+H)⁺: 309.4.

### Step 2: Preparation of tert-butyl 4-(5-aminopyridin-2-yl)piperazine-1-carboxylate.

A mixture of tert-butyl 4-(5-nitropyridin-2-yl)piperazine-1-carboxylate (1.0 g, 3.2 mmol) and Pd/C (100 mg, 10% wt/wt) in MeOH (10 mL) was purged with nitrogen for three times and then hydrogen for three times at rt. The mixture was stirred for 30 min at rt under hydrogen. The catalyst was filtered off and the filtrate was concentrated to provide crude title product, which was used to next step without further purification. ESI-MS (M+H)⁺: 279.0.

### Step 3: Preparation of tert-butyl 4-(5-(2-methylimidazo[1,2-a]pyrazine-6-carboxamido) pyridin-2-yl)piperazine-1-carboxylate.

To a stirred solution of 2-methylimidazo[1,2-a]pyrazine-6-carboxylic acid (76 mg, 0.43 mmol), tert-butyl 4-(5-aminopyridin-2-yl)piperazine-1-carboxylate (100 mg, 0.36 mmol) and DIPEA (138 mg, 1.07 mmol) in DMF (5 mL) was added HATU (204 mg, 0.54 mmol) at rt.The mixture was stirred at rt for 2 hrs. The reaction mixture was treated with EA/Water (50 mL, 1:1). The organic phase was separated and the aqueous phase was extracted with EA (50 mLx1). The combined organic layers was dried over sodium sulfate. After concentration, the crude was pyrified by silica gel column (DCM/MeOH = 10:1) to provide title product (46 mg, Y: 24%) as black solid. ESI-MS (M+H)⁺: 438.2. ¹H NMR (400 MHz, DMSO-d₆) δ 10.51 (s, 1H), 9.30 (s, 1H), 8.99 (s, 1H), 8.60 (s, 1H), 8.04-8.09 (m, 2H), 6.89 (d, *J =* 8.0 Hz, 1H), 3.44 (s, 8H), 2.46 (s, 3H), 1.43 (s, 9H).

### Step 4: Preparation of 2-methyl-N-(6-(piperazin-1-yl)pyridin-3-yl)imidazo[1,2-a]pyrazine-6-carboxamide hydrogen chloride.

To a solution of tert-butyl 4-(5-(2-methylimidazo[1,2-a]pyrazine-6-carboxamido)pyridin-2-yl)piperazine-1-carboxylate (26 mg, 0.059 mmol) in DCM (5 mL) was added HCl-dioxane (4 M, 0.50 mL, 2mmol) at 0 °C and warmed to rt for 1 h. After concentration, the residue was treated with DCM (5 mL x 1)and EA (5 mL x 1). The solid was collected by filtration and washed with EA three times (3 mL x 3). The solid was dried under vacuum at 55 °C to provide title product as light grey solid (8.6 mg, yield: 39%). ESI-MS (M+H)⁺: 338.1. ¹H NMR (400 MHz, DMSO-d₆) δ 10.70 (s, 1H), 9.38 - 9.36 (m, 3H), 9.03 (s, 1H), 8.69 (s, 1H), 8.22-8.13 (m, 2H), 7.08-7.10 (d, *J =* 8.0 Hz, 1H), 3.69 - 3.64 (m, 4H), 3.19 (s, 4H), 2.47 (s, 3H).

### Example 13. (R)-N-(2-fluoro-4-(3-methylpiperazin-1-yl)phenyl)-2-methylimidazo[1,2-a]pyrazine-6-carboxamide hydrochloride (Compound 104).

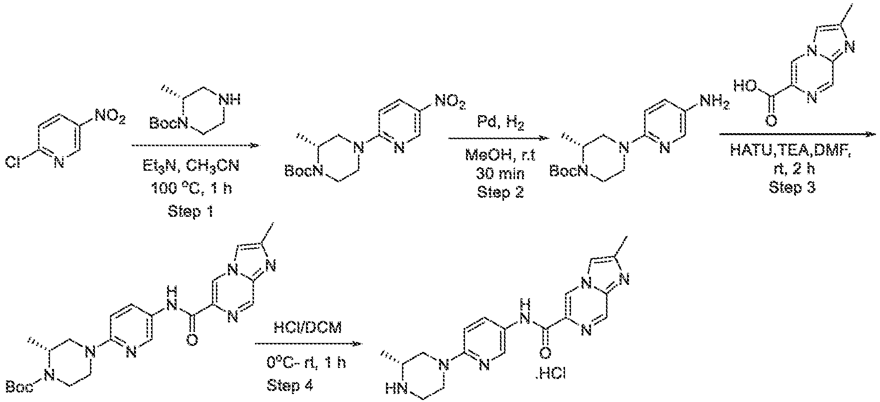

### Step 1: Preparation of tert-butyl (R)-2-methyl-4-(5-nitropyridin-2-yl)piperazine-1-carboxylate.

A mixture of 2-chloro-5-nitropyridine (0.30 g, 1.90 mmol), Et₃N (0.60 g, 5.93 mmol) and tert-butyl (R)-2-methylpiperazine-1-carboxylate (0.46 g, 2.30 mmol) in CH₃CN (10 mL) was stirred 100 °C for 1 h. After cooling to rt, the reaction mixture was diluted with ethyl acetate (100 mL) and washed with water (30 mL), brine (30 mL). The organic layer was dried over Na₂SO₄, concentrated to dryness. The crude was purified by silica gel column (EA:PE=1:5, v/v) to give the title compound (500 mg, Y: 81%) as a yellow solid. ESI-MS (M-56+H)⁺:267.0.

### Step 2: Preparation of tert-butyl (R)-4-(5-aminopyridin-2-yl)-2-methylpiperazine-1-carboxylate.

A mixture of tert-butyl (R)-2-methyl-4-(5-nitropyridin-2-yl)piperazine-1-carboxylate (0.2 g, 0.62 mmol) and Pd/C (20 mg, 10% wt/wt) in MeOH (10 mL) was purged with nitrogen for three times and then hydrogen for three times at rt. The mixture was stirred for 30 min at rt under hydrogen. The solid was filtered off. The mother liquor was concentrated under vacuum to provide title product (180 mg, Y: 99%) as a purple solid, which was used to next step without further purification. ESI-MS (M+H)⁺: 293.4.

### Step 3: Preparation of tert-butyl (R)-2-methyl-4-(5-(2-methylimidazo[1,2-a]pyrazine-6-carboxamido)pyridin-2-yl)piperazine-1-carboxylate.

To a stirred solution of 2-methylimidazo[1,2-a]pyrazine-6-carboxylic acid (73 mg, 0.41 mmol), tert-butyl (R)-4-(5-aminopyridin-2-yl)-2-methylpiperazine-1-carboxylate (100 mg, 0.34 mmol) and DIPEA (133 mg, 1.03 mmol) in DMF (6 mL) was added HATU (195 mg, 0.51 mmol) at rt. The mixture was stirred at rt for 2 hrs. The reaction mixture was treated with EA/Water (80 mL, 1:1). The organic phase was separated and the aqueous phase was extracted with EA (50 mLx1). The combined organic layers was dried over sodium sulfate. After concentration under reduced pressure, the crude was sluried with EA/PE (1:10, v/v, 5 mL) for 30 min at rt. Then the solid was collected by filtration and washed with EA/PE (1:10, v/v, 3 mL x 3). The product was obtained (30 mg, Y: 19.5%) as a light brown solid, which was used to next step without further purification. ESI-MS (M+H)⁺: 452.4.

### Step 4: Preparation of (R)-N-(2-fluoro-4-(3-methylpiperazin-1-yl)phenyl)-2-methylimidazo[1,2-a]pyrazine-6-carboxamide hydrochloride.

To a solution of tert-butyl (R)-2-methyl-4-(5-(2-methylimidazo[1,2-a]pyrazine-6-carboxamido)pyridin-2-yl)piperazine-1-carboxylate (30 mg, 0.067 mmol) in DCM (5 mL) was added HCl-dioxane (4 M, 0.50 mL, 2mmol) at 0 °C and the mixture was allowed to warm to it and stirred for 1 h . After concentration, the residue was treated with DCM (5 mL x 1) and EA (5 mL x 1). The solid was collected by filtration and washed with EA three times (3 mL x 3). The solid was dried uner vacuum at 55 °C to provide title product as light grey solid (8.2 mg, yield: 32%). ESI-MS (M+H)⁺: 352.2. ¹H NMR (400 MHz, CD₃OD) δ 9.49 (s, 1H), 9.32 (s, 1H), 8.85 (s, 1H), 8.41 (d, *J* = 8.0 Hz, 1H), 8.28 (s, 1H), 7.46 (d, *J* = 8.0 Hz, 1H), 4.35-4.32 (m, 2H), 3.49-3.63 (m, 4H), 3.33-3.39 (m, 1H), 2.66 (s, 3H), 1.45 (d, *J* = 8.0 Hz, 3H).

### Example 14. (R)-N-(2-fluoro-4-(3-methylpiperazin-1-yl)phenyl)-2-methylimidazo[1,2a]pyrazine-6-carboxamide hydrochloride (Compound 105).

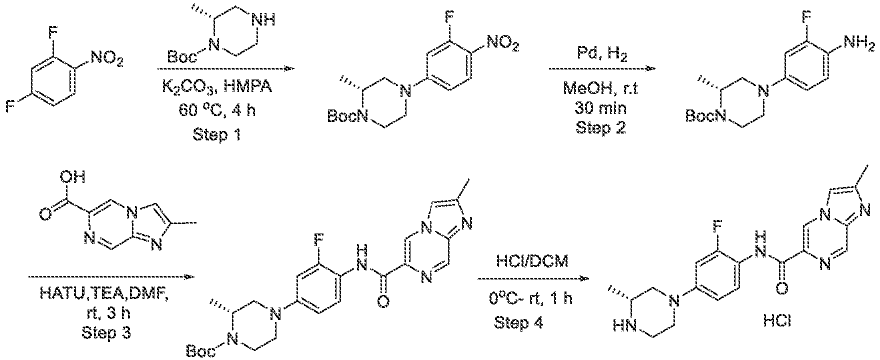

### Step 1: Preparation of tert-butyl (R)-4-(3-fluoro-4-nitrophenyl)-2-methylpiperazine-1-carboxylate.

A mixture of 2,4-difluoro-1-nitrobenzene (318 mg, 2.0 mmol), tert-butyl (R)-2-methylpiperazine-1-carboxylate (400 mg, 2.0 mmol) and potassium carbonate (276 mg, 2.0 mmol) in HMPA (5 mL) was stirred at 60 °C for 4 h. After cooling to rt, the reaction mixture was diluted with ethyl acetate and washed with water and brine. The organic layer was dried over Na₂SO₄, concentrated to dryness. The crude was purified by pre-TLC (EA:PE=1:10, v/v) to give title product (200 mg, Y: 29%) as a brown oil. ESI-MS (M-56+H)⁺: 284.0. ¹H NMR (400 MHz, CDCl₃) δ 8.04 *(*t*, J =* 9.1 Hz, 1H), 6.60 - 6.42 (m, 2H), 4.34 (br s, 1H), 3.98 - 3.89 (m, 1H), 3.75 - 3.67 (m, 1H), 3.61 - 3.54 (m, 1H), 3.44 - 3.32 (m, 2H), 3.22 - 3.12 (m, 1H), 1.49 (s, 9H), 1.22 (d, *J =* 6.7 Hz, 3H).

### Step 2: Preparation of tert-butyl (R)-4-(4-amino-3-fluorophenyl)-2-methylpiperazine-1-carboxylate.

A mixture of tert-butyl (R)-4-(3-fluoro-4-nitrophenyl)-2-methylpiperazine- 1-carboxylate (220 mg, 0.65 mmol) and Pd/C (25 mg,) in MeOH (3 mL) was purged with nitrogen for three times and then hydrogen for three times at rt. The mixture was stirred for 30 min at rt under hydrogen. LCMS showed the starting material was consumed completely and all of starting material was converted to desired product. The reaction was stopped and the solid was filtered off. The mother liquor was removed under vacuum. The residue title compound was used in next step without further purification. ESI-MS (M+H)⁺: 310.4.

### Step 3: Preparation of tert-butyl (R)-4-(3-fluoro-4-(2-methylimidazo[1,2-a]pyrazine-6-carboxamido)phenyl)-2-methylpiperazine-1-carboxylate.

To a stirred solution of tert-butyl (R)-4-(4-amino-3-fluorophenyl)-2-methylpiperazine-1-carboxylate (100mg, 0.32 mmol) in DMF (3 mL) was added 2-methylimidazol[1,2-a]pyrazine-6-carboxylic acid (63 mg, 0.36 mmol), DIEA (120 mg, 0.97 mmol) and HATU (185 mg, 0.49 mmol). The mixture was stirred for 3 h at rt. Water was added and the precipitate was collected by filteration. The filter cake was washed with water (5 mL×3) and dried under vacuum to provide the title compound (96 mg, Y: 62%) as a yellow solid, which was used to next step without further purification. ESI-MS (M+H)⁺: 469.2. ¹H NMR (400 MHz, CDCl₃) δ 9.88 (s, 1H), 9.01 - 8.89 (m, 2H), 8.30 (t, *J* = 8.9 Hz, 1H), 7.62 (s, 1H), 6.76 - 6.62 (m, 2H), 4.35 (br s, 1H), 3.95 (d, *J* = 13.2 Hz, 1H), 3.53 - 3.45 (m, 1H), 3.41 - 3.21 (m, 2H), 3.01 - 2.94 (m, 1H), 2.82 - 2.73 (m, 1H), 2.57 (s, 3H), 1.49 (s, 9H), 1.30 (d, *J* = 6.7 Hz, 3H).

### Step 4: Preparation of (R)-N-(2-fluoro-4-(3-methylpiperazin-1-yl)phenyl)-2-methylimidazo[1,2-a]pyrazine-6-carboxamide hydrochloride.

To a solution of tert-butyl (R)-4-(3-fluoro-4-(2-methylimidazo[1,2-a]pyrazine-6-carboxamido)phenyl)-2-methylpiperazine-1-carboxylate (100 mg, 0.213 mmol) in DCM (3 mL) was added HCl solution in 1.4-dioxane (0.55 mL, 4 mmoL, 4M) at 0 °C. The the mixture was stirred at rt for 1.5 h. The mixture was filtered and the filtrate was concentrated under reduce pressure, the residue was washed by EA (5 mL) and PE (5 mL) to give title product (68 mg, Y:87%) as a yellow solid. ESI-MS (M+H)⁺: 369.1. ¹H NMR (400 MHz, DMSO-d₆) δ 10.13 (s, 1H), 9.42 (br s, 1H), 9.37 (d, *J =* 1.2 Hz, 1H), 9.16 (br s, 1H), 9.08 (s, 1H), 8.15 (s, 1H), 7.81 (t, *J* = 9.0 Hz, 1H), 7.03 (dd, *J* = 14.0, 2.5 Hz, 1H), 6.88 (dd, *J =* 8.9, 2.3 Hz, 1H), 3.90 - 3.76 (m, 2H), 3.40 - 3.29 (m, 2H), 3.11 - 3.00 (m, 2H), 2.84 - 2.80 (m, 1H), 2.49 (s, 3H), 1.31 (d, *J* = 6.5 Hz, 3H).

### Example 15. N-(4-((1R,5S)-3,8-diazabicyclo[3.2.1]octan-3-yl)-2-fluorophenyl)-2-methylimidazo[1,2-a]pyrazine-6-carboxamide (Compound 106).

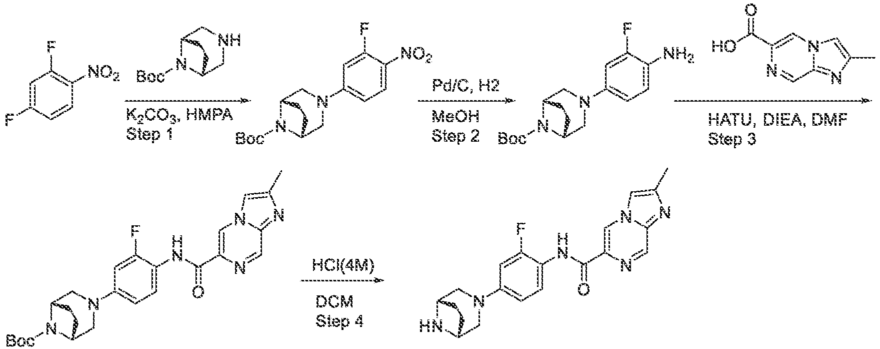

### Step 1: Preparation of tert-butyl (1R,5S)-3-(3-fluoro-4-nitrophenyl)-3,8-diazabicyclo[3.2.1]octane-8-carboxylate.

A mixture of 2,4-difluoro-1-nitrobenzene (100 mg, 0.63 mmol), tert-butyl (1R,5S)-3,8-diazabicyclo[3.2.1]octane-8-carboxylate (133 mg, 0.63 mmol) and potassium carbonate (260 mg, 1.89 mmol) in HMPA (1 mL) was stirred at 60 °C for 2 h. After cooling to rt, the reaction mixture was diluted with ethyl acetate and washed with water and brine. The organic layer was dried over Na₂SO₄, concentrated to dryness. The crude was purified by pre-TLC (EA:PE=1:10, v/v) to give title product (70 mg, Y: 32%) as a yellow solid. ESI-MS (M+H)⁺: 352.1. ¹H NMR (400 MHz, CDCl₃) δ 8.04 (t, *J* = 9.2 Hz, 1H), 6.58 - 6.45 (m, 2H), 4.42 (s, 2H), 3.57 - 3.38 (m, 2H), 3.20 (d, *J =* 9.0 Hz, 2H), 2.08 - 1.98 (m, 2H), 1.81 - 1.75 (m, 2H), 1.48 (s, 9H).

### Step 2: Preparation of tert-butyl (1R,5S)-3-(4-amino-3-fluorophenyl)-3,8-diazabicyclo[3.2.1]octane-8-carboxylate.

A mixture of tert-butyl (1R,5S)-3-(3-fluoro-4-nitrophenyl)-3,8-diazabicyclo[3.2.1]octane-8-carboxylate (70 mg, 0.19 mmol) and Pd/C (10 mg) in MeOH (10 mL) was purged with hydrogen for three times at rt. The mixture was stirred for 1 h at rt under hydrogen. The catalyst was filtered off and the filtrate was concentrated to give title product (60 mg, Y: 94%) as a pink solid, which was used in next step without further purification. ESI-MS (M+H)⁺: 322.0.

### Step 3: Preparation of tert-butyl (1R,5S)-3-(3-fluoro-4-(2-methylimidazo[1,2-a]pyrazine-6-carboxamido)phenyl)-3,8-diazabicyclo[3.2.1]octane-8-carboxylate.

To a stirred solution of 2-methylimidazo[1,2-a]pyrazine-6-carboxylic acid (41 mg, 0.23 mmol), tert-butyl (1R,5S)-3-(4-amino-3-fluorophenyl)-3,8-diazabicyclo[3.2.1]octane-8-carboxylate (50 mg, 0.16 mmol) and DIPEA (60 mg, 0.46 mmol) in DMF (2 mL) was added HATU (89 mg, 0.23 mmol) at rt. After addition was completed, the mixture was stirred for 1 h. After diluting with water, the precipitate was collected by filtration. The solid was washed with PE/EA (10:1) and dried to give title proudct (66 mg, Y: 74%) as a yellow solid. ESI-MS (M+H)⁺: 481.2.

### Step 4: Preparation of N-(4-((1R,5S)-3,8-diazabicyclo[3.2.1]octan-3-yl)-2-fluorophenyl)-2-methylimidazo[1,2-a]pyrazine-6-caboxamide hydrochloride.

To a solution of tert-butyl (1R,5S)-3-(3-fluoro-4-(2-methylimidazo[1,2-a]pyrazine-6-carboxamido)phenyl)-3,8-diazabicyclo[3.2.1]octane-8-carboxylate (66 mg, 0.14 mmol) in DCM (10 mL) was added HCl-dioxane (4 M, 0.5 mL, 2 mmol) at 0 °C and the mixture was allowed to warm to rt and stirred for 2 h. After concentration, the residue was treated with EA. The solid was collected by filtration and washed EA. The solid was dried under vacuum to provide title product as yellow solid (16 mg, yield: 27%). ESI-MS (M+H)⁺: 381.0. ¹H NMR (400 MHz, DMSO-d₆) δ 10.11 (s, 1H), 9.42 - 9.27 (m, 3H), 9.06 (s, 1H), 8.13 (s, 1H), 7.84 - 7.72 (m, 1H), 6.99 - 6.86 (m, 1H), 6.82 - 6.73 (m, 1H), 4.14 (s, 2H), 3.71 - 3.59 (m, 2H), 3.17 - 3.08 (m, 2H), 2.48 (s, 3H), 2.03 - 1.90 (m, 4H).

### Example 16. (S)-8-fluoro-2-methyl-N-(5-(3-methylpiperazin-1-yl)pyridin-2-yl)imidazo[1,2-a]pyridine-6-carboxamide TFA salt (Compound 107).

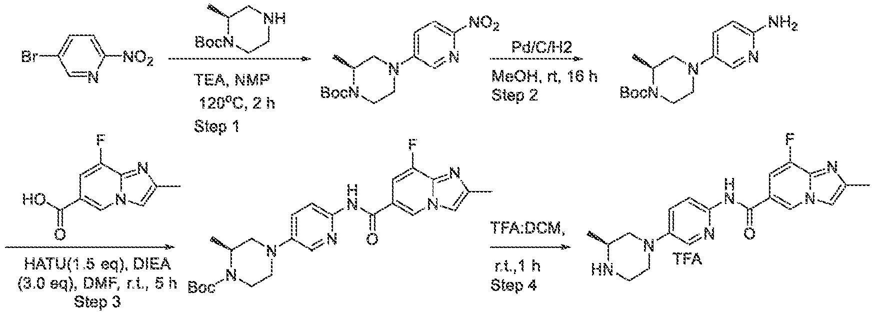

### Step 1: Preparation of tert-butyl (S)-2-methyl-4-(6-nitropyridin-3-yl)piperazine- 1-carboxylate.

To a suspension of 5-bromo-2-nitropyridine (1 g, 4.93 mmol) and tert-butyl (S)-2-methylpiperazine-1-carboxylate (1.1 g, 5.42 mmol) in NMP (15 mL) was added TEA (1.5 g, 14.8 mmol), and the reaction mixture was stirred for 6 h at 120 °C. After cooling to rt and diluting with water, the mixture was extracted with EA. The combined organics were washed with brine and water, dried over Na2SO4 and concentrated to dryness. The crude was purified by silica gel column (PE/EA = 3:1). The product was obtained as yellow solid (1 g, Y: 63 %). ESI-MS (M+H)⁺: 323.0.

### Step 2: Preparation of tert-butyl (S)-4-(6-aminopyridin-3-yl)-2-methylpiperazine-1-carboxylate.

To a mixture of tert-butyl (S)-2-methyl-4-(6-nitropyridin-3-yl)piperazine-1-carboxylate (1 g, 3.11 mmol) in MeOH (10 mL) was added Pd/C (100 mg). The reaction mixture was stirred for 1 h at rt under hydrogen atmosphere (balloon pressure). The solvent was filtered and the filter cake was washed with MeOH (50 mL). The filtrate was concentrated to give desired product as purple semi-solid (750 mg, yield: 83 %), which was used to next step without further purification. ESI-MS (M+H)⁺: 293.1. ¹H NMR (400 MHz, DMSO-d₆) δ 7.60 (d, *J =* 2.8 Hz, 1H), 7.18 - 7.10 (m, 1H), 6.41 (d, *J =* 8.8 Hz, 1H), 5.45 (s, 2H), 4.18 (br s, 1H), 3.81 - 3.72 (m, 1H), 3.27 - 3.20 (m, 1H), 3.15 - 3.07 (m, 2H), 2.66 - 2.60 (m, 1H), 2.49 - 2.40 (m, 1H), 1.42 (s, 9H), 1.22 (d, *J =* 6.7 Hz, 3H).

### Step 3: Preparation of tert-butyl (S)-4-(6-(8-fluoro-2-methylimidazo[1, 2-a]pyridine-6-carboxamido)pyridin-3-yl)-2-methylpiperazine-1-carborylate.

To a mixture of 8-fluoro-2-methylimidazo[1,2-a]pyridine-6-carboxylic acid (300 mg, 1.95 mmol) in DMF (8 mL) was added tert-butyl (S)-4-(6-aminopyridin-3-yl)-2-methylpiperazine-1-carboxylate (299 mg, 1.54 mmol), DIEA (399 mg, 3.09 mmol) and HATU (585 mg, 1.54 mmol). The mixture was stirred at rt for 5 h. After diluting with water, the mixture was filtered and the cake was purified by silica gel column chromatography (DCM:MeOH=20:1) to give title product (280 mg, 33%) as a yellow solid. ESI-MS (M+H)⁺: 469.0.

### Step 4: Preparation of (S)-8-fluoro-2-methyl-N-(5-(3-methylpiperazin-1-yl)pyridin-2-yl)imidazo[1,2-a]pyridine-6-carboxamide TFA salt.

To a solution of tert-butyl (S)-4-(6-(8-fluoro-2-methylimidazo[1,2-a]pyridine-6-carboxamido)pyridin-3-yl)-2-methylpiperazine-1-carboxylate (100 mg, 0.21 mmol) in DCM (10 ml) was added TFA (1mL) at 0 °C. The mixture was stirred for 1 h at rt. After concentration, the residue was diluted with DCM (5 m) and concentrated again. The residue was purified by Prep-HPLC to give title product (31.72 mg, yield: 41%) as a red solid. ESI-MS (M+H)⁺: 369.1. ¹H NMR (400 MHz, DMSO-d₆) δ 10.91 (s, 1H), 9.25 - 9.24 (m, 2H), 8.85 (d, *J =* 9.4 Hz, 1H), 8.19 (d, *J =* 2.9 Hz, 1H), 8.07 - 8.02 (m, 2H), 7.89 - 7.83 (m, 1H), 7.57 (dd, *J =* 9.2, 3.0 Hz, 1H), 3.87 - 3.78 (m, 2H), 3.45 - 3.42 m, 2H), 3.19 - 3.17 (m, 1H), 3.01 - 2.98 (m, 1H), 2.80 - 2.76 (m, 1H), 2.43 (s, 3H), 1.30 (d, *J =* 6.5 Hz, 3H).

### Example 17. 8-fluoro-N-(2-fluoro-4-(piperazin-1-yl)phenyl)-2-methylimidazo[1,2-a] pyridine-6-carboxamide hydrochloride (Compound 108).

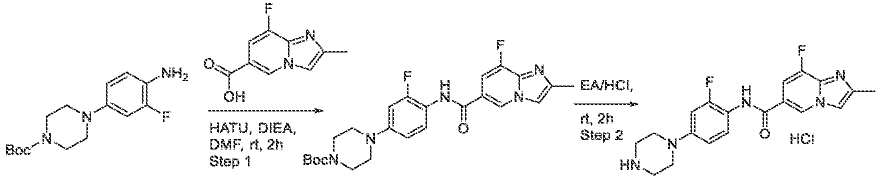

### Step 1: Preparation of tert-butyl 4-(3-fluoro-4-(8-fluoro-2-methylimidazo[1,2-a]pyridine-6-carboxamido)phenyl)piperazine-1-carboxylate.

To a stirred solution of 8-fluoro-2-methylimidazo[1,2-a]pyridine-6-carboxylic acid (100 mg ,0.52 mmol), tert-butyl 4-(4-amino-3-fluorophenyl)piperazine-1-carboxylate (168 mg, 0.57 mmol) and DIEA (335 mg, 2.60 mmol) in DMF (10 mL) was added HATU (396 mg, 1.04 mmol) at rt. After addition was completed, the mixture was stirred for 2 h. After concentration, the crude was purified by prep-HPLC (0.05% FA in water/ CH₃CN) to give title product (123 mg, yield: 51.2% ) as a brown solid. ESI-MS (M+H)⁺: 472.4.

### Step 2: Preparation of -fluoro-N-(2-fluoro-4-(piperazin-1-yl)phenyl)-2-methylimidazo[1,2-a]pyridine-6-carboxamide hydrochloride.

To a solution of tert-butyl 4-(3-fluoro-4-(8-fluoro-2-methylimidazo[1,2-a]pyridine-6-carboxamido)phenyl)piperazine-1-carboxylate (50 mg, 0.11 mmol) in EtOAc (1 mL) was added HCl-EA(3M, 1 mL), the mixture was stirred at rt for 1 h. The precipipate was filtered and lyophilized to give title product (35 mg, yield: 70%). ESI-MS (M+H)⁺: 372.2. ¹H NMR (400 MHz, CD₃OD) δ 9.26 (s, 1H), 8.30 - 8.17 (m, 2H), 7.61 (t, *J* = 8.6 Hz, 1H), 6.93 (dd, *J* = 17.9, 11.5 Hz, 2H), 3.48 (d, *J =* 4.8 Hz, 4H), 3.39 (d, *J =* 4.6 Hz, 4H), 2.62 (s, 3H).

### Example 18. N-(2-fluoro-4-(piperazin-1-yl)phenyl)-2,8-dimethylimidazo[1,2-a] pyrazine-6-carboxamide hydrochloride (Compound 109).

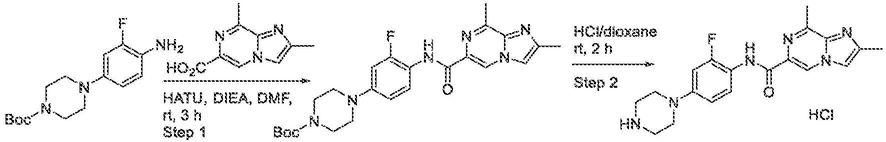

### Step 1: Preparation of tert-butyl 4-(4-(2,8-dimethylimidazo[1,2-a]pyrazine-6-carboxamido)-3- fluorophenyl)piperazine-1-carboxylate.

To a mixture of 2,8-dimethylimidazo[1,2-a]pyrazine-6-carboxylic acid (50 mg, 0.26 mmol) in DMF (8 mL) were added tert-butyl 4-(4-amino-3-fluorophenyl)piperazine -1-carboxylate (115 mg, 0.39 mmol), DIEA (101 mg, 0.78 mmol) and HATU (149 mg, 0.39 mmol). The mixture was stirred at rt for 5 h and quenched with water (10 mL). The mixture was filtered and the cake was purified by silica gel column chromatography (DCM:MeOH=20:1) to give tert-butyl 4-(4-(2,8-dimethylimidazo [1,2-a]pyrazine-6-carboxamido)-3-fluorophenyl)piperazine-1-carboxylate (50 mg, 41 %) as a yellow solid. ESI-MS (M+H)⁺: 469.3.

### Step 2: Preparation of N-(2-fluoro-4-(piperazin-1-yl)phenyl)-2,8-dimethylimidazo[1,2-a]pyrazine-6-carboxamide hydrochloride.

To a solution of tert-butyl 4-(4-(2,8-dimethylimidazo [1,2-a]pyrazine-6-carboxamido) -3-fluorophenyl)piperazine-1-carboxylate (50 mg, 0.11 mmol) was added HCl in dioxane (5 mL) at rt .The mixture was stirred for 1 h at rt. The precipipate was filtered and lyophilized to give N-(2-fluoro-4-(piperazin-1-yl)phenyl)-2,8-dimethylimidazo [1,2-a]pyrazine-6-carboxamide hydrochloride (3 mg, yield: 7.6%) as a yellow solid. ESI-MS (M+H)⁺: 369.3. ¹H NMR (400 MHz, DMSO-d₆) δ 10.05 (s, 1H), 9.20 (s, 1H), 8.94 (s, 2H), 8.07 (s, 1H), 7.88 - 7.83 (m, 1H), 7.02 (d, *J =* 14.2 Hz, 1H), 6.87 (d, *J =* 9.2 Hz, 1H), 3.40 (d, *J =* 4.8 Hz, 4H), 3.23 (s, 4H), 2.82 (s, 3H), 2.46 (s, 3H).

### Example 19. 7-methoxy-2-methyl-N-(5-(piperazin-1-yl)pyridin-2-yl)imidazo[1,2-a] pyridine-6-carboxamide HCl salt (Compound 110).

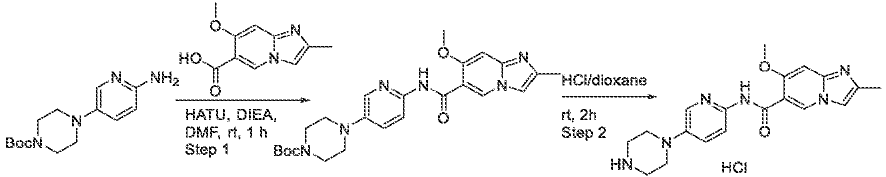

### Step 1: Preparation of tert-butyl 4-(6-(7-methoxy-2-methylimidazo[1,2-a]pyridine-6-carboxamido)pyridin-3-yl)piperazine-1-carboxylate.

To a stirred solution of 7-methoxy-2-methylimidazo[1,2-a]pyridine-6-carboxylic acid (100 mg, 0.48 mmol), tert-butyl 4-(6-aminopyridin-3-yl)piperazine-1-carboxylate (140 mg, 0.49 mmol) and DIEA (190 mg, 1.46 mmol) in DMF (4 mL) was added HATU (289 mg, 0.76 mmol) at rt. After addition was completed, the mixture was stirred for 1 h. After diluting with water, the solid was collected by filtration and washed with MeCN to give product (100 mg, Y: 44.8%) as a light brown solid, which was used to next step without further purification. ESI-MS (M+H)⁺: 467.3. 1H NMR (400 MHz, DMSO-d₆) δ 10.30 (s, 1H), 9.00 (s, 1H), 8.10 - 8.08 (m, 2H), 7.67 (s, 1H), 7.50 *(d, J=* 8.9 Hz, 1H), 7.03 (s, 1H), 4.00 (s, 3H), 3.47 (br.s, 4H), 2.34 (s, 3H), 1.43 (s, 9H).

### Step 2: Preparation of 7-methoxy-2-methyl-N-(5-(piperazin-1-yl)pyridin-2-yl)imidazo[1,2-a]pyridine-6-carboxamide HCl salt.

To a solution of tert-butyl 4-(6-(7-methoxy-2-methylimidazo[1,2-a]pyridine-6-carboxamido)pyridin-3-yl)piperazine-1-carboxylate (100 mg, 0.214 mmol) in 1,4-dioxane (1 mL) was added HCl-dioxane (4 M, 0.5 mL) at rt and stirred for 1 h. The precipipate was filtered and lyophilized to give 7-methoxy-2-methyl-N-(5-(piperazin-1-yl)pyridin-2-yl)imidazo [1,2-a]pyridine-6-carboxamide HCl salt (17 mg, yield: 21. 8%) as a yellow solid. ESI-MS (M+H)⁺: 367.2. ¹H NMR (400 MHz, DMSO-d₆) δ 10.91 (s, 1H), 9.82 (s, 2H), 9.23 (s, 1H), 8.17 - 8.05 (m, 2H), 8.00 (s, 1H), 7.64 (d, *J =* 7.2 Hz, 1H), 7.36 (s, 1H), 4.07 (s, 3H), 3.48 (s, 4H), 3.20 (s, 4H), 2.45 (s, 3H).

### Example 20. N-(2-fluoro-4-(piperazin-1-yl)phenyl)-7-methoxy-2- methylimidazo [1,2-a]pyridine-6-carboxamide hydrogen chloride (Compound 111).

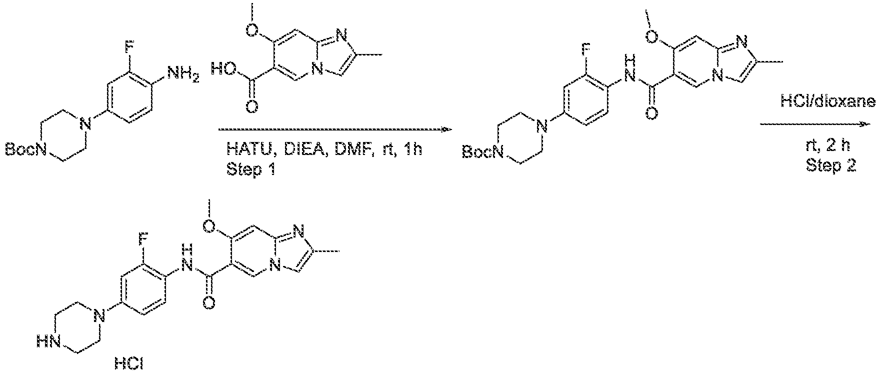

### Step 1: Preparation of tert-butyl 4-(3-fluoro-4-(7-methoxy-2-methylimidazo[1,2-a]pyridine-6-carboxamido)phenyl) piperazine-1-carboxylate.

To a stirred solution of 7-methoxy-2-methylimidazo [1,2-a]pyridine-6-carboxylic acid (100 mg, 0.48 mmol), tert-butyl 4-(4-amino-3-fluorophenyl)piperazine-1-carboxylate (130 mg, 0.49 mmol) and DIEA (190 mg, 1.46 mmol) in DMF (4 mL) was added HATU (289 mg, 0.76 mmol) at rt. After addition was completed, the mixture was stirred for 1 h. After diluting with water, the solid was collected by filtration and washed with MeCN to give title product (30 mg, Y: 12.8%) as a light brown solid, which was used to next step without further purification. ESI-MS (M+H)⁺: 484.3.

### Step 2: Preparation of N-(2-fluoro-4-(piperazin-1-yl)phenyl)-7-methoxy-2-methylimidazo [1,2-a]pyridine-6-carboxamide hydrogen chloride.

To a solution of text-butyl 4-(3-fluoro-4-(7-methoxy-2-methylimidazo[1,2-a] pyridine-6-carboxamido)phenyl)piperazine-1-carboxylate (30 mg, 0.06 mmol) in1,4-dioxane (1 mL) was added HCl-dioxane (4 M, 0.5 mL) at rt and stirred for 1 h. The precipipate was filtered and lyophilized to give N-(2-fluoro-4-(piperazin-1-yl) phenyl)-7-methoxy-2-methylimidazo[1,2-a]pyridine-6-carboxamide (12 mg, yield: 52.2%) as a yellow solid. ¹H NMR (400 MHz, DMSO-d₆) δ 10.11 (s, 1H), 9.68 (s, 2H), 9.24 (s, 1H), 7.98 (s, 1H), 7.68 (t, *J =* 8.9 Hz, 1H), 7.34 (s, 1H), 6.99 (d, *J =* 13.8 Hz, 1H), 6.86 (d, *J* = 7.8 Hz, 1H), 4.09 (s, 3H), 3.45 (s, 4H), 3.18 (s, 4H), 2.46 (s, 3H). ESI-MS (M+H)⁺: 384.2.

### Example 21. 7-methoxy-2,8-dimethyl-N-(5-(piperazin-1-yl)pyridin-2-yl)imidazo[1,2-a] pyridine-6-carboxamide (Compound 112).

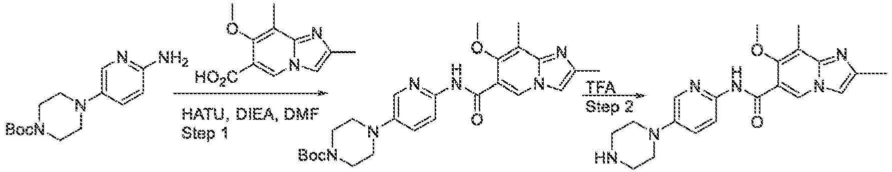

### Step 1: Preparation of tert-butyl4-(6-(7-methoxy-2,8-dimethylimidazo[1,2-a]pyridine-6-carboxamido)pyridin-3-yl)piperazine-1-carboxylate.

To a stirred solution of 7-methoxy-2,8-dimethylimidazo[1,2-a]pyridine-6-carboxylic acid (100 mg, 0.45 mmol), tert-butyl 4-(6-aminopyridin-3-yl)piperazine-1-carboxylate (152 mg, 0.55 mmol) and DIEA (176 mg, 1.36 mmol) in DMF (5 mL) was added HATU (259 mg, 0.681 mmol) at rt. After addition was completed, the mixture was stirred for 2 h. The reaction mixture was treated with EA/Water (100 mL, 1:1). The organic phase was separated and the aqueous phase was extracted with EA (50 mLx1). The combined organic layers was washed with brine and dried over sodium sulfate. The mixture was purified by prep-HPLC (0.05% FA in water/MeCN) to give tert-butyl 4-(6-(7-methoxy-2,8-dimethylimidazo[1,2-a]pyridine-6-carboxamido) pyridin-3-yl)piperazine-1-carboxylate (60 mg, yield: 27.6%) as a white solid. ESI-MS (M+H)⁺: 481.1.

### Step 2: Preparation of 7-methoxy-2,8-dimethyl-N-(5-(piperazin-1-yl)pyridin-2-yl)imidazo[1,2-a]pyridine-6-carboxamide HCl salt.

To a solution of tert-butyl tert-butyl 4-(6-(7-methoxy-2,8-dimethylimidazo[1,2-a] pyridine-6-carboxamido)pyridin- 3-yl)piperazine-1-carboxylate (60 mg, 0.124 mmol) in EA (3 mL) was added HCl-EA (3 M, 2 mL) at 0 °C and the mixture was allowed to warm to rt and stirred for 1 h. After concentration, the residue purified by prep-HPLC (0.05% HCl in water/MeCN) to give title compound (5.0 mg, yield: 10.6%) as a yellow solid. ¹H NMR (400 MHz, MeOD-d₄) δ 9.16 (s, 1H), 8.30 (dd, *J =* 9.5, 2.6 Hz, 1H), 8.07 (s, 1H), 8.00 (s, 1H), 7.80 (d, *J* = 9.4 Hz, 1H), 4.07 (s, 3H), 3.69 - 3.61 (m, 4H), 3.48 - 3.40 (m, 4H), 2.59 (s, 3H), 2.57 (s, 3H). ESI-MS (M+H)⁺: 381.1.

### Example 22. 6-methoxy-2-methyl-N-(5-(piperazin-1-yl)pyridin- 2-yl)-2H-indazole-5-carboxamide HCl salt (Compound 113).

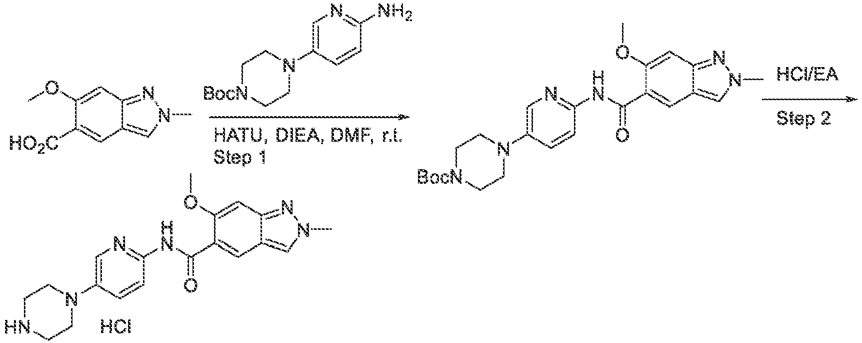

### Step 1: Preparation of tert-butyl 4-(6-(6-methoxy-2-methyl-2H-indazole- 5-carboxamido) pyridin-3-yl)piperazine-1-carboxylate.

To a stirred solution of 6-methoxy-2-methyl-2H-indazole-5-carboxylic acid (80 mg, 0.38 mmol), tert-butyl 4-(6-aminopyridia-3-yl)piperazine-1-carboxylate (128 mg, 0.46 mmol) and DIEA (152 mg, 1.16 mmol) in DMF (6 mL) was added HATU (220 mg,0.58 mmol) at rt. After addition was completed, the mixture was stirred for 2 h. The reaction mixture was treated with EA/Water (40 mL, 1:1). The organic phase was separated and the aqueous phase was extracted with EA (25 mL). The combined organic layers was washed with brine and dried over sodium sulfate. After concentration under reduced pressure, the crude was purificated by C18 flash (0.1% FA in water/CH₃CN) to give tert-butyl 4-(6-(6-methoxy-2-methyl-2H-indazole-5- carboxamido)pyridin-3-yl)piperazine-1-carboxylate (60 mg, Y: 33.2%) as a yellow solid.¹H NMR (400 MHz, CDCl₃) δ 10.31 (s, 1H), 8.70 (s, 1H), 8.35 (d, *J =* 9.1 Hz, 1H), 8.03 - 7.97 (m, 2H), 7.33 (dd, *J* = 9.0, 2.9 Hz, 1H), 7.10 (s, 1H), 4.20 (s, 3H), 4.11 (s, 3H), 3.64 - 3.57 (m, 4H), 3.15 - 3.09 (m, 4H), 1.62 (s, 9H). ESI-MS (M+H)⁺: 467.3.

### Step 2: Preparation of 6-methoxy-2-methyl-N-(S-(piperazin-1-yl)pyridin- 2-yl)-2H-indazole-5- carboxamide HCl salt.

To a solution of 4-(6-(6-methoxy-2- methyl-2H-indazole-5-carboxamido)pyridin-3-yl) piperazine-1-carboxylate (60 mg, 0.129 mmol) in EA (2 mL) was added HCl-EA (3 M, 2 mL) at 0 °C and the mixture was allowed to warm to rt and stirred for 1 h. After concentration, the solid was collected by filtration and washed EA three times (3 mL x3). The solid was dried under vacuum at 55 °C to provide title product (10 mg, yield: 21.2 %) as light yellow solid. ¹H NMR (400 MHz, MeOD-d₄) δ 8.66 (s, 1H), 8.52 (s, 1H), 8.33 (dd, *J =* 9.5, 2.3 Hz, 1H), 8.00 (d, *J =* 2.0 Hz, 1H), 7.77 (d, *J* = 9.5 Hz, 1H), 7.24 (s, 1H), 4.29 (s, 3H), 4.11 (s, 3H), 3.68 - 3.60 (m, 4H), 3.49 - 3.41 (m, 4H). ESI-MS (M+H)⁺: 367.2.

### Example 23. (S)-N-(2-fluoro-4-(3-methylpiperazin-1-yl)phenyl)-6-methoxy-2-methyl-2H-indazole-5-carboxamide HCl salt (Compound 114).

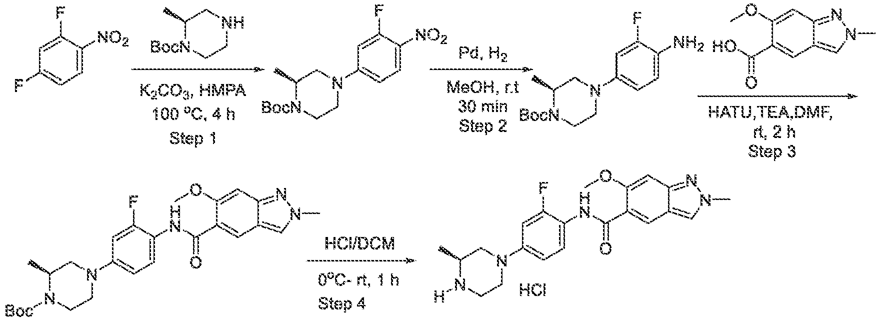

### Step 1: Preparation of tert-butyl (S)-4-(3-fluoro-4-nitrophenyl)-2-methylpiperazine-1-carboxylate.

A mixture of 2,4-difluoro-1-nitrobenzene (5 g, 21 mmol), K₂CO₃ (6.9 g, 50 mmol) and tert-butyl (S)-2-methylpiperazine-1-carboxylate (4.2g, 21 mmol) in HMPA (50ml) was heated to 100 °C for 4 h. After cooling to rt, the reaction mixture was diluted with ethyl acetate (300 mL) and washed with water (100 mL) and brine (100 mL). The organic layer was dried over Na₂SO₄, concentrated to dryness. The crude was purified by silica gel column (EA:PE=1:5, v/v) to give title product (4 g, Y: 80%) as a yellow solid. ¹H NMR (400 MHz, CDCl₃) δ 7.98 (t*, J =* 9.1 Hz, 1H), 6.47 (dd, *J =* 9.5, 2.5 Hz, 1H), 6.40 (dd, *J =* 14.8, 2.6 Hz, 1H), 4.27 (s, 1H), 3.93 - 3.82 (m, 1H), 3.65 - 3.62 (m, 1H), 3.51 (d, *J =* 11.2 Hz, 1H), 3.34 - 3.24 (m, 2H), 3.14 - 3.05 (m, 1H), 1.46 (s, 9H), 1.15 (d, *J =* 6.7 Hz, 3H).

### Step 2: Preparation of tert-butyl (S)-4-(4-amino-3-fluorophenyl)-2-methylpiperazine-1-carboxylate.

A mixture of tert-butyl (S)-4-(3-fluoro-4-nitrophenyl)-2-methylpiperazine-1-carboxylate (2g, 5.9 mmol) and Pd/C (320 mg, 10% wt/wt) in MeOH (50 mL) was purged with hydrogen for three times at rt. The mixture was stirred for 30 min at rt under hydrogen. The catalyst was filtered off and the filtrate was concentrated to give title product, which was used in next step without further purification. ¹H NMR (400 MHz, CDCl₃) δ 6.77 - 6.69 (m, 1H), 6.62 (dd, *J =* 13.4, 2.5 Hz, 1H), 6.55 (dd, *J* = 8.5, 2.1 Hz, 1H), 4.32 (s, 1H), 3.95 - 3.92 (m, 1H), 3.45 (s, 2H), 3.32 - 3.12 (m, 3H), 2.79 - 2.76 (m, 1H), 2.61 - 2.59 (m, 1H), 1.48 (s, 9H), 1.30 (d, *J =* 6.7 Hz, 3H). ESI-MS (M+H)⁺: 310.2.

### Step 3: Preparation of tert-butyl (S)-4-(3-fluoro-4-(6-methoxy-2-methyl-2H-indazole-5-carboxamido)phenyl)-2-methylpiperazine-1-carboxylate.

To a stirred solution of 6-methoxy-2-methyl-2H-indazole-5-carboxylic acid (80 mg, 0.38 mmol), tert-butyl (S)-4-(4-amino-3-fluorophenyl)-2-methylpiperazine-1-carboxylate (181 mg, 0.58 mmol) and DIEA (253 mg, 0.76 mmol) in DMF (10 mL) was added HATU (297 mg, 0.78 mmol) at rt. After addition was completed, the mixture was stirred for 2 h. The reaction mixture was treated with EA/Water (100 mL, 1:1). The organic phase was separated and the aqueous phase was extracted with EA (50 mLx1). The combined organic layers was washed with brine and dried over sodium sulfate. After concentration under reduced pressure, the crude was sluury with EA/PE (1:10, v/v, 5 mL) for 30 min at rt. Then the solid was collected by filtration and washed with EA/PE (1:10, v/v, 3 mL x 3). The product was obtained (90 mg, Y: 49%) as a light brown solid. ESI-MS (M+H)⁺: 498.3. ¹H NMR (400 MHz, CDCl₃) δ 10.21 (d, *J =* 2.3 Hz, 1H), 8.73 (d, *J* = 3.8 Hz, 1H), 8.41 (dd, *J =* 11.7, 6.7 Hz, 1H), 7.98 (d, *J =* 5.2 Hz, 1H), 7.09 (d, *J =* 6.7 Hz, 1H), 6.72 - 6.56 (m, 2H), 4.35 (s, 1H), 4.20 (s, 3H), 4.09 (s, 3H), 3.95 - 3.91 (m, 1H), 3.46 - 3.42 (m, 1H), 3.33 - 3.30 (m, 1H), 3.29 - 3.21 (m, 1H), 2.94 - 2.90 (m, 1H), 2.79 - 2.71 (m, 1H), 1.49 (s, 9H), 1.30 (d, *J =* 6.7 Hz, 3H).

### Step 4: Preparation of (S)-N-(2-fluoro-4-(3-methylpiperazin-1-yl)phenyl)-6-methoxy-2-methyl-2H-indazole-5-carboxamide HCl salt.

To a solution of tert-butyl (S)-4-(3-fluoro-4-(6-methoxy-2-methyl-2H-indazole-5-carboxamido)phenyl)-2-methylpiperazine-1-carboxylate (64 mg, 0.141 mmol) in EtOAc (1 mL) was added HCl-EA(3M, 1 mL), the mixture was stirred at rt for 1 h. The precipipate was filtered and lyophilized to give (S)-N-(2-fluoro-4-(3-methylpiperazin-1-yl) phenyl)-6-methoxy-2-methyl-2H-indazole-5- carboxamide hydrochloride (35.3 mg, yield: 60%)..ESI-MS (M+H)+: 398. 1H NMR (400 MHz, MeOD-d₄) δ 8.71 (s, 1H), 8.55 (s, 1H), 8.05 (t, *J =* 8.9 Hz, 1H), 7.24 (s, 1H), 7.00 - 6.85 (m, 2H), 4.30 (s, 3H), 4.14 (s, 3H), 3.85 - 3.81 (m, 2H), 3.55 - 3.45 (m, 2H), 3.36 - 3.32 (m, 1H), 3.09 - 2.95 (m, 1H), 2.85 - 2.82 (m, 1H), 1.41 (d, *J* = 6.6 Hz, 3H).

### Example 24. N-(2-fluoro-4-(piperazin-1-yl)phenyl)-6-methoxy-2-methyl-2H-indazole-5-carboxamideHCl salt (Compound 115).

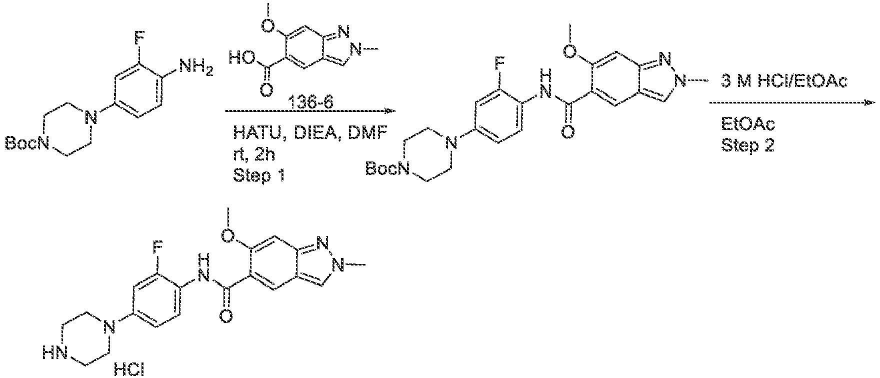

### Step 1: Preparation of tert-butyl 4-(3-fluoro-4-(6-methoxy-2-methyl-2H-indazole-5-carboxamido)phenyl)piperazine-1-carboxylate

To a stirred solution of 6-methoxy-2-methyl-2H-indazole-5-carboxylic acid (60 mg, 0.29 mmol), tert-butyl 4-(4-amino-3-fluorophenyl)piperazine-1-carboxylate (96 mg, 0.32 mmol) and DIEA (114 mg, 0.87 mmol) in DMF (3 mL) was added HATU (150 mg, 0.39 mmol) at rt. After addition was completed, the mixture was stirred for 2 h. The crude was purified by prep-HPLC (0.05% FA in water/ CH₃CN) to give title product (80 mg, yield:57.1%) as a yellow solid. ESI-MS (M+H)⁺: 484.1 ¹H NMR (400 MHz, CDCl₃) δ 10.24 (s, 1H), 8.73 (s, 1H), 8.42 (dd, *J* = 11.6, 6.8 Hz, 1H), 7.99 (s, 1H), 7.10 (s, 1H), 6.75 - 6.68 (m, 2H), 4.20 (s, 3H), 4.09 (s, 3H), 3.60 - 3.57 (m, 4H), 3.14 - 3.09 (m, 4H), 1.49 (s, 9H).

### Step 2: Preparation of N-(2-fluoro-4-(piperazin-1-yl)phenyl)-6-methoxy-2-methyl-2H-indazole-5-carboxamideHCl salt.

To a solution of tert-butyl 4-(3-fluoro-4-(6-methoxy-2-methyl-2H-indazole-5-carboxamido)phenyl)piperazine-1-carboxylatee (80 mg, 0.16 mmol) in EA (0.5 mL) was added HCl-EA (3 M, 0.5 mL) and the mixture was stirred for 1 h. The precipipate was filtered and lyophilized to give title product (35 mg, yield:55.5 %) as a yellow solid. ESI-MS (M+H)⁺: 383.9. ¹H NMR (400 MHz, MeOD-d₄) δ 8.70 (s, 1H), 8.55 (s, 1H), 8.05 (t, *J* = 8.8 Hz, 1H), 7.24 (s, 1H), 6.96 (dd, *J =* 13.6, 2.6 Hz, 1H), 6.88 (dd, *J* = 8.8, 2.4 Hz, 1H), 4.31 (s, 3H), 4.13 (s, 3H), 3.46 - 3.43 (m, 4H), 3.39 - 3.36 (m, 4H).

### Example 25. N-(2-fluoro-4-(2,6-diazaspiro[3.3]heptan-2-yl)phenyl)-6-methoxy-2-methyl-2H-indazole-5-carboxamide TFA salt (Compound 116).

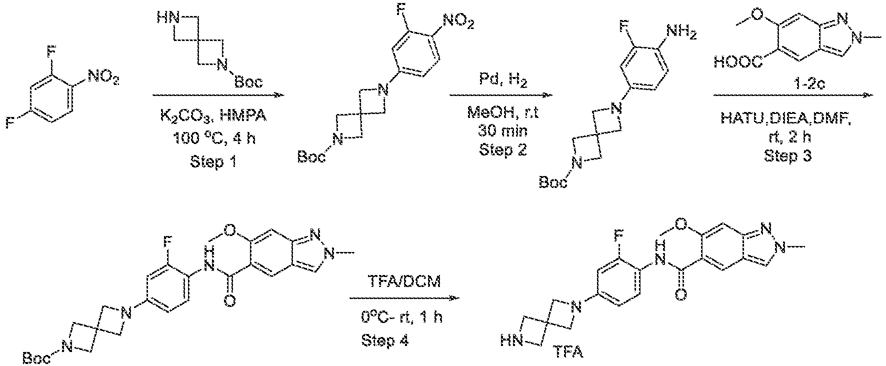

### Step 1: Preparation of tert-butyl6-(3-fluoro-4-nitrophenyl)-2,6-diazaspiro[3.3]heptane-2-carboxylate.

A mixture of 2,4-difluoro-1-nitrobenzene (722 mg, 4.54 mmol), K₂CO₃ (732 mg, 5.30 mmol) and tert-butyl 2,6-diazaspiro[3.3]heptane-2-carboxylate (300 mg, 1.51 mmol) in HMPA (10 mL) was heated to 100 °C for 4 h. After cooling to rt, the reaction mixture was diluted with ethyl acetate (60 mL) and washed with water (60 mL) and brine (60 mL). The organic layer was dried over Na₂SO₄, concentrated to dryness. The crude was purified by pre-TLC (EA:PE=1:5, v/v) to give title product (260 mg, Y: 50.9%) as a yellow solid. ESI-MS (M+H)⁺: 338.1.

### Step 2: Preparation of tert-butyl 4-(4-amino-3-fluorophenyl)piperazine-1-carboxylate.

A mixture of tert-butyl 6-(3-fluoro-4-nitrophenyl)-2,6-diazaspiro[3.3]heptane-2 - carboxylate (460 mg, 1.36 mmol) and Pd/C (92 mg, 20% wt/wt) in MeOH (30 mL) was purged with hydrogen for three times at rt. The mixture was stirred for 30 min at rt under hydrogen. The catalyst was filtered off and the filtrate was concentrated to give title product, which was used in next step without further purification. ¹H NMR (400 MHz, CDCl₃) δ 6.70 (t, *J =* 9.0 Hz, 1H), 6.19 (dd, *J =* 12.5, 2.3 Hz, 1H), 6.11 (dd, *J* = 8.4, 1.7 Hz, 1H), 4.07 (br.s, 4H), 3.87 (br.s, 4H), 1.44 (s, 9H).

### Step 3: Preparation of tert-butyl6-(3-fluoro-4-(6-methoxy-2-methyl-2H-indazole-5-carboxamido) phenyl)-2, 6-diazaspiro[3.3]heptane-2-carboxylate.

To a stirred solution of 6-methoxy-2-methyl-2H-indazole-5-carboxylic acid (60 mg, 0.29 mmol), tert-butyl 6-(4-amino-3-fluorophenyl)-2,6-diazaspiro[3.3]heptane-2- carboxylate (80 mg, 0.26 mmol) and DIEA (114 mg, 0.87 mmol) in DMF (3 mL) was added HATU (150 mg, 0.38 mmol) at rt. After addition was completed, the mixture was stirred for 2 h. The crude was purified by prep-HPLC (0.05% FA in water/ CH₃CN) to give title product (50 mg, yield: 39.0 %) as a yellow solid. ESI-MS (M+H)⁺: 496.1. ¹H NMR (400 MHz, CDCl₃) δ 10.15 (d, *J* = 2.2 Hz, 1H), 8.72 (s, 1H), 8.34 (t, *J* = 8.8 Hz, 1H), 7.99 (s, 1H), 7.09 (s, 1H), 6.24 (dd, *J=* 9.5, 4.9 Hz, 2H), 4.20 (s, 3H), 4.10 (s, 4H), 4.08 (s, 3H), 3.96 (s, 4H), 1.45 (s, 9H).

### Step 4: Preparation ofN-(2-fluoro-4-(2,6-diazaspiro[3.3]heptan-2-yl)phenyl)-6-methoxy-2- methyl-2H-indazole-5-carboxamide TFA salt.

To a solution of tert-butyl 6-(3-fluoro-4-(6-methoxy-2-methyl-2H-indazole-5-carboxamido)phenyl)-2,6-diazaspiro[3.3] heptane-2-carboxylate (50 mg, 0.10 mmol) in DCM (4 mL) was added TFA (1 mL) and the mixture was stirred for 1 h. The mixture was concentrated in vacuo, the residue was purified by prep-HPLC (0.05% FA in water/ CH₃CN) to give title product (10 mg, yeild:25%) as a yellow solid. ¹H NMR (400 MHz, DMSO-d₆) δ 9.89 (s, 1H), 8.78 (s, 1H), 8.43 (s, 1H), 8.33 (s, 1H), 7.82 (t*, J* = 8.8 Hz, 1H), 7.13 (s, 1H), 6.43 - 6.27 (m, 2H), 4.18 (s, 4H), 4.14 (s, 3H), 3.98 (d, *J =* 3.5 Hz, 7H). ESI-MS (M+H)⁺: 396.1.

### Example 26. N-(2-fluoro-4-(hexahydropyrrolo[3,4-c]pyrrol-2(1H)-yl)phenyl)-6-methoxy-2-methyl-2H-indazole-5-carboxamide (Compound 117).

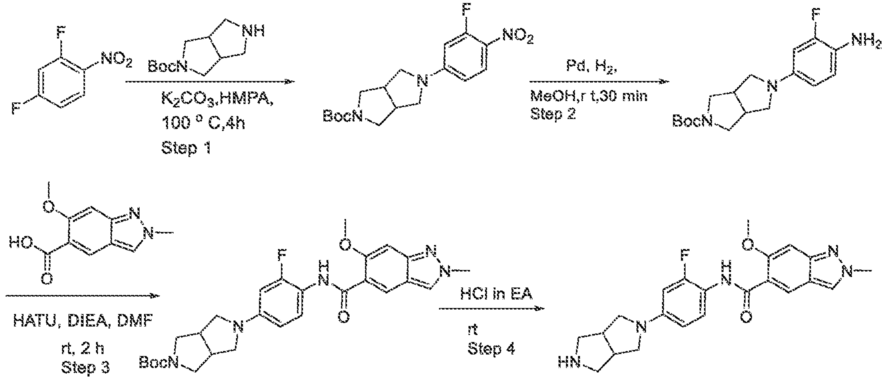

### Step 1: Preparation of tert-butyl 5-(3-fluoro-4-nitrophenyl)hexahydropyrrolo[3,4-c]pyrrole-2(1H)-carboxylate.

A mixture of 2,4-difluoro-1-nitrobenzene (450 mg, 2.83 mmol), K₂CO₃ (391 mg, 2.83 mmol) and tert-butyl hexahydropyrrolo[3,4-c]pyrrole-2(1H)-carboxylate (300 mg, 1.41 mmol) in HMPA (8.00 mL) was heated at 100 °C for 4 h. After cooling to rt, the reaction mixture was diluted with ethyl acetate (100.0 mL) and washed with water (60.0 mL) and brine (30.0 mL). The organic layer was dried over Na₂SO₄. After concentration, the crude was purified by gel silica chromatograph (PE:EA=10:1-5:1) to give title product (270 mg, Y: 64.5%) as a yellow solid. ESI-MS (M+H-56)⁺: 296.1.

### Step 2: Preparation of tert-butyl 5-(4-amino-3-fluorophenyl)hexahydropyrrolo(3,4-c]pyrrole-2(1H)-carboxylate.

A mixture of tert-butyl 5-(3-fluoro-4-nitrophenyl)hexahydropyrrolo[3,4-c]pyrrole-2(1H)-carboxylate (270 mg, 0.912 mmol) and Pd/C (50.0 mg) in EA (5.00 mL) was purged with hydrogen for three times at rt. The mixture was stirred for overnight at rt under hydrogen. The catalyst was filtered off and the filtrate was concentrated to give title product, which was used in next step without further purification. ESI-MS (M+H)⁺: 322.1.

### Step 3: Preparation of tert-butyl 5-(3-fluoro-4-(6-methoxy-2-methyl-2H-indazole-5-carboxamido) phenyl)hexahydropyrrolo[3, 4-c]pyrrole-2(1H)-carboxylate.

To a mixture of tert-butyl 5-(4-amino-3-fluorophenyl)hexahydropyrrolo[3,4-c] pyrrole-2(1H)-carboxylate (100 mg, 0.31 mmol) in DMF (8 mL) were added 6-methoxy-2-methyl-2H-indazole-5-carboxylic acid (128 mg, 0.62 mmol), DIEA (121 mg, 0.93 mmol) and HATU (178 mg, 0.47 mmol). The mixture was stirred at rt for 2 h. After diluting with water, he mixture was filtered and the cake was purified by C18 (0.1% FA in water/CH₃CN) to give title product (40 mg, 25%) as a white solid. ESI-MS (M+H)⁺: 510.1. ¹H NMR (400 MHz, MeOD-d₄) δ 8.51 (s, 1H), 8.31 (s, 1H), 7.75 (dd, *J* = 8.8, 6.2 Hz, 1H), 7.12 (s, 1H), 6.83 (dd, *J =* 11.1*,* 2.8 Hz, 1H), 6.74 - 6.69 (m, 1H), 4.19 (s, 3H), 4.04 (s, 3H), 3.63 (dd, *J =* 11.3, 7.9 Hz, 2H), 3.34 (s, 2H), 3.24 (dd, *J =* 11.3, 4.0 Hz, 2H), 3.09 (d, *J =* 9.0 Hz, 2H), 3.00 (dd, *J =* 8.6, 5.1 Hz, 2H), 1.43 (s, 9H).

### Step 4: Preparation of N-(2-fluoro-4-(hexahydropyrrolo[3,4-c]pyrrol-2(1H)-yl)phenyl)-6- methoxy-2-methyl-2H⁻indazole-5-carboxamide hydrochloride.

To a solution of tert-butyl 5-(3-fluoro-4-(6- methoxy-2-methyl-2H-indazole-5-carboxamido)phenyl)hexahydropyrrolo [3,4-c]pyrrole-2(1H)-carboxylate (40 mg, 0.078 mmol) was added HCl-EA (3 M, 5 mL) at RT and the mixture stirred for 1 h at rt. The precipipate was filtered and lyophilized to give title product (24 mg, yield: 75%) as white solid. ¹H NMR (400 MHz, MeOD-d₄) δ 8.70 (s, 1H), 8.51 (s, 1H), 7.63 (dd, *J =* 8.8, 6.1 Hz, 1H), 7.27 (s, 1H), 6.93 (dd, *J =* 10.8, 2.7 Hz, 1H), 6.83 (dd, *J =* 8.3, 2.7 Hz, 1H), 4.32 (s, 3H), 4.13 (s, 3H), 3.53 - 3.46 (m, 2H), 3.32 (s, 2H), 3.19 - 3.15 (m, 6H). ESI-MS (M+H)⁺: 410.2.

### Example 27. 7-methoxy-2-methyl-N-[6-(piperazin-1-yl)pyridazin-3-yl]imidazo|1,2-a]pyridine-6-carboxamide (Compound 118).

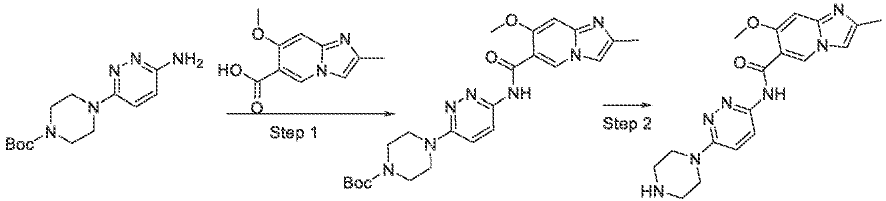

### Step 1: Preparation of 4-(6-7-methoxy-2-methylimidazo[1,2-a]pyridine-6-amidopyridazin-3-yl)piperazine-1-carboxylate.

7-methoxy-2-methylimidazo[1,2-a]pyridine-6-carboxylic acid (0.229 g, 1.11 mmol) was suspended in DMF (4 mL) and ethylbis(propan-2-yl)amine (0.215 g, 1.66 mmol, 290.0 µL, 1.5 equiv.) was added followed by [(dimethylamino)(3H-[1,2,3]triazolo[4,5-b]pyridin-3-yloxy)methylidene]dimethylazanium, hexafluoro-lambda5-phosphanuide (0.506 g, 1.33 mmol). The resulting mixture was stirred for 30 min at r.t. After that, *tert-*Butyl 4-(6-aminopyridazin-3- yl)piperazine-1-carboxylate (0.223 g, 798.34 µmol) was added in one portion and the reaction mixture was stirred overnight at rt. The precipitate formed was filtered, washed with MeCN (2 mL), MTBE (2 mL), dried in vacuo to give pure *tert-*butyl 4-(6-7-methoxy-2-methylimidazo[1,2-a]pyridine-6-amidopyridazin-3-yl)piperazine-1-carboxylate (0.120 g, 256.67 µmol, 23.1% yield). ESI-MS (M+H)⁺: 468.4.

### Step 2: Preparation of 7-methoxy-2-methyl-N-[6-(piperazin-1-yl)pyridazin-3-yl]imidazo[1,2-a]pyridine-6-carboxamide TFA salt.

To a stirred solution of *tert*-butyl 4-(6-7-methoxy-2-methylimidazo[1,2-a]pyridine-6-amidopyridazin- 3-yl)piperazine-1-carboxylate (0.050 g, 106.97 µmol) in dichloromethane (15 mL) 2,2,2-trifluoroacetic acid (0.122 g, 1.07 mmol) was added in one portion and the resulting mixture was stirred overnight at r.t. The solvents were evaporated and the residue was triturated with MTBE/MeCN (5/2 mL). The precipitate was filtered and dried under vacuo to give 7-methoxy-2-methyl-N-[6-(piperazin-1-yl)pyridazin- 3-yl]imidazo[1,2-a]pyridine-6-carboxamide TFA salt (0.042 g, 101.78 µmol, quantitative) as a white solid. ¹H NMR (500 MHz, DMSO-d₆) δ 11.09 (s, 1H), 9.14 (s, 1H), 9.07 - 8.82 (brs, 2H), 8.19 (d, *J* = *9.9 Hz,* 1H), 7.90 (s, 1H), 7.51 (d, *J = 9.8 Hz,* 1H), 7.34 (s, 1H), 4.05 (s, 3H), 3.77 (s, 4H), 3.23 (s, 4H). ESI-MS (M+H)⁺: 368.2.

### Example 28. 7-methoxy-2-methyl-N-[6-(piperazin-1-yl)pyridin-3-yl]imidazo[1,2-a]pyridine-6-carboxamide (Compound 119).

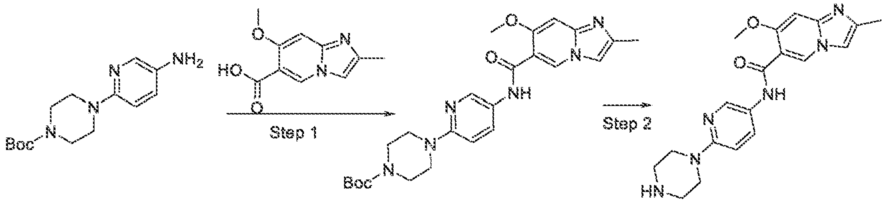

### Step 1: Preparation of tert-butyl 4-(5-7-methoxy-2-methylimidazo[1,2-a]pyridine-6-amidopyridin-2-yl)piperazine-1-carboxylate.

The 7-methoxy-2-methylimidazo[1,2-a]pyridine-6-carboxylic acid (0.200 g, 969.94 µmol) was suspended in DMF (4 mL) and ethylbis(propan-2-yl)amine (0.314 g, 2.43 mmol) was added followed by [(dimethylamino)(3H-[1,2,3]triazolo[4,5-b]pyridin-3-yloxy)methylidene]dimethylazanium, hexafluoro-lambda5-phosphanuide (0.443 g, 1.17 mmol). The resulting mixture was stirred for 30 min at r.t. After that, *tert*-butyl 4-(5-aminopyridin-2-yl)piperazine-1-carboxylate (0 270 g, 970.99 µmol) was added in one portion and the reaction mixture was stirred overnight at rt. The precipitate formed was filtered, washed with MeCN (2 mL), MTBE (2 mL), dried in vacuo to give pure *tert-*butyl 4-(5-7-methoxy-2-methylimidazo[1,2-a]pyridine-6-amidopyridin- 2-yl)piperazine-1-carboxylate (0.250 g, 535.87 µmol, 55.2% yield). ¹H NMR (400 MHz, DMSO-d₆) δ 9.96 (s, 1H), 8.81 (s, 1H), 8.39 (s, 1H), 7.86 (d, *J = 9.2 Hz,* 1H), 7.55 (s, 1H), 6.91 (s, 1H), 6.84 (d, *J = 8.9 Hz,* 1H), 3.89 (s, 3H), 3.39 (s, 8H), 2.24 (s, 3H), 1.39 (s, 9H). ESI-MS (M+H)⁺: 467.2.

### Step 2: Preparation of 7-methoxy-2-methyl-N-[6-(piperazin-1-yl)pyridin-3-yl]midazo[1,2-alpyridine-6-carboxamide TFA salt.

tert-Butyl 4-(5-7-methoxy-2-methylimidazo[1,2-a]pyridine- 6-amidopyridin-2-yl)piperazine-1-carboxylate (0.050 g, 107.25 µmol) was dissolved in dichloromethane (10 mL) and 2,2,2-trifluoroacetic acid (0.122 g, 1.07 mmol) was added. The resulting mixture was stirred overnight at r.t., evaporated and triturated with mixture of MTBE/MeCN (5/2 mL). The precipitate formed was filtered and dried in vacuo to give desired 7-methoxy-2-methyl-N-[6-(piperazin-1-yl)pyridin-3- yl]imidazo[1,2-a]pyridine-6-carboxamide TFA salt (0.109 g, 226.88 µmol, quantitative) as a grey solid. 1H NMR (400 MHz, DMSO-d₆) δ 10.31 (s, 1H), 9.11 (s, 1H), 8.80 (s, 2H), 8.42 (s, 1H). 7.88 (m, 2H), 7.30 (s, 1H), 6.95 (s, 1H), 4.02 (s, 3H), 3.64 (s, 4H), 3.17 (s, 4H), 2.40 (s, 3H). ESI-MS (M+H)⁺: 367.2.

### Example 29. 7-methoxy-2-methyl-N-(2-(piperazin-1-yl)pyrimidin-5-yl)imidazo[1,2-a]pyridine-6-carboxamide (Compound 120).

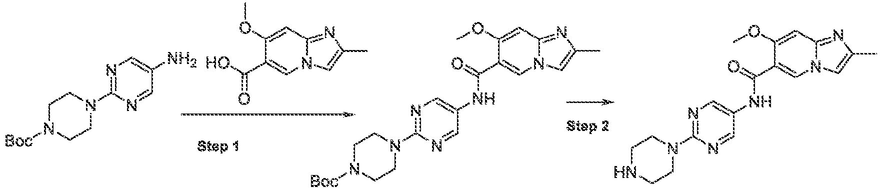

### Step 1: Preparation of tert-butyl 4-(5-(7-methoxy-2-methylimidazo[1,2-a]pyridine-6-carboxamido)pyrimidin-2-yl)piperazine-1-carboxylate.

The 7-methoxy-2-methylimidazo[1,2-a]pyridine-6-carboxylic acid (0.200 g, 969.94 µmol) was suspended in DMF (4 mL) and ethylbis(propan-2-yl)amine (0.313 g, 2.42 mmol) was added followed by [(dimethylamino)(3H-[1,2,3]triazolo[4,5-b]pyridin-3-yloxy)methylidene]dimethylazanium, hexafluoro-lambda5-phosphanuide (0.442 g, 1.16 mmol). The resulting mixture was stirred for 30 min at r.t. After that, tert-butyl 4-(5-aminopyrimidin-2-yl)piperazine-1-carboxylate (0.270 g, 969 µmol) was added in one portion and the reaction mixture was stirred overnight at r.t. The precipitate formed was filtered, washed with MeCN (2 mL), MTBE (2 mL), dried in vacuo to give pure tert-butyl 4-(5-(7-methoxy-2-methylimidazo[1,2-a]pyridine-6-carboxamido)pyrimidin-2-yl)piperazine-1-carboxylate (0.234 g, 500 µmol, 51.7% yield). ESI-MS (M+H)⁺: 468.2.

### Step 2: Preparation of 7-methoxy-2-methyl-N-(2-(piperazin-1-yl)pyrimidin-5-yl)imidazo[1,2-a]pyridine-6-carboxamide TFA salt.

*tert*-butyl 4-(5-(7-methoxy-2-methylimidazo[1,2-a]pyridine-6-carboxamido)pyrimidin-2-yl)piperazine-1-carboxylate (0.234 g, 500 µmol) was dissolved in dichloromethane (10 mL) and 2,2,2-trifluoroacetic acid (0.571 g, 5.01 mmol) was added. The resulting mixture was stirred overnight at r.t., evaporated and triturated with mixture of MTBE/MeCN (5/2 mL). The precipitate formed was filtered and dried in vacuo to give desired 7-methoxy-2-methyl-N-(2-(piperazin-1-yl)pyrimidin-5-yl)imidazo[1,2-a]pyridine-6-carboxamide TFA salt (0.215 g, 447 µmol, quantitative) as a beige solid. 1H NMR (400 MHz, DMSO-d₆) δ 10.38 (s, 1H), 9.14 (s, 1H), 8.94 (s, 2H), 8.68 (d, *J =* 1.7 Hz, 2H), 7.88 (s, 1H), 7.32 (s, 1H), 4.02 (s, 3H), 3.89 (s, 4H), 3.15 (s, 4H), 2.40 (s, 3H). ESI-MS (M+H)⁺: 368.2.

### Example 30. 8-fluoro-2-methyl-N-(6-(piperazin-1-yl)pyridin-3-yl)imidazo[1,2-a]pyridine-6-carboxamide (Compound 121).

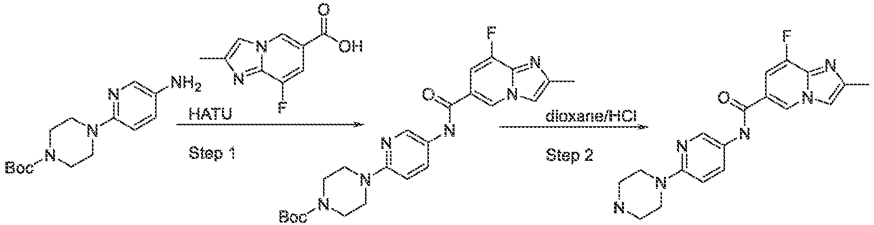

### Step 1: Preparation of tert-butyl 4-(5-(8-fluoro-2-methylimidazo[1,2-a]pyridine-6-carboxamido)pyridin-2-yl)piperazine-1-carboxylate.

A mixture of 8-fluoro-2-methylimidazo[1,2-a]pyridine-6-carboxylic acid (0.5 g; 2.6 mmol), HATU (1.18 g; 3.12 mmol), tert-butyl 4-(5-aminopyridin-2-yl)piperazine-1-carboxylate (0.7 g; 2.5 mmol) and TEA (0.72 mL; 5.2 mmol) in DMF (20 mL) was stirred at r.t. for 14 h. Next, the reaction mixture was diluted with water and extracted with EtOAc. Organics were washed with water, dried with Na₂SO₄ and evaporated to give crude title compound (0.9 g; 2 mmol; 79% yield), which was used in the next step without further purification. ESI-MS: 455.2 (M+H)⁺.

### Step 2: Preparation of 8-fluoro-2-methyl-N-(6-(piperazin-1-yl)pyridin-3-yl)imidazo[1.2-a]pyridine-6-carboxamide HCl.

tert-butyl 4-(5-(8-fluoro-2-methylimidazo[1,2-a]pyridine-6- carboxamido)pyridin-2-yl)piperazine-1-carboxylate (0.9 g; 2 mmol) was dissolved in MeOH (20 mL). Next, 10% dioxane/HCl solution was added and the obtained solution was stirred at r.t. for 10 h. The reaction mixture was evaporated in vacuo and the residue was purified by HPLC to afford the title compound as hydrochloride salt (73 mg; 19% yield). ESI-MS: 355.2 (M+H)⁺; ¹H NMR (400 MHz, DMSO-*d*₆): δ 10.18 (s, 1H), 9.05 (s, 1H), 8.41 (d, *J* = 2.8 Hz, 1H), 7.96 (d, *J=* 2.4 Hz, 1H), 7.86 (dd, *J =* 9.2, 2.8 Hz, 1H), 7.61 (d, *J=* 12 Hz, 1H), 6.83 (d, *J=* 9.6 Hz, 1H), 3.33-3.39 (m, 4H), 2.74-2.82 (m, 4H), 2.38 (s, 3H).

### Example 31. tert-butyl (S)-4-(6-aminopyridazin-3-yl)-2- methylpiperazine-1-carboxylate

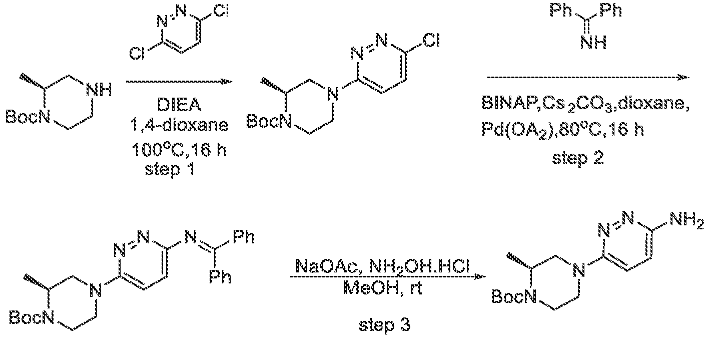

### Step 1: Preparation of tert-butyl (S)-4-(6-chloropyridazin-3-yl)-2-methylpipera zine-1-carboxylate.

To a solution of 3,6-dichloropyridazine (3.0 g, 15 mmol) in 1,4-dioxane (20 mL) were added DIEA (3.87 g, 30 mmol), tert-butyl (S)-2-methylpiperazine-1-carboxylate (2.4 g, 12 mmol). The mixture was stirred at 100 °C overnight. The reaction mixture was diluted with saturated sodium bicarbonate and extracted with EtOAc (50 mLx3), the organic layer was dried over sodium sulfate, filtered and concentrated in vacuo. The residue was purified by column chromatography (PE/EA=5:1)to give title product (1.4 g, Y:36.8%) as a light yellow solid. ESI-MS (M+H)⁺: 313.1. ¹H NMR (400 MHz, CDCl₃) δ 7.22 (d, *J =* 9.5 Hz, 1H), 6.86 (d, *J =* 9.5 Hz, 1H), 4.36 (br s, 1H), 4.15 (d, *J =* 11.6 Hz, 1H), 4.06 - 3.92 (m, 2H), 3.37 (dd, *J=* 13.2, 4.0 Hz, 1H), 3.29 - 3.26 (m, 1H), 3.11 - 3.08 (m, 1H), 1.49 (s, 9H), 1.19 (d, *J =* 6.7 Hz, 3H).

### Step 2: Preparation of tert-butyl (S)-4-(6-((diphenylmethylene)amino) pyridazin-3-yl)-2-methylpiperazine-1-carboxylate.

To a solution of tert-butyl (S)-4-(6-chloropyridazin-3-yl)-2-methylpiperazine-1-carboxylate (1.4 g, 4.5 mmol) in 1,4-dioxane (20 mL) were added Pd(OAc)₂ (412 mg, 0.45 mmol), Cs₂CO₃ (3.0 g, 9.0 mmol), BINAP (518 mg, 0.9 mmol) and diphenylmethanimine (851 mg, 6.6 mmol), and the mixture was stirred at 80°C overnight under nitrogen. The reaction mixture was diluted with water and extracted with EtOAc. The organic layer was dried over sodium sulfate, filtered and concentrated in vacuo. The residue was purified by column chromatography (PE/EA=5:1) to give title product (590 mg, Y: 29.5%) as a light yellow solid. ESI-MS (M+H)⁺: 458.2

### Step 3: Preparation of tert-butyl (S)-4-(6-aminopyridazin-3-yl)-2-methylpiperazine-1-carboxylate.

To a solution of tert-butyl (S)-4-(6-((diphenylmethylene)amino) pyridazin-3-yl)-2-methylpiperazine-1-carboxylate (590 mg, 1.3 mmol) in MeOH (10 mL) was added NaOAc (320 mg, 3.9 mmol), NH₂OH.HCl (449 mg, 6.5 mmol) and the mixture was stirred at rt overnight. The resultant reaction mixture was neutralized with saturated aqueous sodium hydrogen carbonate solution (25 mL), and extracted with ethyl acetate. The organic phases were combined, dried over anhydrous magnesium sulfate and concentrated under reduced pressure. The crude product was washed with *tert*-butyl methyl ether (10 mL) to give title product (330 mg, crude) as a yellow solid. ESI-MS (M+H)⁺: 294.2. ¹H NMR (400 MHz, CDCl₃) δ 6.89 (d, *J=* 9.6 Hz, 1H), 6.74 (d, *J =* 9.5 Hz, 1H), 4.47 - 4.30 (m, 3H), 3.96 - 3.94 (m, 2H), 3.81 - 3.78 (m, 1H), 3.25 - 3.22 (m, 1H), 3.13 - 3.07 (m, 1H), 2.92 - 2.87 (m, 1H), 1.48 (s, 9H), 1.23 (d, *J=* 6.7 Hz, 3H).

### Example 32. 7-ethoxy-2-methylimidazo[1,2-a] pyridine-6-carboxylate.

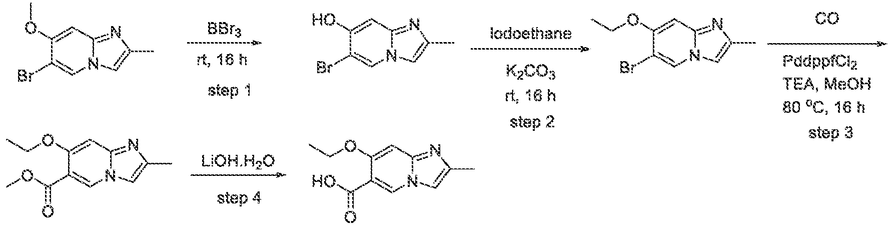

### Step 1 : Preparation of 6-bromo-2-methylimidazo[1,2-a]pyridin-7-ol

A solution of 6-bromo-7-methoxy-2-methylimidazo[1,2-a]pyridine (5 g, 21 mmol) in BBr₃ (210 mL, 1M in DCM) was stirred at rt overnight. The reaction mixture was diluted with saturated sodium bicarbonate and concentrated, the residue was purified by column chromatography (DCM/MeOH=20:1) to give title product (2.8 g, Y: 58%) as a light yellow solid. ESI-MS (M+H)⁺:226.9. ¹HNMR (400 MHz, CDCl₃) δ 8.56 (s, 1H), 7.28 (s, 1H), 6.47 (s, 1H), 2.33 (s, 3H).

### Step 2 : Preparation of 6-bromo-7-ethoxy-2-methylimidazo[1,2-a]pyridine.

To a solution of 6-bromo-2-methylimidazo[1,2-a]pyridin-7-ol (0.8 g, 3.5 mmol) in DMF (10 mL) was added iodoethane (0.6 ml, 7.1 mmol) and K₂CO₃ (0.966 mg, 7 mmol). The mixture was stirred at RT overnight. The reaction mixture was diluted with water and extracted with EtOAc. The organic layer was washed with brine, dried over sodium sulfate, filtered and concentrated in vacuo. The residue was purified by silica gel column (EA/PE=1:1) to give title product (255 mg, Y: 28.2%) as a gray solid. ESI-MS (M+H)⁺: 254.8/256.8. ¹HNMR (400 MHz, CDCl₃) δ 8.07 (s, 1H), 7.06 (s, 1H), 6.73 (s, 1H), 4.18 - 3.92 (m, 2H), 2.32 (d, *J =* 0.7 Hz, 3H), 1.44 (t, *J =* 7.0 Hz, 3H).

### Step 3: Preparation of methyl 7-ethoxy-2-methylimidazo[1, 2-a]pyridine-6-carboxylate.

To a solution of 6-bromo-7-ethoxy-2-methylimidazo[1,2-a]pyridine (255 mg, 1.00 mmol) in MeOH (10 mL) was added TEA (303 mg, 3.00 mmol) and Pd(dppf)Cl₂ (73.2 mg, 0.1mmol). The mixture was charged with CO for three times and stirred at 80°C for 16 h. The reaction mixture was diluted with ethyl acetate (100 mL) and washed with water (50 mL) and brine (50 mL). The organic layer was dried over sodium sulfate, filtered and concentrated in vacuo to give title product (220 mg, crude) as a gray solid. ESI-MS (M+H) ⁺:235.0.

### Step 4 : Preparation of 7-ethoxy-2-methylimidazo[1,2-a]pyridine-6-carboxylic acid.

To a mixture of methyl 7-ethoxy-2-methylimidazo[1,2-a]pyridine-6-carboxylate (210 mg, 0.9 mmol) in MeOH/water (12 mL, 5:1) was added LiOH.H₂O (43 mg, 1.8 mmol). The mixture was stirred at rt for 2 h. After concentration, the residue was adjusted to pH = 5 with 1M HCl. The residue was purified by reverse phase column to give title product (100 mg) as a gray solid. ESI-MS (M+H)⁺: 221.0.

### Example 33. 7-isopropoxy-2-methylimidazo[1,2-a]pyridine-6- carboxylic acid

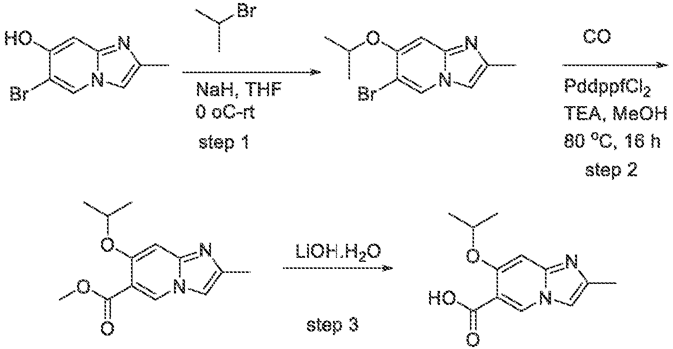

### Step 1: preparation of 6-bromo-7-isopropoxy-2-methylimidazo[1,2-a]pyridine.

To a mixture of 6-bromo-2-methylimidazo [1,2-a]pyridin-7-ol (800 mg, 3.51 mmol) in DMF (10 mL) was added NaH (280 mg, 7.02 mmol) at 0 °C, the mixture was stirred at 0 °C for 0.5 h. Then 2-bromopropane (1.8 g, 10.53 mmol) was added and the reaction mixture was stirred at rt overnight. The reaction mixture was diluted with water and extracted with EtOAc. The organics were washed with brine, dried with Na₂SO₄ and concentrated in vacuo. The residue was purified by silica gel column chromatography (EA) to give title compound (900 mg, 95 % yield), ESI-MS (M+H)⁺: 269.0. ¹H NMR (400 MHz, DMSO-d₆) δ 8.77 (s, 1H), 7.44 (s, 1H), 6.97 (s, 1H), 4.77 - 4.73 (m, 1H), 2.26 (s, 3H), 1.31 (d7.2 Hz, 6H).

### Step 2: preparation of methyl 7-isopropoxy-2-methylimidazo[1,2-a]pyridine- 6-carboxylate.

To a mixture of 6-bromo-7-isopropoxy-2-methylimidazo [1,2-a]pyridine (400 mg, 1.48 mmol) in MeOH (20 mL) were added TEA (449 mg, 4.44 mmol) and Pd(dppf)Cl₂ (108 mg, 0.15 mmol). The resulting mixture was charged with CO for three times and stirred overnight at 80 °C under CO. The mixture was allowed to cool down to room temperature and concentrated. The residue was used directly without purification. ESI-MS (M+H)⁺: 249.0.

### Step 3: preparation of 7-isopropoxy-2-methylimidazo[1,2-a]pyridine-6-carboxylic acid.

To a mixture of methyl 7-isopropoxy-2-methylimidazo [1,2-a]pyridine-6-carboxylate (400 mg, 1.61 mmol) in THF (5 mL) and H₂O (1 mL) was added LiOH.H₂O (145 mg, 6.04 mmol). The reaction mixture was stirred for 2 h at room temperature. The mixture was exacted with DCM (15 mL). The aqueous phase was adjusted to pH to 5 with HCl (1M). The aqueous phase was concentrated to provide as a black solid and the solid was washed with DCM. The organic phase was concentrated to give title product (420 mg, crude). ESI-MS (M+H)⁺: 235.1

### Example 34. 7-cyclobutoxy-2-methylimidazo[1,2-a] pyridine-6-carboxylic acid

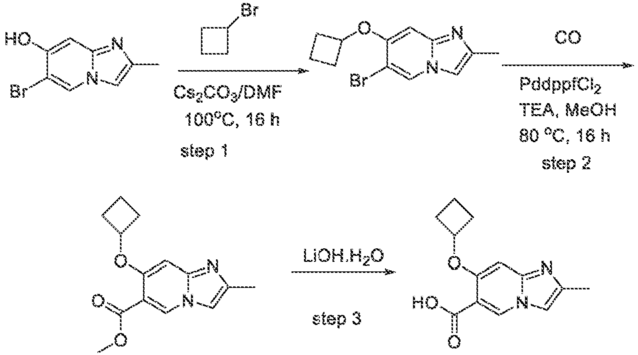

### Step 1: preparation of 6-bromo-7-cyclobutoxy-2-methylimidazo[1,2-a]pyridine.

A mixture of 6-bromo-2-methylimidazo[1,2-a]pyridin-7-ol (800 mg, 3.51 mmol), bromocyclobutane (2.37 g, 17.54 mmol), KI (291 mg, 1.75 mmol) and Cs₂CO₃ (3.43 g, 10.52 mmol) in DMF (15 mL) was stirred at 100 °C for 16 h. The reaction mixture was diluted with water (30 mL) and extracted with EtOAc (60 mL x2), The combined organic layer was washed with brine, dried with Na₂SO₄ and concentrated in vacuo. The residue was purified by silica gel column chromatography (PE/EA=2:1) to give title compound (630 mg, 64% yield) as a yellow solid. ESI-MS(M+H)⁺: 281.0. ¹H NMR (400 MHz, CDCl₃) δ 8.14 (s, 1H), 7.12 (s, 1H), 6.68 (s, 1H), 4.71 (p, *J* = 7.1 Hz, 1H), 2.60 - 2.49 (m, 2H), 2.38 (s, 3H), 2.32 - 2.20 (m, 2H), 1.97 - 1.88 (m, 1H), 1.78 - 1.75 (m, 1H).

### Step 2: preparation of methyl 7-cyclobutoxy-2-methylimidazo [1,2-a]pyridine-6-carboxylate.

To a mixture of 6-bromo-7-cyclobutoxy-2- methylimidazo[1,2-a]pyridine (630 mg, 2.24 mmol) in MeOH (20 mL) were added TEA (679 mg, 6.72 mmol) and Pd(dppf)Cl₂ (183 mg, 0.22 mmol). The resulting mixture was stirred overnight at 80 °C under CO. The mixture was allowed to cool down to room temperature and concentrated. The residue was used directly without purification. ESI-MS (M+H)⁺: 260.9

### Step 3: preparation of 7-cyclobutoxy-2-methylimidazo[1,2-a]pyridine-6-carboxylic acid.

To a mixture of methyl 7-cyclobutoxy-2-methylimidazo[1,2-a]pyridine-6-carboxylate (300 mg, 1.14 mmol) in THF (5 mL) and H₂O (1 mL) was added LiOH.H₂O (138 mg, 5.74 mmol). The reaction mixture was stirred for 2 h at room temperature. The mixture was exacted with DCM (15 mL). The aqueous phase was adjusted pH to 5 with HCl (1M). The aqueous phase was concentrated to provide as a black solid and the solid was washed with DCM. The organic phase was concentrated to give title product (450 mg, crude). ESI-MS (M+H) ⁺: 247.2.

### Example 35. tert-butyl (S)-4-(5-aminopyrazin-2-yl)-2-methylpiperazine-1-carboxylate

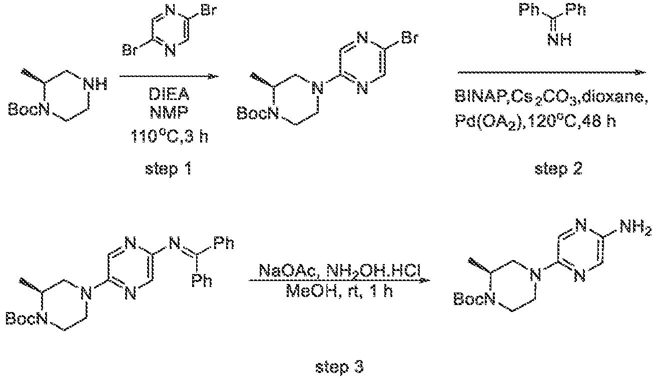

### Step 1: Preparation of tert-butyl (S)-4-(5-bromopyrazin-2-yl)-2-methylpiperazine-1-carboxylate

To a suspension of 2,5-dibromopyrazine (2.95 g, 12.50 mmol) and tert-butyl (S)-2-methylpiperazine- 1-carboxylate (3.00 g, 15.00 mmol) in NMP (60 mL) was added DIEA (3.23 g, 25.00 mmol), the reaction mixture was stirred for 3 h at 110 °C. After cooling to rt, the mixture was diluted with water 150 mL and 150 mL of EA. The EA layer was separated, washed with brine and water, dried over Na₂SO₄ and concentrated to dryness. The crude was purified by silica gel column (10~50% EA in PE). The product was obtained as a yellow solid (3.30 g, Y: 62%). ¹H NMR (400 MHz, CDCl₃) δ 8.12 (d, *J =* 1.2 Hz, 1H), 7.84 (d, J= 1.2 Hz, 1H), 4.35 - 4.34 (m, 1H), 4.07 - 4.02 (m, 1H), 3.97 - 3.93 (m, 2H), 3.30 - 3.21 (m, 2H), 3.08 - 3.01 (m, 1H), 1.48 (s, 9H), 1.18 (d, *J=* 6.4 Hz, 3H).

### Step 2: Preparation of tert-butyl (S)-4-(5-((diphenylmethylene) amino)pyrazin-2-yl)-2-methylpiperazine-1-carboxylate.

A mixture of tert-butyl (S)-4-(5-bromopyrazin-2-yl)-2-methylpiperazine-1-carboxylate (3.30 g, 9.27 mmol), diphenylmethanimine (2.01 g, 11.12 mmol), Pd(OAc)z (104 mg, 0.46 mmol), BINAP (577 mg, 0.93 mmol) and Cs₂CO₃ (6.04 g, 18.54 mmol) in 1,4-dioxane (80 mL) was purged with N₂ for three times at room temperature. Then the reaction mixture was stirred at 120 °C for 48 h. After cooling to rt, the mixture was diluted with water (150 mL) and EA (150 mL). The EA layer was separated, washed with brine and water, dried over Na₂SO₄, concentrated to dryness. The crude was purified by silica gel column (10-50% EA in PE). The product was obtained as a yellow solid (1.50 g, yield: 36%) ESI-MS: [M+H] ⁺ 458.2

### Step 3: Preparation of tert-butyl (S)-4-(5-aminopyrazin-2-yl)-2- methylpiperazine-1-carboxylate.

A mixture of tert-butyl (S)4-(5-((diphenylmethylene)amino)pyrazin-2-yl)-2-methylpiperazine-1-carboxylate (1.5 g, 3.28 mmol), NH₂OH.HCl (1.13 g, 16.4 mmol) and NaOAc (807 mg, 9.84 mmol) in MeOH (30 mL) was stirred for 1 h at rt. After diluting with water, the mixture was extracted with EA (30 mL x 2). The combined organics was washed with brine, dried over Na₂SO₄. After concentration, the crude was used to next step without further purification. ¹H NMR (400 MHz, CDCl₃) δ 7.68 (d, *J =* 1.6 Hz, 1H), 7.64 (d, *J =* 1.6 Hz, 1H), 4.39 - 4.32 (m, 1H), 4.05 (br s, 2H), 3.97 - 3.93 (m, 1H), 3.89 - 3.84 (m, 1H), 3.75 - 3.71 (m, 1H), 3.27 - 3.20 (m, 1H), 3.00 - 2.97 (m, 1H), 2.82 - 2.76 (m, 1H), 1.48 (s, 9H), 1.25 (d, *J=* 6.8 Hz, 3H).

### Example 36. 7-ethoxy-2-methyl-N-(5-(piperazin-1-yl)pyridin-2-yl) imidazo[1,2-a]pyridine-6-carboxamide hydrochloride (Compound 130).

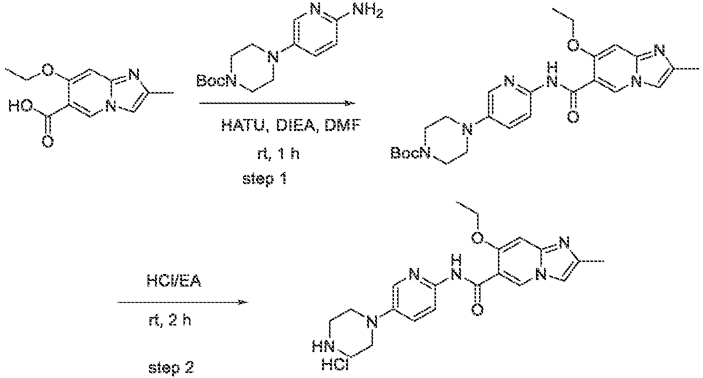

### Step 1: Preparation of tert-butyl 4-(6-(7-ethoxy-2-methylimidazo[1,2-a]pyridine-6-carboxamido)pyridin-3-yl)piperazine-1-carboxylate.

To a mixture of 7-ethoxy-2-methy limidazo[1,2-a]pyridine-6-carboxylic acid (100 mg, 0.45 mmol) in DMF (4 mL) were added HATU (343 mg,0.9 mmol), DIEA(291 mg 0.9 mmol), tert-butyl 4-(6-aminopyridin-3-yl) piperazine-1-carboxylate (63 mg 0.23 mmol). The mixture was stirred at rt for 2 h. The crude was purified by reverse phase column to give title product (20 mg, Y: 9.2%) as a gray solid. ESI-MS (M+H)⁺: 481.57.

### Step 2: Preparation of 7-ethoxy-2-methyl-N-(5-(piperazin-1-yl)pyridin-2-yl) imidazo[1,2-a]pyridine-6-carboxamide hydrochloride.

A mixture of tert-butyl 4-(6-(7-ethoxy-2-methylimidazo[1,2-a pyridine-6-carboxamido)pyridin-3-yl)piperazine-1-carboxylate (70 mg, 0.145 mmol) in 3M HCl/EA (2 mL) was stirred at rt for 2 h. After concentration, the residue was dissolved in water (5 mL) and lyophilized to give title product (8 mg, Y: 47.1 %) as a yellow solid. ESI-MS (M+H)⁺: 382. ¹H NMR (400 MHz, MeOD-d₄) δ 9.20 (s, 1H), 8.07 - 8.04 (m, 2H), 7.89 - 7.87 (m, 2H), 7.36 (s, 1H), 4.47 (q, *J=* 6.8 Hz, 2H), 3.55 - 3.53 (m, 4H), 3.44 - 3.42 (m, 4H), 2.51 (s, 3H), 1.60 (t, *J* = 6.8 Hz, 3H).

### Example 37. 7-isopropoxy-2-methyl-N-(5-(piperazin-1-yl)pyridin-2-yl)imidazo[1,2-a]pyridine-6-carboxamide hydrochloride (Compound 129)

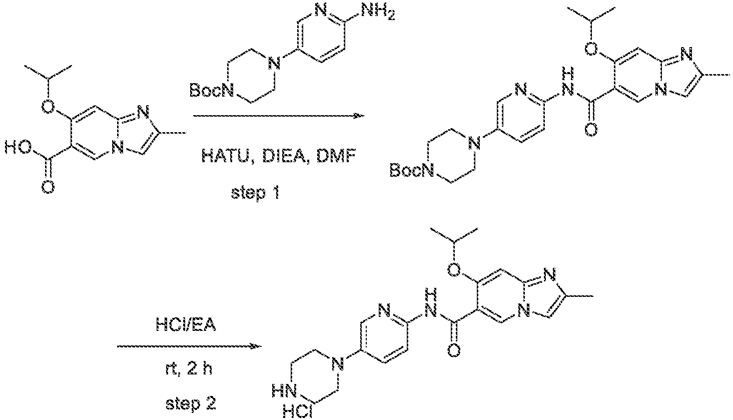

### Step 1: preparation of tert-butyl 4-(6-(7-isopropoxy-2-methylimidazo[1,2-a]pyridine-6-carboxamido)pyridin-3-yl)piperazine-1-carboxylate.

To a mixture of tert-butyl 4-(6- aminopyridin-3-yl) piperazine-1-carboxylate (100 mg, 0.36 mmol) in DMF (5 mL) were added 7-isopropoxy-2-methylimidazo [1,2-a]pyridine-6-carboxylic acid (100 mg, 0.43 mmol), DIEA (140 mg, 1.08 mmol) and HATU (202 mg, 0.54 mmol). The mixture was stirred at rt for 5 h and quenched with water (10 mL). The precipitate was filtered and purified by reverse phase column (0.1% FA in water/CH₃CN) to give title product (30 mg, 34%) as a yellow solid. ESI-MS (M+H)⁺: 495.1

### Step 2: preparation of 7-isopropoxy-2-methyl-N-(5-(piperazin-1-yl)pyridin-2-yl)imidazo[1,2-a]pyridine-6-carboxamide hydrochloride

Tert-butyl 4-(6-(7-isopropoxy-2-methylimidazo [1,2-a]pyridine-6-carboxamido)pyridin-3-yl)piperazine-1-carboxylate (30 mg, 0.11 mmol) was dissolved in 3M HCl /EA (5 mL) at rt. The mixture was stirred for 1 h at rt. The precipitate was filtered and the solid was dried to give title product (3.0 mg, yield: 13%) as a yellow solid. ESI-MS (M+H)⁺: 395.0 ¹H NMR (400 MHz, MeOD-d₄) δ 9.18 (s, 1H), 8.20 - 8.07 (m 2H), 7.87 - 7.75 (m, 2H), 7.38 (d, *J =* 5.5 Hz, 1H), 5.09 - 5.03 (m, 1H), 3.66 - 3.57 (m, 4H), 3.47 - 3.40 (m, 4H), 2.52 (s, 3H), 1.53 (dd, *J=* 5.9, 3.0 Hz, 6H).

### Example 38. 7-cyclobutoxy-2-methyl-N-(5-(piperazin-1-yl)pyridin-2-yl) imidazo[1,2-a]pyridine-6-carboxamide hydrochloride (Compound 128)

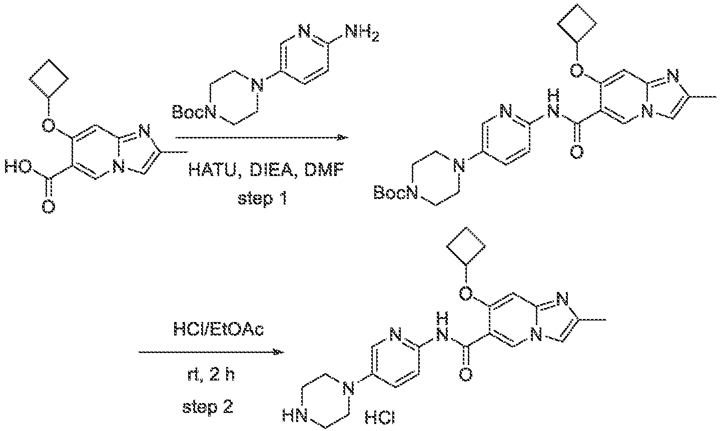

### Step 1: preparation of tert-butyl 4-(6-(7-isopropoxy-2-methylimidazo[1,2-a] pyridine-6-carboxamido)pyridin-3-yl)piperazine-1-carboxylate.

To a mixture of tert-butyl 4-(6-aminopyridin-3-yl)piperazine-1-carboxylate (100 mg, 0.358 mmol) in DMF (5 mL) were added 7-cyclobutoxy-2-methylimidazo [1,2-a]pyridine-6-carboxylic acid (133 mg, 0.54 mmol), DIEA (140 mg, 1.07 mmol) and HATU (205 mg, 0.54 mmol). The mixture was stirred at rt for 5 h and diluted with water (10 mL). The precipitate was filtered and purified by reverse phase column (0.1% FA in water/CH₃CN) to give title product (20 mg, 11%) as a yellow solid. ESI-MS (M+H)⁺: 507.2. ¹H NMR (400 MHz, MeOD-d₄) δ 9.06 (s, 1H), 8.25 - 8.14 (m, 2H), 8.06 (d, *J=* 2.8 Hz, 1H), 7.60 (s, 1H), 7.48 (dd, *J =* 9.1, 3.0 Hz, 1H), 6.83 (s, 1H), 5.03 (p, *J =* 6.9 Hz, 1H), 3.60 - 3.58 (m, 4H), 3.18 - 3.15 (m, 4H), 2.66 - 2.64 (m, 2H), 2.41 - 2.39 (m, 5H), 2.01 - 1.83 (m, 2H), 1.48 (s, 9H).

### Step 2: preparation of 7-cyclobutoxy-2-methyl-N-(5-(piperazin-1-yl)pyridin-2-yl) imidazo[1,2-a]pyridine-6-carboxamide hydrochloride

To a solution of tert-butyl 4-(6-(7-cyclobutoxy-2-methylimidazo [1,2-a] pyridine-6-carboxamido)pyridin-3-yl)piperazine-1-carboxylate (20 mg, 0.04 mmol) in EA (1 mL) was added 3M HCl/EA (5 mL) at rt. The mixture was stirred for 2 h at rt. The precipitate was filtered and the solid was dried under vacuum to give title product (4 mg, yield: 25 %) as a yellow solid. ESI-MS (M+H)⁺: 407.0. ¹H NMR (400 MHz, MeOD) δ 9.20 (s, 1H), 8.29 - 8.17 (m, 1H), 8.07 (d, *J =* 2.4 Hz, 1H), 7.87 (s, 1H), 7.84 - 7.75 (m, 1H), 7.17 (d, *J =* 3.6 Hz, 1H), 5.11 (p, *J=* 7.1 Hz, 1H), 3.73 - 3.60 (m, 4H), 3.50 - 3.41 (m, 4H), 2.71 - 2.58 (m, 2H), 2.52 (d, *J* = 0.9 Hz, 3H), 2.43 - 2.30 (m, 2H), 2.04 - 1.76 (m, 2H).

### Example 39. (S)-6-methoxy-2-methyl-N-(6-(3-methylpiperazin-1-yl) pyridazin-3-yl)-2H-indazole-5-carboxamide hydrochloride (Compound 125).

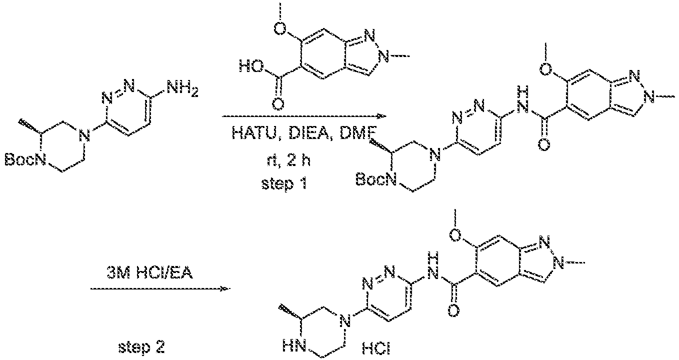

### Step 1 : Preparation of tert-butyl (S)-4-(6-(6-methoxy-2-methyl-2H-indazole-5-carboxamido)pyridazin-3-yl)-2-methylpiperazine-1-carboxylate.

To a mixture of 6-methoxy-2H-indazole-5-carboxylic acid (72 mg, 0.35 mmol) and tert-butyl (S)-4-(6-aminopyridazin-3-yl)-2-methylpiperazine-1-carboxylate (100 mg, 0.35 mmol) in DMF (5 mL) were added HATU (267 mg, 0.7 mmol), DIEA (226 mg 1.75 mmol). The mixture was stirred at rt for 2 h. After concentration, the crude was purified by prep-HPLC (0.05%FA in water / CH₃CN) to give title product (50 mg, Y: 30.5%) as a yellow solid. ESI-MS (M+H)⁺: 482.1.

### Step 2: Preparation of (S)-6-methoxy-2-methyl-N-(6-(3-methylpiperazin-1-yl) pyridazin-3-yl)-2H-indazole-5-carboxamide hydrochloride.

A mixture of tert-butyl (S)-4-(6-(6-methoxy-2-methyl-2H-indazole-5-carboxamido)pyridazin-3-yl)-2-methylpiperazine-1-carboxylate (50 mg, 0.145 mmol) in 3M HCl/EA (4 mL) was stirred at rt for 2 h. The precipitate was filtered and the solid was dried in vacuo to give title product (19 mg, Y: 44.2%) as a yellow solid. ESI-MS (M+H)⁺:382.0. ¹H NMR (400 MHz, MeOD-d₄) δ 8.52 (s, 1H), 8.47 (s, 1H), 8.25 - 8.18 (m, 2H), 7.19 (s, 1H), 4.49 (d, *J =* 14.0 Hz, 2H), 4.23 (s, 3H), 4.10 (s, 3H), 3.61 - 3.46 (m, 3H), 3.33 - 3.31 (m, 2H), 1.44 (d, *J* = 7.2 Hz, 3H).

### Example 40. (S)-2,8-dimethyl-N-(6-(3-methylpiperazin-1-yl)pyridazin-3-yl)imidazo[1,2-a]pyrazine-6-carboxamide hydrochloride (Compound 124).

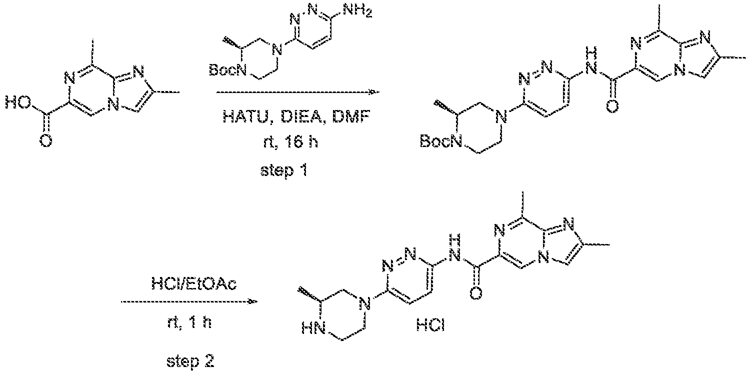

### Step 1: tert-butyl (S)-4-(6-(2,8-dimethylimidazo[1,2-a]pyrazine-6-carboxamido]pyridazin-3-yl)-2-methylpiperazine-1-carboxy/ate.

To a stirred solution of 2,8-dimethylimidazo[1,2-a]pyrazine-6- carboxylic acid (50 mg, 0.26 mmol), tert-butyl (S)-4-(6-aminopyridazin-3-yl)-2- methylpiperazine-1-carboxylate (84 mg, 0.28 mmol) and DIEA (100 mg, 0.78 mmol) in DMF (5 mL) was added HATU (128 mg, 0.33 mmol) at rt. The mixture was stirred at rt for 16 hrs. The reaction mixture was treated with EA/Water (50 mL, 1:1). The organic phase was separated and the aqueous phase was extracted with EA (50 mLx1). The combined organic layer was dried over sodium sulfate. After concentration, the crude was purified by pre-HPLC (0.05% FA in water /CH₃CN) to provide title product (15 mg, Y: 12%) as an off-white solid. ESI-MS (M+H)⁺: 467.2. ¹H NMR (400 MHz, CDCl₃) δ 8.78 (s, 1H), 8.40 (d, *J* = 9.8 Hz, 1H), 7.51 (s, 1H), 7.07 (d, *J=* 9.8 Hz, 1H), 4.35 - 4.07 (m, 2H), 3.95 - 3.92 (m, 2H), 3.30 -3.25 (m, 2H), 3.09 - 3.07 (m, 1H), 2.83 (s, 3H), 2.47 (s, 3H), 1.42 (s, 9H), 1.16 (d, *J=* 6.8 Hz, 3H).

### Step 2 : Preparation of (S)-2,8-dimethyl-N-(6-(3-methylpiperazin-1-yl)pyridazin-3-yl)imidazo[1,2-a]pyrazine-6-carboxamide hydrochloride.

To a solution of tert-butyl (S)-4-(6-(2,8-dimethy limidazo[1,2-a]pyrazine-6-carboxamido)pyridazin-3-yl)-2-methylpiperazine-1-carboxylate (15 mg, 0.03 nmol) in EA (1 mL) was added HCl-EA (3 M, 1.0 mL) at 0 °C and the mixture was allowed to warm to rt and stirred for 1 h. The solid was collected by filtration and washed with EA (2 mL x 3). The solid was dried under vacuum at 55 °C to provide title product as light grey solid (5 mg, yield: 45%). ESI-MS (M+H)⁺: 367.0. ¹H NMR (400 MHz, MeOD-d₄) δ 9.48 (d, *J* = 4.4 Hz, 1H), 8.56 (d, *J =* 9.9 Hz, 1H), 8.29 (d, *J=* 3.9 Hz, 1H), 8.14 - 8.07 (m, 1H), 4.50 (d, *J =* 13.8 Hz, 2H), 3.57 - 3.51 (m, 3H), 3.37 - 3.35 (m, 2H), 3.03 (s, 3H), 2.68 (s, 3H), 1.45 (d, *J=* 6.5 Hz, 3H).

### Example 41. 6-methoxy-2-methyl-N-(6-(piperazin-1-yl)pyridazin-3-yl)-2H-indazole-5-carboxamide hydrochloride (Compound 123).

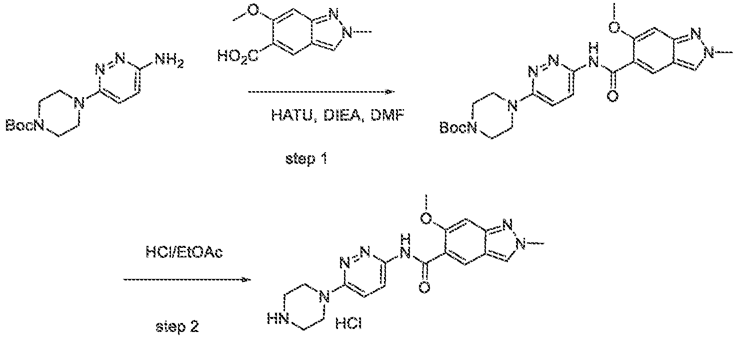

### Step 1: tert-butyl 4-(6-(6-methoxy-2-methyl-2H-indazole-5-carboxamido)pyridazin-3-yl)piperazine-1-carboxylate.

To a stirred solution of 6-methoxy-2-methyl-2H- indazole-5-carboxylic acid (120 mg, 0.58 mmol), tert-butyl 4-(6-aminopyridazin-3-yl) piperazine-1-carboxylate (180 mg, 0.64 mmol) and DIEA (224 mg, 1.74 mmol) in DMF (5 mL) was added HATU (286 mg, 0.75 mmol) at rt. The mixture was stirred at rt for 2 hours. The reaction mixture was treated with EA/Water (50 mL, 1:1). The organic phase was separated and the aqueous phase was extracted with EA (50 mLx1). The combined organic layer was dried over sodium sulfate. After concentration, the crude was purified by prep-HPLC (0.05% FA in water /CH₃CN) to provide title product (60 mg, Y: 22%) as an off-white solid. ESI-MS (M+H) ⁺: 468.2.

### Step 2: Preparation of 6-methoxy-2-methyl-N-(6-(piperazin-1-yl)pyridazin-3-yl)-2H-indazole-5-carboxamide hydrochloride.

To a solution of tert-butyl 4-(6-(6-methoxy-2-methyl-2H-indazole-5-carboxamido)pyridazin-3-yl)piperazine-1-carboxylate (50 mg, 0.107 nmol) in EA (1 mL) was added HCl/EA (3 M, 1.0 mL) at 0 °C and warmed to rt for 1 h . The solid was collected by filtration and washed with EA (2 mL x 3). The solid was concentrated in vacuo to provide title product as a yellow solid (15 mg, yield: 37%). ESI-MS (M+H)⁺: 368.1. ¹H NMR (400 MHz, DMSO-d₆) δ 10.98 (s, 1H), 9.53 (s, 2H), 8.47 (s, 1H), 8.38 (d, *J =* 9.8 Hz, 1H), 8.30 (s, 1H), 7.76 (d, *J* = 9.7 Hz, 1H), 7.15 (s, 1H), 4.15 (s, 3H), 3.98 (br s, 3H), 3.88 (br s, 4H), 3.24 (s, 4H).

### Example 42. (S)-7-cyclobutoxy-2-methyl-N-(6-(3-methylpiperazin-1-yl) pyridazin-3-yl)imidazo[1,2-a]pyridine-6-carboxamide hydrochloride (Compound 122).

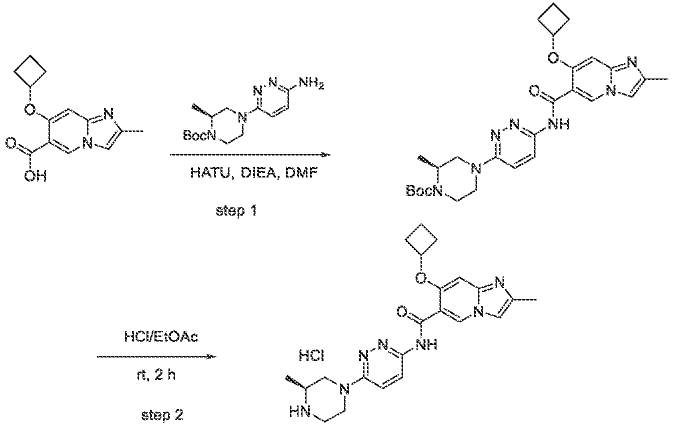

### Step 1: Preparation of tert-butyl (S)-4-(6-(7-cyclobutoxy-2-methylimidazo[1,2-a]pyridine-6-carboxamido)pyridazin-3-yl)-2-methylpiperazine-1-carboxylate.

To a mixture of 7-cyclobutoxy-2-methylimidazo[1,2-a]pyridine-6-carboxylic acid (95 mg, 0.38 mmol) and tert-butyl (S)-4-(6-aminopyridazin-3-yl)-2-methylpiperazine-1-carboxylate(85 mg, 0.29 mmol) in DMF (3 mL) were added HATU (165 mg, 0.44 mmol) and DIPEA (112 mg, 0.87 mmol). The mixture was stirred at RT overnight. Water (10 mL) was added to the mixture and filtered, the crude product was purified by prep-HPLC (0.05% NH₃H₂O in water /CH₃CN) to give title product (15 mg, Y: 9.9 %) as a yellow solid. ESI-MS (M+H)⁺: 522.3. ¹H NMR (400 MHz, CDCl₃) δ 10.68 (s, 1H), 8.98 (s, 1H), 8.42 (d, *J=* 9.8 Hz, 1H), 7.30 (s, 1H), 7.00 (d, *J=* 9.9 Hz, 1H), 6.83 (s, 1H), 4.97 - 4.87 (m, 1H), 4.38 (s, 1H), 4.17 (d, *J=* 12.2 Hz, 1H), 4.05 - 3.94 (m, 2H), 3.36 - 3.25 (m, 2H), 3.11 - 3.03 (m, 1H), 2.69 - 2.62 (m, 2H), 2.50 - 2.44 (m, 2H), 2.44 (s, 3H), 2.00 - 1.96 (m, 1H), 1.86 - 1.84 (m, 1H), 1.49 (s, 9H), 1.23 (d, *J=* 6.7 Hz, 3H).

### Step 2: Preparation of (S)-7-cyclobutoxy-2-methyl-N-(6-(3-methylpiperazin-1-yl) pyridazin-3-yl)imidazo[1,2-a]pyridine-6-carboxamide hydrochloride.

A mixture of tert-butyl (S)-4-(6-(7-cyclobutoxy-2-methylimidazo[1,2-a]pyridine-6-carboxamido)pyridazin-3-yl)-2-methylpiperazine-1-carboxylate (15 mg, 0.029 mmol) in 3M EtOAc/HCl (2 mL) was stirred at rt for 2 h. After concentration, the residue was dissolved in water (5 mL) and lyophilized to give title product (11.2 mg, Y: 85.1 %) as a yellow solid. ESI-MS (M+H)⁺: 422.2. ¹H NMR (400 MHz, CD₃OD) δ 9.19 (s, 1H), 8.51 (d, *J =* 10.0 Hz, 1H), 8.01 (d, *J =* 10.1 Hz, 1H), 7.85 (s, 1H), 7.15 (s, 1H), 5.15 - 5.09 (m, 1H), 4.51 - 4.39 (m, 2H), 3.64 - 3.52 (m, 3H), 3.41 - 3.33 (m, 2H), 2.70 - 2.59 (m, 2H), 2.51 (s, 3H), 2.43 - 2.31 (m, 2H), 2.02 -1.95 (mz, 1H), 1.89 - 1.82 (m, 1H), 1.45 (d, *J =* 6.6 Hz, 3H).

### Example 43. (S)-7-methoxy-2-methyl-N-(5-(3-methylpiperazin- 1-yl)pyrazin-2-yl)imidazo[1,2-a]pyridine-6-carboxamide hydrochloride (Compound 198).

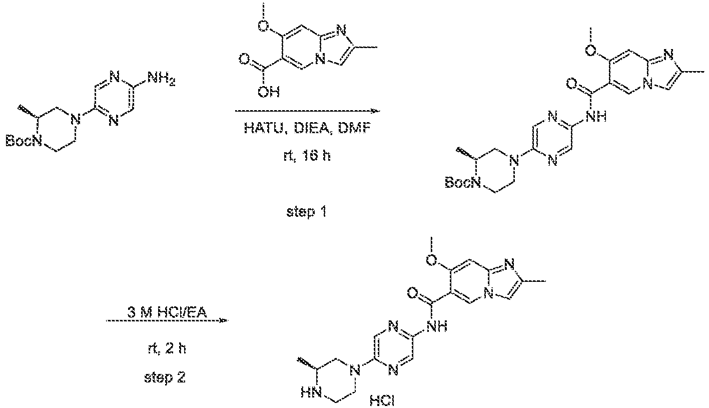

### Step 1: preparation of tert-butyl (S)-4-(5-(7-methoxy-2-methylimidozo[1,2-a] pyridine-6-carboxamido)pyrazin-2-yl)-2-methylpiperazine-1-carboxylate.

A mixture of tert-butyl (S)-4-(5-aminopyrazin-2-yl)-2-methylpiperazine -1-carboxylate (100 mg, 0.34 mmol), HATU (181 mg, 0.48 mmol), 7-methoxy-2-methylimidazo[1,2-a]pyridine-6-carboxylic acid (84 mg, 0.41 mmol) and DIEA (132 mg, 1.02 mmol) in DMF (5 mL) was stirred at r.t. for 16 h. The reaction mixture was diluted with water (20 mL), the precipitate was filtered and concentrated in vacuo. The residue was purification by reverse phase column (0.1% FA in water/CH₃CN) to give title compound (40 mg, 24% yield) as a yellow solid. ESI-MS (M+H)⁺: 482.2. ¹H NMR (400 MHz, CDCl₃) δ 9.95 (s, 1H), 9.16 (s, 1H), 8.99 (s, 1H), 7.83 (s, 1H), 7.30 (s, 1H), 6.92 (s, 1H), 4.40 - 4.34 (m, 1H), 4.10 (s, 3H), 4.09 - 4.07 (m, 1H), 3.99 - 3.95 (m, 2H), 3.31 - 3.23 (m, 2H), 3.05 - 2.98 (m, 1H), 2.43 (s, 3H), 1.49 (s, 9H), 1.22 (d, *J =* 6.8 Hz, 3H).

### Step 2: Preparation of (S)-7-methoxy-2-methyl-N-(5-(3-methylpiperazin- 1-yl)pyrazin-2-yl)imidazo[1,2-a]pyridine-6-carbaxamide hydrochloride.

Tert-butyl (S)-4-(5-(7-methoxy-2-methylimidazo[1,2-a]pyridine-6-carboxamido)pyrazin-2-yl)-2-methylpiperazine-1-carboxylate (40 mg, 0.044 mmol) was dissolved in 3M HCl/EA (4 mL) and the mixture was stirred at r.t for 2 h. The solid was collected by filtration and washed EA three times (3 mL x 3) to afford the title compound as hydrochloride salt (20 mg, 59% yield). ESI-MS (M+H)⁺: 382.0. ¹H NMR (400 MHz, DMSO-d₆) δ 10.82 (s, 1H), 9.59 (br s, 1H), 9.48 (br s, 1H), 9.19 (s, 1H), 8.93 (s, 1H), 8.27 (s, 1H), 7.95 (s, 1H), 7.33 (s, 1H), 4.35 - 4.31 (m, 2H), 4.07 (s, 3H), 3.38 - 3.25 (m, 3H), 3.09 - 3.03 (m, 2H), 2.46 (s, 3H), 1.33 (d, *J =* 6.4 Hz, 3H).

### Example 44. (S)-7-isopropoxy-2-methyl-N-(6-(3-methylpiperazin- 1-yl)pyridazin-3-yl)imidazo[1,2-a]pyridine-6-carboxamide (Compound 199).

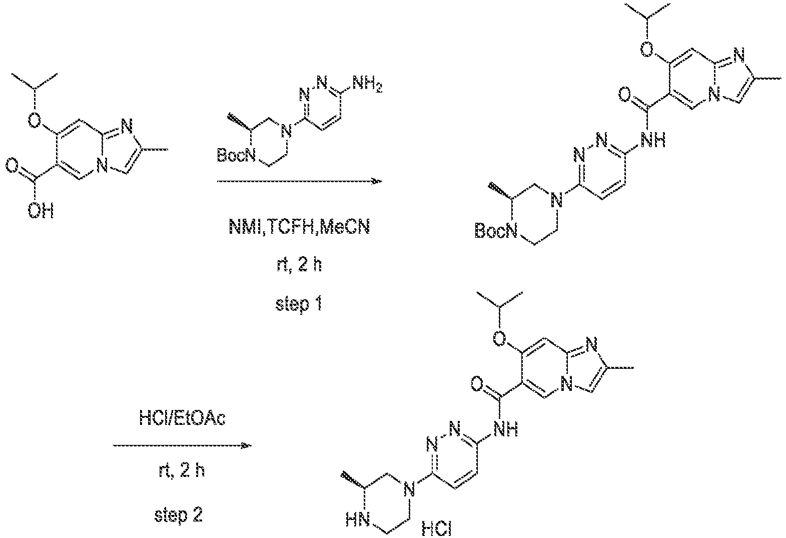

### Step 1: tert-butyl (259-4-(6-(7-isopropoxy-2-methylimidazo[1, 2-a] pyridine-6-carboxamido)pyridazin-3-yl)-2-methylpiperazine-1-carboxylate.

To a stirred solution of 7-isopropoxy-2-methylimidazo[1,2-a]pyridine-6-carboxylic acid (100 mg, 0.427 mmol), tert-butyl (S)-4-(6-aminopyridazin-3-yl)-2-methylpiperazine -1-carboxylate (125 mg, 0.427 mmol) and NMI (105 mg, 1.281 mmol) in MeCN (5 mL) was added TCFH (180 mg, 0.641 mmol) at rt. The mixture was stirred at rt for 2 h. The reaction mixture was treated with EA/water (50 mL, 1:1). The organic phase was separated and the aqueous phase was extracted with EA (50 mL). The combined organic layer was dried over sodium sulfate. After concentration, the crude was purified by prep-HPLC (0.05% NH₃.H₂O in water / CH₃CN) to provide title product (60 mg, Y: 27%) as off-white solid. ESI-MS (M+H)⁺: 510.3. ¹H NMR (400 MHz, CDCl₃) δ 10.71 (s, 1H), 9.01 (s, 1H), 8.41 (d, *J* = 9.8 Hz, 1H), 7.30 (s, 1H), 7.13 (s, 1H), 7.02 - 6.98 (m, 1H), 4.94 - 4.86 (m, 1H), 4.17 (d, *J =* 12.8 Hz, 1H), 4.04 - 3.95 (m, 2H), 3.36 - 3.27 (m, 2H), 3.11 - 3.03 (m, 1H), 2.48 (s, 3H), 2.01 (s, 1H), 1.62 (s, 3H), 1.61 (s, 3H), 1.49 (s, 9H), 1.22 (d, *J =* 6.7 Hz, 3H).

### Step 2 : Preparation of (S)-7-isopropoxy-2-methyl-N-(6-(3-methylpiperazin- 1-yl)pyridazin-3-yl)imidazo[1,2-a]pyridine-6-carboxamide.

To a solution of tert-butyl (S)-4-(6-(7-isopropoxy-2-methylimidazo[1,2-a]pyridine-6-carboxamido) pyridazin-3-yl)-2-methylpiperazine-1-carboxylate (60 mg, 0.177 mmol) in EA (1 mL) was added 3M HCl/EA (1 mL), the mixture was stirred at r.t for 2 h. The solid was collected by filtration and washed EA three times (3 mL x 3) to afford the title compound as hydrochloride salt (25 mg, yield: 52%). ESI-MS (M+H)⁺: 410.1. ¹H NMR (400 MHz, DMSO-d₆) δ 11.23 (s, 1H), 9.97 - 9.75 (m, 2H), 9.26 (s, 1H), 8.29 (d, *J =* 9.3 Hz, 1H), 7.98 (s, 1H), 7.75 (d, *J* = 10.0 Hz, 1H), 7.37 (s, 1H), 5.06 - 4.97 (m, 1H), 4.45 - 4.33 (m, 2H), 3.51 - 3.42 (m, 1H), 3.37 - 3.35 (m, 2H), 3.27 - 3.17 (m, 1H), 3.14 - 3.03 (m, 1H), 2.46 (s, 3H), 1.41 (d, *J =* 6.0 Hz, 6H), 1.

### Example 45. Preparation of compounds C highlighted in Table 2.

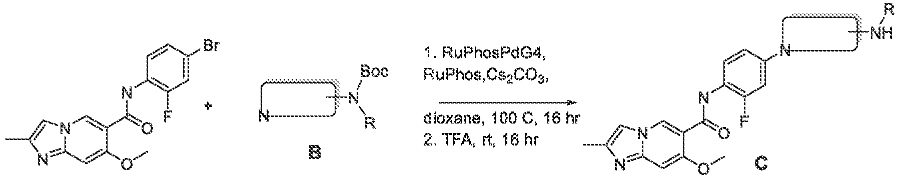

To a mixture of 1 eq. of N-(4-bromo-2-fluorophenyl)-7-methoxy-2-methylimidazo[1,2-a]pyridine-6-carboxamide and 1.5 eq. Amine (B) in 1ml Dioxane under an inert atmosphere were added 0.1 eq. RuPhosPdG4, 0.1 eq. RuPhos and 2 equiv Cs₂CO₃. The reaction mixture was stirred at 100°C for 16 hours. After cooling to room temperature, the solvent was evaporated and 1 ml TFA was added and stirred at room temperature 4h. The mixture was evaporated. The residue was dissolved in DMSO (appr. 1 ml), treated with scavenger SiliaMetS DMT and filtered. Resulting solution was purified by HPLC (Deionized Water (phase A) and HPLC-grade Acetonitrile (phase B) were used as an eluent to obtained final compound (C). In most cases, TFA was used as an additive.
Instrument: Agilent 1260 Infinity systems equipped with DAD and mass-detector
Column: Waters Sunfire C18 OBD Prep Column, 100 A, 5 µm, 19 mm x 100mm with SunFire C18 Prep Guard Cartridge, 100 A, 10 µm, 19 mm x 10 mm

**Table 2. The following compounds were prepared using methodologies similar to those described in Example 45.**

| | |
|---|---|
| 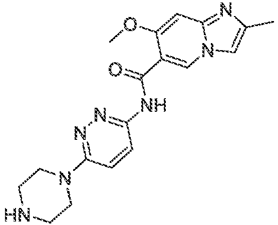 | 7-methoxy-2-methyl-N-[6-(piperazin-1-yl)pyridazin-3-yl]imidazo[1,2-a]pyridine-6-carboxamide (Compound 118) |
| 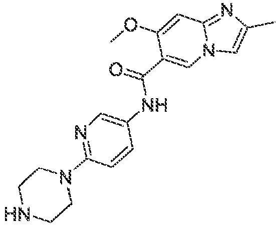 | 7-methoxy-2-methyl-N-[6-(piperazin-1-yl)pyridin-3-yl]imidazo[1,2-a]pyridine-6-carboxamide (Compound 119) |
| 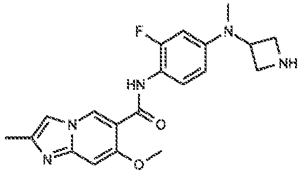 | N-(4-(azetidin-3-yl(methyl)amino)-2-fluorophenyl)-7-methoxy-2-methylimidazo[1,2-a]pyridine-6-carboxamide (Compound 180) |
| 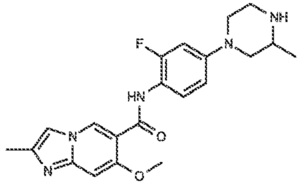 | N-(2-fluoro-4-(3-methylpiperazin-1-yl)phenyl)-7-methoxy-2-methylimidazo[1,2-a]pyridine-6-carboxamide (Compound 179) |
| 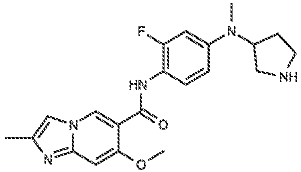 | N-(2-fluoro-4-(methyl(pyrrolidin-3-yl)amino)phenyl)-7-methoxy-2-methylimidazo[1,2-a]pyridine-6-carboxamide (Compound 178) |
| 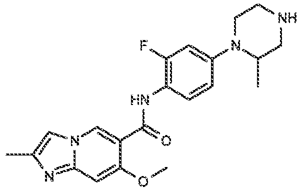 | N-(2-fluoro-4-(2-methylpiperazin-1-yl)phenyl)-7-methoxy-2-methylimidazo[1,2-a]pyridine-6-carboxamide (Compound 177) |
| 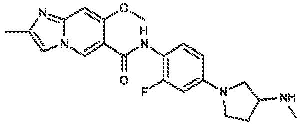 | N-(2-fluoro-4-(3-(methylamino)pyrrolidin-1-yl)phenyl)-7-methoxy-2-methylimidazo[1,2-a]pyridine-6-carboxamide (Compound 176) |
| 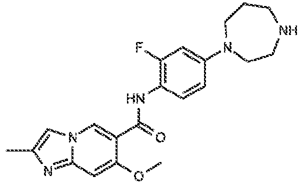 | N-(4-(1,4-diazepan-1-yl)-2-fluorophenyl)-7-methoxy-2-methylimidazo[1,2-a]pyridine-6-carboxamide (Compound 175) |
| 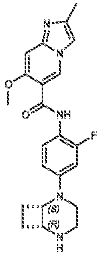 | rac-N-(4-((1R,6S)-2,5-diazabicyclo[4.2.0]octan-2-yl)-2-fluorophenyl)-7-methoxy-2-methylimidazo[1,2-a]pyridine-6-carboxamide (Compound 174) |
| 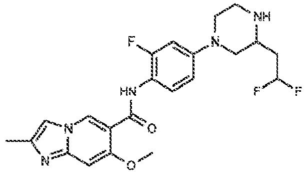 | N-(4-(3-(2,2-difluoroethyl)piperazin-1-yl)-2-fluorophenyl)-7-methoxy-2-methylimidazo[1,2-a]pyridine-6-carboxamide (Compound 173) |
| 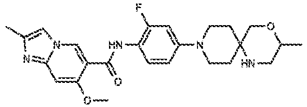 | N-(2-fluoro-4-(3-methyl-4-oxa-1,9-diazaspiro[5.5]undecan-9-yl)phenyl)-7-methoxy-2-methylimidazo[1,2-a]pyridine-6-carboxamide (Compound 172) |
| 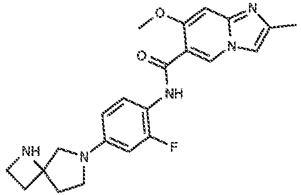 | N-(2-fluoro-4-(1,6-diazaspiro[3.4]octan-6-yl)phenyl)-7-methoxy-2-methylimidazo[1,2-a]pyridine-6-carboxamide (Compound 171) |
| 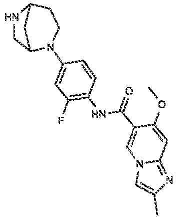 | N-(4-(2,6-diazabicyclo[3.2.1]octan-2-yl)-2-fluorophenyl)-7-methoxy-2-methylimidazo[1,2-a]pyridine-6-carboxamide (Compound 170) |
| 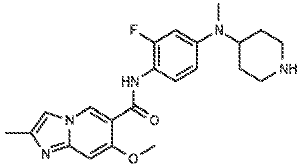 | N-(2-fluoro-4-(methyl(piperidin-4-yl)amino)phenyl)-7-methoxy-2-methylimidazo[1,2-a]pyridine-6-carboxamide (Compound 169) |
| 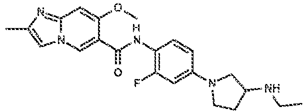 | N-(4-(3-(ethylamino)pyrrolidin-1-yl)-2-fluorophenyl)-7-methoxy-2-methylimidazo[1,2-a]pyridine-6-carboxamide (Compound 168) |
| 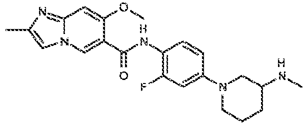 | N-(2-fluoro-4-(3-(methylamino)piperidin-1-yl)phenyl)-7-methoxy-2-methylimidazo[1,2-a]pyridine-6-carboxamide (Compound 167) |
| 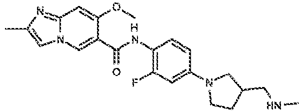 | N-(2-fluoro-4-(3-((methylamino)methyl)pyrrolidin-1-yl)phenyl)-7-methoxy-2-methylimidazo[1,2-a]pyridine-6-carboxamide (Compound 166) |
| 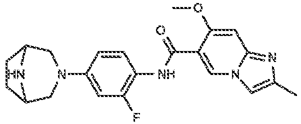 | N-(4-(3,8-diazabicyclo[3.2.1]octan-3-yl)-2-fluorophenyl)-7-methoxy-2-methylimidazo[1,2-a]pyridine-6-carboxamide (Compound 165) |
| 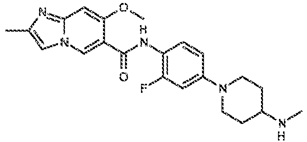 | N-(2-fluoro-4-(4-(methylamino)piperidin-1-yl)phenyl)-7-methoxy-2-methylimidazo[1,2-a]pyridine-6-carboxamide (Compound 164) |
| 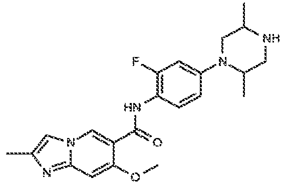 | N-(4-(2,5-dimethylpiperazin-1-yl)-2-fluorophenyl)-7-methoxy-2-methylimidazo[1,2-a]pyridine-6-carboxamide (Compound 163) |
| 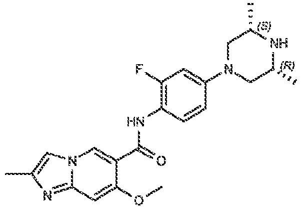 | N-(4-((3S,5R)-3,5-dimethylpiperazin-1-yl)-2-fluorophenyl)-7-methoxy-2-methylimidazo[1,2-a]pyridine-6-carboxamide (Compound 162) |
| 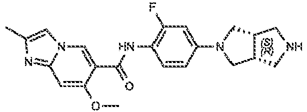 | N-(2-fluoro-4-((3aR,6aS)-hexahydropyrrolo[3,4-c]pyrrol-2(1H)-yl)phenyl)-7-methoxy-2-methylimidazo[1,2-a]pyridine-6-carboxamide (Compound 161) |
| 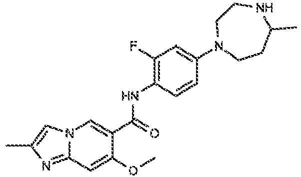 | N-(2-fluoro-4-(5-methyl-1,4-diazepan-1-yl)phenyl)-7-methoxy-2-methylimidazo[1,2-a]pyridine-6-carboxamide (Compound 160) |
| 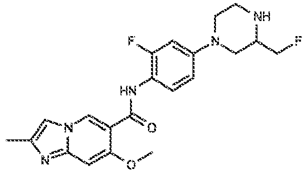 | N-(2-fluoro-4-(3-(fluoromethyl)piperazin-1-yl)phenyl)-7-methoxy-2-methylimidazo[1,2-a]pyridine-6-carboxamide (Compound 159) |
| 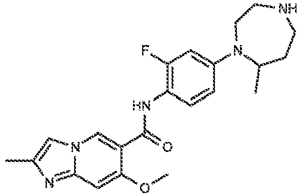 | N-(2-fluoro-4-(7-methyl-1,4-diazepan-1-yl)phenyl)-7-methoxy-2-methylimidazo[1,2-a]pyridine-6-carboxamide (Compound 158) |
| 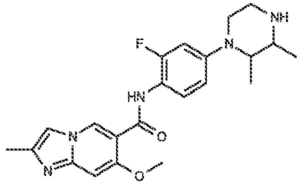 | N-(4-(2,3-dimethylpiperazin-1-yl)-2-fluorophenyl)-7-methoxy-2-methylimidazo[1,2-a]pyridine-6-carboxamide (Compound 157) |
| 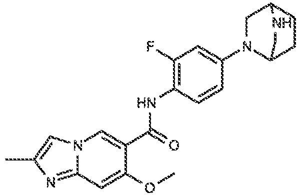 | N-(4-(2,5-diazabicyclo[2.2.2]octan-2-yl)-2-fluorophenyl)-7-methoxy-2-methylimidazo[1,2-a]pyridine-6-carboxamide (Compound 156) |
| 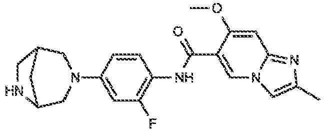 | N-(4-(3,6-diazabicyclo[3.2.1]octan-3-yl)-2-fluorophenyl)-7-methoxy-2-methylimidazo[1,2-a]pyridine-6-carboxamide (Compound 155) |
| 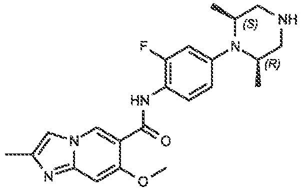 | N-(4-((2S,6R)-2,6-dimethylpiperazin-1-yl)-2-fluorophenyl)-7-methoxy-2-methylimidazo[1,2-a]pyridine-6-carboxamide (Compound 154) |
| 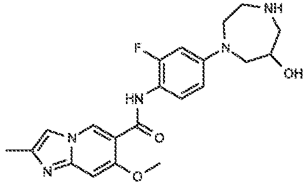 | N-(2-fluoro-4-(6-hydroxy-1,4-diazepan-1-yl)phenyl)-7-methoxy-2-methylimidazo[1,2-a]pyridine-6-carboxamide (Compound 153) |
| 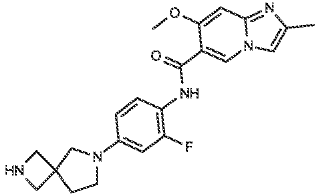 | N-(2-fluoro-4-(2,6-diazaspiro[3.4]octan-6-yl)phenyl)-7-methoxy-2-methylimidazo[1,2-a]pyridine-6-carboxamide (Compound 152) |
| 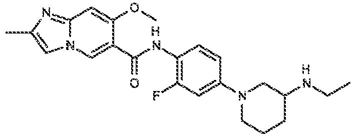 | N-(4-(3-(ethylamino)piperidin-1-yl)-2-fluorophenyl)-7-methoxy-2-methylimidazo[1,2-a]pyridine-6-carboxamide (Compound 151) |
| 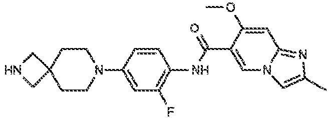 | N-(2-fluoro-4-(2,7-diazaspiro[3.5]nonan-7-yl)phenyl)-7-methoxy-2-methylimidazo[1,2-a]pyridine-6-carboxamide (Compound 150) |
| 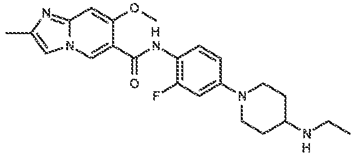 | N-(4-(4-(ethylamino)piperidin-1-yl)-2-fluorophenyl)-7-methoxy-2-methylimidazo[1,2-a]pyridine-6-carboxamide (Compound 149) |
| 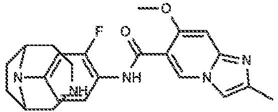 | N-(4-(3,9-diazabicyclo[4.2.1]nonan-9-yl)-2-fluorophenyl)-7-methoxy-2-methylimidazo[1,2-a]pyridine-6-carboxamide (Compound 148) |
| 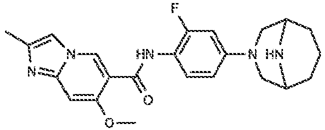 | N-(4-(3,9-diazabicyclo[3.3.1]nonan-3-yl)-2-fluorophenyl)-7-methoxy-2-methylimidazo[1,2-a]pyridine-6-carboxamide (Compound 147) |
| 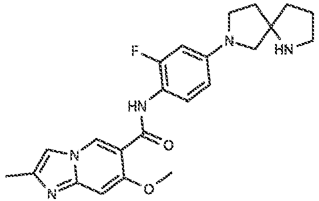 | N-(2-fluoro-4-(1,7-diazaspiro[4.4]nonan-7-yl)phenyl)-1-methoxy-2-methylimidazo[1,2-a]pyridine-6-carboxamide (Compound 146) |
| 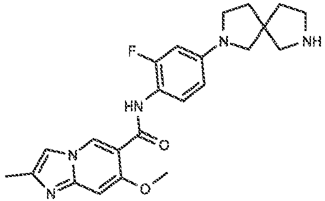 | N-(2-fluoro-4-(2,7-diazaspiro[4.4]nonan-2-yl)phenyl)-7-methoxy-2-methylimidazo[1,2-a]pyridine-6-carboxamide (Compound 145) |
| 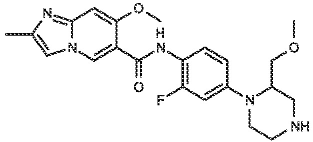 | N-(2-fluoro-4-(2-(methoxymethyl)piperazin-1-yl)phenyl)-7-methoxy-2-methylimidazo[1,2-a]pyridine-6-carboxamide (Compound 144) |
| 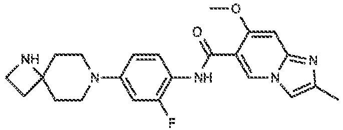 | N-(2-fluoro-4-(1,7-diazaspiro[3.5]nonan-7-yl)phenyl)-7-methoxy-2-methylimidazo[1,2-a]pyridine-6-carboxamide (Compound 143) |
| 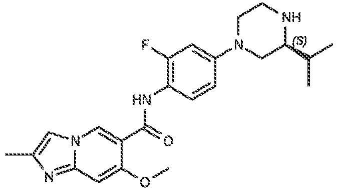 | (S)-N-(2-fluoro-4-(3-isopropylpiperazin-1-yl)phenyl)-7-methoxy-2-methylimidazo[1,2-a]pyridine-6-carboxamide (Compound 142) |
| 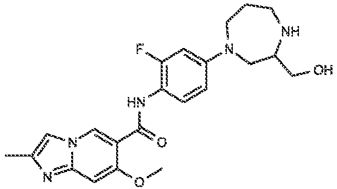 | N-(2-fluoro-4-(3-(hydroxymethyl)-1,4-diazepan-1-yl)phenyl)-7-methoxy-2-methylimidazo[1,2-a]pyridine-6-carboxamide (Compound 141) |
| 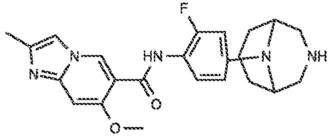 | N-(4-(3,9-diazabicyclo[3.3.1]nonan-9-yl)-2-fluorophenyl)-7-methoxy-2-methylimidazo[1,2-a]pyridine-6-carboxamide (Compound 140) |
| 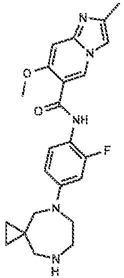 | N-(2-fluoro-4-(5,8-diazaspiro[2.6]nonan-5-yl)phenyl)-7-methoxy-2-methylimidazo[1,2-a]pyridine-6-carboxamide (Compound 139) |
| 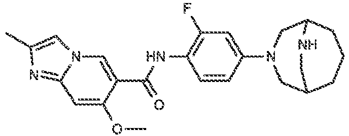 | N-(4-(3,9-diazabicyclo[3.3.2]decan-3-yl)-2-fluorophenyl)-7-methoxy-2-methylimidazo[1,2-a]pyridine-6-carboxamide (Compound 138) |
| 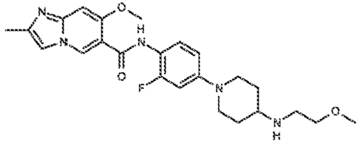 | N-(2-fluoro-4-(4-((2-methoxyethyl)amino)piperidin-1-yl)phenyl)-7-methoxy-2-methylimidazo[1,2-a]pyridine-6-carboxamide (Compound 137) |
| 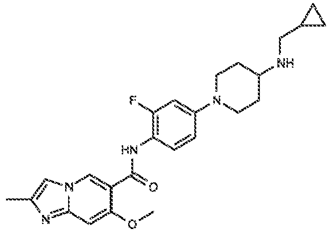 | N-(4-(4-((cyclopropylmethyl)amino)piperidin-1-yl)-2-fluorophenyl)-7-methoxy-2-methylimidazo[1,2-a]pyridine-6-carboxamide (Compound 136) |
| 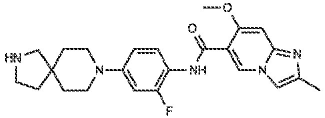 | N-(2-fluoro-4-(2,8-diazaspiro[4.5]decan-8-yl)phenyl)-7-methoxy-2-methylimidazo[1,2-a]pyridine-6-carboxamide (Compound 135) |
| 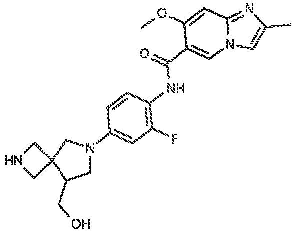 | N-(2-fluoro-4-(8-(hydroxymethyl)-2,6-diazaspiro[3.4]octan-6-yl)phenyl)-7-methoxy-2-methylimidazo[1,2-a]pyridine-6-carboxamide (Compound 134) |
| 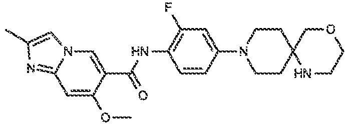 | N-(2-fluoro-4-(4-oxa-1,9-diazaspiro[5.5]undecan-9-yl)phenyl)-7-methoxy-2-methylimidazo[1,2-a]pyridine-6-carboxamide (Compound 133) |
| 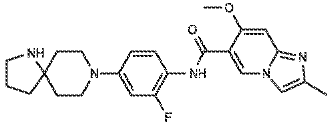 | N-(2-fluoro-4-(1,8-diazaspiro[4.5]decan-8-yl)phenyl)-7-methoxy-2-methylimidazo[1,2-a]pyridine-6-carboxamide (Compound 132) |
| 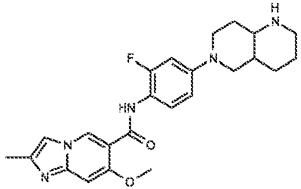 | N-(2-fluoro-4-(octahydro-1,6-naphthyridin-6(2H)-yl)phenyl)-7-methoxy-2-methylimidazo[1,2-a]pyridine-6-carboxamide (Compound 131) |
| 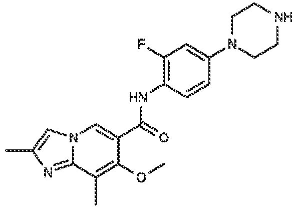 | N-(2-fluoro-4-(piperazin-1-yl)phenyl)-7-methoxy-2,8-dimethylimidazo[1,2-a]pyridine-6-carboxamide (Compound 127) |
| 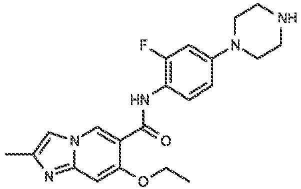 | 7-ethoxy-N-(2-fluoro-4-(piperazin-1-yl)phenyl)-2-methylimidazo[1,2-a]pyridine-6-carboxamide (Compound 126) |

### Example 46. Synthesis of (S)-6-methoxy-2-methyl-N-(6-(3-methylpiperazin-1-yl) pyridazin-3-yl)-2H-indazole-5-carboxamide hydrochloride (Compound 200)

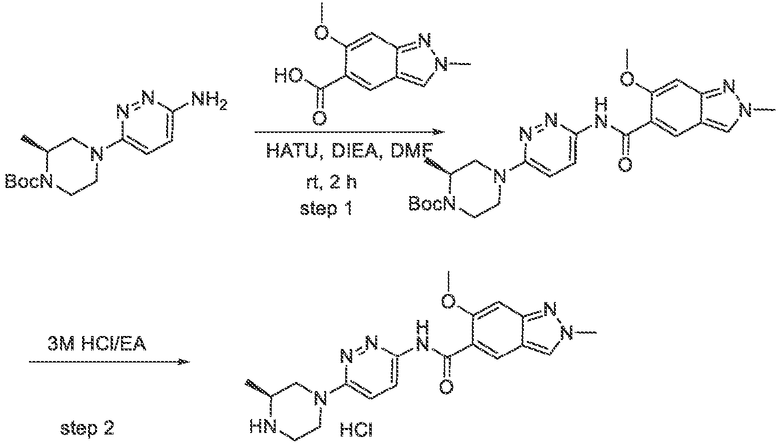

### Step 1: Preparation of tert-butyl (S)-4-(6-(6-methoxy-2-methyl-2H-indazole-5-carboxamido)pyridazin-3-yl)-2-methylpiperazine-1-carboxylate

To a mixture of 6-methoxy-2H-indazole-5-carboxylic acid (72 mg, 0.35 mmol) and tert-butyl (S)-4-(6-aminopyridazin-3-yl)-2-methylpiperazine-1-carboxylate (100 mg, 0.35 mmol) in DMF (5 mL) were added HATU (267 mg, 0.7 mmol), DIEA (226 mg 1.75 mmol). The mixture was stirred at rt for 2 h. After concentration, the crude was purified by prep-HPLC (0.05%FA in water / CH₃CN) to give title product (50 mg, Y: 30.5%) as a yellow solid. ESI-MS (M+H)+: 482.1.

### Step 2: Preparation of (S)-6-methoxy-2-methyl-N-(6-(3-methylpiperazin-1-yl) pyridazin-3-yl)-2H-indazole-5-carboxamide hydrochloride

A mixture of tert-butyl (S)-4-(6-(6-methoxy-2-methyl-2H-indazole-5-carboxamido)pyridazin-3-yl)-2-methylpiperazine-1-carboxylate (50 mg, 0.145 mmol) in 3M HCl/EA (4 mL) was stirred at rt for 2 h. The precipitate was filtered and the solid was dried in vacuo to give title product (19 mg, Y: 44.2%) as a yellow solid. ESI-MS (M+H)+:382.0. ¹H NMR (400 MHz, MeOD-*d₄*) δ 8.52 (s, 1H), 8.47 (s, 1H), 8.25 - 8.18 (m, 2H), 7.19 (s, 1H), 4.49 (d, J = 14.0 Hz, 2H), 4.23 (s, 3H), 4.10 (s, 3H), 3.61 - 3.46 (m, 3H), 3.33 - 3.31 (m, 2H), 1.44 (d, J = 7.2 Hz, 3H).

### Example 47. Synthesis of (S)-2,8-dimethyl-N-(6-(3-methylpiperazin-1-yl)pyridazin-3-yl)imidazo[1,2-a]pyrazine-6-carboxamide hydrochloride (Compound 201)

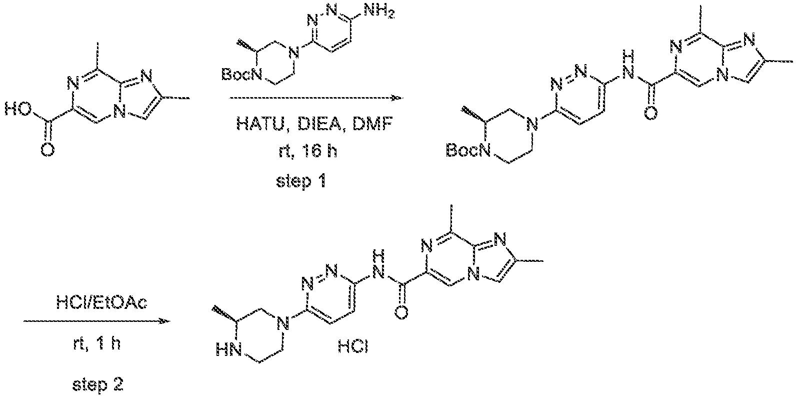

### Step 1: tert-butyl (S)-4-(6-(2,8-dimethylimidazo[1,2-a]pyrazine-6-carboxamido)pyridazin-3-yl)-2-methylpiperazine-1-carboxylate

To a stirred solution of 2,8-dimethylimidazo[1,2-a]pyrazine-6- carboxylic acid (50 mg, 0.26 mmol), tert-butyl (S)-4-(6-aminopyridazin-3-yl)-2- methylpiperazine-1-carboxylate (84 mg, 0.28 mmol) and DIEA (100 mg, 0.78 mmol) in DMF (5 mL) was added HATU (128 mg, 0.33 mmol) at rt. The mixture was stirred at rt for 16 hrs. The reaction mixture was treated with EA/Water (50 mL, 1:1). The organic phase was separated and the aqueous phase was extracted with EA (50 mLx1). The combined organic layer was dried over sodium sulfate. After concentration, the crude was purified by pre-HPLC (0.05% FA in water /CH₃CN) to provide title product (15 mg, Y: 12%) as an off-white solid. ESI-MS (M+H)+: 467.2. ¹H NMR (400 MHz, CDCl₃) δ 8.18 (s, 1H), 8.40 (d, J = 9.8 Hz, 1H), 7.51 (s, 1H), 7.07 (d, J = 9.8 Hz, 1H), 4.35 - 4.07 (m, 2H), 3.95 - 3.92 (m, 2H), 3.30 -3.25 (m, 2H), 3.09 - 3.07 (m, 1H), 2.83 (s, 3H), 2.47 (s, 3H), 1.42 (s, 9H), 1.16 (d, J = 6.8 Hz, 3H).

### Step 2: Preparation of (S)-2,8-dimethyl-N-(6-(3-methylpiperazin-1-yl)pyridazin-3-yl)imidazo[l, 2-a]pyrazine-6-carboxamide hydrochloride

To a solution of tert-butyl (S)-4-(6-(2,8-dimethy limidazo[1,2-a]pyrazine-6-carboxamido)pyridazin-3-yl)-2-methylpiperazine-1-carboxylate (15 mg, 0.03 nmol) in EA (1 mL) was added HCl-EA (3 M, 1.0 mL) at 0 °C and the mixture was allowed to warm to rt and stirred for 1 h. The solid was collected by filtration and washed with EA (2 mL x 3). The solid was dried under vacuum at 55 °C to provide title product as light grey solid (5 mg, yield: 45%). ESI-MS (M+H)+: 367.0. ¹H NMR (400 MHz, MeOD-*d₄*) δ 9.48 (d, J = 4.4 Hz, 1H), 8.56 (d, J = 9.9 Hz, 1H), 8.29 (d, J = 3.9 Hz, 1H), 8.14 - 8.07 (m, 1H), 4.50 (d, J = 13.8 Hz, 2H), 3.57 - 3.51 (m, 3H), 3.37 - 3.35 (m, 2H), 3.03 (s, 3H), 2.68 (s, 3H), 1.45 (d, J = 6.5 Hz, 3H).

### Example 48. Synthesis of 6-methoxy-2-methyl-N-(6-(piperazin-1-yl)pyridazin-3-yl)-2H-indazole-5-carboxamide hydrochloride (Compound 202)

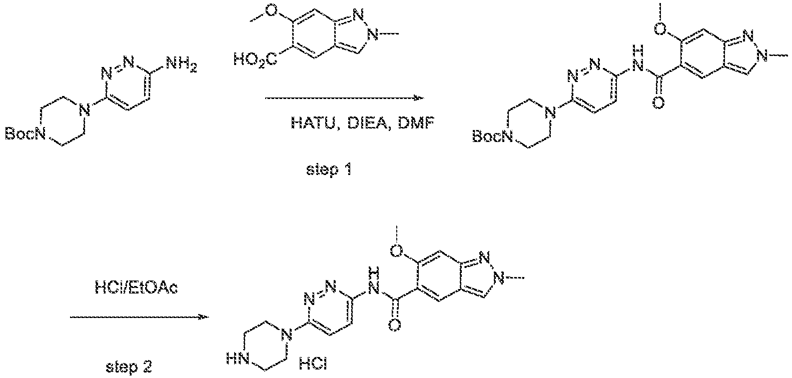

### Step 1: tert-butyl 4-(6-(6-methoxy-2-methyl-2H-indazole-5-carboxamido) pyridazin-3-yl)piperazine-1-carboxylate

To a stirred solution of 6-methoxy-2-methyl-2H- indazole-5-carboxylic acid (120 mg, 0.58 mmol), tert-butyl 4-(6-aminopyridazin-3-yl) piperazine-1-carboxylate (180 mg, 0.64 mmol) and DIEA (224 mg, 1.74 mmol) in DMF (5 mL) was added HATU (286 mg, 0.75 mmol) at rt. The mixture was stirred at rt for 2 hours. The reaction mixture was treated with EA/Water (50 mL, 1:1). The organic phase was separated and the aqueous phase was extracted with EA (50 mLx1). The combined organic layer was dried over sodium sulfate. After concentration, the crude was purified by prep-HPLC (0.05% FA in water /CH₃CN) to provide title product (60 mg, Y: 22%) as an off-white solid. ESI-MS (M+H) +: 468.2.

### Step 2: Preparation of 6-methoxy-2-methyl-N-(6-(piperazin-1-yl)pyridazin-3-yl)-2H-indazole-5-carboxamide hydrochloride

To a solution of tert-butyl 4-(6-(6-methoxy-2-methyl-2H-indazole-5-carboxamido)pyridazin-3-yl)piperazine-1-carboxylate (50 mg, 0.107 nmol) in EA (1 mL) was added HCl/EA (3 M, 1.0 mL) at 0 °C and warmed to rt for 1 h . The solid was collected by filtration and washed with EA (2 mL x 3). The solid was concentrated in vacuo to provide title product as a yellow solid (15 mg, yield: 37%). ESI-MS (M+H)+: 368.1. ¹H NMR (400 MHz, DMSO-*d₆*) δ 10.98 (s, 1H), 9.53 (s, 2H), 8.47 (s, 1H), 8.38 (d, J = 9.8 Hz, 1H), 8.30 (s, 1H), 7.76 (d, J = 9.7 Hz, 1H), 7.15 (s, 1H), 4.15 (s, 3H), 3.98 (br s, 3H), 3.88 (br s, 4H), 3.24 (s, 4H).

### Example 49. Synthesis of (S)-7-cyclobutoxy-2-methyl-N-(6-(3-methylpiperazin-1-yl) pyridazin-3-yl)imidazo[1,2-a]pyridine-6-carboxamide hydrochloride (Compound 203)

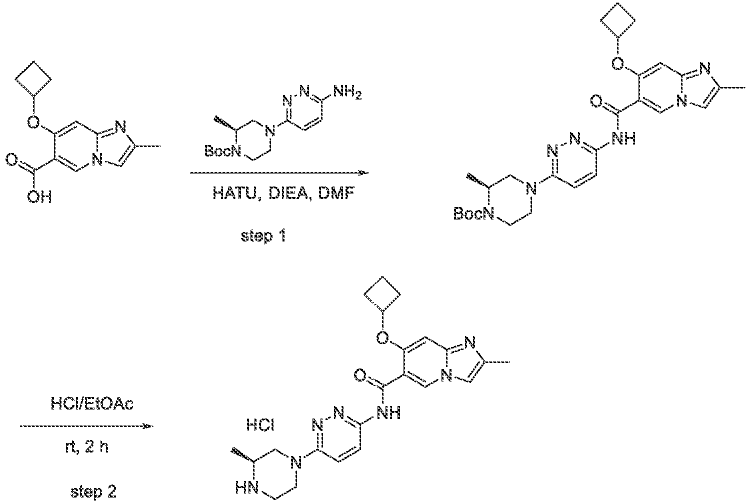

### Step 1: Preparation of tert-butyl (S)-4-(6-(7-cyclobutoxy-2-methylimidazo[1,2-a]pyridine-6-carboxamido)pyridazin-3-yl)-2-methylpiperazine-1-carboxylate

To a mixture of 7-cyclobutoxy-2-methylimidazo[1,2-a]pyridine-6-carboxylic acid (95 mg, 0.38 mmol) and tert-butyl (S)-4-(6-aminopyridazin-3-yl)-2-methylpiperazine-1-carboxylate (85 mg, 0.29 mmol) in DMF (3 mL) were added HATU (165 mg, 0.44 mmol) and DIPEA (112 mg, 0.87 mmol). The mixture was stirred at RT overnight. Water (10 mL) was added to the mixture and filtered, the crude product was purified by prep-HPLC (0.05% NH₃H₂O in water /CH₃CN) to give title product (15 mg, Y: 9.9 %) as a yellow solid. ESI-MS (M+H)+: 522.3. ¹H NMR (400 MHz, CDCl₃) δ 10.68 (s, 1H), 8.98 (s, 1H), 8.42 (d, J = 9.8 Hz, 1H), 7.30 (s, 1H), 7.00 (d, J = 9.9 Hz, 1H), 6.83 (s, 1H), 4.97 - 4.87 (m, 1H), 4.38 (s, 1H), 4.17 (d, J = 12.2 Hz, 1H), 4.05 - 3.94 (m, 2H), 3.36 - 3.25 (m, 2H), 3.11 - 3.03 (m, 1H), 2.69 - 2.62 (m, 2H), 2.50 - 2.44 (m, 2H), 2.44 (s, 3H), 2.00 - 1.96 (m, 1H), 1.86 - 1.84 (m, 1H), 1.49 (s, 9H), 1.23 (d, J = 6.7 Hz, 3H).

### Step 2: Preparation of (S)-7-cyclobutoxy-2-methyl-N-(6-(3-methylpiperazin-1-yl) pyridazin-3-yl)imidazo[1,2-a]pyridine-6-carboxamide hydrochloride

A mixture of tert-butyl (S)-4-(6-(7-cyclobutoxy-2-methylimidazo[1,2-a]pyridine-6-carboxamido)pyridazin-3-yl)-2-methylpiperazine-1-carboxylate (15 mg, 0.029 mmol) in 3M EtOAc/HCl (2 mL) was stirred at rt for 2 h. After concentration, the residue was dissolved in water (5 mL) and lyophilized to give title product (11.2 mg, Y: 85.1 %) as a yellow solid. ESI-MS (M+H)+: 422.2. ¹H NMR (400 MHz, CD₃OD) δ 9.19 (s, 1H), 8.51 (d, J = 10.0 Hz, 1H), 8.01 (d, J = 10.1 Hz, 1H), 7.85 (s, 1H), 7.15 (s, 1H), 5.15 - 5.09 (m, 1H), 4.51 - 4.39 (m, 2H), 3.64 - 3.52 (m, 3H), 3.41 - 3.33 (m, 2H), 2.70 - 2.59 (m, 2H), 2.51 (s, 3H), 2.43 - 2.31 (m, 2H), 2.02 -1.95 (mz, 1H), 1.89 - 1.82 (m, 1H), 1.45 (d, J = 6.6 Hz, 3H).

### Example 50. Synthesis of (S)-7-isopropoxy-2-methyl-N-(6-(3-methylpiperazin- 1-yl)pyridazin-3-yl)imidazo[1,2-a]pyridine-6-carboxamide (Compound 204)

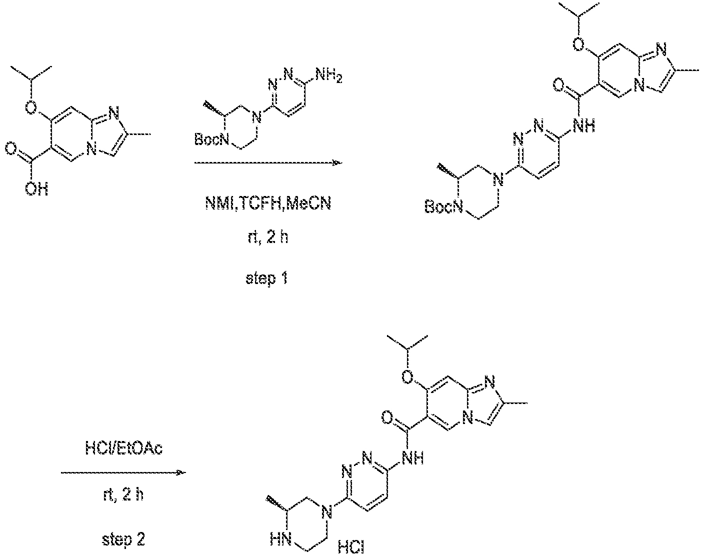

### Step 1: tert-butyl (S)-4-(6-(7-isopropoxy-2-methylimidazo[1,2-a] pyridine-6-carboxamido)pyridazin-3-yl)-2-methylpiperazine-1-carboxylate

To a stirred solution of 7-isopropoxy-2-methylimidazo[1,2-a]pyridine-6-carboxylic acid (100 mg, 0.427 mmol), tert-butyl (S)-4-(6-aminopyridazin-3-yl)-2-methylpiperazine -1-carboxylate (125 mg, 0.427 mmol) and NMI (105 mg, 1.281 mmol) in MeCN (5 mL) was added TCFH (180 mg, 0.641 mmol) at rt. The mixture was stirred at rt for 2 h. The reaction mixture was treated with EA/water (50 mL, 1:1). The organic phase was separated and the aqueous phase was extracted with EA (50 mL). The combined organic layer was dried over sodium sulfate. After concentration, the crude was purified by prep-HPLC (0.05% NH₃.H₂O in water / CH₃CN) to provide title product (60 mg, Y: 27%) as off-white solid. ESI-MS (M+H)+: 510.3. ¹H NMR (400 MHz, CDCl₃) δ 10.71 (s, 1H), 9.01 (s, 1H), 8.41 (d, J = 9.8 Hz, 1H), 7.30 (s, 1H), 7.13 (s, 1H), 7.02 - 6.98 (m, 1H), 4.94 - 4.86 (m, 1H), 4.17 (d, J = 12.8 Hz, 1H), 4.04 - 3.95 (m, 2H), 3.36 - 3.27 (m, 2H), 3.11 - 3.03 (m, 1H), 2.48 (s, 3H), 2.01 (s, 1H), 1.62 (s, 3H), 1.61 (s, 3H), 1.49 (s, 9H), 1.22 (d, J = 6.7 Hz, 3H).

### Step 2: Preparation of (S)-7-isopropoxy-2-methyl-N-(6-(3-methylpiperazin- 1-yl)pyridazin-3-yl)imidazo[1,2-a]pyridine-6-carboxamide

To a solution of tert-butyl (S)-4-(6-(7-isopropoxy-2-methylimidazo[1,2-a]pyridine-6-carboxamido) pyridazin-3-yl)-2-methylpiperazine-1-carboxylate (60 mg, 0.177 mmol) in EA (1 mL) was added 3M HCl/EA (1 mL), the mixture was stirred at r.t for 2 h. The solid was collected by filtration and washed EA three times (3 mL x 3) to afford the title compound as hydrochloride salt (25 mg, yield: 52%). ESI-MS (M+H)+: 410.1. ¹H NMR (400 MHz, DMSO*-d₆*) δ 11.23 (s, 1H), 9.97 - 9.75 (m, 2H), 9.26 (s, 1H), 8.29 (d, J = 9.3 Hz, 1H), 7.98 (s, 1H), 7.75 (d, J = 10.0 Hz, 1H), 7.37 (s, 1H), 5.06 - 4.97 (m, 1H), 4.45 - 4.33 (m, 2H), 3.51 - 3.42 (m, 1H), 3.36 (d, J = 12.3 Hz, 2H), 3.27 - 3.17 (m, 1H), 3.14 - 3.03 (m, 1H), 2.46 (s, 3H), 1.41 (d, J = 6.0 Hz, 6H), 1.35 (d, J = 6.4 Hz, 3H).

### Example 51. Synthesis of 7-cyclobutoxy-2-methyl-N-(6-(piperazin-1-yl)pyridazin-3-yl)imidazo[1,2-a]pyridine-6-carboxamide hydrochloride (Compound 205)

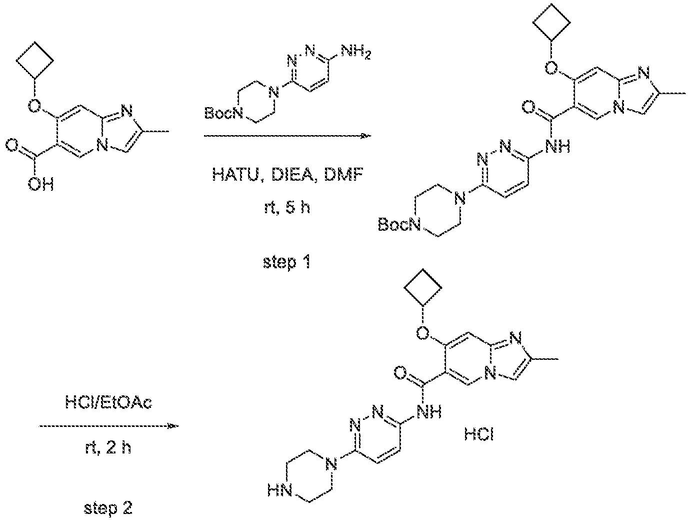

### Step 1: preparation of tert-butyl 4-(6-(7-cyclobutoxy-2-methylimidazo[1,2-a]pyridine-6-carboxamido)pyridazin-3-yl)piperazine-1-carboxylate

To a mixture of tert-butyl 4-(6-aminopyridazin-3-yl)piperazine-1-carboxylate (100 mg, 0.358 mmol) in DMF (5 mL) were added 7-cyclobutoxy-2-methylimidazo[1,2-a]pyridine-6-carboxylic acid (133 mg, 0.54 mmol), DIEA (140 mg, 1.07 mmol) and HATU (205 mg, 0.54 mmol). The mixture was stirred at rt for 5 h and quenched with water (10 mL). The precipitate was filtered and purified by C18 flash (0.1% FA in water / CH3CN) to give title product (11 mg, 6 %) as a yellow solid. ESI-MS (M+H)+: 508.2.

### Step 2: preparation of 7-cyclobutoxy-2-methyl-N-(6-(piperazin-1-yl)pyridazin-3-yl)imidazo[1,2-a]pyridine-6-carboxamide hydrochloride

To a solution of tert-butyl 4-(6-(7-cyclobutoxy-2-methylimidazo[1,2-a]pyridine-6-carboxamido)pyridazin-3-yl)piperazine-1-carboxylate (11 mg, 0.02 mmol) was added 3M HCl/EA (5 mL) at rt. The mixture was stirred for 2 h at rt. The reaction mixture was concentrated in vacuo and the solid was washed with EA and dried to give title product (2 mg, yield: 22.7 %) as a yellow solid. ESI-MS (M+H) +: 408.0. ¹H NMR (400 MHz, MeOD-*d₄*) δ 9.20 (s, 1H), 8.50 (d, J = 9.9 Hz, 1H), 7.98 - 7.91 (m, 1H), 7.85 (s, 1H), 7.15 (s, 1H), 5.18 - 5.07 (m, 1H), 4.00 (br.s, 4H), 3.48 - 3.41 (m, 4H), 2.71 - 2.60 (m, 2H), 2.50 (s, 3H), 2.44 - 2.31 (m, 2H), 2.01 - 1.96 (m, 1H), 1.92 - 1.81 (m, 1H).

### Example 52. 2-methyl-N-(6-((S)-3-methylpiperazin-1-yl)pyridazin-3-yl)-7-((tetrahydrofuran-3-yl)oxy)imidazo[1,2-a]pyridine-6-carboxamide hydrochloride (Compound 206)

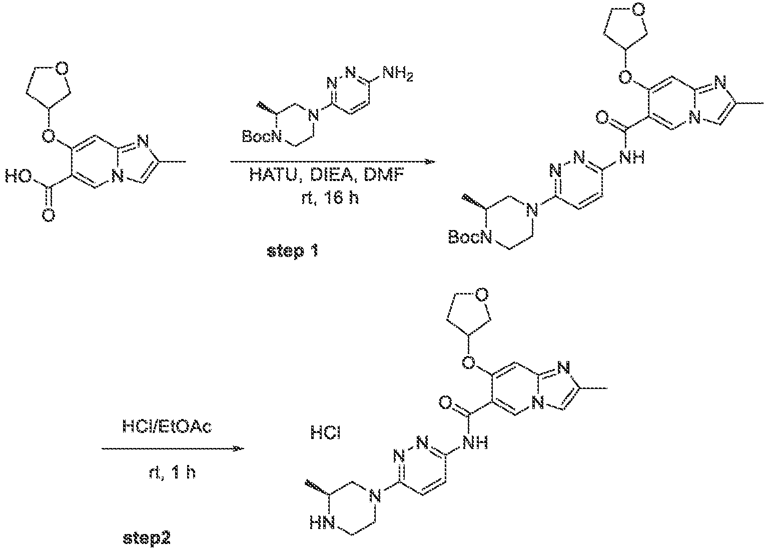

### Preparation of 2-methyl- 7-((tetrahydrofuran-3-yl)oxy)imidazo[1,2-a]pyridine-6-carboxylic acid

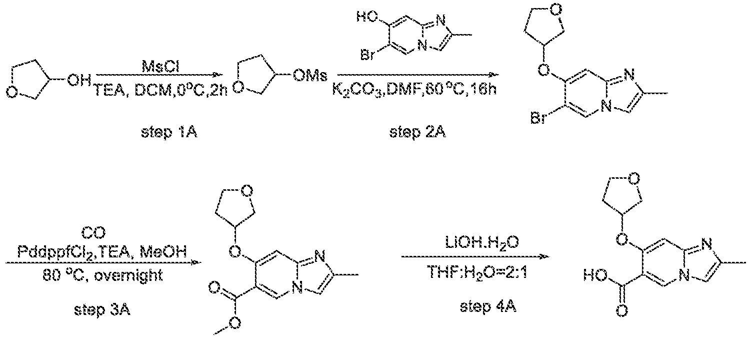

### Step 1A: preparation of tetrahydrofuran-3-yl methanesulfonate

To a solution of tetrahydrofuran-3-ol (4.0 g, 45.4 mmol) in DCM (50 mL) was added TEA (13.6 g, 136 mmol) and MsCl (6.2 g, 54.5 mmol) at 0 °C, the mixture was stirred for 2 h at RT. TLC (MeOH:DCM=1:4, R_{f}=0.3) showed the reaction was completed. The mixture was washed with water and brine, dried over Na₂SO₄, filtered and the filtrate was concentrated to afford crude and purified by silica gel column chromatography eluted with PE:EtOAc=1:2 to afford title compound (6.5 g, crude) as a colorless oil.

### Step 2A: preparation of 6-bromo-2-methyl-7-((tetrahydrofuran-3-yl)oxy)imidazo[1,2-a]pyridine

To a solution of 6-bromo-2-methylimidazo[1,2-a]pyridin-7-ol (650 mg, crude) in DMF (10 mL) was added K₂CO₃ (3.15 g, 23.2 mmol) and tetrahydrofuran-3-yl methanesulfonate (2.86 g, 17.2 mmol), the mixture was stirred for 16 h at 60 °C. The mixture was diluted with water (50 mL) and extracted with EtOAc (50 mL x3), the combined organic layer was washed with brine, dried over Na₂SO₄, filtered and the filtrate was concentrated. The crude was purified by C18 column chromatography eluted with MeCN:H₂O=20% to 60% to afford title compound (500 mg, 37.4% for two steps) as a grey solid. ESI-MS (M+H) ⁺:296.8.
¹H NMR (400 MHz, CDCl₃) δ 8.16 (s, 1H), 7.15 (s, 1H), 6.78 (s, 1H), 5.01 - 4.91 (m, 1H), 4.11 - 4.05 (m, 2H), 4.05 - 3.99 (m, 1H), 3.97 - 3.90 (m, 1H), 2.39 (s, 3H), 2.30 - 2.21 (m, 2H).

### Step 3A: preparation of methyl 2-methyl-7-((tetrahydrofuran-3-yl)oxy)imidazo[1,2-a]pyridine-6-carboxylate

To a solution of 6-bromo-2-methyl-7-((tetrahydrofuran-3-yl)oxy)imidazo[1,2-a]pyridine (480 mg, 1.62 mmol) in MeOH (50 mL) were added Pd(dppf)Cl₂ (237 mg, 0.32 mmol) and TEA(1.64 g, 16.2 mmol), the mixture was charged with CO for three times and stirred overnight at 80 °C under CO balloon. The mixture was filtered and the filtrate was concentrated to afford the title compound (crude 400 mg, 89.4% yield) as a grey solid. ESI-MS (M+H)⁺: 277.2.

*Step 4A: preparation of 2-methyl-7-((tetrahydrofuran-3-yl)oxy)imidazo[1,2-a]pyridine-6-carboxylic acid*

To a solution of methyl 2-methyl-7-((tetrahydrofuran-3-yl)oxy)imidazo[1,2-a]pyridine-6-carboxylate (crude 400 mg, 1.45 mmol) in THF (8 mL) and H₂O (4 mL) was added LiOH (174 mg, 7.2. mmol), the mixture was stirred for 1h at RT. The mixture was diluted with water (10 mL) and extracted with EtOAc (20 mL x3). Aqueous phase was adjusted to pH=2 with 1M HCl and purified by C18 column chromatography eluted with MeCN:H₂O (0.1 % FA) =0% to 15% to afford title compound (270 mg, 70.8%) as a white solid, ESI-MS (M+H) ⁺:263.0

### Step 1: Preparation of tert-butyl (2S)-2-methyl-4-(6-(2-methyl-7-((tetrahydrofuran-3-yl)oxy)imidazo[1,2-a]pyridine-6-carboxamido)pyridazin-3-yl)piperazine-1-carboxylate

To a solution of 2-methyl-7-((tetrahydrofuran-3-yl)oxy)imidazo[1,2-a]pyridine-6-carboxylic acid (100 mg, 0.38 mmol) and tert-butyl (S)-4-(6-aminopyridazin-3-yl)-2-methylpiperazine-1-carboxylate (111mg, 0.38 mmol) in DMF (10 mL) were added DIPEA (147 mg, 1.14 mmol) and HATU (216 mg, 0.57 mmol), the mixture was stirred for 16 h at RT. The mixture was purified by pre-HPLC (0.05% FA in water / CH₃CN) to afford title compound (70 mg, 34.1%) as a grey solid. ESI-MS (M+H) +:538.3. ¹H NMR (400 MHz, DMSO-*d₆*) δ 10.61 (s, 1H), 9.06 (s, 1H), 8.21 (d, J = 9.8 Hz, 1H), 7.67 (s, 1H), 7.42 (d, J = 9.9 Hz, 1H), 7.02 (s, 1H), 5.34 (br s, 1H), 4.27 - 4.16 (m, 2H), 4.09 - 4.06 (m, 1H), 3.98 - 3.95 (m, 3H), 3.87 - 3.77 (m, 2H), 3.23 - 3.15 (m, 2H), 2.99 - 2.88 (m, 1H), 2.42 - 2.34 (m, 1H), 2.30 (s, 3H), 2.22 - 2.13 (m, 1H), 1.44 (s, 9H), 1.13 (d, J = 6.7 Hz, 3H).

### Step 2: Preparation of 2-methyl-N-(6-((S)-3-methylpiperazin-1-yl)pyridazin-3-yl)-7-((tetrahydrofuran-3-yl)oxy)imidazo[l, 2-a]pyridine-6-carboxamide hydrochloride

A solution of tert-butyl (2S)-2-methyl-4-(6-(2-methyl-7-((tetrahydrofuran-3-yl)oxy)imidazo[1,2-a]pyridine-6-carboxamido)pyridazin-3-yl)piperazine-1-carboxylate (70 mg, 0.13 mmol) in 3M HCl/EtOAc (3mL) was stirred for 1h at RT. The precipitate was filtered, washed with EtOAc (1 mL) and dried to afford title compound (50 mg, 82.2%) as a yellow solid. ESI-MS (M+H) +:438.1. ¹H NMR (400 MHz, DMSO-*d₆*) δ 11.18 (s, 1H), 9.77 - 9.68 (m, 2H), 9.25 (s, 1H), 8.26 (d, J = 8.8 Hz, 1H), 7.98 (s, 1H), 7.68 (d, J = 9.8 Hz, 1H), 7.34 (s, 1H), 4.38 (t, J = 12.6 Hz, 2H), 3.96 (br s, 2H), 3.88 - 3.75 (m, 2H), 3.45 - 3.30 (m, 3H), 3.22 - 2.87 (m, 3H), 2.46 (s, 3H), 2.39 - 2.30 (m, 1H), 2.19 - 2.07 (m, 1H), 1.34 (d, J = 6.4 Hz, 3H).

### Example 53. Syntheis of 6-cyclobutoxy-2-methyl-N-(6-(piperazin-1-yl)pyridazin-3-yl)-2H-indazole-5-carboxamide HCl salt (Compound 207)

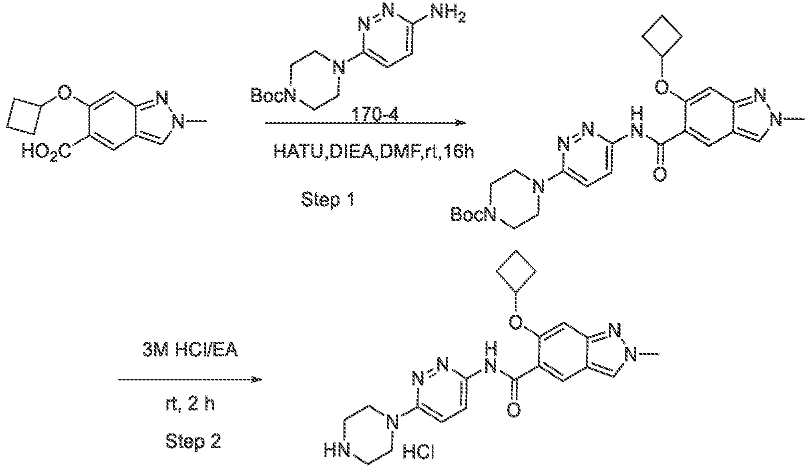

### Step 1 : Preparation of tert-butyl 4-(6-(6-cyclobutoxy-2-methyl-2H-indazole-5-carboxamido)pyridazin-3-yl)piperazine-1-carboxylate

To a stirred solution of 6-cyclobutoxy-2-methyl-2H-indazole-5-carboxylic acid (40 mg, 0.14 mmol), tert-butyl 4-(6-aminopyridazin-3-yl)piperazine-1-carboxylate (40 mg, 0.14 mmol) and DIEA (55 mg, 0.429 mmol) in DMF (1 mL) was added HATU (70 mg, 0.18 mmol) at rt. The mixture was stirred for 16 h. The reaction mixture was diluted with water (10 mL), extracted with EA (25 mL). The combined organic layers was washed with brine and dried over sodium sulfate. After concentration under reduced pressure, the crude was purified by prep-HPLC (0.1% NH₃.H₂O in water / CH3CN) to give title product (34 mg, Y: 47.2%) as a yellow solid. ESI-MS (M+H)+: 508.3

### Step 2: Preparation of 6-cyclobutoxy-2-methyl-N-(6-(piperazin-1-yl)pyridazin-3-yl)-2H-indazole-5-carboxamide HCl salt

To a solution of tert-butyl 4-(6-(6-cyclobutoxy-2-methyl-2H-indazole-5-carboxamido)pyridazin-3-yl)piperazine-1-carboxylate (20 mg, 0.04 mmol) in EA (2 mL) was added HCl/EA (3 M, 2 mL) at RT and the mixture was allowed to warm to rt and stirred for 2 h. After concentration, the solid was washed with EA three times (3 mL x3). and dried under vacuum to provide title product (9 mg, yield: 60 %) as a light yellow solid. ¹H NMR (400 MHz, MeOD-*d₄*) δ 8.52 (d, J = 8.8 Hz, 2H), 8.25 - 8.15 (m, 2H), 7.00 (s, 1H), 5.02 - 4.98 (m, 1H), 4.25 (s, 3H), 4.05 - 4.00 (m, 4H), 3.48 - 3.43 (m, 4H), 2.68 - 2.59 (m, 2H), 2.36 - 2.25 (m, 2H), 1.99 - 1.81 (m, 2H). ESI-MS (M+H)+: 408.1.

### Example 54. Synthesis of (S)-6-ethoxy-2-methyl-N-(6-(3-methylpiperazin-1-yl)pyridazin-3-yl)-2H-indazole-5-carboxamide HCl salt (Compound 208)

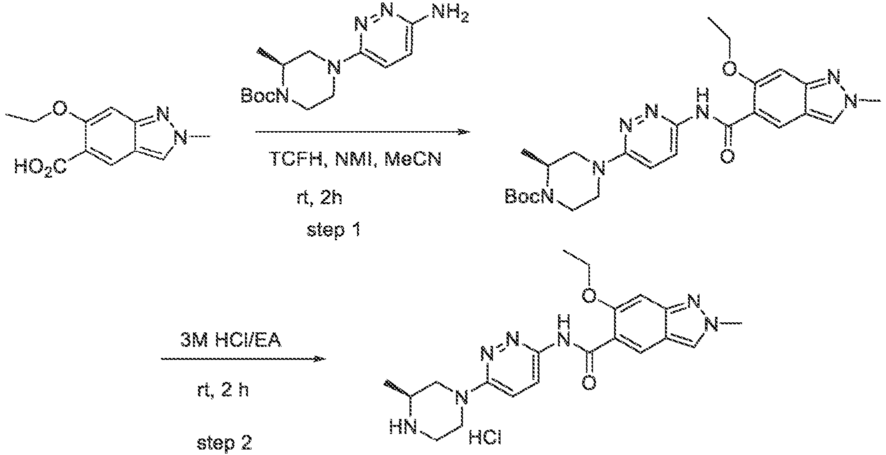

### Step 1: Preparation of tert-butyl (S)-4-(6-(6-ethoxy-2-methyl-2H-indazole-5-carboxamido)pyridazin-3-yl)-2-methylpiperazine-1-carboxylate

To a solution of 6-ethoxy-2-methyl-2H-indazole-5-carboxylic acid (1 g, 4.545 mmol) and tert-butyl (S)-4-(6-aminopyridazin-3-yl)-2-methylpiperazine-1-carboxylate (1.3 g,4.545 mmol) in MeCN (10 mL) was added NMI (1.1g, 13.635mmol) and TCFH (1.9 g, 6.815 mmol), the mixture was stirred for 2 h at RT. The precipitate was filtered to afford title compound (1 g, 45.4% yield) as a grey solid. ¹H NMR (400 MHz, CDCl₃) δ 10.94 (s, 1H), 8.72 (s, 1H), 8.53 - 8.50 (m, 1H), 8.00 (s, 1H), 7.09 (s, 1H), 7.03 - 6.98 (m, 1H), 4.49 - 4.22 (m, 2H), 4.20 (s, 3H), 4.03 - 3.94 (m, 2H), 3.48 (br.s, 2H), 3.32 - 3.25 (m, 2H), 3.09 - 3.01 (m, 1H), 1.70 (t, J = 7.0 Hz, 3H), 1.49 (s, 9H), 1.23 (d, J = 6.7 Hz, 3H). ESI-MS (M+H) +:496.0.

### Step 2: Preparation of (S)-6-ethoxy-2-methyl-N-(6-(3-methylpiperazin-1-yl)pyridazin-3-yl)-2H-indazole-5-carboxamide HCl salt

A solution of tert-butyl (S)-4-(6-(6-ethoxy-2-methyl-2H-indazole-5-carboxamido)pyridazin-3-yl)-2-methylpiperazine-1-carboxylate (1 g, 2.02 mmol) in 3M HCl/EtOAc (10 mL) was stirred for 2h at RT. The precipitate was filtered, washed with EtOAc (1 mL) to afford title compound (740 mg, 85.4%) as a yellow solid. ¹H NMR (400 MHz, DMSO-d₆) δ 11.12 (s, 1H), 9.79 (d, J = 31.1 Hz, 2H), 8.53 - 8.41 (m, 2H), 8.36 (s, 1H), 7.88 (d, J = 9.9 Hz, 1H), 7.14 (s, 1H), 4.39 (t, J = 14.0 Hz, 2H), 4.30 - 4.23 (m, 2H), 4.15 (s, 3H), 3.53 - 3.10 (m, 5H), 1.48 (t, J = 6.9 Hz, 3H), 1.34 (d, J = 6.4 Hz, 3H).. ESI-MS (M+H) +: 396.1.

### Example 55. Synthesis of (S)-6-cyclobutoxy-2-methyl-N-(6-(3-methylpiperazin-1-yl)pyridazin-3-yl)-2H-indazole-5-carboxamide HCl salt (Compound 209)

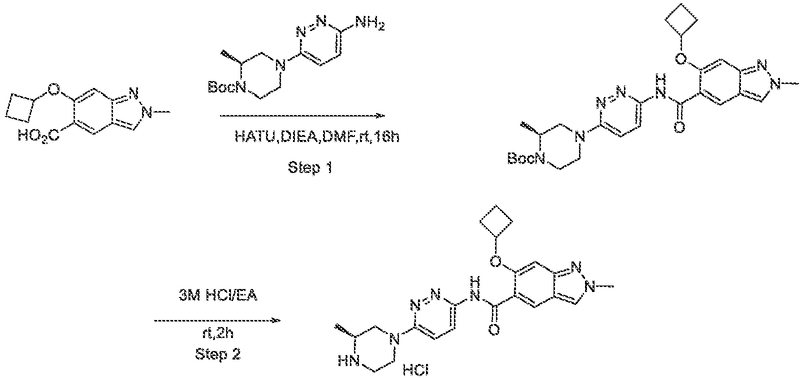

### Step 1: Preparation of tert-butyl (S)-4-(6-(6-cyclobutoxy-2-methyl-2H-indazole-5-carboxamido)pyridazin-3-yl)-2-methylpiperazine-1-carboxylate

To a solution of 6-cyclobutoxy-2-methyl-2H-indazole-5-carboxylic acid (100 mg, 0.406 mmol) and tert-butyl (S)-4-(6-aminopyridazin-3-yl)-2-methylpiperazine-1-carboxylate (120 mg, 0.406 mmol) in DMF (6 mL) were added DIEA (157 mg, 1.218 mmol) and HATU (200 mg, 0.528 mmol), the mixture was stirred for 16 h at RT. The mixture was purified by pre-HPLC (0.1% NH₃.H₂O in water / CH₃CN) to afford title compound (110 mg, 52.3% yield) as a grey solid. ESI-MS (M+H) +:522.2

### Step 2: Preparation of (S)-6-cyclobutoxy-2-methyl-N-(6-(3-methylpiperazin-1 -yl)pyridazin-3-yl)-2H-indazole-5-carboxamide HCl salt

A solution of tert-butyl (S)-4-(6-(6-cyclobutoxy-2-methyl-2H-indazole-5-carboxamido)pyridazin-3-yl)-2-methylpiperazine-1-carboxylate (100 mg, 0.19 mmol) in 3M HCl/EtOAc (4 mL) was stirred for 2 h at RT. The precipitate was filtered, washed with EtOAc (1 mL) and dried to afford title compound (66 mg, 82.5% yield) as a yellow solid. ¹H NMR (400 MHz, DMSO-*d₆*) δ 11.14 (s, 1H), 10.00 - 9.84 (m, 2H), 8.51 - 8.43 (m, 2H), 8.36 (s, 1H), 7.94 (d, J = 10.0 Hz, 1H), 6.94 (s, 1H), 4.99 - 4.90 (m, 1H), 4.46 - 4.35 (m, 2H), 4.15 (s, 3H), 3.59 - 3.48 (m, 1H), 3.42 - 3.28 (m, 3H), 3.19 - 3.08 (m, 1H), 2.60 - 2.54 (m, 2H), 2.26 - 2.15 (m, 2H), 1.90 - 1.81 (m, 1H), 1.73 (d, J = 9.6 Hz, 1H), 1.35 (d, J = 6.2 Hz, 3H). ESI-MS (M+H) +: 422.1.

### Example 56. N-(6-4,7-diazaspiro[2.5]octan-7-ylpyridazin-3-yl)-7-ethoxy-2-methylimidazo[1,2-a]pyridine-6-carboxamide (Compound 210)

### Preparation of Starting Material: 7-ethoxy-2-methylimidazo[1,2-a]pyridine-6-carboxylic acid

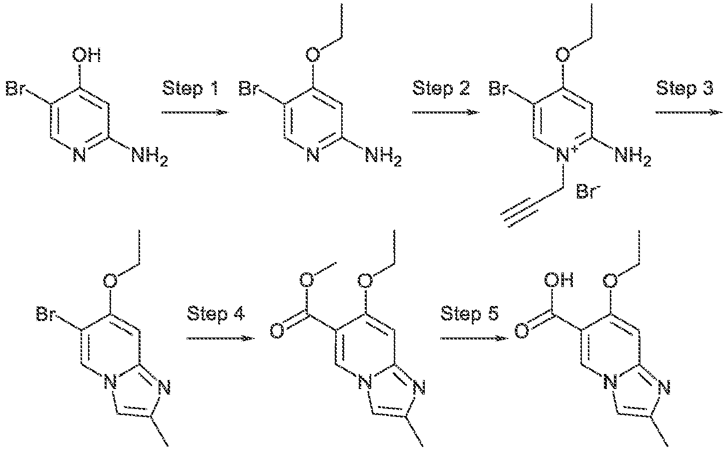

### Step 1: Preparation of 5-bromo-4-ethoxypyridin-2-amine

To a mixture of 2-amino-5-bromopyridin-4-ol (20.8 g, 110.05 mmol) and potassium carbonate (30.42 g, 220.09 mmol) in DMF (100 mL), iodoethane (20.6 g, 132.06 mmol) was added and the reaction mixture was stirred for two days at r.t. The resulting mixture was poured into water (200 mL) and extracted with EtOAc (2 × 100 mL). The organic layer was washed with H₂O (100 mL), brine (100 mL), dried over Na₂SO₄ and evaporated to give crude 5-bromo-4-ethoxypyridin-2-amine (11.4 g, Y: 43%) which was used in the next step without purification. ¹H NMR (500 MHz, DMSO-d₆) δ 7.80 (s, 1H), 6.01 (s, 1H), 6.00 (br s, 2H) 4.02 (q, J = 7.1 Hz, 2H), 1.33 (t, J = 6.9 Hz, 3H).

### Step 2: Preparation of 2-amino-5-bromo-4-ethoxy-1-(prop-2-yn-1-yl)pyridin-1-ium bromide

5-Bromo-4-ethoxypyridin-2-amine (11.4 g, 52.52 mmol), 3-bromoprop-1-yne (7.5 g, 63.02 mmol) and 2-propanol (200 mL) were added to a round bottom flask with a rubber septum and stirred vigorously at 80 °C for 6 hours. After that, the mixture was allowed to cool to room temperature and the excess of solvent/propargyl bromide was removed under high vacuum. The resulting crude residue of 2-amino-5-bromo-4-ethoxy-1-(prop-2-yn-1-yl)pyridin-1-ium bromide (18.0 g, Y: 86.7%) was used in the next step without purification. ¹H NMR (500 MHz, DMSO-d₆) δ 8.53 (br s, 3H), 6.66 (s, 1H), 5.00 (d, J = 2.6 Hz, 2H), 4.22 (q, J = 7.1 Hz, 2H), 3.82 - 3.67 (m, 1H), 1.38 (t, J = 6.9 Hz, 3H).

### Step 3: Preparation of 6-bromo-7-ethoxy-2-methylimidazo[1,2-a]pyridine

To a stirring solution of sodium hydroxide (2.57 g, 64.28 mmol) in deionized H₂O (100 mL), 2-amino-5-bromo-4-ethoxy-1-(prop-2-yn-1-yl)pyridin-1-ium bromide (18.0 g, 53.57 mmol) was added over a period of 5 minutes and the reaction mixture was stirred for 30 min at r.t. Immediately after adding, the solution phase turned yellow and the yellow oil began to disperse as a separate distinct phase. The oil product was subsequently extracted with EtOAc (2 × 60 mL), dried over anhydrous MgSO₄, filtered and concentrated under reduced pressure to afford 6-bromo-7-ethoxy-2-methylimidazo[1,2-a]pyridine (15.1 g, Y: 93.9%) as a pale yellow solid. ¹H NMR (500 MHz, DMSO-d₆) δ 8.73 (d, J = 2.6 Hz, 1H), 7.40 (d, J = 2.6 Hz, 1H), 6.88 (d, J = 2.6 Hz, 1H), 4.09 (q, J = 7.1 Hz, 2H), 2.23 (s, 3H), 1.36 (t, J = 6.9 Hz, 3H).

### Step 4: Preparation of 7-ethoxy-2-methylimidazo[1,2-a]pyridine-6-carboxylate

6-Bromo-7-ethoxy-2-methylimidazo[1,2-a]pyridine (15.1 g, 59.19 mmol) was dissolved in MeOH (100 mL) and [1,1'-bis(diphenylphosphino)ferrocene]dichloropalladium(II) dichloromethane adduct (966.71 mg, 1.18 mmol) was added followed by triethylamine (11.98 g, 118.38 mmol). The reaction mixture was transferred into an autoclave and stirred at 130 °C under CO pressure (40 bar) overnight. Then the MeOH was evaporated, and the residue was partitioned between water (100 mL) and EtOAc (200 mL). The organic layer was separated, dried over Na₂SO₄ and evaporated under reduced pressure to give crude methyl 7-ethoxy-2-methylimidazo[1,2-a]pyridine-6-carboxylate (12.5 g, Y: 72.1%) which was used in the next step without purification. ¹H NMR (500 MHz, DMSO-d₆) δ 8.92 (s, 1H), 7.55 (s, 1H), 6.85 (s, 1H), 4.07 (q, J = 7.1 Hz, 2H), 3.78 (s, 3H), 2.24 (s, 3H), 1.33 (t, J = 6.9 Hz, 3H).

### Step 5: Preparation of 7-ethoxy-2-methylimidazo[1,2-a]pyridine-6-carboxylic acid

A mixture of methyl 7-ethoxy-2-methylimidazo[1,2-a]pyridine-6-carboxylate (12.5 g, 53.36 mmol) and potassium hydroxide (4.49 g, 80.05 mmol) was stirred in a mixture of methanol (80 mL) and H₂O (60 mL) overnight. The resulting mixture was concentrated under reduced pressure to remove methanol, and the resulting aqueous solution was neutralized with 1 N HCl to pH=5 to precipitate the carboxylic acid. The solid 7-ethoxy-2-methylimidazo[1,2-a]pyridine-6-carboxylic acid (6.0 g, Y: 48.5%) was isolated by filtration, dried, and used directly in the next step without further purification. ¹H NMR (500 MHz, DMSO-d₆) δ 8.85 (s, 1H), 7.52 (s, 1H), 6.81 (s, 1H), 4.06 (q, J = 7.1 Hz, 2H), 2.24 (s, 3H), 1.33 (t, J = 6.9 Hz, 3H).

### Preparation of Starting Material: tert-butyl 7-(6-aminopyridazin-3-yl)-4,7-diazaspim[2.5]octane-4-carboxylate

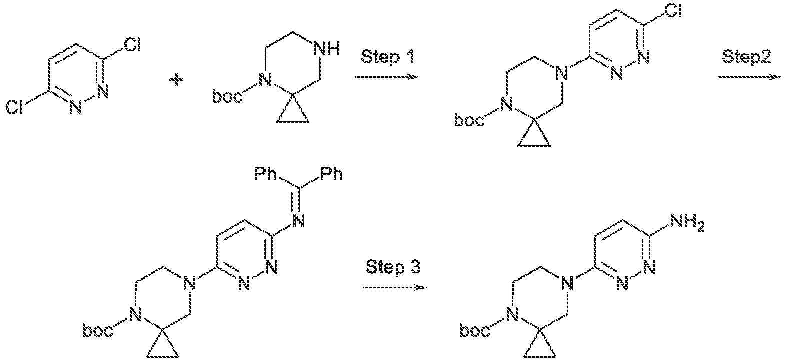

### Step 1: Preparation of tert-butyl 7-(6-chloropyridazin-3-yl)-4,7-diazaspiro[2.5]octane-4-carboxylate

3,6-Dichloropyridazine (842.11 mg, 5.65 mmol) in anhydrous toluene (10 mL) was treated with 4-Boc-4,7-diazaspiro[2.5]octane (1.2 g, 5.65 mmol) and triethylamine (1.72 g, 16.96 mmol). The reaction mixture was heated under reflux for 16 hours, concentrated under reduced pressure, and the residue was purified on SiO₂ (5%MeOH/CH₂Cl₂/1%NH₄OH) to provide the title tert-butyl 7-(6-chloropyridazin-3-yl)-4,7-diazaspiro[2.5]-octane-4-carboxylate (1.05 g, Y: 57.2%) as a white solid. ESI-MS (M+H)+: 325.2. ¹H NMR (400 MHz, DMSO-d₆) δ 7.48 (d, J = 9.5, 1H), 7.33 (d, J = 9.5, 1.6 Hz, 1H), 3.55 (m, 2H), 3.4 (s, 2H), 2.93 (m, 2H), 1.37 (s, 9H), 0.87 (m, 2H), 0.77 (m, 2H).

### Step 2: Preparation of tert-butyl 7-6-[(diphenylmethylidene)-amino]pyridazin-3-yl-4,7-diazaspiro[2.5]-octane-4-carboxylate

Tert-butyl 7-(6-chloropyridazin-3-yl)-4,7-diazaspiro[2.5]octane-4-carboxylate (949.7 mg, 2.92 mmol), diphenylmethanimine (582.9 mg, 3.22 mmol), Pd₂(dba)₃ (133.87 mg, 146.2 µmol), 1-[2-(diphenylphosphanyl)naphthalen-1-yl]naphthalen-2-yldiphenylphosphane (182.06 mg, 292.39 µmol) and cesium carbonate (1.91 g, 5.85 mmol) were suspended in dioxane (20.0 mL), and the suspension was stirred at 100 °C overnight. The resultant reaction mixture was cooled to room temperature and then filtered through Celite, and the precipitate was washed with ethyl acetate (30 mL). The filtrate was washed with saturated brine (10 mL), dried over anhydrous magnesium sulfate and concentrated under reduced pressure. The residue was purified by column chromatography (Hex/EtOAc 2/1 as an eluent) to give pure tert-butyl 7-6-[(diphenylmethylidene)-amino]pyridazin-3-yl-4,7-diazaspiro[2.5]octane-4-carboxylate (1.0 g, Y: 72.8%). ¹H NMR (500 MHz, DMSO-d₆) δ 7.71-7.65 (m, 2H), 7.60-7.55 (m, 1H), 7.53-7.47 (m, 2H), 7.34 (s, 3H), 7.18-7.09 (m, 3H), 6.90-6.85 (m, 1H), 3.55-3.47 (m, , 2H), 3.46-3.39 (m, , 2H), 2.55-2.53 (m, 2H) 1.40 (s, 9H), 0.90-0.85 (m, 2H), 0.78-0.72 (m, 2H).

### Step 3: Preparation of tert-butyl 7-(6-aminopyridazin-3-yl)-4,7-diazaspiro[2.5]octane-4-carboxylate

Tert-butyl 7-6-[(diphenylmethylidene)amino]pyridazin-3-yl-4,7-diazaspiro[2.5]octane-4-carboxylate (999.51 mg, 2.13 mmol) was dissolved in THF (10 mL). To the solution was added an aqueous solution 2-hydroxy-1,2,3-propanetricarboxylic acid monohydrate (2 M; 2.24 g, 10.64 mmol) and the mixture was stirred at room temperature overnight. The resultant reaction mixture was neutralized with a saturated aqueous sodium hydrogen carbonate solution (25 mL), and the mixture was extracted twice with ethyl acetate (2 × 15 mL). The organic phases were combined, dried over anhydrous magnesium sulfate and concentrated under reduced pressure. The crude residue was triturated with MTBE (10 mL) and the precipitate was filtered, washed with MTBE (5 mL) and dried in vacuo to yield tert-butyl 7-(6-aminopyridazin-3-yl)-4,7-diazaspiro[2.5]octane-4-carboxylate (260.0 mg, Y: 38% yield) as a white solid. ESI-MS (M+H)+: 306.2. ¹H NMR (500 MHz, Chloroform-d) δ 6.90 (d, J = 9.6 Hz, 1H), 6.81 (d, J = 9.6 Hz, 1H), 4.69 (br s, 2H), 3.71-3.66 (m, 2H), 3.45 - 3.37 (m, 2H), 3.29 (s, 2H), 1.46 (s, 9H), 1.05-0.99 (m, 2H), 0.85-0.80 (m, 2H).

### N-(6-4,7-diazaspiro[2.5]octan-7-ylpyridazin-3-yl)-7-ethoxy-2-methylimidazo[1,2-a]pyridine-6-carboxamide

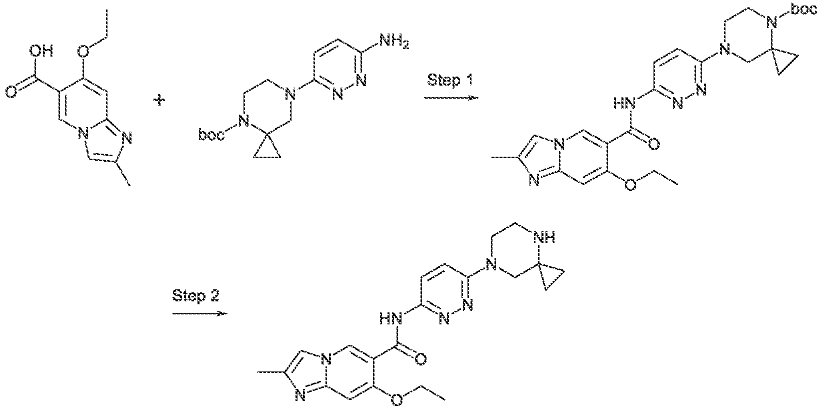

### Step 1: Preparation of tert-butyl 7-(6-amino-pyridazin-3-yl)-4,7-diazaspiro[2.5]octane-4-carboxylate

7-Ethoxy-2-methylimidazo[1,2-a]pyridine-6-carboxylic acid (100.0 mg, 454.08 µmol) was suspended in DMF (4 mL) and ethylbis(propan-2-yl)amine (146.41 mg, 1.13 mmol) was added followed by [(dimethylamino)(3H-[1,2,3]triazolo[4,5-b]pyridin-3-yloxy)methylidene]dimethylazanium; hexafluoro-lambda5-phosphanuide (HATU) (206.75 mg, 543.75 µmol). The resulting mixture was stirred for 30 min at r.t. After that, tert-butyl 7-(6-amino-pyridazin-3-yl)-4,7-diazaspiro[2.5]octane-4-carboxylate (138.37 mg, 453.13 µmol) was added in one portion and the reaction mixture was stirred overnight at r.t. The precipitate formed was filtered, washed with MeCN (2 mL), MTBE (2 mL), dried in vacuo to give pure tert-butyl 7-(6-7-ethoxy-2-methylimidazo[1,2-a]pyridine-6-amidopyridazin-3-yl)-4,7-diazaspiro[2.5]octane-4-carboxylate (88.0 mg, 173.37 µmol, 38.3% yield). ESI-MS (M+H)+: 508.4.

### Step 2: Preparation of N-(6-4,7-diazaspiro[2.5]octan-7-ylpyridazin-3-yl)-7-ethoxy-2-methylimidazo[l, 2-a]pyridine-6-carboxamide

To a solution of tert-butyl 7-(6-7-ethoxy-2-methylimidazo[1,2-a]pyridine-6-amidopyridazin-3-yl)-4,7-diazaspiro-[2.5]octane-4-carboxylate (87.99 mg, 173.35 µmol) in dichloromethane (20 mL) 2,2,2-trifluoroacetic acid (197.66 mg, 1.73 mmol) was added and the resulting mixture was stirred overnight at r.t. Then the solvents were evaporated to dryness under reduced pressure. The crude residue was crystallized from MTBE/MeCN (4/1, ~5 mL) to obtain N-(6-4,7-diazaspiro[2.5]octan-7-ylpyridazin-3-yl)-7-ethoxy-2-methylimidazo[1,2-a]pyridine-6-carboxamide as TFA salt (64.5 mg, Y: 67.8%). ESI-MS (M+H)+: 408.2. ¹H NMR (500 MHz, DMSO-d₆): δ 11.01 (s, 1H), 9.51 (br s, 1H), 9.2 (s, 1H), 8.22 (s, 1H), 7.92 (s, 1H), 7.53 (s, 1H), 7.34 (s, 1H), 4.35 (q, J = 7.1 Hz, 2H), 3.87 (s, 2H) , 3.55 (s, 2H), 2.43 (s, 3H), 1.45 (t, J = 6.9 Hz, 3H), 1.15-1.05 (m, 2H), 0.98-90 (m, 2H).

### Example 57. Synthesis of (S)-2-methyl-N-(6-(3-methylpiperazin-1-yl)pyridazin-3-yl)-7-((tetrahydro-2H-pyran-4-yl)oxy)imidazo[1,2-a]pyridine-6-carboxamide hydrochloride (Compound 211)

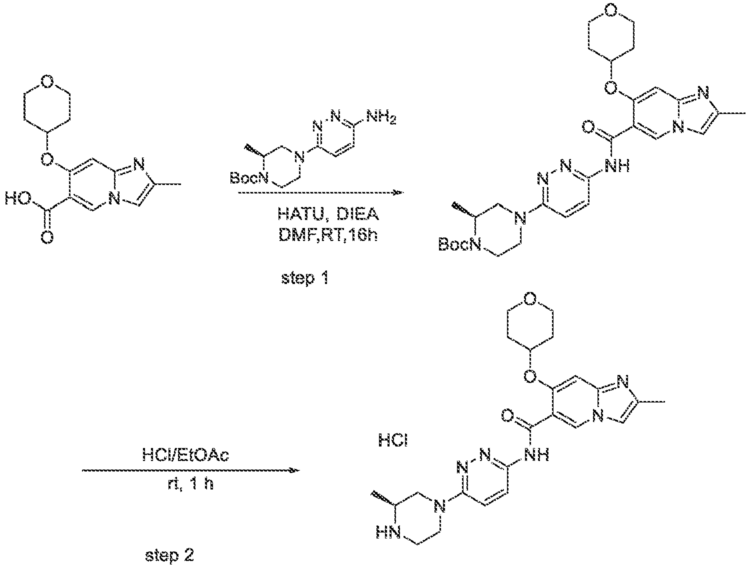

### Preparation of 2-methyl- 7-((tetrahydro-2H-pyran-4-yl)oxy)imidazo[1,2-a]pyridine-6-carboxylic acid

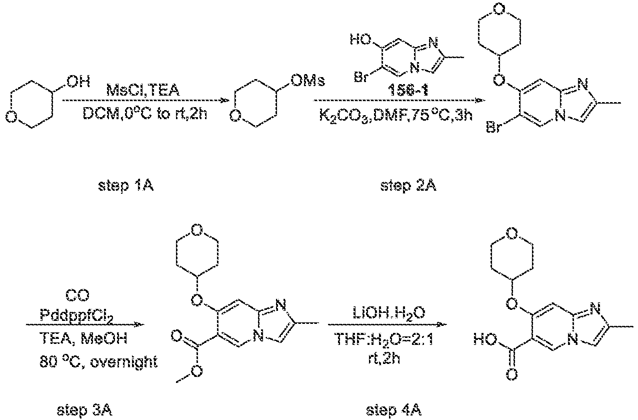

### Step 1A: preparation of tetrahydro-2H-pyran-4-yl methanesulfonate

To a solution of tetrahydro-2H-pyran-4-ol (5.0 g, 49 mmol) in DCM (50 mL) was added TEA (15 g, 147 mmol) and MsCl (6.8 g, 58.8 mmol) at 0 °C, the mixture was stirred for 2 h at RT. The mixture was washed with water and brine, dried over Na₂SO₄ and filtered. The crude was purified by silica gel column chromatography eluted with PE:EtOAc=1:2 to afford title compound (8.3 g, 86.1% yield) as a colorless oil. ¹H NMR (400 MHz, CDCl₃) δ 4.91 - 4.90 (m, 1H), 3.99 - 3.89 (m, 2H), 3.61 - 3.50 (m, 2H), 3.04 (s, 3H), 2.10 - 2.00 (m, 2H), 1.93 - 1.83 (m, 2H).

### Step 2A: preparation of 6-bromo-2-methyl-7-((tetrahydro-2H-pyran-4-yl)oxy)imidazo[1,2-a]pyridine

To a solution of 6-bromo-2-methylimidazo[1,2-a]pyridin-7-ol (500 mg, 2.19 mmol) in DMF (10 mL) were added K₂CO₃ (0.92 g, 6.6 mmol) and tetrahydro-2H-pyran-4-yl methanesulfonate (800 mg, 4.8 mmol), the mixture was stirred for 3 h at 75 °C. The mixture was diluted with water (50 mL) and extracted with EtOAc (50 mL x3). The organics were washed and brine, dried over Na₂SO₄ and filtered. The filtrate was concentrated and the crude was purified by C18 column chromatography eluted with MeCN:H₂O=20% to 60% to afford title compound (400 mg, 58.8% yield) as a grey solid. ESI-MS (M+H) ⁺:310.8.

### Step 3A: preparation of methyl 2-methyl-7- ((tetrahydro-2H-pyran-4-yl)oxy)imidazo[1,2-ajpyndine-6-carboxylate

To a solution of 6-bromo-2-methyl-7-((tetrahydrofuran-3-yl)oxy)imidazo[1,2-a]pyridine (400 mg, 1.29 mmol) in MeOH (50 mL) were added Pd(dppf)Cl₂ (141 mg, 0.2 mmol) and TEA (1.0 g, 10 mmol), the mixture was charged with CO for three times and stirred overnight at 80°C under CO balloon. The mixture was filtered and the filtrate was concentrated to afford title compound (600 mg, crude) as a grey solid. ESI-MS (M+H)⁺: 290.9

### Step 4A: preparation 2-methyl-7-((tetrahydro-2H-pyran-4-yl)oxy)imidazo[1,2-a]pyridine-6-carboxylic acid

To a solution of methyl 2-methyl-7-((tetrahydro-2H-pyran-4-yl)oxy)imidazo[1,2-a]pyridine-6-carboxylate (600 mg, crude) in THF (8 mL) and H₂O (4 mL) was added LiOH (200 mg, 8 mmol), the mixture was stirred for 2h at RT. The mixture was diluted with water (10 mL) extracted with EtOAc (20mL x3). Aqueous phase was adjusted to pH=2 with 1M HCl and purified by C18 column chromatography eluted with MeCN:H₂O (FA)=0% to 15% to afford title compound (70 mg, 19.7% yield for two steps) as a white solid. ESI-MS (M+H) ⁺:277.0.

### Step 1: Preparation of tert-butyl (S)-2-methyl-4-(6-(2-methyl-7-((tetrahydro-2H-pyran-4-yl)oxy)imidazo[1,2-a]pyridine-6-carboxamido)pyridazin-3-yl)piperazine-1-carboxylate

To a solution of 2-methyl-7-((tetrahydro-2H-pyran-4-yl)oxy)imidazo[1,2-a]pyridine-6-carboxylic acid (60 mg, 0.22 mmol) and tert-butyl (S)-4-(6-aminopyridazin-3-yl)-2-methylpiperazine-1-carboxylate (60mg, 0.20 mmol) in DMF (1.5 mL) were added DIPEA (100 mg, 0.775 mmol) and HATU (120 mg, 0.31 mmol), the mixture was stirred for 16 h at RT. The mixture was purified by pre-HPLC (0.05% FA in water / CH₃CN) to afford title compound (10 mg, 8.8% yield) as a grey solid, ESI-MS (M+H) +:552.2.

### Step 2: Preparation of (S)-2-methyl-N-(6-(3-methylpiperazin-1-yl)pyridazin-3-yl)-7-((tetrahydro-2H-pyran-4-yl)oxy)imidazo[1,2-a]pyridine-6-carboxamide hydrochloride

A solution of tert-butyl (S)-2-methyl-4-(6-(2-methyl-7-((tetrahydro-2H-pyran-4-yl)oxy)imidazo[1,2-a]pyridine-6-carboxamido)pyridazin-3-yl)piperazine-1-carboxylate (10 mg, 0.022 mmol) in 3M HCl/EtOAc (2 mL) was stirred for 1h at RT. The precipitate was filtered, washed with EtOAc (1 mL) and dried to afford title compound (5.5 mg, 63.0% yield) as a yellow solid, ESI-MS (M+H) +: 452.2. ¹H NMR (400 MHz, MeOD-*d₄*) δ 9.16 (s, 1H), 8.57 (d, J = 9.2 Hz, 1H), 8.14 (d, J = 9.4 Hz, 1H), 7.87 (s, 1H), 7.52 (s, 1H), 5.12 (br s, 1H), 4.51 - 4.42 (m, 2H), 3.94 (br s, 2H), 3.73 - 3.57 (m, 5H), 3.48 - 3.37 (m, 2H), 2.52 (s, 3H), 2.21 (br s, 2H), 1.92 (br s, 2H), 1.29 (br s, 3H).

### Example 58. Synthesis of 2-methyl-N-(6-((S)-3-methylpiperazin-1-yl)pyridazin-3-yl)-7-((tetrahydrofuran-3-yl)oxy)imidazo[1,2-a]pyridine-6-carboxamide (Compounds 212 and 213)

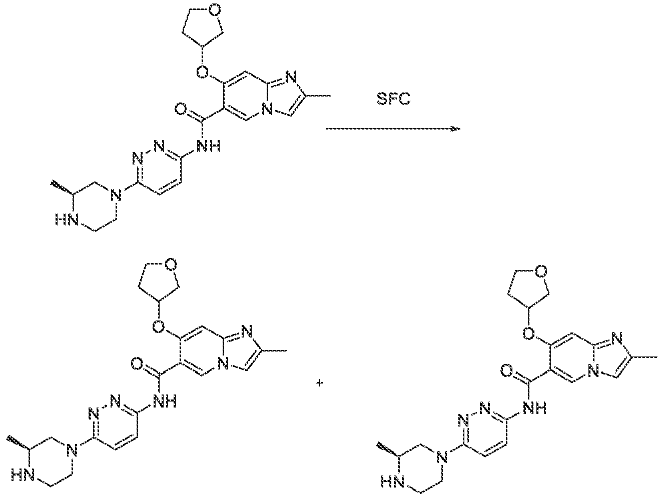

### Step 1: Preparation of 2-methyl-N-(6-((S)-3-methylpiperazin-1-yl)pyridazin-3-yl)-7-((tetrahydrofuran-3-yl)oxy)imidazo[l, 2-a]pyridine-6-carboxamide

2-methyl-N-(6-((S)-3-methylpiperazin-1-yl)pyridazin-3-yl)-7-((tetrahydrofuran-3-yl)oxy)imidazo[1,2-a]pyridine-6-carboxamide (20 mg) was obtained by SFC to afford title compound 212 (7 mg, 35%) as a white solid and compound 213 (7.64 mg, 38.2%) as a gray solid. ESI-MS (M+H) +:438.1.

### Compound 212

¹H NMR (400 MHz, MeOD-*d₄*) δ 8.89 (s, 1H), 8.19 (d, J = 9.8 Hz, 1H), 7.43 (s, 1H), 7.23 (d, J = 9.9 Hz, 1H), 6.79 (s, 1H), 5.28 - 5.21 (m, 1H), 4.11 - 3.96 (m, 5H), 3.89 - 3.79 (m, 1H), 2.98 (d, J = 10.0 Hz, 1H), 2.87 - 2.73 (m, 3H), 2.51 - 2.44 (m, 1H), 2.41 - 2.23 (m, 5H), 1.06 (d, J = 6.3 Hz, 3H).

### Compound 213

¹H NMR (400 MHz, MeOD-*d₄*) δ 8.98 (s, 1H), 8.29 (d, *J=* 9.6 Hz, 1H), 7.53 (s, 1H), 7.32 (d, *J=* 9.9 Hz, 1H), 6.88 (s, 1H), 5.38 - 5.26 (m, 1H), 4.19 - 4.02 (m, 5H), 3.99 - 3.86 (m, 1H), 3.09 - 3.02 (m, 1H), 2.97 - 2.81 (m, 3H), 2.55 (dd, *J=* 24.4, 13.2 Hz, 1H), 2.48 - 2.29 (m, 5H), 1.16 (d, *J =* 6.1 Hz, 3H).

### Example 59. Synthesis of 8-ethyl-2-methyl-N-{6-[(3S)-3-methylpiperazin-1-yl]pyridazin-3-yl}imidazo[1,2-a]pyrazine-6-carboxamide (Compound 214)

### Preparation of Starting Material: 8-ethyl-2-methylimidazo[1,2-a]pyrazine-6-carboxamide

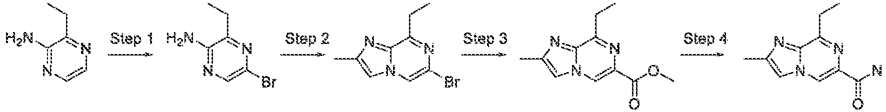

### Step 1: Preparation of 5-bromo-3-ethylpyrazin-2-amine

3-Ethylpyrazin-2-amine (10.73 g, 87.13 mmol) and pyridine (7.58 g, 95.84 mmol, 7.75 mL) were mixed in CHCl₃ (200 mL), and bromine (14.62 g, 91.48 mmol, 4.69 mL) was added dropwise, the mixture was left to stir at r.t. overnight. After that the resulting mixture was washed with water and brine and evaporated under reduced pressure to give pure 5-bromo-3-ethylpyrazin-2-amine (15.8 g, Y: 80.8%). ¹H NMR (500 MHz, Chloroform-d) δ 7.93 (s, 1H), 4.61 (br s, 2H), 2.62 (q, J = 7.4 Hz, 2H), 1.29 (t, J = 7.6 Hz, 3H).

### Step 2: Preparation of 6-bromo-8-ethyl-2-methylimidazo[1,2-a]pyrazine

To a mixture of 4-methylbenzene-1-sulfonic acid hydrate (1.49 g, 7.82 mmol) and pyridine (618.53 mg, 7.82 mmol, 630.0 µL) in i-PrOH (100 mL) was added 5-bromo-3-ethylpyrazin-2-amine (15.8 g, 78.2 mmol) and 1-bromo-2,2-dimethoxypropane (16.46 g, 89.93 mmol, 12.16 mL) and the reaction mixture was heated to 90 °C for 4 h. The resulting mixture was diluted with DCM, washed with a saturated sodium hydrogen carbonate solution, dried (Na₂SO₄) and concentrated under reduced pressure to give pure 6-bromo-8-ethyl-2-methylimidazo[1,2-a]pyrazine (16.0 g, Y: 85.2%). ESI-MS (M+H)+: 240.0. ¹H NMR (400 MHz, DMSO-d6) δ 9.09 (s, 1H), 8.13 (s, 1H), 3.11 (q, J = 7.4 Hz, 2H), 2.50 (s, 3H), 1.28 (t, J = 7.6 Hz, 3H).

### Step 3: Preparation of methyl 8-ethyl-2-methylimidazo[1,2-a]pyrazine-6-carboxylate

6-Bromo-8-ethyl-2-methylimidazo[1,2-a]pyrazine (5.0 g, 20.82 mmol), triethylamine (2.53 g, 24.98 mmol) and [1,1'-bis(diphenylphosphino)ferrocene]dichloropalladium(II), complex with dichloromethane (1.7 g, 2.08 mmol) were dissolved in dry MeOH (200 mL). The reaction mixture was heated at 125 °C in high pressure vessel under CO pressure (20 atm) for 48 h. The solvent was evaporated, and the mixture was poured into water (250 mL). The resulting mixture was extracted with EtOAc (2 × 100 mL) and the organics were dried over Na₂SO₄ and evaporated to dryness to give pure methyl 8-ethyl-2-methylimidazo[1,2-a]pyrazine-6-carboxylate (4.0 g, Y: 87.6%). ESI-MS (M+H)+: 220.2. ¹H NMR (400 MHz, DMSO-d₆) δ 9.21 (s, 1H), 7.97 (s, 1H), 3.88 (s, 3H), 3.12 (q, J = 7.5 Hz, 2H), 2.42 (s, 3H), 1.33 (t, J = 7.6 Hz, 3H).

### Step 4: Preparation of 8-ethyl-2-methylimidazo[1,2-a]pyrazine-6-carboxamide

Methyl 8-ethyl-2-methylimidazo[1,2-a]pyrazine-6-carboxylate (400.0 mg, 1.82 mmol) was dissolved in NH₃/MeOH (10 mL) and the reaction mixture was sealed and heated to 90 °C overnight. After work-up with EtOAc pure 8-ethyl-2-methylimidazo[1,2-a]pyrazine-6-carboxamide (300.0 mg, Y: 80.4%) was obtained. ¹H NMR (400 MHz, DMSO-d₆) δ 8.97 (s, 1H), 7.93 (s, 1H), 7.89 (s, 1H), 7.65 (s, 1H), 3.09 (q, J = 7.5 Hz, 2H), 2.37 (s, 3H), 1.33 (t, J = 7.6 Hz, 3H).

### 8-ethyl-2-methyl-N-{6-[(3S)-3-methylpiperazin-1-yl]pyridazin-3-yl}imidazo[1,2-a]pyrazine-6-carboxamide;

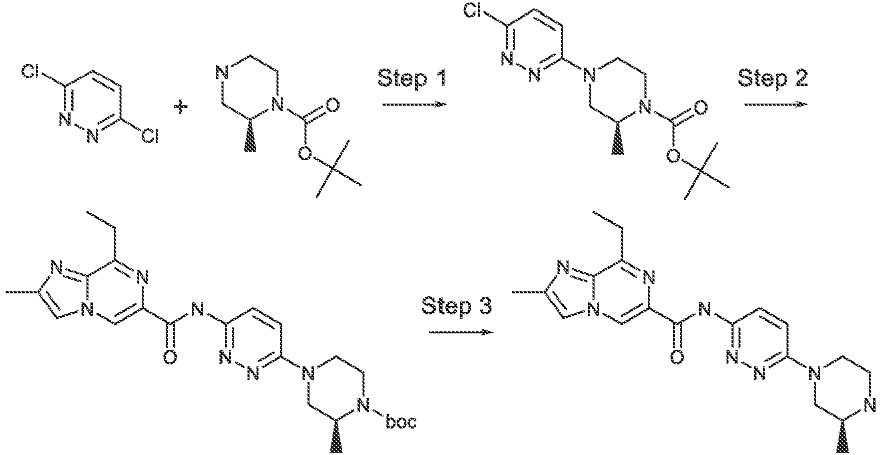

### Step 1: Preparation of tert-butyl (2S)-4-(6-chloropyridazin-3-yl)-2-methylpiperazine-1-carboxylate

To a solution of 3,6-dichloropyridazine (5.0 g, 33.56 mmol) in DMF (100 mL) were added ethylbis(propan-2-yl)-amine (4.54 g, 35.16 mmol, 6.12 mL) and tert-butyl (2S)-2-methylpiperazine-1-carboxylate (6.4 g. 31.96 mmol). The reaction mixture was heated to 90 °C for 17 h and after cooling to room temperature, partitioned between ethyl acetate (200 mL) and H₂O (100 mL). The aqueous layer was extracted with ethyl acetate (2 × 80 mL) and the combined organic layers were washed with H₂O (200 mL), brine (200 mL), dried (Na₂SO₄), filtered and the solvent was removed under reduced pressure to give the crude product. Purification by flash silica chromatography gave the desired tert-butyl (2S)-4-(6-chloropyridazin-3-yl)-2-methylpiperazine-1-carboxylate (5.88 g, Y: 56%). ESI-MS (M+H)+: 313.2

### Step 2: Preparation of tert-butyl (2S)-4-(6-{8-ethyl-2-methylimidazo[1,2-a]pyrazine-6-amido}pyridazin-3-yl)-2-methylpiperazine-1-carboxylate

8-Ethyl-2-methylimidazo[1,2-a]pyrazine-6-carboxamide (118.57 mg, 580.57 µmol), tert-butyl (2S)-4-(6-chloro-pyridazin-3-yl)-2-methylpiperazine-1-carboxylate (199.76 mg, 638.63 µmol), tris((1E,4E)-1,5-diphenylpenta-1,4-dien-3-one) dipalladium (53.16 mg, 58.06 µmol), [5-(diphenylphosphanyl)-9,9-dimethyl-9H-xanthen-4-yl]-diphenylphosphane (67.19 mg, 116.11 µmol), and cesium carbonate (567.48 mg, 1.74 mmol) were mixed in dioxane under an argon atmosphere and the reaction mixture was heated to 100 °C for 17 h. After evaporation and HPLC, tert-butyl (2S)-4-(6-8-ethyl-2-methylimidazo[1,2-a]pyrazine-6-amidopyridazin-3-yl)-2-methyl-piperazine-1-carboxylate (200.0 mg, Y: 61.6%) was obtained. ESI-MS (M+H)+: 481.4.

### Step 3: Preparation of 8-ethyl-2-methyl-N-{6-[(3S)-3-methylpiperazin-1-yl]pyridazin-3-yl}imidazo[1,2-a]pyrazine-6-carboxamide; trifluoroacetic acid

Tert-butyl (2S)-4-(6-8-ethyl-2-methylimidazo[1,2-a]pyrazine-6-amidopyridazin-3-yl)-2-methylpiperazine-1-carboxylate (0.2 g, 0.42 mmol) was dissolved in a mixture of DCM (1 mL) and TFA (1 mL) and the reaction mixture was stirred at r.t. overnight. After full evaporation and HPLC, pure 8-ethyl-2-methyl-N-6-[(3S)-3- methylpiperazin-1-yl]pyridazin-3-ylimidazo[1,2-a]pyrazine-6-carboxamide as trifluoroacetic acid salt (117.9 mg, Y: 58% yield) was obtained. ESI-MS (M+H)+: 381.2. ¹H NMR (400 MHz, DMSO-d₆) δ 10.55 (s, 1H), 9.25 (s, 1H), 9.05 (s, 1H), 8.75 (s, 1H), 8.32 (d, J = 9.8 Hz, 1H), 8.07 (s, 1H), 7.58 (d, J = 9.8 Hz, 1H), 4.36 (d, J = 13.2 Hz, 2H), 3.46-3.35 (m, 2H), 3.29 - 3.07 (m, 4H), 3.00 (q, J = 7.6 Hz, 1H), 2.45 (s, 3H), 1.43 (t, J = 7.6 Hz, 3H), 1.28 (d, J = 6.1 Hz, 3H).

### Example 60. Synthesis of (S)-6-ethoxy-2-methyl-N-(6-(3-methylpiperazin-1-yl)pyridazin-3-yl)pyrazolo[1,5-a]pyridine-5-carboxamide HCl salt (Compound 215)

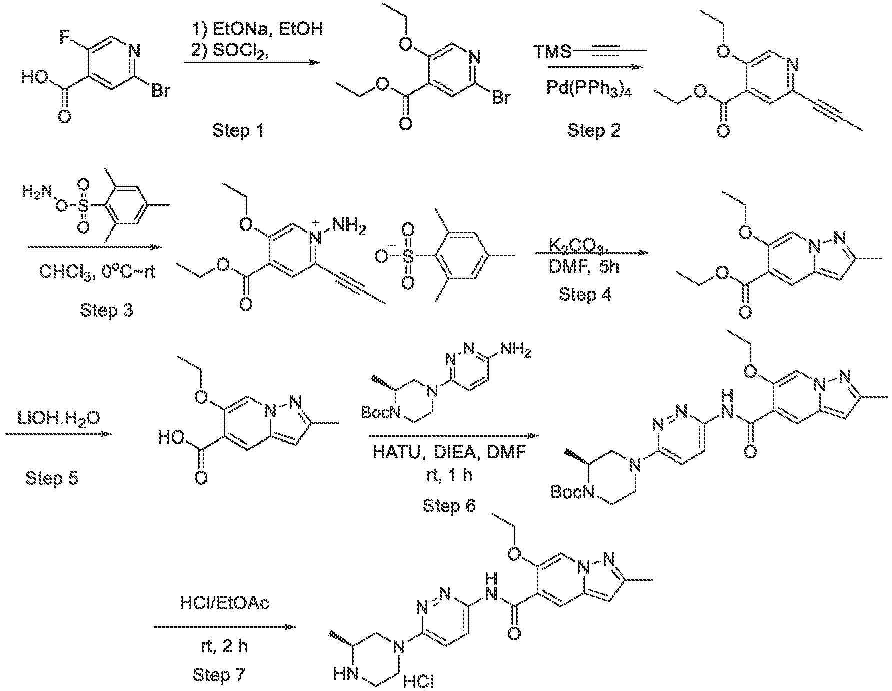

### Step 1: Preparation of ethyl 2-bromo-5-ethoxyisonicotinate.

To a mixture of 2-bromo-5-fluoroisonicotinic acid (3.0 g, 13.6 mmol) in EtOH (30 mL) was added EtONa (2.8 g, 54.6 mmol). The mixture was stirred at 65 °C for 16 h. After cooling to rt, to the reaction mixture was added SOCl₂ (3.0 mL, 4.9 g, 41.3 mmol). The reaction mixture was stirred for 2 days. After concentration, the residue was diluted with EA (100 mL), washed with water and brine, dried with Na₂SO₄. After concentration, the crude product was purified by silica gel column (PE/EA= 5:1) to afford title product (3.0 g, Y: 80.5%) as a white solid. ESI-MS (M+H)+: 273.9.

### Step 2: Preparation of ethyl 5-ethoxy-2-(prop-1-yn-1-yl)isonicotinate.

A mixture of ethyl 2-bromo-5-ethoxyisonicotinate (0.36 g, 1.3 mmol), trimethyl(prop-1-yn-1-yl)silane (0.16 g, 1.4 mmol), CuI (76 mg, 0.4 mmol), Pd(PPh₃)₄ (150 mg, 0.13 mmol), TEA (0.56 mL, 3.9 mmol) and TBAF (1.0 M, 1.3 mL, 1.3 mmol ) in toluene (10 mL) was stirred at r.t. for 5 h. The reaction mixture was diluted with EA (200 mL), washed with brine and water, dried with Na₂SO₄. After concentration, the residue was purified by silica gel column (PE/EA= 3:1) to afford title product (100 mg, Y: 33%) as yellow solid. ESI-MS (M+H)+: 234.1. ¹H NMR (400 MHz, CDCl₃) δ 8.32 (s, 1H), 7.64 (s, 1H), 4.37 (q, J = 7.1 Hz, 2H), 4.23 (q, J = 7.0 Hz, 2H), 2.06 (s, 3H), 1.47 (t, J = 7.1 Hz, 3H), 1.38 (t, J = 7.1 Hz, 3H).

### Step 3 and 4: Preparation of ethyl 6-ethoxy-2-methylpyrazolo[1,5-a]pyridine-5-carboxylate.

A mixture of ethyl 5-ethoxy-2-(prop-1-yn-1-yl)isonicotinate (160 mg, 0.67 mmol) and O-(mesitylsulfonyl)hydroxylamine (740 mg, 3.4 mmol) in trichloromethane (5 mL) was stirred at r.t. for 16 h. The reaction mixture was concentrated, the residue was diluted with DMF (5 mL), K₂CO₃ (180 mg, 1.3 mmol) was added and stirred at r.t. for 5 h. The reaction mixture was diluted with EA (120 mL), washed with brine and water, dried with Na₂SO₄. After concentration, the crude product was purified by silica gel column (PE/EA= 3:1) to afford title product (100 mg, Y: 60%) as a colorless oil. ESI-MS (M+H)+: 249.0.

### Step 5: Preparation of 6-ethoxy-2-methylpyrazolo[1,5-a]pyridine-5-carboxylic acid.

To a mixture of ethyl 6-ethoxy-2-methylpyrazolo[1,5-a]pyridine-5-carboxylate (100 mg, 0.4 mmol) in THF/water (10 mL, 2:1) was added LiOH.H₂O (50 mg, 1.2 mmol). The mixture was stirred at rt for 2 h. After concentration, the residue was adjusted to pH = 5 with 1M HCl. The crude was purified by pre-HPLC to give title product (80 mg, Y: 90%) as a colorless oil. ESI-MS (M+H)+: 221.0. ¹H NMR (400 MHz, CDCl₃) δ 8.38 - 8.24 (m, 2H), 6.48 (s, 1H), 4.26 (q, J = 6.9 Hz, 2H), 2.49 (s, 3H), 1.59 (t, J = 6.9 Hz, 3H).

### Step 6: Preparation of tert-butyl (S)-4-(6-(6-ethoxy-2-methylpyrazolo[1,5-a]pyridine-5-carboxamido)pyridazin-3-yl)-2-methylpiperazine-1-carboxylate.

To a mixture of 6-ethoxy-2-methylpyrazolo[1,5-a]pyridine-5-carboxylic acid (100 mg, 0.45 mmol) in DMF (5 mL) were added tert-butyl (S)-4-(6-aminopyridazin-3-yl)-2-methylpiperazine-1-carboxylate (146 mg, 0.5 mmol), DIEA (116 mg, 0.9 mmol) and HATU (190 mg, 0.5 mmol). The mixture was stirred at rt for 5 h and quenched with water (20 mL), extracted with EA (60mL × 2). Combined the organic layers, dried with Na₂SO₄. After concentration, the residue was purified by silica gel column (PE/EA= 2:1) to afford title product (100 mg, 45 %) as a yellow solid. ESI-MS (M+H)+: 496.1.

### Step 7: Preparation of (S)-6-ethoxy-2-methyl-N-(6-(3-methylpiperazin-1-yl)pyridazin-3-yl)pyrazolo[1,5-a]pyridine-5-carboxamide HCl salt.

To a solution of tert-butyl (S)-4-(6-(6-ethoxy-2-methylpyrazolo[1,5-a]pyridine-5-carboxamido)pyridazin-3-yl)-2-methylpiperazine-1-carboxylate (50 mg, 0.1 mmol) in EA (2 mL) was added HCl-EA (3 M, 2 mL) at 0 °C and the mixture was allowed to warm to rt and stirred for 2 h. After concentration, the residue purified by prep-HPLC (0.05% HCl in water / MeCN) to give title compound (20.0 mg, yield: 50%) as a yellow solid. ESI-MS (M+H)+: 396.1. ¹H NMR (400 MHz, DMSO-d₆) δ 10.98 (s, 1H), 9.50 (s, 1H), 9.36 (s, 1H), 8.51 (s, 1H), 8.32 (d, J = 9.7 Hz, 1H), 8.10 (s, 1H), 7.64 (d, J = 9.9 Hz, 1H), 6.56 (s, 1H), 4.36 (t, J = 12.0 Hz, 2H), 4.25 - 4.19 (m, 2H), 3.39 - 3.33 (m, 3H), 3.15 - 3.08 (m, 2H), 2.39 (s, 3H), 1.45 (t, J = 6.9 Hz, 3H), 1.32 (d, J = 6.5 Hz, 3H).

### Example 61. (S)-2-methyl-N-(6-(3-methylpiperazin-1-yl)pyridazin-3-yl)-7-(2,2,2-trifluoroethoxy)imidazo[1,2-a]pyridine-6-carboxamide (Compound 216)

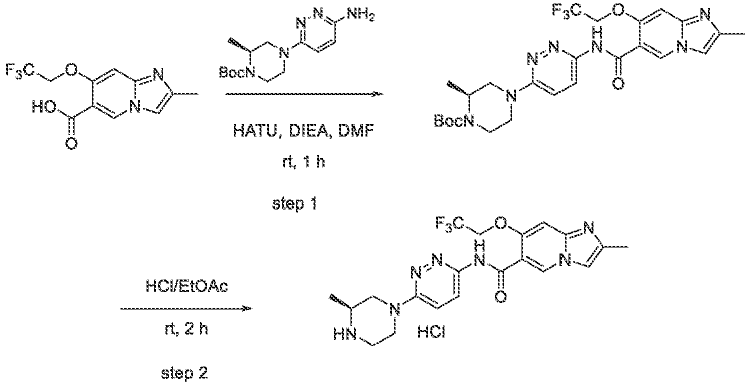

### Preparation of tert-butyl (S)-2-methyl-4-(6-(2-methyl-7-(2, 2,2-trifluoroethoxy)imidazo[1,2-a]pyridine-6-carboxamido)pyridazin-3-yl)piperazine-1-carboxylate

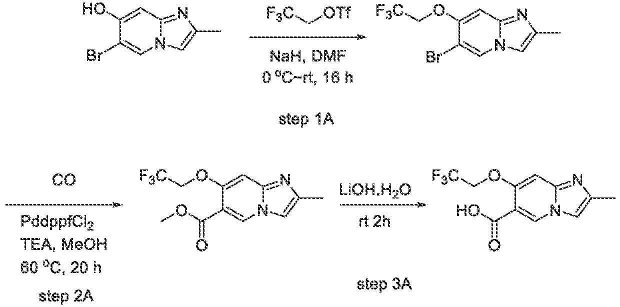

### Step 1A: preparation of 6 bromo -2-methyl-7-(2,2,2-trifluoroethoxy) imidazo [1,2-a]pyridine

To a mixture of 6-bromo-2-methylimidazo[1,2-a]pyridin-7-ol (1.5 g, 6.6 mmol) in DMF (20 mL) was added NaH (0.48 g, 13.2 mmol) at 0 °C and the mixture was stirred at 0 °C for 2 h. 2,2,2-trifluoroethyl trifluoromethanesulfonate(2.23 g, 9.9 mmol) was added to the mixture and stirred at rt for another 16 h. The mixture was diluted with water (20 mL) and extracted with EA (3 x 30 mL). The organic layer washed with brine (3 x 40 mL) and dried over anhydrous Na₂SO₄. The filtrate was concentrated in vacuo. The residue was purified by column chromatography (MeOH/DCM =1:10) to give title product (1.1 g, 53.8%) as a white solid. ESI-MS (M+H)⁺: 310.9. ¹H NMR (400 MHz, DMSO-d₆) δ 8.85 (s, 1H), 7.50 (s, 1H), 7.19 (s, 1H), 4.94 - 4.90 (m, 2H), 2.28 (s, 3H).

### Step 2A: preparation of methyl 2-methyl -7-(2,2,2-trifluoroethoxy) imidazo [1,2-a] pyridine-6-carboxylate

To a mixture of 6-bromo-2-methyl-7-(2,2,2-trifluoroethoxy)imidazo[1,2-a]pyridine (300 mg, 1 mmol) in MeOH (30 mL) were added Pd(dppf)Cl₂ (143 mg, 0.2 mmol) and TEA (2.7 mL, 0.2 mmol). The mixture was stirred at 60 °C for 20 h under CO atmosphere. The mixture was filtered and the filtrate was concentrated in vacuo to give title product (crude 240 mg, 84.6%) as a grey solid. ESI-MS (M+H)⁺: 289.1.

### Step 3A: preparation of methyl 2-methyl-7-(1,2,2-trifluoroethoxy)imidazo[1,2-a]pyridine-6-carboxylic acid

To a solution of methyl 2-methyl-7-(2,2,2-trifluoroethoxy)imidazo[1,2-a]pyridine-6-carboxylate (300 mg, 1 mmol) in THF/H₂O (8 mL/4 mL) was added LiOH ( 100 mg, 4.1 mmol). The mixture was stirred at rt for 2 h. The mixture was adjusted to pH=5 with 1M HCl. The aqueous layer was extracted with EA (3 x 50mL). The organic layer was concentrated in vacuo, the residue was purified by C18 flash (0.1% FA in water / CH₃CN) to give title product (174 mg, 57.8%) as a yellow solid. ESI-MS (M+H)⁺: 275.1.

### Step 1: Preparation of tert-butyl (S)-2-methyl-4-(6-(2-methyl-7-(2,2,2-trifluoroethoxy)imidazo[1, 2-a]pyridine-6-carboxamido)pyridazin-3-yl)piperazine-1-carboxylate

To a solution of 2-methyl-7-(2,2,2-trifluoroethoxy)imidazo[1,2-a]pyridine-6-carboxylic acid (120 mg, 0.44 mmol) and tert-butyl (S)-4-(6-aminopyridazin-3-yl)-2-methylpiperazine-1-carboxylate (107 mg, 0.36 mmol) in DMF (10 mL) were added HATU (208 mg, 0.54 mmol) and DIEA (0.25mL, 1.44 mmol). The mixtre was stirred at rt for 1 h. The reaction mixture was diluted with water (20 mL) and extracted with EA (3 x 50 mL). The organic layer washed with brine (3 x 50 mL), dried over anhydrous Na₂SO₄, filtered and the filtrated was concentrated in vacuo. The residue was purified by prep-HPLC (0.1% FA in water / CH₃CN) to give title product (90 mg, 37.3%) as a gray solid. ESI-MS (M+H)+: 550.3. ¹H NMR (400 MHz, DMSO-*d*₆) δ 10.60 (s, 1H), 8.95 (s, 1H), 8.14 (s, 1H), 7.67 (s, 1H), 7.41 (d, J = 9.9 Hz, 1H), 7.17 (s, 1H), 4.97 (dd, J = 17.4, 8.7 Hz, 2H), 4.19 (d, J = 12.4 Hz, 2H), 4.08 (d, J = 13.1 Hz, 1H), 3.82 (d, J = 13.3 Hz, 1H), 3.17 (dd, J = 13.0, 3.8 Hz, 2H), 2.97 - 2.90 (m, 1H), 2.31 (s, 3H), 1.43 (s, 9H), 1.12 (d, J = 6.7 Hz, 3H).

### Step 2: Preparation of (S)-2-methyl-N-(6-(3-methylpiperazin-1-yl)pyridadn-3-yl)-7-(2,2, 2-trifluoroethoxy)imidazo[1,2-a]pyridine-6-carboxamide

To a mixture of tert-butyl (S)-2-methyl-4-(6-(2-methyl-7-(2,2,2-trifluoroethoxy)imidazo[1,2-a]pyridine-6-carboxamido)pyridazin-3-yl)piperazine-1-carboxylate (70 mg, 0.13 mmol) in EA (5 mL) was added EA/HCl (5 mL) The mixture was stirred at rt for 2 h. The reaction mixture was concentrated in vacuo. The residue was purified by pre-HPLC (0.1% NH₃.H₂O in water / CH₃CN) to give title product (20 mg, 34.9%) as a white solid. ESI-MS (M+H)+: 450.2. ¹H NMR (400 MHz, MeOD-*d₄*) δ 8.95 (s, 1H), 8.29 (d, J = 9.9 Hz, 1H), 7.59 (s, 1H), 7.37 (d, J = 9.9 Hz, 1H), 7.07 (s, 1H), 4.90 (d, J = 8.2 Hz, 2H), 4.17 (dd, J = 15.1, 6.6 Hz, 2H), 3.10 (d, J = 11.2 Hz, 1H), 2.92 (dd, J = 13.2, 2.9 Hz, 3H), 2.62 (dd, J = 12.8, 10.6 Hz, 1H), 2.38 (s, 3H), 1.17 (d, J = 6.4 Hz, 3H).

### Example 62. Synthesis of (S)-7-ethoxy-2-methyl-N-(6-(3-methylpiperazin-1-yl)pyridazin-3-yl)imidazo[1,2-a]pyridine-6-carboxamide HCl salt (Compound 217)

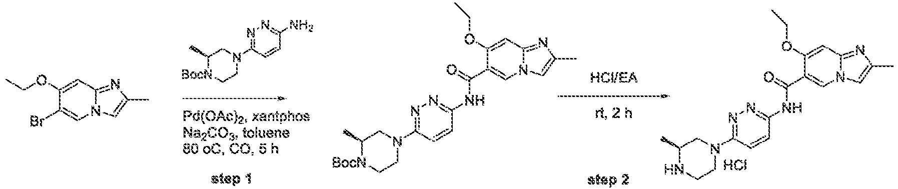

### Step 1: Preparation of tert-butyl (S)-4-(6-(7-ethoxy-2-methylimidazo[1,2-a]pyridine-6-carboxamido)pyridazin-3-yl)-2-methylpiperazine-1-carboxylate

To a solution of 6-bromo-7-ethoxy-2-methylimidazo[1,2-a]pyridine (2 g, 7.87 mmoL) in toluene (40 mL) were added tert-butyl (S)-4-(6-aminopyridazin-3-yl)-2-methylpiperazine-1-carboxylate (2.31 g, 7.87 mmoL), Pd(OAc)₂ (176 mg, 0.787 mmoL), xantphos (455 mg, 0.787 mmoL) and Na₂CO₃ (1.31 g, 15.78 mmoL). The resulting solution was stirred at 80 °C for 5 h under the atmosphere of CO. The resulting solution was concentrated under vacuum. The residue was purified by C18 column (0.1% _{NH3}.H₂0 in water / CH₃CN) to give title product (2.1 g, Y: 53.8%) as an off-white solid. ESI-MS (M+H)+: 496.2. ¹H NMR (400 MHz, CDCl₃) δ 10.48 (s, 1H), 9.11 (s, 1H), 8.36 (d, J = 9.8 Hz, 1H), 7.66 (s, 1H), 7.38 (s, 1H), 7.01 (d, J = 9.9 Hz, 1H), 4.50 (q, J = 7.0 Hz, 2H), 4.38 - 4.37 (m, 1H), 4.17 - 4.16 (m, 1H), 4.07 - 3.89 (m, 2H), 3.44 - 3.21 (m, 2H), 3.09 - 3.08 (m, 1H), 2.59 (s, 3H), 1.74 (t, J = 7.0 Hz, 3H), 1.49 (s, 9H), 1.22 (d, J = 6.7 Hz, 3H).

### Step 2: Preparation of (S)-7-ethoxy-2-methyl-N-(6-(3-methylpiperazin-1-yl)pyridazin-3-yl)imidazo[1,2-a]pyridine-6-carboxamide HCl salt

To a solution of in tert-butyl (S)-4-(6-(7-ethoxy-2-methylimidazo[1,2-a]pyridine-6-carboxamido)pyridazin-3-yl)-2-methylpiperazine-1-carboxylate (2.1 g, 4.24 mmoL) in EA (10 mL) was added 3M HCl/ EA (10 mL), the resulting solution was stirred for 2 h at RT. The precipitate was filtered and dried to give title product (650 mg, Y: 35.7% ) as a yellow solid. ESI-MS (M+H)+: 396.1. ¹H NMR (400 MHz, MeOD-d₄) δ 9.18 (s, 1H), 8.53 (d, J = 10.1 Hz, 1H), 8.16 (d, J = 10.2 Hz, 1H), 7.86 (s, 1H), 7.38 (s, 1H), 4.62 - 4.30 (m, 4H), 3.76 - 3.55 (m, 3H), 3.47 - 3.32 (m, 2H), 2.52 (s, 3H), 1.57 (t, J = 7.0 Hz, 3H), 1.46 (d, J = 6.5 Hz, 3H).

### Example 63. Synthesis of 2-methyl-N-(6-((S)-3-methylpiperazin-1-yl)pyridazin-3-yl)-7-((tetrahydro-2H-pyran-3-yl)oxy)imidazo[1,2-a]pyridine-6-carboxamide (Compound 218)

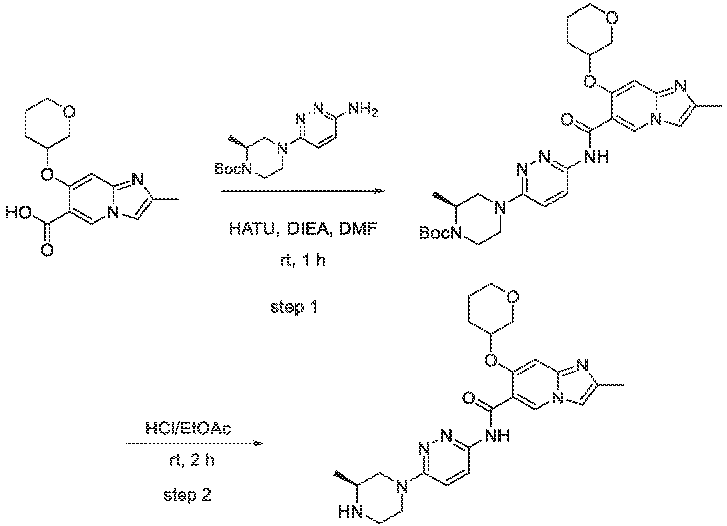

### Preparation of 2-methyl-7-((tetrahydro-2H-pyran-3-yl)oxy)imidazo[1,2-a]pyridine-6-carboxylic acid

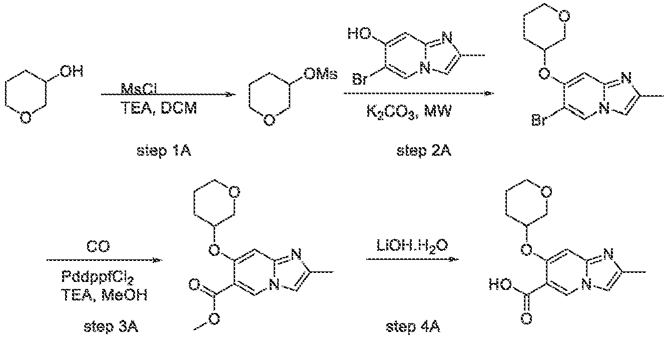

### Step 1A: preparation of tetrahydro-2H-pyran-3-yl methanesulfonate

To a solution of tetrahydro-2H-pyran-3-ol (150 mg, 1.46 mmol) and TEA (445 mg, 4.4 mmol) in DCM (5 mL) was added MsCl (200 mg, 1.2 mmol), the reaction mixture was stirred for 2 h at 0 °C. After cooling to rt and diluting with water, the mixture was extracted with EA. The combined organics were washed with brine and water, dried over Na₂SO₄ and concentrated to give desired product as colorless oil (250 mg, crude), which was used to next step without further purification. ¹H NMR (400 MHz, CDCl₃) δ 4.77 - 4.68 (m, 1H), 3.89 - 3.80 (m, 1H), 3.73 - 3.59 (m, 3H), 3.04 (s, 3H), 2.13 - 2.02 (m, 1H), 1.99 - 1.86 (m, 2H), 1.65 - 1.55 (m, 1H).

### Step 2A: preparation of 6-bromo-2-methyl-7-((tetrahydro-2H-pyran-3-yl)oxy)imidazo[1,2-a]pyridine

A solution of tetrahydro-2H-pyran-3-yl methanesulfonate (1.3 g, 7.2 mmol), 6-bromo-2-methylimidazo[1,2-a]pyridin-7-ol (1.1 g, 5.0 mmol) and K₂CO₃ (2.6 g, 20.0 mmol) in DMF (5 mL) was stirred for 1 h at 75°C in a Sealed tube under microwave. After cooling to rt and diluting with water (80 mL), the mixture was extracted with EA (100 mL x2). The combined organics were washed with brine and water, dried over Na₂SO₄, concentrated. The crude was purified by prep-HPLC (0.05% FA in water/ CH₃CN) to give title product (200 mg, yield: 15%) as a yellow solid. ¹H NMR (400 MHz, CDCl₃) δ 8.16 (s, 1H), 7.14 (s, 1H), 6.85 (s, 1H), 4.44 - 4.32 (m, 1H), 4.00 - 3.92 (m, 1H), 3.79 - 3.61 (m, 3H), 2.38 (s, 3H), 2.19 - 2.07 (m, 1H), 2.01 - 1.85 (m, 2H), 1.73 - 1.56 (m, 1H).

### Step 3A: preparation of methyl 2-methyl-7-((tetrahydro-2H-pyran-3-yl)oxy)imidazo[1,2-a]pyridine-6-carboxylate

To a mixture of 6-bromo-2-methyl-7-((tetrahydro-2H-pyran-3-yl)oxy)imidazo[1,2-a]pyridine (155 mg, 0.5 mmol) in MeOH (5 mL) were added TEA (510 mg, 5.0 mmol) and Pd(dppf)Cl₂ (65 mg, 0.1 mmol). The resulting mixture was stirred overnight at 60 °C under CO (balloon). The mixture was allowed to cooling down to room temperature and concentrated. The residue was purified by silica gel column chromatography (PE:EA=3:1) to give title product (100 mg, 69%) as a brown solid. ESI-MS (M+H)⁺: 291.0.

### Step 4A: preparation of 2-methyl-7-((tetrahydro-2H-pyran-3-yl)oxy)imidazo[1,2-a]pyridine-6-carboxylic acid

To a mixture of methyl 2-methyl-7-((tetrahydro-2H-pyran-3-yl)oxy)imidazo[1,2-a]pyridine-6-carboxylate (150 mg, 0.5 mmol) in THF/water (5 mL, 2:1) was added LiOH (48 mg, 2.0 mmol). The mixture was stirred at rt for 2 h. After concentration, the residue was adjusted to pH = 5 with 1M HCl. The cured was purified by pre-HPLC to give title product (32 mg, Y: 23.1%) as a colorless oil. ESI-MS (M+H)⁺: 277.0.

### Step 1: Preparation of tert-butyl (2S)-2-methyl-4-(6-(2-methyl-7-((tetrahydro-2H-pyran-3-yl)oxy)imidazo[1,2-a]pyridine-6-carboxamido)pyridazin-3-yl)piperazine-1-carboxylate

To a mixture of 2-methyl-7-((tetrahydro-2H-pyran-3-yl)oxy)imidazo[1,2-a]pyridine-6-carboxylic acid (30 mg, 0.11 mmol) in DMF (2 mL) were added tert-butyl (S)-4-(6-aminopyridazin-3-yl)-2-methylpiperazine-1-carboxylate (40 mg, 0.13 mmol), DIEA (30 mg, 0.29 mmol) and HATU (50 mg, 0.13 mmol). The mixture was stirred at rt for 1 h. After diluting with water, the mixture was filtered and the cake was purified by silica gel column chromatography (DCM:MeOH=20:1) to give title product (30 mg, 50%) as a white solid. ESI-MS (M+H)+: 552.4.

### Step 2: Preparation of 2-methyl-N-(6-((S)-3-methylpiperazin-1-yl)pyridazin-3-yl)-7-((tetrahydro-2H-pyran-3-yl)oxy)imidazo[1,2-a]pyridine-6-carboxamide

A solution of tert-butyl (2S)-2-methyl-4-(6-(2-methyl-7-((tetrahydro-2H-pyran-3-yl)oxy)imidazo[1,2-a]pyridine-6-carboxamido)pyridazin-3-yl)piperazine-1-carboxylate (30 mg, 0.05 mmol) in HCl/EA (1.0M, 3 mL) was stirred at rt for 2 h. The mixture was concentrated and the residue was purified by prep-HPLC (0.05% NH3.H2O in water / MeCN) to give title product (24 mg, Y: 98%) as a yellow solid. ESI-MS (M+H)+: 452.1. 1H NMR (400 MHz, MeOD-d₄) δ 9.31 (s, 1H), 8.45 (br s, 1H), 8.11 (br s, 1H), 7.90 (s, 1H), 7.51 (s, 1H), 5.07 - 4.99 (m, 1H), 4.54 - 4.42 (m, 2H), 4.13 - 4.02 (m, 1H), 3.98 - 3.87 (m, 2H), 3.76 - 3.55 (m, 4H), 3.44 - 3.34 (m, 2H), 2.53 (s, 3H), 2.23 - 2.09 (m, 2H), 2.06 - 1.97 (m, 1H), 1.66 - 1.57 (m, 1H), 1.46 (s, 3H).

### Example 64. Synthesis of 7-cyclobutoxy-N-(6-(3,3-dimethylpiperazin-1-yl)pyridazin-3-yl)-2-methylimidazo[1,2-a]pyridine-6-carboxamide (Compound 219)

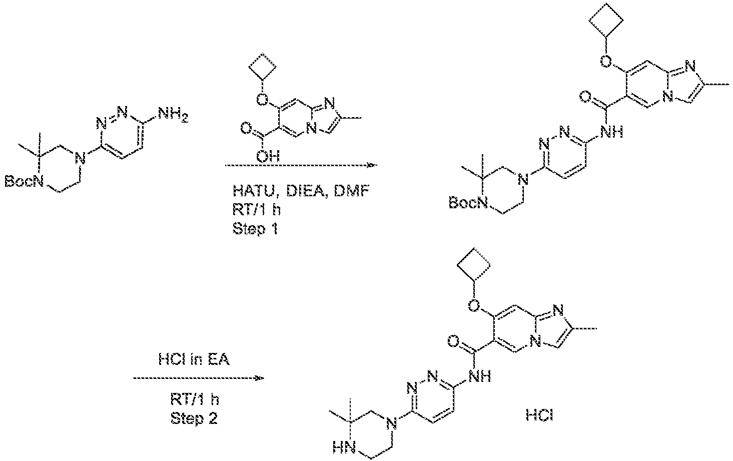

### Step 1: Preparation of tert-butyl 4-(6-(7-cyclobutoxy-2-methylimidazo[1,2-a]pyridine-6-carboxamido)pyridazin-3-yl)-2,2-dimethylpiperazine-1-carboxylate

To a mixture of tert-butyl 4-(6-aminopyridazin-3-yl)-2,2-dimethylpiperazine-1-carboxylate (35 mg, 0.114 mmol) and 7-cyclobutoxy-2-methylimidazo[1,2-a]pyridine-6-carboxylic acid (42 mg, 0.171 mmol) in DMF (3 mL) were added HATU (65 mg, 0.171 mmol) and DIEA (44 mg, 0.342 mmol). The mixture was stirred at rt for 1 hour. After diluting with water, the precipitate was filtered and dried to give title product (30 mg, 49.2%) as a white solid. ESI-MS (M+H)+: 536.2. ¹H NMR (400 MHz, CDCl₃) δ 10.67 (s, 1H), 8.97 (s, 1H), 8.43 (d, J = 9.8 Hz, 1H), 7.29 (s, 1H), 6.85 (d, J = 9.8 Hz, 1H), 6.76 (s, 1H), 4.93 - 4.86 (m, 1H), 3.94 - 3.86 (m, 4H), 3.61 - 3.54 (m, 2H), 2.69 - 2.59 (m, 2H), 2.46 - 2.41 (m, 4H), 2.06 - 1.78 (m, 3H), 1.50 (s, 9H), 1.43 (s, 6H).

### Step 2: Preparation of 7-cyclobutoxy-N-(6-(3,3-dimethylpiperazin-1-yl)pyridazin-3-yl)-2-methylimidazo[1,2-a]pyridine-6-carboxamide

To a mixture of tert-butyl 4-(6-(7-cyclobutoxy-2-methylimidazo[1,2-a]pyridine-6-carboxamido)pyridazin-3-yl)-2,2-dimethylpiperazine-1-carboxylate (25 mg, 0.047 mmol) in EA (2 mL) was added EA/HCl (2 mL, 3 M). The mixture was stirred at rt for 1 h. After concentration, the residue was lyophilized to give title product (18.18 mg, 82.6%) as a white solid. ESI-MS (M+H)+: 436.1. ¹H NMR (400 MHz, MeOD-*d₄*) δ 9.21 (d, J = 13.7 Hz, 1H), 8.47 (d, J = 10.0 Hz, 1H), 8.01 (d, J = 10.1 Hz, 1H), 7.85 (s, 1H), 7.16 (s, 1H), 5.20 - 5.06 (m, 1H), 4.05 - 3.97 (m, 2H), 3.87 (s, 2H), 3.53 - 3.45 (m, 2H), 2.64 - 2.61 (m, 2H), 2.51 (d, J = 1.0 Hz, 3H), 2.43 - 2.31 (m, 2H), 2.03 - 1.84 (m, 2H), 1.51 (s, 6H).

### Example 65. Synthesis of N-(6-(3,3-dimethylpiperazin-1-yl)pyridazin-3-yl)-6-ethoxy-2-methyl-2H-indazole-5-carboxamide hydrochloride (Compound 220)

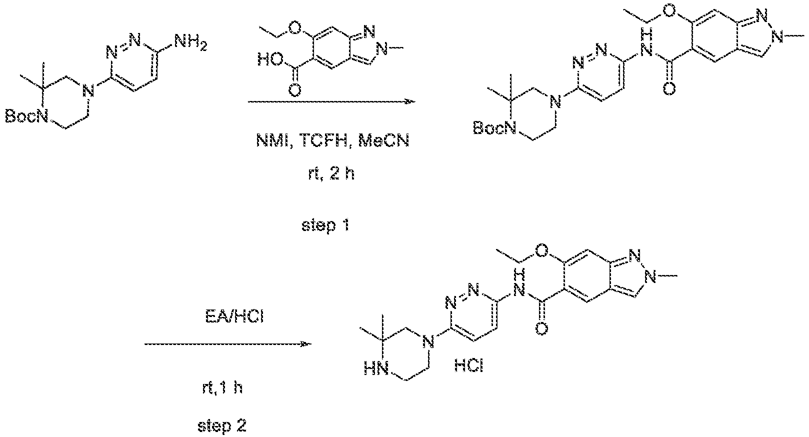

### Step 1: Preparation of tert-butyl 4-(6-(6-ethoxy-2-methyl-2H-indazole-5-carboxamido)pyridazin-3-yl)-2,2-dimethylpiperazine-1-carboxylate

To a mixture of tert-butyl 4-(6-aminopyridazin-3-yl)-2,2-dimethylpiperazine-1-carboxylate (42 mg, 0.136 mmol) and 6-ethoxy-2-methyl-2H-indazole-5-carboxylic (30 mg, 0.136 mmol) in MeCN (5 mL) were added NMI (33.7 mg, 0.411 mmol) and TCFH (59 mg, 0.20 mmol). The mixture was stirred at rt for 2 hours. The precipitate was filtered and dried to give title product (50 mg, 72%) as a white solid. ESI-MS (M+H)+: 510.3.

### Step 2: Preparation of N-(6-(3,3-dimethylpiperazin-1-yl)pyridazin-3-yl)-6-ethoxy-2-methyl-2H-indazole-5-carboxamide hydrochloride

To a mixture of 4- (6-(6-ethoxy-2-methyl-2H-indazole-5-carboxamido) pyridazin-3-yl)-2,2-dimethylpiperazine -1-carboxylate (50 mg, 0.1 mmol) in EA (5 mL) was added EA/HCl (5 mL, 3 mol). The mixture was stirred at rt for 1 h. After concentration, the residue was lyophilized to give title product (24 mg, 58%) as a white solid. ESI-MS (M+H)+: 410.1. ¹H NMR (400 MHz, MeOD-*d₄*) δ 8.49 (s, 1H), 8.44 (s, 1H), 8.18 - 8.16 (m, 2H), 7.15 (s, 1H), 4.32 (q, J = 7.0 Hz, 2H), 4.22 (s, 3H), 4.02 - 3.98 (m, 2H), 3.86 (s, 2H), 3.50 - 3.46 (m, 2H), 1.57 (t, J = 7.0 Hz, 3H), 1.50 (s, 6H).

### Exaple 66. Synthesis of N-(6-(3,3-dimethylpiperazin-1-yl)pyridazin-3-yl)-7-ethoxy-2-methylimidazo[1,2-a]pyridine-6-carboxamide hydrochloride (Compound 221)

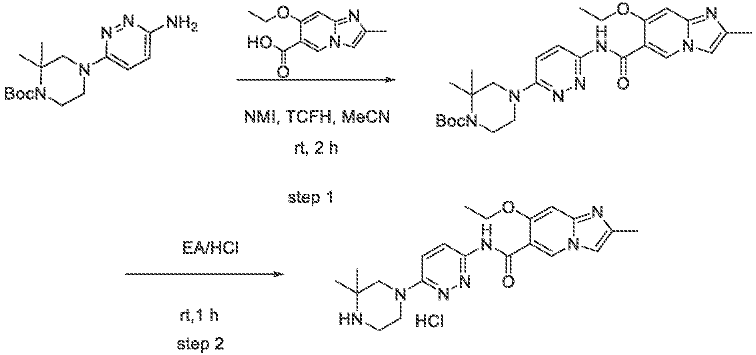

### Step 1: Preparation of tert-butyl 4-(6-(7-ethoxy-2-methylimidazo[1,2-a]pyridine-6-carboxamido)pyridazin-3-yl)-2, 2-dimethylpiperazine-1-carboxylate

To a mixture of tert-butyl 4-(6-aminopyridazin-3-yl)-2,2-dimethylpiperazine-1-carboxylate (42 mg, 0.136 mmol) and 7-ethoxy-2-methylimidazo[1,2-a]pyridine-6-carboxylic acid (30 mg, 0.136 mmol) in MeCN (5 mL) were added NMI (33.7 mg, 0.411 mmol) and TCFH (59 mg, 0.20 mmol). The mixture was stirred at rt for 2 hours. The precipitate was filtered to give title product (50 mg, 72%) as a white solid. ESI-MS (M+H)+: 510.3.

### Step 2: Preparation of N-(6-(3,3-dimethylpiperazin-1-yl)pyridazin-3-yl)-7-ethoxy-2-methylimidazo[1,2-a]pyridine-6-carboxamide hydrochloride

To a mixture of tert-butyl 4-(6-(7-ethoxy-2-methylimidazo[1,2-a]pyridine-6-carboxamido)pyridazin-3-yl)-2,2-dimethylpiperazine-1-carboxylate (50 mg, 0.1 mmol) in EA (5 mL) was added EA/HCl (5 mL, 3 mol). The mixture was stirred at rt for 1 h. After concentration, the residue was purified by pre-HPLC to give title product (20 mg, 55%) as a white solid. ESI-MS (M+H)+: 410.1. ¹H NMR (400 MHz, MeOD-*d₄*) δ 9.19 (s, 1H), 8.48 (br s, 1H), 8.10 (br s, 1H), 7.87 (s, 1H), 7.38 (s, 1H), 4.46 (br s, 2H), 4.04 (br.s, 2H), 3.90 (br.s, 2H), 3.51 (br.s, 2H), 2.52 (s, 3H), 1.59 (br.s, 3H), 1.52 (s, 6H).

### Example 67. Synthesis of (S)-6-ethoxy-2-methyl-N-(5-methyl-6-(3-methylpiperazin-1-yl) pyridazin-3-yl)-2H-indazole-5-carboxamide hydrochloride (Compound 222)

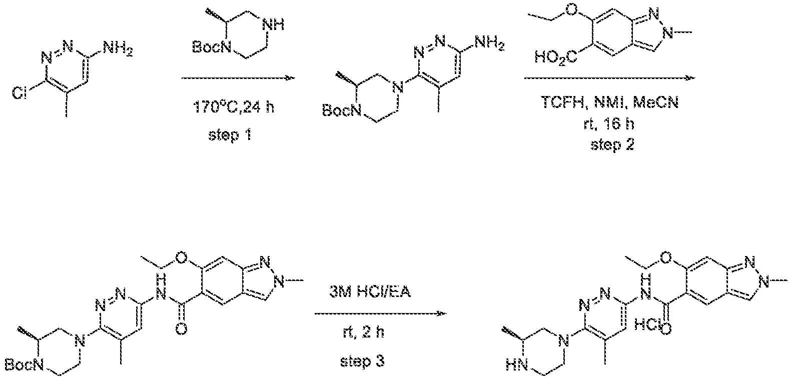

### Step 1: Preparation of tert-butyl (S)-4-(6-amino-4-methylpyridazin-3-yl)-2-methylpiperazine-1-carboxylate

A solution of 6-chloro-5-methylpyridazin-3-amine (1.2 g, 8.4 mmol) in tert-butyl (S)-2-methylpiperazine-1-carboxylate (13.44 g, 67.2 mmol) was stirred at 170 °C for 24 h. After cooling to rt, the mixture was concentrated and purified by silica gel column chromatography eluted with (EA/PE=1:5) to give title compound (1 g, 38%) as a yellow oil. ESI-MS (M+H) +: 308.3.

### Step 2: Preparation of tert-butyl (S)-4-(6-(6-ethoxy-2-methyl-2H-indazole-S-carboxamido)-4-methylpyridazin-3-yl)-2-methylpiperazine-1-carboxylate

To a solution of tert-butyl (S)-4-(6-amino-4-methylpyridazin-3-yl)-2-methylpiperazine-1-carboxylate (50 mg, 0.163 mmol), 6-ethoxy-2-methyl-2H-indazole-5-carboxylic acid (36 mg, 0.163 mmol) in MeCN (3 mL) were added NMI (40.1 mg, 0.489 mmol) and TCFH (68.5 mg, 0.2445 mmol), the mixture was stirred at RT for 16 h. The precipitate was filtered to give crude title compound (20 mg, 24.3 %) as a white solid. ESI-MS (M+H) +: 510.2.

### Step 3: Preparation of (S)-6-ethoxy-2-methyl-N-(5-methyl-6-(3-methylpiperazin-1 -yl) pyridazin-3-yl)-2H-indazole-5-carboxamide hydrochloride.

To a solution of tert-butyl (S)-4-(6-(6-ethoxy-2-methyl-2H-indazole-5-carboxamido)-4-methylpyridazin-3-yl)-2-methylpiperazine-1-carboxylate (20 mg, 0.04 mmol) in EtOAc (1 mL) was added 3M HCl/EtOAc (1 mL) at room temperature. The reaction mixture was stirred for 2 h. The precipitate was filtered and dried to give to give title compound (5 mg, 31 %) as a white solid. ESI-MS (M+H) +: 410.3. ¹H NMR (400 MHz, MeOD-d₄) δ 8.53 (s, 1H), 8.49 (s, 1H), 7.92 (s, 1H), 7.18 (s, 1H), 4.35 (q. J = 6.9 Hz, 2H), 4.25 (s, 3H), 3.90 - 3.88 (m, 2H), 3.66 - 3.65 (m, 1H), 3.54 - 3.53 (m, 1H), 3.44 - 3.43 (m, 2H), 3.26 - 3.09 (m, 1H), 2.63 (s, 3H), 1.56 (t, J = 6.9 Hz, 3H), 1.43 (d, J = 6.3 Hz, 3H).

### Example 68. Synthesis of 8-ethyl-2-methyl-N-{6-[(3S)-3-methylpiperazin-1-yl]pyridazin-3-yl}-7-oxo-7H,8H-imidazo[1,2-a]pyrimidine-6-carboxamide (Compound 223)

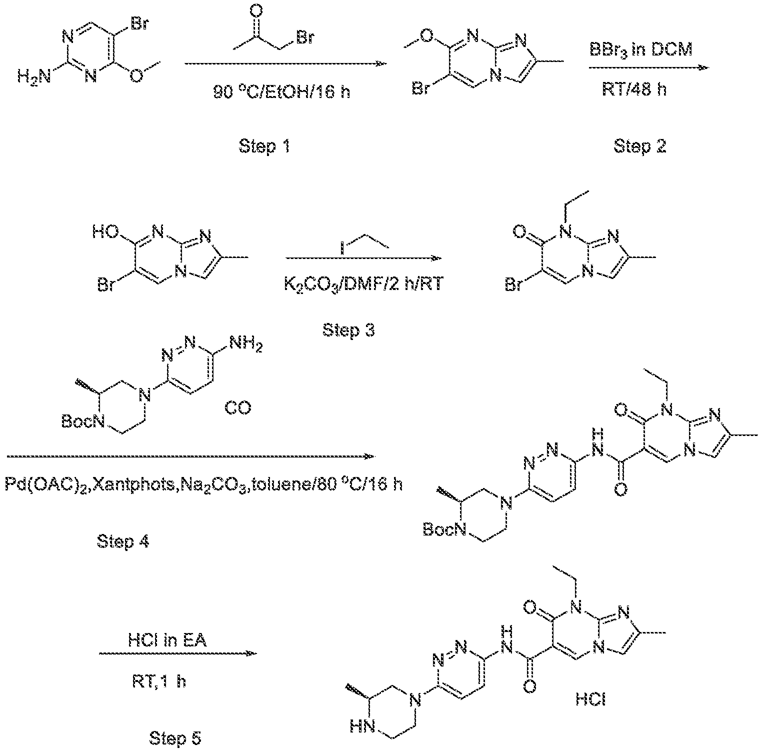

### Step 1 : Preparation of 6-bromo-7-methoxy-2-methylimidazo[1,2-a]pyrimidine.

To a solution of 5-bromo-4-methoxypyrimidin-2-amine (4.5 g, 22.057 mmol) in EtOH (100 mL) was added 1-bromopropan-2-one (6 g, 44.113 mmol). The mixture was stirred at 90 °C for 16 h. After cooling to RT, the mixture was concentrated under reduced pressure, then the residue was washed by EA and filtered. The residue was dissolved in NaOH (2 mol/L, 100 mL) and stirred at RT for 2 h. Then the solid was collected by filtration to give the tide compound (1.1 g, 20%) as a white solid. ESI-MS (M+H) ⁺: 242.0/243.9. ¹H NMR (400 MHz, CDCl₃) δ 8.32 (s, 1H), 7.04 (s, 1H), 4.10 (s, 3H), 2.39 (s, 3H).

### Step 2 : Preparation of 6-bromo-2-methylimidazo[1,2-a]pyrimidin-7-ol.

To a solution of 6-bromo-7-methoxy-2-methylimidazo[1,2-a]pyrimidine (1 g, 4.15 mmol) in DCM (20 mL) was added BBr₃ (1 mol/L in DCM, 50 mL) at ice bath temperature. The mixture was stirred at RT for 48 h.. After quenching with MeOH (30 mL), the mixture was basified with saturated aqueous sodium bicarbonate solution and extracted with DCM (50 mLx3). The aqueous phase was concentrated in vacuo and the residue was purified by silica gel column chromatography (DCM:MeOH=10:1) to give title product (307 mg, 32 %) as a brown solid. ESI-MS (M+H)⁺:227.9. ¹H NMR (400 MHz, DMSO-d₆) δ 8.68 (s, 1H), 6.99 (d, *J=* 1.2 Hz, 1H), 2.15 (d, *J* = 1.1 Hz, 3H).

### Step 3: Preparation of 6-bromo-8-ethyl-2-methylimidazo[1,2-a]pyrimidin-7(8H)-one.

To a solution of 6-bromo-2-methylimidazo[1,2-a]pyrimidin-7-ol (150 mg, 0.658 mmol) in DMF (10 mL) were added iodoethane (205 mg, 1.316 mmol) and K₂CO₃ (272 mg, 1.974 mmol). The mixture was was stirred at rt for 2 h. After diluting with water (30 mL), the mixture was extracted with EA (50 mLx3). The organic layer was washed with brine, dried over sodium sulfate, filtered and concentrated in vacuo to give the title product (145 mg, 86 %) as a white solid . ESI-MS (M+H)⁺: 257.9. ¹H NMR (400 MHz, DMSO-d₆) δ 8.96 (s, 1H), 7.18 (d, *J=* 1.1 Hz, 1H), 4.21 (q, *J =* 7.1 Hz, 2H), 2.24 (d, *J* = 1.0 Hz, 3H), 1.29 (t, *J* = 7.1 Hz, 3H).

### Step 4: Preparation of tert-butyl (S)-4-(6-(8-ethyl-2-methyl-7-oxo-7,8-dihydroimidazo[1,2-a]pyrimidine-6-carboxamido)pyridazin-3-yl)-2-methylpiperazine-1-carboxylate.

To a solution of 6-bromo-8-ethyl-2-methylimidazo[1,2-a]pyrimidin-7(8H)-one (105 mg, 0.412 mmol), tert-butyl (S)-4-(6-aminopyridazin-3-yl)-2-methylpiperazine-1-carboxylate (121 mg, 0.412 mmol) and Na₂CO₃ (66 mg, 0.618 mmol) in toluene (15 mL) were added Xantphots (9 mg, 0.016 mmol) and Pd(OAc)z (4 mg, 0.016 mmol). The mixture was charged with CO three times and stirred at 80 °C under CO for 16 h. The mixture was concentrated under reduced pressure to give title product (100 mg, crude) as a dark solid, which was used to next step without further purification. ESI-MS (M+H)⁺: 497.2.

### Step 5 : Preparation of (S)-8-ethyl-2-methyl-N-(6-(3-methylpiperazin-1-yl)pyridazin-3-yl)-7-oxo-7,8-dihydroimidazo[1,2-a]pyrimidine-6-carboxamide hydrochloride.

To a solution of tert-butyl (S)-4-(6-(8-ethyl-2-methyl-7-oxo-7,8-dihydroimidazo[1,2-a]pyrimidine-6-carboxamido)pyridazin-3-yl)-2-methylpiperazine-1-carboxylate (crude 100 mg, 0.202 mmol) in EA (4 mL) was added EA/HCl (4 mL, 3 mol/L). The mixture was stirred at rt for 1 h. The mixture was concentrated in vacuo and the residue was purified by prep-HPLC (0.05 % HCl in water / CH₃CN) to give title compound (33 mg, yield: 38% for two steps) as a yellow solid. ESI-MS (M+H)⁺:397.1. ¹H NMR (400 MHz, MeOD-*d₄*) δ 9.29 (s, 1H), 8.51 (d, *J* = 10.0 Hz, 1H), 7.89 (d, *J* = 10.0 Hz, 1H), 7.36 (d, *J* = 1.1 Hz, 1H), 4.45 (d, *J* = 14.4 Hz, 2H), 4.39 - 4.34 (m, 2H), 3.60 - 3.53 (m, 2H), 3.52 - 3.40 (m, 2H), 3.25 - 3.21 (m, 1H), 2.34 (s, 3H), 1.44 - 1.40 (m, 6H).

### Example 69. Synthesis of (S)-7-ethoxy-2-methyl-N-(6-(3-methylpiperazin-1-yl)pyridazin-3-yl)imidazo[1,2-a]pyrimidine-6-carboxamide hydrochloride (Compound 224)

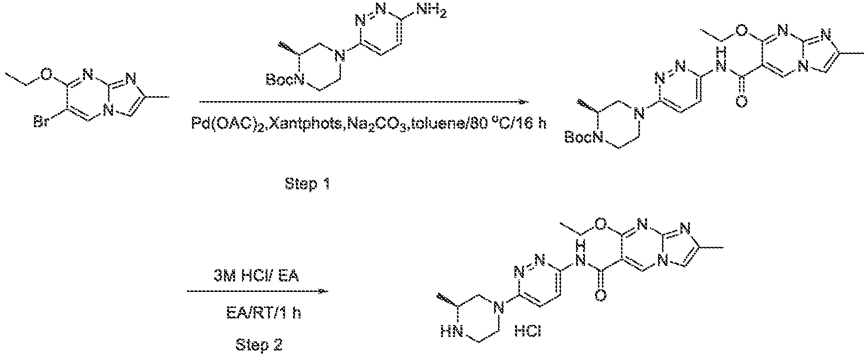

### Preparation of 6-bromo-7-ethoxy-2-methylimidazo[1,2-a]pyrimidine

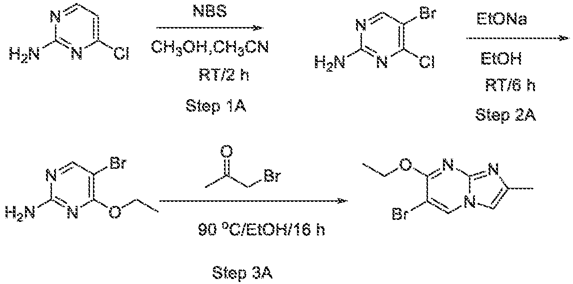

### Step 1A: preparation of 5-bromo-4-chloropyrimidin-2-amine

To a solution of 4-chloropyrimidin-2-amine (15 g, 115.385 mmol) in methanol (210 mL) and acetonitrile (150 mL), N-bromosuccinimide (20.4 g, 115.385 mmol) was added. The mixture was stirred at rt for 2 h then diethyl ether (300 mL) was added and stirred for 1 h at 5 °C. Then the solid was collected by filtration and dried to give the title compound (18.4 g, Y: 77%) as a white solid. ESI-MS (M+H) ⁺: 209.9. ¹H NMR (400 MHz, DMSO-d₆) δ 8.41 (s, 1H), 7.34 (s, 2H).

### Step 2A: preparation of 5-bromo-4-ethoxypyrimidin-2-amine

A solution of 5-bromo-4-chloropyrimidin-2-amine (10 g, 48.309 mmol) and sodium ethoxide (20 % in ethanol, 6.57 g, 96.61 mmol) in ethanol (200 mL) stirred at RT for 6 h. Then the mixture was concentrated in vacuo, the residue was diluted with water (100 mL) and extracted with DCM (200 mLx3). The organic layer was concentrated in vacuo to give title compound (11.4 g, yield: 95 %). ESI-MS (M+H)⁺:218.0. ¹H NMR (400 MHz, CDCl₃) δ 8.10 (s, 1H), 4.98 (s, 2H), 4.40 (q, *J* = 7.1 Hz, 2H), 1.41 (t, *J =* 7.1 Hz, 3H).

### Step 3A: preparation of 6-bromo-7-ethoxy-2-methylimidazo[1,2-a]pyrimidine

To a solution of 5-bromo-4-ethoxypyrimidin-2-amine (2 g, 9.217 mmol) in EtOH (40 mL) was added 1-bromopropan-2-one (2.5 g, 18.433 mmol). The mixture was was stirred at 90 °C for 16 h. After cooling to RT, the mixture was concentrated under reduced pressure, then the residue was washed by EA and filtered. The residue was dissolved in NaOH (2 mol/L, 20mL) and stirred at RT for 2 h. Then the solution was extracted with EA (40 mLx3) and the organic layer was washed with saturated NaCl solution and concentrated in vacuo to get crude compound. The crude was purified by silica gel column (PE: EA=4:1∼1:1) to give the title compound (500 mg, Y: 17%) as a white solid. ESI-MS (M+H) ⁺: 257.9. ¹H NMR (400 MHz, CDCl₃) δ 8.31 (s, 1H), 7.02 (d, *J =* 0.9 Hz, 1H), 4.55 (q, *J* = 7.1 Hz, 2H), 2.38 (d, *J= 0.8* Hz, 3H), 1.46 (t, *J =* 7.1 Hz, 3H).

### Step 1: Preparation of tert-butyl (S)-4-(6-(7-ethoxy-2-methylimidazo[1,2-a]pyrimidine-6-carboxamido)pyridazin-3-yl)-2-methylpiperazine-1-carboxylate

To a solution of 6-bromo-7-ethoxy-2-methylimidazo[1,2-a]pyrimidine (100 mg, 0.389 mmol), tert-butyl (S)-4-(6-aminopyridazin-3-yl)-2-methylpiperazine-1-carboxylate (114 mg, 0.389 mmol) and Na₂CO₃ (62 mg, 0.584 mmol) in toluene (6 mL) were added Xantphots (10 mg, 0.016 mmol) and Pd(OAc)₂ (4 mg, 0.016 mmol). The mixture was charged CO three times and stirred at 80 °C under CO for 16 h. The mixture was concentrated under reduced pressure to give title product (300 mg, crude) as a dark solid, which was used to next step without further purification. ESI-MS (M+H)+: 497.2.

### Step 2: Preparation of (S)-7-ethoxy-2-methyl-N-(6-(3-methylpiperazin-1-yl)pyridazin-3-yl)imidazo[1,2-a]pyrimidine-6-carboxamide hydrochloride

To a solution of tert-butyl (S)-4-(6-(7-ethoxy-2-methylimidazo[1,2-a]pyrimidine-6-carboxamido)pyridazin-3-yl)-2-methylpiperazine-1-carboxylate (250 mg, crude) in EA (4 mL) was added EA/HCl (4 mL, 3 mol/L). The mixture was stirred at rt for 1 h. The mixture was concentrated in vacuo and the residue was purified by prep-HPLC (0.05 % HCl in water / CH₃CN) to give title compound (40 mg, yield: 26%) as a yellow solid. ESI-MS (M+H)+:397.1. ¹H NMR (400 MHz, MeOD-*d₄*) δ 9.49 (s, 1H), 8.59 (d, J = 10.1 Hz, 1H), 8.20 (d, J = 10.1 Hz, 1H), 7.79 (d, J = 1.0 Hz, 1H), 4.77 (d, J = 7.1 Hz, 2H), 4.51 - 4.45 (m, 2H), 3.70 - 3.66 (m, 1H), 3.64 - 3.60 (m, 2H), 3.47 - 3.40 (m, 2H), 2.51 (s, 3H), 1.55 (t, J = 7.1 Hz, 3H), 1.47 (d, J = 6.5 Hz, 3H).

### Example 70. Synthesis of (S)-6-ethoxy-2-methyl-N-(6-(3-methylpiperazin-1-yl) pyridazin-3-yl)-2H-pyrazolo [3, 4-b] pyridine-5-carboxamide hydrochloride (Compound 225)

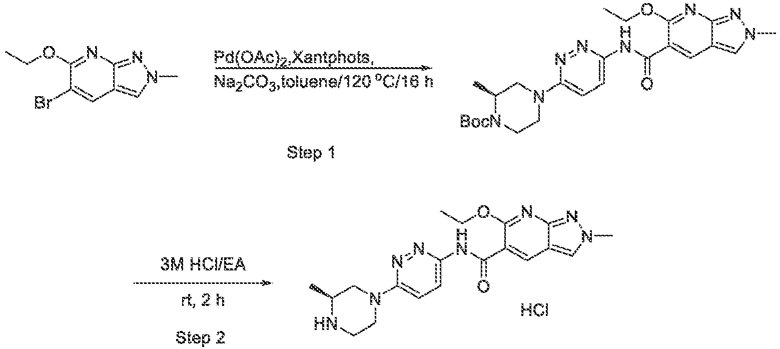

### Preparation of 5-bromo-6-ethoxy-2-methyl-2H-pyrazolo [3, 4-b] pyridine

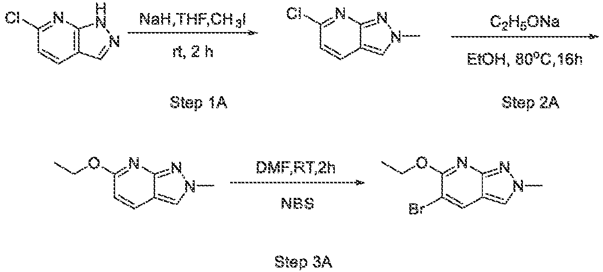

### Step 1A: preparation of 6-ethoxy-2-methyl-2H-pyrazolo [3, 4-b] pyridine

To a mixture of 6-chloro-2-methyl-2H-pyrazolo [3, 4-b] pyridine (10.3 g, 67.3 mmol) in THF (250 mL) was added NaH (5.38 g, 224 mmol) at 0 °C, the mixture was stirred at this temperature for 30 min, CH₃I (36.3 g, 256 mmoL) was added and the resultant was stirred at RT for 2 h. LCMS showed the starting material was consumed completely. The mixture was diluted with water (100 mL), extracted with EA (150 mL), washed with brine (100 mL *3). The combined organic layer was dried over Na₂SO₄ and concentrated in vacuo. The residue was purified by column chromatography (EA) to give title product (9.5 g, Y: 85 %) as a white solid. ESI-MS (M+H⁺): 168.0. ¹H NMR (400 MHz, CDCl₃) δ 7.96 (d, *J* = 8.6 Hz, 1H), 7.94 (s, 1H), 7.03 (d, *J =* 8.6 Hz, 1H), 4.23 (s, 3H).

### Step 2A: preparation of 6-ethoxy-2-methyl-2H-pyrazolo [3, 4-b] pyridine

To a mixture of 6-ethoxy-2-methyl-2H-pyrazolo [3, 4-b] pyridine (1.1 g, 6.6 mmol) in EtOH (20 mL) was added NaOEt (4.47 g, 24 mmol). The mixture was stirred at 80 °C for 16 h. LCMS showed the starting material was consumed completely. The mixture was diluted water (40 mL), extracted with EA (50 mL), wash with brine (40 mL *3). The combined organic layer was dried over Na₂SO₄ and concentrated in vacuo. The residue was purified by column chromatography (EA) to give title product (1 g, Y: 94 %) as a white solid. ESI-MS (M+H⁺): 178.1. ¹H NMR (400 MHz, CDCl₃) δ 7.80 (d, *J* = 8.9 Hz, 1H), 7.72 (s, 1H), 6.55 (d, *J* = 8.9 Hz, 1H), 4.50 (q, *J* = 7.2 Hz 2H), 4.12 (s, 3H), 1.42 (t, *J* = 7.2 Hz, 3H).

### Step 3A: preparation of 5-bromo-6-ethoxy-2-methyl-2H-pyrazolo [3, 4-b] pyridine

To a mixture of 6-ethoxy-2-methyl-2H-pyrazolo [3, 4-b] pyridine (800 mg, 4.52 mmol) in DMF (20 mL) was added NBS (950 g, 5.37 mmol). The mixture was stirred at rt for 2h. The mixture was diluted wtih water (30 mL), extracted with EA (40 mL), wash with brine (30 mL *3). The combined organic layer was dried over Na₂SO₄ and concentrated in vacuo. The residue was purified by column chromatography (EA) to give title product (800 mg, Y: 69 %) as a white solid. ESI-MS (M+H⁺): 257.9. ¹H NMR (400 MHz, CDCl₃) δ 8.10 (s, 1H), 7.71 (s, 1H), 4.56 (q, *J* = 6.8 Hz, 2H), 4.13 (s, 3H), 1.47 (t, *J* = 6.8 Hz, 3H).

### Step 1: Preparation of tert-butyl (S)-4-(6-(6-ethoxy-2-methyl-2H-pyrazolo [3, 4-b] pyridine-5-carboxamido) pyridazin-3-yl)-2-methylpiperazine-1-carboxylate

To a mixture of 5-bromo-6-ethoxy-2-methyl-2H-pyrazolo [3, 4-b] pyridine (100 mg, 0.4 mmol) and tert-butyl (S)-4-(6-aminopyridazin-3-yl)-2-methylpiperazine-1-carboxylate (115 mg, 0.4 mmol) in toluene (10 mL) were added Pd(OAc)₂ (3.5 mg, 0.02 mmol), Xantphots (10 mg, 0.02 mmol) and Na₂CO₃ (62 mg, 0.6 mmoL). The mixture was charged with CO for three times and stirred at 120 °C for 16 h. After cooling to rt,the mixture was filtered and the the filtrate was concentrated to give title product (100 mg, crude) as a black solid, which was used in next step directly. ESI-MS (M+H+): 497.4.

### Step 2: Preparation of (S)-6-ethoxy-2-methyl-N-(6-(3-methylpiperazin-1-yl) pyridazin-3-yl)-2H-pyrazolo [3, 4-b] pyridine-5-carboxamide hydrochloride

A mixture of tert-butyl (S)-4-(6-(6-ethoxy-2-methyl-2H-pyrazolo [3, 4-b] pyridine-5-carboxamido) pyridazin-3-yl)-2-methylpiperazine-1-carboxylate (crude, 100 mg, 0.2 mmol) in HCl/EA (8.00 mL) was stirred at rt for 2 h. The mixture was concentrated in vacuo and the residue was purified by prep-HPLC (0.05 % HCl in water / CH₃CN) to give title product (13.00 mg, Y: 16.25 % for two steps) as a yellow solid. ESI-MS (M+H+): 397.3. ¹H NMR (400 MHz, MeOD-*d₄*) δ 8.87 (s, 1H), 8.43 (s, 1H), 8.36 - 8.29 (m, 1H), 8.27 - 8.18 (m, 1H), 4.69 (q, J = 6.6 Hz, 2H), 4.53 - 4.44 (m, 2H), 4.20 (s, 3H), 3.64 - 3.55 (m, 3H), 3.32 - 3.30 (m, 2H), 1.56 - 1.52 (m, 3H), 1.49 - 1.45 (m, 3H).

### Preparation of Starting Material: N-(6-chloropyridazin-3-yl)-7-ethoxy-2-methylimidazo[1,2-a]pyridine-6-carboxamide

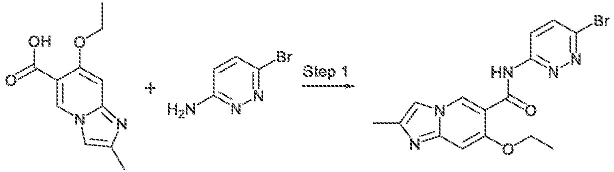

### Step 1: Preparation of N-(6-chloropyridazin-3-yl)-7-ethoxy-2-methylimidazo[1,2-a]pyridine-6-carboxamide

7-Ethoxy-2-methylimidazo[1,2-a]pyridine-6-carboxylic acid (6.1 g, 27.7 mmol) was suspended in DMF (50 mL) and 6-bromopyridazin-3-amine (4.82 g, 27.7 mmol) was added followed by N,N-dimethylpyridin-4-amine (4.06 g, 33.24 mmol). To the resulting mixture N-(3-dimethylaminopropyl)-N'-ethylcarbodiimide hydrochloride (6.37 g, 33.24 mmol) was added in one portion and the reaction mixture was stirred overnight at r.t. The precipitate formed was filtered, washed with MeCN (30 mL), MTBE (30 mL), dried in vacuo to give pure N-(6-chloropyridazin-3-yl)-7-ethoxy-2-methylimidazo[1,2-a]pyridine-6-carboxamide (9.06 g, Y: 87%). ESI-MS (M+H)+: 377.0 ¹H NMR (400 MHz, DMSO-d₆) δ 11.03 (s, 1H), 9.01 (s, 1H), 8.4 (d, J = 9 Hz, 1H), 8.01 (d, J = 9 Hz), 7.64 (s, 1H), 6.97 (s, 1H), 4.29 (q, J = 7.1 Hz, 2H), 2.29 (s, 3H), 1.49 (t, J = 6.9 Hz, 3H).

### Example 71. Synthesis of the Compounds of Table 3.

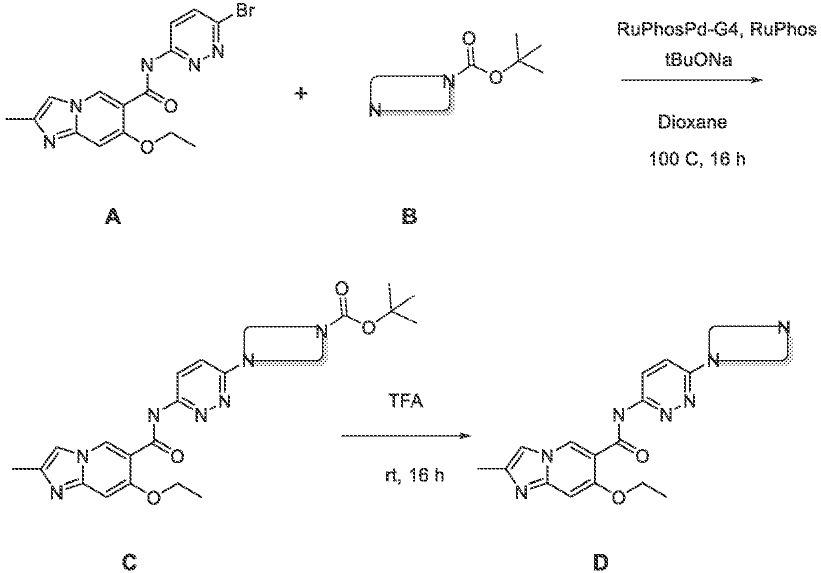

To a mixture of 1 eq. of ArBr (A) and 1.5 eq. amine (B) in 1 mL Dioxane under an inert atmosphere were added 0.1 eq. RuPhosPdG4, 0.1 eq. RuPhos and 3 eq. tBuONa*. The reaction mixture was stirred at 100 °C for 16 hours. After cooling to room temperature, the solvent was evaporated and 1 ml of cleavage cocktail** was added. The mixture was left to react for 4h and evaporated to dryness. The residue was dissolved in DMSO and subjected to HPLC purification***.
*In case of using Reagent 1 and/or Reagent 2 as salt, an additional amount t-BuONa (1.1 eq. for each eq. of acid) was added to the reaction mixture to convert the reagent into a free form.
**The cleavage cocktail was prepared by mixing TFA (92.5% v/v), water (5% v/v) and TIPS (2.5% v/v).
***Resulting solution was purified by HPLC (Deionized Water (phase A) and HPLC-grade Acetonitrile (phase B) were used as an eluent to obtained final compound (D). In most cases, TFA was used as an additive.
Instrument: Agilent 1260 Infinity systems equipped with DAD and mass-detector
Column: Waters Sunfire C18 OBD Prep Column, 100 A, 5 µm, 19 mm x 100mm with SunFire C18 Prep Guard Cartridge, 100 A, 10 µm, 19 mm x 10 mm.

**Table 3. Compounds synthesized using the procedure of Example 71.**

| **Compound No.** | **Compound** | **ESI-MS (M+H)+** |
|---|---|---|
| 226 | 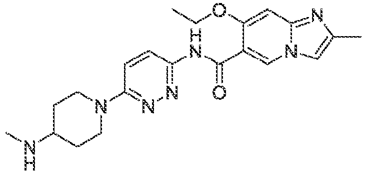 | 411.0 |
| 227 | 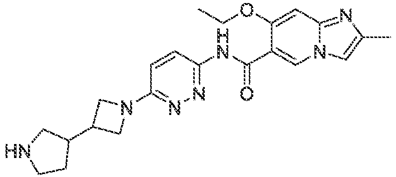 | 423.1 |
| 228 | 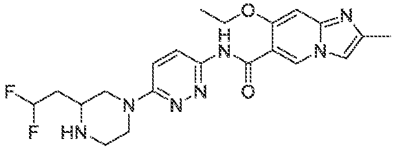 | 446.2 |
| 229 | 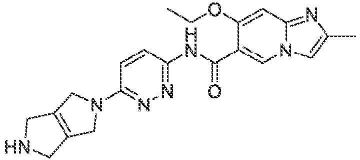 | 406.0 |
| 230 | 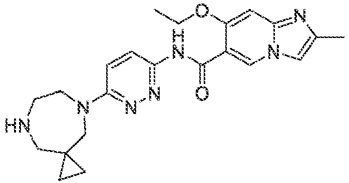 | 422.2 |
| 231 | 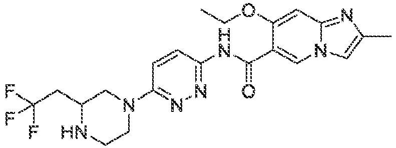 | 464.2 |
| 232 | 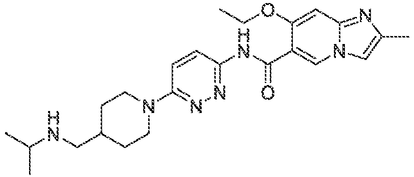 | 452.2 |
| 233 | 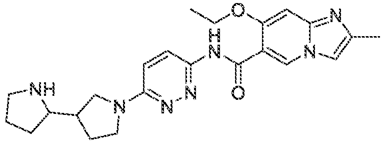 | 437.2 |
| 234 | 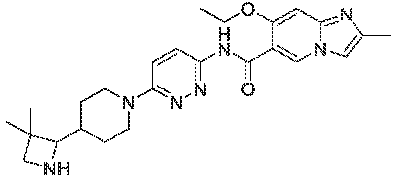 | 464.4 |
| 235 | 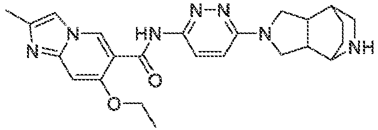 | 448.4 |
| 236 | 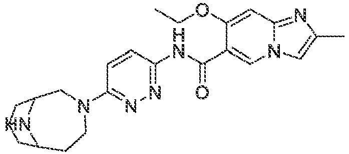 | 436.3 |
| 237 | 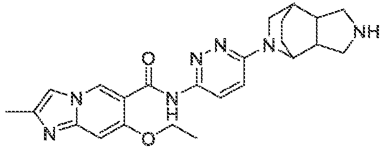 | 448.4 |
| 238 | 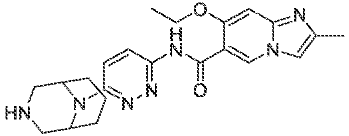 | 422.1 |
| 239 | 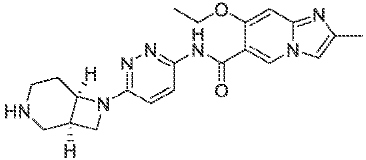 | 408.2 racemate |
| 240 | 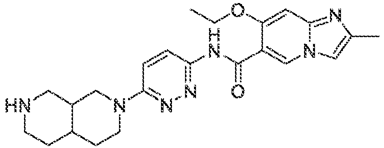 | 436.2 |
| 241 | 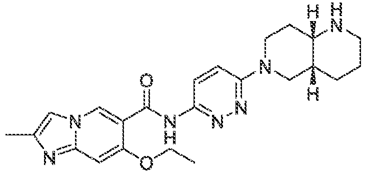 | 436.2 racemate |
| 242 | 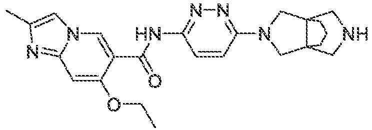 | 448.2 |
| 243 | 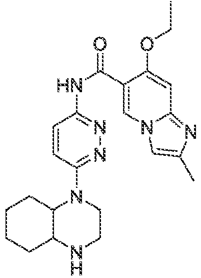 | 436.2 |
| 244 | 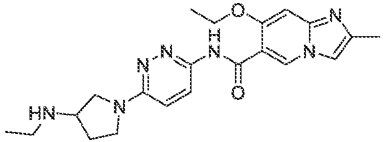 | 410.2 |
| 245 | 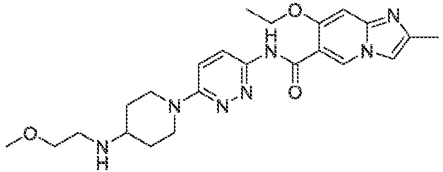 | 454.2 |
| 246 | 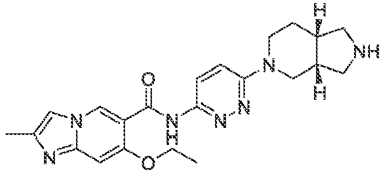 | 422.2 racemate |
| 247 | 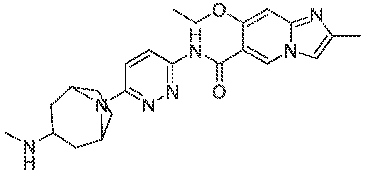 | 436.2 |
| 248 | 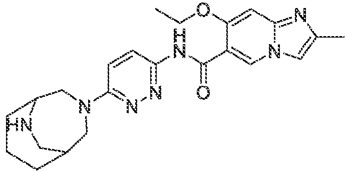 | 437.1 |
| 249 | 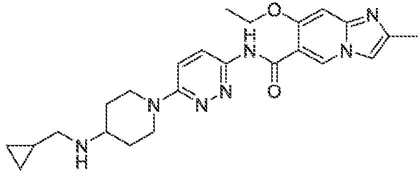 | 450.2 |
| 250 | 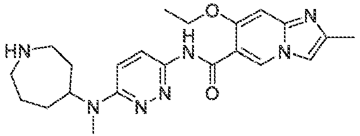 | 424.2 |
| 251 | 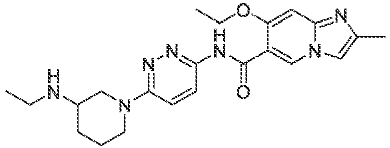 | 424.2 |
| 252 | 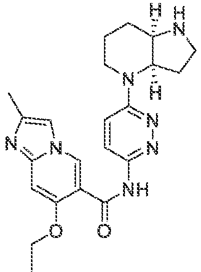 | 422.2 racemate |
| 253 | 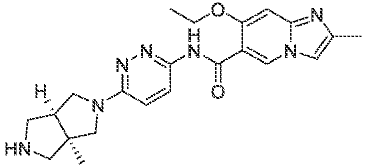 | 422.2 racemate |
| 254 | 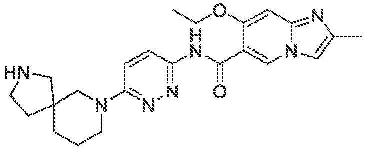 | 436.1 |
| 255 | 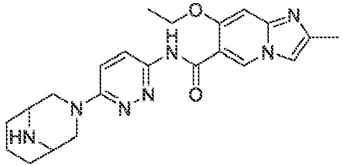 | 422.2 |
| 256 | 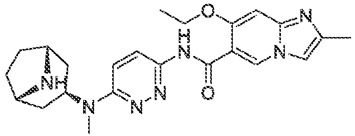 | 436.2 |
| 257 | 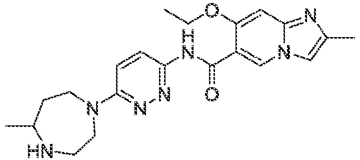 | 410.2 |
| 258 | 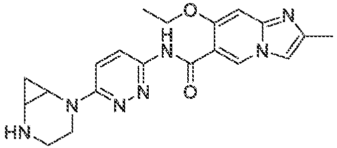 | 394.4 |
| 259 | 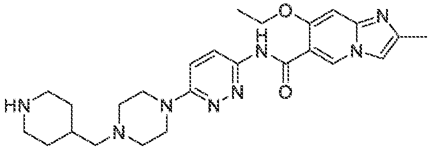 | 479.2 |
| 260 | 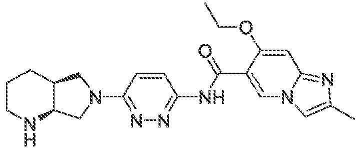 | 422.2 |
| 261 | 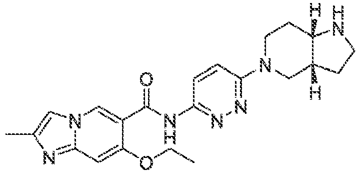 | 422.1 racemate |
| 262 | 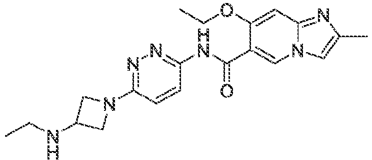 | 396.2 |
| 263 | 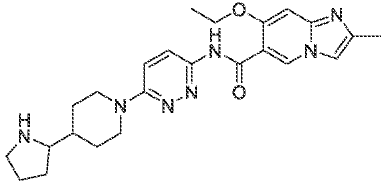 | 450.2 |
| 264 | 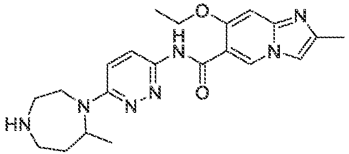 | 410.2 |
| 265 | 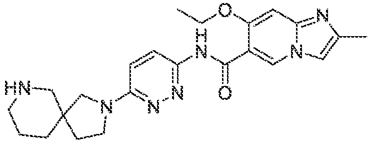 | 436.2 |
| 266 | 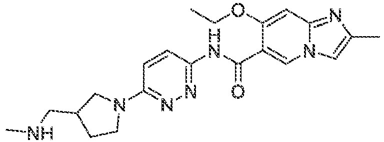 | 410.4 |
| 267 | 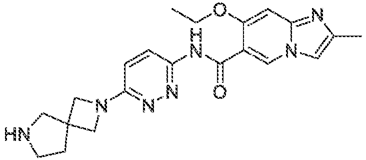 | 408.2 |
| 268 | 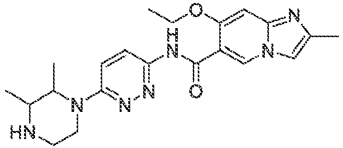 | 410.2 |
| 269 | 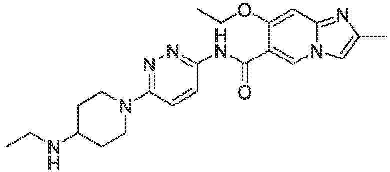 | 424.4 |
| 270 | 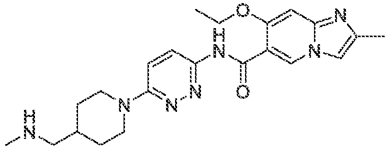 | 424.4 |
| 271 | 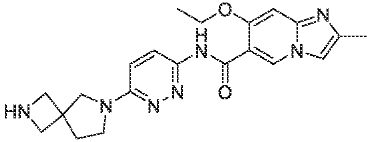 | 408.2 |
| 272 | 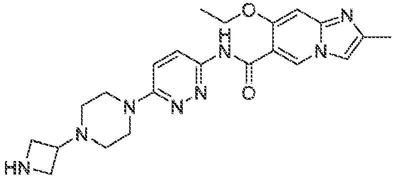 | 437.3 |
| 273 | 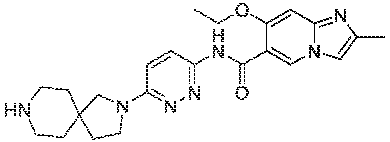 | 436.2 |
| 274 | 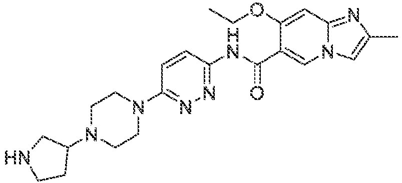 | 451.4 |
| 275 | 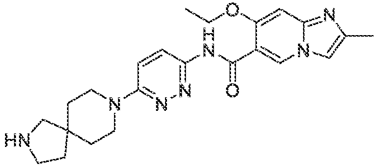 | 436.2 |
| 276 | 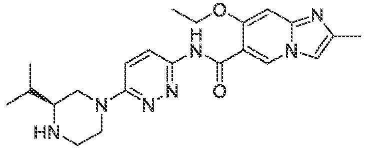 | 424.4 |
| 277 | 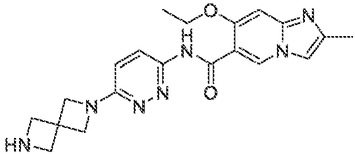 | 394.2 |
| 278 | 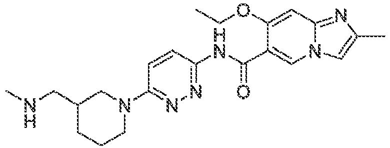 | 424.2 |
| 279 | 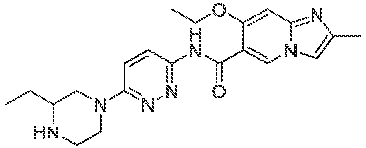 | 410.2 |
| 280 | 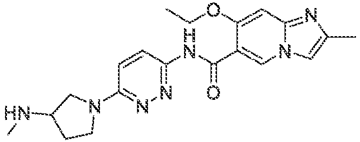 | 396.4 |
| 281 | 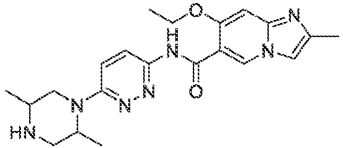 | 410.1 |
| 282 | 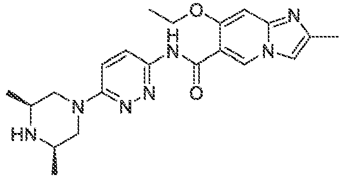 | 410.2 |
| 283 | 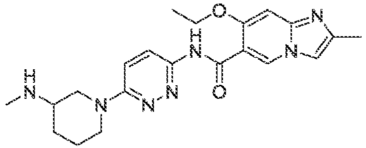 | 410.2 |
| 284 | 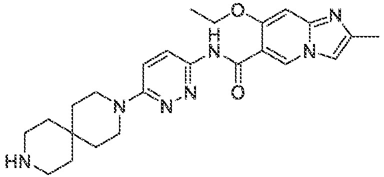 | 450.4 |
| 285 | 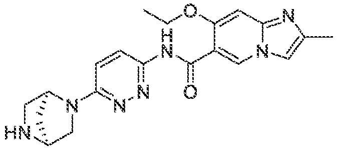 | 394.2 |
| 286 | 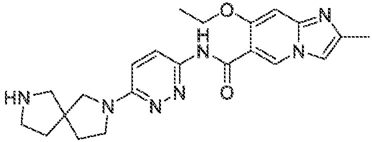 | 422.4 |
| 287 | 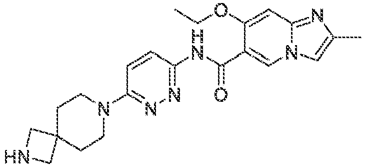 | 422.4 |
| 288 | 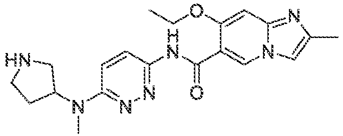 | 396.2 |
| 289 | 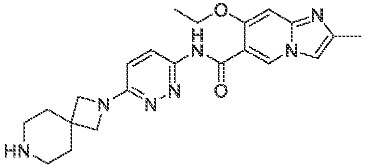 | 422.4 |
| 290 | 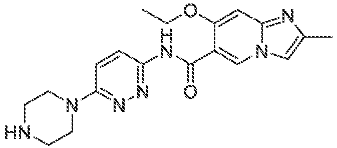 | 382.4 |
| 291 | 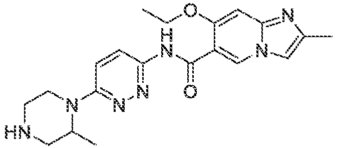 | 396.4 |
| 292 | 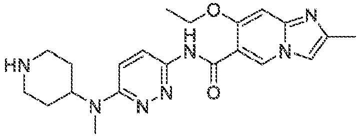 | 410.4 |
| 293 | 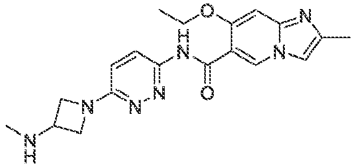 | 382.2 |
| 294 | 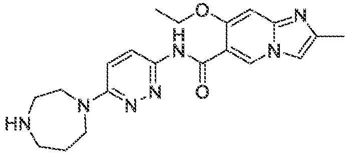 | 396.2 |
| 295 | 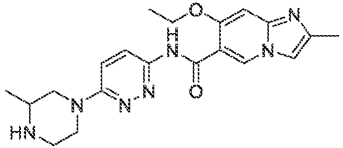 | 396.2 |
| 296 | 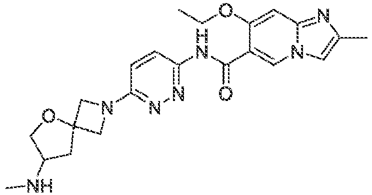 | 438.1 |
| 297 | 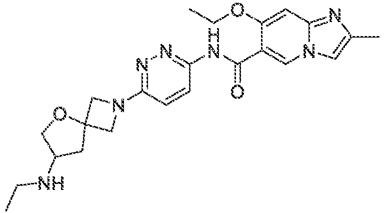 | 452.2 |
| 298 | 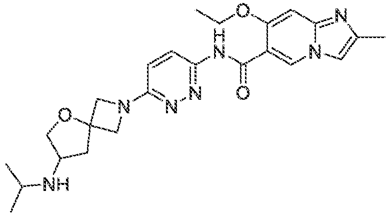 | 466.2 |

### Example 72. Synthesis of (S)-N-(6-(3,4-dimethylpiperazin-1-yl)pyridazin-3-yl)-6-ethoxy-2-methyl-2H-indazole-5-carboxamide hydrochloride (Compound 299)

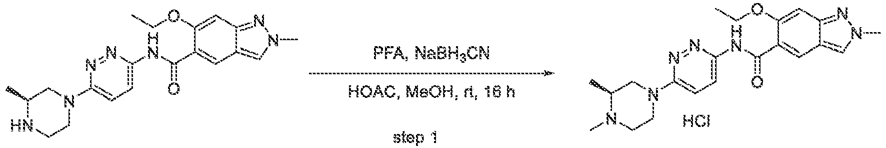

### Step 1: Preparation of (S)-N-(6-(3,4-dimethylpiperazin-1-yl)pyridazin-3-yl)-6-ethoxy-2-methyl-2H-indazole-5-carboxamide hydrochloride

To a solution of (S)-6-ethoxy-2-methyl-N-(6-(3-methylpiperazin-1-yl)pyridazin-3-yl)-2H-indazole-5-carboxamide (50 mg, 0.13 mmol) in MeOH (5 mL ) were added PFA (19 mg, 0.63 mmol) and acetic acid (50 mg, 0.83 mmol). The mixture was stirred at RT for 1 h, then sodium cyanoborohydride (24 mg, 0.38 mmol) was added under ice bath and the mixture was stirred at rt for 16 h, then water (10 mL) was added and the solution and concentrated in vacuo. The crude was purified by prep-HPLC (0.05% HCl in water / CH₃CN) to give title compound (17 mg, yield: 32.8 %) as a white solid. ESI-MS (M+H)+: 410.3. ¹H NMR (400 MHz, MeOD-*d₄*) δ 8.50 (s, 1H), 8.41 (s, 1H), 8.15 (s, 1H), 8.12 (s, 1H), 7.14 (s, 1H), 4.54 (d, J = 14.7 Hz, 2H), 4.36 - 4.31 (m, 2H), 4.21 (s, 3H), 3.77 - 3.69 (m, 1H), 3.58 - 3.46 (m, 2H), 3.42 - 3.34 (m, 2H), 3.01 (s, 3H), 1.60 - 1.56 (m, 3H), 1.54 - 1.47 (m, 3H).

### Example 73. Synthesis of (S)-6-ethoxy-N-(6-(4-ethyl-3-methylpiperazin-1-yl)pyridazin-3-yl)-2-methyl-2H-indazole-5-carboxamide hydrochloride (Compound 300)

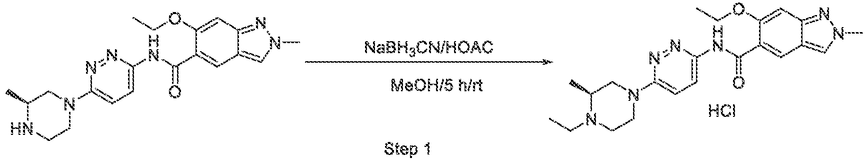

### Step 1: Preparation of (S)-6-ethoxy-N-(6-(4-ethyl-3-methylpiperazin-1-yl)pyridazin-3-yl)-2-methyl-2H-indazole-5-carboxamide hydrochloride.

To a mixture of (S)-6-ethoxy-2-methyl-N-(6-(3-methylpiperazin-1-yl)pyridazin-3-yl)-*2H-indazole-5-carboxamide* (100 mg, 0.25 mmol) and CH₃CHO (56.3 mg, 1.28 mmol) in MeOH (8 mL) and HOAc (45.6 mg, 0.76 mmol) was added NaBH₃CN (50 mg, 0.8 mmol). The mixture was stirred at RT for 5 h. The mixture was diluted with H₂O (3 mL) and concentrated in vacuo. The residue was purified by prep-HPLC (0.05 % HCl in water / ACN) to give title product (33 mg, Y: 28 %) as a yellow solid. ESI-MS (M+H+): 424.2. ¹H NMR (400 MHz, MeOD-d₄) δ 8.72 (s, 1H), 8.49 (s, 1H), 8.27 (br.s, 2H), 7.20 (s, 1H), 4.56 (d, J = 8.1 Hz, 2H), 4.39 - 4.33 (m, 2H), 4.31 (s, 3H), 3.77 (br s, 2H), 3.69 - 3.45 (m, 3H), 3.44 - 3.32 (m, 1H), 3.30 - 3.12 (m, 1H), 1.56 (br.s, 6H), 1.43 (br.s, 3H).

### Example 74. Synthesis of 6-ethoxy-2-methyl-N-{6-[(3S)-3-methyl-4-(oxan-4-yl)piperazin-1-yl]pyridazin-3-yl}-2H-indazole-S-carboxamide (Compound 301)

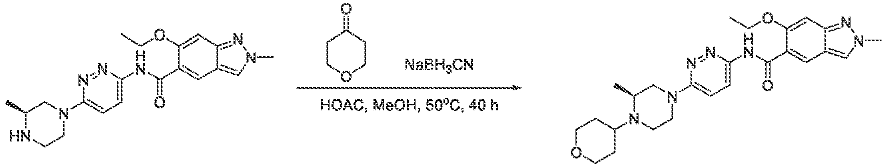

### Step 1: Preparation of (S)-6-ethoxy-2-methyl-N-(6-(3-methyl-4-(tetrahydro-2H-pyran-4-yl)piperazin-1-yl)pyridazin-3-yl)-2H-indazole-5-carboxamide

To a solution of (S)-6-ethoxy-2-methyl-N-(6-(3-methylpiperazin-1-yl)pyridazin-3-yl)-2H-indazole-5-carboxamide (100 mg, 0.25 mmol) in MeOH (5 mL ) were added tetrahydro-4H-pyran-4-one (253 mg, 2.5 mmol) and acetic acid (100 mg, 1.66 mmol). The mixture was stirred at RT for 1 h, then sodium cyanoborohydride (47 mg, 0.76 mmol) was added under ice bath and the mixture was stirred at 50 °C for 40 h. After concentration, the crude was purified by prep-HPLC (0.05% NH₃.H₂O in water / CH₃CN) to give title compound (7 mg, yield: 5.8 %) as a white solid. ESI-MS (M+H)⁺: 480.4. ¹H NMR (400 MHz, DMSO-d₆) δ 10.86 (s, 1H), 8.46 (d, *J* = 4.9 Hz, 2H), 8.25 (d, *J* = 9.9 Hz, 1H), 7.41 (d, *J* = 9.8 Hz, 1H), 7.15 (s, 1H), 4.31 - 4.26 (m, 2H), 4.14 (s, 3H), 3.92 - 3.82 (m, 4H), 3.31 - 3.24 (m, 4H), 3.21 - 3.15 (m, 1H), 2.97 - 2.90 (m, 2H), 2.87 - 2.80 (m, 2H), 1.67 - 1.59 (m, 2H), 1.54 - 1.51 (m, 3H), 1.43 - 1.34 (m, 1H), 1.10 - 1.05 (m, 3H).

### Example 76. Synthesis of 7-methoxy-2-methyl-N-{5-methyl-6-[(3S)-3-methylpiperazin-1-yl]pyridazin-3-yl}imidazo[1,2-a]pyridine-6-carboxamide (Compound 302)

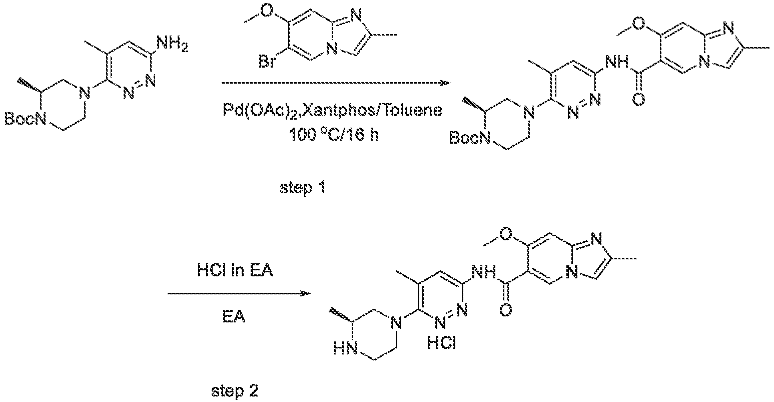

### Step 1: preparation of tert-butyl (S)-4-(6-(7-methoxy-2-methylimidazo[1,2-a]pyridine-6-carboxamido)-4-methylpyridazin-3-yl)-2-methylpiperazine-1-carboxylate

To a mixture of tert-butyl (S)-4-(6-amino-4-methylpyridazin-3-yl)-2-methylpiperazine-1-carboxylate (100 mg, 0.417 mmol) in toluene ( 15 mL) were added 6-bromo-7-methoxy-2-methylimidazo [1,2-a]pyridine ( 86 mg, 0.417 mmol), Pd(OAc)₂ (9 mg, 0.042 mmol), Xantphos (48 mg, 0083 mmol ) and Na₂CO₃ (88.4 mg, 0.834 mmol). The resulting mixture was stirred overnight at 100 °C under CO. The mixture was allowed to cool down to room temperature and concentrated. Then diluted with water (15 mL) and extracted with EA (30 mL *2). The combined organic layer was concentrated to provide the crude product (150 mg, crude), which was used to next step without further purifiaction . ESI-MS (M+H)⁺: 496.2

### Step 2: preparation of (S)-7-methoxy-2-methyl -N-(5-methyl-6-(3-methylpiperazin-1-yl)pyridazin-3-yl)imidazo[1,2-a]pyridine-6-carboxamide hydrochloride

To a solution of tert-butyl (*S*)-4-(6-(7-methoxy-2-methylimidazo[1,2-a]pyridine-6-carboxamido)-4-methylpyridazin-3-yl)-2-methylpiperazine-1-carboxylate (150 mg, crude) in EA (5 mL) was added 3M HCl in EA (5 mL) at rt .The mixture was stirred for 2 h at rt. The reaction was concentrated, the residue was purified by prep-HPLC (0.05% HCl in water / CH₃CN) to give title product (7 mg, yield: 5 % two steps) as a yellow solid. ESI-MS (M+H)⁺: 396.1. ¹H NMR (400 MHz, DMSO-d₆) δ 9.09 (s, 1H), 8.20 (s, 1H), 7.91 (s, 1H), 7.38 (s, 1H), 4.07 (s, 3H), 3.60 (d, *J* = 11.3 Hz, 2H), 3.53 - 3.47 (m, 1H), 3.43 - 3.37 (m, 1H), 3.29 - 3.19 (m, 2H), 3.06 - 2.97 (m, 1H), 2.46 (s, 3H), 2.38 (s, 3H), 1.32 (d, *J* = 6.5 Hz, 3H).

### Example 77. Preparation of 6-ethoxy-2-methyl-2H-indazole-5-carboxylic acid

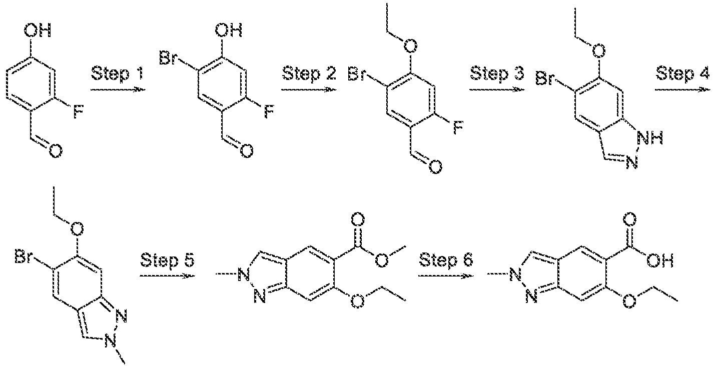

### Step 1: Preparation of 5-bromo-2-fluoro-4-hydroxybenzaldehyde

To a solution of 2-fluoro-4-hydroxybenzaldehyde (30.0 g, 214.11 mmol) in acetic acid (250 mL) was added a solution of bromine (35.93 g, 224.82 mmol, 11.52 mL) in acetic acid (20 mL), and the mixture was stirred at 45 °C for 26 h. The reaction mixture was concentrated under reduced pressure, brine was added to the residue, and the resulting mixture was extracted with ethyl acetate. The extract was dried over anhydrous magnesium sulfate and concentrated under reduced pressure to give 5-bromo-2-fluoro-4-hydroxybenzaldehyde (45.4 g, 85.0% purity, 176.2 mmol, Y: 82.3%) as colorless crystals. 1H NMR (500 MHz, DMSO-d₆) δ 11.95 (s, 1H), 9.92 (s, 1H), 7.88 (d, J = 8.1, 1H), 6.82 (d, J = 12.2 Hz, 1H).

### Step 2: Preparation of 5-bromo-4-ethoxy-2-fluorobenzaldehyde

To a solution of 5-bromo-2-fluoro-4-hydroxybenzaldehyde (45.4 g, 207.3 mmol) in DMF (300 mL) was added potassium carbonate (57.3 g, 414.59 mmol) and the solution was stirred at 25 °C for 30 min. Iodoethane (38.8 g, 248.76 mmol) was added and the reaction mixture was stirred at 50 °C for 16 h. A mixture of water (300 mL) and ethyl acetate (300 mL) was added, and the organic layer was separated, washed with water (100 mL), brine (100 mL), dried over Na2SO4 and evaporated under vacuo to afford 5-bromo-4-ethoxy-2-fluorobenzaldehyde (41.3 g, 85.0% purity, 142.09 mmol, Y: 68.5%) as a white solid. 1H NMR (500 MHz, DMSO-d₆) δ 9.99 (s, 1H), 7.93 (d, J = 7.5 Hz, 1H), 7.19 (d, J = 12.7 Hz, 1H), 4.21 (q, J = 7.2 Hz, 2H), 1.37 (t, J = 6.8 Hz, 3H).

### Step 3: Preparation of 5-bromo-6-ethoxy-2H-indazole

To a solution of 5-bromo-4-ethoxy-2-fluorobenzaldehyde (41.3 g, 167.17 mmol) in DMSO (350 mL) was added potassium carbonate (27.72 g, 200.6 mmol) and hydrazine hydrate (20.92 g, 417.91 mmol) and the reaction mixture was heated at 100 °C for 16 h. The cooled resulting mixture was diluted with water (500 mL) and extracted with EtOAc (3 × 250 mL). The combined organic layers were washed with brine (300 mL), dried over Na2SO4, filtered, and concentrated in vacuo to give 5-bromo-6-ethoxy-2H-indazole (26.0 g, 85.0% purity, 91.67 mmol, Y: 54.8%) as a yellow solid. ESI-MS (M+H)+: 241.0. 1H NMR (500 MHz, CDCl₃) δ 10.30 (s, 1H), 7.94 (s, 1H), 6.89 (s, 1H), 4.16 (q, J = 7.2 Hz, 2H), 1.49 (t, J = 6.9 Hz, 3H).

### Step 4: Preparation of 5-bromo-6-ethoxy-2-methyl-2H-indazole

To a stirred solution of 5-bromo-6-ethoxy-2H-indazole (26.6 g, 110.33 mmol) in EtOAc (400 mL) was added trimethyloxonium tetrafluoroborate (48.96 g, 331.01 mmol) at rt. The solution was stirred at rt for 2 days. The resulting mixture was poured into sat. NaHCO3 and the organic layer was separated, dried over Na₂SO₄, and evaporated to dryness. The residue was purified by FCC to give 5-bromo-6-ethoxy-2-methyl-2H-indazole (9.6 g, 95.0% purity, 35.75 mmol, Y: 32.4%). 1H NMR (400 MHz, DMSO-d₆) δ 8.20 (s, 1H), 7.96 (s, 1H), 7.04 (s, 1H), 4.09 (d, J = 7.5 Hz, 2H), 3.32 (s, 3H), 1.38 (t, J = 6.9 Hz, 3H).

### Step 5: Preparation of methyl 6-ethoxy-2-methyl-2H-indazole-5-carboxylate

5-Bromo-6-ethoxy-2-methyl-2H-indazole (9.6 g, 37.63 mmol) was dissolved in MeOH (150 mL) and Pd(dppf)Cl¬¬2 (614.61 mg, 752.61 µmol) was added followed by triethylamine (7.62 g, 75.26 mmol). The resulting mixture was transferred into an autoclave and stirred at 130 °C under 40 bar pressure of CO overnight. Then the MeOH was evaporated, and the residue was partitioned between water (100 mL) and EtOAc (150 mL). The organic layer was separated, dried over Na2SO4, and evaporated under reduced pressure to give crude methyl 6-ethoxy-2-methyl-2H-indazole-5-carboxylate (8.2 g, 90.0% purity, 31.5 mmol, Y: 83.7%) which was used in the next step without purification. ESI-MS (M+H)+: 235.2. 1H NMR (500 MHz, DMSO-d₆) δ 8.35 (s, 1H), 8.04 (s, 1H), 6.98 (s, 1H), 4.09 (s, 3H), 4.04 (q, J = 8.6, 2H), 3.30 (s, 3H), 1.32 (t, J = 7.1, 3H).

### Step 6: Preparation of 6-ethoxy-2-methyl-2H-indazole-5-carboxylic acid

A mixture of methyl 6-ethoxy-2-methyl-2H-indazole-5-carboxylate (8.2 g, 35.01 mmol) and potassium hydroxide (2.36 g, 42.01 mmol) was stirred in methanol (150 mL) and H2O (30 mL) overnight. The reaction mixture was then concentrated under reduced pressure to remove methanol, and the resulting aqueous solution was neutralized with 1 N HCl to pH=5 to precipitate the carboxylic acid. The solid 6-ethoxy-2-methyl-2H-indazole-5-carboxylic acid (6.5 g, 95.0% purity, 28.04 mmol, Y: 80.1%) was isolated by filtration, dried, and used directly in the next step without further purification. ESI-MS (M+H)+: 221.0. 1H NMR (500 MHz, DMSO-d₆) δ 8.31 (s, 1H), 8.01 (s, 1H), 6.95 (s, 1H), 4.15-3.90 (m, 5H), 1.32 (d, J = 7.2 Hz, 3H).

### Example 78. Preparation of N-(5-bromopyrazin-2-yl)-6-ethoxy-2-methyl-2H-indazole-5-carboxamide

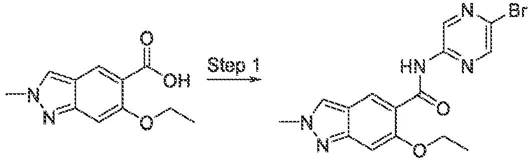

### Step 1: Preparation of N-(5-bromopyrazin-2-yl)-6-ethoxy-2-methyl-2H-indazole-5-carboxamide

A mixture of 6-ethoxy-2-methyl-2H-indazole-5-carboxylic acid (3.2 g, 14.53 mmol), 5-bromopyrazin-2-amine (2.53 g, 14.53 mmol), and pyridine (3.45 g, 43.6 mmol) was dissolved in anhydrous acetonitrile (4 mL) and stirred at room temperature for 5 minutes, followed by addition of T3P (18.5 g, 58.13 mmol) (50 wt % in EtOAc) over 10 minutes. The reaction mixture was stirred for 12 hours, then filtered and dried under *vacuo* to afford N-(5-bromopyrazin-2-yl)-6-ethoxy-2-methyl-2H-indazole-5-carboxamide (4.68 g, 95.0% purity, 11.82 mmol, Y: 81.3%). ESI-MS (M+H)⁺: 376.1. ¹H NMR (400 MHz, DMSO-d₆) δ 10.80 (s, 1H), 9.30 (s, 1H), 8.58 (s, 1H), 8.45 (s, 1H), 8.37 (s, 1H), 7.14 (s, 1H), 4.30 (q, *J* = 7.2 Hz, 2H), 4.13 (s, 3H), 1.50 (t, *J* = 6.8 Hz, 3H).

### Example 79. General procedure for parallel synthesis:

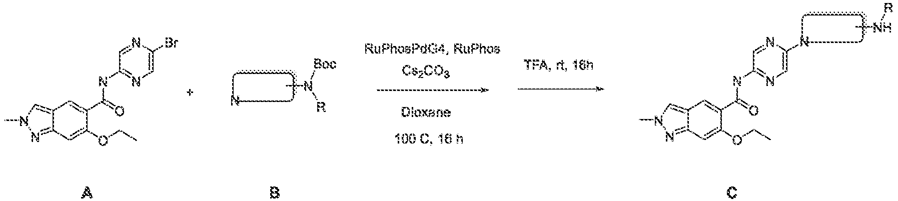

To a mixture of 1 eq. of ArBr **(A)** and 1.5 eq. Amine **(B)** in 1ml dry Dioxane under an inert atmosphere were added 0.05 eq. RuPhosPdG4, 0.05 eq. RuPhos and 2.5 eq. Cs₂CO₃. The reaction mixture was stirred at 100 °C for 16 hours. After cooling to room temperature, the solvent was evaporated and 1 ml TFA was added and stirred at room temperature for 4h. The mixture was evaporated. The residue was dissolved in DMSO (appr. 1 ml), treated with scavenger SiliaMetS DMT and filtered. Resulting solution was purified by HPLC (Deionized Water (phase A) and HPLC-grade Acetonitrile (phase B) were used as an eluent to obtain final compound (C). In most cases, TFA was used as an additive.
Instrument: Agilent 1260 Infinity systems equipped with DAD and mass-detector
Column: Waters Sunfire C18 OBD Prep Column, 100 A, 5 µm, 19 mm x 100mm with SunFire C18 Prep Guard Cartridge, 100 A, 10 µm, 19 mm x 10 mm.

**Table 4. Compounds synthesized using the procedure of Example 79.**

| **Compound no.** | **Compound** | **ESI-MS** |
|---|---|---|
| 303 | 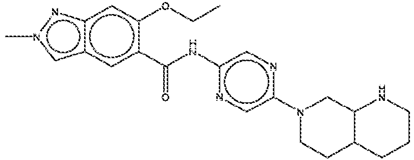 | 436.2 |
| 304 | 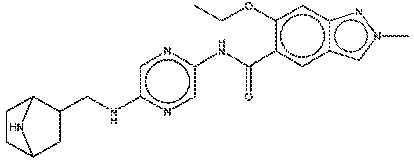 | 422.2 |
| 305 | 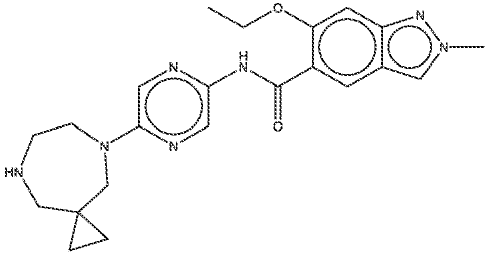 | 422.2 |
| 306 | 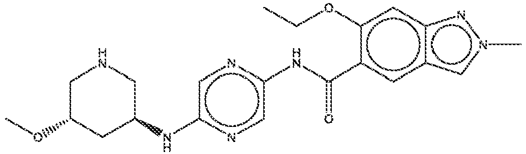 | 426.2 |
| 307 | 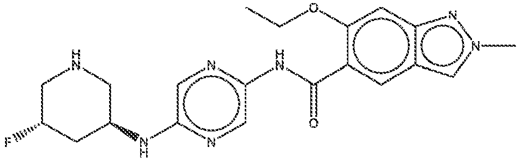 | 414 |
| 308 | 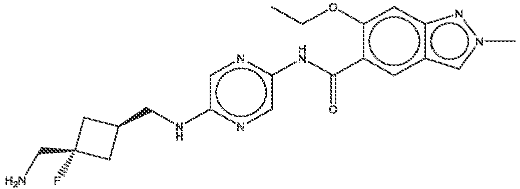 | 428.4 |
| 309 | 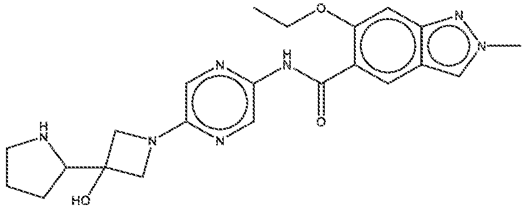 | 438 |
| 310 | 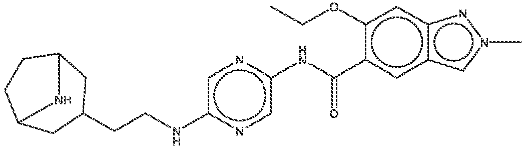 | 450.2 |
| 311 | 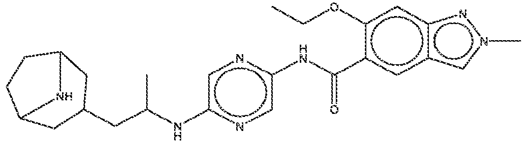 | 464.2 |
| 312 | 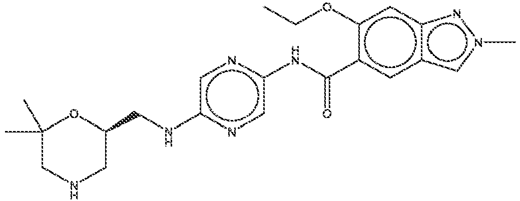 | 440.4 |
| 313 | 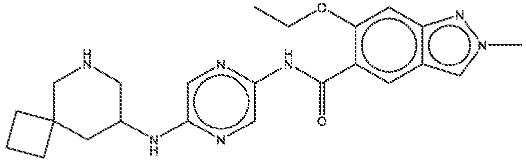 | 436.4 |
| 314 | 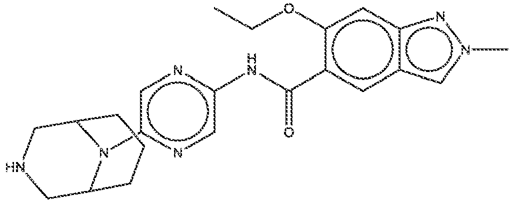 | 422.2 |
| 315 | 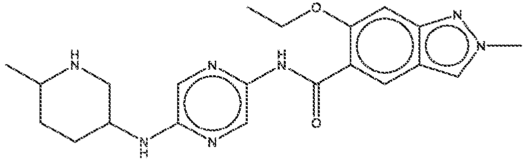 | 410.2 |
| 316 | 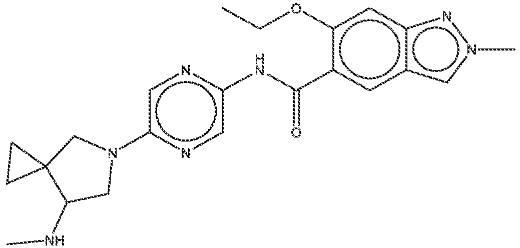 | 422.4 |
| 317 | 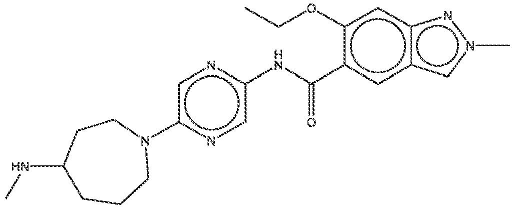 | 424.2 |
| 318 | 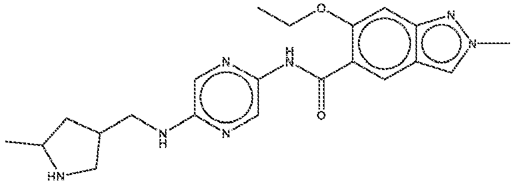 | 410.1 |
| 319 | 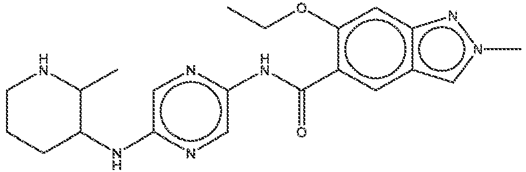 | 410.2 |
| 320 | 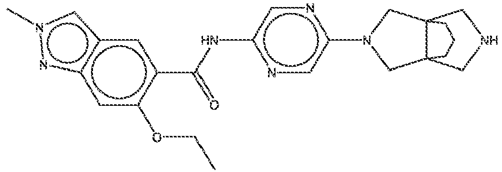 | 448.2 |
| 321 | 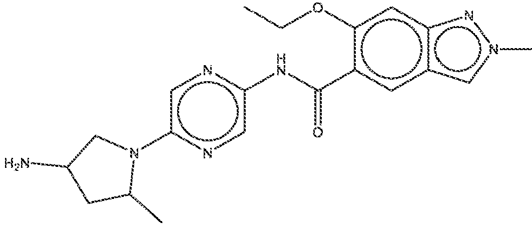 | 396.1 |
| 322 | 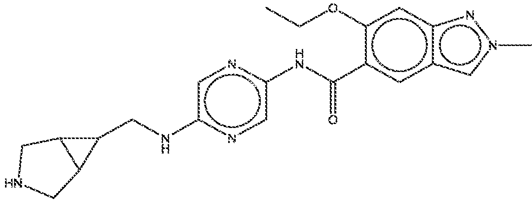 | 408.2 |
| 323 | 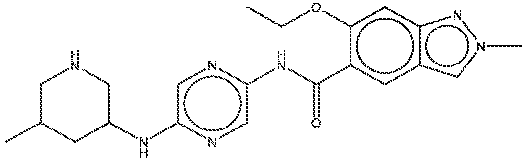 | 410.2 |
| 324 | 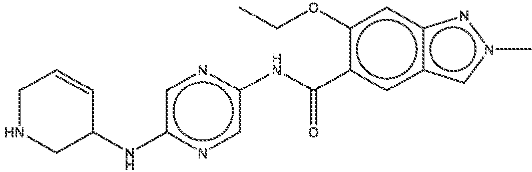 | 394.2 |
| 325 | 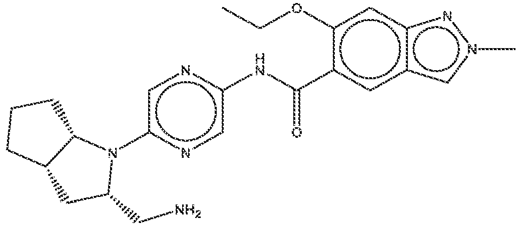 | 436.2 |
| 326 | 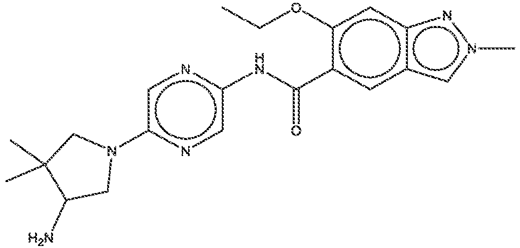 | 410.2 |
| 327 | 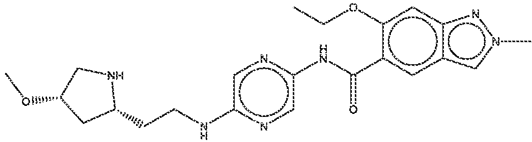 | 440.2 |
| 328 | 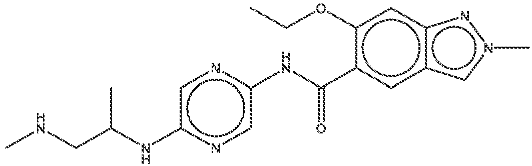 | 384.2 |
| 329 | 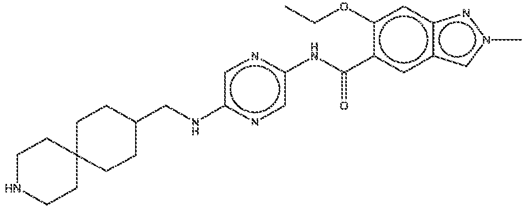 | 478.4 |
| 330 | 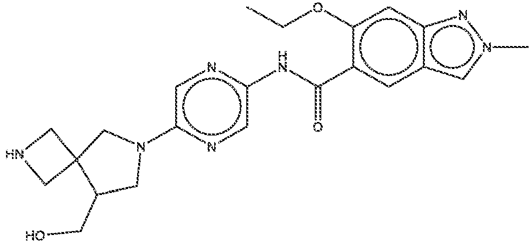 | 438.1 |
| 331 | 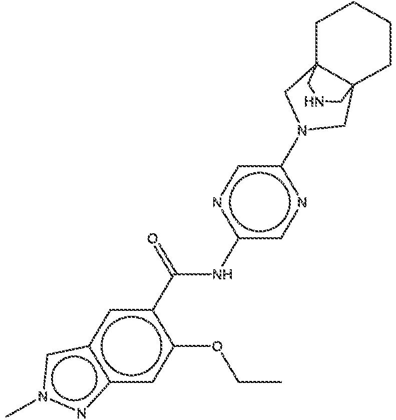 | 462.1 |
| 332 | 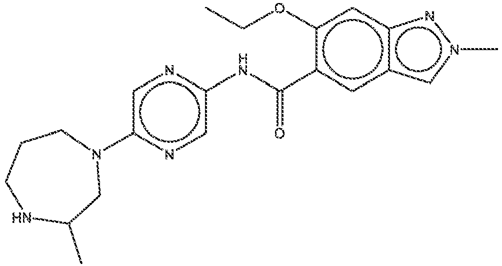 | 410.2 |
| 333 | 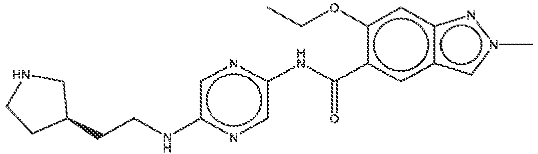 | 410.2 |
| 334 | 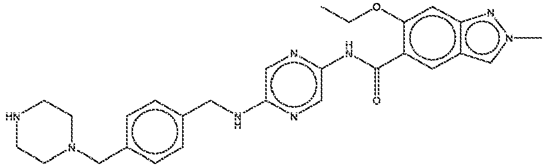 | 501.2 |
| 335 | 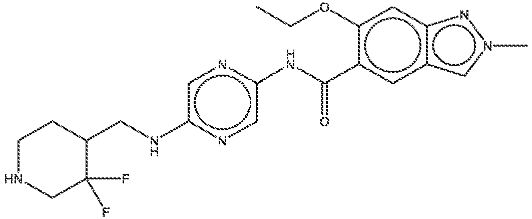 | 446.2 |
| 336 | 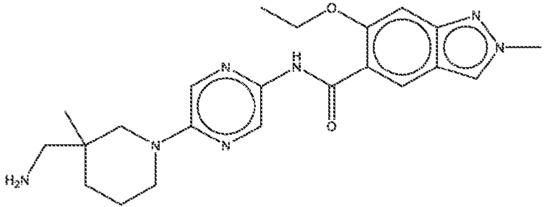 | 424.2 |
| 337 | 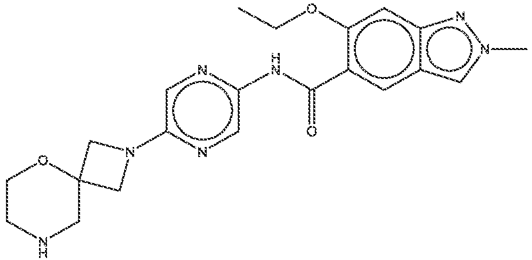 | 424.2 |
| 338 | 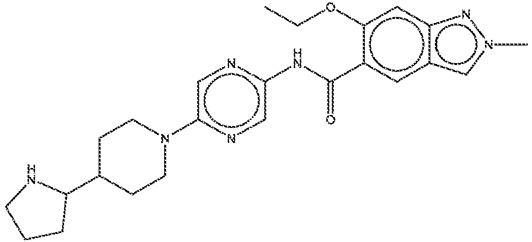 | 450.2 |
| 339 | 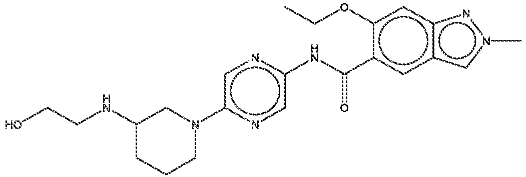 | 440.2 |
| 340 | 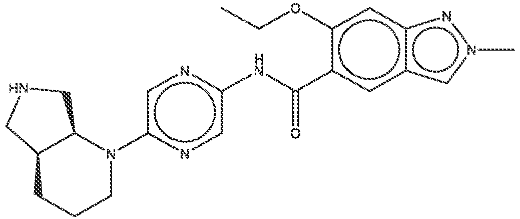 | 422.2 |
| 341 | 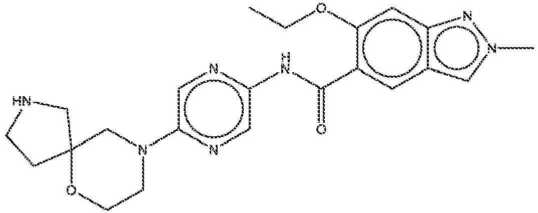 | 438.2 |
| 342 | 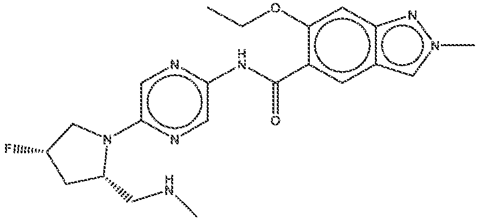 | 428.2 |
| 343 | 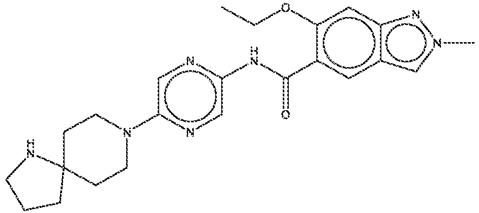 | 436.2 |
| 344 | 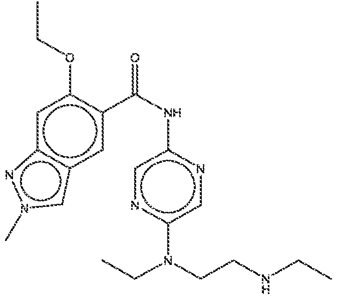 | 412.2 |
| 345 | 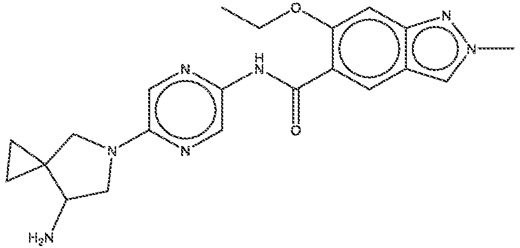 | 408.2 |
| 346 | 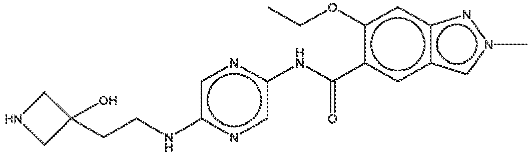 | 412.2 |
| 347 | 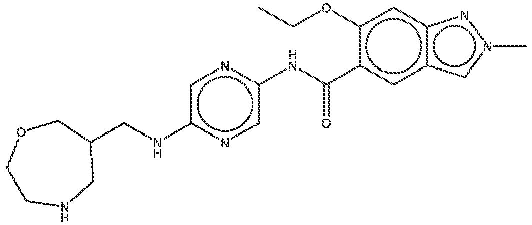 | 426.2 |
| 348 | 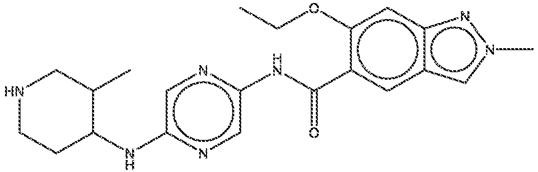 | 410.1 |
| 349 | 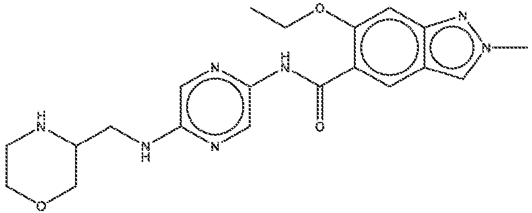 | 412.2 |
| 350 | 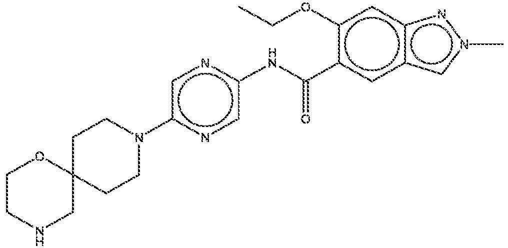 | 452.2 |
| 351 | 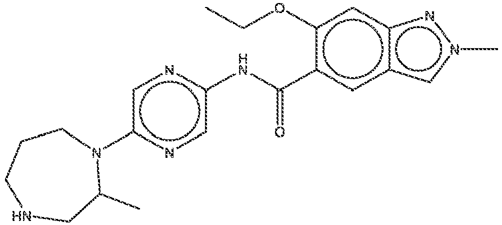 | 410.2 |
| 352 | 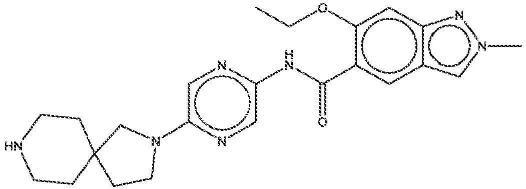 | 436.2 |
| 353 | 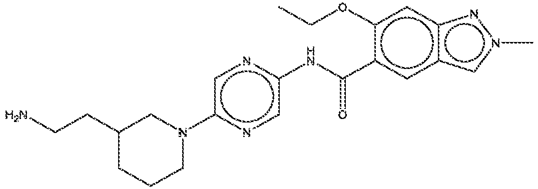 | 424.2 |
| 354 | 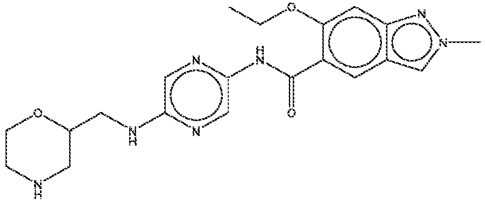 | 412.2 |
| 355 | 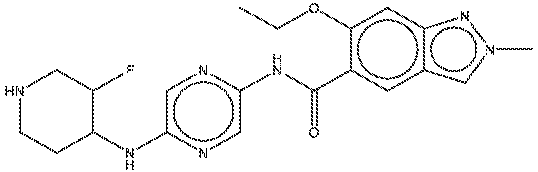 | 414.2 |
| 356 | 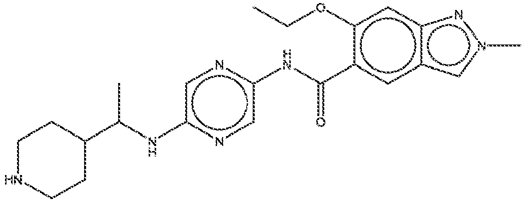 | 424.2 |
| 357 | 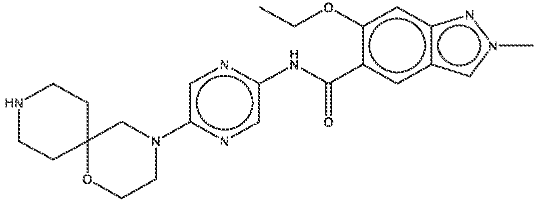 | 452.2 |
| 358 | 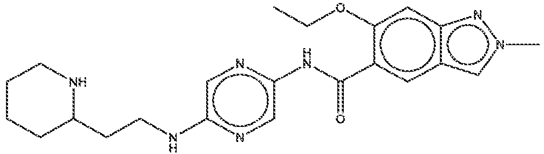 | 424.2 |
| 359 | 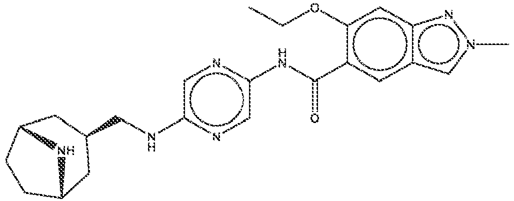 | 436.2 |
| 360 | 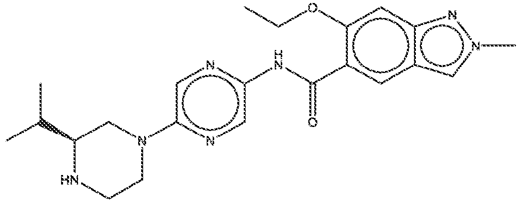 | 424.2 |
| 361 | 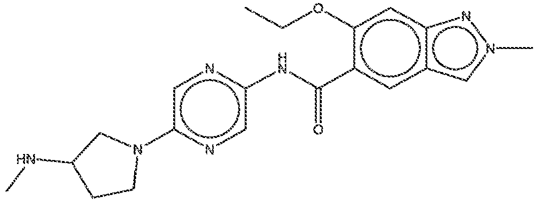 | 396.2 |
| 362 | 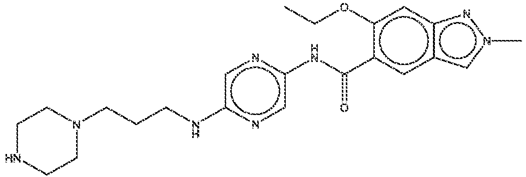 | 439.2 |
| 363 | 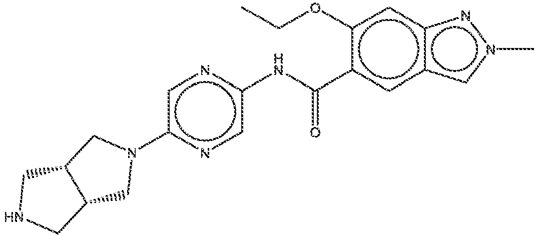 | 408.2 |
| 364 | 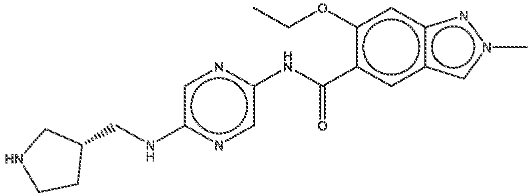 | 396.2 |
| 365 | 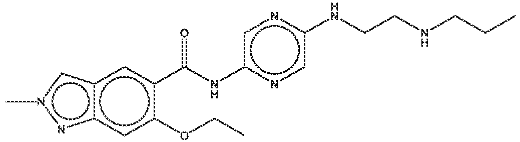 | 398.2 |
| 366 | 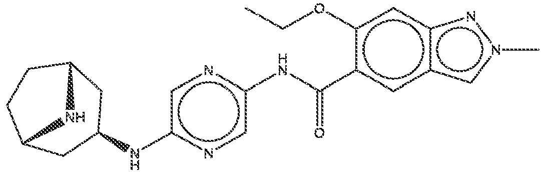 | 422.2 |
| 367 | 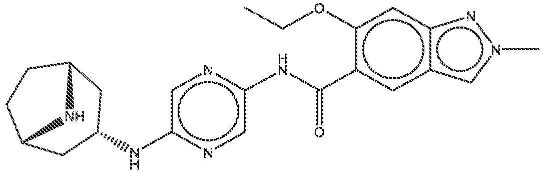 | 422.2 |
| 368 | 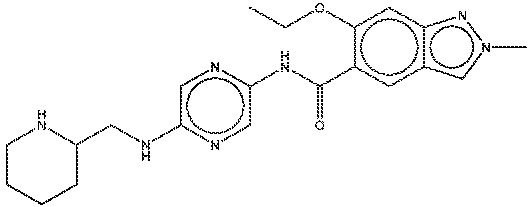 | 410.2 |
| 369 | 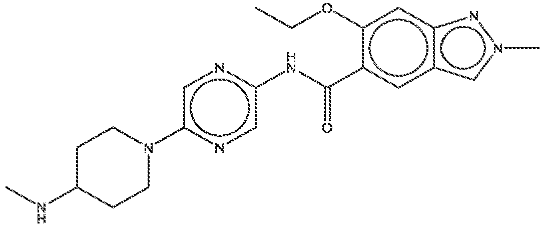 | 410.2 |
| 370 | 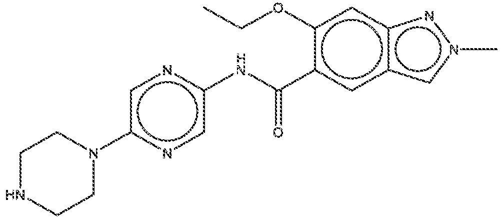 | 382.2 |
| 371 | 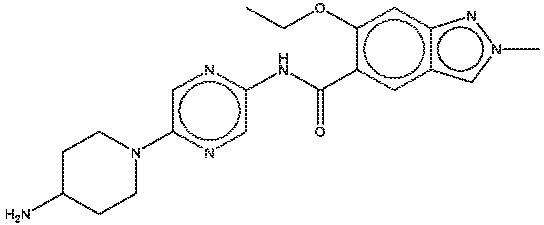 | 396.2 |
| 372 | 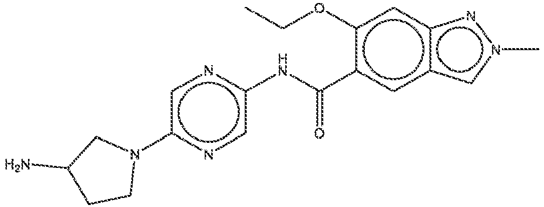 | 382.2 |
| 373 | 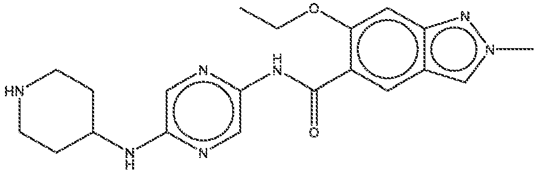 | 396.2 |
| 374 | 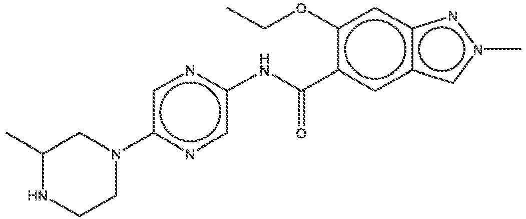 | 396.2 |
| 375 | 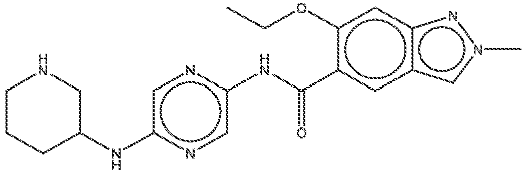 | 396 |
| 376 | 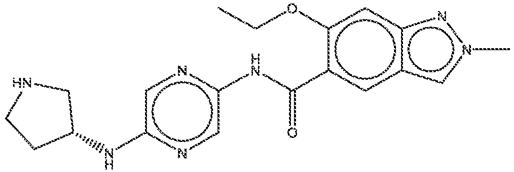 | 382.2 |
| 377 | 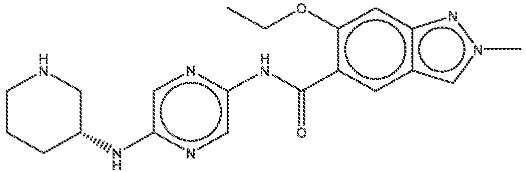 | 396.2 |
| 378 | 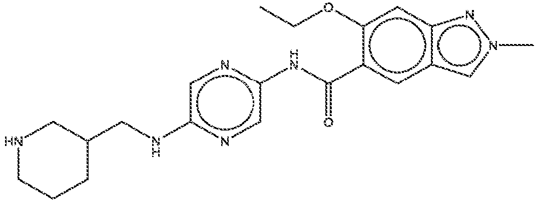 | 410.2 |

### Example 80. Preparation of N-(2-bromopyrimidin-5-yl)-6-ethoxy-2-methyl-2H-indazole-5-carboxamide

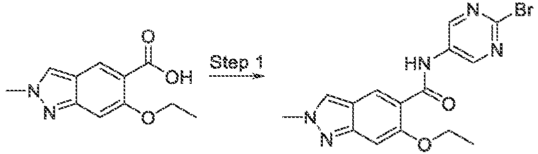

### Step 1: Preparation of N-(2-bromopyrimidin-5-yl)-6-ethoxy-2-methyl-2H-indazole-5-carboxamide

6-Ethoxy-2-methyl-2H-indazole-5-carboxylic acid (3.38 g, 15.35 mmol) was suspended in DMF (30 mL) and DIPEA (3.97 g, 30.7 mmol) was added followed by DIPEA (7.0 g, 18.42 mmol). The resulting mixture was stirred for 30 min and 2-bromopyrimidin-5-amine (2.67 g, 15.35 mmol) was added in one portion and the reaction mixture was stirred overnight at rt. The precipitate formed was filtered, washed with MeCN (60 mL), MTBE (60 mL), dried in *vacuo* to give pure N-(2-bromopyrimidin-5-yl)-6-ethoxy-2-methyl-2H-indazole-5-carboxamide (1.57 g, 95.0% purity, 3.96 mmol, Y: 25.8%). ESI-MS (M+H)⁺: 376.2. ¹H NMR (400 MHz, DMSO-*d*₆) δ 10.37 (s, 1H), 9.02 (s, 2H), 8.36 (s, 1H), 8.16 (s, 1H), 7.08 (s, 1H), 4.22 (q, *J* = 6.7 Hz, 2H), 4.13 (s, 3H), 1.43 (t, *J =* 6.7 Hz, 3H).

### Example 81. General procedure for parallel synthesis:

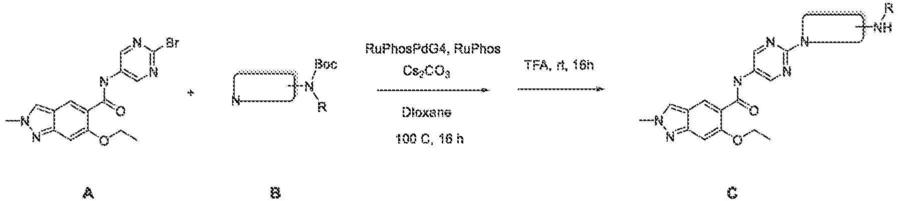

To a mixture of 1 eq. of ArBr **(A)** and 1.5 eq. Amine **(B)** in 1ml dry Dioxane under an inert atmosphere were added 0.05 eq. RuPhosPdG4, 0.05 eq. RuPhos and 2.5 eq. Cs₂CO₃. The reaction mixture was stirred at 100°C for 16 hours. After cooling to room temperature, the solvent was evaporated and 1 ml TFA was added and stirred at room temperature for 4h. The mixture was evaporated. The residue was dissolved in DMSO (appr. 1 ml), treated with scavenger SiliaMetS DMT and filtered.
Resulting solution was purified by HPLC (Deionized Water (phase **A)** and HPLC-grade Acetonitrile (phase B) were used as an eluent to obtain final compound **(C).** In most cases, TFA was used as an additive.
Instrument: Agilent 1260 Infinity systems equipped with DAD and mass-detector
Column: Waters Sunfire C18 OBD Prep Column, 100 A, 5 µm, 19 mm x 100mm with SunFire C18 Prep Guard Cartridge, 100 A, 10 µm, 19 mm x 10 mm.

**Table 5. Compounds Synthesized using the procedure of Example 81.**

| **Compound no.** | **Compound** | **ESI-MS (M+H)⁺** |
|---|---|---|
| 379 | 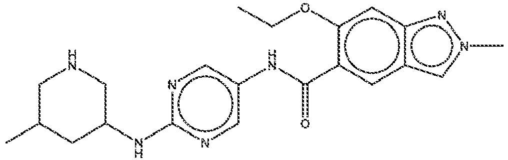 | 410.2 |
| 380 | 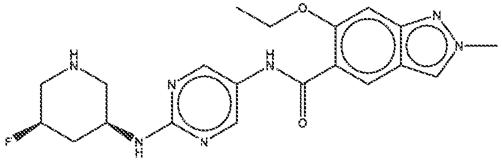 | 414.0 |
| 381 | 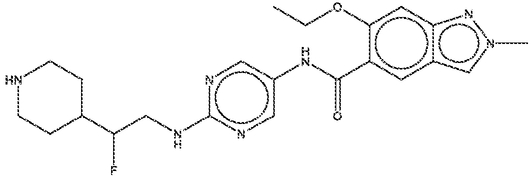 | 442.0 |
| 382 | 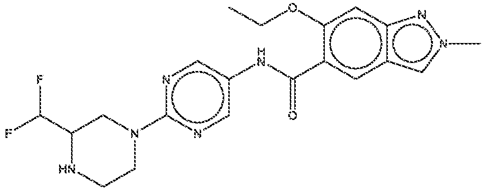 | 432.0 |
| 383 | 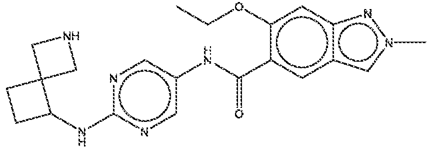 | 408.2 |
| 384 | 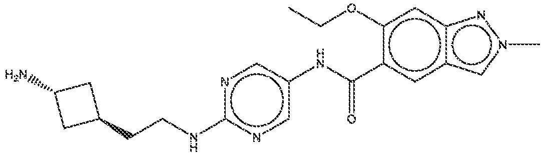 | 410.2 |
| 385 | 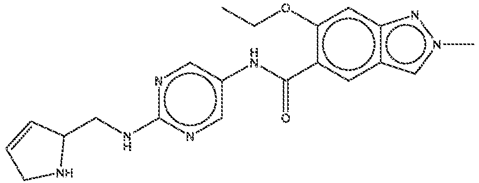 | 394.2 |
| 386 | 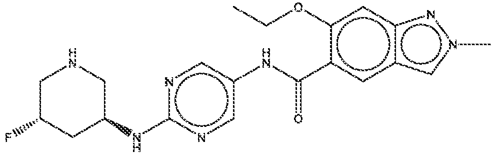 | 414.2 |
| 387 | 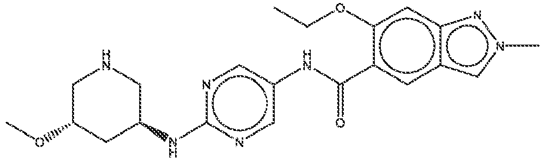 | 426.2 |
| 388 | 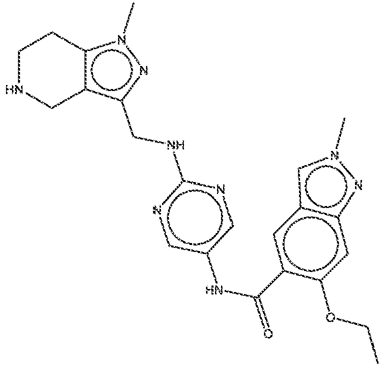 | 462.2 |
| 389 | 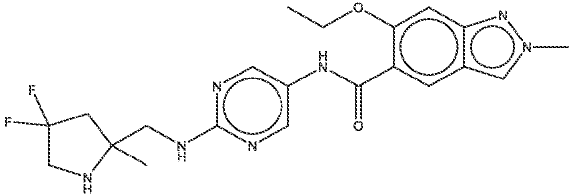 | 446.2 |
| 390 | 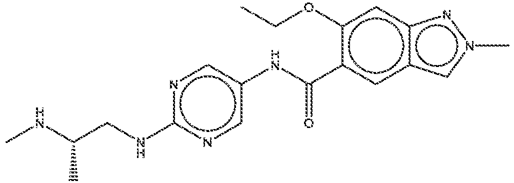 | 384.2 |
| 391 | 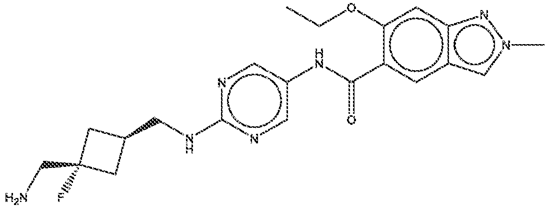 | 428.2 |
| 392 | 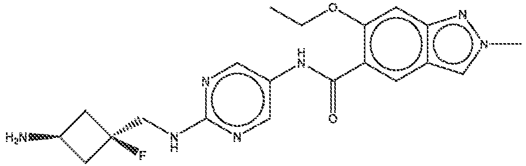 | 414.0 |
| 393 | 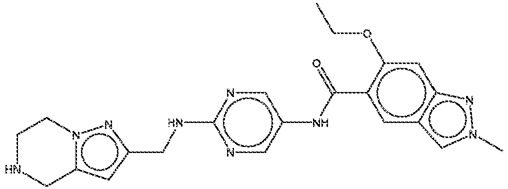 | 448.2 |
| 394 | 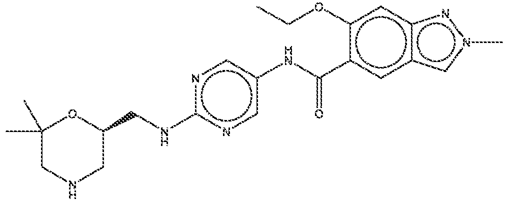 | 440.2 |
| 395 | 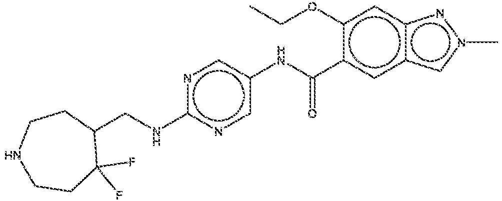 | 460.2 |
| 396 | 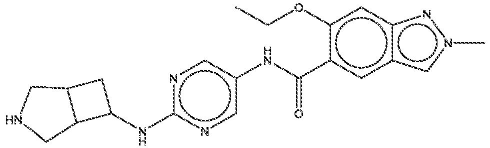 | 408.2 |
| 397 | 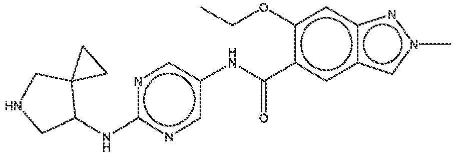 | 408.2 |
| 398 | 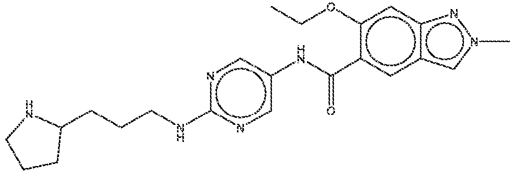 | 424.2 |
| 399 | 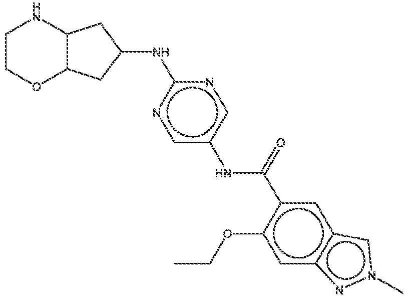 | 438.0 |
| 400 | 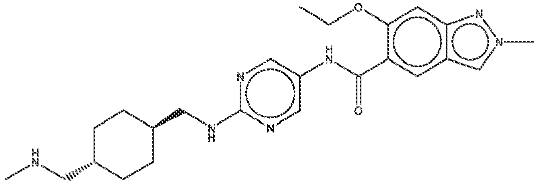 | 452.2 |
| 401 | 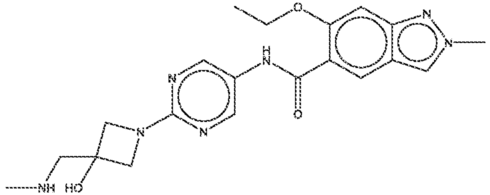 | 412.2 |
| 402 | 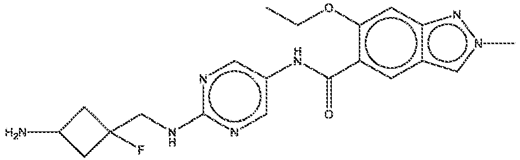 | 414.0 |
| 403 | 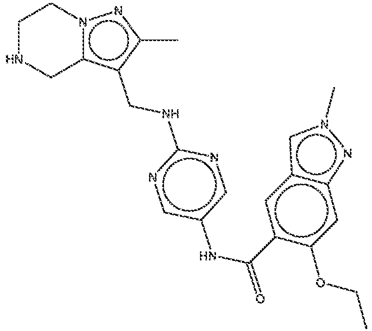 | 462.4 |
| 404 | 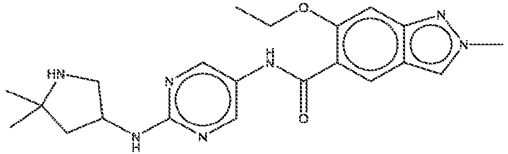 | 410.2 |
| 405 | 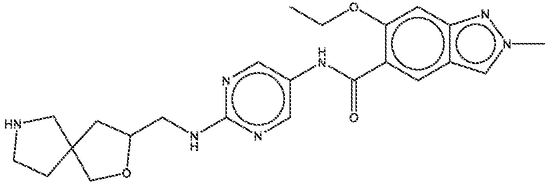 | 452.2 |
| 406 | 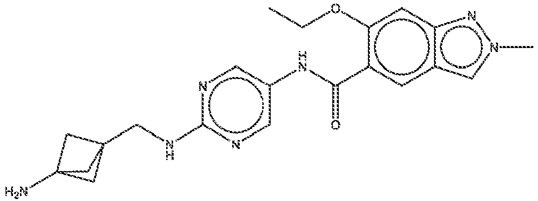 | 408.2 |
| 407 | 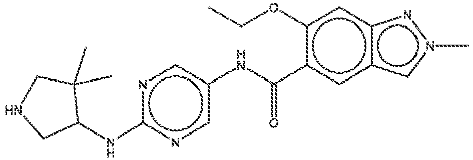 | 410.4 |
| 408 | 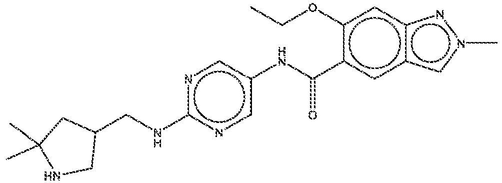 | 424.2 |
| 409 | 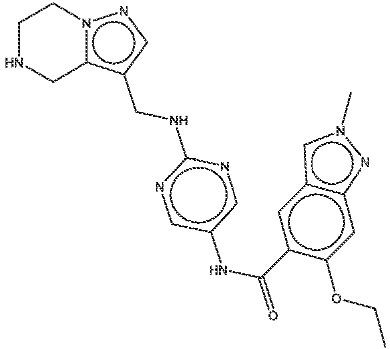 | 448.2 |
| 410 | 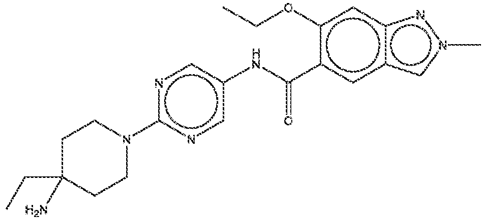 | 424.2 |
| 411 | 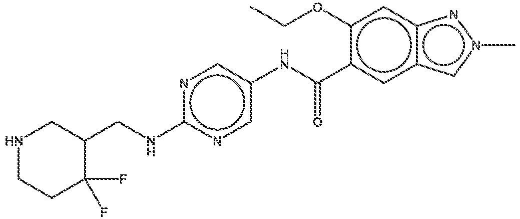 | 446.2 |
| 412 | 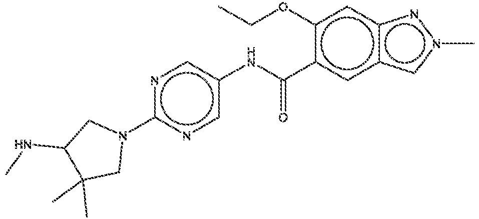 | 424.2 |
| 413 | 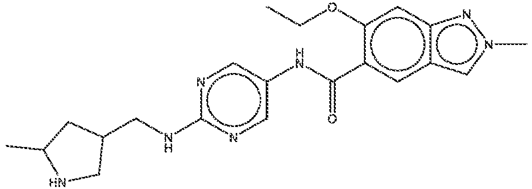 | 410.2 |
| 414 | 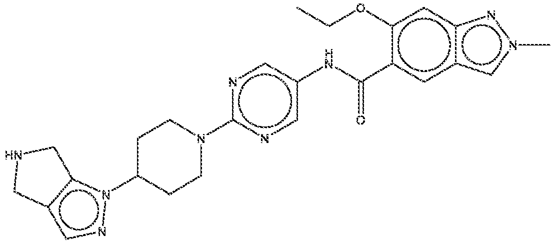 | 488.2 |
| 415 | 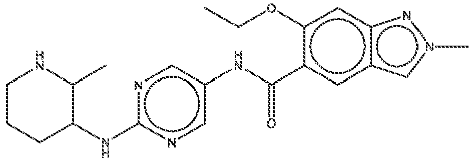 | 410.2 |
| 416 | 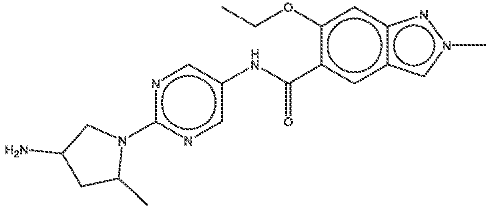 | 396.2 |
| 417 | 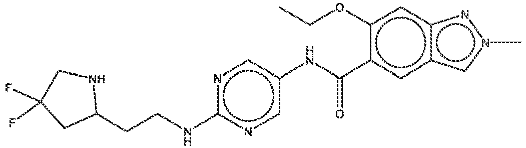 | 446.2 |
| 418 | 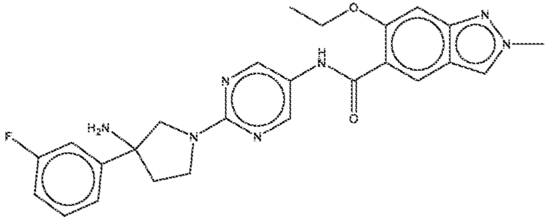 | 476.0 |
| 419 | 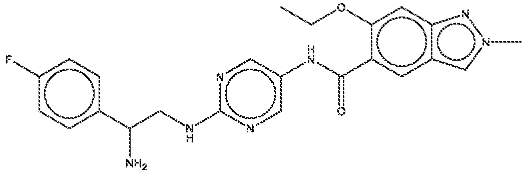 | 450.0 |
| 420 | 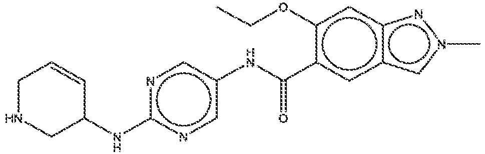 | 394.0 |
| 421 | 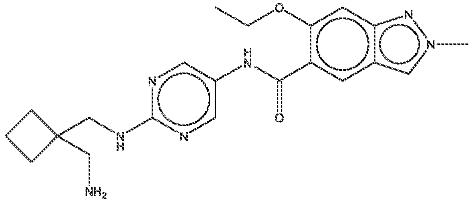 | 410.2 |
| 422 | 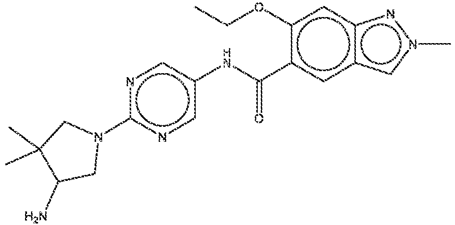 | 410.2 |
| 423 | 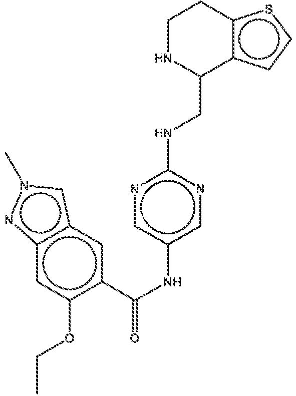 | 464.2 |
| 424 | 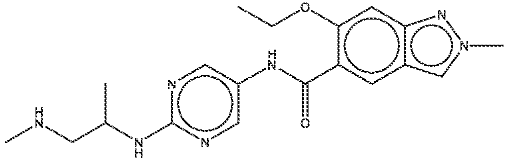 | 384.2 |
| 425 | 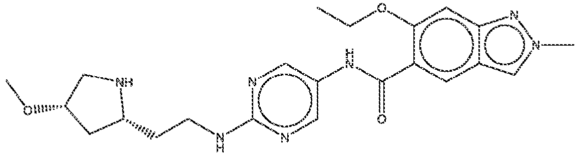 | 440.2 |
| 426 | 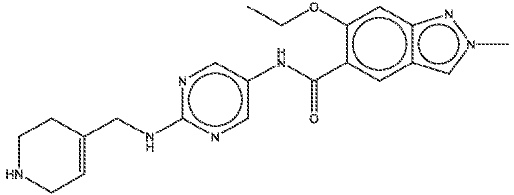 | 408.2 |
| 427 | 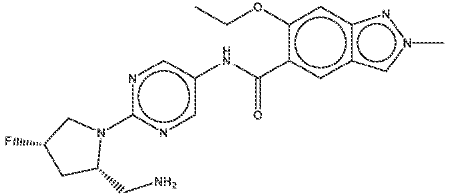 | 414.0 |
| 428 | 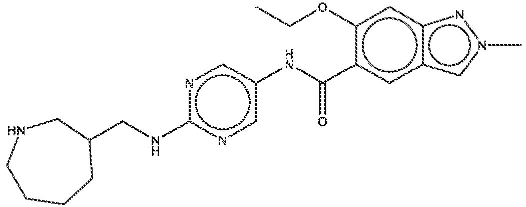 | 424.0 |
| 429 | 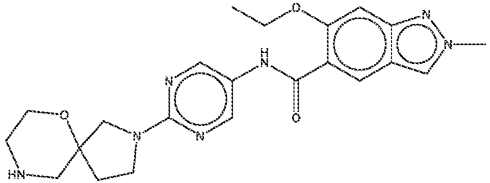 | 438.2 |
| 430 | 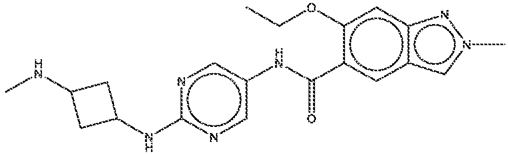 | 396.0 |
| 431 | 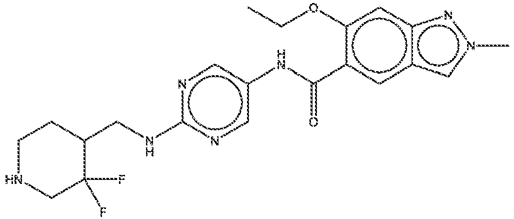 | 446.0 |
| 432 | 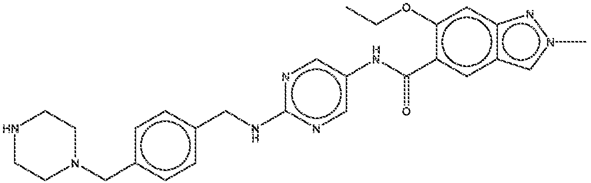 | 502.2 |
| 433 | 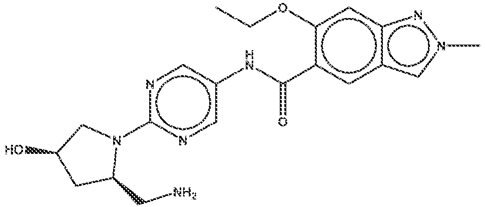 | 412.2 |
| 434 | 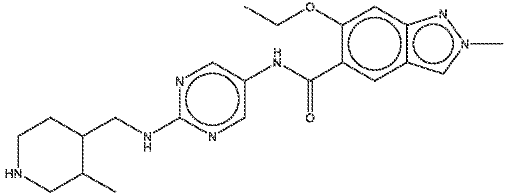 | 424.2 |
| 435 | 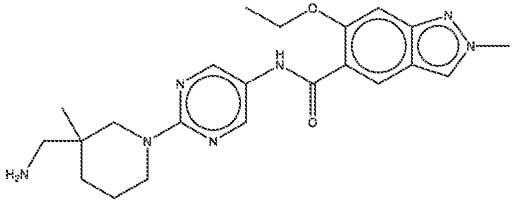 | 424.2 |
| 436 | 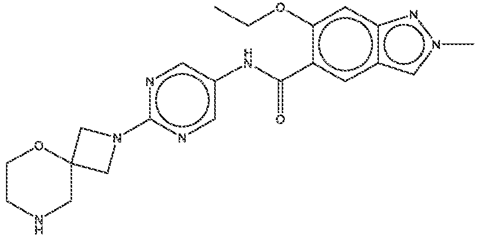 | 424.0 |
| 437 | 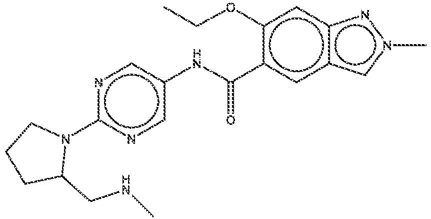 | 410.0 |
| 438 | 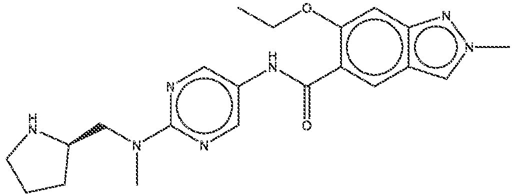 | 410.2 |
| 439 | 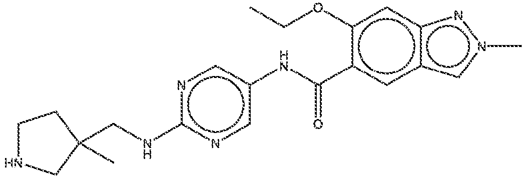 | 410.2 |
| 440 | 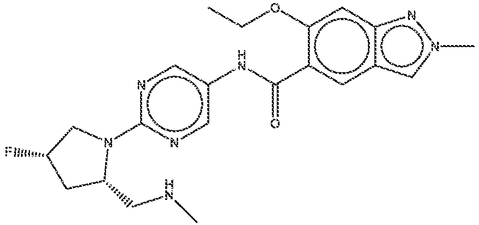 | 428.2 |
| 441 | 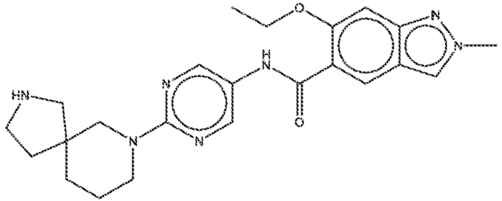 | 436.2 |
| 442 | 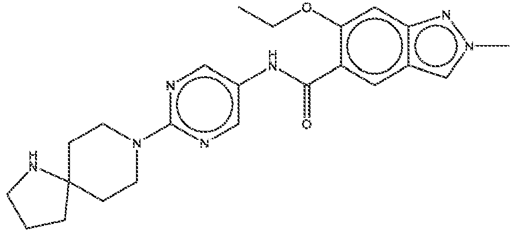 | 436.2 |
| 443 | 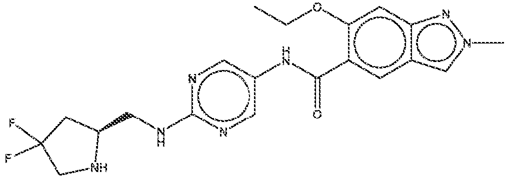 | 432.0 |
| 444 | 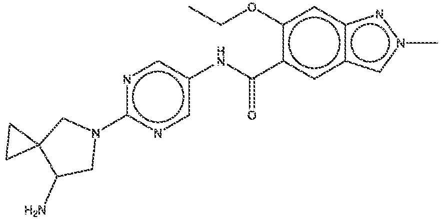 | 408.2 |
| 445 | 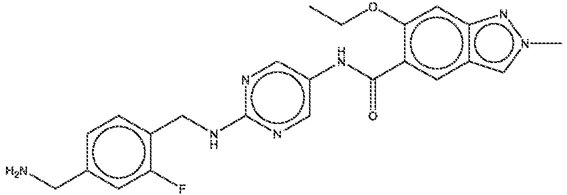 | 450.0 |
| 446 | 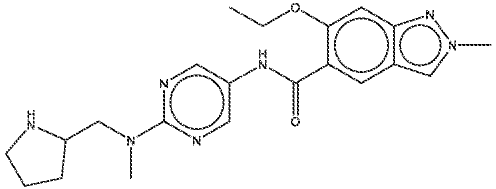 | 410.2 |
| 447 | 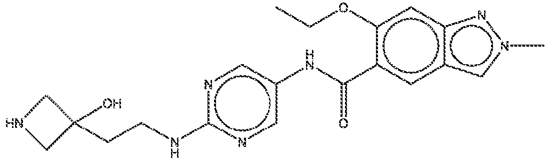 | 412.2 |
| 448 | 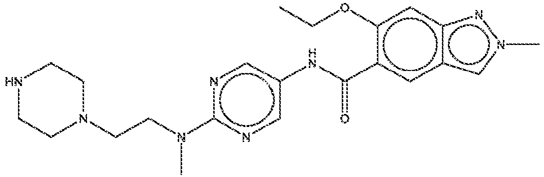 | 439.2 |
| 449 | 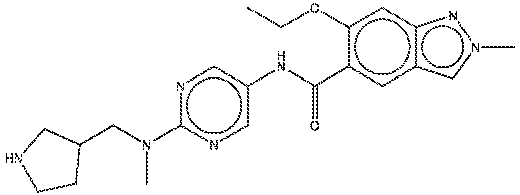 | 410.4 |
| 450 | 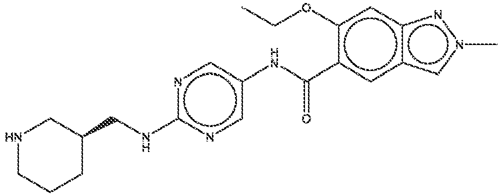 | 410.2 |
| 451 | 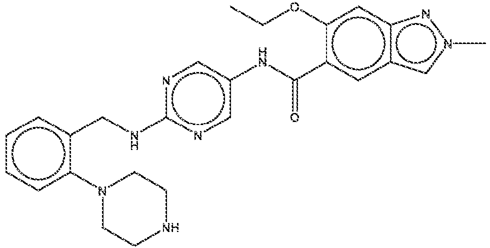 | 487.2 |
| 452 | 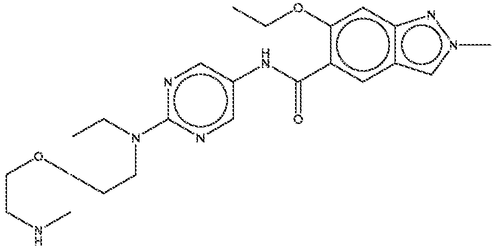 | 452.2 |
| 453 | 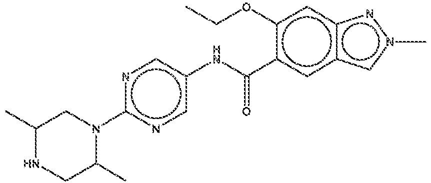 | 410.2 |
| 454 | 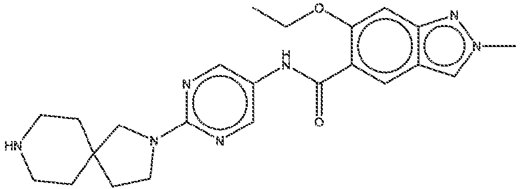 | 436.2 |
| 455 | 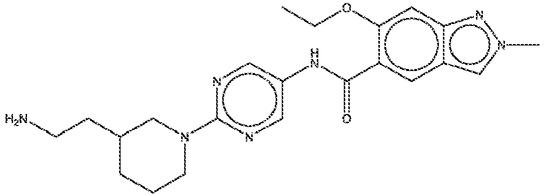 | 424.2 |
| 456 | 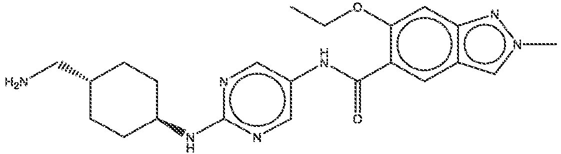 | 424.0 |
| 457 | 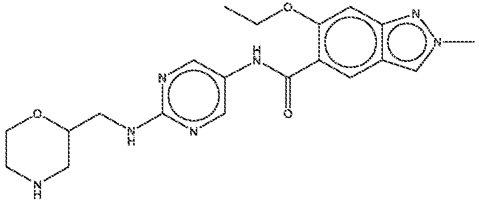 | 412.2 |
| 458 | 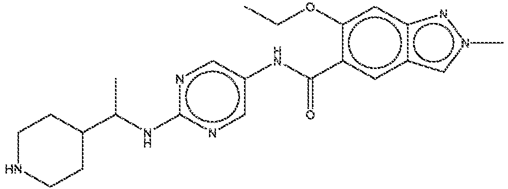 | 424.2 |
| 459 | 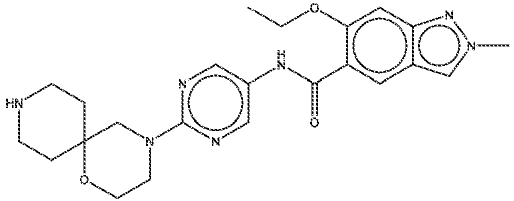 | 452.2 |
| 460 | 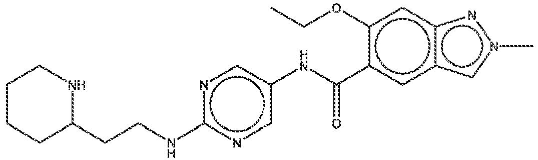 | 424.2 |
| 461 | 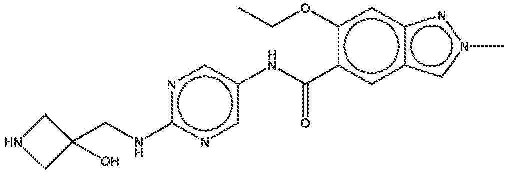 | 398.0 |
| 462 | 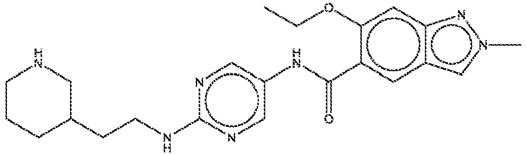 | 424.2 |
| 463 | 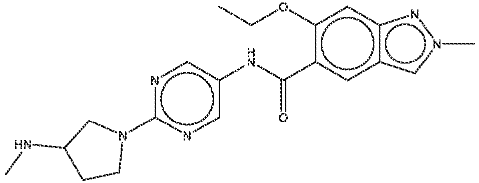 | 396.0 |
| 464 | 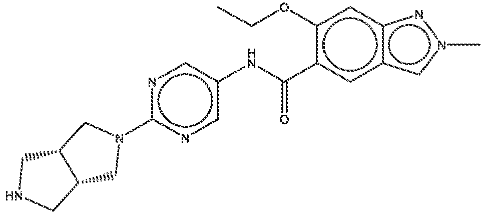 | 408.2 |
| 465 | 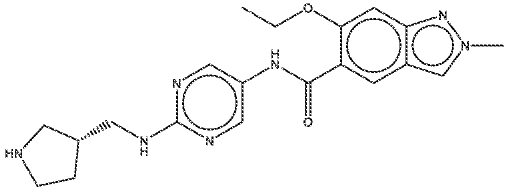 | 396.2 |
| 466 | 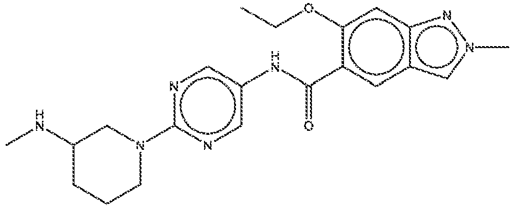 | 410.2 |
| 467 | 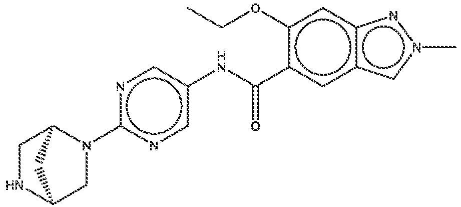 | 394.2 |
| 468 | 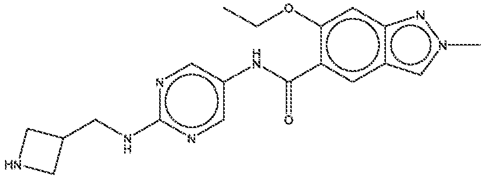 | 382.2 |
| 469 | 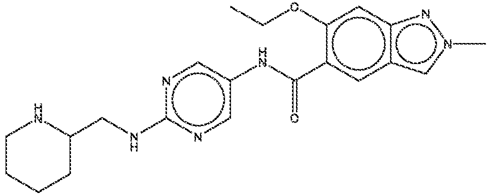 | 410.2 |
| 470 | 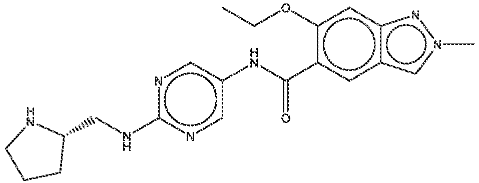 | 396.2 |
| 471 | 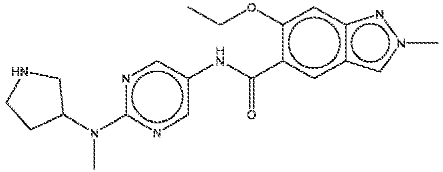 | 396.2 |
| 472 | 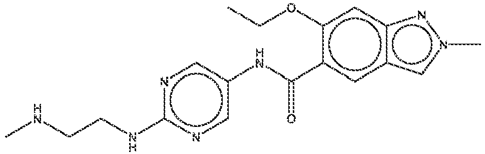 | 370.2 |
| 473 | 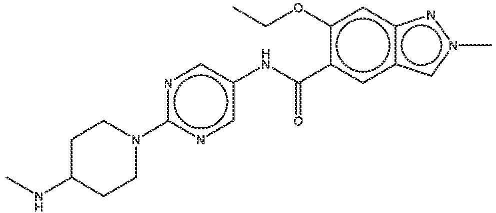 | 410.4 |
| 474 | 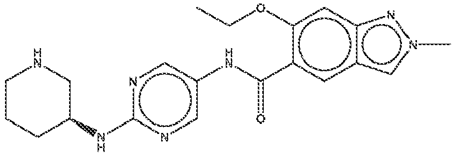 | 396.0 |
| 475 | 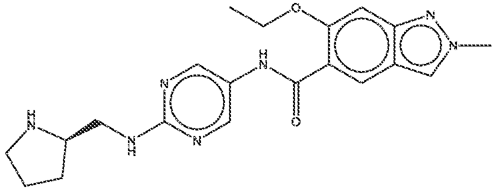 | 396.2 |
| 476 | 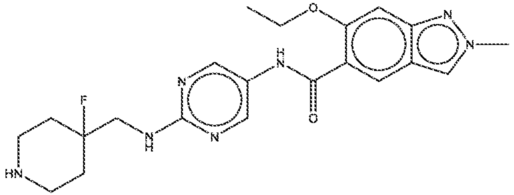 | 428.0 |
| 477 | 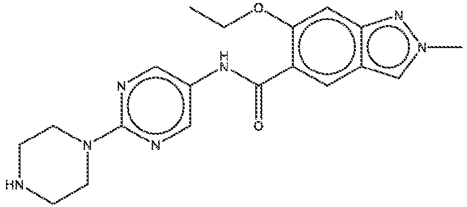 | 382.2 |
| 478 | 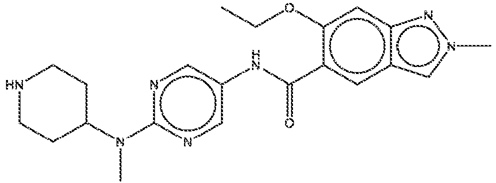 | 410.2 |
| 479 | 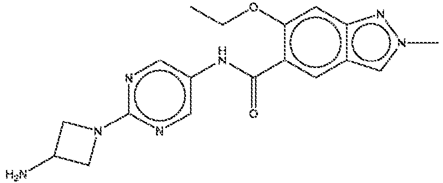 | 368.0 |
| 480 | 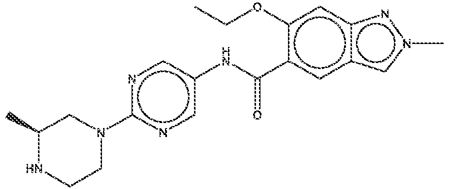 | 396.2 |
| 481 | 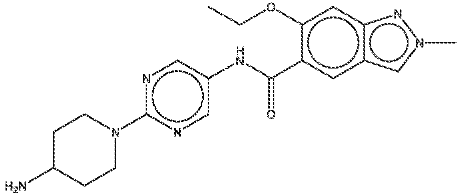 | 396.2 |
| 482 | 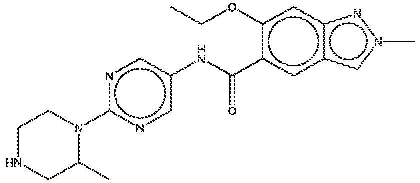 | 396.2 |
| 483 | 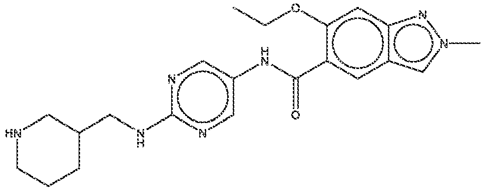 | 410.2 |
| 484 | 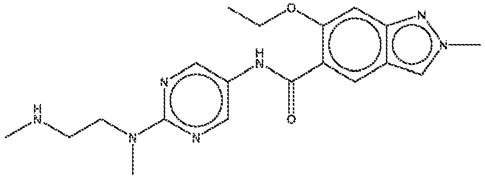 | 384.2 |
| 485 | 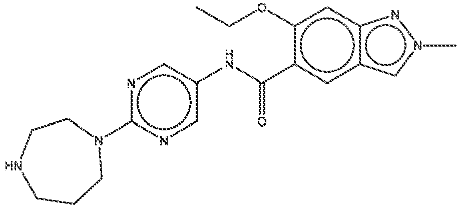 | 396.1 |
| 486 | 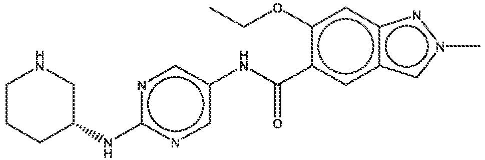 | 396.2 |
| 487 | 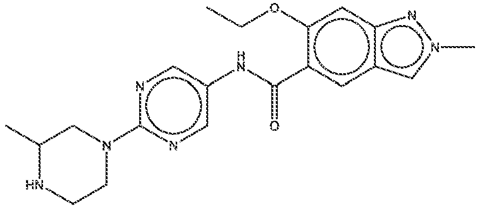 | 396.0 |

### Example 82. Preparation of N-(5-bromopyrazin-2-yl)-6-ethoxy-2-methylpyrazolo[1,5-a]pyridine-5-carboxamide

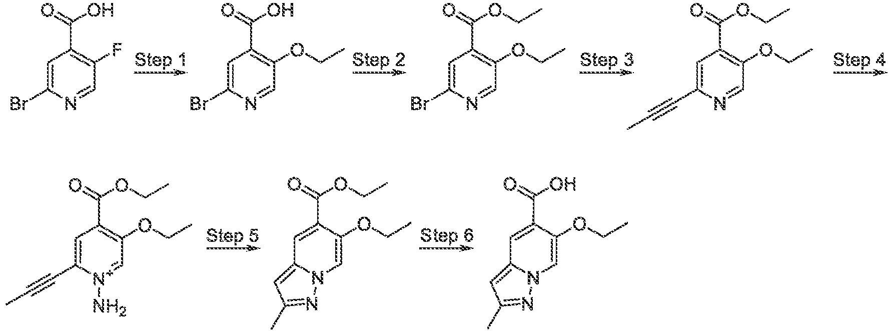

### Step 1: Preparation of 2-bromo-5-ethoxypyridine-4-carboxylic acid

A solution of 2-bromo-5-fluoropyridine-4-carboxylic acid (30.0 g, 136.37 mmol) in EtOH (100 mL) was added to a freshly prepared EtONa from sodium (9.41 g, 409.1 mmol) and EtOH (300 mL). The reaction mixture was heated at 60 °C for 24 hours then cooled to room temperature and the solvent was removed in *vacuo* to give crude 2-bromo-5-ethoxypyridine-4-carboxylic acid (32.0 g, 95.0% purity, 123.55 mmol, Y: 90.6%) as sodium salt. ¹H NMR (400 MHz, DMSO-*d*₆) δ 8.00 (s, 1H), 7.24 (s, 1H), 4.08 (q, *J =* 7.0 Hz, 2H), 1.27 (t, *J* = 6.9 Hz, 3H).

### Step 2: Preparation of ethyl 2-bromo-5-ethoxypyridine-4-carboxylate

To 2-bromo-5-ethoxypyridine-4-carboxylic acid (32.0 g, 130.05 mmol) in EtOH (400 mL) thionyl chloride (46.42 g, 390.15 mmol, 28.3 mL) was added dropwise and the reaction mixture was stirred overnight at 60 °C. After that the solvent was evaporated and the residue was partitioned between DCM (300 mL) and water (200 mL), and the aqueous phase was extracted with DCM (200 mL). The combined organic extracts were washed with a saturated aqueous NaHCO₃ solution (100 mL), brine (100 mL), dried (Na₂SO₄), filtered and concentrated under reduced pressure to give ethyl 2-bromo-5-ethoxypyridine-4-carboxylate (32.0 g, 90.0% purity, 105.07 mmol, Y: 80.8%). ¹H NMR (500 MHz, DMSO-*d*₆) δ 8.34 (s, 1H), 7.71 (s, 1H), 4.29 (q, *J =* 7.0 Hz, 2H), 4.19 (q, *J =* 7.0 Hz, 2H), 1.35 - 1.21(m, 6H).

### Step 3: Preparation of ethyl 5-ethoxy-2-(prop-1 -yn-1-yl)pyridine-4-carboxylate

A 500 mL four-necked flask was charged with ethyl 2-bromo-5-ethoxypyridine-4-carboxylate (32.0 g, 116.74 mmol), 1-(trimethylsilyl)-1-propyne (15.72 g, 140.09 mmol), copper(I) iodide (2.22 g, 11.67 mmol), and tetrabutyl ammonium fluoride (61.05 g, 233.48 mmol) in THF (150 mL) and triethylamine (150 mL). After the solution was degassed and purged with argon, Pd(dppf)Cl₂ (4.1 g, 5.84 mmol) was added to the reaction flask. The reaction mixture was stirred at 40 °C for 15 h under an argon atmosphere. After that the solvent was evaporated under reduced pressure and the residue was partitioned between ethyl acetate (300 mL) and H₂O (150 mL). The organic phase was washed with water (50 mL), dried with anhydrous magnesium sulfate, filtered, concentrated, and purified by FCC to give crude ethyl 5-ethoxy-2-(prop-1-yn-1-yl)-pyridine-4-carboxylate (18.17 g, 95.0% purity, 74.0 mmol, Y: 63.4%). ESI-MS (M+H)⁺: 234.0. ¹H NMR (400 MHz, DMSO-*d*₆) δ 8.47 (s, 1H), 7.54 (s, 1H), 4.35 - 4.18 (m, 4H), 2.04 (s, 3H), 1.37 - 1.24 (m, 6H).

### Step 4: Preparation of 1-amino-5-ethoxy-4-(ethoxycarbonyl)-2-(prop-1-yn-1-yl)pyridin-1-ium

Amino 2,4,6-trimethylbenzene-1-sulfonate (13.37 g, 62.11 mmol) was dissolved in dry DCM (200 mL) and cooled under argon atmosphere to 0 °C, then ethyl 5-ethoxy-2-(prop-1-yn-1-yl)pyridine-4-carboxylate (13.17 g, 56.46 mmol) was added dropwise over 1 min. The reaction mixture was slowly (without removal of the cooling bath) warmed to RT (about 3 h) and stirred at RT overnight (18 h) under an argon atmosphere. The solvent was evaporated to give crude 1-amino-5-ethoxy-4-(methoxycarbonyl)-2-(prop-1-yn-1-yl)pyridin-1-ium 2,4,6-trimethylbenzene-1-sulfonate (25 g, 55.74 mmol) which was used in the next step without purification. ESI-MS (M)⁺: 249.2.

### Step 5: Preparation of ethyl 6-ethoxy-2-methylpyrazolo[1,5-a]pyridine-5-carboxylate

1-Amino-5-ethoxy-4-(methoxycarbonyl)-2-(prop-1-yn-1-yl)pyridin-1-ium 2,4,6-trimethylbenzene-1-sulfonate (25.0 g, 55.74 mmol) and potassium carbonate (23.11 g, 167.21 mmol) were mixed in anhydrous DMF (250 mL). The reaction mixture was stirred at ambient temperature for 3 days. A mixture of water (300 mL) and ethyl acetate (300 mL) was added and the organic layer was separated, washed with water (100 mL), brine (100 mL), dried over Na₂SO₄ and evaporated in *vacuo.* The residue was purified by FCC to give ethyl 6-ethoxy-2-methylpyrazolo[1,5-a]pyridine-5-carboxylate (10.1 g, 95.0% purity, 38.65 mmol, Y: 69.3%). ESI-MS (M+H)⁺: 249.2. ¹H NMR (500 MHz, DMSO-*d*₆) δ 8.35 (s, 1H), 7.90 (s, 1H), 6.47 (s, 1H), 4.27 (q, *J* = 7.1 Hz, 3H), 4.02 (q, *J =* 6.9 Hz, 2H), 2.35 (s, 3H), 1.35 - 1.25 (m, 6H).

### Step 6: Preparation of 6-ethoxy-2-methylpyrazalo[1,5-a]pyridine-5-carboxylic acid

A mixture of ethyl 6-ethoxy-2-methylpyrazolo[1,5-a]pyridine-5-carboxylate (14.0 g, 56.39 mmol) and sodium hydroxide (2.48 g, 62.03 mmol) was stirred in methanol (150 mL) and H₂O (50 mL) overnight. The reaction mixture was then concentrated under reduced pressure to remove methanol, and the resulting aqueous solution was neutralized with 1 N HCl to pH=5 to precipitate the carboxylic acid. Solid 6-ethoxy-2-methylpyrazolo[1,5-a]pyridine-5-carboxylic acid (7.0 g, 95.0% purity, 30.2 mmol, Y: 53.6%) was isolated by filtration, dried, and used directly in the next step without further purification. ESI-MS (M+H)⁺: 221.0. ¹H NMR(400 MHz, DMSO-*d*₆) δ 8.33 (s, 1H), 7.87 (s, 1H), 6.45 (s, 1H), 4.03 (q, *J* = 6.9 Hz, 2H), 2.35 (s, 3H), 1.32 (t, *J* = 6.9, 3H).

### Example 83. Preparation of N-(5-bromopyrazin-2-yl)-6-ethoxy-2-methylpyrazolo[1,5-a]pyridine-5-carboxamide

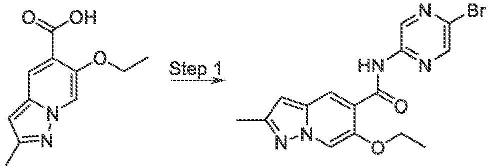

### Step 1: Preparation of N-(5-bromopyrazin-2-yl)-6-ethoxy-2-methylpyrazolo[1,5-a]pyridine-5-carboxamide

A mixture of 6-ethoxy-2-methylpyrazolo[1,5-a]pyridine-5-carboxylic acid (2.8 g, 12.71 mmol), 5-bromo-pyrazin-2-amine (2.21 g, 12.71 mmol), and pyridine (3.02 g, 38.14 mmol) was dissolved in anhydrous acetonitrile (40 mL). The mixture was stirred at room temperature for 5 minutes, followed by addition of T3P (16.18 g, 50.85 mmol, 50 wt % in EtOAc) over 10 minutes. The reaction mixture was stirred for 12 hours, then the resulting mixture was filtered and dried in *vacuo* to afford N-(5-bromopyrazin-2-yl)-6-ethoxy-2-methylpyrazolo[1,5-a]pyridine-5-carboxamide (3.83 g, 95.0% purity, 9.67 mmol, Y: 76.1%). ESI-MS (M+H)⁺: 376.2. ¹H NMR (400 MHz, Chloroform-*d*) δ 10.56 (s, 1H), 9.48 (s, 1H), 8.41 (s, 1H), 8.35 (s, 1H) 8.10 (s, 1H), 6.46 (s, 1H), 4.23 (q, *J =* 6.9 Hz, 2H), 2.47 (s, 3H), 1.65 (t, *J* = 6.9 Hz, 3H).

### Example 84. General procedure for parallel synthesis:

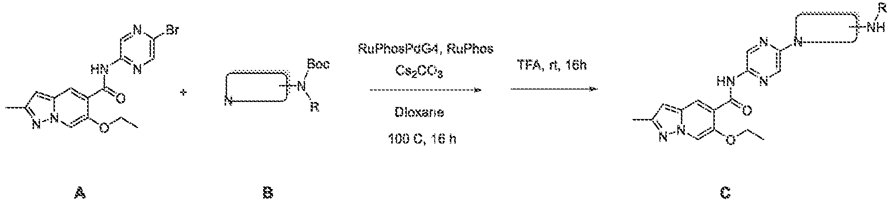

To a mixture of 1 eq. of ArBr (A) and 1.5 eq. Amine (B) in 1ml dry Dioxane under an inert atmosphere were added 0.05 eq. RuPhosPdG4, 0.05 eq. RuPhos and 2.5 eq. Cs₂CO₃. The reaction mixture was stirred at 100°C for 16 hours. After cooling to room temperature, the solvent was evaporated and 1 ml TFA was added and stirred at room temperature for 4h. The mixture was evaporated. The residue was dissolved in DMSO (appr. 1 ml), treated with scavenger SiliaMetS DMT and filtered.
Resulting solution was purified by HPLC (Deionized Water (phase A) and HPLC-grade Acetonitrile (phase B) were used as an eluent to obtain final compound (C). In most cases, TFA was used as an additive.
Instrument: Agilent 1260 Infinity systems equipped with DAD and mass-detector
Column: Waters Sunfire C18 OBD Prep Column, 100 A, 5 µm, 19 mm x 100mm with SunFire C18 Prep Guard Cartridge, 100 A, 10 µm, 19 mm x 10 mm.

**Table 6. Compounds synthesized using the procedure from Example 84**

| **Compound no.** | **Compound** | **ESI-MS (M+H)⁺** |
|---|---|---|
| 488 | 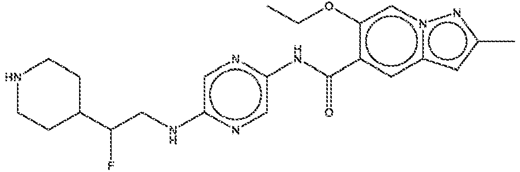 | 442.0 |
| 489 | 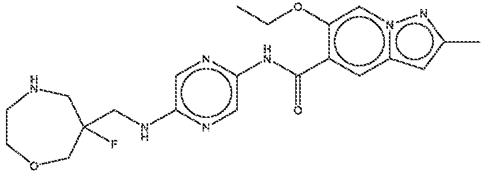 | 444.4 |
| 490 | 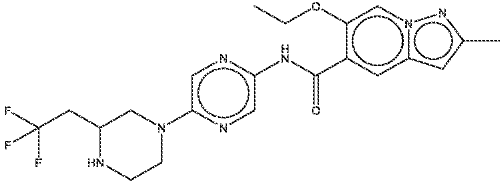 | 464.1 |
| 491 | 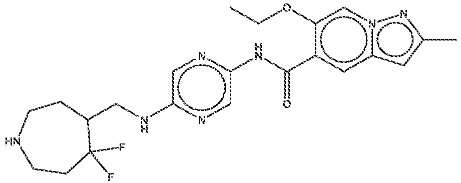 | 460.0 |
| 492 | 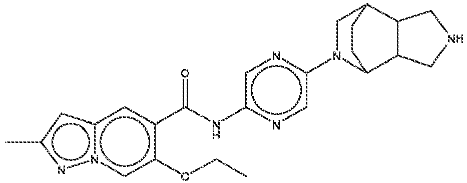 | 448.0 |
| 493 | 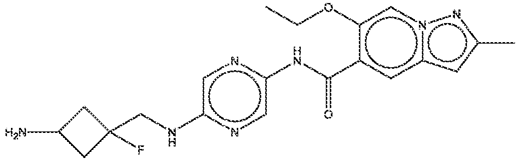 | 414.4 |
| 494 | 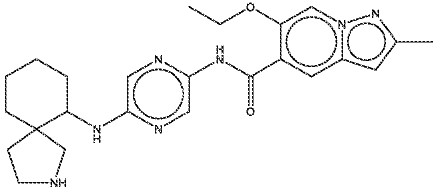 | 450.2 |
| 495 | 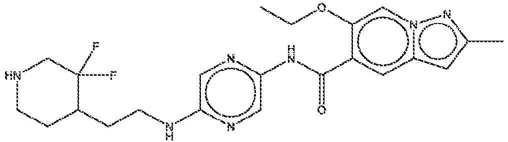 | 460.4 |
| 496 | 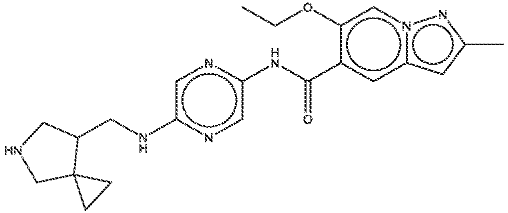 | 422.0 |
| 497 | 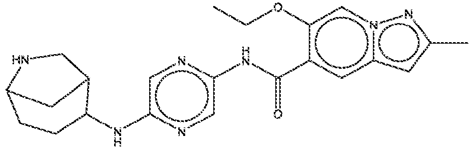 | 422.0 |
| 498 | 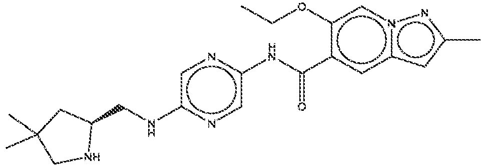 | 424.4 |
| 499 | 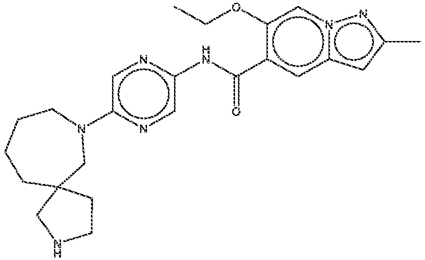 | 450.4 |
| 500 | 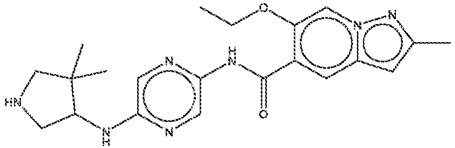 | 410.4 |
| 501 | 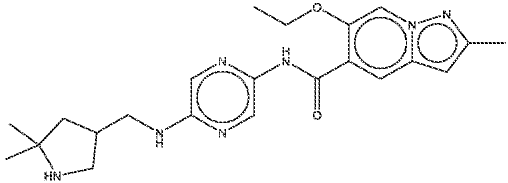 | 424.4 |
| 502 | 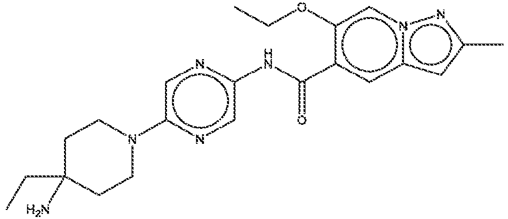 | 424.4 |
| 503 | 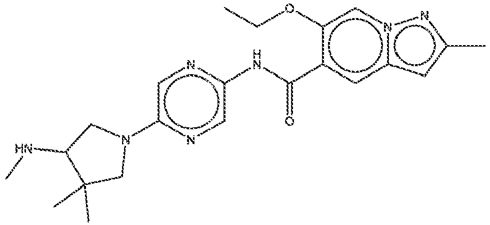 | 424.2 |
| 504 | 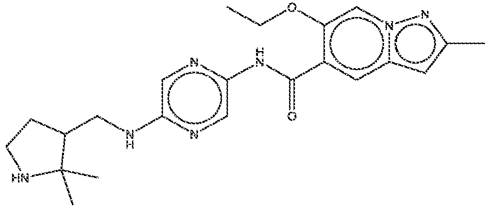 | 424.2 |
| 505 | 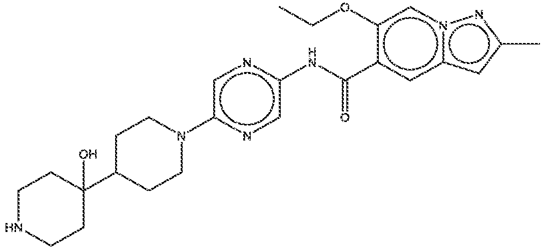 | 480.0 |
| 506 | 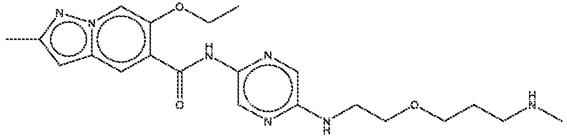 | 428.0 |
| 507 | 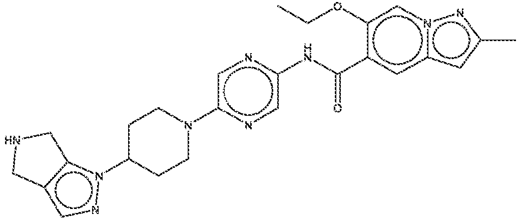 | 488.0 |
| 508 | 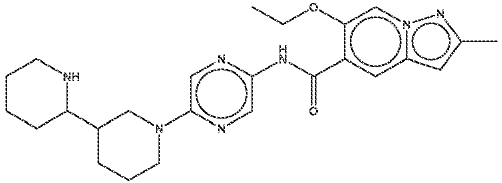 | 464.2 |
| 509 | 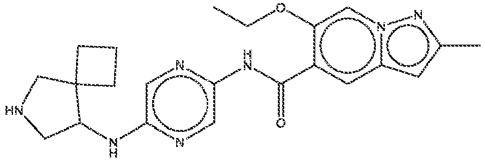 | 422.4 |
| 510 | 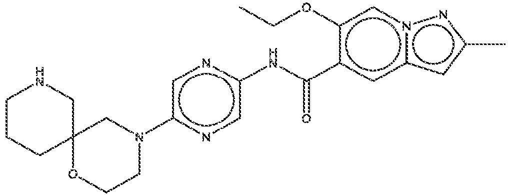 | 452.4 |
| 511 | 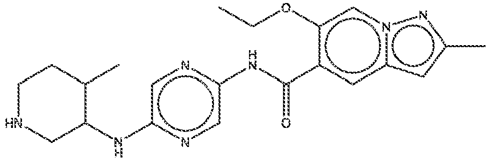 | 410.4 |
| 512 | 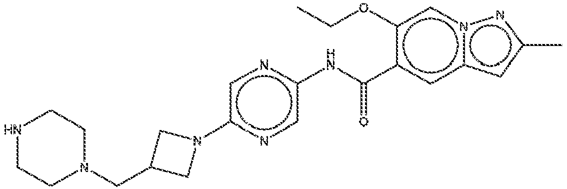 | 451.4 |
| 513 | 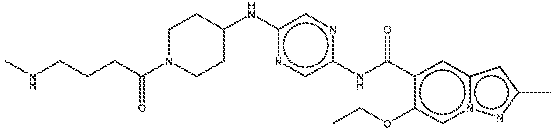 | 495.4 |
| 514 | 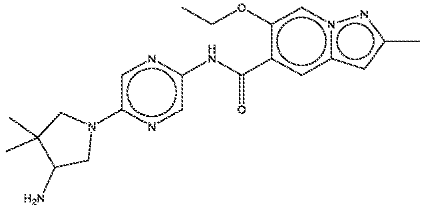 | 410.0 |
| 515 | 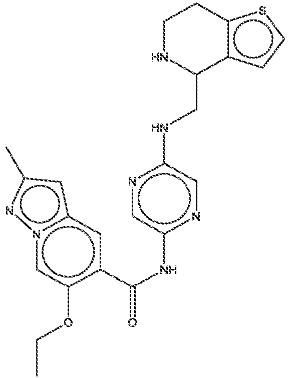 | 464.2 |
| 516 | 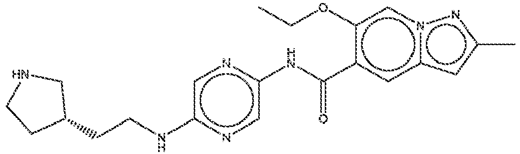 | 410.2 |
| 517 | 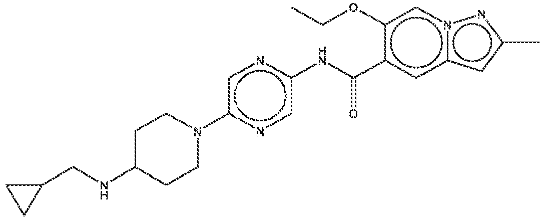 | 450.4 |
| 518 | 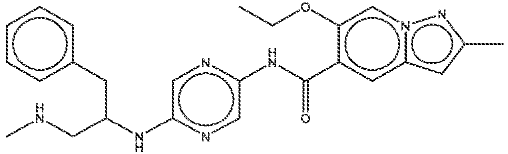 | 460.4 |
| 519 | 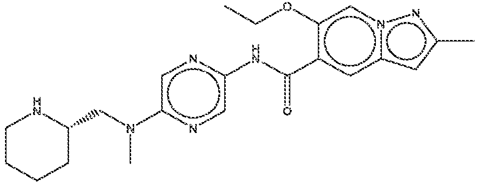 | 424.4 |
| 520 | 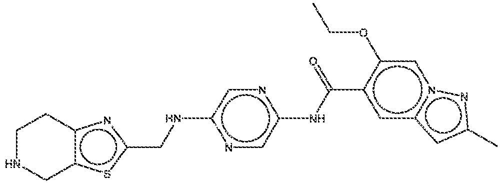 | 465.2 |
| 521 | 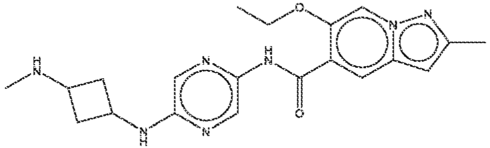 | 396.0 |
| 522 | 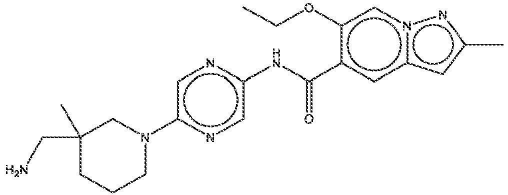 | 424.4 |
| 523 | 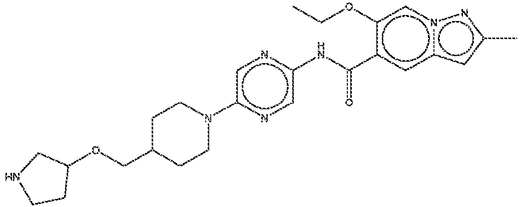 | 480.2 |
| 524 | 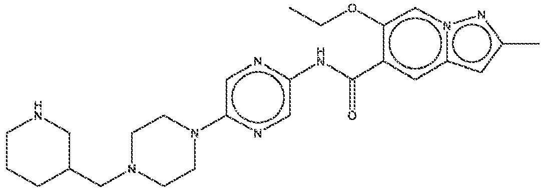 | 479.2 |
| 525 | 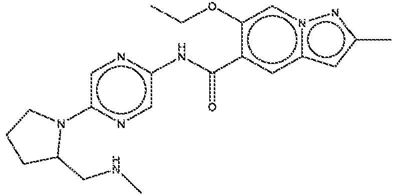 | 410.4 |
| 526 | 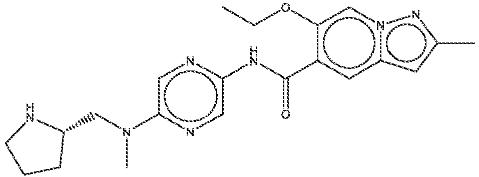 | 410.4 |
| 527 | 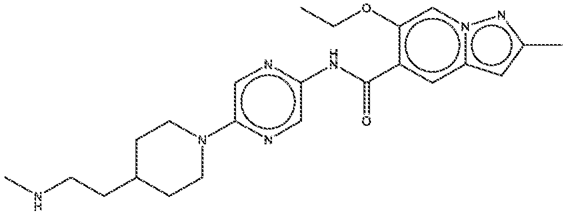 | 438.4 |
| 528 | 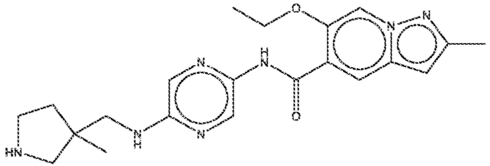 | 410.4 |
| 529 | 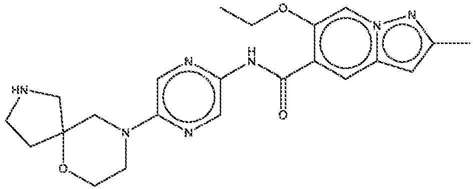 | 438.4 |
| 530 | 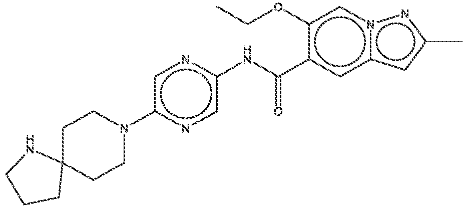 | 436.4 |
| 531 | 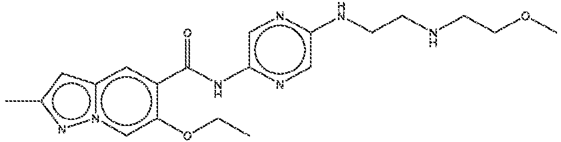 | 414.0 |
| 532 | 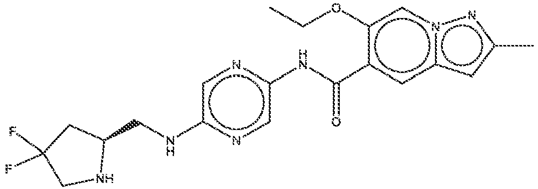 | 432.1 |
| 533 | 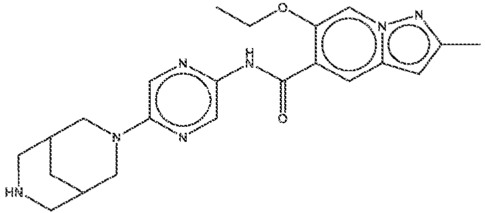 | 422.4 |
| 534 | 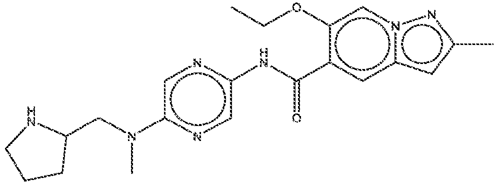 | 410.4 |
| 535 | 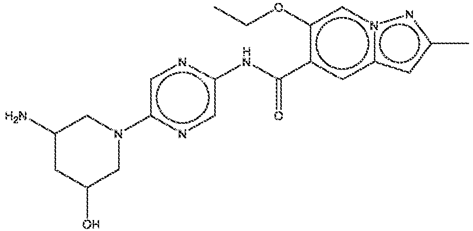 | 412.2 |
| 536 | 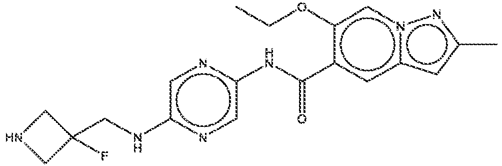 | 400.4 |
| 537 | 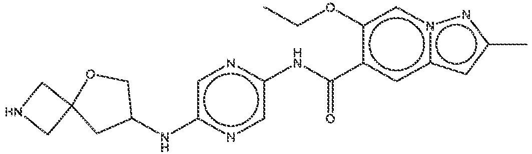 | 424.2 |
| 538 | 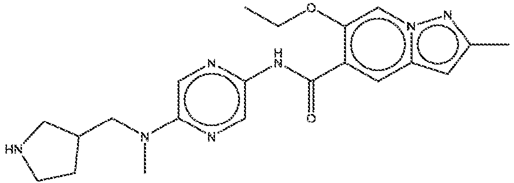 | 410.4 |
| 539 | 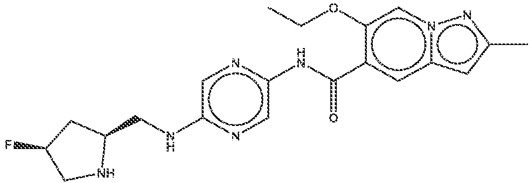 | 414.4 |
| 540 | 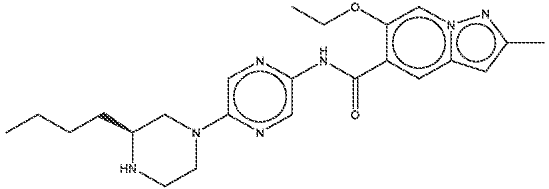 | 438.4 |
| 541 | 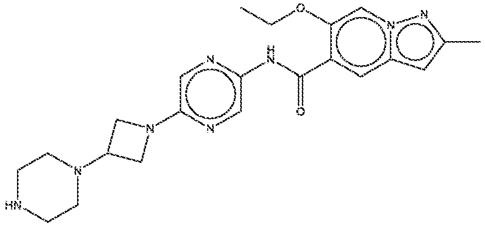 | 437.4 |
| 542 | 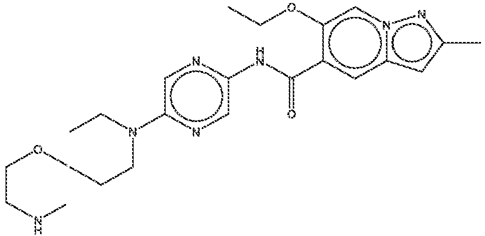 | 452.4 |
| 543 | 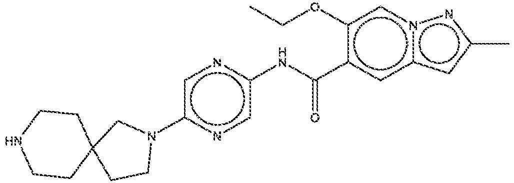 | 436.2 |
| 544 | 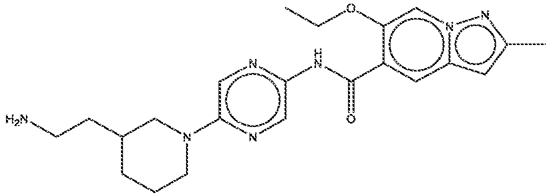 | 424.4 |
| 545 | 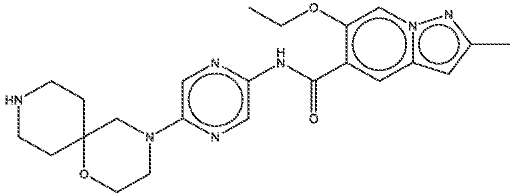 | 452.2 |
| 546 | 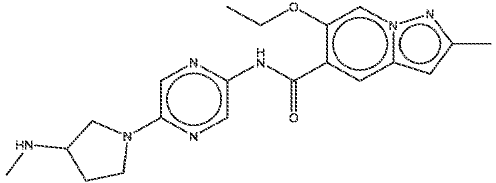 | 396.2 |
| 547 | 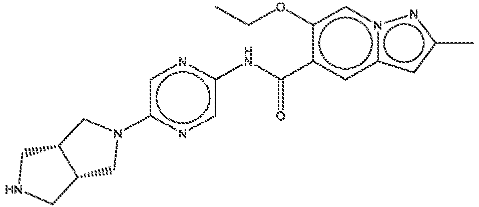 | 408.4 |
| 548 | 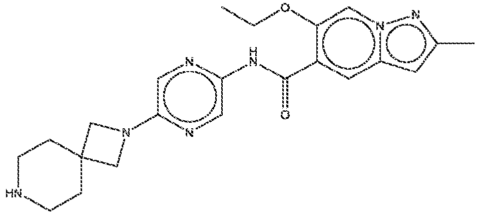 | 422.4 |
| 549 | 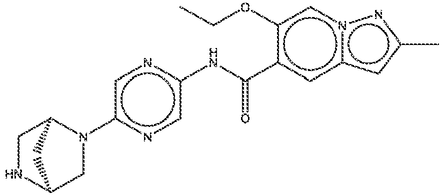 | 394.4 |
| 550 | 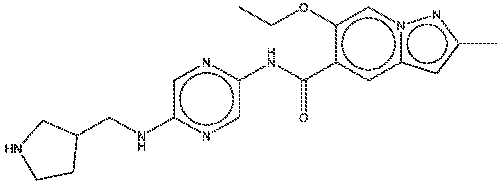 | 396.4 |
| 551 | 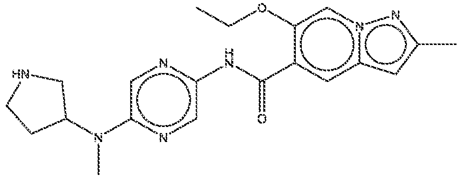 | 396.4 |
| 552 | 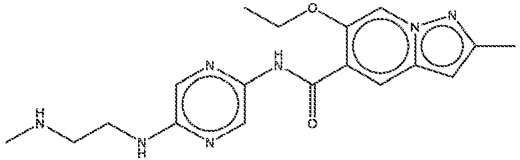 | 370.4 |
| 553 | 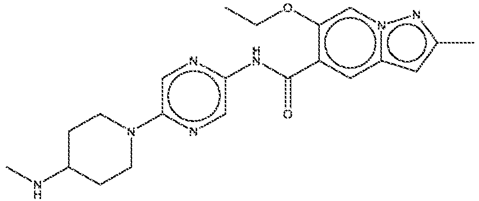 | 410.4 |
| 554 | 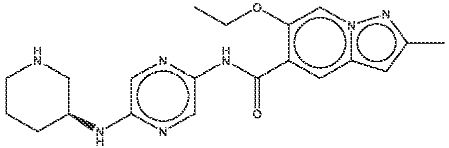 | 396.4 |
| 555 | 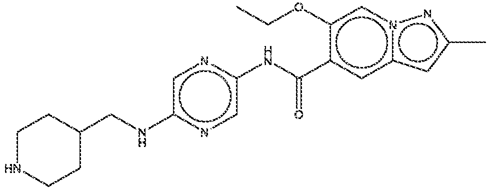 | 410.4 |
| 556 | 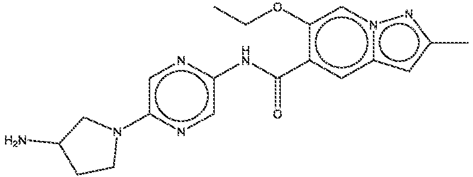 | 382.2 |
| 557 | 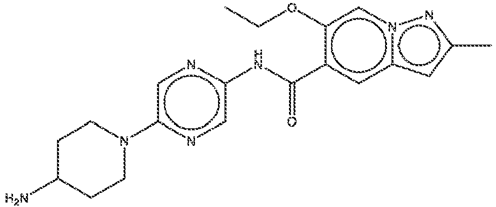 | 396.4 |
| 558 | 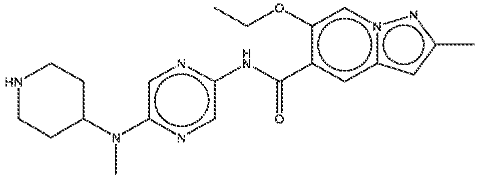 | 410.4 |
| 559 | 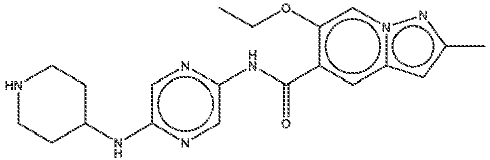 | 396.2 |
| 560 | 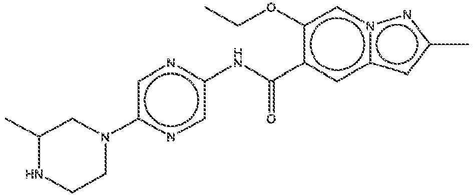 | 396.2 |
| 561 | 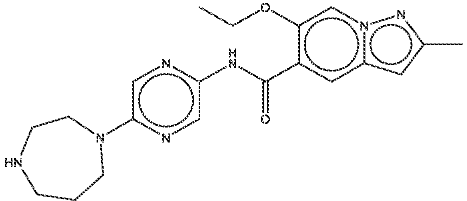 | 396.2 |

### Example 85. N-(2-bromopyrimidin-5-yl)-6-ethoxy-2-methylpyrazolo[1,5-a]pyridine-5-carboxamide

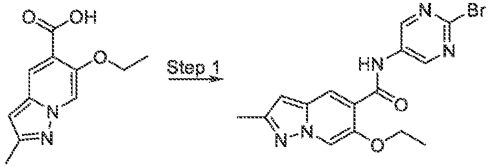

### Step 1: Preparation of N-(2-bromopyrimidin-5-yl)-6-ethoxy-2-methylpyrazolo[1,5-a]pyridine-5-carboxamide

6-Ethoxy-2-methylpyrazolo[1,5-a]pyridine-5-carboxylic acid (3.5 g, 15.89 mmol) was suspended in DMF (30 mL) and DIPEA (4.11 g, 31.79 mmol) was added followed by HATU (7.25 g, 19.07 mmol). The resulting mixture was stirred for 30 min and 2-bromopyrimidin-5-amine (2.77 g, 15.89 mmol) was added in one portion and the reaction mixture was stirred overnight at rt. The precipitate formed was filtered, washed with MeCN (60 mL), MTBE (60 mL), dried in *vacuo* to give pure N-(2-bromopyrimidin-5-yl)-6-ethoxy-2-methylpyrazolo[1,5-a]pyridine-5-carboxamide (4.45 g, 95.0% purity, 11.24 mmol, Y: 70.7%). ESI-MS (M+H)⁺: 376.0. ¹H NMR (400 MHz, DMSO-*d*₆) δ 10.51 (s, 1H), 9.02 (s, 2H), 8.42 (s, 1H), 7.95 (s, 1H), 6.51 (s, 1H), 4.18 (q, *J =* 7.1, 2H), 2.40 (s, 3H), 1.45 - 1.39 (t, *J =* 7.0, 3H).

### Example 86. General procedure for parallel synthesis:

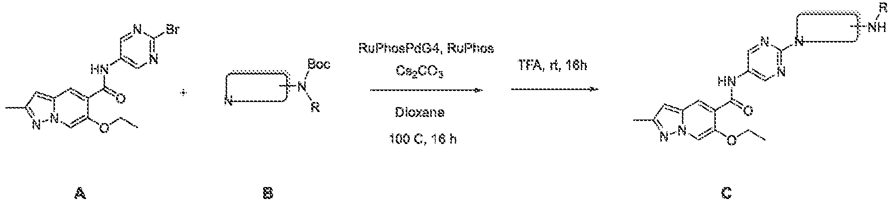

To a mixture of 1 eq. of ArBr (A) and 1.5 eq. Amine (B) in 1ml dry Dioxane under an inert atmosphere were added 0.05 eq. RuPhosPdG4, 0.05 eq. RuPhos and 2.5 eq. Cs₂CO₃. The reaction mixture was stirred at 100⁰C for 16 hours. After cooling to room temperature, the solvent was evaporated and 1 ml TFA was added and stirred at room temperature for 4h. The mixture was evaporated. The residue was dissolved in DMSO (appr. 1 ml), treated with scavenger SiliaMetS DMT and filtered.
Resulting solution was purified by HPLC (Deionized Water (phase A) and HPLC-grade Acetonitrile (phase B) were used as an eluent to obtain final compound (C). In most cases, TFA was used as an additive.
Instrument: Agilent 1260 Infinity systems equipped with DAD and mass-detector
Column: Waters Sunfire C18 OBD Prep Column, 100 A, 5 µm, 19 mm x 100mm with SunFire C18 Prep Guard Cartridge, 100 A, 10 µm, 19 mm x 10 mm.

**Table 6. Compounds synthesized using the procedure from Example 86**

| | | |
|---|---|---|
| **Compound no.** | **Compound** | **ESI-MS (M+H)⁺** |
| 562 | 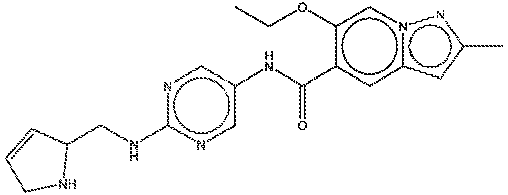 | 394.2 |
| 563 | 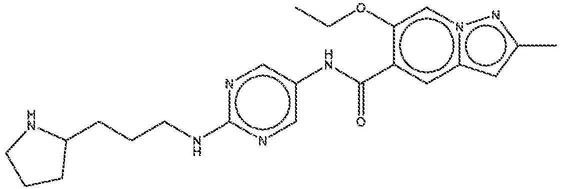 | 424.0 |
| 564 | 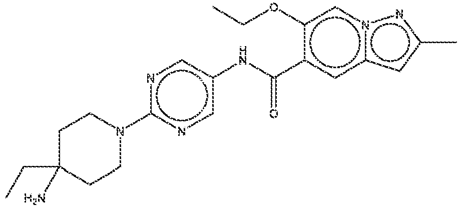 | 424.2 |
| 565 | 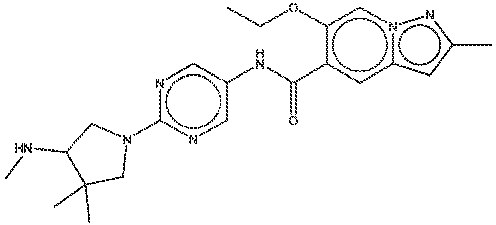 | 424.2 |
| 566 | 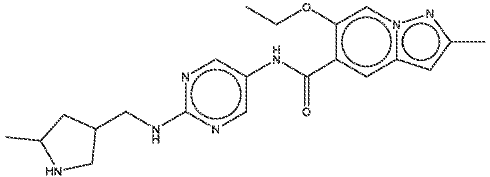 | 410.2 |
| 567 | 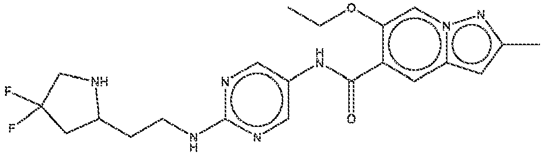 | 446.0 |
| 568 | 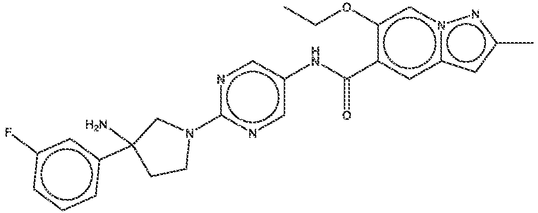 | 476.2 |
| 569 | 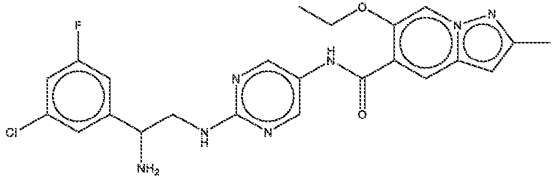 | 484.2 |
| 570 | 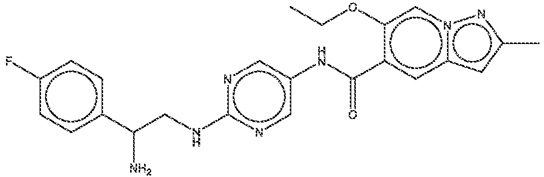 | 450.1 |
| 571 | 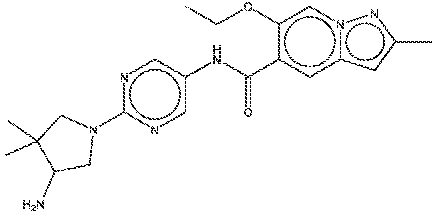 | 410.2 |
| 572 | 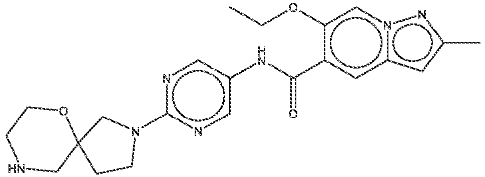 | 438.2 |
| 573 | 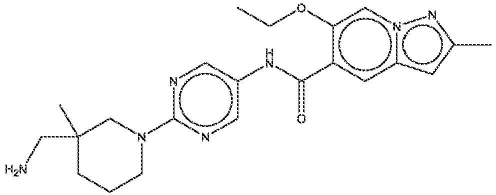 | 424.2 |
| 574 | 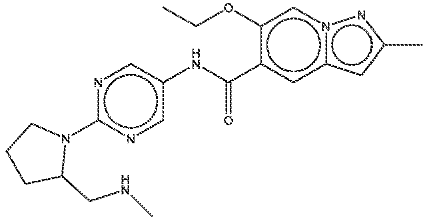 | 410.2 |
| 575 | 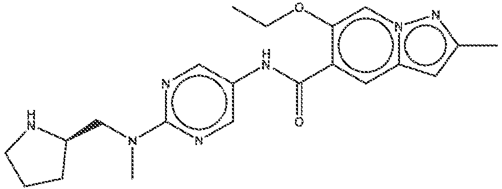 | 410.2 |
| 576 | 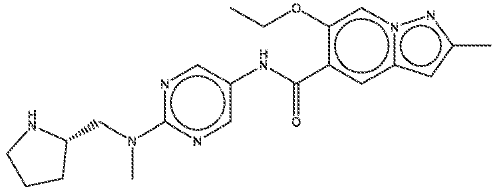 | 410.2 |
| 577 | 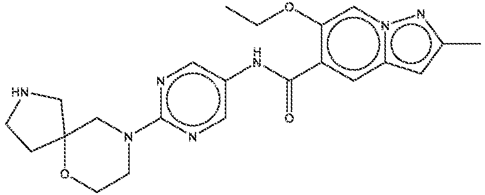 | 438.2 |
| 578 | 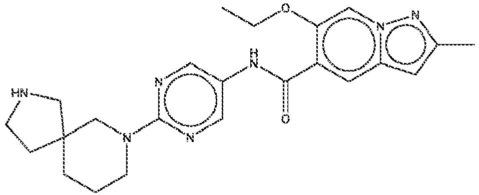 | 436.2 |
| 579 | 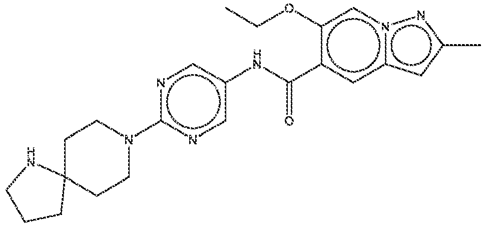 | 436.2 |
| 580 | 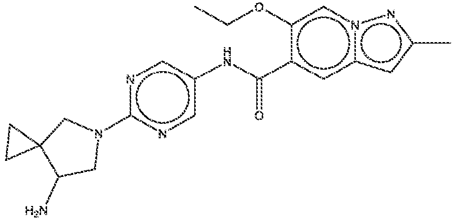 | 408.2 |
| 581 | 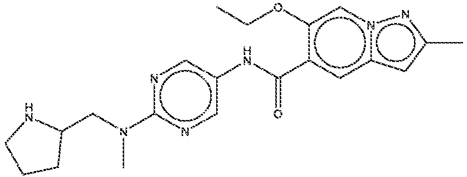 | 410.2 |
| 582 | 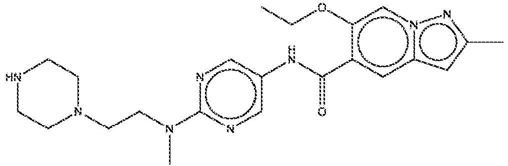 | 439.2 |
| 583 | 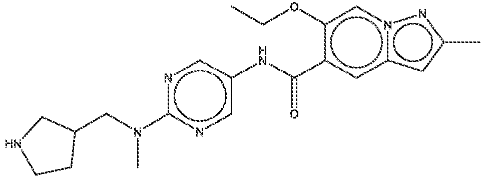 | 410.2 |
| 584 | 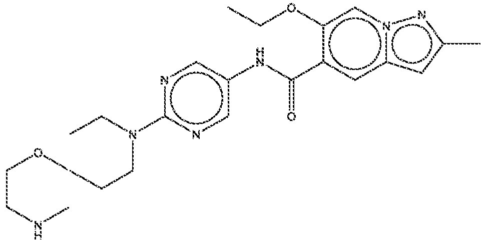 | 452.2 |
| 585 | 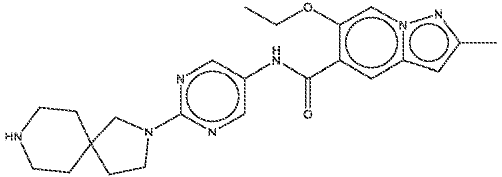 | 436.2 |
| 586 | 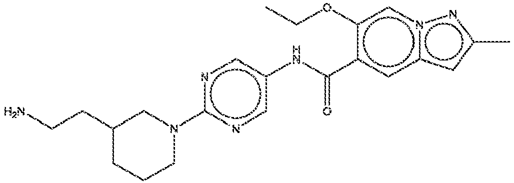 | 424.2 |
| 587 | 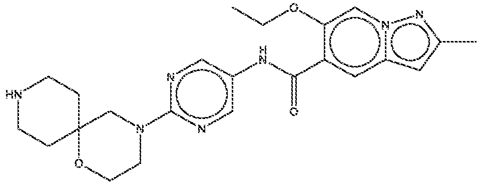 | 452.2 |
| 588 | 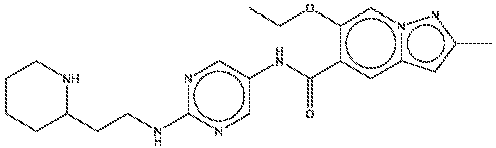 | 424.2 |
| 589 | 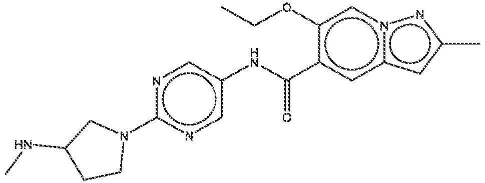 | 396.2 |
| 590 | 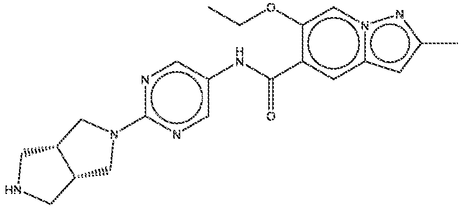 | 408.2 |
| 591 | 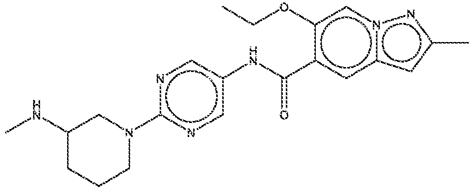 | 410.2 |
| 592 | 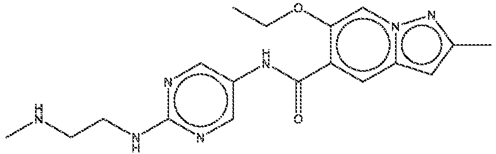 | 370.2 |
| 593 | 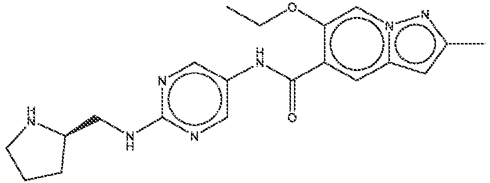 | 396.2 |
| 594 | 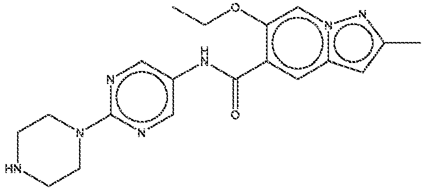 | 382.2 |
| 595 | 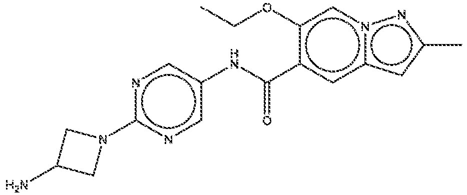 | 368.4 |
| 596 | 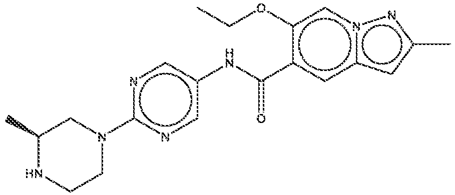 | 396.3 |
| 597 | 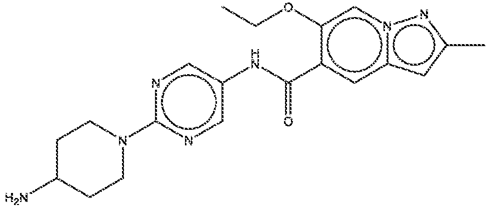 | 396.3 |
| 598 | 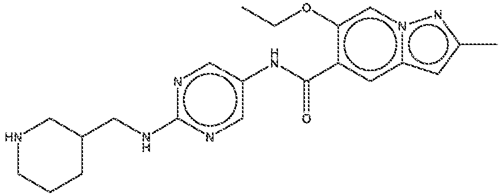 | 410.2 |
| 599 | 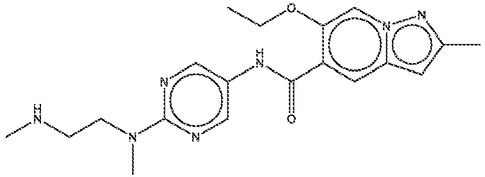 | 384.2 |
| 600 | 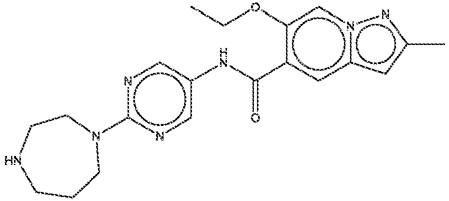 | 396.2 |
| 601 | 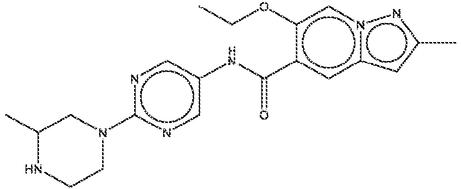 | 396.2 |
| 602 | 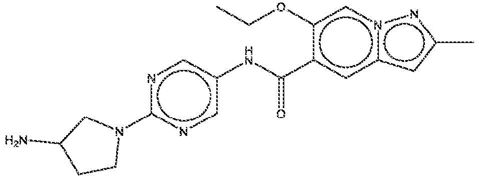 | 382.2 |
| 603 | 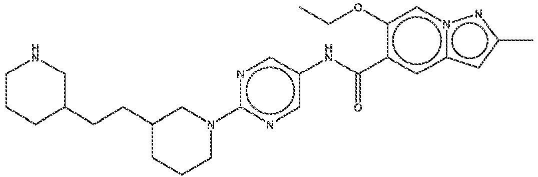 | 492.1 |
| 604 | 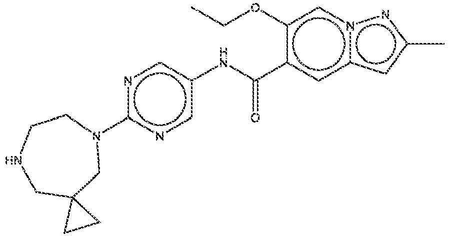 | 422.1 |
| 605 | 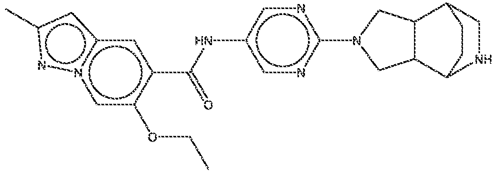 | 448.4 |
| 606 | 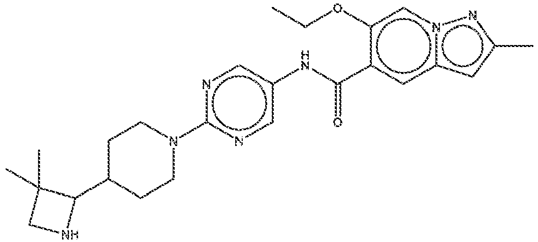 | 464.2 |
| 607 | 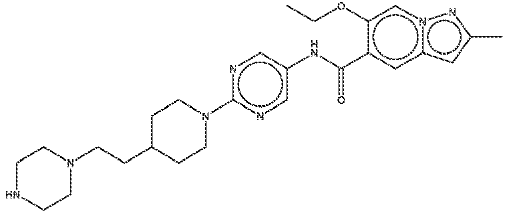 | 493.2 |
| 608 | 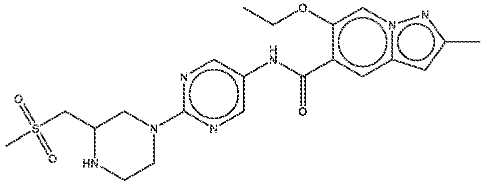 | 474.2 |
| 609 | 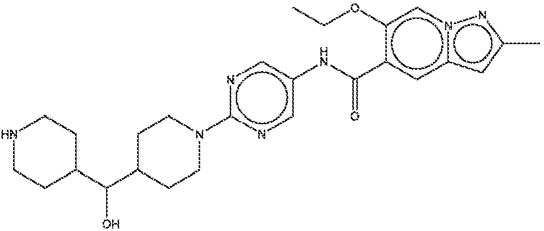 | 494.2 |
| 610 | 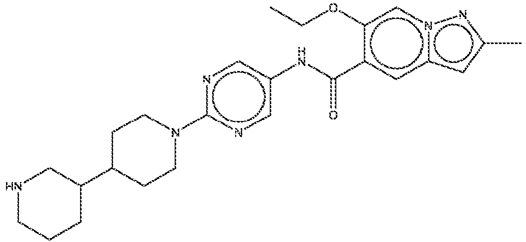 | 464.2 |
| 611 | 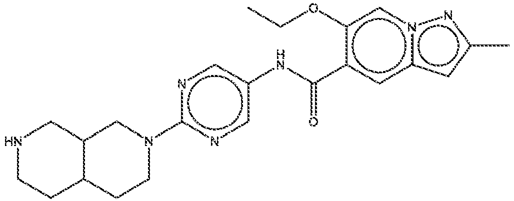 | 436.2 |
| 612 | 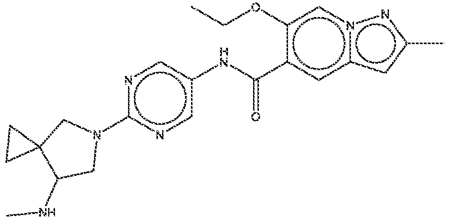 | 422.2 |
| 613 | 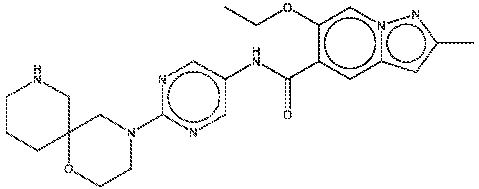 | 452.2 |
| 614 | 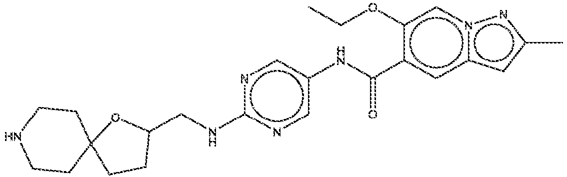 | 466.2 |
| 615 | 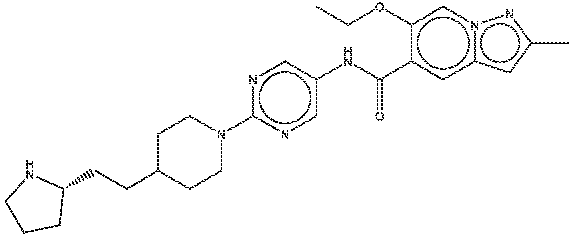 | 478.2 |
| 616 | 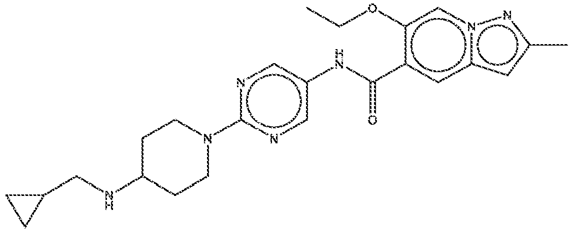 | 450.2 |
| 617 | 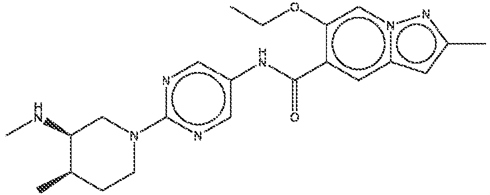 | 424.2 |
| 618 | 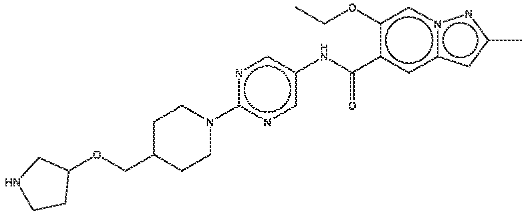 | 480.2 |
| 619 | 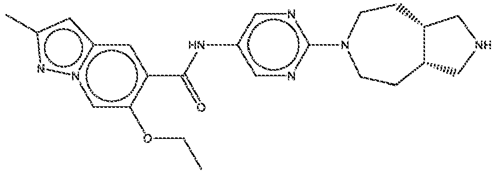 | 436.2 |
| 620 | 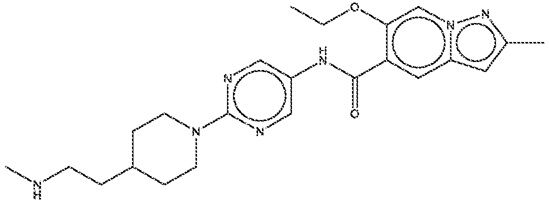 | 438.2 |
| 621 | 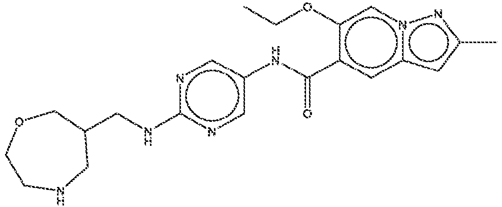 | 426.4 |
| 622 | 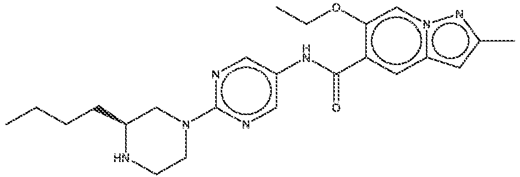 | 438.2 |
| 623 | 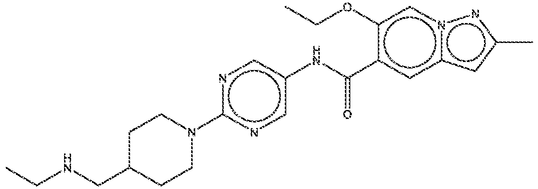 | 438.2 |
| 624 | 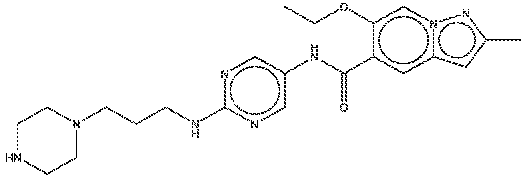 | 439.2 |
| 625 | 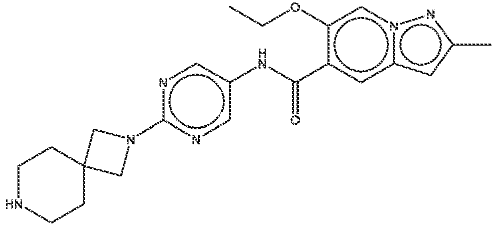 | 422.2 |
| 626 | 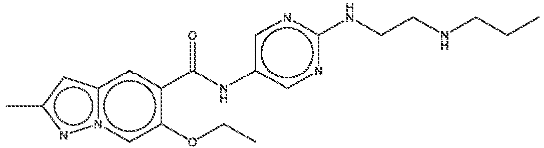 | 398.2 |

### Example 87. Preparation of compounds C highlighted in Table 2. (table is already in provisional)

| **Compound no.** | **Compound** | **ESI-MS (M+H)⁺** |
|---|---|---|
| 627 | 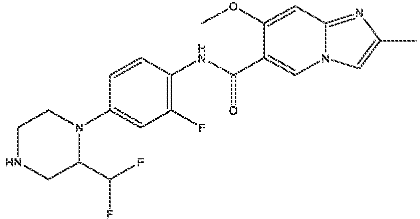 | 434.1 |
| 628 | 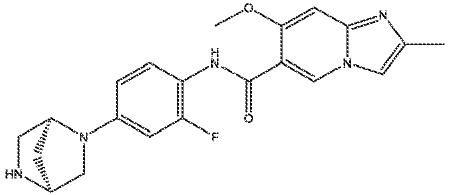 | 396.2 |
| 629 | 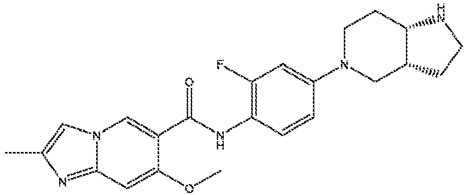 | 424.0 |
| 630 | 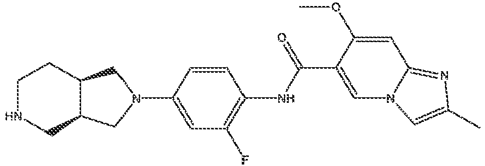 | 424.0 |

### Preparation of Starting Material: N-(2-bromopyrimidin-5-yl)-7-ethoxy-2-methylimidazo[1,2-a]pyridine-6-carboxamide

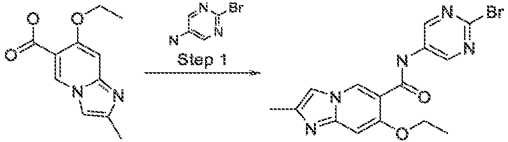

The 7-ethoxy-2-methylimidazo[1,2-a]pyridine-6-carboxylic acid (4.0 g, 18.16 mmol) was suspended in DMF (2mL) and DIPEA (4.69 g, 36.33 mmol) was added followed by HATU (8.29 g, 21.8 mmol) . The resulting mixture was stirred for 30 min and 2-bromopyrimidin-5-amine (3.16 g, 18.16 mmol) was added in one portion and the reaction mixture was stirring overnight at rt. The precipitate formed was filtered, washed with MeCN (60 ml), MTBE (60 mL), dried under vacuo to give pure N-(2-bromopyrimidin-5-yl)-7-ethoxy-2-methylimidazo[1,2-a]pyridine-6-carboxamide (4.9 g, Y: 68.1%). ESI-MS (M+H)⁺: 376.0. ¹H NMR (400 MHz, DMSO-*d*₆) δ 10.42 (s, 1H), 9.01 (s, 2H), 8.89 (s, 1H), 7.60 (s, 1H), 6.95 (s, 1H), 4.23 (d, J = 7.8 Hz, 2H), 2.29 (s, 3H), 1.44 (t, J = 6.7 Hz, 3H).

### Example 88. Parallel synthesis:

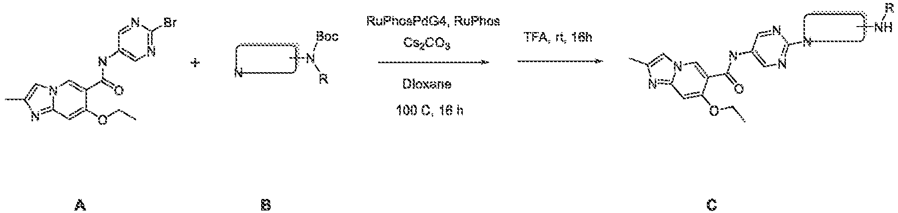

To a mixture of 1 eq. of ArBr (A) and 1.5 eq. Amine (B) in 1ml dry Dioxane under an inert atmosphere were added 0.05 eq. RuPhosPdG4, 0.05 eq. RuPhos and 2.5 eq. Cs₂CO₃. The reaction mixture was stirred at 100°C for 16 hours. After cooling to room temperature, the solvent was evaporated and 1 ml TFA was added and stirred at room temperature for 4h. The mixture was evaporated. The residue was dissolved in DMSO (appr. 1 ml), treated with scavenger SiliaMetS DMT and filtered.
Resulting solution was purified by HPLC (Deionized Water (phase A) and HPLC-grade Acetonitrile (phase B) were used as an eluent to obtain final compound (C). In most cases, TFA was used as an additive.
Instrument: Agilent 1260 Infinity systems equipped with DAD and mass-detector
Column: Waters Sunfire C18 OBD Prep Column, 100 A, 5 µm, 19 mm x 100mm with SunFire C18 Prep Guard Cartridge, 100 A, 10 µm, 19 mm x 10 mm

**Table 7. Compounds synthesized using the procedure from Example 88.**

| **Compound no.** | **Compound** | **ESI-MS (M+H)⁺** |
|---|---|---|
| 631 | 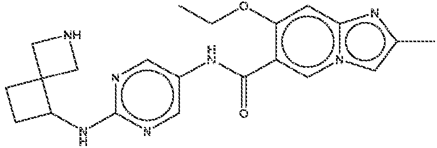 | 408.2 |
| 632 | 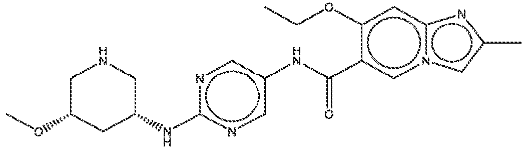 | 426.2 |
| 633 | 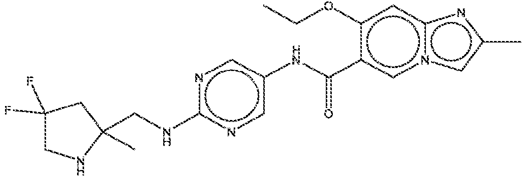 | 446 |
| 634 | 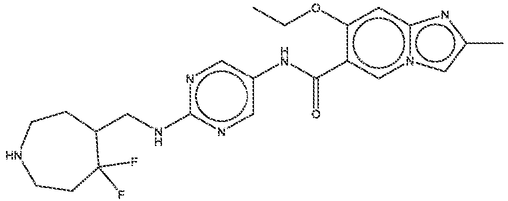 | 460 |
| 635 | 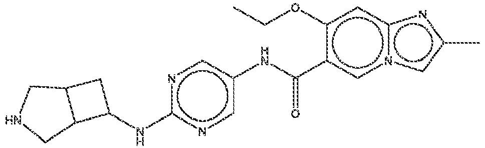 | 408 |
| 636 | 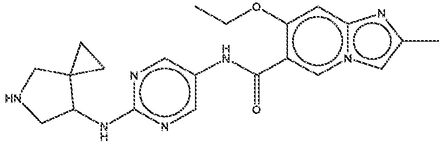 | 408.1 |
| 637 | 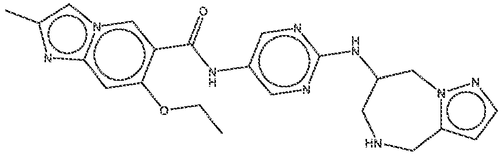 | 448.2 |
| 638 | 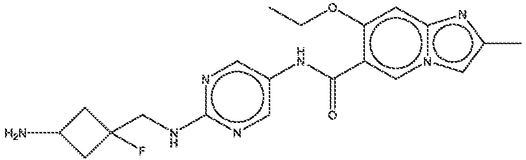 | 414.2 |
| 639 | 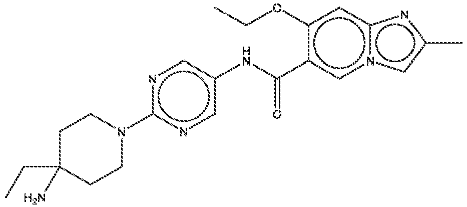 | 424.2 |
| 640 | 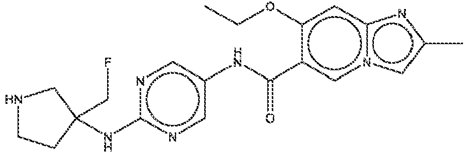 | 415.2 |
| 641 | 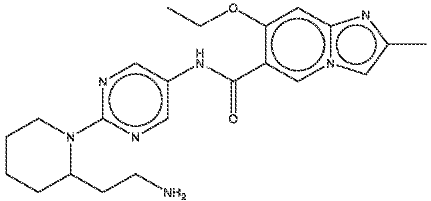 | 424.4 |
| 642 | 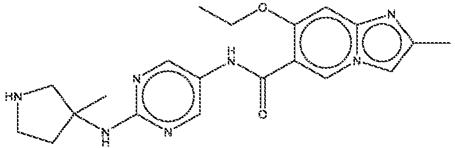 | 396.2 |
| 643 | 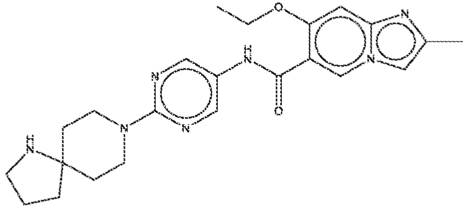 | 437.2 |
| 644 | 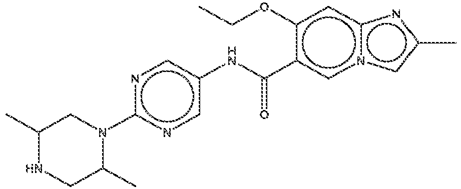 | 411.3 |
| 645 | 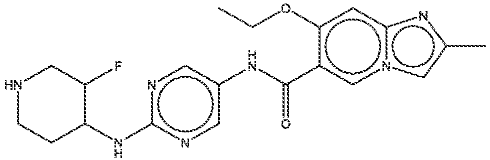 | 414 |
| 646 | 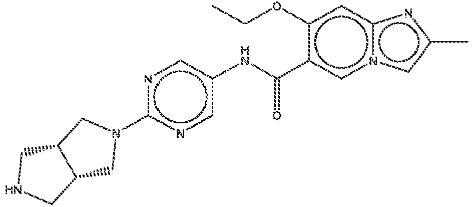 | 408.2 |
| 647 | 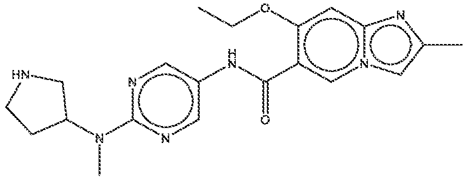 | 396.2 |
| 648 | 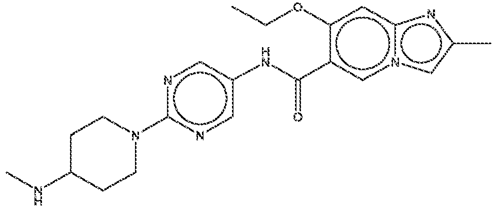 | 410.2 |
| 649 | 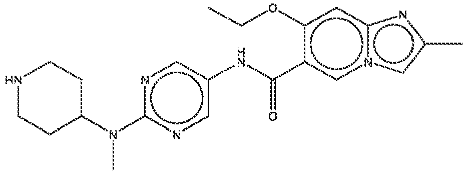 | 410 |
| 650 | 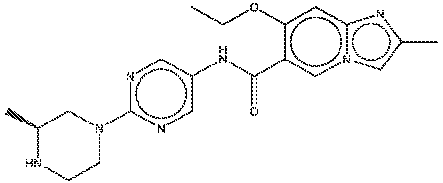 | 397.2 |
| 651 | 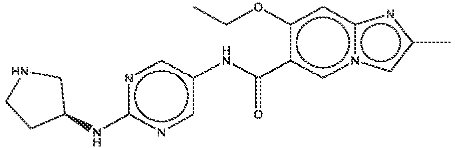 | 382.2 |
| 652 | 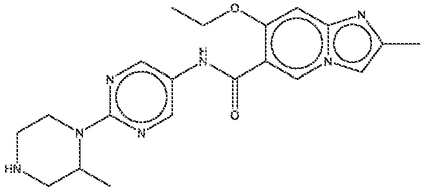 | 397.2 |
| 653 | 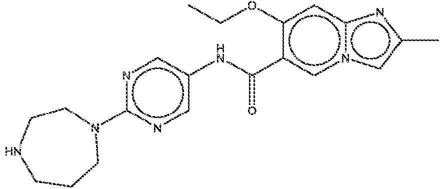 | 396.2 |
| 654 | 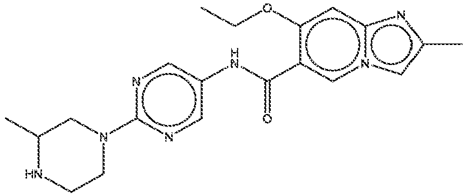 | 397 |
| 655 | 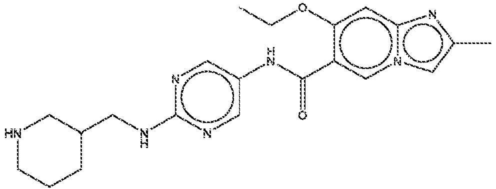 | 411.2 |

### Preparation of Starting Material: N-(5-bromopyrazin-2-yl)-7-ethoxy-2-methylimidazo[1,2-a]pyridine-6-carboxamide

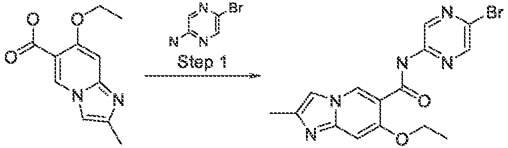

Mixture of 7-ethoxy-2-methylimidazo[1,2-a]pyridine-6-carboxylic acid (3.6 g, 16.35 mmol), 5-bromopyrazin-2-amine (2.84 g, 16.35 mmol) , and pyridine (3.88 g, 49.04 mmol) were dissolved in anhydrous acetonitrile (4 mL). The reaction was stirred at room temperature for 5 minutes, followed by addition of 2,4,6-tripropyl-1,3,5,2,4,6-trioxatriphosphorinane-2,4,6-trioxide (T3P) (20.81 g, 65.39 mmol) (50 wt % in EtOAc) over the course of 10 minutes. The reaction continued to stir for 12 hours, then the reaction mixture was filtered and solid was dried under vacuo to afford N-(5-bromopyrazin-2-yl)-7-ethoxy-2-methylimidazo[ 1,2-a]pyridine-6-carboxamide (4.65 g, Y: 71.8%). ESI-MS (M+H)⁺: 376.0. ¹H NMR (500 MHz, Chloroform-*d*) δ 10.53 (s, 1H), 9.45 (s, 1H), 8.96 (s, 1H), 8.37 (s, 1H), 7.29 (s, 1H), 6.92 (s, 1H), 4.31 (J = 7.8 Hz, 2H), 2.41 (s, 3H), 1.66 (J = 6.7 Hz, 3H).

### Example 89. Parallel synthesis:

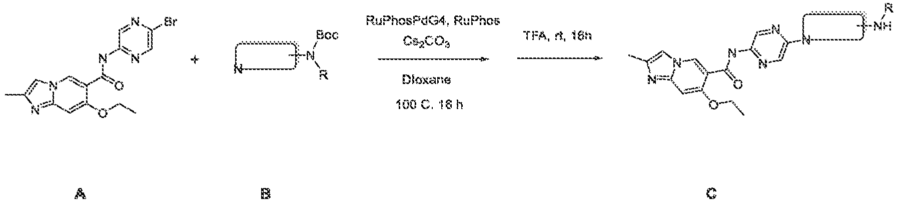

To a mixture of 1 eq. of ArBr (A) and 1.5 eq. Amine (B) in 1ml dry Dioxane under an inert atmosphere were added 0.05 eq. RuPhosPdG4, 0.05 eq. RuPhos and 2.5 eq. Cs₂CO₃. The reaction mixture was stirred at 100⁰C for 16 hours. After cooling to room temperature, the solvent was evaporated and 1 ml TFA was added and stirred at room temperature for 4h. The mixture was evaporated. The residue was dissolved in DMSO (appr. 1 ml), treated with scavenger SiliaMetS DMT and filtered.
Resulting solution was purified by HPLC (Deionized Water (phase A) and HPLC-grade Acetonitrile (phase B) were used as an eluent to obtained final compound (C). In most cases, TFA was used as an additive.
Instrument: Agilent 1260 Infinity systems equipped with DAD and mass-detector
Column: Waters Sunfire C18 OBD Prep Column, 100 A, 5 µm, 19 mm x 100mm with SunFire C18 Prep Guard Cartridge, 100 A, 10 µm, 19 mm x 10 mm

**Table 8. Compounds synthesized using the procedure in Example 89**

| **Compound no.** | **Compound** | **ESI-MS (M+H)⁺** |
|---|---|---|
| 656 | 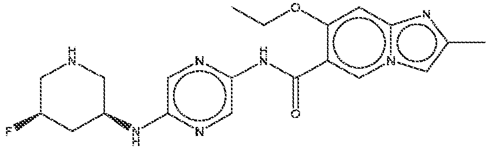 | 415.2 |
| 657 | 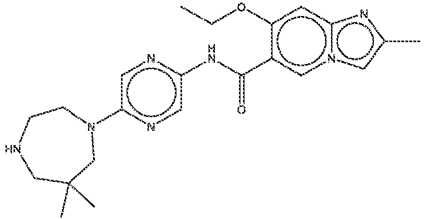 | 425.2 |
| 658 | 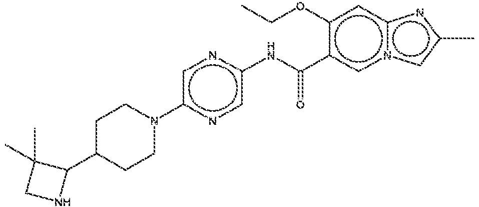 | 464.2 |
| 659 | 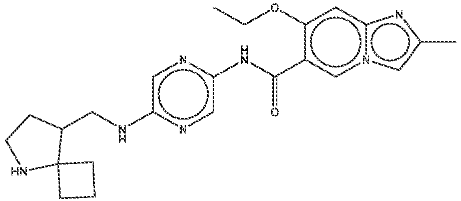 | 436.2 |
| 660 | 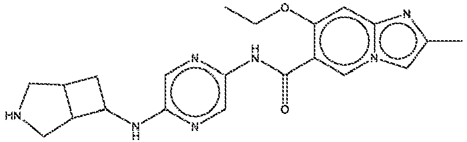 | 408.2 |
| 661 | 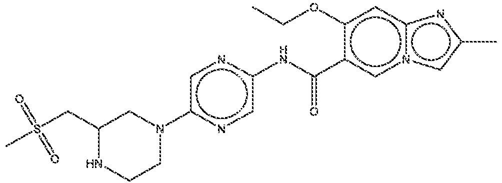 | 475.2 |
| 662 | 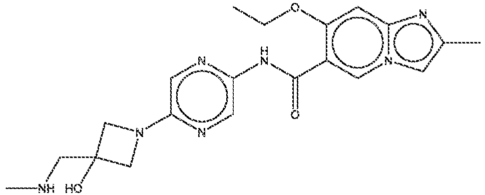 | 413.2 |
| 663 | 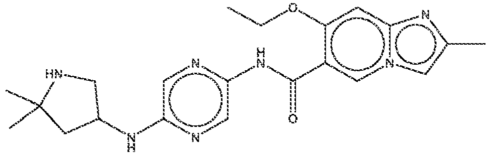 | 410.2 |
| 664 | 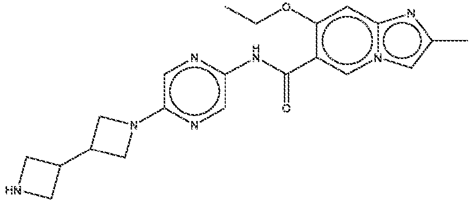 | 408.2 |
| 665 | 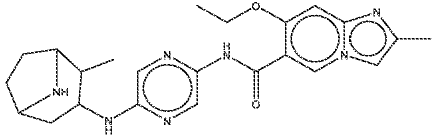 | 436.2 |
| 666 | 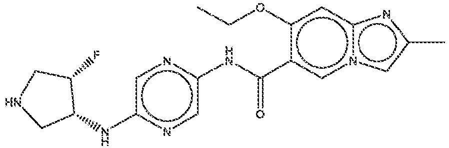 | 400.2 |
| 667 | 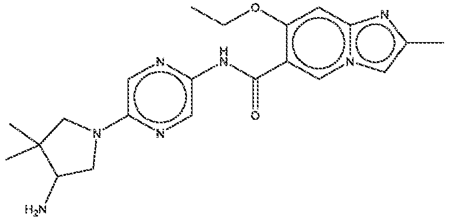 | 410.2 |
| 668 | 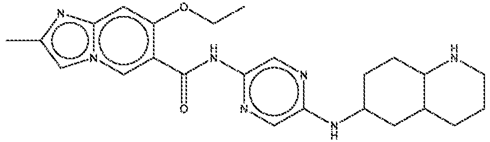 | 451.2 |
| 669 | 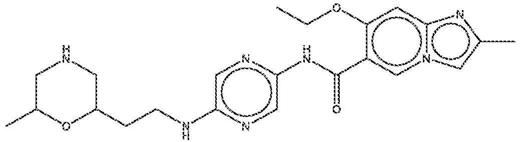 | 440.2 |
| 670 | 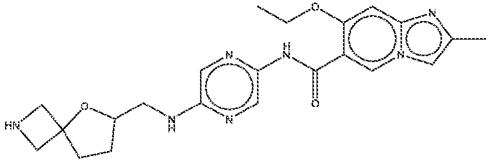 | 438.2 |
| 671 | 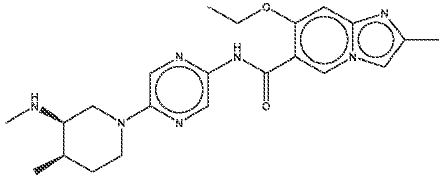 | 424.2 |
| 672 | 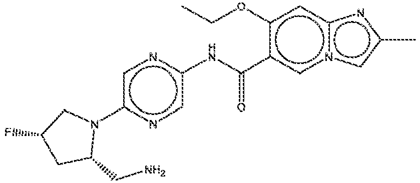 | 414.2 |
| 673 | 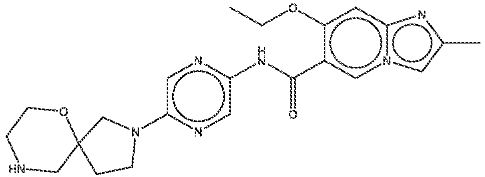 | 438 |
| 674 | 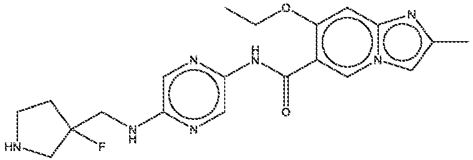 | 414.2 |
| 675 | 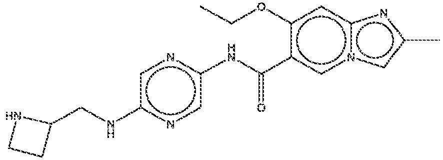 | 482.2 |
| 676 | 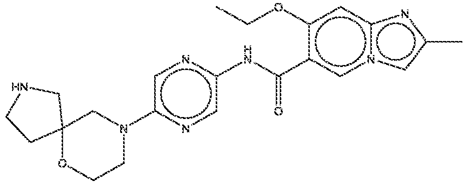 | 438.1 |
| 677 | 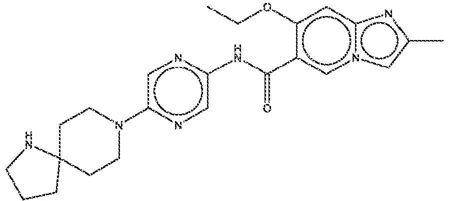 | 437.2 |
| 678 | 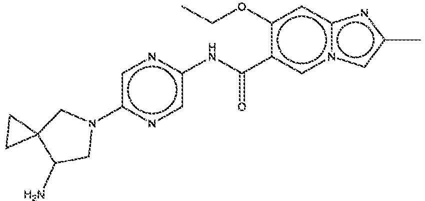 | 408.1 |
| 679 | 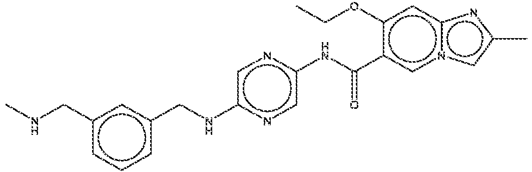 | 447 |
| 680 | 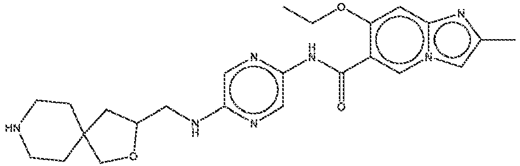 | 466.2 |
| 681 | 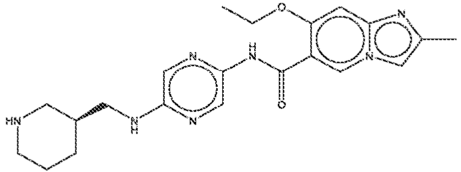 | 411.2 |
| 682 | 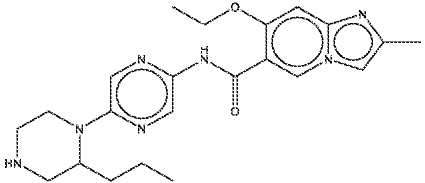 | 424.2 |
| 683 | 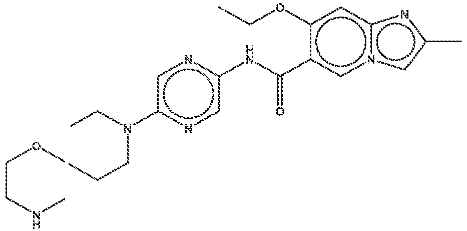 | 453.1 |
| 684 | 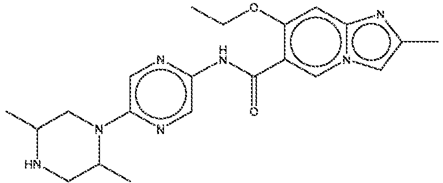 | 410.2 |
| 685 | 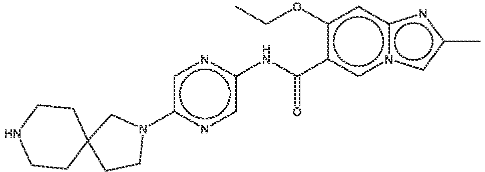 | 437.2 |
| 686 | 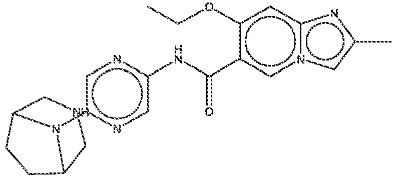 | 408.2 |
| 687 | 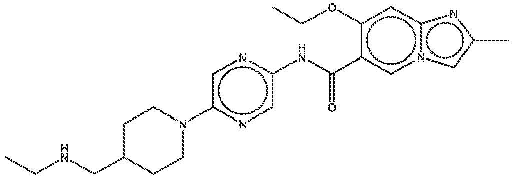 | 439.4 |
| 688 | 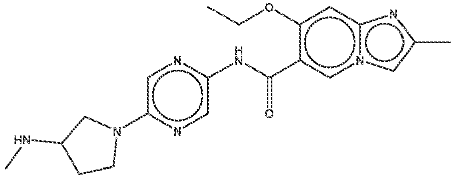 | 396.2 |
| 689 | 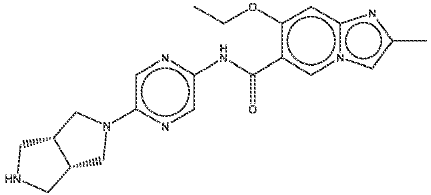 | 409.4 |
| 690 | 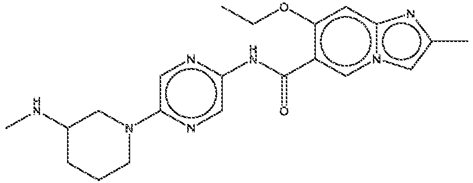 | 410.2 |
| 691 | 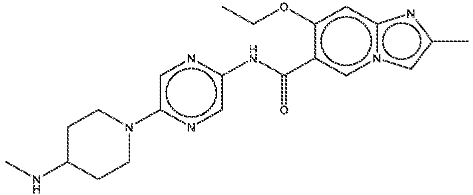 | 410.2 |
| 692 | 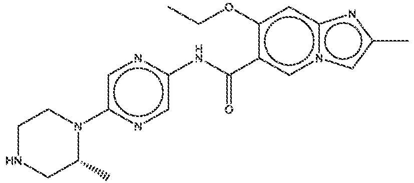 | 396.2 |
| 693 | 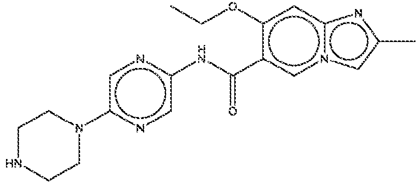 | 383.2 |
| 694 | 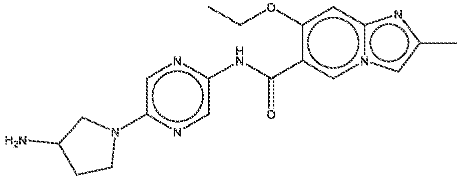 | 382.2 |
| 695 | 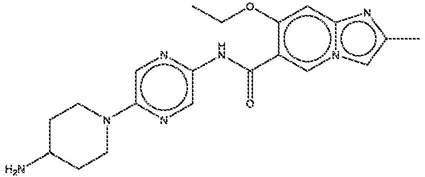 | 396.2 |
| 696 | 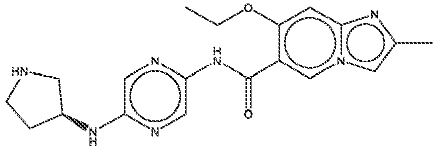 | 383 |
| 697 | 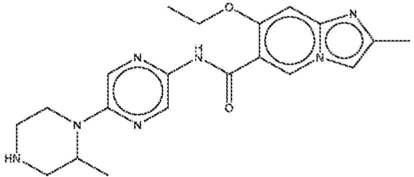 | 396.2 |
| 698 | 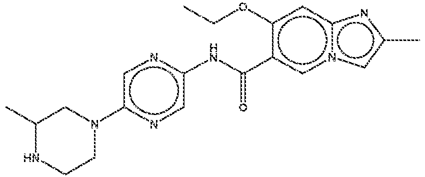 | 396.2 |
| 699 | 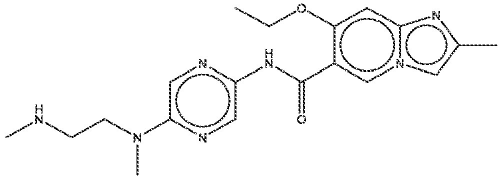 | 384.2 |

### Example 90. (2S)-4-(5-7-ethoxy-2-methylimidazo[1,2-a]pyridine-6-amidopyrazin-2-yl)-2-methylpiperazine-1-carboxylate (Compound 700)

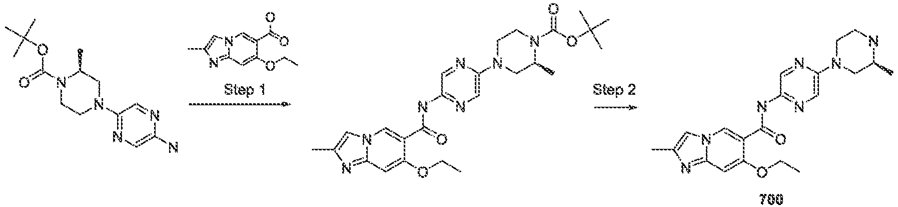

### Step 1: Preparation of tert-butyl (2S)-4-(5-7-ethoxy-2-methylimidazo[1,2-a]pyridine-6-amidopyrazin-2-yl)-2-methylpiperazine-1-carboxylate

The 7-ethoxy-2-methylimidazo[1,2-a]pyridine-6-carboxylic acid (100.0 mg, 454.08 µmol) was suspended in DMF (4mL) and DIPEA (146.7 mg, 1.14 mmol) was added followed by HATU (207.16 mg, 544.82 µmol) . The resulting mixture was stirred for 30 min at rt. After that tert-butyl (2S)-4-(5-aminopyrazin-2-yl)-2-methylpiperazine-1-carboxylate (133.19 mg, 454.02 µmol) was added in one portion and the reaction mixture was stirring overnight at rt. The precipitate formed was filtered, washed with MeCN (2 ml), MTBE (2mL), dried under vacuo to give pure tert-butyl (2S)-4-(5-7-ethoxy-2-methylimidazo[1,2-a]pyridine-6-*amidopyrazin-2-yl)-2-methylpiperazine-1-carboxylate* (40.0 mg, Y: 17.8%). ESI-MS (M+H)⁺: 496.4.

### Step 2: Preparation of 7-ethoxy-2-methyl-N-5-[(3S)-3-methylpiperazin-1-yl]pyrazin-2-ylimidazo[1,2-a]pyridine-6-carboxamide

To a solution of tert-butyl (2S)-4-(5-7-ethoxy-2-methylimidazo[1,2-a]pyridine-6-*amidopyrazin-2-yl)-2-methylpiperazine-1-carboxylate* (40.0 mg, 80.71 µmol) in dichloromethane (3 mL) TFA (91.98 mg, 806.7 µmol) was added and the mixture was stirred overnight. Then the mixture was evaporated to dryness under reduced pressure. The obtained crude residue was crystallized from MTBE/MeCN to give 7-ethoxy-2-methyl-N-5-[(38)-3-methylpiperazin-1-yl]pyrazin-2-ylimidazo[1,2-a]pyridine-6-carboxamide as TFA salt (18.6 mg, Y: 45.3%). ESI-MS (M+H)⁺: 396.2. ¹H NMR (400 MHz, DMSO-*d*6) δ 10.69(s, 1H), 9.19 (br s, 2H), 8.96 (s, 1H), 8.90 (br s, 1H), 8.28 (s, 1H), 7.90 (s, 1H), 7.33 (s, 1H), 4.35 (q, J = 7.1 Hz, 2H), 3.65-3.25 (m, 4H), 3.20 - 3.07 (m, 2H), 2.99 - 2.95 (m, 1H) 2.43 (s, 3H), 1.45 (t, J = 6.9 Hz, 3H)), 1.28 (d, *J* = 8.0 Hz, 3H).

### Example 91. 6-ethoxy-2-methyl-N-{5-[(3S)-3-methylpiperazin-1-yl]pyrazin-2-yl}pyrazolo[1,5-a]pyridine-5-carboxamide (Compound 701)

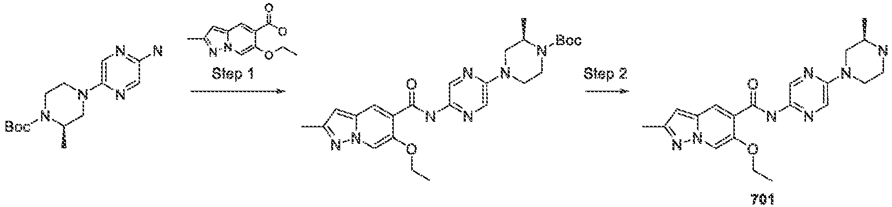

### Step 1: Preparation of tert-butyl (2S)-4-(5-6-ethoxy-2-methylpyrazolo[1,5-a]pyridine-5-amidopyrazin-2-yl)-2-methylpiperazine-1-carboxylate

The 6-ethoxy-2-methylpyrazolo[1,5-a]pyridine-5-carboxylic acid (130.0 mg, 591 µmol) was suspended in DMF (4mL) and DIPEA (146.7 mg, 1.14 mmol) was added followed by HATU (270 mg, 710 µmol) . The resulting mixture was stirred for 30 min at rt. After that tert-butyl (2S)-4-(5-aminopyrazin-2-yl)-2-methylpiperazine-1-carboxylate (173 mg, 590 µmol) was added in one portion and the reaction mixture was stirring overnight at rt. The precipitate formed was filtered, washed with MeCN (2 ml), MTBE (2mL), dried under vacuo to give pure tert-butyl (2S)-4-(5-7-ethoxy-2-methylimidazo[1,2-a]pyridine-6-amidopyrazin-2-yl)-2-methylpiperazine-1-carboxylate (40.0 mg, Y: 17.8%). ESI-MS (M+H)⁺: 496.2.

### Step 2: Preparation of 6-ethoxy-2-methyl-N-5-[(3S)-3-methylpiperazin-1-yl]pyrazin-2-yipyrazolo[1,5-a]pyridine-5-carboxamide

To a solution of tert-butyl (2S)-4-(5-6-ethoxy-2-methylpyrazolo[1,5-a]pyridine-5-*amidopyrazin-2-yl)-2-methylpiperazine-1-carboxylate* (79.95 mg, 161.33 µmol) in dichloromethane (3 mL) TFA (91.98 mg, 806.66 µmol) was added and the mixture was stirred overnight. Then the mixture was evaporated to dryness under reduced pressure. The obtained crude residue was crystallized from MTBE/MeCN to give 6-ethoxy-2-methyl-N-5-[(3S)-3-methylpiperazin-1-yl]pyrazin-2-ylpyrazolo[1,5-a]pyridine-5-carboxamide as TFA salt (62.0 mg, Y: 87.2%) . ESI-MS (M+H)⁺: 396.2. ¹H NMR (400 MHz, DMSO-d6) δ 10.55 (s, 1H), 9.09 (br m, 1H) 9.01 (s, 1H), 8.76 (br s, 1H), 8.49 (s, 1H), 8.27 (s, 1H), 8.09 (s, 1H), 6.53 (s, 1H), 4.39 - 4.29 (m, 2H), 4.21 (d, *J* = 6.9 Hz, 2H), 3.21 - 3.06 (m, 3H), 2.96 (t, *J* = 12.4 Hz, 1H), 2.38 (s, 3H), 1.43 (t, *J* = 7.1 Hz, 3H), 1.27 (d, *J* = 6.3 Hz, 3H).

### Example 92. 7-ethoxy-N-2-fluoro-4-[(3S)-3-methylpiperazin-1-yl]phenyl-2-methylimidazo[1,2-a]pyridine-6-carboxamide (Compound 702)

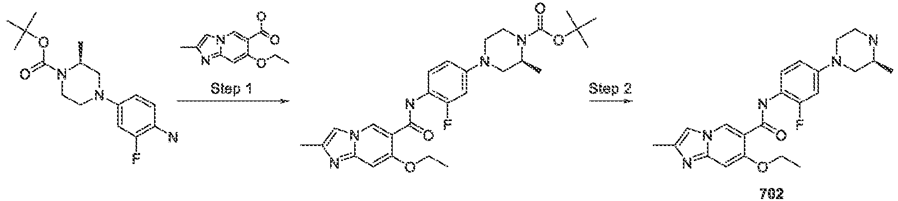

### Step 1: Preparation of tert-butyl (2S)-4-(4-7-ethoxy-2-methylimidazo[1,2-a]pyridine-6-amido-3-fluorophenyl)-2-methylpiperazine-1-carboxylate

The 7-ethoxy-2-methylimidazo[1,2-a]pyridine-6-carboxylic acid (100.0 mg, 454.08 µmol) was suspended in DMF (40mL) and DIPEA (146.53 mg, 1.13 mmol) was added followed by HATU (206.92 mg, 544.19 µmol) . The resulting mixture was stirred for 30 min at rt. After that tert-butyl (2S)-4-(4-amino-3-fluorophenyl)-2-methylpiperazine-1-carboxylate (140.3 mg, 453.49 µmol) was added in one portion and the reaction mixture was stirring overnight at rt. The precipitate formed was filtered, washed with MeCN (20 ml), MTBE (20mL), dried under vacuo to give pure tert-butyl (2S)-4-(4-7-ethoxy-2-methylimidazo[1,2-a]pyridine-6-amido-3-fluorophenyl)-2-methylpiperazine-1-carboxylate (78.0 mg, Y: 33.6%) ESI-MS (M+H)⁺: 512.4.

### Step 2: Preparation of 7-ethoxy-N-2-fluoro-4-[(3S)-3-methylpiperazin-1-yl]phenyl-2-methylimidazo[1,2-a]pyridine-6-carboxamide

The tert-butyl (2S)-4-(4-7-ethoxy-2-methylimidazo[1,2-a]pyridine-6-amido-3-fluorophenyl)-2-methylpiperazine-1-carboxylate (78.0 mg, 152.47 µmol) was dissolved in dichloromethane ( 10 mL) and TFA (173.8 mg, 1.52 mmol) was added. The resulting mixture was stirred ar rt overnight and then evaporated to dryness to provide crude material. The residue was triturated with MeCN/MTBE (1/4) and the resulting precipitate was filtered and dried under vacuo to give pure 7-ethoxy-N-2-fluoro-4-[(3S)-3-methylpiperazin-1-yl]phenyl-2-methylimidazo[1,2-a]pyridine-6-carboxamide as TFA salt (67.0 mg, 127.5 µmol, 83.6% yield). ESI-MS (M+H)⁺: 412.0. ¹H NMR (400 MHz, DMSO-*d*6) δ 9.98 (s, 1H), 9.22 (s, 1H), 9.11 (br s, 1H), 8.73 (br s, 1H), 7.91 (s, 1H), 7.81 (t, *J* = 9.0 Hz, 1H), 7.33 (s, 1H), 7.03 (dd, *J* = 14.0, 2.5 Hz, 1H), 6.88 (dd, *J* = 8.9, 2.3 Hz, 1H), 3.90 - 3.70 (m, 2H), 3.40 - 3.30 (m, 2H), 3.13 - 3.08 (m, 1H), 2.97- 2.90 (m, 1H), 2.74 - 2.70 (m, 1H), 2.41 (s, 3H), 1.46 (t, *J =* 7.1 Hz, 3H), 1.27 (d, *J* = 6.3 Hz, 3H).

### Example 93. Preparation of tert-butyl 7-(4-amino-3-fluorophenyl)-4,7-diazaspiro[2.5]octane-4-carboxylate (Compound 703)

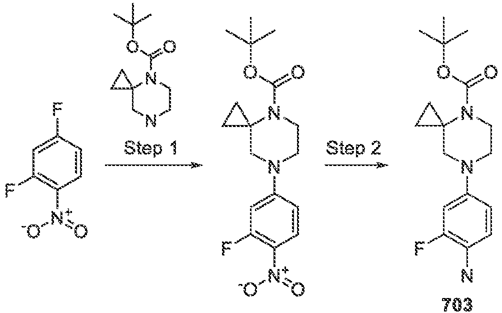

### Step 1: Preparation of tert-butyl 7-(3-fluoro-4-nitrophenyl)-4,7-diazaspiro[2.5]octane-4-carboxylate

2,4-Difluoro-1-nitrobenzene (374.89 mg, 2.36 mmol), tert-butyl 4,7-diazaspiro[2.5]octane-4-carboxylate (500.25 mg, 2.36 mmol) and potassium carbonate (651.35 mg, 4.71 mmol) were dissolved in dimethylformamide (5 mL) and stirred at 60° C overnight. Then the reaction mixture was poured into ice water and extracted with EtOAc (20 mL). The organic layer was separated, washed with H₂O (10 mL), brine (10 mL) dried and evaporated to give crude, which was purified by flash chromatography (EA:PE=1:10, v/v) to give title product tert-butyl 7-(3-fluoro-4-nitrophenyl)-4,7-diazaspiro[2.5]octane-4-carboxylate (260.0 mg, Y: 31.4%). EST-MS (M-56+H)⁺: 296.0.

### Step 2: Preparation of tert-butyl 7-(4-amino-3-fluorophenyl)-4,7-diazaspiro[2.5]octane-4-carboxylate

tert-butyl 7-(3-fluoro-4-nitrophenyl)-4,7-diazaspiro[2.5]octane-4-carboxylate (260.0 mg, 739.96 µmol) was dissolved in methanol and treated with 10% Pd/C (0.17g). The resulting mixture was stirred vigorously at ambient hydrogen pressure and room temperature until the reaction was complete. The catalyst was filtered off, the filtrate was evaporated and purified by column chromatography using hexane/EtOAc (2:1) as an eluent to afford tert-butyl 7-(4-amino-3-fluorophenyl)-4,7-diazaspiro[2.5]octane-4-carboxylate (200.0 mg, Y: 84%). ESI-MS (M+H)⁺: 322.0. ¹H NMR (500 MHz, DMSO-d6) δ 6.69 - 6.56 (m, 2H), 6.48 (d, J = 8.5 Hz, 1H), 4.52 (br s, 2H), 3.50 (t, J = 4.9 Hz, 2H), 2.88 (t, J = 4.9 Hz, 2H), 2.74 (s, 2H), 0.91 - 0.86 (m, 2H), 0.81 - 0.76 (m, 2H).

### Example 94. Preparation of N-(4-4,7-diazaspiro[2.5]octan-7-yl-2-fluorophenyl)-7-ethoxy-2-methylimidazo[1,2-a]pyridine-6-carboxamide (Compound 704).

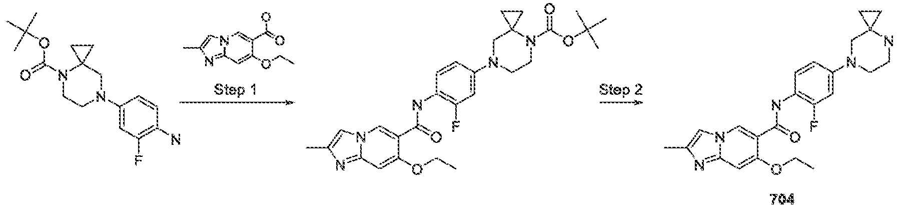

### Step 1: Preparation of tert-butyl 7-(4-7-ethoxy-2-methylimidazo[1,2-a]pyridine-6-amido-3-fluorophenyl)-4,7-diazaspiro[2.5]octane-4-carboxylate

The 7-ethoxy-2-methylimidazo[1,2-a]pyridine-6-carboxylic acid (100.0 mg, 454.08 µmol) was suspended in DMF (3 mL) and DIPEA (176.24 mg, 1.36 mmol) was added followed HATU (207.4 mg, 545.46 µmol). The resulting mixture was stirred for 30 min at rt. After that tert-butyl 7-(4-amino-3-fluorophenyl)-4,7-diazaspiro[2.5]octane-4-carboxylate (146.09 mg, 454.55 µmol) was added in one portion and the reaction mixture was stirring overnight at rt. The precipitate formed was filtered, washed with MeCN (5 ml), MTBE (2mL), dried under vacuo to give pure tert-butyl 7-(4-7-ethoxy-2-methylimidazo[1,2-a]pyridine-6-amido-3-fluorophenyl)-4,7-diazaspiro[2.5]octane-4-carboxylate (75.0 mg, Y: 31.5% yield). ESI-MS (M+H)⁺: 524.0.

### Step 2: Preparation of N-(4-4,7-diazaspiro[2.5]octan-7-yl-2-fluorophenyl)-7-ethoxy-2-methylimidazo[1, 2-a]pyridine-6-carboxamide

The tert-butyl 7-(4-7-ethoxy-2-methylimidazo[1,2-a]pyridine-6-amido-3-fluorophenyl)-4,7-diazaspiro[2.5]octane-4-carboxylate (75.01 mg, 143.25 µmol) was dissolved in DCM ( 10 mL) and TFA (163.34 mg, 1.43 mmol) was added. The resulting mixture was stirred ar rt overnight and then evaporated to dryness to provide crude material. The residue was triturated with MeCN/MTBE (1:4) and the resulting precipitate was filtered and dried under vacuo to give pure N-(4-4,7-diazaspiro[2.5]octan-7-yl-2-fluorophenyl)-1-ethoxy-2-methylimidazo[1,2-a]pyridine-6-carboxamide as TFA salt (72.0 mg, Y: 93.5%). ESI-MS (M+H)⁺: 424.2. ¹H NMR (400 MHz, DMSO-*d*6) δ 9.96 (s, 1H), 9.21 (br s, 3H), 7.89 (s, 1H), 7.83 (t, *J =* 8.9 Hz, 1H), 7.30 (s, 1H), 6.98 (d, *J* = 13.8 Hz, 1H), 6.82 (d, *J* = 9.2 Hz, 1H), 4.33 (d, *J* = 7.3 Hz, 2H), 3.31 - 3.21 (m, 6H), 2.40 (s, 3H), 1.44 (t, *J* = 7.1 Hz, 3H), 1.01 (s, 2H), 0.89 (s, 2H).

### Example 95. 7-cyclobutoxy-N-2-fluoro-4-[(3S)-3-methylpiperazin-1-yl]phenyl-2-methylimidazo[1,2-a]pyridine-6-carboxamide (Compound 705)

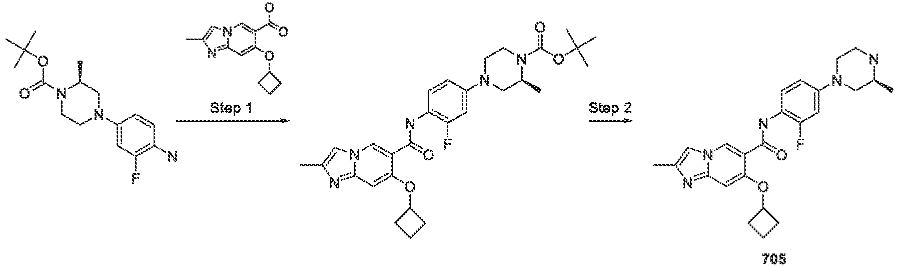

### Step 1: Preparation of tert-butyl (2S)-4-(4-7-cyclobutoxy-2-methylimidazo[1,2-a]pyridine-6-amido-3-fluorophenyl)-2-methylpiperazine-1-carboxylate

The 7-cyclobutoxy-2-methylimidazo[1,2-a]pyridine-6-carboxylic acid (260.18 mg, 1.06 mmol) was suspended in DMF (4mL) and DIPEA (341.36 mg, 2.64 mmol) was added followed by HATU (482.05 mg, 1.27 mmol) . The resulting mixture was stirred for 30 min at rt. After that tert-butyl (2S)-4-(4-amino-3-fluorophenyl)-2-methylpiperazine- 1-carboxylate (326.86 mg, 1.06 mmol) was added in one portion and the reaction mixture was stirring overnight at rt. The precipitate formed was filtered, washed with MeCN (5 ml), MTBE (2mL), dried under vacuo to give pure tert-butyl (2S)-4-(4-7-cyclobutoxy-2-methylimidazo[1,2-a]pyridine-6-amido-3-fluorophenyl)-2-methylpiperazine-1-carboxylate (130.0 mg, Y: 17.9%). ESI-MS (M+H)⁺: 538.4.

### Step 2: Preparation of 7-cyclobutoxy-N-2-fluoro-4-[(3S)-3-methylpiperazin-1-yl]phenyl-2-methylimidazo[1,2-a]pyridine-6-carboxamide

The tert-butyl (2S)-4-(4-7-cyclobutoxy-2-methylimidazo[1,2-a]pyridine-6-mido-3-fluorophenyl)-2-methylpiperazine-1-carboxylate (100.4 mg, 186.75 µmol) was dissolved in DCM (10 mL) and TFA (212.94 mg, 1.87 mmol) was added. The resulting mixture was stirred ar rt overnight and then evaporated to dryness to provide crude material. The residue was triturated with MeCN/MTBE (1/4) and the resulting precipitate was filtered and dried under vacuo to give pure 7-cyclobutoxy-N-2-fluoro-4-[(3S)-3-methylpiperazin-1-yl]phenyl-2-methylimidazo[1,2-a]pyridine-6-carboxamide as TFA salt (13.0 mg, Y:12.6%). ESI-MS (M+H)⁺: 438.0. ¹H NMR (400 MHz, DMSO-*d*6) δ 9.96 (s, 1H), 9.20 (s, 1H), 9.09 (br s, 1H), 8.71 (br s, 1H), 7.90 - 7.83 (m, 2H), 7.11 (s, 1H), 7.01 (d, *J* = 14.0 Hz, 1H), 6.85 (d, *J* = 9.1 Hz, 1H), 5.13 - 4.96 (m, 1H), 3.78 (dd, *J =* 22.4, 13.2 Hz, 2H), 3.16 - 3.02 (m, 3H), 2.93 (t, *J* = 12.3 Hz, 1H), 2.71 (t, *J* = 11.9 Hz, 1H), 2.38 (s, 3H), 2.28 - 2.16 (m, 2H), 1.94 - 1.84 (m, 1H), 1.77 - 1.68 (m, 1H), 1.23 (d, *J* = 6.6 Hz, 3H).

### Example 96. rel-7-ethoxy-2-methyl-N-16-[(3R)-3-(methylamino)pyrrolidin-1-yl]pyridazin-3-yl}imidazo[1,2-a]pyridine-6-carboxamide and rel-7-ethoxy-2-methyl-N-{6-[(3S)-3-(methylamino)pyrrolidin-1-yl]pyridazin-3-yl}imidazo[1,2-a]pyridine-6-carboxamide (Compound 706 and 707)

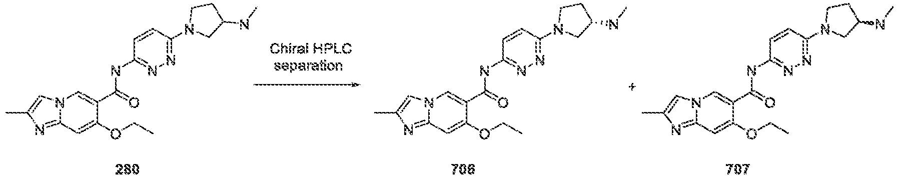

Column: Chiralpak IA (250x4.6 mm, 5 mkm)-1
Mobile Phase: Hexane(0.1% EDA):IPA:MeOH, 50:25:25
Flow Rate: 0.6 ml/min
ESI-MS (M+H)⁺: 396.4.
RetTime (enantiomer A) = 45.24 min; RetTime (enantiomer B) = 67.97 min.

### Example 97. Preparation of Starting Material: tert-butyl (2R,6S)-4-(6-aminopyridazin-3-yl)-2,6-dimethylpiperazine-1-carboxylate

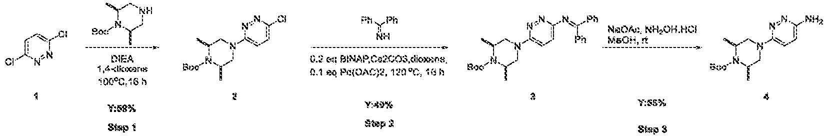

### Step 1: Preparation of tert-butyl (2R,6S)-4-(6-chlompyridazin-3-yl)-2,6-dimethyl piperazine-1-carboxylate.

A mixture of 3,6-dichloropyridazine (100 g, 0.67 mol), DIEA (174 g, 1.34 mol) and tert-butyl (2R,6S)-2,6-dimethylpiperazine-1-carboxylate (144 g, 0.67 mol) in 1,4-dioxane (1.5 L) was stirred at 100 °C for 16 h. The mixture was cooled to room temperature and concentrated in vacuum. The residue was recrystallized with MTBE to give tert-butyl(2R,6S)-4-(6-chloropyridazin-3-yl)-2,6-dimethylpiperazine -1-carboxylate (130 g, 59%yield) as a white solid. ¹H NMR (400 MHz, CDCl₃) δ 7.21 (d, *J =* 9.5 Hz, 1H), 6.89 (d, *J* = 9.5 Hz, 1H), 4.34 - 4.27 (m, 2H), 4.15 (d, *J* = 13.2 Hz, 2H), 3.21 (dd, *J =* 13.1, 4.4 Hz, 2H), 1.48 (s, 9H), 1.25 (d, *J* = 6.9 Hz, 6H). ESI-MS (M+H)⁺: 327.2.

### Step 2: Preparation of butyl (2R,6S)-4-(6-((diphenylmethylene)amino)pyridazin-3-yl)-2,6-dimethylpiperazine-1-carboxylate.

To a solution of tert-butyl(2R,6S)-4-(6-chloropyridazin-3-yl)- 2,6-dimethylpiperazine-1-carboxylate (100 g, 306 mmol), diphenylmethanimine (66.5 g, 367 mmol), BINAP (38.1 g, 61.2 mmol) and Cs₂CO₃ (299 g, 918 mmol) in 1,4-dioxane (2.0 L) was added Pd(OAc)₂ (6.87 g, 30.6 mmol) at rt and the mixture was stirred at 120 °C for 16 h. The reaction mixture was diluted with ethyl acetate (3 L) and washed with water (1.5 L) and brine (1.5 L). The organic layer was dried over sodium sulfate, filtered and concentrated under reduced pressure. The residue was purified by silica gel column chromatography (15% EA in PE) to give tert-butyl (2R,6S)-4-(6-((diphenylmethylene)amino) pyridazin-3-yl)-2,6-dimethylpiperazine-1-carboxylate (70 g, 49%yield) as a yellow solid. ¹H NMR (400 MHz, CDC₃) δ 7.81 (d, *J* = 7.0 Hz, 2H), 7.51 - 7.28 (m, 6H), 7.21 - 7.19 (m, 2H), 6.79 (d, *J* = 9.5 Hz, 1H), 6.71 (d, *J* = 9.5 Hz, 1H), 4.29 - 4.21 (m, 2H), 4.03 (d, *J* = 13.0 Hz, 2H), 3.10 (dd, *J* = 13.0, 4.5 Hz, 2H), 1.48 (s, 9H), 1.24 (d, J = 6.8 Hz, 6H).

### Step 3: Preparation of tert-butyl (2R, 6S)-4-(6-aminopyridazin-3-yl)-2,6-dimethylpiperazine-1-carboxylate.

The mixture of tert-butyl (2R,6S)-4-(6-((diphenylmethylene)amino)pyridazin-3-yl)-2,6-dimethylpiperazine-1-carboxylate (70 g, 148 mmol), NaOAc (36.5 g, 445 mmol) and NH₂OH.HCl (51.6 g, 742 mmol) in MeOH (500 mL) was stirred at 25 °C for 15 min. The mixture was adjusted to pH 4 with solid Citric Acid, then the mixture was diluted with brine (300 mL) and extracted with EtOAc (300 mL x2). Then the aqueous phase was adjusted to pH 10 with solid Na₂CO₃ and extracted with EtOAc (300 mL x2). The organic layer was dried over sodium sulfate, filtered and concentrated under reduced pressure to give tert-butyl (2R,6S)-4-(6-aminopyridazin-3-yl)-2,6-dimethylpiperazine-1-carboxylate (25 g, 55%yield) as a white solid. ¹H NMR (400 MHz, DMSO-*d*₆) δ 7.15 (d, *J* = 9.6 Hz, 1H), 6.76 (d, *J* = 9.5 Hz, 1H), 5.69 (s, 2H), 4.14 - 4.06 (m, 2H), 3.84 (d, *J* = 12.6 Hz, 2H), 2.78 (dd, *J* = 12.6, 4.3 Hz, 2H), 1.42 (s, 9H), 1.20 (d, *J =* 6.8 Hz, 6H). ESI-MS (M+H)⁺: 308.1

### Example 98. Preparation of Starting Material: tert-butyl 7-(6-aminopyridazin-3-yl)-4,7-diazaspiro[2.5]octane-4-carboxylate.

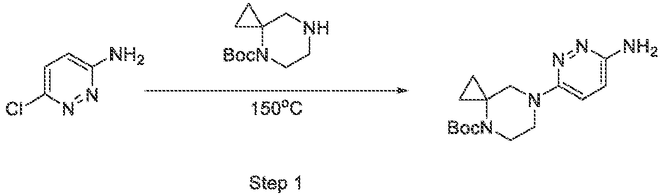

### Step 1: preparation of tert-butyl 7-(6-aminopyridazin-3-yl)-4,7-diazaspiro[2.5]octane-4-carboxylate.

A mixture of 6-chloropyridazin-3-amine (306 mg, 2.36 mmol) and tert-butyl 4,7-diazaspiro[2.5]octane-4-carboxylate (500 mg, 2.36 mmol) was stirred at 150°C for 5 hours. The mixture was purified by silica gel column chromatography (EA) to provide the title compound (200 mg, yield: 27.8%). ESI-MS (M+H)⁺:306.1.

### Example 99. Preparation of Starting Material: tert-butyl 4-(6-aminopyridazin-3-yl)-3,6-dihydropyridine-1(2H)-carboxylate.

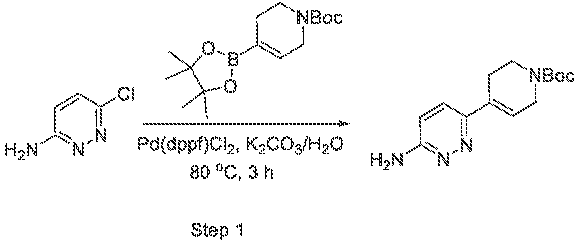

### Step 1: Preparation of tert-butyl 4-(6-aminopyridazin-3-yl)-3,6-dihydropyridine-1(2H)-carboxylate.

To a mixture of tert-butyl 4-(4,4,5,5-tetramethyl-1,3,2-dioxaborolan-2-yl)-3,6-dihydropyridine-1(2H)-carboxylate (1.0 g, 3.24 mmol) and 6-chloropyridazin-3-amine (626 mg, 4.85 mmol) in 1,4-dioxane (20 mL) and water (4 mL) were added K2CO₃ (1.34 g, 9.72 mmol) and Pd(dppf)Cl₂ (236 mg, 0.32 mmol). The mixture was stirred at 80°C for 3 h. The reaction mixture was concentrated in vacuo. The residue was purified by silica gel column chromatography (PE/ EA=2:1) to give title product (700 mg, yield 79.3%) as a brown solid. ESI-MS (M+H)⁺: 277.3. ¹H NMR (400 MHz, DMSO-*d*₆) δ 7.54 (d, *J* = 9.3 Hz, 1H), 6.75 (d, *J* = 9.3 Hz, 1H), 6.36 (br s, 3H), 4.01 (br s, 2H), 3.52 (t, *J* = 5.5 Hz, 2H), 2.65 - 2.55 (m, 2H), 1.43 (s, 9H).

### Example 100. Preparation of Starting Material: tert-butyl 4-(6-aminopyridazin-3-yl)piperidine-1-carboxylate.

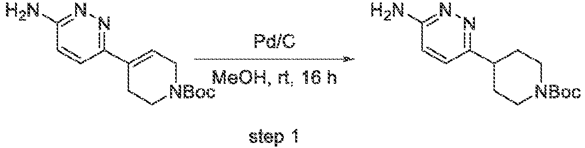

### Step 1: Preparation of tert-butyl 4-(6-aminopyridazin-3-yl)piperidine-1-carboxylate.

To a solution of tert-butyl 4-(6-aminopyridazin-3-yl)-3,6-dihydropyridine-1(2H)-carboxylate (1 g, 3.62 mmol) in MeOH (10 mL) was added Pd/C (100 mg, 10% w.t.). The mixture was stirred at rt for 16 h under H₂. The mixture was filtered and the filtrate was concentrated in vacuo to give title product (1 g, 99.28%) as a brown oil. ESI-MS (M+H)⁺:279.2. ¹H NMR (400 MHz, DMSO-*d*₆) δ 7.19 (d, *J* = 9.1 Hz, 1H), 6.73 (d*, J* = *9.1* Hz, 1H), 6.13 (s, 2H), 4.22 - 3.83 (m, 4H), 2.84 - 2.81 (m, 1H), 1.77 (d, *J =* 13.8 Hz, 2H), 1.56 - 1.49 (m, 2H), 1.41 (s, 9H).

### Example 101. Preparation of Starting Material: 7-cyclopropoxy-2-methylimidazo[1,2-a]pyridine-6-carboxylic acid.

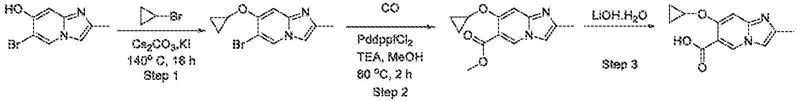

### Step 1: Preparation of 6-bromo-7-cyclopropoxy-2-methylimidazo[1,2-a]pyridine.

To a solution of 6-bromo-2-methylimidazo[1,2-a]pyridin-7-ol (500 mg, 2.25 mmol) in DMA (15 mL) were added bromocyclopropane (2.15 g, 22.50 mmol), K2CO₃ (2.2 g, 6.75 mmol) and KI (187 mg 1.6 mmol). The mixture was stirred at 140 °C overnight. The reaction mixture was diluted with water (20 mL), extracted with EtOAc (40 mLx2). The organic layer was washed with brine (50 mL), dried over sodium sulfate, filtered and concentrated in vacuum. The residue was purified by C18 flash (0.1% NH₃.H₂O in water / CH₃CN) to give title product (660 mg, Y: 62 %) as a gray solid. ESI-MS (M+H) ⁺: 267.0. ¹H NMR (400 MHz, CDCl₃) δ 8.06 (s, 1H), 7.16 (s, 1H), 7.08 (s, 1H), 3.78 - 3.71 (m, 1H), 2.33 *(d, J= 0.8* Hz, 3H), 0.81 - 0.78 (m, 4H).

### Step 2: Preparation of methyl 7-cyclopropoxy-2-methylimidazo[1, 2-a]pyridine-6-carboxylate.

To a solution of 6-bromo-7-cyclopropoxy-2-methylimidazo[1,2-a]pyridine (640 mg, 2.40 mmol) in MeOH (10 mL) were added TEA (3.6 g, 36.00 mmol) and Pd(dppf)Cl₂ (176 mg, 0.24 mmol). The mixture was charged with CO for three times and stirred at 60 °C for 16 h. The reaction mixture was diluted with water (50 mL), extracted with ethyl acetate (100 mL). The organic layer was washed with brine (50 mL), dried over sodium sulfate, filtered and concentrated in vacuum to give title product (410 mg, crude) as a gray solid, which was used to next step without further purification. ESI-MS (M+H)⁺:246.9.

### Step 3: Preparation of 7-cyclopropoxy-2-methylimidazo[1.2-a]pyridine-6-carboxylic acid.

To a solution of methyl 7-cyclopropoxy-2-methylimidazo[1,2-a]pyridine-6-carboxylate (400 mg, 1.63 mmol) in MeOH (8 mL) and H₂O (4 mL) was added LiOH (77 mg, 3.26 mmol), the mixture was stirred for 1h at rt. After concentration, the residue was diluted with water (10 mL) and adjusted pH to 5~6 with 1M HCl. The precipitate was collected by filtration, washed with water and dried under vacuum to provide title compound (240 mg, 63.6%) as a white solid, ESI-MS (M+H)⁺:233.0. ¹H NMR (400 MHz, DMSO-*d*₆) δ 9.27 (s, 1H), 7.91 (s, 1H), 7.49 (s, 1H), 4.29 - 4.08 (m, 1H), 2.44 *(d, J=* 1.0 Hz, 3H), 0.97 - 0.92 (m, 2H), 0.83 - 0.76 (m, 2H).

### Example 102. Preparation of Starting Material: tert-butyl (S)-4-(5-aminopyrazin-2-yl)-2-ethylpiperazine-1-carboxylate.

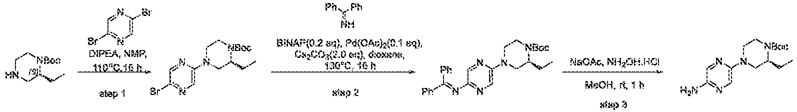

### Step 1: tert-butyl (S)-4-(5-bromopyrazin-2-yl)-2-ethylpiperazine-1-carboxylate.

To a suspension of tert-butyl (S)-2-ethylpiperazine-1-carboxylate (4.8 g, 22.40 mmol) and 2,5-dibromopyrazine (5.34 g, 22.40 mmol) in NMP (50 mL) was added DIEA (8.67 g, 67.2 mmol), and the reaction mixture was stirred for 16 h at 110 °C. The mixture was diluted with water (100 mL), extracted with EA (80 mL x3). The combined organic was washed with brine, dried over Na₂SO₄ and concentrated in vacuo. The crude was purified by silica gel column (PE/EA = 3:1) to provide title product (6.2 g, Y: 74.5 %) as a yellow solid. ESI-MS (M+H)⁺: 371.2. ¹H NMR (400 MHz, CDCl₃) δ 8.11 (d, J = 4.0 Hz, 1H), 7.84 (d, J = 4.0 Hz, 1H), 4.16 - 4.08 (m, 2H), 4.08 - 3.96 (m, 2H), 3.19 - 3.08 (m, 2H), 3.05 - 2.93 (m, 1H), 1.72 - 1.60 (m, 1H), 1.56 - 1.51 (m, 1H), 1.48 (s, 9H), 0.90 (t, J = 8 Hz, 3H).

### Step 2: tert-butyl (S)-4-(5-((diphenylmethylene)amino)pyrazin-2-yl)-2-ethylpiperazine-1-carboxylate.

To a solution of *tert*-butyl (S)-4-(5-((diphenylmethylene)amino)pyrazin-2-yl)-2-ethylpiperazine-1-carboxylate (5 g, 13.48 mmol) in 1,4-dioxane (70 mL) were added diphenylmethanimine (2.92 g, 16.17 mmol), Pd(OAc)₂ (421.5 mg, 1.88 mmol), BINAP (2.35 g, 3.77 mmol) and Cs₂CO₃ (12.68 g, 37.74 mmoL). The mixture was stirred at 130 °C for 16 h. The mixture was concentrated in vacuo. The crude was purified by silica gel column (EtOAc / PE =3:1) to give title product (6 g, Y: 94.3%) as a grey solid. ESI-MS (M+H)⁺: 472.3.

### Step 3: tert-butyl (S)-4-(5-aminopyrazin-2-yl)-2-ethylpiperazine-1-carboxylate.

To a solution of tert-butyl (S)-4-(5-((diphenylmethylene)amino)pyrazin-2-yl)-2-ethylpiperazine-1-carboxylate (5.8 g, 13.48 mmol) in MeOH (80 mL) were added NaOAc (3.02 g, 36.86 mmol) and NH₂OH·HC1 (4.27 g, 61.44 mmol). The mixture was stirred at r.t. for 1 h. The mixture was concentrated in vacuo. The crude was purified by silica gel column (EtOAc / PE=3:1) to give title product (3.47 g, Y: 91.8%) as a grey solid. ESI-MS (M+H)⁺: 308.2. ¹H NMR (400 MHz, DMSO-*d*₆) δ 7.66 (s, 1H), 7.57 (s, 1H), 5.55 (s, 2H), 4.00 - 3.96 (m, 1H), 3.87 - 3.79 (m, 3H), 3.07 - 2.97 (m, 1H), 2.72 - 2.66 (m, 1H), 2.59 - 2.54 (m, 1H), 1.69 - 1.56 (m, 2H), 1.41 (s, 9H), 0.83 (t, J = 7.4 Hz, 3H).

### Example 103. Preparation of Starting Material: tert-butyl (S)-4-(6-aminopyridazin-3-yl)-6-methyl-3,6-dihydropyridine-1(2H)-carboxylate.

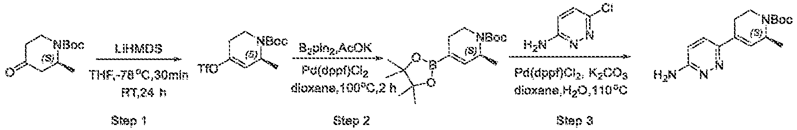

### Step 1: Preparation of tert-butyl (S)-6-methyl-4-(((trifluoromethyl)sulfonyl)oxy)-3,6-dihydropyridine-1(2H)-carboxylate.

To a solution of tert-butyl (S)-2-methyl-4-oxopiperidine-1-carboxylate (5.0 g, 23.47 mmol) in dry THF (50 mL) was added LiHMDS (28.16 mL, 28.16 mmol, 1.0 M) dropwise at - 78 °C. The mixture was stirred at this temperature for 30 min. 1,1,1-trifluoro-N-phenyl-N-((trifluoromethyl)sulfonyl)methanesulfonamide (10.89 g, 30.51 mmol) in dry THF (20 mL) was added to the solution. The mixture was allowed to warm to room temperature and stirred at rt for 24 h. The mixture was diluted with water (40 mL) and extracted with EtOAc (50 mLx3). The combined organic layer was washed with brine, dried over Na₂SO₄ and concentrated in vacuo. The crude was purified by silica gel column chromatography (EA/PE=1:20) to give title product (3 g, 37.05 %) as a yellow oil. ESI-MS (M -t-Bu +H)⁺: 290.0.

### Step 2: Preparation of tert-butyl (S)-6-methyl-4-(4, 4, 5, 5-tetramethyl-1, 3, 2-dioxaborolan-2-yl)-3,6-dhydropyridine-1(2H)-carboxylate.

To a solution of tert-butyl (S)-6-methyl-4-(((trifluoromethyl)sulfonyl)oxy)-3,6-dihydropyridine-1(2H)-carboxylate (3 g, 8.70 mmol) in 1,4-dioxane (30 mL) were added B₂Pin₂ (5.5 g, 21.75 mmol) and KOAc (3 g, 30.45 mmol). The mixture was charged with N₂ for three times and stirred at 100 °C for 2 h. The reaction mixture was diluted with water (30 mL) and extracted with EtOAc (50 mLx3). The organic layer was washed with brine, dried over Na₂SO₄ and concentrated in vacuo. The crude was purified by silica gel column chromatography (EA/PE=1:10) to give title product (1.2 g, 42.86 %) as a yellow oil. ESI-MS (M-56 +H)⁺:268.2.

### Step 3: Preparation of tert-butyl (S)-4-(6-aminopyridazin-3-yl)-6-methyl-3,6-dihydropyridine-1(2H)-carboxylate.

To a solution of tert-butyl (S)-6-methyl-4-(4,4,5,5-tetramethyl-1,3,2-dioxaborolan-2-yl)-3,6-dihydropyridine-1(2H)-carboxylate (1.2 g, 3.72 mmol) in 1,4-dioxane (14 mL) and H₂O (2 mL) were added 6-chloropyridazin-3-amine (480 mg, 3.72 mmol), K2CO₃ (1.5 g, 11.16 mmol) and Pd(dppf)Cl₂ (302 mg, 0.37 mmol). The mixture was charged with N₂ for three times and stirred at 110 °C for 16 h. The reaction mixture was diluted with water (20 mL) and extracted with EA (30 mLx3). The organic layer was washed with brine, dried over Na₂SO₄ and concentrated in vacuo. The crude was purified by silica gel column chromatography (EA/PE=1:3) to give title product (400 mg, 37.14 %) as a yellow solid. ESI-MS (M+H)⁺: 291.2.

### Example 104. Preparation of Starting Material: tert-butyl (2S)-4-(6-aminopyridazin-3-yl)-2-methylpiperidine-1-carboxylate.

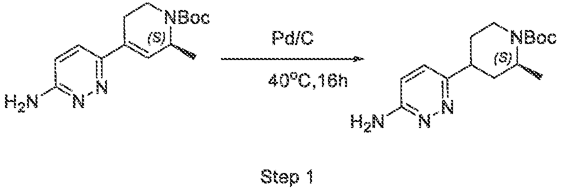

### Step 1: Preparation of tert-butyl (2S)-4-(6-aminopyridazin-3-yl)-2-methylpiperidine-1-carboxylate.

To a mixture of tert-butyl (S)-4-(6-aminopyridazin-3-yl)-6-methyl-3,6-dihydropyridine-1(2H)-carboxylate (1 g, 3.45 mmol) in MeOH (10 mL) was added Pd/C (200 mg, 10% w.t), the mixture was charged with H₂ for three times and stirred at 40°C for 16 h. The mixture was filtered and the filtrate was concentrated in vacuo to give title product (400 mg, crude) as a yellow solid. ESI-MS (M+H⁺): 293.1.

### Example 105. Preparation of Starting Material: tert-butyl (2R,6S)-4-(6-aminopyridin-3-yl)-2,6-dimethylpiperazine-1-carboxylate.

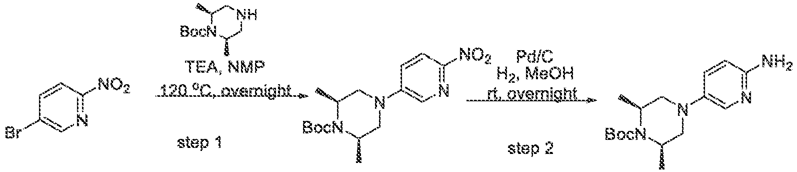

### Step 1: Preparation of tert-butyl (2R,6S)-2,6-dimethyl-4-(6-nitropyridin-3-yl)piperazine-1-carboxylate.

To mixture of 5-bromo-2-nitropyridine (2.50 g, 12.40 mmol) in NMP (15 mL) were added tert-butyl (2R,6S)-2,6-dimethylpiperazine-1-carboxylate (1.64 g, 7.66 mmol) and TEA (2.49 g, 24.8 mmol), and the mixture was stirred at 120 °C overnight. The mixture was diluted with water (50 mL), extracted with EA (50 mL x 3). The combined organic layer was washed with brine (50 mLx2), dried over anhydrous Na₂SO₄, filtered and concentrated. The residue was purified by silica gel column chromatography (PE/EA= 100/1 to 5/1) to afford tert-butyl (2R,6S)-2,6-dimethyl-4-(6-nitropyridin-3-yl)piperazine-1-carboxylate (2.3 g, 55.3% yield) as a yellow solid. ESI-MS (M+H⁺):337.1.

### Step 2: Preparation of tert-butyl (2R, 6S)-4-(6-aminopyridin-3-yl)-2, 6-dimethylpiperazine-1-carboxylate.

To a mixture of tert-butyl (2R,6S)-2,6-dimethyl-4-(6-nitropyridin-3-yl)piperazine-1-carboxylate (2.30 g, 6.84 mmol) in MeOH (20 mL) was added Pd/C (10%) (230 mg), the mixture was stirred under H₂ atmosphere overnight. The mixture was filtered and the filtrate was concentrated under vacuum to afford tert-butyl (2R,6S)-4-(6-aminopyridin-3-yl)-2,6-dimethylpiperazine-1-carboxylate (1.1 g, 52.5% yield) as a yellow solid. ESI-MS (M+H⁺):307.2.

### Example 106. Preparation of Starting Material: tert-butyl (S)-4-(6-amino-5-methylpyridazin-3-yl)-2-methylpiperazine-1-carboxylate.

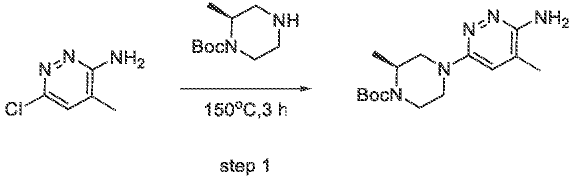

### Step 1: Preparation of tert-butyl (S)-4-(6-amino-5-methylpyridazin-3yl)-2-methylpiperazine-1-carboxylate.

A solution of 6-chloro-4-methylpyridazin-3-amine (0.80 g, 5.59 mmol) in tert-butyl (S)-2-methylpiperazine-1-carboxylate (4.40 g, 22.37 mmol) was stirred at 150 °C for 3 h. The crude was purified by C18 flash (0.1% FA in water / CH₃CN) to give title product (0.90 g, 53%) as a yellow solid. ESI-MS (M+H)⁺:308.1. ¹H NMR (400 MHz, CDCl₃) δ 6.72 (s, 1H), 4.31 - 4.29 (m, 3H), 3.96 - 3.94 (m, 2H), 3.80 - 3.79 (m, 1H), 3.27 - 3.17 (m, 1H), 3.15 - 3.02 (m, 1H), 2.95 - 2.84 (m, 1H), 2.15 (s, 3H), 1.48 (s, 9H), 1.23 (d, *J* = 6.8 Hz, 3H). **Example 107. Preparation of Starting Material: tert-butyl 6-methyl-4-(4, 4,** 5, 5-**tetramethyl-1, 3,2-dioxaborolan-2-yl)-3,6-dihydropyridine-1(2H)-carboxylate.**

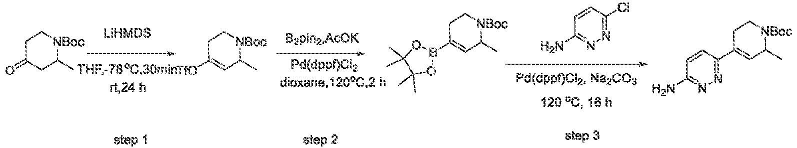

### Step 1: Preparation of tert-butyl 6-methyl-4-(((trifluoromethyl)sulfonyl)oxy)-3,6-dihydropyridine-1(2H)-carboxylate.

To a solution of 1-(tert-butoxycarbonyl)-2-methylpiperidin-4-one (10.00 g, 47 mmol) in THF (100 mL) was added LiHMDS (56 mL, 56 mmol, 1.0 M in THF) at -78° C. The mixture was stirred for 10 min, then a solution of N-phenylbis(trifluoromethanesulfonimide) (19 g, 56 mmol) in THF (50 mL) was added dropwise to the mixture. The reaction mixture was gradually warmed to room temperature and stirred for 16 hours. The mixture was diluted with water (50 mL), extracted with EA (80 mLx3). The organic layer was washed with brine, dried over Na₂SO₄ and concentrated in vacuo. The residue was purified by silica gel column chromatography (PE/EA=5:1) to give the title compound (16 g, crude) as a yellow oil.

### Step 2: Preparation of tert-butyl 6-methyl-4-(4. 4, 5, 5-tetramethyl-1, 3, 2-dioxaborolan-2-yl)-3, 6-dihydropyridine-1(2H)-carboxylate.

A solution of tert-butyl 6-methyl-4-(((trifluoromethyl)sulfonyl)oxy)-3,6-dihydropyridine-1(2H)-carboxylate(5 g, 14.50 mmol), B₂Pin₂ (7.5 g, 29.00 mmol), KOAc (4.3 g, 43.50 mmol) and Pd(dppf)Cl₂ (1 g, 1.45 mmol) in 1,4-dioxane (50 mL) was stirred at 120 °C for 16 h under N₂.The mixture was concentrated in vacuo. The residue was purified by silica gel column chromatography (PE: EA=5:1) to give tittle product (4 g, crude) as a yellow oil. ¹H NMR (400 MHz, CDCl₃) δ 6.40 (d, *J* = 31.5 Hz, 1H), 4.42 - 4.41 (m, 1H), 4.28 - 3.87 (m, 1H), 2.11 - 2.08 (m, 3H), 1.45 (s, 9H), 1.26 - 1.24 (m, 15H).

### Step 3: Preparation of tert-butyl 4-(6-aminopyridazin-3-yl)-6-methyl-3,6-dihydropyridine-1 (2H)-carboxylate.

A mixture of 6-chloropyridazin-3-amine (670 mg, 5.20 mmol ), tert-butyl 6-methyl-4-(4,4,5,5-tetramethyl-1,3,2-dioxaborolan-2-yl)-3,6-dihydropyridine-1(2H)-carboxylate (2 g, 6.24 mmol), Pd(dppf)Cl₂ (377 mg, 0.52 mmol) and Na₂CO₃ (2.15 g, 15.60 mmol ) in 1,4-dioxane (20 mL) and H₂O (4 mL) was stirred at 120 °C for 16 h under N₂ atmosphere. After cooling to rt, the mixture was diluted with water (20 mL), extracted with EA (20 mLx3). The organic layer was washed with brine, dried over Na₂SO₄ and concentrated in vacuo. The residue was purified by column chromatography (SiO₂, Ethyl acetate /Petroleum ether=0% to 50%) to give title product (200 mg, yield: 13.21%) as a yellow solid. ESI-MS (M+H) ⁺: 291.0 ¹H NMR (400 MHz, CDCl₃) δ 7.49 - 7.33 (m, 1H), 6.74 (d, *J=* 9.2 Hz, 1H), 6.34 - 6.16 (m, 1H), 4.89 - 4.56 (m, 3H), 3.10 - 2.68 (m, 2H), 1.49 (s, 9H), 1.27 (d, *J=* 6.8 Hz, 3H).

### Example 108. Preparation of Starting Material: 7-ethoxy-2-methylimidazo [1,2-a] pyrimidine-6-carboxylic acid.

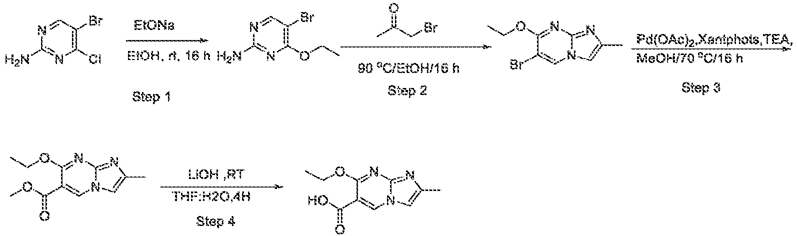

### Step 1: Preparation of 5-bromo-4-ethoxypyrimidin-2-amine.

To a mixture of 5-bromo-4-chloropyrimidin-2-amine (6.4 g, 30.92 mmol) in EtOH (130 mL) was added EtONa (3.154 g, 46.38 mmol) and the mixture was stirred at rt for 6 h. The mixture was concentrated in vacuo. The residue was diluted with water (100 mL), extracted with DCM (100 mLx3). The organic phase was washed with brine, dried over sodium sulfate and concentrated in vacuo to give title product (6.5 g, Y: 97%) as a white solid. ESI-MS (M+H⁺):219.9.

### Step 2: Preparation of 6-bromo-7-ethoxy-2-methylimidazo [1,2-a]pyrimidine.

A mixture of 5-bromo-4-ethoxypyrimidin-2-amine (14.4 g, 66.36 mmol) and 1-bromopropan-2-one (22.73 g, 165.90 mmol) in EtOH(100 mL) was stirred at 80 °C for 16 h. The mixture concentrated in vacuo. The residue was basified with 2M NaOH and the solid was filtered and further purified by flash chromatography (silica gel, DCM:MeOH= 10: 1) to give title product (6 g,Y:28%) as a white solid. ESI-MS (M+H⁺):256.2. ¹H NMR (400 MHz, DMSO-*d*₆) δ 9.12 (s, 1H), 7.35 (s, 1H), 4.42 (q, *J* = 7.0 Hz, 2H), 2.26 (s, 3H), 1.38 (t, *J=* 7.1 Hz, 3H).

### Step 3: preparation of methyl 7-ethoxy-2-methylimidazo [1,2-a]pyrimidine-6-carboxylate.

To a mixture of 6-bromo-7-ethoxy-2-methylimidazo[1,2-a]pyrimidine (5.5 g, 21.48 mmol) in MeOH (400 mL) were added and Pd(OAc)₂ (481 mg, 2.15 mmol), Xantphos (2.485 g, 4.30 mmol ) and TEA (400 mL). The resulting mixture was stirred overnight at 70 °C under CO for 16 h. The mixture was allowed to cool down to room temperature and concentrated. The residue was diluted with water and extracted with DCM (100 mLx3). The organic layer was washed with brine, dried over sodium sulfate and concentrated to provide the crude product (6 g, crude). The crude was used to next step without further purification. ESI-MS (M+H)⁺: 236.0.

### Step 4: Preparation of 7-ethoxy-2-methylimidazo [1,2-a] pyrimidine-6-carboxylic acid.

To a mixture of methyl 7-ethoxy-2-methylimidazo [1,2-a]pyrimidine-6-carboxylate (5 g, 21.22 mmol) in THF/H₂O (200:50 mL), LiOH (2.128 g, 106.383 mmol) was added and the mixture was stirred at rt for 4 h. The mixture was concentrated in vacuo, the residue was purified with reversed phase column chromatography to give title product (800 mg, Y: 17%) as a white solid. ESI-MS (M+H⁺):222.0. ¹H NMR (400 MHz, DMSO-*d*₆) δ 9.29 (d,*J* = 7.5 Hz, 1H), 7.47 (s, 1H), 4.42 *(q, J =* 7.0 Hz, 2H), 2.26 (s, 3H), 1.36 (t, *J* = 7.0 Hz, 3H).

### Example 109. Preparation of Starting Material: 6-methoxy-2-methylpyrazolo[1,5-alpyridine-5-carboxylic acid.

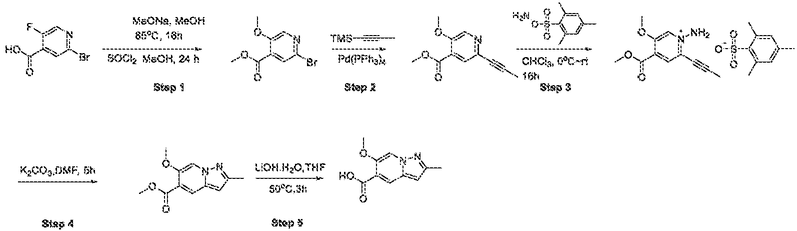

### Step 1: Preparation of 2-bromo-5-methoxyisonicotinic acid

To a mixture of 2-bromo-5-fluoroisonicotinic acid (6.0 g, 0.027 mol) in MeOH (30 mL) was added MeONa (4.4 g, 0.082 mol). The mixture was stirred at 65 °C for 16 h. The mixture was concentrated in vacuo. The residue was dissolved in MeOH (30 mL), SOCl₂ (9.8 g, 0.082 mol) was added dropwise to the mixture and stirred at rt for 16 h. After concentration, the residue was diluted with EA (300 mL), washed with water and brine, dried with Na₂SO₄ and concentrated in vacuo. The crude product was purified by silica gel column (PE/EA= 5:1) to afford title product (6 g, 94.8 %) as a white solid. ESI-MS (M+H)⁺: 245.9.

### Step 2: Preparation of methyl 5-methoxy-2-(prop-1-yn-1yl)isonicotinate.

To a mixture of methyl 2-bromo-5-methoxyisonicotinate (6.0 g, 24.39 mmol) in toluene (60 mL) were added trimethyl(prop-1-yn-1-yl)silane (2.7 g, 24.39 mmol), CuI (1.3 g, 7.31 mmol), Pd(PPh₃)₄ (2.8 g, 2.43 mmol), TEA (7.3 g, 73.17 mmol) and TBAF (6.3 g, 24.39 mmol). The mixture was stirred at 50 °C for 12 h. LCMS showed the reaction was completed. The mixture was concentrated in vacuo, the residue was diluted with water (50 mL), extracted with EA (60 mLx2). The organic layer was washed with brine, dried over Na₂SO₄ and concentrated in vacuo. The residue was purified by silica gel column (PE: EA=4:1) to give the title product (1.7 g, Y: 33%) as a yellow solid. ESI-MS (M+H⁺):206.0. ¹H NMR (400 MHz, CDCl₃) δ 8.35 (s, 1H), 7.66 (s, 1H), 4.01 (s, 3H), 3.92 (s, 3H), 2.07 (s, 3H).

### Step 3 and 4: Preparation of methyl 6-methoxy-2-methylpyrazolo[1,5-a]pyridine-5-carboxylate.

To a mixture of methyl 5-methoxy-2-(prop-1-yn-1-yl)isonicotinate (1.7 g, 8.29 mmol) in CHCl₃ (50 mL) was added O-(mesitylsulfonyl)hydroxylamine (5.3 g, 24.87 mmol). The mixture was stirred at 25°C for 16 h. The mixture was concentrated in vacuo. The residue was diluted with DMF (30 mL), K₂CO₃ (2.1 g, 14.92 mmol) was added and the mixture was stirred at 25 °C for 5 h. LCMS showed the reaction was completed. The mixture was diluted with water (50 mL), extracted with EA (50 mL x2). The organic layer was washed with brine, dried with Na₂SO₄ and concentration in vacuo. The residue was purified by silica gel column (PE: EA=3:1) to give the title product (500 mg, Y: 27%) as a yellow solid. ESI-MS (M+H⁺):221.0.

### Step 5: Preparation of 6-methoxy-2-methylpyrazolo[1,5-a]pyndine-5-carboxylic acid.

To a mixture of methyl 6-methoxy-2-methylpyrazolo[1,5-a]pyridine-5-carboxylate (500 mg, 2.27mmol) in THF (5 mL) and H₂O (5 mL) was added LiOH (104 mg, 4.54 mmol). The mixture was stirred at 50 °C for 3 h. LCMS showed the reaction was completed. The reaction mixture was concentrated in vacuo to remove most THF. The mixture was adjusted to pH=5 with 1M HCl. The precipitate was filtered and dried under vacuum to give title product (180 mg, Y: 38%) as a white solid. ESI-MS (M+H⁺):207.1. ¹H NMR (400 MHz, CDCl₃) δ 8.32 (s, 1H), 8.21 (s, 1H), 6.48 (s, 1H), 4.05 (s, 3H), 2.49 (s, 3H).

### Example 110. Preparation of Starting Material: tert-butyl 4-(6-aminopyridazin-3-yl)-6,6-dimethyl-3,6-dihydropyridine-1(2H)-carboxylate.

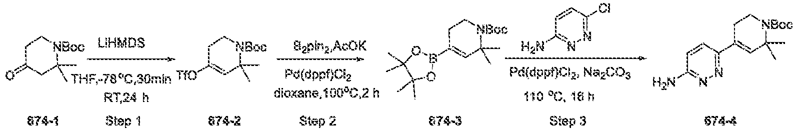

### Step 1: Preparation of tert-butyl 6,6-dimethyl-4-(((trifluoromethyl)sulfonyl)oxy)-3,6-dihydropyridine-1(2H)-carboxylate.

To a solution of tert-butyl 2,2-dimethyl-4-oxopiperidine-1-carboxylate (1 g, 4.41 mmol) in dry THF (10 mL) was added LiHMDS (5.29 mL, 5.29 mmol, 1.0 M in THF) dropwise at - 78 °C. The mixture was stirred at this temperature for 30 min. 1,1,1-trifluoro-N-phenyl-N-((trifluoromethyl)sulfonyl)methanesulfonamide (2.05 g, 5.73 mmol) in dry THF (5 mL) was added. The mixture was allowed to warm to room temperature and stirred at rt for 24 h. The mixture was diluted with H₂O (10 mL) and extracted with EtOAc (50 mLx3). The combined organic layer was washed with brine, dried over Na₂SO₄ and concentrated in vacuo. The crude was purified by silica gel column chromatography (EA/PE=1:20) to give title product (500 mg, 31.66 %) as a yellow oil. ESI-MS (M-100+H)⁺: 260.0. ¹H NMR (400 MHz, CDCl₃) δ 5.80 - 5.73 (m, 1H), 4.09 - 4.04 (m, 2H), 2.41 - 2.36 (m, 2H), 1.49 (s, 6H), 1.46 (s, 9H).

### Step 2: Preparation of tert-butyl 6,6-dimethyl-4-(4,4,5,5-tetramethyl-1,3,2-dioxaborolan-2-yl)-3,6-dihydropyridine-1(2H)-carboxylate.

To a solution of tert-butyl 6,6-dimethyl-4-(((trifluoromethyl)sulfonyl)oxy)-3,6-dihydropyridine-1(2H)-carboxylate (500 mg, 1.39 mmol) in 1,4-dioxane (10 mL) were added B₂Pin₂ (884 mg, 3.48 mmol), Pd(dppf)Cl₂ (102 mg, 0.14 mmol)and KOAc (477 mg, 4.87 mmol). The mixture was charged with N₂ for three times and stirred at 100°C for 2 h. The reaction mixture was diluted with water (10 mL) and extracted with EtOAc (20 mLx3). The organic layer was washed with bine, dried over Na₂SO₄ and concentrated in vacuo. The crude was purified by silica gel column chromatography (EA/PE=1:10) to give title product (200 mg, 42.64 %) as a yellow oil. ESI-MS (M+H)⁺:338.2.

### Step 3: Preparation of tert-butyl 4-(6-aminopyridazin-3-yl)-6,6-dimethyl-3,6-dihydropyridine-1(2H)-carboxylate.

To a solution of tert-butyl 6,6-dimethyl-4-(4,4,5,5-tetramethyl-1,3,2-dioxaborolan-2-yl)-3,6-dihydropyridine-1(2H)-carboxylate (200 mg, 0.59 mmol) in 1,4-dioxane (7 mL) and H₂O (1 mL) were added 6-chloropyridazin-3-amine (76 mg, 0.59 mmol), K2CO₃ (244 mg, 1.77 mmol) and Pd(dppf)Cl₂ (49 mg, 0.06 mmol). The mixture was charged with N₂ for three times and stirred at 110 °C for 16 h. The reaction mixture was diluted with water (10 mL) and extracted with EA (20 mLx3). The organic layer was washed with brine, dried over Na₂SO₄ and concentrated in vacuo. The crude was purified by silica gel column chromatography (EA/PE=1:3) to give title product (70 mg, 38.89 %) as a yellow solid. ESI-MS (M+H)⁺: 305.2.

### Example 111. Preparation of Starting Material: tert-butyl (R)-4-(6-aminopyridazin-3-yl)-6-methyl-3,6-dihydropyridine-1(2H)-carboxylate.

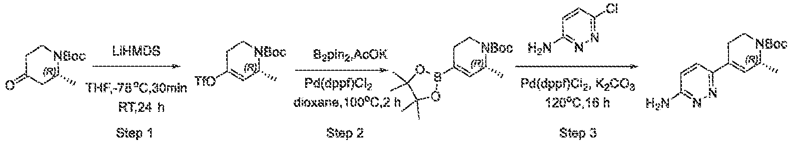

### Step 1: Preparation of tert-butyl (R)-6-methyl-4-(((trifluoromethyl)sulfonyl)oxy)-3,6-dihydropyridine-1(2H)-carboxylate.

To a solution of tert-butyl (R)-2-methyl-4-oxopiperidine-1-carboxylate (3 g, 14.08 mmol) in dry THF (30 mL) was added LiHMDS (16.90 mL, 16.90 mmol, 1.0 M in THF) dropwise at - 78 °C. The mixture was stirred at this temperature for 30 min. 1,1,1-trifluoro-N-phenyl-N-((trifluoromethyl)sulfonyl)methanesulfonamide (6.5 g, 18.30 mmol) in dry THF (30 mL) was added. The mixture was allowed to warm to room temperature and stirred at rt for 24 h. The mixture was diluted with H₂O (50 mL) and extracted with EtOAc (70 mLx3). The combined organic layer was washed with brine, dried over Na₂SO₄ and concentrated in vacuo. The crude was purified by silica gel column chromatography (EA/PE=1:20) to give title product (1.5 g, 30.86 %) as a yellow oil. ESI-MS (M-t-Bu+ACN+H)⁺: 331.1.

### Step 2: Preparation of tert-butyl (R)-6-methyl-4-(4, 4, 5, 5-tetramethyl-1, 3, 2-dioxaborolan-2-yl)-3,6-dihydropyridine-1(2H)-carboxylate.

To a solution of tert-butyl (R)-6-methyl-4-(((trifluoromethyl)sulfonyl)oxy)-3,6-dihydropyridine-1(2H)-carboxylate (1.5 g, 4.18 mmol) in 1,4-dioxane (20 mL) were added B₂pin₂ (2.7 g, 10.45 mmol) Pd(dppf)Cl₂ (300 mg, 0.41 mmol) and KOAc (1.4 g, 14.63 mmol). The mixture was charged with N₂ for three times and stirred at 100°C for 2 h. The reaction mixture was diluted with water (20 mL) and extracted with EtOAc (30 mLx3). The organic layer was washed with brine, dried over Na₂SO₄ and concentrated in vacuo. The crude was purified by silica gel column chromatography (EA/PE=1:10) to give title product (800 mg, 59.26 %) as a yellow oil. ESI-MS (M-Boc+H)⁺:224.0

### Step 3: Preparation of tert-butyl (R)-4-(6-aminopyridazin-3-yl)-6-methyl-3,6-dihydropyridine-1(2H)-carboxylate.

To a solution of tert-butyl (R)-4-(6-aminopyridazin-3-yl)-6-methyl-3,6-dihydropyridine-1(2H)-carboxylate (800 mg, 2.48 mmol) in 1,4-dioxane (14 mL) and H₂O (2 mL) were added 6-chloropyridazin-3-amine (320 mg, 2.48 mmol), K2CO₃ (1 g, 7.44 mmol) and Pd(dppf)Cl₂ (204 mg, 0.25 mmol). The mixture was charged with N₂ for three times and stirred at 120 °C for 16 h. The reaction mixture was diluted with water (10 mL) and extracted with EA (20 mLx3). The organic layer was washed with brine, dried over Na₂SO₄ and concentrated in vacuo. The crude was purified by silica gel column chromatography (EA/PE=1:3) to give title product (400 mg, 55.71 %) as a yellow solid. ESI-MS (M+H)⁺: 291.1.

### Example 112. 6-ethoxy-2-methyl-N-(6-(1,2,3,6-tetrahydropyridin-4-yl)pyridazin-3-yl)-2H-indazole-5-carboxamide formate (Compound 708).

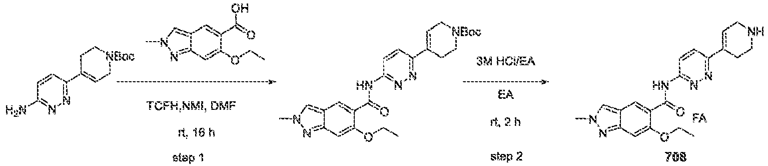

### Step 1: Preparation of tert-butyl 4-(6-(6-ethoxy-2-methyl-2H-indazole-5-carboxamido)pyridazin-3-yl)-3,6-dihydropyridine-1 (2H)-carboxylate.

A mixture of tert-butyl 4-(6-aminopyridazin-3-yl)-3,6-dihydropyridine-1(2H)-carboxylate (150 mg, 0.54 mmol), 6-ethoxy-2-methyl-2H-indazole-5-carboxylic acid (132 mg, 0.60 mmol), TCFH (234 mg, 0.82 mmol) and NMI (134 mg, 1.64 mmol) in ACN (10 mL) was stirred at rt for 16 h. The mixture was diluted with H₂O (20 mL) and extracted with EA (20 mL x 3). The organic phase was washed with brine (20 mL), dried over anhydrous Na₂SO₄ and concentrated in vacuo. The residue was purified by column chromatography (SiO₂, Petroleum ether/Ethyl acetate=5/1 to 1/1) to afford title product (75 mg, 0.15 mmol, 29.18%) as a yellow solid. ESI-MS (M+H⁺); 479.2.

### Step 2: Preparation of 6-ethoxy-2-methyl-N-(6-(1, 2, 3, 6-tetrahydropyridin-4-yl)pyridazin-3-yl)-2H-indazole-5-carboxamide formate.

To a solution of tert-butyl 4-(6-(6-ethoxy-2-methyl-2H-indazole-5-carboxamido)pyridazin-3-yl)-3,6-dihydropyridine-1(2H)-carboxylat (75 mg, 0.16 mmol) in EA (4 mL) was added 3M HCl/EA (4 mL). The mixture was stirred at rt for 2 h. The mixture was diluted with water (15 mL) and extracted with EA (20 mL×3). The aqueous phase was concentrated in vacuo. The residue was purified by prep-HPLC (0.05% FA in water / ACN) to give title product (37 mg, 54.4 %) as a white solid. ESI-MS (M+H⁺): 379.2. ¹H NMR (400 MHz, MeOD-*d*₄) δ 8.67 (d, *J =* 9.5 Hz, 1H), 8.61 (s, 1H), 8.52 (s, 1H), 8.35 (s, 1H), 8.01 (d, *J* = 9.4 Hz, 1H), 7.15 (s, 1H), 6.70 (s, 1H), 4.38 (q, *J* = 7.0 Hz, 2H), 4.20 (s, 3H), 3.90 (s, 2H), 3.46 (t, *J* = 6.1 Hz, 2H), 3.00 - 2.98 (m, 2H), 1.66 (t, *J* = 7.0 Hz, 3H).

### Example 113. 7-ethoxy-2-methyl-N-(6-(1,2,3,6-tetrahydropyridin-4-yl)pyridazin-3-yl)imidazo[1,2-a]pyridine-6-carboxamide hydrochloride (Compound 709).

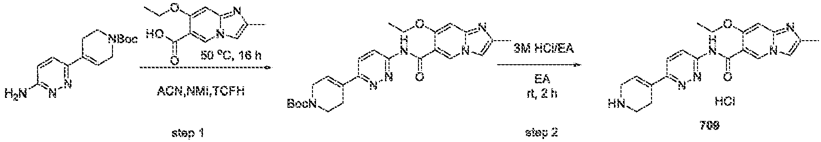

### Step 1: Preparation of tert-butyl 4-(6-(7-ethoxy-2-methylimidazo[1,2-a]pyridine-6-carboxamido)pyridazin-3-yl)-3, 6-dihydropyridine-1 (2H)-carboxylate.

To a solution of tert-butyl 4-(6-aminopyridazin-3-yl)-3,6-dihydropyridine-1(2H)-carboxylate (200 mg, 0.72 mmol) and 7-ethoxy-2-methylimidazo[1,2-a]pyridine-6-carboxylic acid (237.6 mg, 1.08 mmol) in ACN (5 mL) were added NMI (358.4 mg, 4.32 mmol) and TCFH (604.8 mg, 2.16 mmol). The resulting mixture was stirred at 50 °C for 16 h. The reaction mixture was diluted with water (20 mL) and extracted with EtOAc (20 mLx3). The organic layer was washed with brine, dried over Na₂SO₄ and concentrated in vacuo to give title product (150 mg, 47.9 %) as a white solid. ESI-MS (M+H)⁺:479.4. ¹H NMR (400 MHz, CDCl₃) δ 10.95 (s, 1H), 8.99 (s, 1H), 8.58 (d, *J* = 9.4 Hz, 1H), 7.66 (d, *J =* 9.6 Hz, 1H), 7.31 (s, 1H), 6.98 (s, 1H), 6.57 (s, 1H), 4.39 - 4.34 (m, 2H), 4.18 (br s, 2H), 3.68 (br s, 2H), 2.81 (br s, 2H), 2.44 (s, 3H), 1.74 (d, *J* = 7.0 Hz, 3H), 1.50 (s, 9H).

### Step 2: Preparation of 7-ethoxy-2-methyl-N-(6-(1,2,3, 6-tetrahydropyridin-4-yl)pyridazin-3-yl)imidazo[1,2-a]pyridine-6-carboxamide hydrochloride.

To a solution of tert-butyl 4-(6-(7-ethoxy-2-methylimidazo[1,2-a]pyridine-6-carboxamido)pyridazin-3-yl)-3,6-dihydropyridine-1(2H)-carboxylate (100 mg, 0.20 mmol) in 3M HCl/EA (2 mL) was stirred at rt for 2 h. The precipitate was filtered, washed with EtOAc (3 mL) and dried in vacuo to afford title compound (51.18 mg, 64.5%) as a yellow solid. ESI-MS (M+H)⁺: 379.1. ¹H NMR (400 MHz, DMSO-*d*₆) δ 11.51 (s, 1H), 9.36 (s, 2H), 9.26 (s, 1H), 8.44 (d, *J* = 9.2 Hz, 1H), 8.12 (d, *J* = 9.6 Hz, 1H), 7.97 (s, 1H), 7.33 (s, 1H), 6.75 (s, 1H), 4.37 (q, *J* = 6.8 Hz, 2H), 3.85 (br s, 2H), 3.35 (br s, 2H), 2.90 (br s, 2H), 2.46 (s, 3H), 1.44 (t, *J* = 6.8 Hz, 3H).

### Example 114. (S)-6-ethoxy-2-methyl-N-(5-(3-methylpiperazin-1-yl)pyrazin-2-yl)-2H-indazole-5-carboxamide formate (Compound 710).

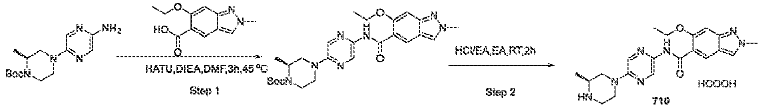

### Step 1: Preparation of tert-butyl (S)-4-(5-(6-ethoxy-2-methyl-2H-indazole-5-carboxamido)pyrazin-2-yl)-2-methylpiperazine-1-carboxylate.

To a mixture of tert-butyl (S)-4-(5-aminopyrazin-2-yl)-2-methylpiperazine-1-carboxylate (300 mg, 0.971 mmol) in DMF (6 mL) were added HATU (443 mg, 1.17 mmol), DIEA (501 mg, 3.88 mmol) and 6-ethoxy-2-methyl-2H-indazole-5-carboxylic acid (320 mg, 1.46 mmol). The mixture was stirred at 45 °C for 3 h. LCMS showed the reaction was completed. The reaction was diluted with water (10 mL), extracted with EA (30 mL), and the organic phase was washed with brine, dried and concentrated to give title product (400 mg, 83 %) as a brown solid. ESI-MS (M+H)⁺: 496.4.

### Step 2: Preparation of (S)-6-ethoxy-2-methyl-N-(5-(3-methylpiperazin-1-yl)pyrazin-2-yl)-2H-indazole-5-carboxamide formate.

To a mixture of tert-butyl (S)-4-(5-(6-ethoxy-2-methyl-2H-indazole-5-carboxamido) pyrazin-2-yl)-2-methylpiperazine-1-carboxylate (100 mg, 0.202 mmol) in EA (2 mL) was added 3M HCl/EA (2 mL). The mixture was stirred at RT for 2 h. The mixture was concentrated in vacuo, the residue was purified by prep-HPLC (0.05 % FA in water / ACN) to give title product (51 mg, Y: 57.3%) as a white solid. ESI-MS (M+H)⁺:396.3. ¹H NMR (400 MHz, DMSO-*d*₆) δ 10.50 (s, 1H), 9.01 (s, 1H), 8.45 (d, *J =* 6.9 Hz, 2H), 8.19 (s, 1H), 8.14 (s, 1H), 7.13 (s, 1H), 4.27 (q, *J =* 6.8 Hz, 2H), 4.16 - 4.09 (m, 5H), 3.06 - 3.02 (m, 1H), 2.90 - 2.76 (m, 4H), 1.50 (t, *J =* 6.9 Hz, 3H), 1.08 (d, *J* = 6.3 Hz, 3H).

### Example 11.5. 7-ethoxy-2-methyl-N-(5-(piperazin-1-yl)pyridin-2-yl)imidazo[1,2-a]pyrimidine-6-carboxamide formate (Compound 711).

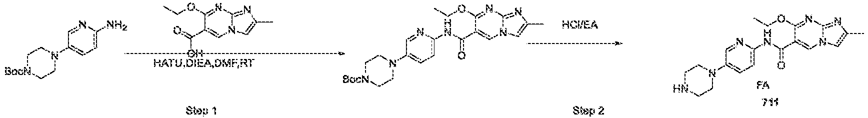

### Step 1: preparation of tert-butyl 4-(6-(7-ethoxy-2-methylimidazo[1,2-a]pyrimidine-6-carboxamido)pyridin-3-yl)piperazine-1-carboxylate.

To a mixture of 7-ethoxy-2-methylimidazo[1,2-a]pyrimidine-6-carboxylic acid (50 mg, 0.22 mmol) in DMF (5 mL) were added HATU (128.9 mg, 0.34 mmol) and DIEA(145.9 mg, 1.13 mmol), the mixture was stirred at rt for 0.5 h. tert-butyl 4-(6-aminopyridin-3-yl)piperazine-1-carboxylate (63 mg, 0.22 mmol ) was added. The resulting mixture was stirred at rt for 2h. The mixture was diluted with water (15 mL), the precipitate was filtered, washed with water and dried in vacuo to provide the crude title product (90 mg, crude) as a yellow solid. ESI-MS (M+H)⁺: 482.1.

### Step 2: preparation of 7-ethoxy-2-methyl-N-(5-(piperazin-1-yl)pyridin-2-yl)imidazo[1,2-a]pyrimidine-6-carboxamide formate.

To a solution of tert-butyl 4-(6-(7-ethoxy-2-methylimidazo[1,2-a]pyrimidine-6-carboxamido)pyridin-3-yl)piperazine-1-carboxylate (70 mg, crude) in EA (3 mL) was added 3M HCl in EA (3 mL) at rt. The mixture was stirred for 2 h at rt. The reaction was concentrated, the residue was purified by prep-HPLC (0.1% FA in water / CH₃CN) to give title product (39 mg, yield: 70%) as a yellow solid. ESI-MS (M+H)⁺: 382.1. ¹H NMR (400 MHz, DMSO-*d*₆) δ 10.26 (s, 1H), 9.33 (s, 1H), 8.22 (s, 1H), 8.11 - 8.08 (m, 2H), 7.58 - 7.45 (m, 2H), 4.55 (q, *J* = 7.0 Hz, 2H), 3.18 - 3.14 (m, 4H), 2.99 - 2.92 (m, 4H), 2.29 (s, 3H), 1.46 (t, *J* = 7.0 Hz, 3H).

### Example 116. 7-cyclopropoxy-2-methyl-N-(5-(piperazin-1-yl)pyridin-2-yl) imidazo[1,2-a]pyridine-6-carboxamide hydrochloride (Compound 712).

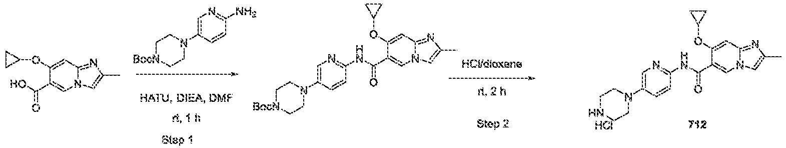

### Step 1: Preparation of tert-butyl 4-(6-(7-cyclopropoxy-2-methylimidazo[1,2-a]pyridine-6-carboxamido)pyridin-3-yl)piperazine-1-carboxylate.

To a mixture of 7-cyclopropoxy-2-methylimidazo[1,2-a]pyridine-6-carboxylic acid (60 mg, 0.26 mmol) in DMF (4 mL) were added HATU (198 mg, 0.52 mmol), DIEA (180 mg, 1.32 mmol) and tert-butyl 4-(6-aminopyridin-3-yl)piperazine-1-carboxylate (72 mg, 0.26 mmol). The mixture was stirred at rt for 1 h. After concentration, the residue was purified by reverse phase column (0.1% NH₃.H₂O in water / CH₃CN) to give title product (32 mg, Y: 45%) as a gray solid. ESI-MS (M+H) ⁺: 493.1. ¹H NMR (400 MHz, CDCl₃) δ 9.95 (s, 1H), 8.89 (s, 1H), 8.16 (d, *J =* 9.0 Hz, 1H), 7.95 (d, *J =* 2.8 Hz, 1H), 7.30 - 7.22 (m, 3H), 3.93 - 3.92 (m, 1H), 3.56 - 3.51 (m, 4H), 3.06 (d, *J=* 4.8 Hz, 4H), 2.37 (s, 3H), 1.42 (s, 9H), 0.97 - 0.93 (m, 4H).

### Step 2: Preparation of 7-cyclopropoxy-2-methyl-N-(5-(piperazin-1yl)pyridin-2-yl) imidazo[1,2-a]pyridine-6-carboxamide hydrochloride.

A mixture of tert-butyl 4-(6-(7-cyclopropoxy-2-methylimidazo[1,2-a]pyridine-6-carboxamido)pyridin-3-yl)piperazine-1-carboxylate (32 mg, 0.065 mmol) in 3M HCl/EA (4 mL) was stirred at rt for 2 h. After concentration, the residue was dissolved in water (5 mL) and lyophilized to give title product (15 mg, 65%) as a yellow solid. ESI-MS (M+H) ⁺: 393.0. ¹H NMR (400 MHz, MeOD-*d*₄) δ 9.13 (s, 1H), 8.13 (s, 1H), 8.04 (d, *J* = 9.1 Hz, 1H), 7.88 - 7.81 (m, 1H), 7.74 - 7.70 (m, 2H), 4.27 - 4.26 (m, 1H), 3.60 - 3.51 (m, 4H), 3.49 - 3.41 (m, 4H), 2.54 (s, 3H), 1.06 (d, *J=* 16.7 Hz, 4H).

### Example 117. (S)-7-methoxy-2-methyl-N-(6-(3-methylpiperazin-1-yl)pyridazin-3-yl)imidazo[1,2-a]pyridine-6-carboxamide hydrochloride (Compound 713).

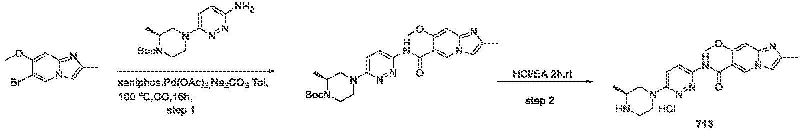

### Step 1: Preparation of tert-butyl (S)-4-(6-(7-methoxy-2-methylimidazo[1,2-a]pyridine-6-carboxamido)pyridazin-3-yl)-2-methylpiperazine-1-carboxylate.

To a mixture of tert-butyl (S)-4-(6-aminopyridazin-3-yl)-2-methylpiperazine-1-carboxylate (244 mg, 0.83 mmol) and 6-bromo-7-methoxy-2-methylimidazo[1,2-a]pyridine (200 mg, 0.83 mmol) in toluene (15 mL) were added Pd(OAc)₂ (20 mg, 0.08 mmol), Xantphos (96 mg, 0.17 mmol) and Na₂CO₃ (264 mg, 2.49 mmol), the mixture was charged with CO for three times and stirred at 100 °C for 16 h. The mixture was filtered and the filtrate was concentrated to give 431-2(200 mg, 64%) as a solid, which was used to next step without further purification. ESI-MS (M+H⁺): 482.1.

### Step 2: Preparation of (S)-7-methoxy-2-methyl-N-(6-(3-methylpiperazin-1-yl)pyridazin-3-yl)imidazo[1,2-a]pyridine-6-carboxamide hydrochloride.

A mixture of tert-butyl (S)-4-(6-(7-methoxy-2-methylimidazo[1,2-a]pyridine-6-carboxamido)pyridazin-3-yl)-2-methylpiperazine-1-carboxylate (200 mg, 0.414 mmol) in EA/HCl(6 mL) was stirred at rt for 2 h. The mixture was concentrated in vacuo. The residue was purified by prep-HPLC (0.1 % HCl in water / ACN) to give title product (66 mg, 38%) as a red solid. ESI-MS (M+H⁺): 382.1.¹H NMR (400 MHz, MeOD-*d*₄) δ 9.17 (s, 1H), 8.41 (d, *J =* 9.8 Hz, 1H), 7.80 (s, 1H), 7.52 (d, *J =* 9.9 Hz, 1H), 7.31 (s, 1H), 4.50 - 4.38 (m, 2H), 4.22 (s, 3H), 3.56 - 3.43 (m, 2H), 3.40 - 3.32 (m, 1H), 3.29 - 3.23 (m, 1H), 3.13 - 3.05 (m, 1H), 2.49 (s, 3H), 1.42 (d, *J* = 6.6 Hz, 3H).

### Example 118. N-(6-((3R,5SS)-3,5-dimethylpiperazin-1-yl)pyridazin-3-yl)-7-ethoxy-2-methylimidazo[1,2-a]pyrimidine-6-carboxamide hydrochloride (Compound 714).

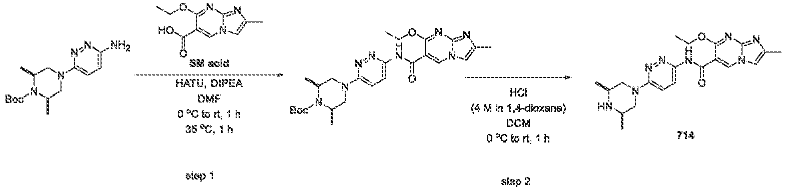

### Step 1: Preparation of tert-butyl (2R,6S)-4-(6-(7-ethoxy-2-methylimidazo[1,2-a]pyrimidine-6-carboxamido)pyridazin-3-yl)-2,6-dimethylpiperazine-1-carboxylate.

To a stirred solution of 7-ethoxy-2-methylimidazo[1,2-a]pyrimidine-6-carboxylic acid (40 mg, 0.181 mmol) in DMF (1.5 mL) under argon with an ice bath was added HATU (104 mg, 0.274 mmol). The reaction was stirred under argon with an ice bath for 10 minutes. Tert-butyl (2R,6S)-4-(6-aminopyridazin-3-yl)-2,6-dimethylpiperazine-1-carboxylate (72 mg, 0.234 mmol) was added, followed by DIPEA (0.15 mL, 0.908 mmol). The reaction was then stirred under argon at room temperature for 1 hour and then at 35 °C for 1 hour. The reaction mixture was diluted with water (20 mL), stirred at room temperature for 30 minutes and filtered. The filter cake was washed with water, dissolved in EtOAc (50 mL), dried over sodium sulfate and concentrated to dryness to afford crude desired product (18 mg, yield: 15%) as a yellow solid. ESI-MS (M+H⁺): 511.4.

### Step 2: Preparation ofN-(6-((3R,5S)-3,5-dimethylpiperazin-1-yl)pyridazin-3-yl)-7-ethoxy-2-methylimidazo[1,2-a]pyrimidine-6-carboxamide hydrochloride.

To a stirred solution of tert-butyl (2R,6S)-4-(6-(7-ethoxy-2-methylimidazo[1,2-a]pyrimidine-6-carboxamido)pyridazin-3-yl)-2,6-dimethylpiperazine-1-carboxylate (18 mg, 0.035 mmol) in DCM (2.0 mL) under argon with an ice bath was added HCl (4 M in 1,4-dioxane) (0.5 mL, 2.0 mmol). The reaction was stirred under argon at room temperature for 1 hour, solid precipitated. The reaction mixture was concentrated. The residue was suspended in DCM (2.0 mL), the precipitate was filtered to afford title product (11 mg, yield: 69%) as a pale yellow solid. ESI-MS (M+H⁺):411.3. ¹H NMR (400 MHz, DMSO-*d*₆) δ 11.10 (s, 1H), 9.73 - 9.67 (m, 1H), 9.54 (s, 1H), 9.37 - 9.26 (m, 1H), 8.22 (d, *J* = 9.6 Hz, 1H), 7.87 (s, 1H), 7.61 (*d, J =* 9.6 Hz, 1H), 4.62 (*q, J =* 6.9 Hz, 2H), 4.46 *(d, J =* 12.4 Hz, 2H), 3.40 - 3.27 (m, 2H), 3.01 (t, *J =* 12.6 Hz, 2H), 2.44 (s, 3H), 1.45 (t, *J =* 7.0 Hz, 3H), 1.34 (d, *J* = 6.4 Hz, 6H).

### Example 119. 6-ethoxy-2-methyl-N-(6-(piperazin-1-yl)pyridazin-3-yl)-2H-pyrazolo[3,4-b]pyridine-5-carboxamide (Compound 715).

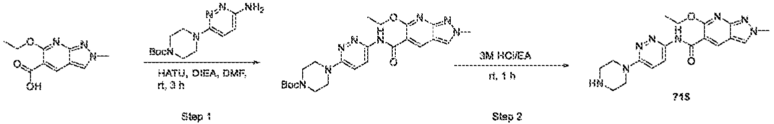

### Step 1: Preparation of tert-butyl 4-(6-(6-ethoxy-2-methyl-2H-pyrazolo[3,4-b]pyridine-5-carboxamido)pyridazin-3-yl)piperazine-1-carboxylate.

To a mixture of 6-ethoxy-2-methyl-2H-pyrazolo[3,4-b]pyridine-5-carboxylic acid (100 mg, 0.45 mmol) in DMF (3 mL) were added tert-butyl 4-(6-aminopyridazin-3-yl)piperazine-1-carboxylate (151 mg, 0.54 mmol), HATU (258 mg, 0.68 mmol) and DIEA (292 mg, 2.26 mmol). The mixture was stirred at rt for 3 h. LCMS showed the reaction was completed. The mixture was poured into H₂O (5 mL) and the precipitate was filtered, washed with water and dried in vacuo to give title product (80 mg, Y: 37 %) as a yellow solid. ESI-MS (M+H)⁺: 483.2

### Step 2: Preparation of 6-ethoxy-2-methyl-N-(6-(piperazin-1-yl)pyridazin-3-yl)-2H-pyrazolo[3,4-b]pyridine-5-carboxamide.

A mixture of tert-butyl 4-(6-(6-ethoxy-2-methyl-2H-pyrazolo[3,4-b]pyridine-5-carboxamido)pyridazin-3-yl)piperazine-1-carboxylate (80 mg, 0.14 mmol) in 3M HCl/EA (3 mL) was stirred at rt for 1 h. LCMS showed the reaction was completed. The mixture was concentrated in vacuo and the residue was purified by prep-HPLC (0.05 % NH₃H₂O in water / ACN) to give title product (22.6 mg, Y: 36.9 %) as a yellow solid. ESI-MS (M+H⁺): 383.1. ¹H NMR (400 MHz, DMSO-*d*₆) δ 10.76 (s, 1H), 8.75 (s, 1H), 8.46 (s, 1H), 8.22 (d, *J* = 9.2 Hz, 1H), 7.38 (d, *J =* 9.8 Hz, 1H), 4.54 (q, *J* = 7.0 Hz, 2H), 4.13 (s, 3H), 3.49 - 3.42 (m, 4H), 2.85 - 2.76 (m, 4H), 1.47 (*t, J=* 7.0 Hz, 3H).

### Example 120. (S)-6-ethoxy-N-(5-(3-ethylpiperazin-1-yl)pyrazin-2-yl)-2-methyl-2H-indazole-5-carboxamide HCl salt (Compound 716).

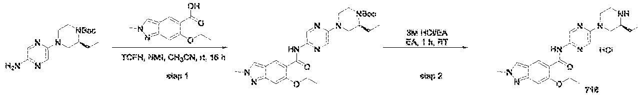

### Step 1: tert-butyl (S)-4-(5-(6-ethoxy-2-methyl-2H-indazole-5-carboxamido)pyrazin-2-yl)-2-ethylpiperazine-1-carboxylate.

To a stirred solution of tert-butyl (S)-4-(5-aminopyrazin-2-yl)-2-ethylpiperazine-1-carboxylate (200 mg, 0.65 mmol), 6-ethoxy-2-methyl-2H-indazole-5-carboxylic acid (143.3 mg, 0.65 mmol) and NMI (160 mg, 1.95 mmol) in MeCN (3 mL) was added TCFH (273 mg, 0.98 mmol). The mixture was stirred at rt for 16 h. The precipitate was filtered and washed with ACN (2 × 15 mL), dried in vacuo to give title product (200 mg, Y: 60.24%) as an off-white solid. ESI-MS (M+H)⁺: 510.3.

### Step 2: (S)-6-ethoxy-N-(5-(3-ethylpiperazin-1-yl)pyrazin-2-yl)-2-methyl-2H-indazole-5-carboxamide HCl salt.

A mixture of tert-butyl (S)-4-(5-(6-ethoxy-2-methyl-2H-indazole-5-carboxamido)pyrazin-2-yl)-2-ethylpiperazine-1-carboxylate (100 mg, 0.20 mmol) in 3M EtOAc/HCl (3 mL) was stirred at rt for 2 h. After concentration, the residue was dissolved in water (5 mL) and lyophilized to give title product (37.46 mg, Y: 46.6 %) as a yellow solid. ESI-MS (M+H)⁺: 410.2. ¹H NMR (400 MHz, DMSO-*d*₆) δ 10.59 (s, 1H), 9.30 - 9.10 (m, 2H), 9.07 (s, 1H), 8.47 (s, 1H), 8.44 (s, 1H), 8.29 (s, 1H), 7.14 (s, 1H), 4.38 - 4.24 (m, 4H), 4.14 (s, 3H), 3.36 (d, *J* = 12 Hz, 1H), 3.28 - 3.14 (m, 2H), 3.12 - 2.97 (m, 2H), 1.74 - 1.62 (m, 2H), 1.50 (t, *J* = 8 Hz, 3H), 1.02 (t, *J* = 8 Hz, 3H).

### Example 121. 6-ethoxy-2-methyl-N-(6-((2S)-2-methylpiperidin-4-yl) pyridazin-3-yl)-2H-indazole-5-carboxamide (Compound 717).

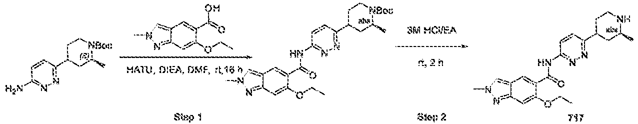

### Step 1: Preparation of tert-butyl (2S)-4-(6-(6-ethoxy-2-methyl-2H-indazole-5-carboxamido) pyridazin-3-yl)-2-methylpiperidine-1-carboxylate.

To a mixture of 6-ethoxy-2-methyl-2H-indazole-5-carboxylic acid (53 mg, 0.24 mmol) in DMF (2 mL) were added tert-butyl (2S)-4-(6-aminopyridazin-3-yl)-2-methylpiperidine-1-carboxylate (70 mg, 0.24 mmol), HATU (137 mg, 0. 36 mmol) and DIEA (154 mg, 1.20 mmol). The mixture was stirred at rt for 16 h. The mixture was diluted with H₂O (5 mL) and the precipitate was filtered, washed with water and dried in vacuo to give title product (50 mg, Y: 38.6 %) as a yellow solid. ESI-MS (M+H⁺): 495.2.

### Step 2: Preparation of 6-ethoxy-2-methyl-N-(6-((2S)-2-methylpiperidin-4-yl) pyridazin-3-yl)-2H-indazole-5-carboxamide.

A mixture of tert-butyl (2S)-4-(6-(6-ethoxy-2-methyl-2H-indazole-5-carboxamido) pyridazin-3-yl)-2-methylpiperidine-1-carboxylate (50 mg, 0.10 mmol) in 3 M HCl/EA (2 mL) was stirred at rt for 2 h. The mixture was diluted with H₂O (5 mL), extracted with EA (10 mLx3). The aqueous layer was concentrated in vacuo and the residue was purified by prep-HPLC (0.1 % NH₃.H₂O in water / ACN) to give title product (5.6 mg, 14.1 %) as a yellow solid. ESI-MS (M+H⁺): 395.2. ¹H NMR (400 MHz, MeOD-*d*₄) δ 8.60 (d, *J* = 7.2 Hz, 1H), 8.58 (s, 1H), 8.33 (s, 1H), 7.68 (d, *J* = 7.3 Hz, 1H), 7.12 (s, 1H), 4.35 (q, *J* = 7.0 Hz, 2H), 4.19 (s, 3H), 3.22 - 3.03 (m, 2H), 2.87 - 2.76 (m, 2H), 1.94 (t, *J* = 14.1 Hz, 2H), 1.76 - 1.68 (m, 1H), 1.65 (t, *J =* 7.0 Hz, 3H), 1.41 (dd, *J* = 23.9, 12.4 Hz, 1H), 1.16 (d, *J=* 6.3 Hz, 3H).

### Example 122. (S)-7-methoxy-2-methyl-N-(5-(3-methylpiperazin-1-yl)pyridin-2-yl)imidazo[1,2-a]pyridine-6-carboxamide formate (Compound 718).

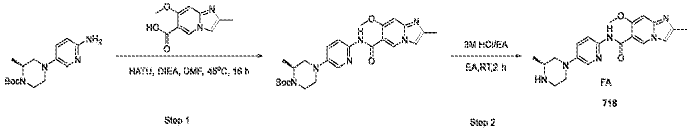

### Step 1: Preparation of tert-butyl (S)-4-(6-(7-methoxy-2-methylimidazo[1,2-a]pyridine-6-carboxamido)pyridin-3-yl)-2-methylpiperazine-1-carboxylate.

To a mixture of tert-butyl (S)-4-(6-aminopyridin-3-yl)-2-methylpiperazine-1-carboxylate (100 mg, 0.30 mmol) in DMF (5 mL) were added HATU (148 mg, 0.36 mmol), DIEA (168 mg, 1.20 mmol) and 7-methoxy-2-methylimidazo[1,2-a]pyridine-6-carboxylic acid (80 mg, 0.36 mmol). The mixture was stirred at 45 °C for 16 h. LCMS showed the starting material was consumed completely. The mixture was diluted with water (10 mL), extracted with EA (20 mL×3). The organic layer was washed with brine, dried over Na₂SO₄ and concentrated in vacuo to give title product (120 mg, 69.36%) as a yellow solid. ESI-MS (M+H)⁺: 481.3.

### Step 2: Preparation of (S)-7-methoxy-2-methyl-N-(5-(3-methylpiperazin-1-yl)pyridin-2-yl)imidazo[1,2-a]pyridine-6-carboxamideformate.

To a mixture of tert-butyl (S)-4-(6-(7-methoxy-2-methylimidazo[1,2-a]pyridine-6-carboxamido)pyridin-3-yl)-2-methylpiperazine-1-carboxylate (100 mg, 0.20 mmol) in EA (3 mL) was added 3 M HCl/EA (3 mL). The mixture was stirred at rt for 2 h. LCMS showed the starting material was consumed completely. The mixture was concentrated in vacuo. The crude was purified by prep-HPLC (0.05 % FA in water / CH₃CN) to give title product (38 mg, Y: 48.10 %) as a yellow solid. ESI-MS (M+H) ⁺: 381.2. ¹H NMR (400 MHz, MeOD-*d*₄) δ 9.06 (s, 1H), 8.43 (s, 1H), 8.26 (d, *J* = 9.0 Hz, 1H), 8.15 - 8.07 (m, 1H), 7.62 - 7.53 (m, 2H), 7.01 (s, 1H), 4.16 (s, 3H), 3.82 (t, *J =* 15.3 Hz, 2H), 3.57 - 3.48 (m, 2H), 3.32 - 3.27 (m, 1H), 3.08 (t, *J* = 10.9 Hz, 1H), 2.89 - 2.79 (m, 1H), 2.40 (s, 3H), 1.42 (d, *J* = 6.5 Hz, 3H).

### Example 123. N-(5-((3S,5R)-3,5-dimethylpiperazin-1-yl)pyrazin-2-yl)-7-ethoxy-2-methylimidazo[1,2-a]pyridine-6-carboxamide (Compound 719).

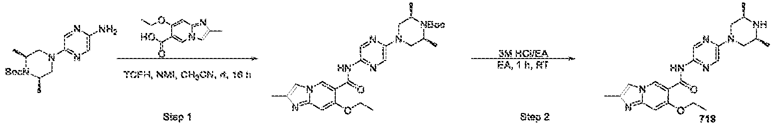

### Step 1: Preparation of tert-butyl (2S,6R)-4-(5-(7-ethoxy-2-methylimidazo[1,2-a]pyridine-6-carboxamido)pyrazin-2-yl)-2,6-dimethylpiperazine-1-carboxylate.

To mixture of 7-ethoxy-2-methylimidazo[1,2-a]pyridine-6-carboxylic acid (72 mg, 0.33 mmol), tert-butyl (2R,6S)-4-(5-aminopyrazin-2-yl)-2,6-dimethylpiperazine-1-carboxylate (100 mg, 0.33 mmol) and NMI (80 mg, 0.99 mmol) in ACN (5 mL) was added TCFH (137 mg, 0.49 mmol) and the mixture was stirred at rt for 16 h. The precipitate was collected by filtration, washed with water and dried to give title product (130 mg, Y: 78.3 %) as a yellow solid. ESI-MS (M+H)⁺:510.3.

### Step 2: Preparation of N-(5-((3,5,5R)-3,5-dimethylpiperazin-1-yl)pyrazin-2-yl)-7-ethoxy-2-methylimidazo[1,2-a]pyridine-6-carboxamide.

A mixture of tert-butyl (2S,6R)-4-(5-(7-ethoxy-2-methylimidazo[1,2-a]pyridine-6-carboxamido)pyrazin-2-yl)-2,6-dimethylpiperazine-1-carboxylate (100 mg, 0.20 mmol) in 3M EA/HCl (4 mL) was stirred at rt for 1 h. The mixture was concentrated in vacuo, the residue was purified by prep-HPLC (0.1% FA in water / CH₃CN) to give title compound (40 mg, yield: 50%) as a yellow solid. ESI-MS (M+H⁺): 410.2. ¹H NMR (400 MHz, DMSO-*d*₆) δ 10.41 (s, 1H), 9.05 (s, 1H), 9.00 (s, 1H), 8.27 (s, 1H), 7.65 (s, 1H), 7.00 (s, 1H), 4.39 (d, *J=* 11.7 Hz, 2H), 4.27 (q, *J* = 6.8 Hz, 2H), 3.27 - 3.20 (m, 2H), 2.80 (t, *J* = 12.4 Hz, 2H), 2.29 (s, 3H), 1.48 (*t, J =* 6.8 Hz, 3H), 1.26 (d, *J* = 6.4 Hz, 6H).

### Example 124. N-(S-((3R,SS)-3,5-dimethylpiperazin-1-yl)pyrazin-2-yl)-6-ethoxy-2-methyl-2H-indazole-5-carboxamide hydrochloride (Compound 720).

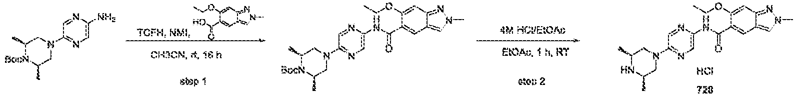

### Step 1: tert-butyl (2R, 65)-4-(5-(6-ethoxy-2-methyl-2H-indazole-5-carboxamido)pyrazin-2-yl)-2,6-dimethylpiperazine-1-carboxylate.

To a mixture of tert-butyl (2R,6S)-4-(5-aminopyrazin-2-yl)-2,6-dimethylpiperazine-1-carboxylate (100 mg, 0.33 mmol) in CH₃CN (2 mL) was added 6-ethoxy-2-methyl-2H-indazole-5-carboxylic acid (71.7 mg, 0.33 mmol), TCFH (136.92 mg, 0.49 mmol) and NMI (80.2 mg, 0.98 mmol). The mixture was stirred at rt for 16 h. LCMS showed the starting material was consumed completely. The reaction mixture was filtered and the filter cake was washed with CH₃CN (15 mLx3), dried in vacuum to give title product (85 mg, Y: 51.3 %) as a yellow solid.

### Step 2: Preparation ofN-(5-((3R,5S)-3,5-dimethylpiperazin-1-yl)pyrazin-2-yl)-6-ethoxy-2-methyl-2H-indazole-5-carboxamide hydrochloride.

To a mixture of tert-butyl (2R,6S)-4-(5-(6-ethoxy-2-methyl-2H-indazole-5-carboxamido)pyrazin-2-yl)-2,6-dimethylpiperazine-1-carboxylate (85 mg, 0.157 mmol) in EtOAc (5 mL) was added 4 M EtOAC·HCl (5 mL). The mixture was stirred at R.T. for 1 h. LCMS showed the starting material was consumed completely. The solid was collected by filtration, washed with DCM and dried under vacuum to afford the title compound. ESI-MS (M+H⁺): 410.3. ¹H NMR (400 MHz, DMSO-*d*₆) δ 10.58 (s, 1H), 9.58 (s, 1H), 9.14 (s, 1H), 9.05 (s, 1H), 8.47 (s, 1H), 8.43 (s, 1H), 8.28 (s, 1H), 7.14 (s, 1H), 4.42 (d, *J =* 12.3 Hz, 2H), 4.27 (q, *J =* 6.8 Hz, 2H), 4.14 (s, 3H), 3.38 - 3.26 (m, 2H), 2.99 - 2.85 (m, 2H), 1.50 (t, *J* = 6.9 Hz, 3H), 1.32 (d, *J =* 6.5 Hz, 6H).

### Example 125. N-(5-((3R,5S)-3,5-dimethylpiperazin-1-yl)pyrazin-2-yl)-7-ethoxy-2-methylimidazo[1,2-a]pyrimidine-6-carboxamide hydrochloride (Compound 721).

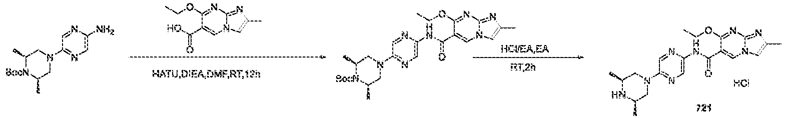

### Step 1: Preparation of tert-butyl (2R, 6S)-4-(5-(7-ethoxy-2-methylimidazo[1,2-a]pyrimidine-6-carboxamido)pyrazin-2-yl)-2,6-dimethylpiperazine-1-carboxylate.

To a mixture of tert-butyl (2R,6S)-4-(5-aminopyrazin-2-yl)-2,6-dimethylpiperazine-1-carboxylate (556 mg, 1.81 mmol) in DMF (10 mL) were added 7-ethoxy-2-methylimidazo[1,2-a]pyrimidine-6-carboxylic acid (400 mg, 1.81 mmol), DIEA (1.17 g, 9.05 mmol) and HATU (2.1 g, 5.43 mmol). The mixture was stirred at rt for 16 h. After diluting with water, the mixture was filtered and the cake was washed with MeOH (6 mL) and dried to give title product (380 mg, 41.2%) as a white solid ESI-MS (M+H)⁺: 511.2.

### Step 2: Preparation of N-(5-((3R,5S)-3,5-dimethylpiperazin-1-yl)pyrazin-2-yl)-7-ethoxy-2-methylimidazo[1,2-a]pyrimidine-6-carboxamide hydrochloride.

To a solution of tert-butyl (2R,6S)-4-(5-(7-ethoxy-2-methylimidazo[1,2-a]pyrimidine-6-carboxamido)pyrazin-2-yl)-2,6-dimethylpiperazine-1-carboxylate (380 mg, 0.75 mmol) in EA (3 mL) was added 3M HCl/EA (3 mL) was stirred at rt for 2 h. After concentration, the residue was filtered and the solid was dried under vacuum to give title product (200 mg, 60.2 %) as a white solid. ESI-MS (M+H)⁺: 411.1. ¹H NMR (400 MHz, DMSO-*d*6) δ 10.72 (s, 1H), 9.84 - 9.72 (m, 1H), 9.55 (s, 1H), 9.42 (d, *J* = 9.9 Hz, 1H), 8.93 (s, 1H), 8.31 (s, 1H), 7.87 (d, *J* = 1.1 Hz, 1H), 4.61 (q, *J* = 7.0 Hz, 2H), 4.43 *(d, J =* 12.6 Hz, 2H), 3.29 (s, 2H), 3.04 - 2.95 (m, 2H), 2.44 (d, *J* = 0.9 Hz, 3H), 1.45 (t, *J* = 7.0 Hz, 3H), 1.35 (d, *J* = 6.4 Hz, 6H).

### Example 126. (S)-7-ethoxy-2-methyl-N-(5-(3-methylpiperazin-1-yl)pyrazin-2-yl)imidazo[1,2-a]pyrimidine-6-carboxamide formate (Compound 722).

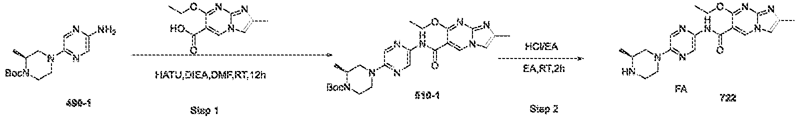

### Step 1: Preparation of tert-butyl (S)-4-(5-(7-ethoxy-2-methylimidazo[1,2-a]pyrimidine-6-carboxamido)pyrazin-2-yl)-2-methylpiperazine-1-carboxylate.

To a mixture of tert-butyl (S)-4-(5-aminopyrazin-2-yl)-2-methylpiperazine-1-carboxylate (100 mg, 0.34 mmol) in DMF (2 mL) were added HATU (155 mg, 0.41 mmol), DIEA (176 mg, 1.37 mmol) and 7-ethoxy-2-methylimidazo[1,2-a]pyrimidine-6-carboxylic acid (113 mg, 0.51 mmol). The mixture was stirred at 25°C for 12 h. LCMS showed the reaction was completed. The reaction was diluted with water (10 mL), extracted with EA (30 mL), and the organic phase was washed with brine, dried and concentrated to give title product (150 mg, 89 %) as a yellow solid. ESI-MS (M+H)⁺: 497.1.

### Step 2: Preparation of (S)-7-ethoxy-2-methyl-N-(5-(3-methylpiperazin-1-yl)pyrazin-2-yl)imidazo[1,2-a]pyrimidine-6-carboxamide formate.

A mixture of tert-butyl (S)-4-(5-(7-ethoxy-2-methylimidazo[1,2-a]pyrimidine-6-carboxamido)pyrazin-2-yl)-2-methylpiperazine-1-carboxylate (150 mg, 0.30 mmol) in EA/HCl (4 mL) was stirred at RT for 2 h. LCMS showed the reaction was completed, the mixture was concentrated and the residue was purified by prep-HPLC (0.05 % FA in water / ACN) to give title product (24 mg, Y: 20%) as an off-white solid. ESI-MS (M+H⁺): 397.1. ¹H NMR (400 MHz, DMSO-*d*₆) δ 10.27 (s, 1H), 9.33 (s, 1H), 8.95 (s, 1H), 8.19 (s, 1H), 8.15 (s, 1H), 7.55 (s, 1H), 4.53 (q, *J* = 7.0 Hz, 2H), 4.22 - 4.16 (m, 2H), 3.15 - 3.12 (m, 1H), 2.99 - 2.93 (m, 2H), 2.91 - 2.88 (m, 1H), 2.67 - 2.64 (m, 1H), 2.29 (s, 3H), 1.46 (d, *J* = 6.8 Hz, 3H), 1.14 (d, *J* = 6.3 Hz, 3H).

### Example 127. (R)-N-(6-(3-(tert-butylamino)pyrrolidin-1-yl)pyridazin-3-yl)-7-ethoxy-2-methylimidazo[1,2-a]pyridine-6-carboxamide (Compound 723).

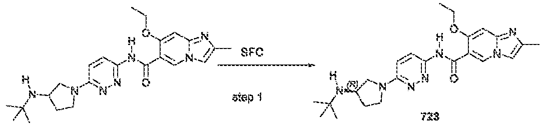

### Step 1: Preparation of (R)-N-(6-(3-(tert-butylamino)pyrrolidin-1-yl)pyridazin-3-yl)-7-ethoxy-2-methylimidazo[1,2-a]pyridine-6-carboxamide.

The N-(6-(3-(tert-butylamino)pyrrolidin-1-yl)pyridazin-3-yl)-7-ethoxy-2-methylimidazo[1,2-a]pyridine-6-carboxamide (40 mg, 0.092 mmol ) was separated by chiral SFC (EtOH / n-Hexane) to afford (R)-N-(6-(3-(tert-butylamino)pyrrolidin-1-yl)pyridazin-3-yl)-7-ethoxy-2-methylimidazo[1,2-a]pyridine-6-carboxamide (14.85 mg, Y: 37.1 %) as a white solid. ¹ H NMR (400 MHz, DMSO-*d*₆) δ 10.62 (s, 1H), 9.06 (s, 1H), 8.18 (d, *J* = 9.7 Hz, 1H), 7.66 (s, 1H), 7.00 (s, 2H), 4.28 (q, *J* = 6.8 Hz, 2H), 3.75 - 3.68 (m, 1H), 3.57 - 3.48 (m, 2H), 3.38 - 3.36 (m, 1H), 3.26 - 2.86 (m, 1H), 2.29 (s, 3H), 2.20 - 2.17 (m, 1H), 1.77 - 1.75 (m, 1H), 1.50 (t, *J* = 6.9 Hz, 3H), 1.11 (s, 9H). ESI-MS (M+H)⁺: 438.2.

### Example 128. N-(5-((3R,5S)-3,5-dimethylpiperazin-1-yl)pyridin-2-yl)-7-methoxy-2-methylimidazo[1,2-a]pyridine-6-carboxamide (Compound 724).

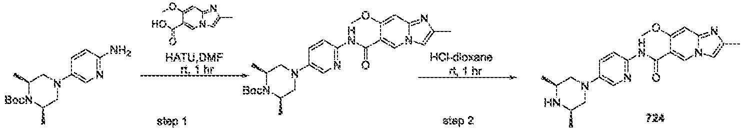

### Step 1: Preparation of tert-butyl (2R, 6S)-4-(6-(7-methoxy-2-methylimidazo[1,2-a]pyridine-6-carboxamido)pyridin-3-yl)-2,6-dimethylpiperazine-1-carboxylate.

To a mixture of tert-butyl (2R,6S)-4-(6-aminopyridin-3-yl)-2,6-dimethylpiperazine-1-carboxylate (48.0 mg, 0.16 mmol) in DMF (5 mL) was added DIEA (60.4 mg, 0.47 mmol), HATU (89.0 mg, 0.23 mmol) and 7-methoxy-2-methylimidazo[ 1,2-a]pyridine-6-carboxylic acid (32.1 mg, 0.17 mmol) and the mixture was stirred at RT for 1h. The mixture was diluted with H₂O (20 mL) and the resulting mixture was extracted with EA (50 mL x 2). The organic layer was combined, washed with brine (20 mL x3), dried over anhydrous Na₂SO₄, filtered and concentrated in vacuum to afford tert-butyl (2R,6S)-4-(6-(7-methoxy-2-methylimidazo[1,2-a]pyridine-6-carboxamido)pyridin-3-yl)-2,6-dimethylpiperazine-1-carboxylate (60 mg, 69.7% yield) as a white solid. ESI-MS (M+H⁺):495.4.

### Step 2: Preparation of N-(5-((3R,5S)-3,5-dimethylpiperazin-1-yl)pyridin-2yl)-7-methoxy-2-methylimidazo[1,2-a]pyridine-6-carboxamide.

A mixture of tert-butyl (2R,6S)-4-(6-(7-methoxy-2-methylimidazo[1,2-a]pyridine-6-carboxamido)pyridin-3-yl)-2,6-dimethylpiperazine-1-carboxylate (60.0 mg, 0.12 mmol) in 1N HCl-dioxane (5 mL) was stirred at rt for 1h. The mixture was concentrated and purified by prep-HPLC (0.05% NH4HCO₃ in water / CH₃CN) to afford the title compound to afford title product (12.48 mg, 26.7% yield) as a white solid. ESI-MS (M+H⁺): 395.2. ¹H NMR (400 MHz, DMSO-*d₆*) δ 10.22 (s, 1H), 8.97 (s, 1H), 8.07 (d, *J* = 8.9 Hz, 1H), 8.03 (d, *J* = 3.0 Hz, 1H), 7.64 (s, 1H), 7.44 (dd, *J=* 9.1, 3.1 Hz, 1H), 7.00 (s, 1H), 3.99 (s, 3H), 3.57 - 3.51 (m, 2H), 2.90-2.82 (m, 2H), 2.29 (d, *J =* 1.0 Hz, 3H), 2.13 (t, *J* = 10.8 Hz, 2H), 1.03 (d, *J* = 6.3 Hz, 6H).

### Example 129. N-(6-(4,7-diazaspiro[2.5]octan-7-yl)pyridazin-3-yl)-7-ethoxy-2-methylimidazo[1,2-a]pyrimidine-6-carboxamide formic acid (Compound 725).

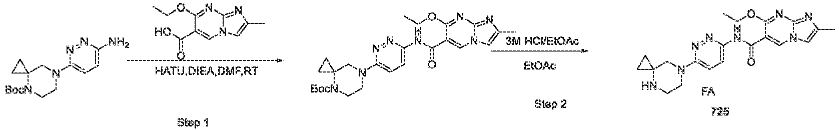

### Step 1: preparation of tert-butyl 7-(6-(7-ethoxy-2-methylimidazo[1,2-a]pyrimidine-6-carboxamido)pyridazin-3-yl)-4, 7-diazaspiro[2. 5]octane-4-carboxylate.

To a mixture of 7-ethoxy-2-methylimidazo[1,2-a]pyrimidine-6-carboxylic acid (54.34 mg, 0.25 mmol) in DMF (5 mL) were added HATU (93 mg, 0.25 mmol) and DIEA (105.7 mg, 0.82 mmol), the mixture was stirred at rt for 0.5 h. tert-butyl 7-(6-aminopyridazin-3-yl)-4,7-diazaspiro[2.5]octane-4-carboxylate (50 mg, 0.16 mmol ) was added. The resulting mixture was stirred at rt for 2h. The mixture was purified by reversed phase column chromatography to provide the title compound (50 mg, yield: 60%). ESI-MS (M+H)⁺:509.3.

### Example 130. Preparation of compounds C highlighted in Table 2.

To a solution of tert-butyl 7-(6-(7-ethoxy-2-methylimidazo[1,2-a]pyrimidine-6-carboxamido)pyridazin-3-yl)-4,7-diazaspiro[2.5]octane-4-carboxylate (50 mg, 0.098 mmol) in EA (3 mL) was added 3M HCl in EA (3 mL).The mixture was stirred for 2 h at rt. The reaction mixture was concentrated, the residue was purified by prep-HPLC (0.05% FA in water / CH₃CN) to give title product (5.6 mg, yield: 14%) as a yellow solid. ESI-MS (M+H)⁺: 409.1. ¹H NMR (400 MHz, DMSO-*d*₆₎ δ 10.59 (s, 1H), 9.33 (s, 1H), 8.20 (s, 1H), 8.15 (d, *J* = 9.9 Hz, 1H), 7.56 (s, 1H), 7.37 (d, *J* = 9.9 Hz, 1H), 4.54 (q, *J* = 7.1 Hz, 2H), 3.52 - 3.49 (m, 2H), 3.40 (s, 2H), 2.91 - 2.84 (m, 2H), 2.29 (s, 3H), 1.46 (t, *J* = 7.0 Hz, 3H), 0.56 - 0.44 (m, 4H).

### Example 131. (S)-6-methoxy-2-methyl-N-(6-(3-methylpiperazin-1-yl)pyridazin-3-yl)pyrazolo[1,5-a]pyridine-5-carboxamide hydrochloride (Compound 726).

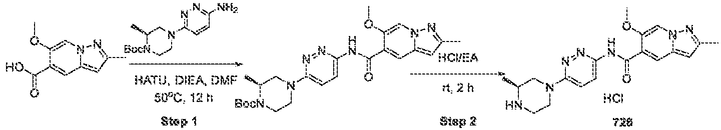

### Step 1: Preparation of tert-butyl (S)-4-(6-(6-methoxy-2-methylpyrazolo[1,5-a]pyridine-5-carboxamido)pyridazin-3-yl)-2-methylpiperazine-1-carboxylate.

To a mixture of 6-methoxy-2-methylpvrazolo[1,5-a]pyridine-5-carboxylic acid (160 mg, 0.77 mmol) in DMF (3 mL) were added tert-butyl (S)-4-(6-aminopyridazin-3-yl)-2-methylpiperazine-1-carboxylate (336 mg, 1.16 mmol), HATU (354 mg, 0.92 mmol) and DIEA (400 mg, 3.10 mmol). The mixture was diluted with H₂O (10 mL), extracted with EA (20 mLx3), the combined organic layer was concentrated in vacuo to give the title product (250 mg, Y: 62%) as a yellow solid. ESI-MS (M+H⁺):482.2.

### Step 2: Preparation of (S)-6-methoxy-2-methyl-N-(6-(3-methylpiperazin-1-yl)pyridazin-3-yl)pyrazolo[1,5-a]pyridine-5-carboxamide hydrochloride.

A mixture of tert-butyl (S)-4-(6-(6-methoxy-2-methylpyrazolo[1,5-a]pyridine-5-carboxamido)pyridazin-3-yl)-2-methylpiperazine-1-carboxylate (250 mg, 0.519 mmol) in 3M HCl/EA (4 mL) was stirred at 25°C for 2 h. LCMS showed the reaction was completed. The precipitate was filtered to give the title product (70 mg, Y: 35%) as a yellow solid. ESI-MS (M+H⁺):382.5. ¹H NMR (400 MHz, MeOD-*d*₄) δ 8.33 - 8.30 (m, 2H), 8.22 - 8.15 (m, 2H), 6.61 (s, 1H), 4.48 (d, *J* = 13.1 Hz, 2H), 4.05 (s, 3H), 3.62 - 3.51 (m, 3H), 3.41 - 3.32 (m, 2H), 2.47 (s, 3H), 1.45 (d, *J* = 6.6 Hz, 3H).

### Example 132. (S)-6-ethoxy-2-methyl-N-(6-(6-methyl-1,2,3,6-tetrahydropyridin-4-yl)pyridazin-3-yl)-2H-indazole-5-carboxamide formate (Compound 727).

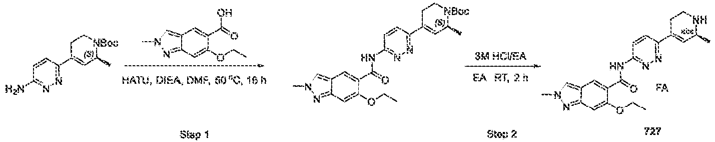

### Step 1: Preparation of tert-butyl (S)-4-(6-(6-ethoxy-2-methyl-2H-indazole-5-carboxamido)pyridazin-3-yl)-6-methyl-3,6-dihydropyridine-1(2H)-carboxylate.

To a solution of tert-butyl (S)-4-(6-aminopyridazin-3-yl)-2-methyl-3,6-dihydropyridine-1(2H)-carboxylate (400 mg, 1.38 mmol) in DMF (5 mL) were added 6-ethoxy-2-methyl-2H-indazole-5-carboxylic acid (365 mg, 1.66 mmol), DIEA (712 mg, 5.52 mmol) and HATU (630 mg, 1.66 mmol). The mixture was stirred at 50°C for 16 h. The mixture was diluted with water (10 mL), the precipitate was filtered, washed with water (20 mL) and concentrated in vacuo to give title product (300 mg, 44.12 %) as a yellow solid. ESI-MS (M+H)⁺: 493.2.

### Step 2: Preparation of (S)-6ethoxy-1-methyl-N-(6-(6-methyl-1,2,3,6tetrahydropyridin-4-yl)pyridazin-3-yl)-2H-indazole-5-carboxamide formate.

To a solution of tert-butyl (S)-4-(6-(6-ethoxy-2-methyl-2H-indazole-5-carboxamido)pyridazin-3-yl)-6-methyl-3,6-dihydropyridine-1(2H)-carboxylate (300 mg, 0.61 mmol) in EA (5 mL) was added 3 M HCl/EA (6 mL). The mixture was stirred at rt for 2 h. The mixture was concentrated in vacuo. The crude was purified by prep-HPLC (0.05 % FA in water / CH₃CN) to give title product (48.75 mg, Y: 18.26 %) as a yellow solid. ESI-MS (M+H⁺): 393.3. ¹H NMR (400 MHz, MeOD-*d*₄) δ 8.65 (d, *J* = 9.5 Hz, 1H), 8.59 (s, 1H), 8.52 (s, 1H), 8.33 (s, 1H), 8.00 (t, *J* = 9.1 Hz, 1H), 7.11 (s, 1H), 6.70 - 6.59 (m, 1H), 4.36 (q, *J =* 6.9 Hz, 2H), 4.18 (s, 3H), 3.97 (br s, 1H), 3.67 - 3.56 (m, 1H), 3.42 - 3.35 (m, 1H), 3.28 - 3.05 (m, 1H), 3.04 - 2.96 (m, 1H), 1.65 (t, *J* = 6.9 Hz, 3H), 1.52 (t, *J* = 7.1 Hz, 3H).

### Example 133. 6-ethoxy-2-methyl-N-(6-(piperidin-4-yl)pyridazin-3-yl)-2H-indazole-5-carboxamide hydrochloride (Compound 728).

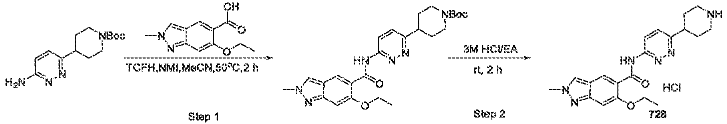

### Step 1: Preparation of tert-butyl 4-(6-(6-ethoxy-2-methyl-2 carboxamido)pyridazin-3-yl)piperidine-1-carboxylate.

To a mixture of tert-butyl 4-(6-aminopyridazin-3-yl)piperidine-1-carboxylate (240 mg, 0.86 mmol) and 6-ethoxy-2-methyl-2H-indazole-5-carboxylic acid (241.2 mg, 1.1 mmol) in MeCN( 10 mL) were added NMI (212.4 mg. 2.59 mmol) and TCFH (363.9 mg, 1.29 mmol). The mixture was stirred at 50 °C for 2 h. The precipitate was filtered and dried under vacuum to give title product (200 mg, 48.4%) as a white solid, which was used to next step without further purification. ESI-MS (M+H)⁺: 481.3. ¹H NMR (400 MHz, DMSO-*d*₆) δ 11.14 (s, 1H), 8.51 - 8.40 (m, 3H), 7.71 (d, *J* = 9.2 Hz, 1H), 7.16 (s, 1H), 4.29 (q, *J* = 6.9 Hz, 2H), 4.15 (s, 3H), 4.13 - 4.03 (m, 2H), 3.06 (t, *J* = 12.0 Hz, 1H), 2.98 - 2.72 (m, 2H), 1.92 - 1.84 (m, 2H), 1.69 - 1.58 (m, 2H), 1.53 (t, *J* = 6.9 Hz, 3H), 1.43 (s, 9H).

### Step 2: Preparation of 6-ethoxy-2-methyl-N-(6-(piperidin-4-yl)pyridazin-3-yl)-2H-indazole-5-carboxamide hydrochloride.

A mixture of tert-butyl 4-(6-(6-ethoxy-2-methyl-2H-indazole-5-carboxamido)pyridazin-3-yl)piperidine-1-carboxylate (200 mg, 0.42 mmol) in EA/HCl (5 mL) was stirred at rt for 2 h. The precipitate was filtered and lyophilized to give title product (132 mg, 75.6%) as a white solid. ESI-MS (M+H)⁺: 381.3. ¹H NMR (400 MHz, DMSO-*d*₆) δ 11.23 (s, 1H), 9.26 (s, 1H), 9.03 (s, 1H), 8.58 - 8.40 (m, 3H), 7.74 (d, *J* = 9.3 Hz, 1H), 7.16 (s, 1H), 4.29 (q, *J* = 6.9 Hz, 2H), 4.15 (s, 3H), 3.38 *(d, J=* 12.6 Hz, 2H), 3.27 - 3.17 (m, 1H), 3.13 - 2.97 (m, 2H), 2.16 - 1.97 (m, 4H), 1.52 (t, *J* = 6.9 Hz, 3H).

### Example 134. 7-ethoxy-2-methyl-N-(6-(4-methylpiperazin-1-yl)pyridazin-3-yl)imidazo[1,2-a]pyridine-6-carboxamide (Compound 729).

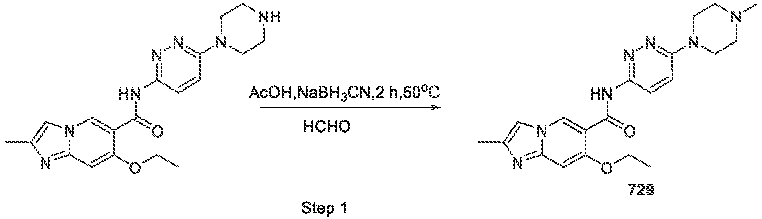

### Step 1: Preparation of 7-ethoxy-2-methyl- N-(6-(4-methylpiperazin-1-yl)pyridazin-3-yl)imidazo[1,2-a]pyridine-6-carboxamide.

To a mixture of 7-ethoxy-2-methyl-N-(6-(piperazin-1-yl)pyridazin-3-yl)imidazo[1,2-a]pyridine-6-carboxamide (70 mg, 0.18 mmol) and (HCHO)n (27.6 mg, 0.92 mmol) in MeOH (5 mL) were added HOAc (33 mg, 0.8 mmol) and NaBH₃CN (58 mg, 1.34 mmol). The mixture was stirred at 50 °C for 2 h. The mixture was poured into water (2 mL). Filtered and concentrated in vacuo. The residue was purified by prep-HPLC (0.05 % NH₃.H₂O in water / ACN) to give title product (26.32 mg, Y: 32.8%) as a yellow solid. ESI-MS (M+H⁺): 396.2. ¹H NMR (400 MHz, MeOD-*d*₄) δ 9.03 (s, 1H), 8.37 (d, J = 9.8 Hz, 1H), 7.55 (s, 1H), 7.38 (d, J = 9.9 Hz, 1H), 6.95 (s, 1H), 4.37 (q, J = 6.9 Hz, 2H), 3.66 - 3.59 (m, 4H), 2.62 - 2.56 (m, 4H), 2.38 - 2.34 (m, 6H), 1.64 (t, J = 7.0 Hz, 3H).

### Example 135. 6-ethoxy-2-methyl-N-(5-(piperazin-1-yl)pyridin-2-yl)-2H-pyrazolo[3,4-b]pyridine-5-carboxamide (Compound 730).

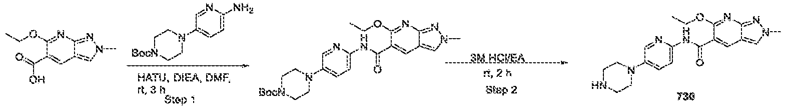

### Step 1: Preparation of tert-butyl 4-(6-(6-ethox-2-methyl-2H-pyrazolo[3,4-b]pyridine-5-carboxamido)pyridin-3-yl)piperazine-1-carboxylate.

To a mixture of 6-ethoxy-2-methyl-2H-pyrazolo[3,4-b]pyridine-5-carboxylic acid (150 mg, 0.68 mmol) in DMF (3 mL) were added tert-butyl 4-(6-aminopyridin-3-yl)piperazine-1-carboxylate (226 mg, 0.81 mmol), HATU (773 mg, 2.04 mmol) and DIEA (438 mg, 3.4 mmol). The mixture was stirred at rt for 3 h. LCMS showed the reaction was completed. The mixture was diluted with water (5 mL) and the precipitate was filtered, washed with water and dried in vacuo to give crude product (150 mg, Y: 45.9 %) as a yellow solid. ESI-MS (M+H)⁺: 482.3.

### Step 2: Preparation of 6-ethoxy-2-methyl-N-(5-(piperazin-1-yl)pyridin-2-yl)-2H-pyrazolo[3,4-b]pyridine-5-carboxamide.

A mixture of tert-butyl 4-(6-(6-ethoxy-2-methyl-2H-pyrazolo[3,4-b]pyridine-5-carboxamido)pyridin-3-yl)piperazine-1-carboxylate (150 mg, 0.31 mmol) in 3M HCl/EA (3 mL) was stirred at rt for 2 h. LCMS showed the reaction was completed. The mixture was added H₂O (5 mL), extracted with EA (10 mL*3), the aqueous layer was concentrated in vacuo and the residue was purified by prep-HPLC (0.05 % NH₃.H₂O in water / ACN) to give title product (72.6 mg, Y: 55.8 %) as a yellow solid. ESI-MS (M+H⁺): 382.1. ¹H NMR (400 MHz, DMSO-*d*₆) δ 8.77 (s, 1H), 8.46 (s, 1H), 8.13 (d, *J* = 8.9 Hz, 1H), 8.07 - 8.02 (m, 1H), 7.47 - 7.41 (m, 1H), 4.55 (q, *J* = 7.0 Hz, 2H), 4.13 (s, 3H), 3.09 - 3.04 (m, 4H), 2.87 - 2.82 (m, 4H), 1.47 (t, *J =* 7.0 Hz, 3H).

### Example 136. 7-ethoxy-2-methyl-N-(6-(4-methylpiperazin-1-yl)pyridazin-3-yl)imidazo[1,2-a]pyrimidine-6-carboxamide formic acid (Compound 731).

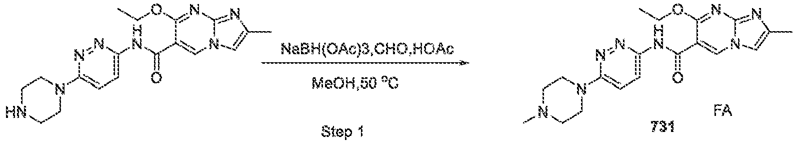

### Step 1: Preparation of (6-(4-methylpiperazin-1-yl)pyridazin-3-yl)imidazo[1,2-a]pyrimidine-6-carboxamide formic acid.

To a mixture of 7-ethoxy-2-methyl-N-(6-(piperazin-1-yl)pyridazin-3-yl)imidazo[1,2-a]pyrimidine-6-carboxamide (110 mg, 0.288 mmol), AcOH (52 mg, 0.864 mmol) in MeOH (10 mL) were added NaBH(OAc)₃ (305 mg, 1.440 mmol) and (HCHO)n (43 mg, 1.44 mmoL). The mixture was stirred at 50°C for 5 h. The mixture was filtered and the filtrate was concentrated in vacuo. The residue was purified by prep-HPLC (0.1 % FA in water/ACN) to give title product (11.0 mg, Y: 10 %) as a yellow solid. ESI-MS (M+H⁺): 397.1. ¹H NMR (400 MHz, MeOD-*d*₄) δ 9.34 (s, 1H), 8.50 (s, 1H), 8.40 (d, J = 9.9 Hz, 1H), 7.50 (s, 1H), 7.42 (d, J = 9.9 Hz, 1H), 4.76 (q, J = 7.1 Hz, 2H), 3.72 - 3.66 (m, 4H), 2.73 - 2.68 (m, 4H), 2.45 (s, 3H), 2.39 (s, 3H), 1.62 (t, J = 7.1 Hz, 3H).

### Example 137. 7-ethoxy-2-methyl-N-(6-(piperazin-1-yl)pyridazin-3-yl)imidazo[1,2-alpyrimidine-6-carboxamide formate (Compound 732).

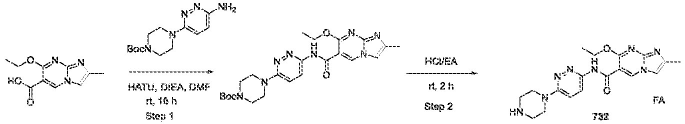

### Step 1: Preparation of tert-butyl 4-(6-(7-ethoxy-2-methylimidazo [1,2-a]pyrimidine-6-carboxamido)pyridazin-3-yl)piperazine-1-carboxylate.

To a mixture of 7-ethoxy-2-methylimidazo[1,2-a]pyrimidine-6-carboxylic acid (159 mg, 0.72 mmol) in DMF (10 mL) were added HATU (408 mg, 1.08 mmol) and DIEA (462 mg, 3.58 mmol) and the mixture was stirred at 40 °C for 0.5 h. Then tert-butyl 4-(6-aminopyridazin-3-yl)piperazine-1-carboxylate (200 mg, 0.72 mmol) was added and the mixture was stirred at 40 °C for 16 h. The mixture was diluted with H₂O (15 mL) and stirred for 10 min. The precipitate was filtered, washed with water and concentrated in vacuo to give title product (250 mg, crude) as a yellow solid. ESI-MS (M+H)⁺:483.1.

### Step 2: Preparation of 7-ethoxy-2-methyl-N-(6-(piperazin-1-yl)pyridazin-3-yl)imidazo[1,2-a]pyrimidine-6-carboxamide formate.

A mixture of tert-butyl 4-(6-(7-ethoxy-2-methylimidazo [1, 2-a] pyrimidine-6-carboxamido) pyridazin-3-yl)piperazine-1-carboxylatet (90 mg, 0.187mmol) in 3M HCl / EA (2 mL) was stirred at rt for 1 h. The mixture was concentrated in vacuo and the residue was purified by prep-HPLC (0.1 FA in water / CH₃CN) to give title product (19.96 mg, Y: 28 %) as a yellow solid. ESI-MS (M+H⁺):383.1. ¹H NMR (400 MHz, DMSO-*d*₆) δ 10.64 (s, 1H), 9.34 (s, 1H), 8.28 - 8.08 (m, 2H), 7.56 (d, *J* = 0.9 Hz, 1H), 7.44 (d, *J* = 9.9 Hz, 1H), 4.54 (q, *J* = 7.0 Hz, 2H), 3.61 - 3.56 (m, 4H), 3.01 - 2.94 (m, 4H), 2.29 (s, 3H), 1.46 (t, *J* = 7.0 Hz, 3H).

### Example 138. N-(6-(3-(tert-butylamino)pyrrolidin-1-yl)pyridazin-3-yl)-7-ethoxy-2-methylimidazo[1,2-a]pyridine-6-carboxamide (Compound 733).

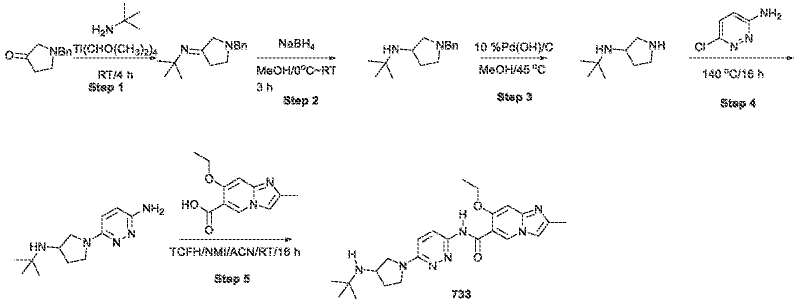

### Step 1: Preparation of (E)-1-benzyl-N-(tert-butyl)pyrrolidin-3-imine.

A dry vial was charged with 1-benzylpyrrolidin-3-one (20 g, 0.114 mol), 2-methylpropan-2-amine (19.2 g, 0.263 mol) and Titanium tetraisopropanolate (29.2 mL, 0.103 mol). The mixture was purged with N₂ for 15 min and then allowed to stir at room temperature for 2 h. The resulting (E)-1-benzyl-N-(tert-butyl)pyrrolidin-3-imine was used to next step without further purification(30 g, crude). ESI-MS (M+H)⁺: 231.2.

### Step 2: Preparation of 1-benzyl-N-(tert-butyl)pyrrolidin-3-amine.

To the mixture of (E)-1-benzyl-N-(tert-butyl)pyrrolidin-3-amine (26.3 g, 0.11 mol) was added dry methanol (260 mL) and the reaction mixture was cooled to 0 °C. NaBH₄ (8 g, 0.22 mol) was added slowly in portions (caution: very exothermic reaction). Once evolution of the gas subsided, the mixture was warmed to room temperature and stirred for 2 h at RT. Upon completion, 0.1 M NaOH solution (100 mL) was added to precipitate the titanium salts. The biphasic mixture was filtered through celite and washed with methanol. The solvent was removed under vacuum and the crude oil was purified by silica gel column chromatography (DCM/MeOH=10:1) to give title compound (10 g, 37.7 % yield) as a black solid. ESI-MS (M+H)⁺: 233.1. ¹H NMR (400 MHz, CDCl₃) δ 7.33 - 7.30 (m, 4H), 7.26 - 7.22 (m, 1H), 3.62 - 3.53 (m, 2H), 3.47 - 3.39 (m, 1H), 2.96 - 2.91 (m, 1H), 2.71 - 2.63 (m, 1H), 2.51 - 2.43 (m, 1H), 2.24 - 2.14 (m, 1H), 2.11 (dd, *J* = 8.9. 7.3 Hz, 1H), 1.51 - 1.42 (m, 1H), 1.07 (s, 9H).

### Step 3: Preparation of N-(tert-butyl)pyrrolidin-3-amine.

To an oven-dry round bottom flask containing palladium hydroxide on activated carbon (300 mg, 10 % wt) was added 1-benzyl-N-(tert-butyl)pyrrolidin-3-amine (3 g, 0.013 mol) and MeOH (20 mL). The mixture was charged with H₂ for 5 minutes and a balloon of H₂ was placed on top of the flask and the reaction was stirred for 16 h at 45 °C. The reaction mixture was filtered through Celite, washed with MeOH and concentrated to afford N-(tert-butyl)pyrrolidin-3-amine (1.5 g, 81.7 % yield) as a colorless oil. ESI-MS (M+H)⁺: 143.2. ¹H NMR (400 MHz, CDCl₃) δ 3.43 - 3.38 (m, 1H), 3.18 - 3.08 (m, 2H), 2.98 - 2.90 (m, 1H), 2.70 - 2.62 (m, 1H), 2.17 - 2.05 (m, 1H), 1.57 - 1.46 (m, 1H), 1.14 - 1.08 (m, 9H).

### Step 4: Preparation of 6-(3-(tert-butylamino)pyrrolidin-1-yl)pyridazin-3-amine.

A mixture of N-(tert-butyl)pyrrolidin-3-amine (1 g, 6.99 mmol) and 6-chloropyridazin-3-amine (757 mg, 6.99 mmol) was heated at 140 °C for 16 h. After cooling to rt, the reaction mixture was diluted with MeOH (10 mL), The precipitate was filtered, washed with MeOH (2 mL) and dried in vacuo to give 6-(3-(tert-butylamino)pyrrolidin-1-yl)pyridazin-3-amine (500 mg, 30.2 % yield) as a brown solid. ESI-MS (M+H)⁺: 236.2. ¹H NMR (400 MHz, DMSO-*d*₆) δ 7.22 (d, *J* = 9.6 Hz, 1H), 7.09 (d, *J* = 9.7 Hz, 1H), 6.69 (s, 2H), 4.08 - 4.00 (m, 1H), 3.82 - 3.74 (m, 1H), 3.66 - 3.61 (m, 1H), 3.61 - 3.56 (m, 1H), 3.37 - 3.33 (m, 1H), 2.45 - 2.24 (m, 2H), 1.37 (s, 9H).

### Step 5: Preparation of N-(6-(3-(tert-butylamino)pyrrolidin-1-yl)pyridazin-3-yl)-7-ethoxy-2-methylimidazo[1,2-a]pyridine-6-carboxamide.

To a mixture of 6-(3-(tert-butylamino)pyrrolidin-1-yl)pyridazin-3-amine (200 mg, 0.85 mmol) and 7-ethoxy-2-methylimidazo[1,2-a]pyridine-6-carboxylic acid (187 mg, 0.85 mmol) in ACN (10 mL) were added TCFH (359 mg, 1.28 mmol) and NMI (209 mg, 2.55 mmol). The mixture was stirred at RT overnight. The mixture was filtered, the crude solid was purified by prep-HPLC (0.05% NH₃.H₂O in water /CH₃CN) to give title product (60 mg, Y: 16.1 %) as a white solid. ESI-MS (M+H)⁺: 438.1. ¹H NMR (400 MHz, MeOD-*d*₄) δ 9.03 (s, 1H), 8.33 *(d, J=* 9.7 Hz, 1H), 7.55 (s, 1H), 7.02 (d, *J* = 9.8 Hz, 1H), 6.94 (s, 1H), 4.37 (q, *J=* 6.9 Hz, 2H), 3.87 - 3.80 (m, 1H), 3.70 - 3.61 (m, 2H), 3.50 - 3.41 (m, 1H), 3.20 - 3.14 (m, 1H), 2.38 (s, 3H), 2.37 - 2.30 (m, 1H), 1.94 - 1.86 (m, 1H), 1.66 (t, *J=* 7.0 Hz, 3H), 1.22 (s, 9H).

### Example 139. N-(5-(3,3-dimethylpiperazin-1-yl)pyrazin-2-yl)-6-ethoxy-2-methyl-2H-indazole-5-carboxamide hydrochloride (Compound 734).

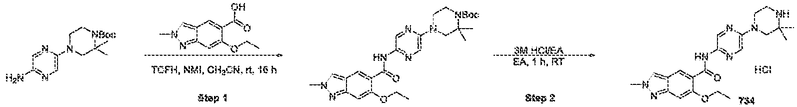

### Step 1: Preparation of tert-butyl 4-(5-(6-ethox),-2-methyl-2H-indazole-5-carboxamido)pyrazin-2-yl)-2,2-dimethylpiperazine-1-carboxylate.

To a mixture of tert-butyl 4-(5-aminopyrazin-2-yl)-2,2-dimethylpiperazine-1-carboxylate (556 mg, 1.81 mmol) and 6-ethoxy-2-methyl-2H-indazole-5-carboxylic acid (400 mg,1.81 mmol) in MeCN (15 mL) were added DIEA (1.17 g, 9.050 mmol) and HATU (2.1 g, 5.43 mmol). The mixture was stirred at rt for 3 h. After diluting with water, the mixture was filtered and the cake was washed with MeOH (6 mL) and dried to give title product (300 mg, 32.4 %) as a white solid. ESI-MS (M+H)⁺: 510.3. ¹H NMR (400 MHz, CDCl₃) δ 10.45 (s, 1H), 9.22 (d, J = 1.4 Hz, 1H), 8.74 (s, 1H), 7.98 (s, 1H), 7.69 (d, J = 1.4 Hz, 1H), 7.08 (s, 1H), 4.31 (q, J = 7.0 Hz, 2H), 4.20 (s, 3H), 3.89 (t, J = 5.7 Hz, 2H), 3.81 (s, 2H), 3.56 (t, J = 5.7 Hz, 2H), 1.69 - 1.66 (m, 3H), 1.50 (s, 9H), 1.42 (s, 6H).

### Step 2: Preparation of N-(5-(3,3-dimethylpiperazin-1-yl)pyrazin-2-yl)-6-ethoxy-2-methyl-2H-indazole-5-carboxamide hydrochloride.

To a mixture of tert-butyl 4-(5-(6-ethoxy-2-methyl-2H-indazole-5-carboxamido)pyrazin-2-yl)-2,2-dimethylpiperazine-1-carboxylate (300 mg, 0.59 mmol) in EA (5 mL) was added EA/HCl (5 mL). The mixture was stirred at rt for 1 h. After concentration, the residue was purified by pre-HPLC to give title product (97 mg, 37 %) as a white solid. ESI-MS (M+H)⁺: 410.1. ¹H NMR (400 MHz, DMSO-*d*₆) δ 10.56 (s, 1H), 9.51 (s, 2H), 9.02 (s, 1H), 8.47 (s, 1H), 8.43 (s, 1H), 8.25 (s, 1H), 7.14 (s, 1H), 4.27 (q, J = 6.6 Hz, 2H), 4.14 (s, 3H), 3.79 (br s, 2H), 3.65 (s, 2H), 3.22 (br s, 2H), 1.50 (t, J = 6.8 Hz, 3H), 1.37 (s, 6H).

### Example 140. 7-methoxy-2-methyl-N-(5-(piperidin-4-yl)pyridin-2-yl) imidazo[1,2-a]pyridine-6-carboxamide hydrochloride (Compound 735).

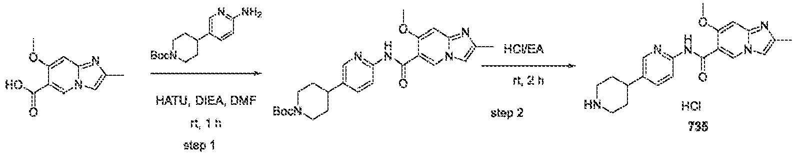

### Step 1: preparation of tert-butyl 4-(6-(7-methoxy-2-methylimidazo[1, 2-a]pyridine-6-carboxamido)pyridin-3-yl)piperidine-1-carboxylate.

A mixture of 7-methoxy-2-methylimidazo[1,2-a]pyridine-6-carboxylic acid (74 mg, 0.36 mmol), HATU (103 mg, 0.27 mmol), tert-butyl 4-(6-aminopyridin-3-yl)piperidine-1-carboxylate (50 mg, 0.18 mmol) and DIEA (70 mg, 0.54 mmol) in DMF (5 mL) was stirred at r.t. for 1 h. The reaction mixture was diluted with water (10 mL) and extracted with EtOAc (30 mL). The organic layer was washed with brine, dried with Na₂SO₄ and evaporated to give crude title compound. The crude was purified by reverse phase column (0.1% FA in water/CH₃CN) to give title compound (40 mg, 48% yield), ESI-MS (M+H)⁺: 466.3.

### Step 2: Preparation of 7-methoxy-2-methyl-N-(5-(piperidin-4yl)pyridin-2-yl) imidazo[1,2-aJpyridine-6-carboxamide hydrochloride.

tert-Butyl 4-(6-(7-methoxy-2-methylimidazo[1,2-a] pyridine-6-carboxamido)pyridin-3-yl)piperidine-1-carboxylate (40 mg, 0.086 mmol) was dissolved in 3M HCl/EA solution (5 mL) and the mixture was stirred at r.t. for 2 h. The precipitate was collected by filtration, washed with EA (3 mL x 3) and dried under vacuum to afford the title compound as hydrochloride salt (15 mg, 48% yield). ESI-MS (M+H)⁺: 366.2. ¹H NMR (400 MHz, DMSO-*d*₆) δ 10.94 (s, 1H), 9.20 (br s, 3H), 8.27 (s, 1H), 8.17 (d, *J* = 8.1 Hz, 1H), 7.96 (s, 1H), 7.78 (dd, *J* = 8.6, 2.1 Hz, 1H), 7.32 (s, 1H), 4.07 (s, 3H), 3.44 - 3.27 (m, 2H), 3.07 - 2.86 (m, 3H), 2.46 (s, 3H), 2.02 - 1.80 (m, 4H).

### Example 141. (S)-6-methoxy-2-methyl-N-(5-(3-methylpiperazin-1-yl)pyrazin-2-yl)-2H-indazole-5-carboxamide hydrochloride (Compound 736).

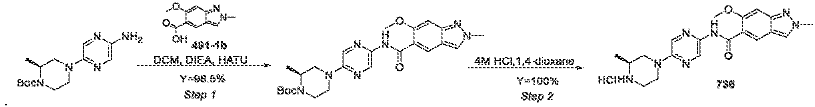

### Step 1: Preparation of tert-butyl (S)-4-(5-(6-methoxy-2-methyl-2H-indazole-5-carboxamido)pyrazin-2-yl)-2-methylpiperazine-1-carboxylate.

To a solution of 6-methoxy-2-methyl-2H-indazole-5-carboxylic acid (214 mg, 1.04 mmol) in DCM (10 mL) were added DIEA (0.48 mL, 3.12 mmol), HATU (488 mg, 1.56 mmol), and tert-butyl (S)-4-(5-aminopyrazin-2-yl)-2-methylpiperazine-1-carboxylate compound (300 mg, 1.04 mmol), and the mixture was stirred at room temperature overnight. The reaction mixture was diluted with DCM (50 mL) and washed with brine.The organic layer was dried over anhydrous Na₂SO₄, filtered, concentrated under vacuum. The residue was purified by silica gel column chromatography (EA/PE = 100/1 to 40/1) to afford the title compound (460 mg, 0.96 mmol) as a yellow solid. ESI-MS (M+H⁺): 482.1.

### Step 2: Preparation of (S)-6-methoxy-2-methyl-N-(5-(3-methylpiperazin-1-yl)pyrazin-2-yl)-2H-indazole-5-carboxamide hydrochloride.

A suspension of tert-butyl (S)-4-(5-(6-methoxy-2-methyl-2H-indazole-5-carboxamido)pyrazin-2-yl)-2-methylpiperazine-1-carboxylate (460 mg, 096 mmol) in 4M HCl 1,4-dioxance (20 mL) was stirred at room temperature overnight. The mixture was filtered and the solid was dried in vacuum to afford title product (350 mg, 87.5%,) as a yellow solid. ESI-MS (M+H⁺): 382.3. ¹H NMR (400 MHz, DMSO-*d*₆) δ 10.40 (s, 1H), 9.45 (s, 1H), 9.27 (s, 1H), 9.02 (s, 1H), 8.45 (s, 1H), 8.31 (s, 1H), 8.23 (s, 1H), 7.14 (s, 1H), 4.31 (t, *J* = 11.0 Hz, 2H), 4.14 (s, 3H), 3.98 (s, 3H), 3.40-3.30 (m, 2H), 3.28-3.19 (m, 1H), 3.13 - 2.97 (m, 2H), 1.31 (d, *J* = 6.5 Hz, 3H).

### Example 142. (S)-N-(6-(3,4-dimethylpiperazin-1-yl)pyridazin-3-yl)-7-ethoxy-2-methylimidazo[1,2-a]pyridine-6-carboxamide formate (Compound 737).

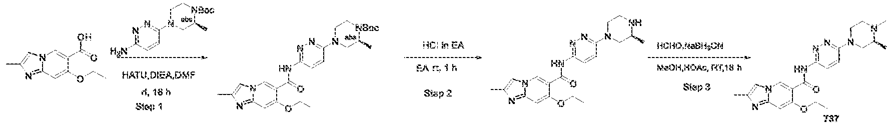

### Step 1: Preparation of tert-butyl (S)-4-(6-(7-ethoxy-2-methylimidazo[1,2-a]pyridine-6-carboxamido)pyridazin-3-yl)-2-methylpiperazine-1-carboxylatetert-butyl.

To a solution of 7-ethoxy-2-methylimidazo[1,2-a]pyridine-6-carboxylic acid (188 mg, 0.85 mmol), tert-butyl (S)-4-(6-aminopyridazin-3-yl)-2-methylpiperazine-1-carboxylate (200 mg, 0.68 mmol) and DIEA (551 mg, 4.30 mmol) in DMF (5 mL) was added HATU (486 mg, 1.28 mmol) at rt. the mixture was stirred for 16 h. After diluting with water, the solid was collected by filtration, washed with H₂O and dried under vacuum to give title product (200 mg, Y: 59.2%) as a yellow solid. ESI-MS (M+H)⁺: 496.2.

### Step 2: Preparation of (S)-7ethoxy-1-methylN(6-(3-methylpiperazin-1yl)pyridazin-3-yl)imidazo[1,2-]pyridine-6-carboxamide.

To a solution of the tert-butyl (S)-4-(6-(7-ethoxy-2-methylimidazo[1,2-a]pyridine-6-carboxamido)pyridazin-3-yl)-2-methylpiperazine-1-carboxylatetert-butyl (200 mg, 0.40 mmol) in EtOAc (3 mL) was added HCl-EA (3M, 3 mL), the mixture was stirred at rt for 1 h. After concentration, title product (160 mg, yield: 94%) was obtained as a yellow solid. ESI-MS (M+H)⁺: 396.5.

### Step 3: Preparation of (S)-N-(6-(3,4-dimethylpiperazin-1-yl)pyridazin-3-yl)-7-ethoxy-2-methylimidazo[1,2-a]pyridine-6-carboxamide formate.

To a solution of (S)-7-ethoxy-2-methyl-N-(6-(3-methylpiperazin-1-yl)pyridazin-3-yl)imidazo[1,2-a]pyridine-6-carboxamide (180 mg, 0.46 mmol) in MeOH (5 mL) were added (HCHO)n (68 mg, 2.28 mmol) and acetic acid (82 mg, 1.37 mmol). The mixture was stirred at 50 °C for 1 h, then sodium cyanoborohydride (24 mg, 0.38 mmol) was added and the mixture was stirred at 50 °C for 16 h. Water (5 mL) was added and the solution was concentrated in vacuo. The crude was purified by prep-HPLC (0.1% FA in water / CH₃CN) to give title compound (42 mg, yield: 22.6 %) as a yellow solid. ESI-MS (M+H)⁺: 410.2. ¹H NMR (400 MHz, MeOD-*d*₄) δ 9.04 (s, 1H), 8.41 (s, 1H), 8.39 (s, 1H), 7.56 (s, 1H), 7.43 (d, *J* = 9.9 Hz, 1H), 6.97 (s, 1H), 4.37 (q, *J* = 6.9 Hz, 2H), 4.24 (d, *J =* 9.3 Hz, 2H), 3.28 (br s, 2H), 3.05 - 2.95 (m, 1H), 2.89 - 2.86 (m, 2H), 2.67 (s, 3H), 2.37 (s, 3H), 1.63 (t, *J=* 6.9 Hz, 3H), 1.33 (d, *J=* 6.3 Hz, 3H).

### Example 143. (S)-6-ethoxy-N-(6-(hexahydropyrrolo[1,2-a]pyrazin-2(1H)-yl)pyridazin-3-yl)-2-methyl-2H-indazole-5-carboxamide (Compound 738).

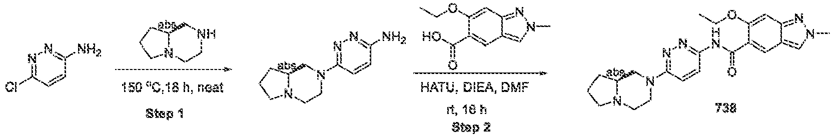

### Step 1: Preparation of (S)-6-(hexahydropyrrolo[1,2-a]pyrazin-2(1H)-yl)pyridazin-3-amine.

A mixture of 6-chloropyridazin-3-amine (150 mg, 1.16 mmol) and (S)-octahydropyrrolo[1,2-a]pyrazine (293 mg, 2.33 mmol) was stirred at 150 °C under Ar atmosphere for 18 h. The mixture was diluted with water (10 mL), extracted with EA (20 mL x3). The organic layer was washed with brine, dried with Na₂SO₄ and concentration in vacuo to give title product (260 mg, crude) as a black solid. ESI-MS (M+H)⁺: 220.2.

### Step 2: Preparation of (S)-6-ethoxy-N-(6-(hexahydropyrrolo[1,2-a]pyrazin-2(1H)-yl)pyridazin-3-yl)-2-methyl-2H-indazole-5-carboxamide.

To a solution of (S)-6-(hexahydropyrrolo[1,2-a]pyrazin-2(1H)-yl)pyridazin-3-amine (200 mg, 0.91 mmol), 6-ethoxy-2-methyl-2H-indazole-5-carboxylic acid (201 mg, 0.91 mmol) and HATU (530 mg, 1.39 mmol) in DMF (4 mL) was added DIEA (235 mg, 1.82 mmol). The mixture was stirred at rt under Ar atmosphere for 16 h. The mixture was diluted with water (20 mL), extracted with EA (40 mLx2). The organic layer was washed with brine (8 mL), dried over sodium sulfate, filtered and concentrated in vacuo. The residue was purified by prep-HPLC (0.1% FA in H₂O / MeCN ) to give title product (30 mg, 7.83%yield) as a white solid. ESI-MS (M+H)⁺: 422.2. ¹H NMR (400 MHz, CDCl₃) δ 10.94 (s, 1H), 8.71 (s, 1H), 8.49 (d, J = 9.6 Hz, 1H), 7.99 (s, 1H), 7.08 (s, 1H), 7.04 (d, J = 9.6 Hz, 1H), 4.44-4.29 (m, 3H), 4.25-4.20 (m, 4H), 3.17-3.09 (m, 3H), 2.76 (dd, J = 10.4 Hz, 1H), 2.37-2.31 (m, 1H), 2.20-2.08 (m, 2H), 1.92-1.77 (m, 3H), 1.70 (t, J = 7.2 Hz, 3H), 1.53-1.49 (m, 1H).

### Example 144. (R)-6-ethoxy-N-(6-(hexahydropyrrolo[1,2-a]pyrazin-2(1H)-yl)pyridazin-3-yl)-2-methyl-2H-indazole-5-carboxamide formate (Compound 739).

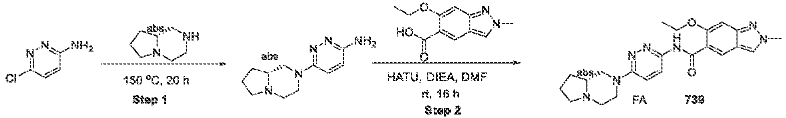

### Step 1: Preparation of (R)-6-(hexahydropyrrolo[1,2-alpyrazin-2(1H)-yl)pyridazin-3-amine.

A mixture of 6-chloropyridazin-3-amine (200 mg, 1.55 mmol) and (R)-octahydropyrrolo[1,2-a]pyrazine (390 mg, 3.10 mmol) was stirred at 150 °C under Ar atmosphere for 20 h. The mixture was diluted with water (10 mL), extracted with EA (20 mL x3). The organic layer was washed with brine, dried with Na₂SO₄ and concentrated in vacuo to give title product (350 mg, crude) as a black solid. ESI-MS (M+H)⁺: 220.2.

### Step 2: Preparation of (6-(hexahydropyrrolo[1,2-alpyrazin-2(1H)-yl)pyridazin-3-yl)-2-methyl-2H-indazole-5-carboxamide formate.

To a mixture of (R)-6-(hexahydropyrrolo[1,2-a]pyrazin-2(1H)-yl)pyridazin-3-amine (200 mg, 0.91 mmol), 6-ethoxy-2-methyl-2H-indazole-5-carboxylic acid (201 mg, 0.91 mmol) and HATU (530 mg, 1.39 mmol) in DMF (4 mL) was added DIEA (235 mg, 1.82 mmol). The mixture was stirred at rt under Ar atmosphere for 16 h. The mixture was diluted with water (20 mL), extracted with EA (40 mLx2). The organic layer was washed with brine (20 mL), dried over sodium sulfate, filtered and concentrated in vacuo. The residue was purified by prep-HPLC (0.1% FA in H₂O / MeCN) to give title product (32 mg, 8.35%yield) as a white solid. ESI-MS (M+H)⁺: 422.2. ¹H NMR (400 MHz, CDCl₃) δ 10.94 (s, 1H), 8.71 (s, 1H), 8.49 (d, J = 10 Hz, 1H), 8.09 - 8.06 (m, 1H), 7.99 (s, 1H), 7.08 (s, 1H), 7.04 (d, J = 9.6 Hz, 1H), 4.44 - 4.29 (m, 4H), 4.25 - 4.20 (m, 4H), 3.17 - 3.09 (m, 3H), 2.75 (t, J = 12 Hz, 1H), 2.37 - 2.31 (m, 1H), 2.20 - 2.09 (m, 2H), 1.94 - 1.75 (m, 3H), 1.70 (t, J = 7.2 Hz, 3H), 1.54 - 1.51 (m, 1H).

### Example 145. 7-ethoxy-2-methyl-N-(6-((3S,5R)-3,4,5-trimethylpiperazin-1-yl)pyridazin-3-yl)imidazo[1,2-a]pyridine-6-carboxamide (Compound 740).

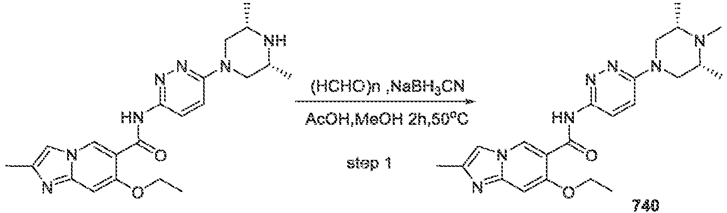

### Step 1: Preparation of (6-((3S,5R)-3,4,5-trimethylpiperazin-1-yl)pyridazm-3-yl)imidazo[1,2-a]pyridine-6-carboxamide.

To a mixture of N-(6-((3S,5R)-3,5-dimethylpiperazin-1-yl)pyridazin-3-yl)-7-ethoxy-2-methylimidazo[1,2-a]pyridine-6-carboxamide (70 mg, 0.17 mmol) and AcOH (26 mg, 0.43 mmol) in MeOH (7 mL) were added NaBH₃CN (54 mg, 0.80 mmol) and (HCHO)n (25.3 mg, 0.80 mmol), the mixture was stirred at 50 °C for 2 hours. The mixture was filtered and the filtrate was concentrated in vacuo. The residue was purified by prep-HPLC (0.1 % FA in water/ACN) to give title product (12 mg, 16.7%) as a white solid. ESI-MS (M+H)⁺: 424.3. ¹H NMR (400 MHz, CD₃OD-*d*₄) δ 9.03 (s, 1H), 8.36 (d, *J* = 10.0 Hz, 1H), 7.54 (s, 1H), 7.39 (d, *J =* 9.9 Hz, 1H), 6.95 (s, 1H), 4.36 (q, *J* = 7.0 Hz, 2H), 4.16 (d, *J* = 12.6 Hz, 2H), 2.85 - 2.67 (m, 2H), 2.46 - 2.19 (m, 8H), 1.63 (t, *J* = 6.9 Hz, 3H), 1.22 (d, *J* = 6.2 Hz, 6H).

### Example 146. (S)-7-ethoxy-2-methyl-N-(5-methyl-6-(3-methylpiperazin-1-yl)pyridazin-3-yl)imidazo[1,2-a]pyridine-6-carboxamide formate (Compound 741).

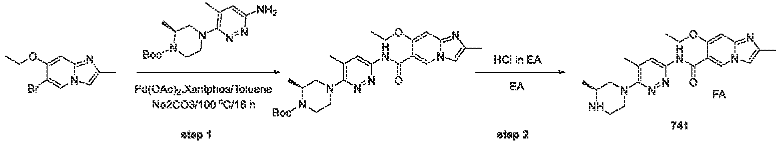

### Step 1: Preparation of tert-butyl (S)-4-(6-(7-ethoxy-2-methylimidazo[1,2-a]pyridine-6-carboxamido)-4-methylpyridazin-3-yl)-2-methylpiperazine-1-carboxylate.

To a solution of 6-bromo-7-ethoxy-2-methylimidazo[1,2-a]pyridine (200 mg, 0.79 mmol) in toluene (15 mL) were added Pd(OAc)₂ (18 mg, 0.079 mmol), Xantphos (91 mg, 0.16 mmol), Na₂CO₃ (251 mg, 2.37 mmol) and tert-butyl (S)-4-(6-amino-4-methylpyridazin-3-yl)-2-methylpiperazine-1-carboxylate (244 mg, 1.18 mmol ), the mixture was charged with CO for three times and stirred at 100°C for 16 h under CO balloon. The mixture was filtered and the filtrate was concentrated to afford the title compound (250 mg, crude) as a grey solid. ESI-MS (M+H)⁺: 510.2.

### Step 2: Preparation of (S)-7-ethoxy-2-methyl-N-(5-methyl-6-(3-methylpiperazin-1-yl)pyridazin-3-yl)imidazo[1,2-a]pypidine-6-carboxamide formate.

A solution of tert-butyl (S)-4-(6-(7-ethoxy-2-methylimidazo[1,2-a]pyridine-6-carboxamido)-4-methylpyridazin-3-yl)-2-methylpiperazine-1-carboxylate (250 mg, 0.49 mmol) in 3M HCl/EtOAc (5 mL) was stirred for 2 h at RT. The mixture was concentrated in vacuo. The residue was purified by prep-HPLC (0.05 % FA in water / ACN) to give title product (6 mg, 1.7 % with two steps) as a yellow solid. ESI-MS (M+H) ⁺: 410.5. ¹H NMR (400 MHz, DMSO-*d*₆) δ 10.83 (s, 1H), 9.04 (s, 1H), 8.25 (s, 1H), 8.15 (s, 1H), 7.67 (s, 1H), 7.01 (s, 1H), 4.28 (q, *J* = 6.8 Hz, 2H), 3.52 (d, *J =* 11.4 Hz, 2H), 3.28 - 3.25 (m, 2H), 3.16 - 3.07 (m, 2H), 2.92 - 2.84 (m, 1H), 2.36 (s, 3H), 2.29 (s, 3H), 1.50 (t, *J* = 6.9 Hz, 3H), 1.23 (d, *J* = 6.4 Hz, 3H).

### Example 147. 6-ethoxy-2-methyl-N-(5-(piperazin-1-yl)pyrazin-2-yl)-2H-pyrazolo[3,4-b]pyridine-5-carboxamide hydrochloride (Compound 742).

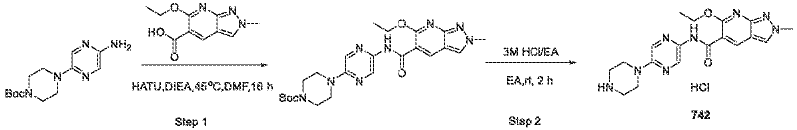

### Step 1: Preparation of tert-butyl 4-(5-(6-ethoxy-2-methyl-2H-pyrazolo[3,4-b]pyridine-5-carboxamido)pyrazin-2-yl)piperazine-1-carboxylate.

To a mixture of tert-butyl 4-(5-aminopyrazin-2-yl)piperazine-1-carboxylate (150 mg, 0.69 mmol) in DMF (5 mL) were added HATU (314 mg, 0.83 mmol), DIEA (356 mg, 2.76 mmol) and 6-ethoxy-2-methyl-2H-pyrazolo[3,4-b]pyridine-5-carboxylic acid (203 mg, 1.56 mmol). The mixture was stirred at 25°C for 16 h. LCMS showed the starting material was consumed completely. The mixture was diluted with water (10 mL), extracted with EA (20 mLx3). The organic layer was washed with brine, dried over Na₂SO₄ and concentrated in vacuo to give title product (120 mg, 46.33%) as a yellow solid. ESI-MS (M+H)⁺: 483.3.

### Step 2: Preparation of 6-ethoxy-2-methyl-N-(5-(piperazin-1-yl)pyrazin-2-yl)-2H-pyrazolo [3,4-b]pyridine-5-carboxamide hydrochloride.

To a mixture of tert-butyl 4-(5-(6-ethoxy-2-methyl-2H-pyrazolo[3,4-b]pyridine-5-carboxamido)pyrazin-2-yl)piperazine-1-carboxylate (120 mg, 0.25 mmol) in EA (3 mL) was added 3 M HCl/EA (3 mL). The mixture was stirred at rt for 2 h. LCMS showed the starting material was consumed completely. The mixture was concentrated in vacuo. The crude was purified by prep-HPLC (0.05 % HCl in water / CH₃CN) to give title product (83 mg, Y: 87.28 %) as a yellow solid. ESI-MS (M+H⁺): 383.1. ¹H NMR (400 MHz, DMSO-*d*₆) δ 10.49 (s, 1H), 9.21 (brs, 2H), 9.04 (s, 1H), 8.73 (s, 1H), 8.47 (s, 1H), 8.25 (s, 1H), 4.54 (q, *J* = 6.9 Hz, 2H), 4.13 (s, 3H), 3.77 - 3.74 (m, 4H), 3.21 - 3.19 (m, 4H), 1.45 (t, *J* = 7.0 Hz, 3H).

### Example 148. (S)-N-(5-(3,4-dimethylpiperazin-1-yl)pyrazin-2-yl)-6-ethoxy-2-methyl-2H-indazole-5-carboxamide formate (Compound 743).

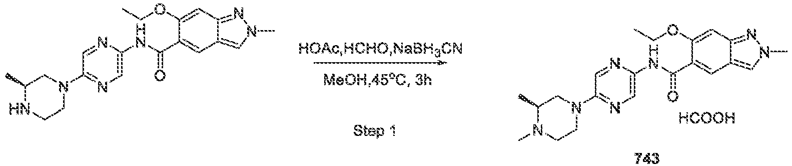

### Step 1: Preparation of (S)-N-(5-(3,4-dimethylpiperazin-1-yl)pyrazin-2-yl)-6-ethoxy-2-methyl-2H-indazole-5-carboxamide formate.

To a mixture of (S)-6-ethoxy-2-methyl-N-(5-(3-methylpiperazin-1-yl)pyrazin-2-yl)-2H-indazole-5-carboxamide (100 mg, 0.25 mmol) in MeOH (10 mL) were added HOAc (76 mg, 1.27 mmol) and (HCHO)n (38 mg, 1.27 mmol). The mixture was stirred at 45°C for 1 h. Then NaBH₃CN (49 mg, 0.76 mmol) was added and the mixture was stirring at 45°C for 2 h. The reaction mixture was diluted with H₂O (3 mL) and concentrated, the residue was purified by prep-HPLC (0.05 % FA in water / ACN) to give title product (44 mg, Y: 38.3%) as a white solid. ESI-MS (M+H)⁺:410.2. ¹H NMR (400 MHz, DMSO-*d*₆) δ 10.51 (s, 1H), 9.01 (s, 1H), 8.45 - 8.44 (m, 2H), 8.15 (d, *J* = 13.9 Hz, 1H), 7.13 (s, 1H), 6.51 (s, 1H), 4.27 (q, *J* = 6.8 Hz, 2H), 4.14 (s, 3H), 4.12 - 4.03 (m, 2H), 3.13 - 2.86 (m, 3H), 2.76 - 2.58 (m, 2H), 2.30 (s, 3H), 1.50 (t, *J* = 6.9 Hz, 3H), 1.10 (d, *J=* 6.1 Hz, 3H).

### Example 149. N-(5-((3S,5R)-3,5-dimethylpiperazin-1-yl)pyrazin-2-yl)-6-ethoxy-2-methylpyrazolo[1,5-a]pyridine-5-carboxamide hydrochloride (Compound 744).

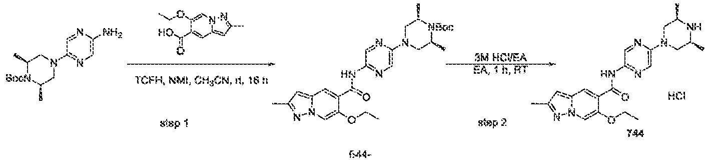

### Step 1: Preparation of tert-butyl (2S,6R)-4-(5-(6-ethoxy-2-methylpyrazolo[1,5-a]pyridine-5-carboxamido)pyrazin-2-yl)-2,6-dimethylpiperazine-1-carboxylate.

To a solution of tert-butyl (2R,6S)-4-(5-aminopyrazin-2-yl)-2,6-dimethylpiperazine-1-carboxylate (100 mg, 0.32 mmol) in ACN (10 mL) were added 6-ethoxy-2-methylpyrazolo[1,5-a]pyridine-5-carboxylic acid (78.82 mg, 0.36 mmol) and TCFH (140 mg, 0.48 mmol) and NMI (80 mg, 0.98 mmol ). The mixture was stirred at r.t. for 16 hours. LCMS showed the reaction was completed. The mixture was diluted with H₂O (10 mL), extracted with EA (20 mLx3). The organic phase was washed with brine, dried over sodium sulfate and concentrated in vacuo to give title compound (100 mg, 61.7%) as a yellow oil. ESI-MS (M+H)⁺: 510.4. ¹H NMR (400 MHz, CDCl₃) δ 10.25 (s, 1H), 9.19 (s, 1H), 8.44 (s, 1H), 8.11 (s, 1H), 7.90 (s, 1H), 6.45 (s, 1H), 4.33 - 4.27 (m, 2H), 4.23 (q, *J=* 7.0 Hz, 2H), 4.15 - 4.13 (m, 2H), 3.13 - 3.11 (m, 2H), 2.48 (s, 3H), 2.01 (s, 3H), 1.50 (s, 9H), 1.29 (d, *J* = 6.8 Hz, 6H).

### Step 2: Preparation of N-(5-((3S,5R)-3,5-dimethylpiperazin-1-yl)pyrazin-2-yl)-6-ethoxy-2-methylpyrazolo[1,5-a]pyridine-5-carboxamide hydrochloride.

A solution of tert-butyl (2S,6R)-4-(5-(6-ethoxy-2-methylpyrazolo[1,5-a]pyridine-5-carboxamido)pyrazin-2-yl)-2,6-dimethylpiperazine-1-carboxylate (100 mg, 0.19 mmol) in 3M HCl/EA(2 mL) was stirred at rt for 1 h. The mixture was diluted with water (15 mL) and extracted with EA (20 mLx3), the aqueous phase was concentrated in vacuo and lyophilized to give title compound (85.07 mg, 93.4%) as a yellow solid. ESI-MS (M+H)⁺: 410.2. ¹H NMR (400 MHz, DMSO-*d*₆) δ 10.54 (s, 1H), 9.67 (s, 1H), 9.24 (s, 1H), 9.00 (s, 1H), 8.49 (s, 1H), 8.28 (s, 1H), 8.10 (s, 1H), 6.54 (s, 1H), 4.42 (d, *J* = 12.7 Hz, 2H), 4.21 (d, *J* = 6.5 Hz, 2H), 3.31 - 3.30 (m, 2H), 2.95 (t, *J* = 12.4 Hz, 2H), 2.39 (s, 3H), 1.44 (s, 3H), 1.33 (d, *J=* 6.0 Hz, 6H).

### Example 150. 7-ethoxy-2-methyl-N-(6-(5-methylhexahydropyrrolo|3,4-c]pyrrol-2(1H)-yl)pyridazin-3-yl)imidazo[1,2-a]pyridine-6-carboxamide formate (Compound 745).

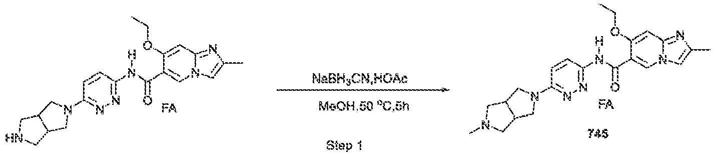

### Step 1: Preparation of 7-ethoxy-2-methyl-N-(6-(5-methylhexahydropyrrolo[3, 4-cJpyrrol-2(1H)-yl)pyridazin-3-yl)imidazo[1, 2-a]pyridine-6-carboxamide formate.

To a mixture of 7-ethoxy-N-(6-(hexahydropyrrolo[3,4-c]pyrrol-2(1H)-yl)pyridazin-3-yl)-2-methylimidazo[1,2-a]pyridine-6-carboxamide formate (130 mg, 0.32 mmol), AcOH (57 mg, 0.96 mmol) in MeOH (10 mL) were added NaBH₃CN (100 mg, 0.64 mmol) and (HCHO)n (135 mg, 1.60 mmol). The mixture was stirred at 50°C for 5 h. The mixture was filtered and the filtrate was concentrated in vacuo. The residue was purified by prep-HPLC (0.1 % FA in water /ACN) to give title product (52.0 mg, Y: 38.7 %) as a yellow solid. ESI-MS (M+H⁺): 422.2. ¹H NMR (400 MHz, MeOD-*d*₄) δ 9.04 (s, 1H), 8.40 (s, 1H), 8.37 (d, J = 9.8 Hz, 1H), 7.56 (s, 1H), 7.16 (d, J = 9.8 Hz, 1H), 6.95 (s, 1H), 4.36 (q, J = 6.9 Hz, 2H), 3.69 (dd, J = 11.0, 1.7 Hz, 2H), 3.58 (dd, J = 10.6, 7.0 Hz, 4H), 3.30 - 3.19 (m, 4H), 2.87 (s, 3H), 2.38 (s, 3H), 1.64 (t, J = 6.9 Hz, 3H).

### Example 151. 6-methoxy-2-methyl-N-(5-(piperazin-1-yl)pyrazin-2-yl)-2H-indazole-5-carboxamide formate (Compound 746).

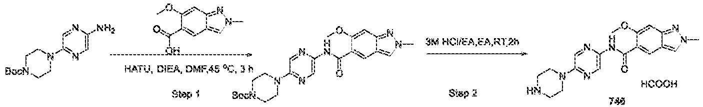

### Step 1: Preparation of tert-butyl 4-(5-(6-methoxy-2-methyl-2H-indazole-5-carboxamido)pyrazin-2-yl)piperazine-1-carboxylate.

To a mixture of tert-butyl 4-(5-aminopyrazin-2-yl)piperazine-1-carboxylate (200 mg, 0.72 mmol) in DMF (6 mL) were added HATU (327 mg, 0.86 mmol), DIEA (370 mg, 2.87 mmol) and 6-methoxy-2-methyl-2H-indazole-5-carboxylic acid (222 mg, 1.08 mmol). The mixture was stirred at 45°C for 3 h. LCMS showed the reaction was completed. The reaction mixture was diluted with H₂O (10 mL) and extracted with EA (30 mL). The organic phase was washed with brine, dried over sodium sulfate and concentrated to give title product (200 mg, 60 %) as a brown solid. ESI-MS (M+H)⁺: 468.2.

### Step 2: Preparation of 6-methoxy-2-methyl-N-(5-(piperazin-1-yl)pyrazin-2-yl)-2H-indazole-5-carboxamide formate.

To a mixture of tert-butyl 4-(5-(6-methoxy-2-methyl-2H-indazole-5-carboxamido)pyrazin-2-yl)piperazine-1-carboxylate (100 mg, 0.21 mmol) in EA (2 mL) was added 3M HCl/EA (2 mL). The mixture was stirred at RT for 2 h. LCMS showed the reaction was completed. The mixture was concentrated in vacuo, the residue was purified by prep-HPLC (0.05 % FA in water / ACN) to give title product (35 mg, Y: 40 %) as a yellow solid. ESI-MS (M+H)⁺:368.2. ¹H NMR (400 MHz, DMSO-*d*₆) δ 10.32 (s, 1H), 8.98 (s, 1H), 8.44 (s, 1H), 8.32 (s, 1H), 8.20 (s, 1H), 8.11 (s, 1H), 7.14 (s, 1H), 4.14 (s, 3H), 3.98 (s, 3H), 3.49 - 3.47 (m, 4H), 2.90 - 2.87 (m, 4H).

### Example 152. 1-(isobutyryloxy)ethyl (2S)-4-(6-(6-ethoxy-2-methyl-2H-indazole-5-carboxamido)pyridazin-3-yl)-2-methylpiperazine-1-carboxylate (Compound 747).

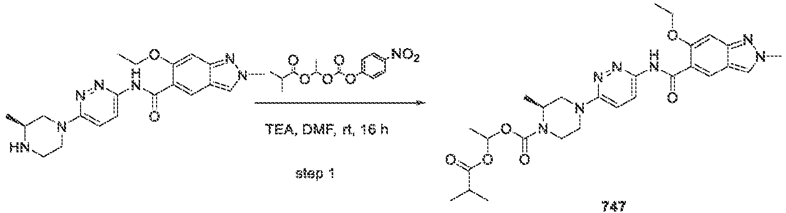

### Step 1: Preparation of 1-(isobutyryloxy)ethyl (2S)-4-(6-(6-ethoxy-2-methyl-2H-indazole-5-carboxamido)pyridazin-3-yl)-2-methylpiperazine-1-carboxylate.

To a solution of (S)-6-ethoxy-2-methyl-N-(6-(3-methylpiperazin-1-yl)pyridazin-3-yl)-2H-indazole-5-carboxamide hydrogen chloride (80 mg, 0.20 mmol) in DMF (5 mL) were added 1-(((4-nitrophenoxy)carbonyl)oxy)ethyl isobutyrate (90 mg, 0.30 mmol) and TEA (78 mg, 0.60 mmol). The mixture was stirred at rt overnight. Then mixture was concentrated in vacuo, and the residue was purified by prep-HPLC (0.05 % NH₃· H₂O in H₂O / CH₃CN) to give title product (50 mg, Y: 44.6 %) as a white solid. ESI-MS (M+H)⁺: 554.2. ¹H NMR (400 MHz, DMSO-*d*₆) δ 10.88 (s, 1H), 8.46 (d, J = 6.3 Hz, 2H), 8.28 (d, J = 9.9 Hz, 1H), 7.42 (d, J = 9.9 Hz, 1H), 7.15 (s, 1H), 6.74 - 6.66 (m, 1H), 4.32 - 4.18 (m, 4H), 4.13 - 4.12 (m, 4H), 3.89 - 3.82 (m, 1H), 3.29 - 3.15 (m, 2H), 3.01 - 2.90 (m, 1H), 2.59 - 2.52 (m, 1H), 1.52 (t, J = 6.9 Hz, 3H), 1.46 (d, J = 5.4 Hz, 3H), 1.15 (t, J = 6.9 Hz, 3H), 1.09 - 1.07 (m, 6H).

### Example 153. (7-ethoxy-N-(6-(hexahydropyrrolo[3,4-c]pyrrol-2(1H)-yl)pyridazin-3-yl)-2-methylimidazo[1,2-a]pyridine-6-carboxamide formate (Compound 748).

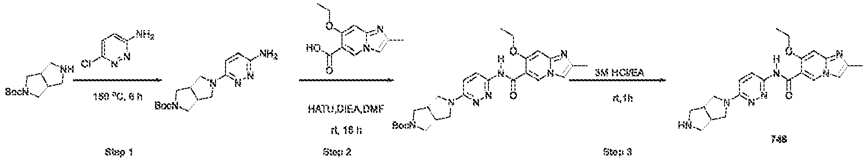

### Step 1: Preparation of tert-butyl 5-(6-aminopyndazin-3-yl)hexahydropyrrolo[3,4-c]pyrrole-2(1H)-carboxylate.

A solution of tert-butyl hexahydropyrrolo[3,4-c]pyrrole-2(1H)-carboxylate (5 g, 23.58 mmol) and 6-chloropyridazin-3-amine (1.533 g, 11.79 mmol) was stirred at 150 °C for 6 h. The mixture was purified by C18 flash (0.1% FA in water / CH₃CN) to give title product (3.2 g, 89%) as a yellow solid. ESI-MS (M+H)⁺: 306.2.

### Step 2: Preparation of tert-butyl 5-(6-(7-ethoxy-2-methylimidazo[1,2-a]pyridine-6-carboxamido)pyridazin-3-yl)hexahydropyrrolo[3,4-c]pyrrole-2(1H)-carboxylate.

To a solution of 7-ethoxy-2-methylimidazo[1,2-a]pyridine-6-carboxylic acid (173 mg, 0.79 mmol), tert-butyl 5-(6-aminopyridazin-3-yl)hexahydropyrrolo[3,4-c]pyrrole-2(1H)-carboxylate (200 mg, 0.66 mmol) and DIEA (423 mg, 3.28 mmol) in DMF (5 mL) was added HATU (374 mg, 0.99 mmol) at rt, the mixture was stirred for 2 h. After diluting with water, the solid was collected by filtration, washed with H₂O and dried to give title product (300 mg, Y: 90%) as a yellow solid. ESI-MS (M+H)⁺: 508.5.

### Step 3: Preparation of (7-ethoxy-N-(6-(hexahydropyrrolo[3,4-c]pyrrol-2(1H)-yl)pyridazin-3-yl)-2-methylimidazo[1,2-a]pyridine-6-carboxamide formate.

To a solution of the tert-butyl 5-(6-(7-ethoxy-2-methylimidazo[1,2-a]pyridine-6-carboxamido)pyridazin-3-yl)hexahydropyrrolo[3,4-c]pyrrole-2(1H)-carboxylate (300 mg, 0.59 mmol) in EtOAc (3 mL) was added HCl-EA (3M, 3 mL), the mixture was stirred at rt for 1 h. After concentration, the crude was purified by prep-HPLC (0.1 % FA in water / CH₃CN) to give title product (71 mg, yield: 29.4%) as a yellow solid. ESI-MS (M+H)⁺: 408.2. ¹H NMR (400 MHz, MeOD-*d*₄) δ 9.02 (s, 1H), 8.40 (s, 1H), 8.35 (d, *J* = 9.7 Hz, 1H), 7.56 (s, 1H), 7.09 (d, *J* = 9.8 Hz, 1H), 6.93 (s, 1H), 4.35 (q, *J=* 6.9 Hz, 2H), 3.72 - 3.58 (m, 6H), 3.30 - 3.25 (m, 4H), 2.37 (s, 3H), 1.63 (t, *J* = 6.9 Hz, 3H).

### Example 154. 6-ethoxy-2-methyl-N-(6-(piperazin-1-yl)pyridazin-3-yl)-2H-indazole-5-carboxamide hydrochloride (Compound 749).

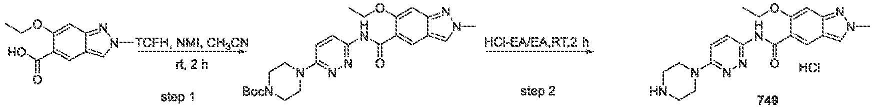

### Step 1: Preparation of tert-butyl 4-(6-(6-ethoxy-2-methyl-2H-indazole-5-carboxamido)pyridazin-3-yl)piperazine-1-carboxylate.

To a mixture of 6-ethoxy-2-methyl-2H-indazole-5-carboxylic acid (200 mg, 0.90 mmol) in ACN (10 mL) were added tert-butyl 4-(6-aminopyridazin-3-yl)piperazine-1-carboxylate (228 mg, 0.81 mmol), NMI (298 mg, 3.63 mmol) and TCFH (510 mg, 1.81 mmol). The mixture was stirred at rt for 2 h. The precipitate was filtered and concentrated in vacuo to give title product (280 mg, Y: 75.2 %) as a white solid. ESI-MS (M+H) ⁺: 482.4. ¹H NMR (400 MHz, CDCl₃) δ 10.96 (s, 1H), 8.72 (s, 1H), 8.53 (d, *J* = 9.8 Hz, 1H), 8.00 (s, 1H), 7.09 (s, 1H), 7.04 (d, *J* = 9.8 Hz, 1H), 4.32 (t, *J*= 7.0 Hz, 2H), 4.20 (s, 3H), 3.59 (br s, 8H), 1.70 (t, *J* = 7.0 Hz, 3H), 1.49 (s, 9H).

### Step 2: Preparation of 6-ethoxy-2-methyl-N-(6-(piperazin-1-yl)pyridazin-3-yl)-2H-indazole-5-carboxamide hydrochloride.

To a solution of tert-butyl 4-(6-(6-ethoxy-2-methyl-2H-indazole-5-carboxamido)pyridazin-3-yl)piperazine-1-carboxylate (250 mg, 0.51 mmol) in EA (5 mL) was added 4M HCl/EA (10 mL). The mixture was stirred at rt for 2 h. The precipitate was filtered, washed with EA (10 mL) and lyophilized to give title product (261 mg, yield: 98.3 %) as a yellow solid. ESI-MS (M+H) ⁺: 382.2. ¹H NMR (400 MHz, DMSO-*d*₆) δ 11.08 (s, 1H), 9.59 (s, 2H), 8.48 (s, 1H), 8.41 (d, *J* = 9.9 Hz, 1H), 8.38 (s, 1H), 7.79 - 7.67 (m, 1H), 7.14 (s, 1H), 4.26 (q, *J* = 6.8 Hz, 2H), 4.15 (s, 3H), 3.93 - 3.87 (m, 4H), 3.24 - 3.22 (m, 4H), 1.49 (t, *J* = 6.9 Hz, 3H).

### Example 155. (R)-6-ethoxy-2-methyl -N-(6-(3-methylpiperazin-1-yl) pyridazin-3-yl)-2H-indazole-5-carboxamide hydrochloride (Compound 750).

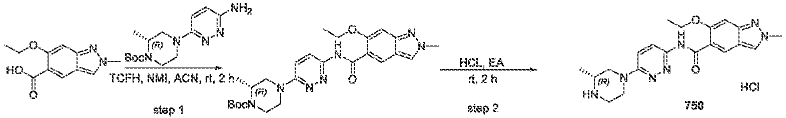

### Step 1: Preparation of tert-butyl (P)-4-(6-(6-ethoxy-2-methyl-2H-indazole-5-carboxamido) pyridazin-3-yl)-2-methylpiperazine-1-carboxylate.

To a solution of 6-ethoxy-2-methyl-2H-indazole-5-carboxylic acid (200 mg, 0.90 mmol), tert-butyl (R)-4-(6-aminopyridazin-3-yl)-2-methylpiperazine-1-carboxylate (266 mg, 0.90 mmol) and NMI (149 mg, 1.81 mmol) in ACN (5 mL) was added TCFH (380 mg 1.36 mmol). The mixture was stirred at rt for 2 h. The precipitate was filtered and dried under vacuum to give title product (117mg, 33 %) as an off-white solid. ESI-MS (M+H) ⁺: 496.3. *Step 2: Preparation of (R)-6-ethoxy-2-methyl -N-(6-(3-methylpiperazin-1-yl) pyridazin-3-yl)-2H-indazole-5-carboxamide hydrochloride.*

To a solution of tert-butyl (R)-4-(6-(6-ethoxy-2-methyl-2H-indazole-5-carboxamido) pyridazin-3-yl)-2-methylpiperazine-1-carboxylate (100 mg, 0.20 mmol) in EA (2 mL) was added 4M HCl in EA (2 mL). The mixture was stirred at rt. for 2 h. The precipitate was filtered, washed with EA (10 mL) and lyophilized to give title product (31.33 mg, 40 %) as an off-white solid. ESI-MS (M+H) ⁺: 396.6. ¹H NMR (400 MHz, DMSO-*d*₆) δ 11.09 (s, 1H), 9.85 - 9.77 (m, 2H), 8.51 - 8.34 (m, 3H), 7.83 (d, *J* = 10.1 Hz, 1H), 7.14 (s, 1H), 4.38 (t, *J* = 13.6 Hz, 2H), 4.26 (q, *J* = 6.9 Hz, 2H), 4.15 (s, 3H), 3.47 - 3.46 (m, 1H), 3.38 - 3.37 (m, 2H), 3.26 - 3.25 (m, 1H), 3.12 - 3.11 (m, 1H), 1.48 (t, *J=* 6.9 Hz, 3H), 1.34 (d, *J* = 6.4 Hz, 3H).

### Example 156. N-(6-((3S,5R)-3,5-dimethylpiperazin-1-yl)pyridazin-3-yl)-6-ethoxy-2-methyl-2H-indazole-5-carboxamide hydrochloride (Compound 751).

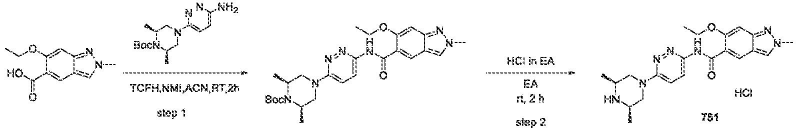

### Step 1: Preparation of tert-butyl (2S,6R)-4-(6-(6-ethoxy-2-methyl-2H-indazole-5-carboxamido)pyridazin-3-yl)-2,6-dimethylpiperazine-1-carboxylate.

To a mixture of 6-ethoxy-2-methyl-2H-indazole-5-carboxylic acid (350 mg, 1.59 mmol), tert-butyl (2S,6R)-4-(6-aminopyridazin-3-yl)-2,6-dimethylpipemzine-1-carboxylate (420 mg, 1.36 mmol) and NMI (336 mg, 4.00 mmol) in ACN (15 mL) was added TCFH (573 mg, 2.05 mmol) at rt. The mixture was stirred at rt for 2 h. The precipitate was filtered, washed with MeCN and dried under vacuum to provide title product (450 mg, Y: 55.6%) as an off-white solid. ESI-MS (M+H)⁺: 510.4. ¹H NMR (400 MHz, DMSO-*d*₆) δ 10.86 (s, 1H), 8.46 (d, *J* = 3.5 Hz, 2H), 8.27 (d, *J* = 9.8 Hz, 1H), 7.47 (d, *J* = 9.9 Hz, 1H), 7.15 (s, 1H), 4.26 - 4.24 (m, 6H), 4.14 (s, 3H), 3.05 (dd, *J =* 13.0, 4.3 Hz, 2H), 1.53 (t, *J* = 6.9 Hz, 3H), 1.44 (s, 9H), 1.19 (d, *J* = 6.8 Hz, 6H).

### Step 2: Preparation of N-(6-((3S,5R)-3,5-dimethylpiperazin-1-yl)pyridazin-3-yl)-6-ethoxy-2-methyl-2H-indazole-5-carboxamide hydrochloride.

To a mixture of tert-butyl (2S,6R)-4-(6-(6-ethoxy-2-methyl-2H-indazole-5-carboxamido)pyridazin-3-yl)-2,6-dimethylpiperazine-1-carboxylate (450 mg, 0.88 mmol) in EA (10 mL) was added 3 M HCl/EA (10 mL). The mixture was stirred at rt for 2 h. The precipitate was filtered, washed with EA (20 mL) and lyophilized to give title product (321 mg, Y: 88.9 %) as a white solid. ESI-MS (M+H) ⁺: 410.3. ¹H NMR (400 MHz, MeOD-*d*₄) δ 8.48 (d, *J=* 10.0 Hz, 2H), 8.20 - 8.19 (m, 2H), 7.16 (s, 1H), 4.56 (d, *J* = 12.6 Hz, 2H), 4.34 (q, *J* = 6.9 Hz, 2H), 4.23 (s, 3H), 3.56 - 3.55 (m, 2H), 3.19 (dd, *J* = 14.3, 11.6 Hz, 2H), 1.57 (t, *J* = 6.9 Hz, 3H), 1.46 (d, *J* = 6.5 Hz, 6H).

### Example 157. 6-ethoxy-2-methyl-N-(6-(6-methyl-1,2,3,6-tetrahydropyridin-4-yl)pyridazin-3-yl)-2H-indazole-5-carboxamide (Compound 752).

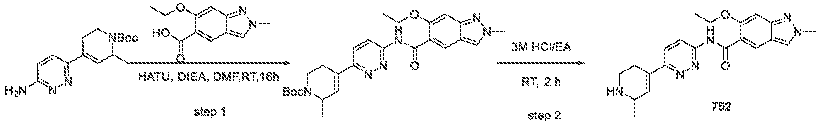

### Step 1: Preparation of tert-butyl 4-(6-(6-ethoxy-2-methyl-2H-indazole-5-carboxamido)pyridazin-3-yl)-6-methyl-3,6-dihydropyridine-1(2H)-carboxylate.

To a solution of 6-ethoxy-2-methyl-2H-indazole-5-carboxylic acid (185 mg, 0.84 mmol) in DMF (10 mL) were added DIEA (400 mg, 2.12 mmol), HATU (400 mg, 1.05 mmol) and 4-(6-aminopyridazin-3-yl)-6-methyl-3,6-dihydropyridine-1(2H)-carboxylate (200 mg, 0.70 mmol). The reaction mixture was stirred at rt for 16 h. The mixture was diluted with water (30 mL). The precipitate was filtered, washed with water and dried in vacuo to give title product (45 mg, 10.87 %) as a yellow solid. ESI-MS (M+H)⁺: 493.3.

### Step 2: Preparation of 6-ethoxy-2-methyl-N-(6-(6-methyl-1,2,3,6-tetrahydropyridin-4-yl)pyridazin-3-yl)-2H-indazole-5-carboxamide.

A mixture of 6-ethoxy-2-methyl-N-(6-(6-methyl-1,2,3,6-tetrahydropyridin-4-yl)pyridazin-3-yl)-2H-indazole-5-carboxamide (45 mg, 0.091 mmol) in 3 M HCl/EA (3 mL) was stirred at rt for 2 h. The mixture was concentrated in vacuo. The residue was purified by prep-HPLC (0.05 % FA in water / CH₃CN) to give title product (10 mg, Y: 28.03 %) as a yellow solid. ESI-MS (M+H) ⁺: 393.7. ¹H NMR (400 MHz, MeOD-*d*₄) δ 8.69 - 8.54 (m, 2H), 8.35 (s, 1H), 7.96 (dd, *J* = 9.4, 5.5 Hz, 1H), 7.14 (s, 1H), 6.86 - 6.39 (m, 1H), 4.38 (q, *J* = 7.0 Hz, 2H), 4.21 (s, 3H), 3.69 (d, *J* = 18.9 Hz, 1H), 3.19 - 2.24 (m, 4H), 1.68 (t, *J =* 6.9 Hz, 3H), 1.34 (t, *J* = 6.7 Hz, 3H).

### Example 158. 6-ethoxy-2-methyl-N-(6-(piperazin-1-yl)pyridazin-3-yl)pyrazolo[1,5-a]pyridine-5-carboxamide hydrochloride (Compound 753).

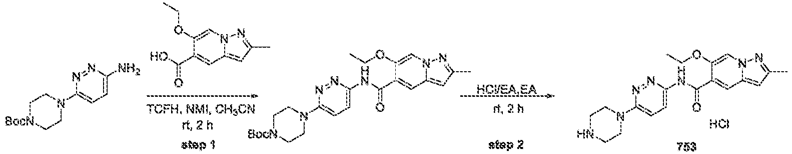

### Step 1: Preparation of tert-butyl 4-(6-(6-ethoxy-2-methylpyrazolo[1,5-a]pyridine-5-carboxamido)pyridazin-3-yl)piperazine-1-carboxylate.

To a mixture of 6-ethoxy-2-methylpyrazolo[1,5-a]pyridine-5-carboxylic acid (4.2 g, 19.09 mmol) in ACN (80 mL) were added tert-butyl 4-(6-aminopyridazin-3-yl)piperazine-1-carboxylate (5.8 g, 20.90 mmol), NMI (4.7 g, 57.27 mmol), TCFH (8.1 g, 28.63 mmol). The mixture was stirred at rt for 2 h. LCMS showed the reaction was completed. The precipitate was filtered, washed with MeCN and concentrated in vacuo to give the title product (8.0 g, 87%) as a yellow solid. ESI-MS (M+H⁺):482.1. ¹H NMR (400 MHz, CDCl₃) δ 10.73 (s, 1H), 8.49 (d, *J* = 9.8 Hz, 1H), 8.42 (s, 1H), 8.13 (s, 1H), 7.04 (d, *J* = 9.8 Hz, 1H), 6.46 (s, 1H), 4.25 (q, *J* = 6.9 Hz, 2H), 3.64 - 3.57 (m, 8H), 2.49 (s, 3H), 1.69 (t, *J* = 7.0 Hz, 3H), 1.49 (s, 9H).

### Step 2: Preparation of 6-ethoxy-2-methyl-N-(6-(piperazin-1-yl)pyridazin-3-yl)pyrazolo[1,5-a]pyridine-5-carboxamide hydrochloride.

To a mixture of tert-butyl 4-(6-(6-ethoxy-2-methylpyrazolo[1,5-a]pyridine-5-carboxamido)pyridazin-3-yl)piperazine-1-carboxylate (4 g, 8.31 mmol) in EA (50 mL) was added 3M HCl/EA (50 mL). The mixture was stirred at rt for 2 h. LCMS showed the reaction was completed. The precipitate was filtered and dried in vacuo to give the title product (3.5 g, 86.4%) as a yellow solid. ESI-MS (M+H⁺):382.4. ¹ H NMR (400 MHz, DMSO-*d*₆) δ 10.97 (s, 1H), 9.32 (s, 2H), 8.51 (s, 1H), 8.31 (d, *J* = 9.7 Hz, 1H), 8.10 (s, 1H), 7.59 (s, 1H), 6.56 (s, 1H), 4.24 - 4.20 (m, 2H), 3.86 - 3.79 (m, 4H), 3.27 - 3.17 (m, 4H), 2.39 (s, 3H), 1.45 (t, *J =* 6.9 Hz, 3H).

### Example 159. 6-ethoxy-2-methyl-N-(6-(1,2,3,6-tetrahydropyridin-4-yl)pyridazin-3-yl)pyrazolo[1,5-a]pyridine-5-carboxamide hydrochloride (Compound 754).

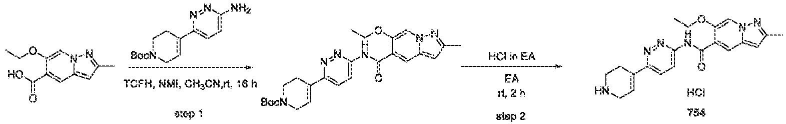

### Step 1: Preparation of tert-butyl 4-(6-(6-ethoxy-2-methylpyrazolo[1,5-a]pyridine-5-carboxamido)pyndazin-3-yl)-3, 6-dihydropyndine-1 (2H)-carboxylate.

To a solution of 6-ethoxy-2-methylpyrazolo[1,5-a]pyridine-5-carboxylic acid (150 mg, 0.68 mmol) in CH₃CN (20 mL) were added tert-butyl 4-(6-aminopyridazin-3-yl)-3,6-dihydropyridine-1(2H)-carboxylate (188.16 mg, 0.68 mmol), TCFH (285.60 mg, 1.03 mmol) and NMI (279.54 mg, 3.42 mmol). The mixture was stirred at room temperature for 16 hours. The precipitate was filtered, washed with CH₃CN and dried to give title product (200 mg, yield: 61%) as a yellow solid. ESI-MS: [M+H] ⁺:479.1.

### Step 2: Preparation of 6-ethoxy-2-methyl-N-(6-(1,2,3,6-tetrahydropyridin-4-yl)pyridazin-3-yl)pyrazolo[1,5-a]pypidine-5-carboxamide hydrochloride.

To a solution of tert-butyl 4-(6-(6-ethoxy-2-methylpyrazolo[1,5-a]pyridine-5-carboxamido)pyridazin-3-yl)-3,6-dihydropyridine-1(2H)-carboxylate (200 mg, 0.42 mmol) in EA (5 mL) was added 3M HCl/EA (5 mL). The mixture was stirred at room temperature for 2 h. The precipitate was filtered, washed with EA (50 mL) and lyophilized to give title product (151.6 mg, yield: 95%) as a yellow solid. ESI-MS: [M+H] ⁺:379.2. ¹H NMR (400 MHz, DMSO-*d*₆) δ 11.27 (s, 1H), 9.25 - 9.07 (m, 2H), 8.55 - 8.47 (m, 2H), 8.15 - 8.07 (m, 2H), 6.74 (s, 1H), 6.57 (s, 1H), 4.27 - 4.21 (m, 2H), 3.88 - 3.83 (m, 2H), 3.38 - 3.34 (m, 2H), 2.93 - 2.87 (m, 2H), 2.40 (s, 3H), 1.46 (t, *J* = 6.9 Hz, 3H).

### Example 160. N-(6-(3,3-dimethylpiperazin-1-yl) pyridazin-3-yl)-6-ethoxy-2-methylpyrazolo[1,5-a] pyridine-5-carboxamide hydrochloride (Compound 755).

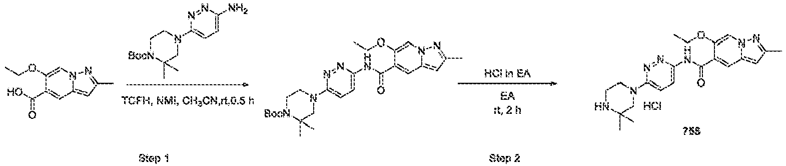

### Step 1: Preparation of tert-butyl 4-(6-(6-ethoxy-2-methylpyrazolo[1,5-a] pyridine-5-carboxamido)pyridazin-3-yl)-2,2-dimethylpiperazine-1-carboxylate.

To a mixture of 6-ethoxy-2-methylpyrazolo[1,5-a] pyridine-5-carboxylic acid (150 mg, 0.68 mmol), tert-butyl 4-(6-aminopyridazin-3-yl)-2,2-dimethylpiperazine-1-carboxylate (210 mg, 0.68 mmol) and NMI (117 mg, 1.42 mmol) in ACN (3 mL) was added TCFH (381 mg, 1.36 mol). The mixture was stirred at rt. for 0.5 h. The precipitate was filtered, washed with CH₃CN and dried to give title product (300 mg, 86.4% yield) as a white solid. ESI-MS (M+H)⁺: 510.3. ¹H NMR (400 MHz, DMSO-*d*₆) δ 10.77 (s, 1H), 8.48 (s, 1H), 8.30 - 8.02 (m, 2H), 7.22 (d, *J* = 9.8 Hz, 1H), 6.54 (s, 1H), 4.22 (dd, *J* = 13.6, 6.7 Hz, 2H), 3.80 - 3.76 (m, 4H), 3.65 (s, 2H), 3.51 (d, *J* = 5.9 Hz, 3H), 2.37 (s, 3H), 1.45 (t, *J* = 7.0 Hz, 3H), 1.42 (s, 9H), 1.34 (s, 6H).

### Step 2: Preparation of N-(6-(3,3-dimethylpiperazin-1-yl) pyridazin-3-yl)-6-ethoxy-2-methylpyrazolo[1,5-a] pyridine-5-carboxamide hydrochloride.

To a mixture of tert-butyl 4-(6-(6-ethoxy-2-methylpyrazolo[1,5-a]pyridine-5-carboxamido)pyridazin-3-yl)-2,2-dimethylpiperazine-1-carboxylate (280 mg, 0.55 mmol) in EA (2 mL) was added HCl (2 mL, 4M in EA). The mixture was stirred at rt for 2 h. The precipitate was filtered, washed with EA (20 mL) and lyophilized to give title product (251.78 mg, 95.91% yield) as a white solid. ESI-MS (M+H)⁺: 410.3. ¹H NMR (400 MHz, DMSO-*d*₆) δ 11.10 (s, 1H), 9.90 (s, 2H), 8.50 (s, 1H), 8.36 (d, *J* = 9.8 Hz, 1H), 8.06 (s, 1H), 7.83 (d, *J* = 9.9 Hz, 1H), 6.55 (s, 1H), 4.20 (d, *J* = 6.9 Hz, 2H), 3.94 (br s, 2H), 3.81 (br s, 2H), 3.26 (br s, 2H), 2.39 (s, 3H), 1.46 - 1.38 (m, 9H).

### Example 161. N-(6-(6,6-dimethyl-1,2,3,6-tetrahydropyridin-4-yl)pyridazin-3-yl)-6-ethoxy-2-methyl-2H-indazole-5-carboxamide formate (Compound 756).

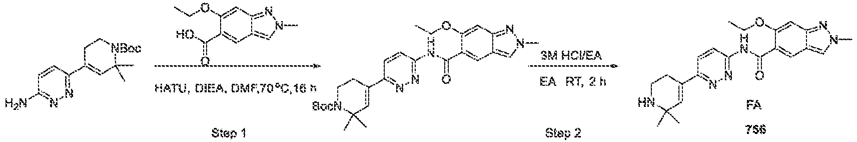

### Step 1: Preparation of tert-butyl (S)-4-(6-(6-ethoxy-2-methyl-2H-indazole-5-carboxamido)pyridazin-3-yl)-2-methyl-3, 6-dihydropyridine-J (2H)-carboxylate.

To a solution of tert-butyl (S)-4-(6-aminopyridazin-3-yl)-2-methyl-3,6-dihydropyridine-1(2H)-carboxylate (400 mg, 1.38 mmol) in DMF (5 mL) were added 6-ethoxy-2-methyl-2H-indazole-5-carboxylic acid (365 mg, 1.66 mmol), DIEA (712 mg, 5.52 mmol) and HATU (630 mg, 1.66 mmol). The mixture was stirred at 50 °C for 16 h. The mixture was diluted with H₂O (10 mL) and the precipitate was filtered, washed with water (20 mL) and dried in vacuo to give title product (300 mg, 44.12 %) as a yellow solid. ESI-MS (M+H)⁺: 507.4. ¹H NMR (400 MHz, CDCl₃) δ 11.24 (s, 1H), 8.74 (s, 1H), 8.67 (d, *J* = 9.3 Hz, 1H), 8.01 (s, 1H), 7.72 (d, *J* = 9.7 Hz, 1H), 7.11 (s, 1H), 6.65 - 6.59 (m, 1H), 4.39 - 4.32 (m, 2H), 4.21 (s, 3H), 4.18 - 4.14 (m, 2H), 2.89 (s, 2H), 1.52 (s, 9 H).

### Step 2: Preparation of N-(6-(6, 6-dimethyl-1, 2, 3,6-tetrahydropyridin-4-yl)pypidazin-3-yl)-6-ethoxy-2-methyl-2H-indazole-5-carboxamide formate.

To a solution of tert-butyl 4-(6-(6-ethoxy-2-methyl-2H-indazole-5-carboxamido)pyridazin-3-yl)-6,6-dimethyl-3,6-dihydropyridine-1(2H)-carboxylate (70 mg, 0.14 mmol) in EA (2 mL) was added 3 M HCl/EA (1 mL). The mixture was stirred at rt for 2 h. The mixture was concentrated in vacuo. The crude was purified by prep-HPLC (0.05 % FA in water / CH₃CN) to give title product (6.78 mg, Y: 10.85 %) as a yellow solid. ESI-MS (M+H⁺): 407.3. ¹H NMR (400 MHz, MeOD-*d*₄) δ 8.69 (d, *J* = 9.4 Hz, 1H), 8.63 (s, 1H), 8.55 (s, 1H), 8.37 (s, 1H), 8.03 (d, *J* = 9.5 Hz, 1H), 7.17 (s, 1H), 6.71 (s, 1H), 4.40 (q, *J* = 7.0 Hz, 2H), 4.22 (s, 3H), 4.03 - 3.85 (m, 2H), 2.98 - 2.86 (m, 2H), 1.68 (t, *J* = 6.9 Hz, 3H), 1.60 - 1.47 (m, 6H).

### Example 162. 6-ethoxy-2-methyl-N-(6-(piperidin-4-yl)pyridazin-3-yl)pyrazolo[1,5-a]pyridine-5-carboxamide hydrochloride (Compound 757).

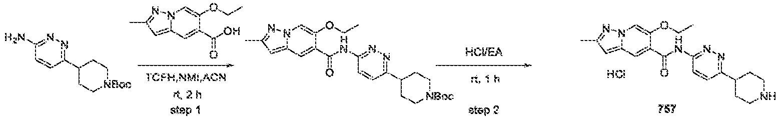

### Step 1: Preparation of tert-butyl 4-(6-(6-ethoxy-2-methylpyrazolo[1,5-a]pyridine-5-carboxamido)pyridazin-3-yl)piperidine-1-carboxylate.

To a mixture of tert-butyl 4-(6-aminopyridazin-3-yl)piperidine-1-carboxylate (150 mg, 0.54 mmol), 6-ethoxy-2-methylpyrazolo[1,5-a]pyridine-5-carboxylic acid (130.58 mg, 0.54 mmol) in ACN (10 mL) were added NMI (132.84 mg, 1.62 mmol) and TCFH (227.61 mg, 0.81 mmol). The mixture was stirred at rt for 2 h. The precipitate was filtered and concentrated in vacuo to give title product (80 mg, 30.86%) as a white solid. ESI-MS (M+H⁺): 481.3. ¹H NMR (400 MHz, DMSO-*d*₆) δ 11.14 (s, 1H), 8.52 (s, 1H), 8.40 *(d, J=* 9.2 Hz, 1H), 8.11 (s, 1H), 7.73 (d, *J* = 9.2 Hz, 1H), 6.56 (s, 1H), 4.23 (q, *J=* 6.9 Hz, 2H), 4.09 (d, *J* = 12.3 Hz, 2H), 3.09 - 3.08 (m, 1H), 2.89 - 2.88 (m, 2H), 2.39 (s, 3H), 1.89 - 1.87 (m, 2H), 1.68 - 1.57 (m, 2H), 1.47 - 1.39 (m, 12H).

### Step 2: Preparation of 6-ethoxy-2-methyl-N-(6-(piperidin-4-yl)pyridazin-3-yl)pyrazolo[1,5-a]pyridine-5-carboxamide hydrochloride.

A mixture of tert-butyl 4-(6-(6-ethoxy-2-methylpyrazolo[1,5-a]pyridine-5-carboxamido)pyridazin-3-yl)piperidine-1-carboxylate (80 mg, 0.17 mmol) in EA/HCl (5 mL) was stirred at rt for 1 h. The precipitate was filtered and dried under vacuum to give title product (31.83 mg, 49%) as a yellow solid. ESI-MS (M+H⁺): 381.2. ¹H NMR (400 MHz, DMSO-*d*₆) δ 11.20 (s, 1H), 9.14 (s, 1H), 8.91 (s, 1H), 8.52 (s, 1H), 8.45 (d, *J* = 9.2 Hz, 1H), 8.11 (s, 1H), 7.72 (d, *J =* 9.2 Hz, 1H), 6.56 (s, 1H), 4.22 (t, *J* = 6.9 Hz, 2H), 3.39 (d, *J =* 12.6 Hz, 2H), 3.25 - 3.19 (m, 1H), 3.04 (d, *J* = 11.1 Hz, 2H), 2.40 (s, 3H), 2.12 - 1.99 (m, 4H), 1.45 (t, *J* = 6.9 Hz, 3H).

### Example 163. 7-ethoxy-N-(6-(3-ethyl-5-methylpiperazin-1-yl)pyridazin-3-yl)-2-methylimidazo[1,2-a]pyridine-6-carboxamide (Compound 758).

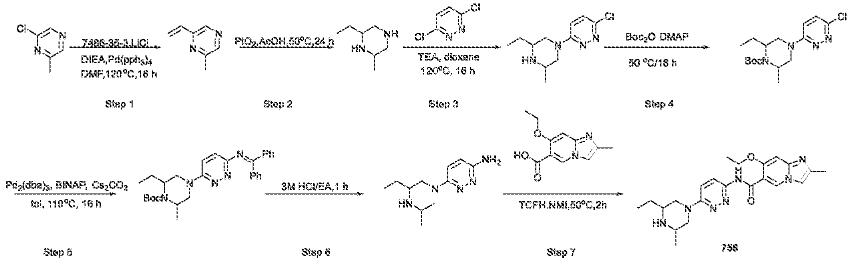

### Step 1: Preparation of 2-methyl-6-vinylpyrazine.

To a mixture of 2-chloro-6-methylpyrazine (4.85 g, 42.92 mmol) and Tributyl(vinyl)tin (13.2 g, 42.92 mmol) in DMF (100 mL) were added LiCl (1.75 g, 42.92 mmol), DIEA (5.37 g, 42.92 mmol) and Pd(PPh₃)₄ (2.19 g, 1.90 mmol), the mixture was charged with N₂ for three times and stirred at 120 °C for 16 h. The reaction mixture was diluted with water (200 mL), extracted with DCM (250 mLx3). The combined organics were washed with brine (200 mLx3), dried over Na₂SO₄ and evaporated in vacuo. The crude was purified by silica gel column chromatography (PE: EA=4:1) to give tittle product (900 mg, Y: 87.7 %) as a yellow solid. ESI-MS (M+H)⁺: 121.2. ¹H NMR (400 MHz, DMSO-*d*₆) δ 8.55 (s, 1H), 8.42 (s, 1H), 6.85 (dd, *J* = 17.5, 10.9 Hz, 1H), 6.35 (dd, *J* = 17.5, 1.4 Hz, 1H), 5.59 (d, *J* = 10.9 Hz, 1H), 2.53 - 2.49 (m, 3H).

### Step 2: Preparation of 2-ethyl-6-methylpiperazine.

To a mixture of 2-methyl-6-vinylpyrazine (4 g, 33.30 mmol) in AcOH (50 mL) was added PtO₂ (1 g, 4.40 mmol), the mixture was charged with H₂ for three times and stirred at 50 °C for 24 h. The reaction mixture was filtered and the filtrate was evaporated in vacuo. The crude was purified by silica gel column chromatography eluted with (DCM/ MeOH=10:1) to give title compound (2 g, 47%) as a yellow solid. ESI-MS (M+H)⁺: 129.2. ¹H NMR (400 MHz, DMSO-*d*₆) δ 2.88 - 2.65 (m, 3H), 2.19 - 2.04 (m, 2H), 1.84 - 1.82 (m, 3H), 1.33 - 1.17 (m, 2H), 0.96 - 0.89 (m, 3H), 0.88 - 0.82 (m, 3H).

### Step 3: Preparation of 3-chloro-6-(3-ethyl-5-methylpiperazin-1-yl)pyridazine.

To a mixture of 2-ethyl-6-methylpiperazine (2 g, 15.50 mmol) in 1,4-dioxane (50 mL) were added 3,6-dichloropyridazine (3.47 g, 23.26 mmol) and TEA (4.73 g, 46.50 mmol), the mixture was stirred at 120°C for 16 h. The reaction mixture was evaporated in vacuo. The crude was purified by silica gel column chromatography eluted with (DCM/MeOH=10:1) to give title compound (1.5 g, 40%) as a yellow solid. ESI-MS (M+H)⁺: 241.0.

### Step 4: Preparation of tert-butyl 4-(6-chloropyridazin-3-yl)-2-ethyl-6-methylpiperazine-1-carboxylate.

To a mixture of 3-chloro-6-(3-ethyl-5-methylpiperazin-1-yl)pyridazine (1 g, 4.17 mmol) in DCM (10 mL) were added Di-tert-butyl dicarbonate (7 g, 41.70 mmol) and DMAP (1.52 g, 12.5 mmol). The mixture was stirred at 50 °C for 16 h. The reaction was diluted with water (15 mL), extracted with DCM (20 mLx3). The combined organic was washed with brine (20 mL), dried over Na₂SO₄ and evaporated in vacuo. The crude was purified by silica gel column chromatography (PE: EA=4:1) to give tittle product (250 mg, Y: 17.5 %) as a yellow solid. ESI-MS (M+H⁺): 341.3. ¹H NMR (400 MHz, CDCl₃) δ 7.21 (d, *J* = 9.5 Hz, 1H), 6.87 (d, *J* = 9.5 Hz, 1H), 4.37 - 4.27 (m, 2H), 4.13 - 4.03 (m, 2H), 3.25 (dd, *J* = 13.2, 4.6 Hz, 1H), 3.11 (dd, *J* = 13.3, 4.5 Hz, 1H), 1.64 - 1.62 (m, 2 H), 1.49 (s, 9H), 1.27 - 1.20 (m, 3H), 0.97 (t, *J* = 7.4 Hz, 3H).

### Step 5: Preparation of tert-butyl 4-(6-((diphenylmethylene)amino)pyridazin-3-yl)-2-ethyl-6-methylpiperazine-1-carboxylate.

To a mixture of tert-butyl 4-(6-chloropyridazin-3-yl)-2-ethyl-6-methylpiperazine-1-carboxylate (140 mg, 0.41 mmol) and Cs₂CO₃ (401 mg, 1.23 mmol) in 1,4 dioxane (5 mL) were added BINAP (51 mg, 0.24 mmol), Pd(dba)₃ (38 mg, 0.04 mmol) and diphenylmethanimine (82 mg, 0.45 mmol). The mixture was stirred at 110 °C for 16 h. The reaction mixture was diluted with water (15 mL), extracted with DCM (20 mLx3). The combined organics were washed with brine (20 mL), dried over Na₂SO₄ and evaporated in vacuo to give crude title product (100 mg, Y: 51.5 %) as a yellow oil. ESI-MS (M+H⁺):486.3.

### Step 6: Preparation of 6-(3-ethyl-5-methylpiperazin-1-yl)pyridazin-3-amine hydrochloride.

To a mixture of tert-butyl 4-(6-((diphenylmethylene)amino)pyridazin-3-yl)-2-ethyl-6-methylpiperazine-1-carboxylate (100 mg, 0.21 mmol) in EtOAc (2 mL) was added 3M HCl/EtOAc (2 mL), the mixture was stirred for 1 h at rt. The precipitate was filtered to afford title compound (30 mg, 58.3 %) as a yellow solid. ESI-MS (M+H) ⁺: 222.2. ¹H NMR (400 MHz, DMSO-*d*₆) δ 7.11 (d, *J* = 9.6 Hz, 1H), 6.72 (d, *J* = 9.5 Hz, 1H), 5.59 (s, 2H), 3.90 - 3.77 (m, 2H), 2.79 - 2.73 (m, 1H), 2.61 - 2.55 (m, 1H), 2.15 (t, *J* = 10.6 Hz, 2H), 1.42 - 1.30 (m, 2H), 1.01 (d, *J =* 6.3 Hz, 3H), 0.92 (t, *J =* 7.5 Hz, 3H).

### Step 7: Preparation of 7-ethoxy-N-(6-(3-ethyl-5-methylpiperazin-1-yl)pyridazin-3-yl)-2-methylimidazo[1,2-a]pyridine-6-carboxamide.

To a solution of 6-(3-ethyl-5-methylpiperazin-1-yl)pyridazin-3-amine hydrochloride (30 mg, 0.11 mmol) and 7-ethoxy-2-methylimidazo[1,2-a]pyridine-6-carboxylic acid (35 mg, 0.16 mmol) in MeCN (3 mL) were added NMI (34 mg, 0.44 mmol) and TCFH (58 mg, 0.22 mmol), the mixture was stirred for 1 h at 50 °C for 2 h. The precipitate was filtered and purified by prep-HPLC (0.05 % NH₃.H₂O in water / CH₃CN) to give title compound (1.93 mg, 4.1% yield) as a yellow solid. ¹H NMR (400 MHz, MeOD-*d*₄) δ 9.03 (d, *J* = 4.1 Hz, 1H), 8.40 - 8.33 (m, 1H), 7.55 (s, 1H), 7.44 - 7.36 (m, 1H), 6.95 (d, *J* = 4.5 Hz, 1H), 4.61 (br s, 2H), 4.40 - 4.33 (m, 2H), 4.19 (d, *J* = 12.8 Hz, 1H), 3.02 - 2.91 (m, 1H), 2.81 - 2.70 (m, 1H), 2.56 - 2.50 (m, 1H), 2.38 (s, 3H), 1.66 (t, *J* = 6.9 Hz, 3H), 1.61 - 1.50 (m, 2H), 1.21 (d, *J* = 6.4 Hz, 3H), 1.07 (t, *J* = 7.5 Hz, 3H). ESI-MS (M+H) ⁺:424.3

### Example 164. 7-ethoxy-2-methyl-N-(6-(1-methyl-1,2,3,6-tetrahydropyridin-4-yl)pyridazin-3-yl)imidazo[1,2-a]pyridine-6-carboxamide (Compound 759).

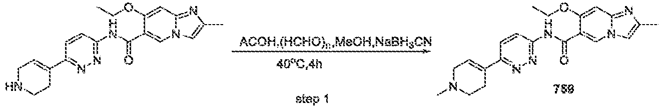

### Step 1: Preparation of 7-ethoxy-2-methyl-N-(6-(1-methyl-1, 2, 3, 6-tetrahydropyridin-4-yl)pyridazin-3-yl)imidazo[1,2-alpyridine-6-carboxamide.

To a solution of 7-ethoxy-2-methyl-N-(6-(1,2,3,6-tetrahydropyridin-4-yl)pyridazin-3-yl)imidazo[1,2-a]pyridine-6-carboxamide (40 mg, 0.10 mmol), AcOH (32 mg, 0.52 mmol) and (HCHO)ₙ(16 mg, 0.52 mmol) in MeOH (4 mL) was added NaBH₃CN (20 mg, 0.31 mmol). The mixture was stirred at 40 °C for 4 h. The mixture was filtered and the filtrate was concentrated in vacuo, the residue was purified by prep-HPLC (0.05 % NH₃.H₂O in water / CH₃CN) to give title product (20.41 mg, 100 %) as a yellow solid. ESI-MS (M+H)⁺: 393.2. ¹H NMR (400 MHz, MeOD-*d*₄) δ 9.16 (s, 1H), 8.65 (d, *J =* 9.4 Hz, 1H), 8.06 (d, *J* = 9.5 Hz, 1H), 7.70 (s, 1H), 7.14 (s, 1H), 6.71 (s, 1H), 4.44 (q, *J* = 6.9 Hz, 2H), 4.06 (br s, 2H), 3.61 (t, *J=* 5.8 Hz, 2H), 3.15 (br s, 2H), 3.06 (s, 3H), 2.45 (s, 3H), 1.67 (t, *J =* 6.9 Hz, 3H)..

### Example 165. (R)-6-ethoxy-2-methyl-N-(6-(6-methyl-1,2,3,6-tetrahydropyridin-4-yl)pyridazin-3-yl)-2H-indazole-5-carboxamide formate (Compound 760).

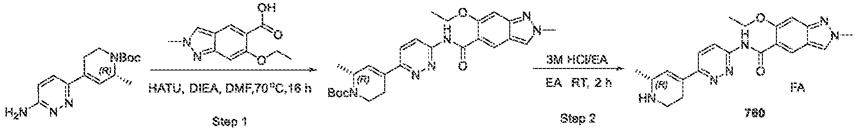

### Step 1: Preparation of tert-butyl (R)-4-(6-(6-ethoxy-2-methyl-2H-indazole-5-carboxamido)pyridazin-3-yl)-6-methyl-3, 6-dihydropyridine-J (2H)-carboxylate.

To a solution of tert-butyl (R)-4-(6-aminopyridazin-3-yl)-6-methyl-3,6-dihydropyridine-1(2H)-carboxylate (400 mg, 1.38 mmol) in DMF (5 mL) were added 6-ethoxy-2-methyl-2H-indazole-5-carboxylic acid (365 mg, 1.66 mmol), DIEA (712 mg, 5.52 mmol) and HATU (630 mg, 1.66 mmol). The mixture was stirred at 70 °C for 16 h. The mixture was diluted with H₂O (10 mL) and the precipitate was filtered, washed with H₂O (20 mL) and dried in vacuo to give title product (250 mg, 36.76 %) as a yellow solid. ESI-MS (M+H)⁺: 493.3. ¹H NMR (400 MHz, CDCl₃) δ 11.24 (s, 1H), 8.74 (s, 1H), 8.66 (d, *J* = 9.1 Hz, 1H), 8.02 (s, 1H), 7.72 - 7.60 (m, 1H), 7.11 (s, 1H), 6.52 (d, *J* = 11.2 Hz, 1H), 4.82 - 4.65 (m, 1H), 4.35 (q, *J* = 13.9, 6.9 Hz, 2H), 4.21 (s, 3H), 3.02 - 2.79 (m, 2H), 1.74 - 1.71 (m, 2H), 1.50 (s, 9H), 1.31 (d, *J=* 6.6 Hz, 3H), 1.17 (d, *J* = 6.7 Hz, 3H).

### Step 2: Preparation of (R)-6-ethoxy-2-methyl-N-(6-(6-methyl-1, 2, 3, 6-tetrahydropyridin-4-yl)pyridazin-3-yl)-2H-indazole-5-carboxamide formate.

To a solution of tert-butyl (R)-4-(6-(6-ethoxy-2-methyl-2H-indazole-5-carboxamido)pyridazin-3-yl)-6-methyl-3,6-dihydropyridine-1(2H)-carboxylate (250 mg, 0.51 mmol) in EA (5 mL) was added 3 M HCl/EA (5 mL). The mixture was stirred at rt for 2 h. The mixture was concentrated in vacuo. The crude was purified by prep-HPLC (0.05 % FA in water / CH₃CN) to give title product (95.07 ing, Y: 42.79 %) as a yellow solid. ESI-MS (M+H⁺): 393.2. ¹H NMR (400 MHz, DMSO-*d*₆) δ 11.23 (s, 1H), 8.52 - 8.44 (m, 3H), 8.27 (s, 1H), 8.02 (d, *J* = 9.5 Hz, 1H), 7.17 (s, 1H), 6.68 (d, *J* = 35.3 Hz, 1H), 4.30 (q, *J* = 6.8 Hz, 2H), 4.15 (s, 3H), 3.12 - 2.98 (m, 2H), 2.94 - 2.85 (m, 1H), 2.75 - 2.61 (m, 2H), 1.54 (t, *J* = 6.9 Hz, 3H), 1.27 (t, *J* = 6.5 Hz, 3H).

### Example 166. N-(6-((3R,5S)-3,5-dimethylpiperazin-1-yl)pyridazin-3-yl)-6-ethoxy-2-methylpyrazolo[1,5-a]pyridine-5-carboxamide hydrochloride (Compound 761).

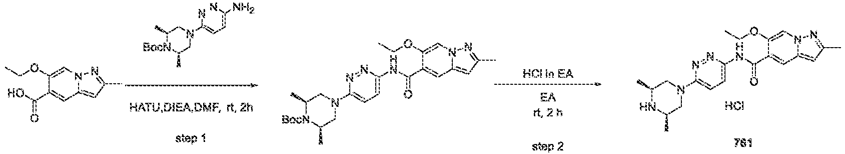

### Step 1: Preparation of tert-butyl (2R,6S)-4-(6-(6-ethoxy-2-methylpyrazolo[1,5-a]pyridine-5-carboxamido)pyridazin-3-yl)-2,6-dimethylpiperazine-1-carboxylate.

To a mixture of 6-ethoxy-2-methylpyrazolo[1,5-a]pyridine-5-carboxylic acid (500 mg, 2.27 mmol), tert-butyl (2S,6R)-4-(6-aminopyridazin-3-yl)-2,6-dimethylpiperazine-1-carboxylate (696.89 mg, 2.27 mmol) in DMF (10 mL) were added HATU (1293.9 mg, 3.40 mmol) and DIEA (1171.32 mg, 9.08 mmol). The mixture was stirred at rt for 2 h. The reaction mixture was diluted with H2O (30 mL), the precipitate was filtered, washed with water and dried in vacuo to give title product (520 mg, yield: 44.93%) as a brown solid. ESI-MS (M+H+): 510.2. 1H NMR (400 MHz, DMSO-d6) δ 10.81 (s, 1H), 8.50 (s, 1H), 8.29 - 8.05 (m, 2H), 7.48 (d, J = 9.9 Hz, 1H), 6.55 (s, 1H), 4.29 - 4.22 (m, 4H), 4.20 - 4.14 (m, 2H), 3.06 (dd, J = 13.0, 4.3 Hz, 2H), 2.39 (s, 3H), 1.50 - 1.42 (m, 12H), 1.18 (d, J = 6.8 Hz, 6H). *Step 2: Preparation of N-(6-((3R,5S)-3,5-dimethylpiperazin-1-yl)pyridazin-3-yl)-6-ethoxy-2-methylpyrazolo[1,5-a]pyridine-5-carboxamide hydrochloride.*

A mixture of tert-butyl (2R,6S)-4-(6-(6-ethoxy-2-methylpyrazolo[1,5-a]pyridine-5-carboxamido)pyridazin-3-yl)-2,6-dimethylpiperazine-1-carboxylate (520 mg, 1.02 mmol) in EA/HCl (5 mL) was stirred at rt for 2 h. The precipitate was filtered, washed with EA and dried in vacuo to give title product (350 mg, 83.69%) as a yellow solid. ESI-MS (M+H⁺): 410.4. ¹H NMR (400 MHz, DMSO-*d*₆) δ 11.03 (s, 1H), 9.99 (s, 1H), 9.64 (s, 1H), 8.50 (s, 1H), 8.34 (d, J = 9.9 Hz, 1H), 8.08 (s, 1H), 7.75 (d, J = 10.0 Hz, 1H), 6.55 (s, 1H), 4.47 (d, J = 13.0 Hz, 2H), 4.21 (q, J = 6.9 Hz, 2H), 3.39 - 3.29 (m, 2H), 3.22 - 3.11 (m, 2H), 2.39 (s, 3H), 1.44 (t, J = 6.9 Hz, 3H), 1.36 (d, J = 6.4 Hz, 6H).

### Biological Activity of the Compounds of the Present Disclosure

The biological activity of the compounds of the present disclosure was determined utilizing the assay described herein.

### Example 76. Mini-gene reporter assay - PMS1

Mini-gene reporter constructs for each target site can be constructed by first PCR amplifying the region-of-interest including the sequence of the alternatively skipped, sequences of the immediate upstream and downstream introns, and sequences of the immediate upstream and downstream exons. Then the 3'end of the amplified sequence can be ligated to a firefly luciferase reporter gene and cloned into the pcDNA3.1 vector backbone. The final reporter construct can be transiently transfected into HEK293 cells using Lipofectamine 3000 transfection reagents. Compounds that can induce the inclusion of the skipped exon in the reporter construct would increase the reporter firefly luciferase activity.

Test compounds may be diluted in duplicates and dispensed into an assay plate. Transient mini-gene reporter cell line stock is resuspended and dispensed into the assay plate. Assay plates are then centrifuged and incubated. A luciferase reagent (e.g. ONE-Glo firefly luciferase reagent from Promega^{®}) may be added to the assay plate and the luminescence (RLU) signals recorded. EC₅₀ values may be determined by curve fitting in Levenberg-Marquardt algorithm.

In Table D below, A indicates a IC₅₀ (µM) < 1 µM, B indicates a IC₅₀ (µM) 1 µM to < 10 µM, and C indicates a IC₅₀ (µM) 10 µM to < 50 µM. In Table D below, A indicates a >75% Effect at 5 µM µM, B indicates a 50 to <75% Effect at 5 µM µM, and C indicates a <50% Effect at 5 µM.

**Table D. - PMS1 Assay Data**

| **Compound No. #** | **PMS1 IC50 (uM)** | **PMS1 % Effect at SuM** |
|---|---|---|
| 101 | | C |
| 102 | | C |
| 103 | | C |
| 104 | | C |
| 105 | | C |
| 106 | | C |
| 107 | | B |
| 108 | B | |
| 109 | | A |
| 110 | A | |
| 111 | A | |
| 112 | | C |
| 113 | B | |
| 114 | A | |
| 115 | A | |
| 116 | | C |
| 117 | | C |
| 118 | A | |
| 119 | B | |
| 120 | B | |
| 121 | | C |
| 122 | A | |
| 123 | B | |
| 124 | B | |
| 125 | A | |
| 126 | A | |
| 127 | B | |
| 128 | A | |
| 129 | B | |
| 130 | A | |
| 131 | | B |
| 132 | A | |
| 133 | A | |
| 134 | | C |
| 135 | | C |
| 136 | | B |
| 137 | | B |
| 138 | | A |
| 139 | | A |
| 140 | | A |
| 141 | | B |
| 142 | | A |
| 143 | | A |
| 144 | | B |
| 145 | | C |
| 146 | | C |
| 147 | A | |
| 148 | | C |
| 149 | | A |
| 150 | | C |
| 151 | | C |
| 152 | | C |
| 153 | | C |
| 154 | A | |
| 155 | A | |
| 156 | A | |
| 157 | A | |
| 158 | A | |
| 159 | | B |
| 160 | A | |
| 161 | | A |
| 162 | | |
| 163 | | A |
| 164 | | A |
| 165 | A | |
| 166 | | C |
| 167 | | B |
| 168 | | A |
| 169 | | B |
| 170 | | A |
| 171 | | A |
| 172 | | B |
| 173 | B | |
| 174 | A | |
| 175 | A | |
| 176 | A | |
| 177 | A | |
| 178 | A | |
| 179 | A | |
| 180 | A | |
| 198 | A | |
| 199 | A | |
| 205 | A | |
| 206 | A | |
| 207 | A | |
| 208 | A | A |
| 209 | A | |
| 210 | A | |
| 211 | C | |
| 212 | B | |
| 213 | A | |
| 214 | A | |
| 215 | A | |
| 216 | A | |
| 217 | A | |
| 218 | B | |
| 219 | A | |
| 220 | A | |
| 221 | A | |
| 222 | B | |
| 224 | A | |
| 225 | A | |
| 226 | B | |
| 227 | B | |
| 228 | A | |
| 229 | A | |
| 230 | B | |
| 231 | B | |
| 232 | B | |
| 233 | B | |
| 234 | B | |
| 235 | B | |
| 236 | B | |
| 237 | B | |
| 238 | B | |
| 239 | B | |
| 240 | B | |
| 241 | B | |
| 242 | B | |
| 243 | B | |
| 244 | A | |
| 245 | B | |
| 246 | B | |
| 247 | B | |
| 248 | B | |
| 249 | B | |
| 250 | B | |
| 251 | B | |
| 252 | B | |
| 253 | B | |
| 254 | C | |
| 255 | A | |
| 256 | B | |
| 257 | B | |
| 258 | B | |
| 259 | B | |
| 260 | B | |
| 261 | B | |
| 262 | B | |
| 263 | B | |
| 264 | B | |
| 265 | B | |
| 266 | A | |
| 267 | B | |
| 268 | B | |
| 269 | B | |
| 270 | B | |
| 271 | B | |
| 272 | B | |
| 273 | B | |
| 274 | B | |
| 275 | B | |
| 276 | A | |
| 277 | B | |
| 278 | B | |
| 279 | A | |
| 280 | A | |
| 281 | B | |
| 282 | A | |
| 283 | B | |
| 284 | B | |
| 285 | B | |
| 286 | B | |
| 287 | B | |
| 288 | B | |
| 289 | B | |
| 290 | A | |
| 291 | B | |
| 292 | B | |
| 293 | B | |
| 294 | A | |
| 295 | A | |
| 296 | B | |
| 297 | B | |
| 298 | B | |
| 299 | B | |
| 300 | B | |
| 301 | B | |
| 302 | A | |
| 303 | | B |
| 304 | | A |
| 305 | | A |
| 306 | | C |
| 307 | | B |
| 308 | | C |
| 309 | | A |
| 310 | | C |
| 311 | | C |
| 312 | | C |
| 313 | | C |
| 314 | | C |
| 315 | | C |
| 316 | | B |
| 317 | | C |
| 318 | | C |
| 319 | | C |
| 320 | | C |
| 321 | | A |
| 322 | | C |
| 323 | | C |
| 324 | | C |
| 325 | | C |
| 326 | | A |
| 327 | | C |
| 328 | | C |
| 329 | | C |
| 330 | | C |
| 331 | | C |
| 332 | | A |
| 333 | | C |
| 334 | | C |
| 335 | | C |
| 336 | | |
| 337 | | C |
| 338 | | A |
| 339 | | A |
| 340 | | B |
| 341 | | C |
| 342 | | C |
| 343 | | A |
| 344 | | C |
| 345 | | |
| 346 | | |
| 347 | | C |
| 348 | | B |
| 349 | | C |
| 350 | | B |
| 351 | | C |
| 352 | | A |
| 353 | | C |
| 354 | | C |
| 355 | | A |
| 356 | | C |
| 357 | | C |
| 358 | | B |
| 359 | | C |
| 360 | | A |
| 361 | | A |
| 362 | | C |
| 363 | | A |
| 364 | | B |
| 365 | | A |
| 366 | | A |
| 367 | | A |
| 368 | | |
| 369 | | |
| 370 | | A |
| 371 | | A |
| 372 | | A |
| 373 | | A |
| 374 | | A |
| 375 | | B |
| 376 | | A |
| 377 | | B |
| 378 | | C |
| 379 | | |
| 380 | | C |
| 381 | | C |
| 382 | | C |
| 383 | | C |
| 384 | | C |
| 385 | | C |
| 386 | | C |
| 387 | | |
| 388 | | C |
| 389 | | |
| 390 | | C |
| 391 | | C |
| 392 | | C |
| 393 | | C |
| 394 | | C |
| 395 | | C |
| 396 | | |
| 397 | | C |
| 398 | | C |
| 399 | | C |
| 400 | | C |
| 401 | | |
| 402 | | |
| 403 | | C |
| 404 | | |
| 405 | | C |
| 406 | | C |
| 407 | | C |
| 408 | | C |
| 409 | | C |
| 410 | | A |
| 411 | | C |
| 412 | | C |
| 413 | | C |
| 414 | | C |
| 415 | | C |
| 416 | | B |
| 417 | | C |
| 418 | | C |
| 419 | | C |
| 420 | | |
| 421 | | C |
| 422 | | C |
| 423 | | C |
| 424 | | C |
| 425 | | C |
| 426 | | C |
| 427 | | C |
| 428 | | C |
| 429 | | C |
| 430 | | |
| 431 | | C |
| 432 | | C |
| 433 | | Cc |
| 434 | | C |
| 435 | | C |
| 436 | | C |
| 437 | | C |
| 438 | | C |
| 439 | | C |
| 440 | | C |
| 441 | | C |
| 442 | | A |
| 443 | | C |
| 444 | | C |
| 445 | | C |
| 446 | | C |
| 447 | | C |
| 448 | | C |
| 449 | | C |
| 450 | | C |
| 451 | | C |
| 452 | | C |
| 453 | | B |
| 454 | | C |
| 455 | | C |
| 456 | | |
| 457 | | |
| 458 | | |
| 459 | | C |
| 460 | | C |
| 461 | | |
| 462 | | C |
| 463 | | A |
| 464 | | B |
| 465 | | C |
| 466 | | C |
| 467 | | C |
| 468 | | C |
| 469 | | C |
| 470 | | C |
| 471 | | |
| 472 | | C |
| 473 | | C |
| 474 | | |
| 475 | | C |
| 476 | | |
| 477 | | B |
| 478 | | B |
| 479 | | C |
| 480 | | A |
| 481 | | C |
| 482 | | |
| 483 | | C |
| 484 | | C |
| 485 | | C |
| 486 | | |
| 487 | | A |
| 488 | | |
| 489 | | C |
| 490 | | B |
| 491 | | |
| 492 | | B |
| 493 | | C |
| 494 | | C |
| 495 | | C |
| 496 | | A |
| 497 | | A |
| 498 | | |
| 499 | | C |
| 500 | | C |
| 501 | | |
| 502 | | A |
| 503 | | A |
| 504 | | |
| 505 | | B |
| 506 | | |
| 507 | | C |
| 508 | | |
| 509 | | C |
| 510 | | C |
| 511 | | |
| 512 | | |
| 513 | | C |
| 514 | | |
| 515 | | |
| 516 | | C |
| 517 | | A |
| 518 | | C |
| 519 | | A |
| 520 | | |
| 521 | | |
| 522 | | C |
| 523 | | C |
| 524 | | C |
| 525 | | B |
| 526 | | A |
| 527 | | C |
| 528 | | C |
| 529 | | B |
| 530 | | A |
| 531 | | C |
| 532 | | C |
| 533 | | C |
| 534 | | B |
| 535 | | A |
| 536 | | C |
| 537 | | C |
| 538 | | B |
| 539 | | C |
| 540 | | A |
| 541 | | C |
| 542 | | C |
| 543 | | |
| 544 | | C |
| 545 | | C |
| 546 | | A |
| 547 | | A |
| 548 | | A |
| 549 | | A |
| 550 | | A |
| 551 | | A |
| 552 | | A |
| 553 | | A |
| 554 | | A |
| 555 | | C |
| 556 | | A |
| 557 | | A |
| 558 | | A |
| 559 | | A |
| 560 | | |
| 561 | | A |
| 562 | | C |
| 563 | | C |
| 564 | | C |
| 565 | | |
| 566 | | C |
| 567 | | C |
| 568 | | C |
| 569 | | C |
| 570 | | C |
| 571 | | C |
| 572 | | C |
| 573 | | C |
| 574 | | C |
| 575 | | C |
| 576 | | C |
| 577 | | C |
| 578 | | C |
| 579 | | A |
| 580 | | |
| 581 | | C |
| 582 | | C |
| 583 | | C |
| 584 | | C |
| 585 | | |
| 586 | | |
| 587 | | C |
| 588 | | C |
| 589 | | |
| 590 | | A |
| 591 | | C |
| 592 | | C |
| 593 | | C |
| 594 | | B |
| 595 | | C |
| 596 | | B |
| 597 | | C |
| 598 | | C |
| 599 | | C |
| 600 | | C |
| 601 | | |
| 602 | | |
| 603 | | C |
| 604 | | C |
| 605 | | C |
| 606 | | C |
| 607 | | C |
| 608 | | C |
| 609 | | C |
| 610 | | C |
| 611 | | C |
| 612 | | C |
| 613 | | C |
| 614 | | |
| 615 | | C |
| 616 | | |
| 617 | | C |
| 618 | | C |
| 619 | | C |
| 620 | | |
| 621 | | |
| 622 | | |
| 623 | | C |
| 624 | | |
| 625 | | B |
| 626 | | |
| 627 | B | |
| 628 | A | |
| 629 | A | |
| 630 | A | |
| 631 | | |
| 632 | | |
| 633 | | |
| 634 | | |
| 635 | | |
| 636 | | |
| 637 | | |
| 638 | | |
| 639 | | A |
| 640 | | |
| 641 | | |
| 642 | | |
| 643 | | A |
| 644 | | B |
| 645 | | |
| 646 | | B |
| 647 | | |
| 648 | | C |
| 649 | | B |
| 650 | | A |
| 651 | | |
| 652 | | B |
| 653 | | C |
| 654 | | A |
| 655 | | |
| 656 | | C |
| 657 | | |
| 658 | | |
| 659 | | A |
| 660 | | |
| 661 | | C |
| 662 | | A |
| 663 | | A |
| 664 | | A |
| 665 | | |
| 666 | | B |
| 667 | | A |
| 668 | | |
| 669 | | C |
| 670 | | |
| 671 | | C |
| 672 | | B |
| 673 | | C |
| 674 | | B |
| 675 | | |
| 676 | | B |
| 677 | | A |
| 678 | | A |
| 679 | | C |
| 680 | | |
| 681 | | |
| 682 | | |
| 683 | | C |
| 684 | | |
| 685 | | B |
| 686 | | A |
| 687 | | A |
| 688 | | A |
| 689 | | A |
| 690 | | B |
| 691 | | A |
| 692 | | A |
| 693 | | A |
| 694 | | A |
| 695 | | A |
| 696 | | B |
| 697 | | A |
| 698 | | A |
| 699 | | |
| 700 | A | |
| 701 | | A |
| 702 | A | |
| 704 | A | |
| 705 | A | |
| 706 | | A |
| 707 | | A |
| 708 | A | |
| 709 | A | |
| 710 | A | |
| 711 | A | |
| 712 | A | |
| 713 | A | |
| 714 | A | |
| 715 | A | |
| 716 | A | |
| 717 | A | |
| 718 | A | |
| 719 | A | |
| 720 | A | |
| 721 | B | |
| 722 | B | |
| 723 | B | |
| 724 | B | |
| 725 | B | |
| 726 | B | |
| 727 | B | |
| 728 | B | |
| 729 | B | |
| 730 | B | |
| 731 | B | |
| 732 | B | |
| 733 | B | |
| 734 | B | |
| 735 | B | |
| 736 | B | |
| 737 | B | |
| 738 | B | |
| 739 | B | |
| 740 | B | |
| 741 | B | |
| 742 | B | |
| 743 | B | |
| 744 | B | |
| 745 | B | |
| 746 | B | |
| 747 | | A |
| 748 | | A |
| 749 | | A |
| 750 | | A |
| 751 | | A |
| 752 | | A |
| 753 | | A |
| 754 | | A |
| 755 | | A |
| 756 | | A |
| 757 | | A |
| 758 | | A |
| 759 | | A |
| 760 | | A |
| 761 | | A |

### EQUIVALENTS

The details of one or more embodiments of the disclosure are set forth in the accompanying description above. Although any methods and materials similar or equivalent to those described herein can be used in the practice or testing of the present disclosure, the preferred methods and materials are now described. Other features, objects, and advantages of the disclosure will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this disclosure belongs.

The foregoing description has been presented only for the purposes of illustration and is not intended to limit the disclosure to the precise form disclosed, but by the claims appended hereto.

## Claims

1. A compound of Formula (I): 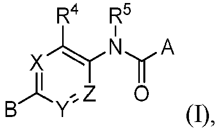 or a pharmaceutically acceptable salt thereof, wherein:
X=N or CR¹; Y=N or CR²; Z=N or CR³;
R¹, R², R³ and R⁴ are each independently selected from the group consisting of H, halogen, hydroxy, cyano, -COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₁-C₆cycloalkyloxy, C₃-C₈cycloalkyl, NH₂, NH(C₁-C₆alkyl), N(C₁-C₆alkyl)₂, -NHC(O)-C₁-C₆alkyl, -N(C₁-C₆alkyl)-C(O)-C₁-C₆alky C(O)-NH₂, -C(O)-NH(C₁-C₆alkyl), and -C(O)-N(C₁-C₆alkyl)₂, wherein the alkyl, alkeny alkynyl, and alkoxy, are optionally substituted with one or more halogen, hydroxyl, methoxy, C₃-C₈cycloalkyl, or NH₂;
R⁵ is H, C₁-C₆alkyl, or C₁-C₆haloalkyl;
A is selected from the group consisting of 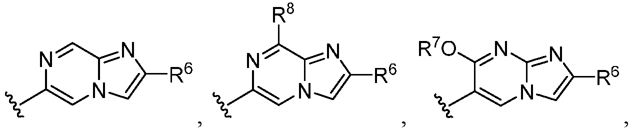 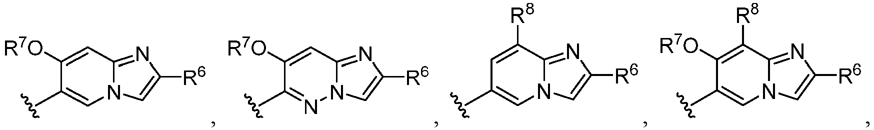 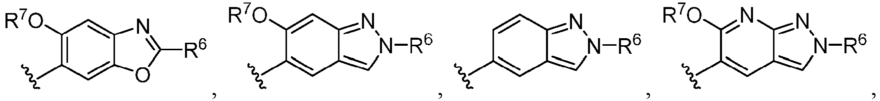 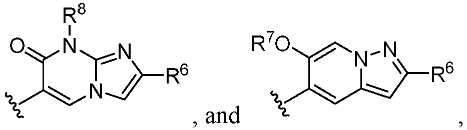
wherein A is optionally substituted with 1-4 R⁹;
R⁶ is C₁-C₃alkyl or C₁-C₃haloalkyl;
R⁷ is H, C₁-C₆alkyl, C₁-C₆haloalkyl, C₁-C₆cycloalkyl, or heterocycloalkyl, wherein heterocycloalkyl is optionally substituted with 1-3 substituents independently selected from halogen and C₁-C₆alkyl;
R⁸ is halogen, C₁-C₆alkyl, C₁-C₆alkoxy, C₁-C₆haloalkyl, or C₁-C₆cycloalkyl;
each R⁹ is independently selected from the group consisting of halogen, hydroxy, cyano, -COOH, -C(O)-C₁-C₆alkyl, -C(O)O-C₁-C₆alkyl, C₁-C₆alkyl, C₂-C₆alkenyl, C₂-C₆alkynyl, C₁-C₆alkoxy, C₃-C₈cycloalkyloxy, C₃-C₈cycloalkyl, NH₂, NH(C₁-C₆alkyl), N(C₁-C₆alkyl)₂, -NHC(O)-C₁-C₆alkyl, -N(C₁-C₆alkyl)-C(O)-C₁-C₆alkyl, -C(O)-NH₂, - C(O)-NH(C₁-C₆alkyl), and -C(O)-N(C₁-Csalkyl)₂, wherein the alkyl, alkenyl, alkynyl, and alkoxy are optionally substituted with one or more halogen, hydroxyl, methoxy, C₃-C₈cycloalkyl, or NH₂;
B is heterocycloalkyl not linked to formula (I) by a nitrogen atom, optionally substituted with 1 to 6 R¹²; or
B is NR¹⁰R¹¹, wherein
R¹⁰ is -(CH₂)₀₋₃aryl, -{CH₂)₀₋₃heteroaryl, -(CH₂)₀₋₃heterocycloalkyl comprising at least 1 nitrogen ring atom, or C₁-C₈heteroalkyl comprising at least one nitrogen atom, each R¹⁰ optionally substituted with 1 to 6 R¹²; and
R¹¹ is hydrogen, C₁₋₇alkyl, C₁₋₇haloalkyl, or C₃₋₈cycloalkyl; or
R¹⁰ and R¹¹ are taken together with the nitrogen atom to which they are attached to form a heterocycloalkyl comprising 1, 2 or 3 total nitrogen ring atoms and 0 or 1 additional ring heteroatoms selected from O and S, and the heterocycloalkyl is optionally substituted with 1 to 6 R¹²;
each R¹² is independently selected from the group consisting of halogen, hydroxy, cyano, -COOH, -C(O)-C₁-C₆alkyl, -C(O)O-C₁-C₆alkyl, C₁-C₆alkyl, C₁-C₆heteroalkyl, C₂-C₆alkenyl, C₂-C₆alkynyl, C₁-C₆alkoxy, -(CH₂)₀₋₂-C₃-C₈cycloalkyl, -(CH₂)₀₋₂-SO₂-C₁-C₆alkyl, C₁-C₆heteroalkylene-C₃-C₈cycloalkyl, -O-C₃-C₈cycloalkyl, -4-7-membered monocyclic heterocycloalkyl, C₁-C₆heteroalkylene-(4-7-membered monocyclic heterocycloalkyl), -O-(4-7-membered monocyclic heterocycloalkyl), -(CH₂)₀₋₂-(4-7-membered monocyclic heterocycloalkyl), NH₂, NH(C₁-C₆alkyl), N(C₁-C₆alkyl)₂, - NHC(O)-C₁-C₆alkyl, -N(C₁-C₆alkyl)-C(O)-C₁-C₆alkyl, -C(O)-NH₂, -C(O)-NH(C₁-C₆alkyl), and -C(O)-N(C₁-C₆alkyl)₂, wherein the alkyl, alkenyl, alkynyl, and alkoxy are optionally substituted with one or more halogen, hydroxyl or NH₂, and wherein the cycloalkyl and heterocycloalkyl are optionally substituted with one or more halogen, hydroxyl, C₁-C₆alkyl, C₁-C₆heteroalkyl, C₁-C₆alkoxy, or NH₂; or
two R¹² on the same carbon can be taken together as keto (=O).

2. The compound of claim 1, wherein 0, 1, or 2 of X, Y, and Z are N.

3. The compound of claim 1 or 2, wherein the compound is selected from:
(a) Formula (Ia), 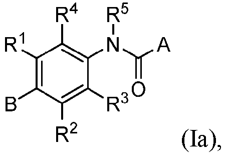 or a pharmaceutically acceptable salt thereof;
(b) Formula (Ib), 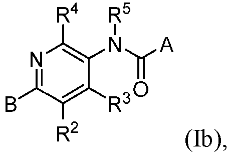 or a pharmaceutically acceptable salt thereof;
(c) Formula (Ic), 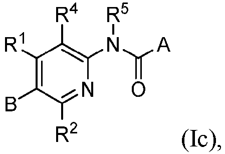 or a pharmaceutically acceptable salt thereof;
(d) Formula (Id), 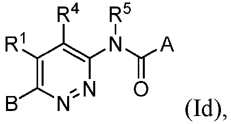 or a pharmaceutically acceptable salt thereof;
(e) Formula (Ie), 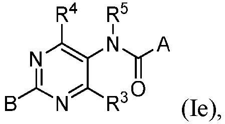 or a pharmaceutically acceptable salt thereof; or
(f) Formula (If), 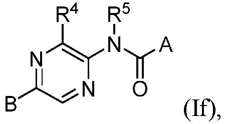 or a pharmaceutically acceptable salt thereof.

4. The compound of any one of claims 1-3, wherein:
(a) R¹, R², R³ and R⁴ are each independently selected from the group consisting of H, halogen, C₁-C₆alkyl, C₁-C₆haloalkyl, C₁-C₆alkoxy, and C₁-C₆cycloalkyloxy; optionally wherein R¹, R², R³ and R⁴ are each independently selected from the group consisting of H, halogen, C₁-C₆alkoxy, and C₁-C₆alkyl; optionally wherein R¹, R², R³ and R⁴ are each H; and/or
(b) R³ is H; and/or
(c) A is selected from the group consisting of 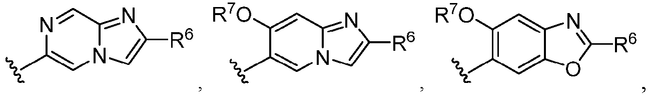 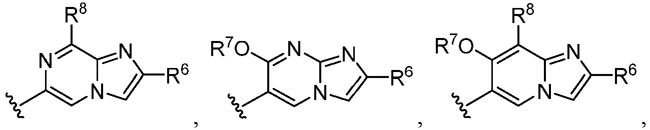 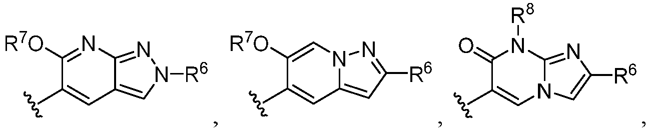 and 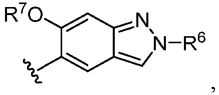 wherein A is optionally substituted with 1-3 R⁹.

5. The compound of any one of claims 1-4, wherein:
(a) A is substituted by one R⁹ selected from the group consisting of halogen and C₁-C₆alkyl, or wherein A is not substituted by R⁹; and/or
(b) R⁶ is Me; and/or
(c)R⁷ is C₁-C₆alkyl, C₁-C₆cycloalkyl, or heterocycloalkyl; optionally wherein R⁷ is Me, Et, isopropyl, or cyclobutyl.

6. The compound according to any one of claims 1-5, wherein A is selected from the group consisting of:

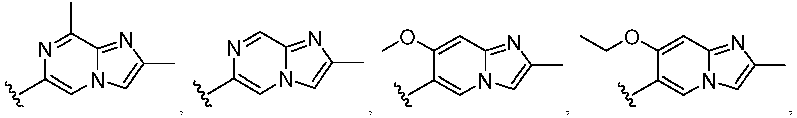

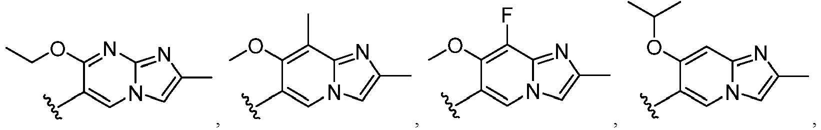

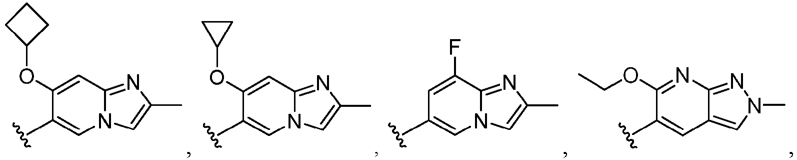

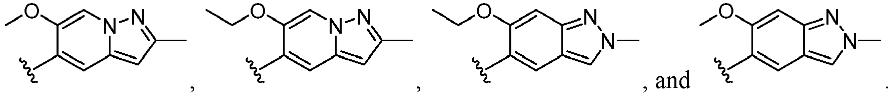

7. The compound of any one of claims 1-6, wherein A is
(a) 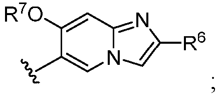 optionally wherein
A is 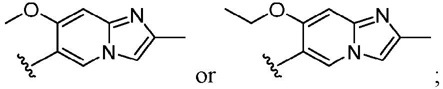 or
(b) 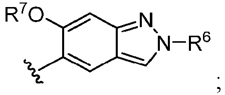 optionally wherein
A is 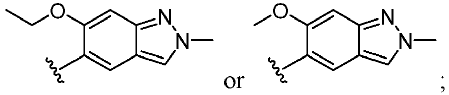 or
(c)

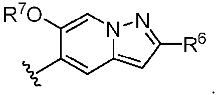

8. The compound according to any one of claims 1-7, wherein B is NR¹⁰R¹¹, wherein R¹⁰ and R¹¹ are taken together with the nitrogen atom to which they are attached to form a monocyclic or bicyclic heterocycloalkyl comprising 1, 2 or 3 total nitrogen ring atoms and 0 or 1 additional ring heteroatoms selected from O and S, and the heterocycloalkyl is optionally substituted with 1, 2, 3, or 4 R¹².

9. The compound of any one of claims 1-8, wherein R¹⁰ and R¹¹ are taken together with the nitrogen atom to which they are attached to form a monocyclic heterocycloalkyl of 4-7 ring atoms with 1 or 2 total nitrogen ring atoms and 0 or 1 additional ring heteroatoms selected from O and S, and the heterocycloalkyl is optionally substituted with 1, 2, 3, or 4 R¹².

10. The compound of any one of claims 1-9, wherein B is 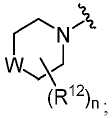
W is NR¹³ or CR¹⁴R¹⁴;
R¹³ is selected from the group consisting of hydrogen, C₁-C₆alkyl, C₂-C₆alkenyl, C₂-C₆alkynyl, and -(CH₂)₀₋₂-C₃-C₈cycloalkyl, wherein alkyl, alkenyl, alkynyl, are optionally substituted with one or more halogen, hydroxyl, methoxy, or NH₂, and wherein the cycloalkyl is optionally substituted with one or more halogen, hydroxyl, C₁-C₆alkyl, C₁-C₆alkoxy, or NH₂;
each R¹⁴ is independently H or R¹²; and
n = 0, 1, 2, 3, or 4; optionally wherein:
(a) each R¹² is independently selected from the group consisting of halogen, hydroxy, 4-7-membered monocyclic heterocycloalkyl, C₁-C₆heteroalkyl, and C₁-C₆alkyl, wherein alkyl is optionally substituted with one or more halogen, hydroxyl, methoxy, C₃-C₈cycloalkyl, or NH₂, and heterocycloalkyl is optionally substituted with one or more halogen, hydroxyl, methoxy, or C₁-C₆alkyl; and R¹³ is H or unsubstituted C₁-C₆alkyl; optionally wherein each R¹² is independently C₁-C₆alkyl; and/or
(b) B is 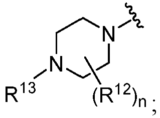 optionally wherein R¹³ is H or unsubstituted C₁-C₆alkyl; and/or wherein each R¹² is independently C₁-C₆alkyl.

11. The compound of any one of claims 1-8, wherein B is a bicyclic 6-14 membered heterocycloalkyl comprising 1, 2 or 3 total nitrogen ring atoms and 0 or 1 additional ring heteroatoms selected from O and S, and the heterocycloalkyl is optionally substituted with 1, 2, 3, or 4 R¹²; optionally wherein each R¹² is independently C₁-C₆alkyl.

12. The compound of any one of claims 1-7, wherein B is NR¹⁰R¹¹ and R¹¹ is hydrogen, C₁₋₇alkyl, C₁₋₇haloalkyl, or C₃₋₇ycloalkyl.

13. The compound of any one of claims 1-7 or 12, wherein:
(a) R¹¹ is H or C₁₋₇alkyl, and R¹⁰ is C₁-C₈heteroalkyl comprising at least one nitrogen atom; or
(b) R¹⁰ is-(CH₂)₀₋₃heterocycloalkyl comprising at least 1 nitrogen ring atom, each R¹⁰ optionally substituted with 1 to 6 R¹².

14. The compound of claim 1, wherein the compound is of:
(a) formula (Ig) 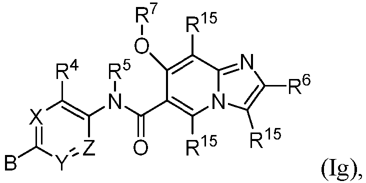
or a pharmaceutically acceptable salt thereof,
wherein each R¹⁵ is independently H, halogen, C₁-C₆alkyl, or C₁-C₆haloalkyl; optionally wherein R⁵ is H;
(b) formula (Ih) 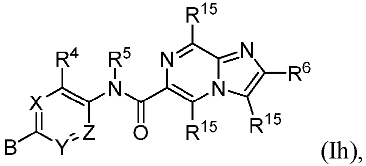
or a pharmaceutically acceptable salt thereof,
wherein each R¹⁵ is independently H, halogen, C₁-C₆alkyl, or C₁-C₆haloalkyl; optionally wherein R⁵ is H;
(c) formula (Ii) 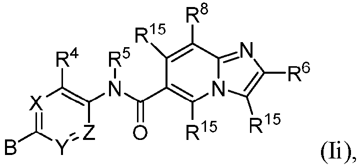
or a pharmaceutically acceptable salt thereof,
wherein each R¹⁵ is independently H, halogen, C₁-C₆alkyl, or C₁-C₆haloalkyl; optionally wherein R⁵ is H;
(d) formula (Ij) 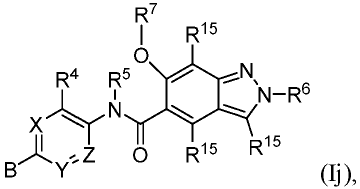
or a pharmaceutically acceptable salt thereof,
wherein each R¹⁵ is independently H, halogen, C₁-C₆alkyl, or C₁-C₆haloalkyl; optionally wherein R⁵ is H;
(e) formula (Ik) 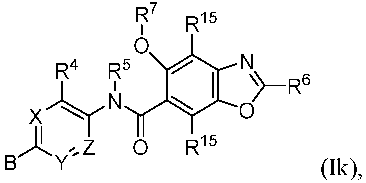
or a pharmaceutically acceptable salt thereof,
wherein each R¹⁵ is independently H, halogen, C₁-C₆alkyl, or C₁-C₆haloalkyl; optionally wherein R⁵ is H;
(f) formula (Im) 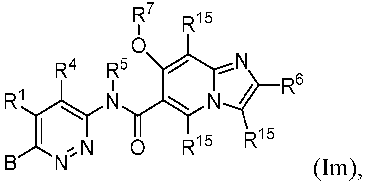
or a pharmaceutically acceptable salt thereof,
wherein each R¹⁵ is independently H, halogen, C₁-C₆alkyl, or C₁-C₆haloalkyl; optionally wherein R⁵ is H;
(g) formula (In) 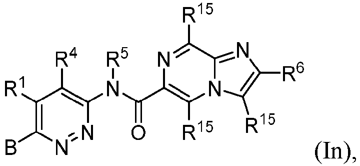
or a pharmaceutically acceptable salt thereof,
wherein each R¹⁵ is independently H, halogen, C₁-C₆alkyl, or C₁-C₆haloalkyl; optionally wherein R⁵ is H;
(h) formula (Io) 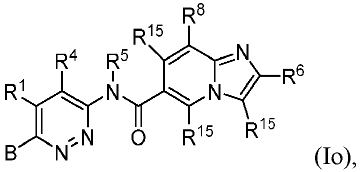
or a pharmaceutically acceptable salt thereof,
wherein each R¹⁵ is independently H, halogen, C₁-C₆alkyl, or C₁-C₆haloalkyl; optionally wherein R⁵ is H;
(i) formula (Ip) 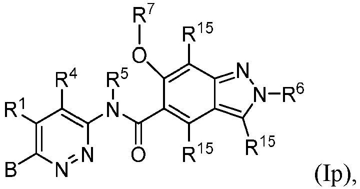
or a pharmaceutically acceptable salt thereof,
wherein each R¹⁵ is independently H, halogen, C₁-C₆alkyl, or C₁-C₆haloalkyl; optionally wherein R⁵ is H;
(j) formula (Iq) 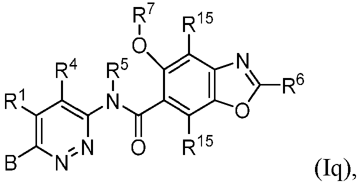
or a pharmaceutically acceptable salt thereof,
wherein each R¹⁵ is independently H, halogen, C₁-C₆alkyl, or C₁-C₆haloalkyl; optionally wherein R⁵ is H;
(k) formula (Ir) 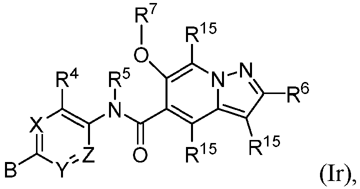
or a pharmaceutically acceptable salt thereof,
wherein each R¹⁵ is independently H, halogen, C₁-C₆alkyl, or C₁-C₆haloalkyl; optionally wherein R⁵ is H;
(l) formula (Is) 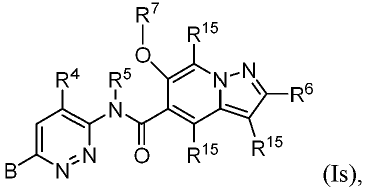
or a pharmaceutically acceptable salt thereof,
wherein each R¹⁵ is independently H, halogen, C₁-C₆alkyl, or C₁-C₆haloalkyl; optionally wherein R⁵ is H;
(m) formula (It) 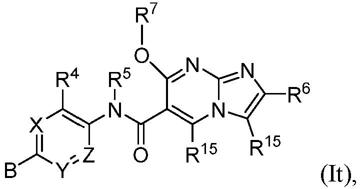
or a pharmaceutically acceptable salt thereof,
wherein each R¹⁵ is independently H, halogen, C₁-C₆alkyl, or C₁-C₆haloalkyl; optionally wherein R⁵ is H; or
(n) formula (Iu) 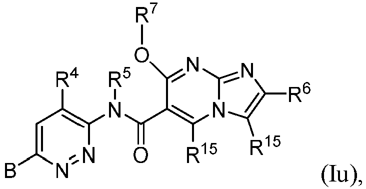
or a pharmaceutically acceptable salt thereof,
wherein each R¹⁵ is independently H, halogen, C₁-C₆alkyl, or C₁-C₆haloalkyl; optionally wherein R⁵ is H.

15. The compound of any one of claims 1-14, selected from:

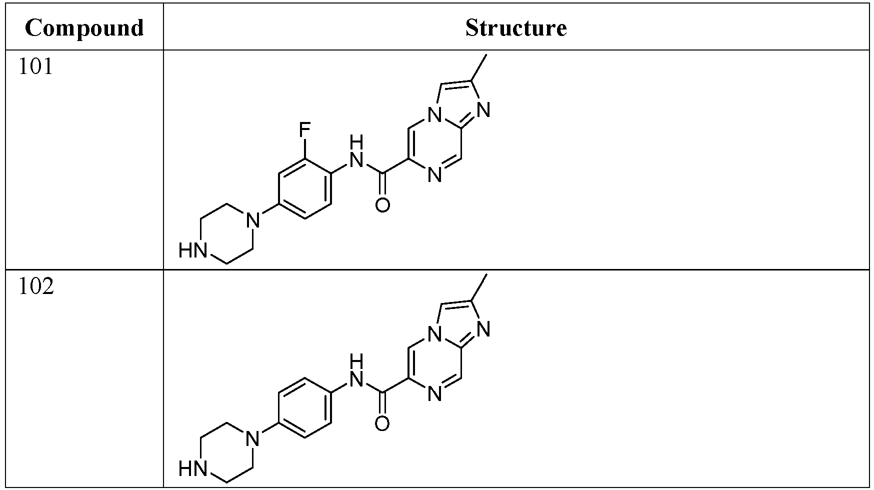

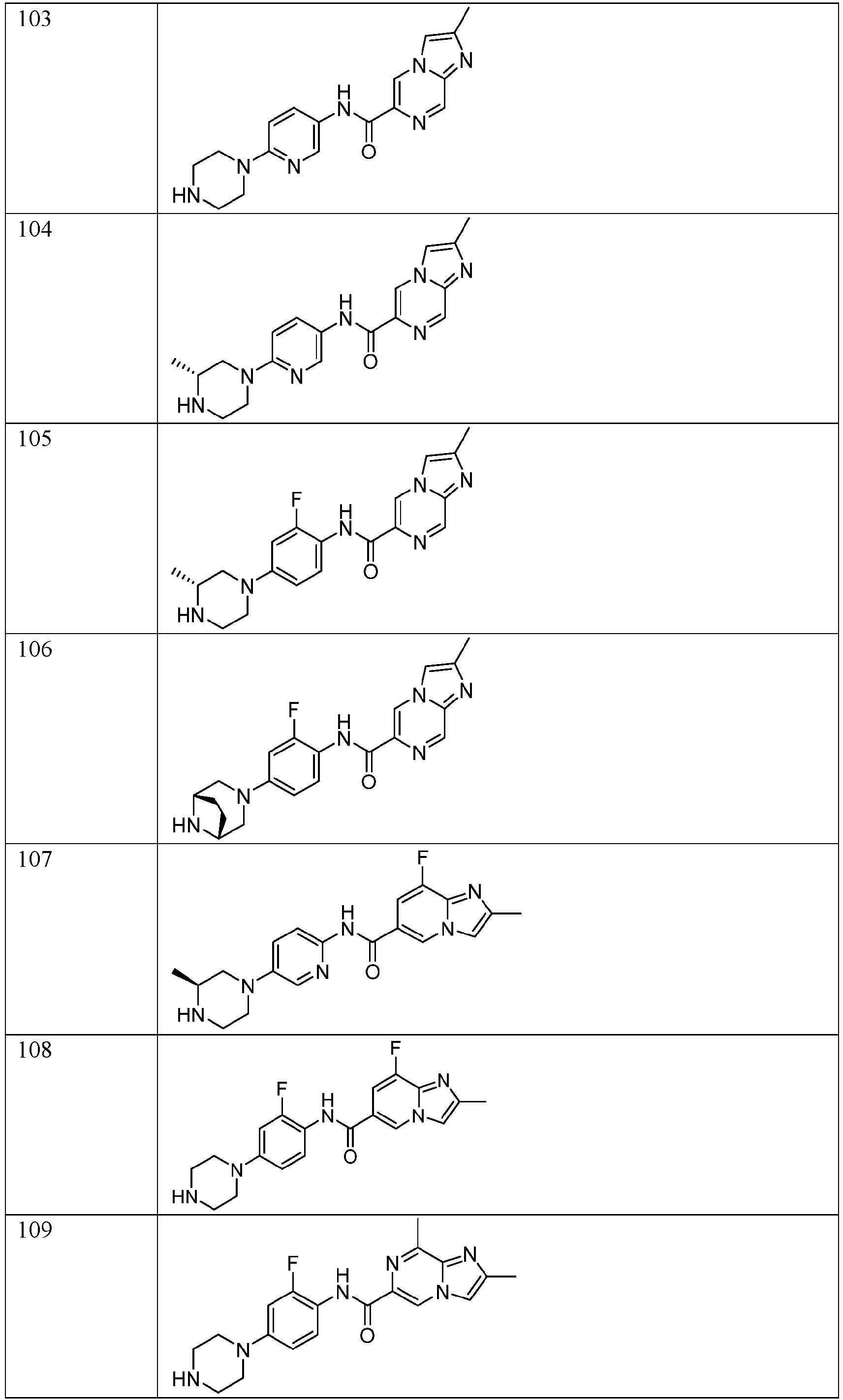

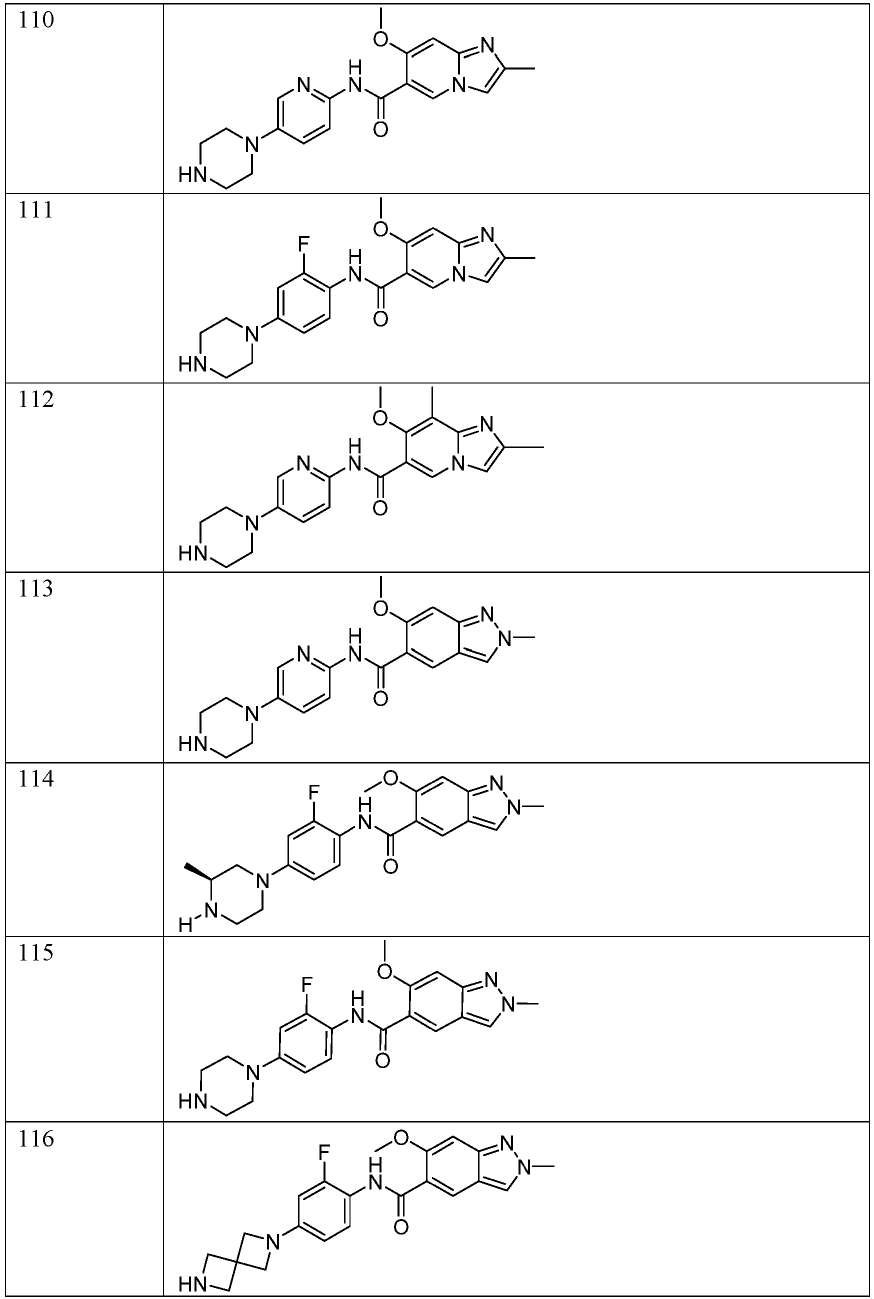

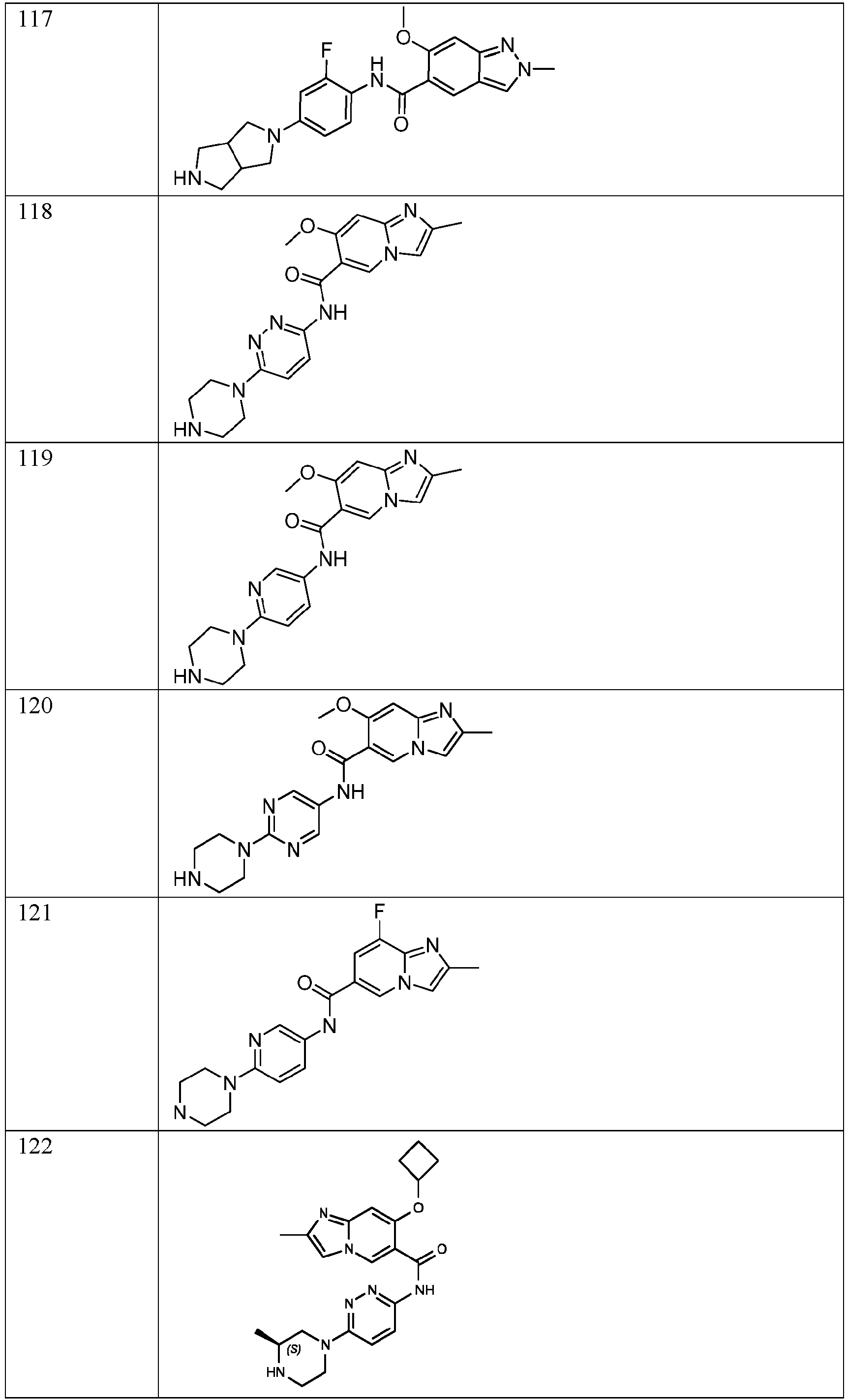

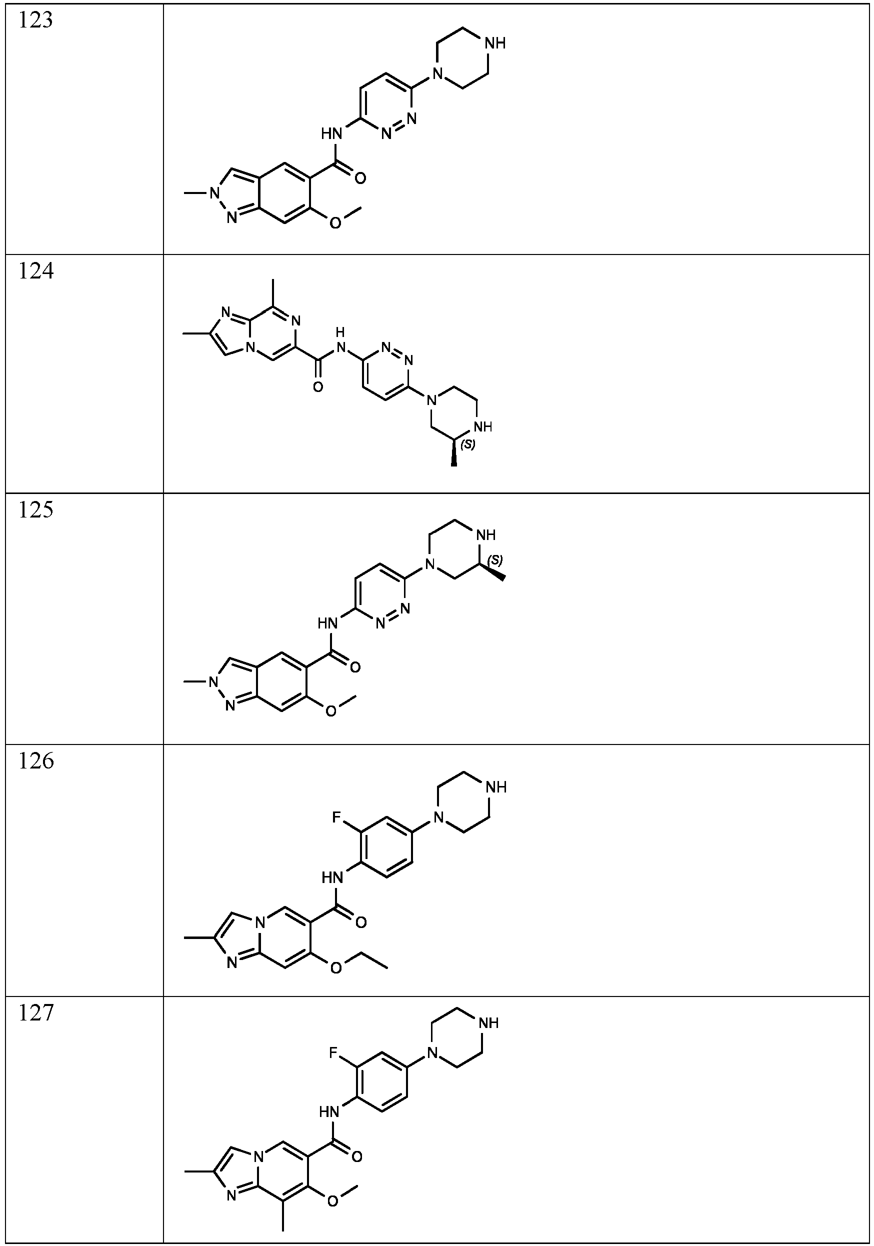

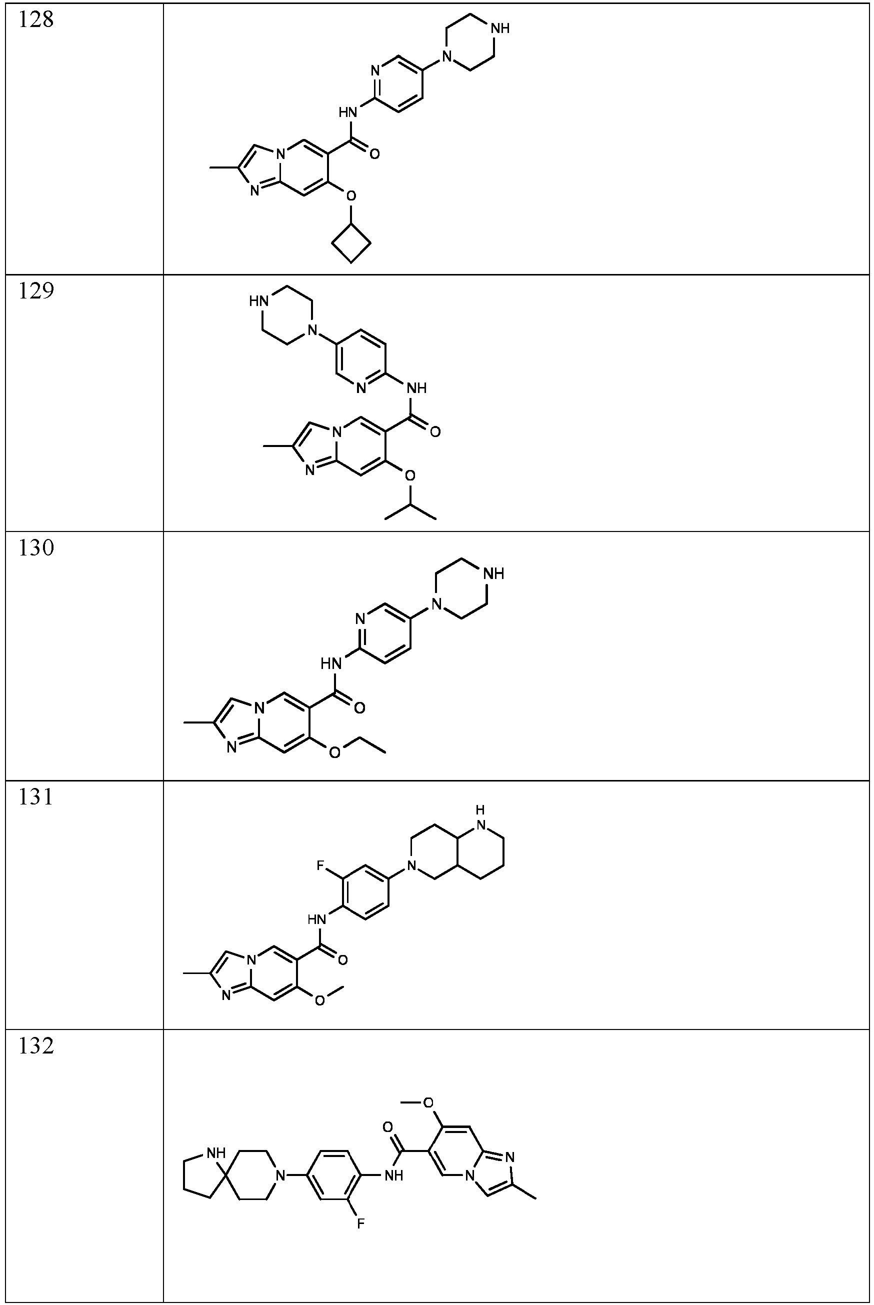

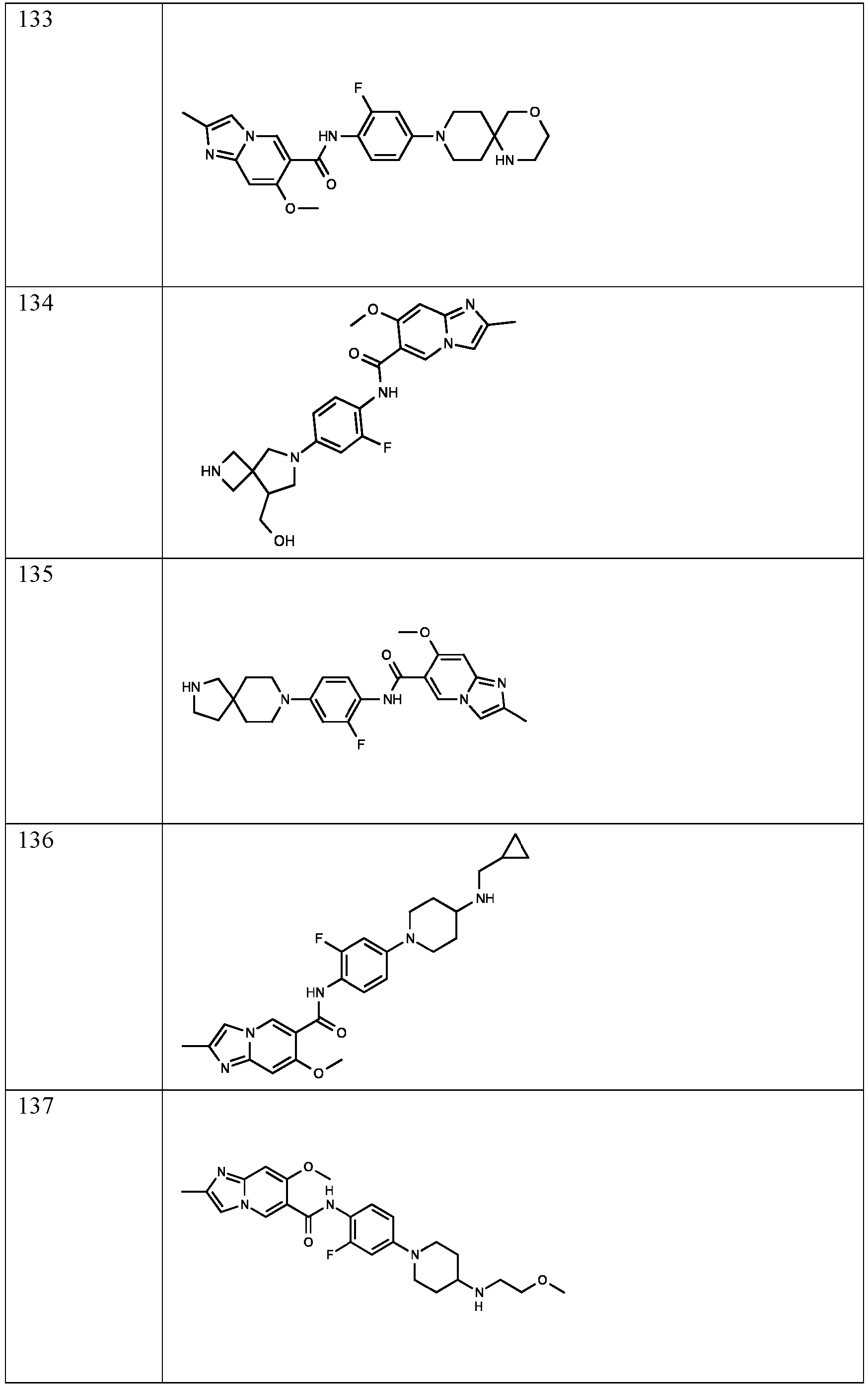

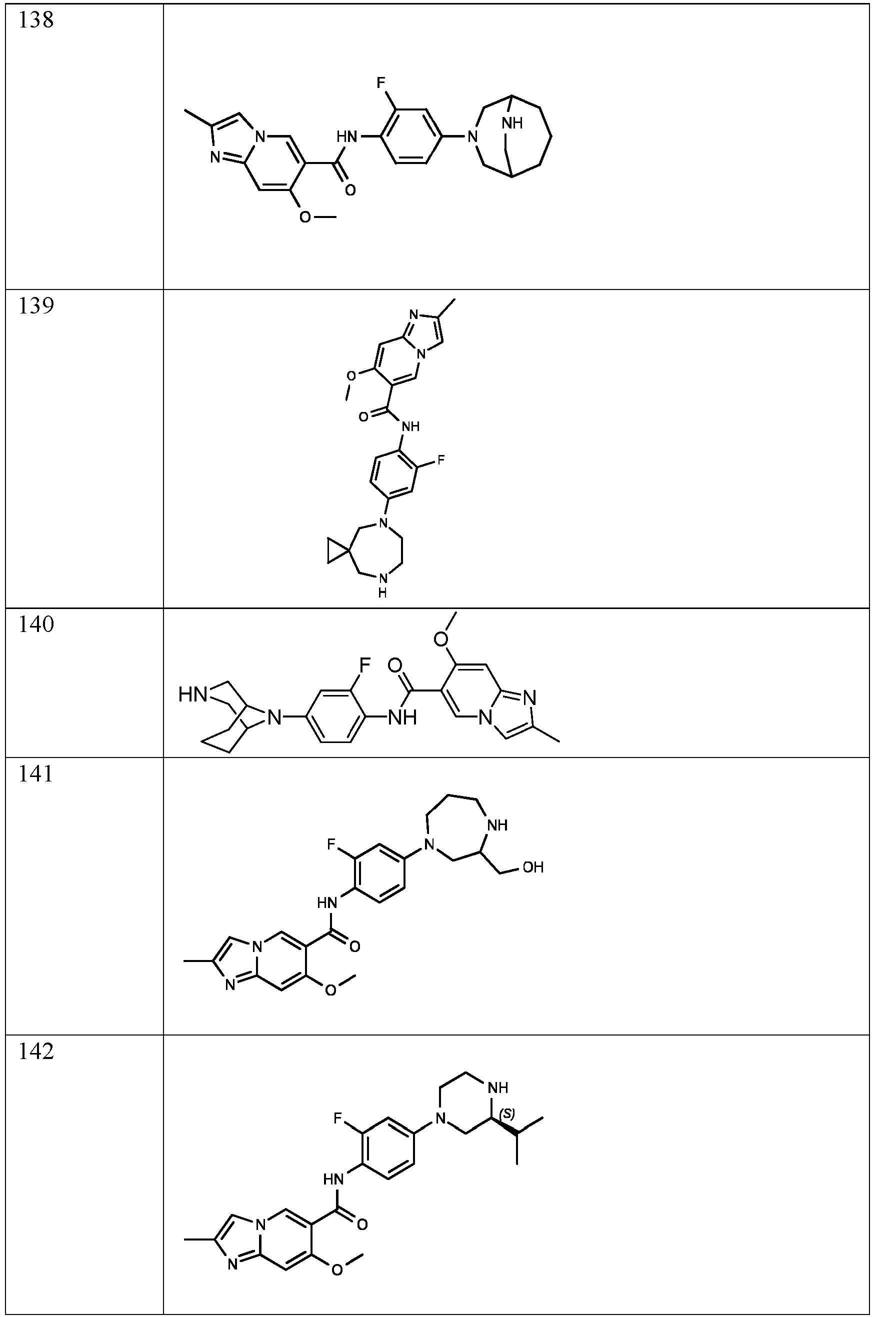

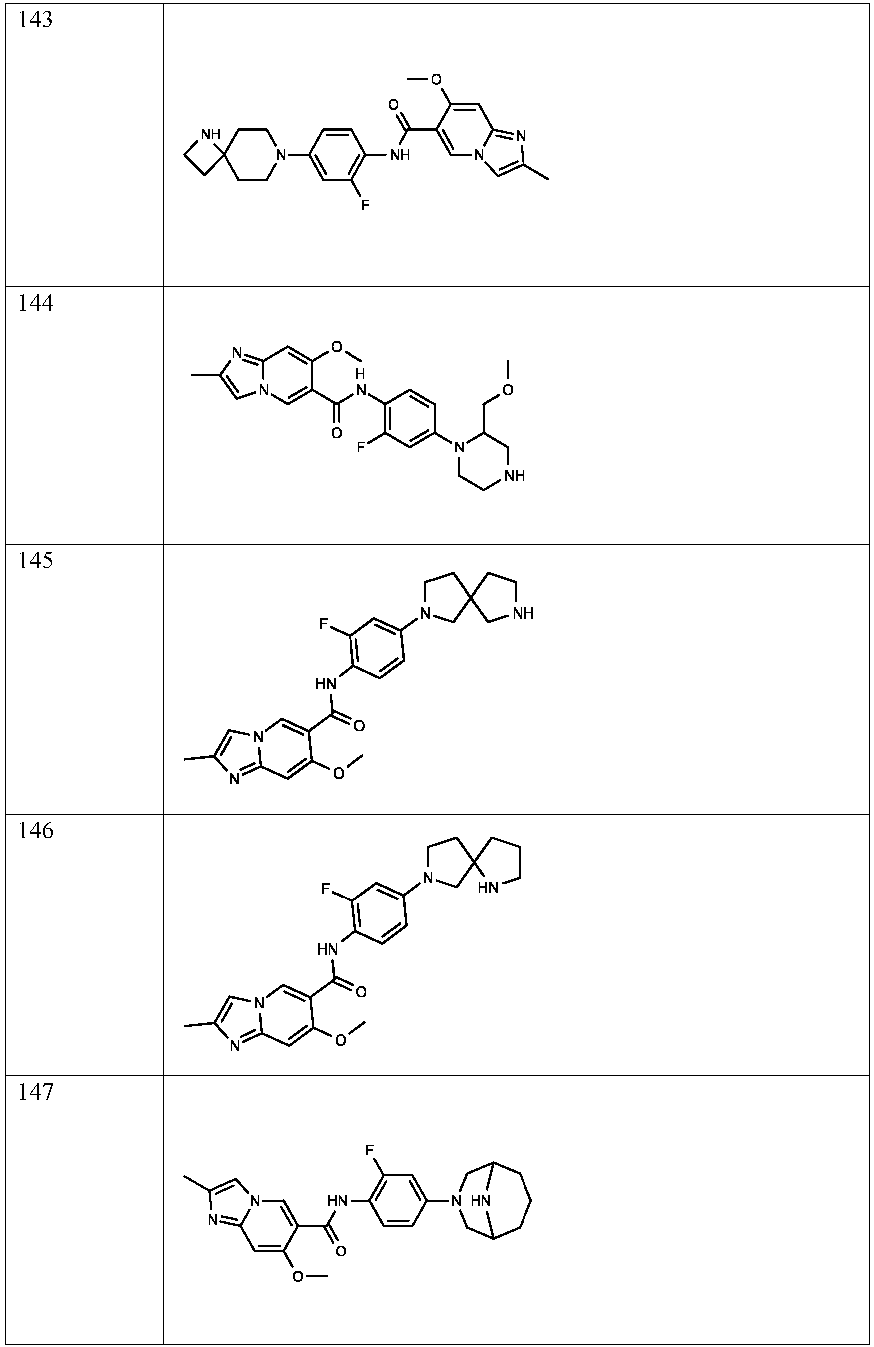

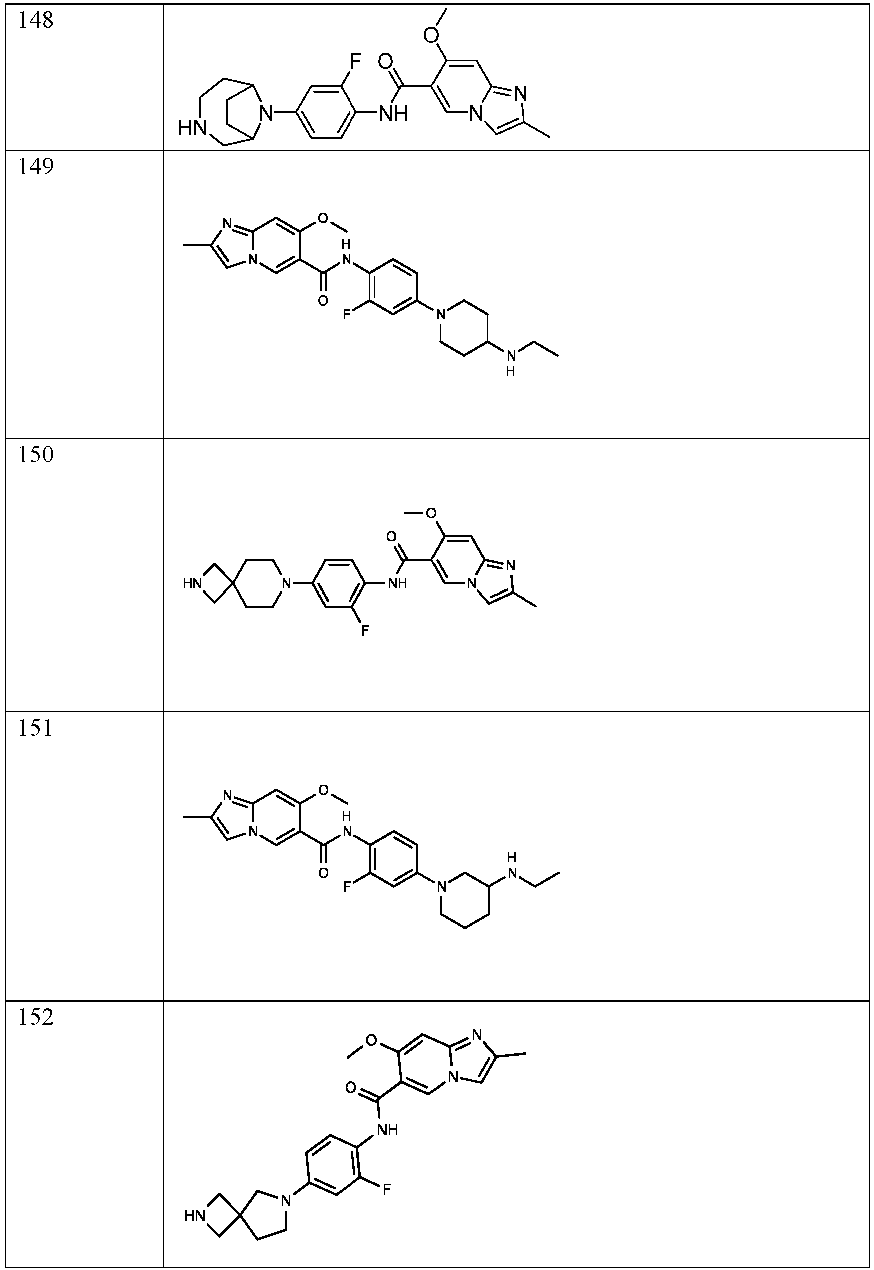

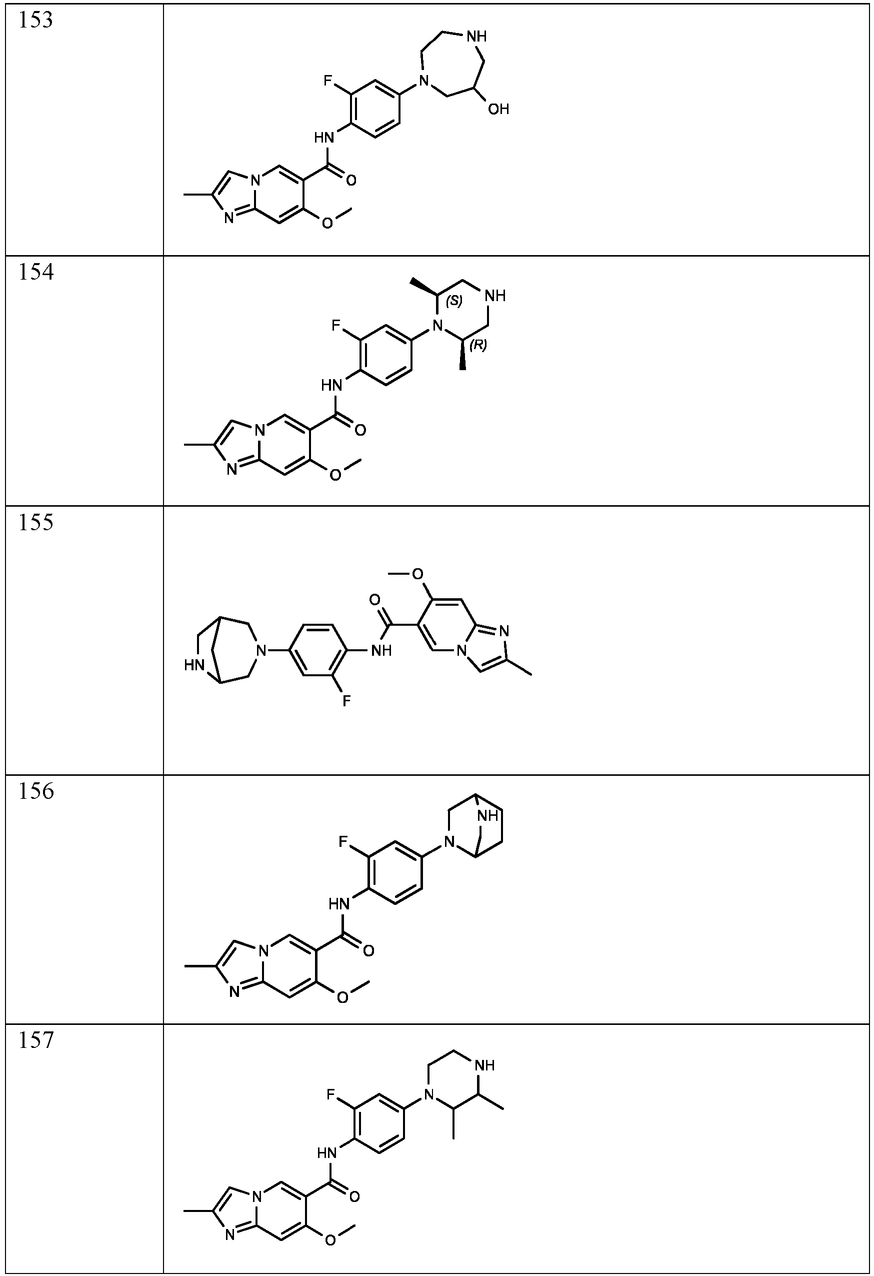

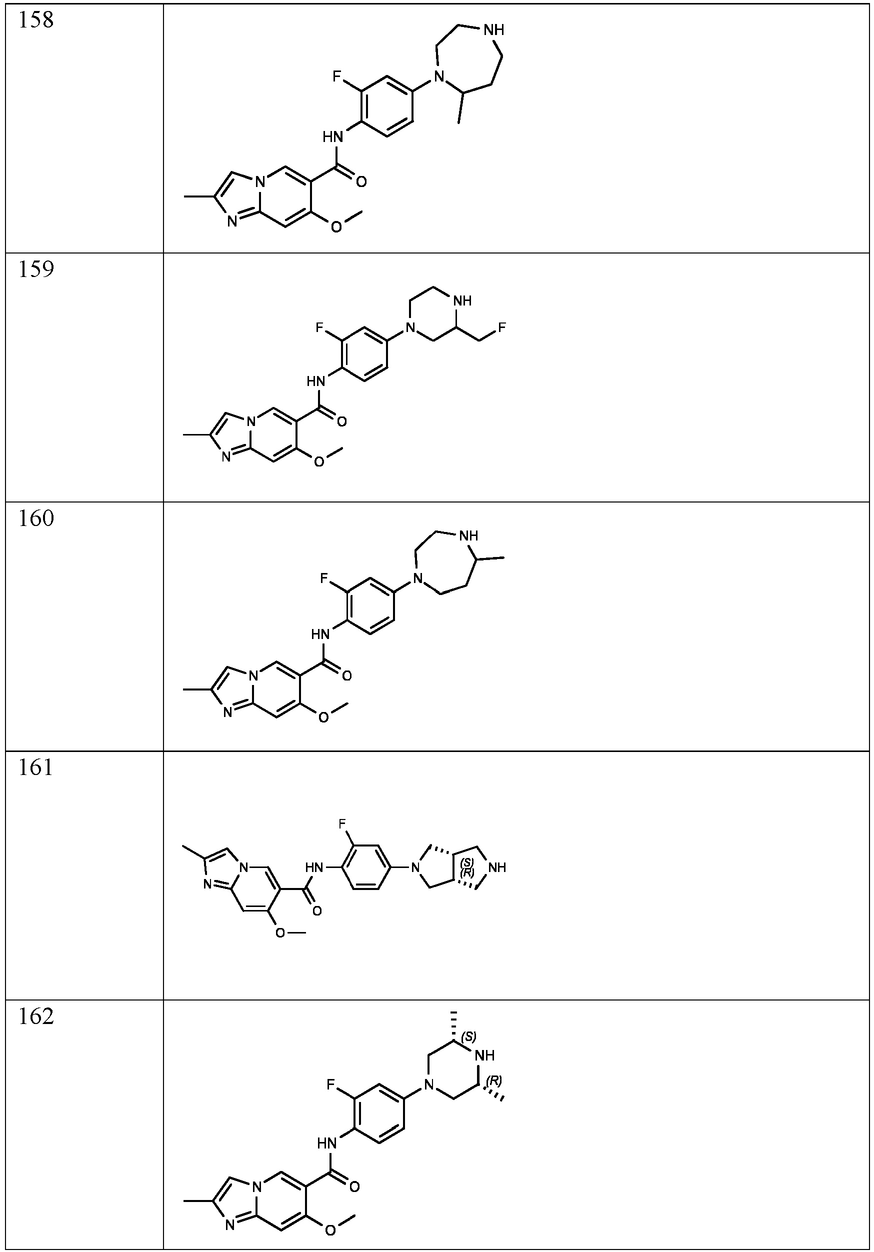

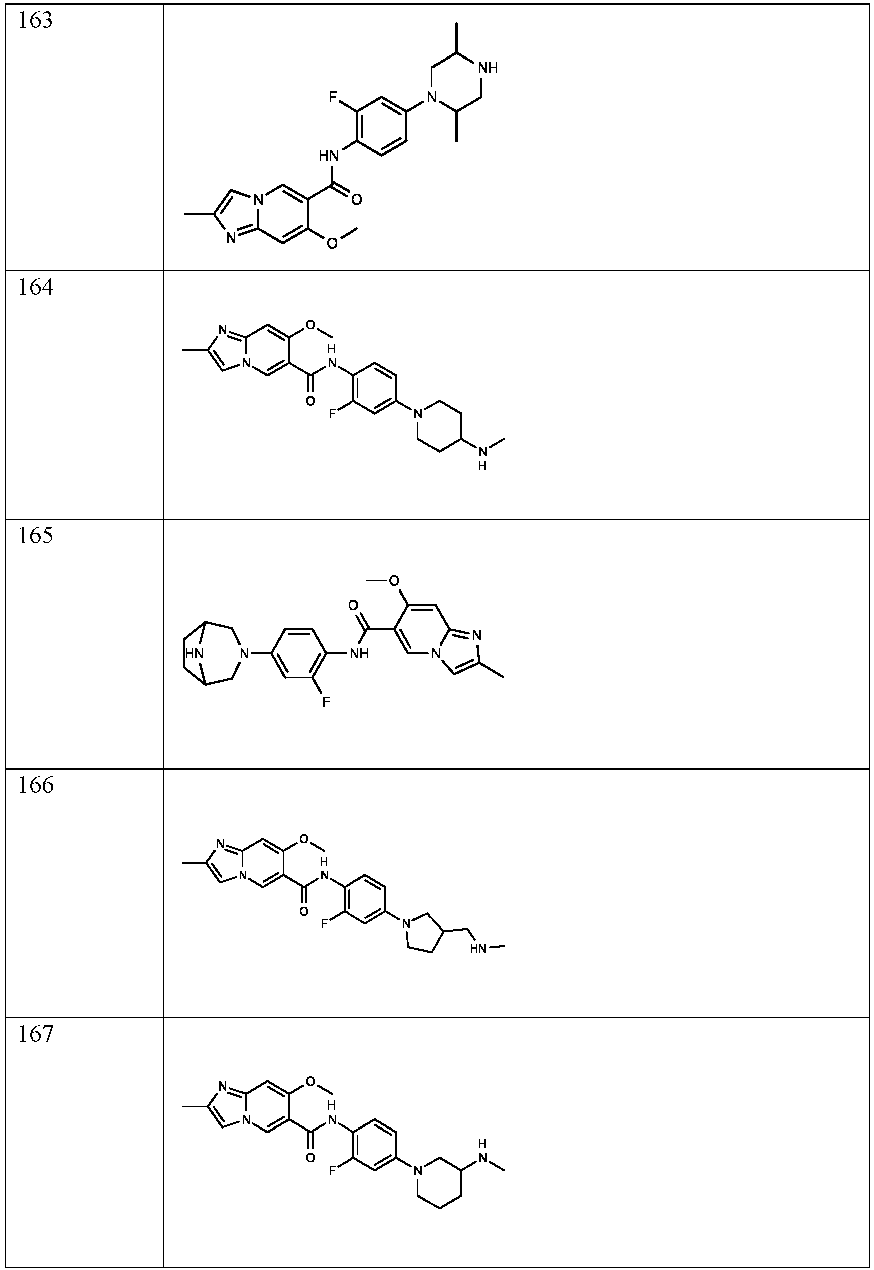

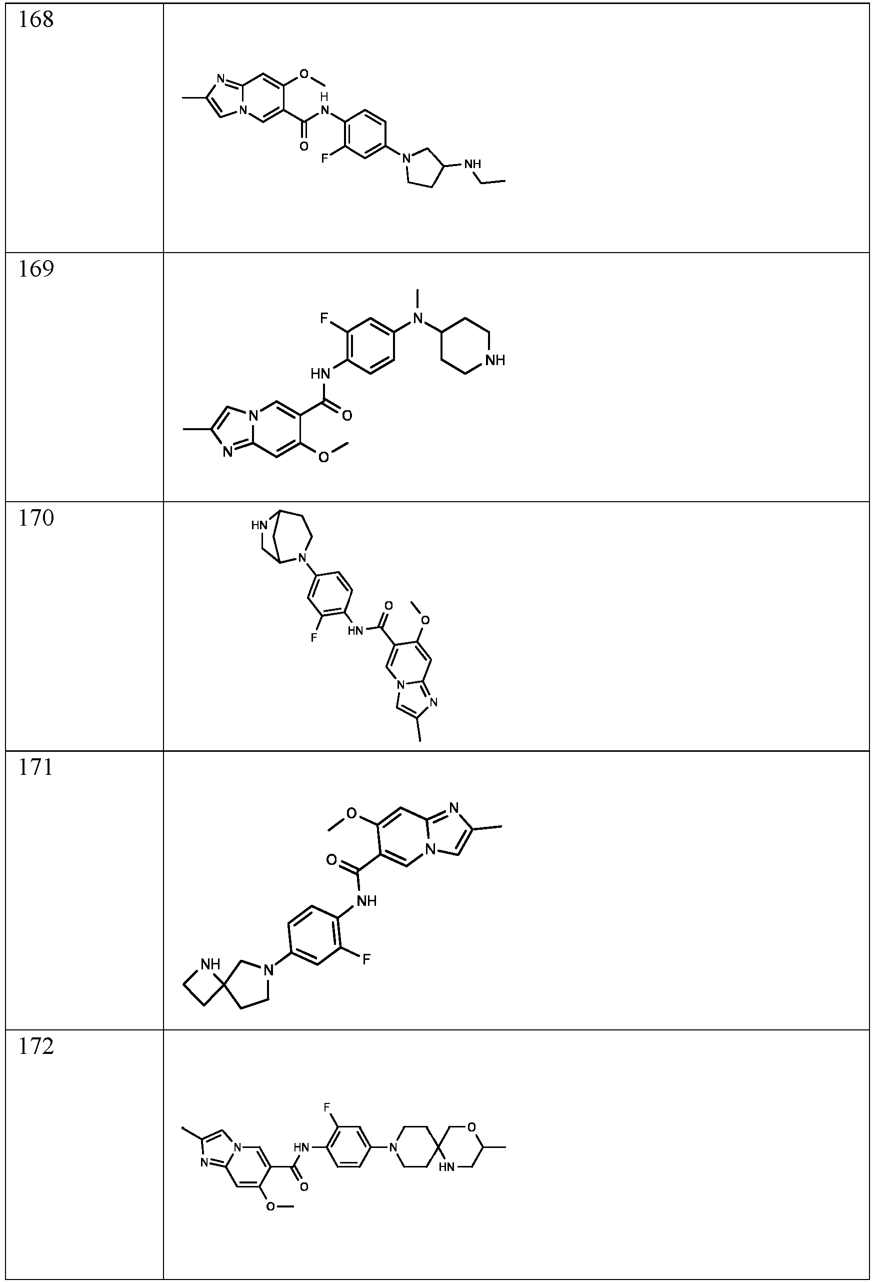

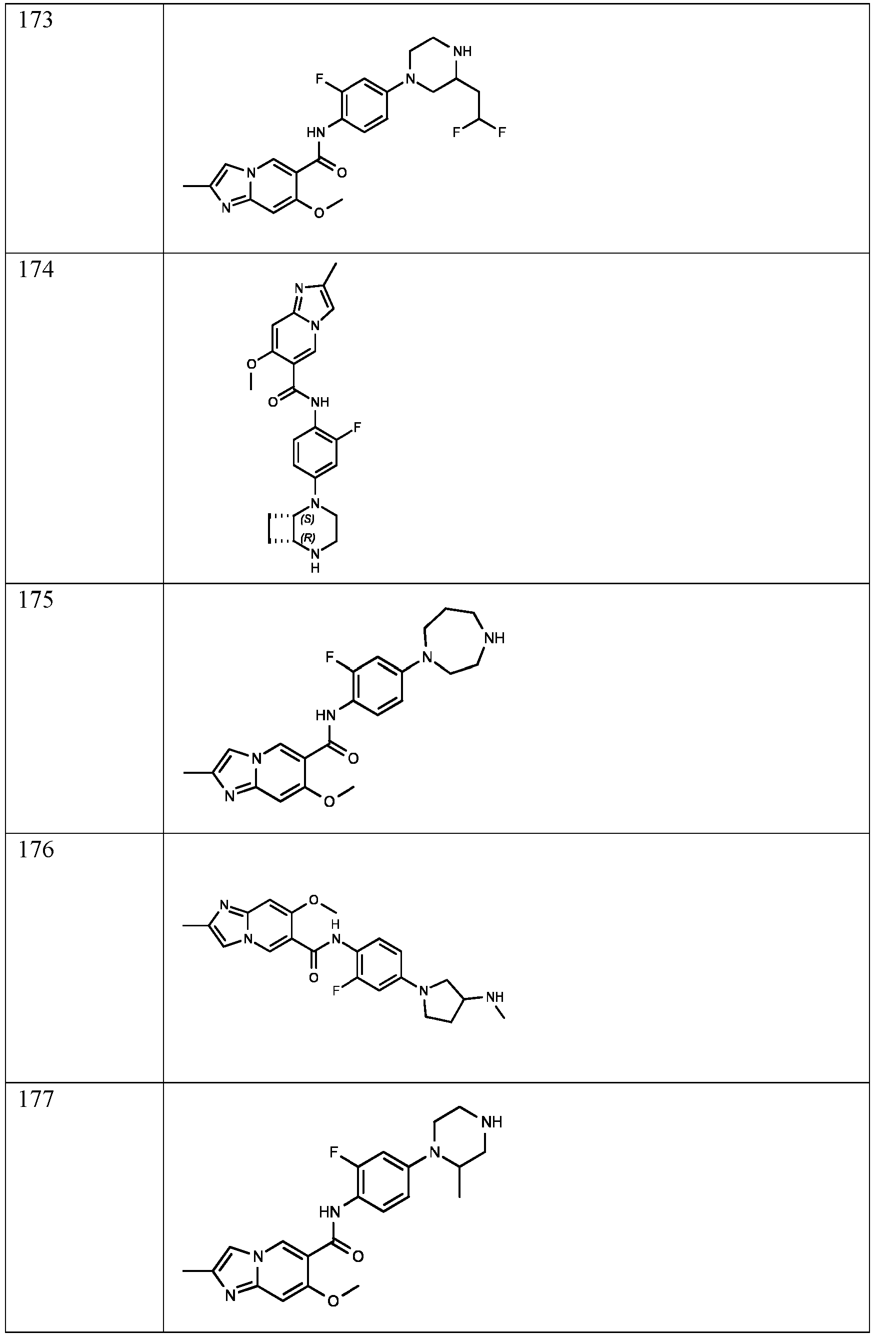

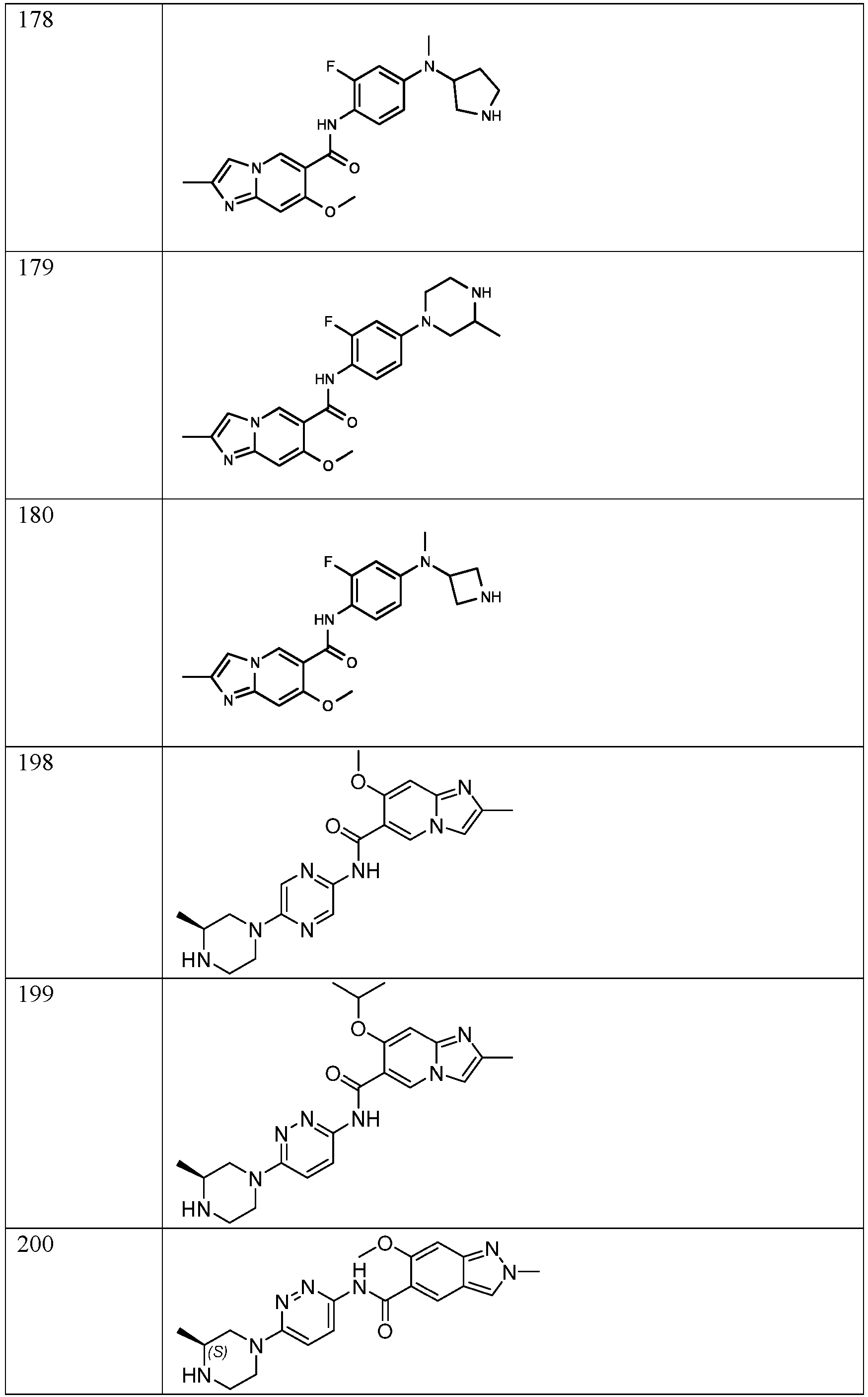

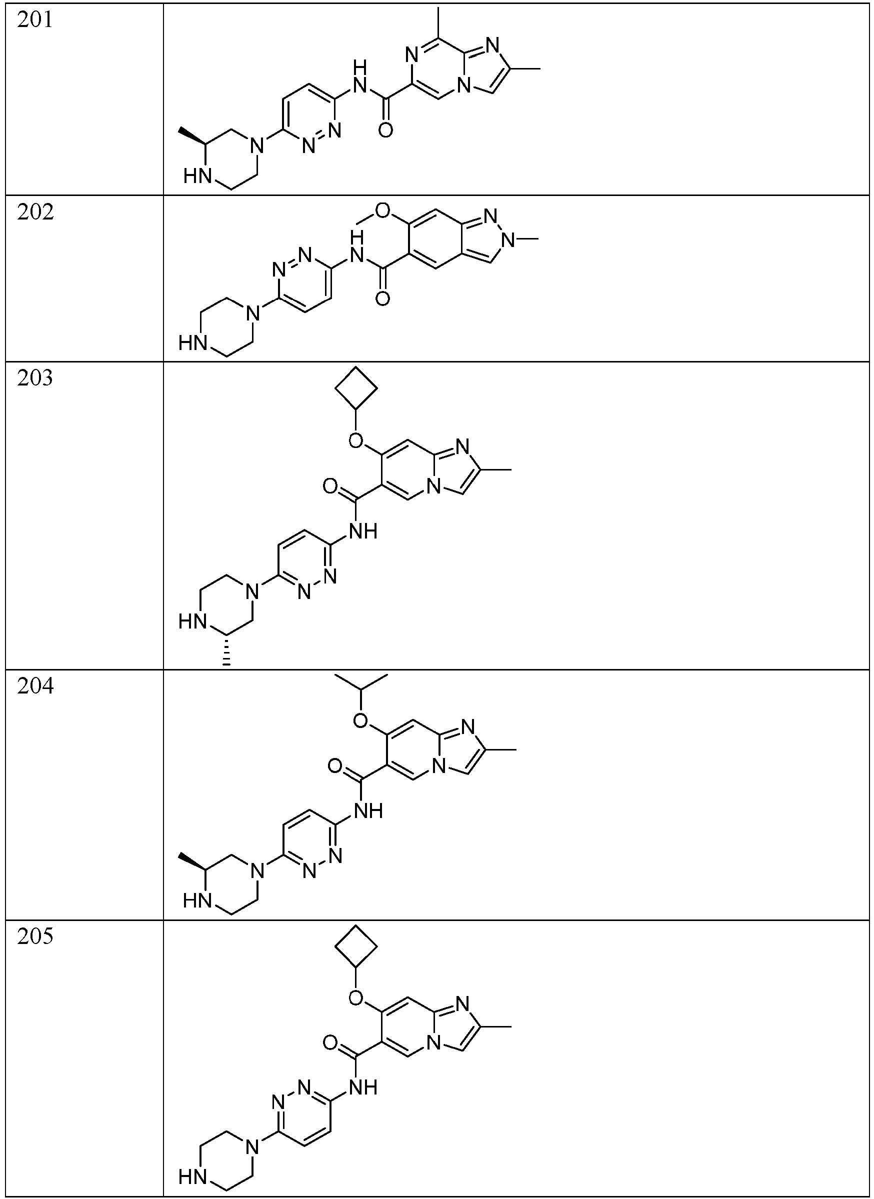

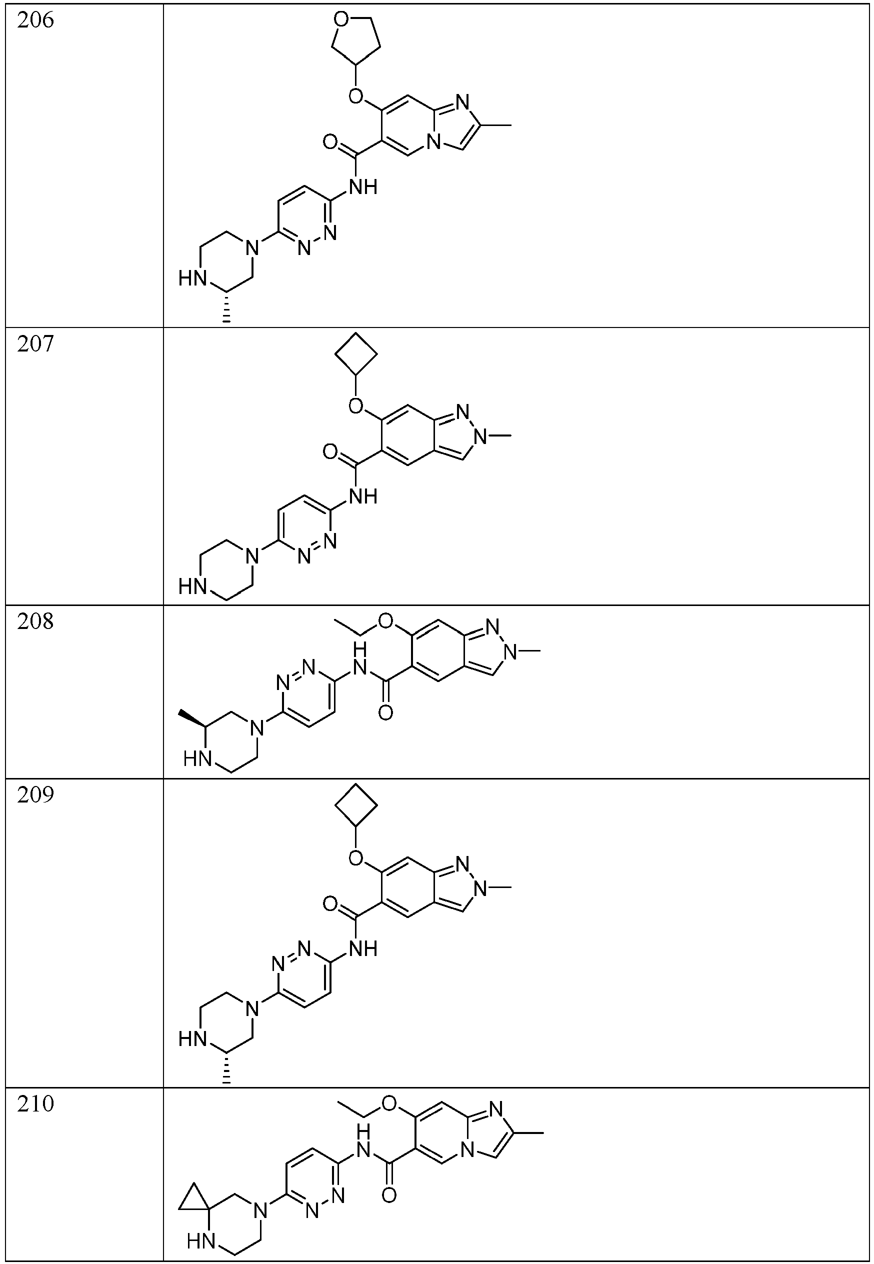

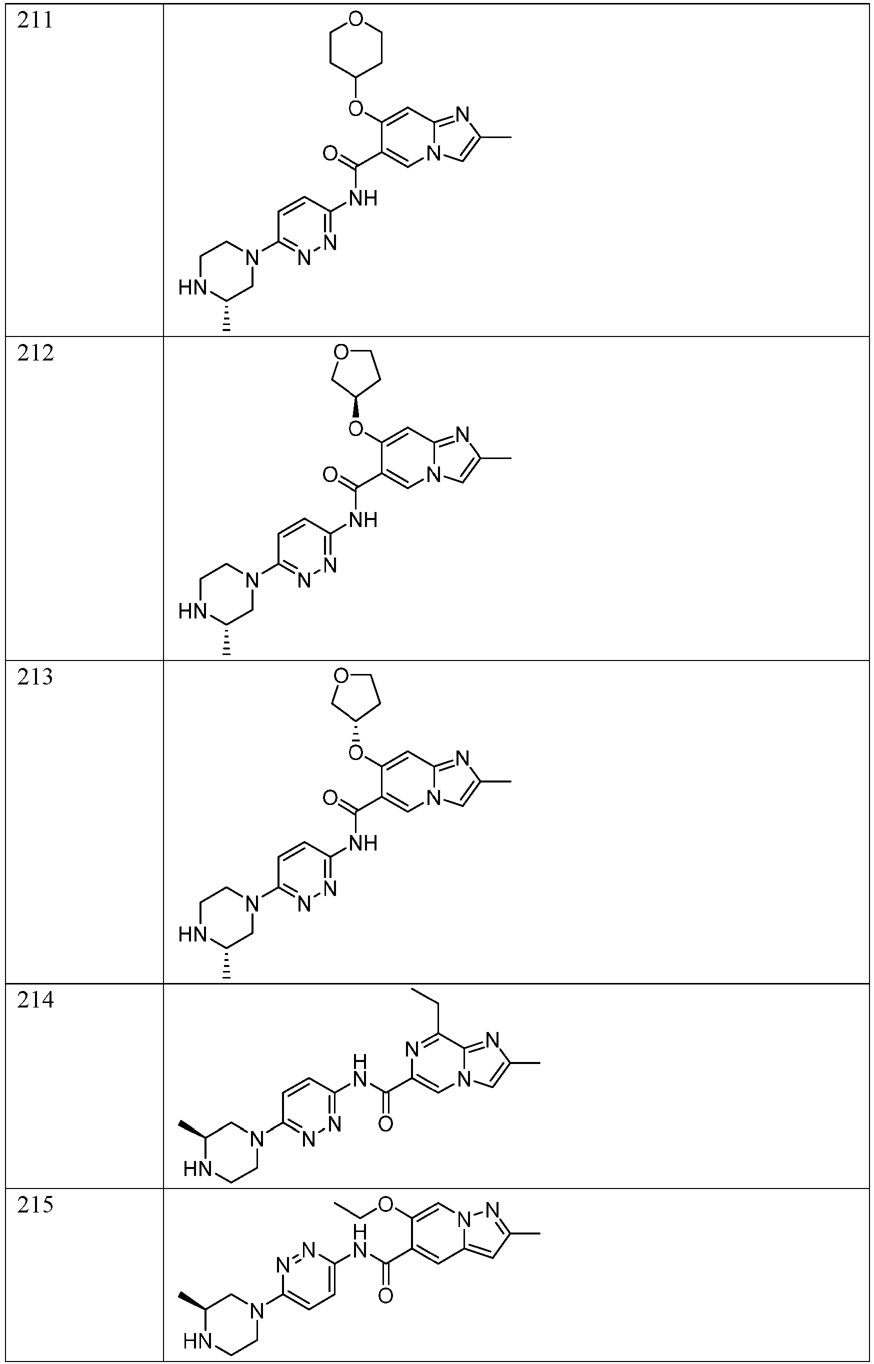

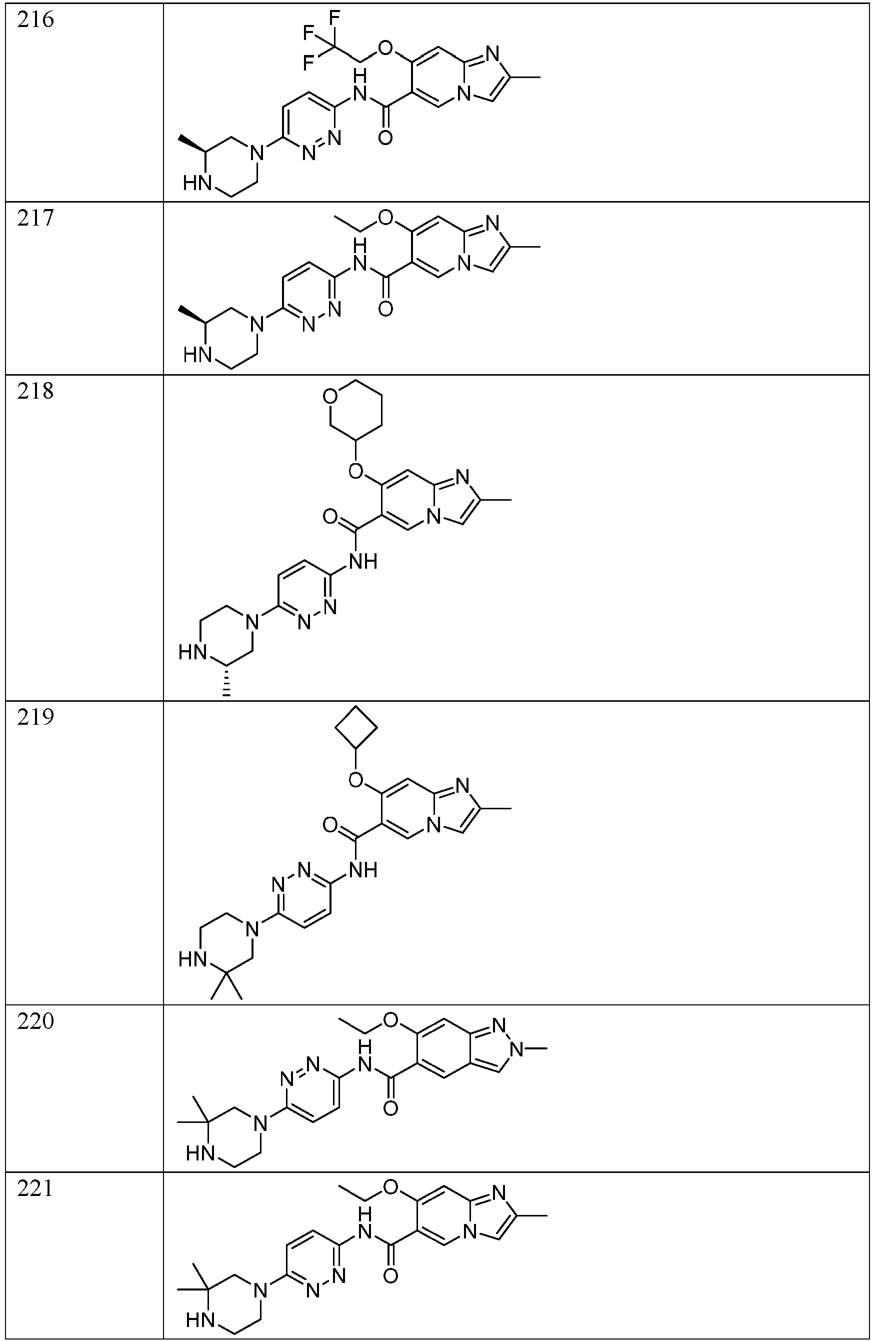

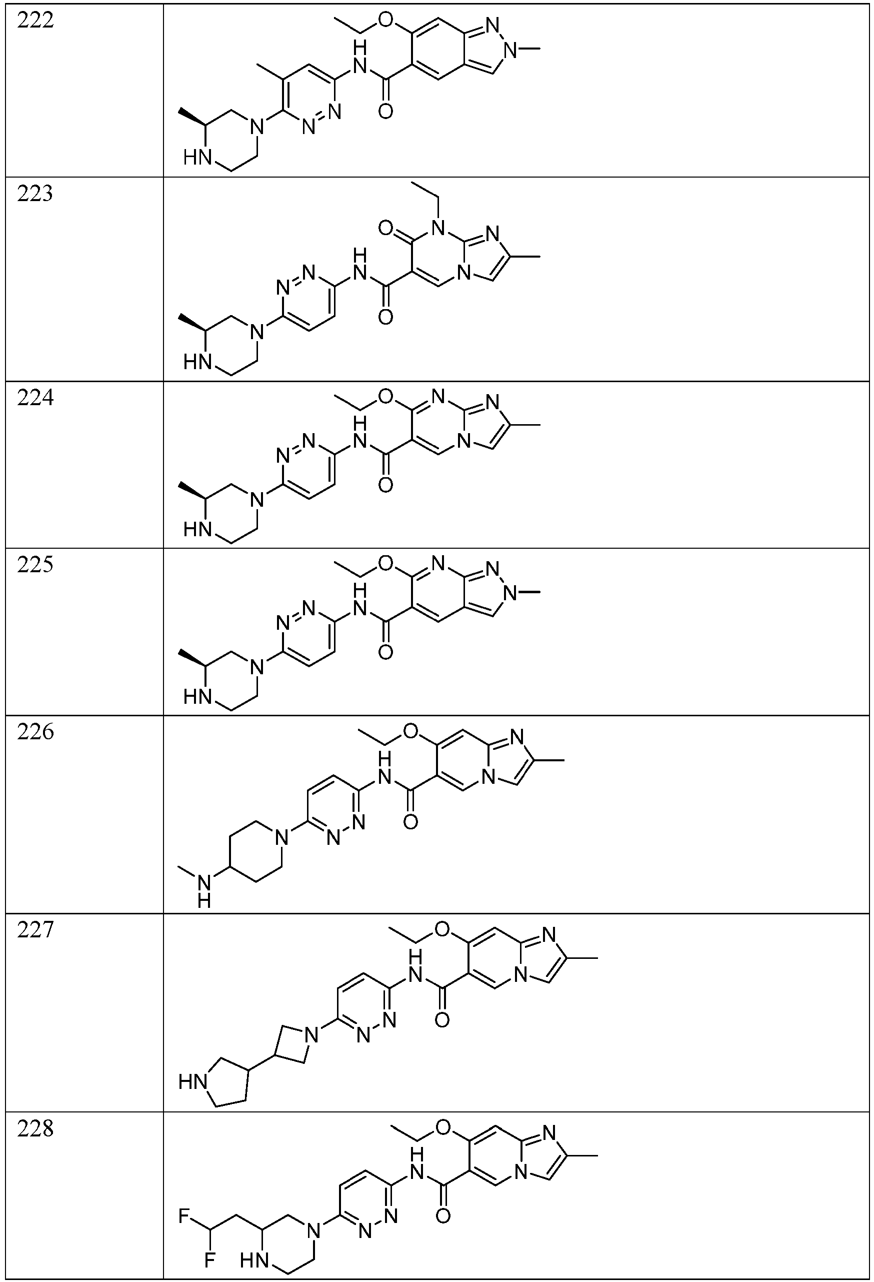

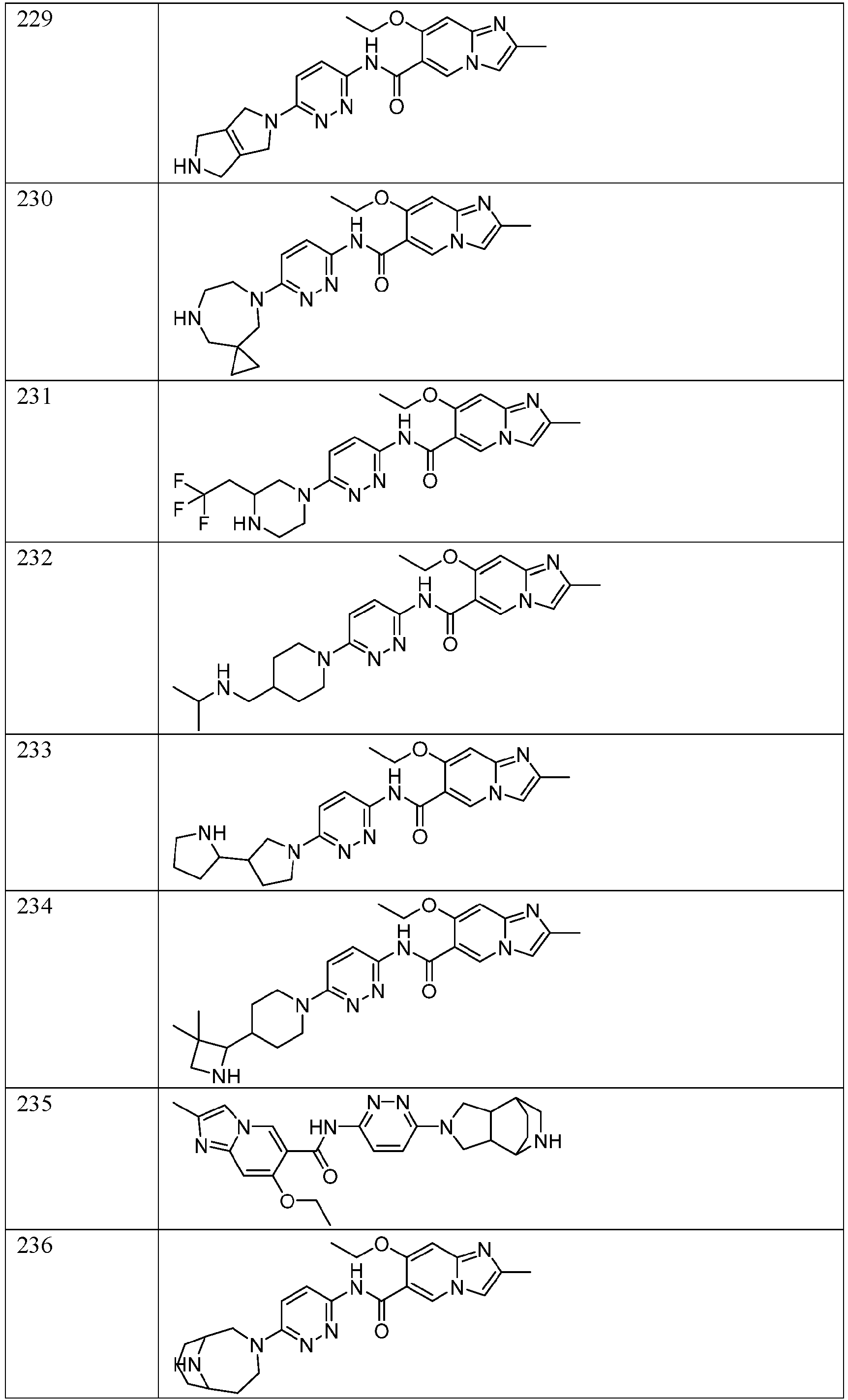

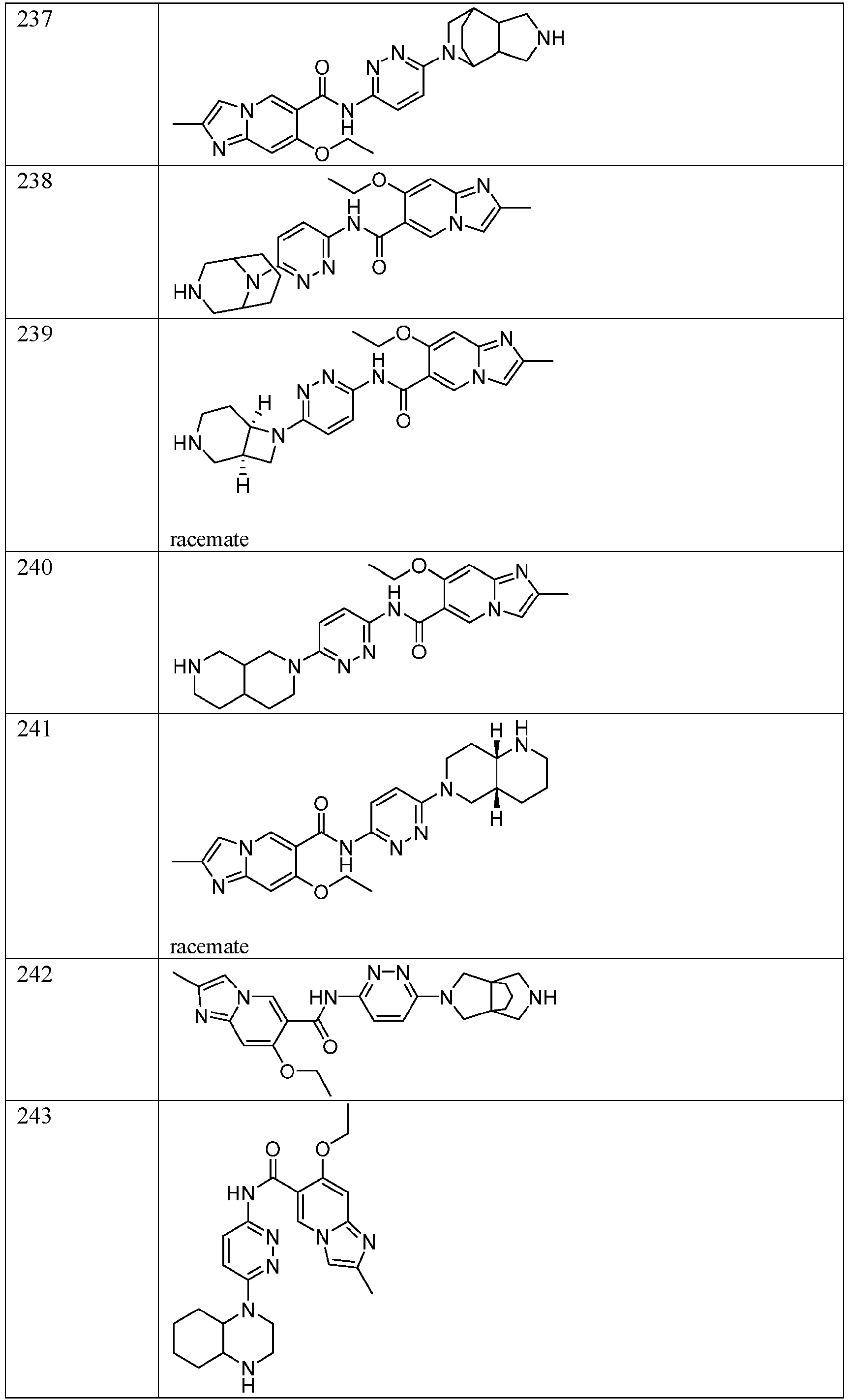

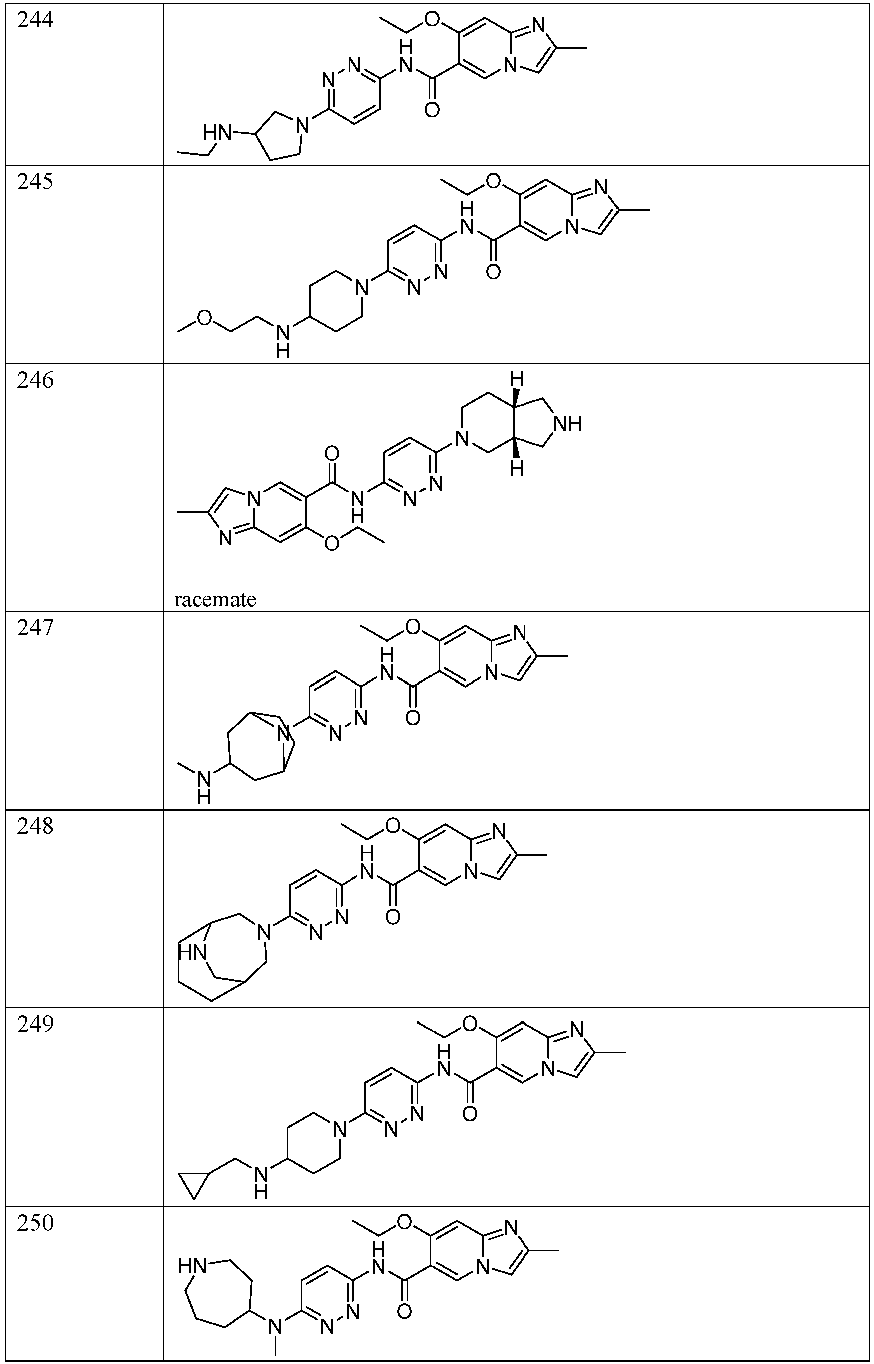

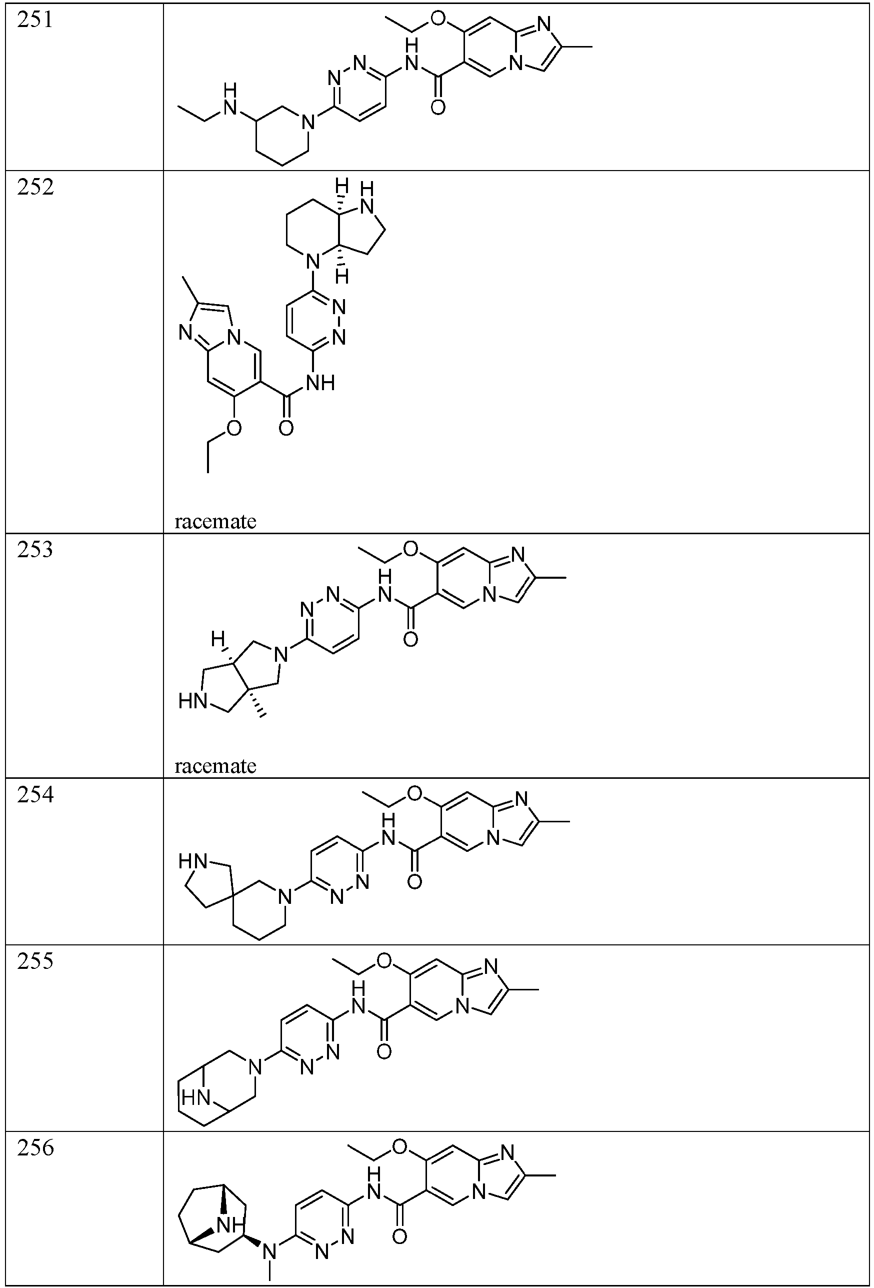

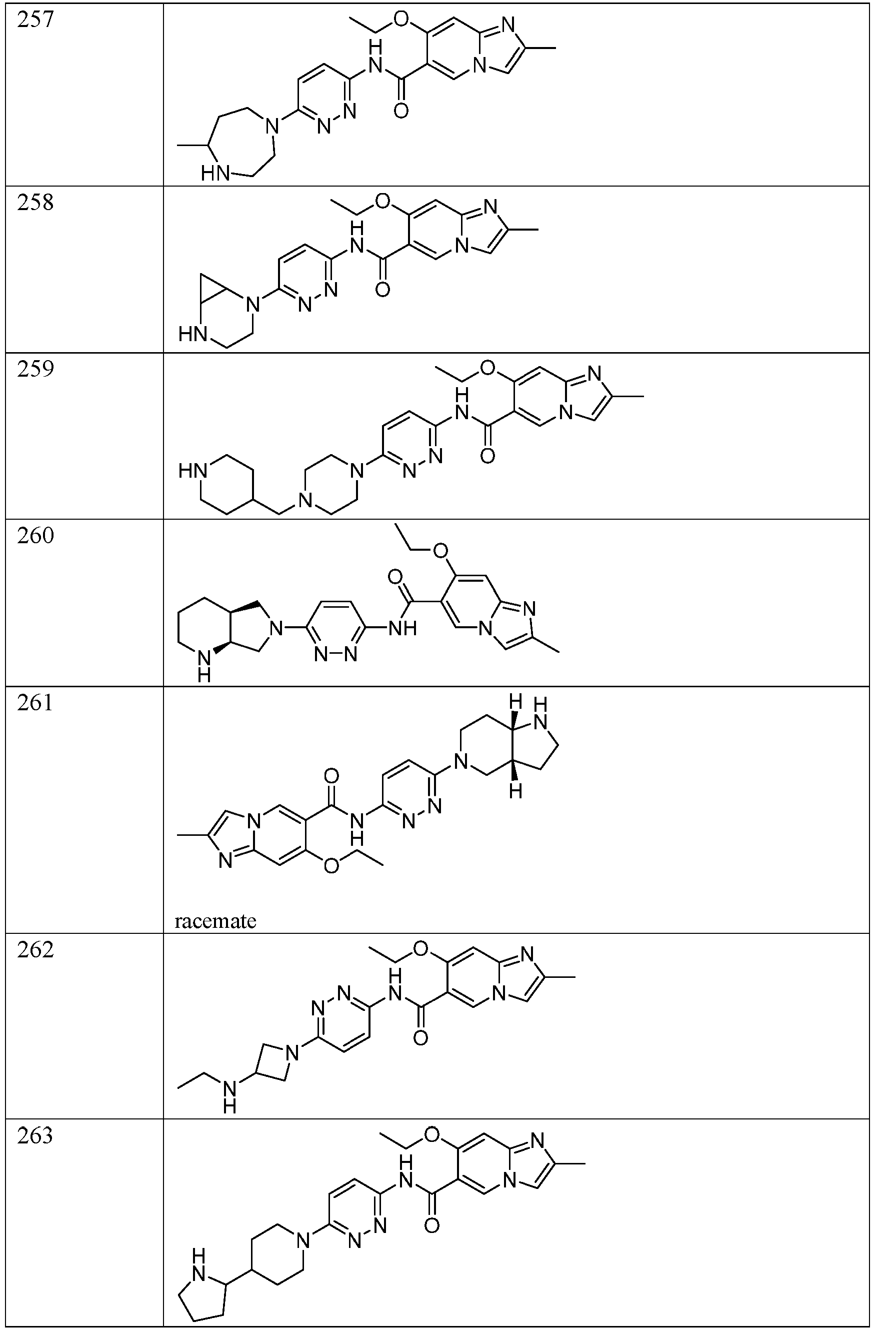

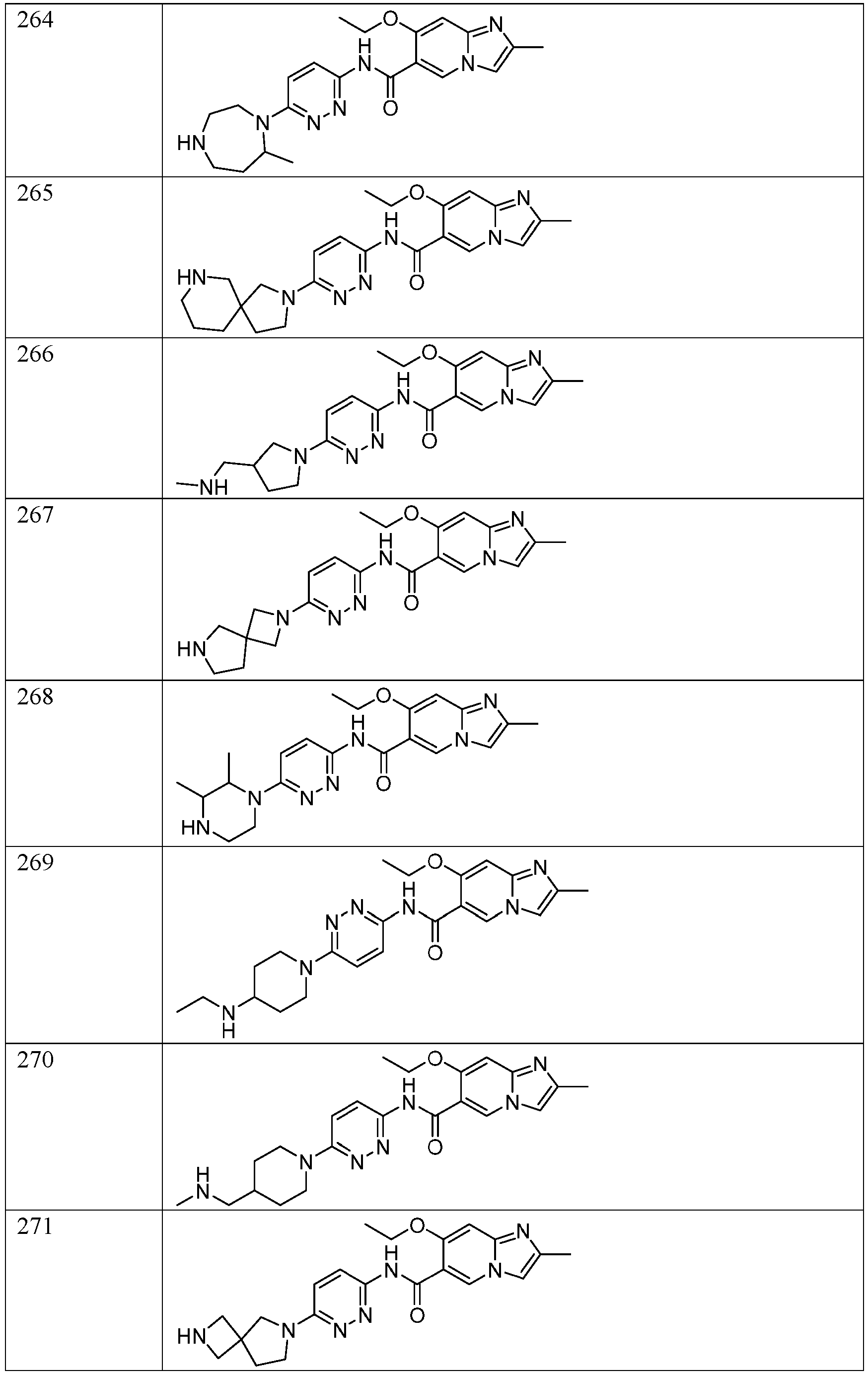

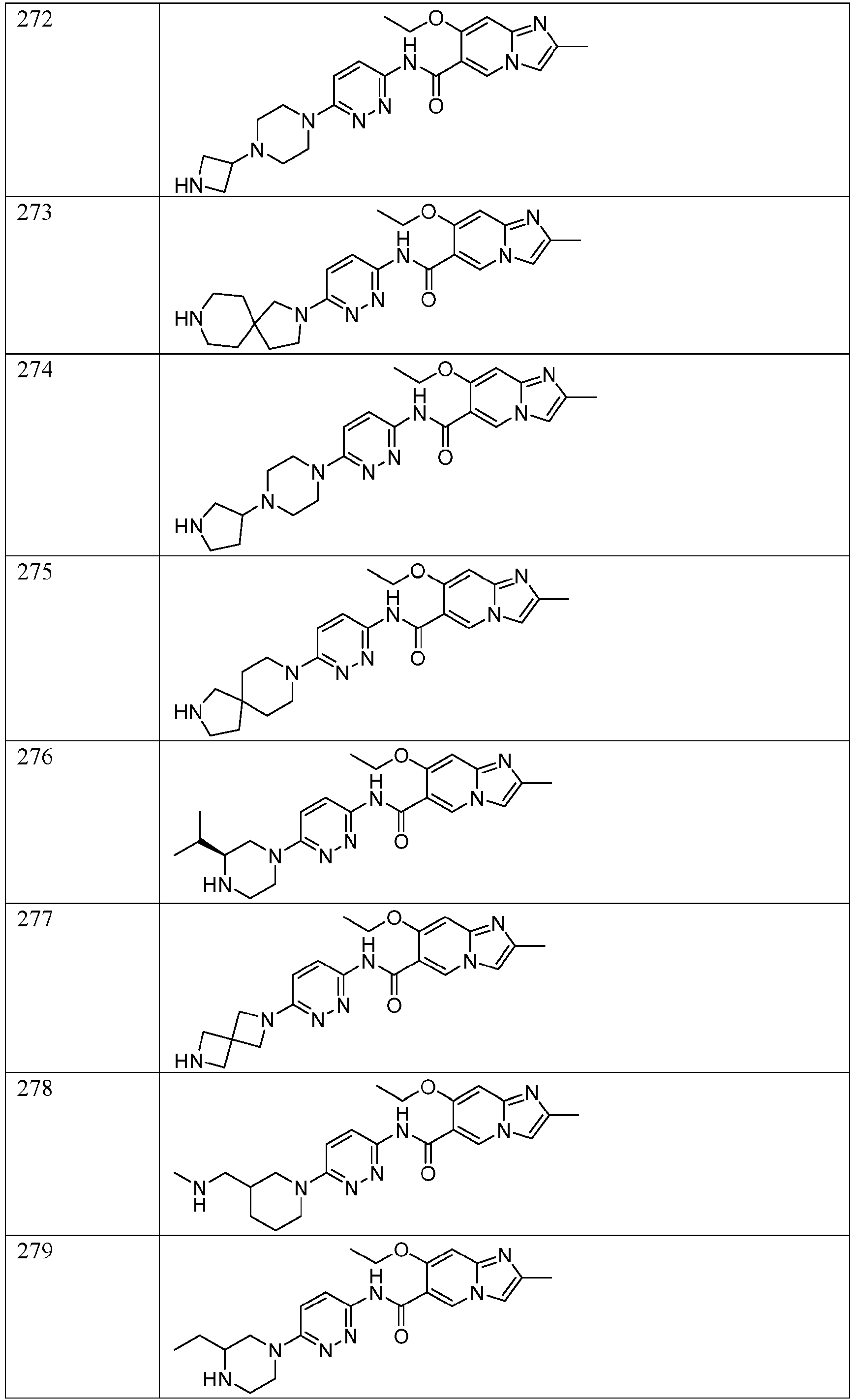

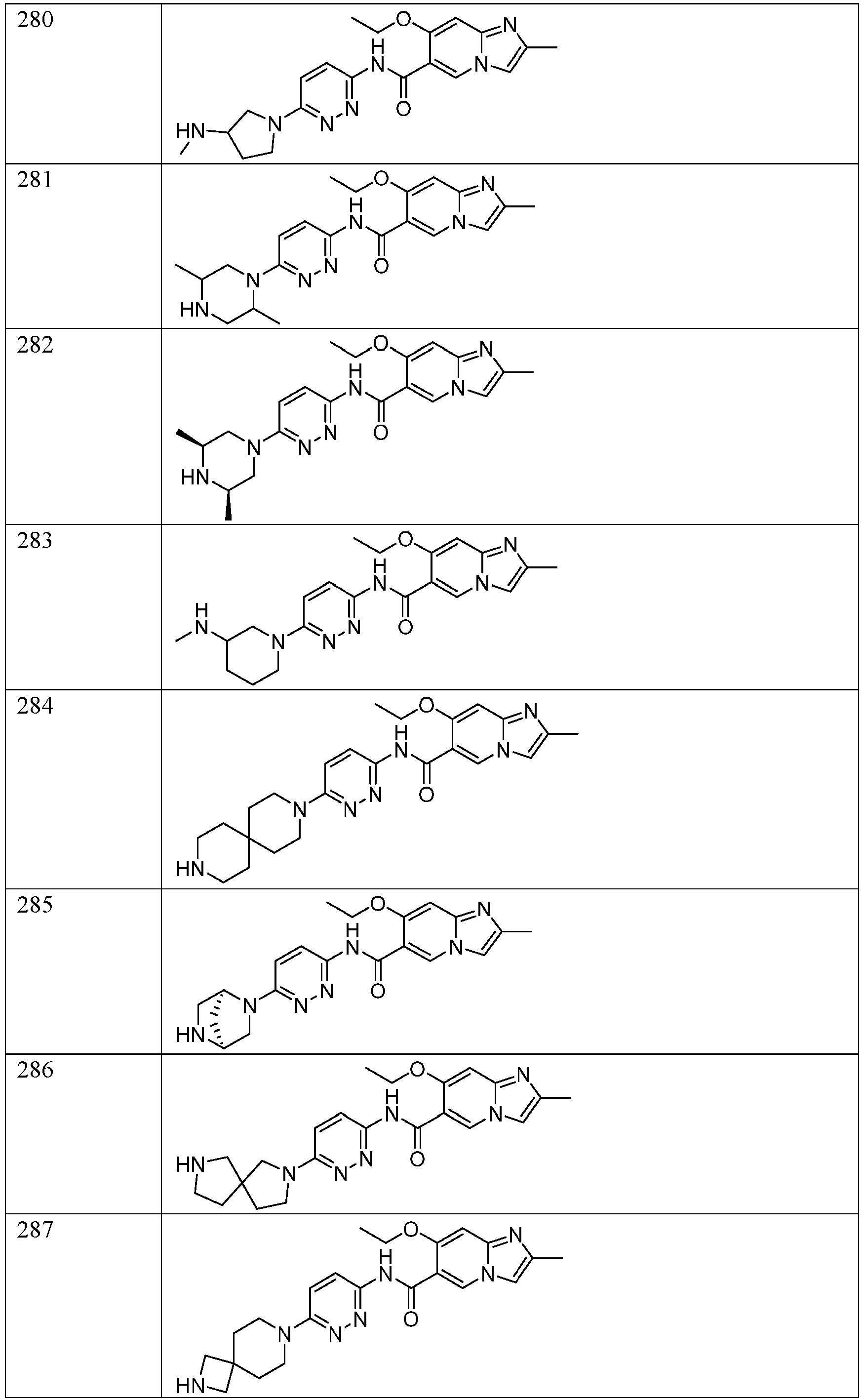

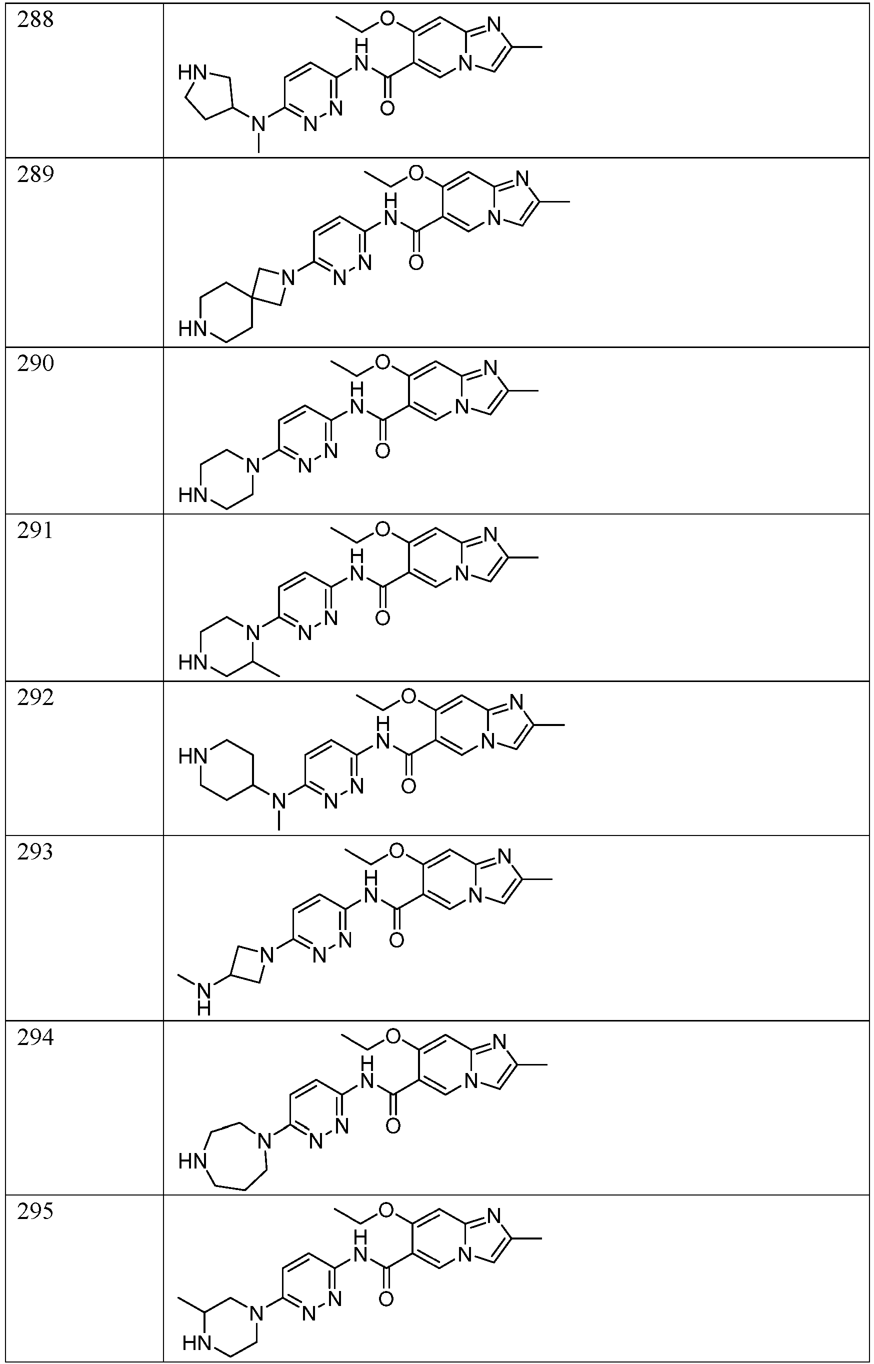

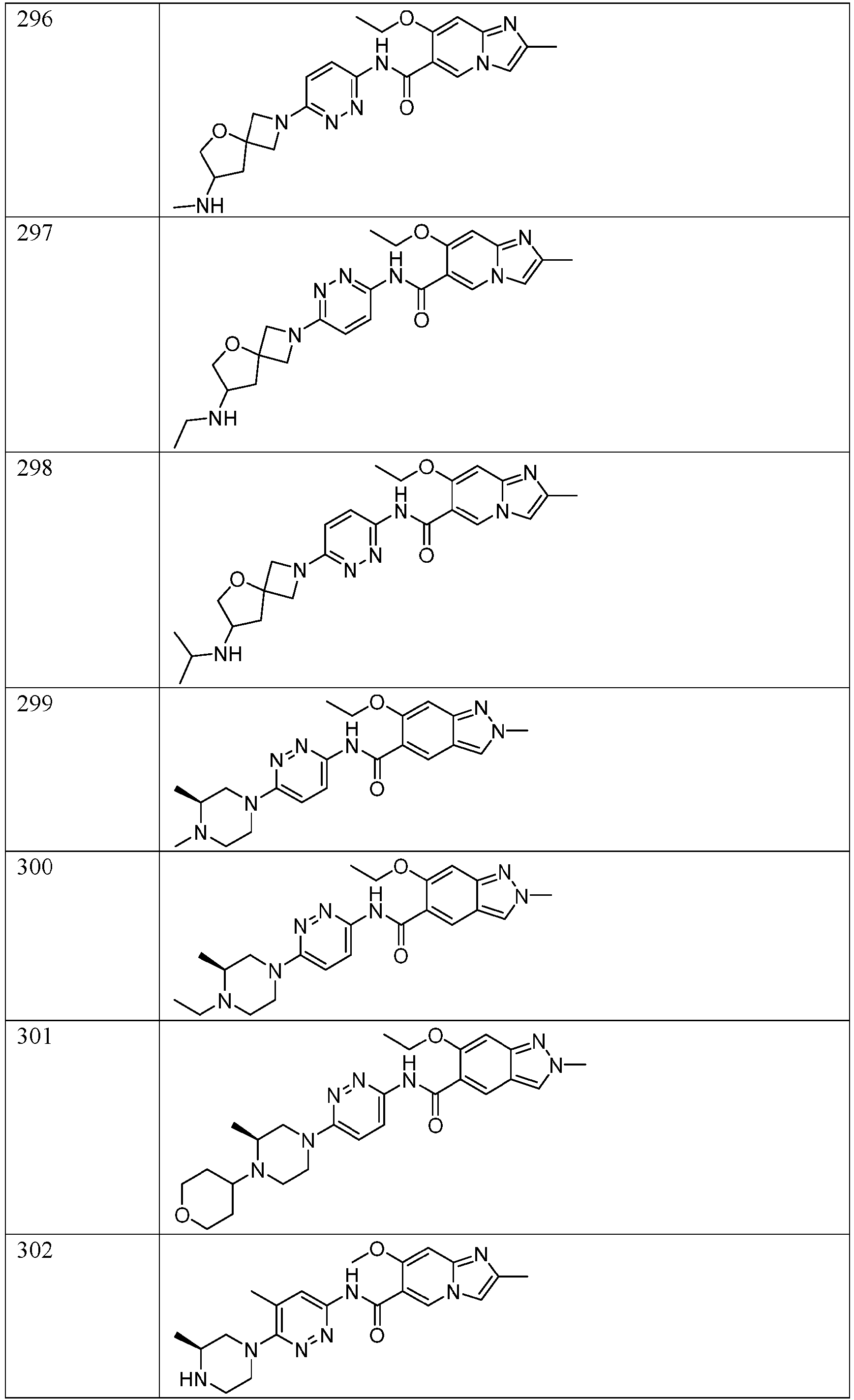

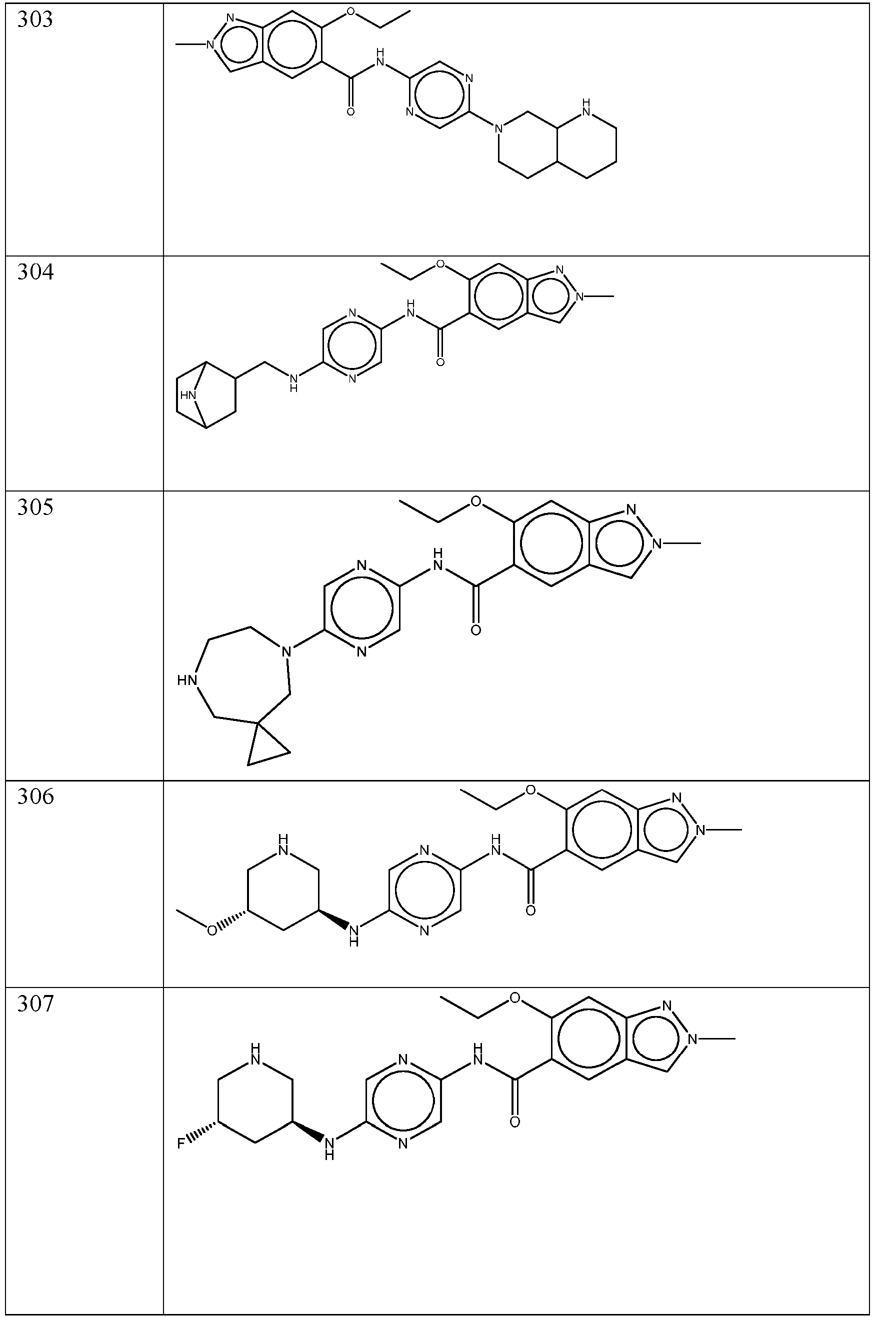

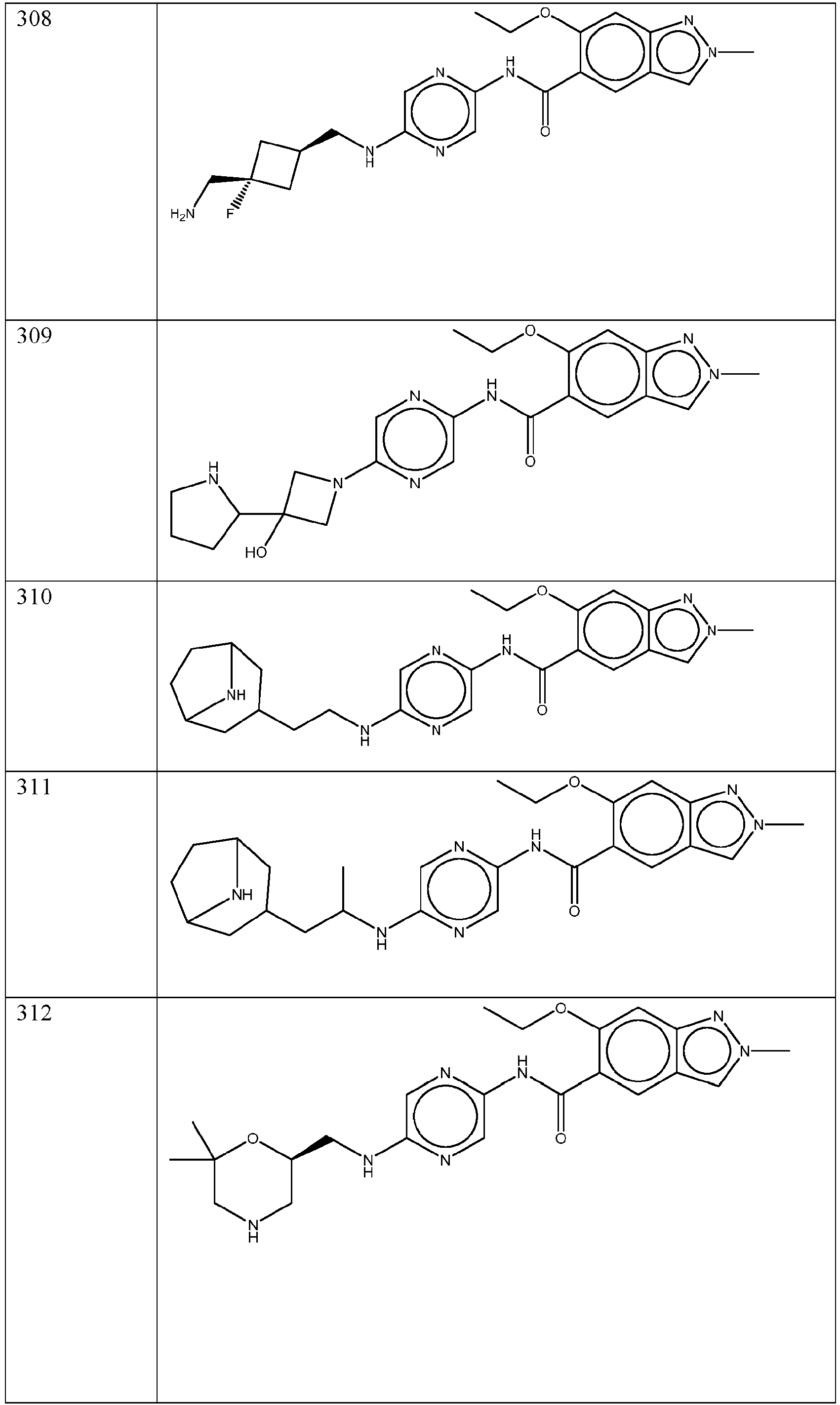

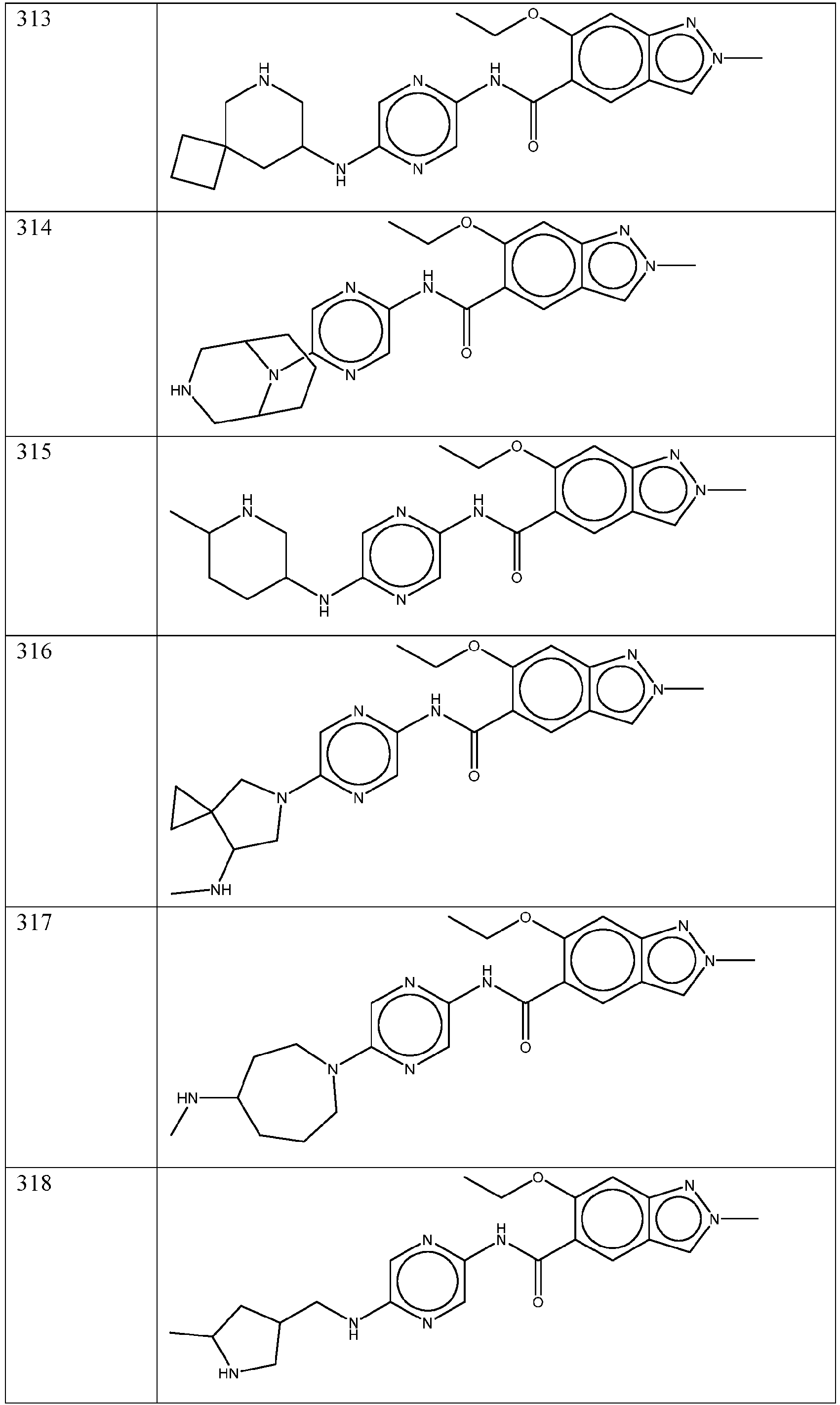

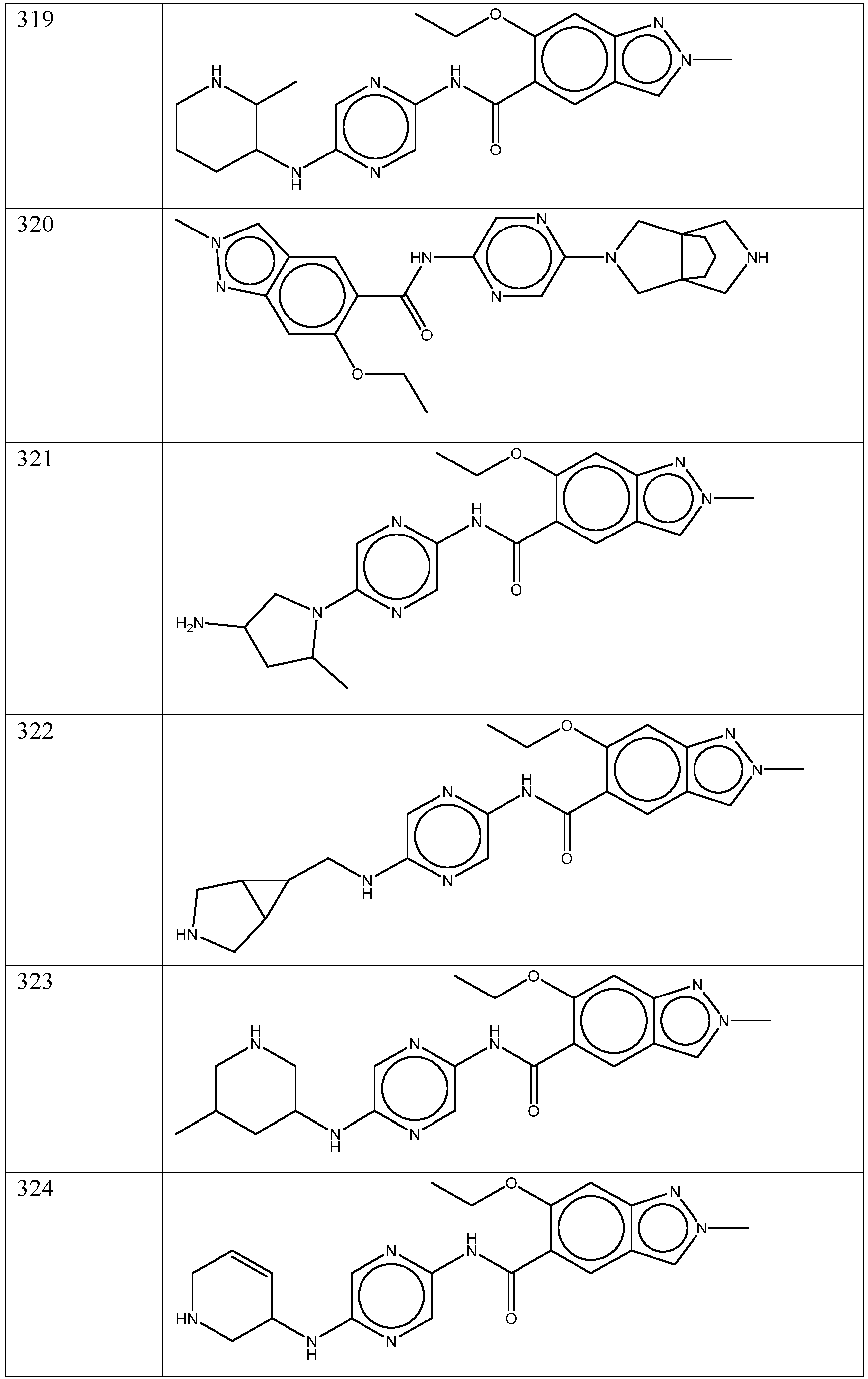

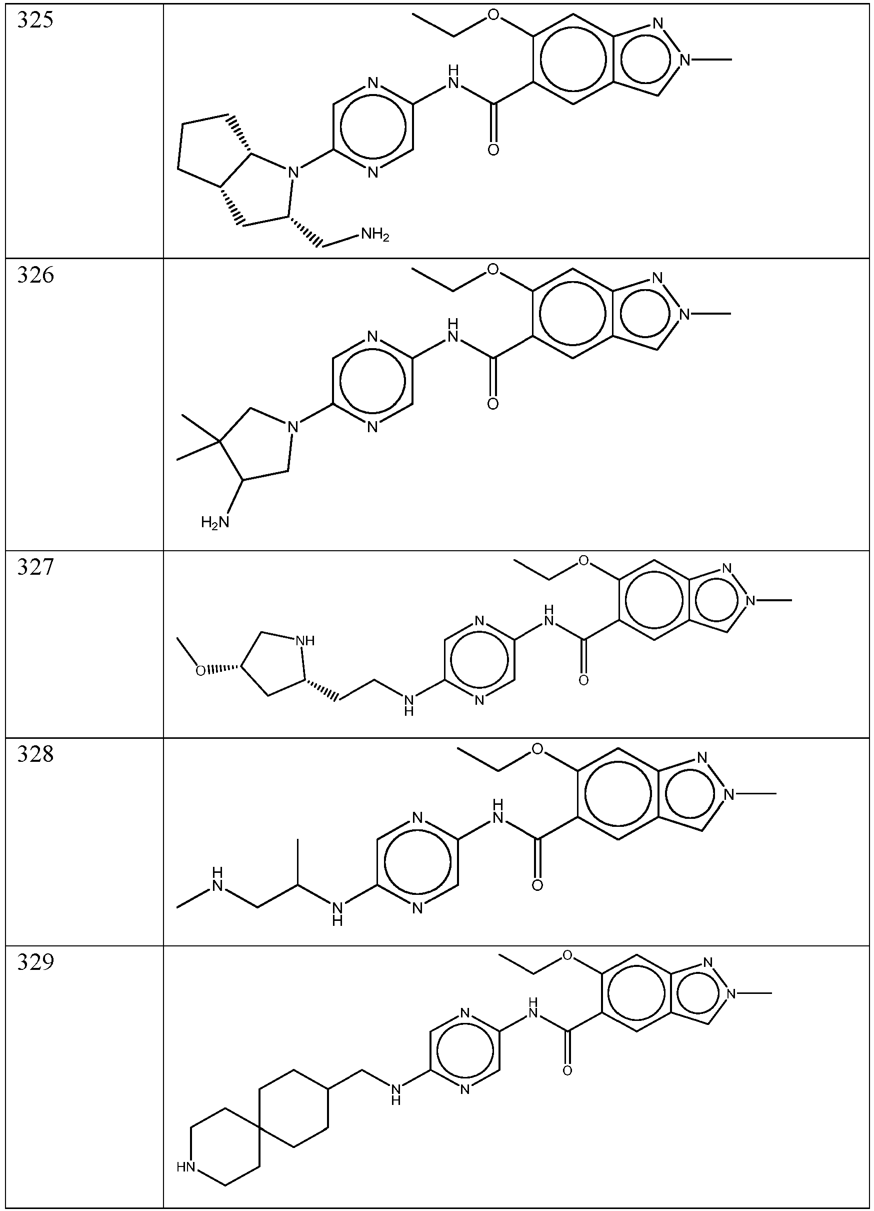

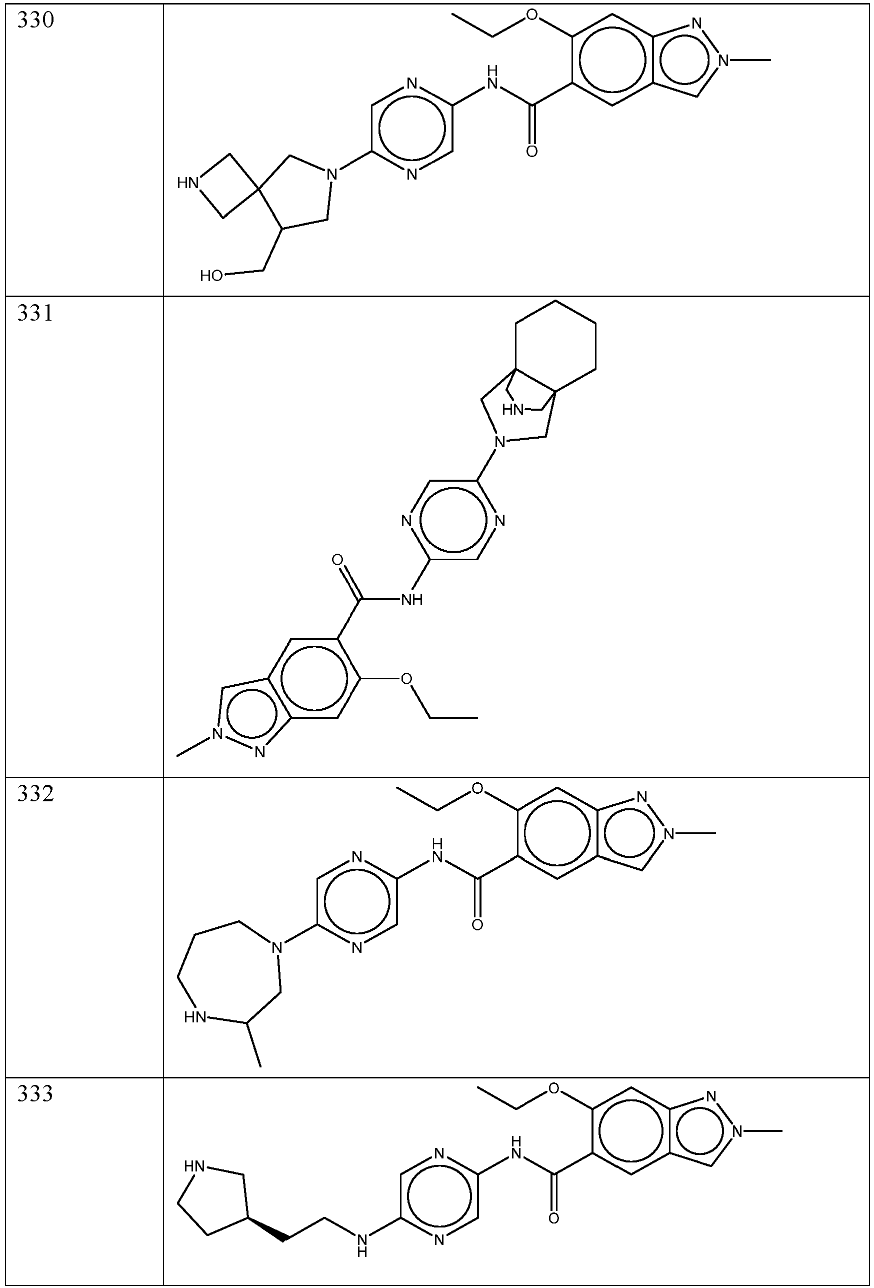

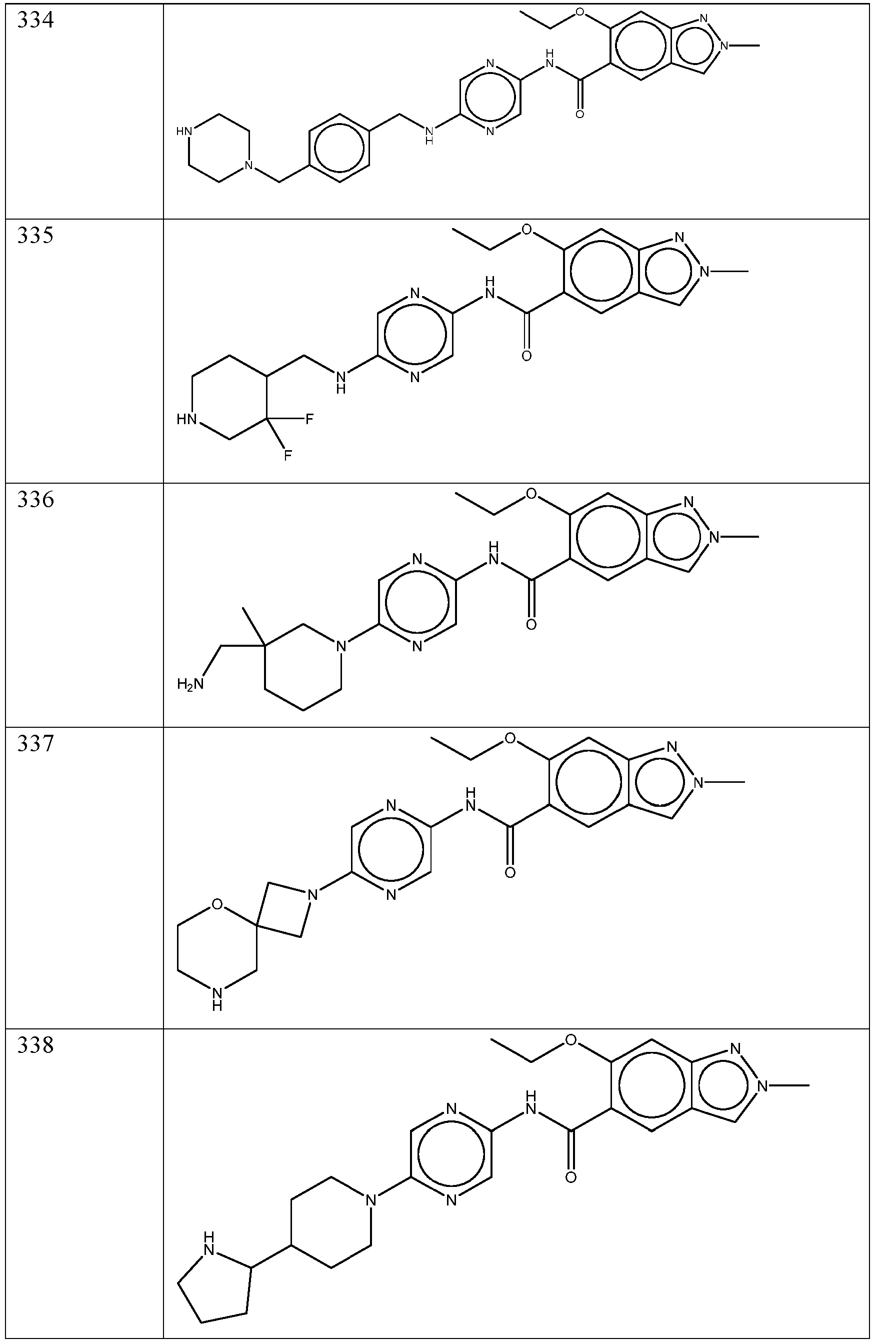

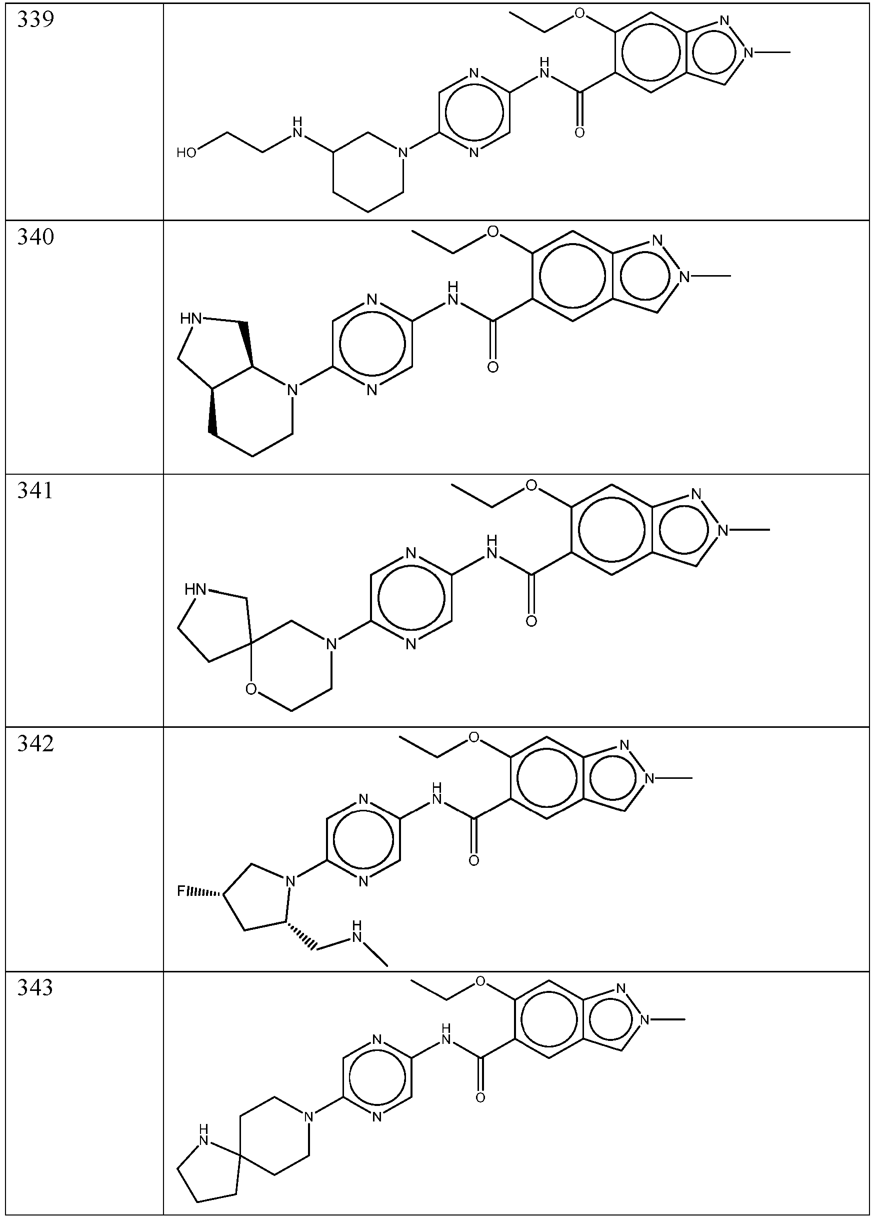

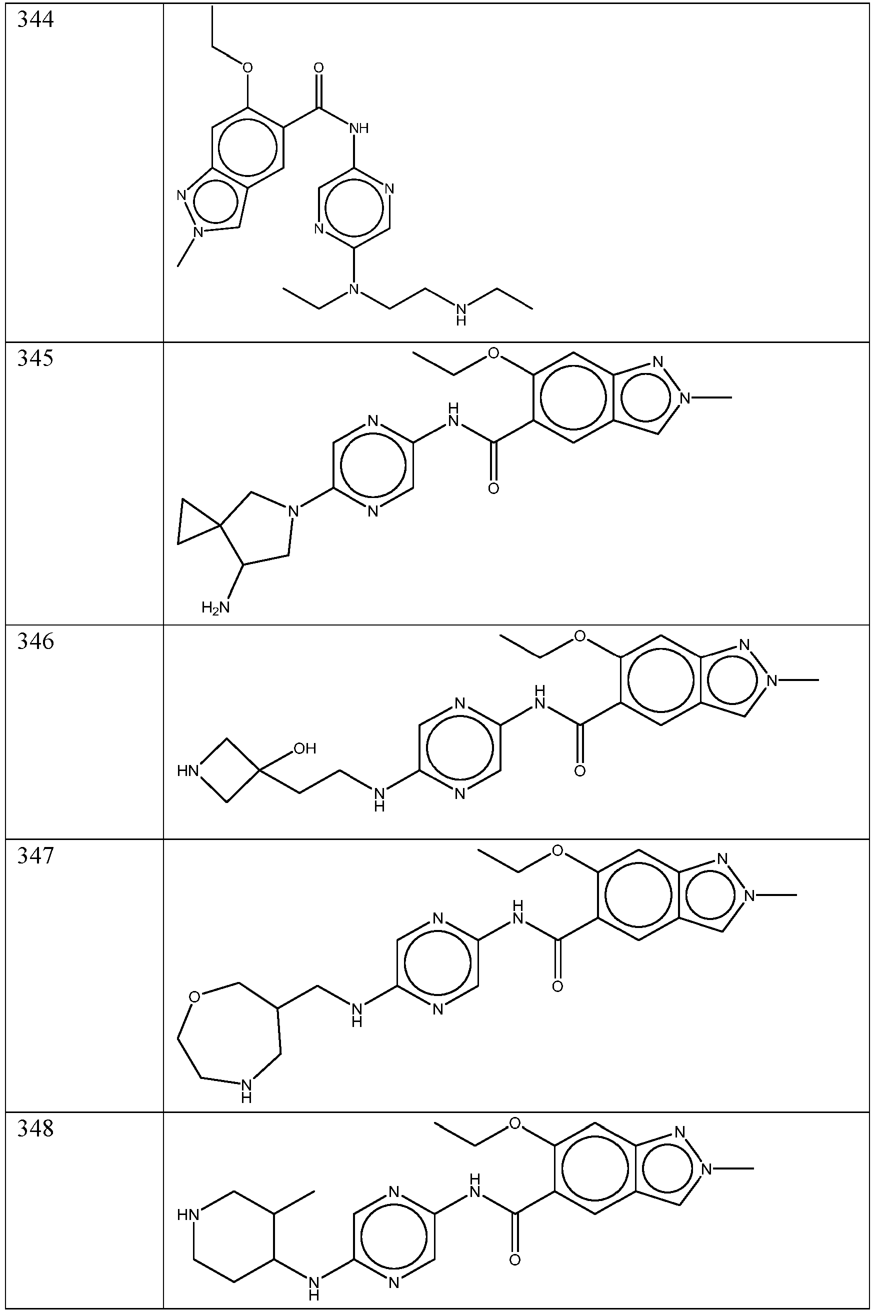

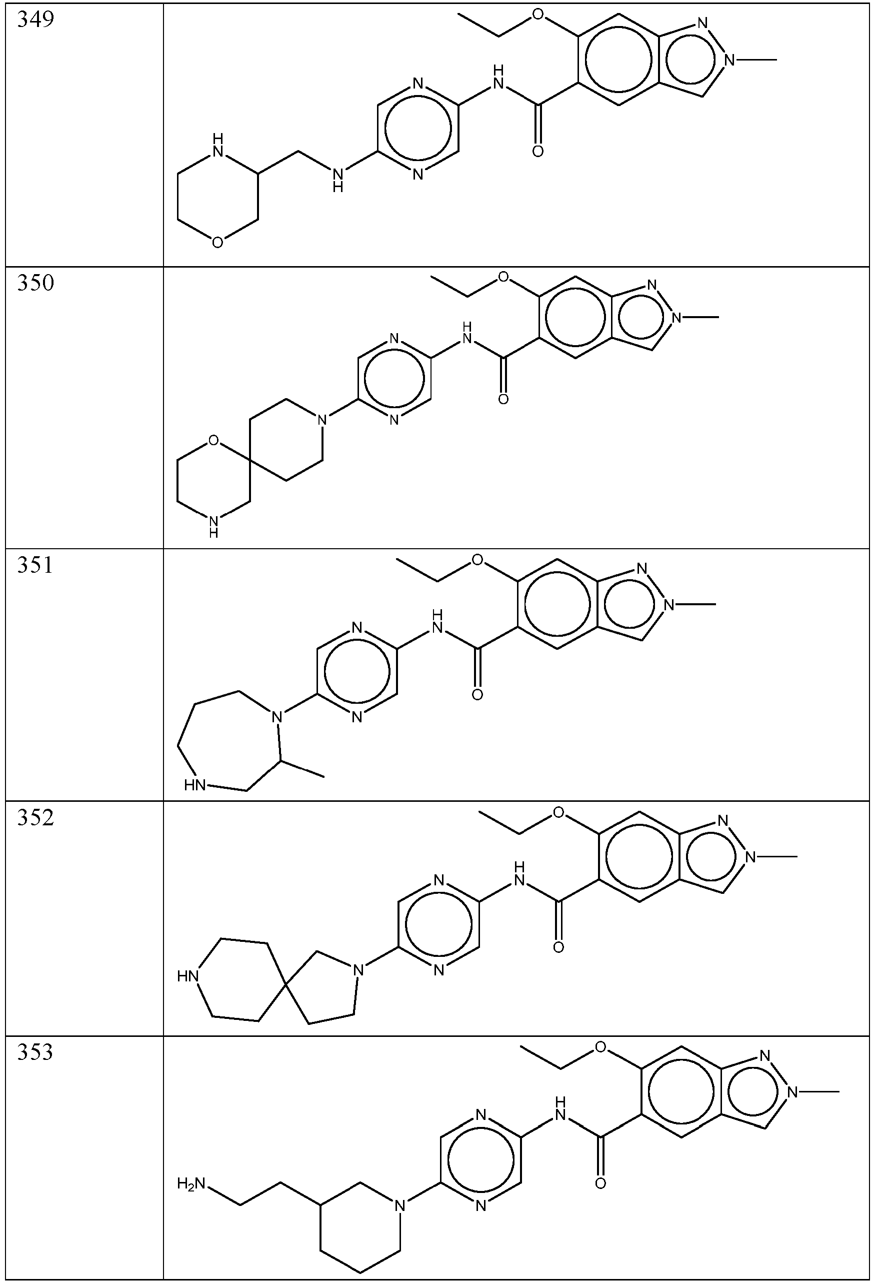

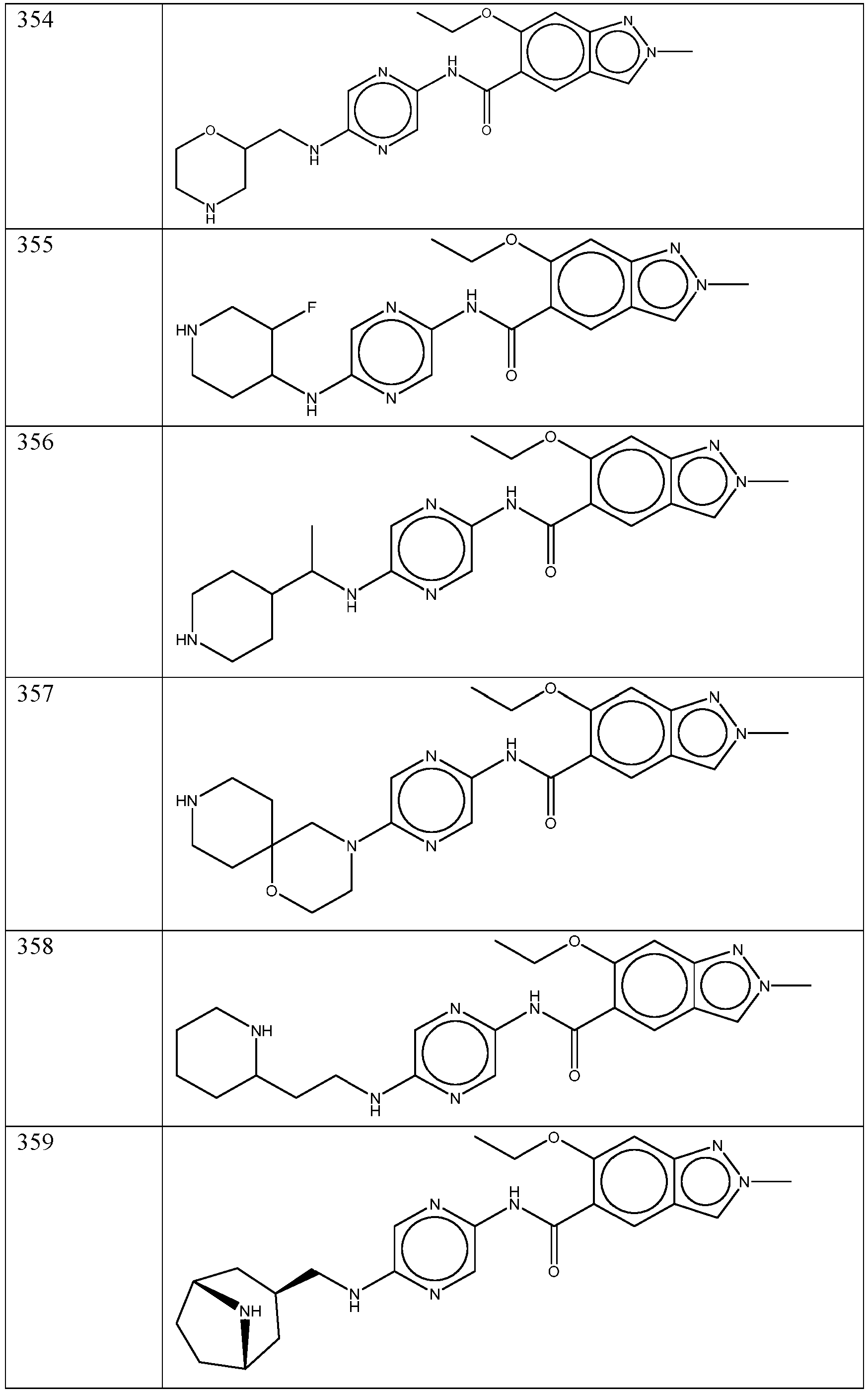

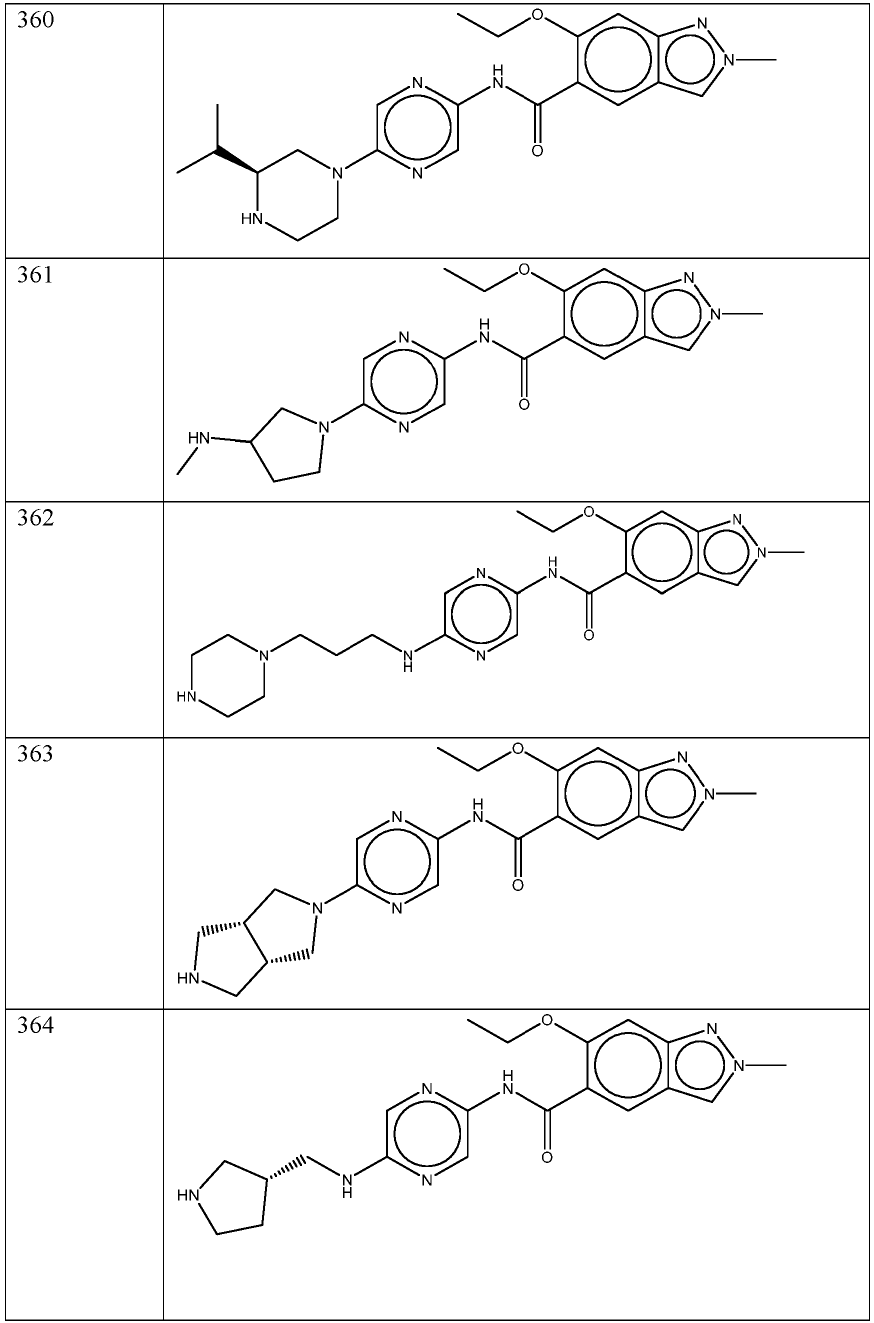

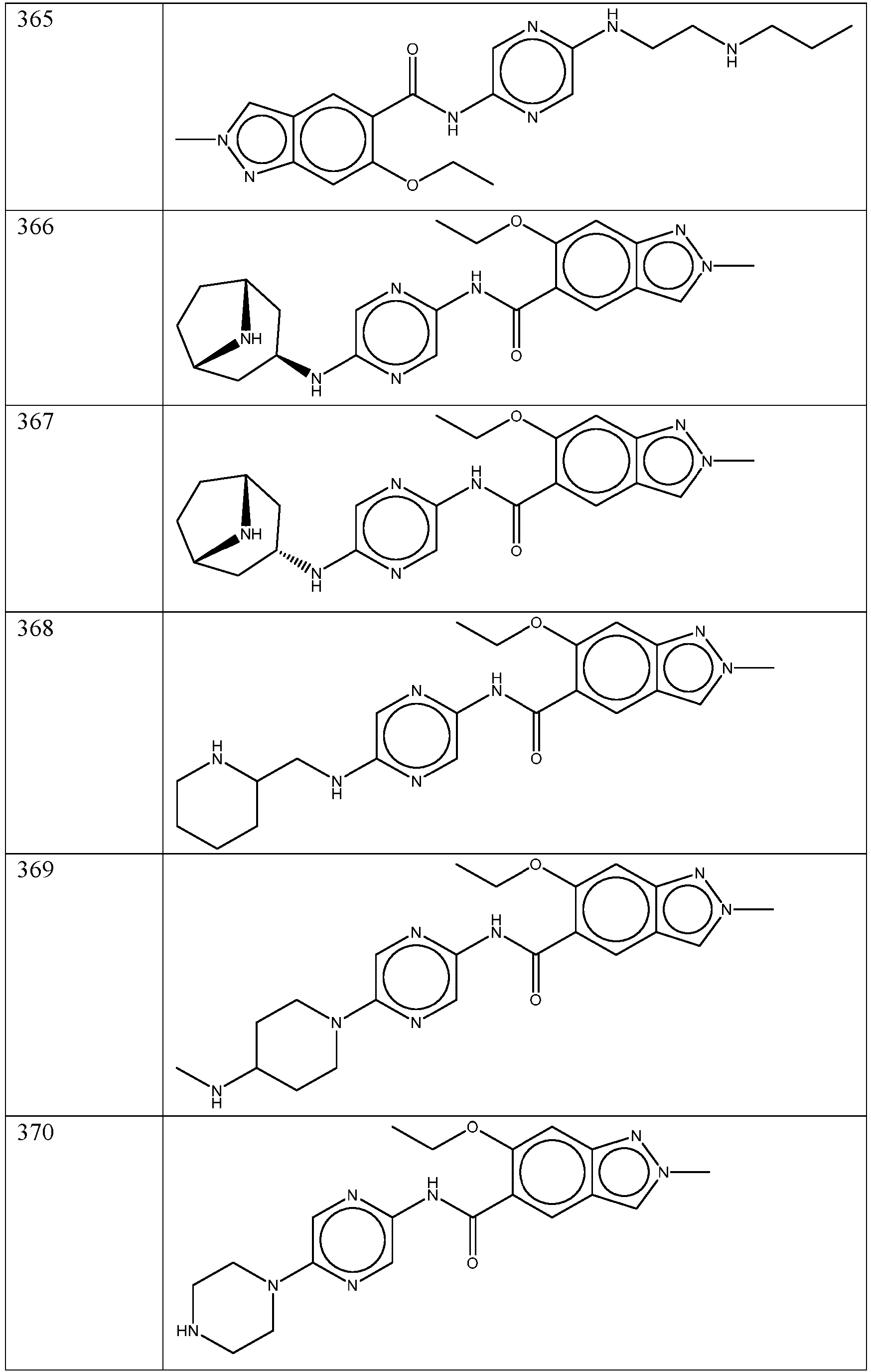

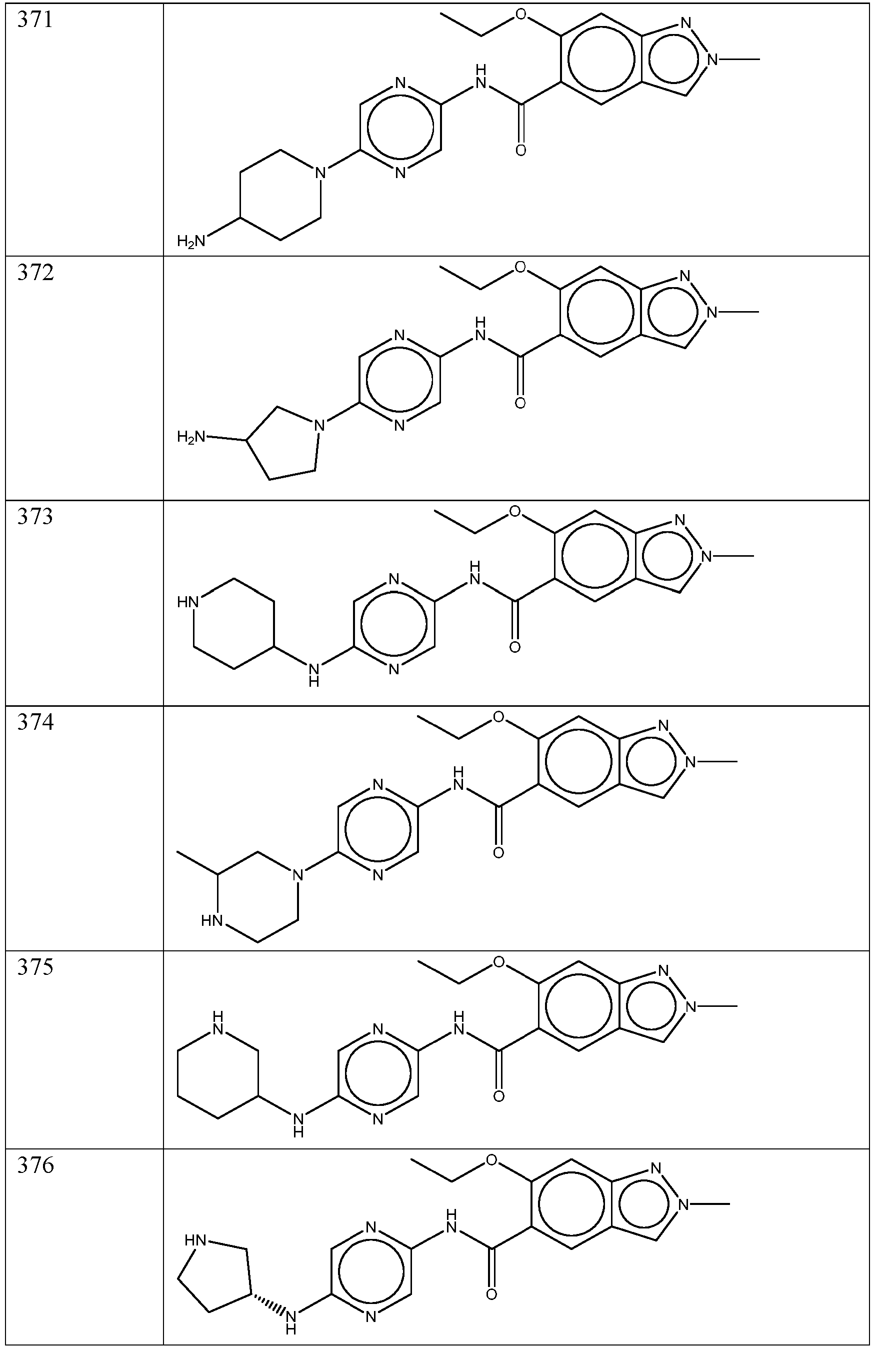

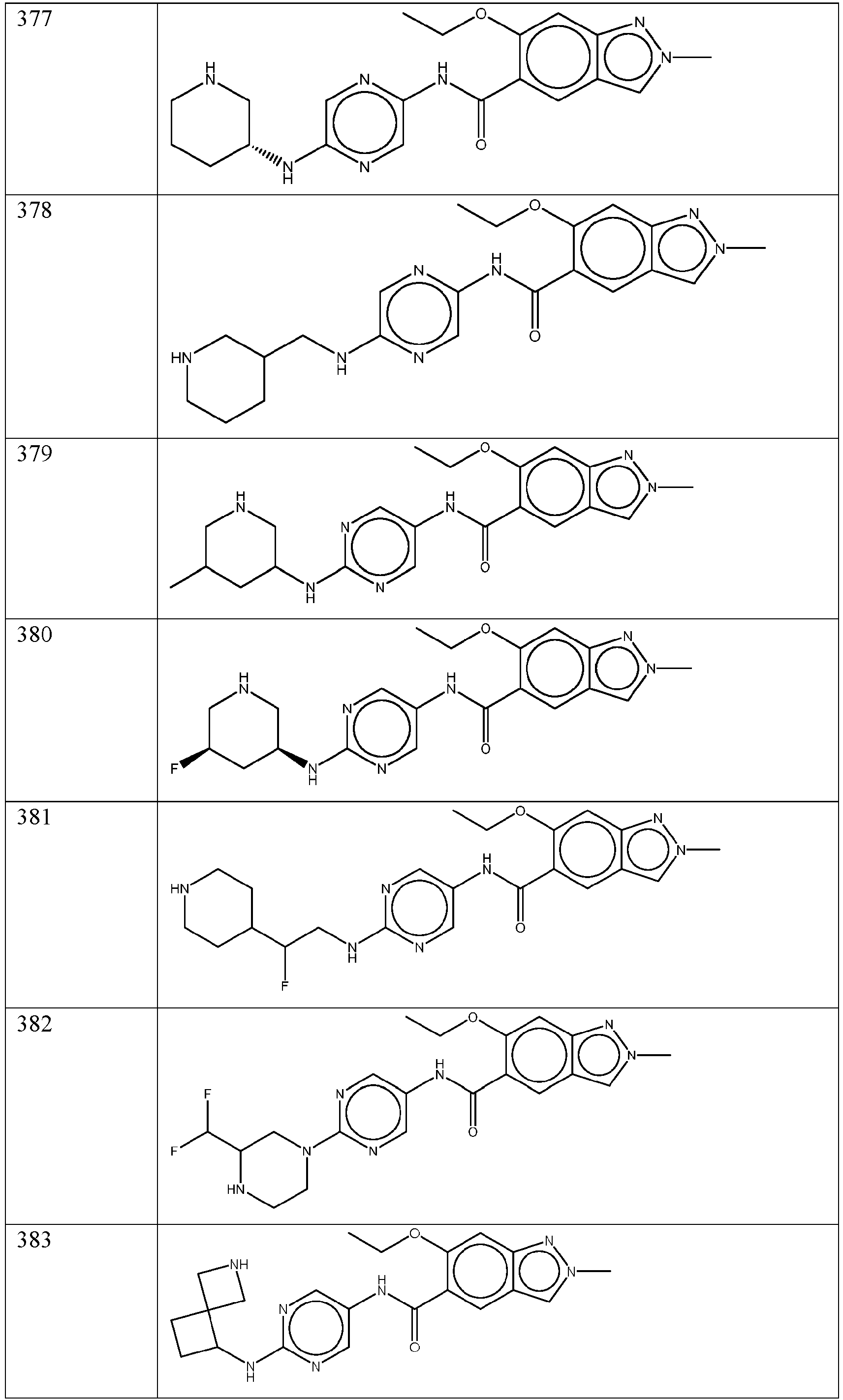

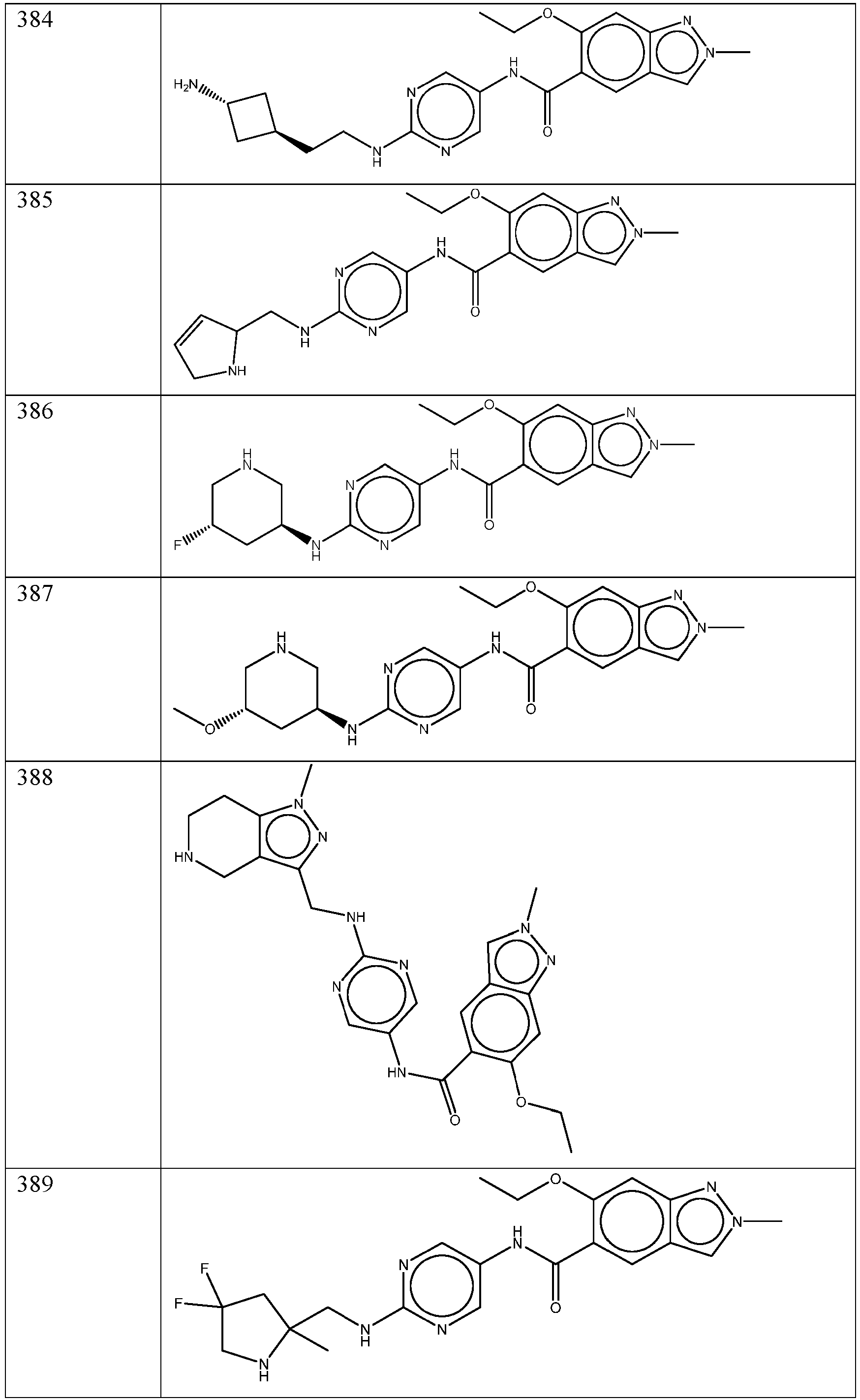

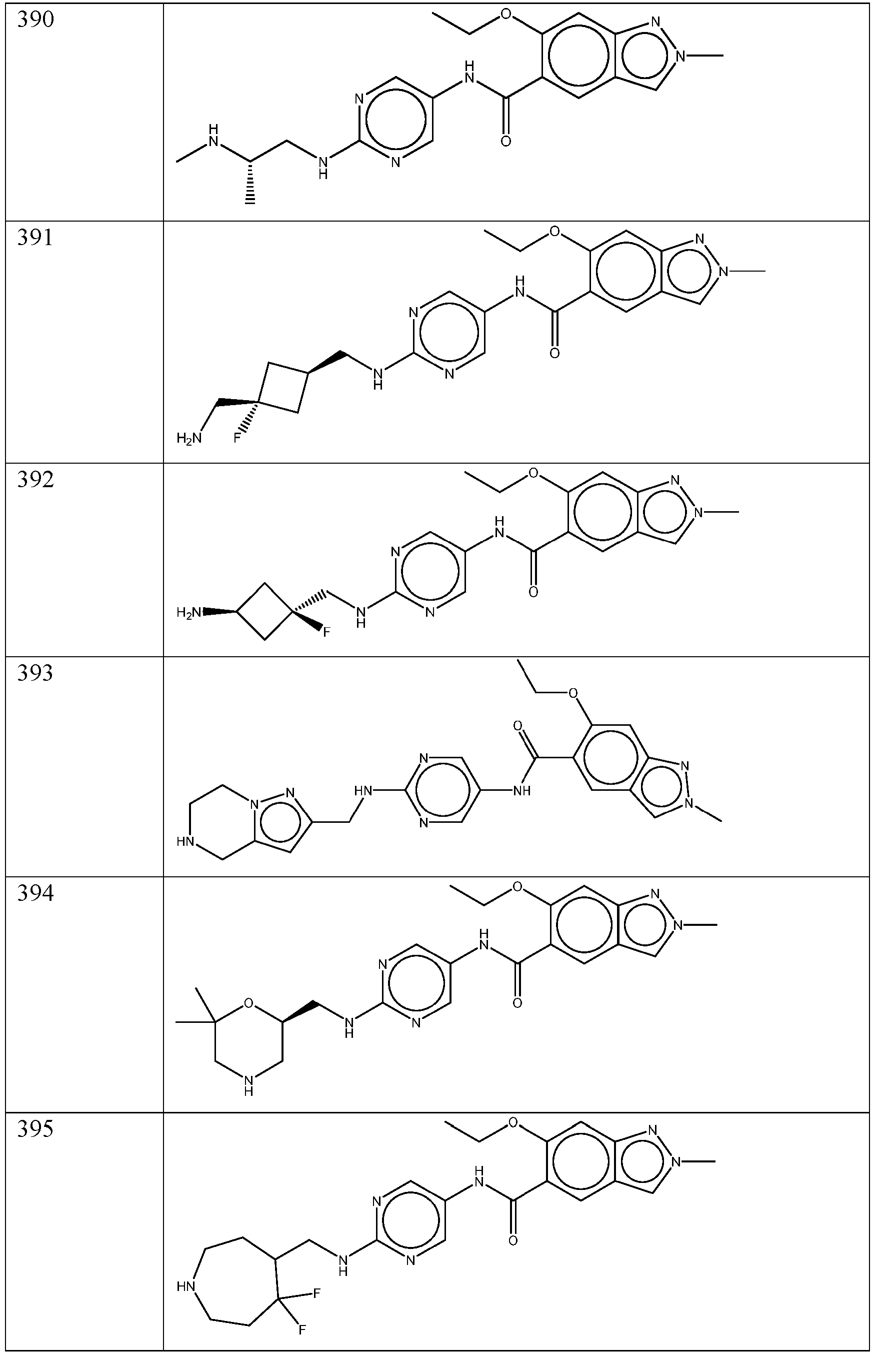

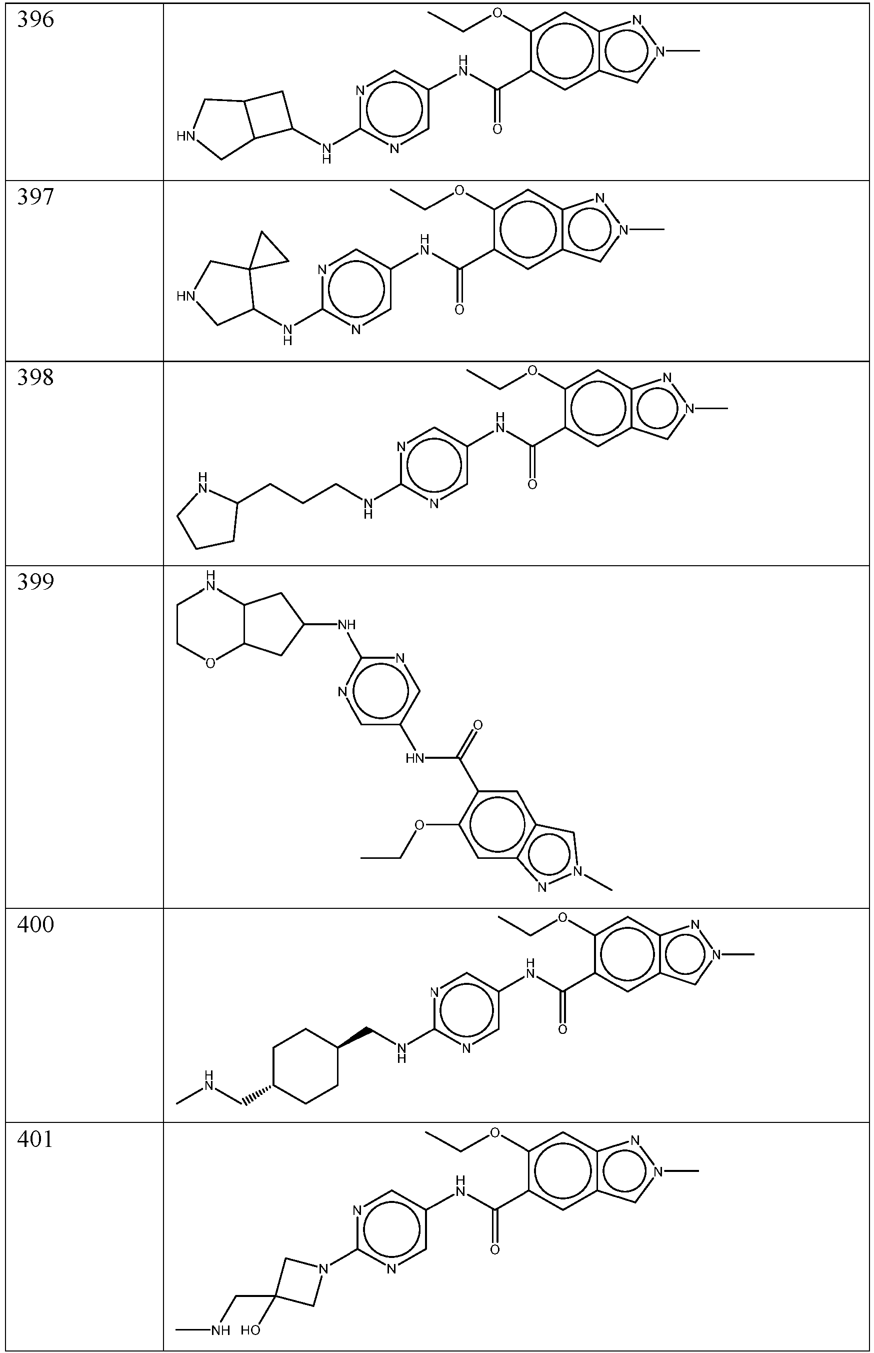

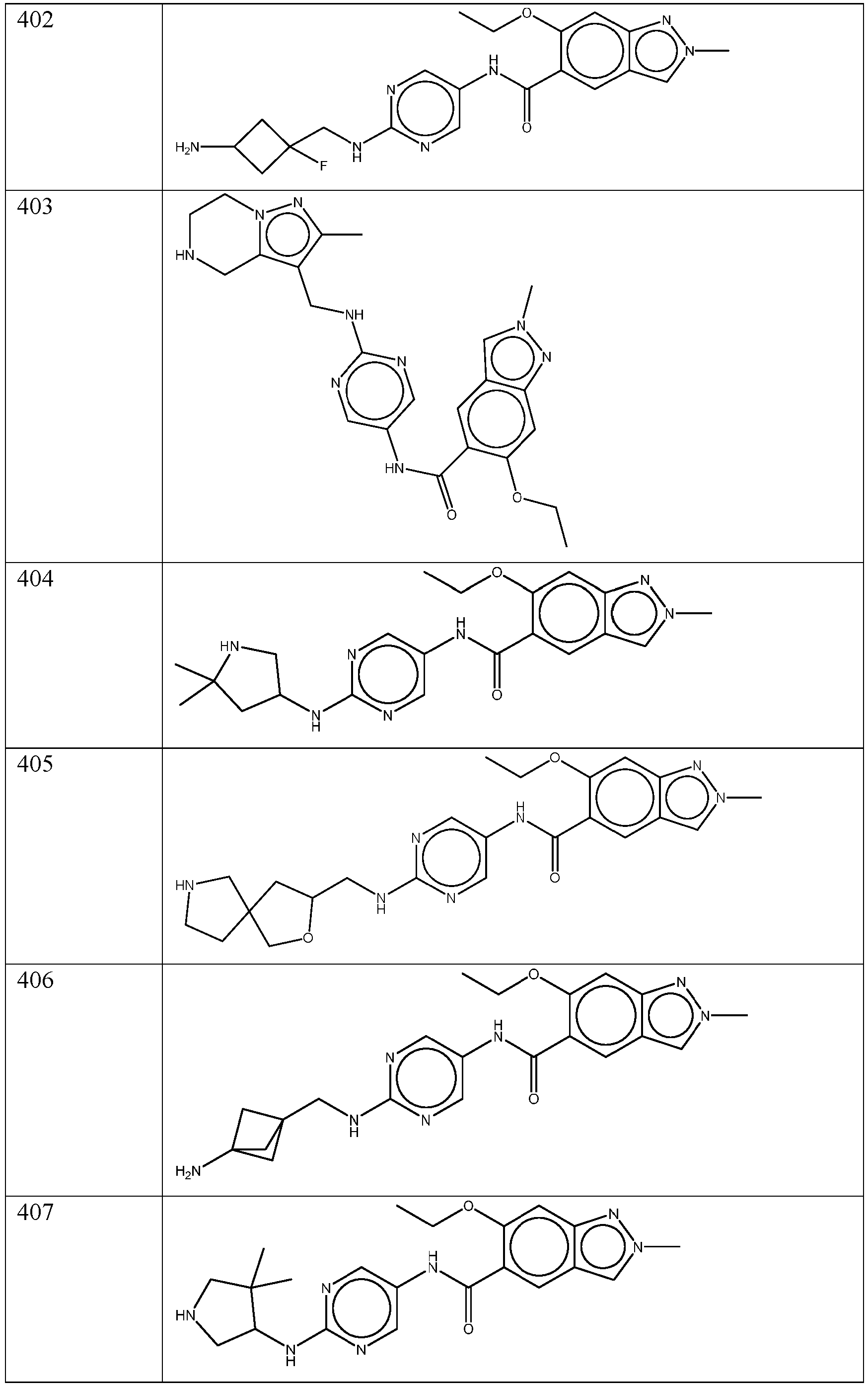

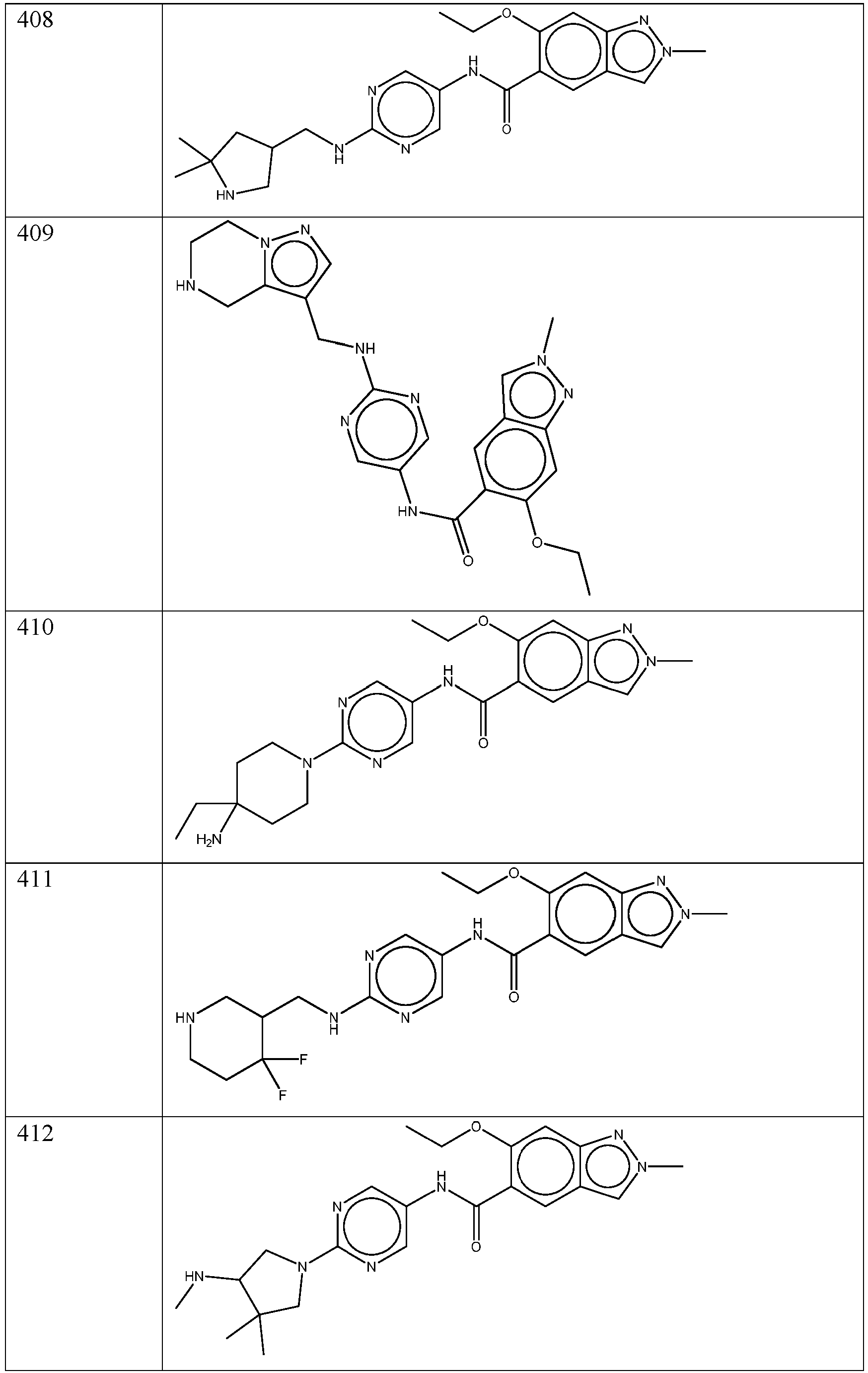

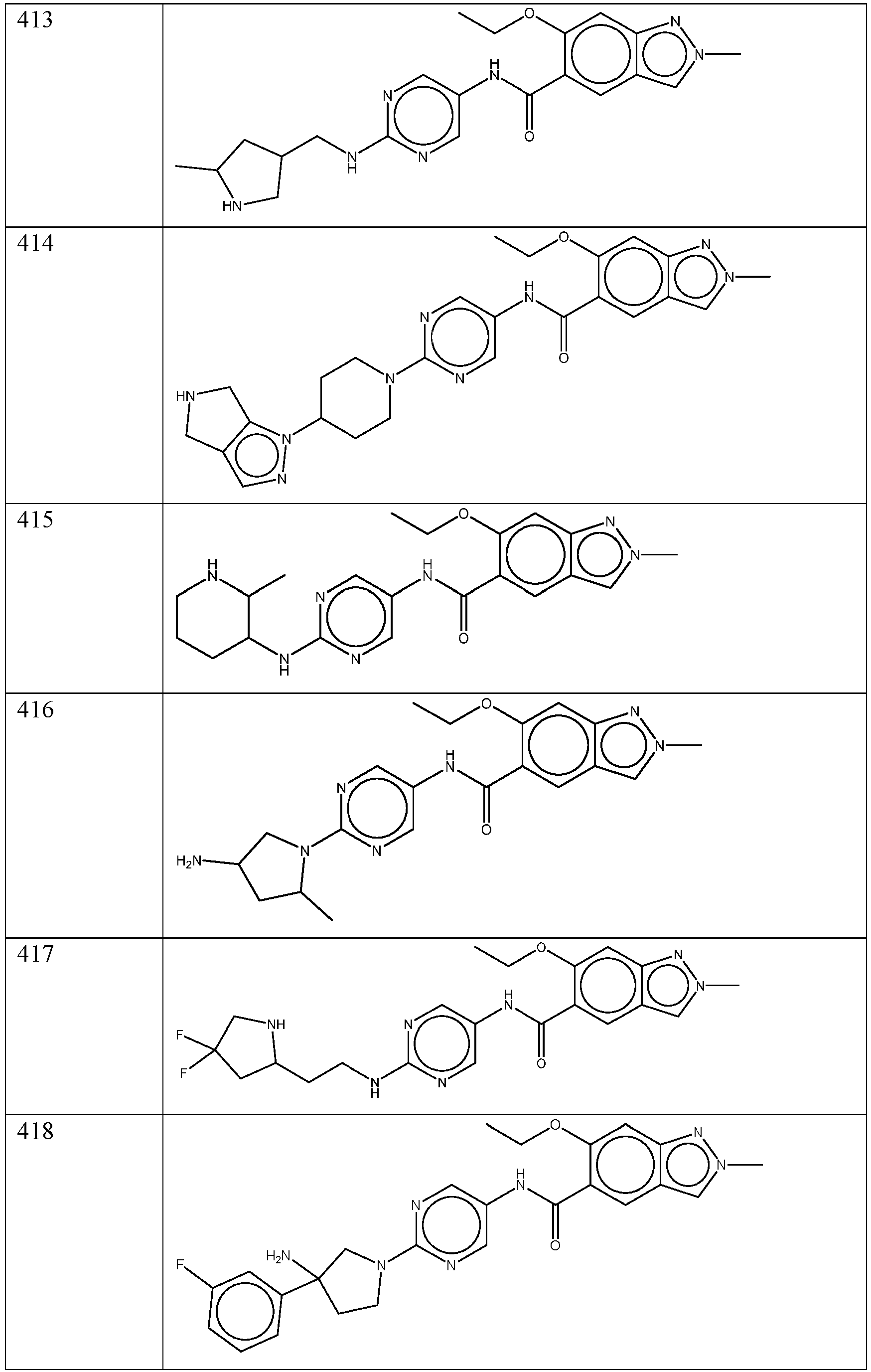

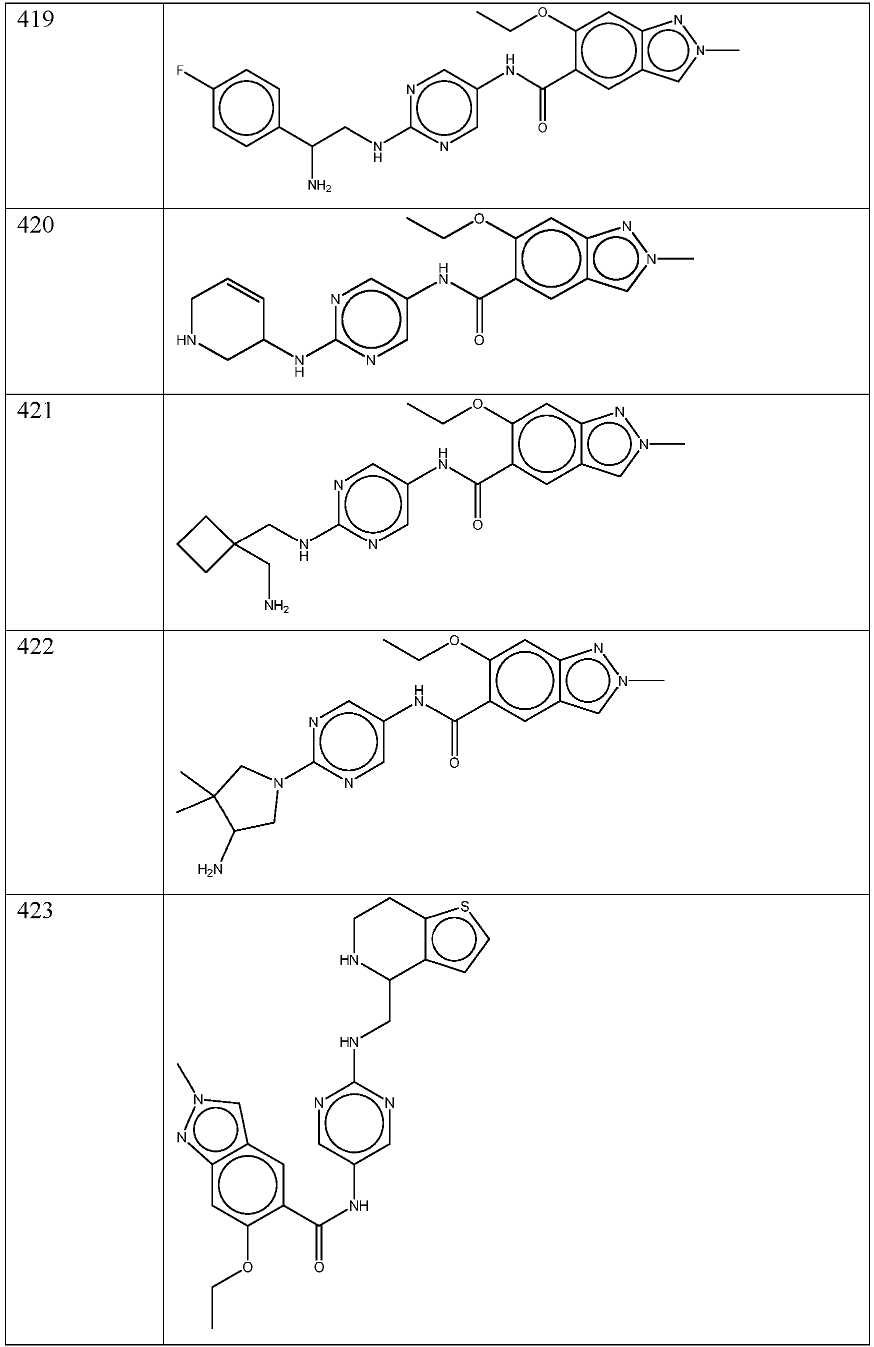

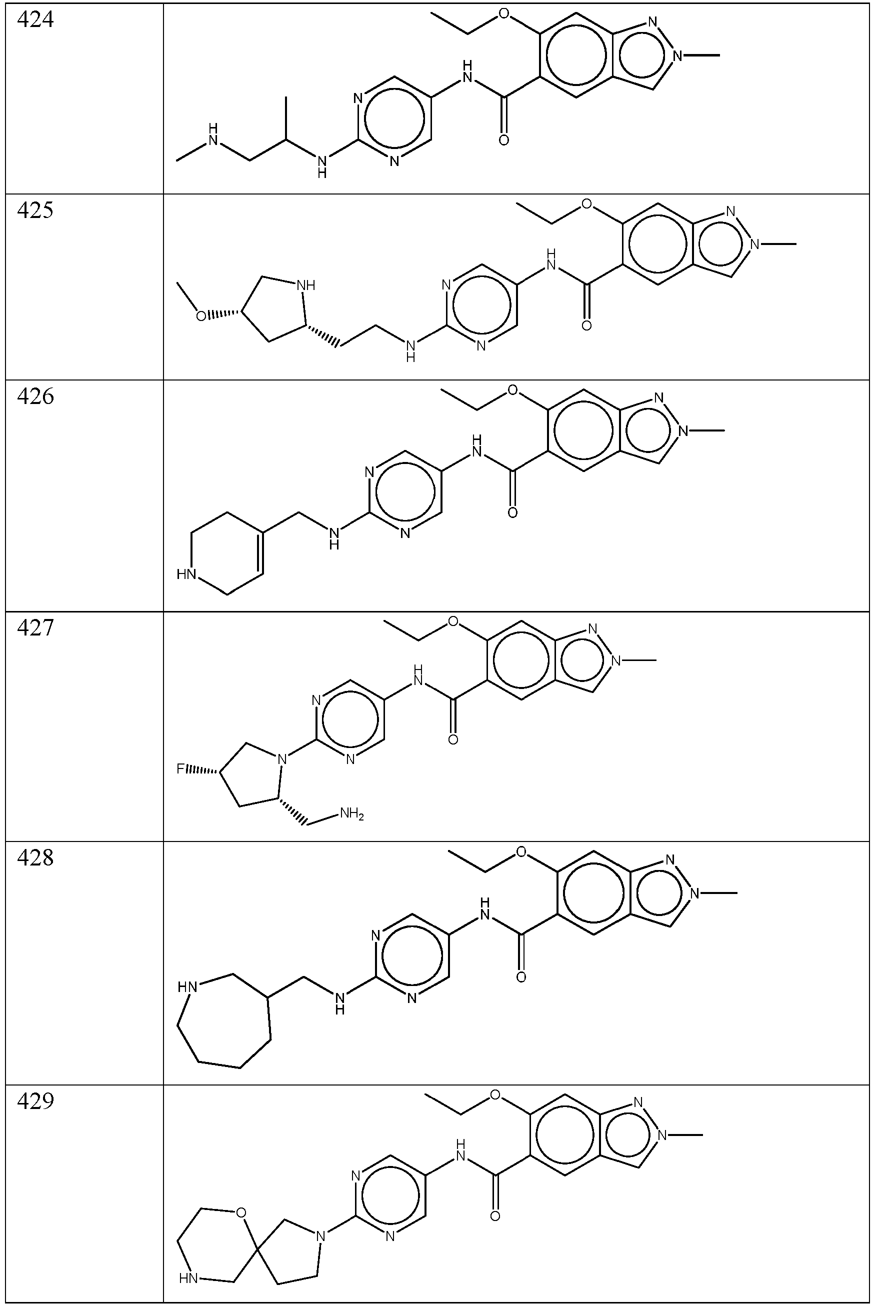

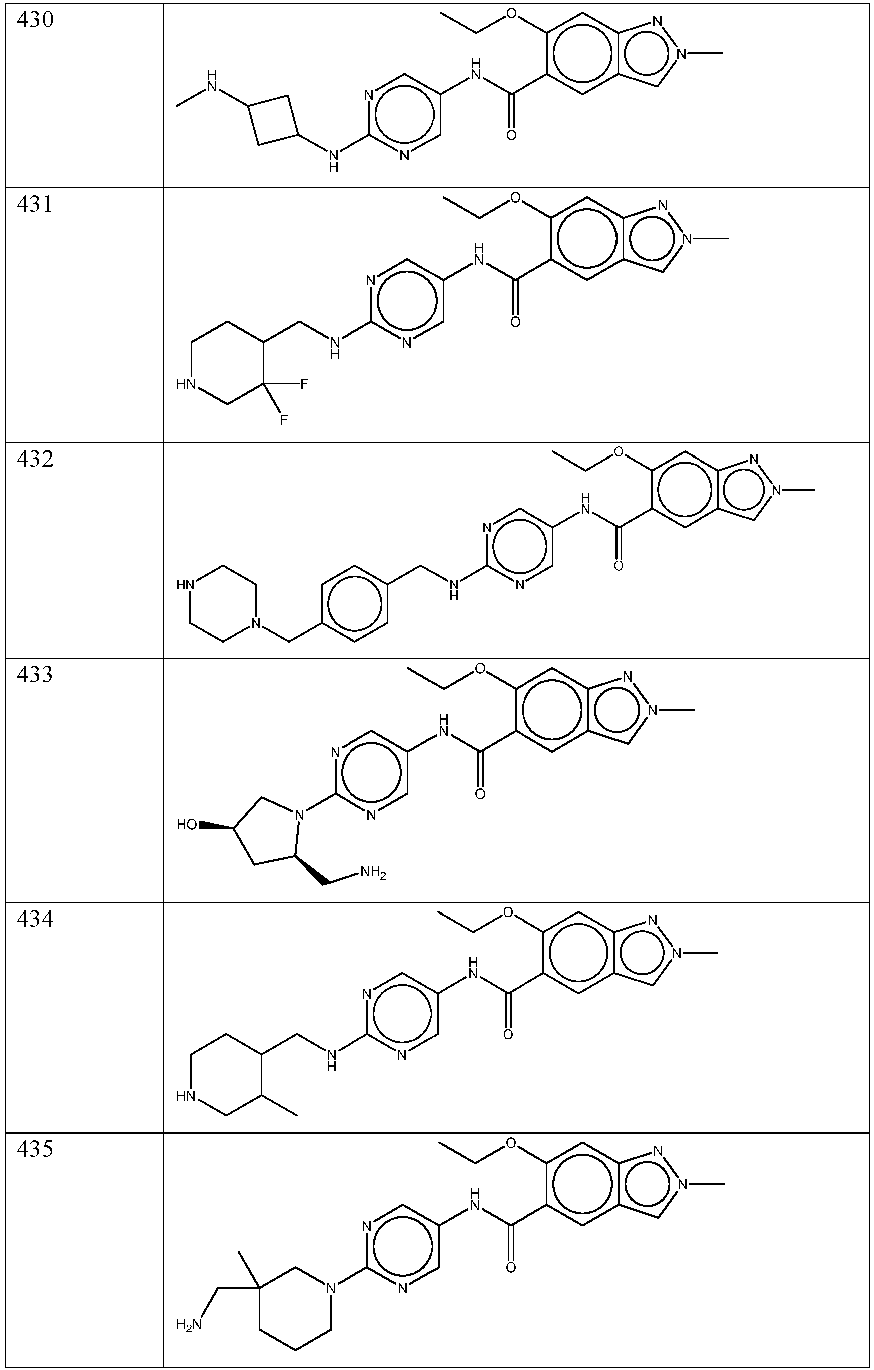

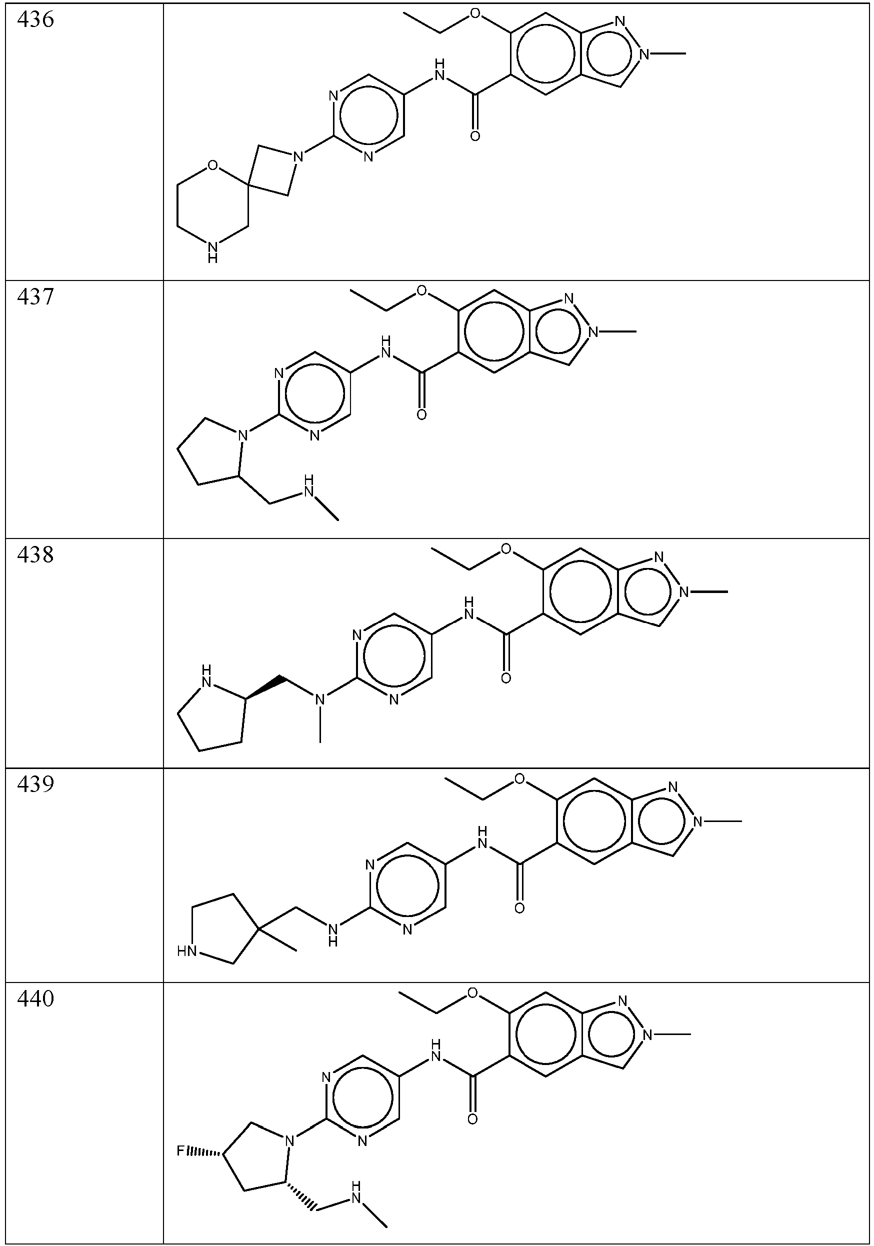

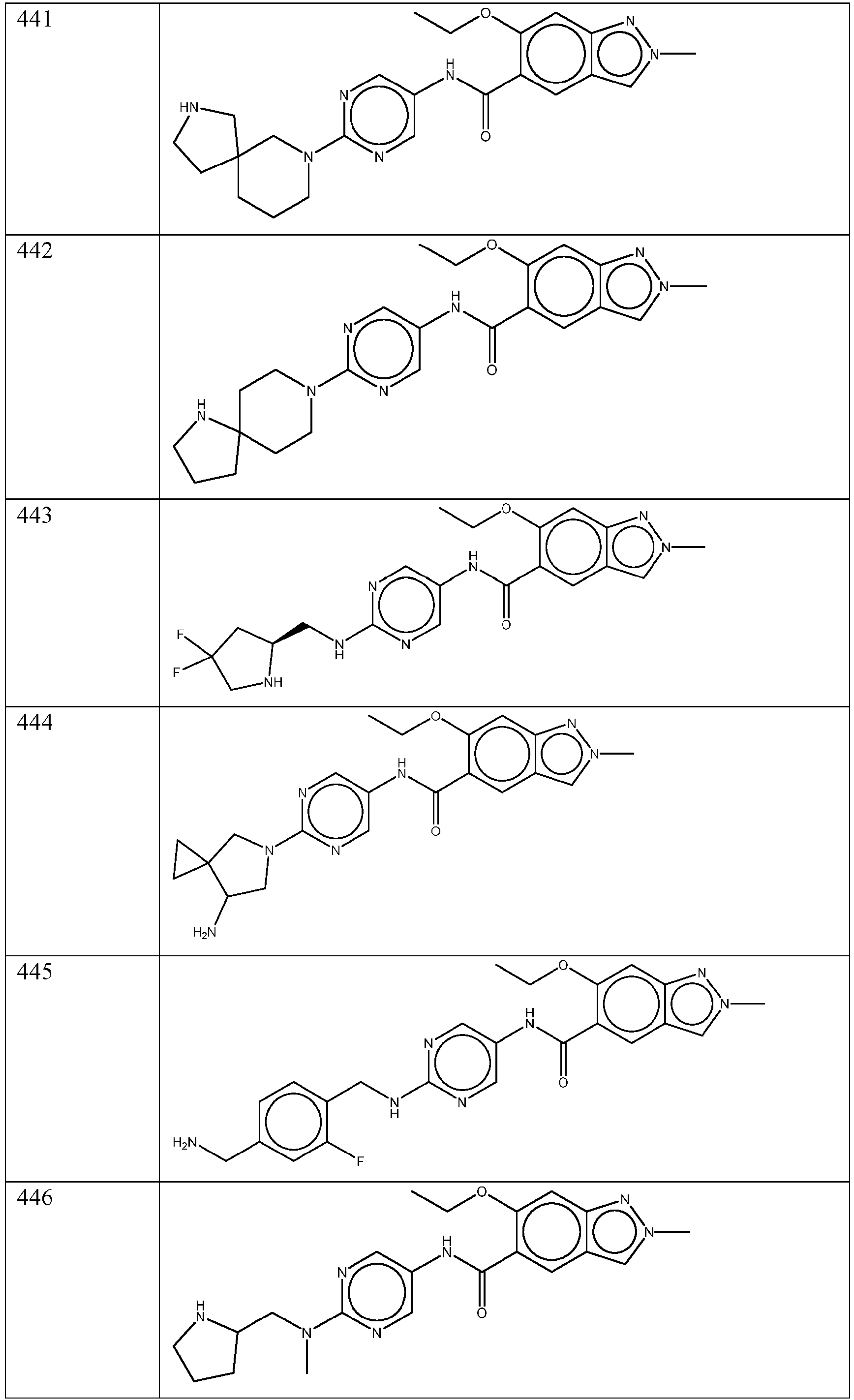

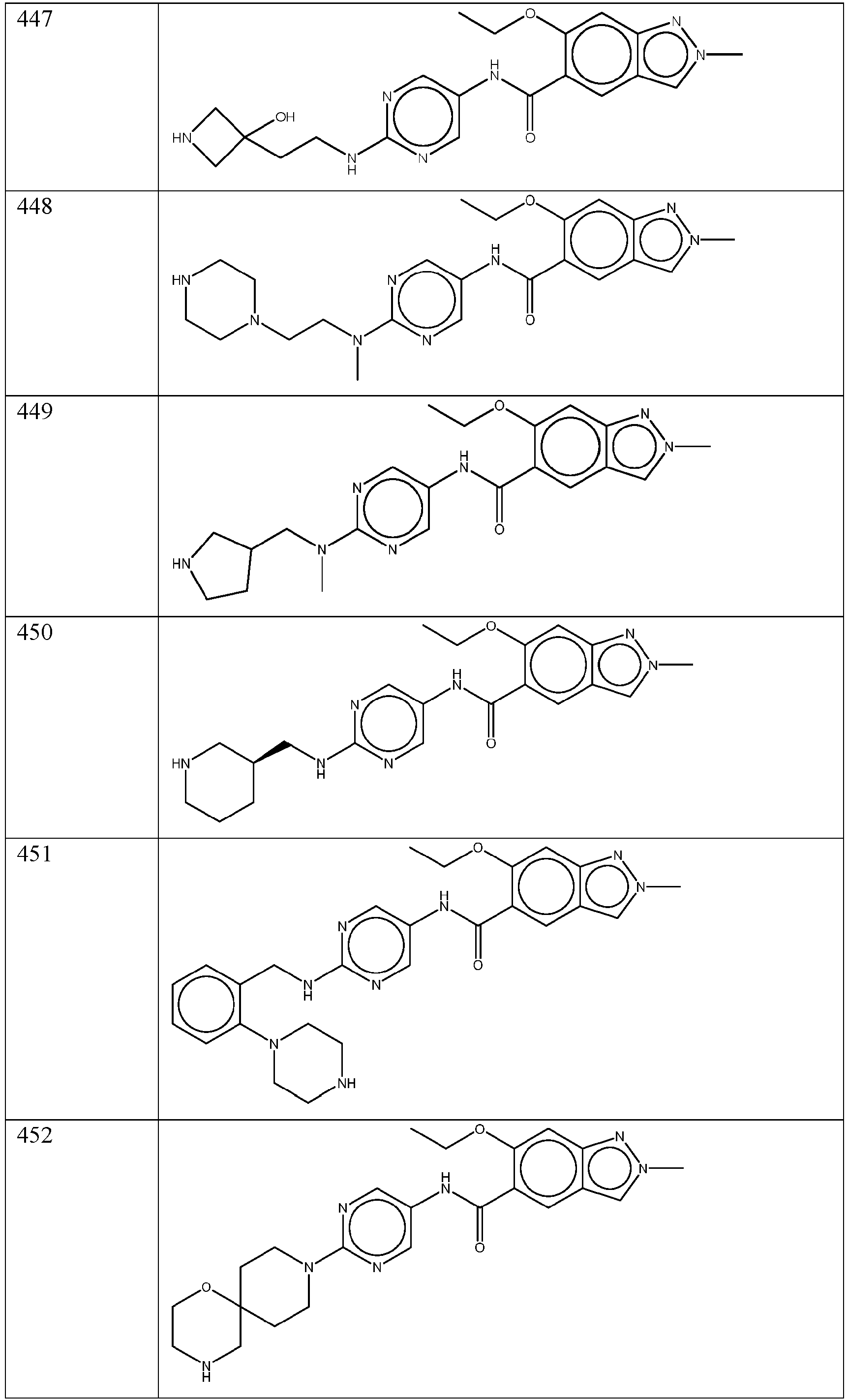

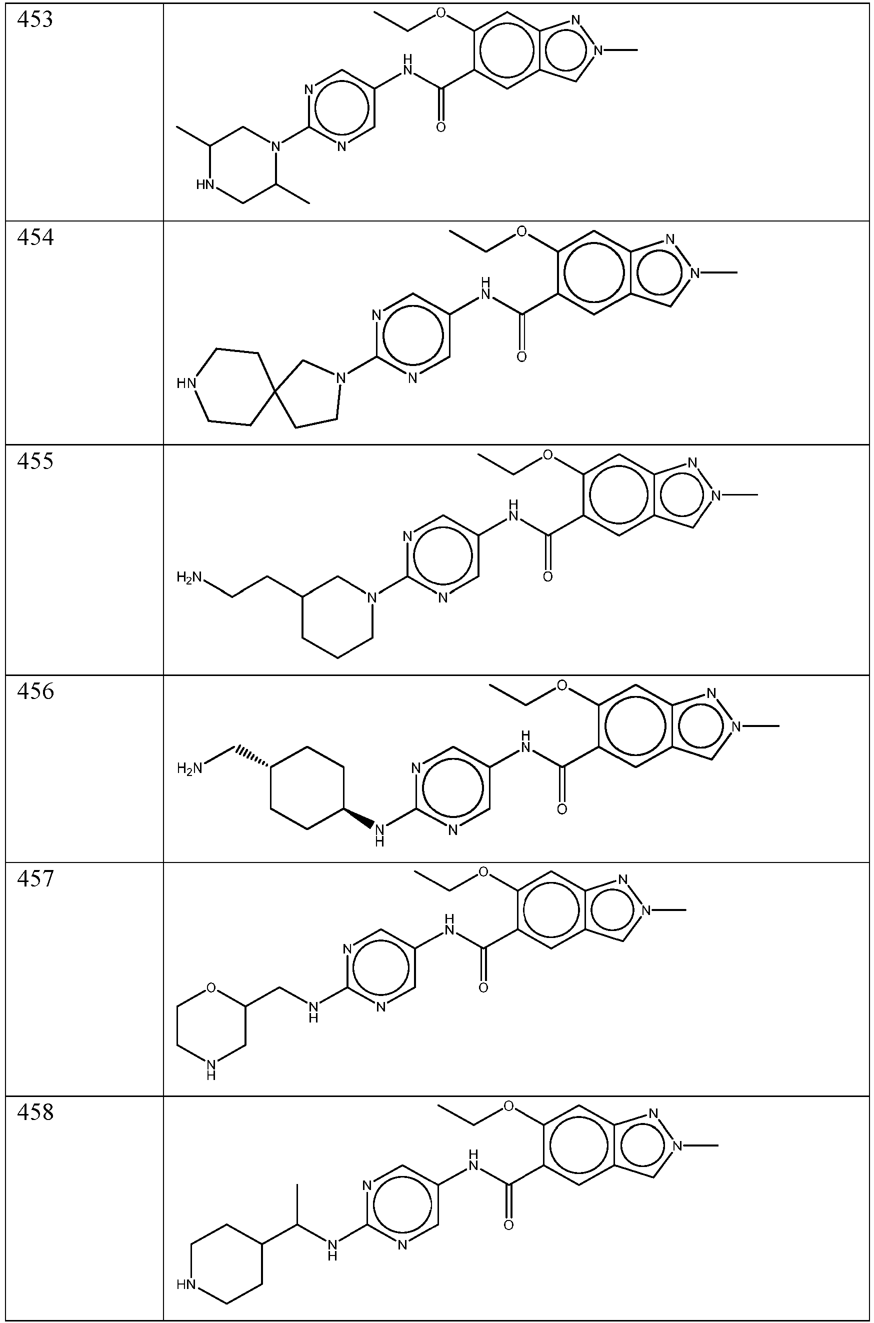

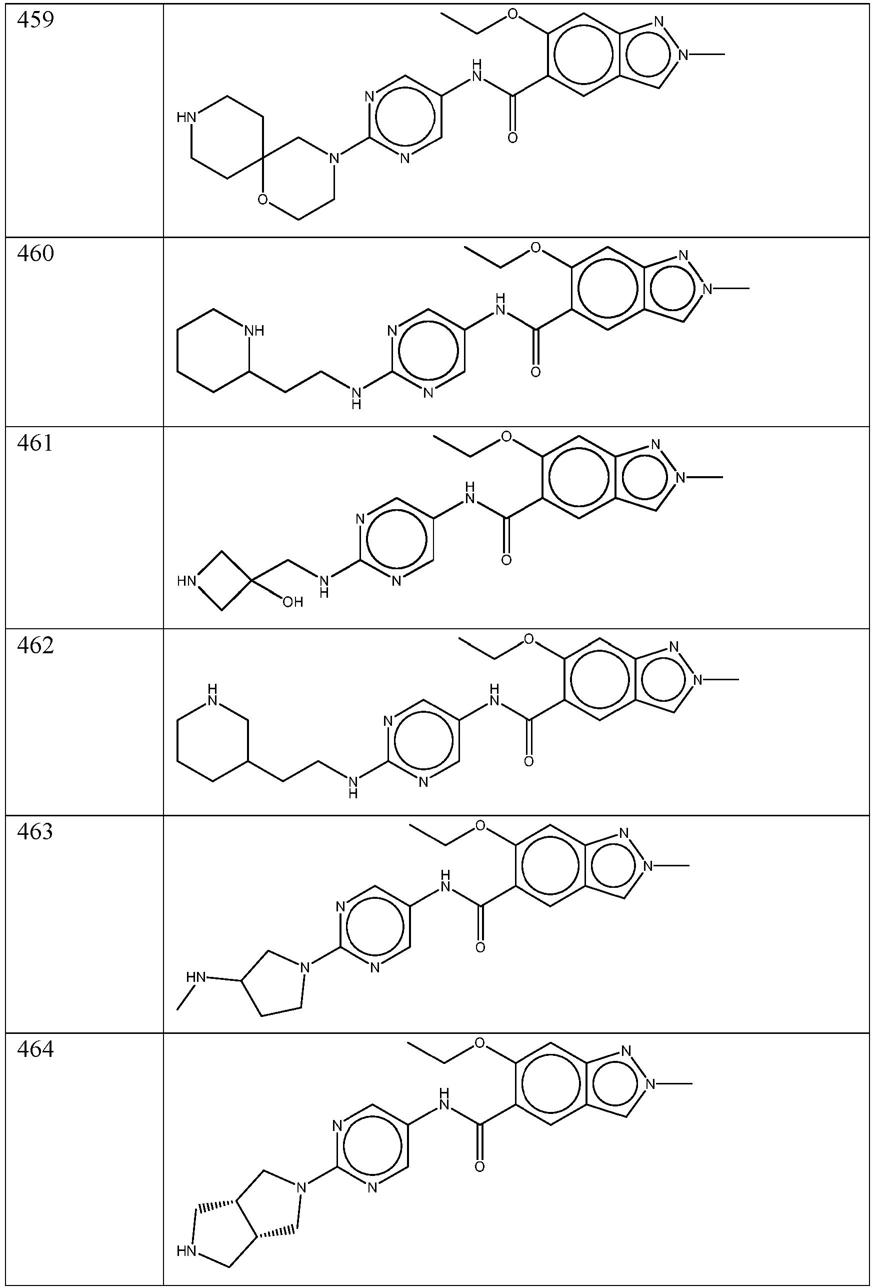

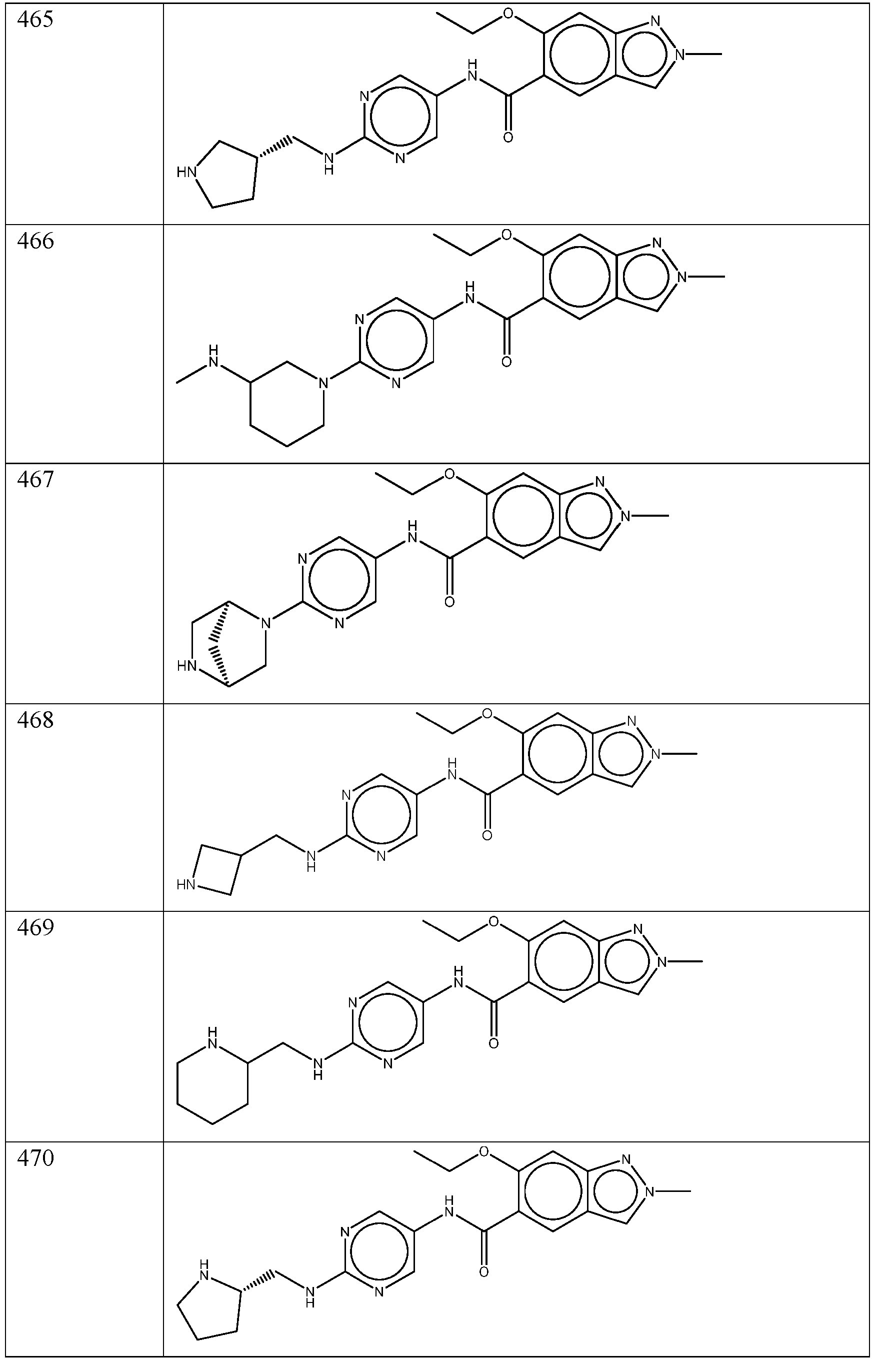

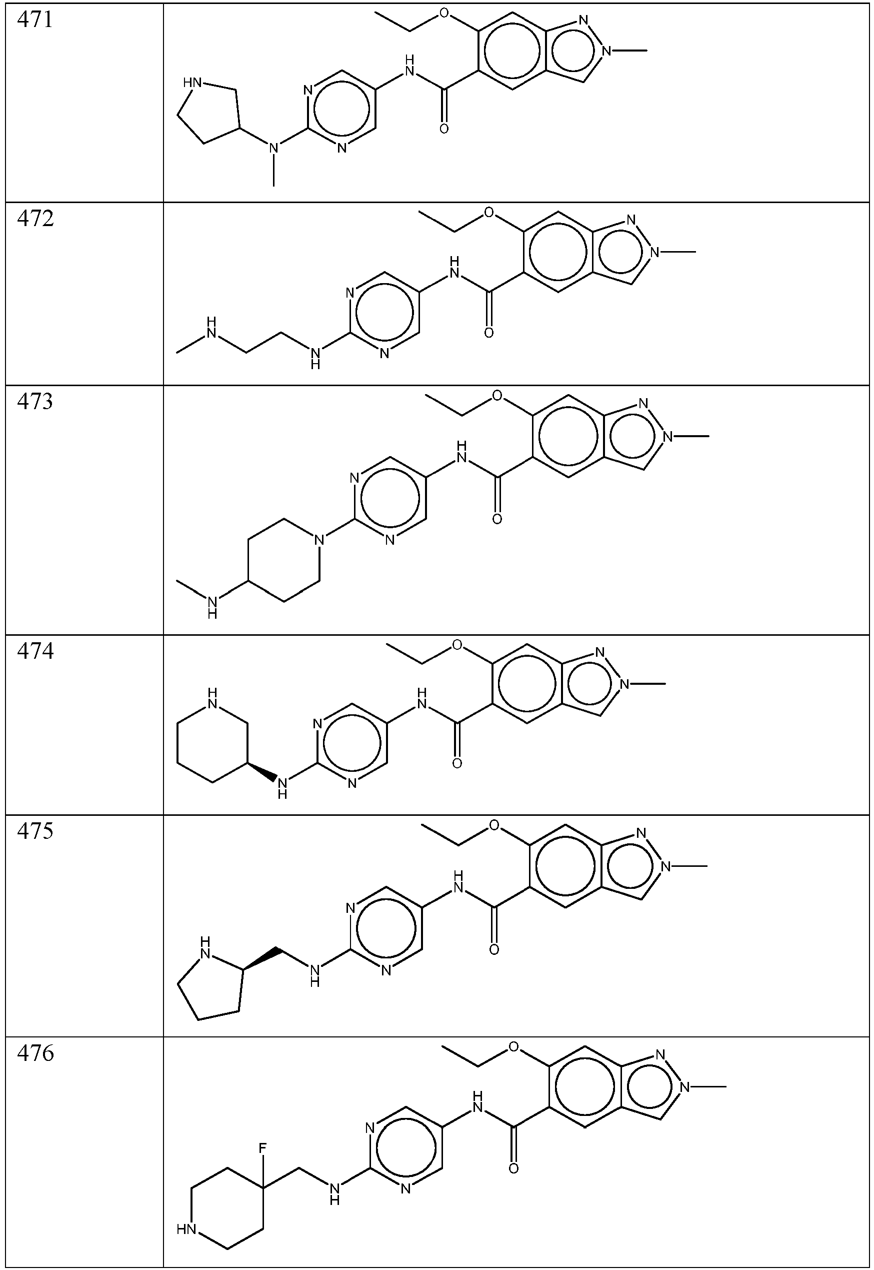

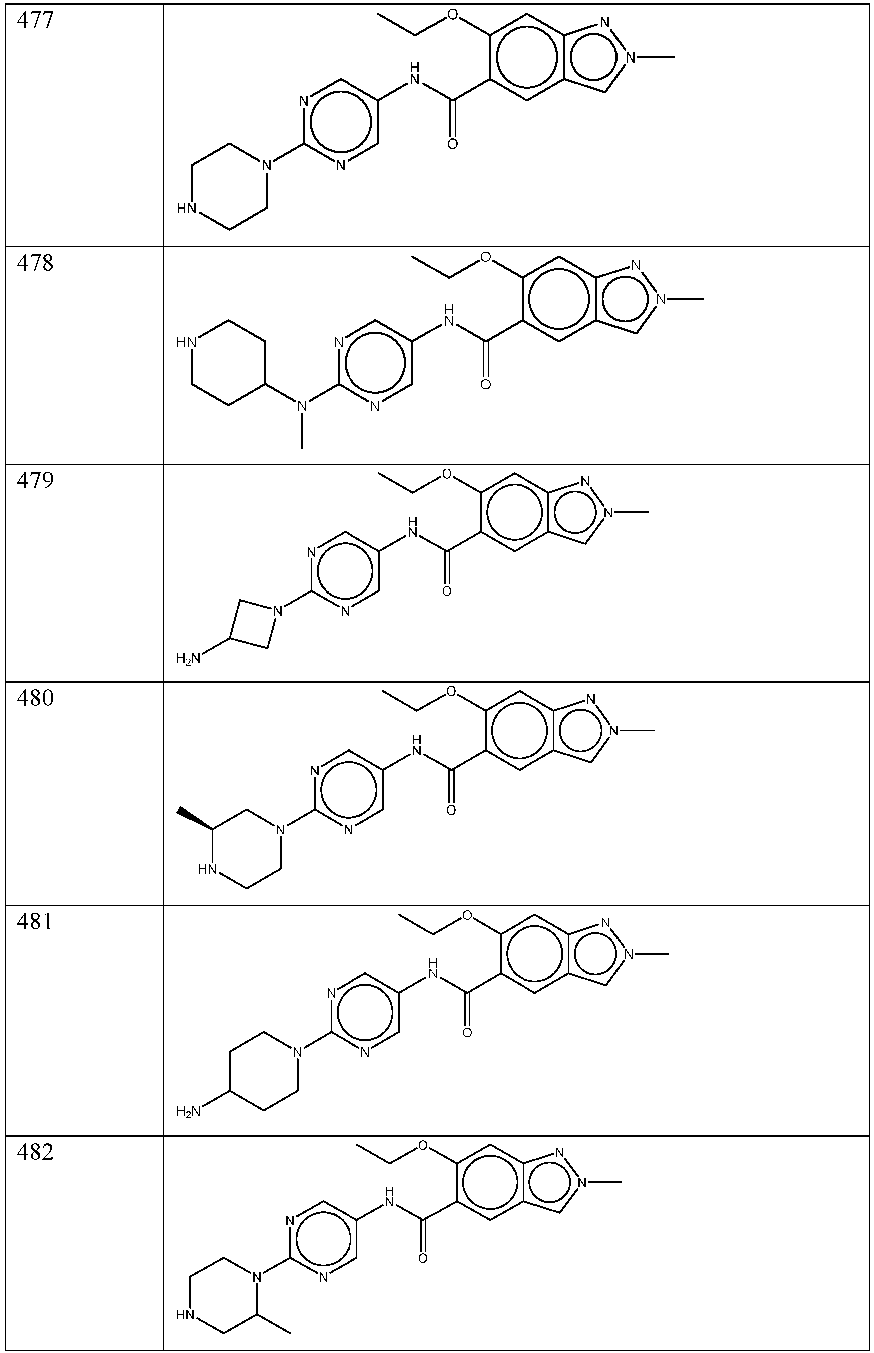

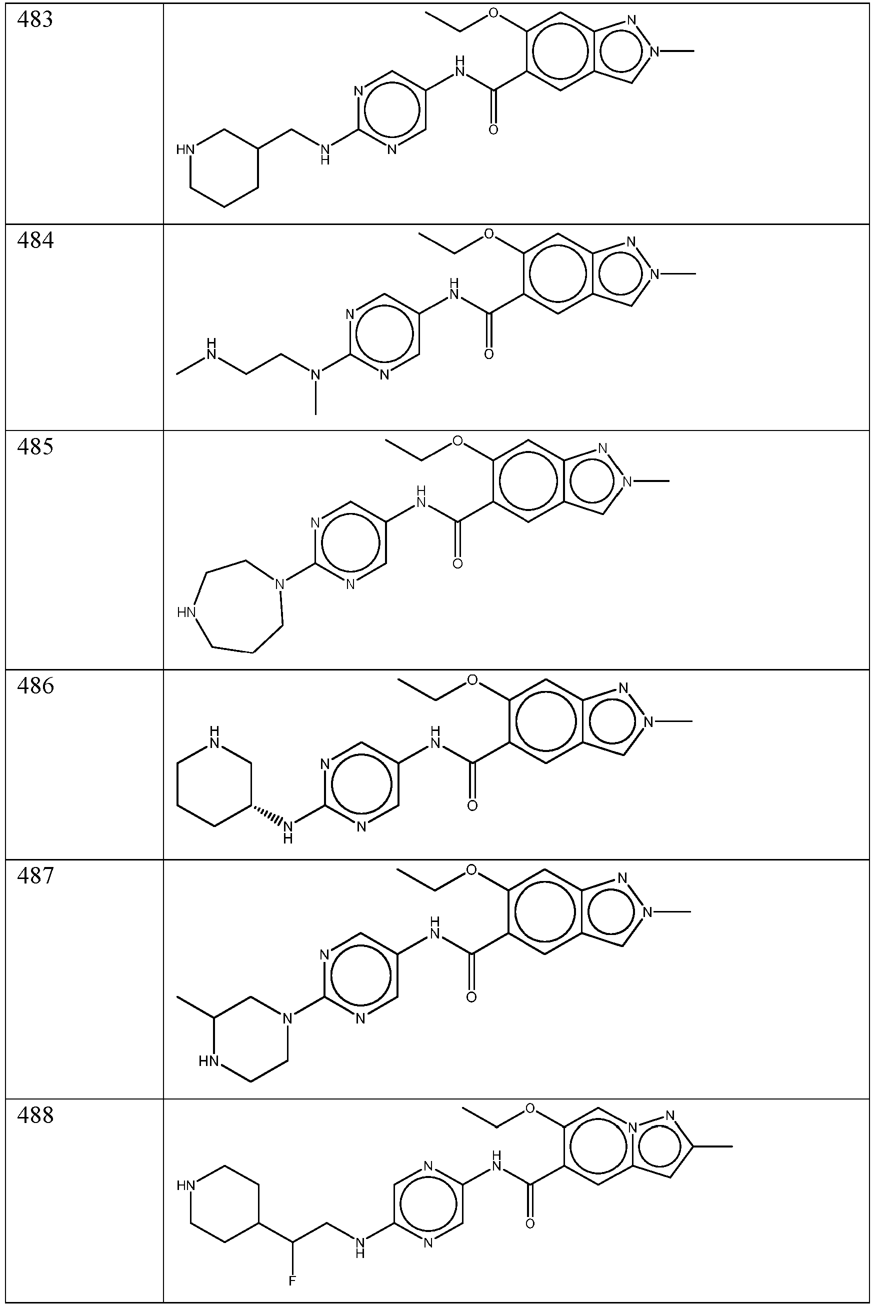

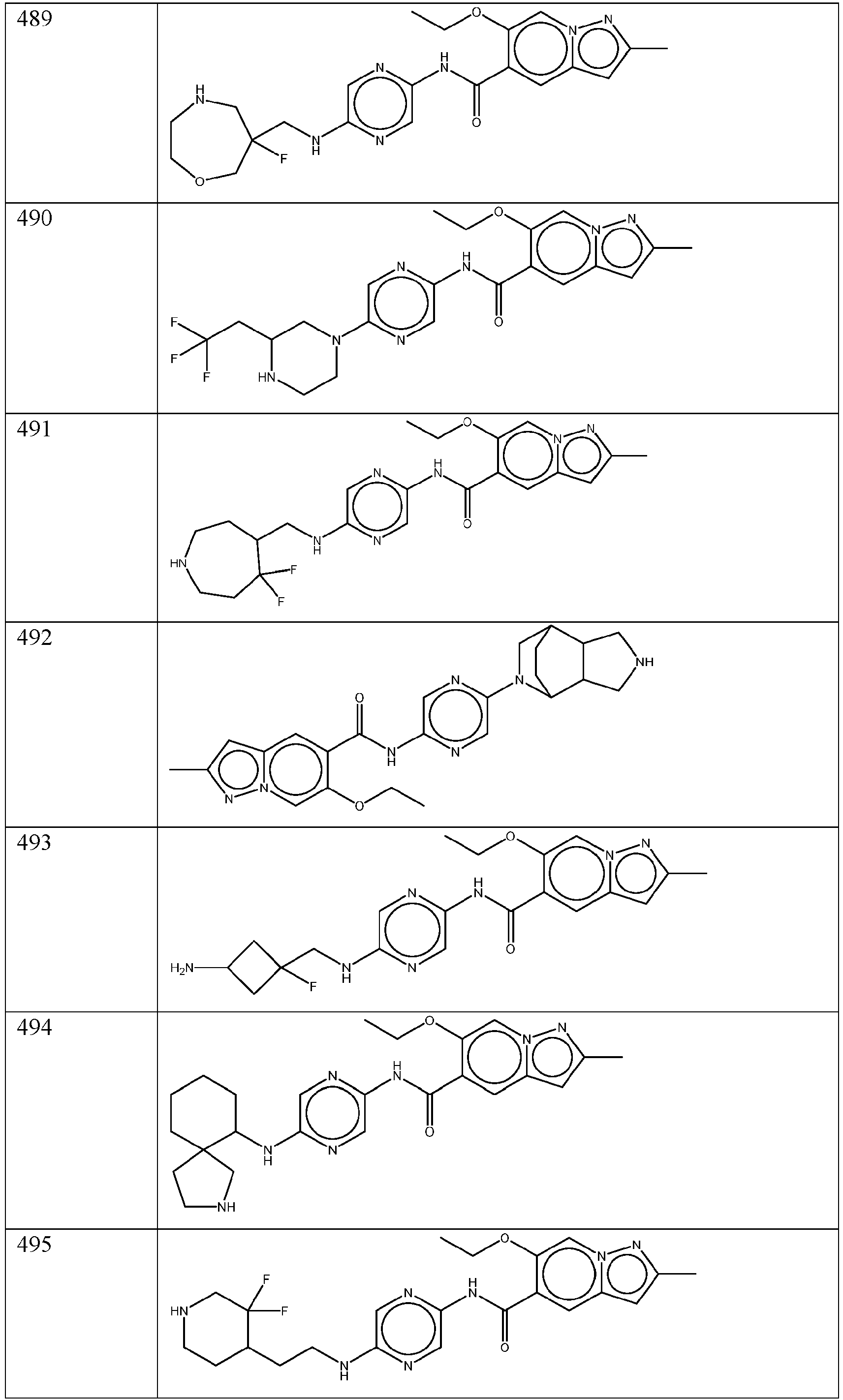

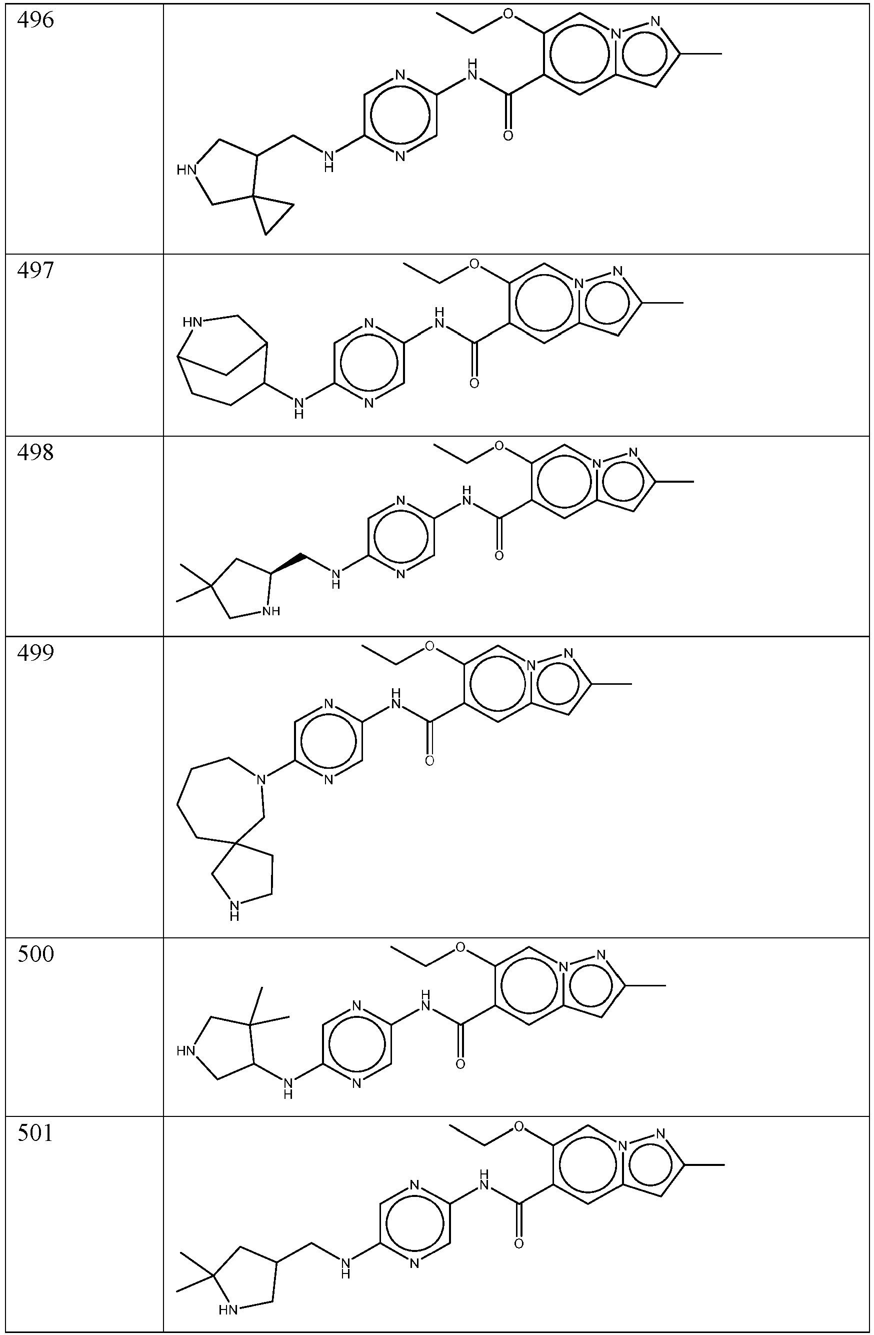

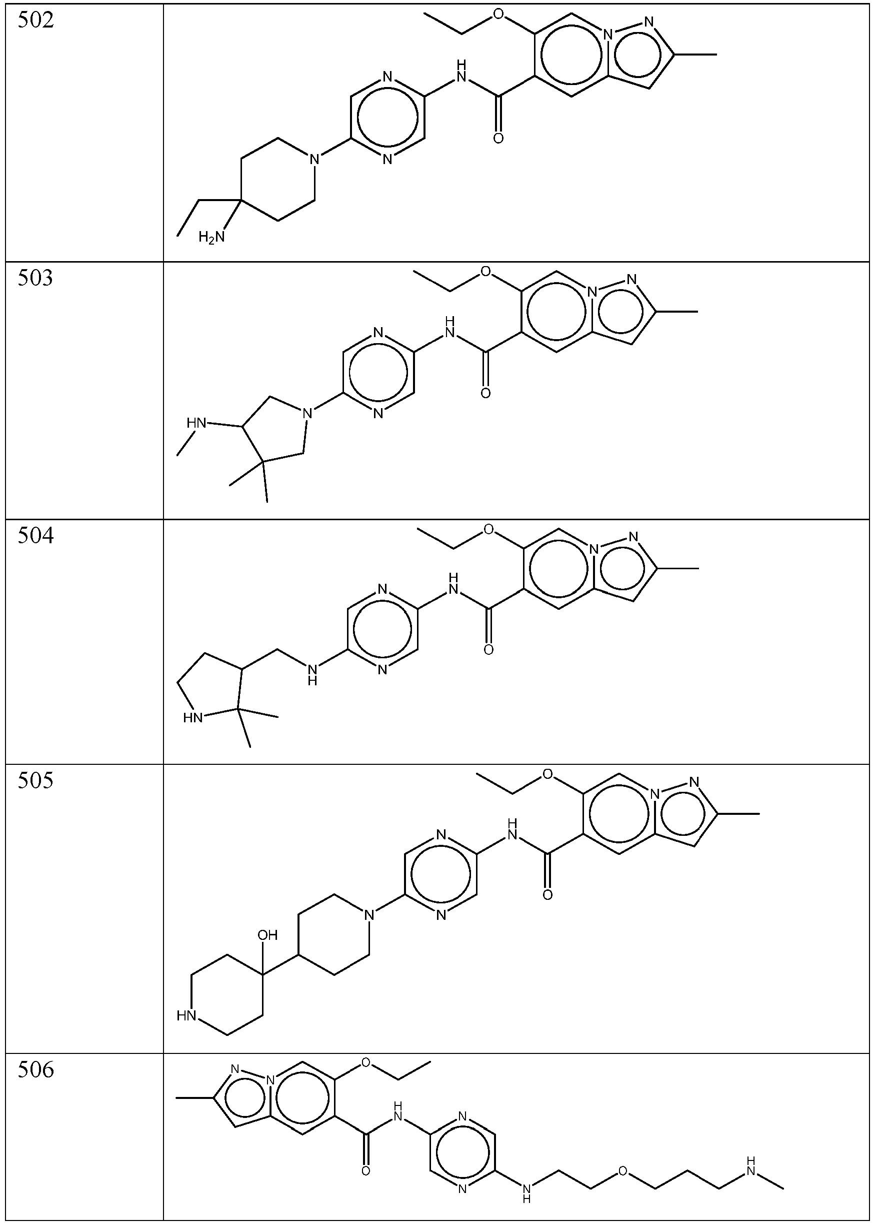

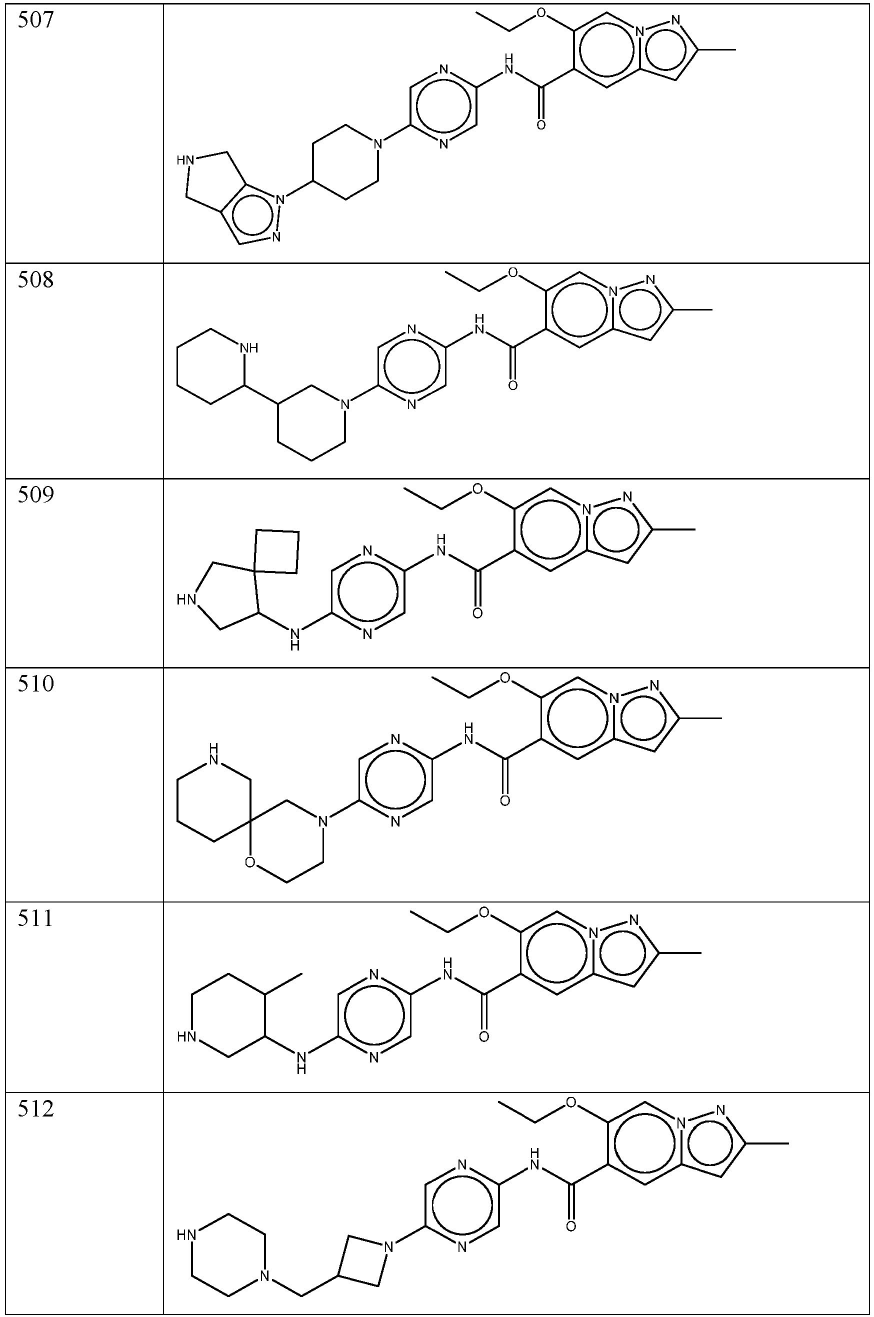

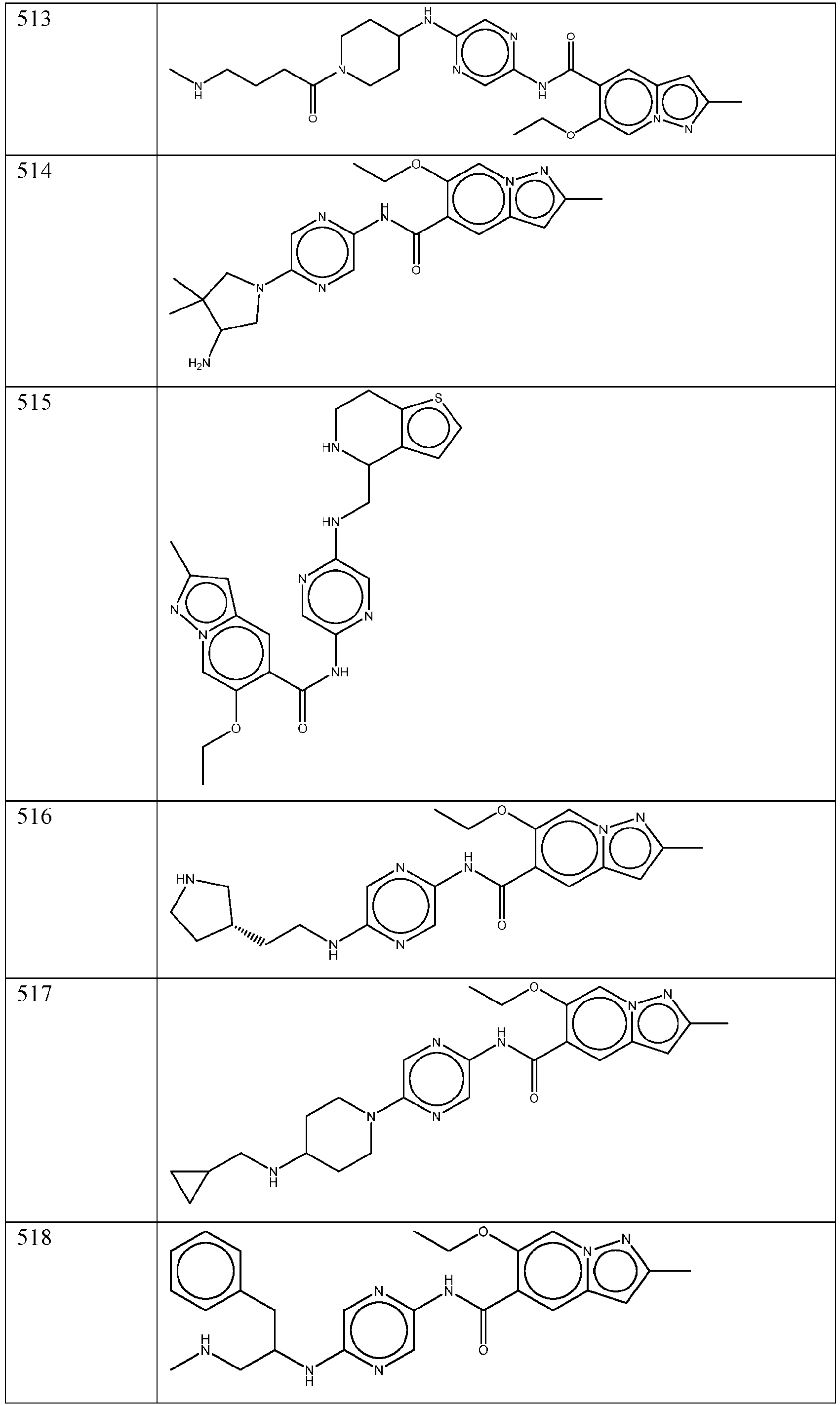

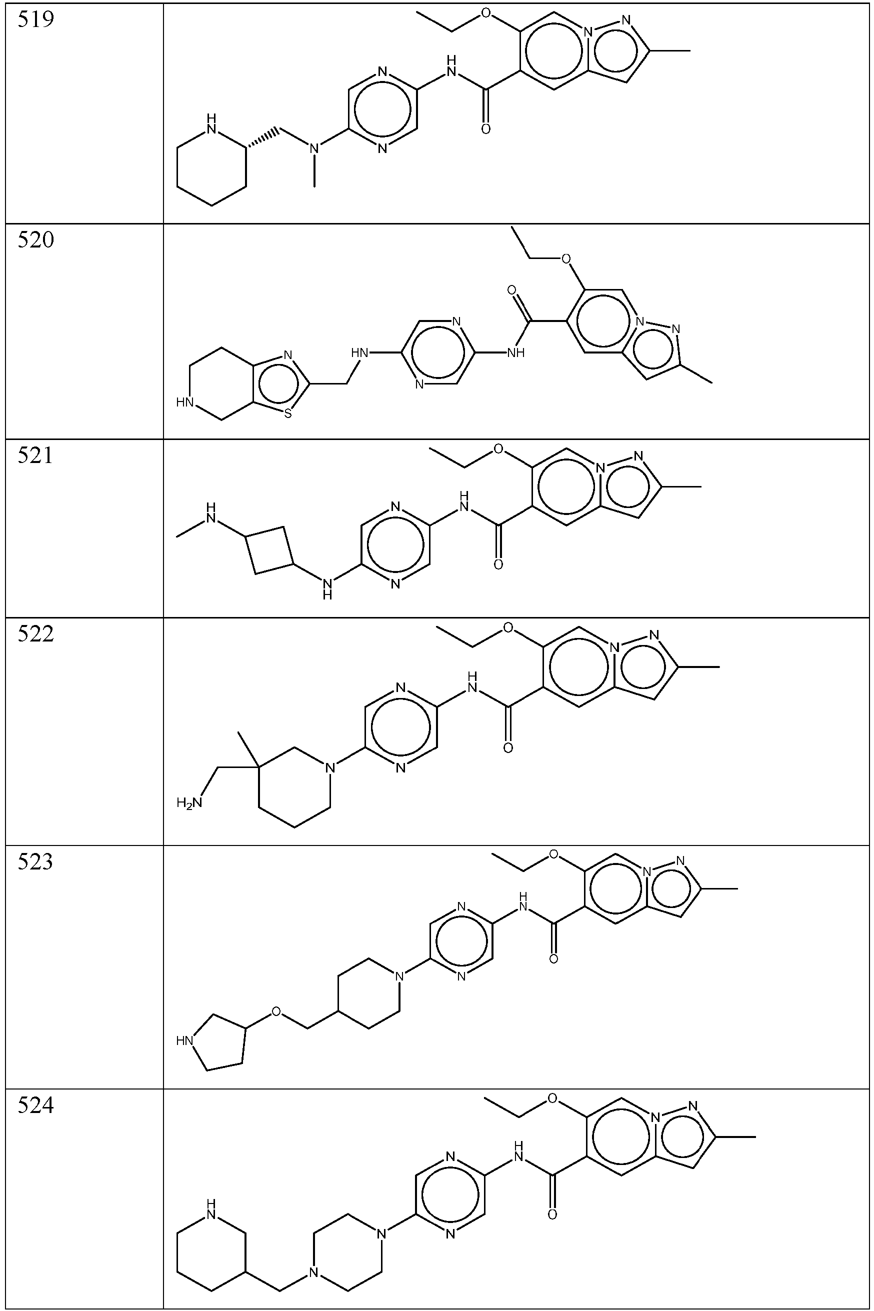

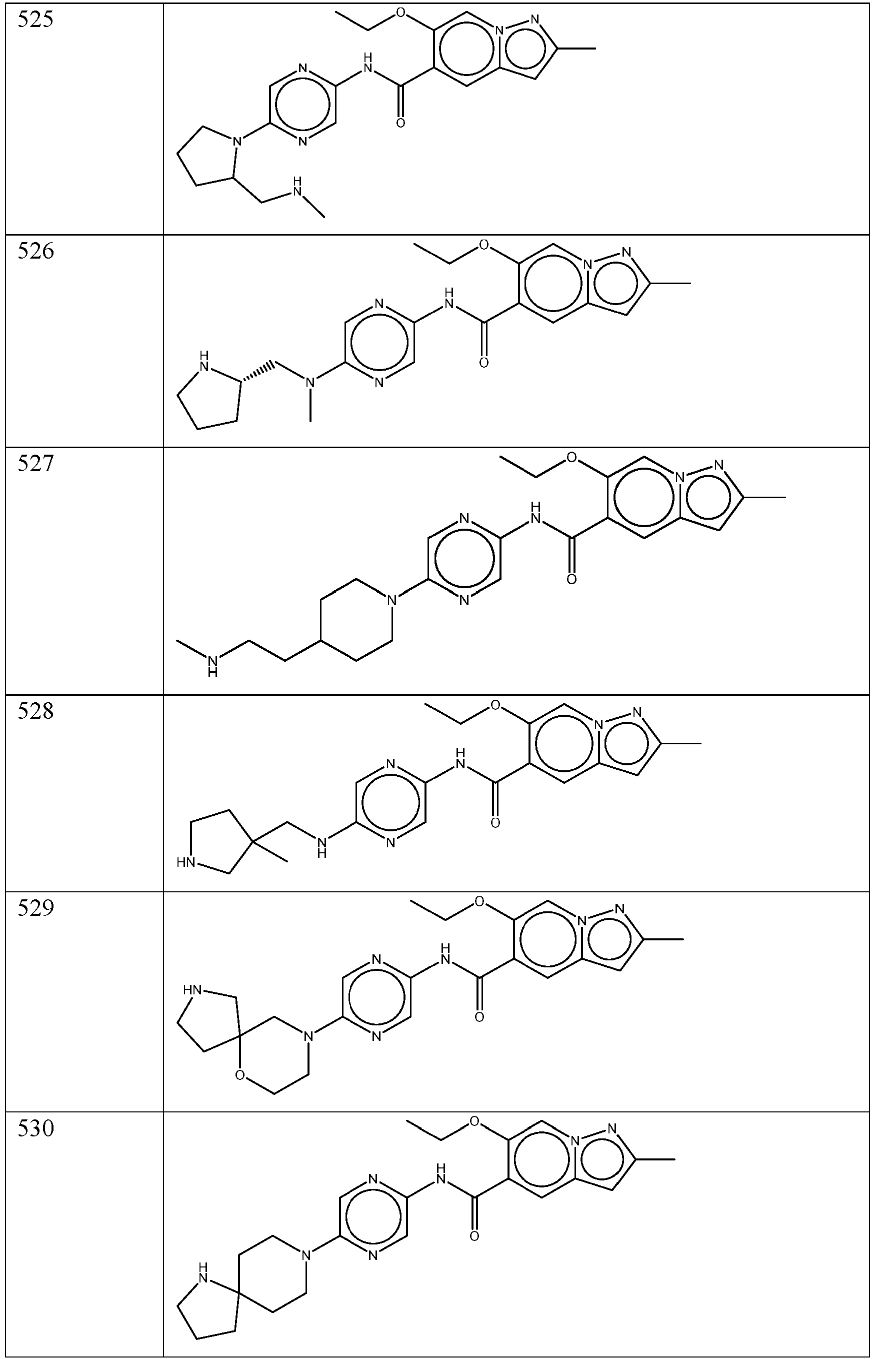

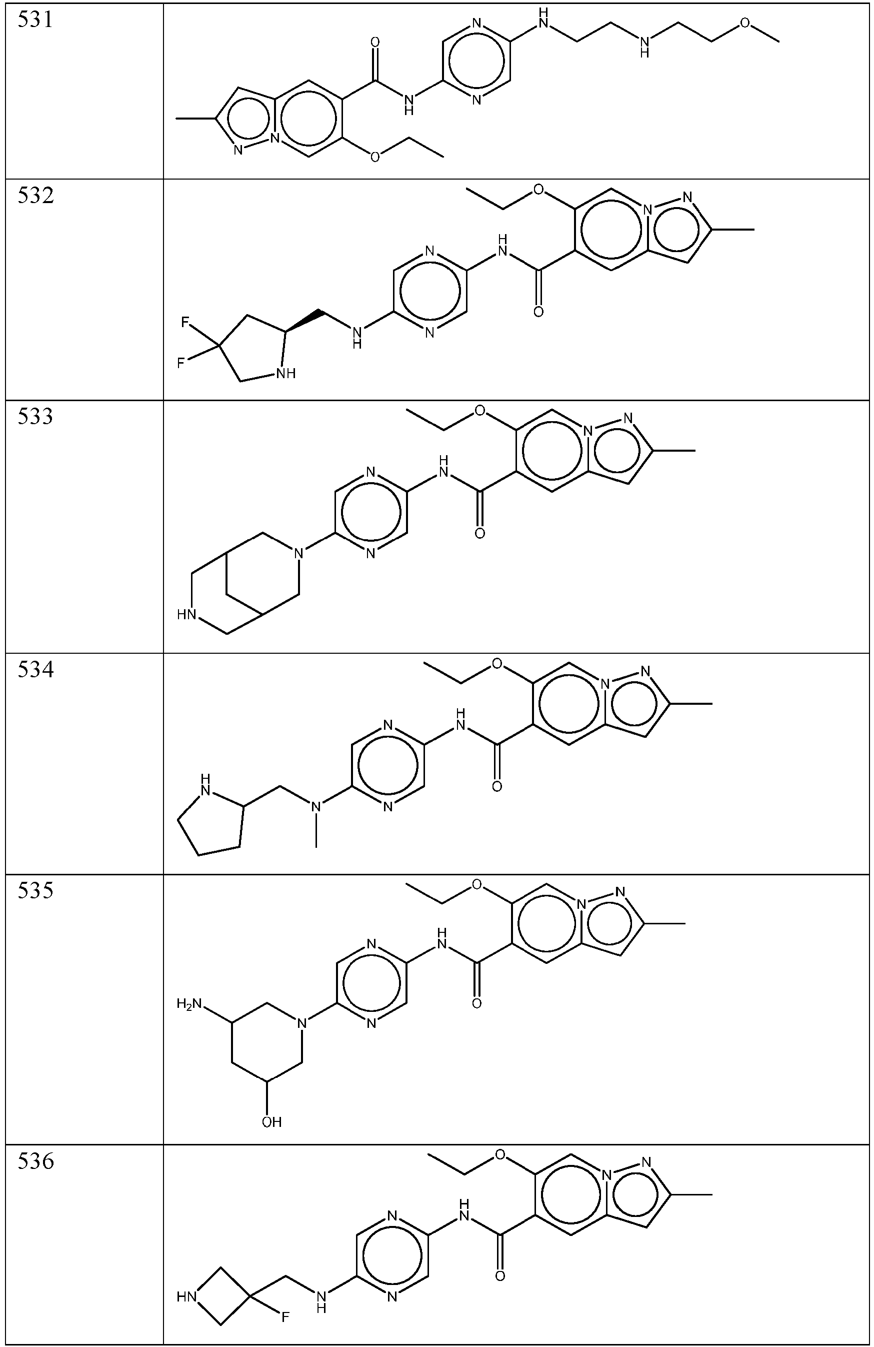

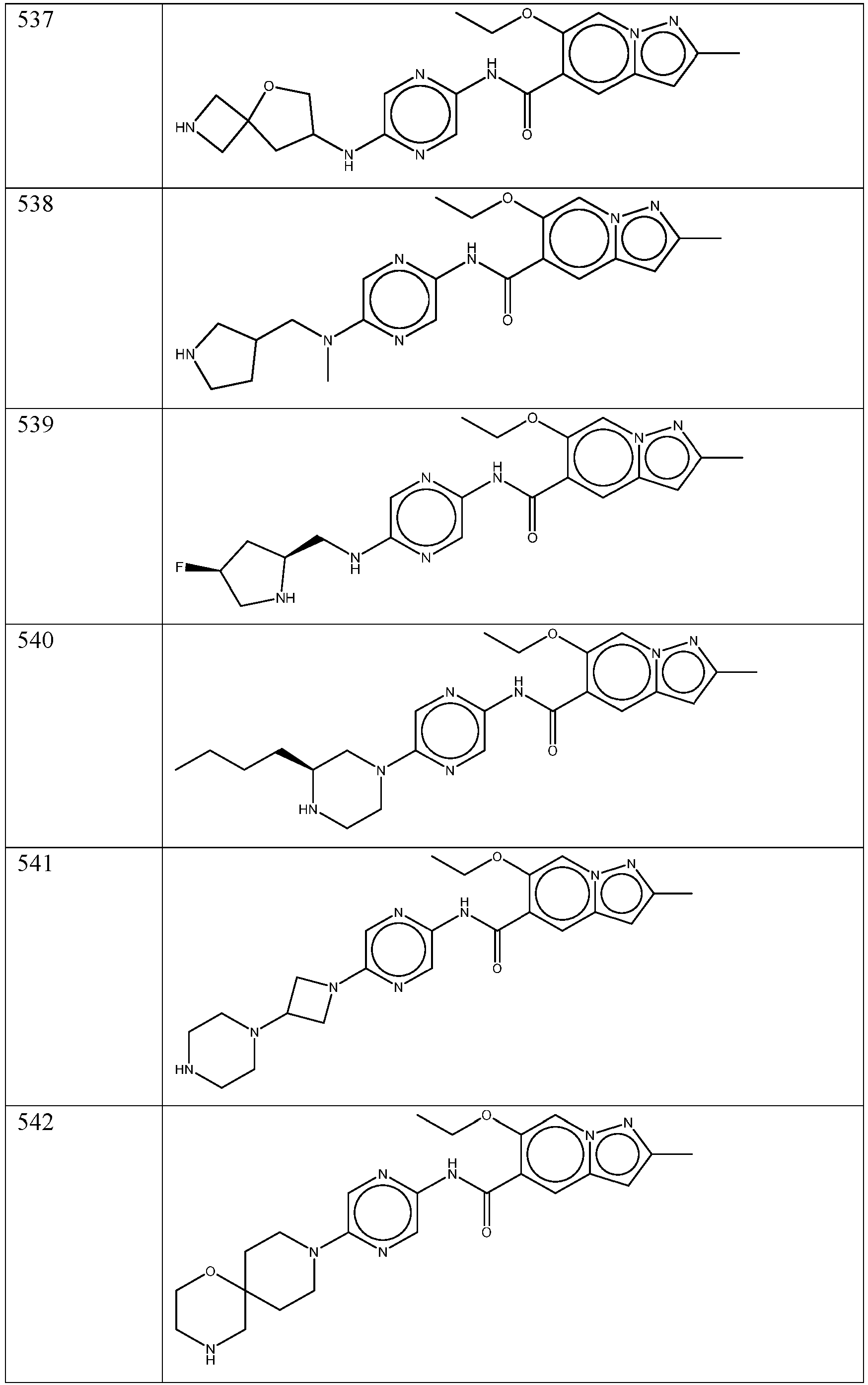

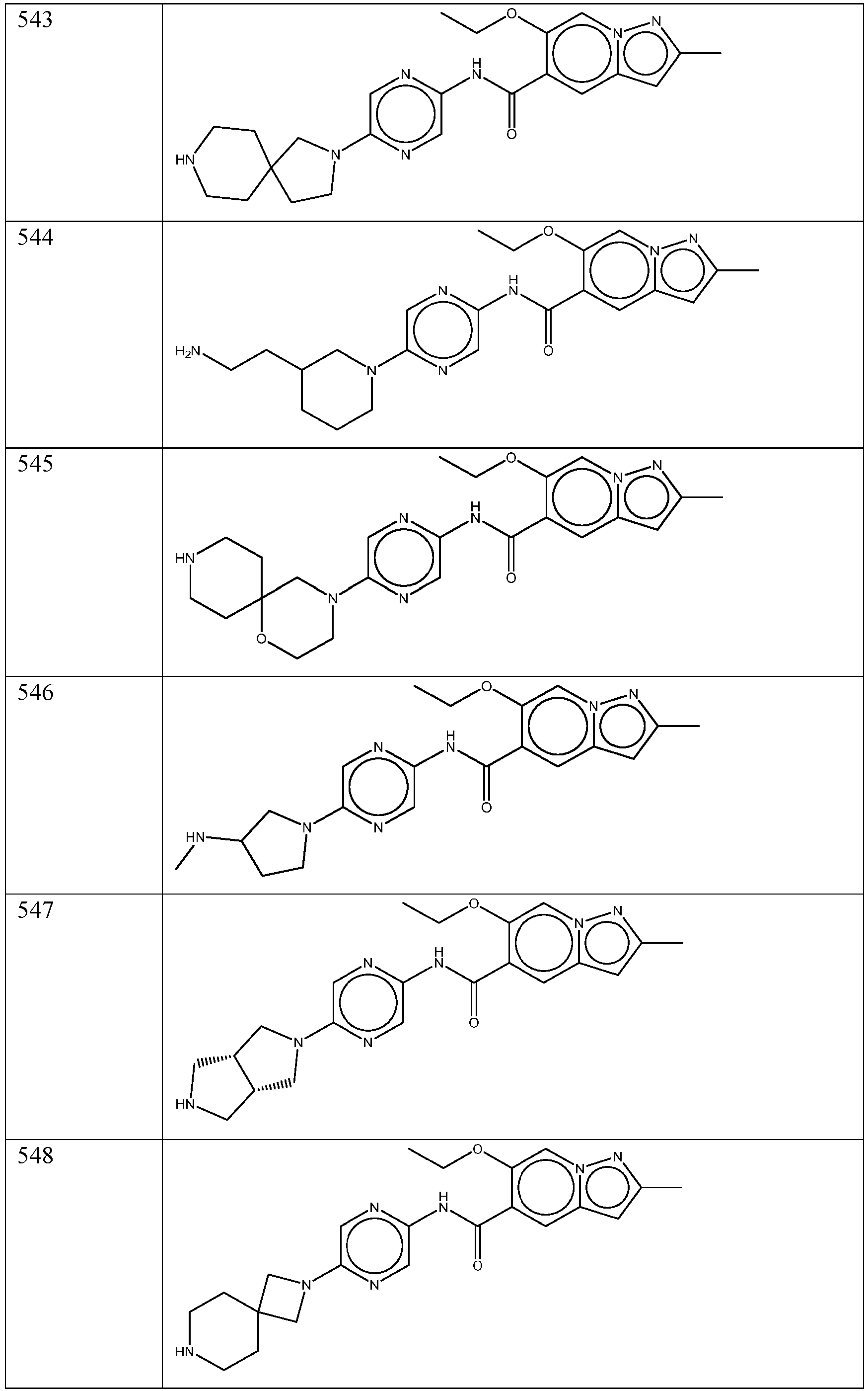

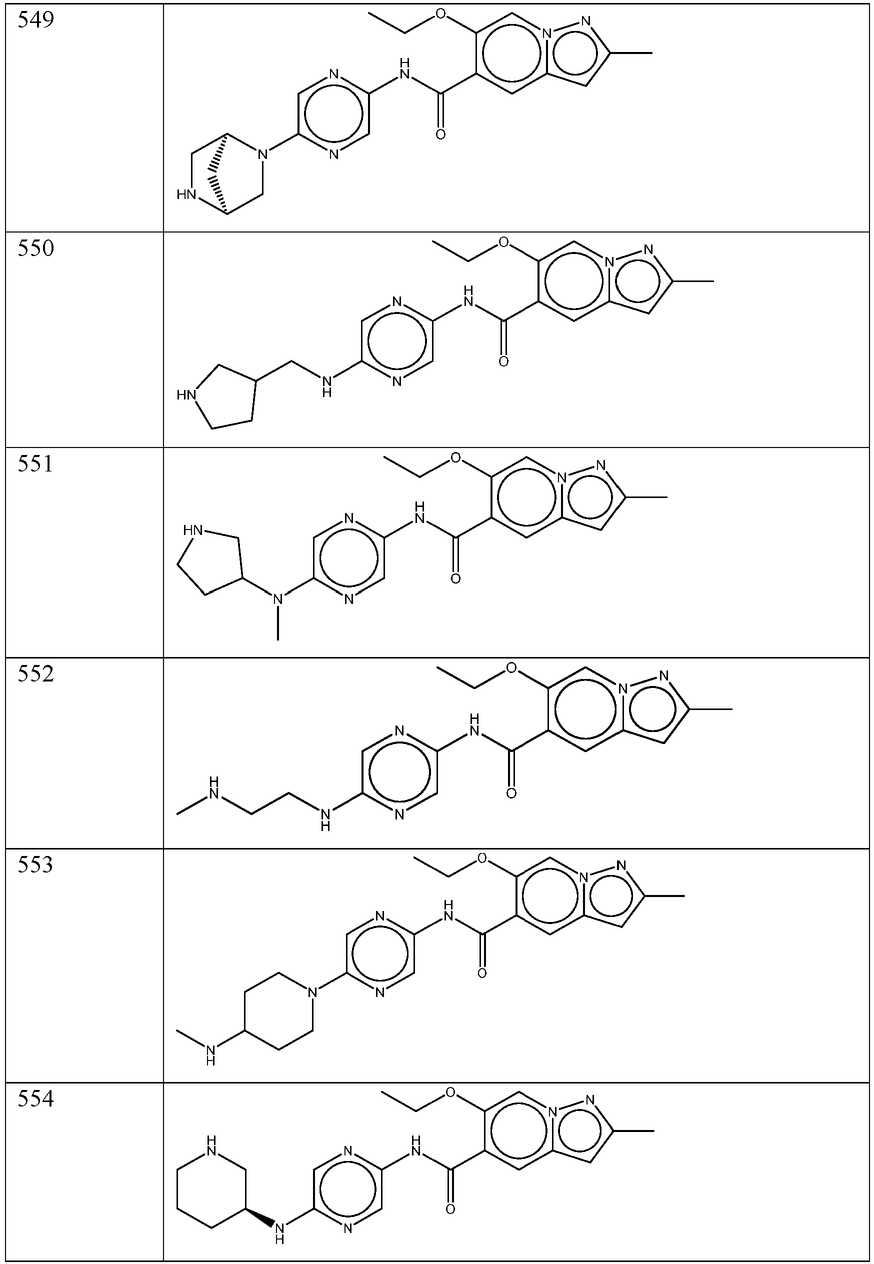

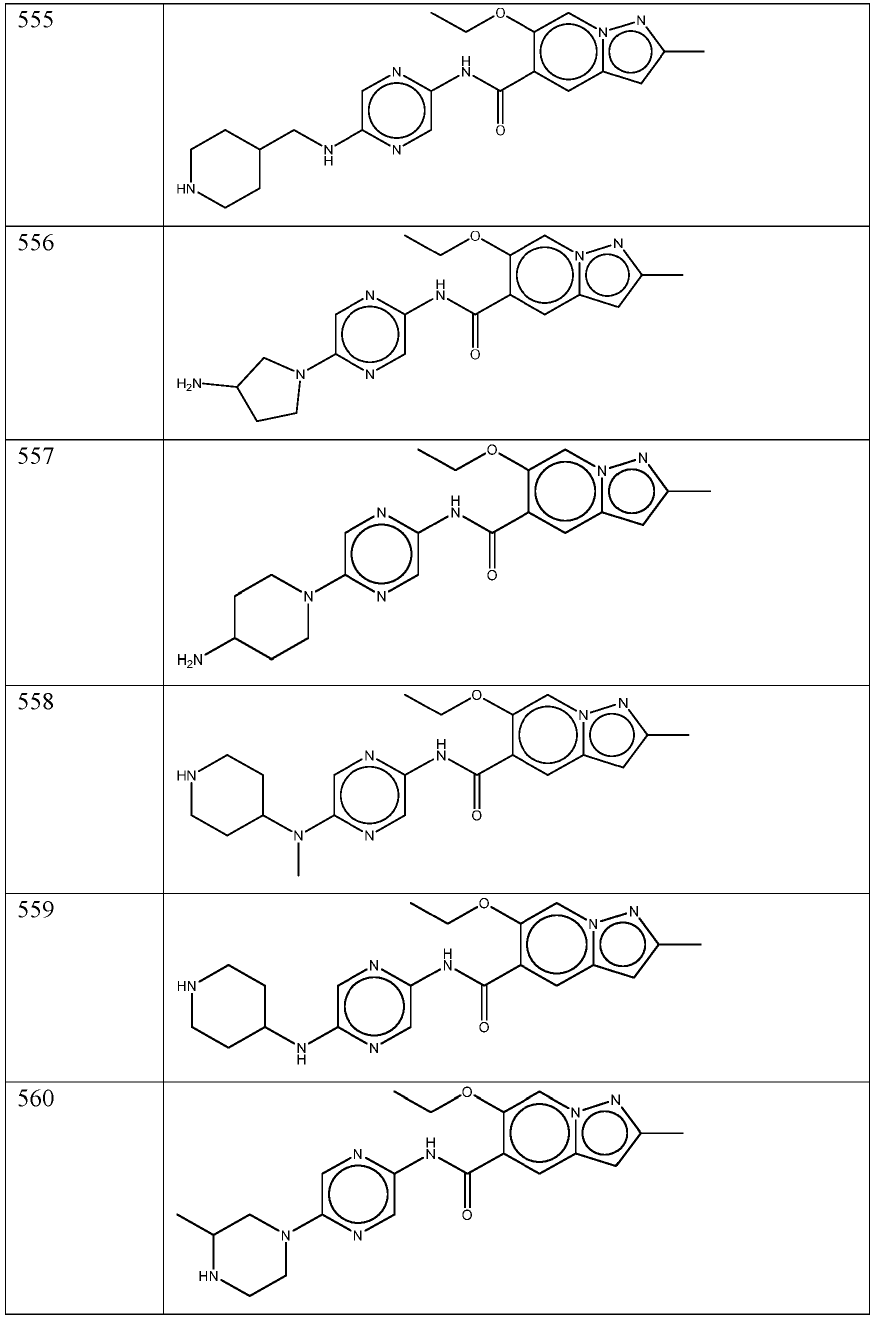

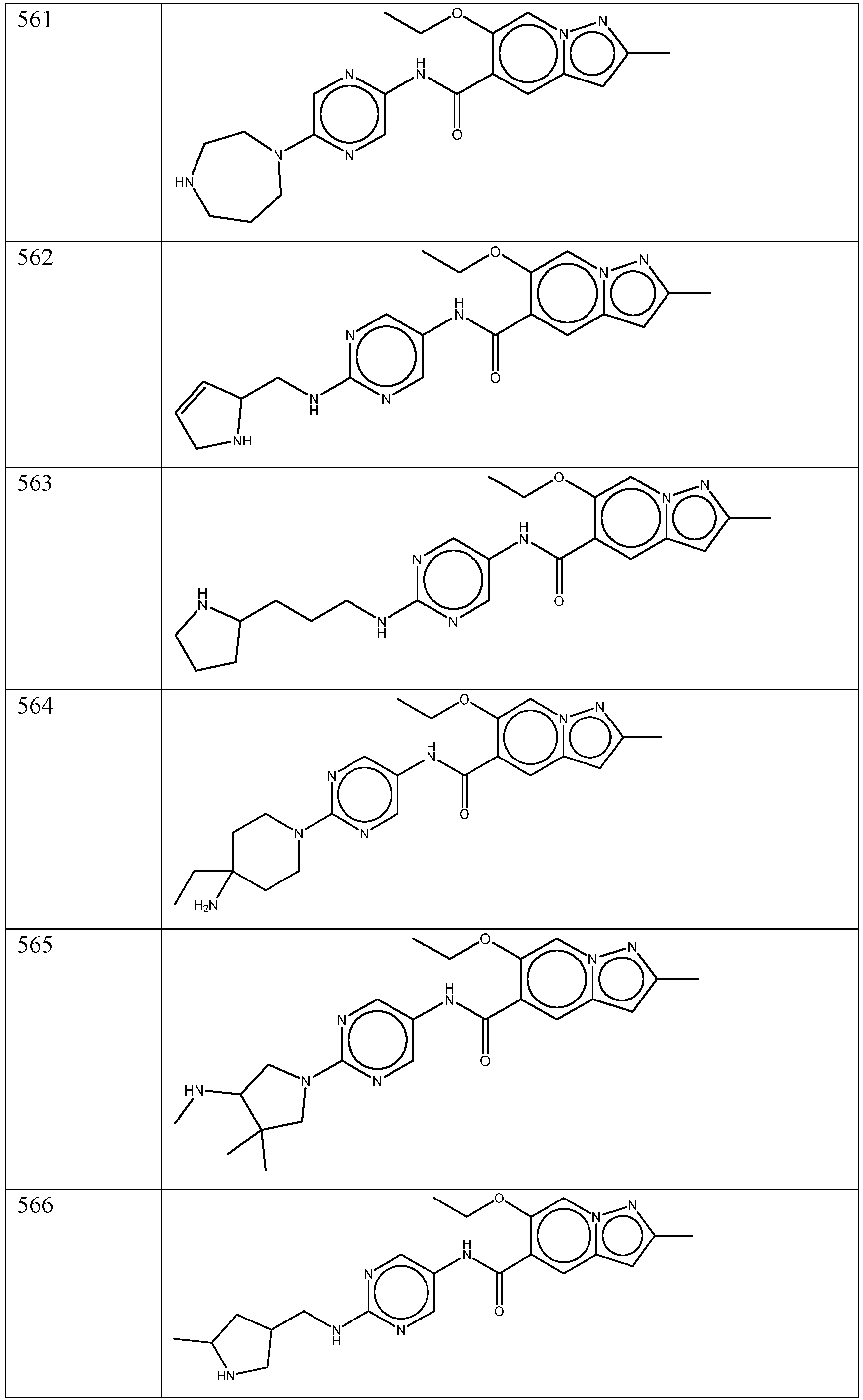

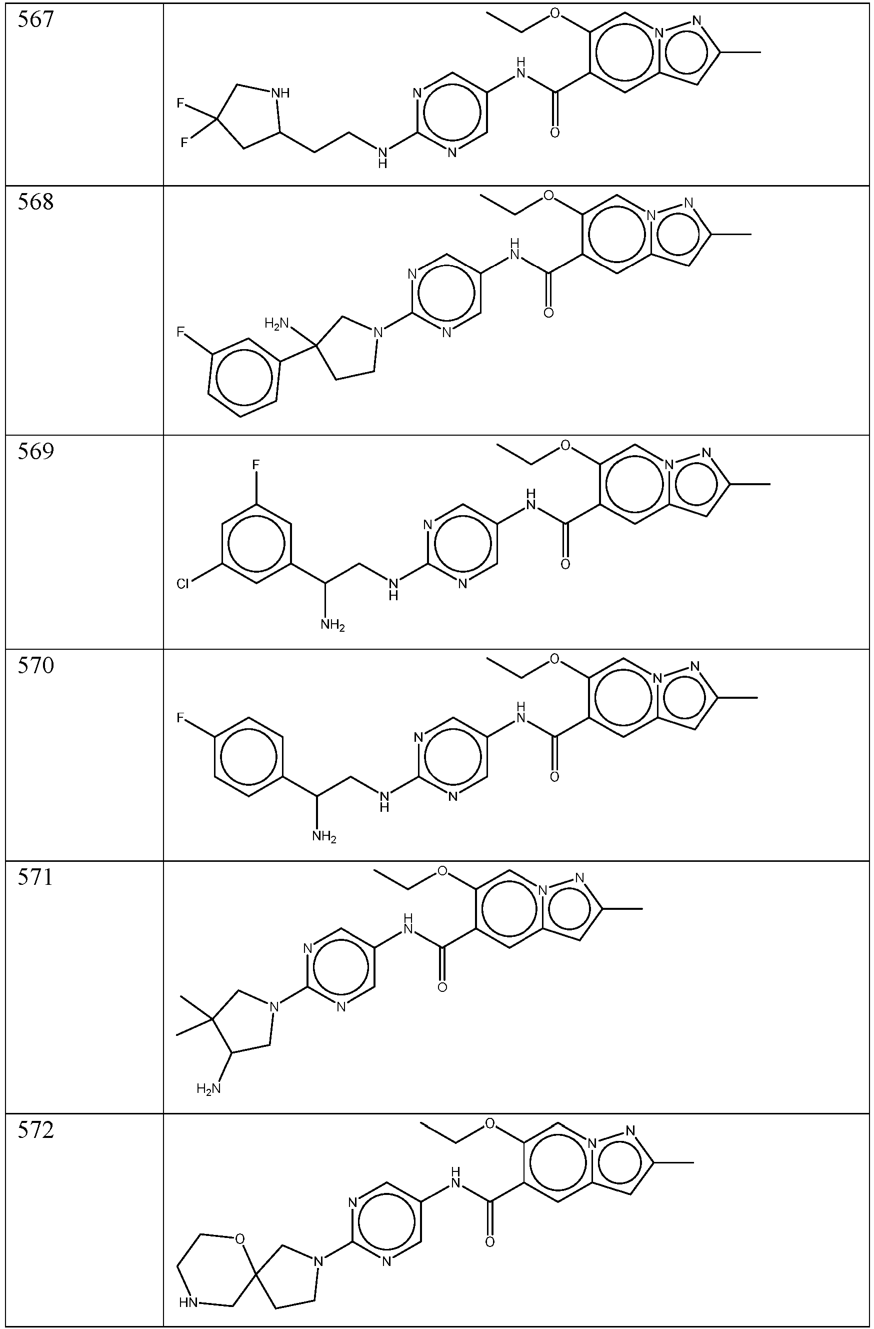

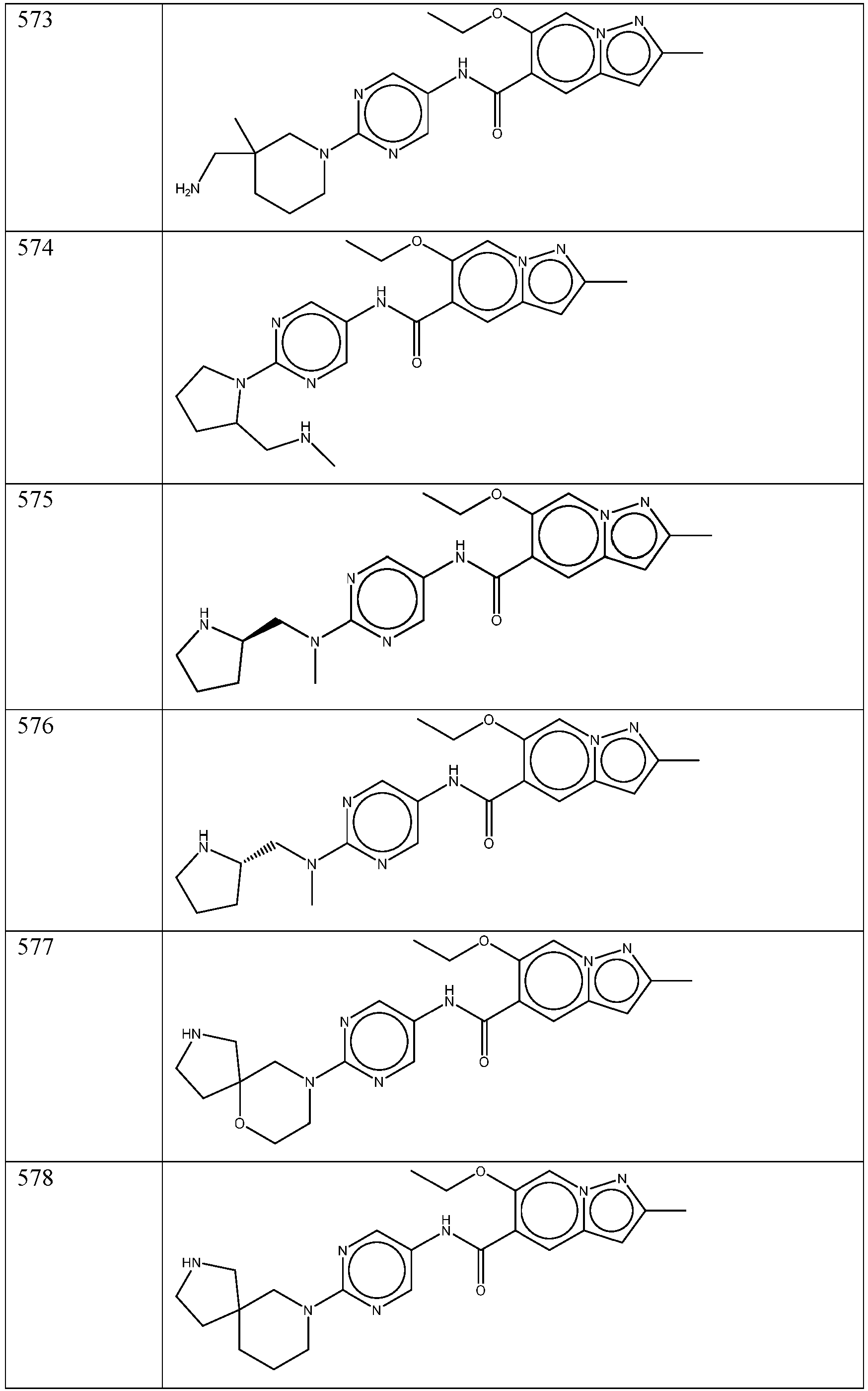

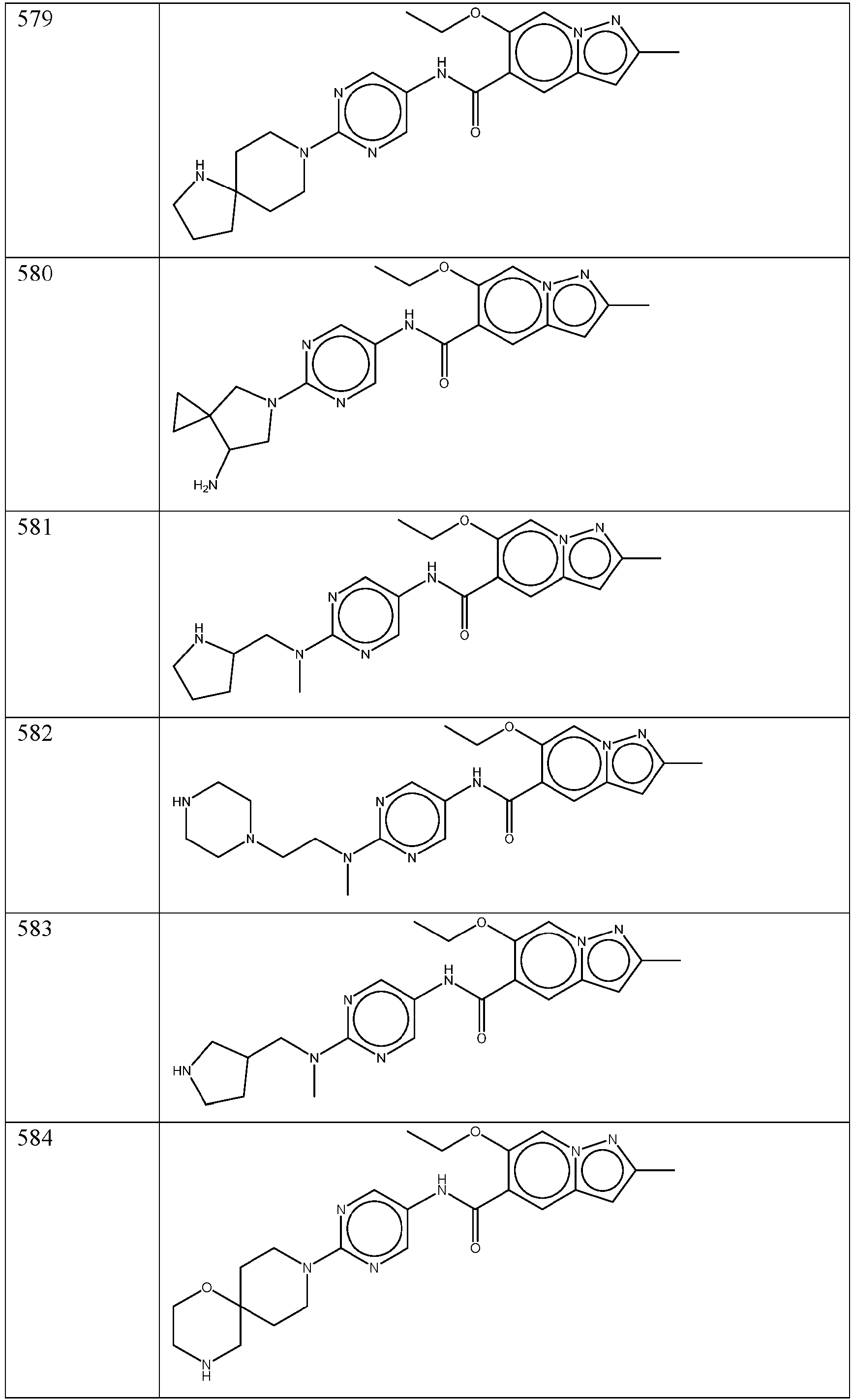

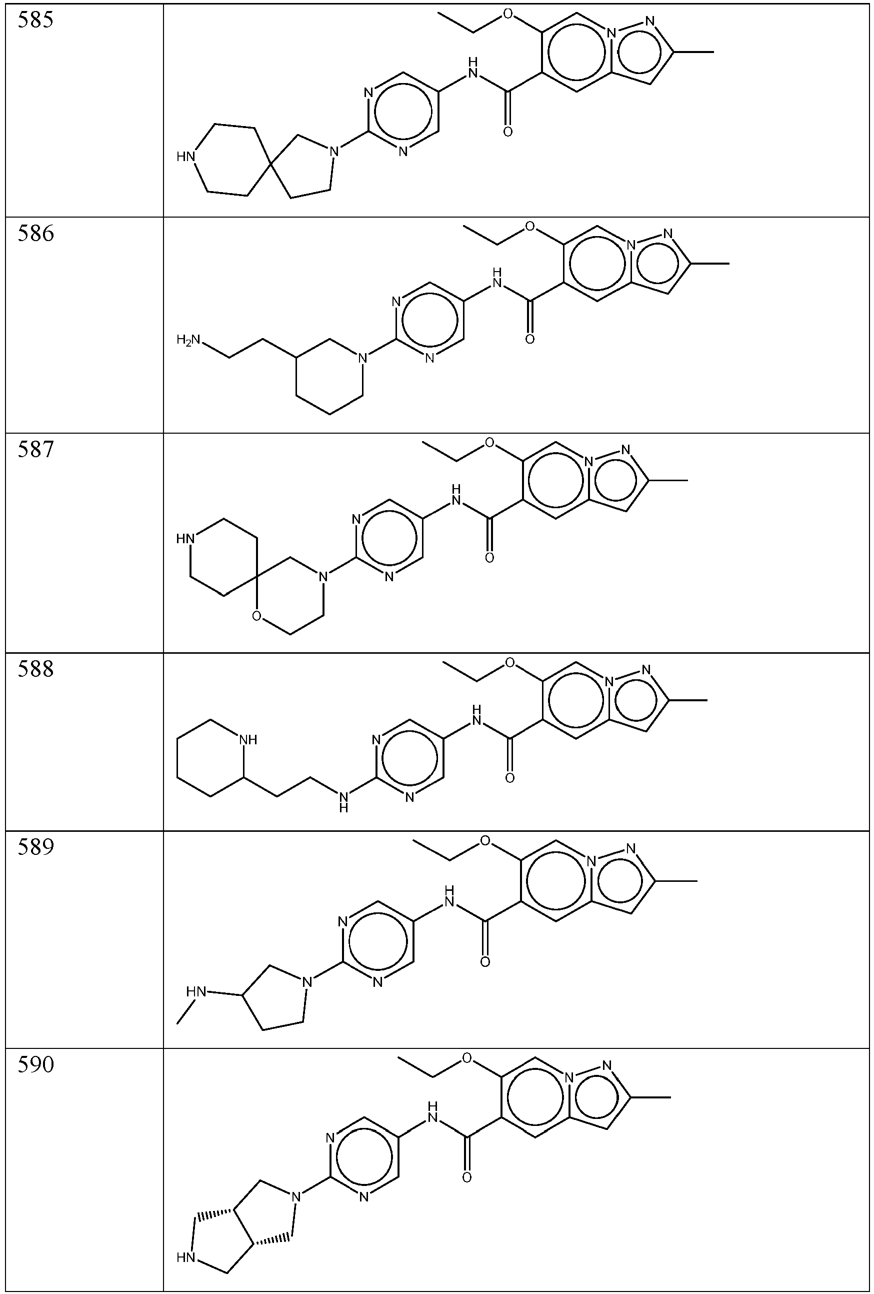

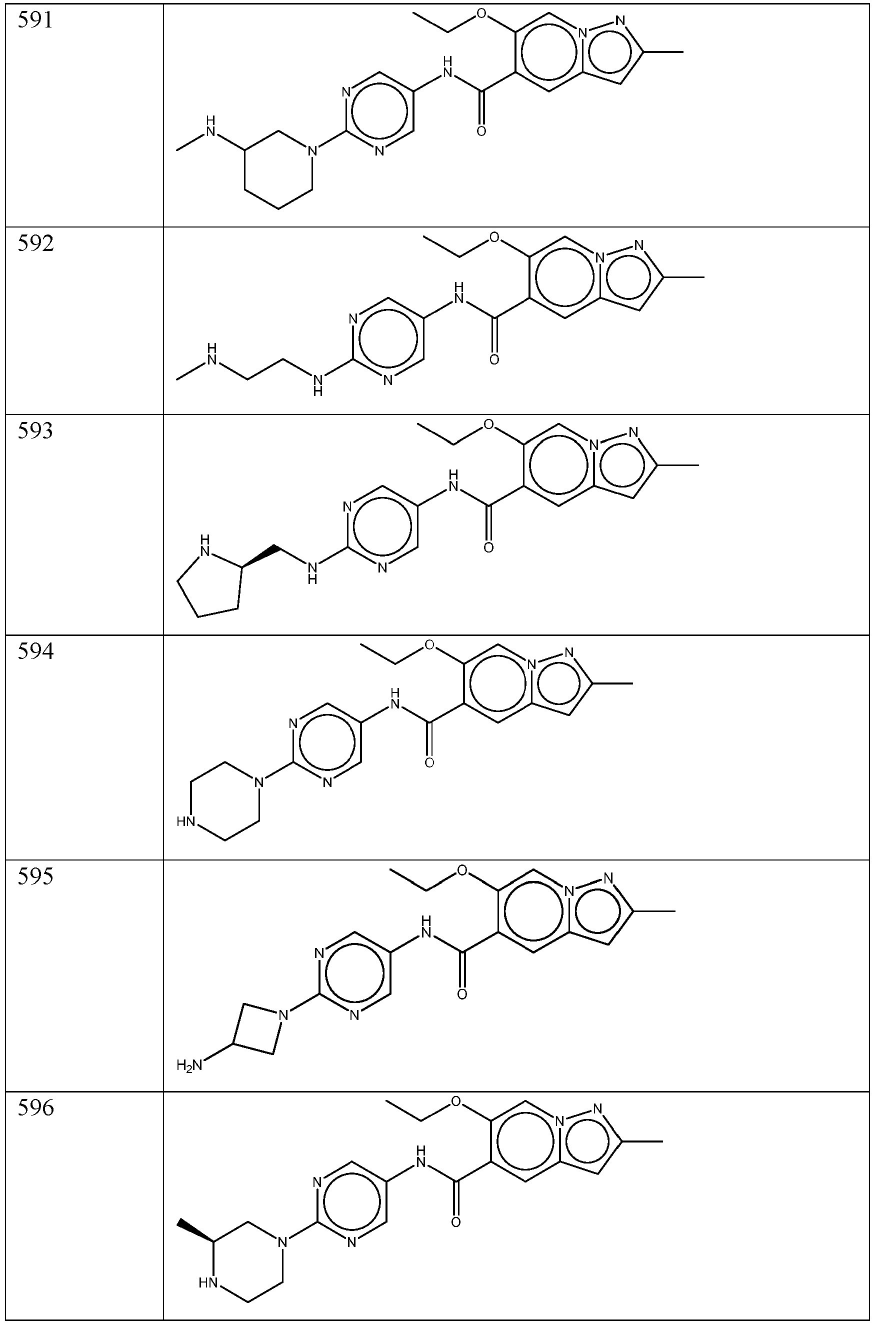

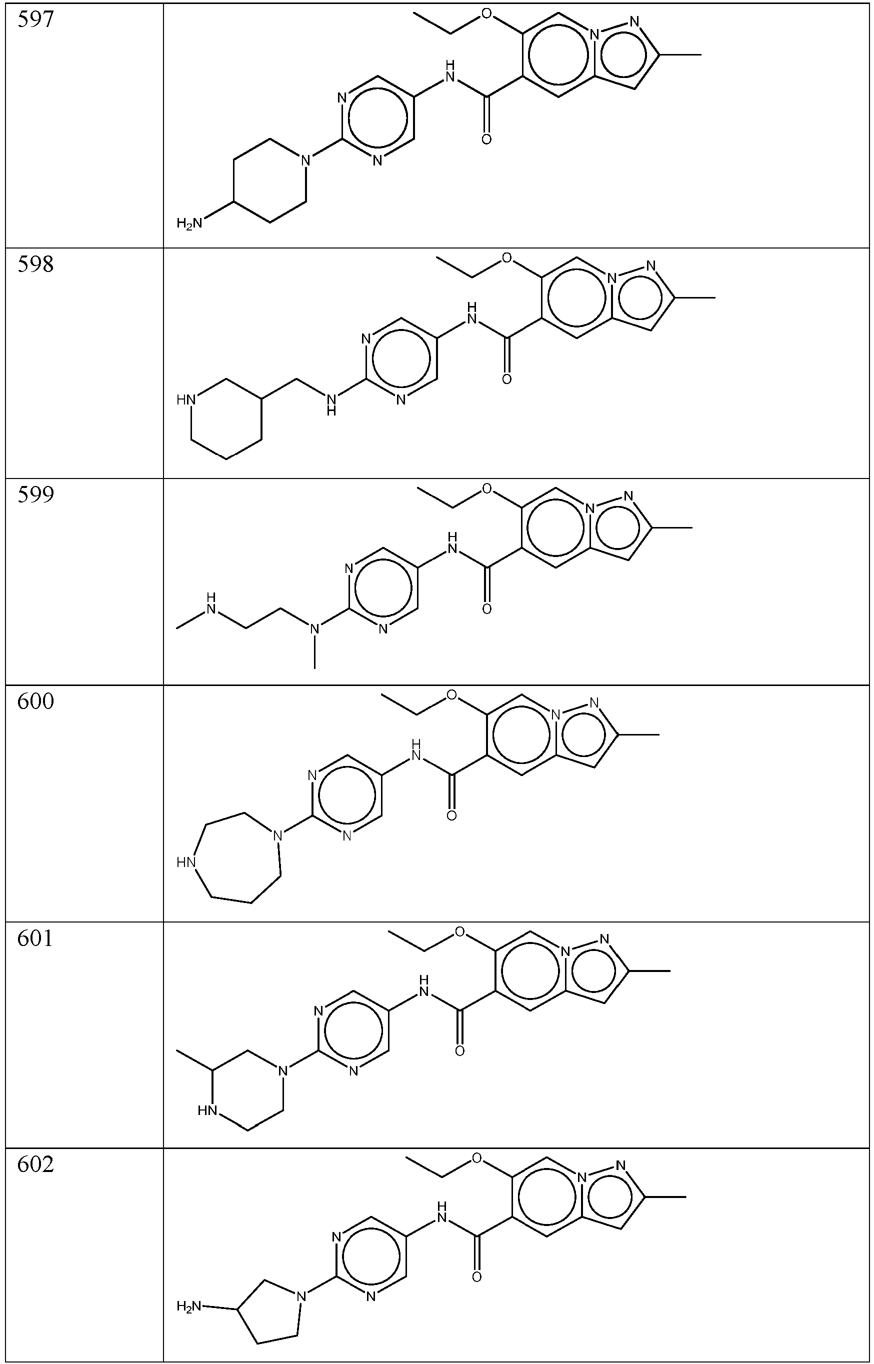

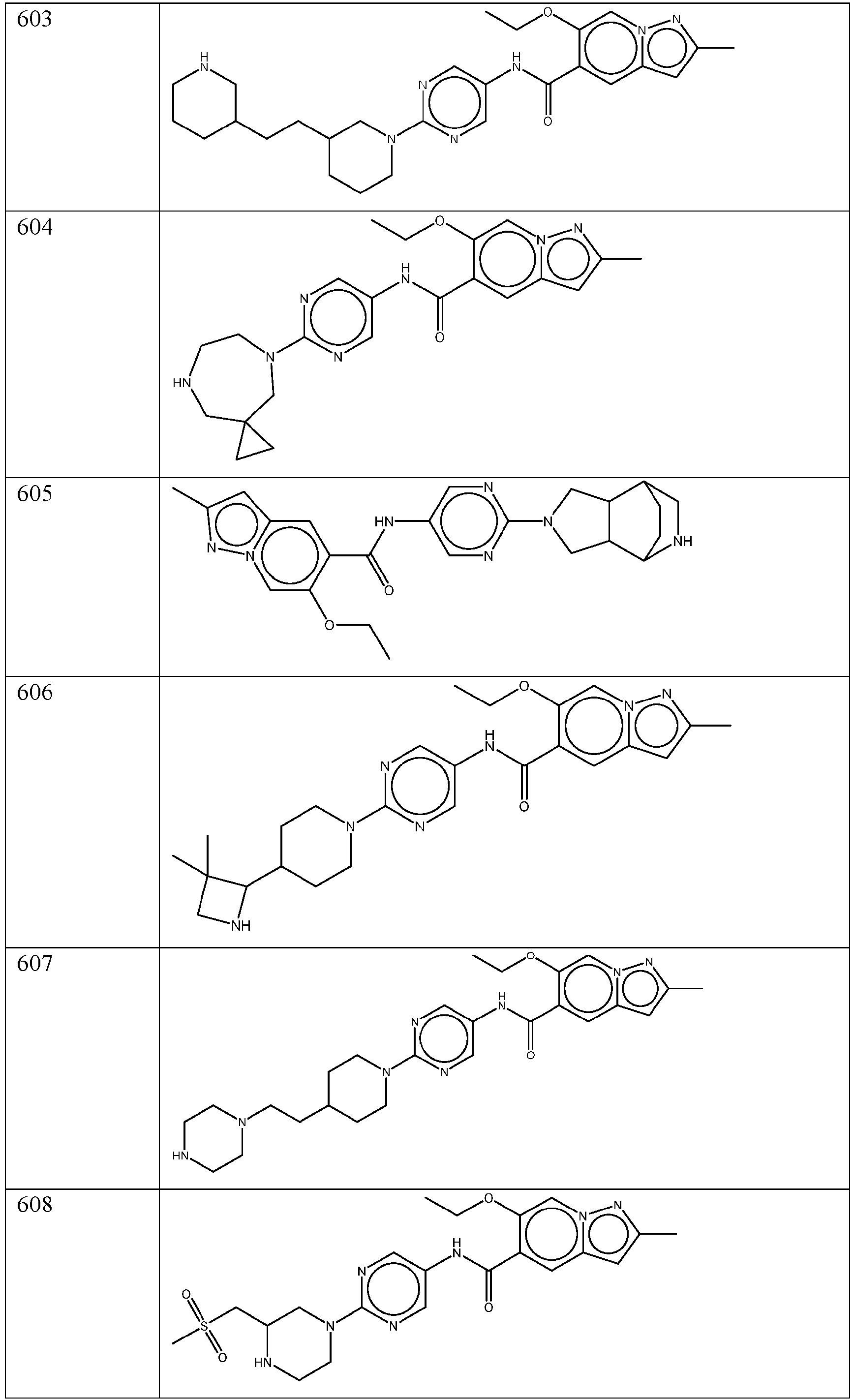

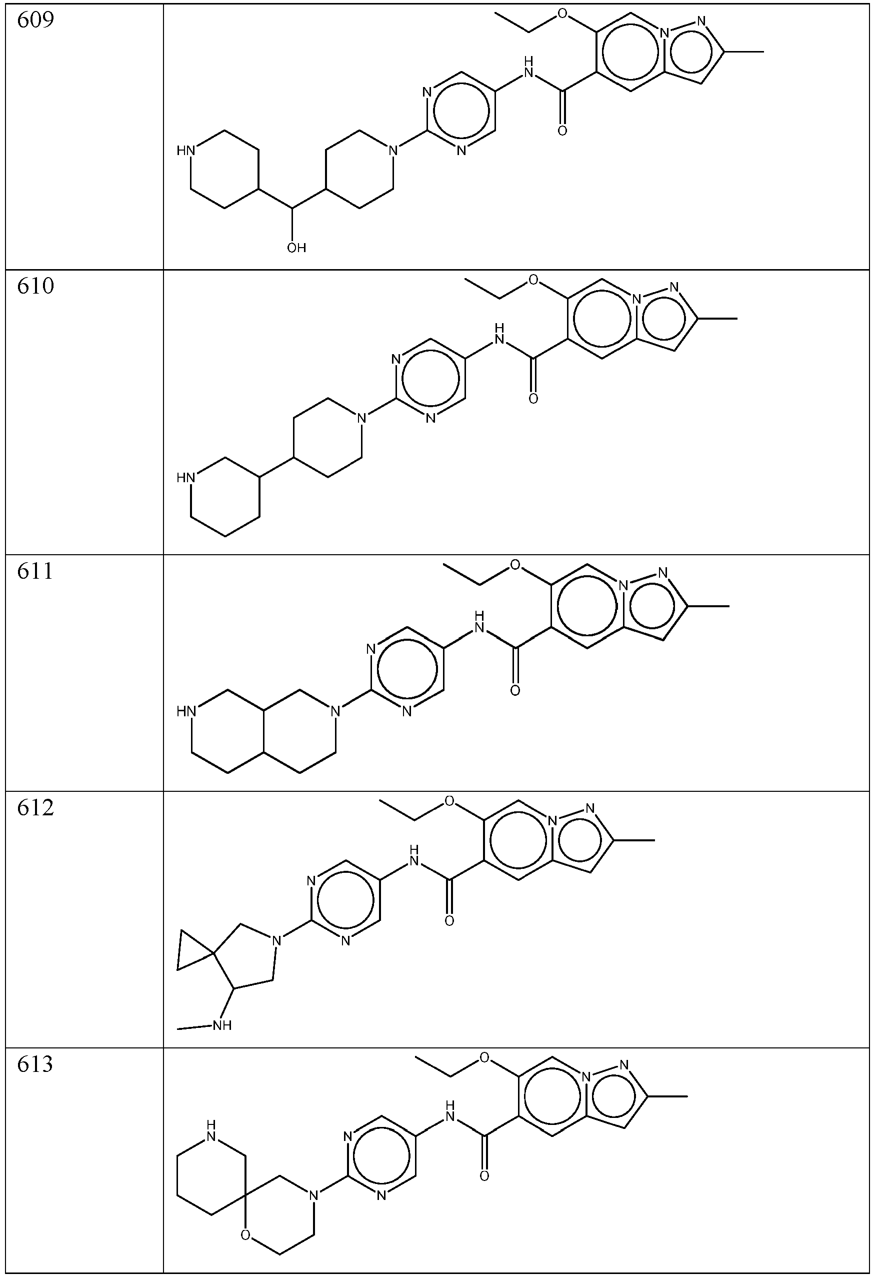

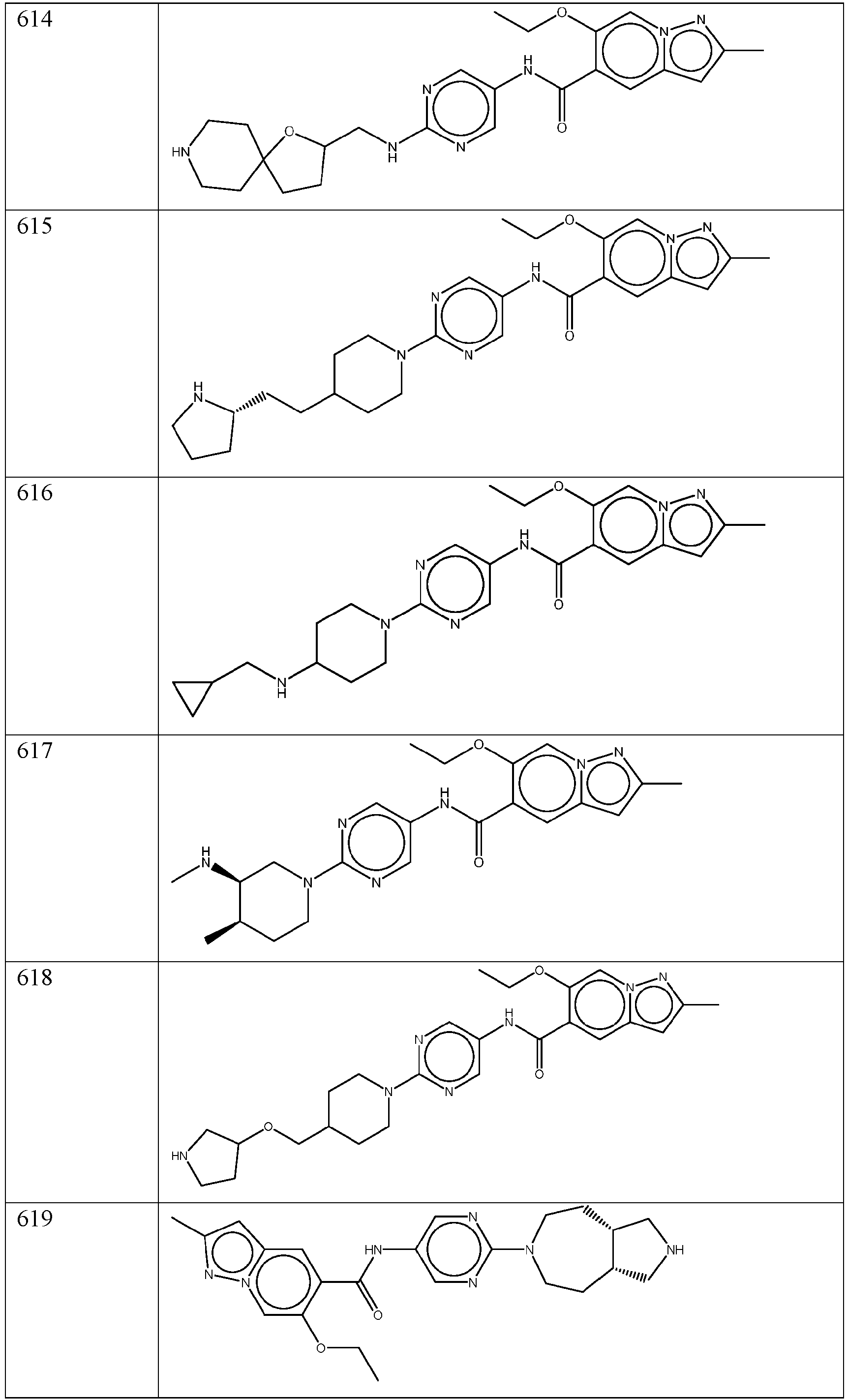

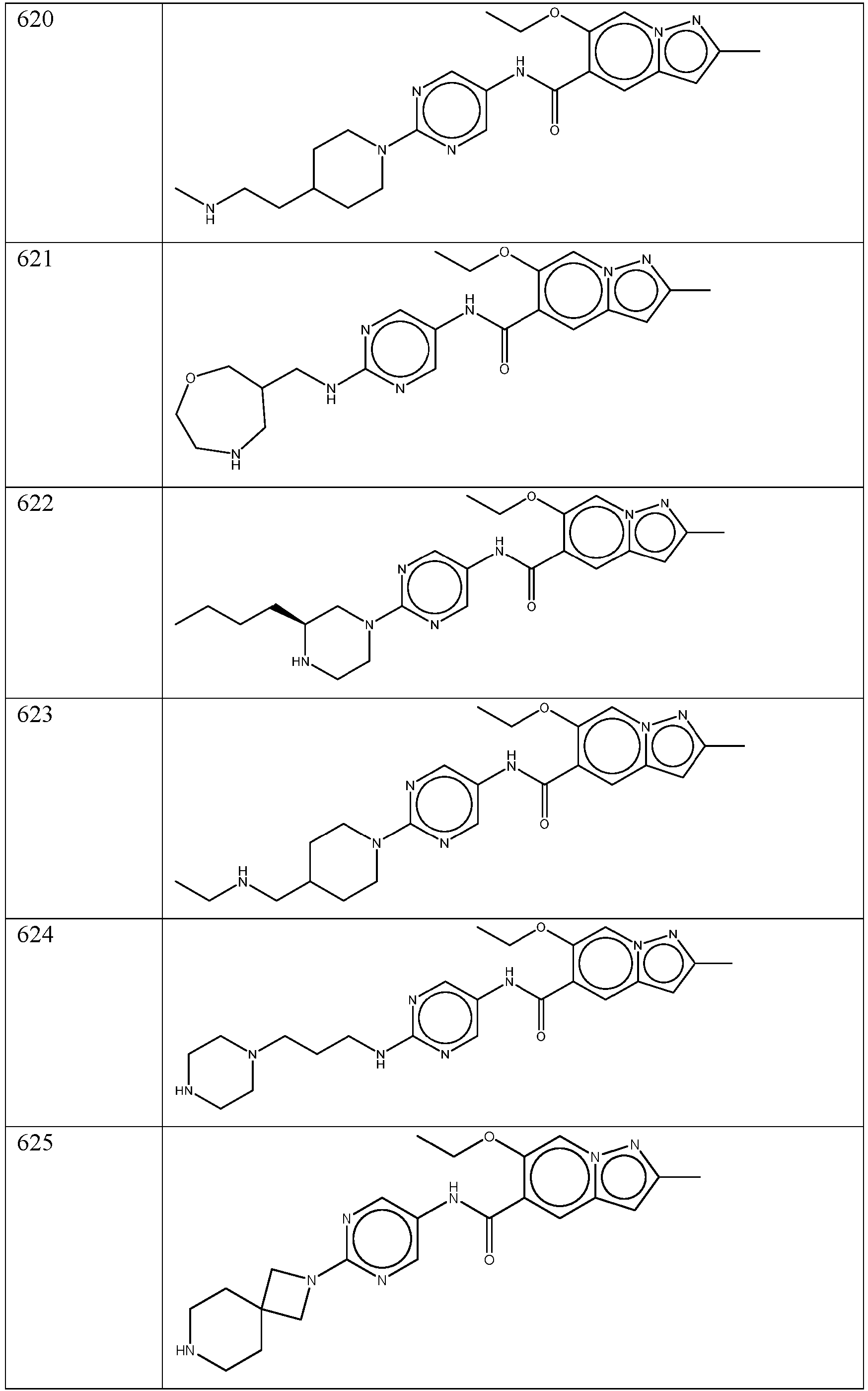

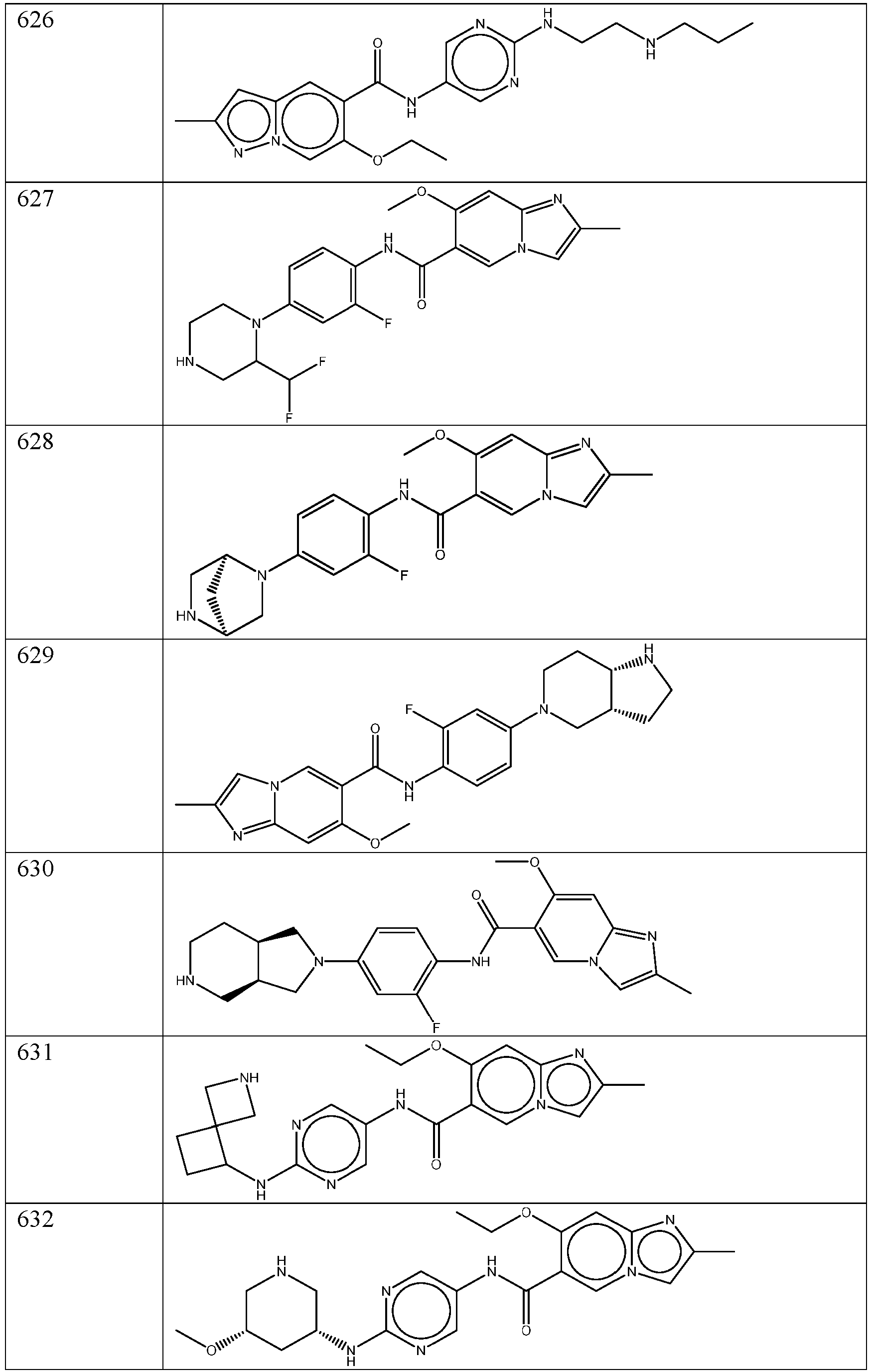

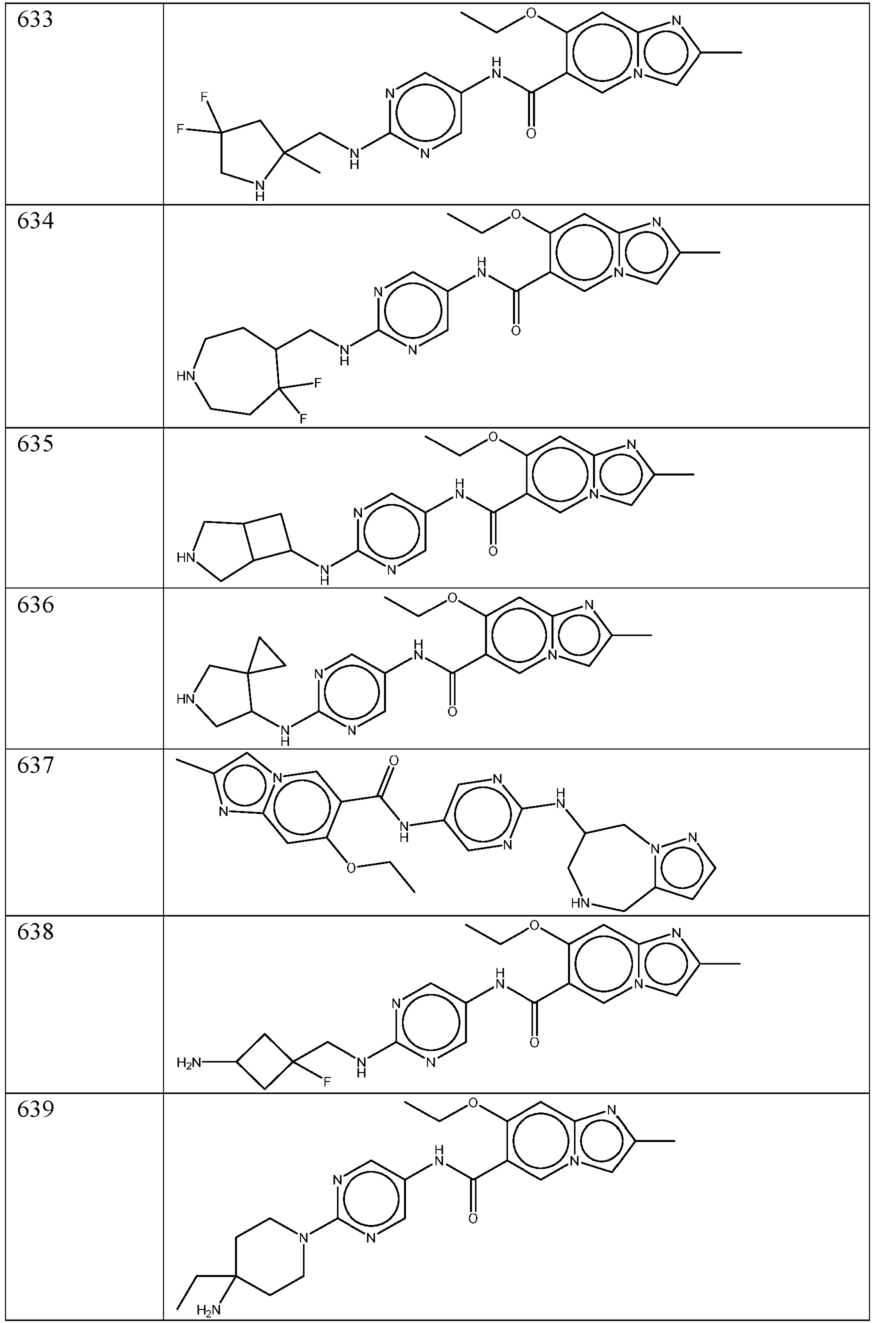

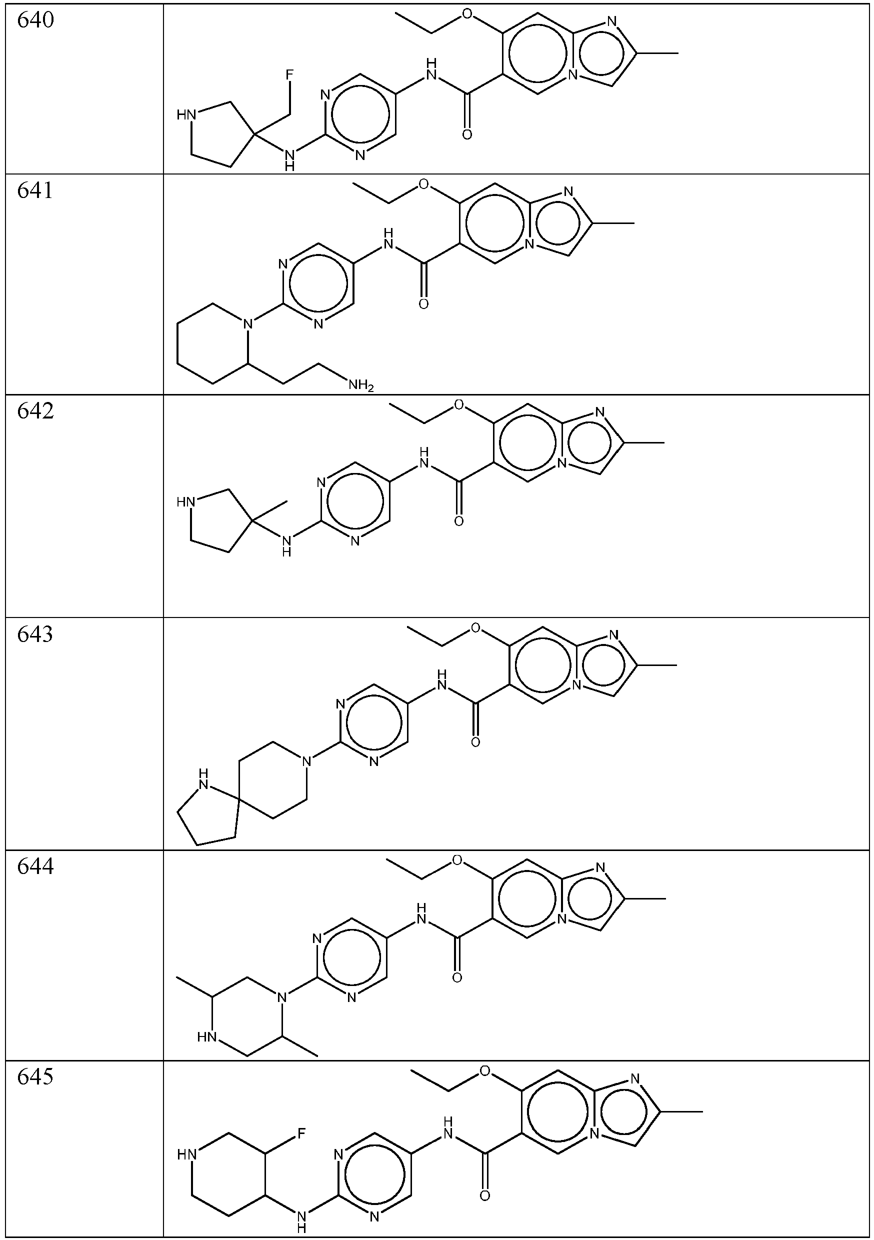

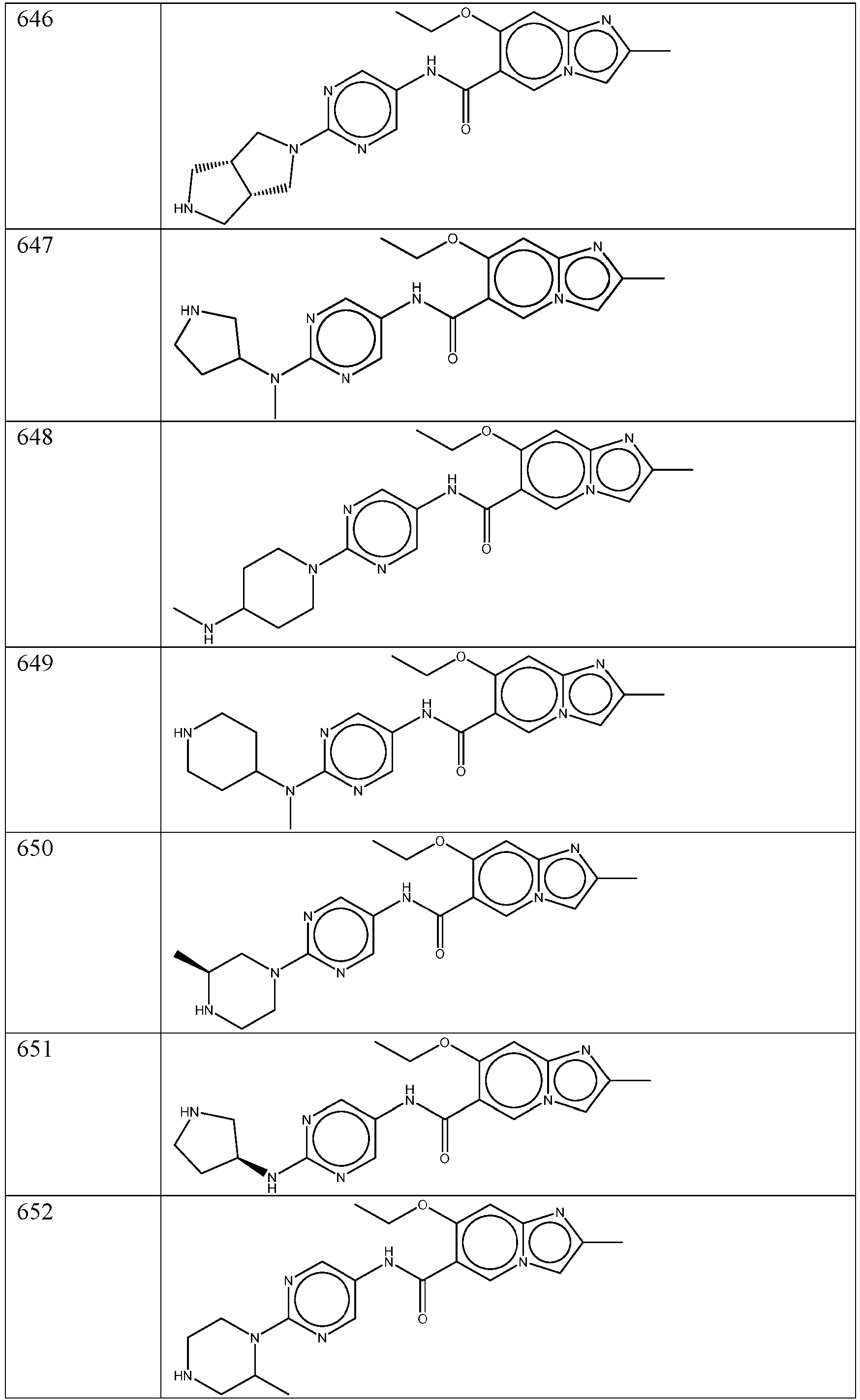

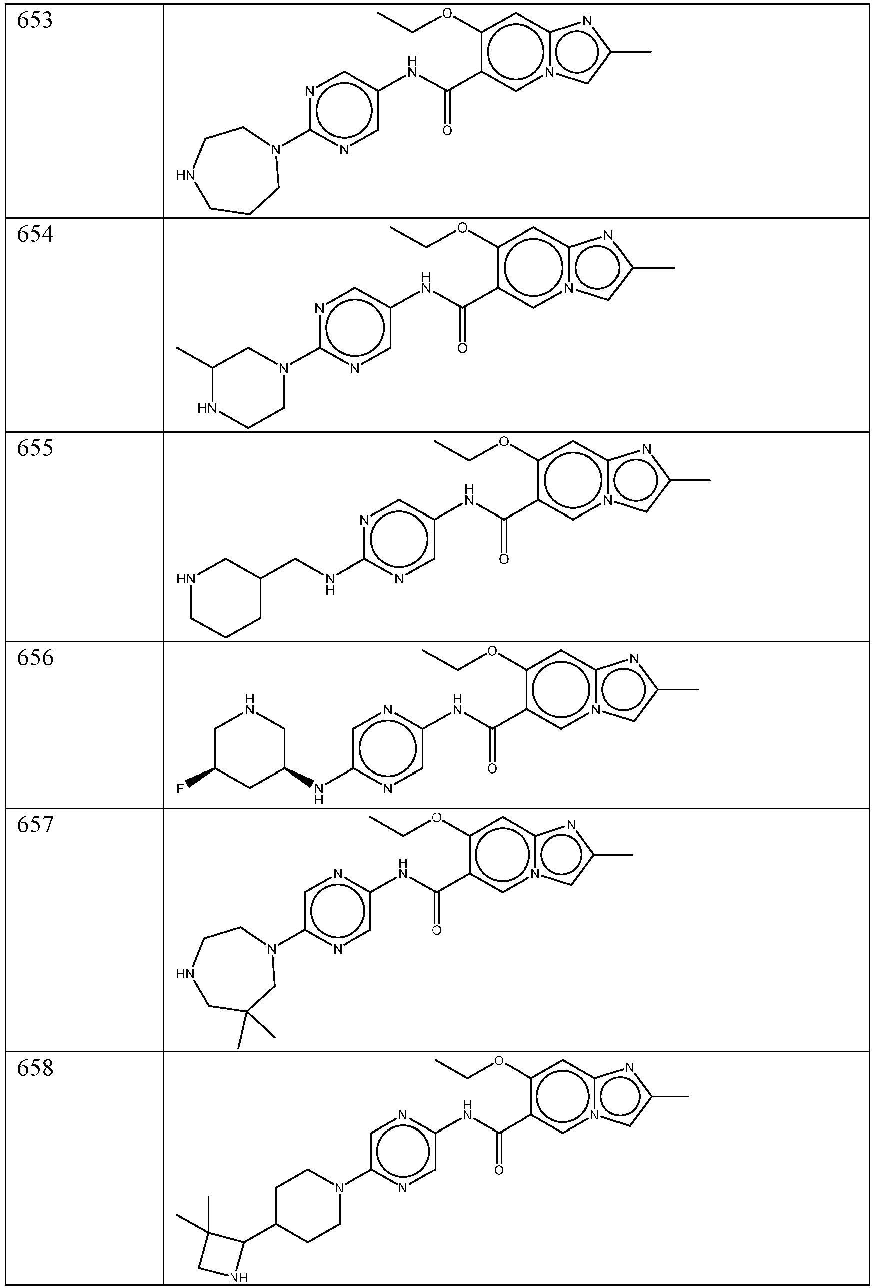

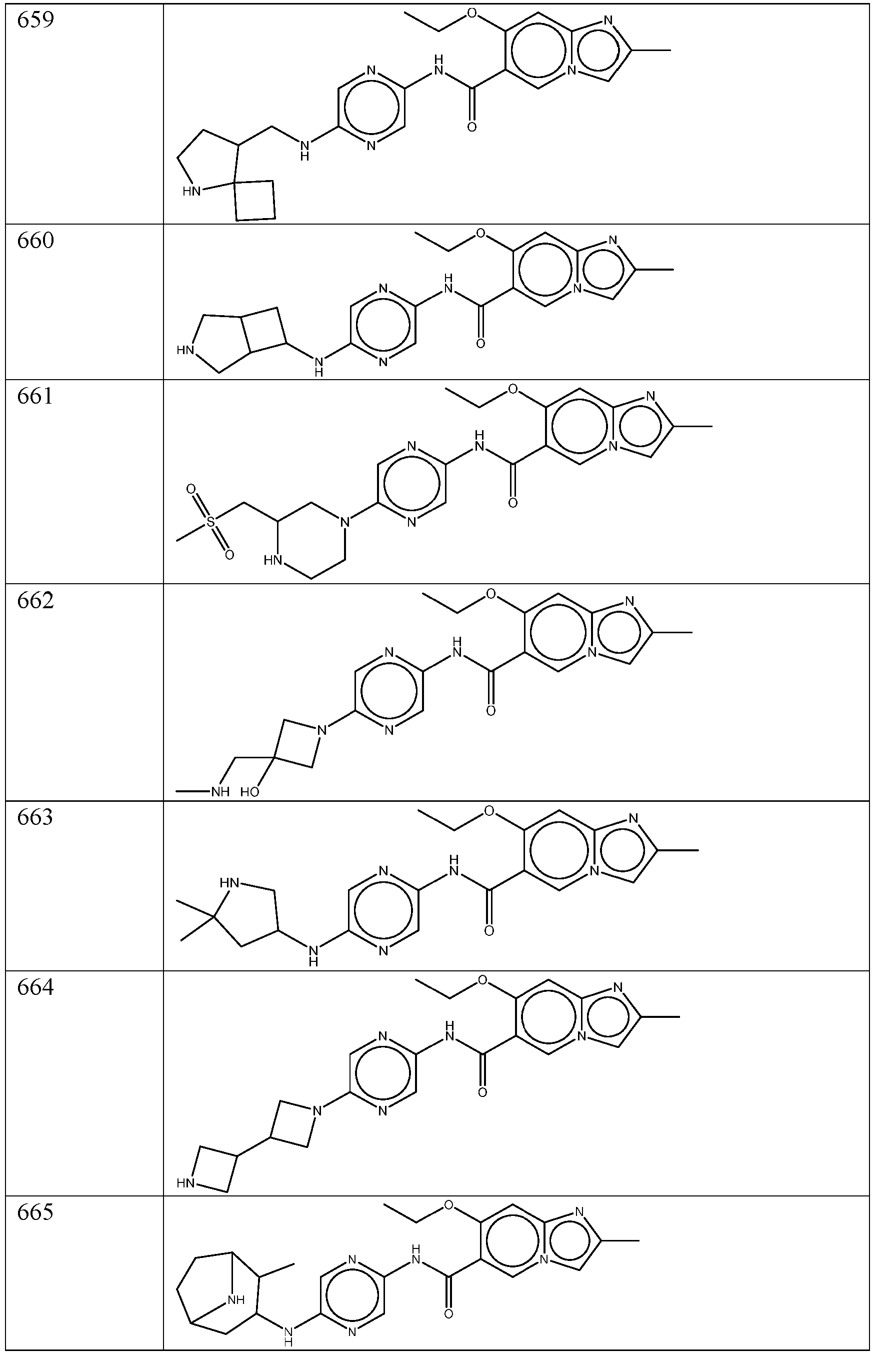

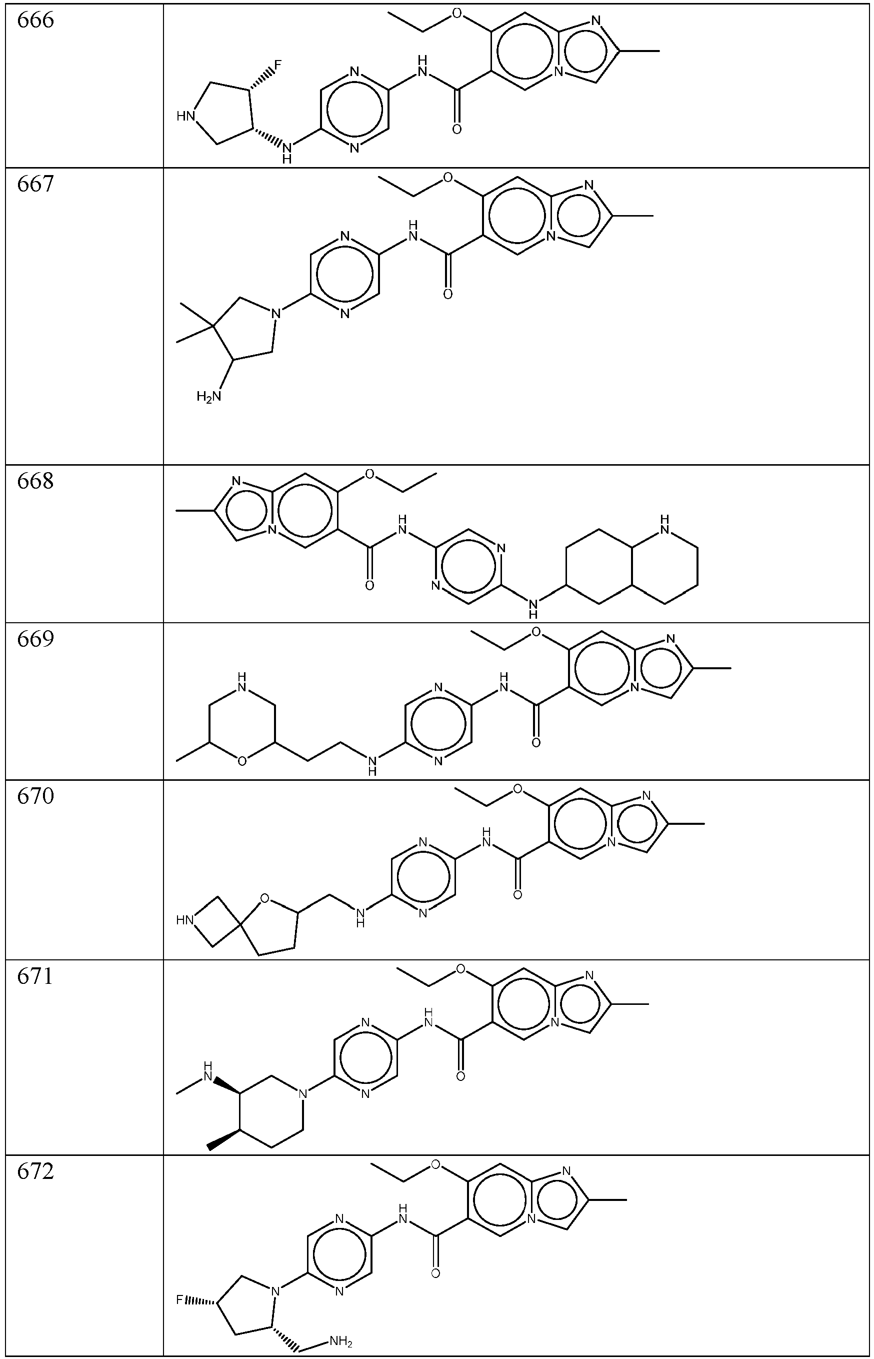

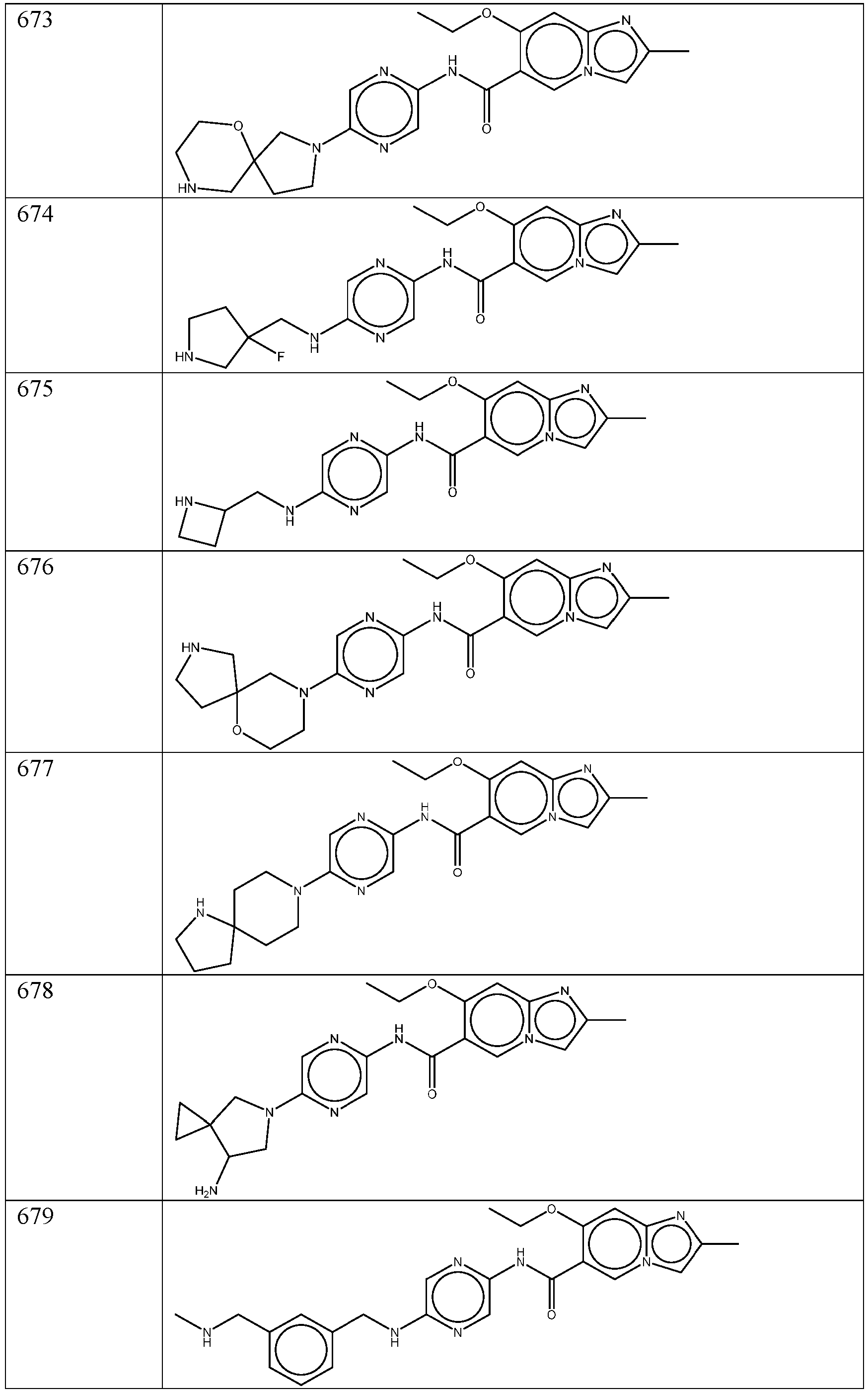

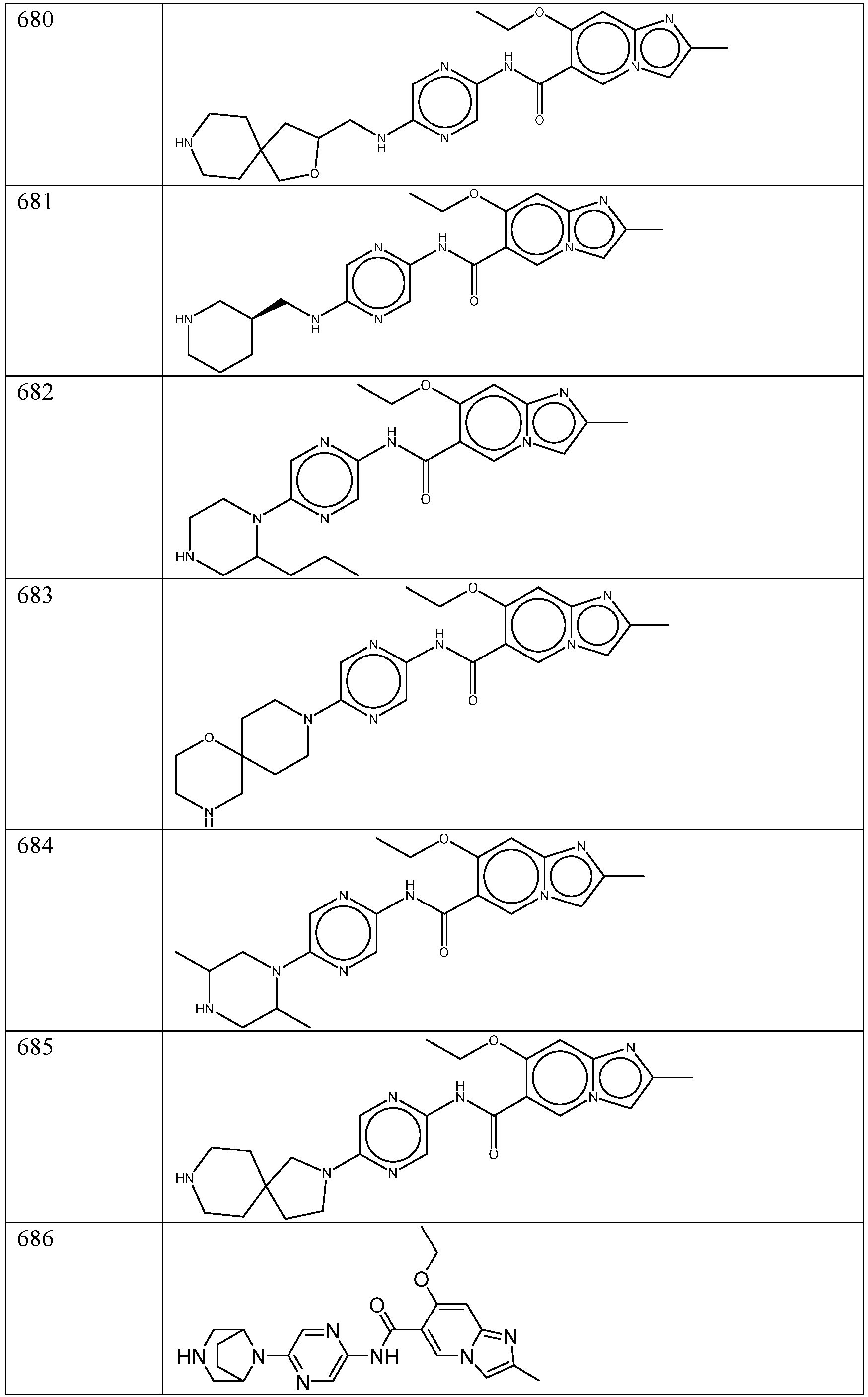

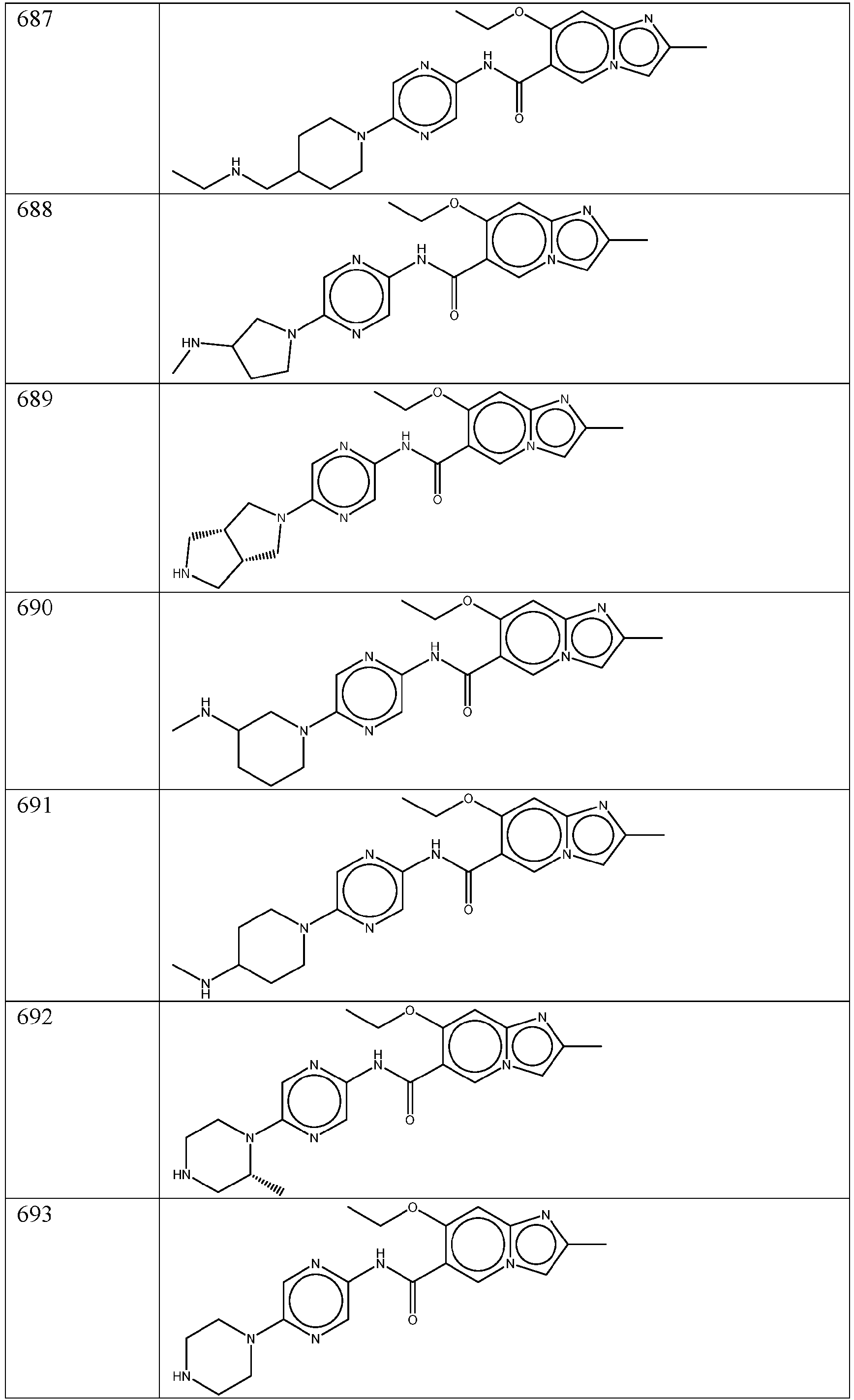

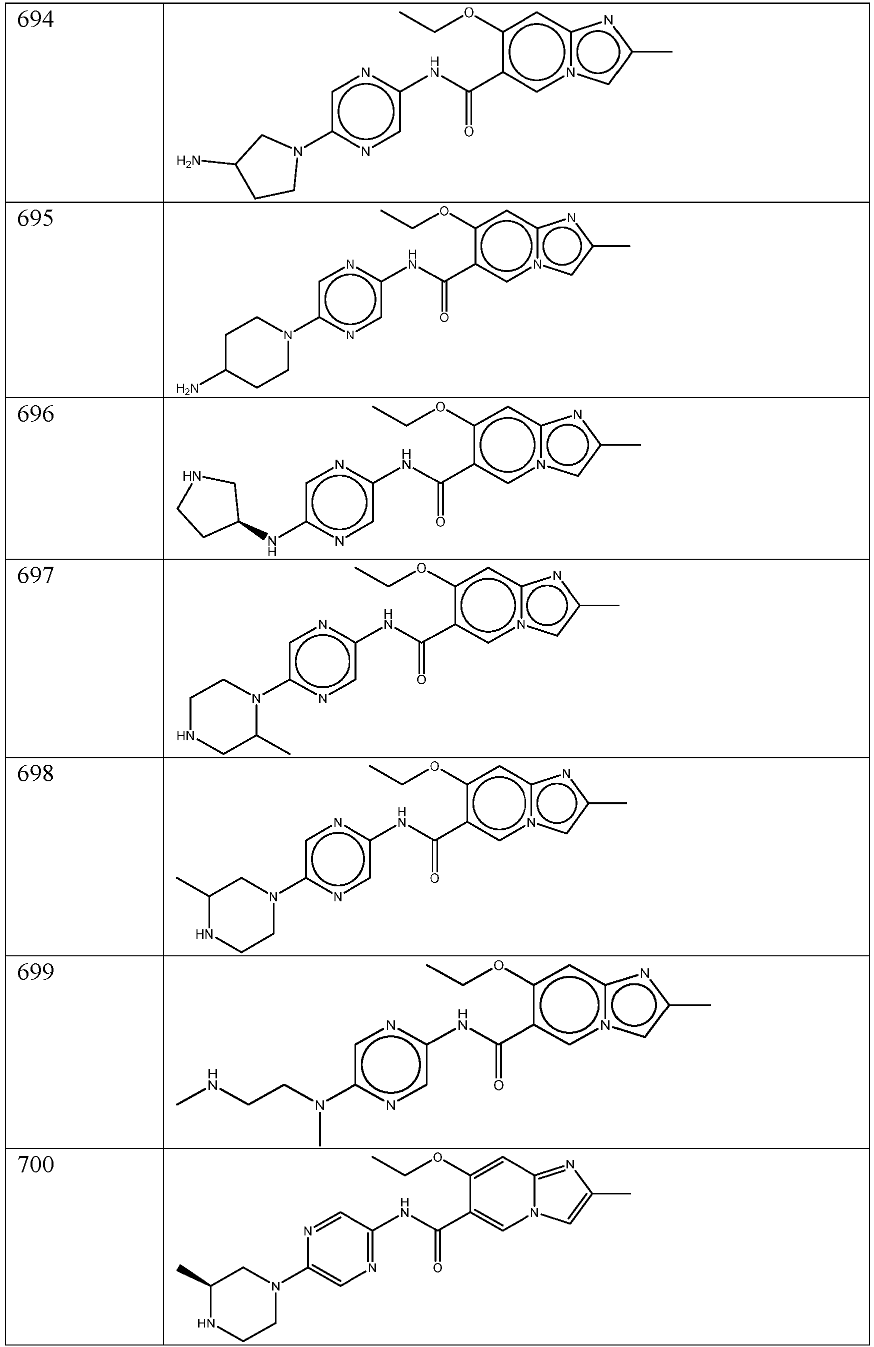

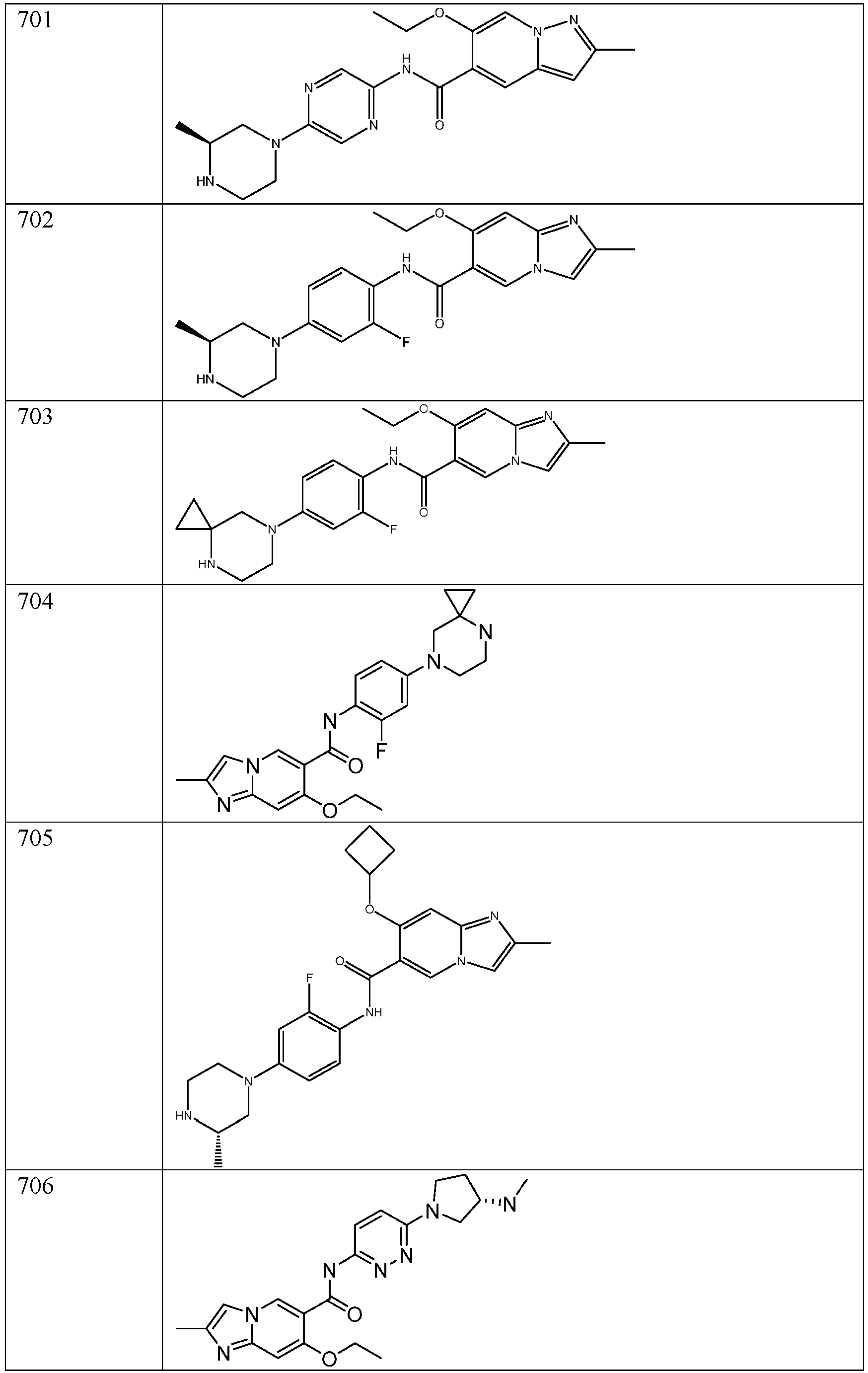

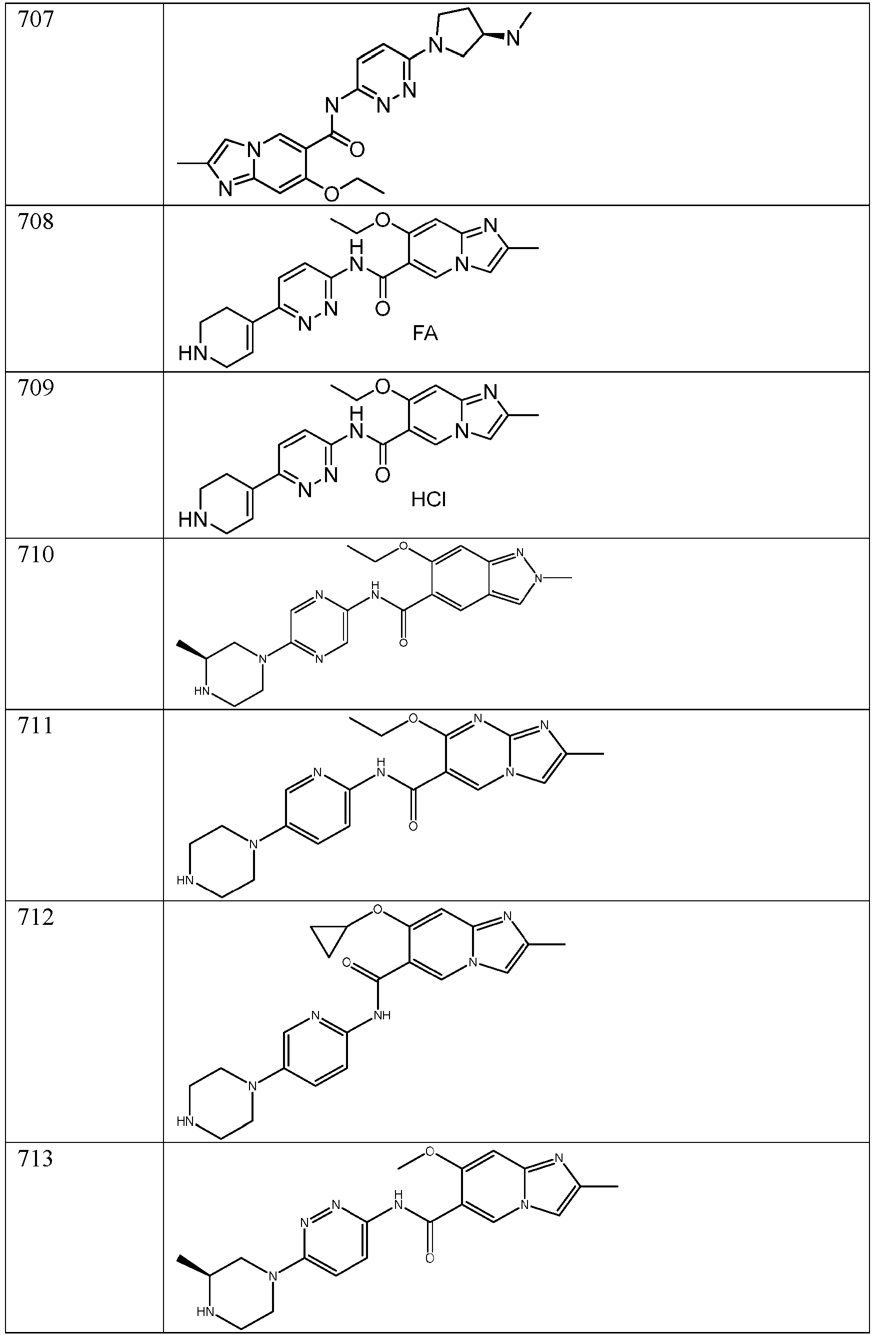

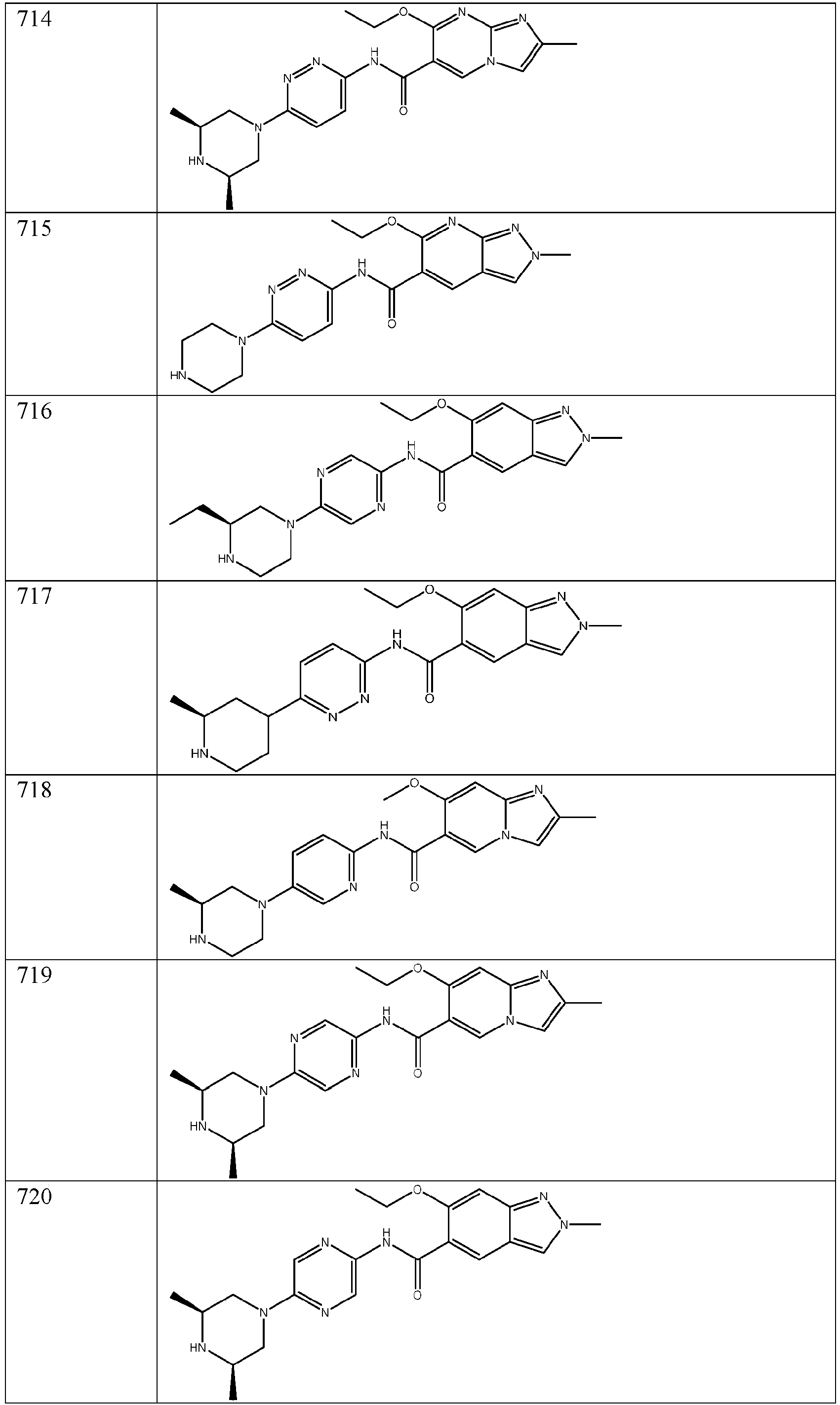

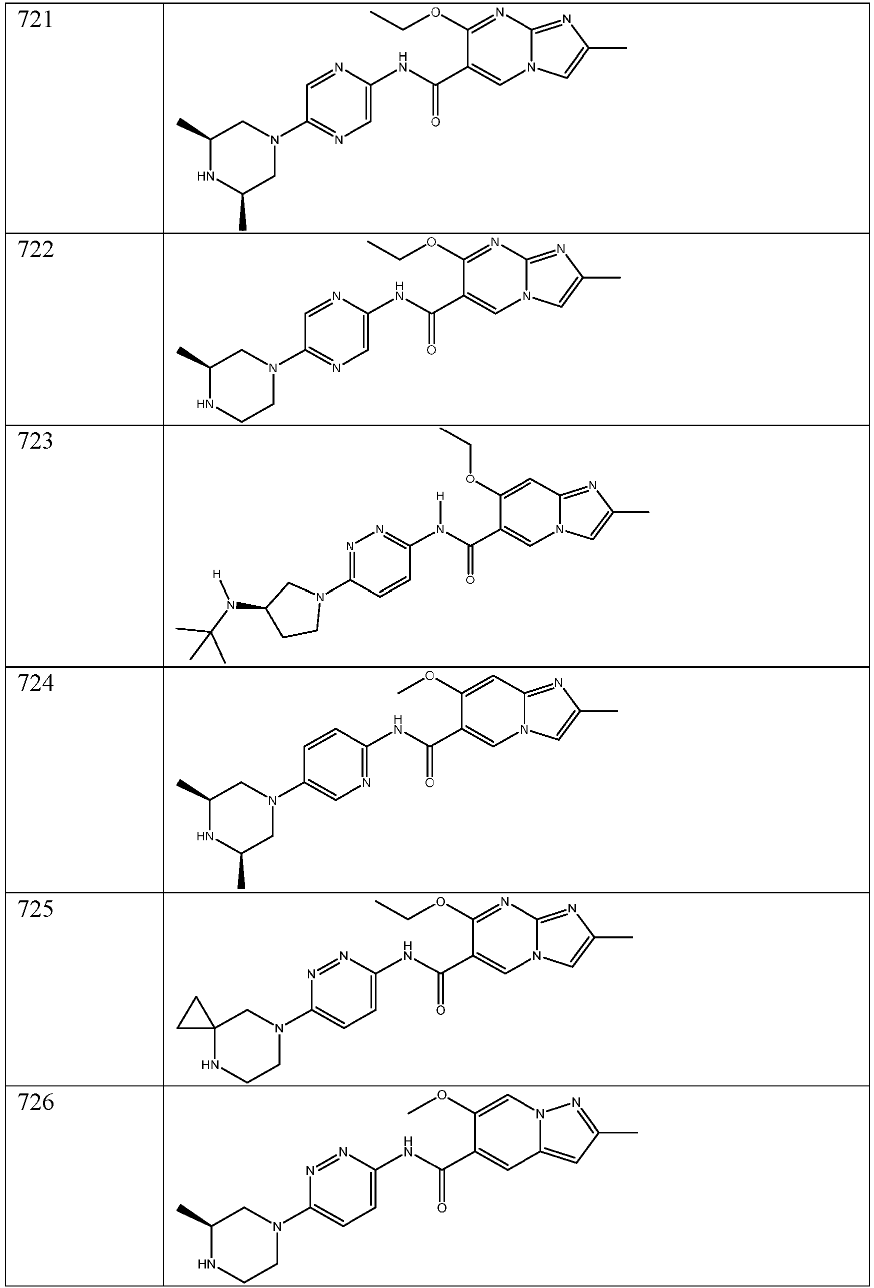

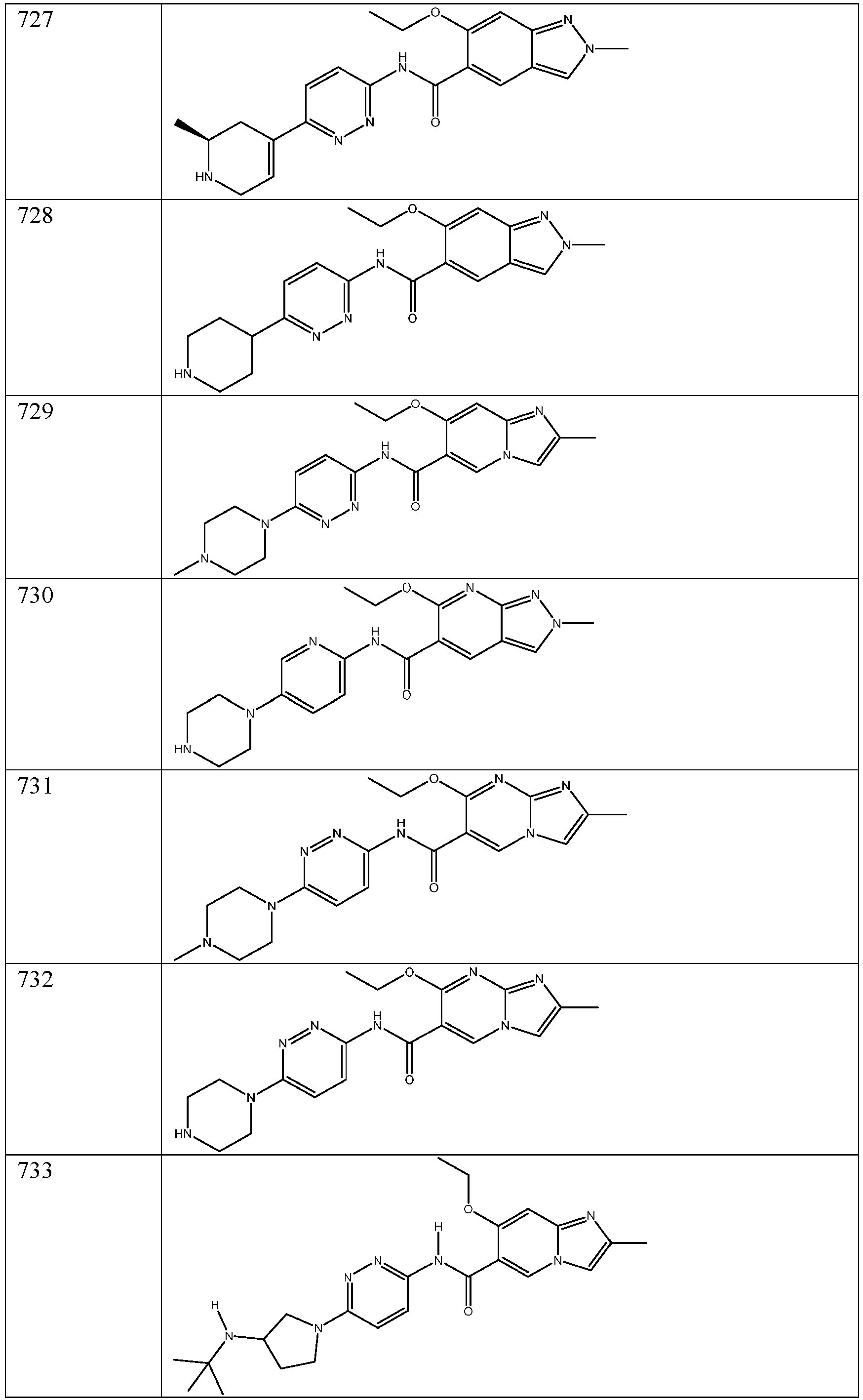

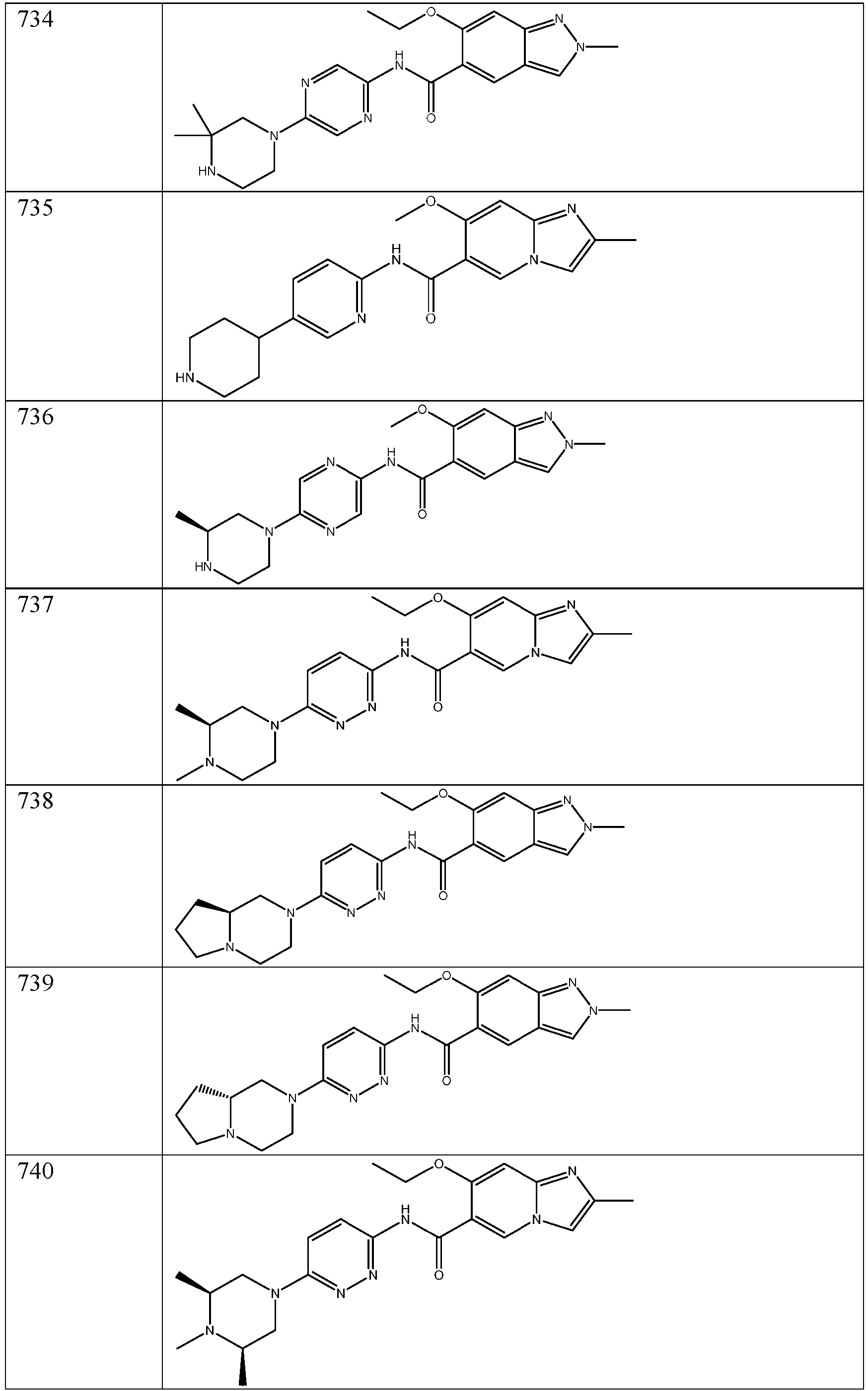

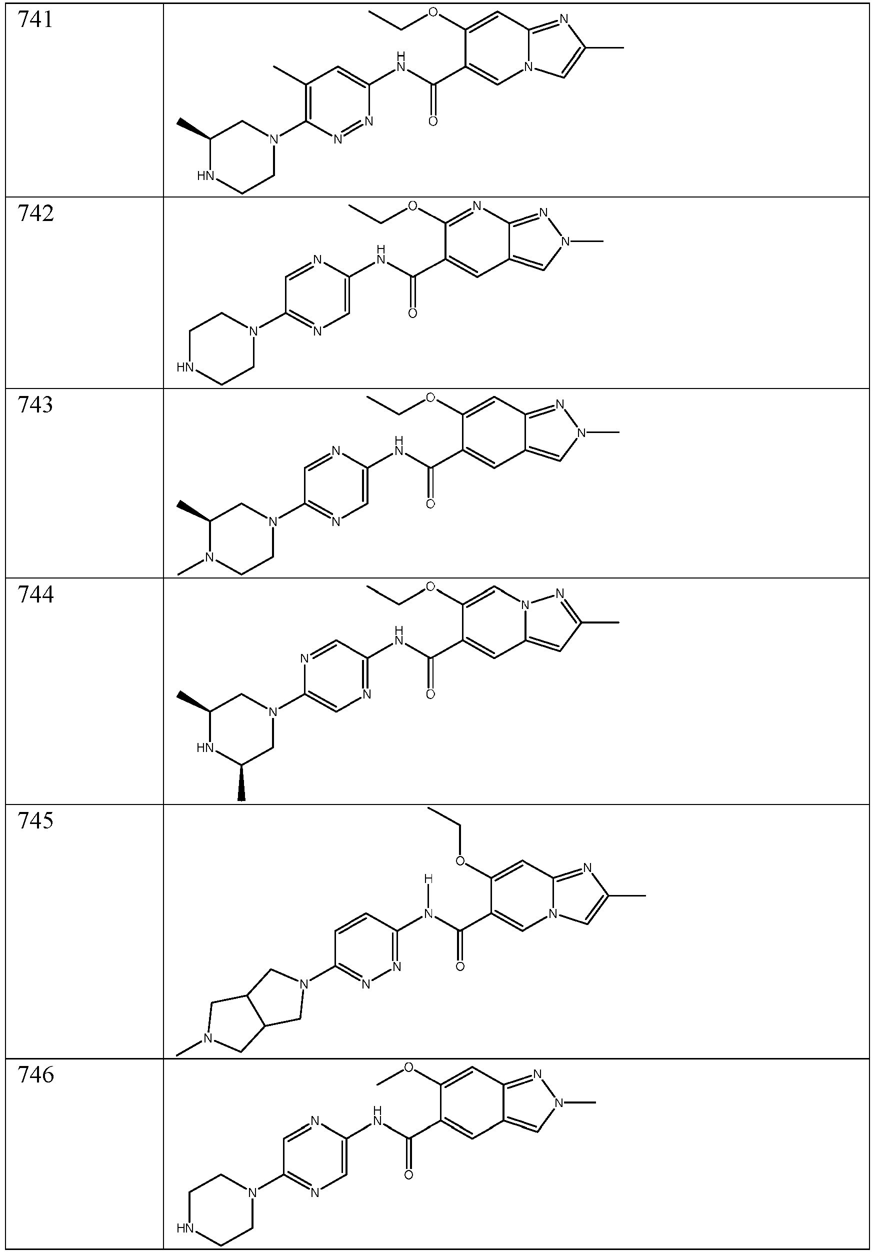

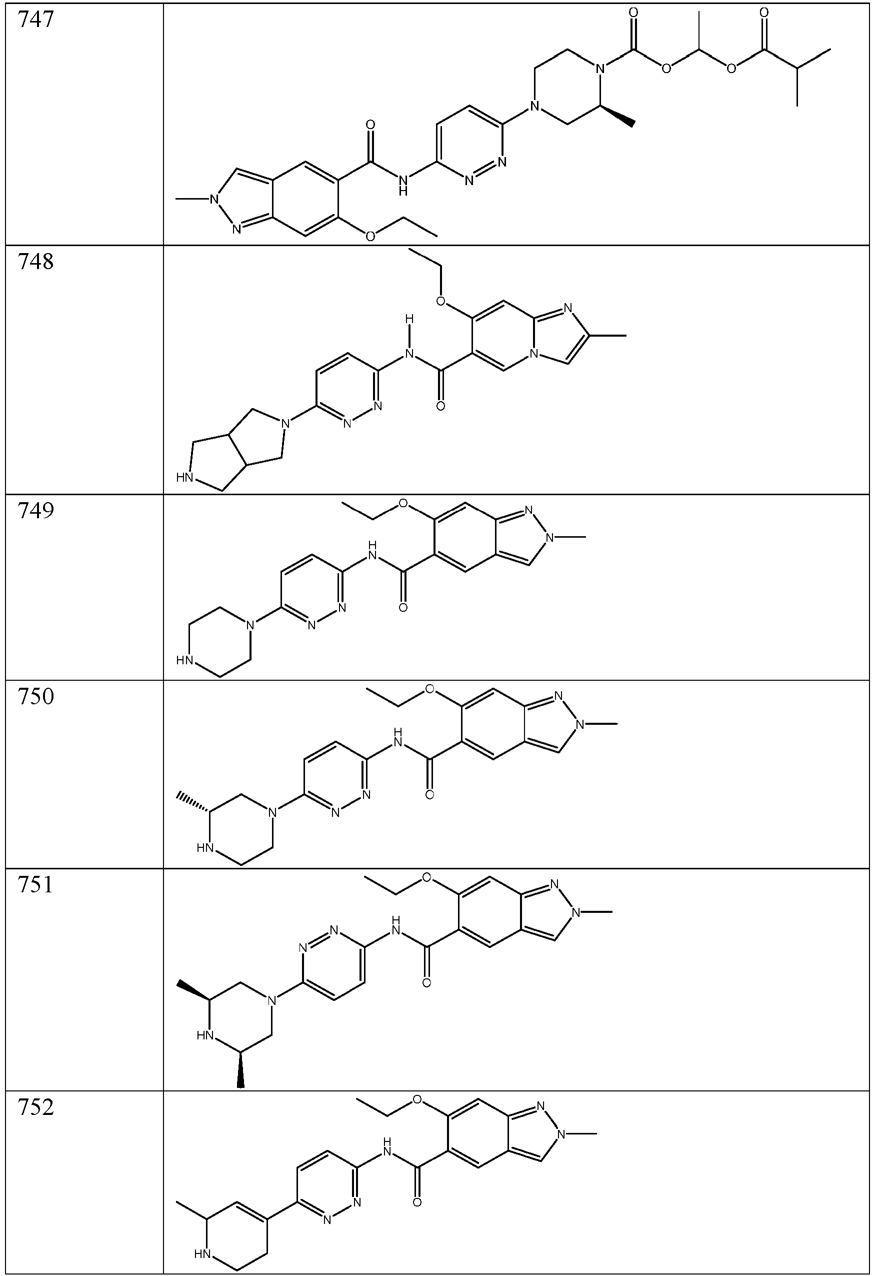

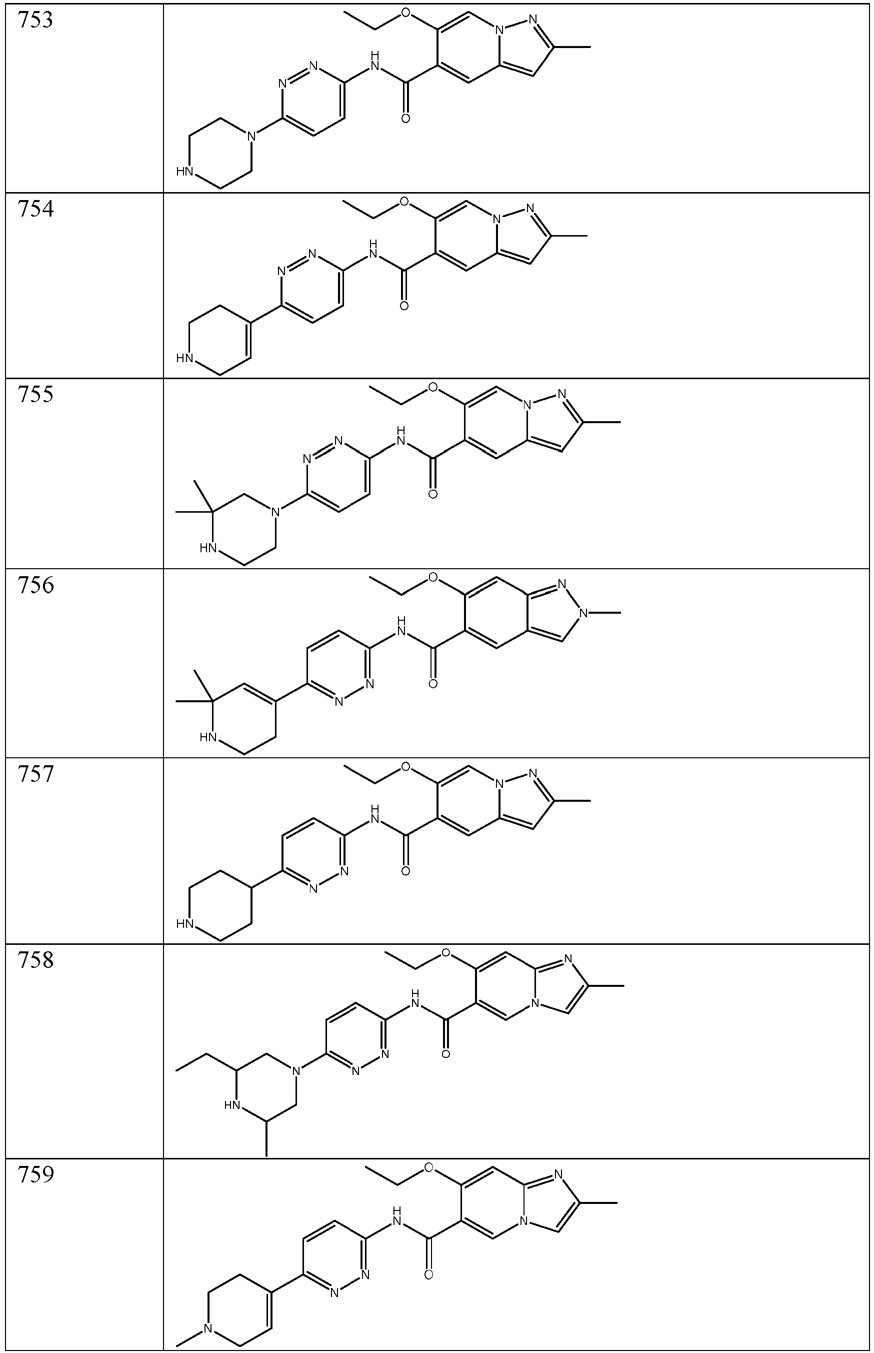

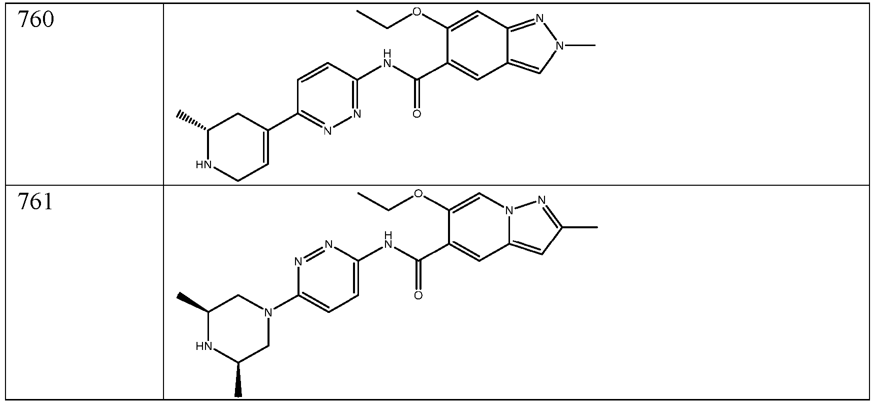

16. A pharmaceutical composition comprising a compound of any one of claims 1-15, or a pharmaceutically acceptable salt thereof and one or more pharmaceutically acceptable excipients.

17. A compound of any one of claims 1-15, or a pharmaceutically acceptable salt, or a pharmaceutical composition of claim 16 for use in a method of treating:
(a) a disorder related to a nucleotide repeat expansion; optionally wherein the nucleotide repeat expansion comprises:
(i) a nucleotide sequence repeated two or more times, wherein the nucleotide sequence is selected from the group consisting of CAG, CAG/CTG, GCG, GCN, CGG, CCG, CCCCGCCCCGCG, GCA, GGGGCC, CTG, GAA, ATTCT, TGGAA, GGCCTG, AAGGG, CCCTCT, ATTTT/ATTTC, and CCCTCT; or
(ii) a trinucleotide sequence repeated two or more times, wherein the trinucleotide sequence is selected from the group consisting of CAG, CTG, CGG, and GCN; or
(b) a disease, wherein the disease is selected from the group consisting of Dentatorubropallidoluysian atrophy, Huntington's disease, Spinal and bulbar muscular atrophy, SCA1 (Spinocerebellar ataxia Type 1), SCA2 (Spinocerebellar ataxia Type 2), SCA3 (Spinocerebellar ataxia Type 3 or Machado-Joseph disease), SCA6 (Spinocerebellar ataxia Type 6), SCA7 (Spinocerebellar ataxia Type 7), SCA12 (Spinocerebellar ataxia Type 12), SCA17 (Spinocerebellar ataxia Type 17), FRAXA (Fragile X syndrome), FXTAS (Fragile X-associated tremor/ataxia syndrome), FRAXE (Fragile XE mental retardation), Baratela-Scott syndrome, FRDA (Friedreich's ataxia), DM1 (Myotonic dystrophy Type 1), DM2 (Myotonic dystrophy Type 2) SCA8 (Spinocerebellar ataxia Type 8), Fuchs endothelial corneal dystrophy, Desbuquois dysplasia, amyotrophic lateral sclerosis, frontotemporal dementia; optionally wherein the disease is:
(i) Huntington's disease;
(ii) Myotonic dystrophy1; or
(iii) selected from the group consisting of FRAXA (Fragile X syndrome), FXTAS (Fragile X-associated tremor/ataxia syndrome), FRAXE (Fragile XE mental retardation).

## Patentansprüche

1. Verbindung der Formel (I): 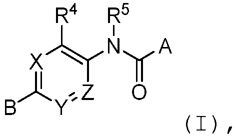 oder pharmazeutisch unbedenkliches Salz davon, wobei:
X = N oder CR¹, Y = N oder CR², Z = N oder CR³, R¹, R², R³ und R⁴ jeweils unabhängig aus der aus H, Halogen, Hydroxy, Cyano, -COOH, -C(O)-C₁-C₆-Alkyl, - C(O)O-C₁-C₆-Alkyl, C₁-C₆-Alkyl, C₂-C₆-Alkenyl, C₂-C₆-Alkinyl, C₁-C₆-Alkoxy, C₁-C₆-Cycloalkyloxy, C₃-C₈-Cycloalkyl, NH₂, NH (C₁-C₆-Alkyl) , N (C₁-C₆-Alkyl) ₂, - NHC(O)-C₁-C₆-Alkyl, -N (C₁-C₆-Alkyl) -C (O) -C₁-C₆-alkyl, - C(O)-NH₂, -C (O) -NH (C₁-C₆-Alkyl) und -C(O)-N(C₁-C₆-Alkyl) ₂ bestehenden Gruppe ausgewählt sind, wobei Alkyl, Alkenyl, Alkinyl und Alkoxy gegebenenfalls durch ein oder mehrere Halogen, Hydroxyl, Methoxy, C₃-C₈-Cycloalkyl oder NH₂ substituiert sind,
R⁵ für H, C₁-C₆-Alkyl oder C₁-C₆-Halogenalkyl steht,
A ausgewählt ist aus der Gruppe bestehend aus 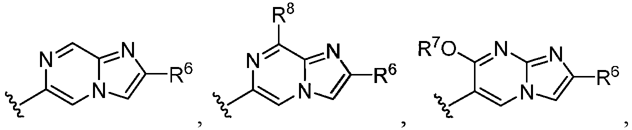 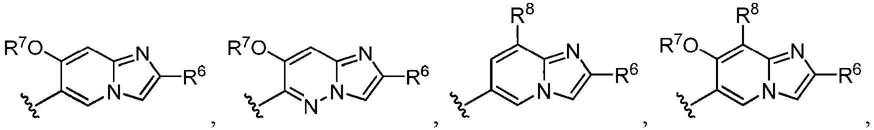 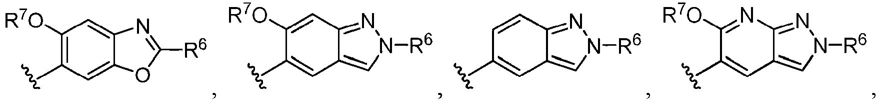 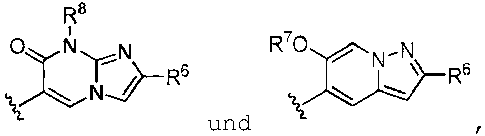
wobei A gegebenenfalls durch 1-4 R⁹ substituiert ist,
R⁶ für C₁-C₃-Alkyl oder C₁-C₃-Halogenalkyl steht,
R⁷ für H, C₁-C₆-Alkyl, C₁-C₆-Halogenalkyl, C₁-C₆-Cycloalkyl oder Heterocycloalkyl steht, wobei Heterocycloalkyl gegebenenfalls durch 1-3 unabhängig aus Halogen und C₁-C₆-Alkyl ausgewählte Substituenten substituiert ist,
R⁸ für Halogen, C₁-C₆-Alkyl, C₁-C₆-Alkoxy, C₁-C₆-Halogenalkyl oder C₁-C₆-Cycloalkyl steht,
R⁹ jeweils unabhängig aus der aus Halogen, Hydroxy, Cyano, -COOH, -C(O)-C₁-C₆-Alkyl, -C(O)O-C₁-C₆-Alkyl, C₁-C₆-Alkyl, C₂-C₆-Alkenyl, C₂-C₆-Alkinyl, C₁-C₆-Alkoxy, C₃-C₈-Cycloalkyloxy, C₃-C₈-Cycloalkyl, NH₂, NH(C₁-C₆-Alkyl), N (C₁-C₆-Alkyl) ₂, -NHC (O) -C₁-C₆-Alkyl, -N(C₁-C₆-Alkyl) - C(O)-C₁-C₆-alkyl, -C(O)-NH₂, -C(O)-NH(C₁-C₆-Alkyl) und - C (O)-N (C₁-C₆-Alkyl) ₂ bestehenden Gruppe ausgewählt ist, wobei Alkyl, Alkenyl, Alkinyl und Alkoxy gegebenenfalls durch ein oder mehrere Halogen, Hydroxyl, Methoxy, C₃-C₈-Cycloalkyl oder NH₂ substituiert sind,
B für gegebenenfalls durch 1 bis 6 R¹² substituiertes Heterocycloalkyl steht, das nicht über ein Stickstoffatom mit der Formel (I) verknüpft ist, oder
B für NR¹⁰R¹¹ steht, wobei
R¹⁰ für - (CH₂)₀₋₃-Aryl, - (CH₂)₀₋₃-Heteroaryl, - (CH₂)₀₋₃-Heterocycloalkyl, das mindestens 1 Stickstoffringatom umfasst, oder C₁-C₈-Heteroalkyl, das mindestens ein Stickstoffatom umfasst, steht, wobei jedes R¹⁰ gegebenenfalls durch 1 bis 6 R¹² substituiert ist, und R¹¹ für Wasserstoff, C₁₋₇-Alkyl, C₁₋₇-Halogenalkyl oder C₃-₈-Cycloalkyl steht oder
R¹⁰ und R¹¹ zusammen mit dem Stickstoffatom, an das sie gebunden sind, ein Heterocycloalkyl bilden, das insgesamt 1, 2 oder 3 Stickstoffringatome und 0 oder 1 zusätzliche, aus O und S ausgewählte Ringheteroatome umfasst, wobei das Heterocycloalkyl gegebenenfalls durch 1 bis 6 R¹² substituiert ist,
R¹² jeweils unabhängig aus der aus Halogen, Hydroxy, Cyano, -COOH, -C(O)-C₁-C₆-Alkyl, -C(O)O-C₁-C₆-Alkyl, C₁-C₆-Alkyl, C₁-C₆-Heteroalkyl, C₂-C₆-Alkenyl, C₂-C₆-Alkinyl, C₁-C₆-Alkoxy, - (CH₂)₀-₂-C₃-C₈-Cycloalkyl, - (CH₂)₀-₂-SO₂-C₁-C₆-Alkyl, C₁-C₆-Heteroalkylen-C₃-C₆-cycloalkyl, -O-C₃-C₈-Cycloalkyl, 4- bis 7-gliedrigem monocyclischem Heterocycloalkyl, C₁-C₆-Heteroalkylen- (4- bis 7-gliedriges monocylisches Heterocycloalkyl), -O-(4- bis 7-gliedriges monocylisches Heterocycloalkyl), - (CH₂) ₀-₂-(4- bis 7-gliedriges monocylisches Heterocycloalkyl), NH₂, NH(C₁-C₆-Alkyl) , N(C₁-C₆-Alkyl) ₂, -NHC(O)-C₁-C₆-Alkyl, -N (C₁-C₆-Alkyl) -C(O)-C₁-C₆-alkyl, -C(O)-NH₂, -C(O)-NH(C₁-C₆-Alkyl) und -C(O)-N(C₁-C₆-Alkyl) ₂ bestehenden Gruppe ausgewählt ist, wobei Alkyl, Alkenyl, Alkinyl und Alkoxy gegebenenfalls durch ein oder mehrere Halogen, Hydroxyl oder NH₂ substituiert sind und wobei Cycloalkyl und Heterocycloalkyl gegebenenfalls durch ein oder mehrere Halogen, Hydroxyl, C₁-C₆-Alkyl, C₁-C₆-Heteroalkyl, C₁-C₆-Alkoxy oder NH₂ substituiert sind, oder
zwei R¹² an demselben Kohlenstoff zusammen Keto (=O) bilden können.

2. Verbindung nach Anspruch 1, wobei 0, 1 oder 2 der Reste X, Y und Z für N stehen.

3. Verbindung nach Anspruch 1 oder 2, wobei die Verbindung ausgewählt ist aus:
(a) Formel (Ia), 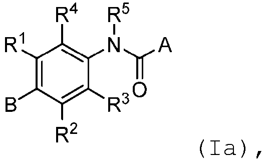 oder einem pharmazeutisch unbedenklichen Salz davon,
(b) Formel (Ib), 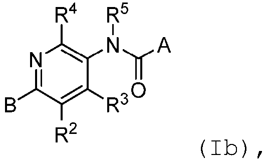 oder einem pharmazeutisch unbedenklichen Salz davon,
(c) Formel (Ic), 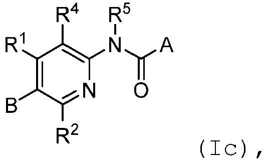 oder einem pharmazeutisch unbedenklichen Salz davon,
(d) Formel (Id), 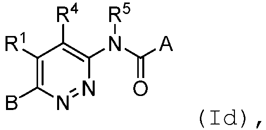 oder einem pharmazeutisch unbedenklichen Salz davon,
(e) Formel (Ie), 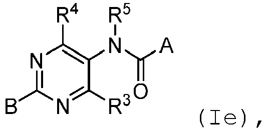 oder einem pharmazeutisch unbedenklichen Salz davon oder
(f) Formel (If), 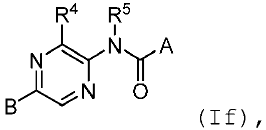 oder einem pharmazeutisch unbedenklichen Salz davon.

4. Verbindung nach einem der Ansprüche 1-3, wobei:
(a) R¹, R², R³ und R⁴ jeweils unabhängig aus der aus H, Halogen, C₁-C₆-Alkyl, C₁-C₆-Halogenalkyl, C₁-C₆-Alkoxy und C₁-C₆-Cycloalkyloxy bestehenden Gruppe ausgewählt sind, wobei gegebenenfalls R¹, R², R³ und R⁴ jeweils unabhängig aus der aus H, Halogen, C₁-C₆-Alkoxy und C₁-C₆-Alkyl bestehenden Gruppe ausgewählt sind, wobei gegebenenfalls R¹, R², R³ und R⁴ jeweils für H stehen, und/oder
(b) R⁵ für H steht und/oder
(c) A ausgewählt ist aus der Gruppe bestehend aus 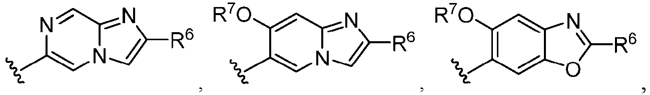 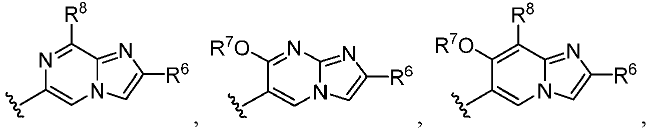 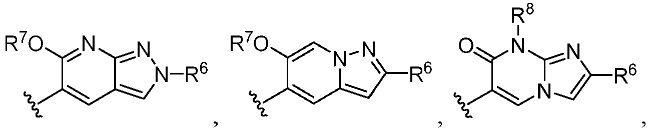 und 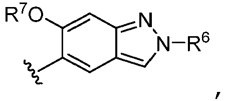 wobei A gegebenenfalls durch 1-3 R⁹ substituiert ist.

5. Verbindung nach einem der Ansprüche 1-4, wobei:
(a) A durch ein aus der aus Halogen und C₁-C₆-Alkyl bestehenden Gruppe ausgewähltes R⁹ substituiert ist oder wobei A nicht durch R⁹ substituiert ist und/oder
(b) R⁶ für Me steht und/oder
(c) R⁷ für C₁-C₆-Alkyl, C₁-C₆-Cycloalkyl oder Heterocycloalkyl steht, wobei gegebenenfalls R⁷ für Me, Et, Isopropyl oder Cyclobutyl steht.

6. Verbindung nach einem der Ansprüche 1-5, wobei A ausgewählt aus der Gruppe bestehend aus: 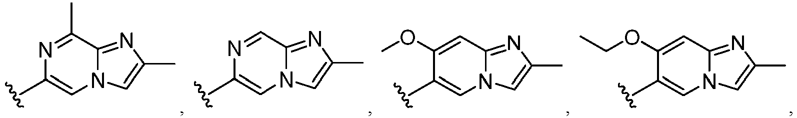 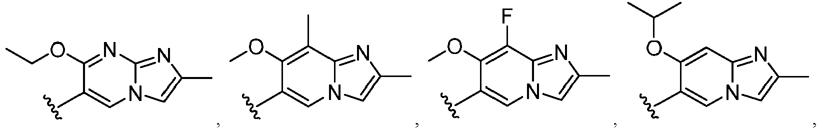 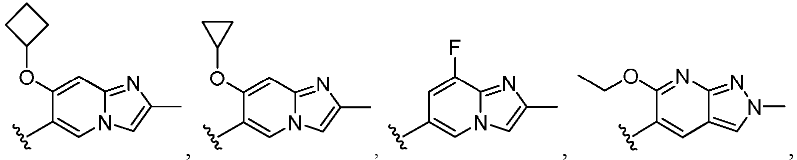 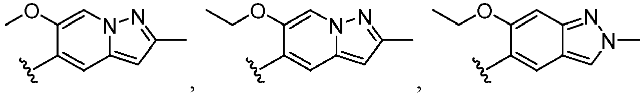 und

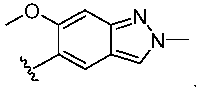

7. Verbindung nach einem der Ansprüche 1-6, wobei A für
(a) 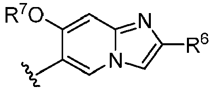 steht, wobei gegebenenfalls
A für 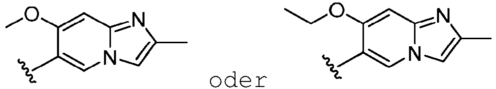 steht, oder für
(b) 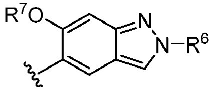 steht, wobei gegebenenfalls
A für 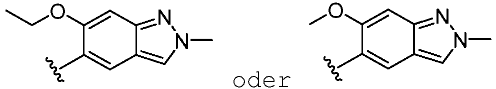 steht, oder für
(c) 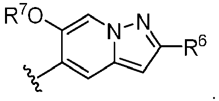 steht.

8. Verbindung nach einem der Ansprüche 1-7, wobei B für NR¹⁰R¹¹ steht, wobei R¹⁰ und R¹¹ zusammen mit dem Stickstoffatom, an das sie gebunden sind, ein monocyclisches oder bicyclisches Heterocycloalkyl bilden, das insgesamt 1, 2 oder 3 Stickstoffringatome und 0 oder 1 zusätzliche, aus O und S ausgewählte Ringheteroatome umfasst, wobei das Heterocycloalkyl gegebenenfalls durch 1, 2, 3 oder 4 R¹² substituiert ist.

9. Verbindung nach einem der Ansprüche 1-8, wobei R¹⁰ und R¹¹ zusammen mit dem Stickstoffatom, an das sie gebunden sind, ein monocyclisches Heterocycloalkyl mit 4-7 Ringatomen mit insgesamt 1 oder 2 Stickstoffringatomen und 0 oder 1 zusätzlichen, aus O und S ausgewählten Ringheteroatomen bilden, wobei das Heterocycloalkyl gegebenenfalls durch 1, 2, 3 oder 4 R¹² substituiert ist.

10. Verbindung nach einem der Ansprüche 1-9, wobei B für Folgendes steht: 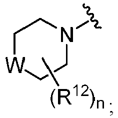
W für NR13 oder CR¹⁴R¹⁴ steht,
R¹³ aus der aus Wasserstoff, C₁-C₆-Alkyl, C₂-C₆-Alkenyl, C₂-C₆-Alkinyl und -(CH₂)₀₋₂-C₃-C₈-Cycloalkyl bestehenden Gruppe ausgewählt ist, wobei Alkyl, Alkenyl, Alkinyl gegebenenfalls durch ein oder mehrere Halogen, Hydroxyl, Methoxy oder NH₂ substituiert sind und wobei Cycloalkyl gegebenenfalls durch ein oder mehrere Halogen, Hydroxyl, C₁-C₆-Alkyl, C₁-C₆-Alkoxy oder NH₂ substituiert ist,
R¹⁴ jeweils unabhängig für H oder R¹² steht und
n = 0, 1, 2, 3 oder 4, wobei gegebenenfalls:
(a) R¹² jeweils unabhängig aus der aus Halogen, Hydroxy, 4- bis 7-gliedrigem monocyclischem Heterocycloalkyl, C₁-C₆-Heteroalkyl und C₁-C₆-Alkyl bestehenden Gruppe ausgewählt ist, wobei Alkyl gegebenenfalls durch ein oder mehrere Halogen, Hydroxyl, Methoxy, C₃-C₈-Cycloalkyl oder NH₂ substituiert ist und Heterocycloalkyl gegebenenfalls durch ein oder mehreren Halogen, Hydroxyl, Methoxy oder C₁-C₆-Alkyl substituiert ist, und
R¹³ für H oder unsubstituiertes C₁-C₆-Alkyl steht, wobei gegebenenfalls R¹² jeweils unabhängig für C₁-C₆-Alkyl steht, und/oder
(b) B für 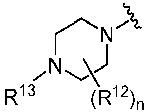 steht, wobei gegebenenfalls R¹³ für H oder unsubstituiertes C₁-C₆-Alkyl steht und/oder wobei R¹² jeweils unabhängig für C₁-C₆-Alkyl steht.

11. Verbindung nach einem der Ansprüche 1-8, wobei B für bicyclisches 6- bis 14-gliedriges Heterocycloalkyl steht, das insgesamt 1, 2 oder 3 Stickstoffringatome und 0 oder 1 zusätzliche, aus O und S ausgewählte Ringheteroatome umfasst, und Heterocycloalkyl gegebenenfalls durch 1, 2, 3 oder 4 R¹² substituiert ist; wobei gegebenenfalls R¹² jeweils unabhängig für C₁-C₆-Alkyl steht.

12. Verbindung nach einem der Ansprüche 1-7, wobei B für NR¹⁰R¹¹ steht und R¹¹ für Wasserstoff, C₁₋₇-Alkyl, C₁₋₇-Halogenalkyl oder C₃₋₈-Cycloalkyl steht.

13. Verbindung nach einem der Ansprüche 1-7 oder 12, wobei:
(a) R¹¹ für H oder C₁₋₇-Alkyl steht und R¹⁰ für C₁-C₈-Heteroalkyl steht, das mindestens ein Stickstoffatom umfasst, oder
(b) R¹⁰ für - (CH₂)₀₋₃-Heterocycloalkyl steht, das mindestens 1 Stickstoffringatom umfasst, wobei R¹⁰ jeweils gegebenenfalls durch 1 bis 6 R¹² substituiert ist.

14. Verbindung nach Anspruch 1, wobei es sich bei der Verbindung um Folgende handelt:
(a) Formel (Ig) 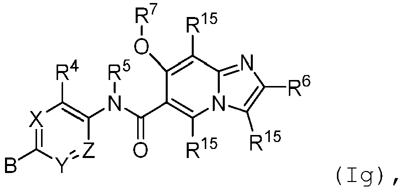
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(b) Formel (Ih) 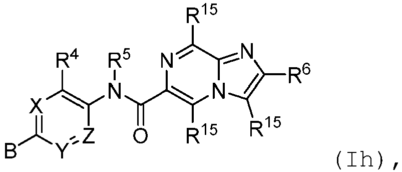
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(c) Formel (Ii) 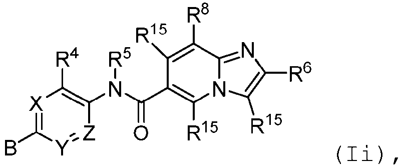
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(d) Formel (Ij) 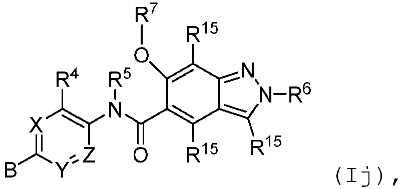
oder ein pharmazeutisch unbedenkliches Salz davon,
wobei R¹⁵ jeweils unabhängig für H, Halogen, C₁-C₆-Alkyl oder C₁-C₈-Halogenalkyl steht, wobei R⁵ gegebenenfalls für H steht,
(e) Formel (Ik) 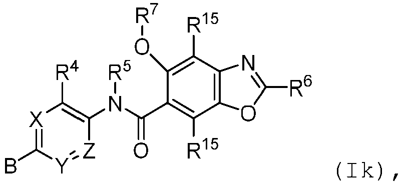
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(f) Formel (Im) 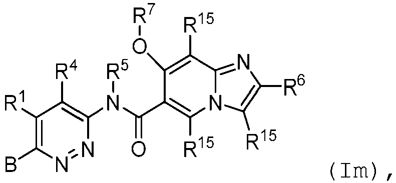
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(g) Formel (In) 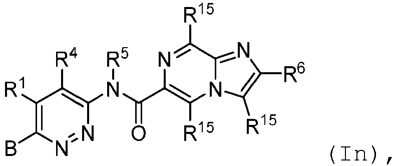
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(h) Formel (Io) 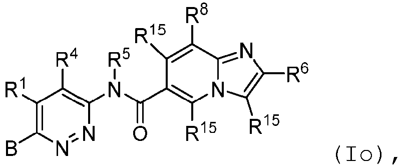
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(i) Formel (Ip) 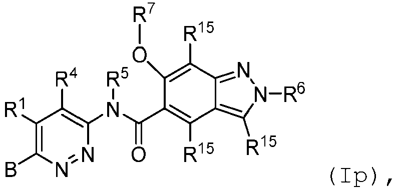
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(j) Formel (Iq) 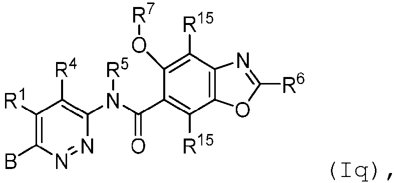
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(k) Formel (Ir) 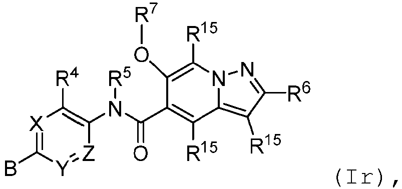
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(l) Formel (Is) 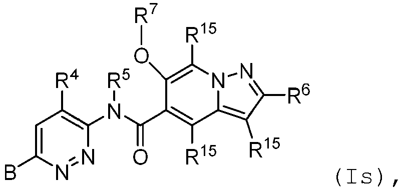
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht,
(m) Formel (It) 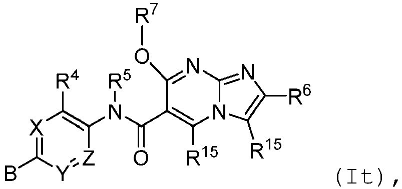
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht, oder
(n) Formel (Iu) 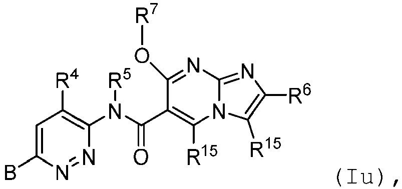
oder ein pharmazeutisch unbedenkliches Salz davon,
wobei R¹⁵ jeweils unabhängig für H, Halogen, C₁-C₆-Alkyl oder C₁-C₆-Halogenalkyl steht, wobei R⁵ gegebenenfalls für H steht.

15. Verbindung nach einem der Ansprüche 1-14, ausgewählt aus:

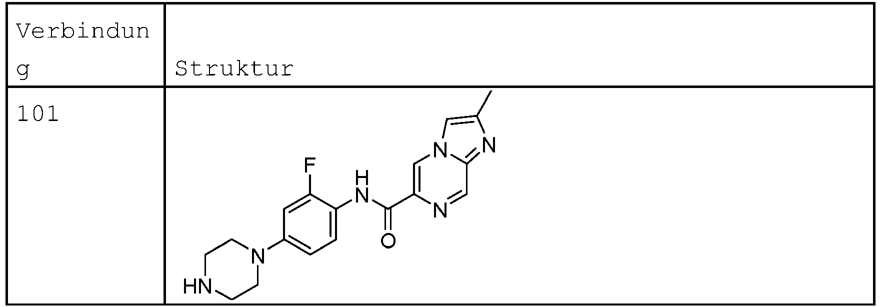

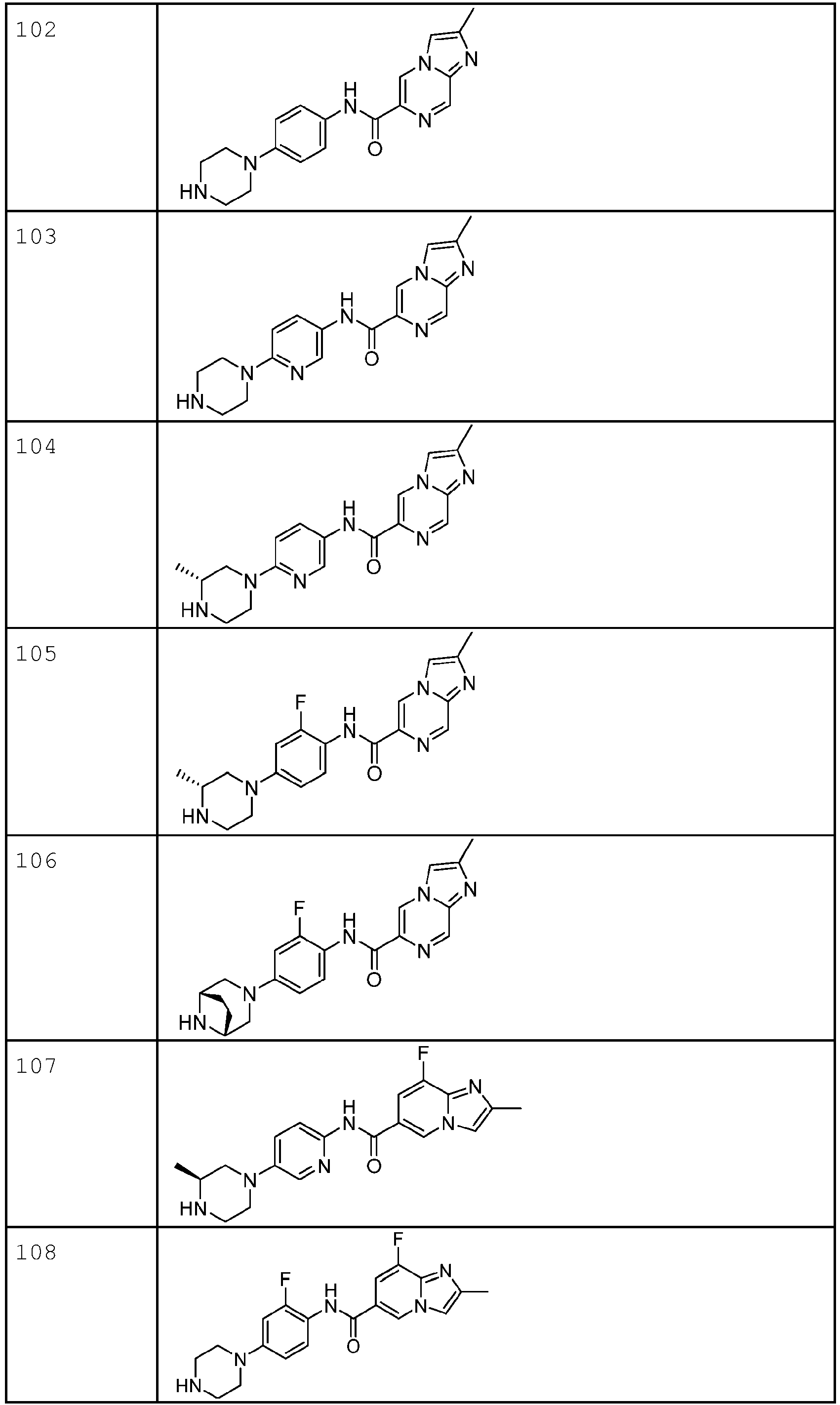

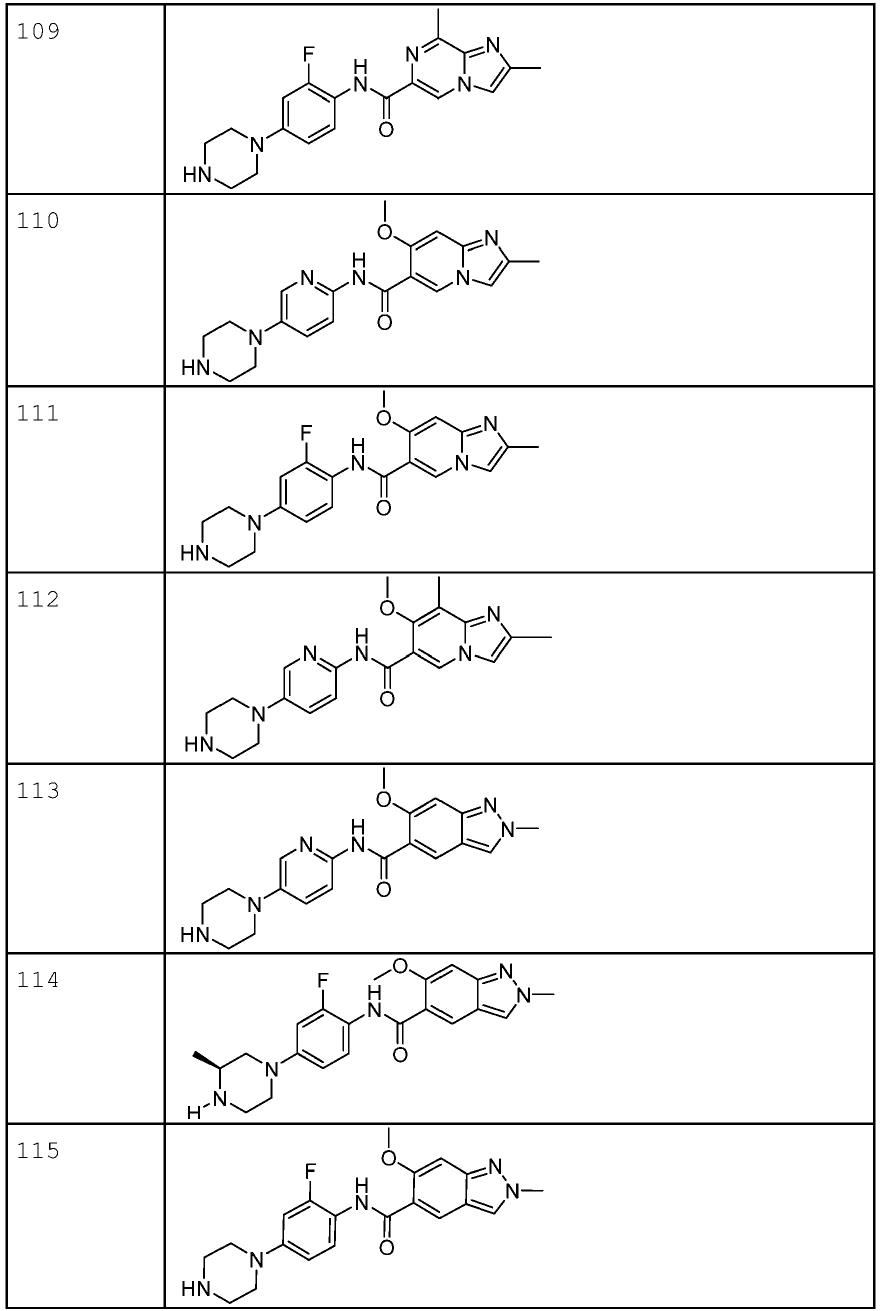

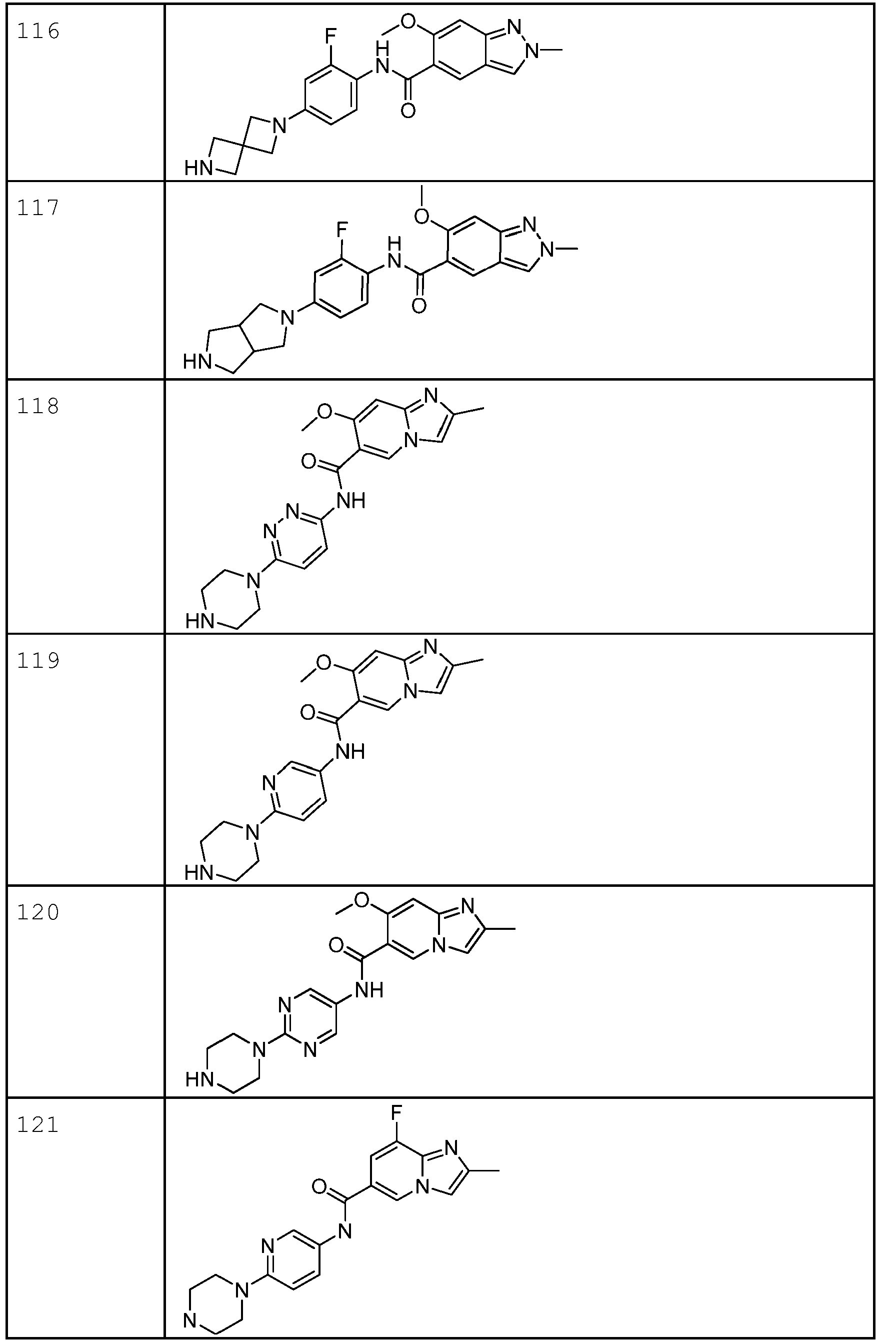

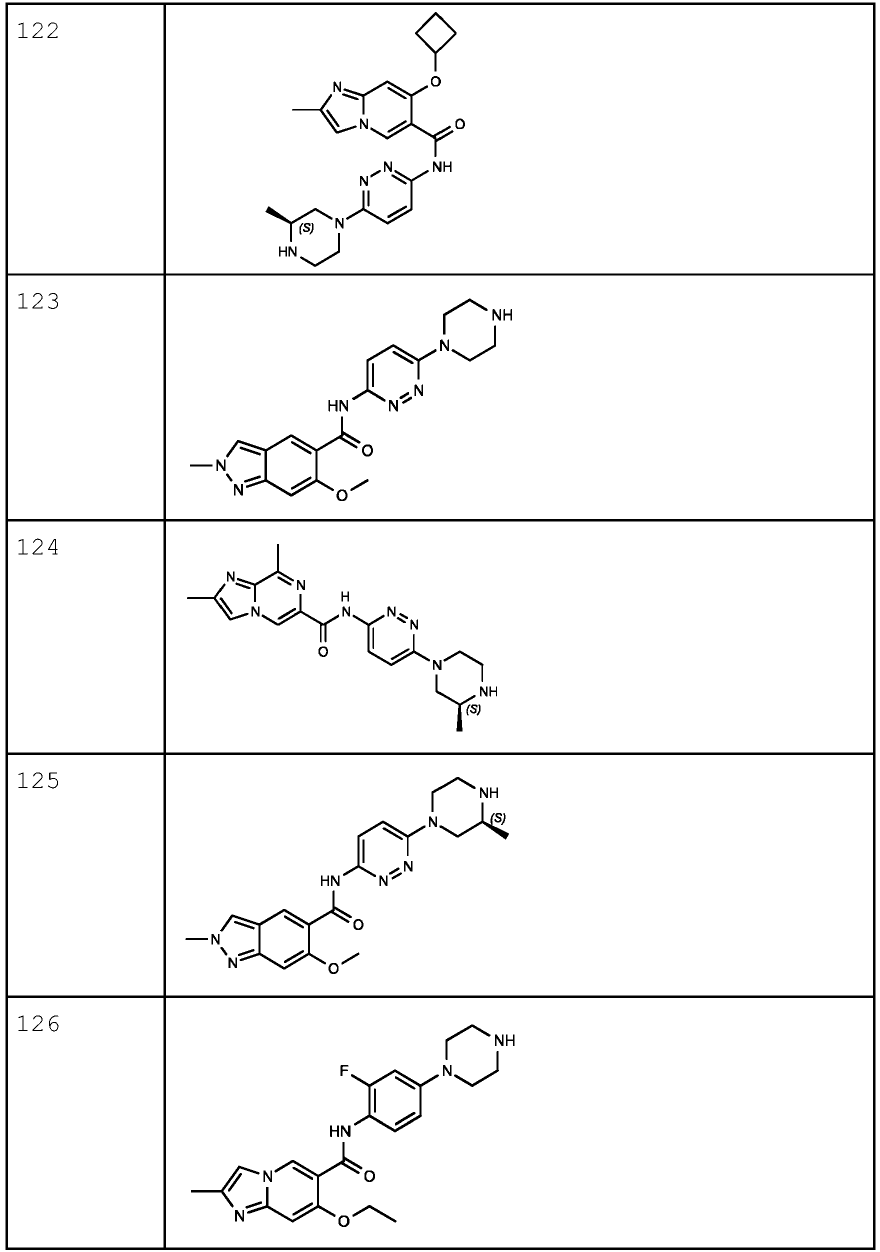

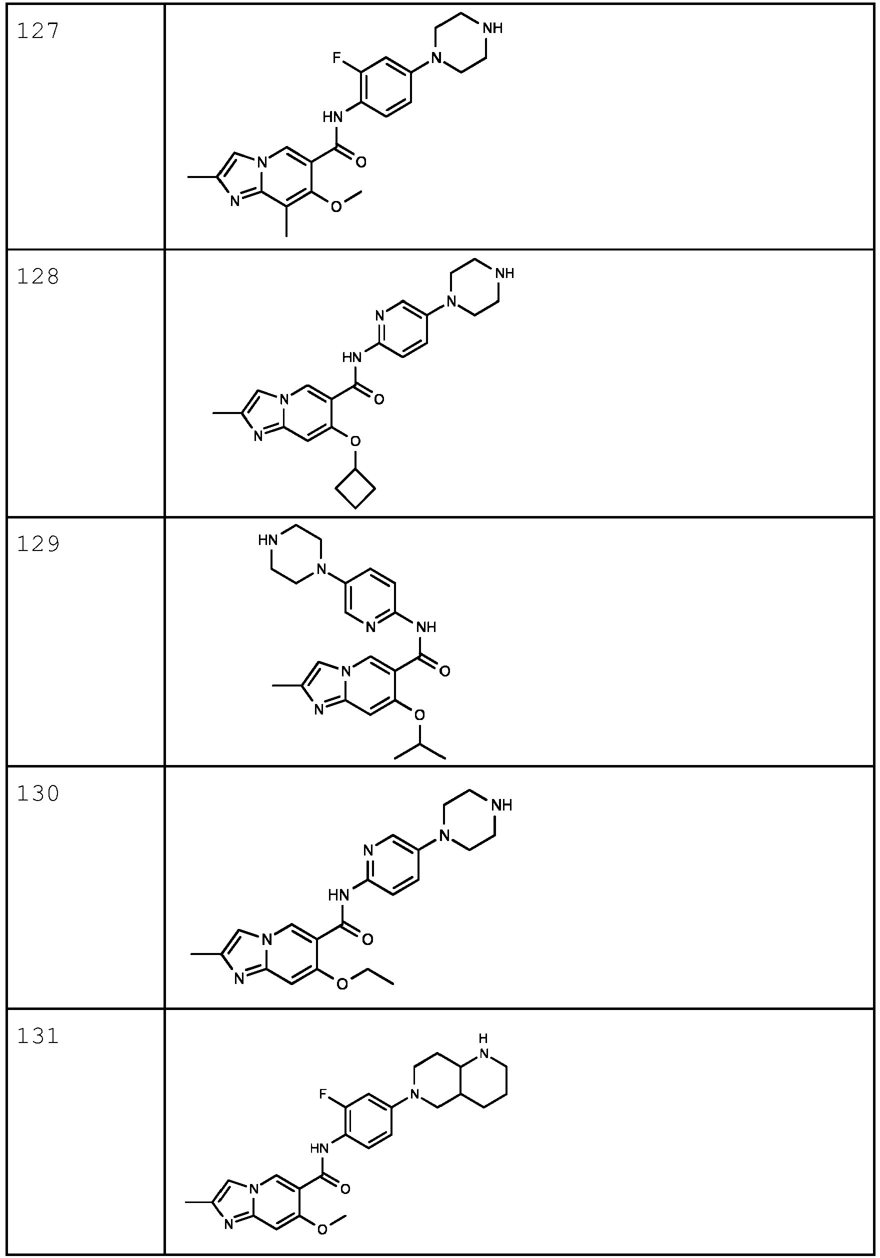

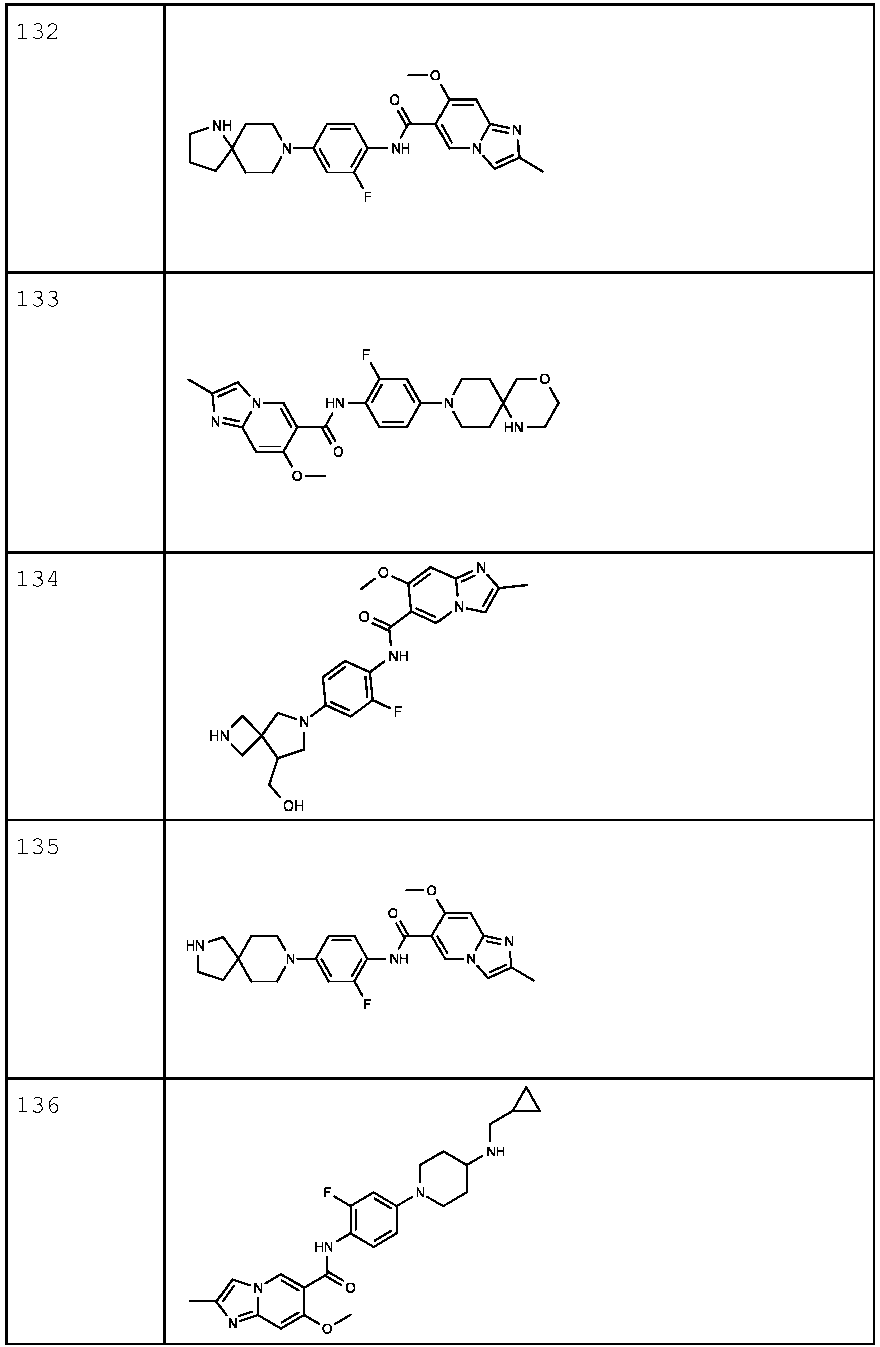

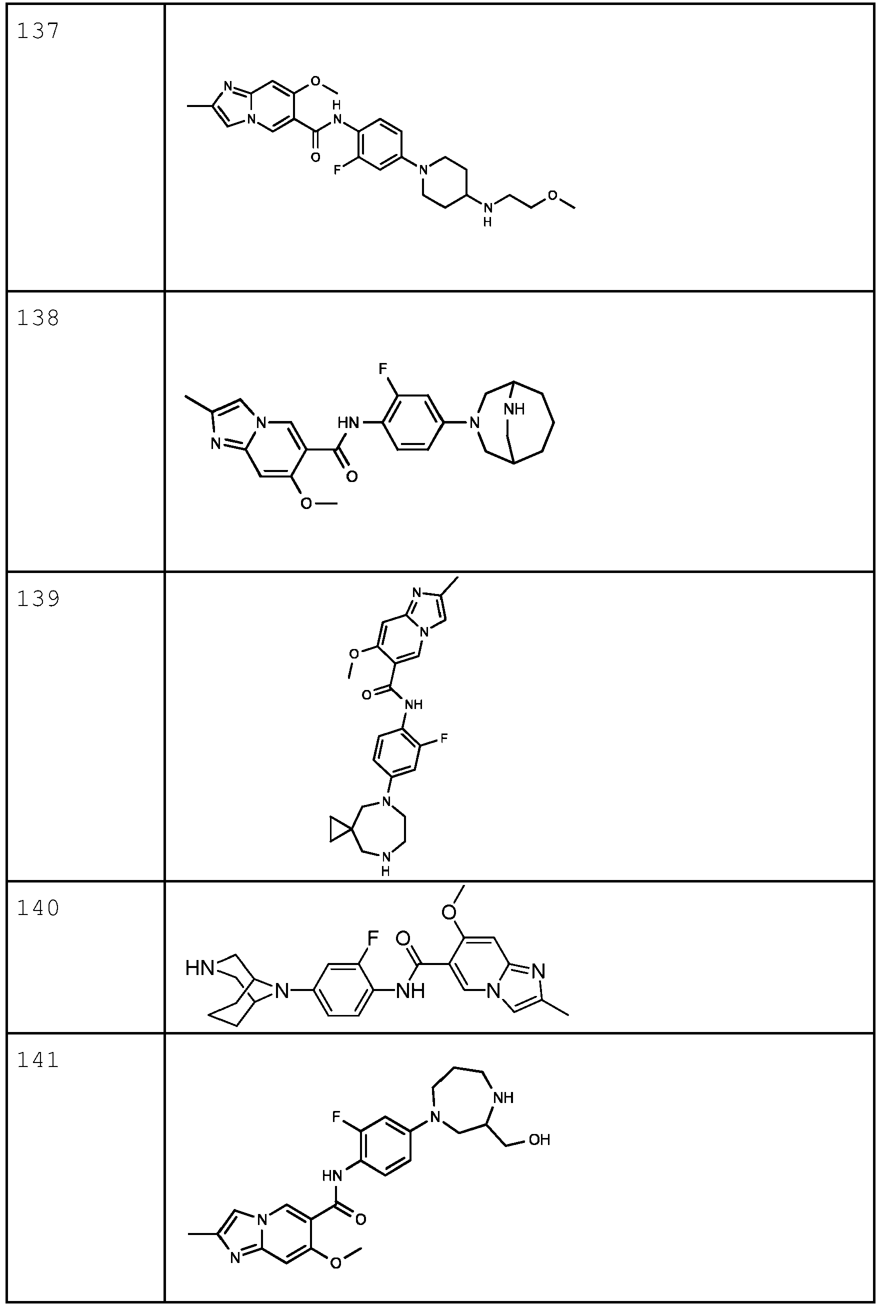

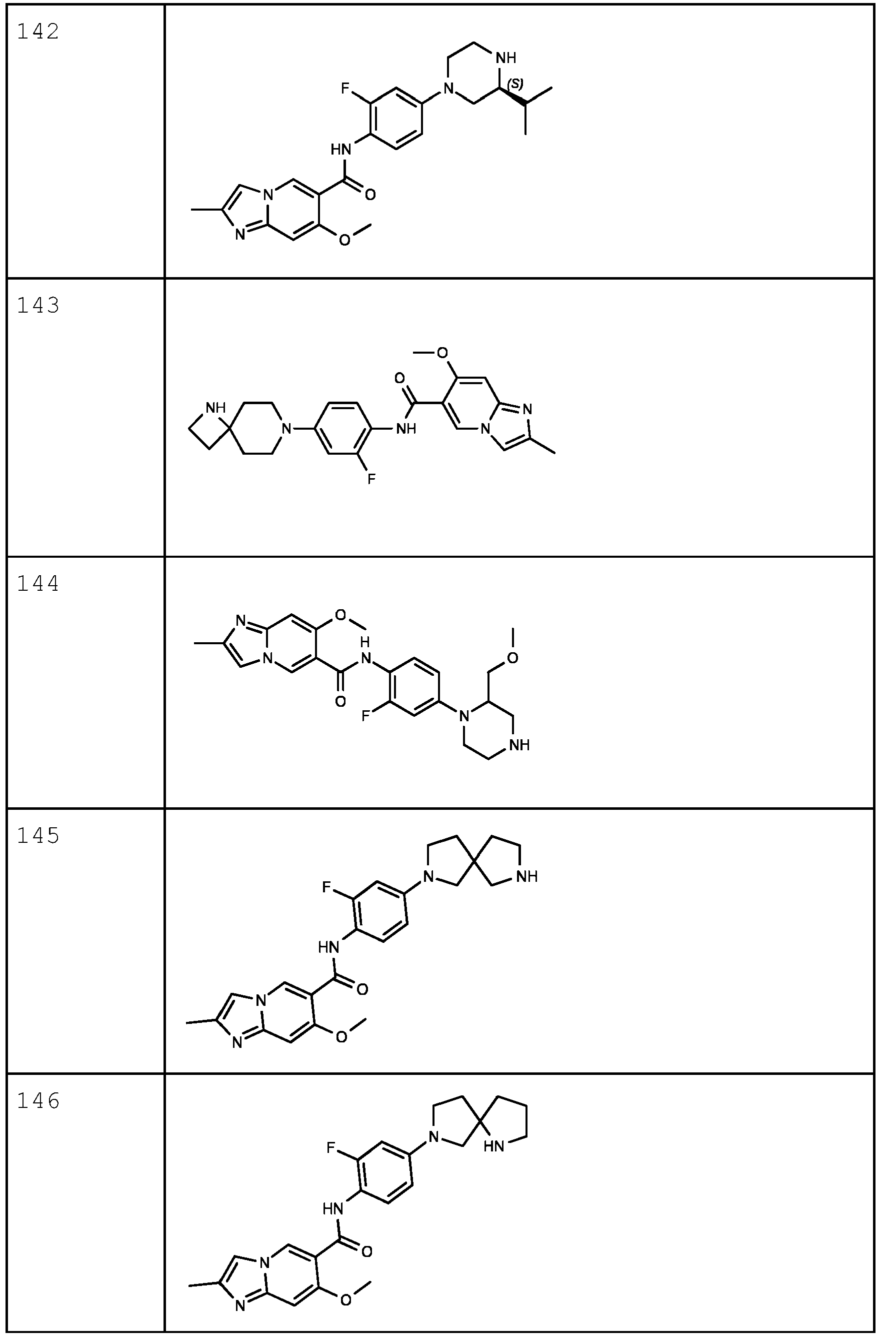

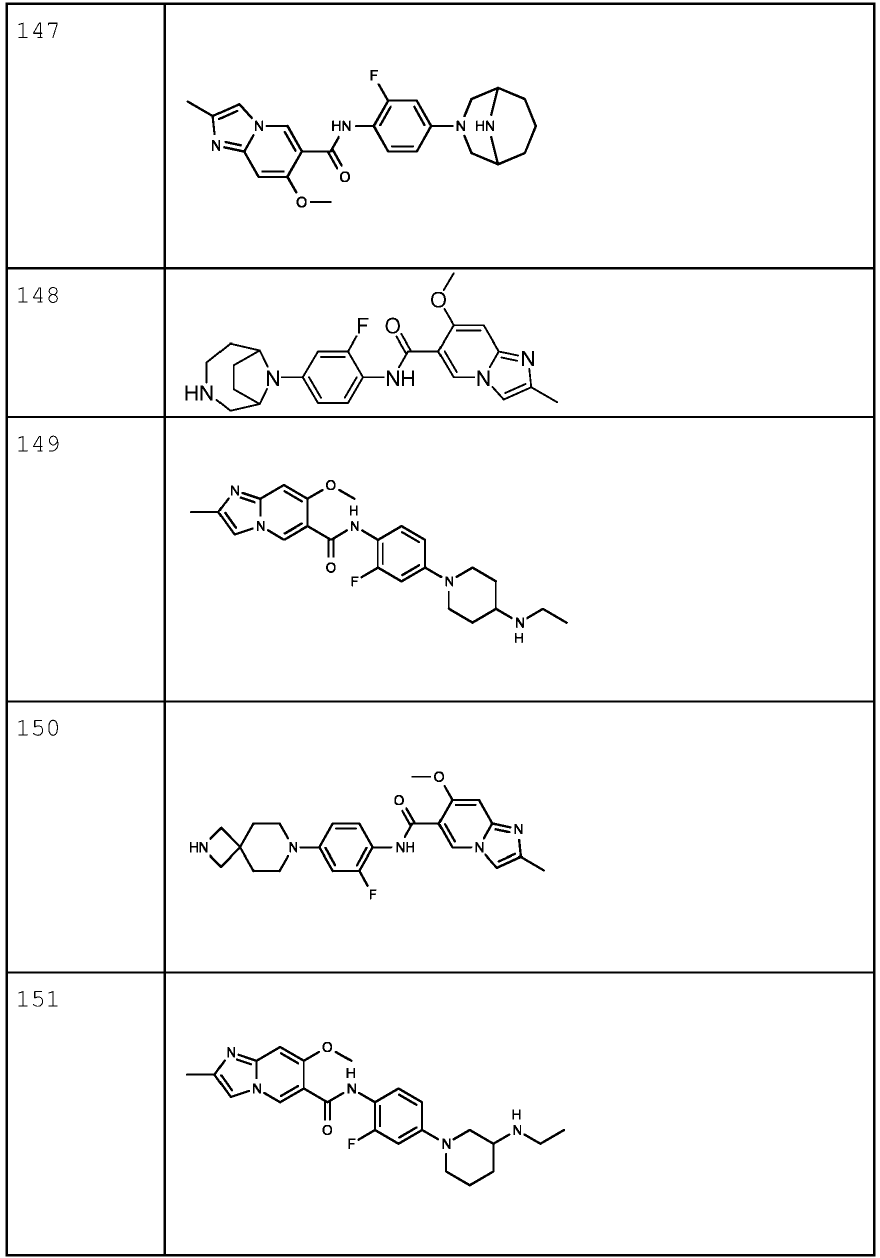

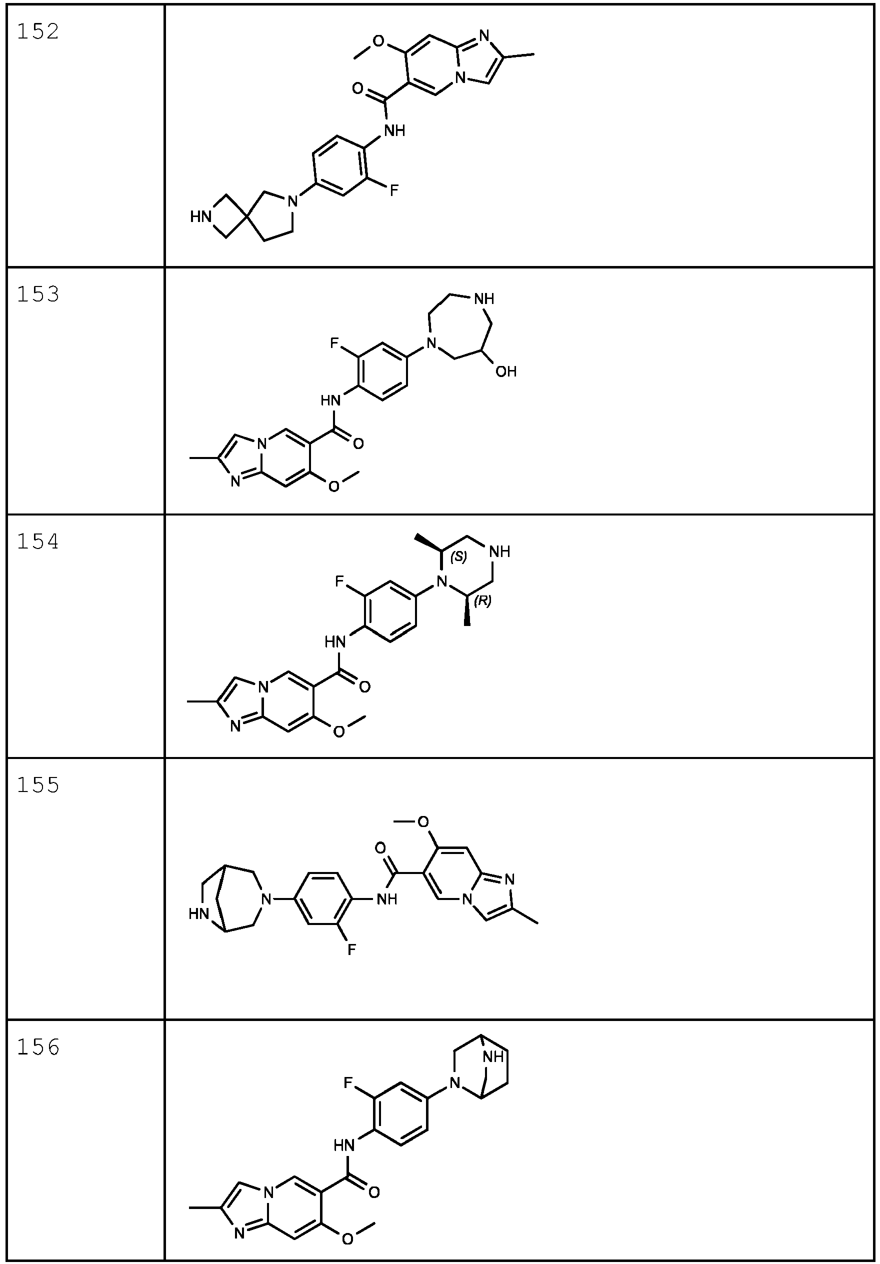

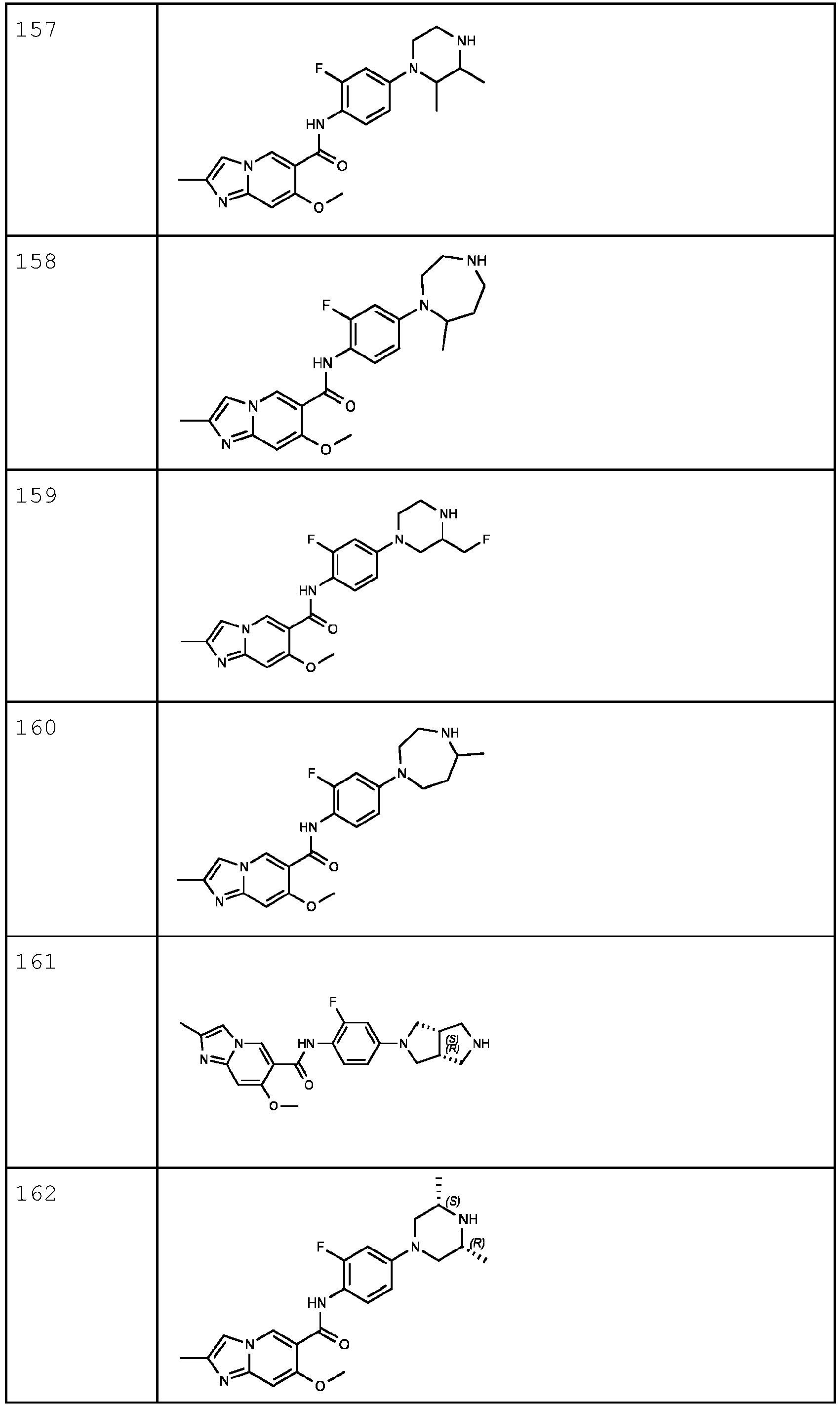

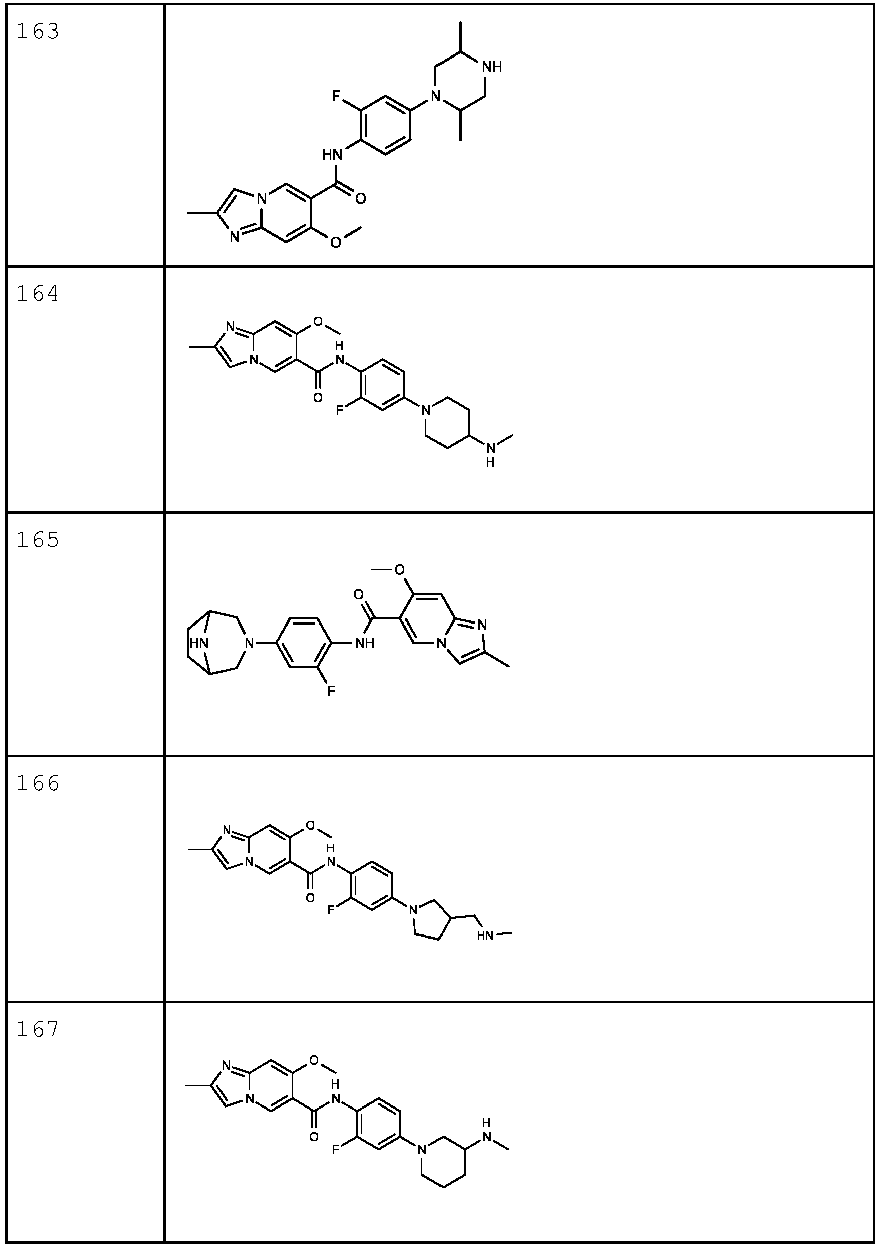

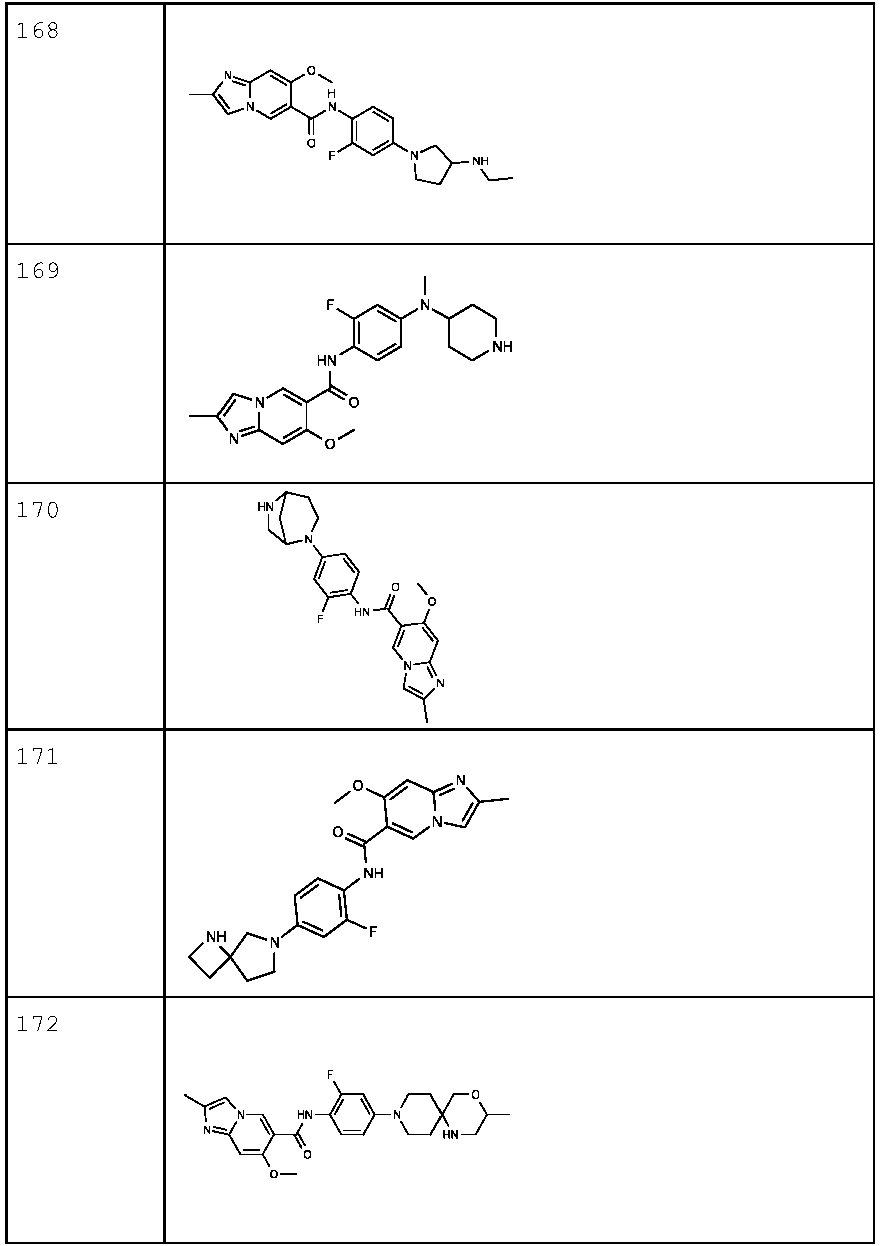

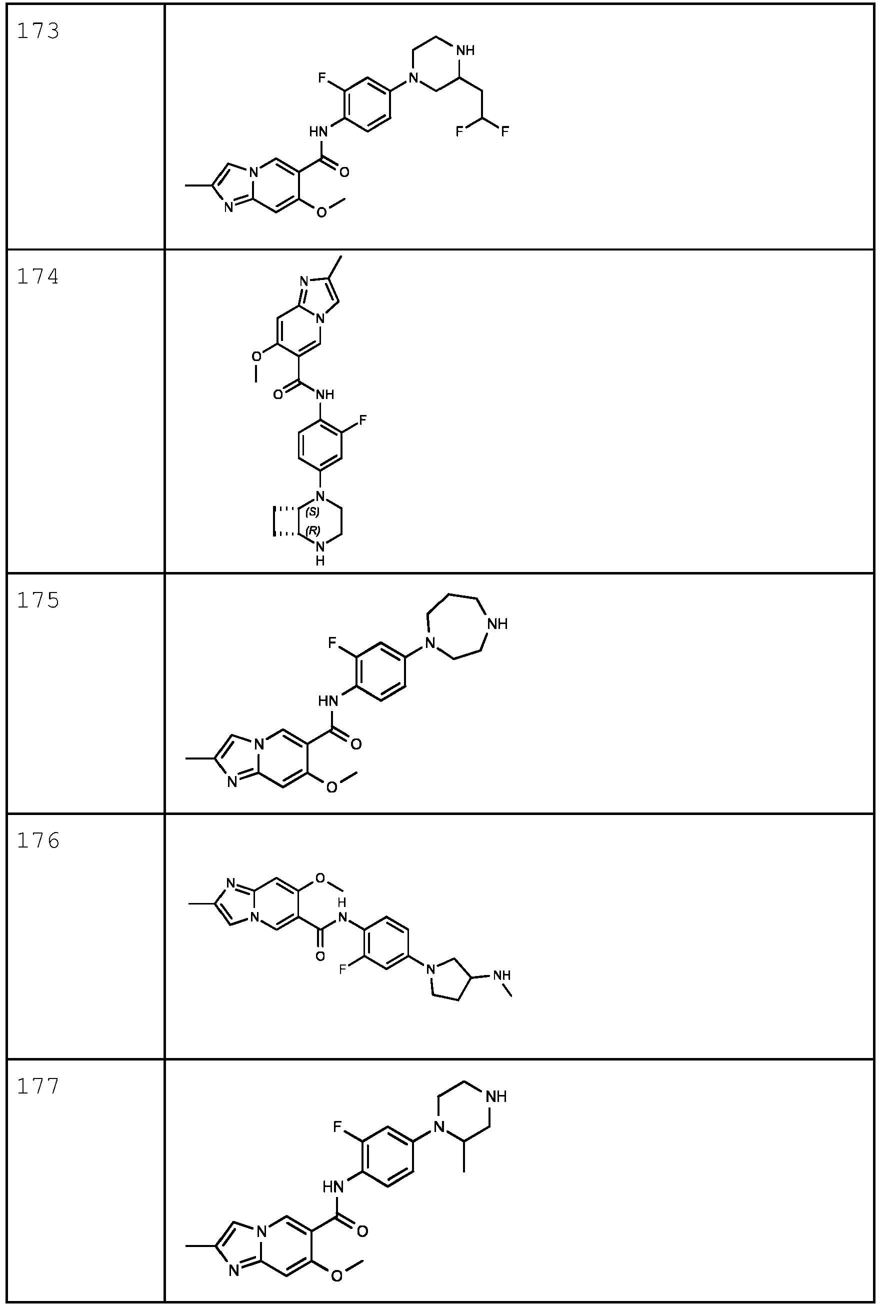

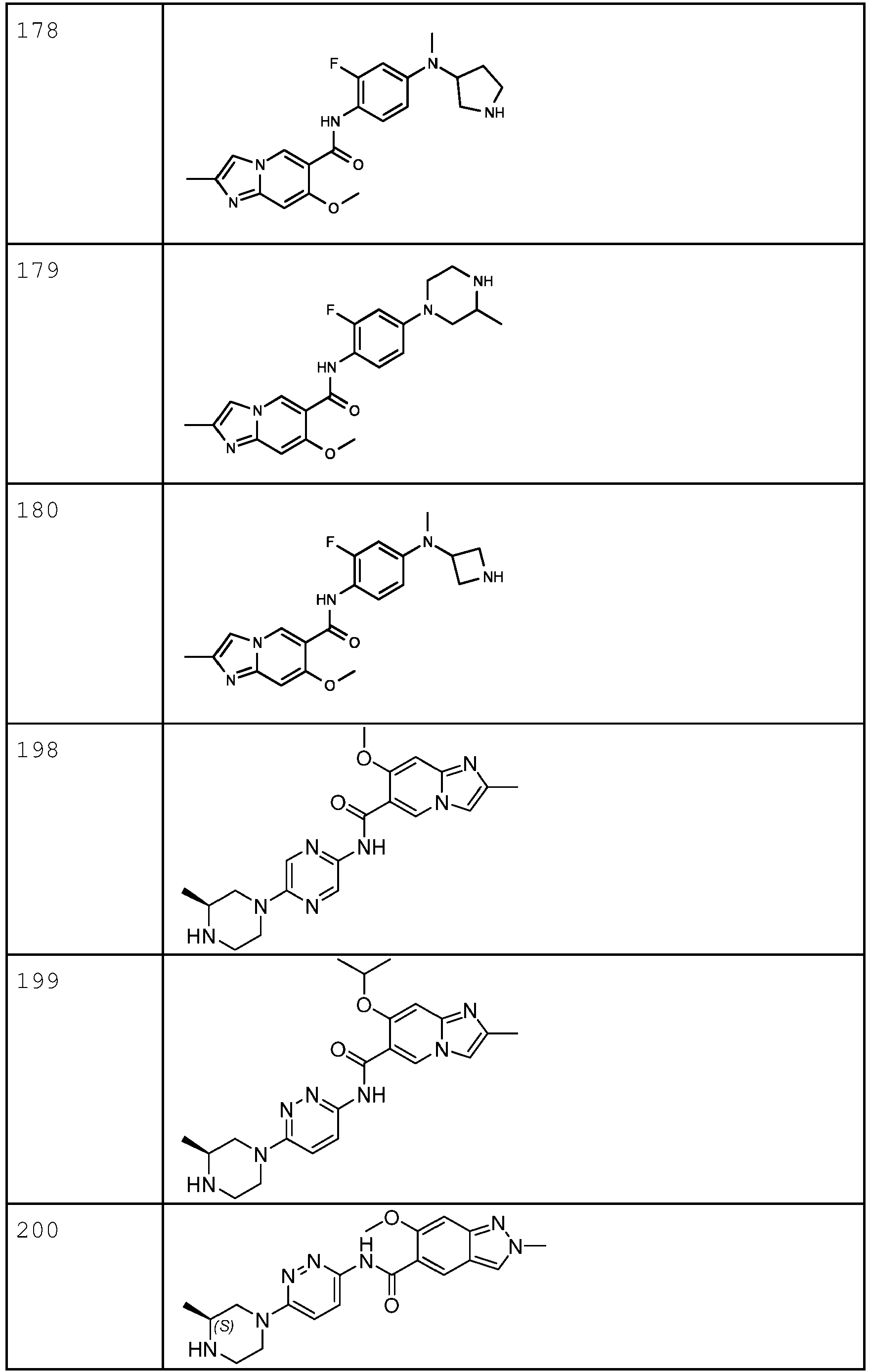

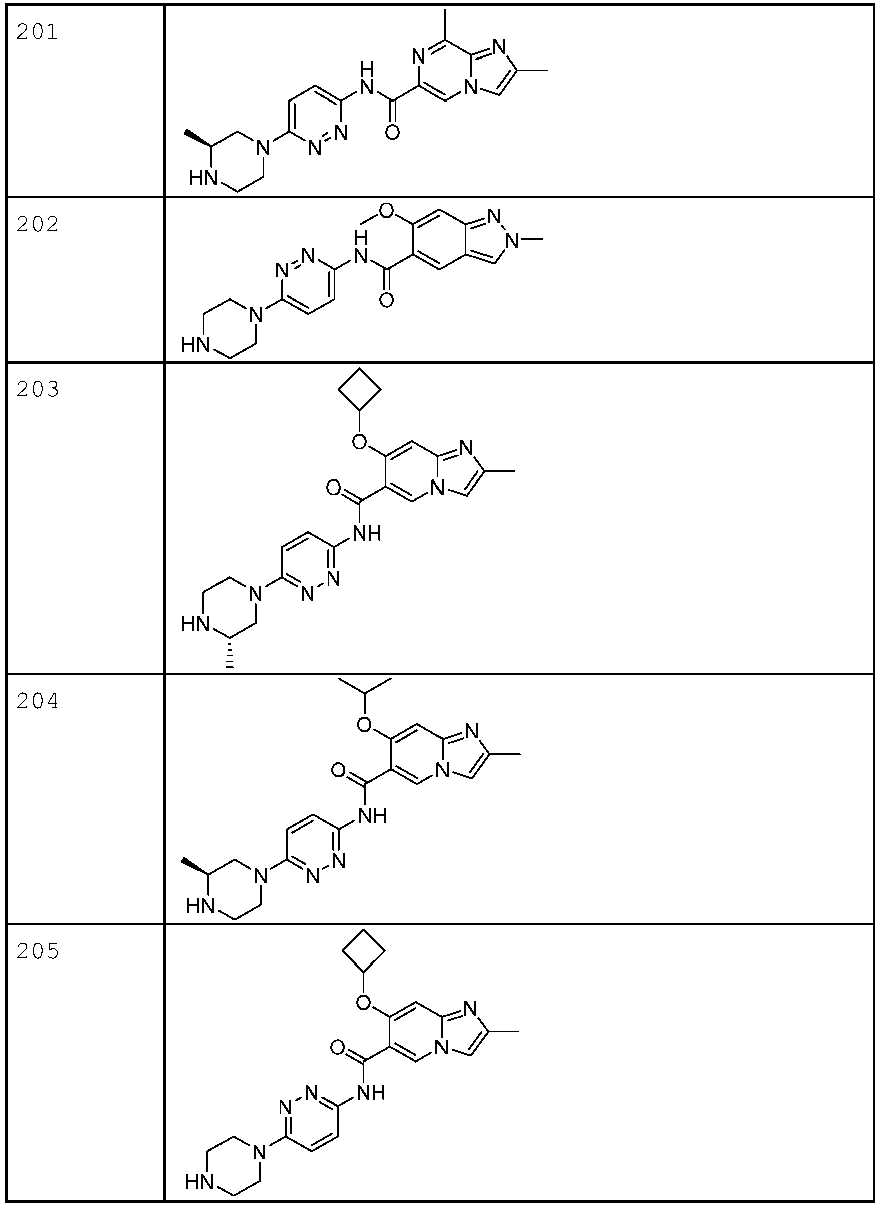

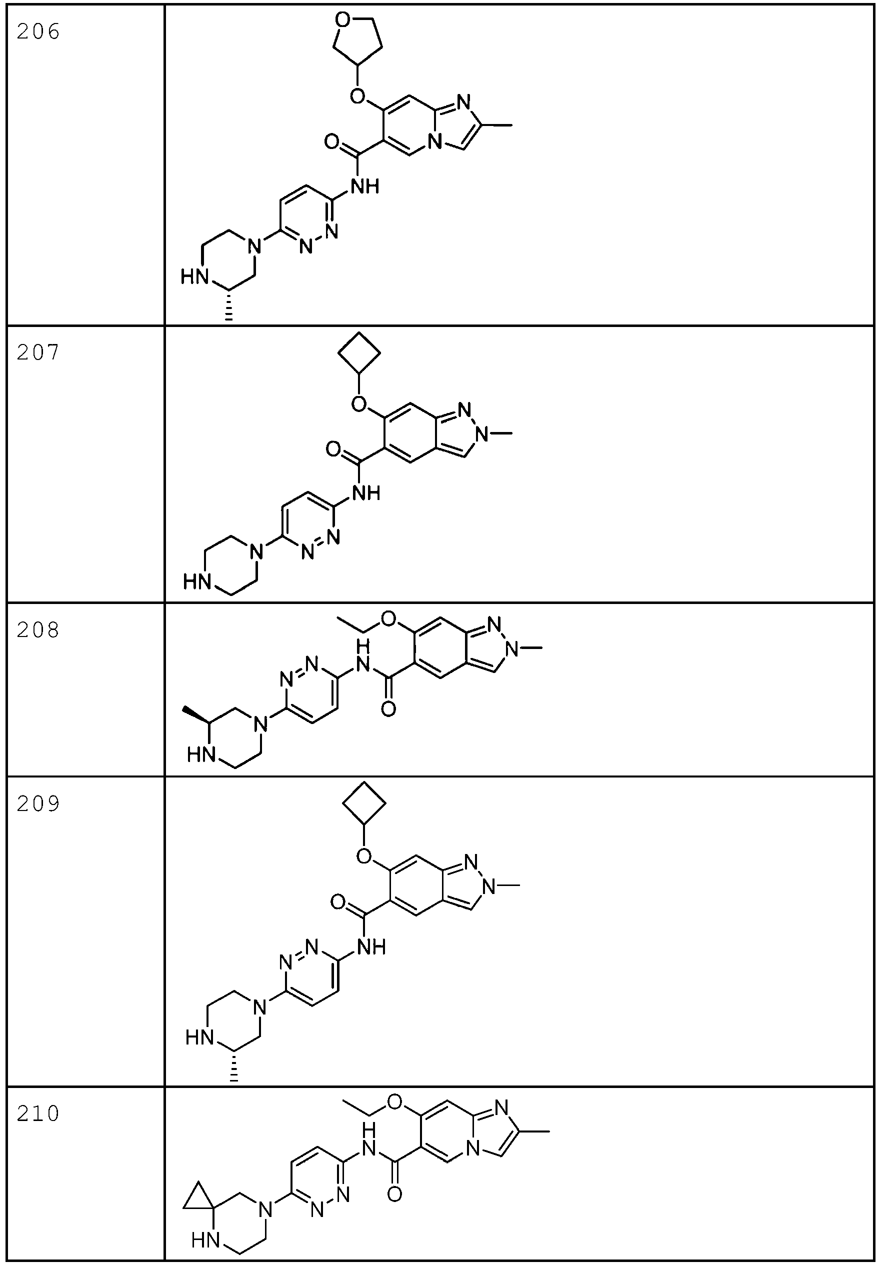

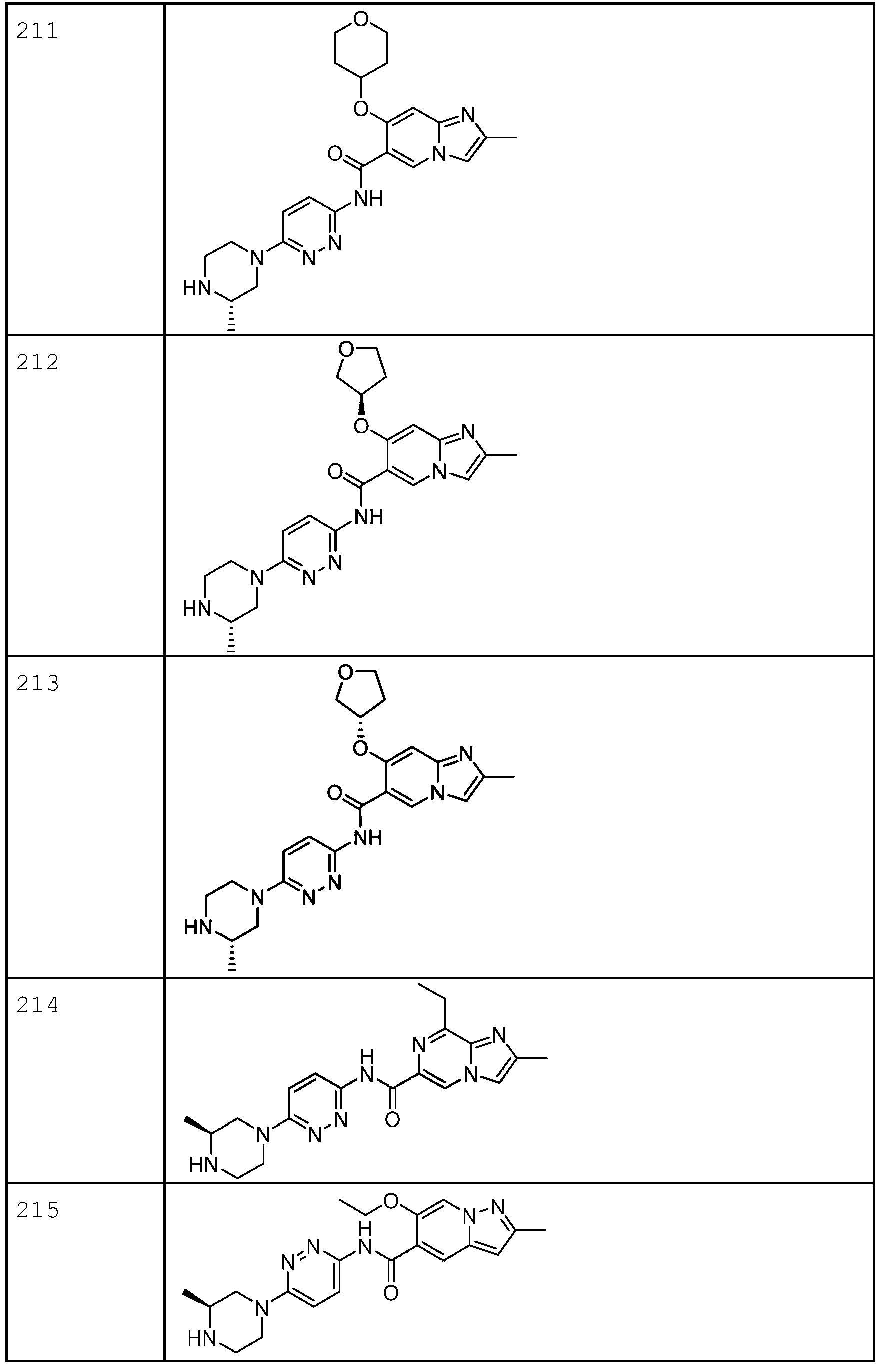

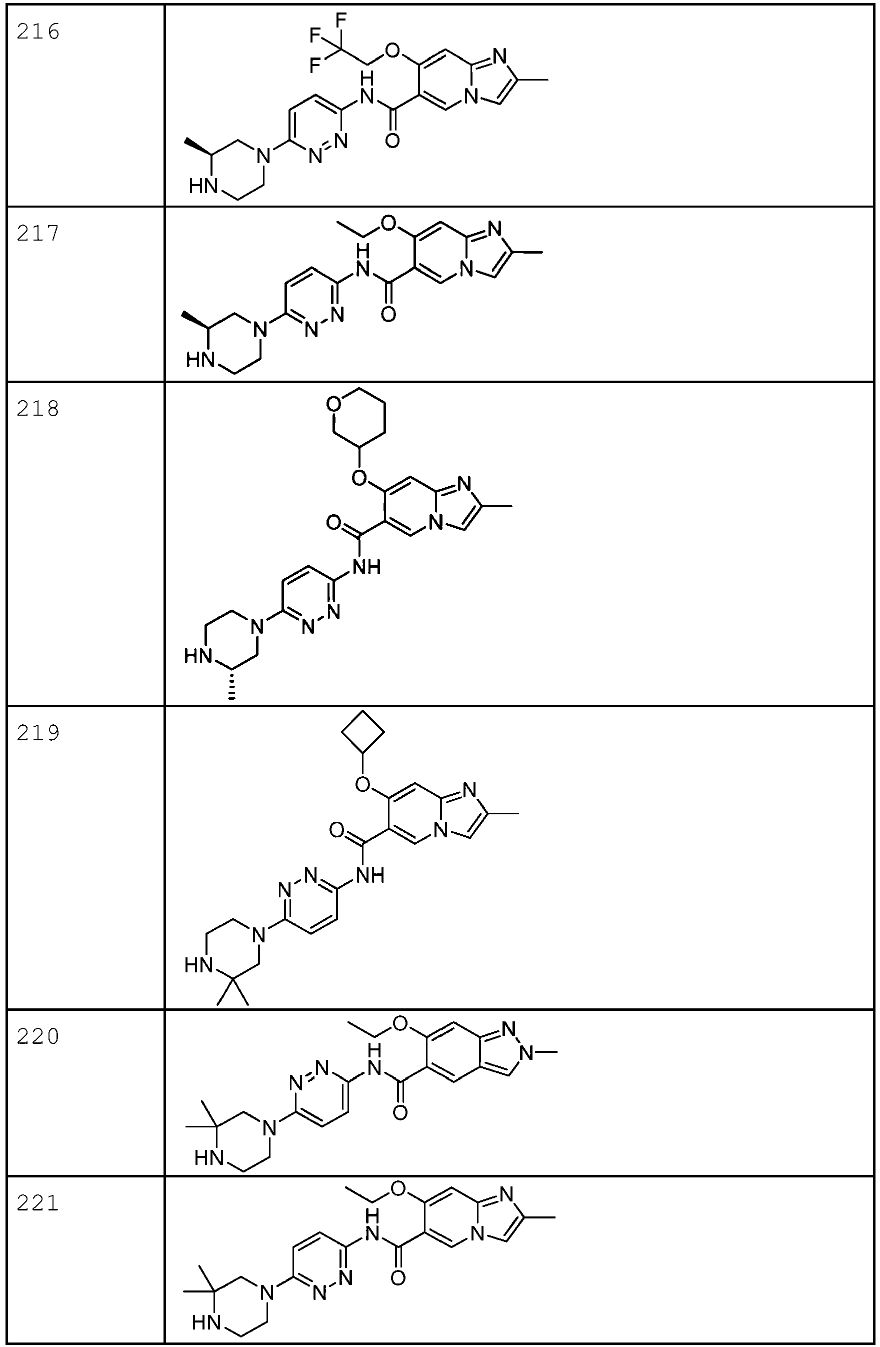

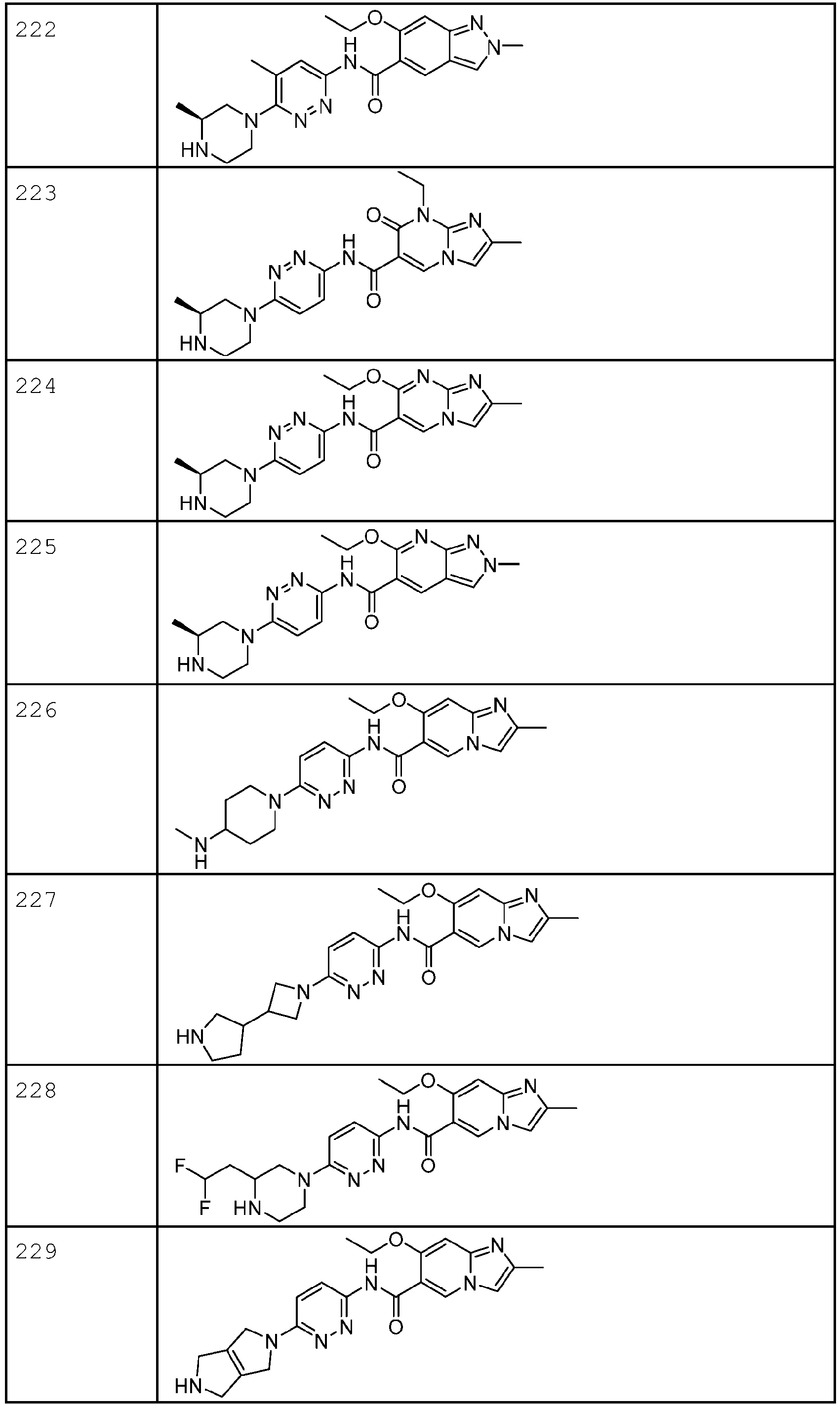

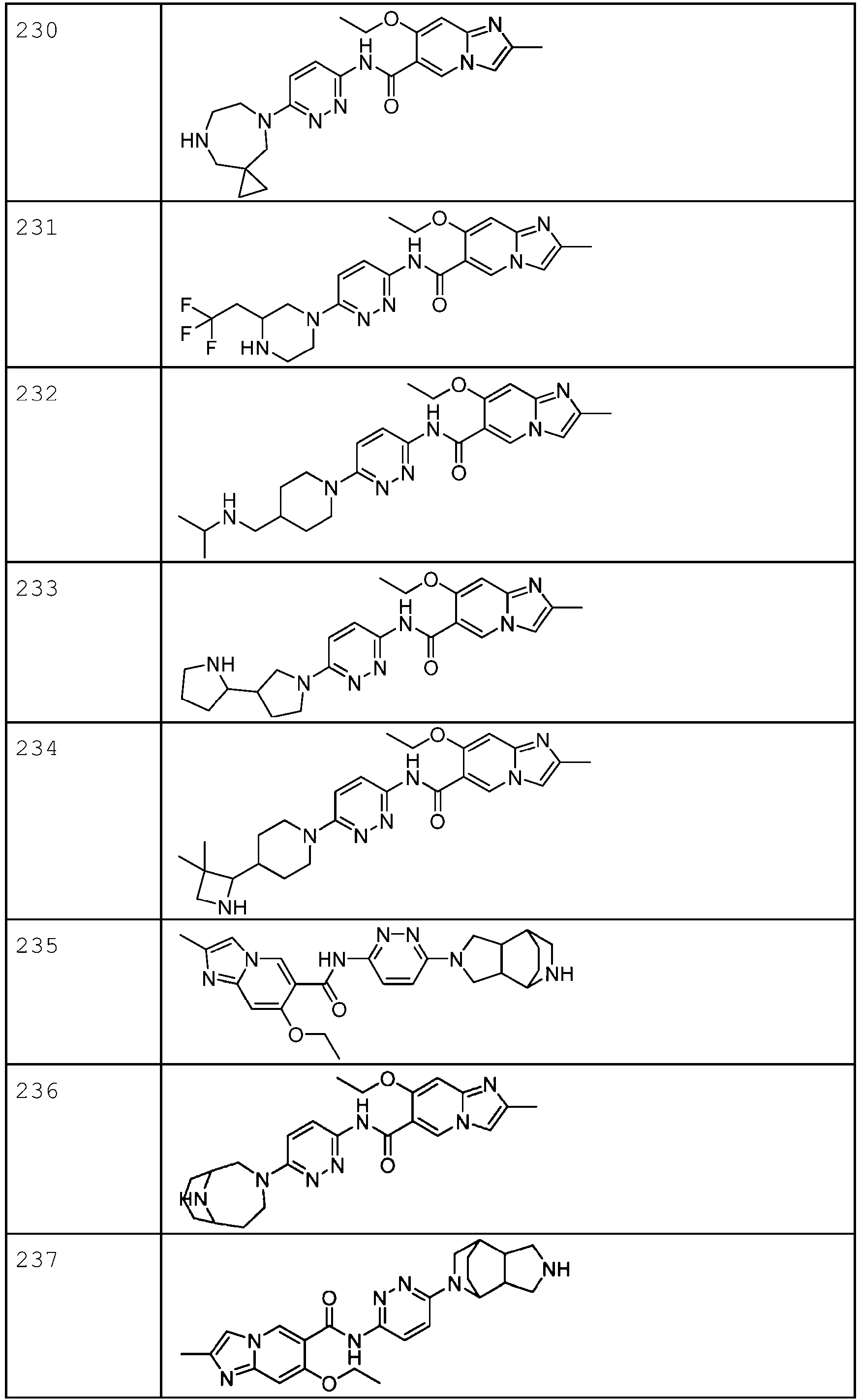

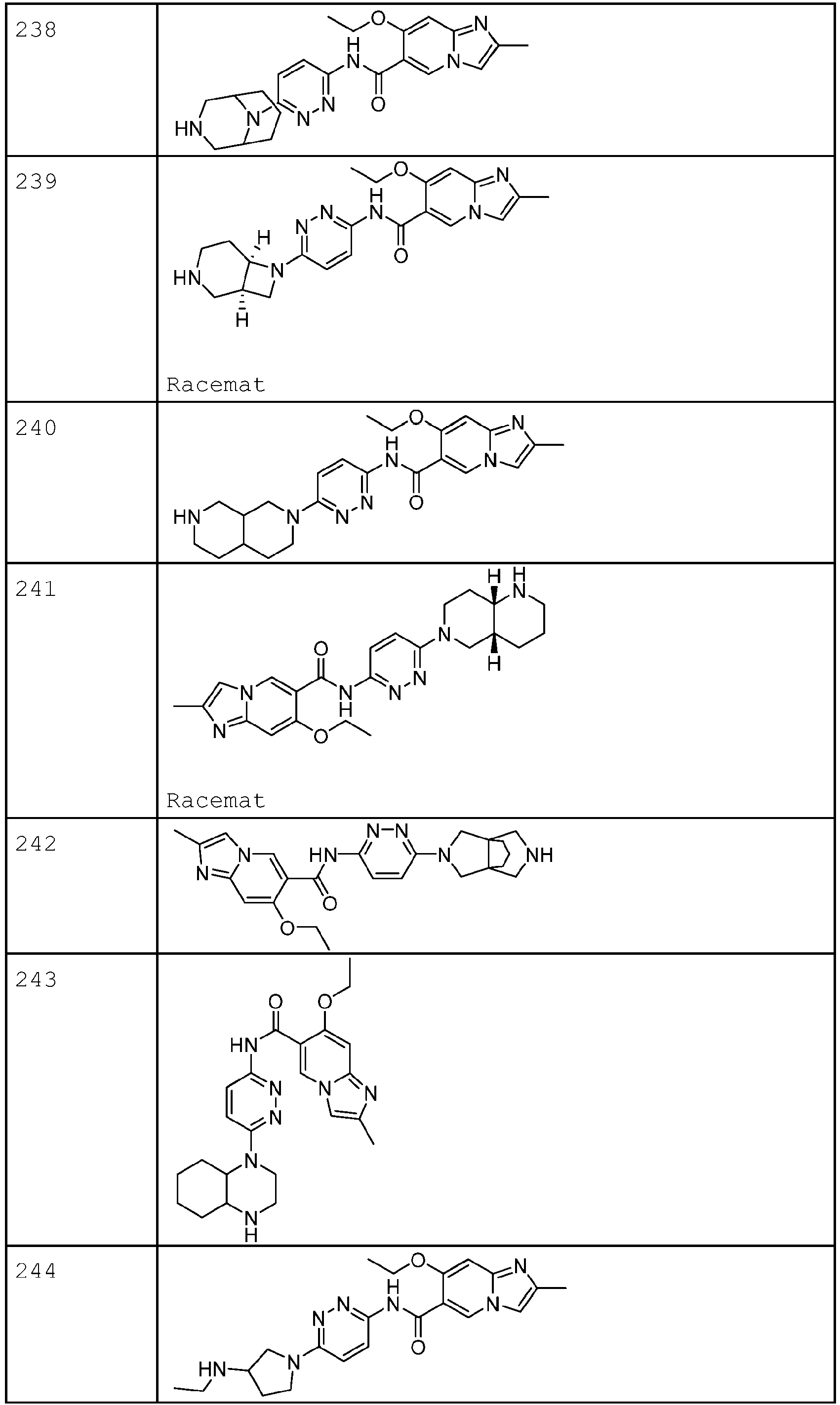

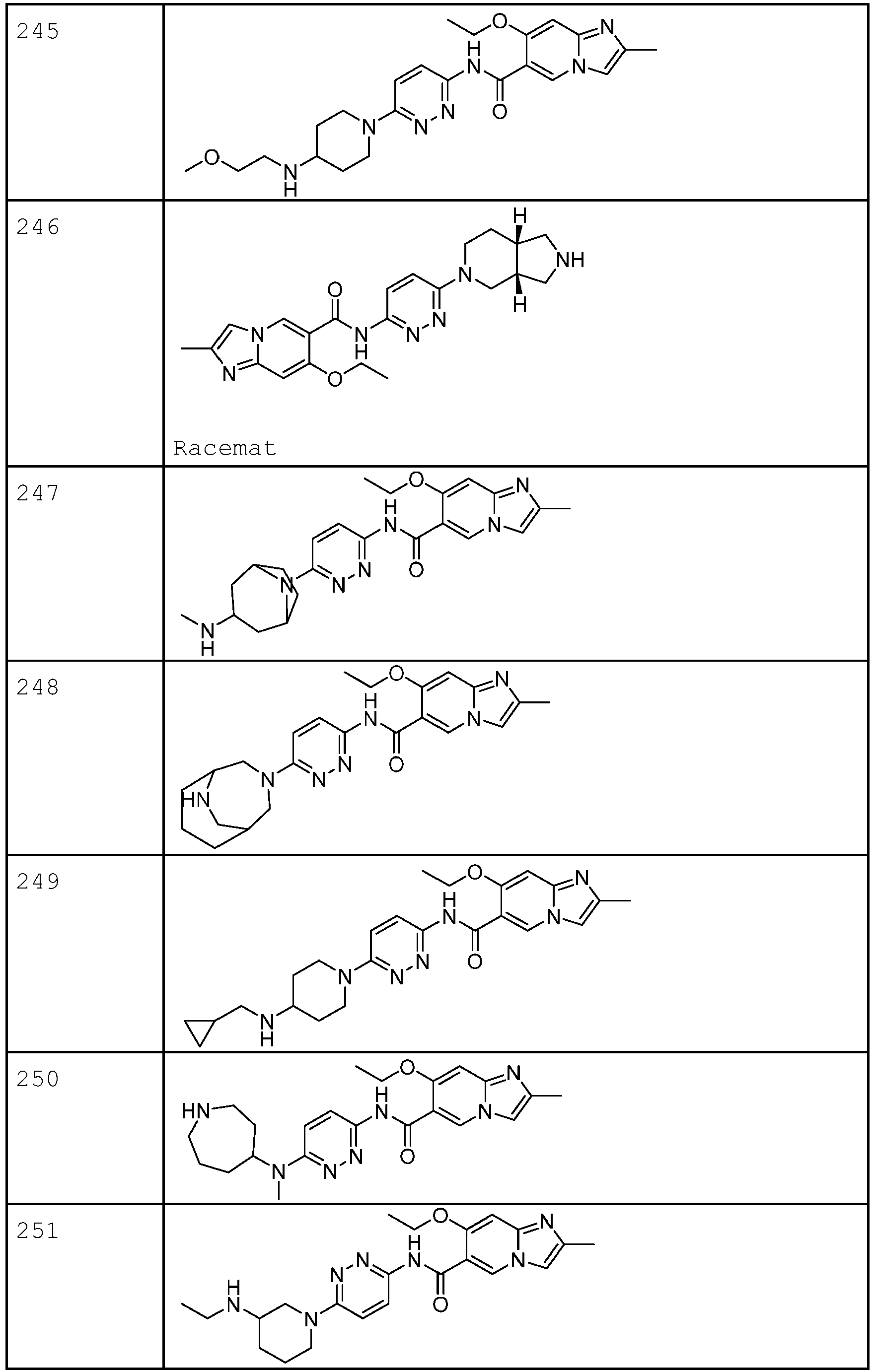

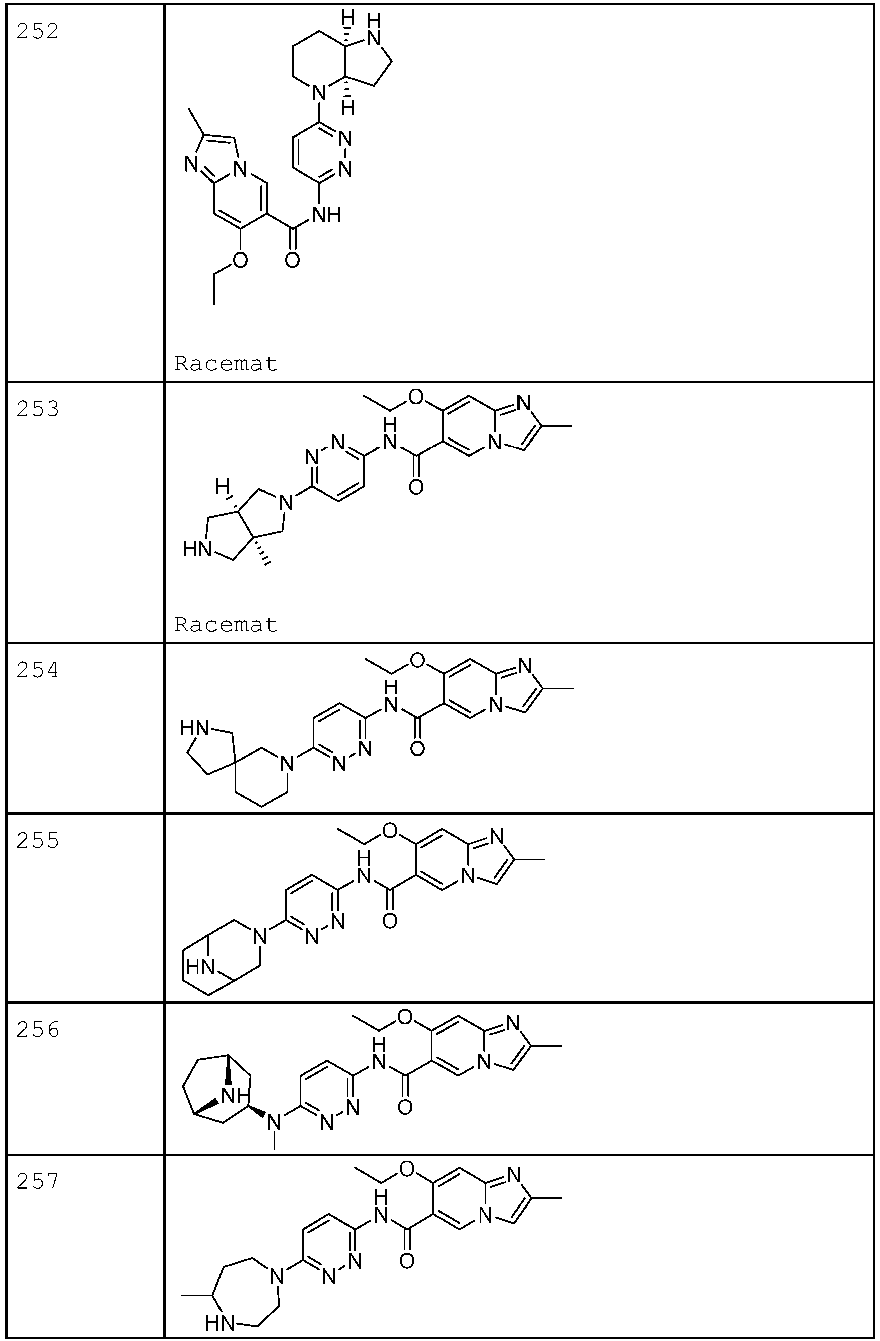

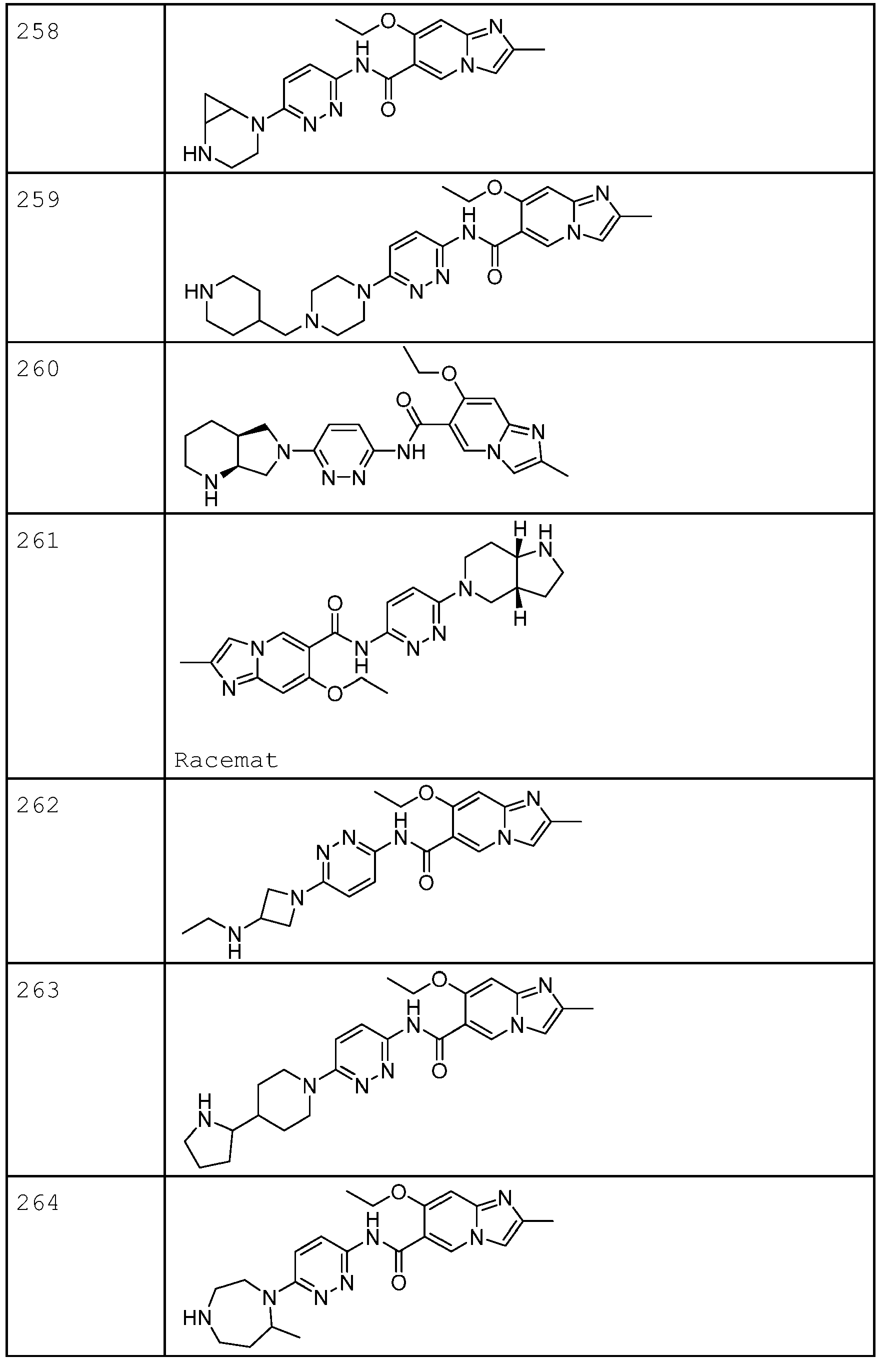

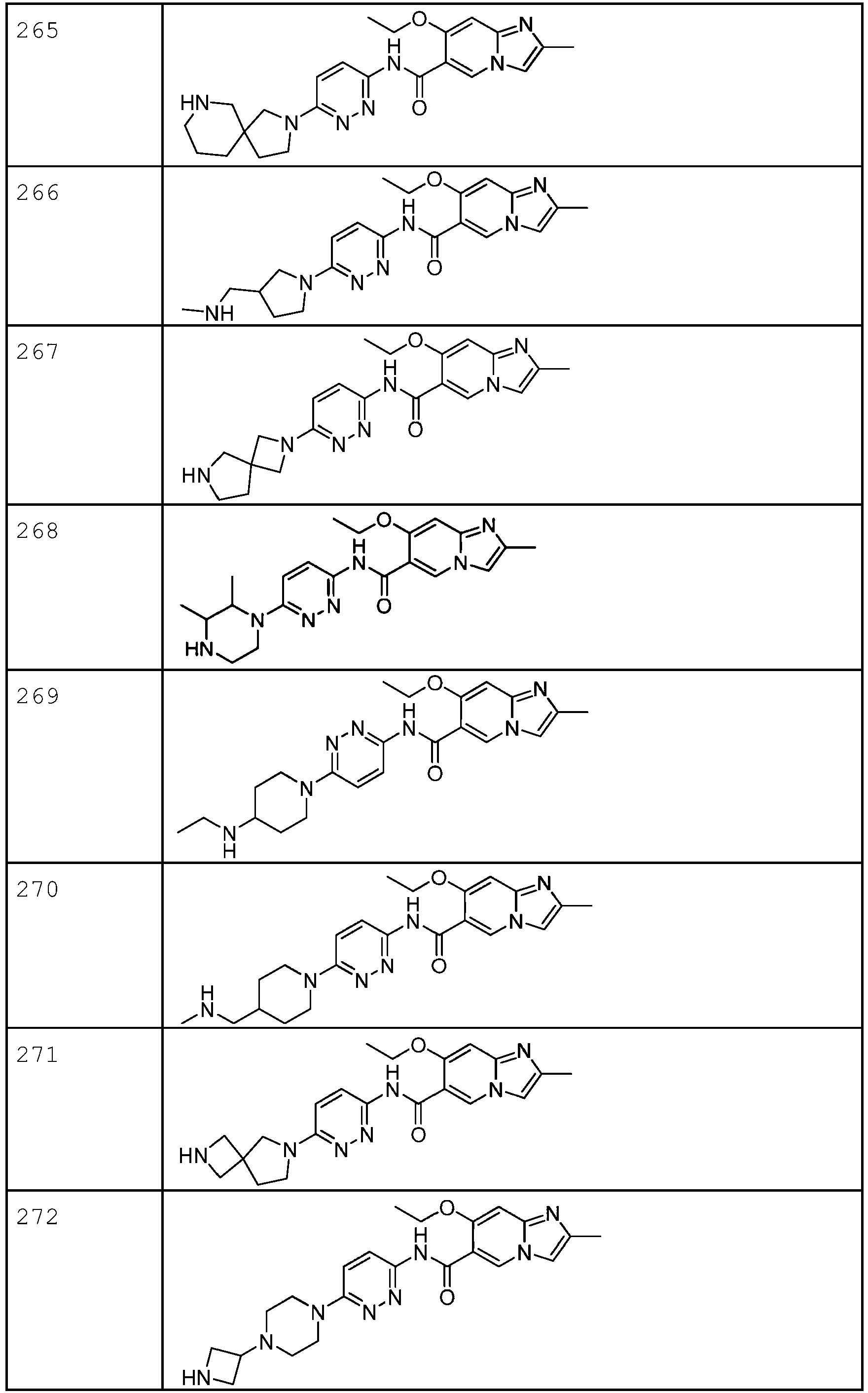

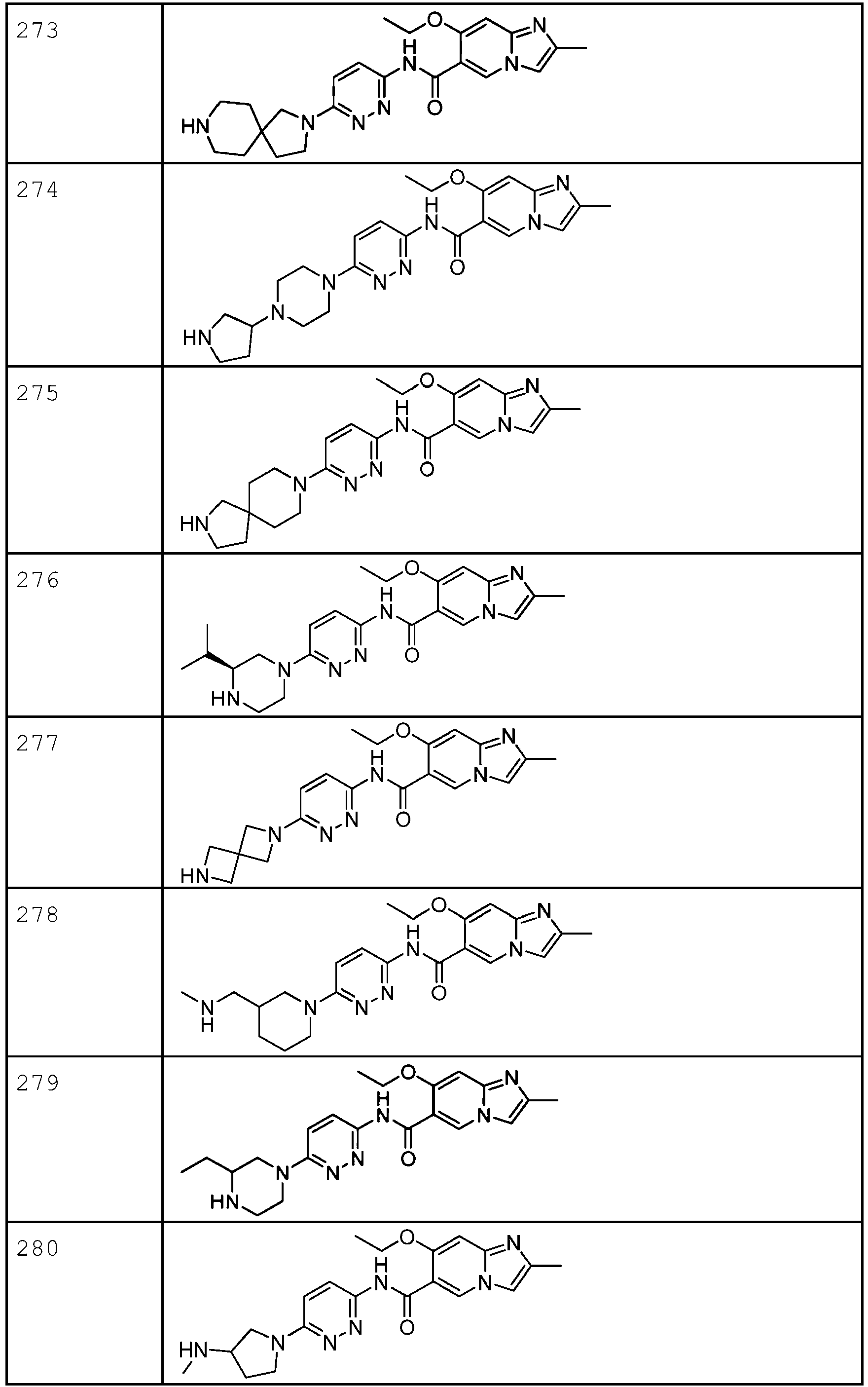

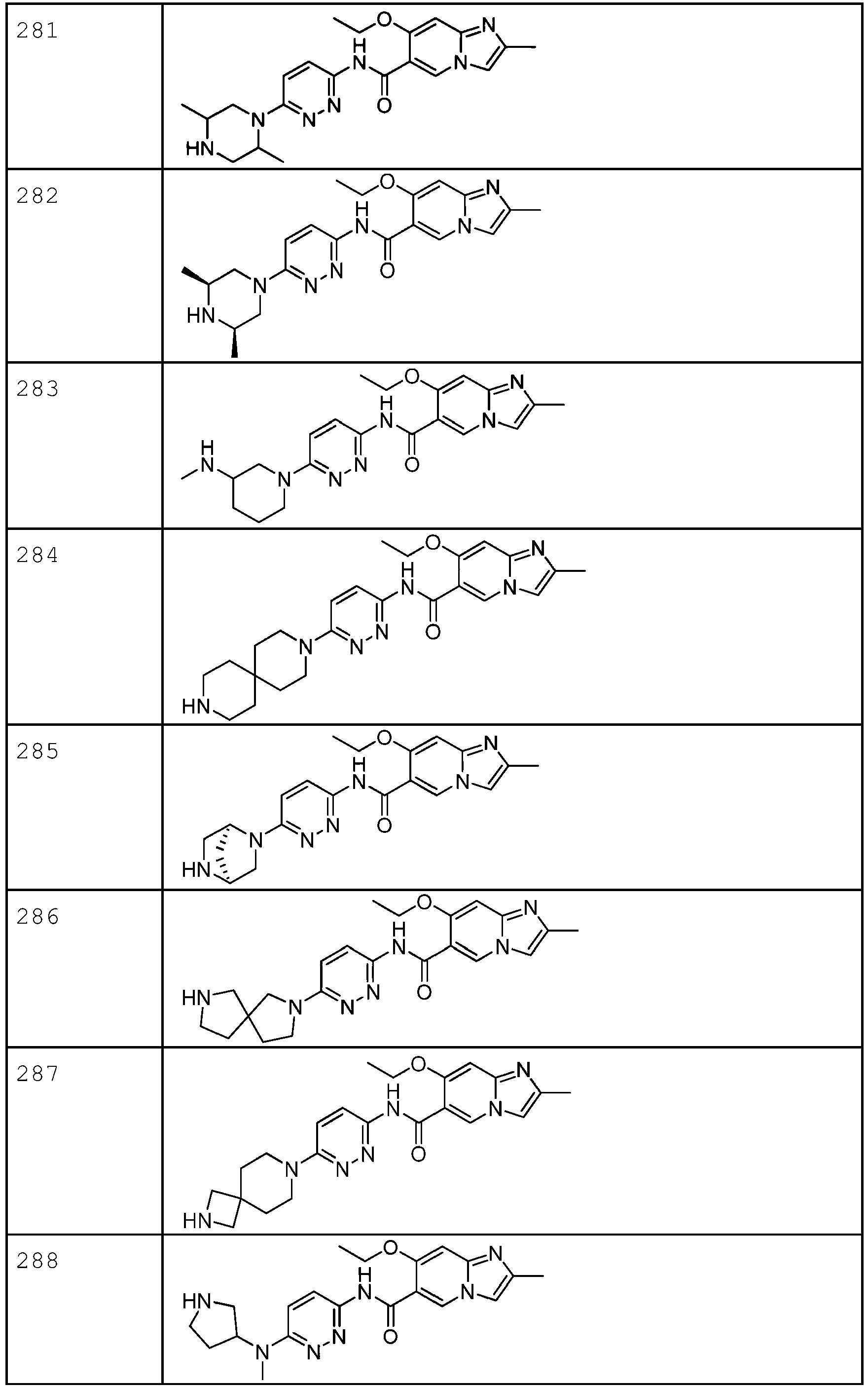

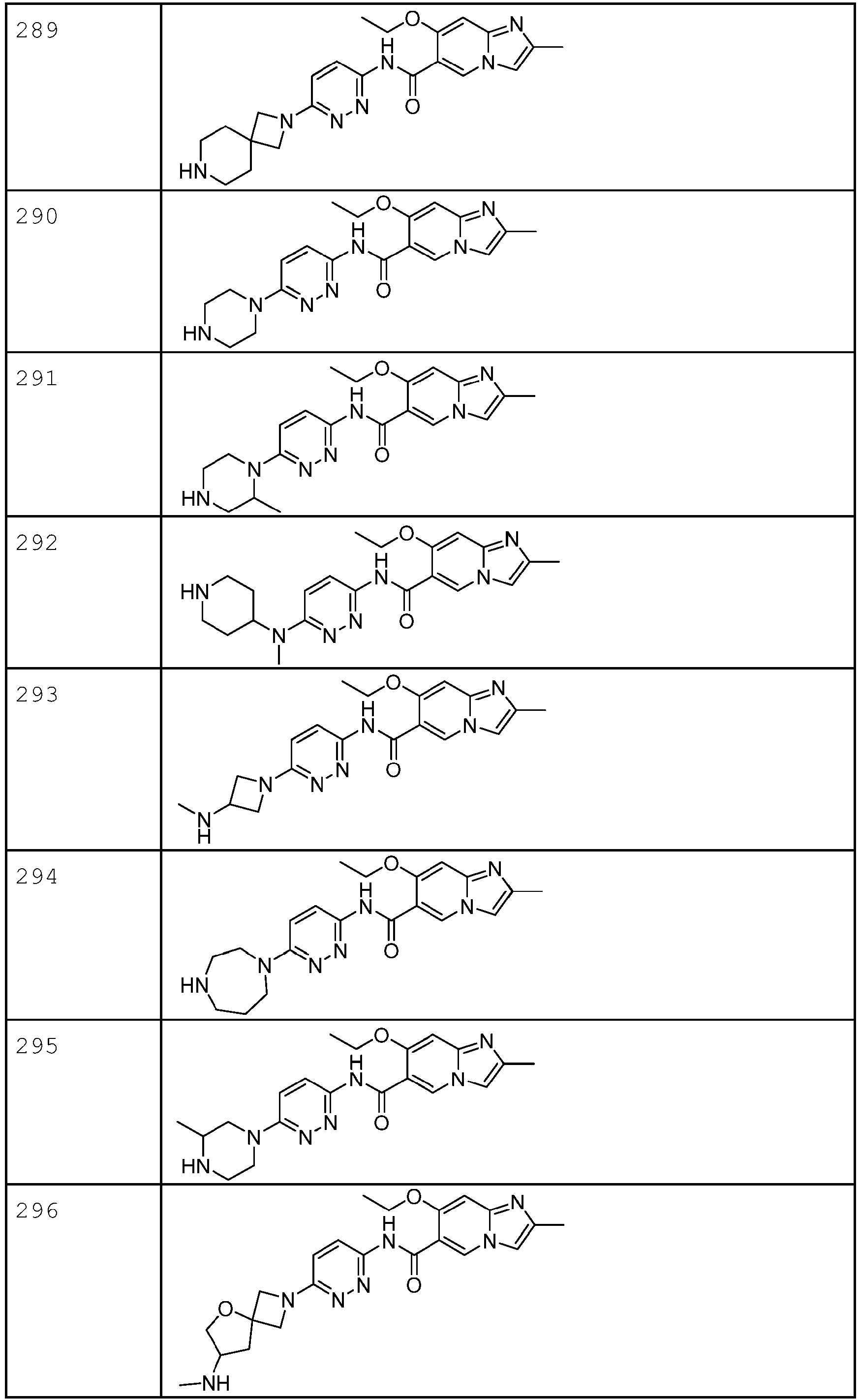

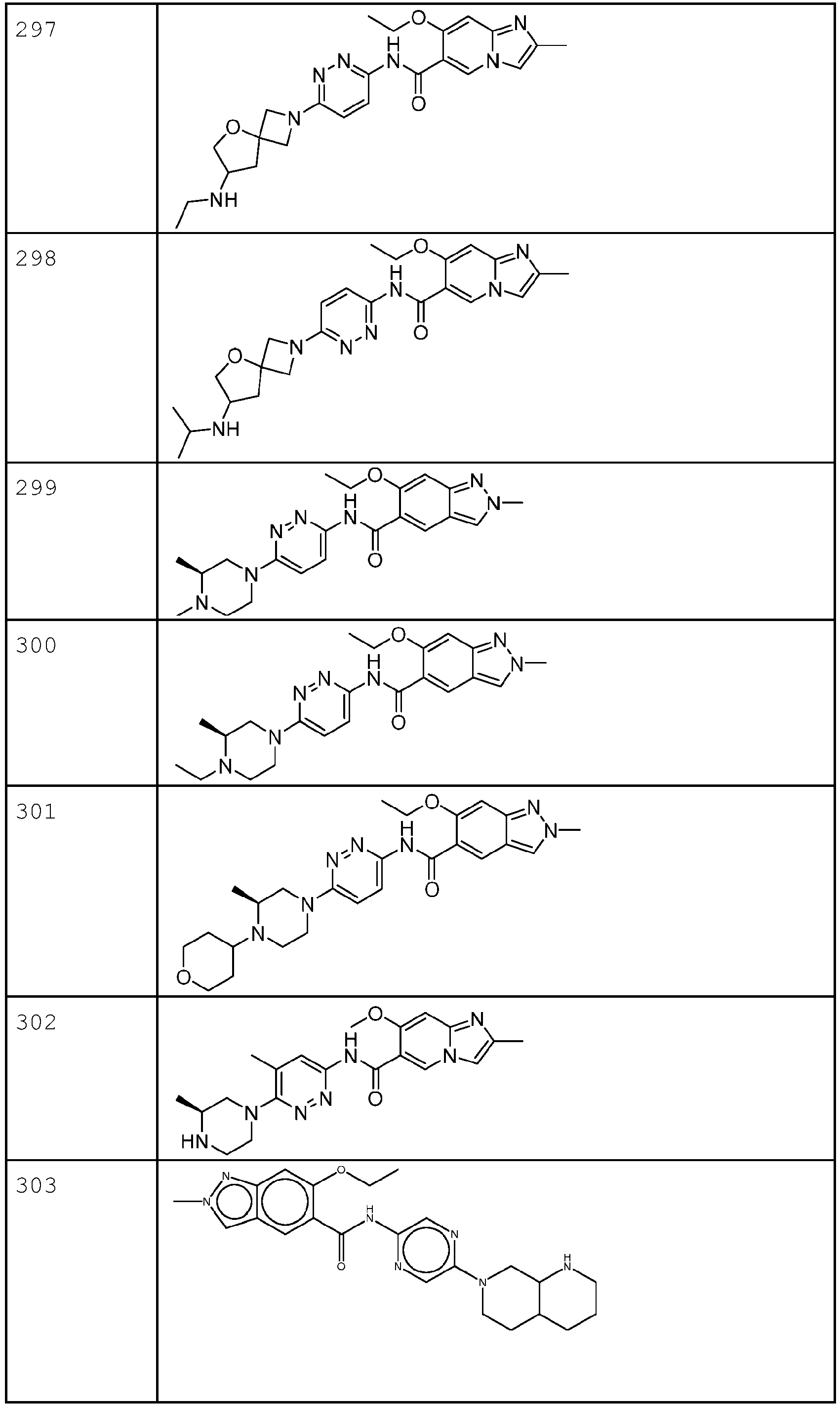

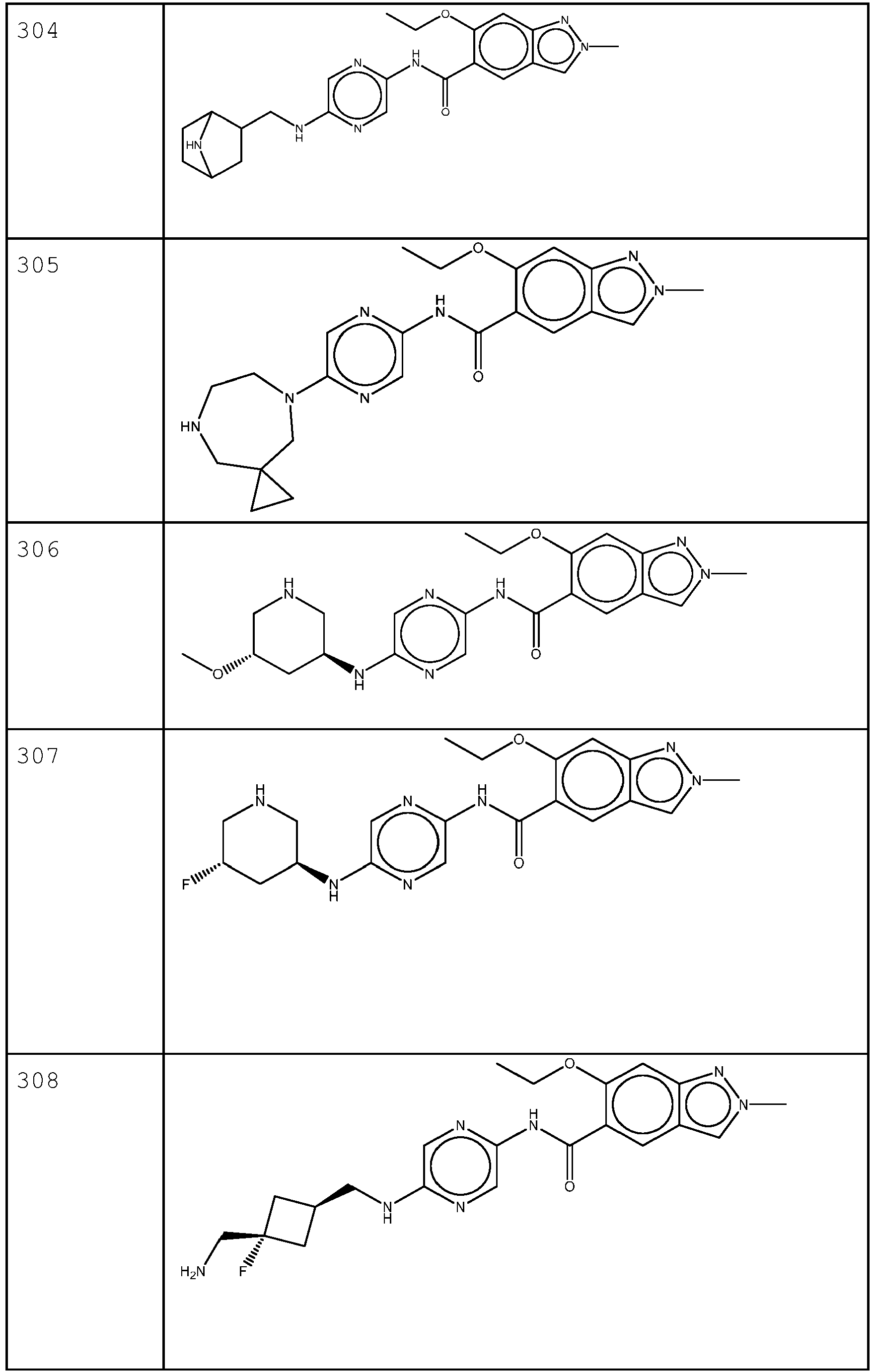

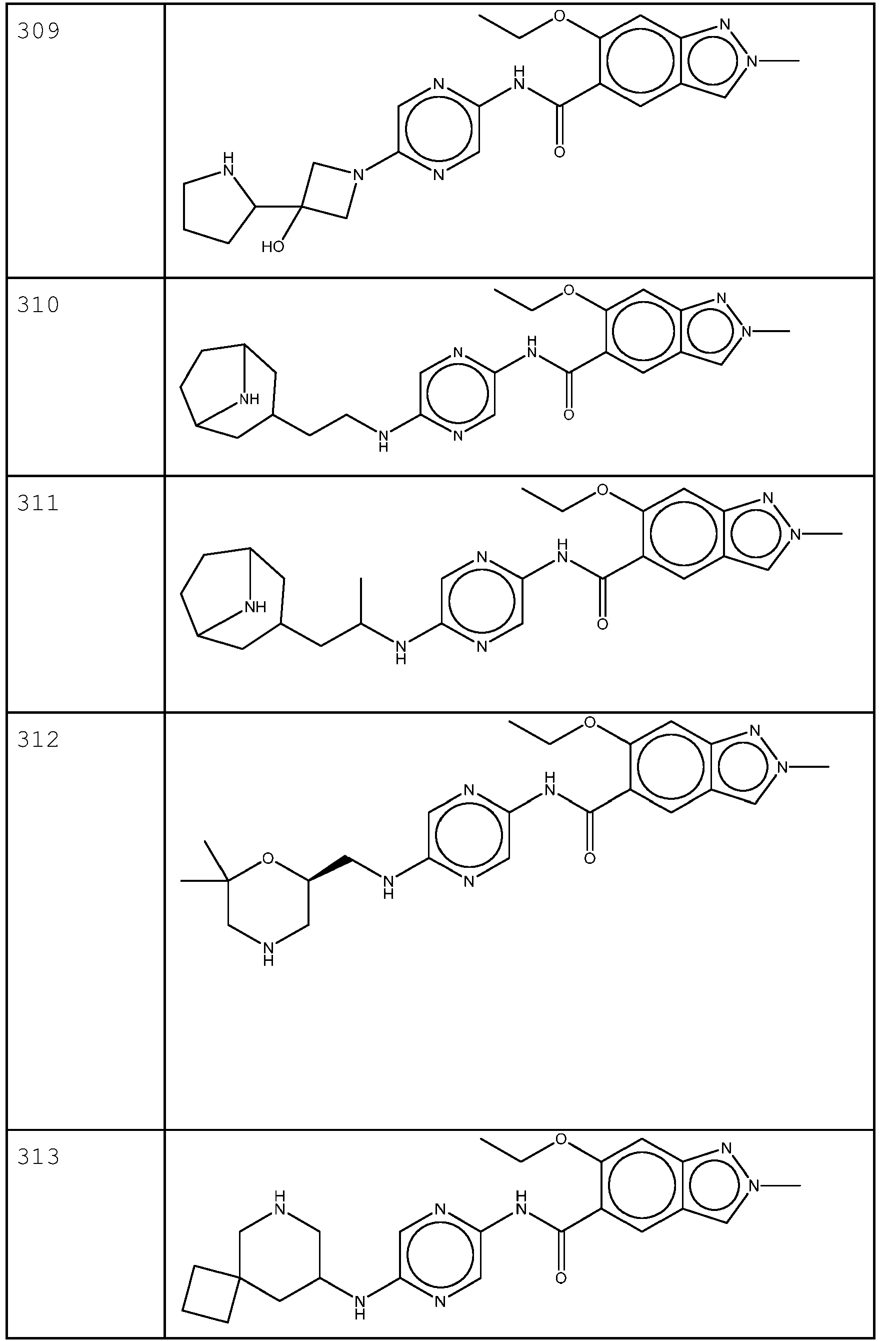

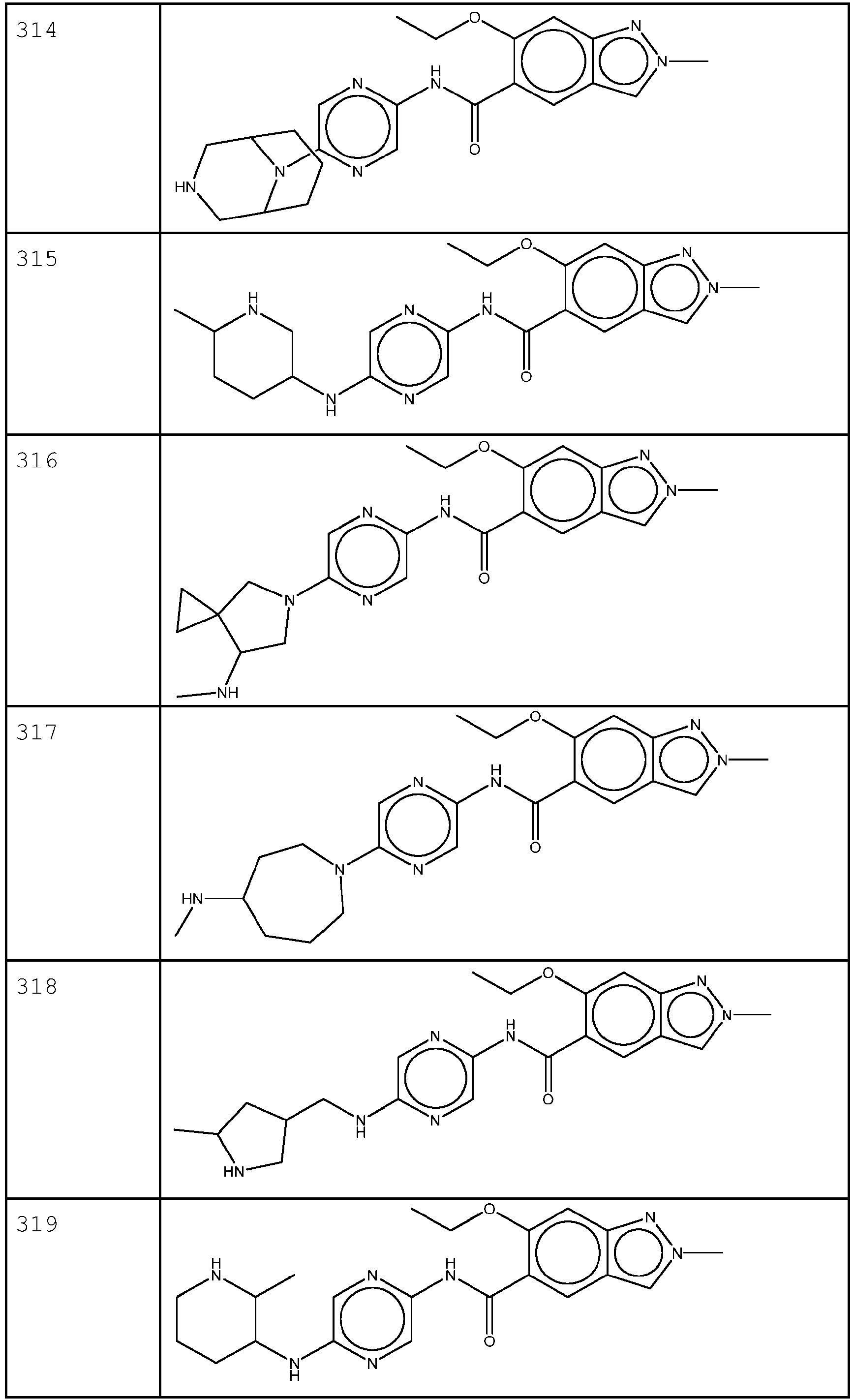

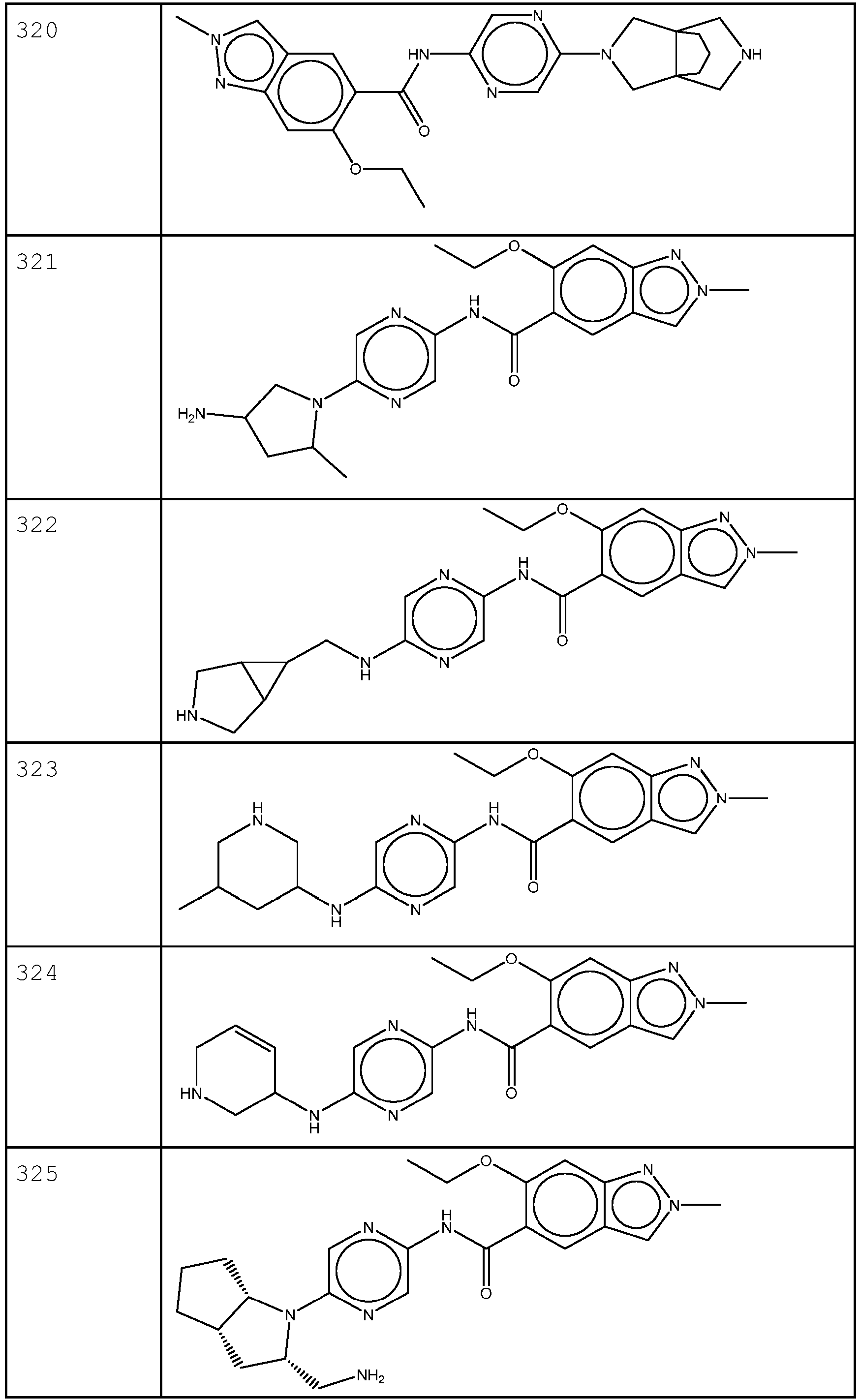

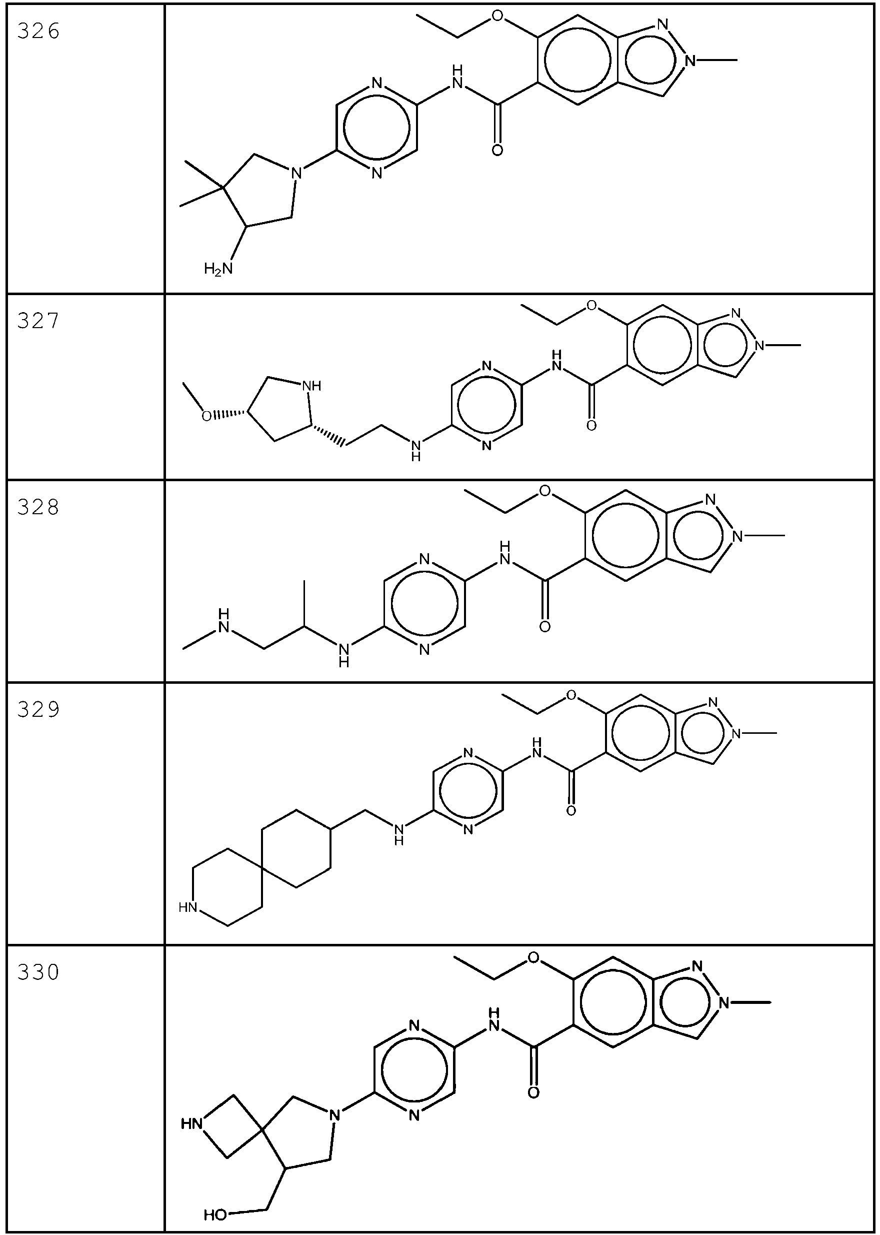

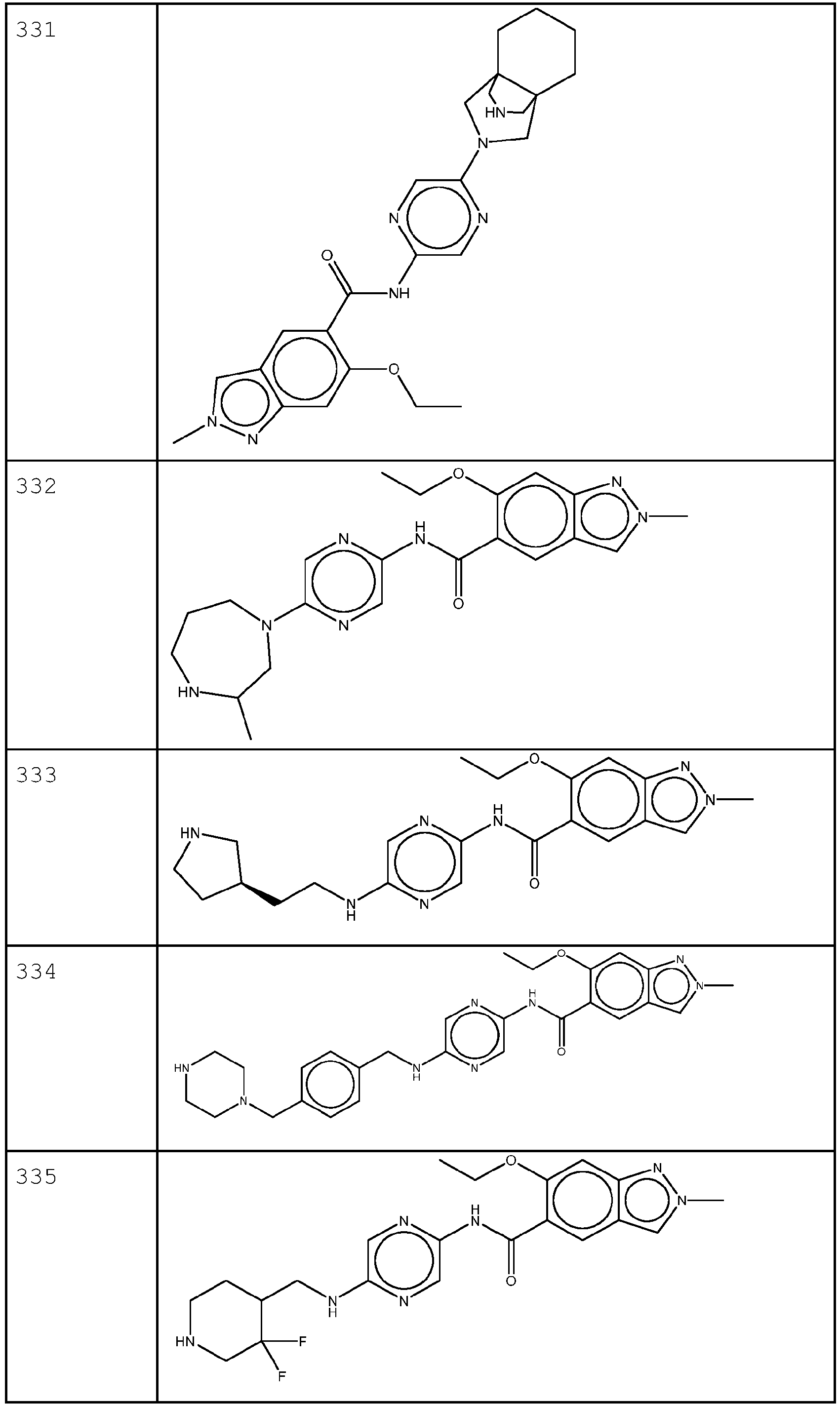

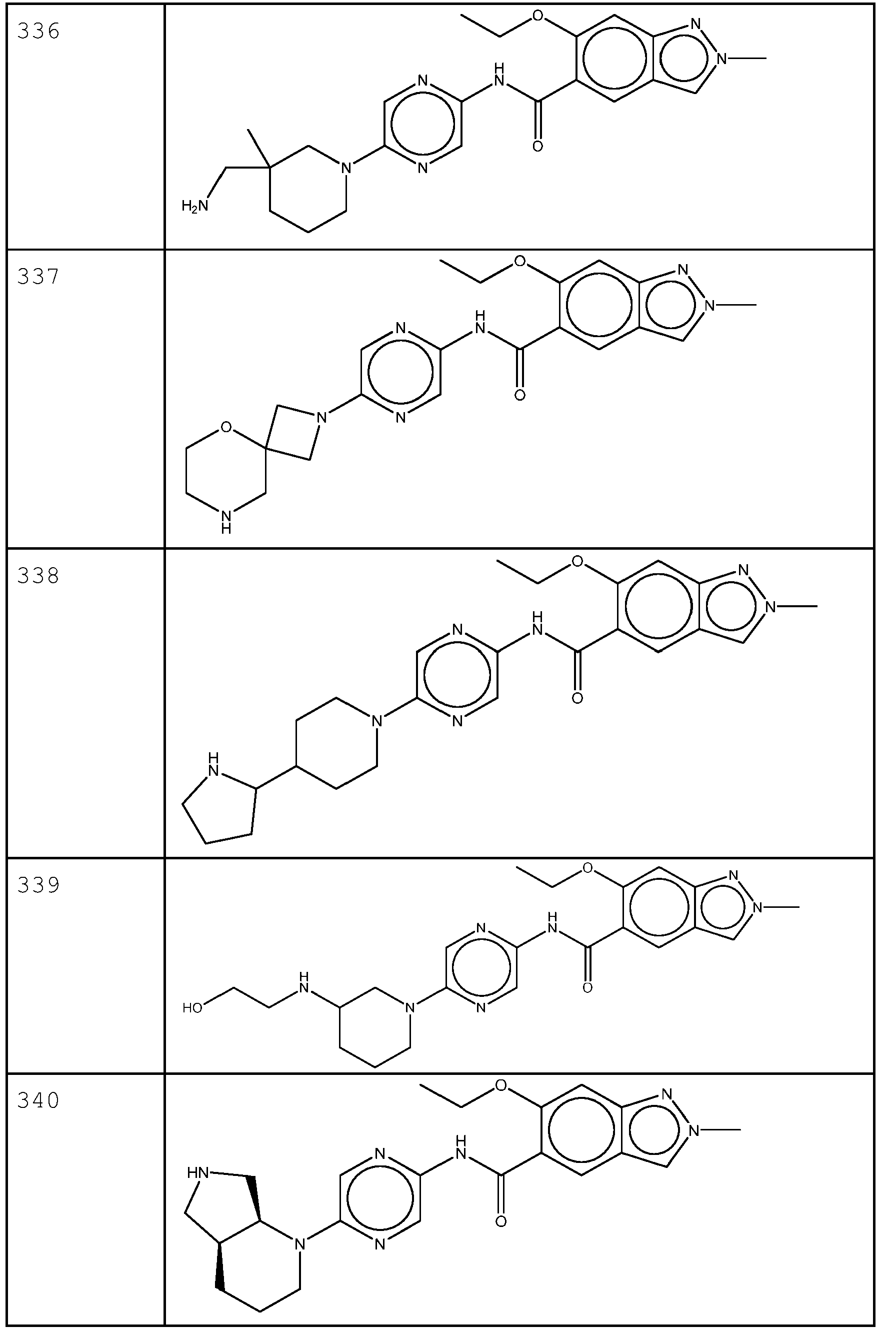

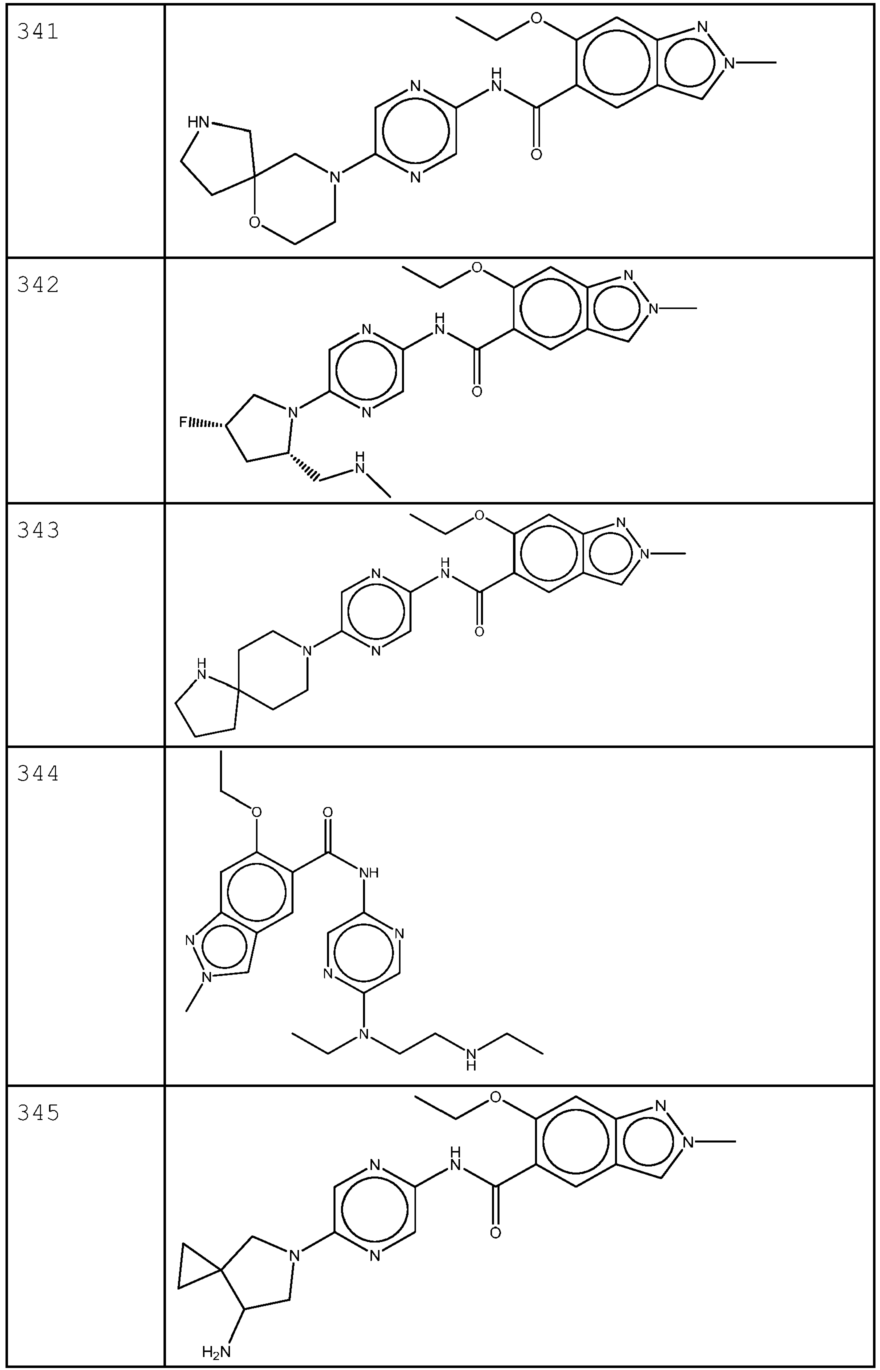

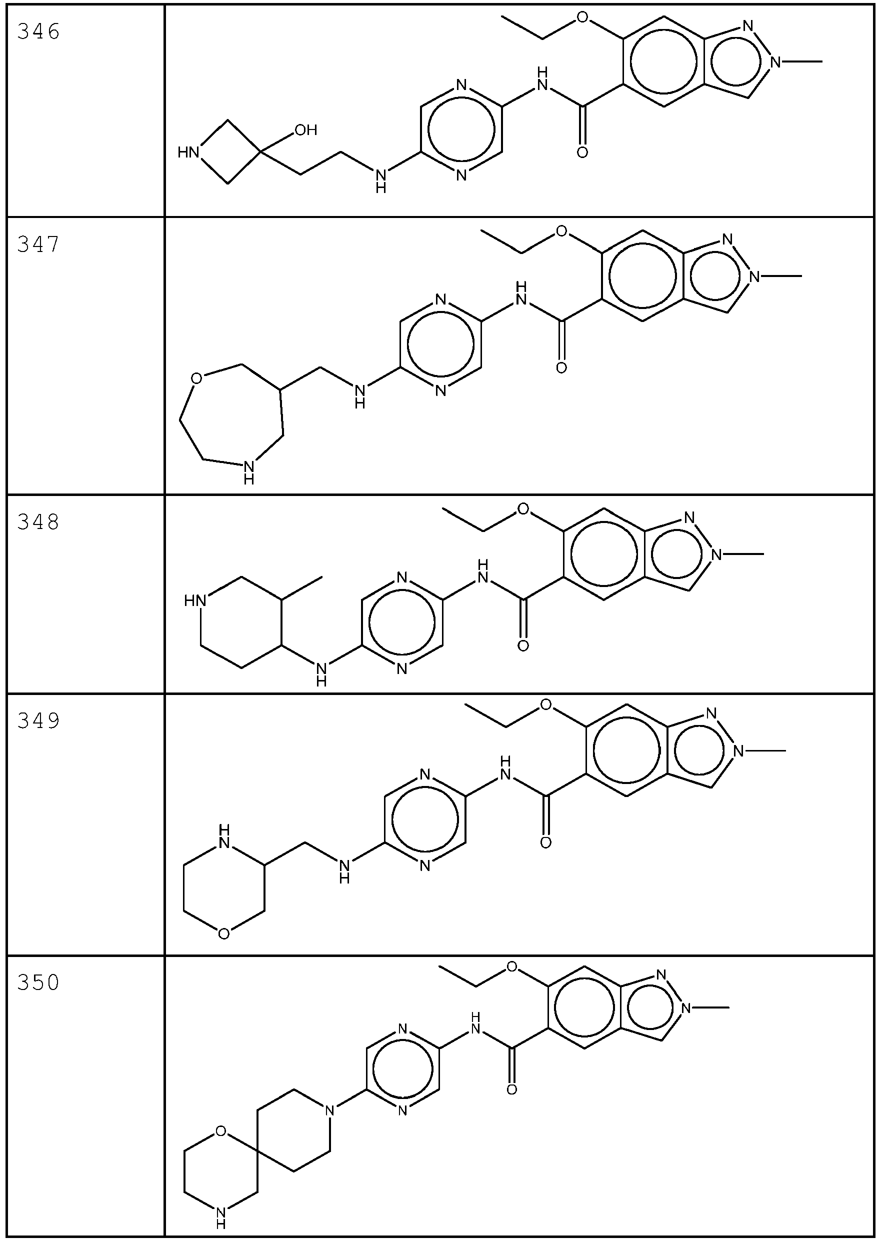

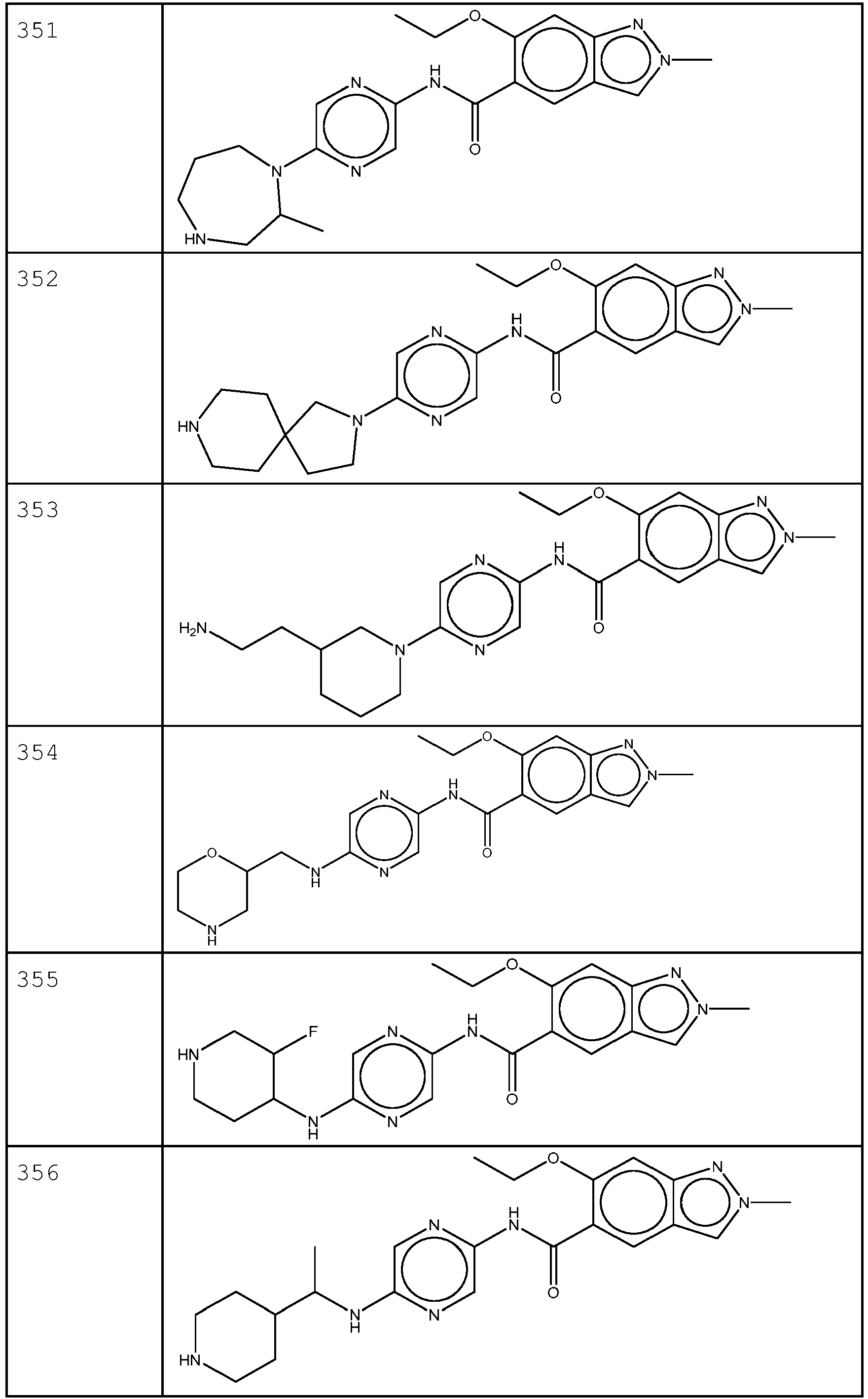

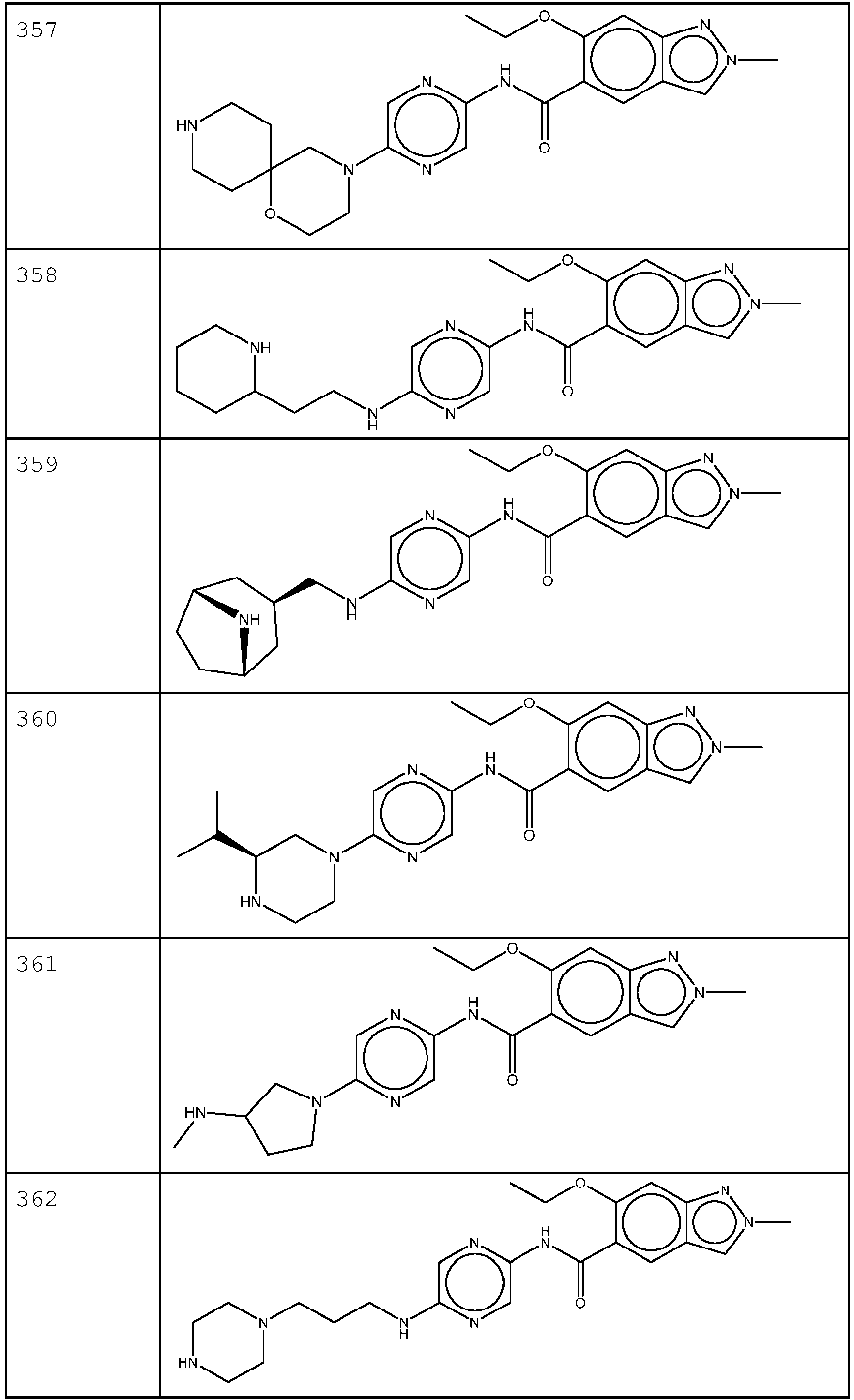

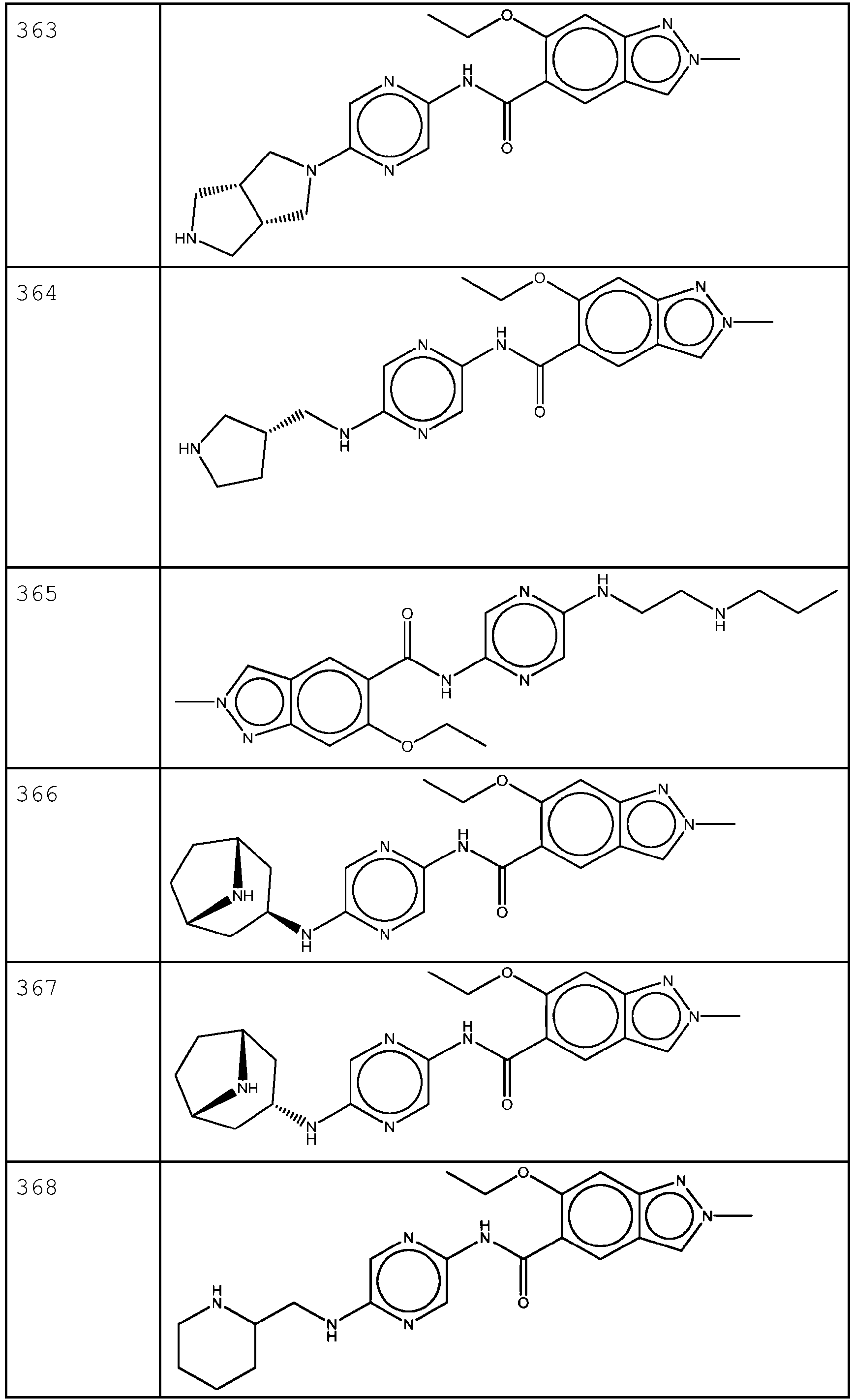

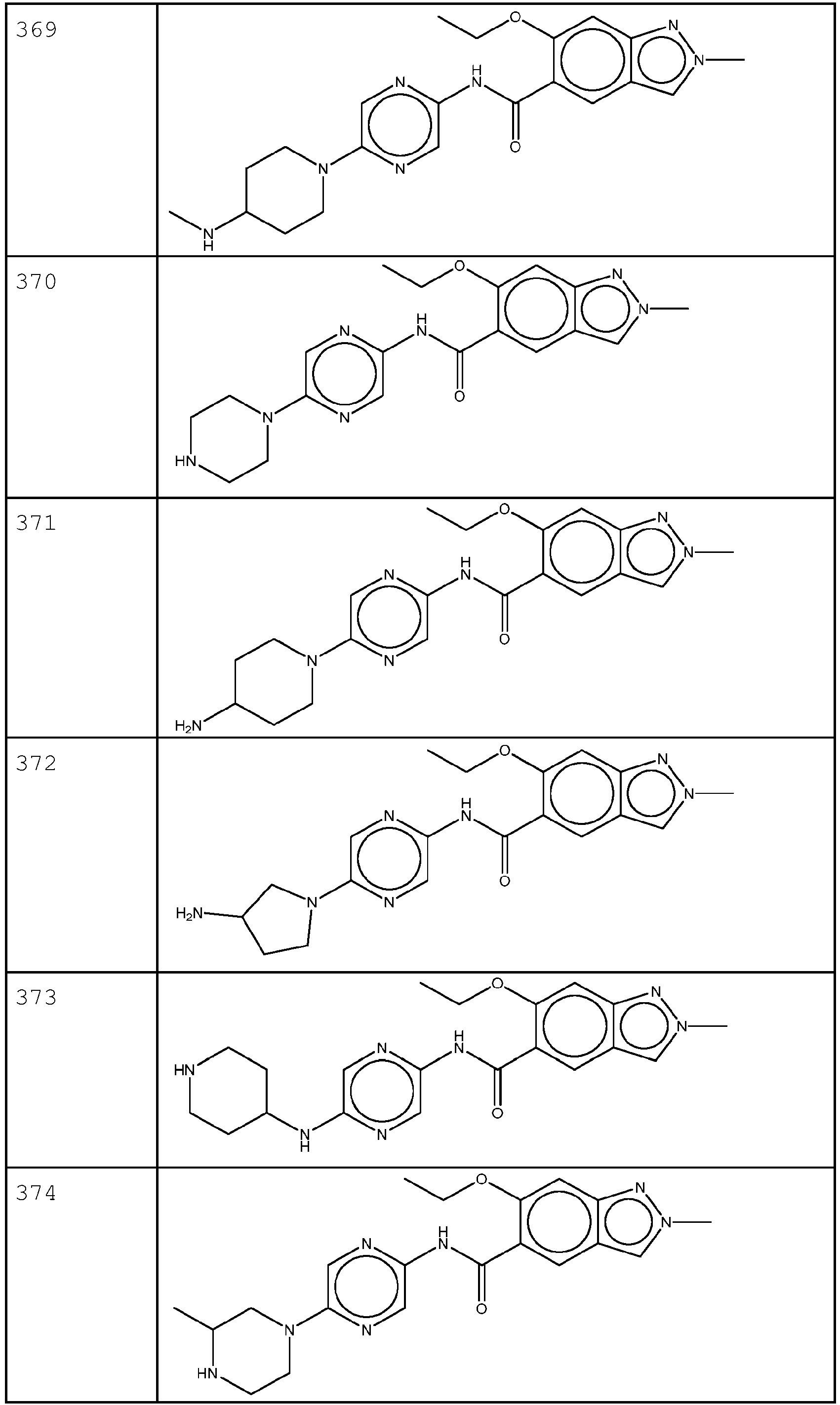

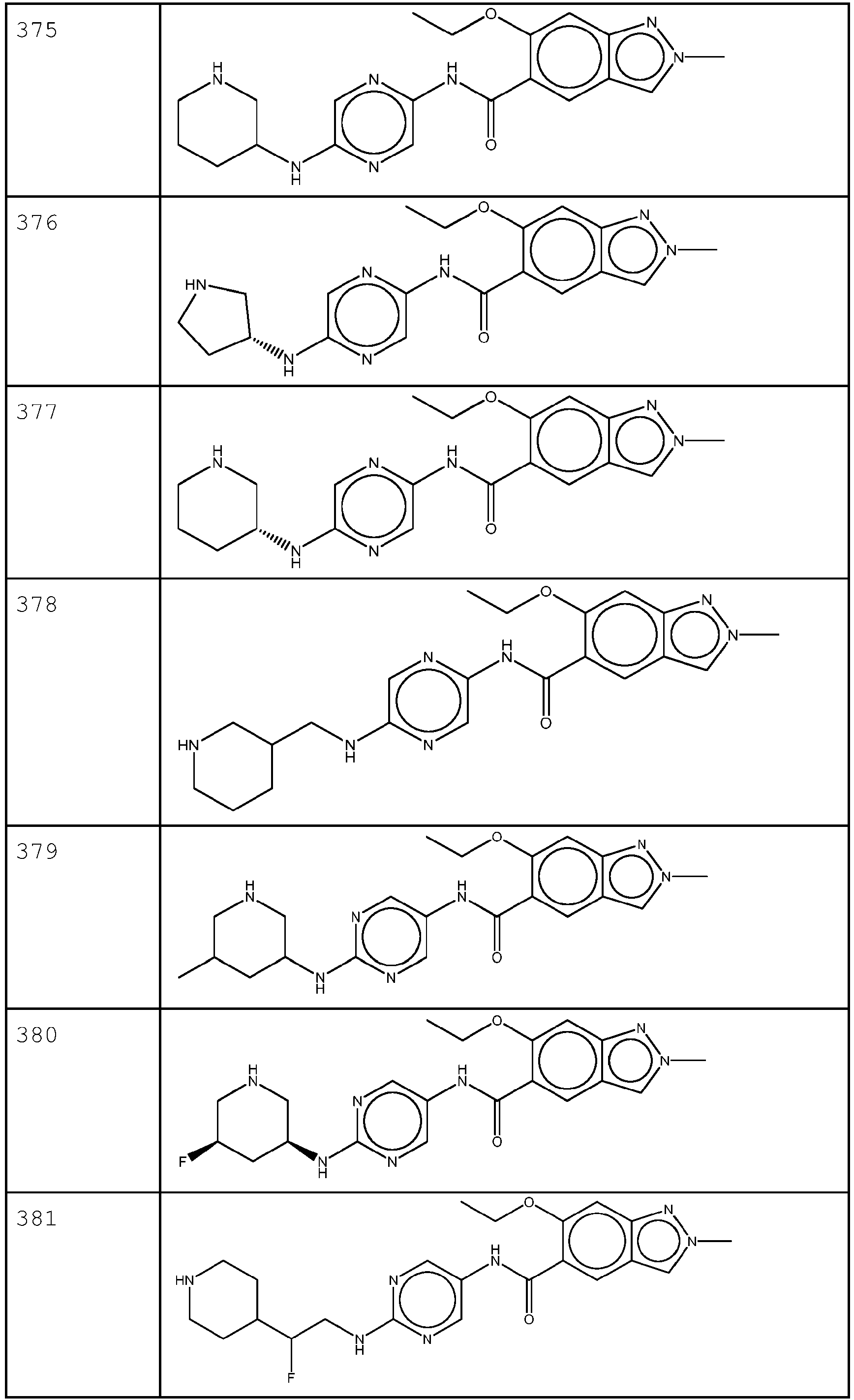

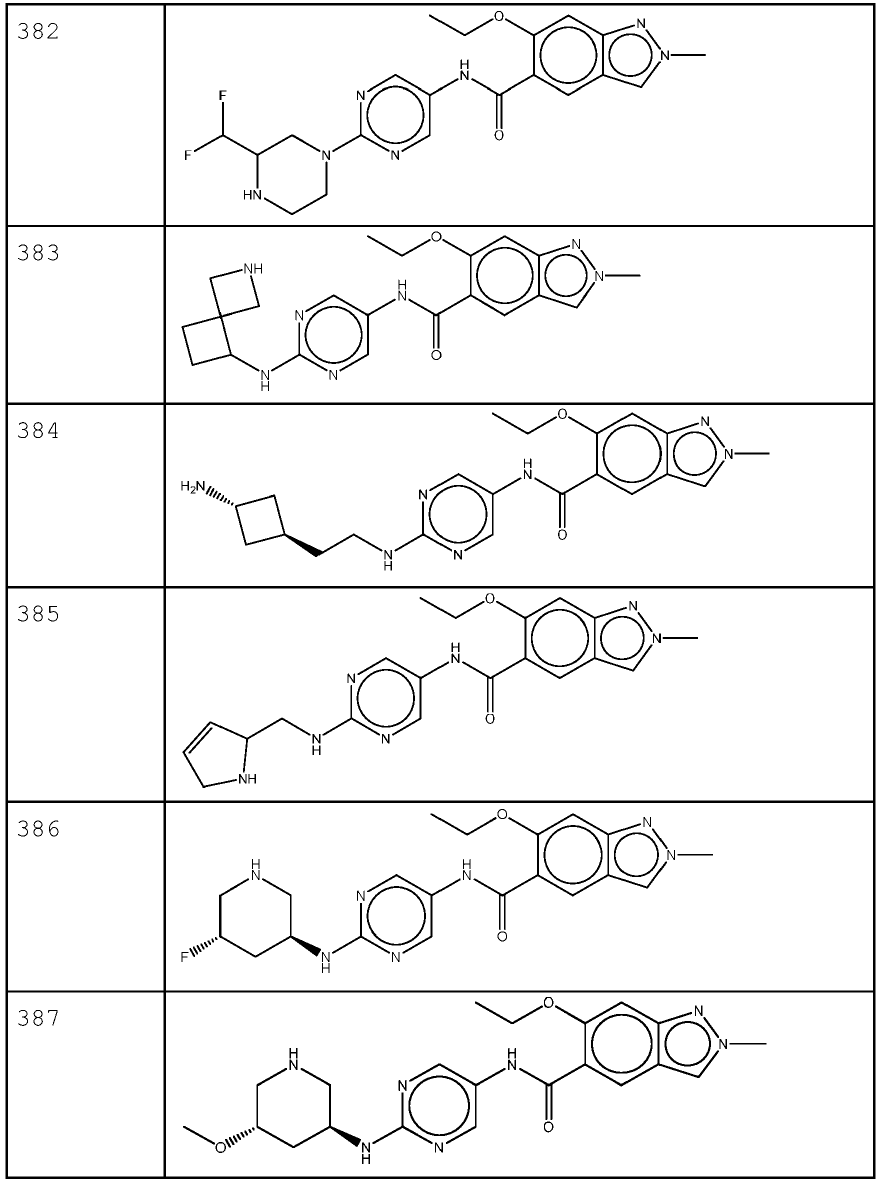

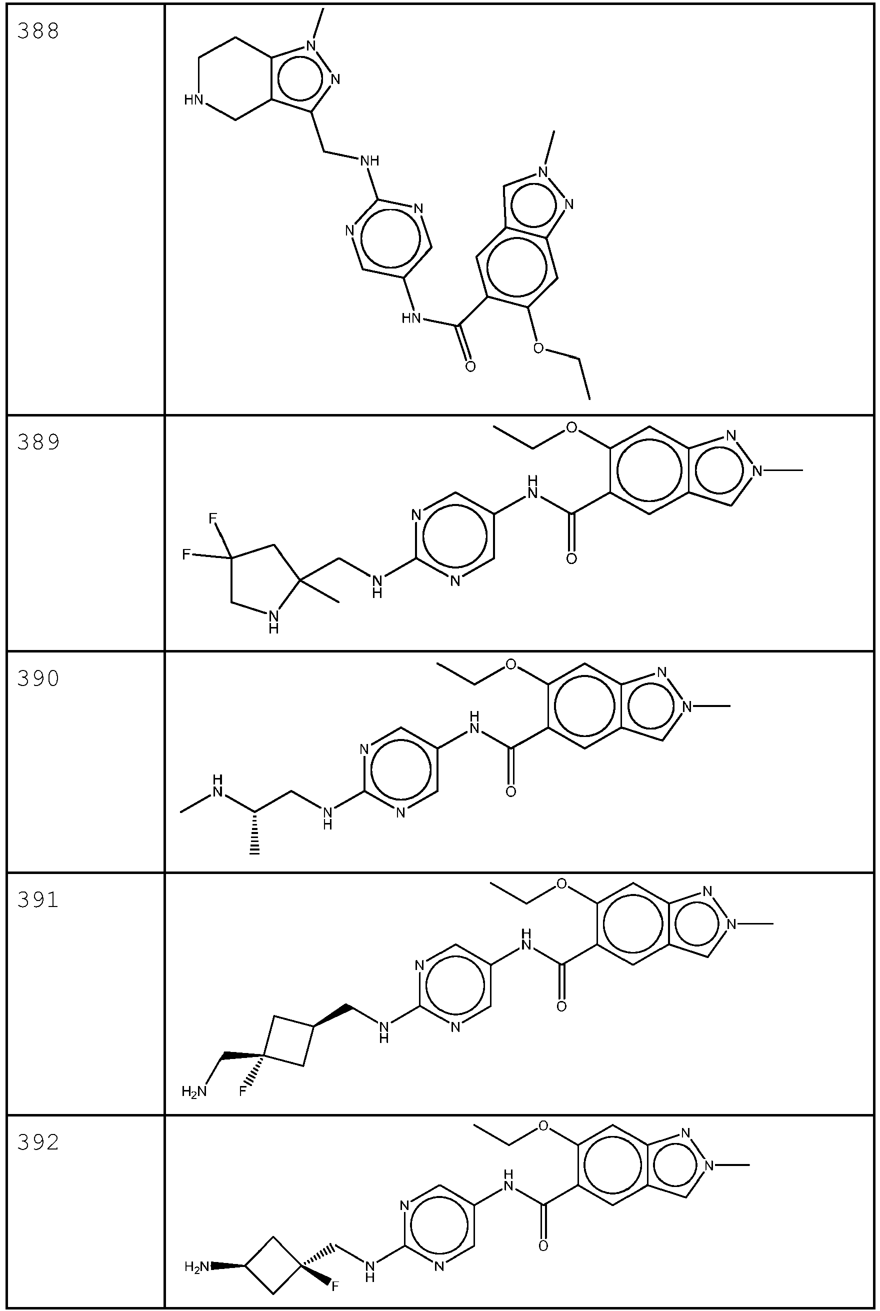

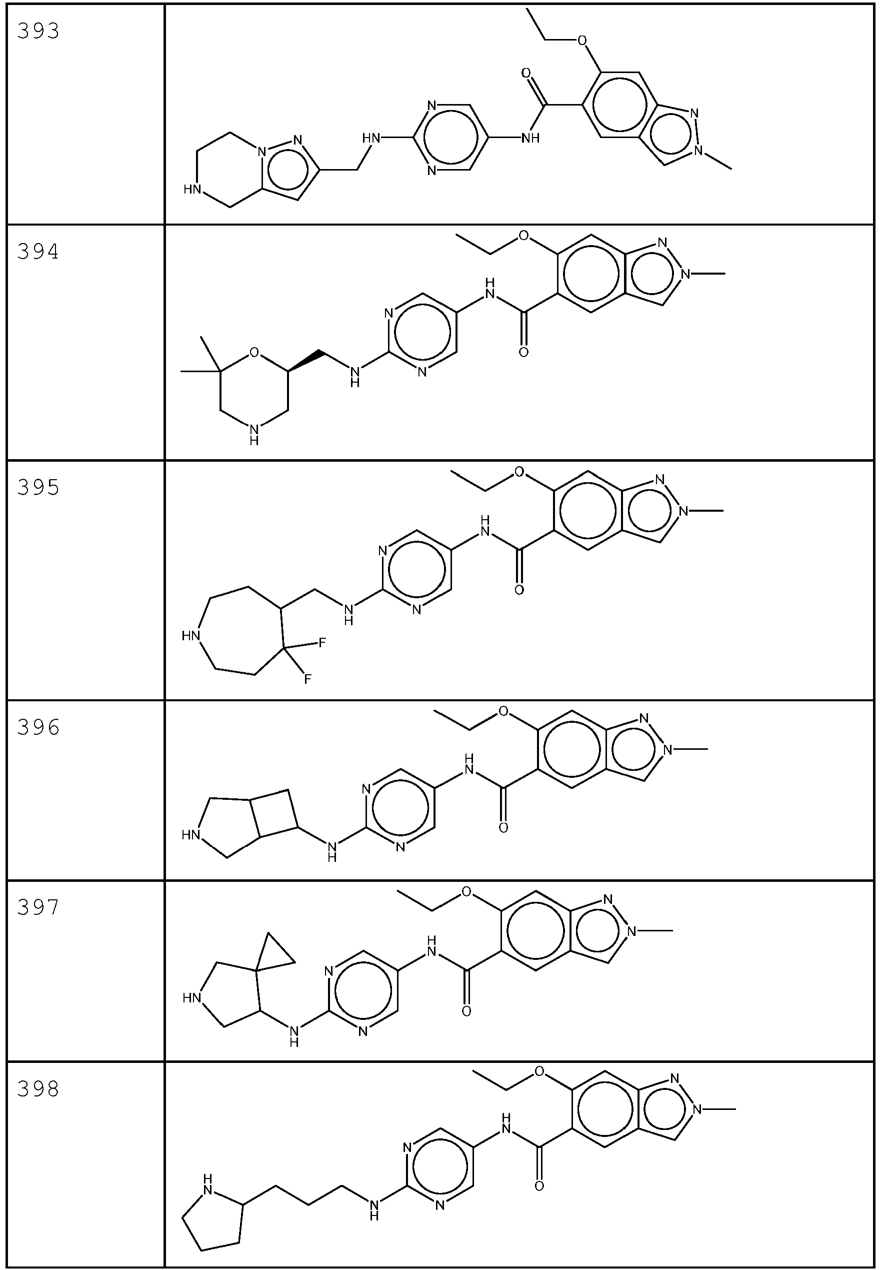

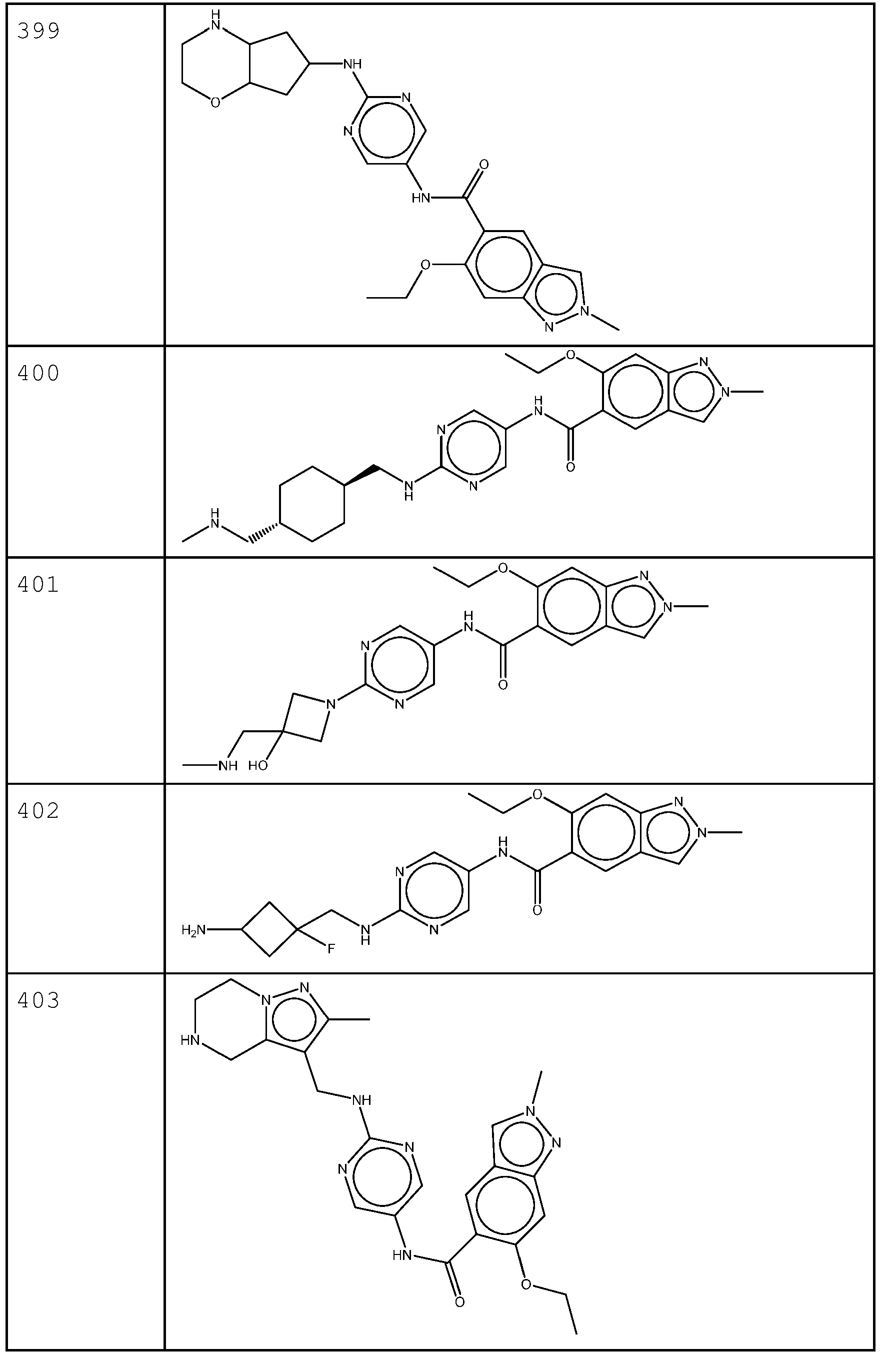

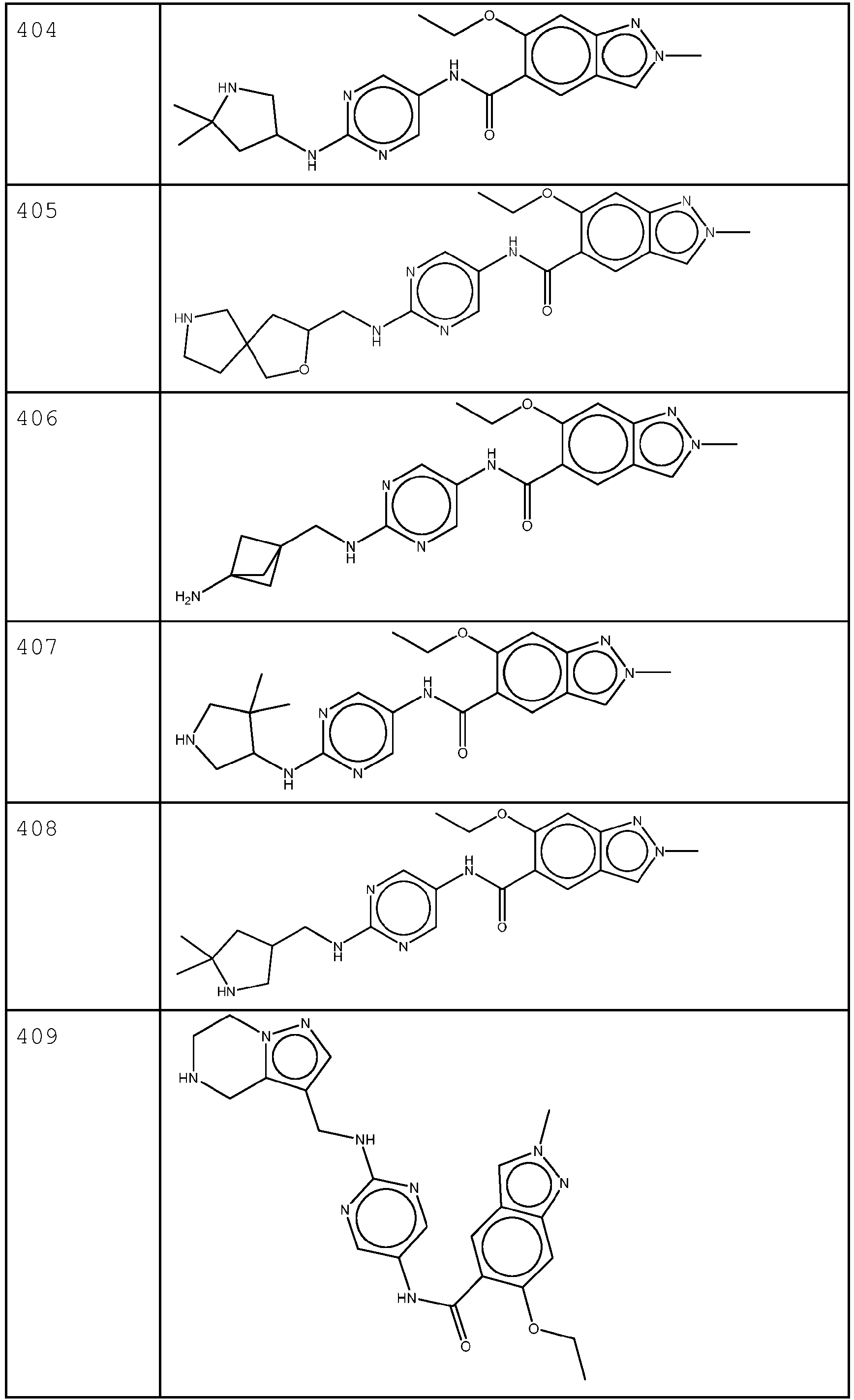

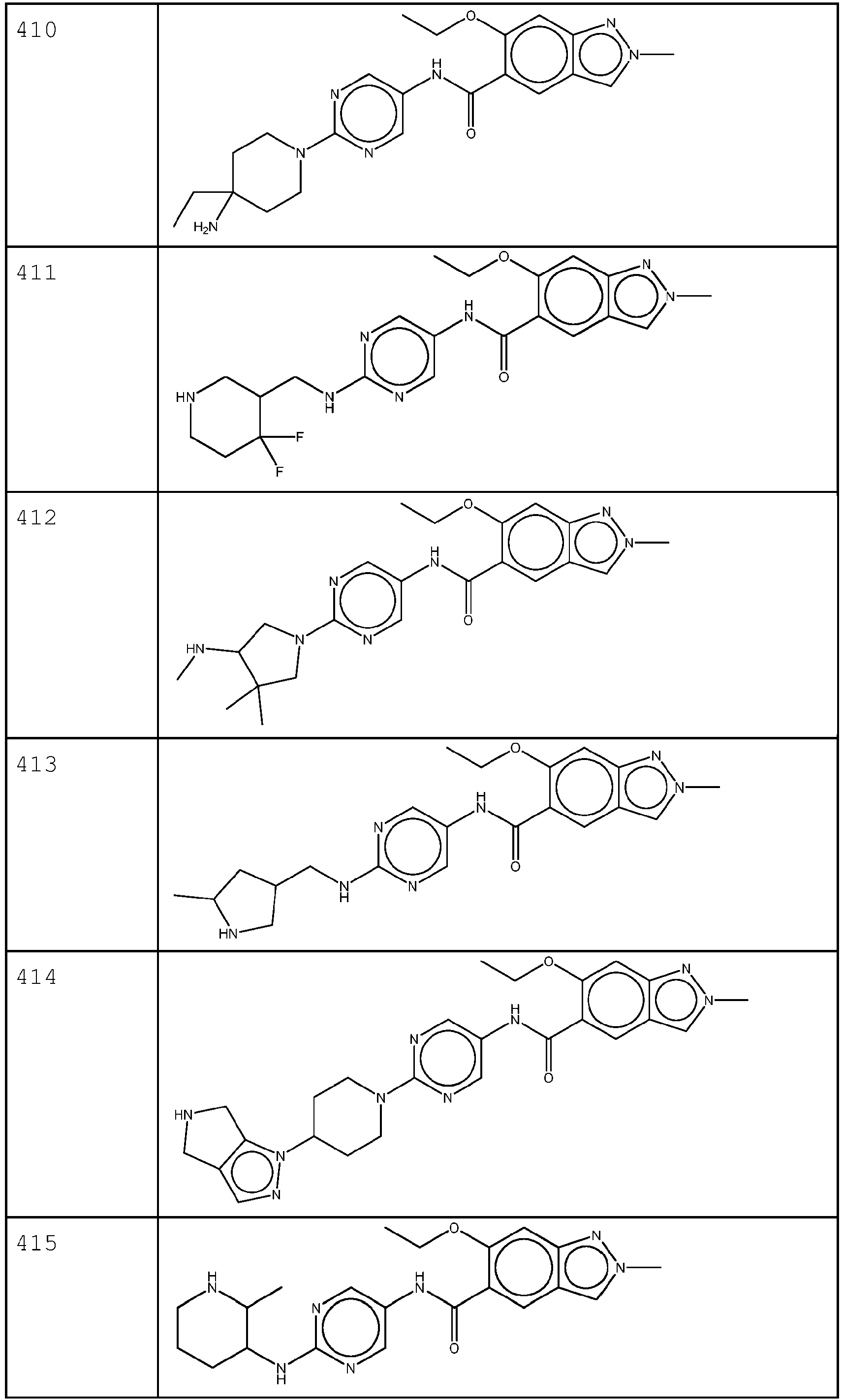

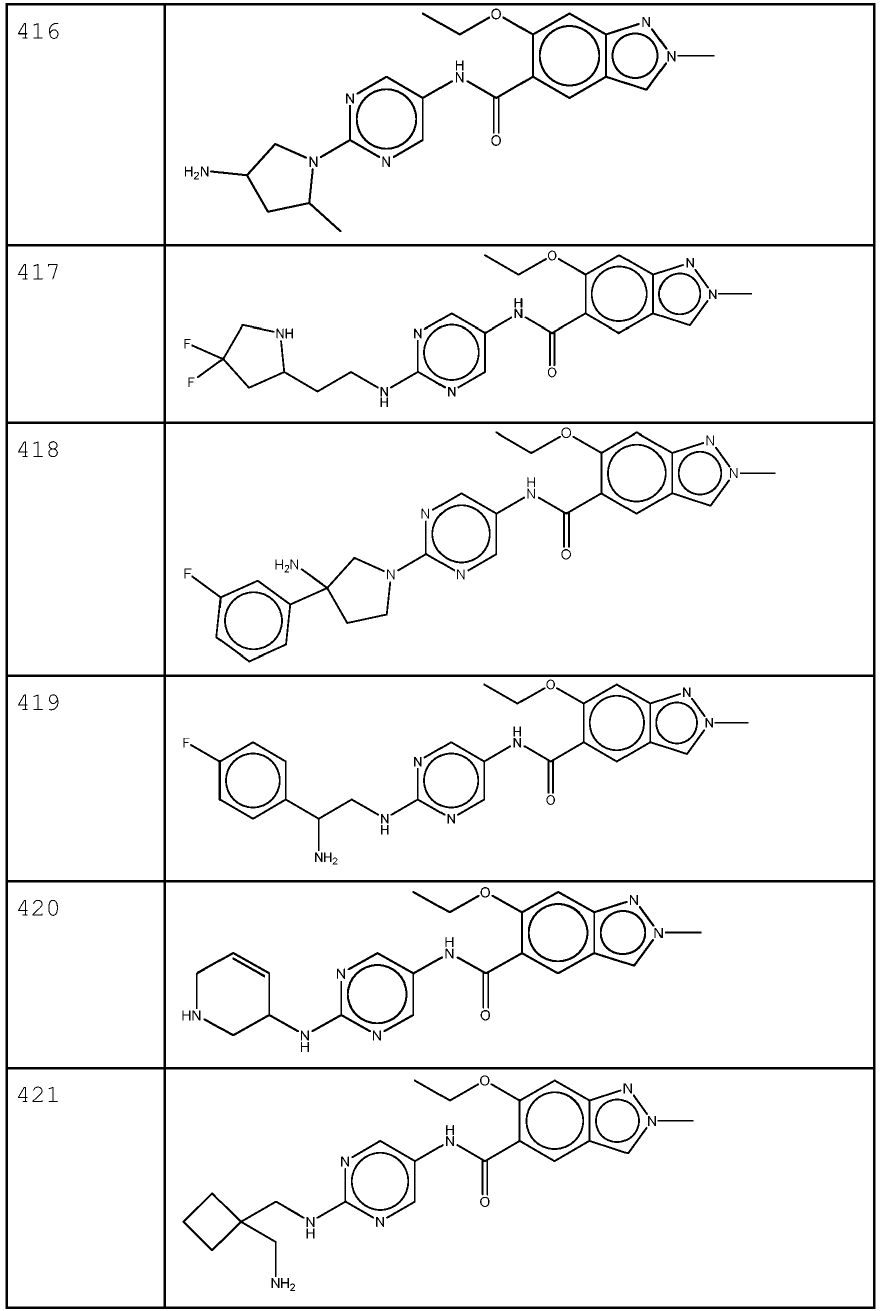

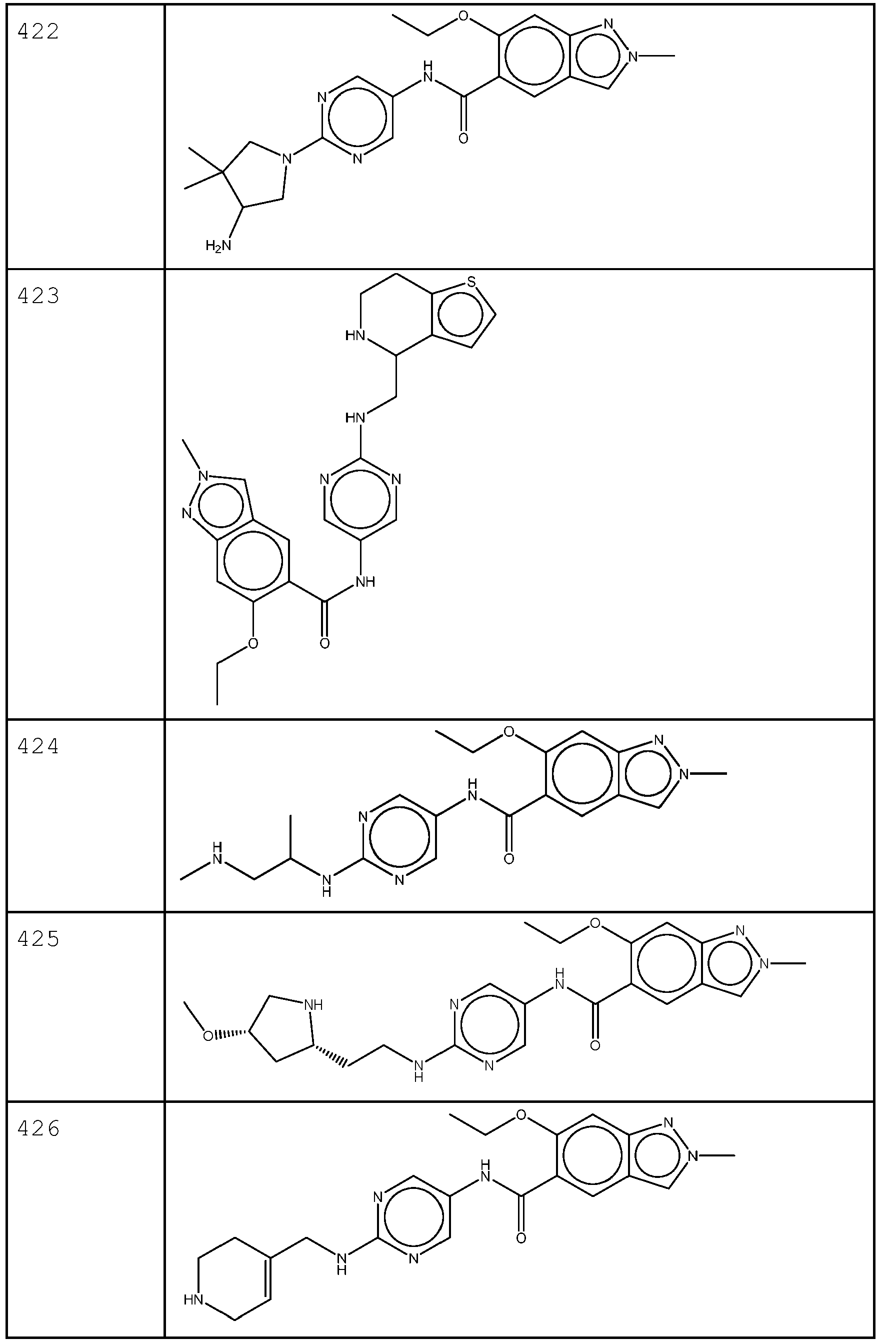

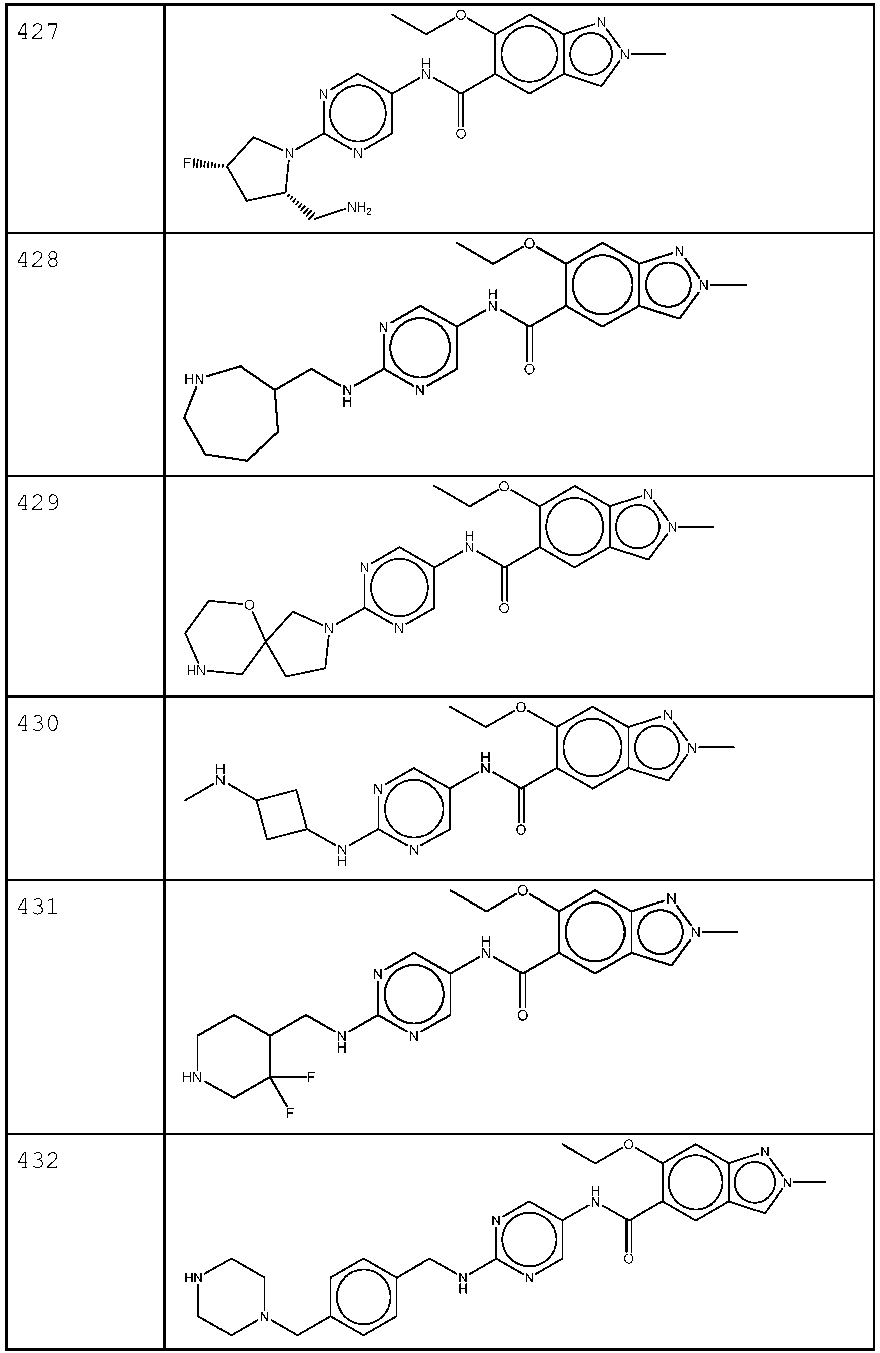

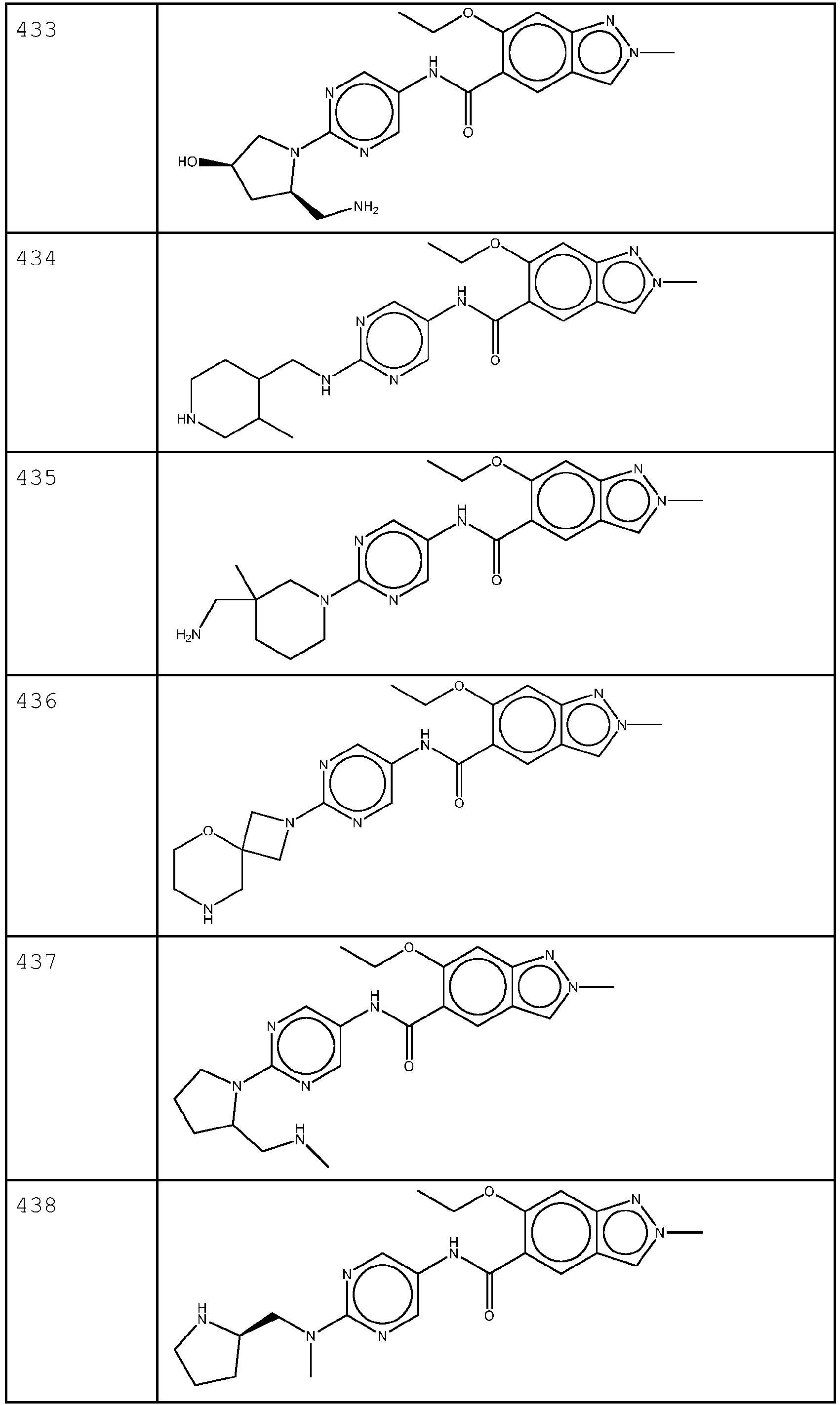

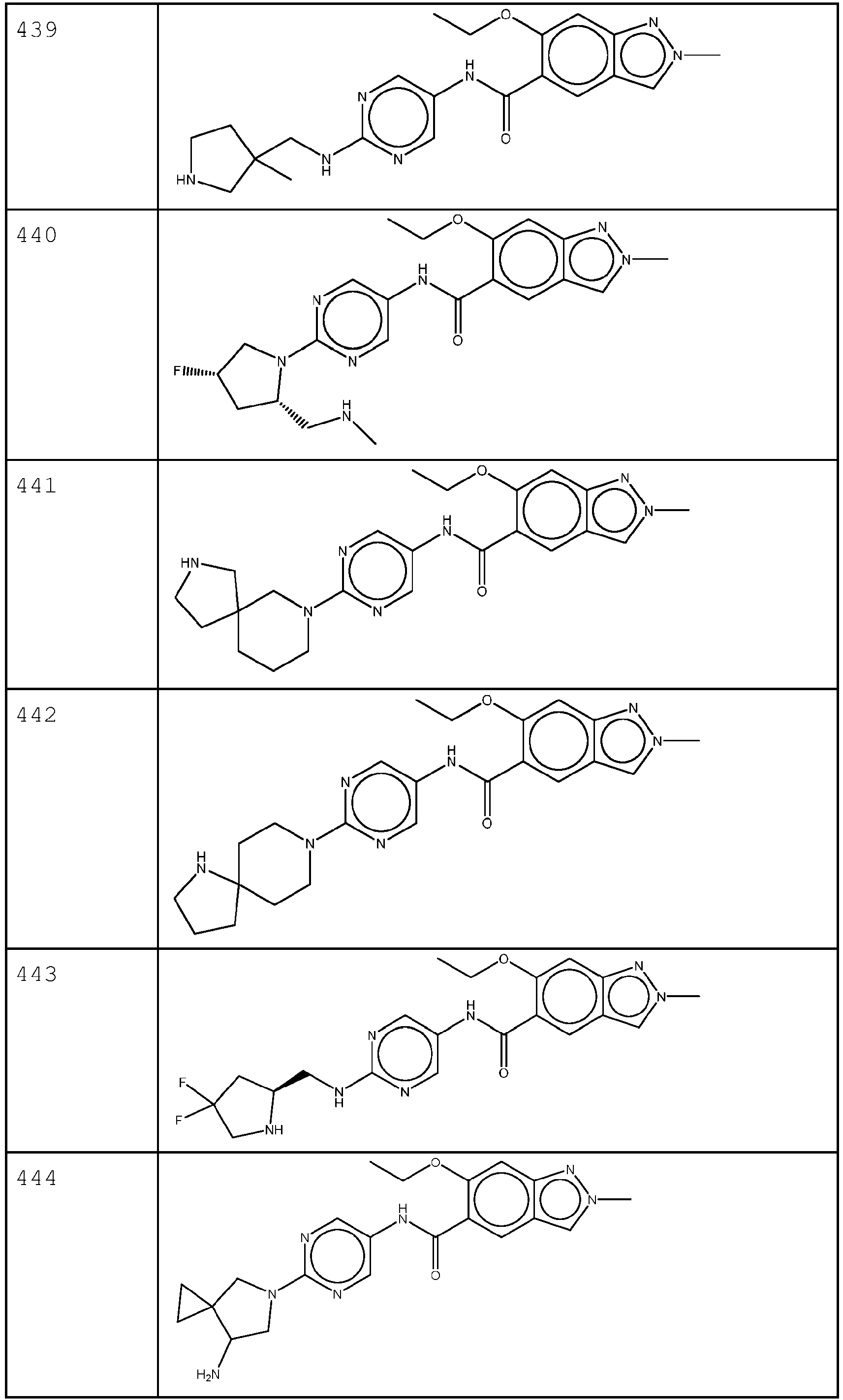

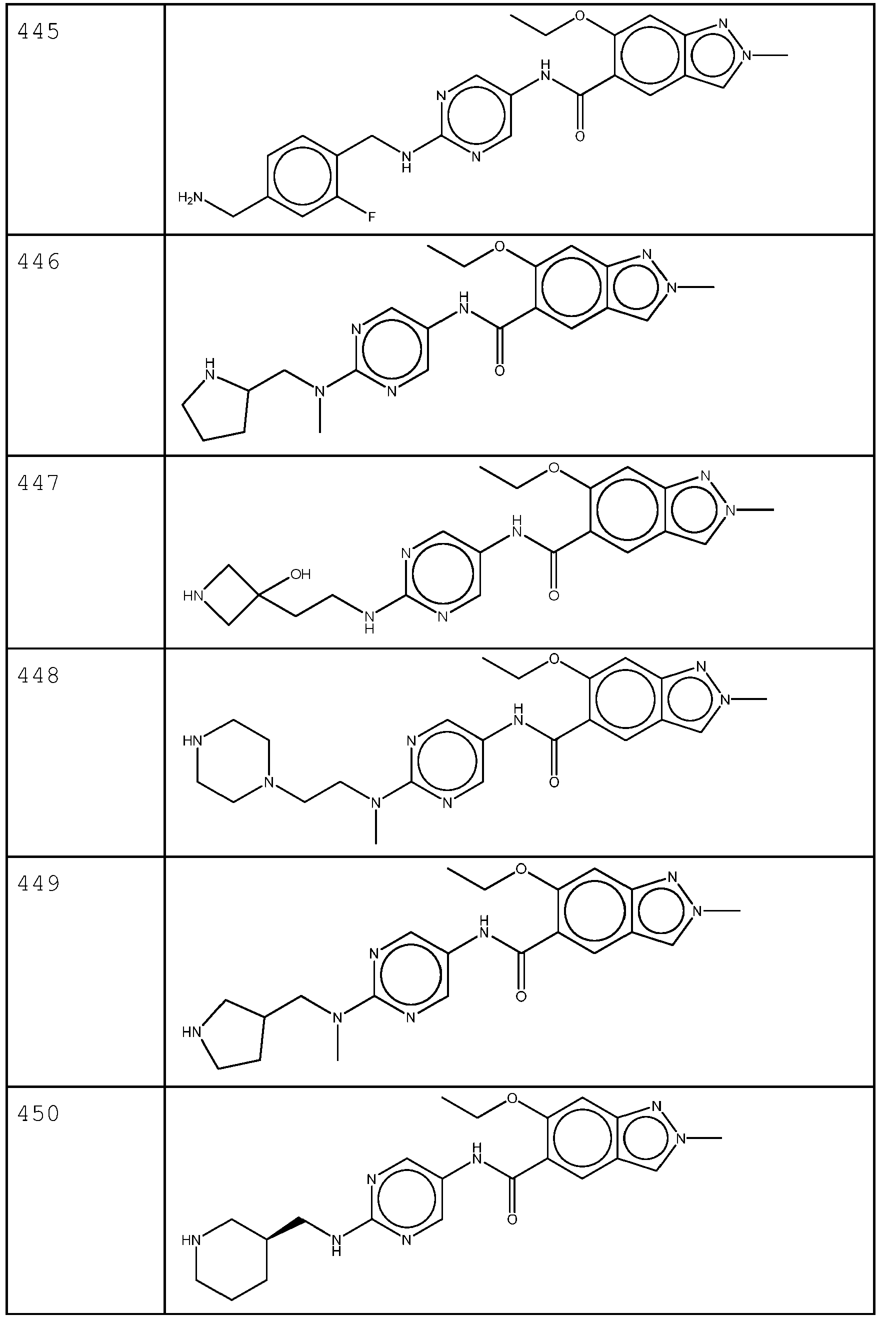

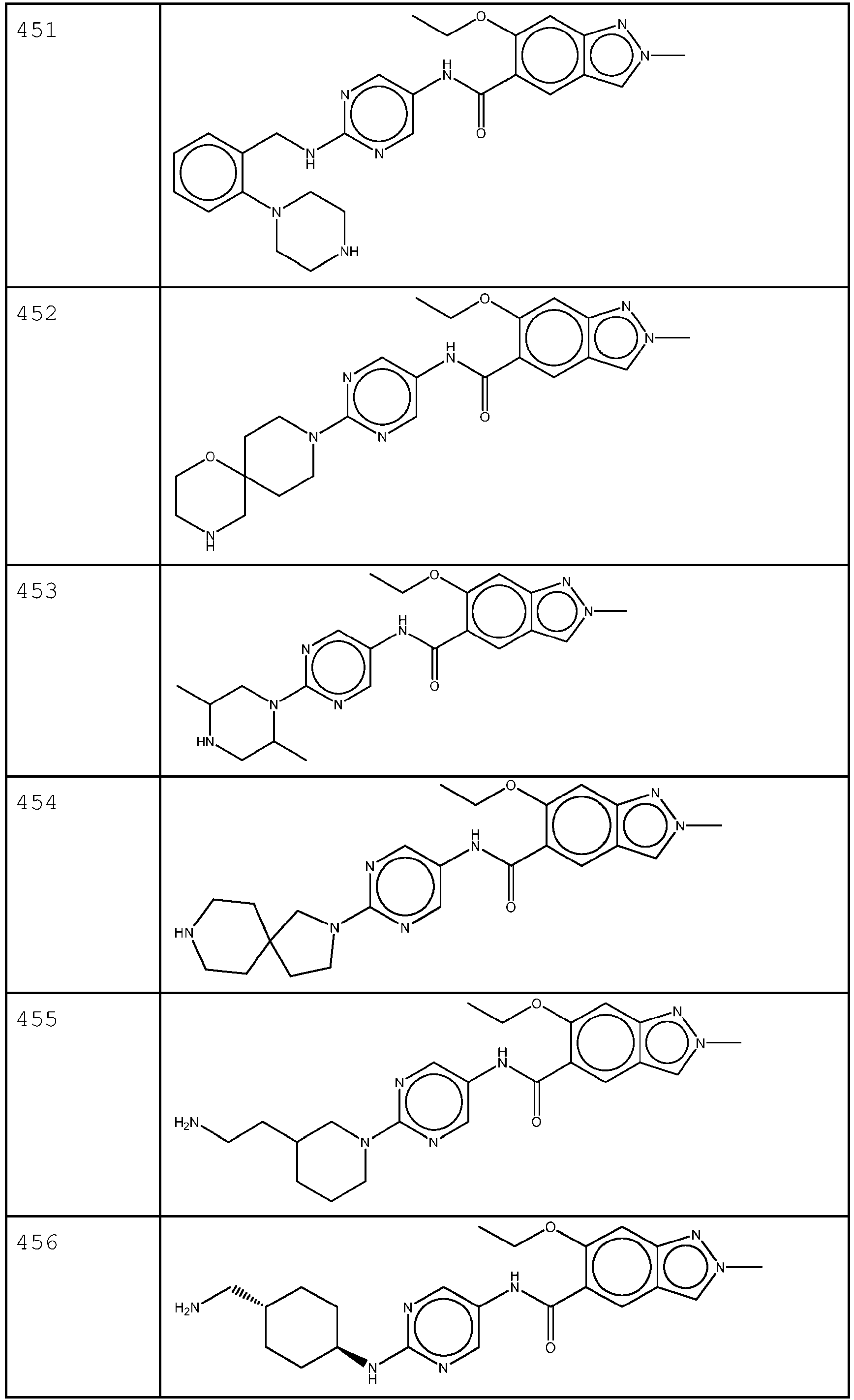

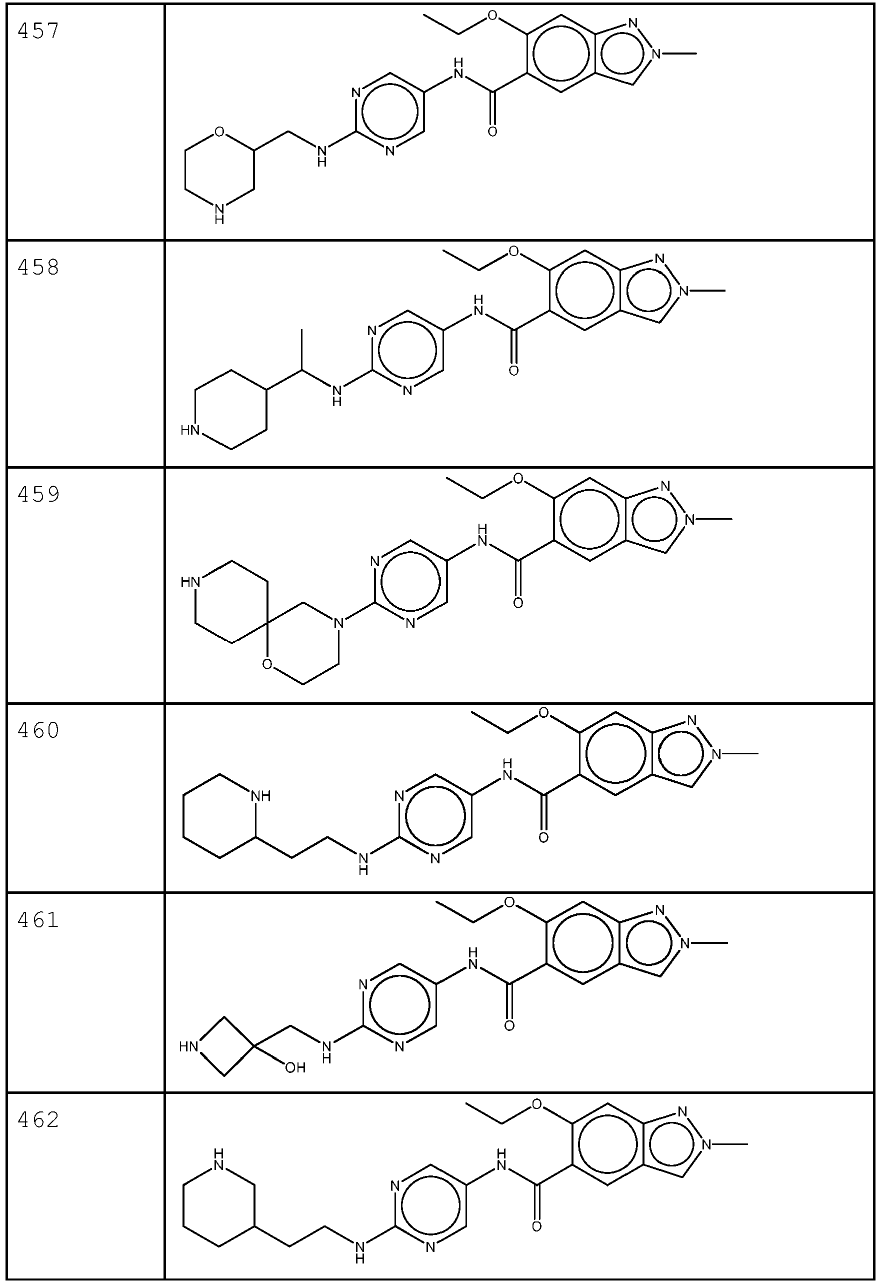

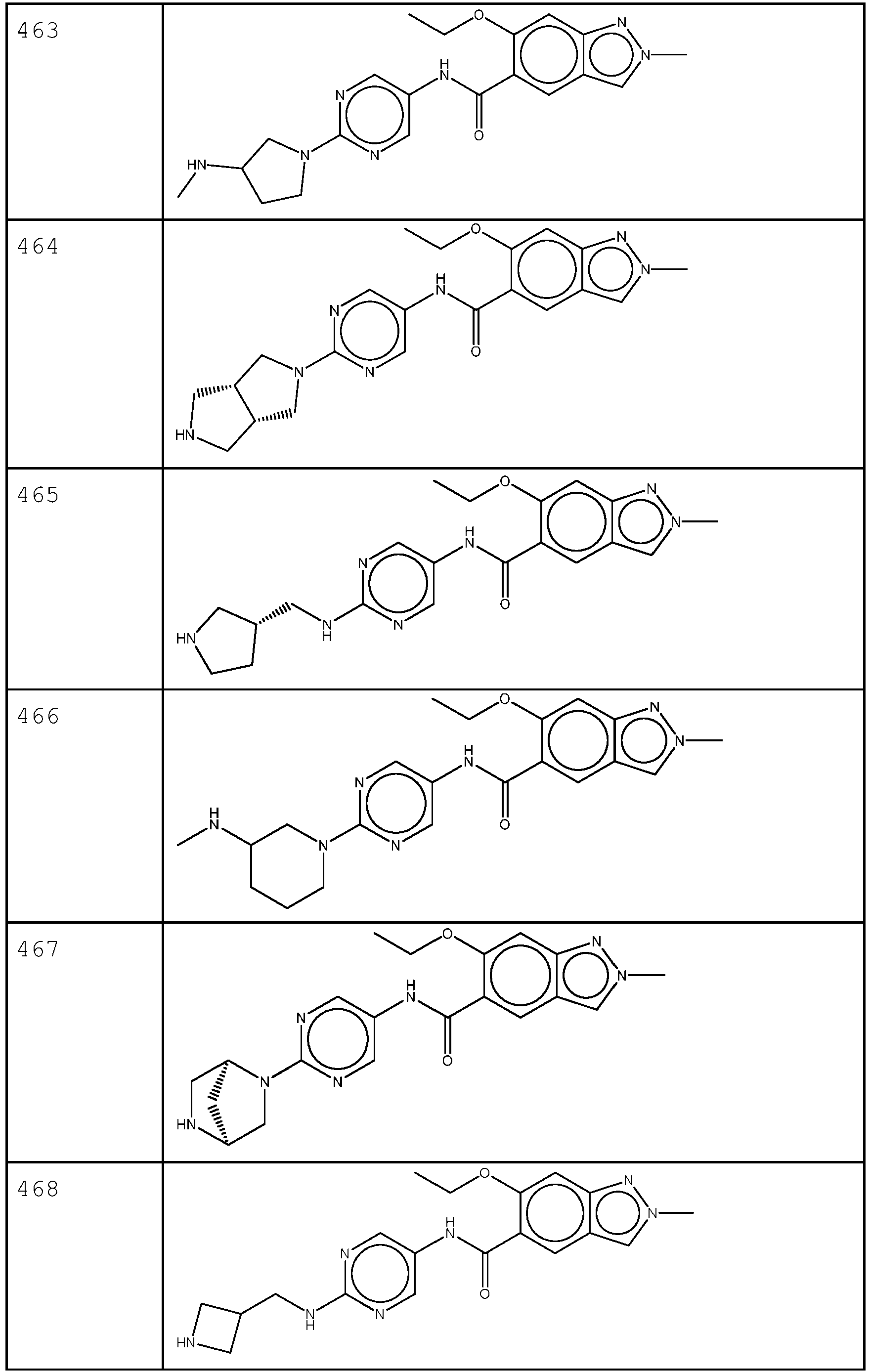

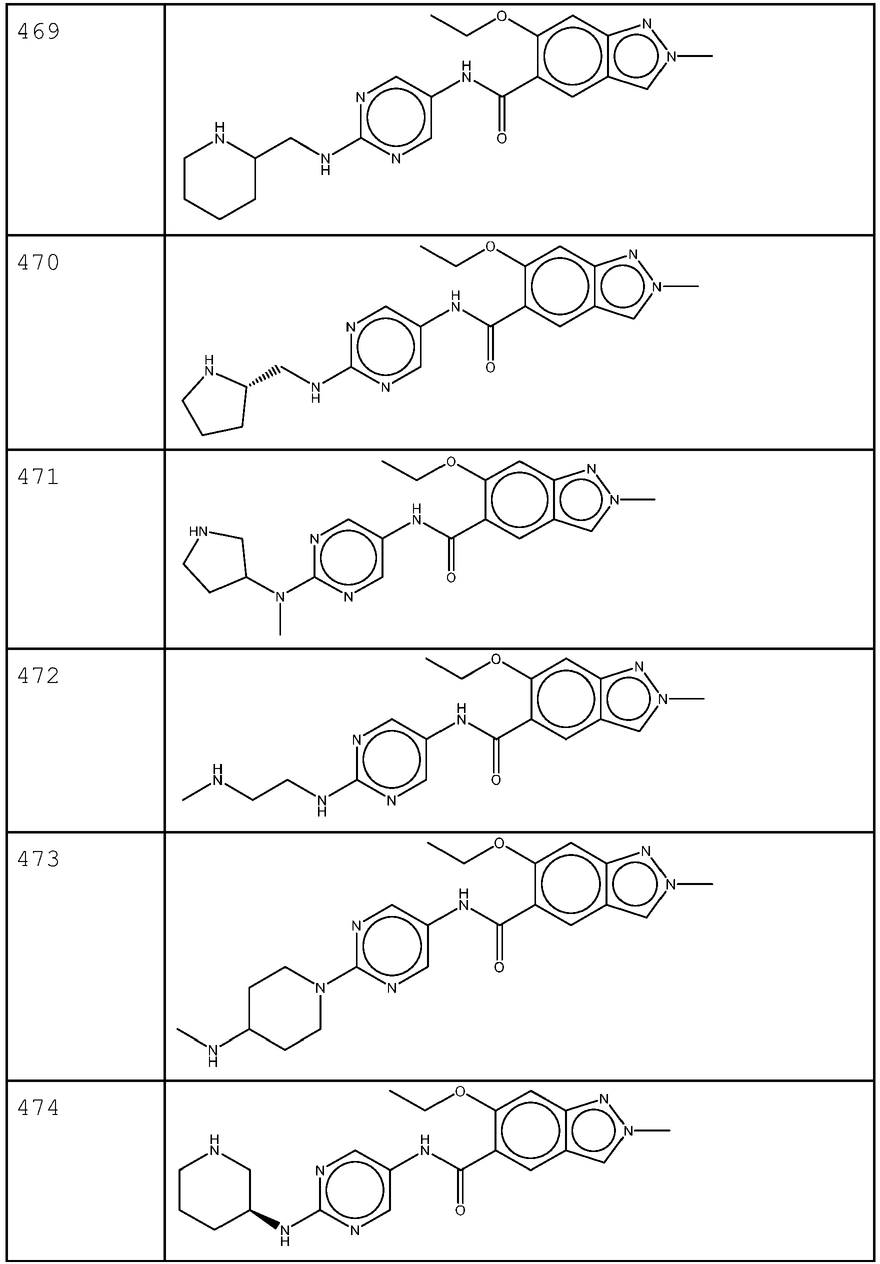

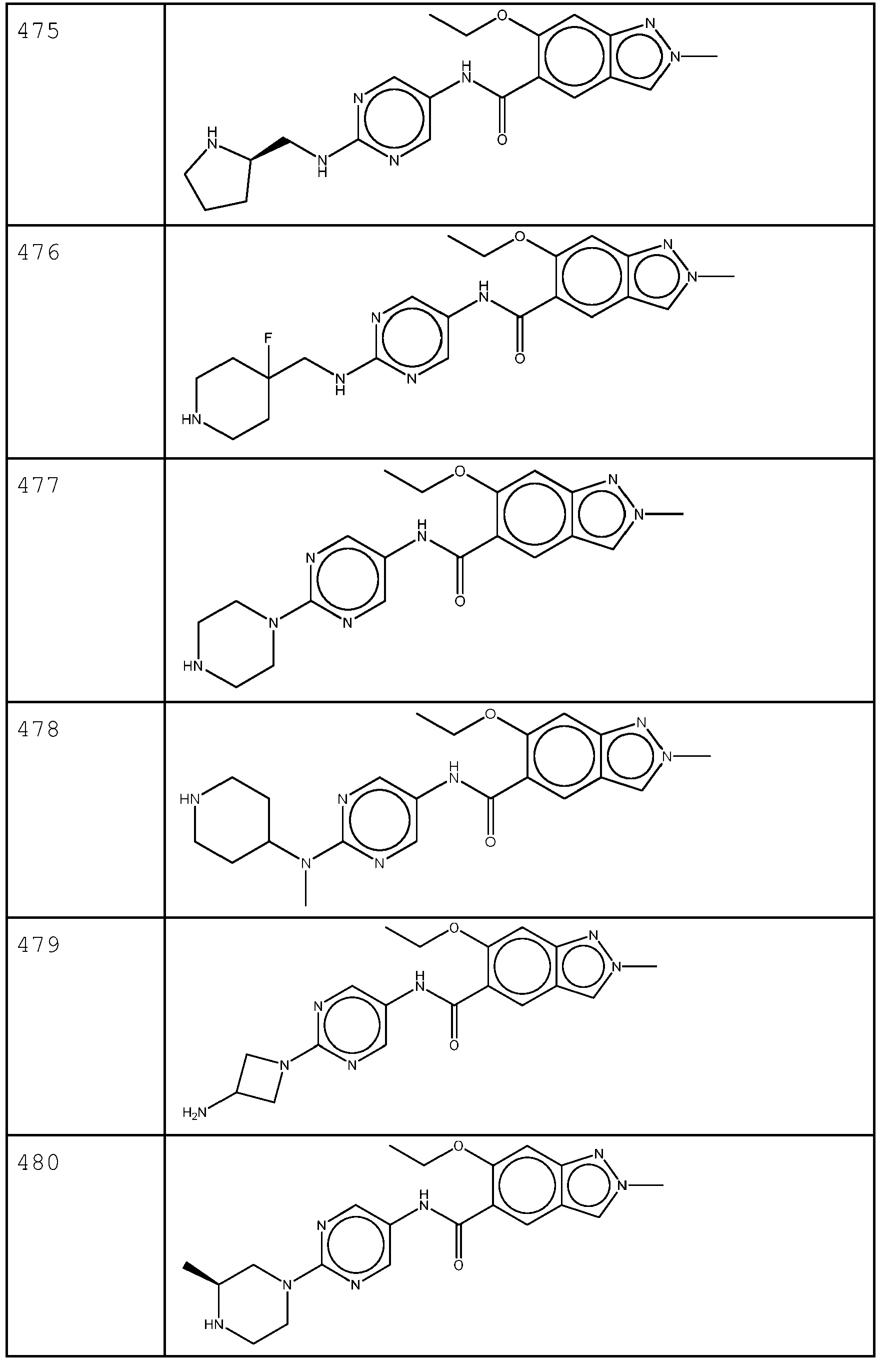

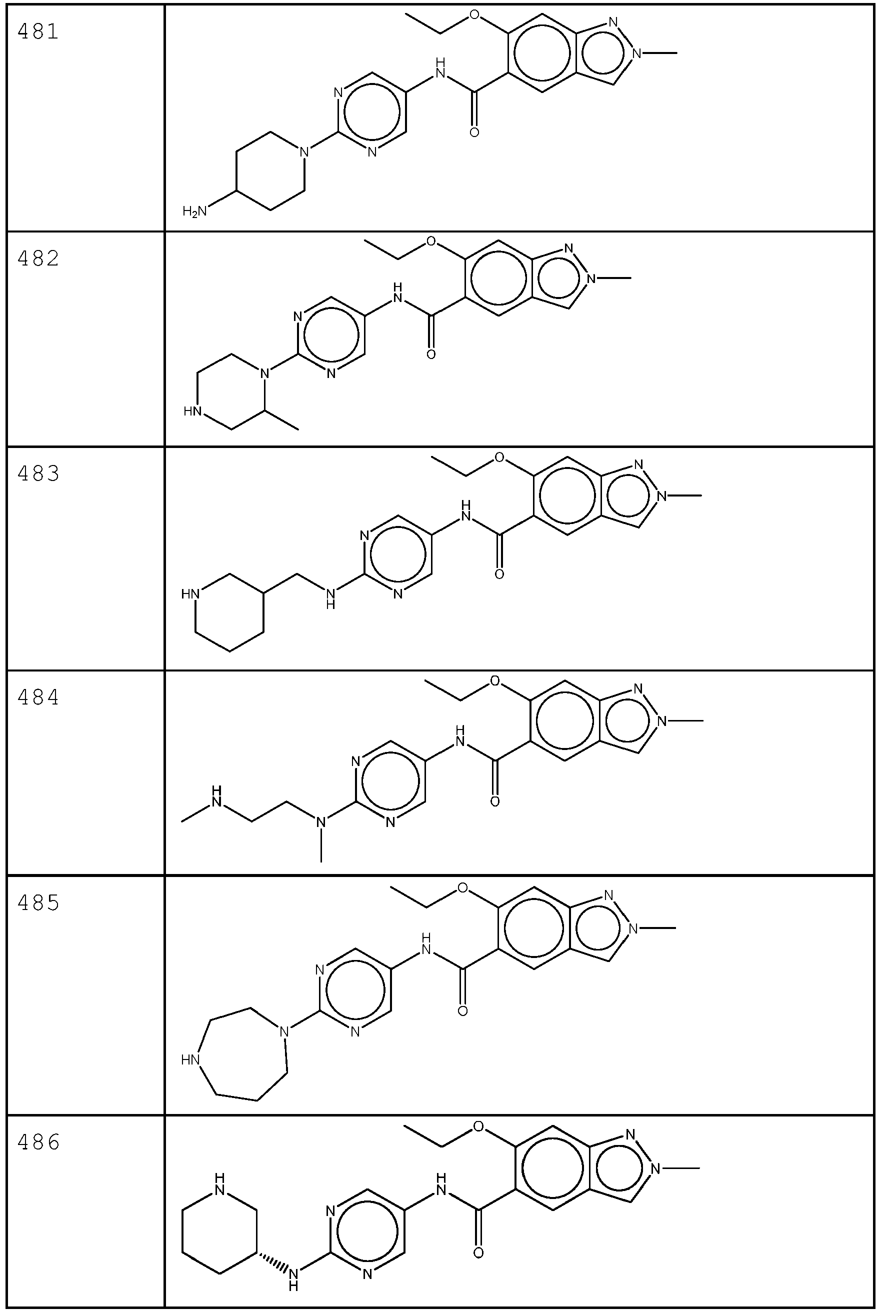

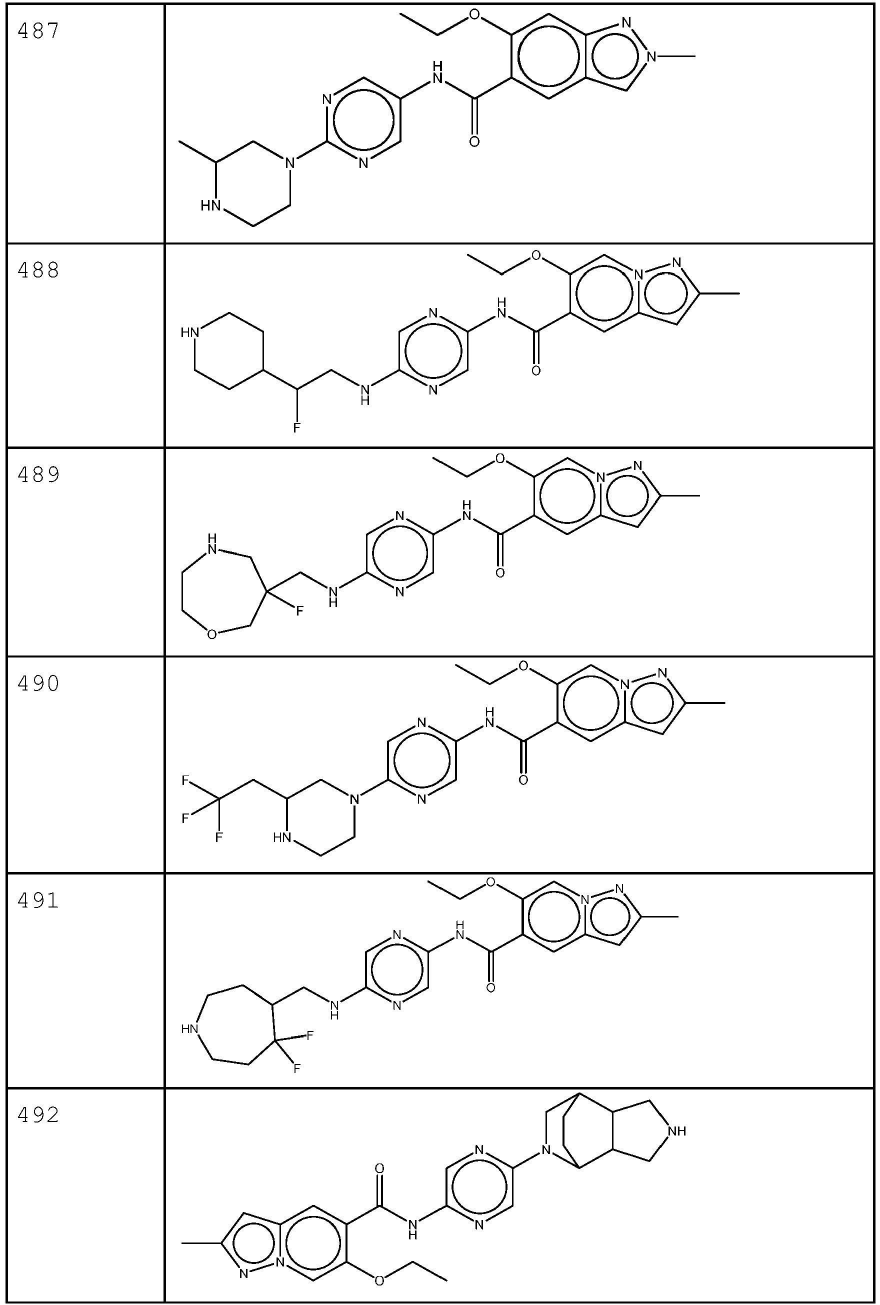

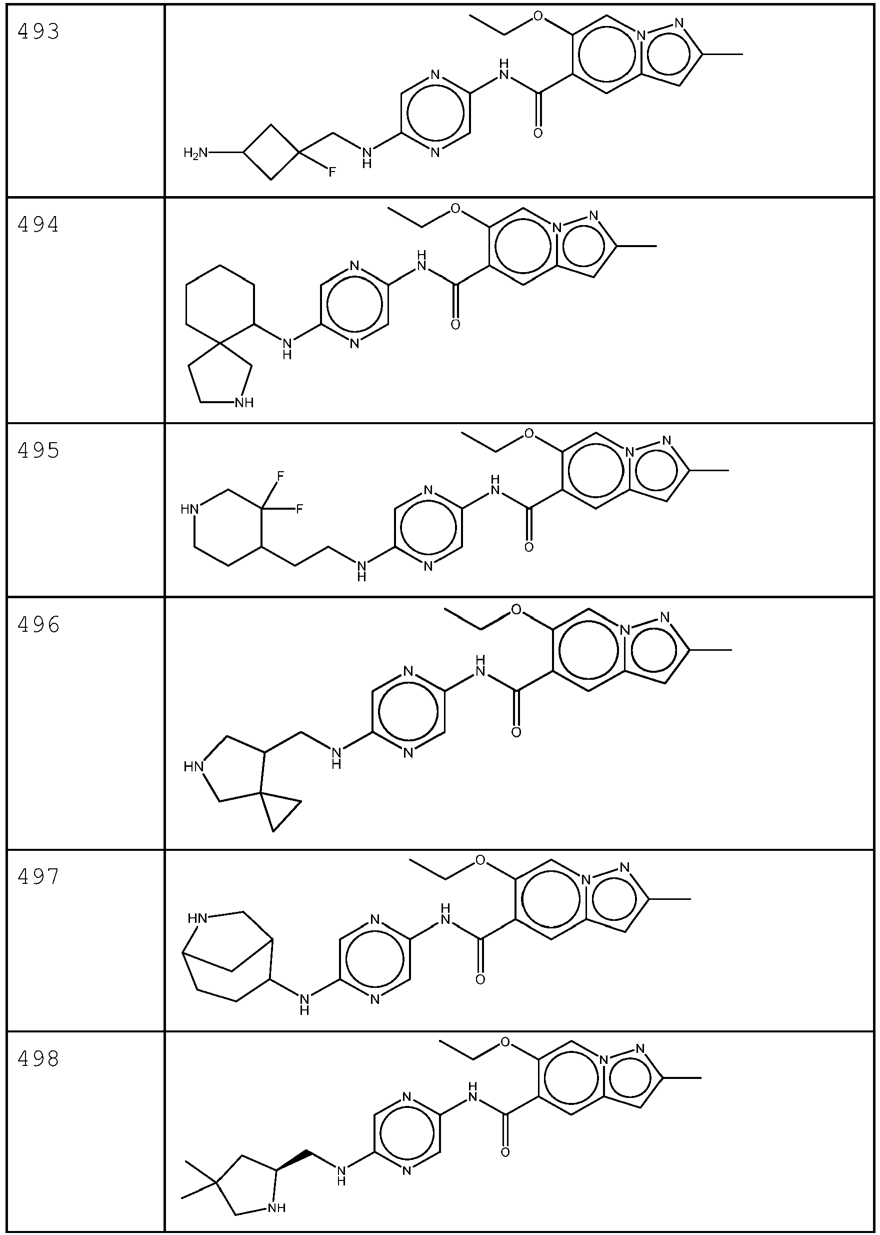

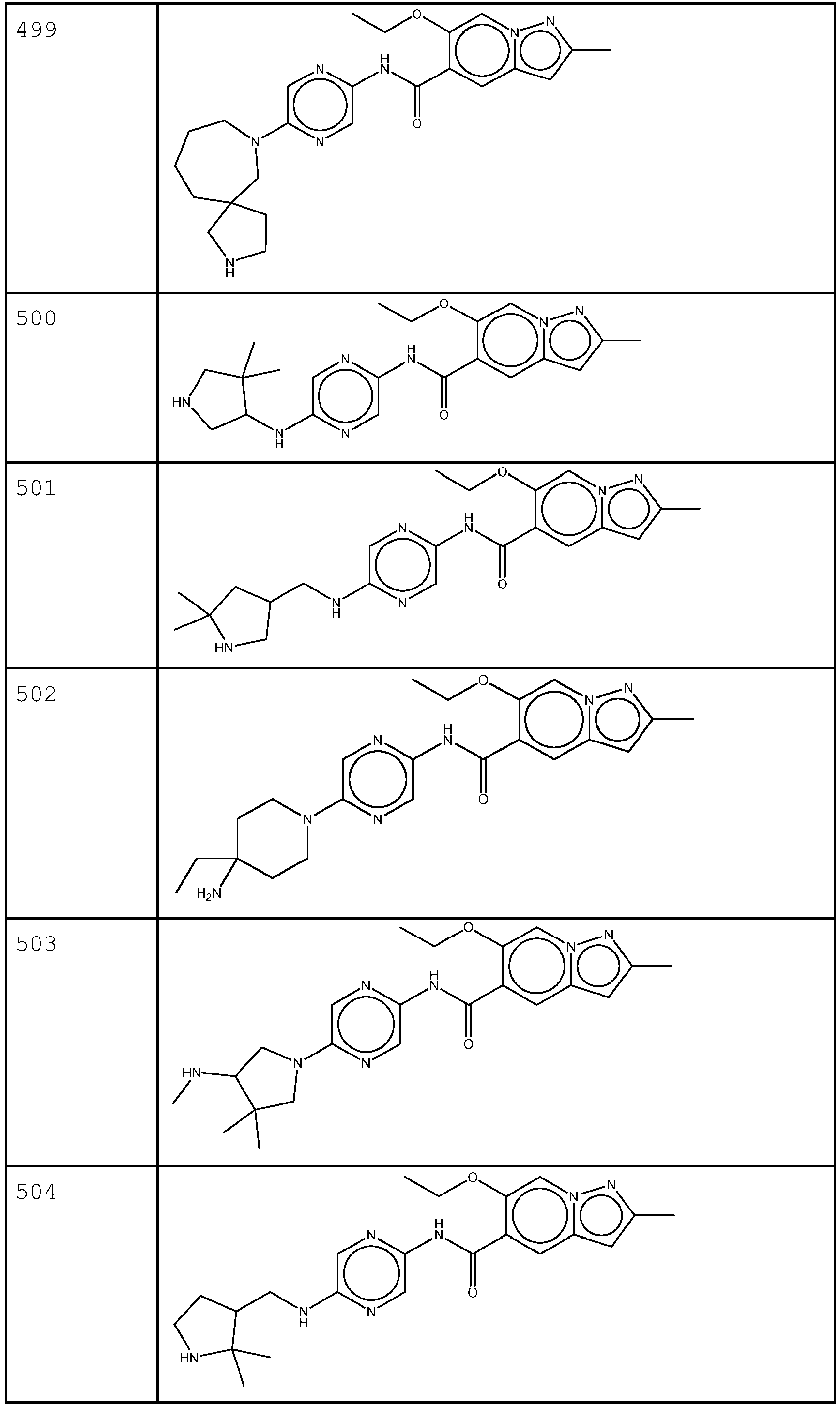

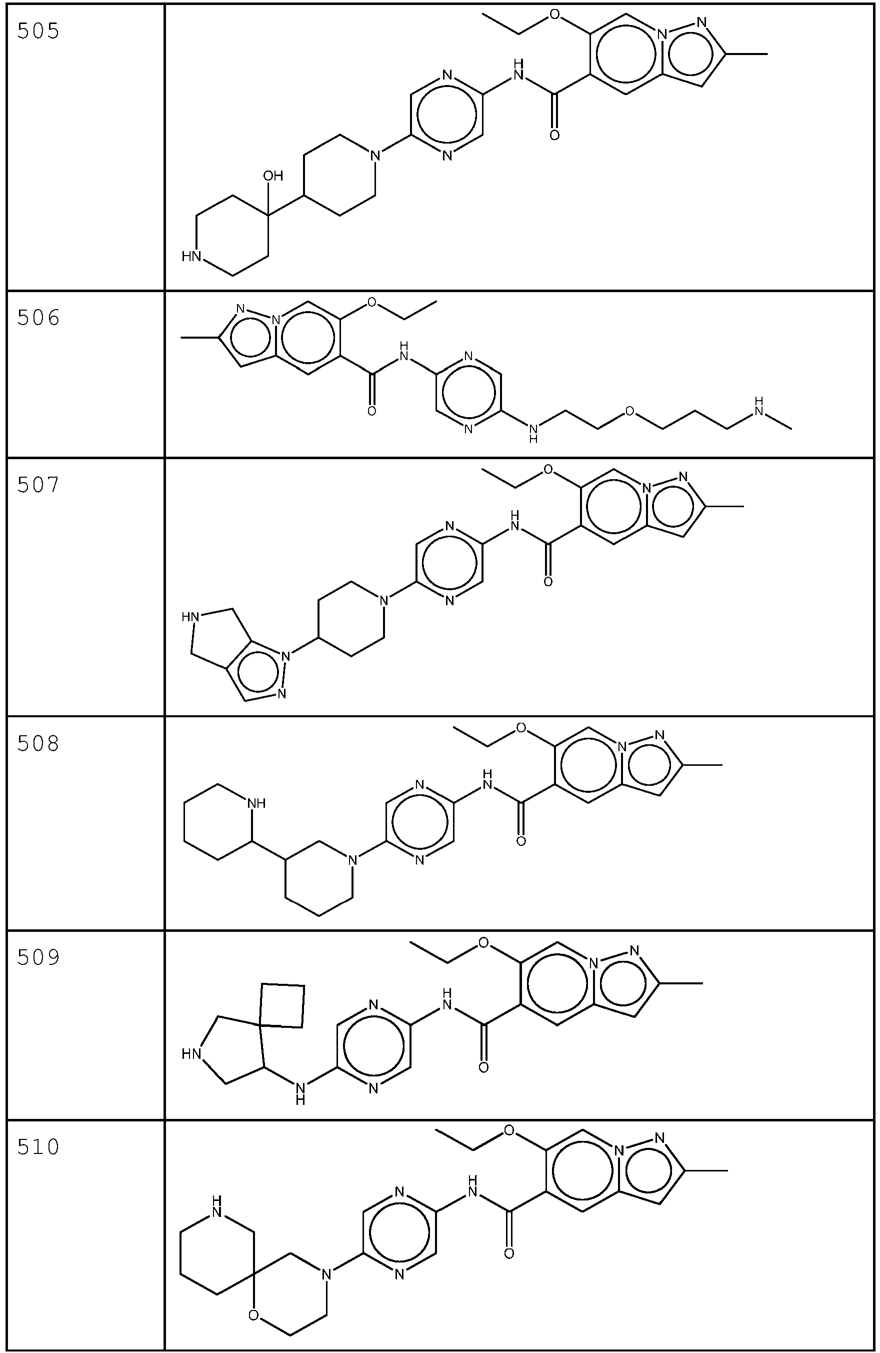

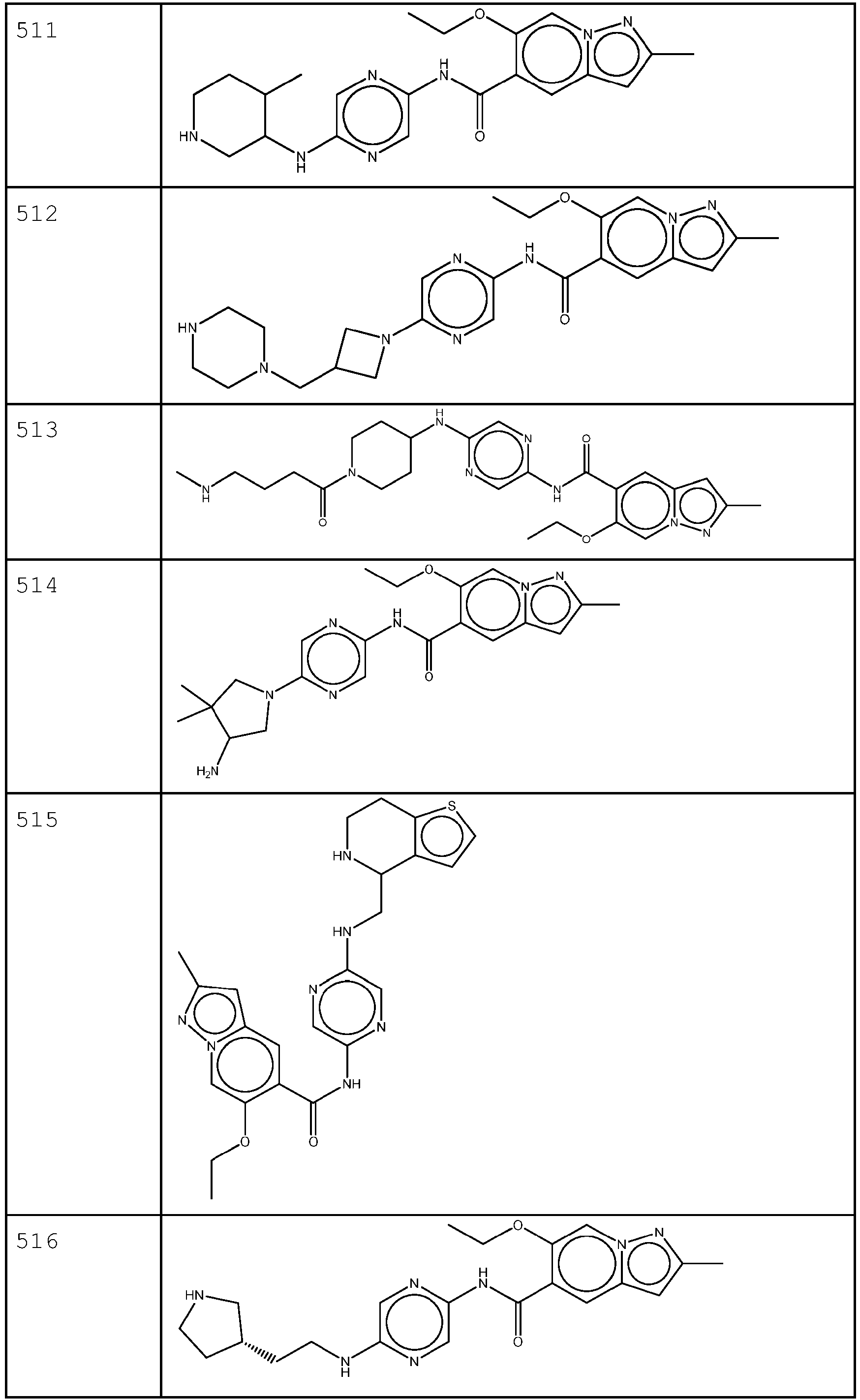

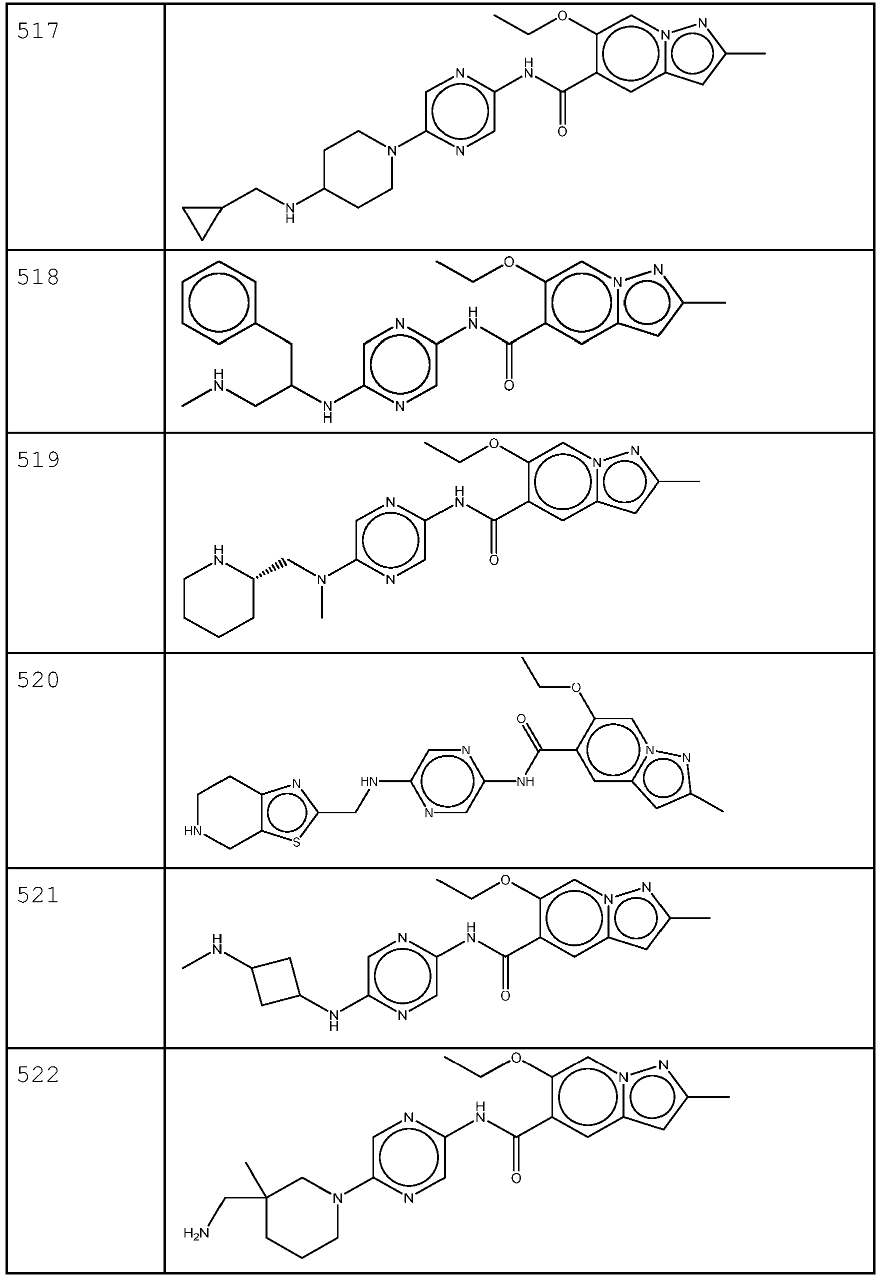

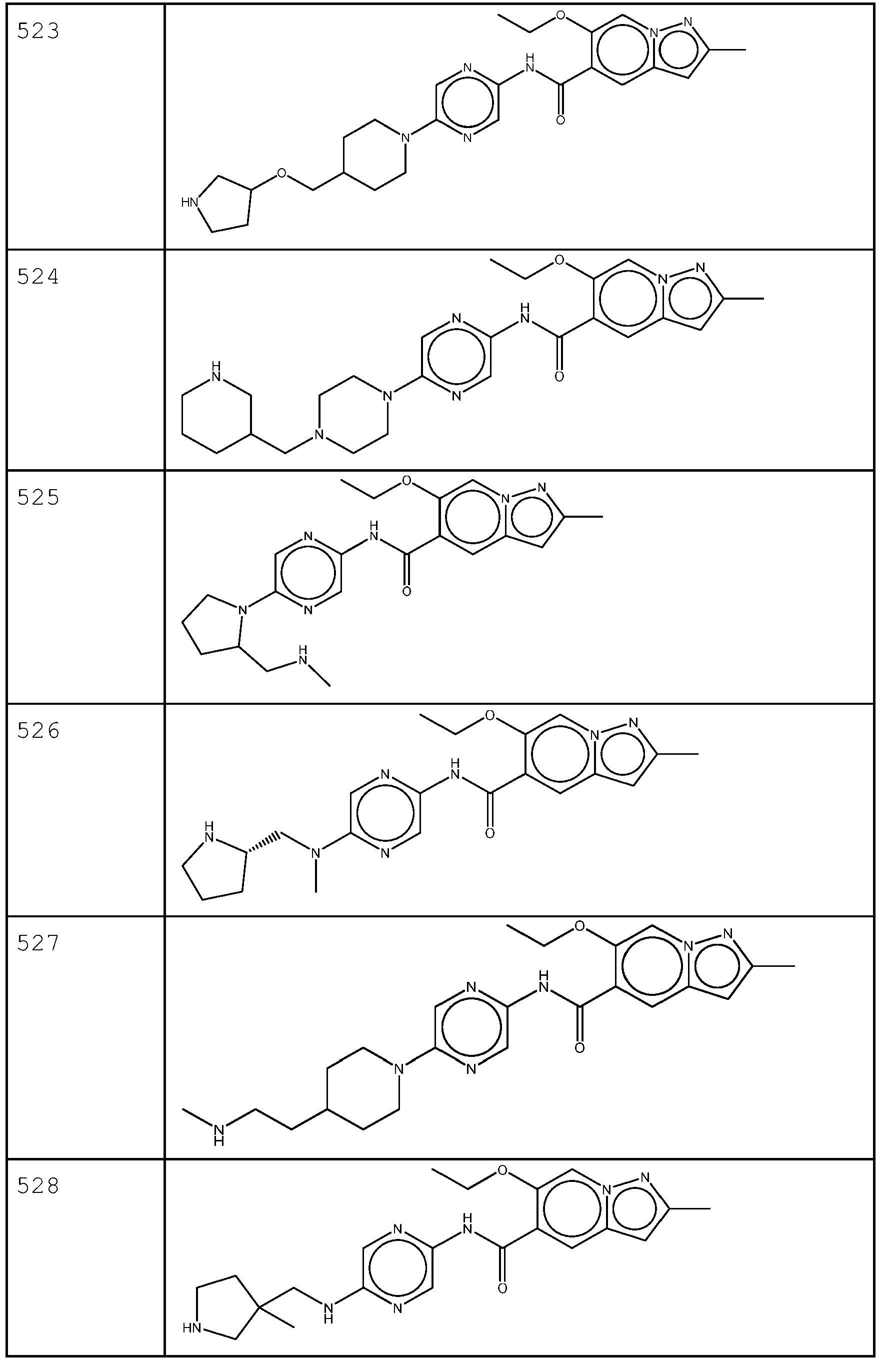

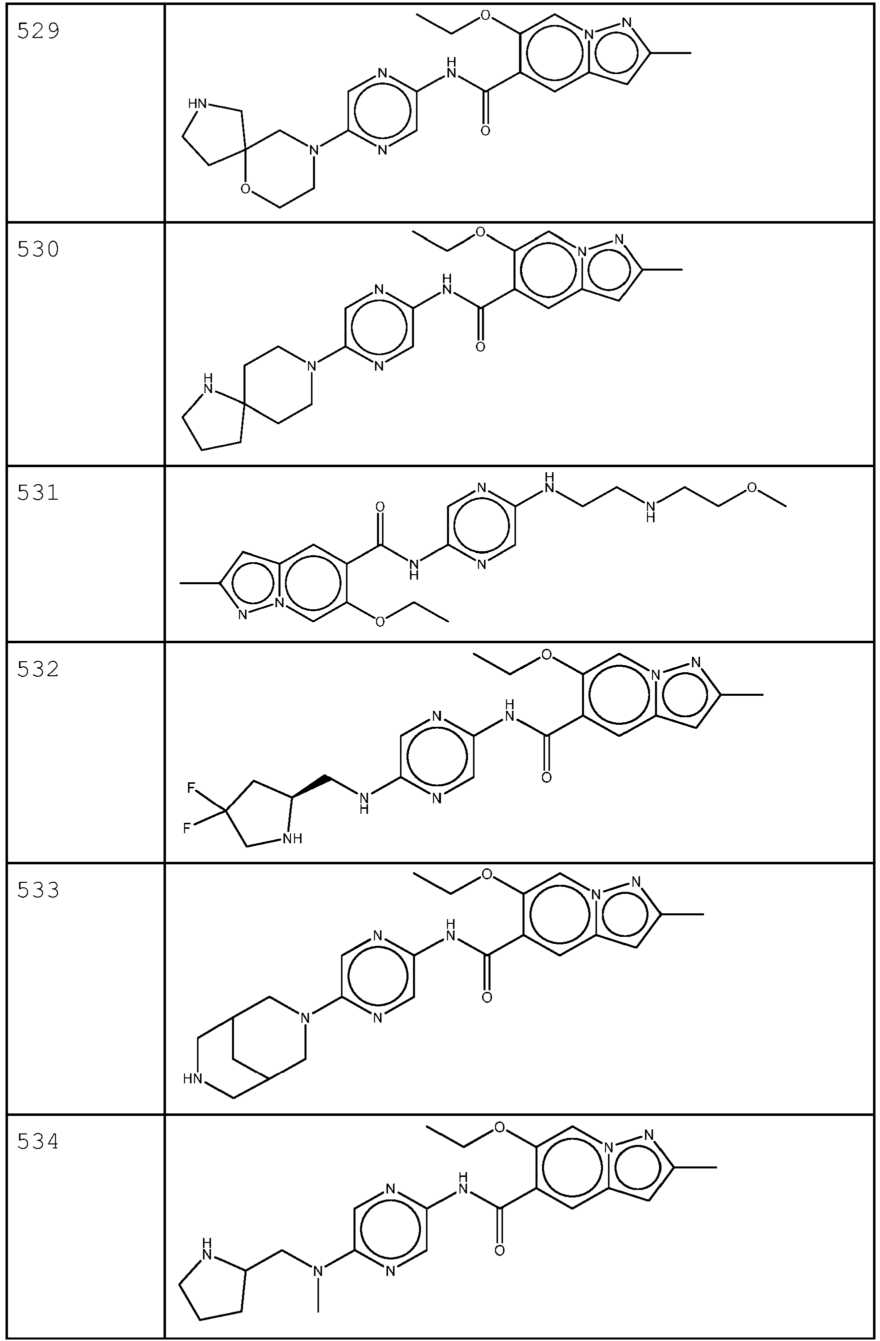

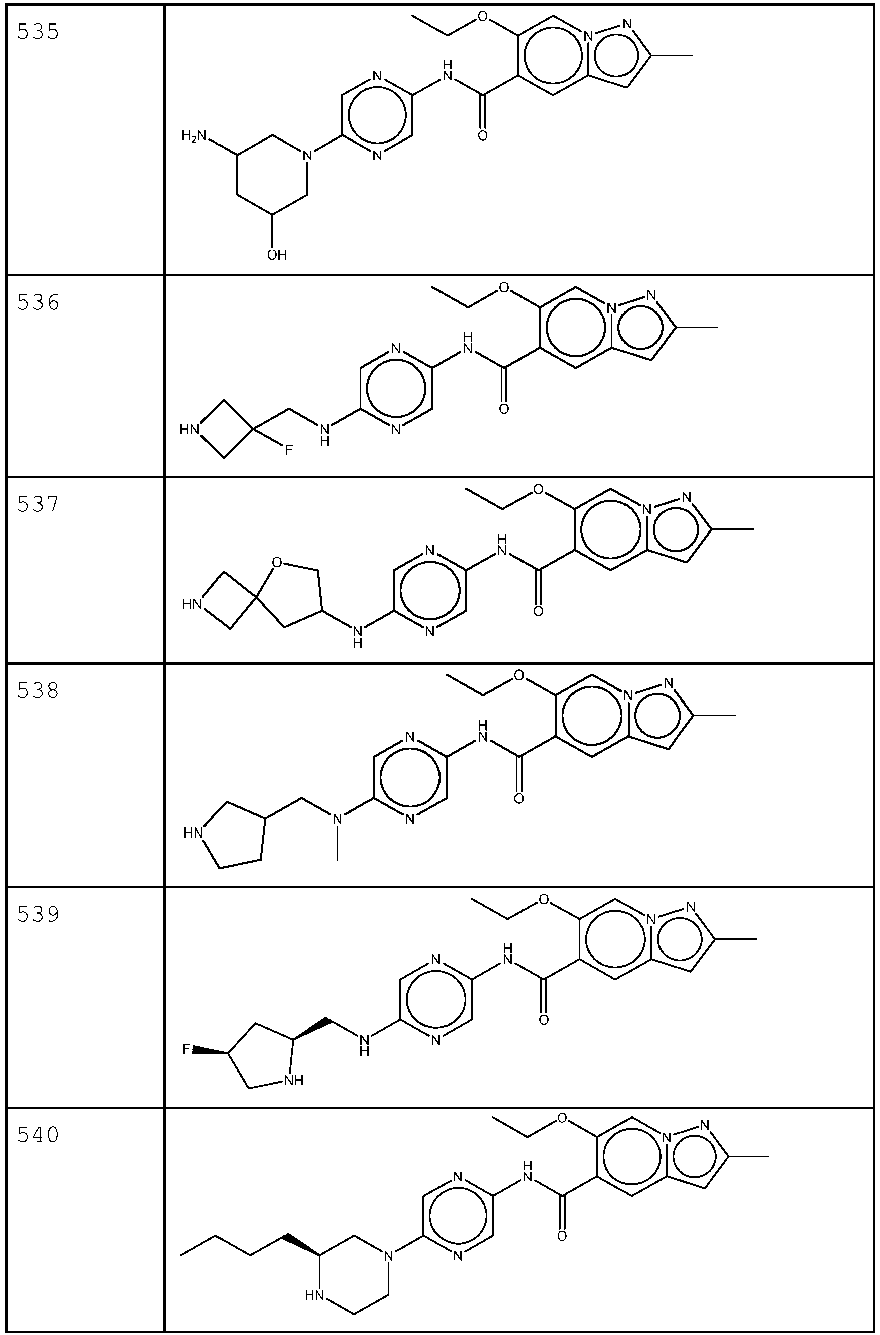

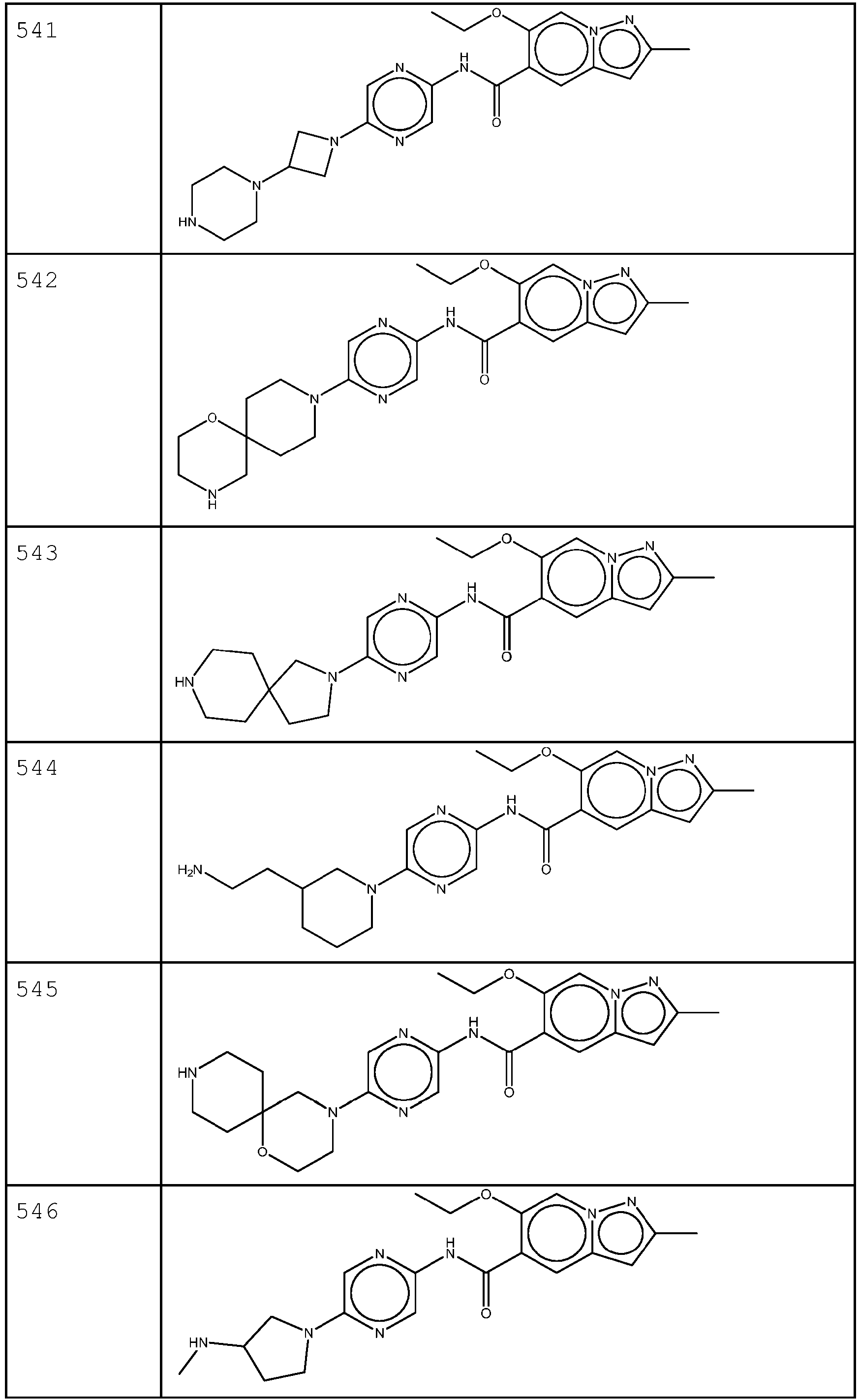

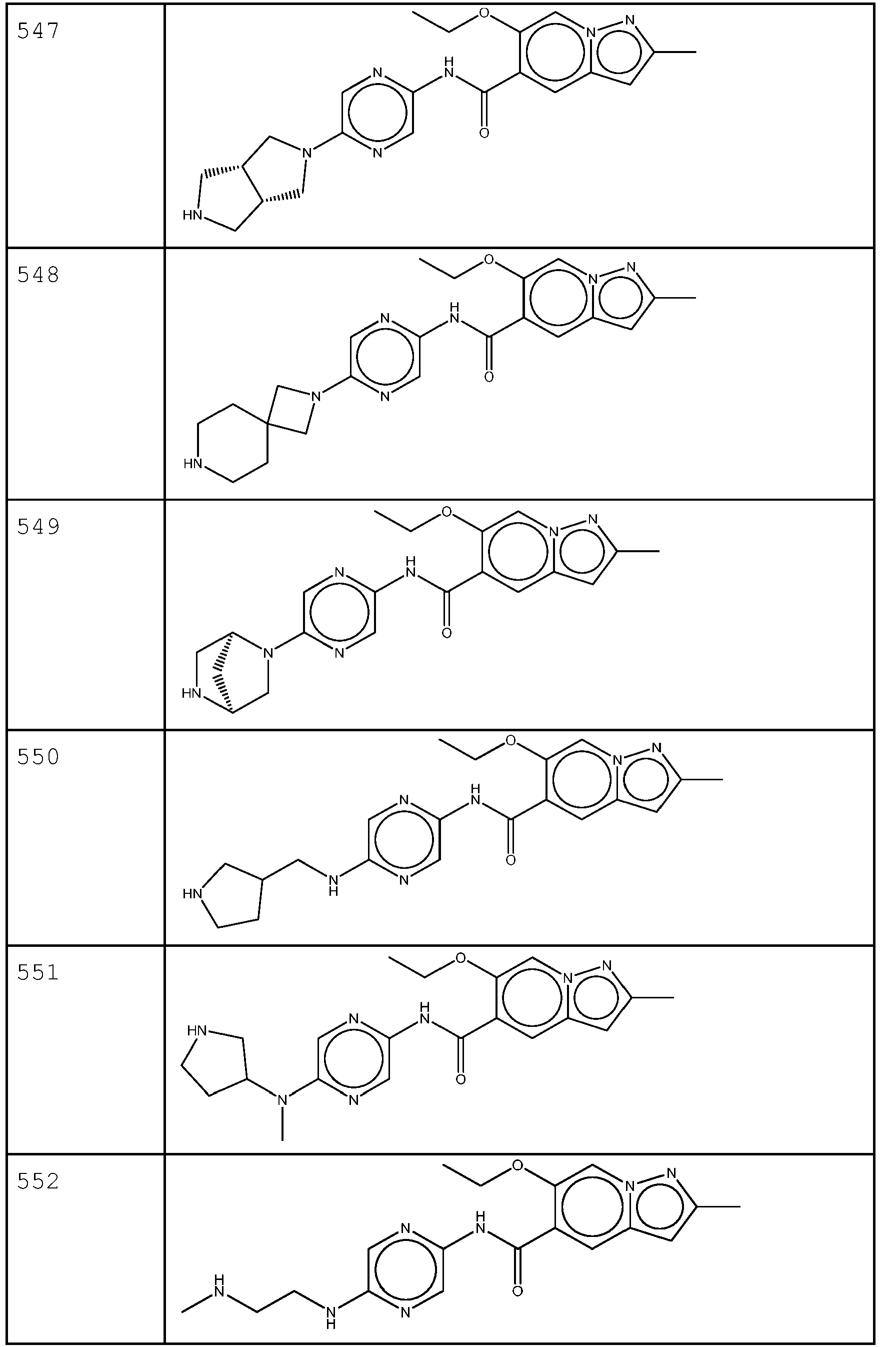

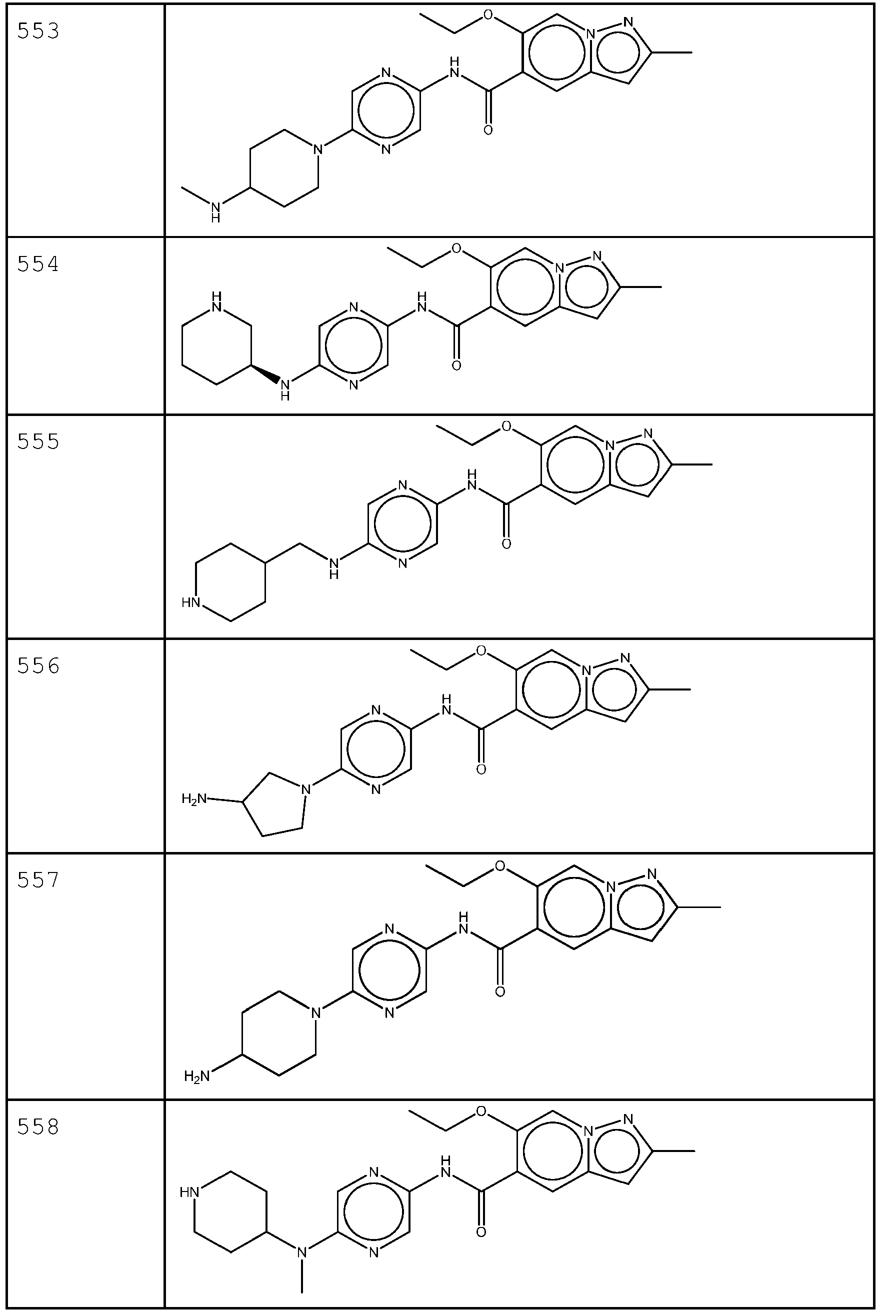

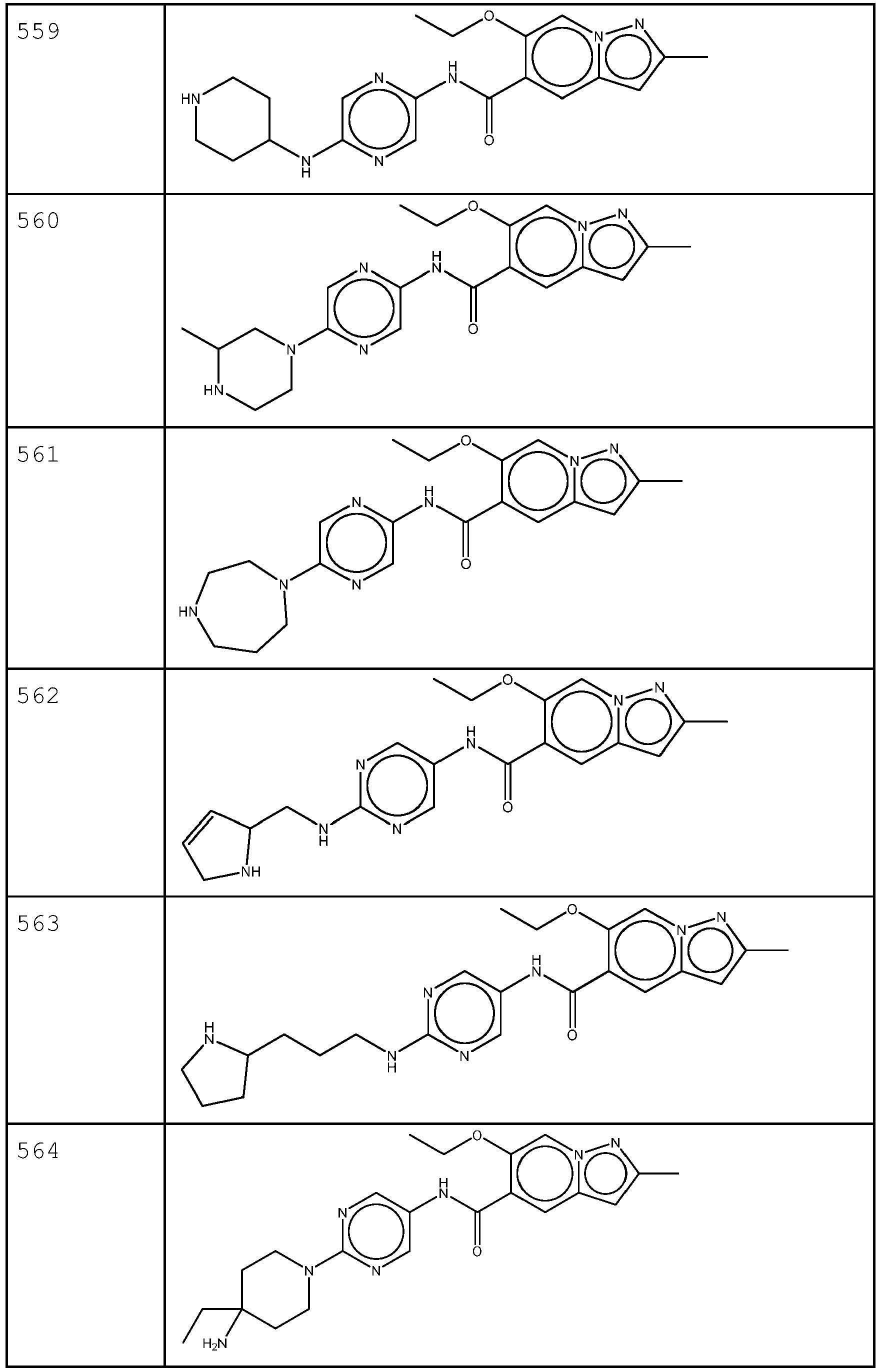

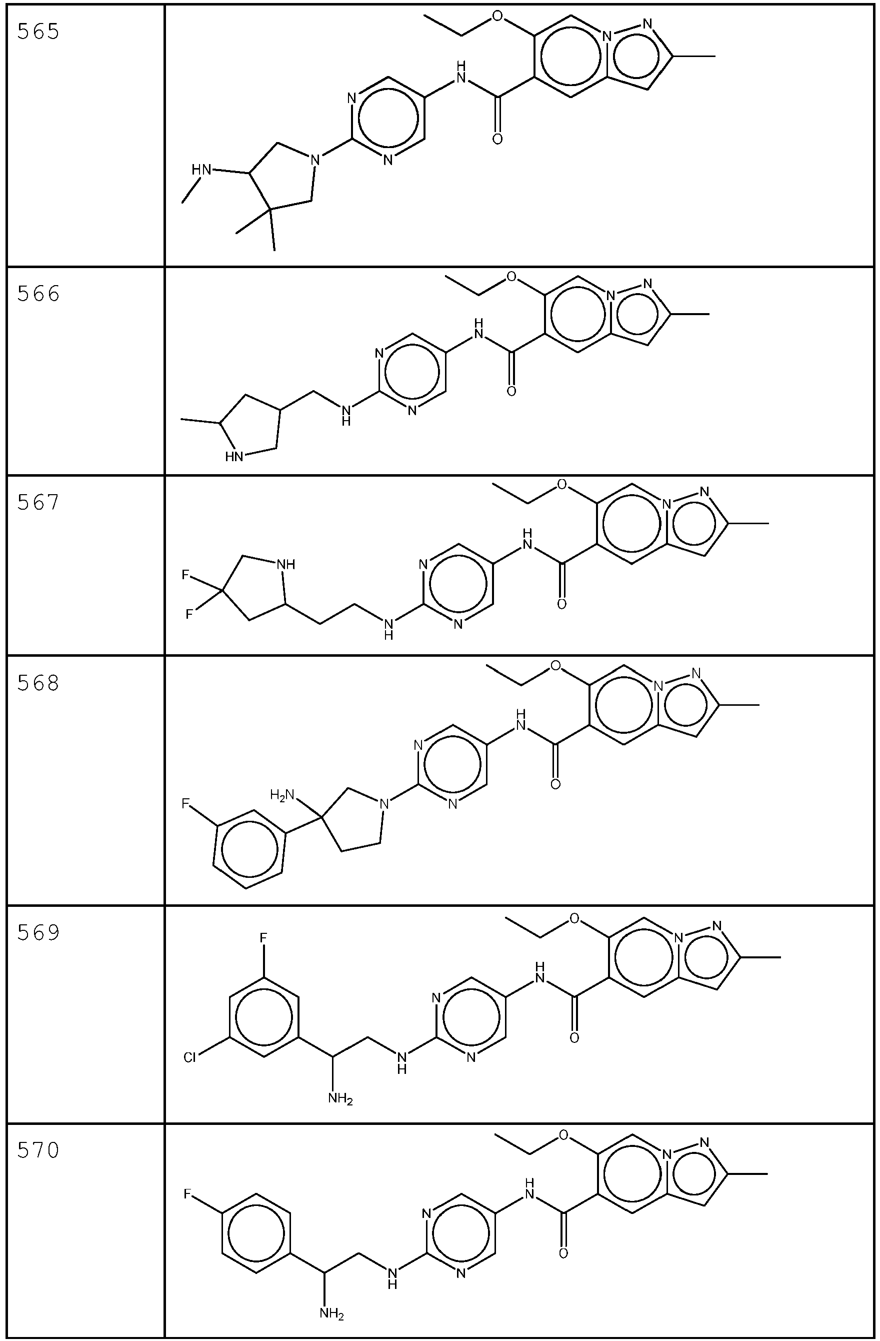

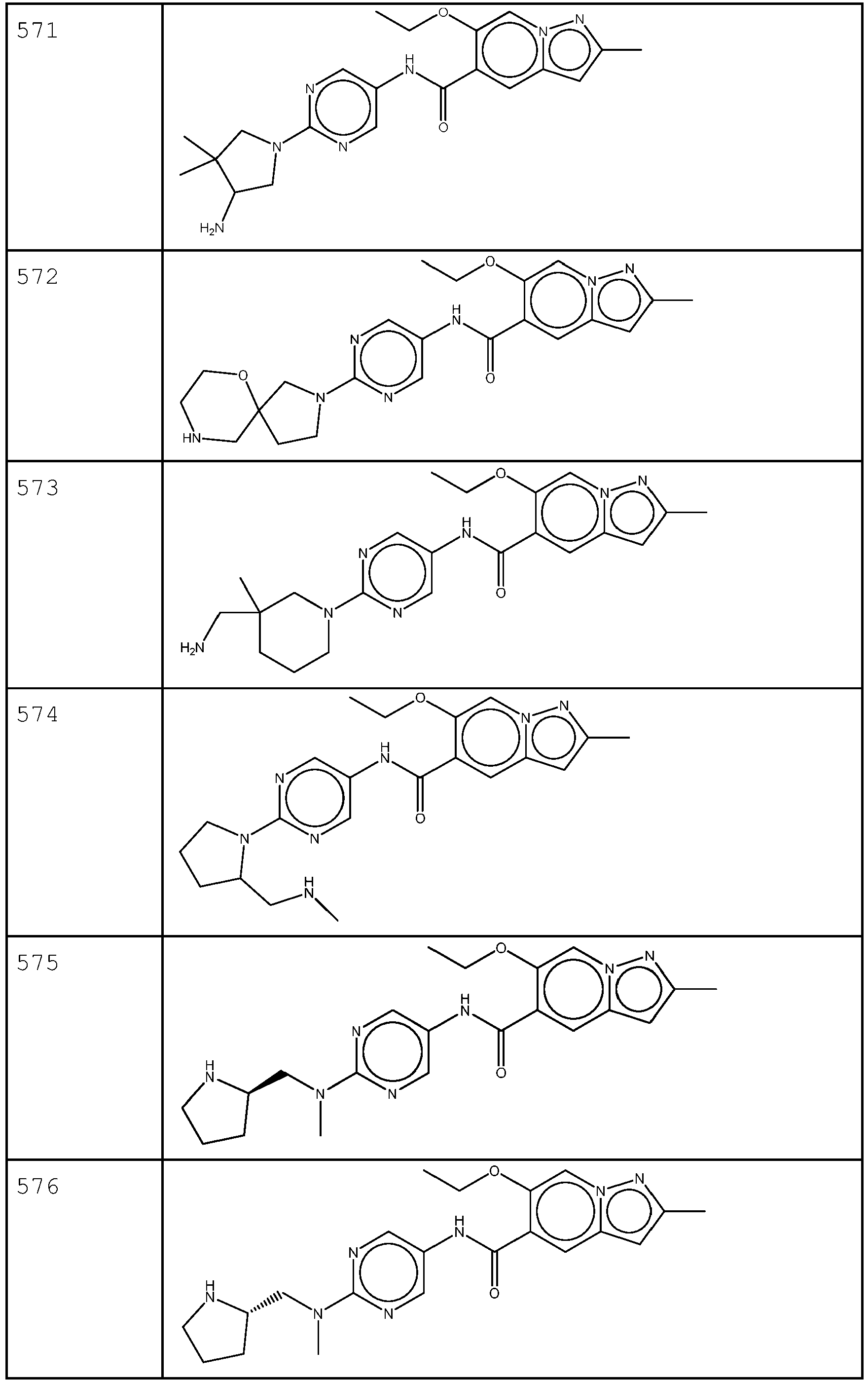

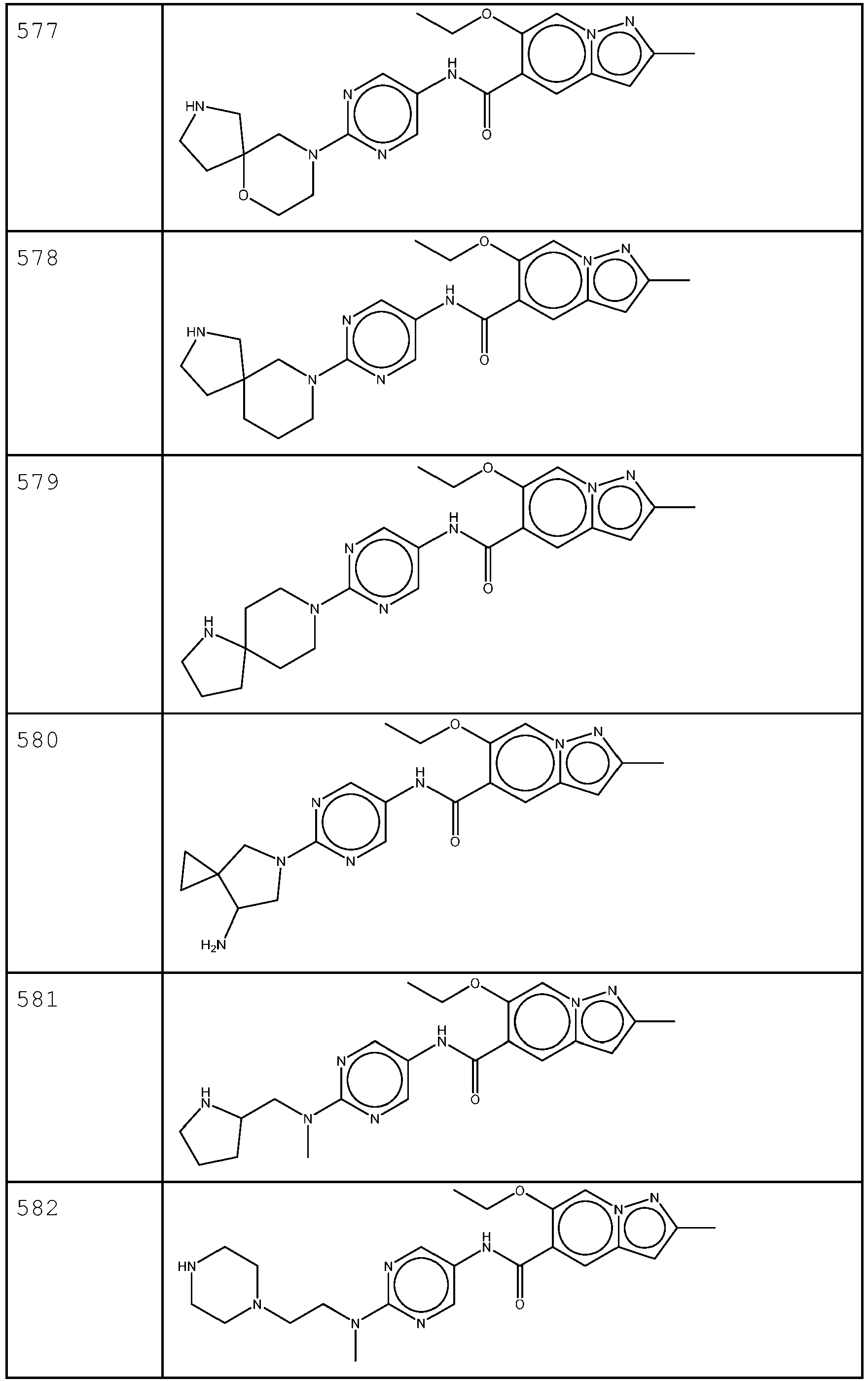

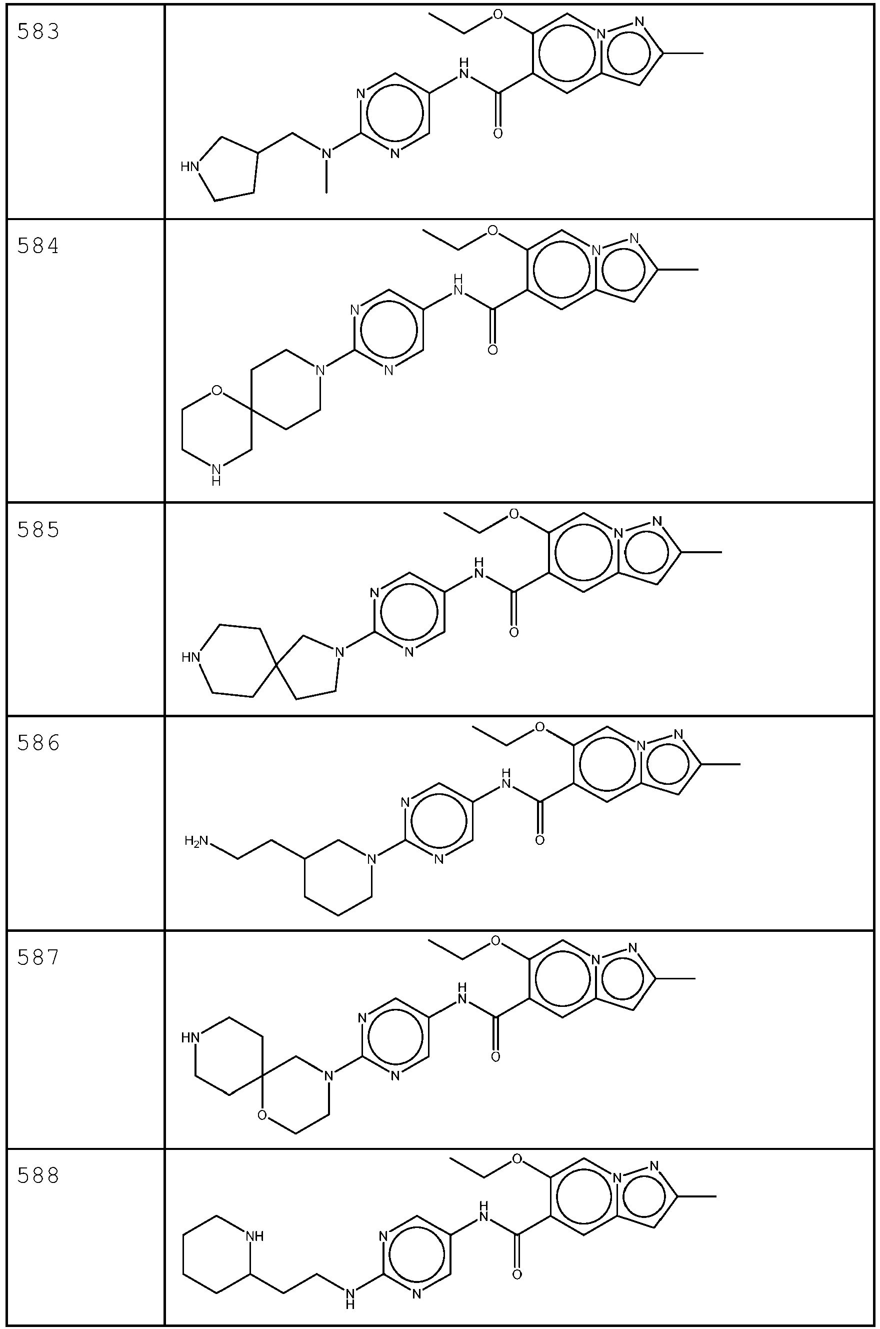

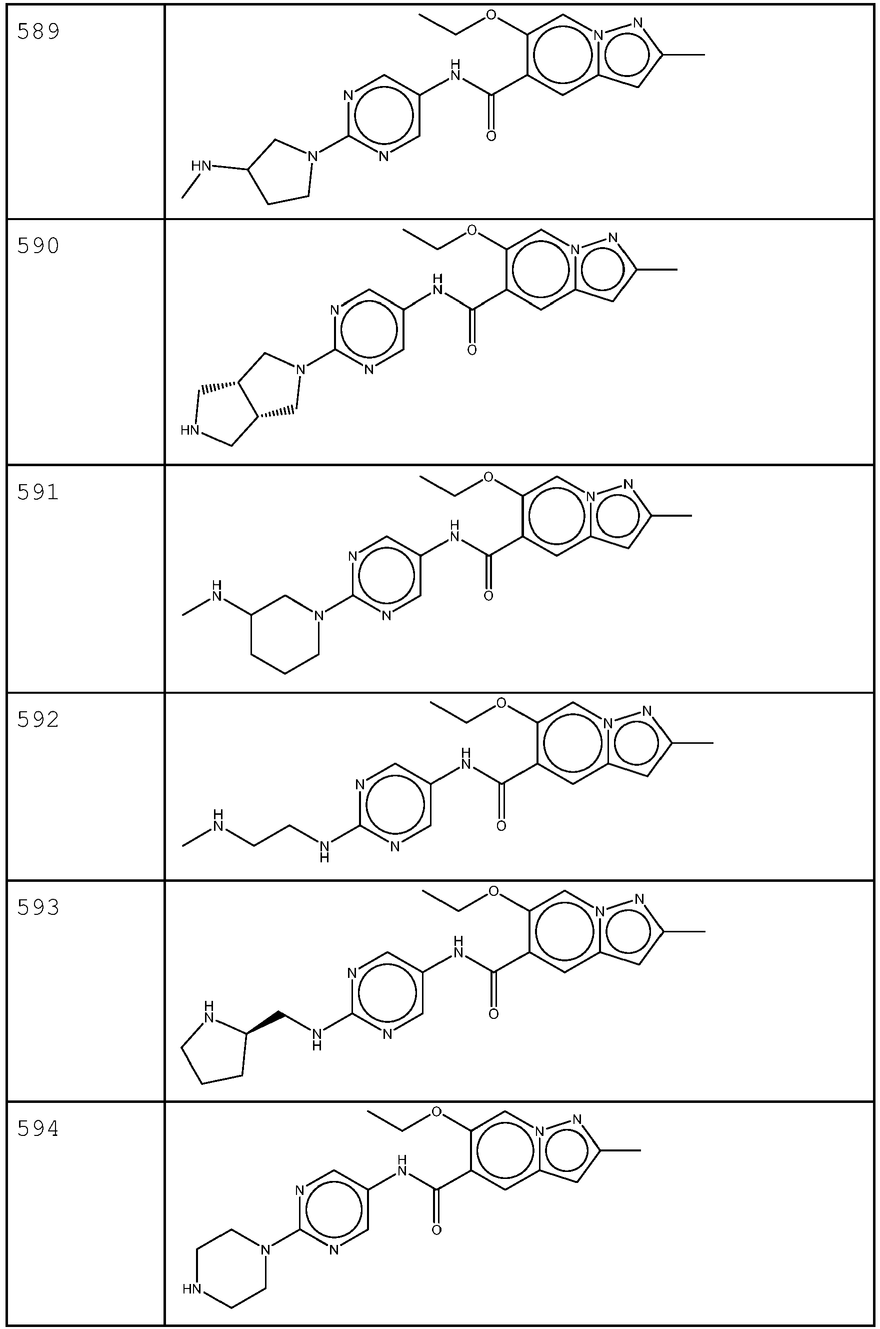

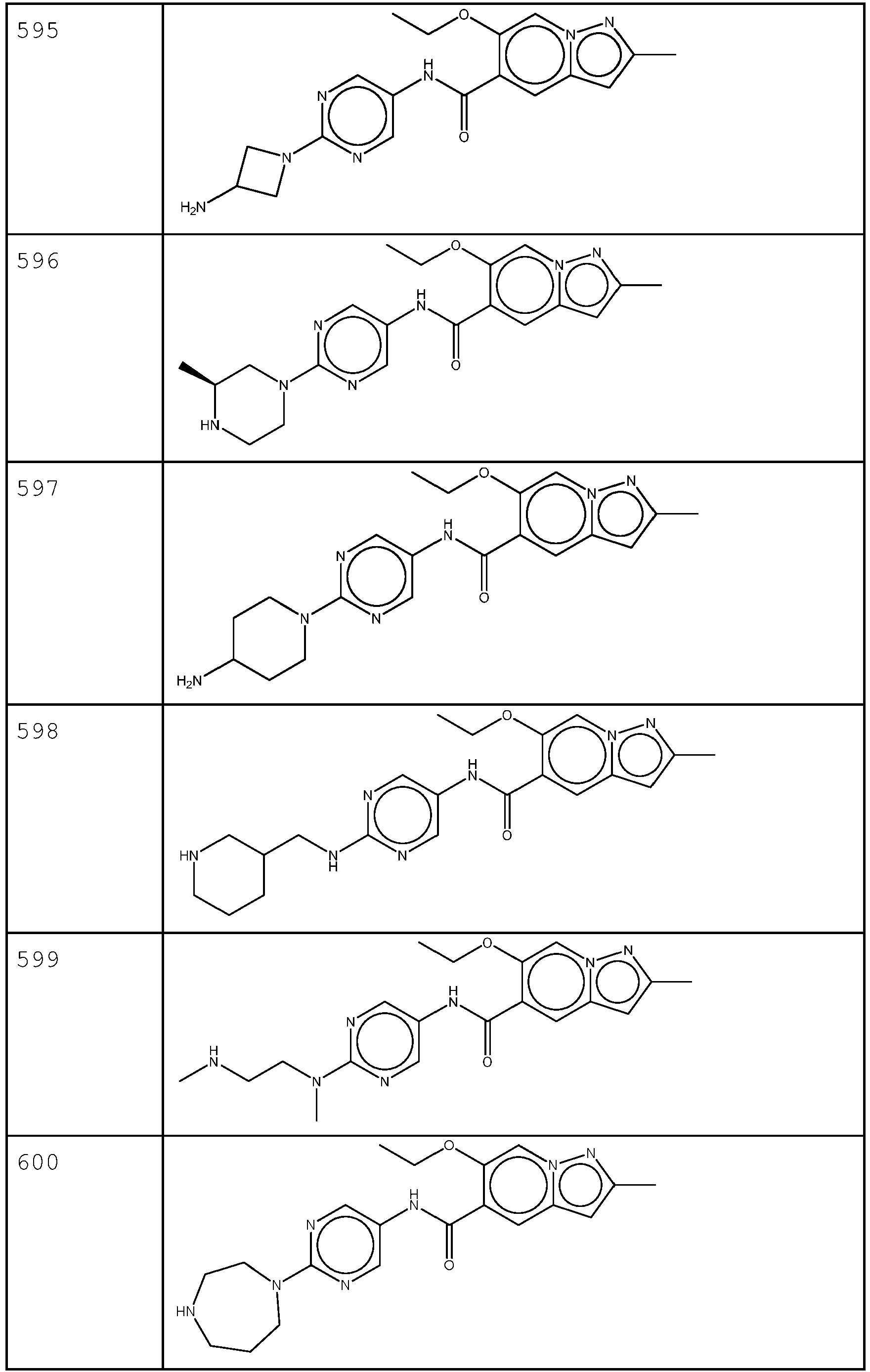

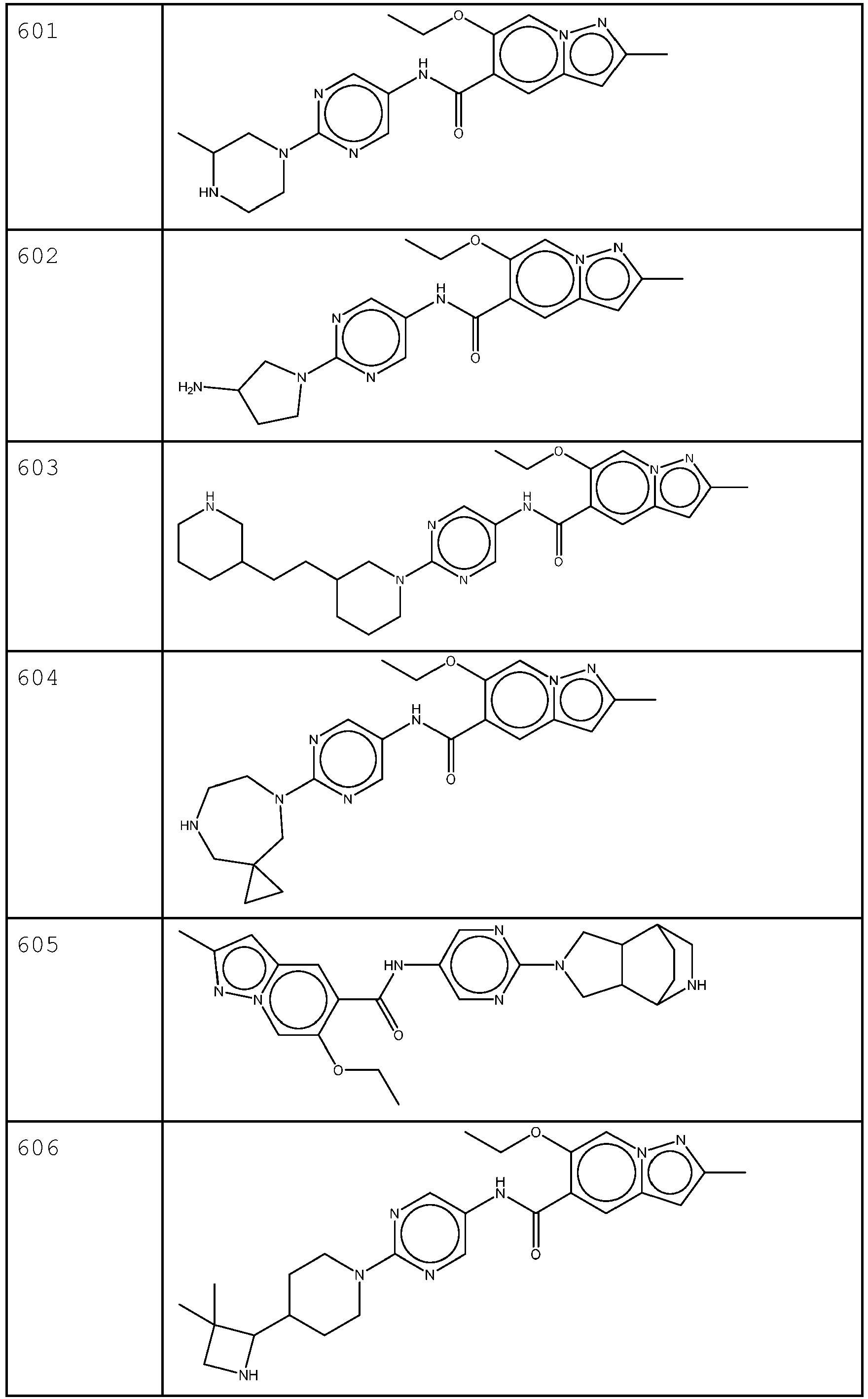

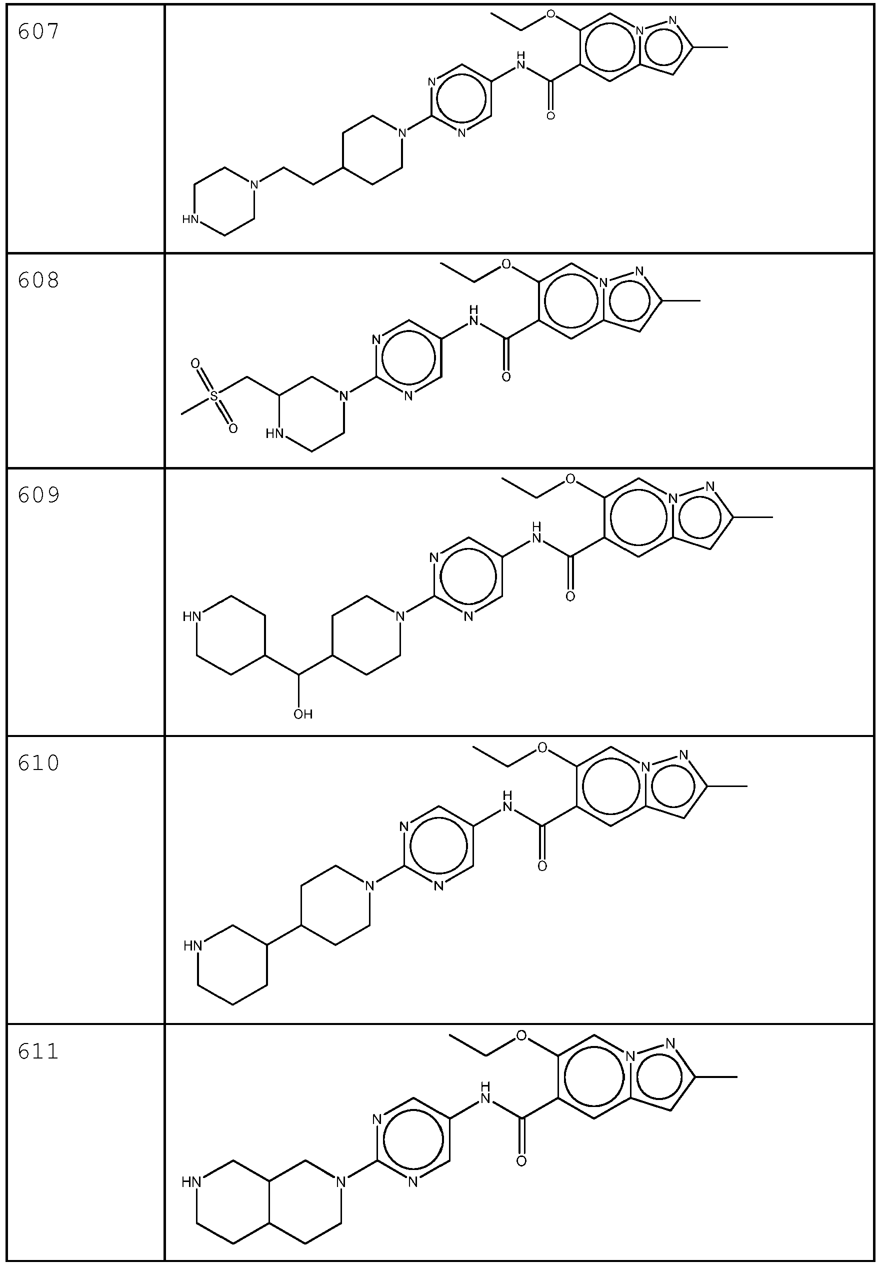

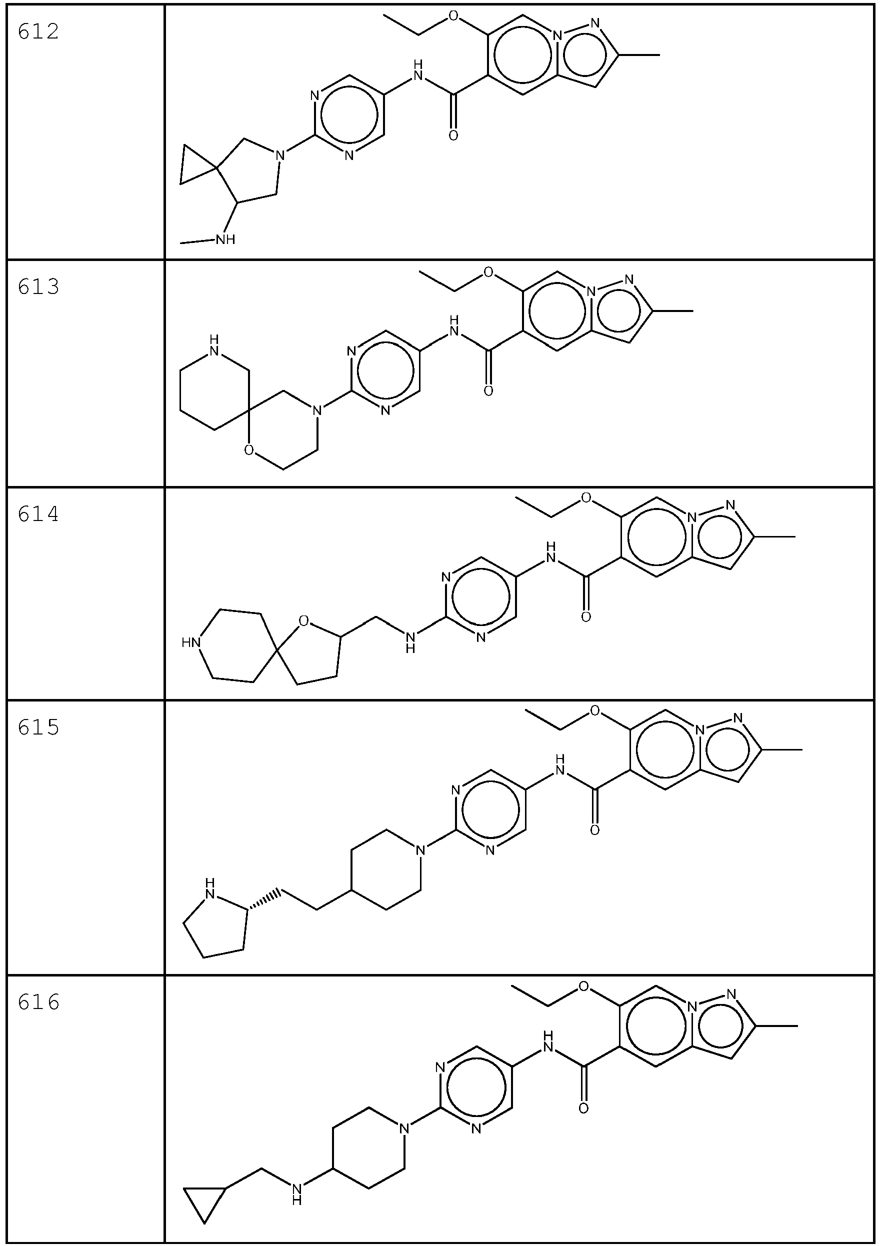

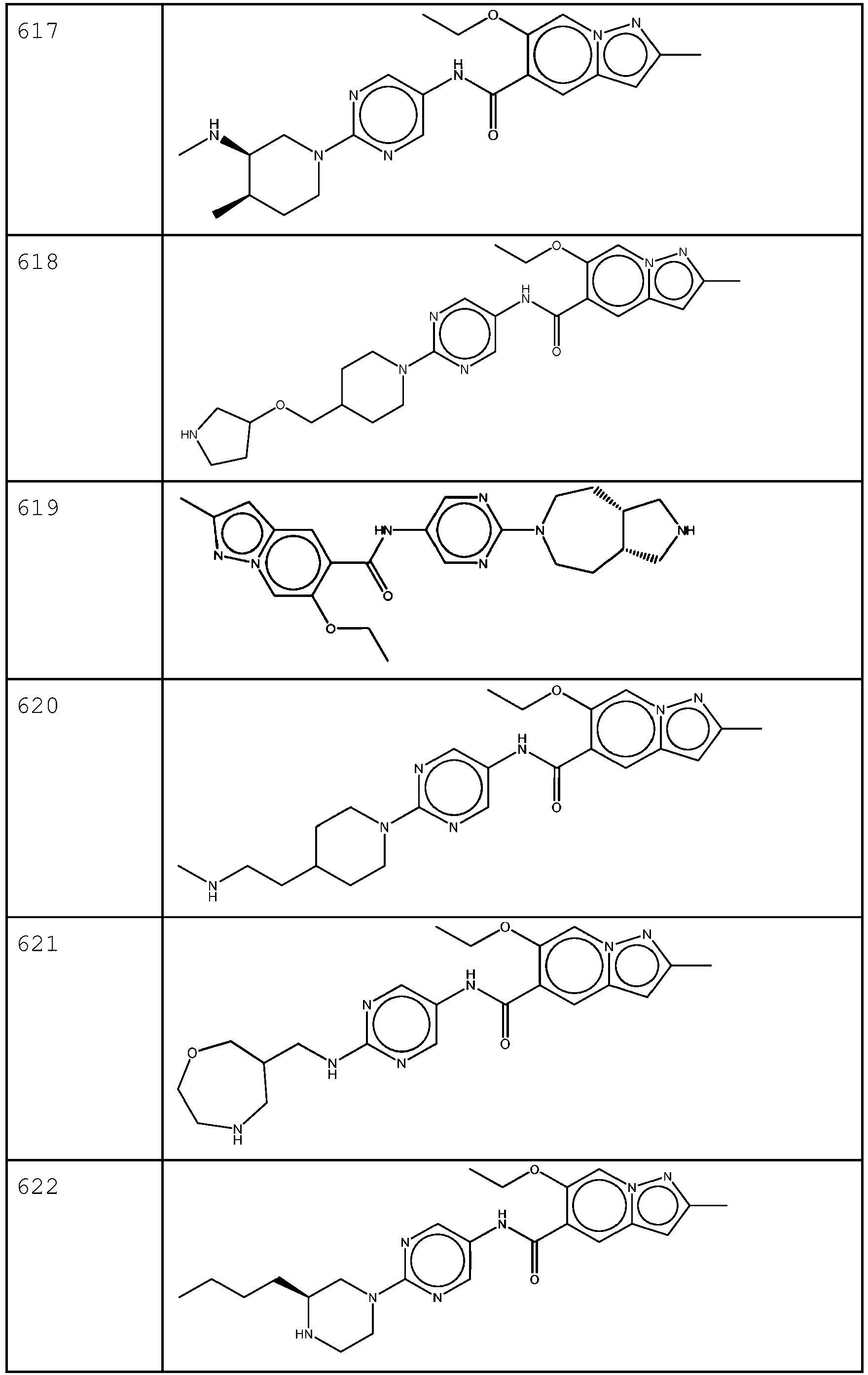

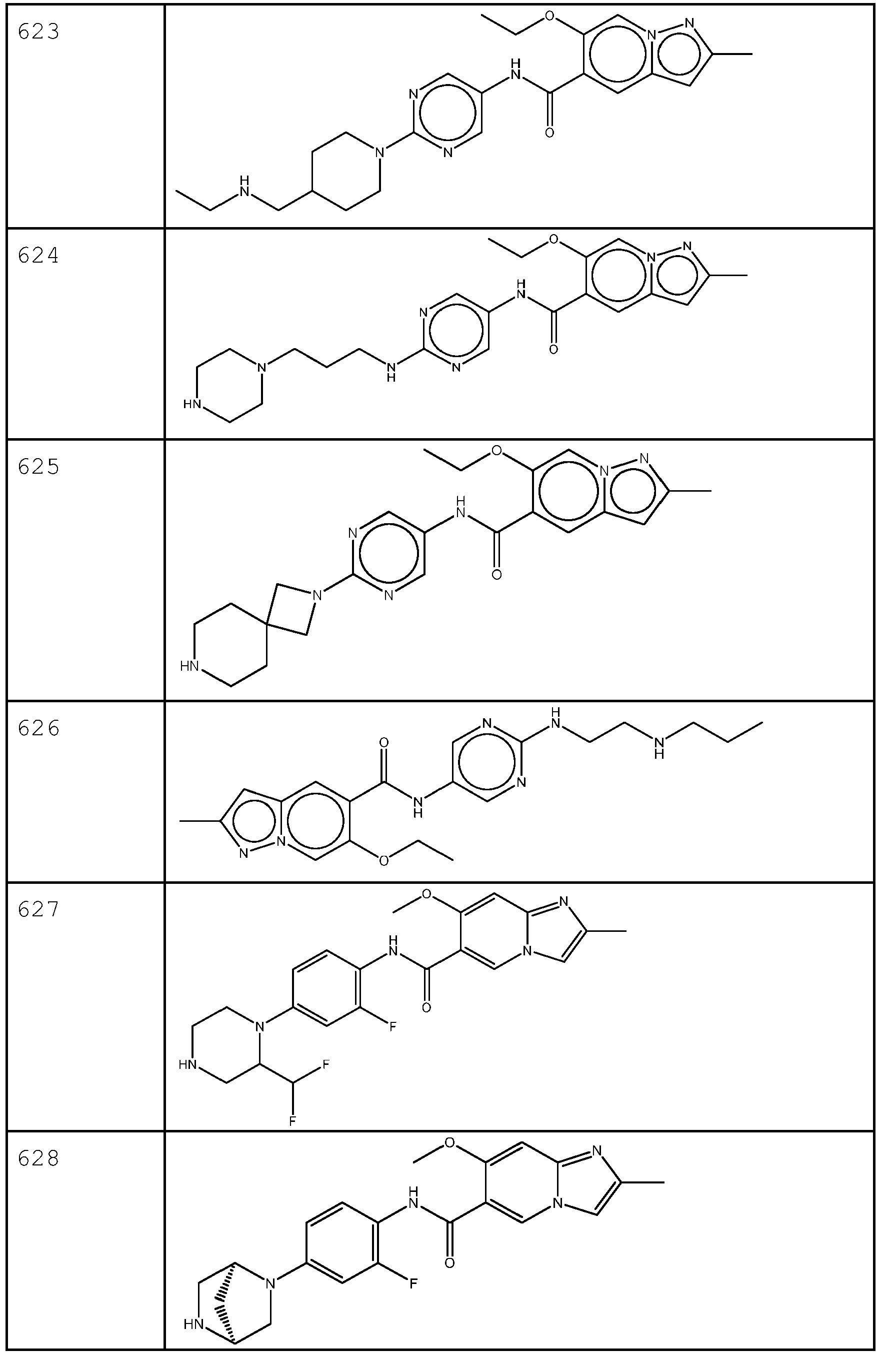

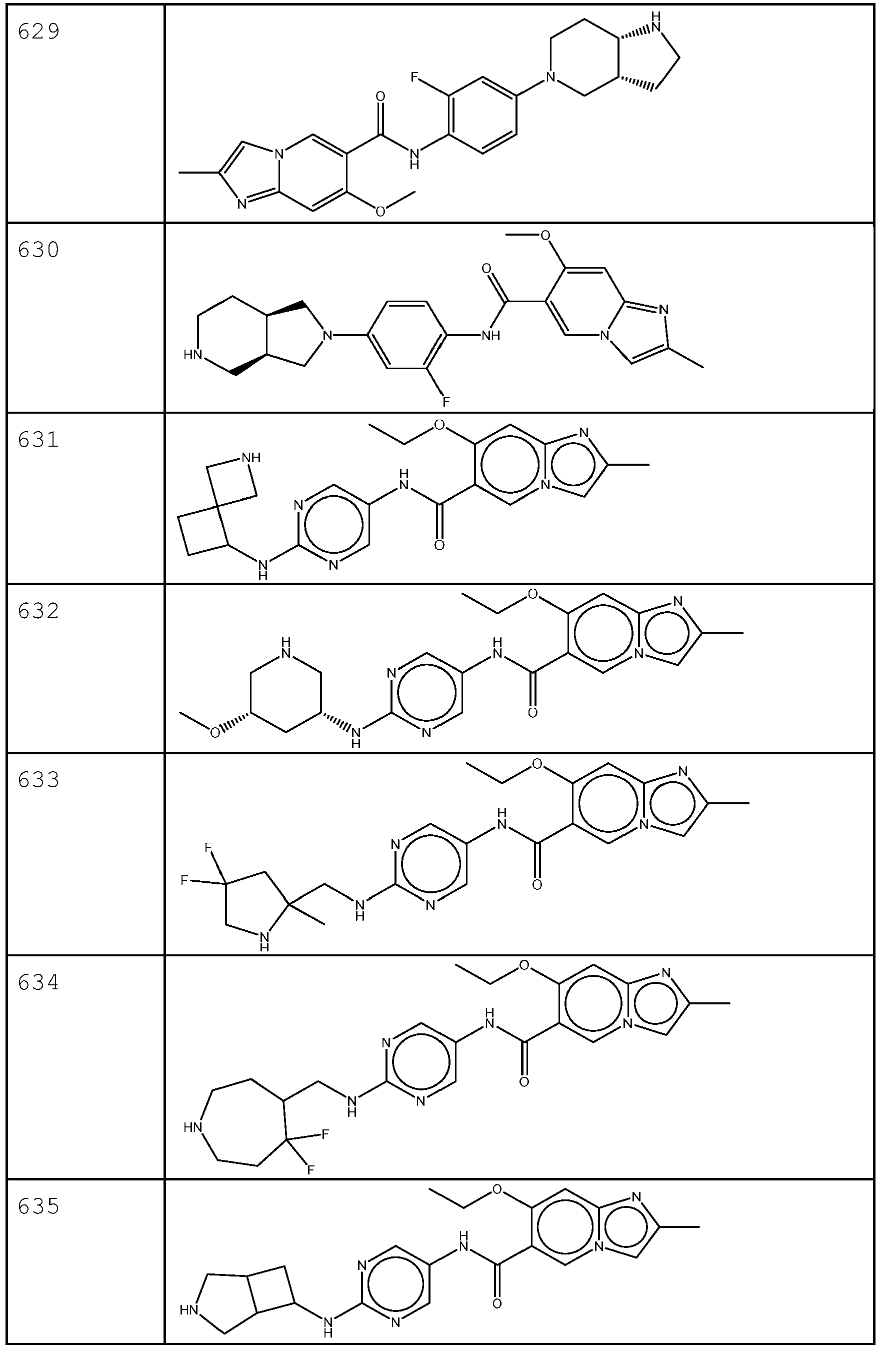

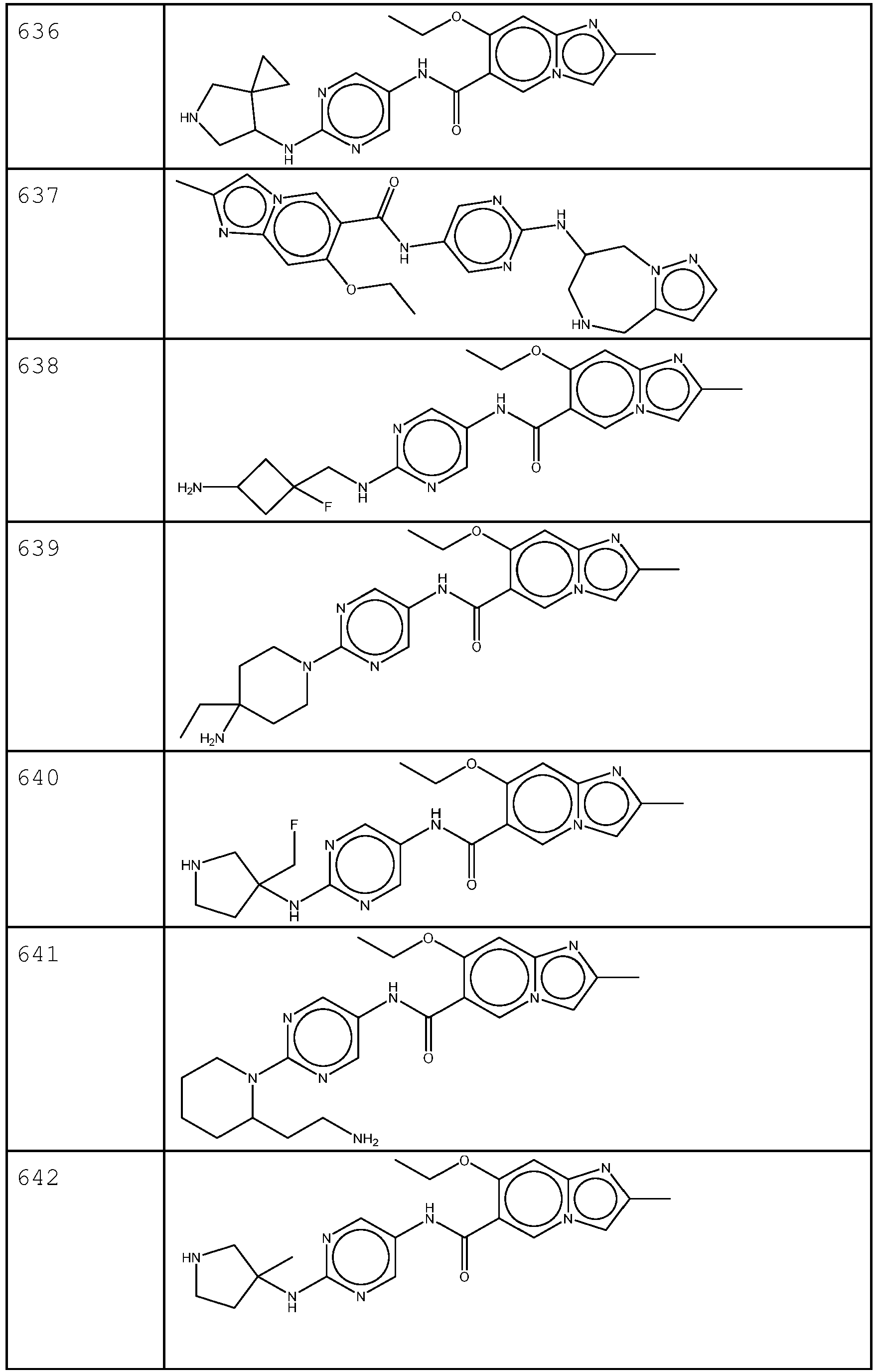

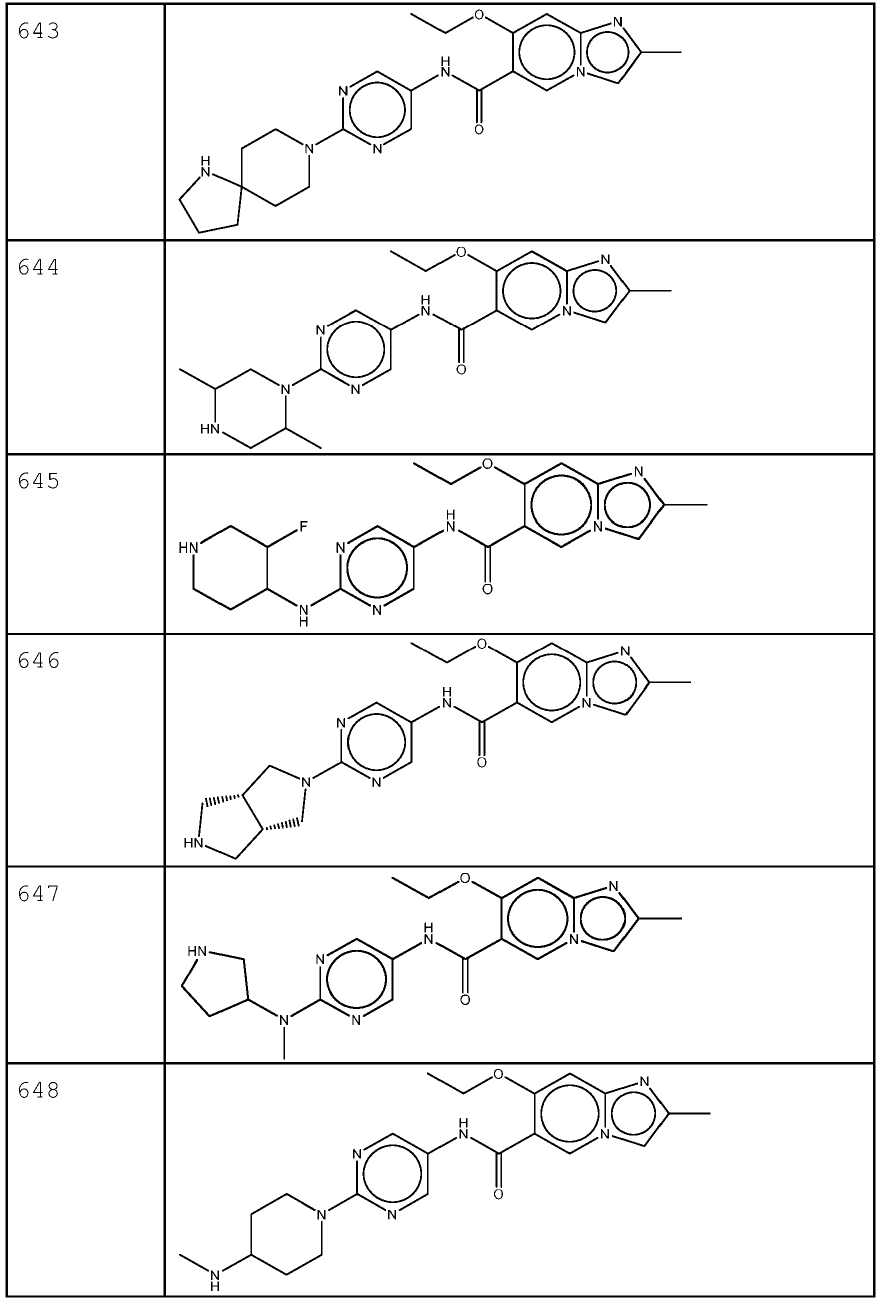

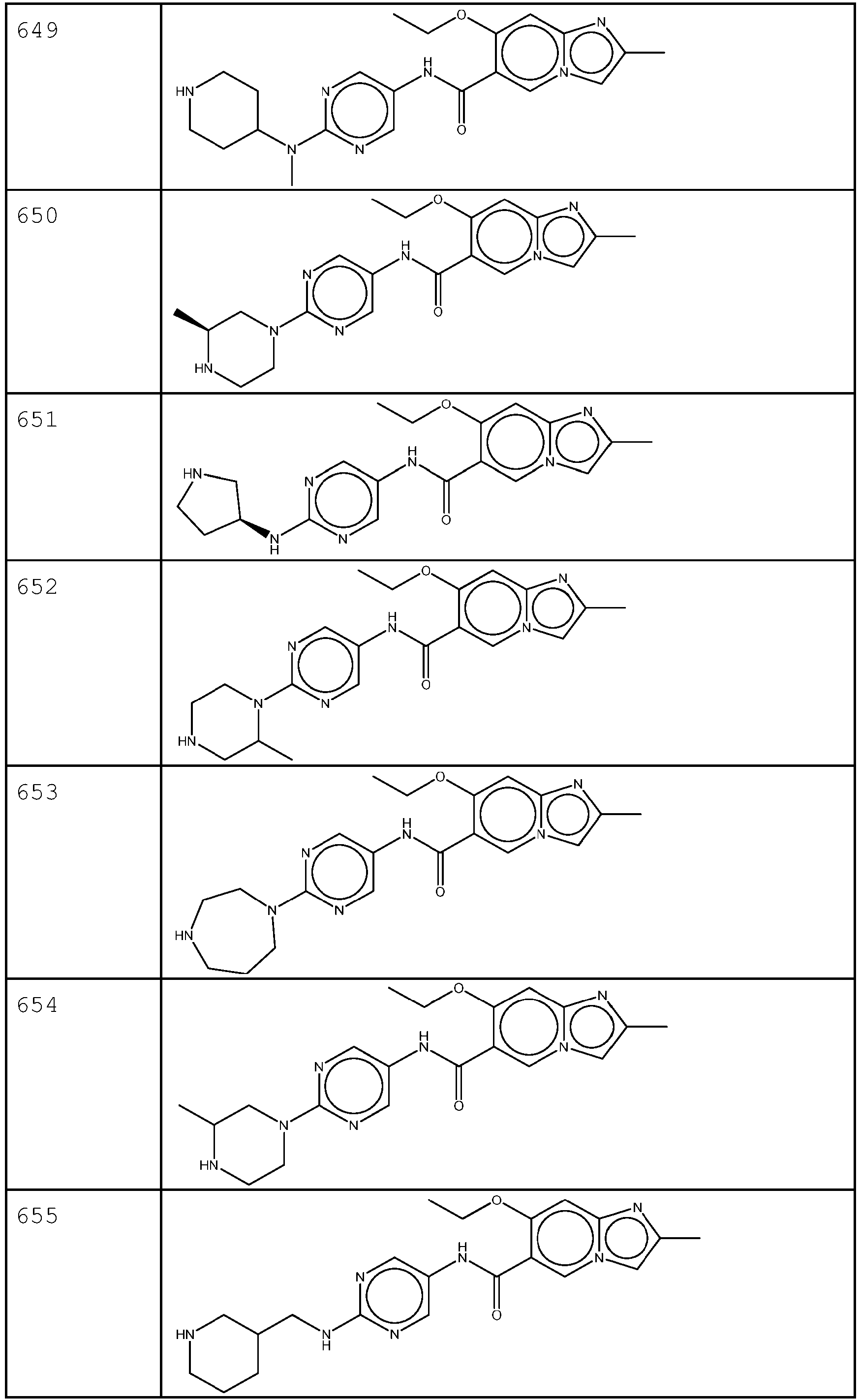

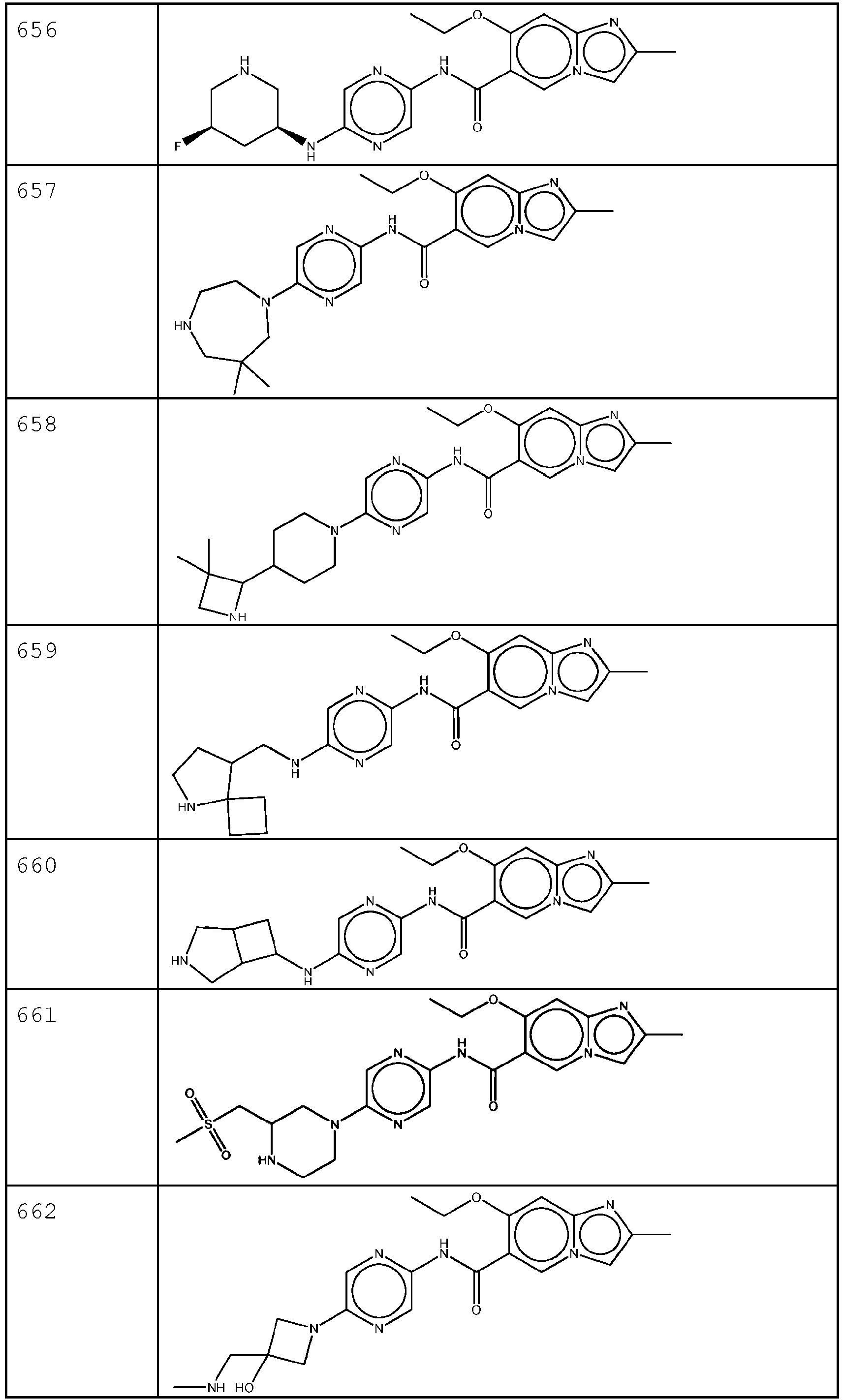

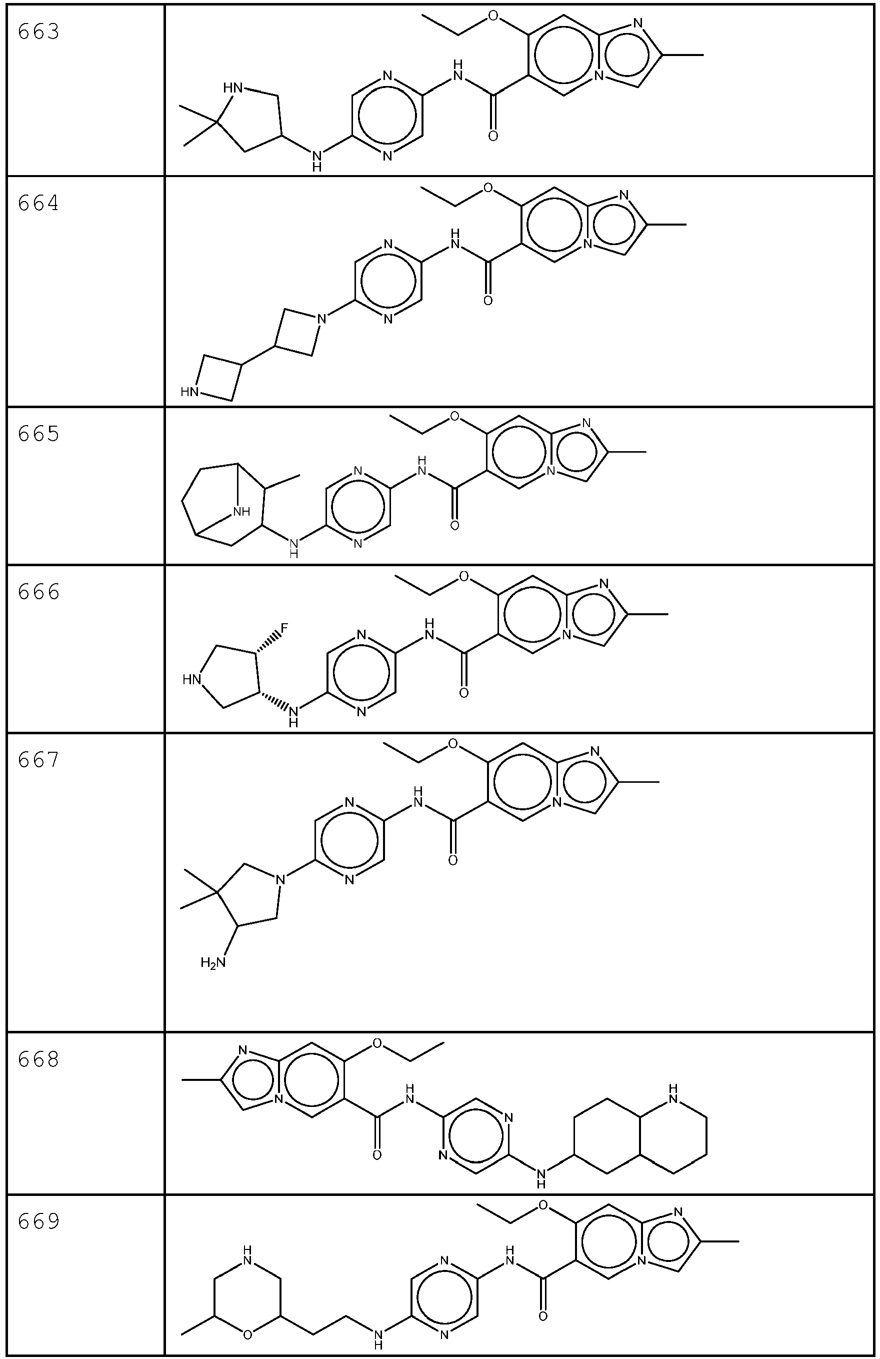

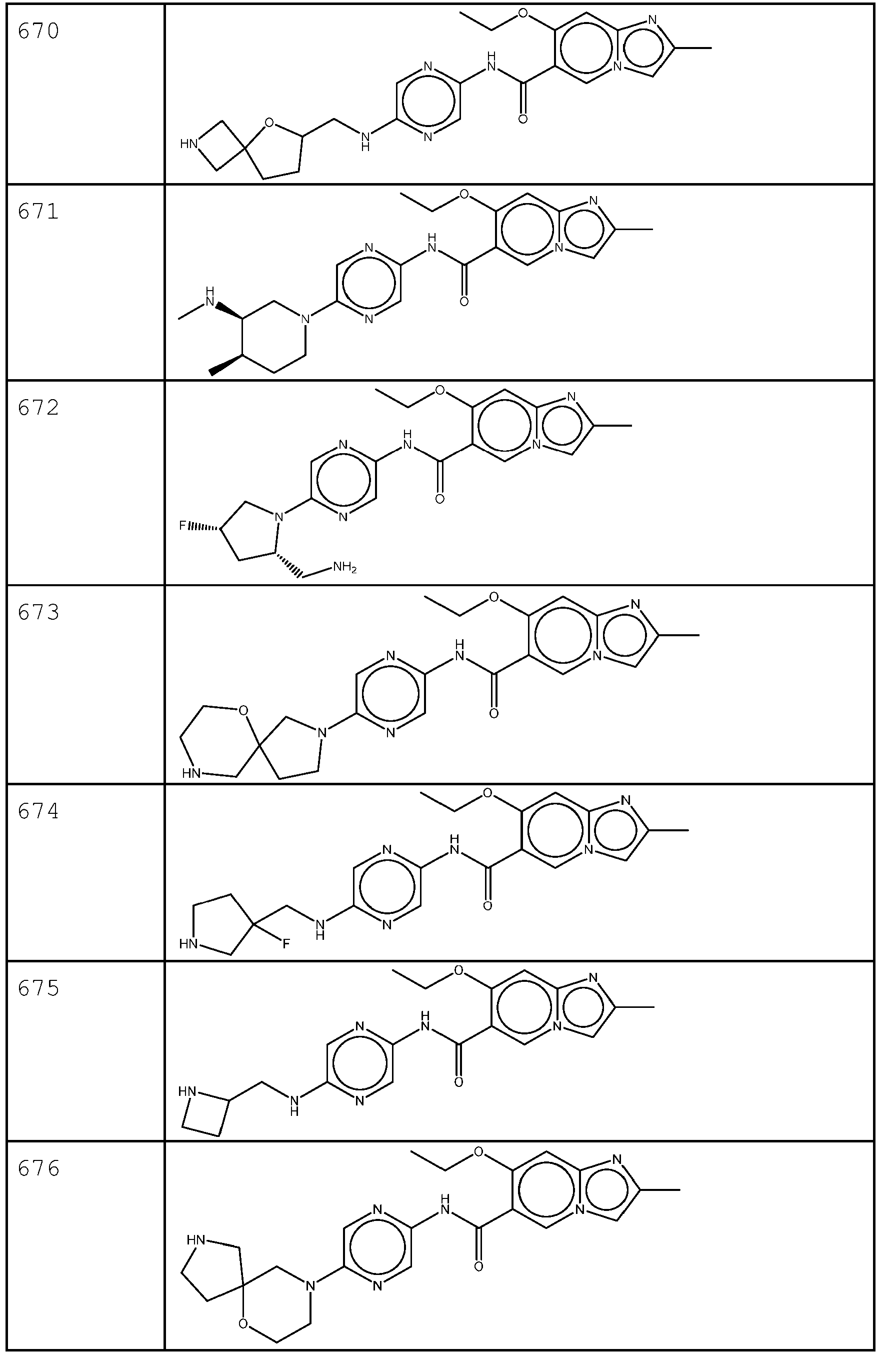

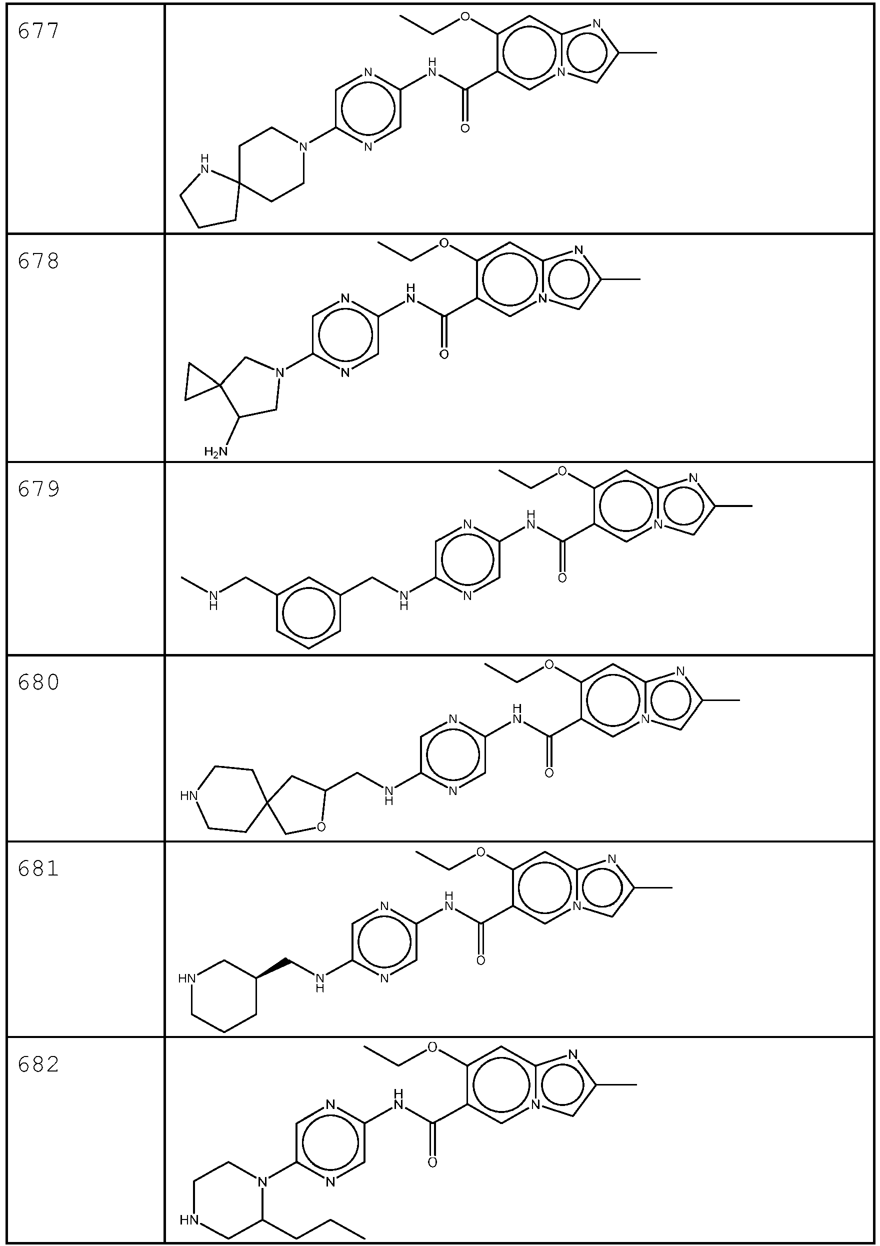

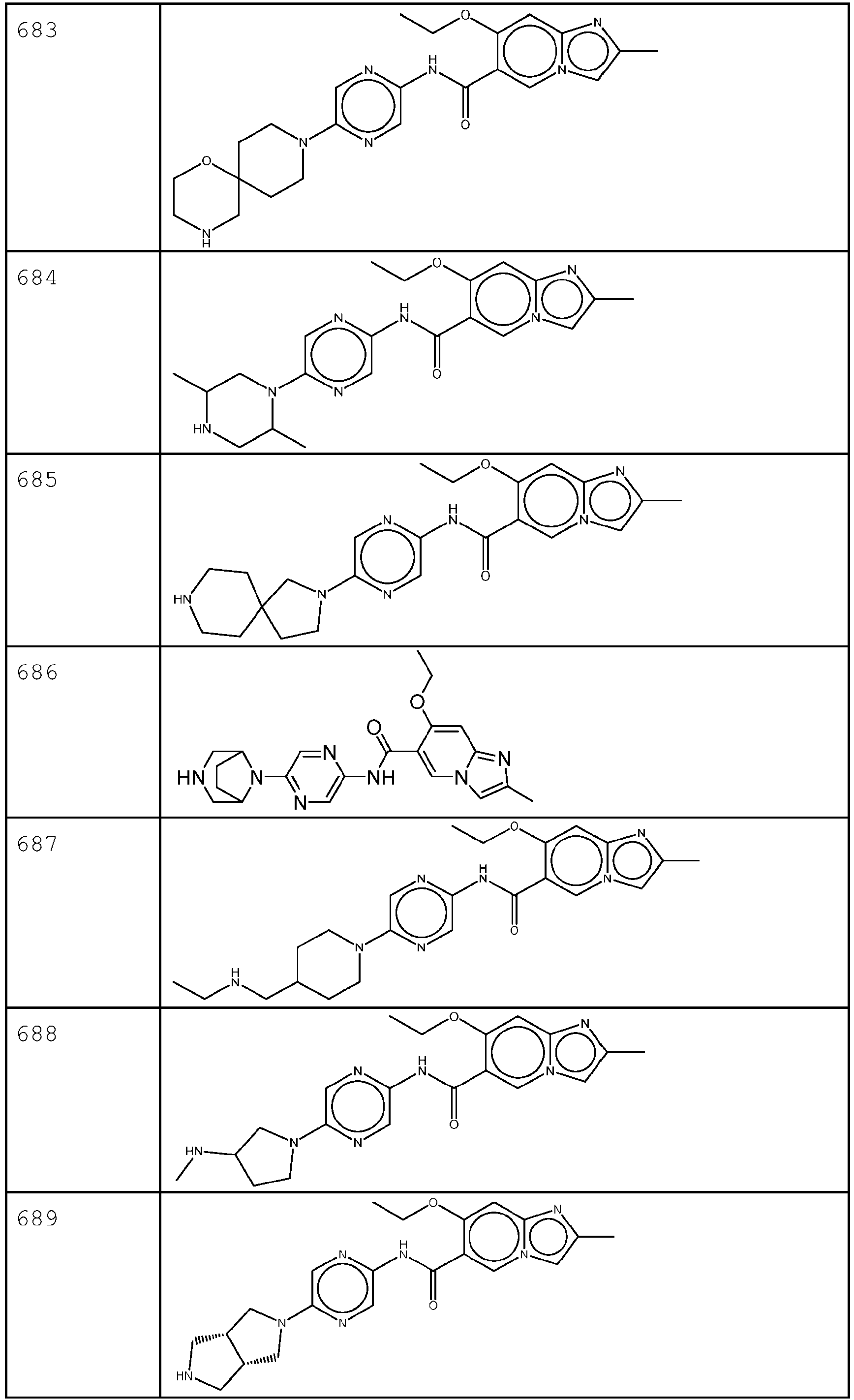

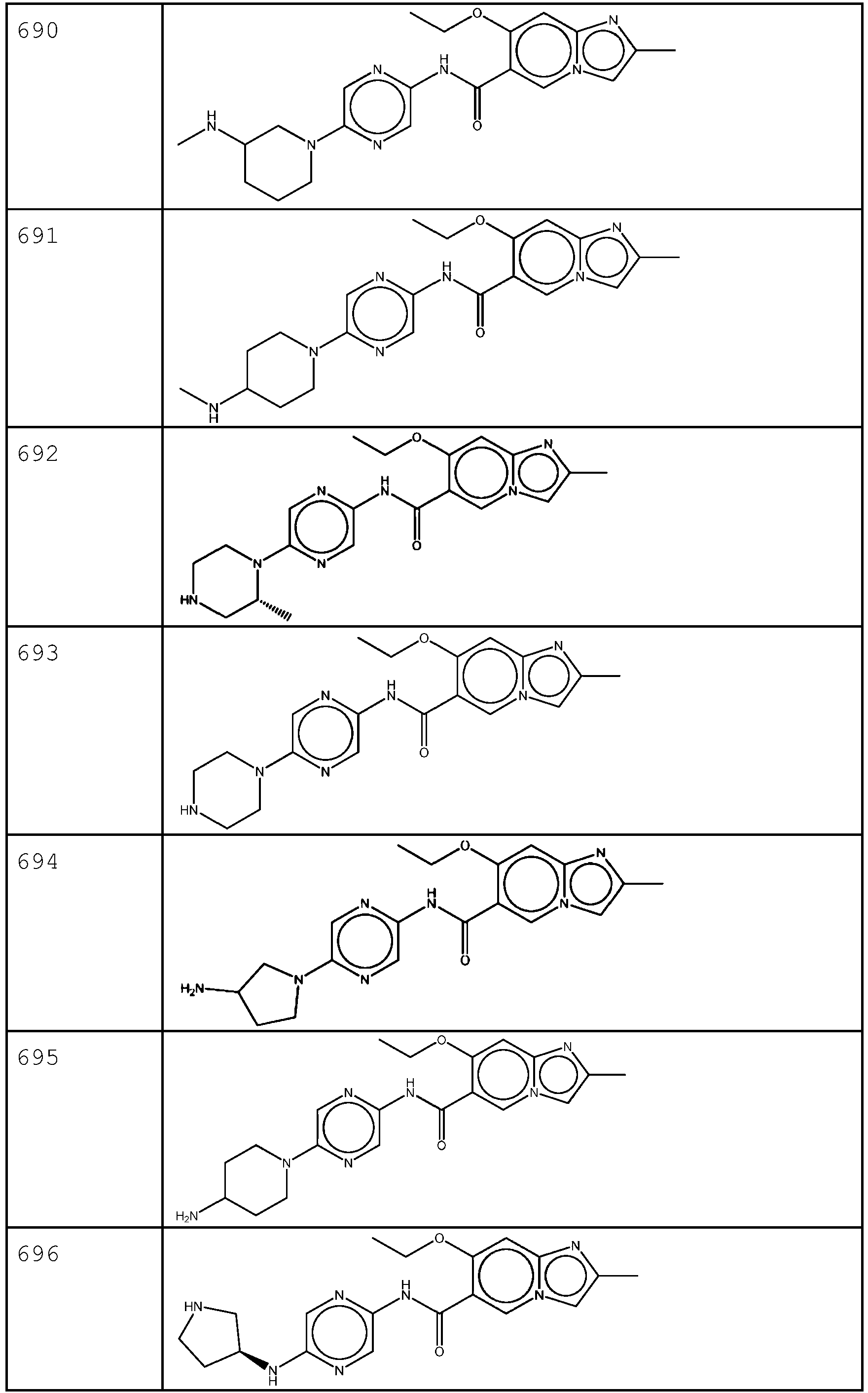

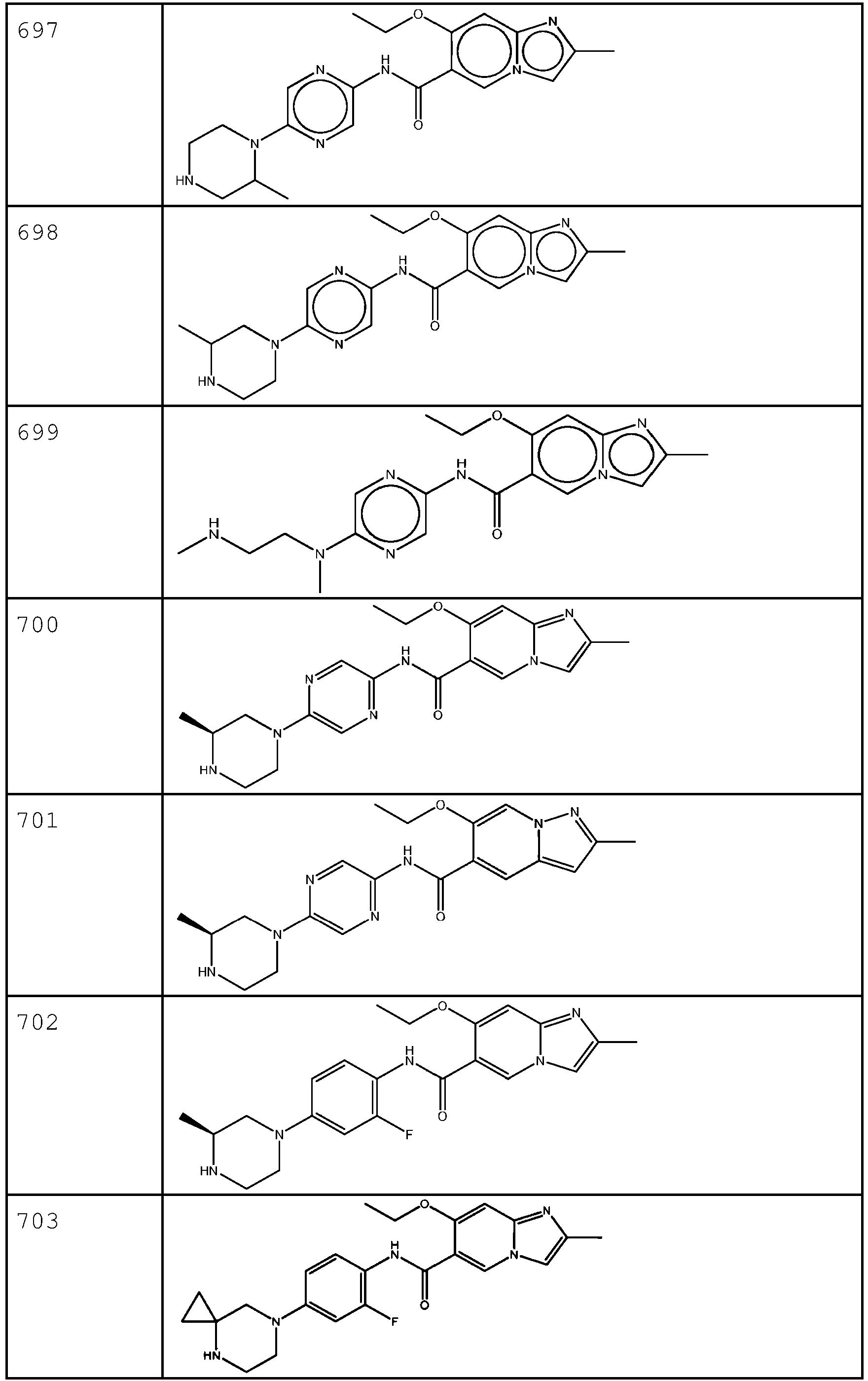

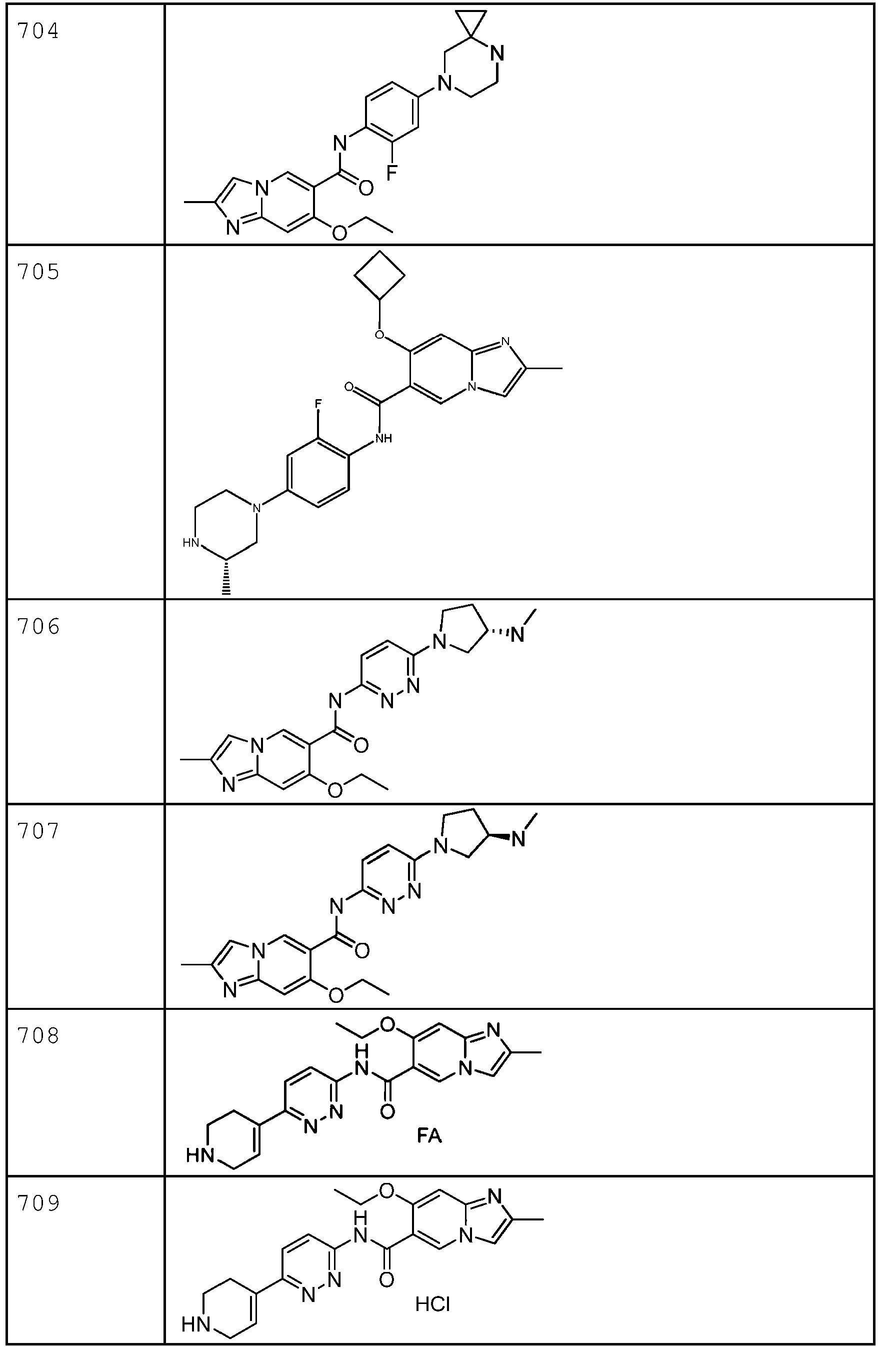

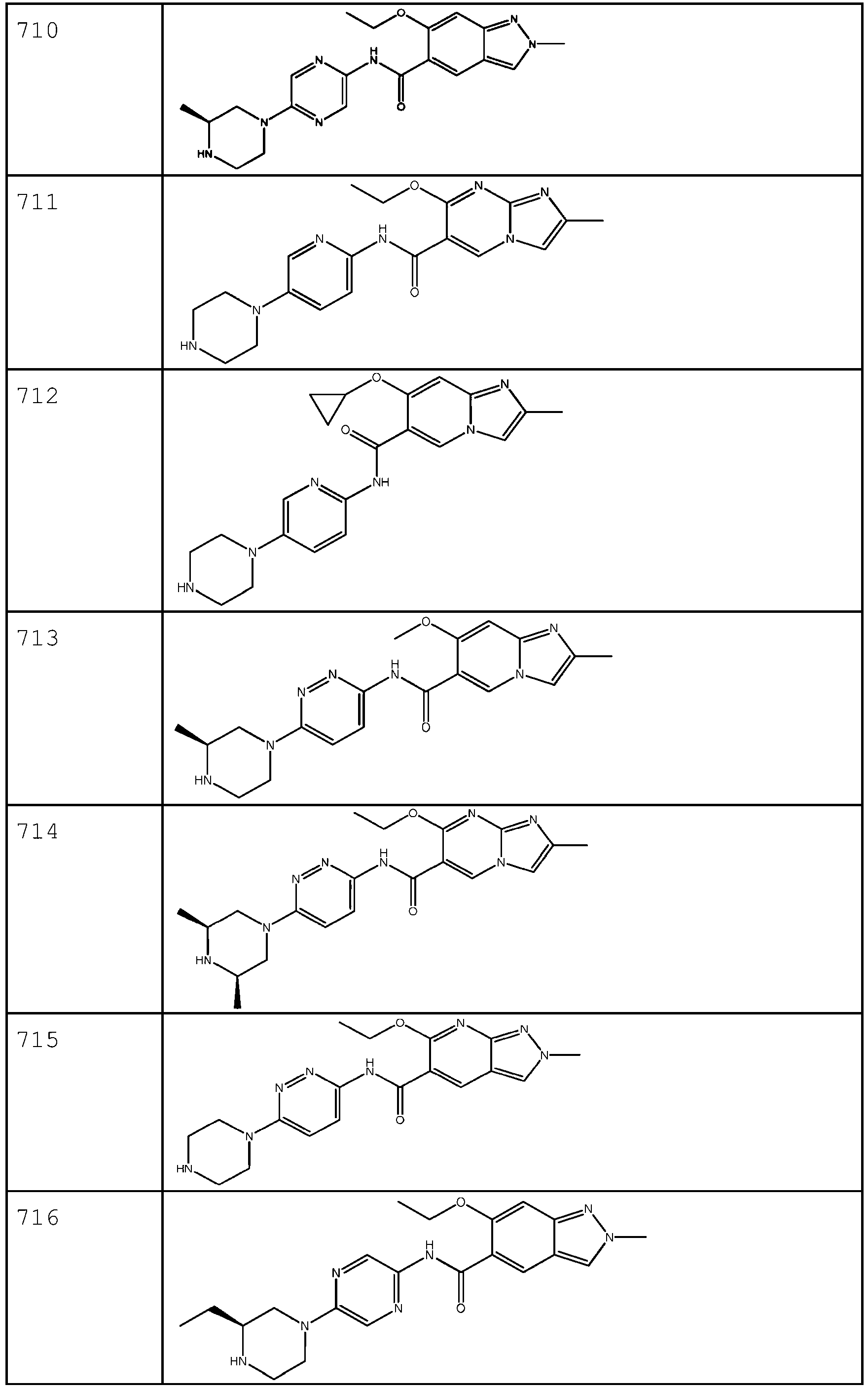

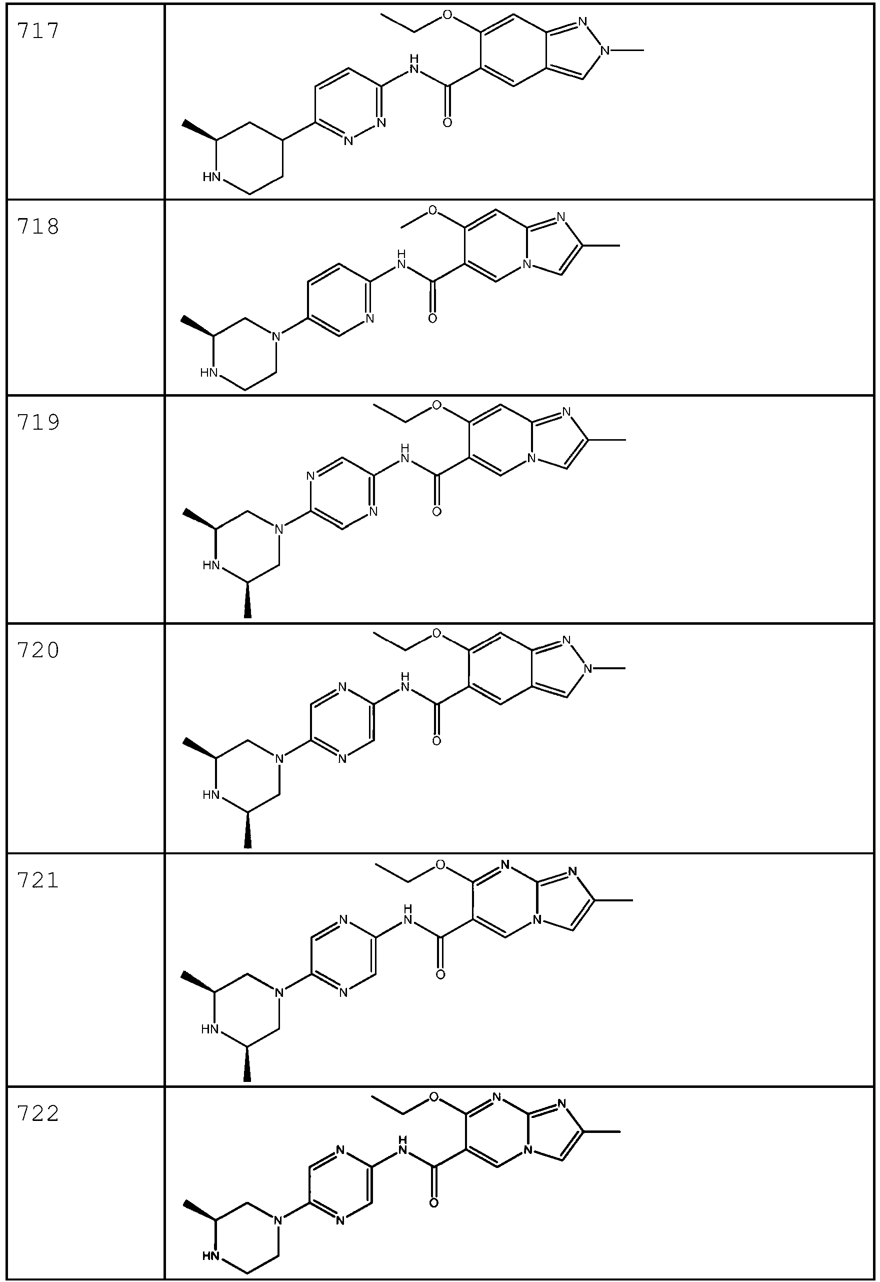

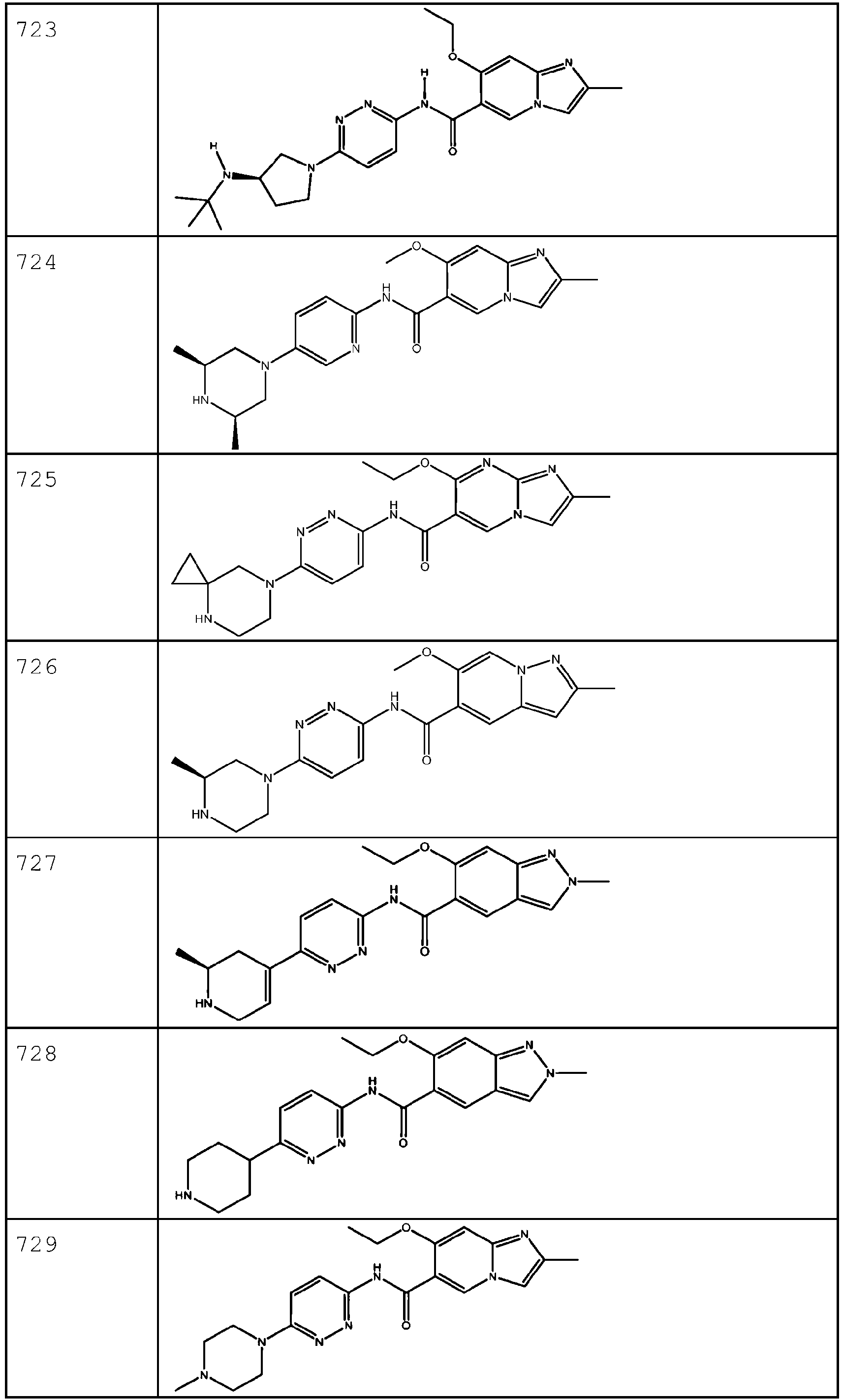

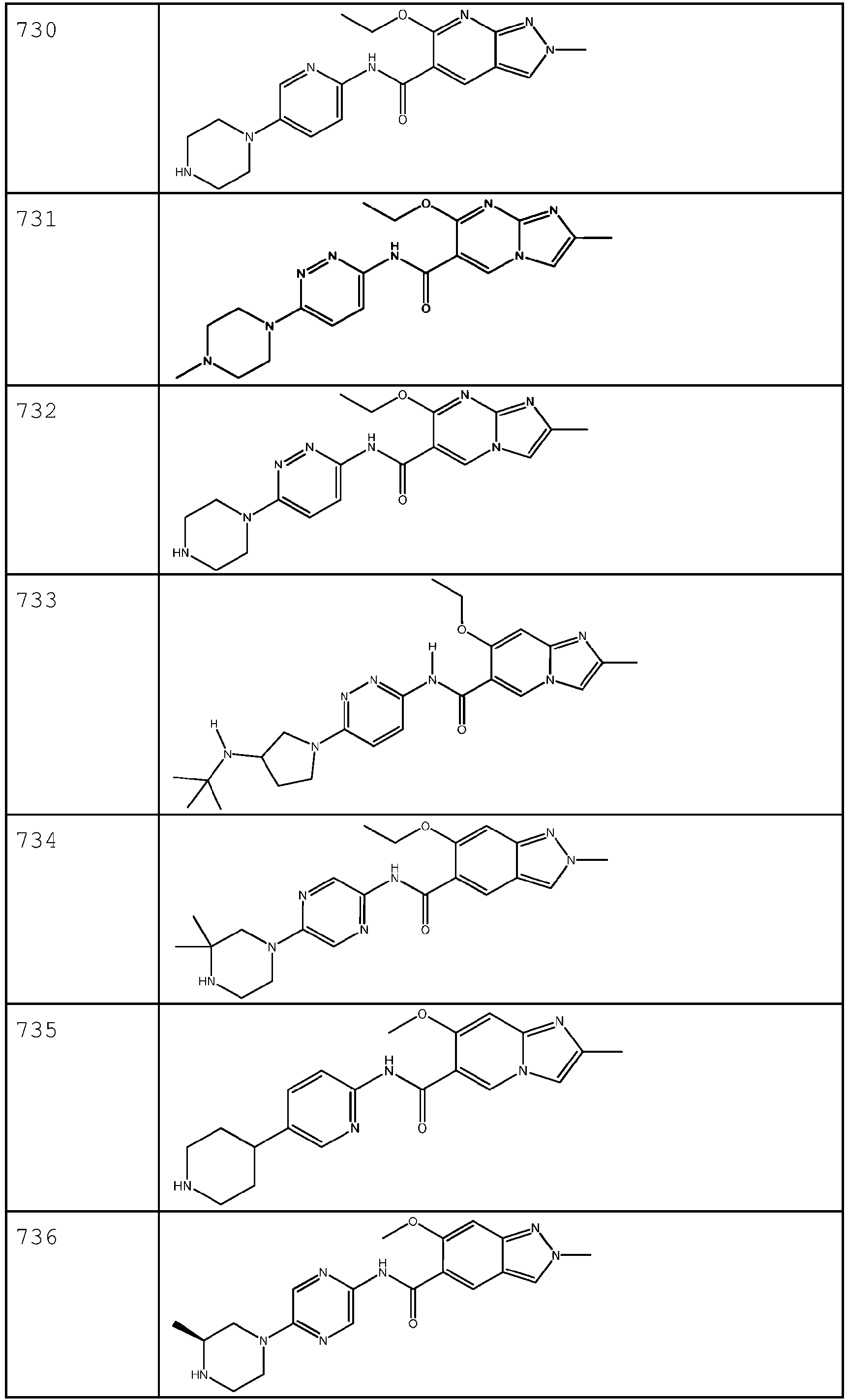

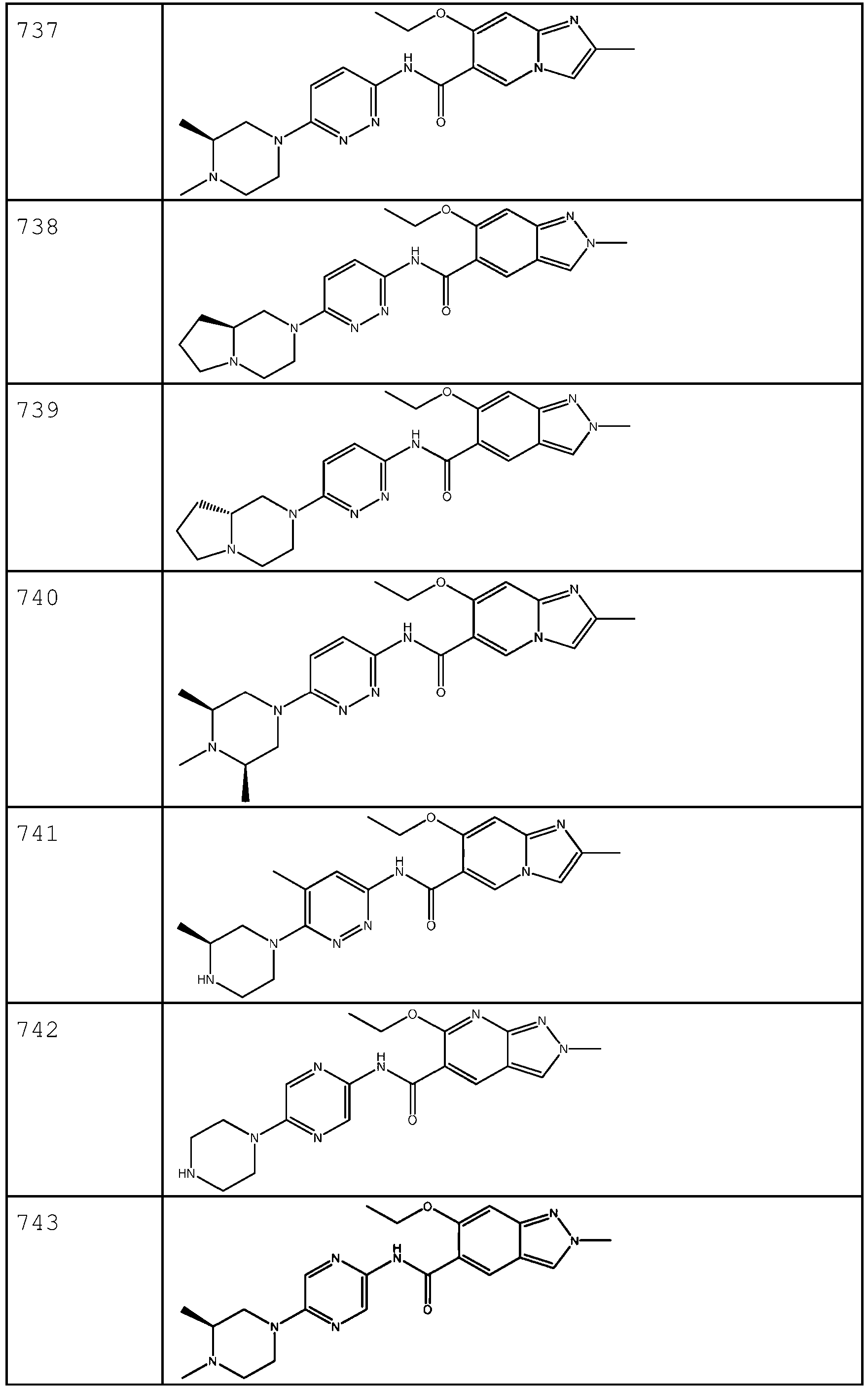

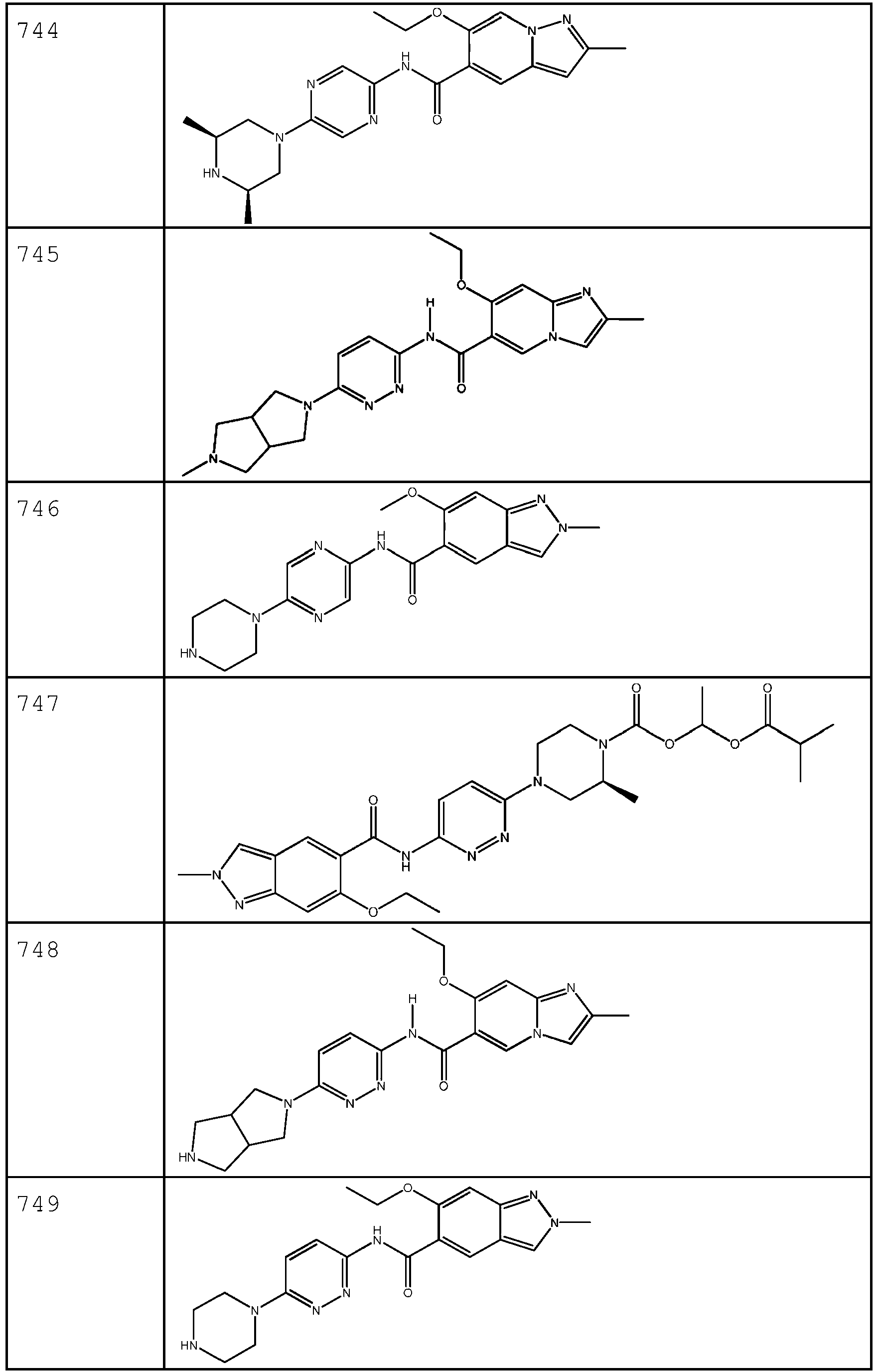

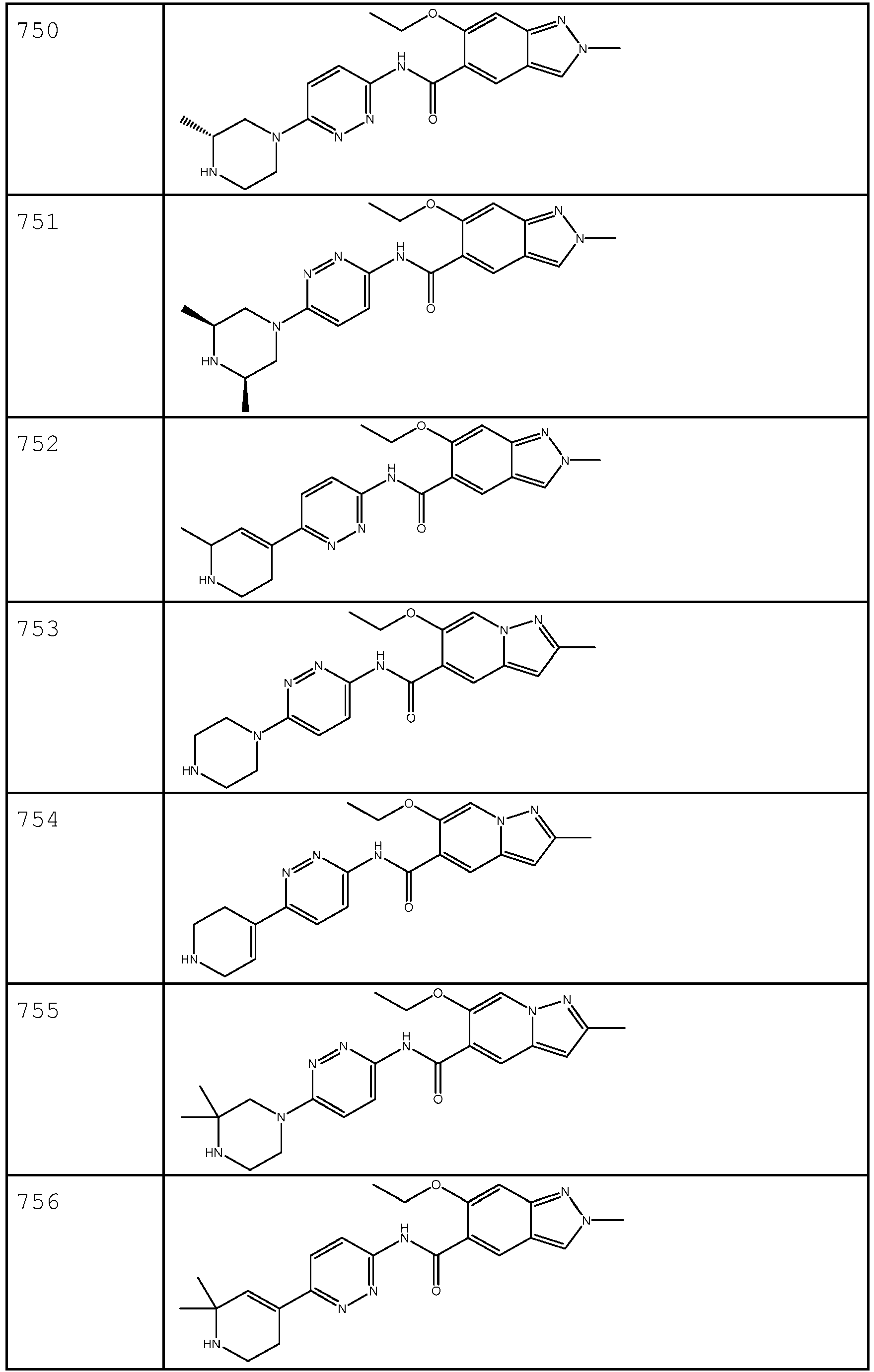

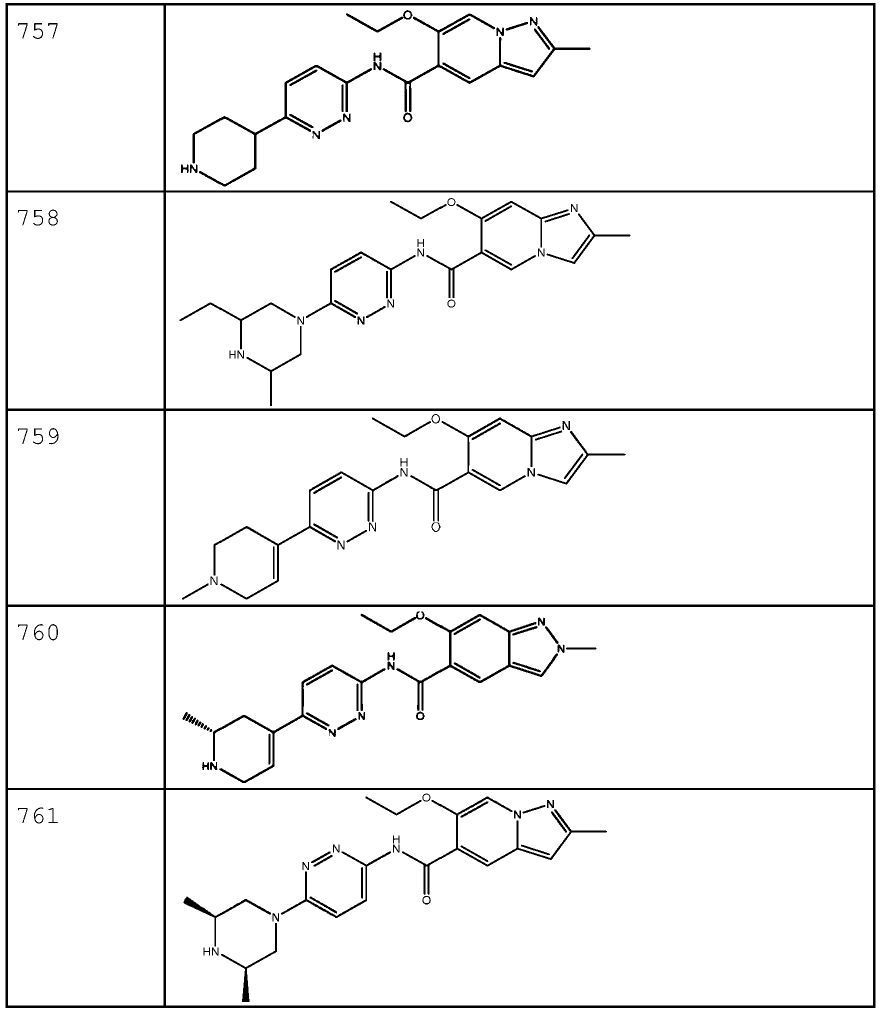

16. Pharmazeutische Zusammensetzung, umfassend eine Verbindung nach einem der Ansprüche 1-15 oder ein pharmazeutisch unbedenkliches Salz davon und einen oder mehrere pharmazeutisch unbedenkliche Exzipienten.

17. Verbindung nach einem der Ansprüche 1-15 oder pharmazeutisch unbedenkliches Salz oder pharmazeutische Zusammensetzung nach Anspruch 16 zur Verwendung in einem Verfahren zur Behandlung:
(a) einer Störung im Zusammenhang mit einer Nukleotid-Wiederholungssequenz-Expansion, wobei die Nukleotid-Wiederholungssequenz-Expansion gegebenenfalls Folgendes umfasst:
(i) eine zwei- oder mehrmals wiederholte Nukleotidsequenz, wobei die Nukleotidsequenz aus der aus CAG, CAG/CTG, GCG, GCN, CGG, CCG, CCCCGCCCCGCG, GCA, GGGGCC, CTG, GAA, ATTCT, TGGAA, GGCCTG, AAGGG, CCCTCT, ATTTT/ATTTC und CCCTCT bestehenden Gruppe ausgewählt ist, oder
(ii) eine zwei- oder mehrmals wiederholte Trinukleotidsequenz, wobei die Trinukleotidsequenz aus der aus CAG, CTG, CGG und GCN bestehenden Gruppe ausgewählt ist, oder
(b) einer Krankheit, wobei die Krankheit aus der aus dentatorubropallidoluysischer Atrophie, Huntington-Krankheit, spinaler und bulärer Muskelatrophie, SCA1 (spinozerebelläre Ataxie Typ 1), SCA2 (spinozerebelläre Ataxie Typ 2), SCA3 (spinozerebelläre Ataxie Typ 3 oder Machado-Joseph-Krankheit),
SCA6 (spinozerebelläre Ataxie Typ 6), SCA7 (spinozerebelläre Ataxie Typ 7), SCA12 (spinozerebelläre Ataxie Typ 12), SCA17 (spinozerebelläre Ataxie Typ 17), FRAXA (Fragiles-X-Syndrom), FXTAS (Fragiles-X-assoziiertes Tremor-/Ataxie-Syndrom), FRAXE (Fragiles-X-Syndrom mit geistiger Behinderung), Baratela-Scott-Syndrom, FRDA (Friedreich-Ataxie), DM1 (myotone Dystrophie Typ 1), DM2 (myotone Dystrophie Typ 2), SCA8 (spinozerebelläre Ataxie Typ 8), Fuchs-Endothel-Hornhautdystrophie, Desbuquois-Dysplasie, amyotropher Lateralsklerose und frontotemporaler Demenz bestehenden Gruppe ausgewählt ist, wobei es sich bei der Krankheit gegebenenfalls um:
(i) Huntington-Krankheit oder
(ii) myotone Dystrophie Typ 1 handelt oder
(iii) die Krankheit aus der aus FRAXA (Fragiles-X-Syndrom), FXTAS (Fragiles-X-assoziiertes Tremor-/Ataxie-Syndrom), FRAXE (Fragiles-X-assoziierte geistige Behinderung) bestehenden Gruppe ausgewählt ist.

## Revendications

1. Composé de formule (I) : 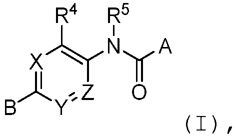 ou sel pharmaceutiquement acceptable correspondant :
X étant = N ou CR¹ ; y étant = N ou CR² ; Z étant = N ou CR³ ;
R¹, R², R³ et R⁴ étant chacun indépendamment choisis dans le groupe constitué par H, halogène, hydroxy, cyano, - COOH, -C(O)-alkyle en C₁-C₆, -C(O)O-alkyle en C₁-C₆, alkyle en C₁-C₆, alcényle en C₂-C₆, alcynyle en C₂-C₆, alcoxy en C₁-C₆, cycloalcoxy en C₁-C₆, cycloalkyle en C₃-C₈, NH₂, NH(alkyle en C₁-C₆), N(alkyle en C₁-C₆)₂, -NHC (O)-alkyle en C₁-C₆, -N(alkyl en C₁-C₆) -C (O) -alkyle en C₁-C₆, -C(O)-NH₂, -C(O)-NH(alkyle en C₁-C₆) et -C(O)-N(alkyle en C₁-C₆)₂, l'alkyle, l'alcényle, l'alcynyle et l'alcoxy étant éventuellement substitués par un ou plusieurs substituants halogéno, hydroxy, méthoxy, cycloalkyle en C₃-C₈ ou NH₂ ;
R⁵ étant H, alkyle en C₁-C₆ ou halogénoalkyle en C₁-C₆ ;
A étant choisi dans le groupe constitué par 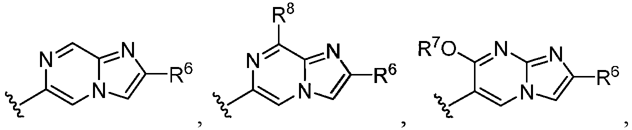 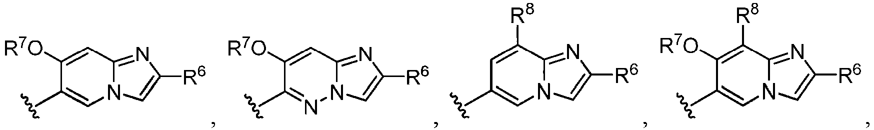 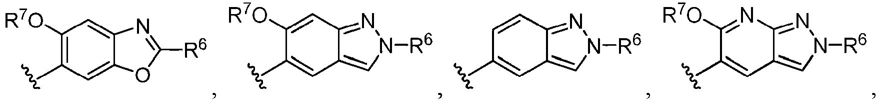 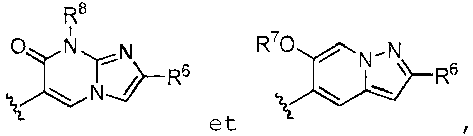
A étant éventuellement substitué par 1 à 4 R⁹ ;
R⁶ étant alkyle en C₁-C₃ ou halogénoalkyle en C₁-C₃ ;
R⁷ étant H, alkyle en C₁-C₆, halogénoalkyle en C₁-C₆, cycloalkyle en C₁-C₆ ou hétérocycloalkyle, l'hétérocycloalkyle étant éventuellement substitué par 1 à 3 substituants indépendamment choisis parmi les substituants halogéno et alkyle en C₁-C₆ ;
R⁶ étant halogène, alkyle en C₁-C₆, alcoxy en C₁-C₆, halogénoalkyle en C₁-C₆ ou cycloalkyle en C₁-C₆ ;
chaque R⁹ étant indépendamment choisi dans le groupe constitué par halogène, hydroxy, cyano, -COOH, -C(O)-alkyle en C₁-C₆, -C(O)O-alkyle en C₁-C₆, alkyle en C₁-C₆, alcényle en C₂-C₆, alcynyle en C₂-C₆, alcoxy en C₁-C₆, cycloalcoxy en C₃-C₈, cycloalkyle en C₃-C₈, NH₂, NH(alkyle en C₁-C₆), N(alkyle en C₁-C₆)₂, -NHC(O)-alkyle en C₁-C₆, - N(alkyl en C₁-C₆)-C(O) -alkyle en C₁-C₆, -C(O)-NH₂, -C(O)-NH(alkyle en C₁-C₆) et -C(O)-N(alkyle en C₁-C₆)₂, l'alkyle, l'alcényle, l'alcynyle et l'alcoxy étant éventuellement substitués par un ou plusieurs substituants halogéno, hydroxy, méthoxy, cycloalkyle en C₃-C₈ ou NH₂ ;
B étant hétérocycloalkyle non relié à la formule (I) par un atome d'azote, éventuellement substitué par 1 à 6 R¹² ; ou
B étant NR¹⁰R¹¹,
R¹⁰ étant -(CH₂)₀₋₃-aryle, -(CH₂)₀₋₃-hétéroaryle, -(CH₂)₀₋₃-hétérocycloalkyle comprenant au moins 1 atome d'azote de cycle ou hétéroalkyle en C₁-C₈ comprenant au moins un atome d'azote, chaque R¹⁰ étant éventuellement substitué par 1 à 6 R¹² ; et
R¹¹ étant hydrogène, alkyle en C₁₋₇, halogénoalkyle en C₁₋₇ ou cycloalkyle en C₃₋₈ ; ou
R¹⁰ et R¹¹ étant pris conjointement avec l'atome d'azote auquel ils sont liés pour former un hétérocycloalkyle comprenant 1, 2 ou 3 atomes d'azote de cycle au total et 0 ou 1 hétéroatome de cycle supplémentaire choisi parmi O et S et l'hétérocycloalkyle étant éventuellement substitué par 1 à 6 R¹² ;
chaque R¹² étant indépendamment choisi dans le groupe constitué par halogène, hydroxy, cyano, -COOH, -C(O)-alkyle en C₁-C₆, -C(O)O-alkyle en C₁-C₆, alkyle en C₁-C₆, hétéroalkyle en C₁-C₆, alcényle en C₂-C₆, alcynyle en C₂-C₆, alcoxy en C₁-C₆, -(CH₂)₀₋₂-cycloalkyle en C₃-C₈, -(CH₂)₀-₂-SO₂-alkyle en C₁-C₆, hétéroalkylène en C₁-C₆-cycloalkyle en C₃-C₈, -O-cycloalkyle en C₃-C₈, hétérocycloalkyle monocyclique à 4 à 7 chaînons, hétéroalkylène en C₁-C₆-(hétérocycloalkyle monocyclique à 4 à 7 chaînons), -O-(hétérocycloalkyle monocyclique à 4 à 7 chaînons), - (CH₂)₀-₂-(hétérocycloalkyle monocyclique à 4 à 7 chaînons), NH₂, NH(alkyle en C₁-C₆), N(alkyle en C₁-C₆)₂, -NHC(O)-alkyle en C₁-C₆, -N(alkyl en C₁-C₆)-C(O)-alkyle en C₁-C₆, -C(O)-NH₂, -C(O)-NH(alkyle en C₁-C₆) et -C(O)-N(alkyle en C₁-C₆)₂, l'alkyle, l'alcényle, l'alcynyle et l'alcoxy étant éventuellement substitués par un ou plusieurs substituants halogéno, hydroxy ou NH₂ et le cycloalkyle et l'hétérocycloalkyle étant éventuellement substitués par un ou plusieurs substituants halogéno, hydroxy, alkyle en C₁-C₆, hétéroalkyle en C₁-C₆, alcoxy en C₁-C₆ ou NH₂ ; ou
deux R¹² sur le même carbone pouvant être pris ensemble en tant que groupe céto (=O).

2. Composé selon la revendication 1, 0, 1 ou 2 de X, Y et Z étant N.

3. Composé selon la revendication 1 ou 2, le composé étant choisi parmi :
(a) la formule (Ia), 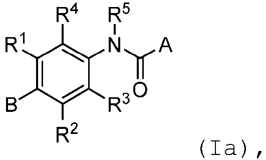 ou un sel pharmaceutiquement acceptable correspondant ;
(b) la formule (Ib), 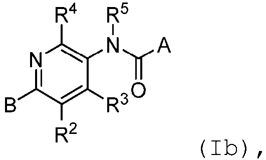 ou un sel pharmaceutiquement acceptable correspondant ;
(c) la formule (Ic), 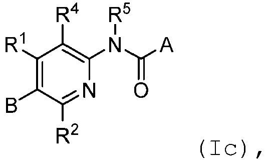 ou un sel pharmaceutiquement acceptable correspondant ;
(d) la formule (Id), 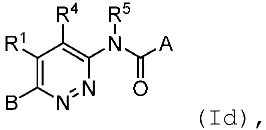 ou un sel pharmaceutiquement acceptable correspondant ;
(e) la formule (Ie), 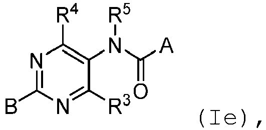 ou un sel pharmaceutiquement acceptable correspondant ; ou
(f) la formule (If), 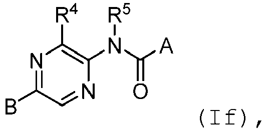 ou un sel pharmaceutiquement acceptable correspondant.

4. Composé selon l'une quelconque des revendications 1 à 3 :
(a) R¹, R², R³ et R⁴ étant chacun indépendamment choisis dans le groupe constitué par H, halogène, alkyle en C₁-C₆, halogénoalkyle en C₁-C₆, alcoxy en C₁-C₆ et cycloalcoxy en C₁-C₆ ; éventuellement R¹, R², R³ et R⁴ étant chacun indépendamment choisis dans le groupe constitué par H, halogène, alcoxy en C₁-C₆ et alkyle en C₁-C₆ ; éventuellement R¹, R², R³ et R⁴ étant chacun H ; et/ou
(b) R⁵ étant H ; et/ou
(c) A étant choisi dans le groupe constitué par 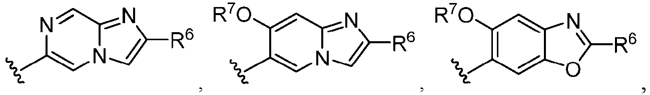 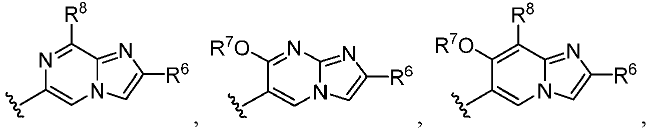 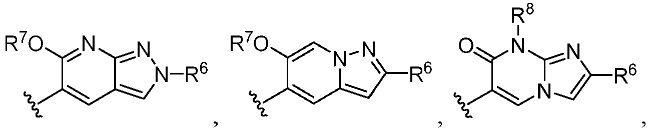 et 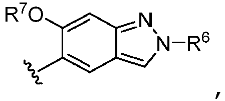 A étant éventuellement substitué par 1 à 3 R⁹.

5. Composé selon l'une quelconque des revendications 1 à 4 :
(a) A étant substitué par un R⁹ choisi dans le groupe constitué par halogène et alkyle en C₁-C₆ ou A n'étant pas substitué par R⁹ ; et/ou
(b) R⁶ étant Me ; et/ou
(c) R⁷ étant alkyle en C₁-C₆, cycloalkyle en C₁-C₆ ou hétérocycloalkyle ; éventuellement R⁷ étant Me, Et, isopropyle ou cyclobutyle.

6. Composé selon l'une quelconque des revendications 1 à 5, A étant choisi dans le groupe constitué par : 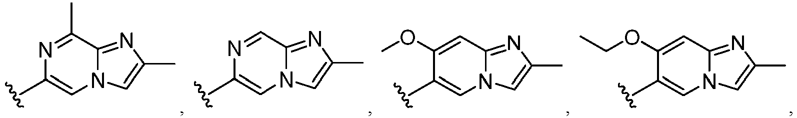 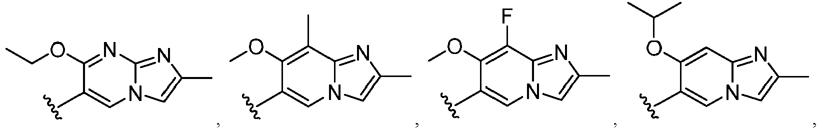 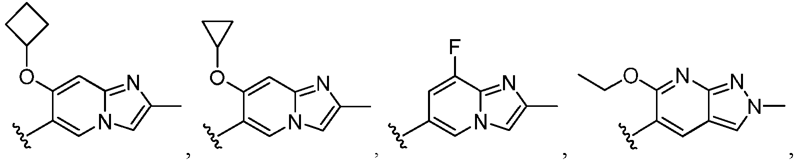 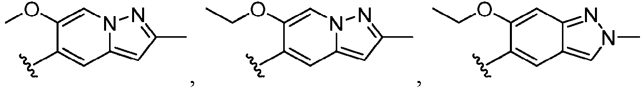 et

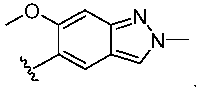

7. Composé selon l'une quelconque des revendications 1 à 6, A étant
(a) 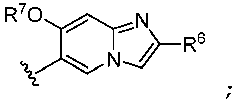 éventuellement
A étant 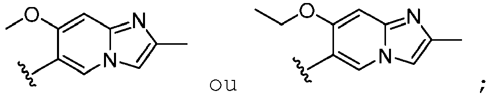 ou
(b) 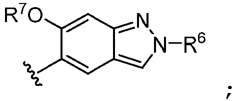 éventuellement
A étant 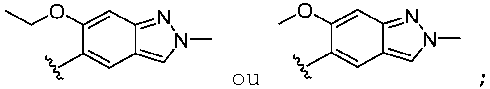 ou
(c)

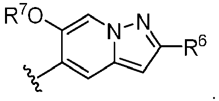

8. Composé selon l'une quelconque des revendications 1 à 7, B étant NR¹⁰R¹¹, R¹⁰ et R¹¹ étant pris conjointement avec l'atome d'azote auquel ils sont liés pour former un hétérocycloalkyle monocyclique ou bicyclique comprenant 1, 2 ou 3 atomes d'azote de cycle au total et 0 ou 1 hétéroatome de cycle supplémentaire choisi parmi O et S et l'hétérocycloalkyle étant éventuellement substitué par 1, 2, 3 ou 4 R¹².

9. Composé selon l'une quelconque des revendications 1 à 8, R¹⁰ et R¹¹ étant pris conjointement avec l'atome d'azote auquel ils sont liés pour former un hétérocycloalkyle monocyclique de 4 à 7 atomes de cycle renfermant 1 ou 2 atomes d'azote de cycle au total et 0 ou 1 hétéroatome de cycle supplémentaire choisi parmi O et S et l'hétérocycloalkyle étant éventuellement substitué par 1, 2, 3 ou 4 R¹².

10. Composé selon l'une quelconque des revendications 1 à 9, B étant 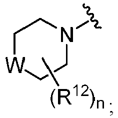
W étant NR¹³ ou CR¹⁴R¹⁴ ;
R¹³ étant choisi dans le groupe constitué par hydrogène, alkyle en C₁-C₆, alcényle en C₂-C₆, alcynyle en C₂-C₆ et - (CH₂)₀-₂-cycloalkyle en C₃-C₈, l'alkyle, l'alcényle, l'alcynyle étant éventuellement substitués par un ou plusieurs substituants halogéno, hydroxy, méthoxy ou NH₂ et le cycloalkyle étant éventuellement substitué par un ou plusieurs substituants halogéno, hydroxy, alkyle en C₁-C₆, alcoxy en C₁-C₆ ou NH₂ ;
chaque R¹⁴ étant indépendamment H ou R¹² ; et
n étant = 0, 1, 2, 3 ou 4 ; éventuellement :
(a) chaque R¹² étant indépendamment choisi dans le groupe constitué par halogène, hydroxy, hétérocycloalkyle monocyclique à 4 à 7 chaînons, hétéroalkyle en C₁-C₆ et alkyle en C₁-C₆, l'alkyle étant éventuellement substitué par un ou plusieurs substituants halogéno, hydroxy, méthoxy, cycloalkyle en C₃-C₈ ou NH₂ et l'hétérocycloalkyle étant éventuellement substitué par un ou plusieurs substituants halogéno, hydroxy, méthoxy ou alkyle en C₁-C₆ ; et
R¹³ étant H ou alkyle en C₁-C₆ non substitué ; éventuellement chaque R¹² étant indépendamment alkyle en C₁-C₆ ; et/ou
(b) B étant 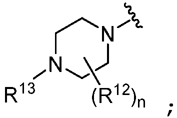 éventuellement R¹³ étant H ou alkyle en C₁-C₆ non substitué ; et/ou chaque R¹² étant indépendamment alkyle en C₁-C₆.

11. Composé selon l'une quelconque des revendications 1 à 8, B étant un hétérocycloalkyle bicyclique à 6 à 14 chaînons comprenant 1, 2 ou 3 atomes d'azote de cycle au total et 0 ou 1 hétéroatome de cycle supplémentaire choisi parmi O et S et l'hétérocycloalkyle étant éventuellement substitué par 1, 2, 3 ou 4 R¹² ; éventuellement chaque R¹² étant indépendamment alkyle en C₁-C₆.

12. Composé selon l'une quelconque des revendications 1 à 7, B étant NR¹⁰R¹¹ et R¹¹ étant hydrogène, alkyle en C₁-₇, halogénoalkyle en C₁₋₇ ou cycloalkyle en C₃₋₈.

13. Composé selon l'une quelconque des revendications 1 à 7 ou 12 :
(a) R¹¹ étant H ou alkyle en C₁₋₇ et R¹⁰ étant hétéroalkyle en C₁-C₈ comprenant au moins un atome d'azote ; ou
(b) R¹⁰ étant - (CH₂)₀-₃-hétérocycloalkyle comprenant au moins 1 atome d'azote de cycle, chaque R¹⁰ étant éventuellement substitué par 1 à 6 R¹².

14. Composé selon la revendication 1, le composé étant :
(a) de formule (Ig) 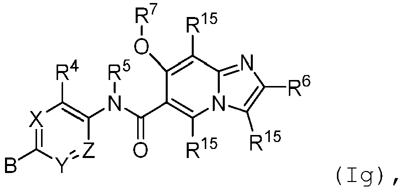 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(b) de formule (Ih) 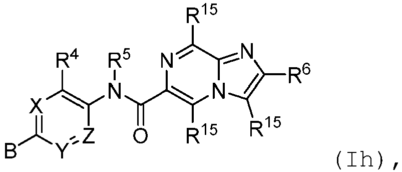 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(c) de formule (Ii) 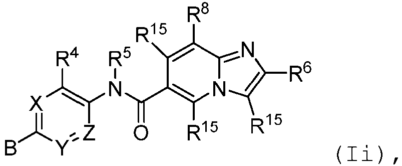 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(d) de formule (Ij) 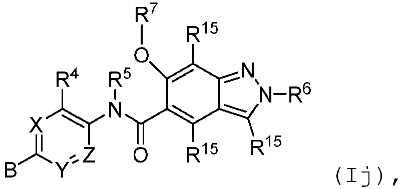 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(e) de formule (Ik) 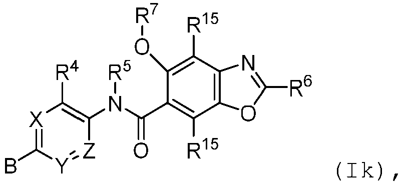 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(f) de formule (Im) 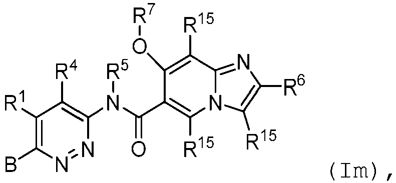 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(g) de formule (In) 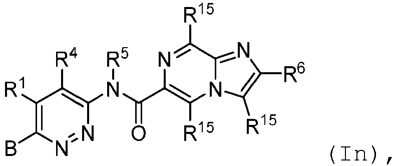 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(h) de formule (Io) 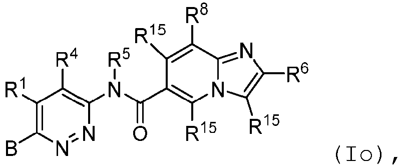 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(i) de formule (Ip) 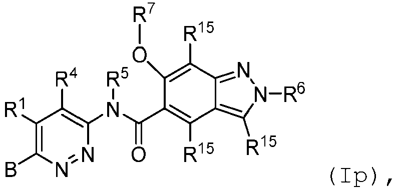 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(j) de formule (Iq) 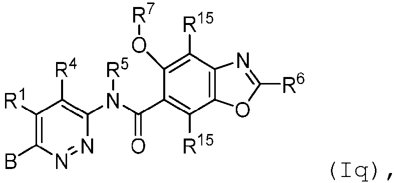 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(k) de formule (Ir) 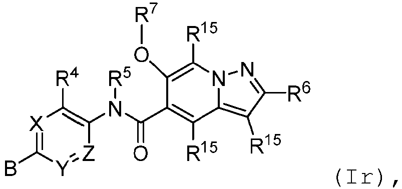 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(l) de formule (Is) 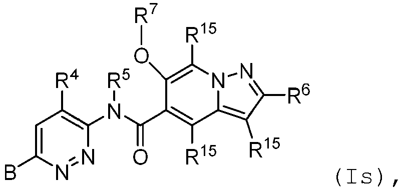 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ;
(m) de formule (It) 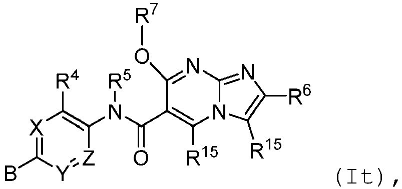 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H ; ou
(n) de formule (Iu) 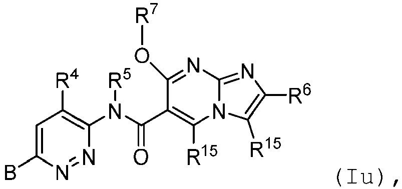 ou un sel pharmaceutiquement acceptable correspondant, chaque R¹⁵ étant indépendamment H, halogène, alkyle en C₁-C₆ ou halogénoalkyle en C₁-C₆ ; éventuellement R⁵ étant H.

15. Composé selon l'une quelconque des revendications 1 à 14, choisi parmi :

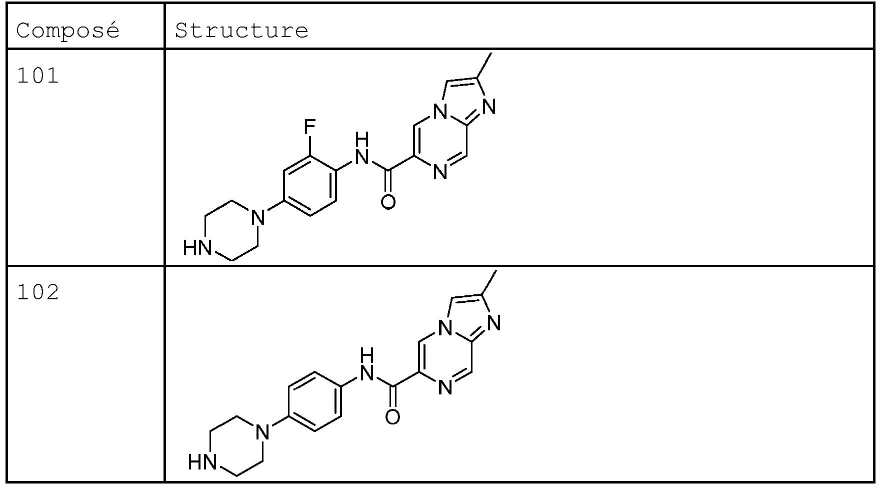

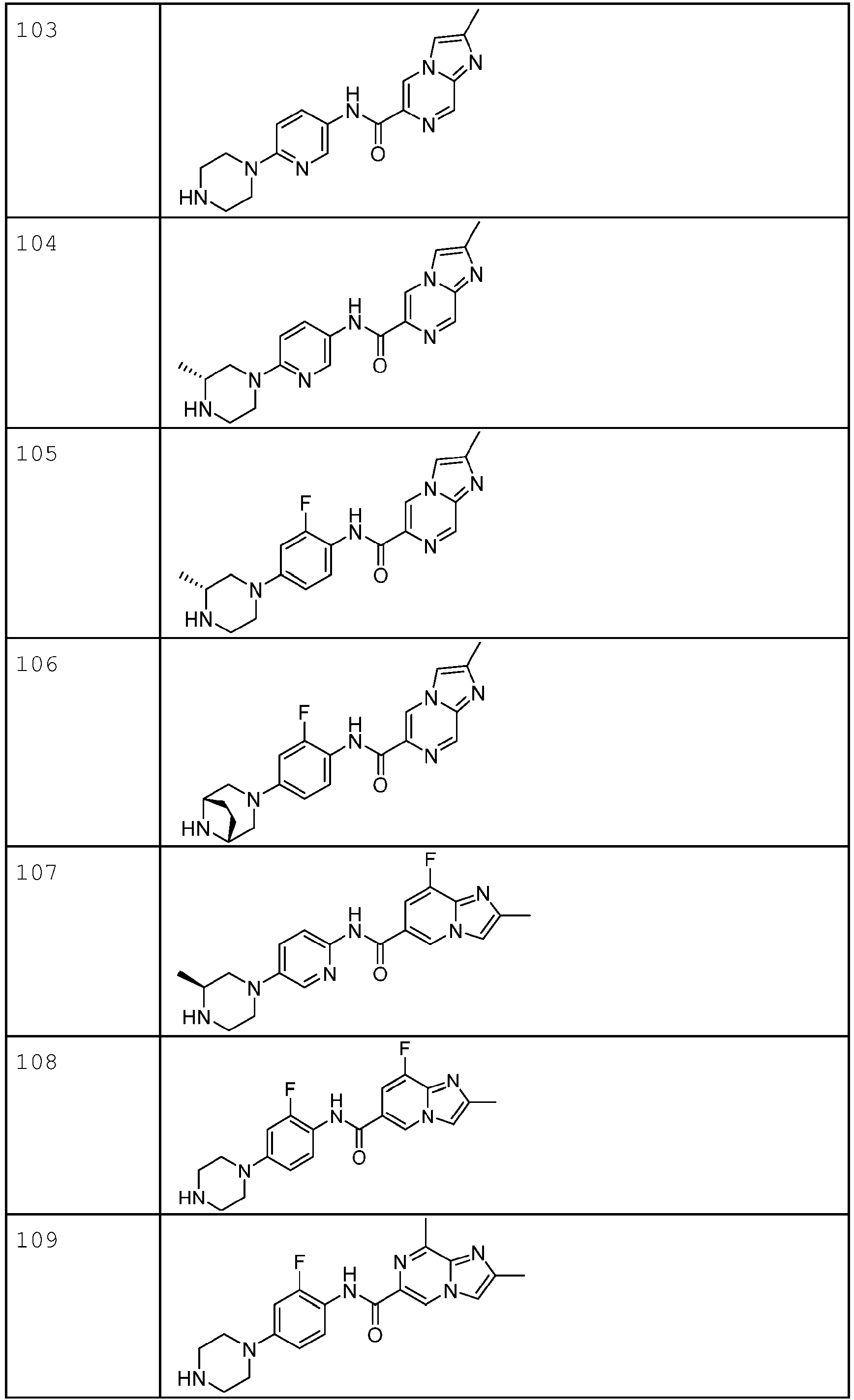

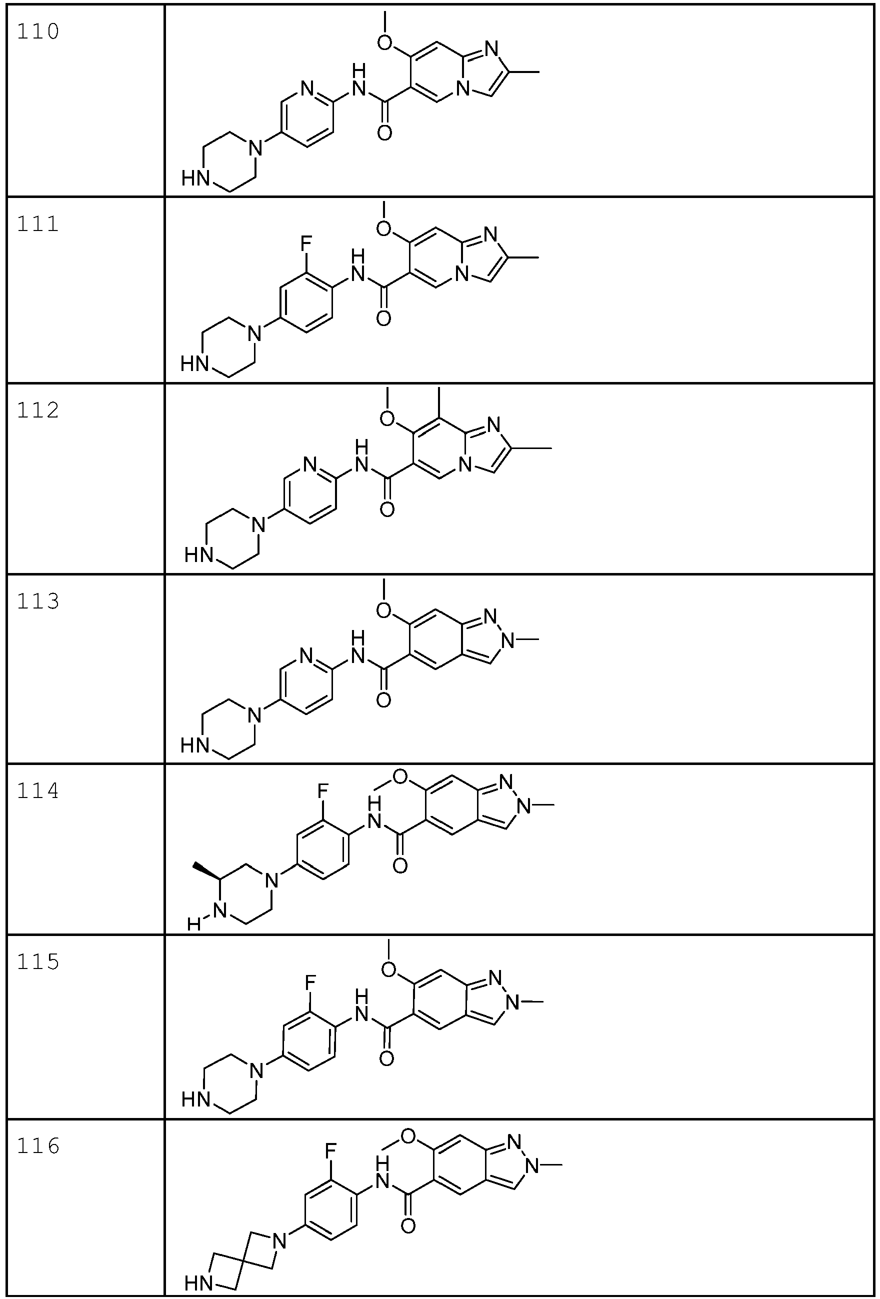

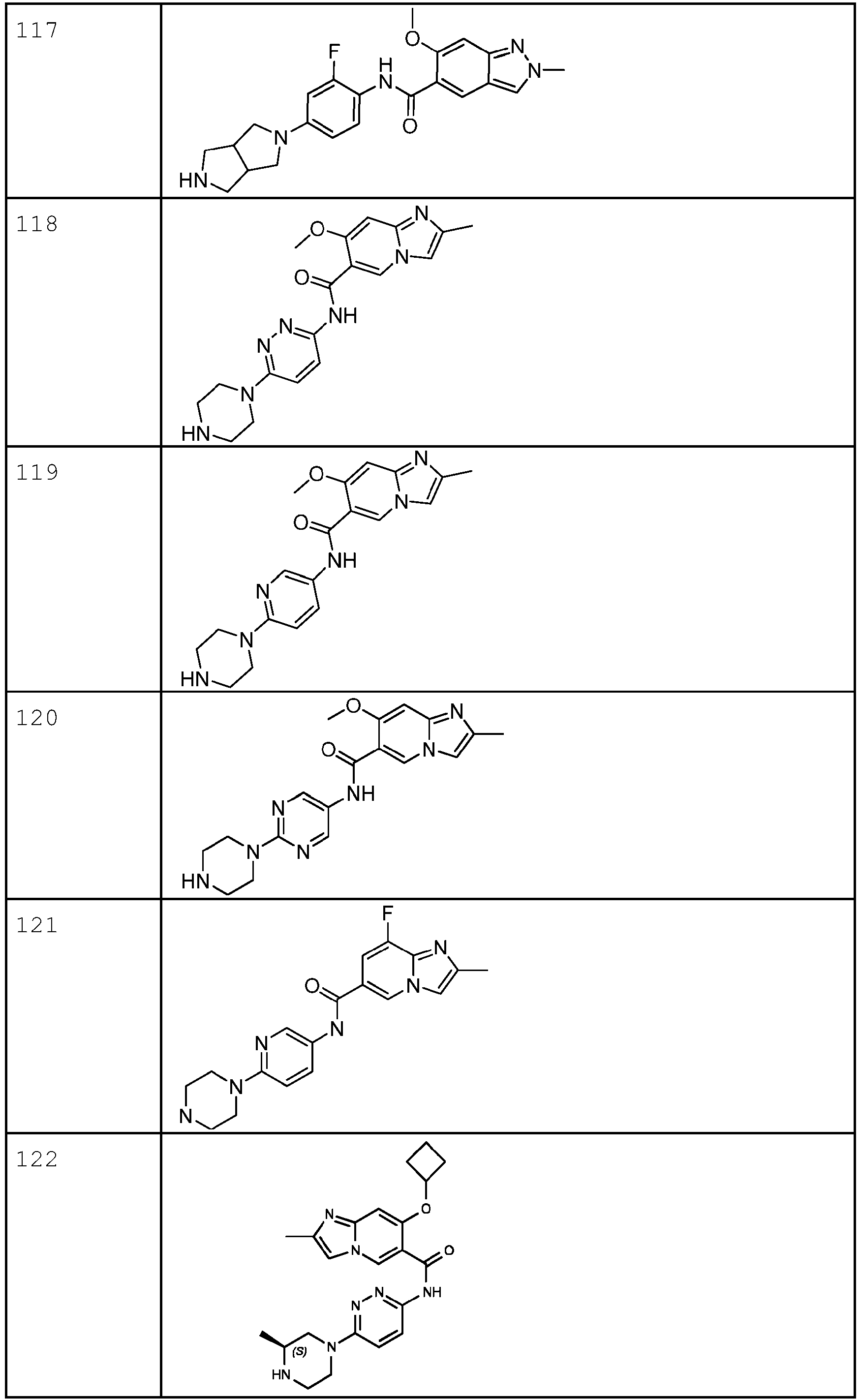

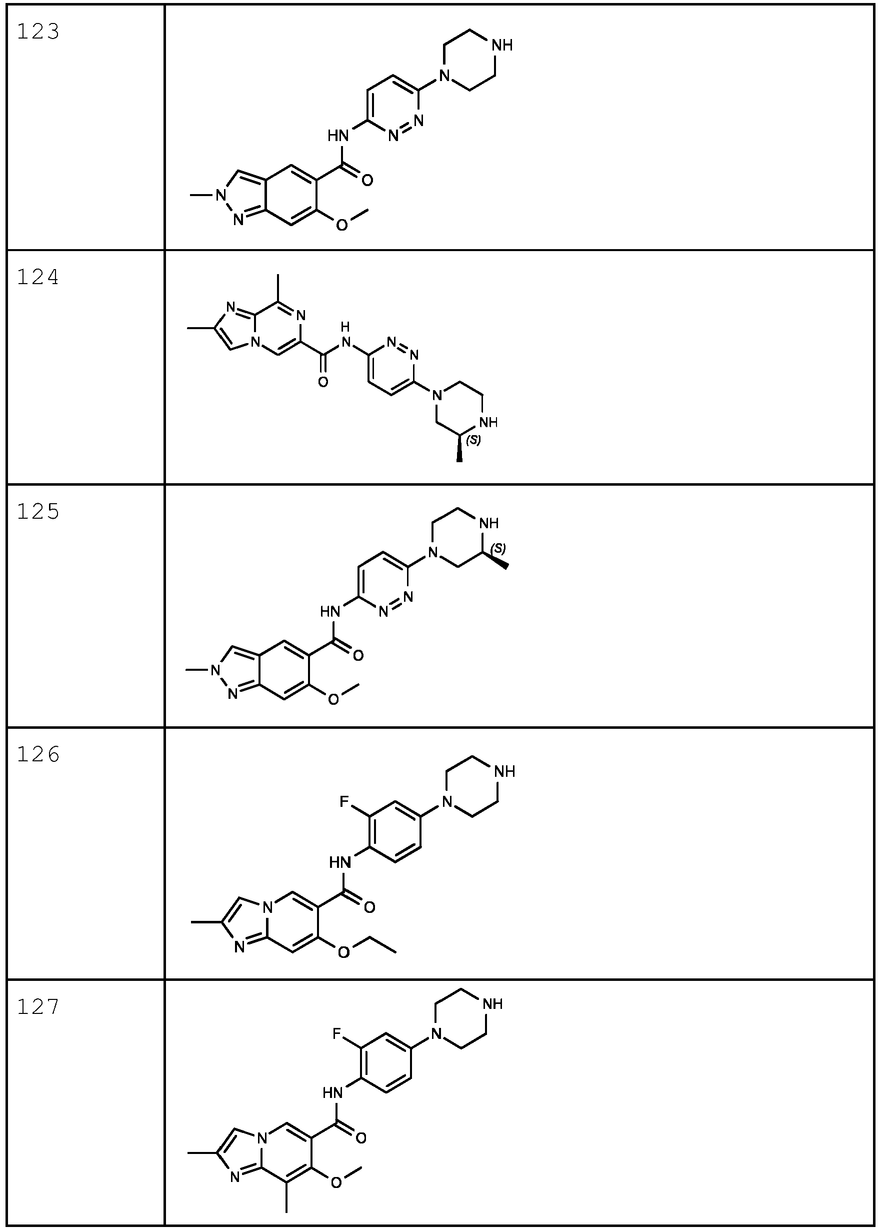

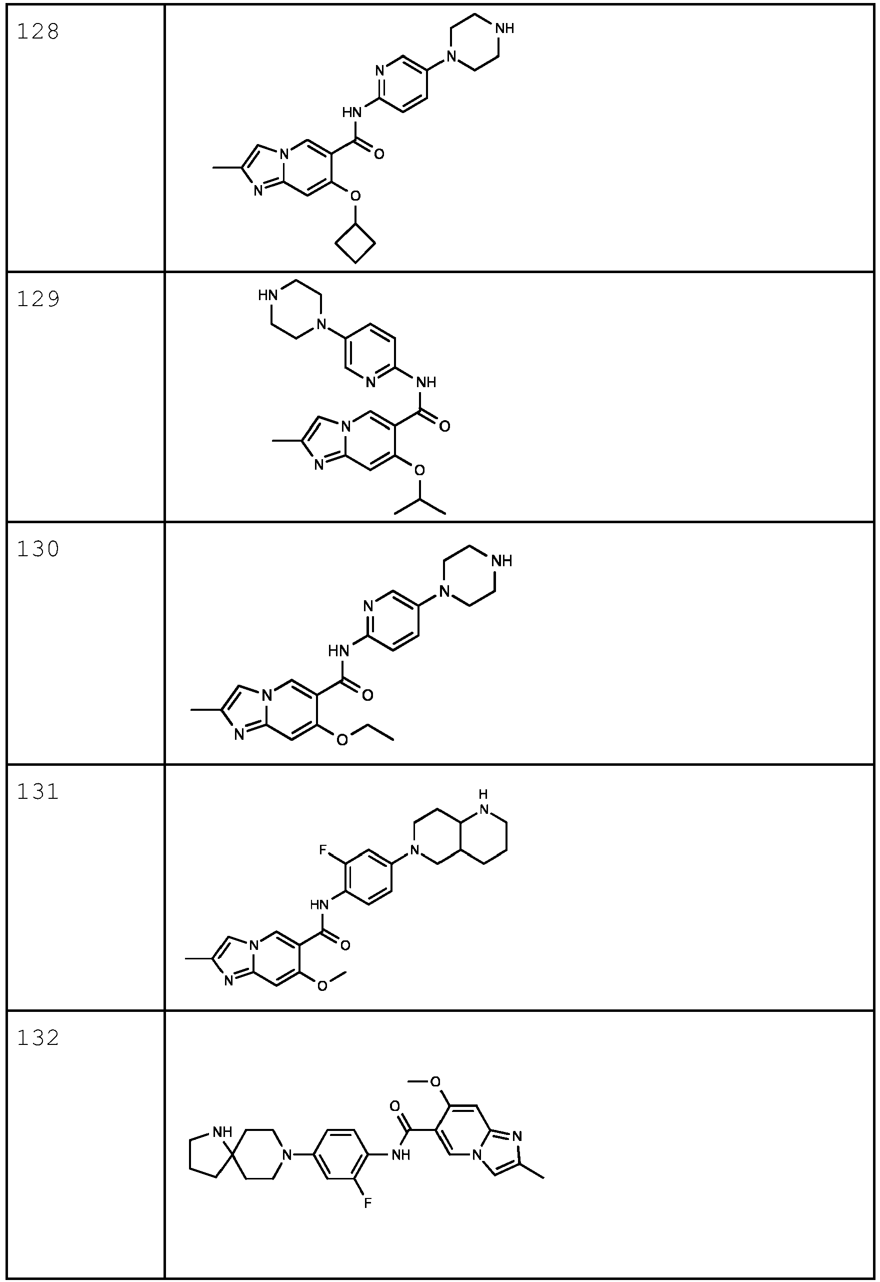

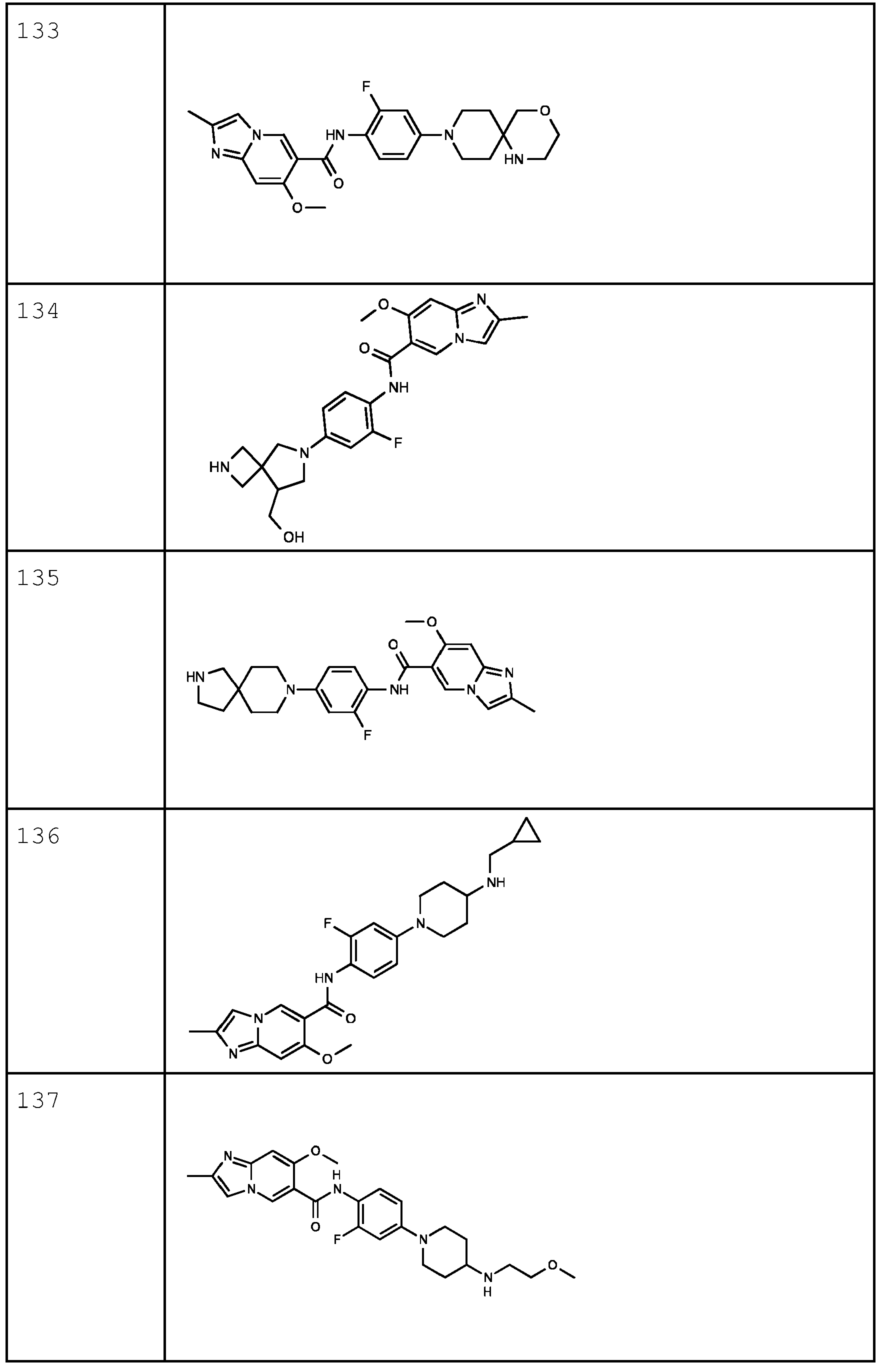

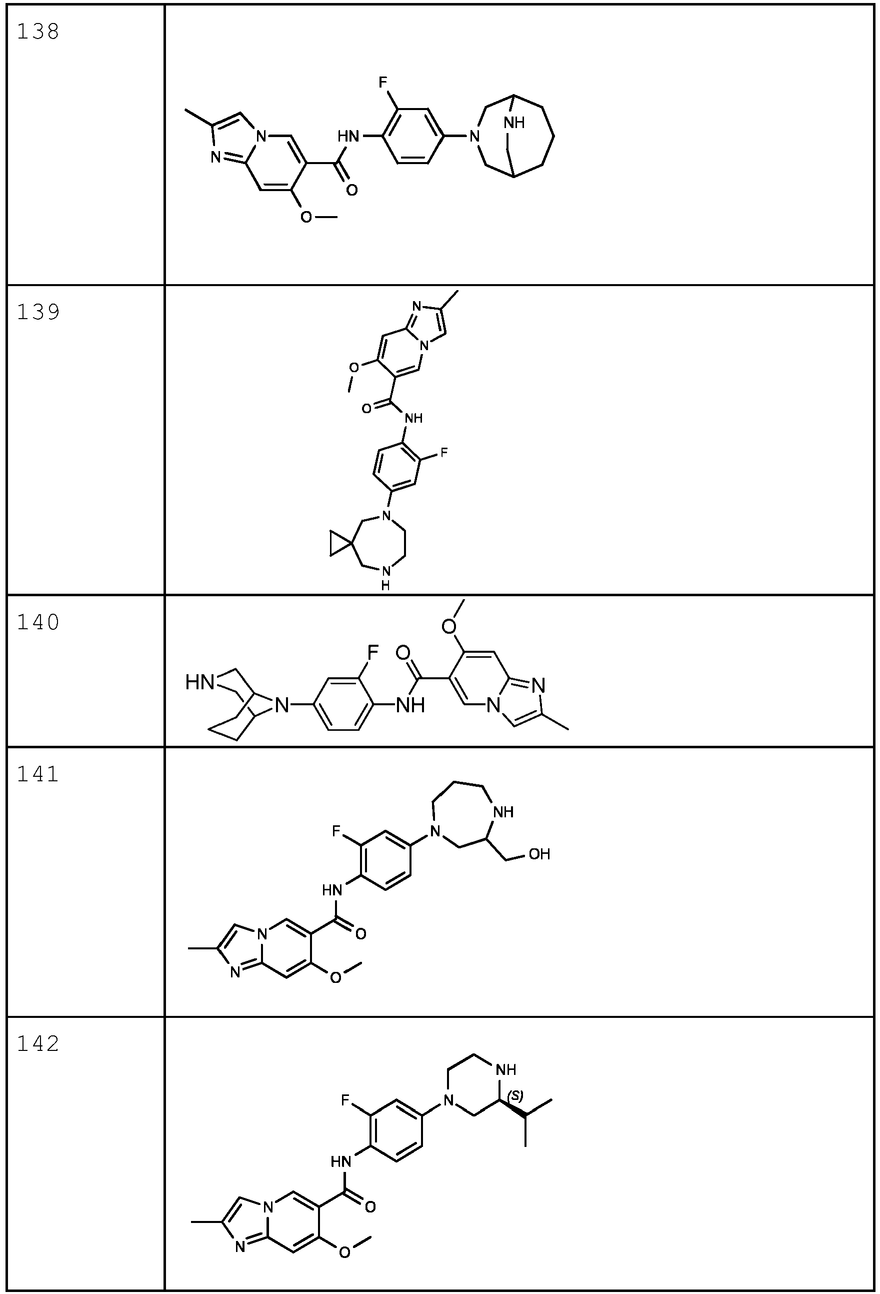

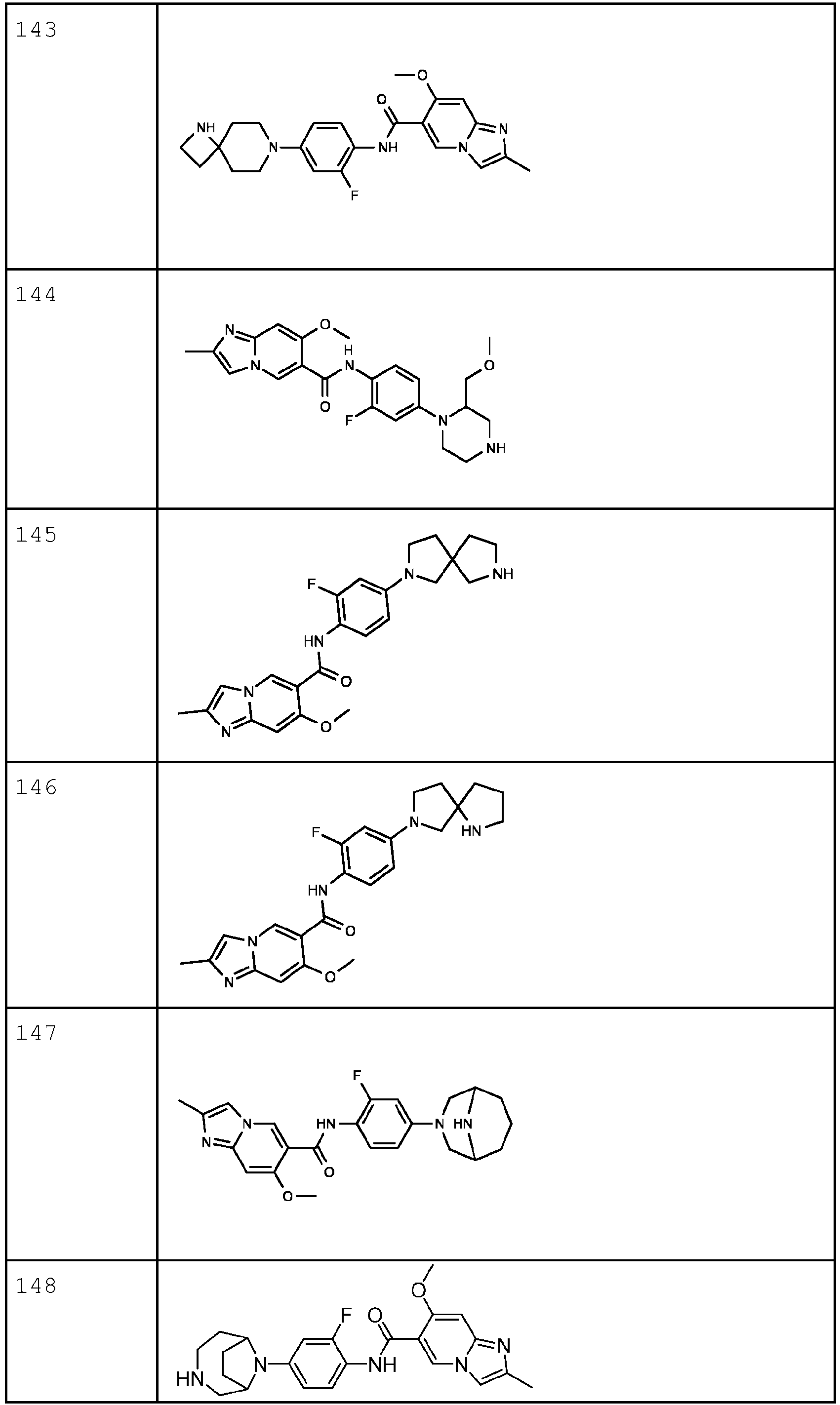

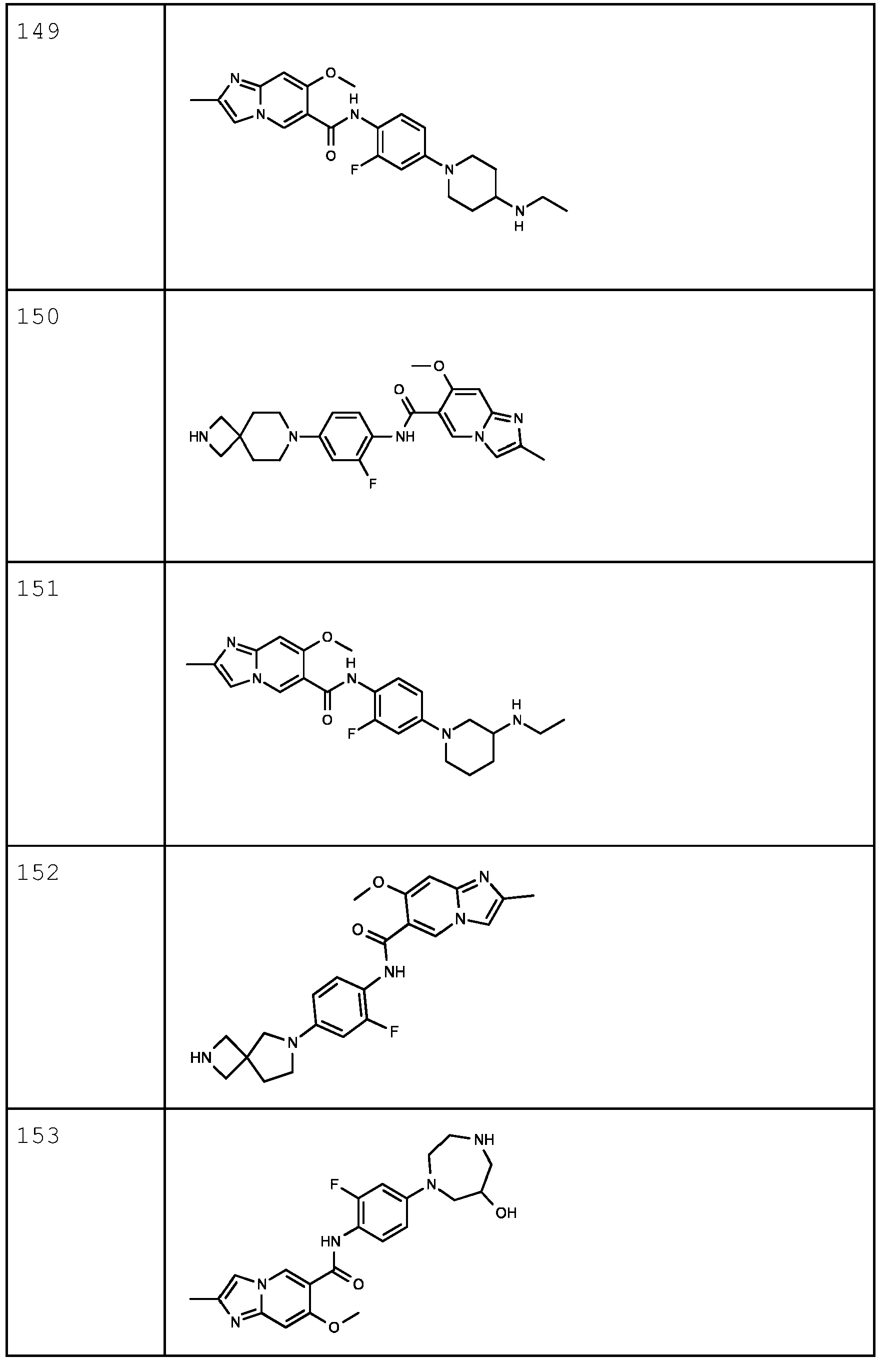

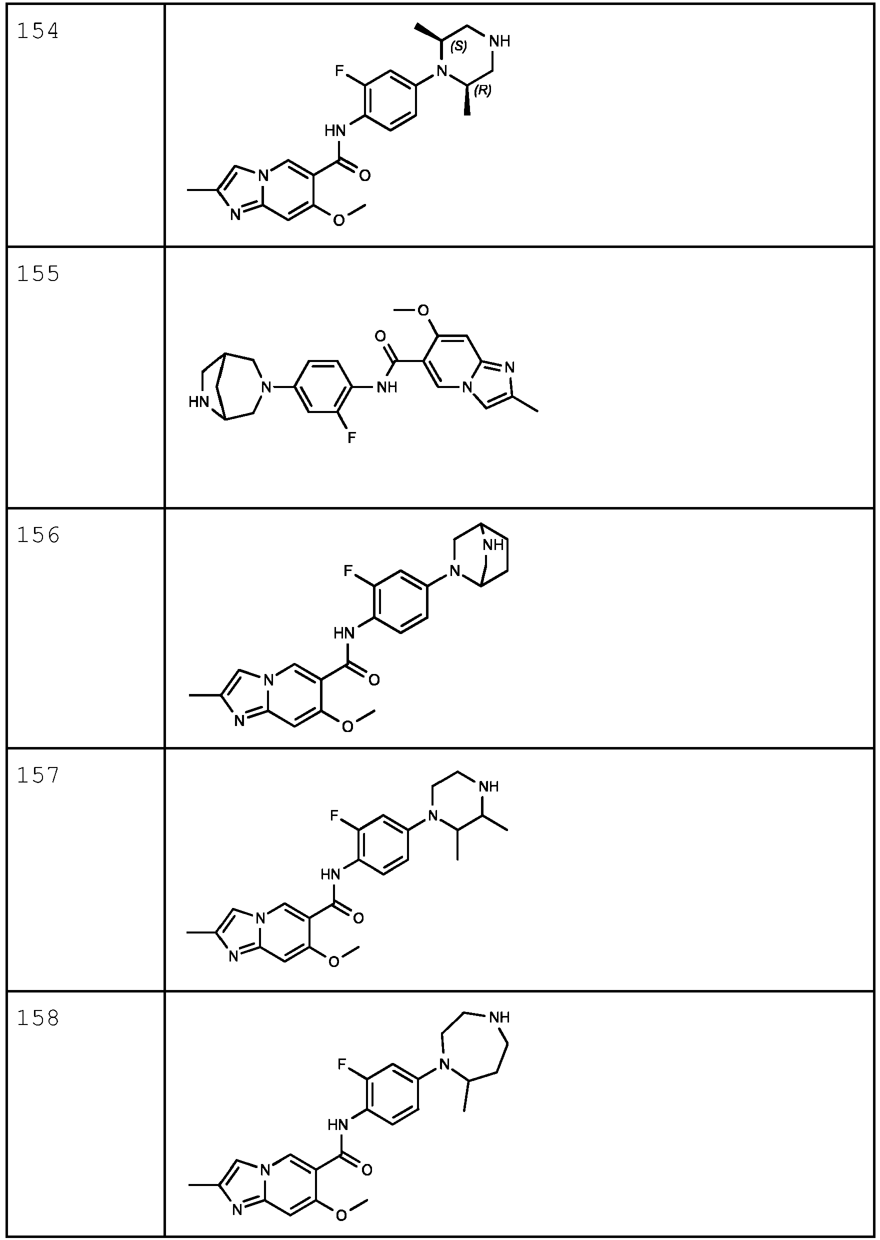

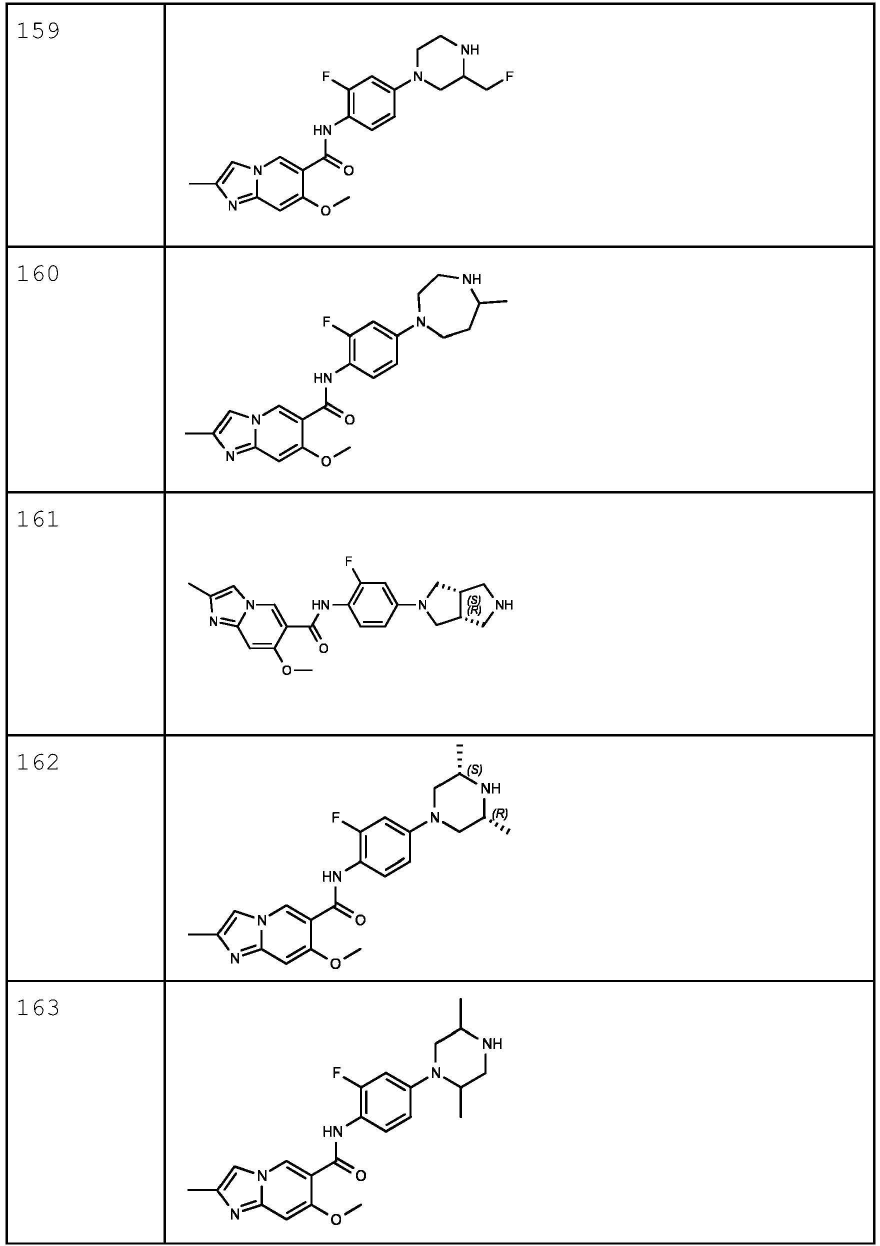

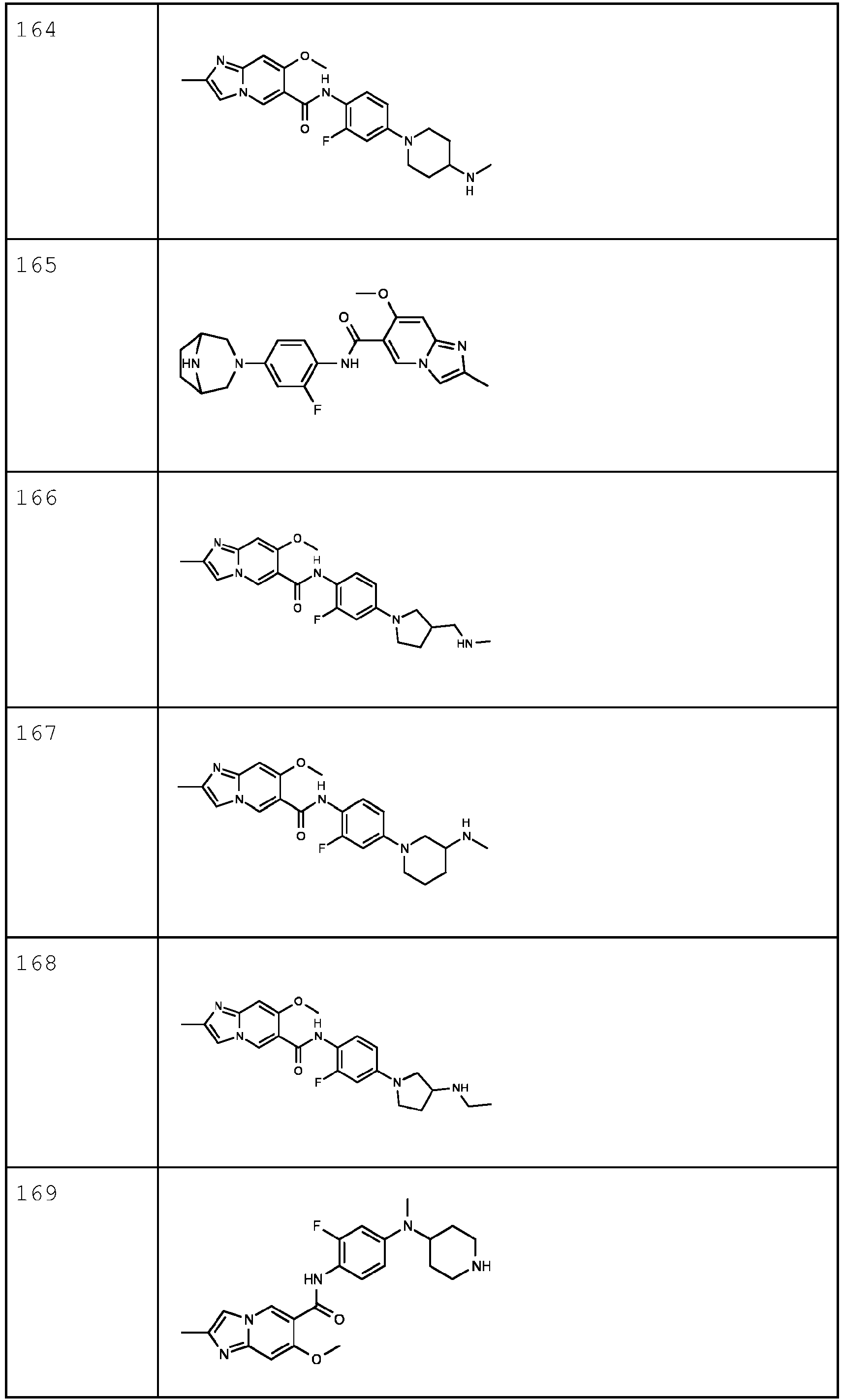

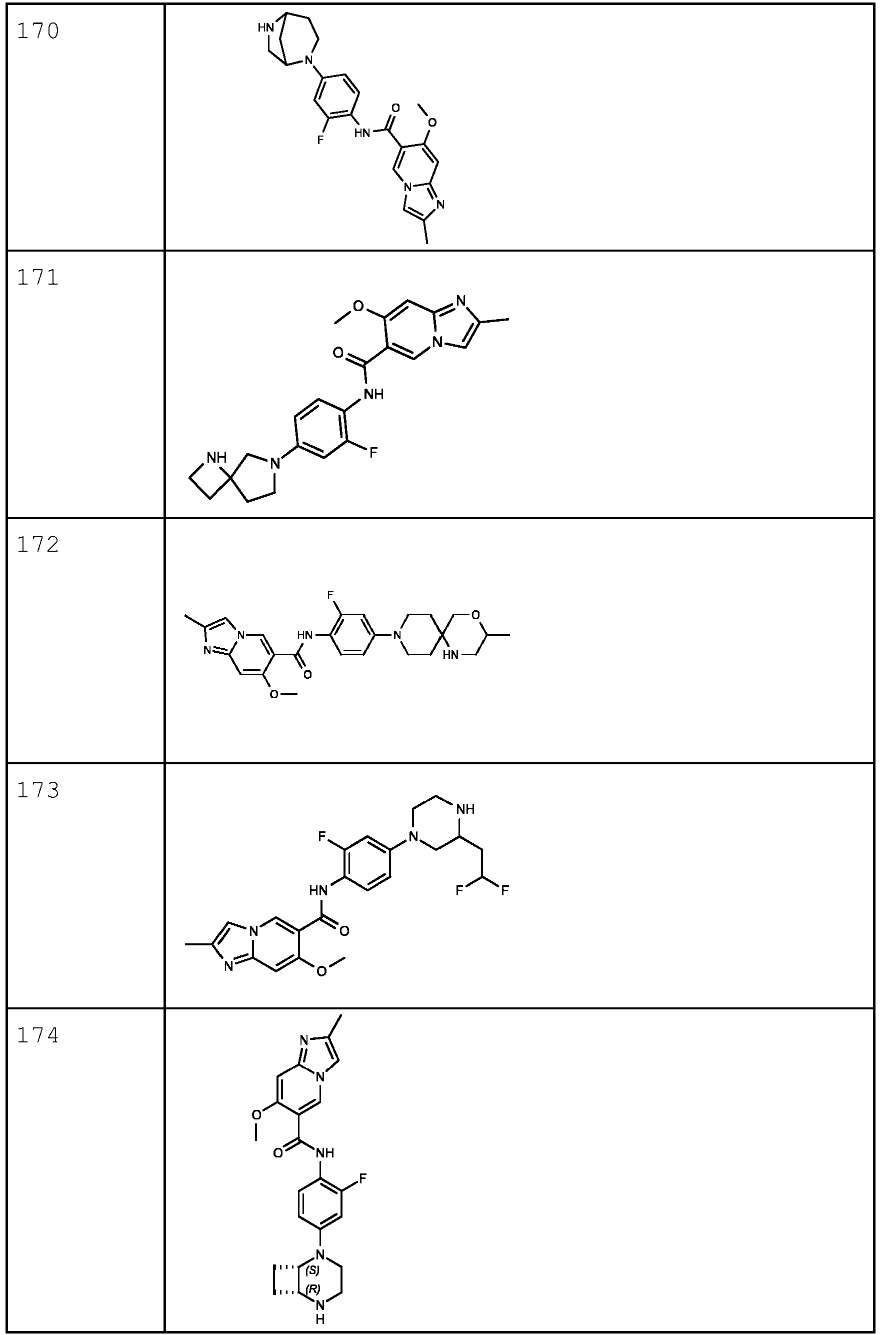

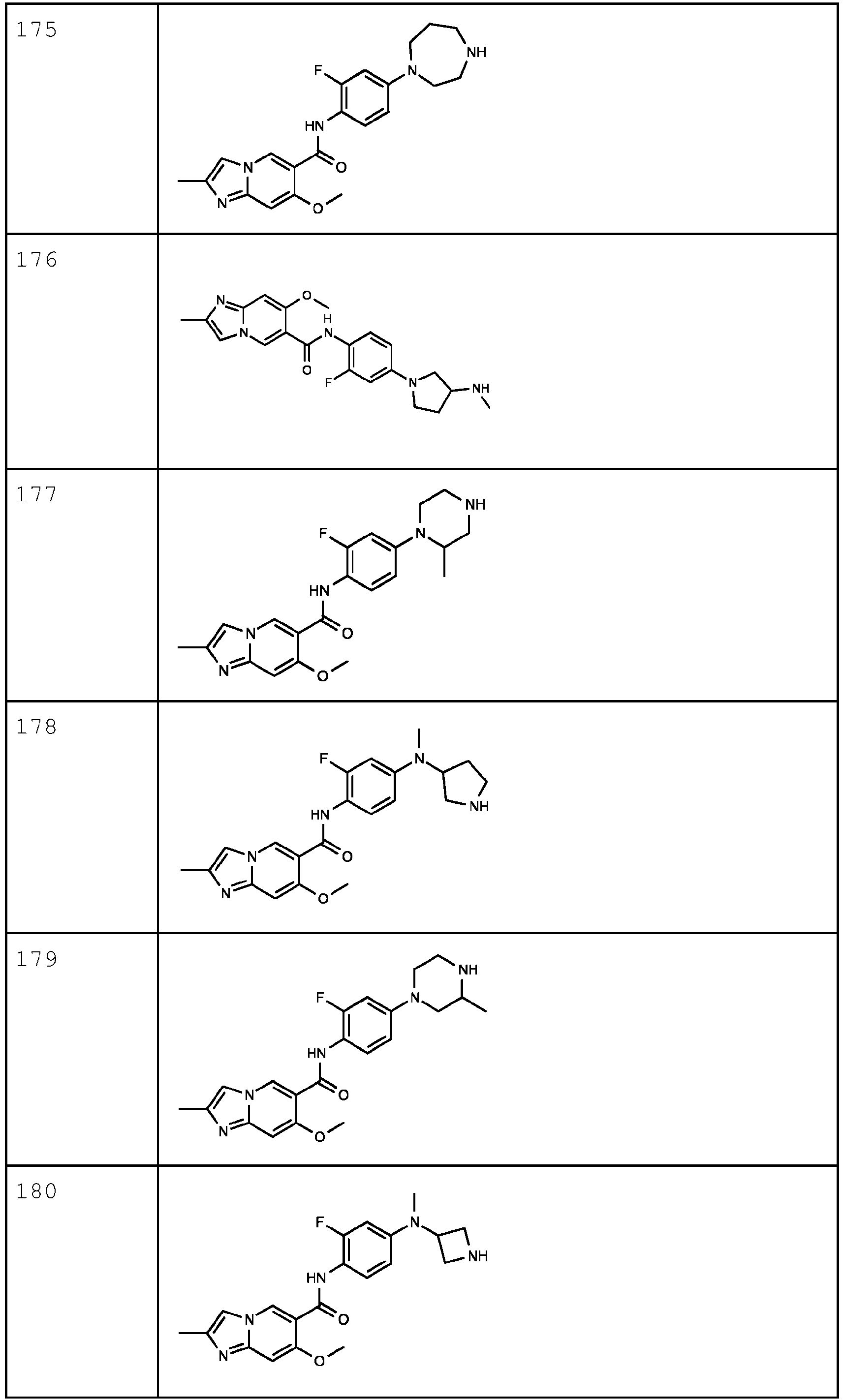

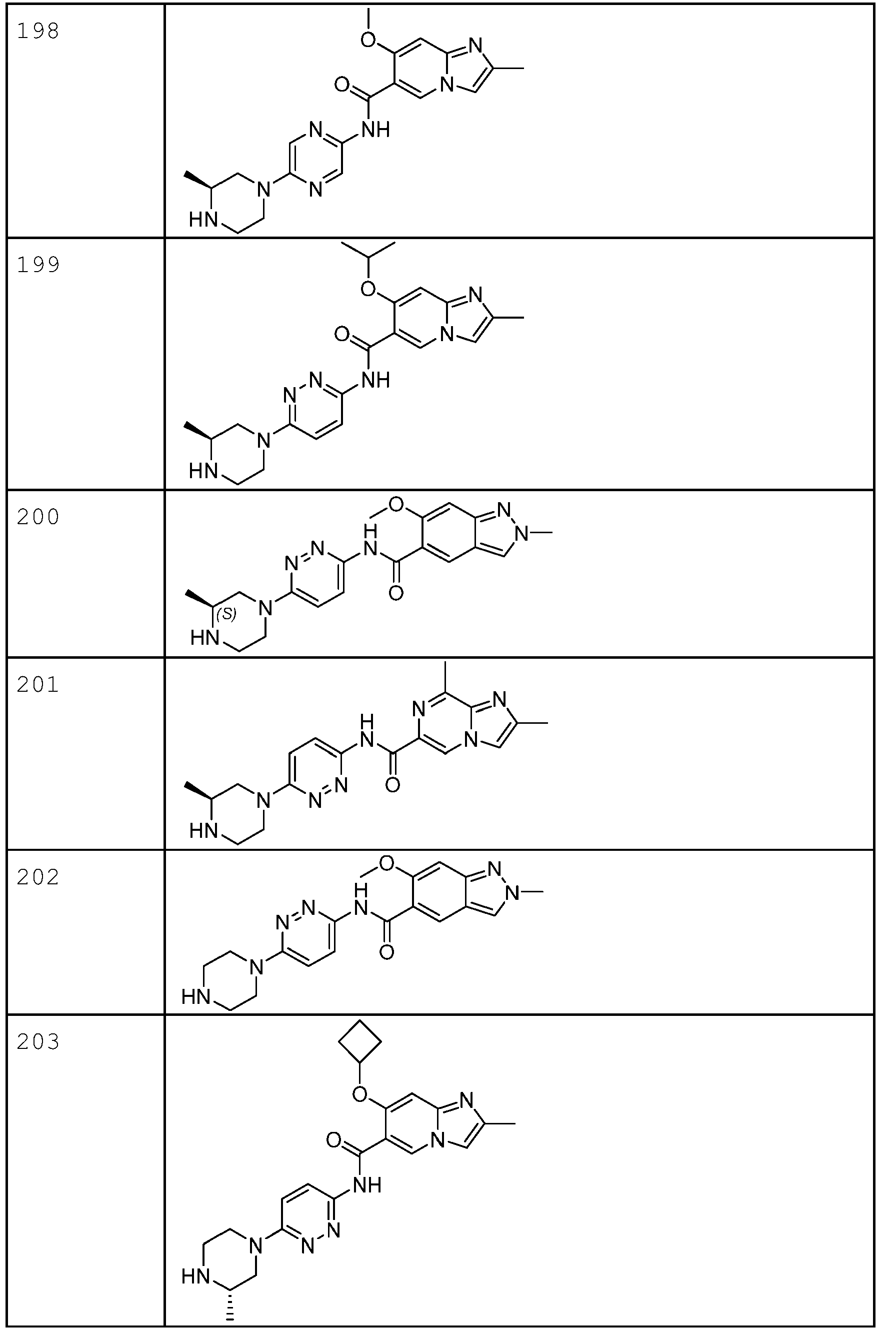

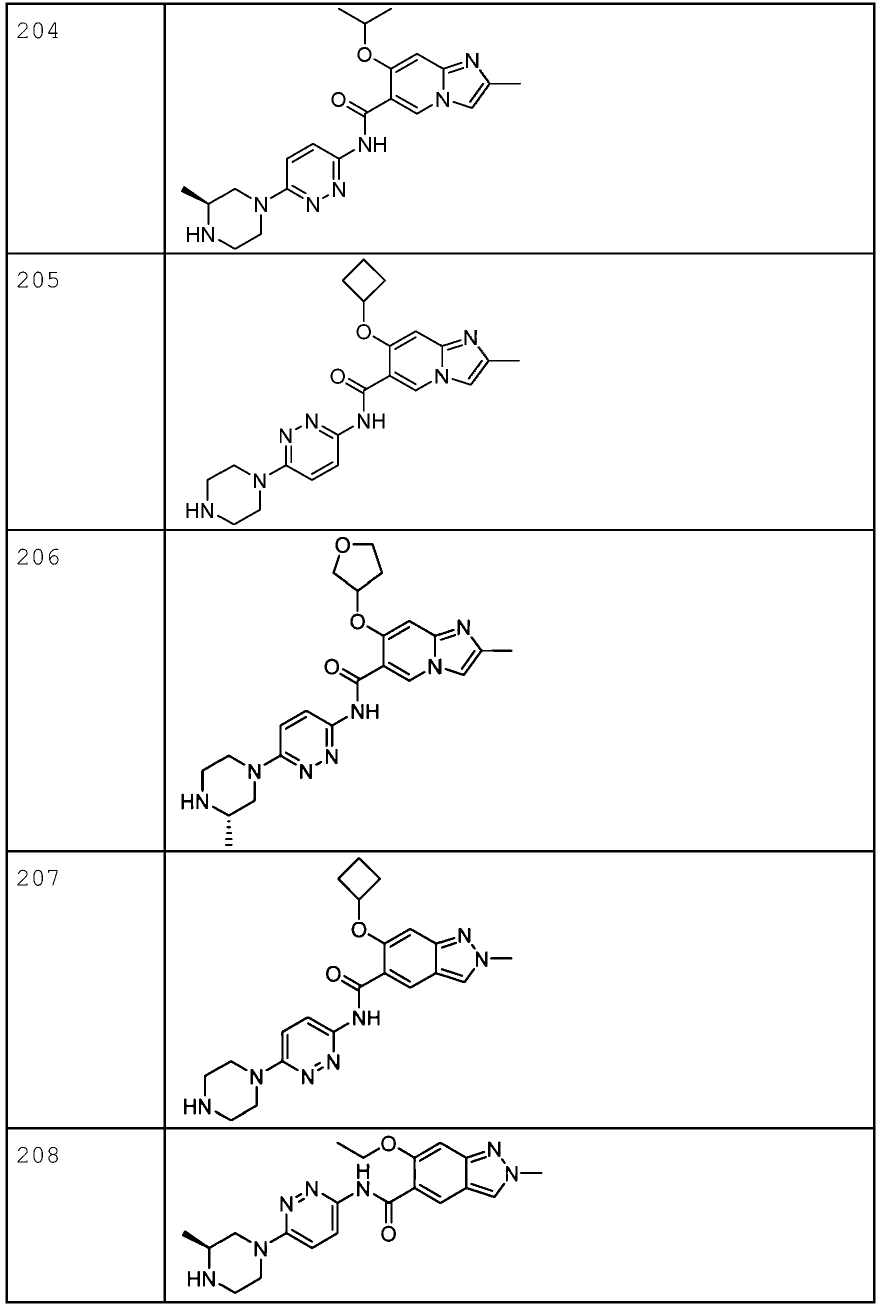

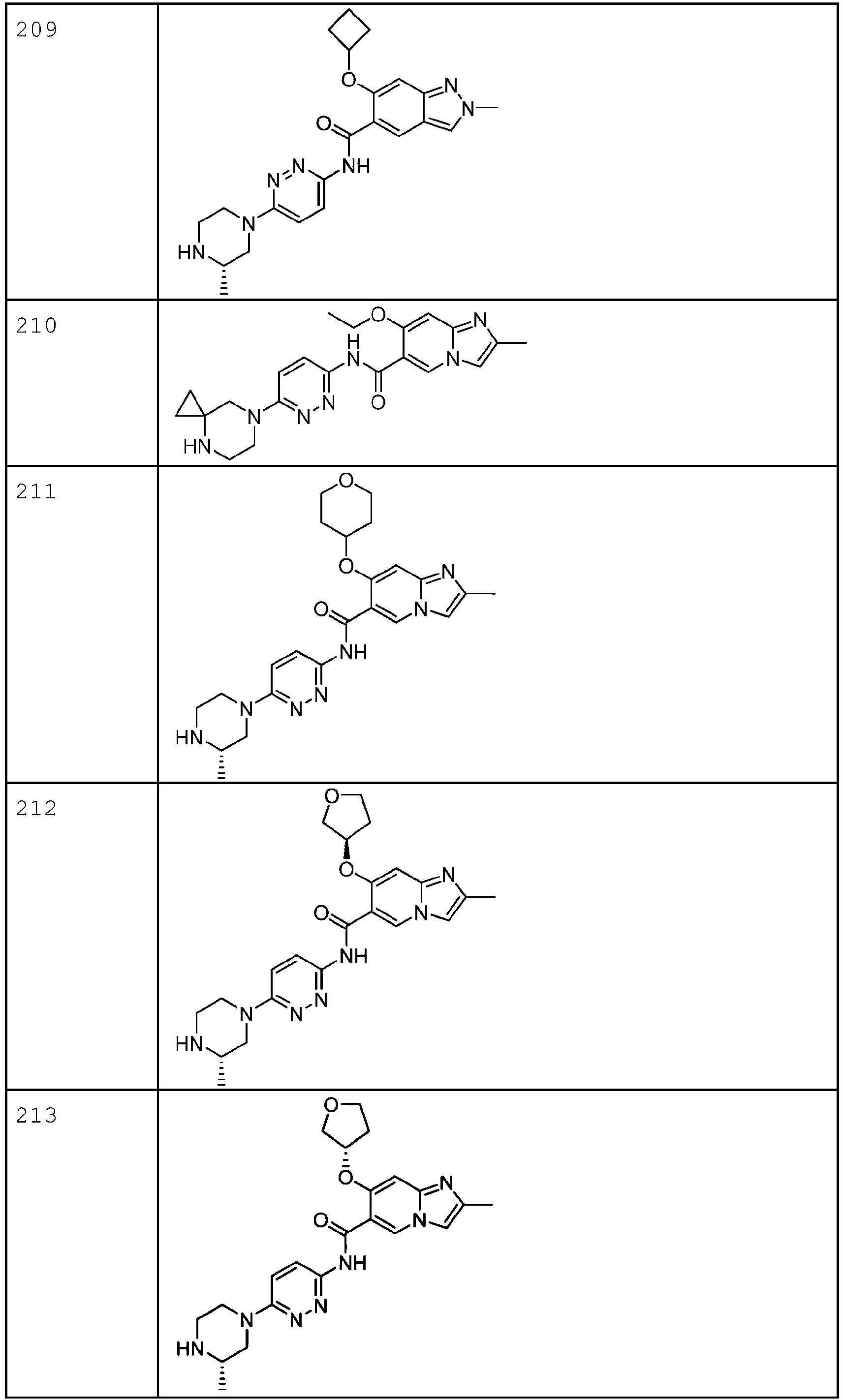

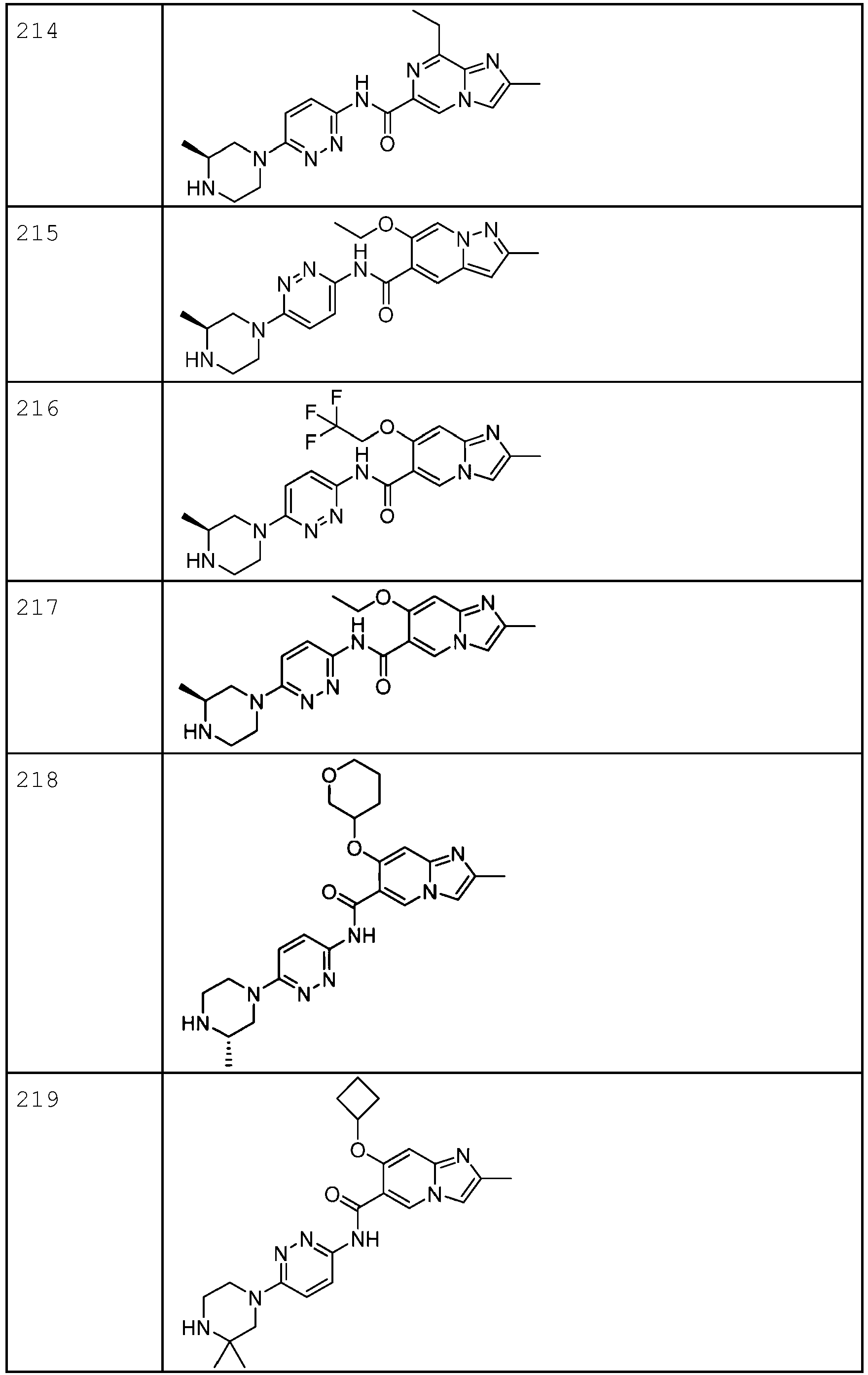

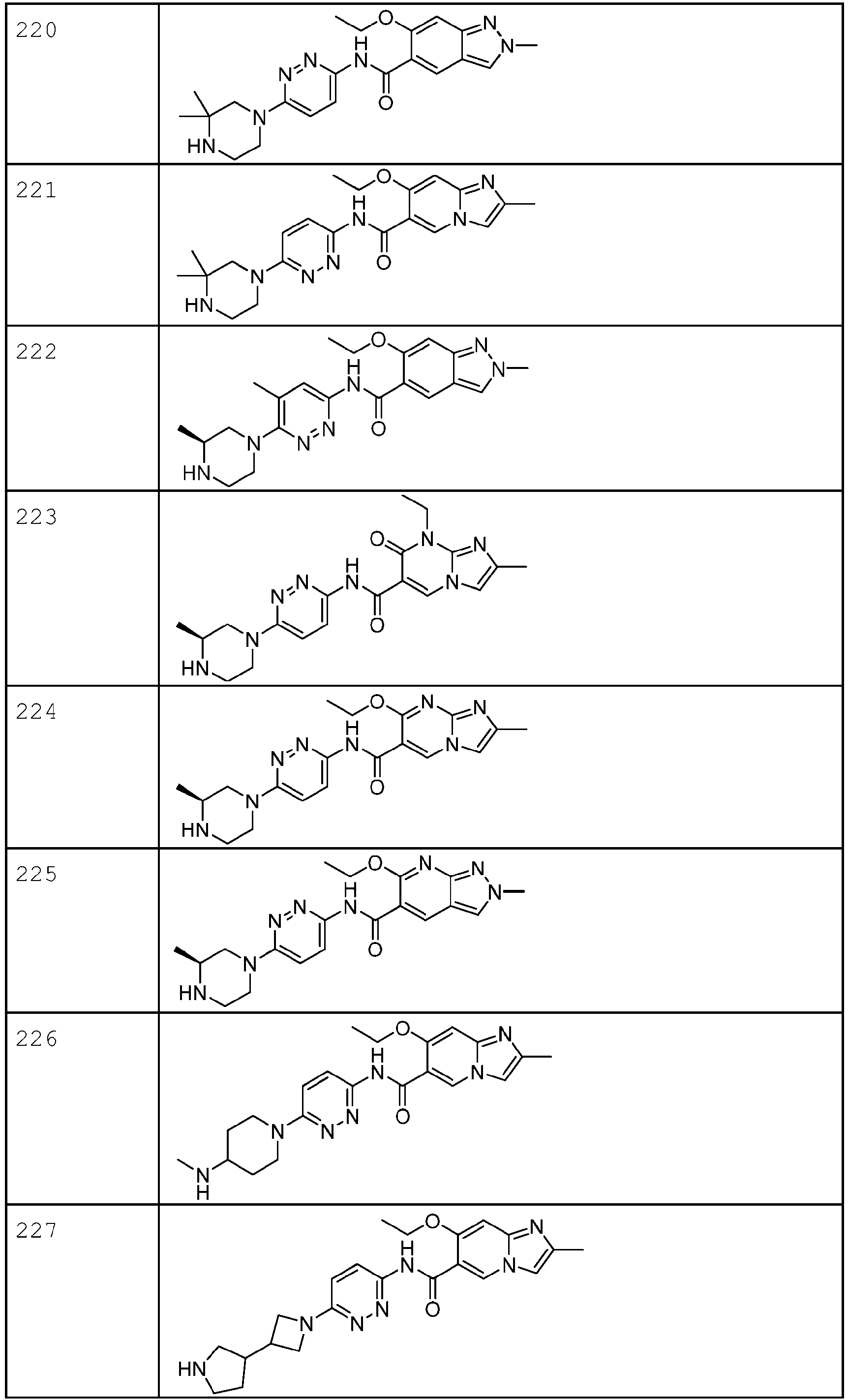

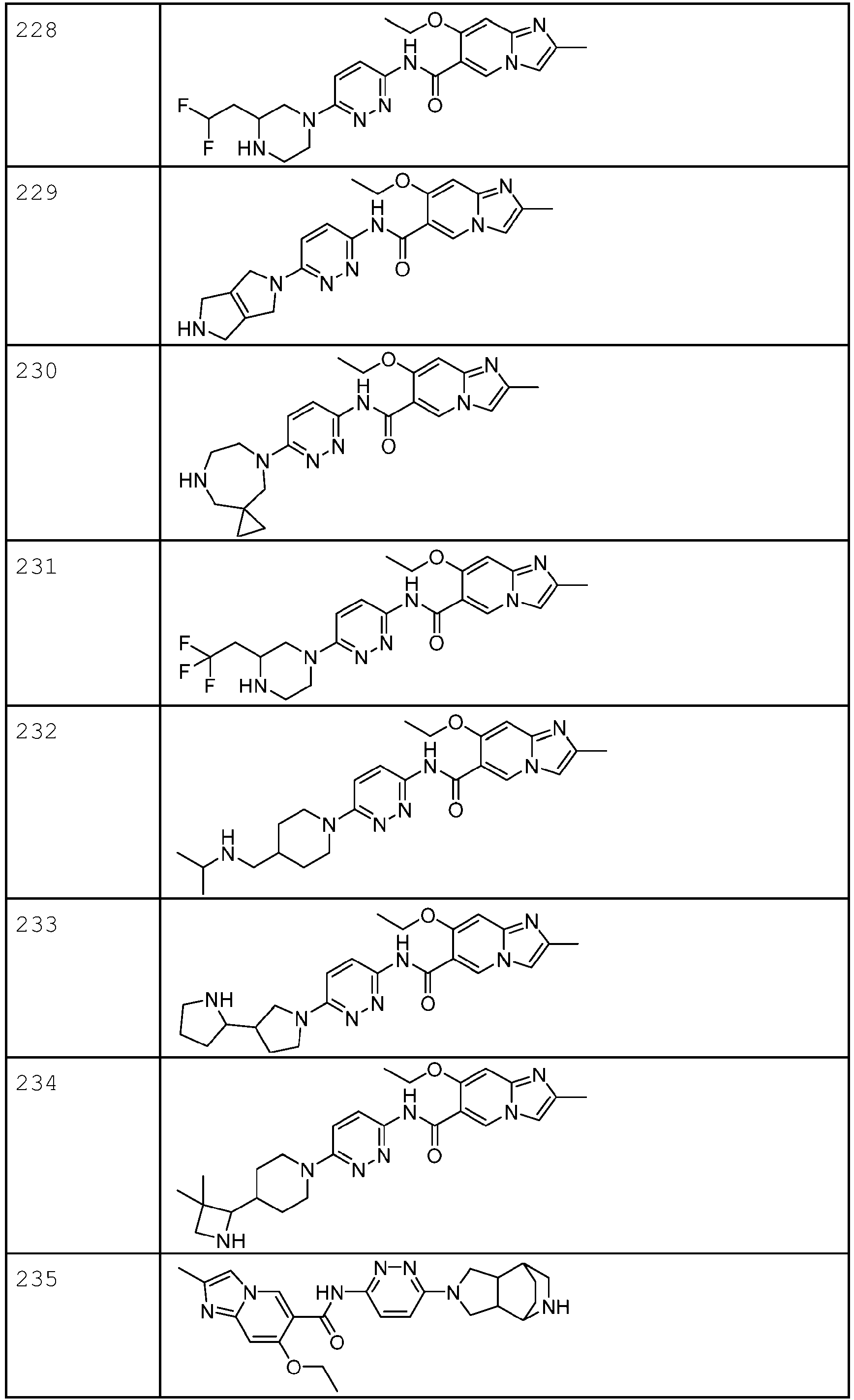

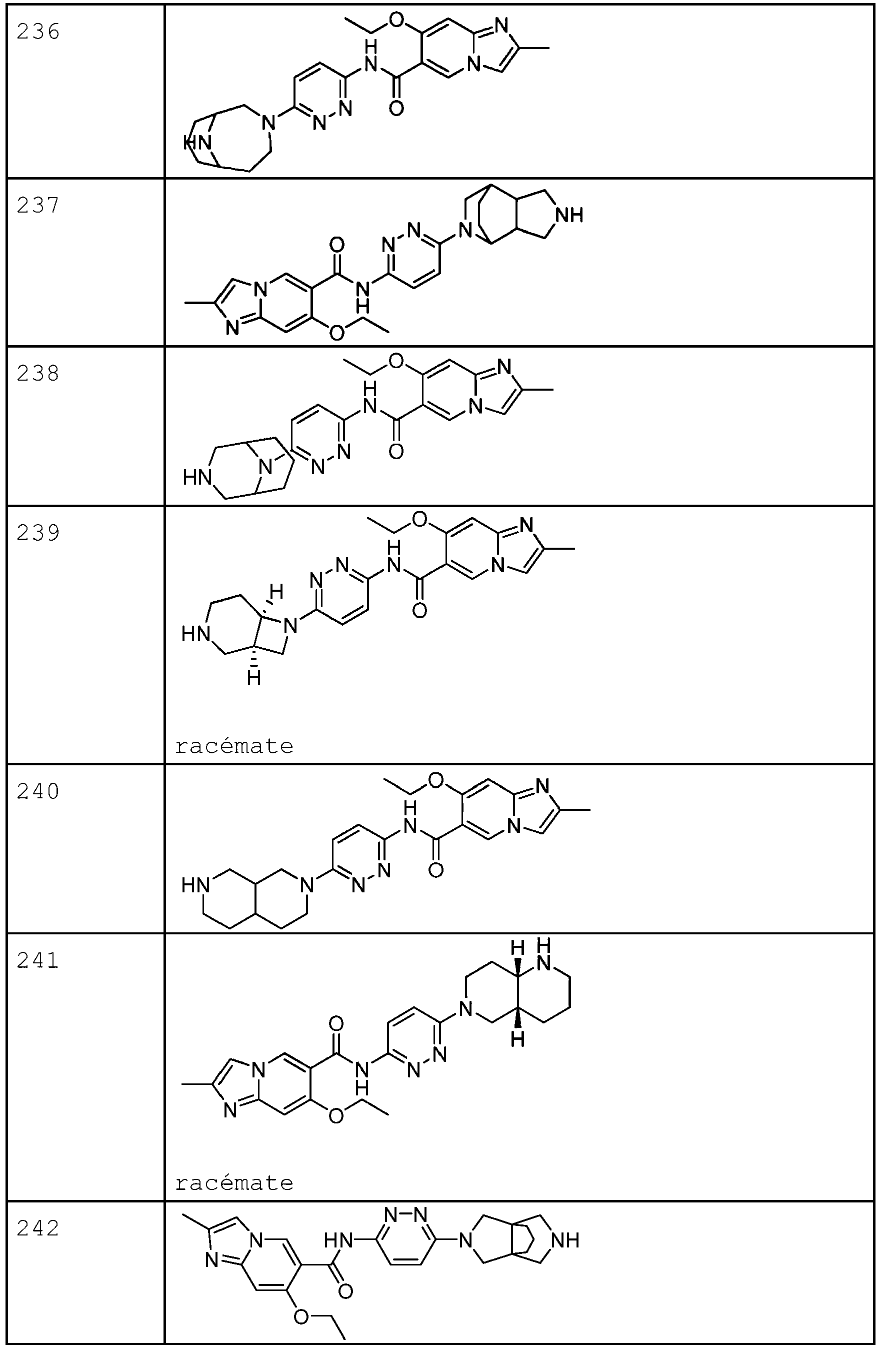

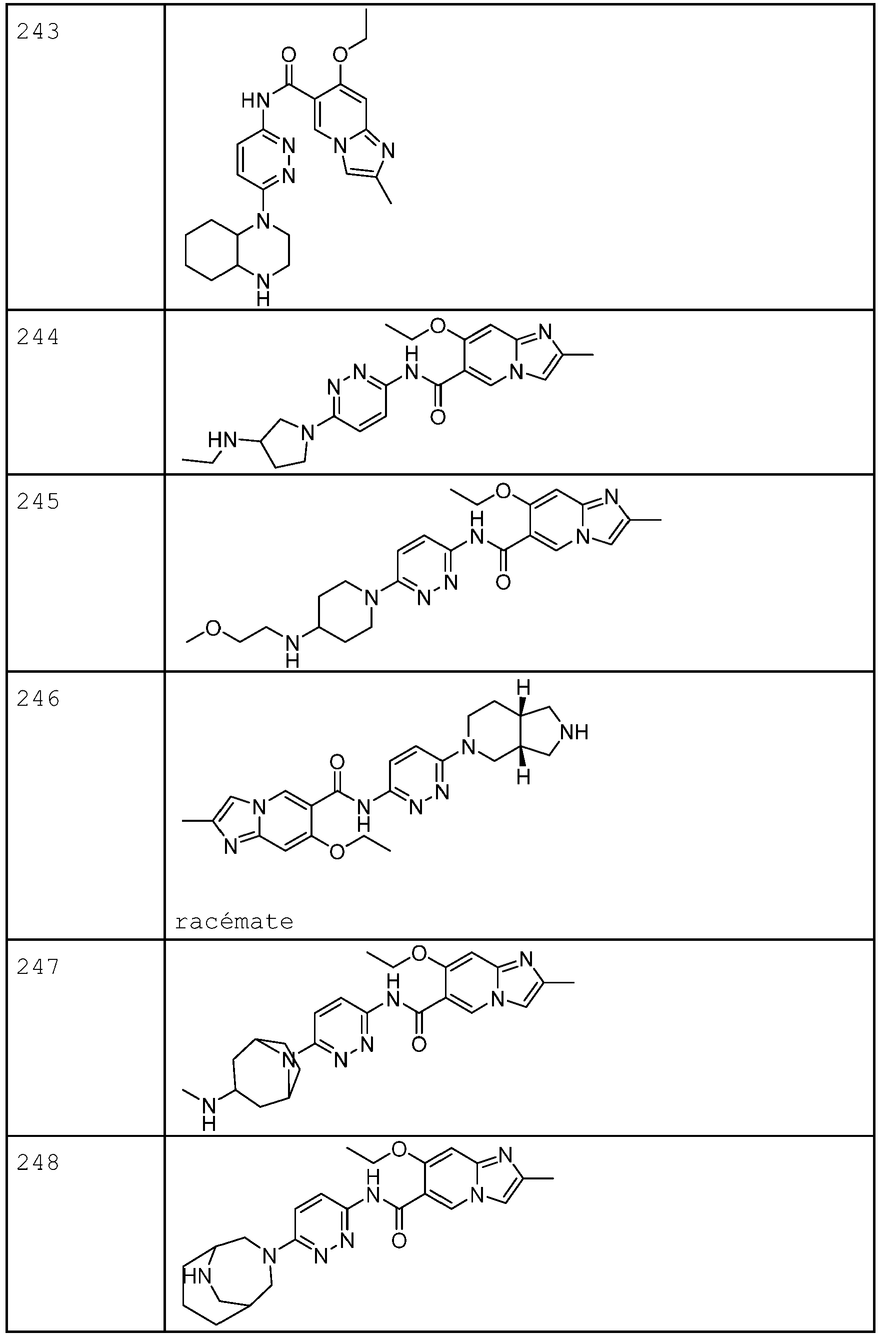

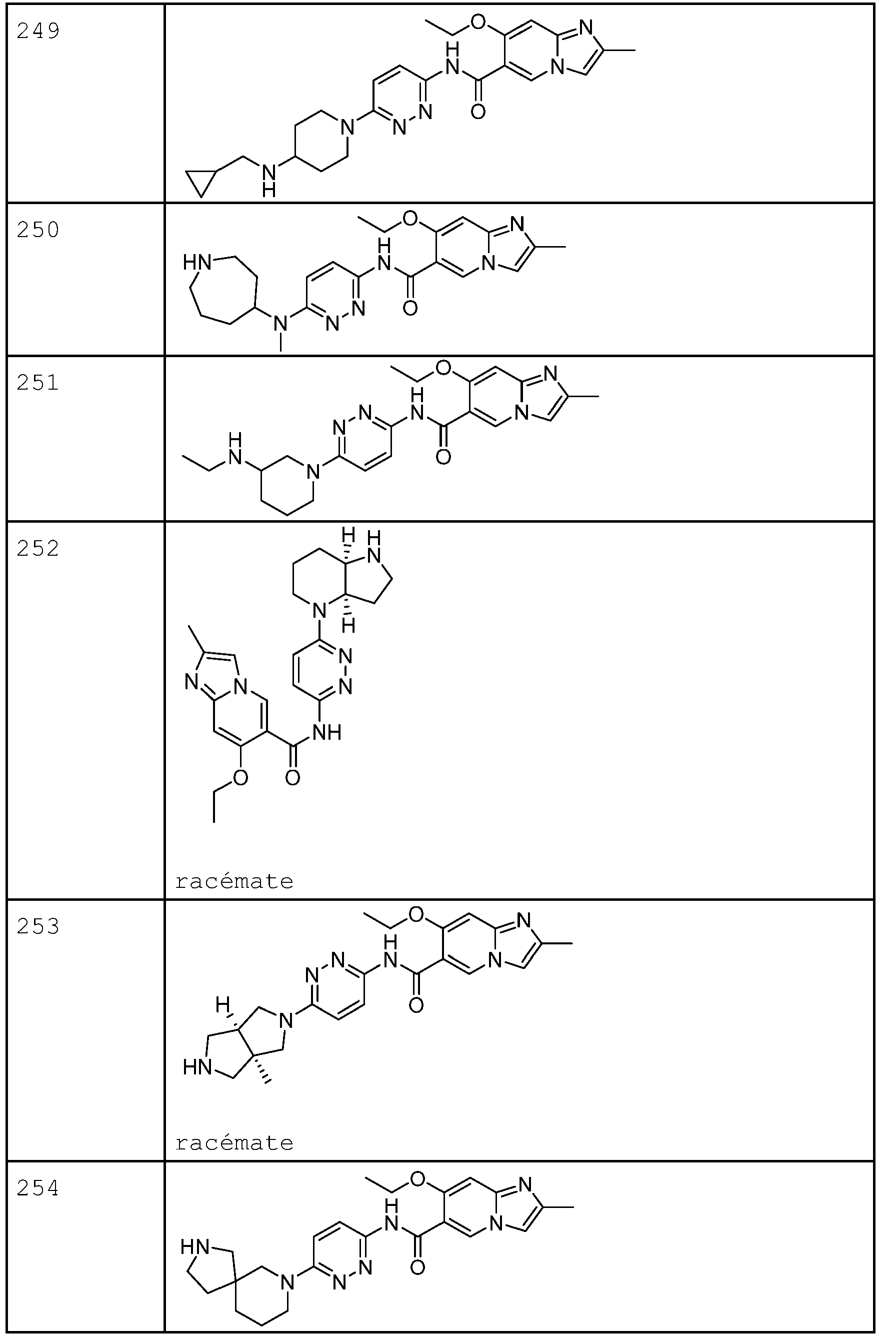

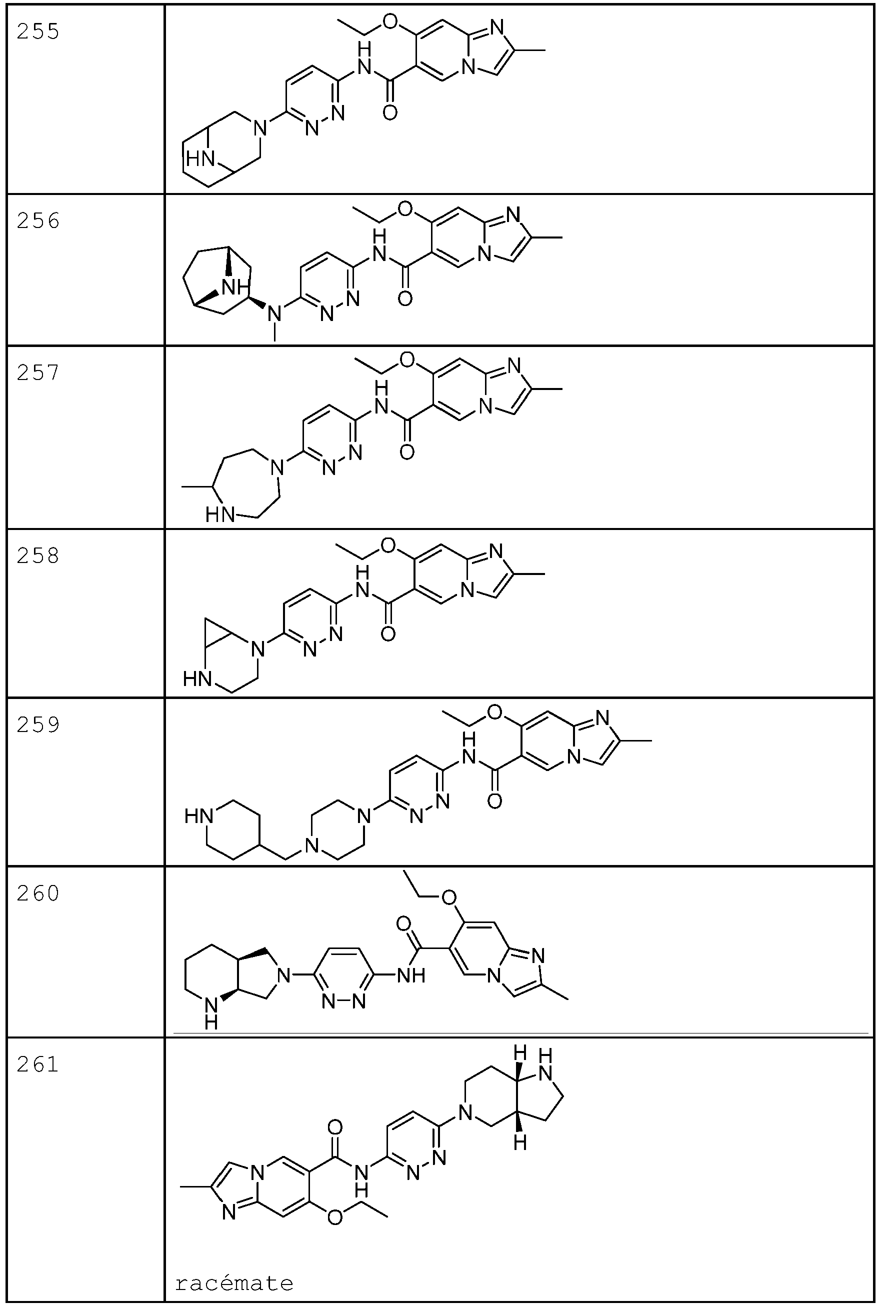

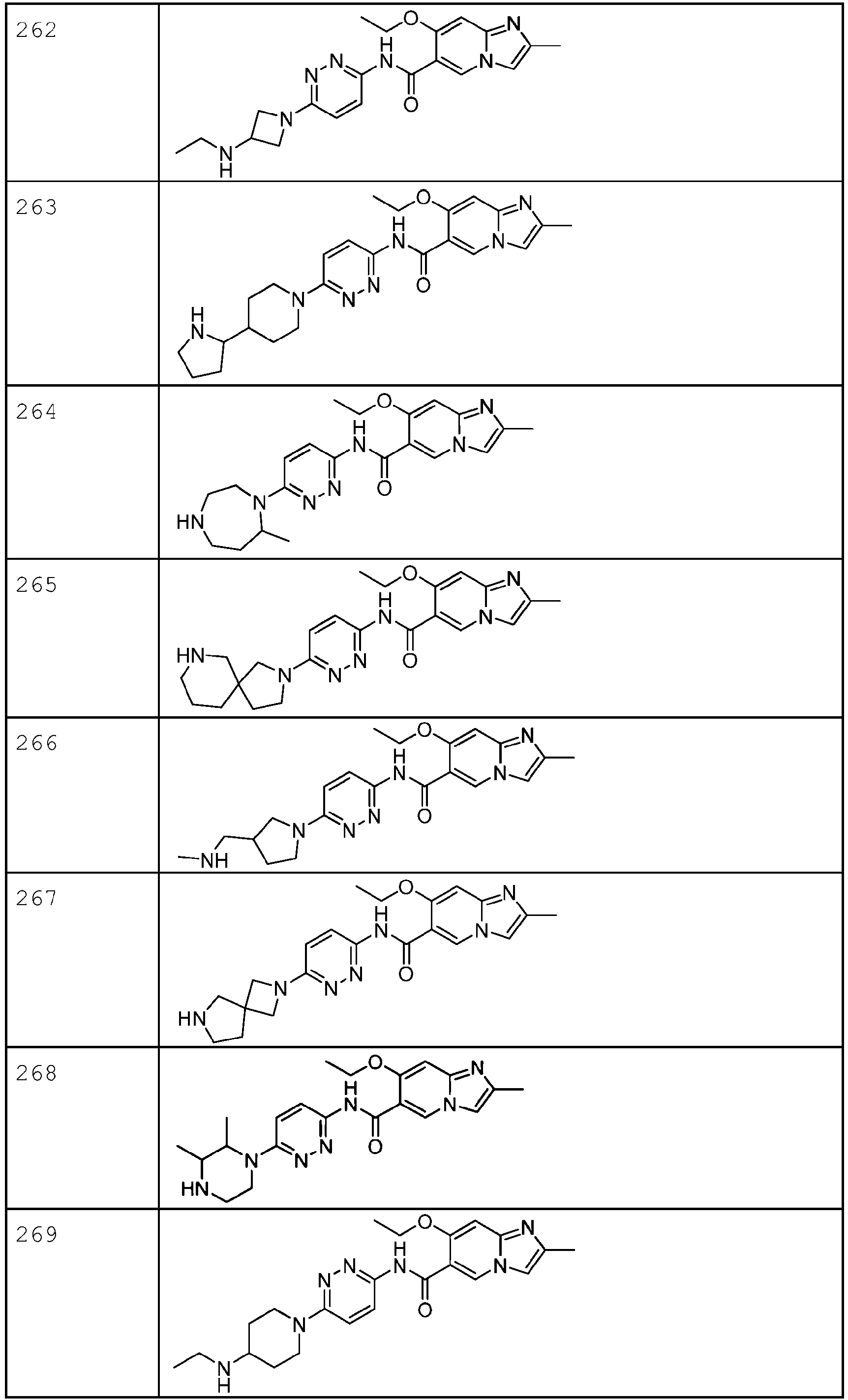

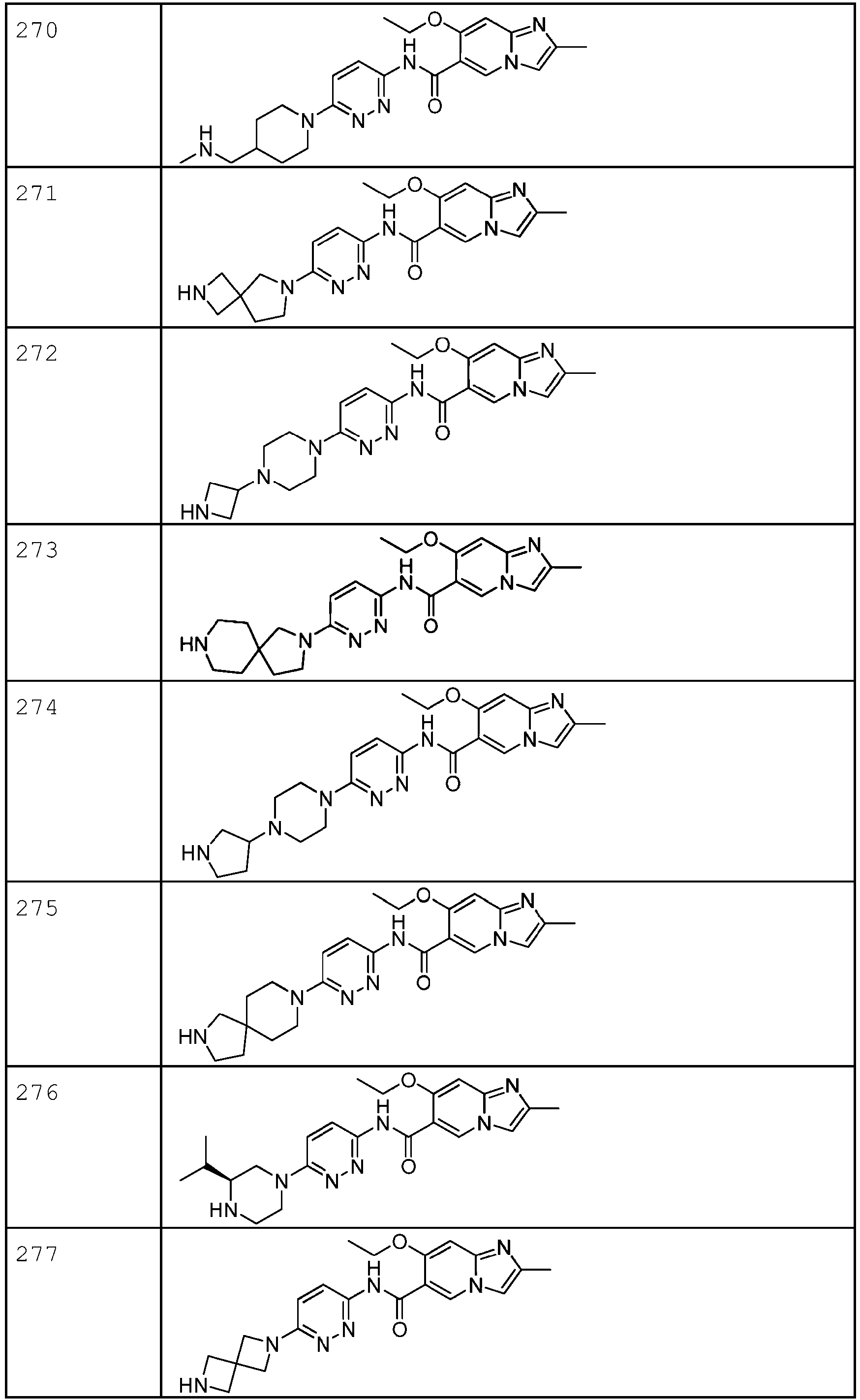

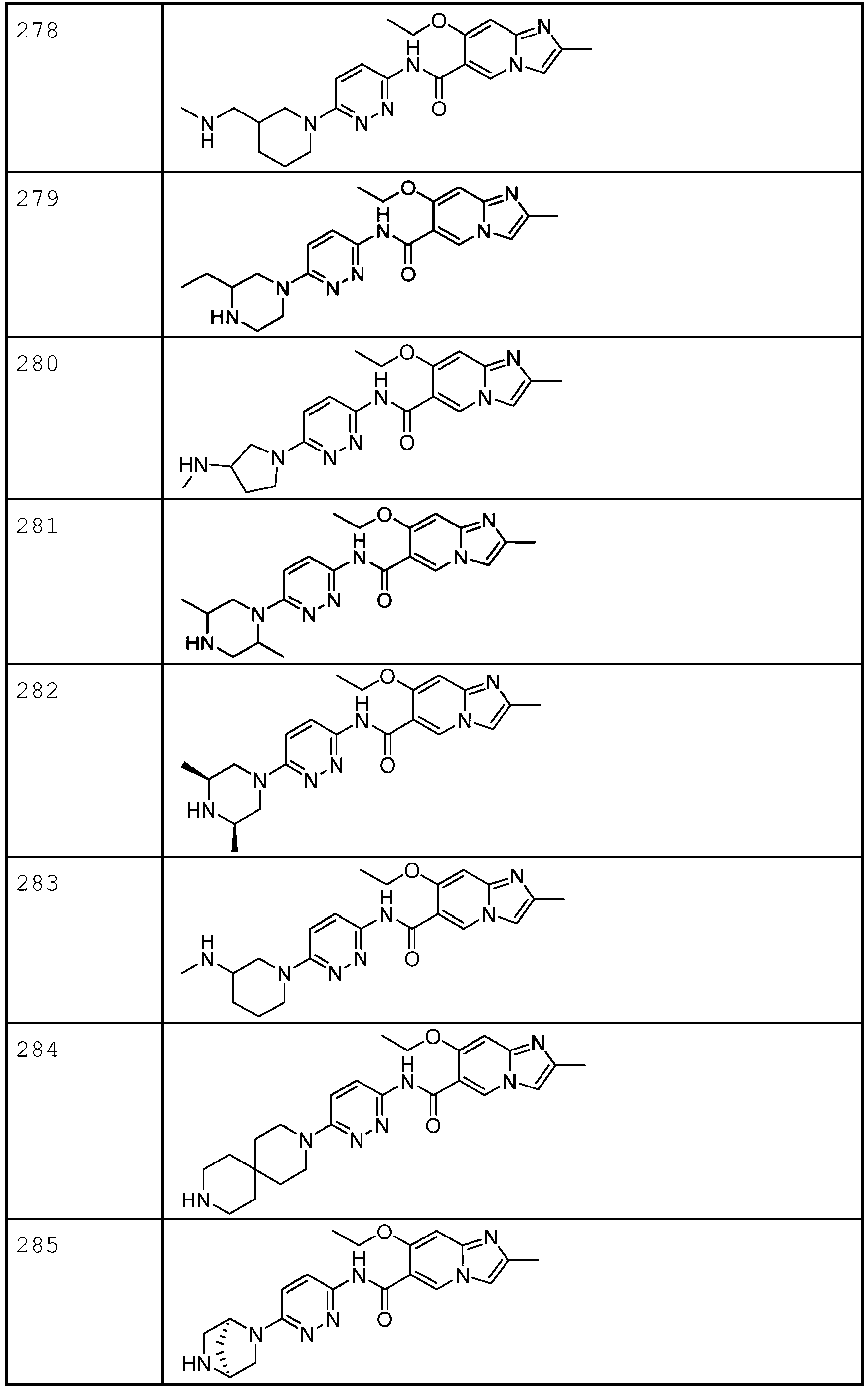

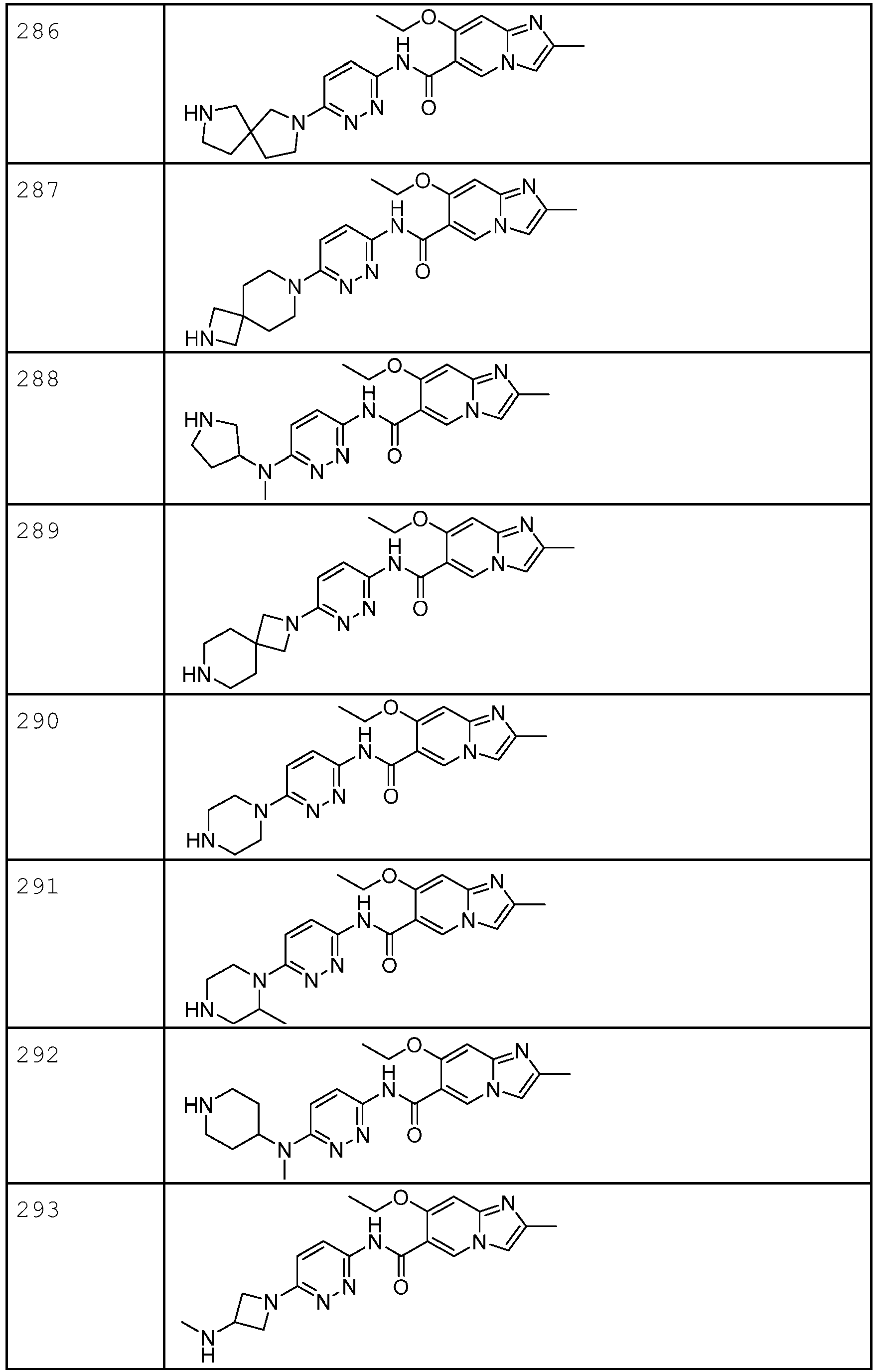

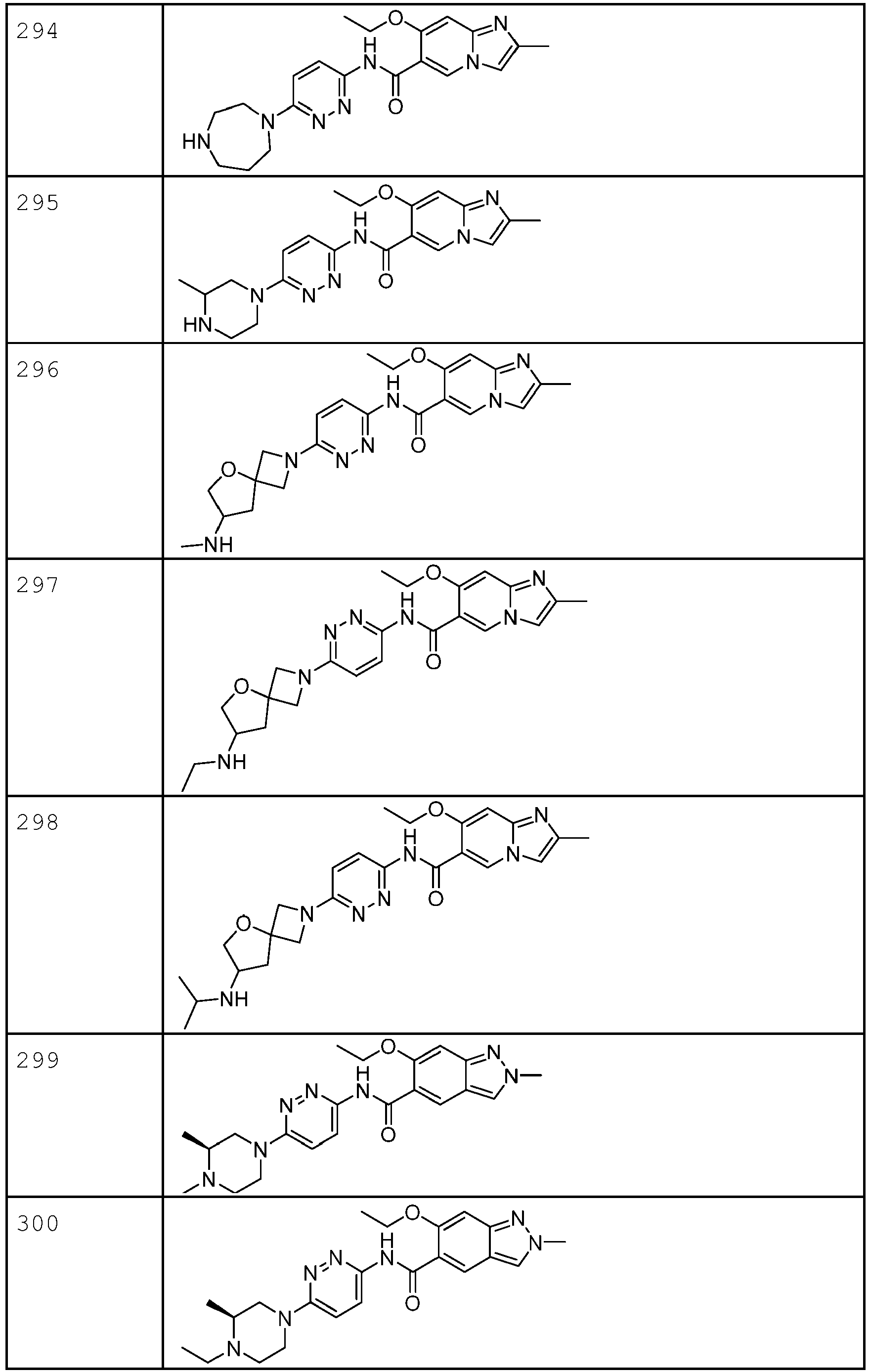

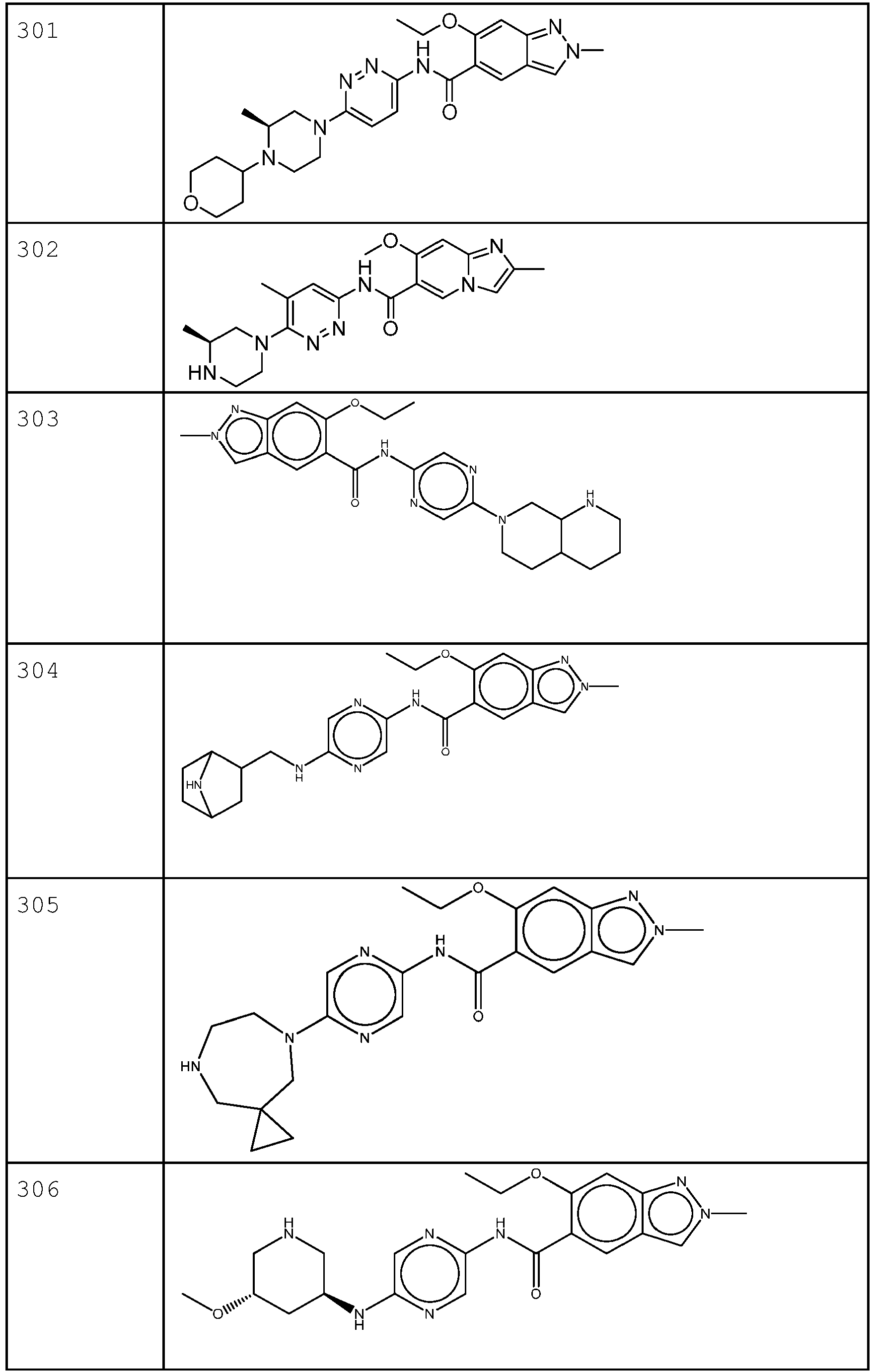

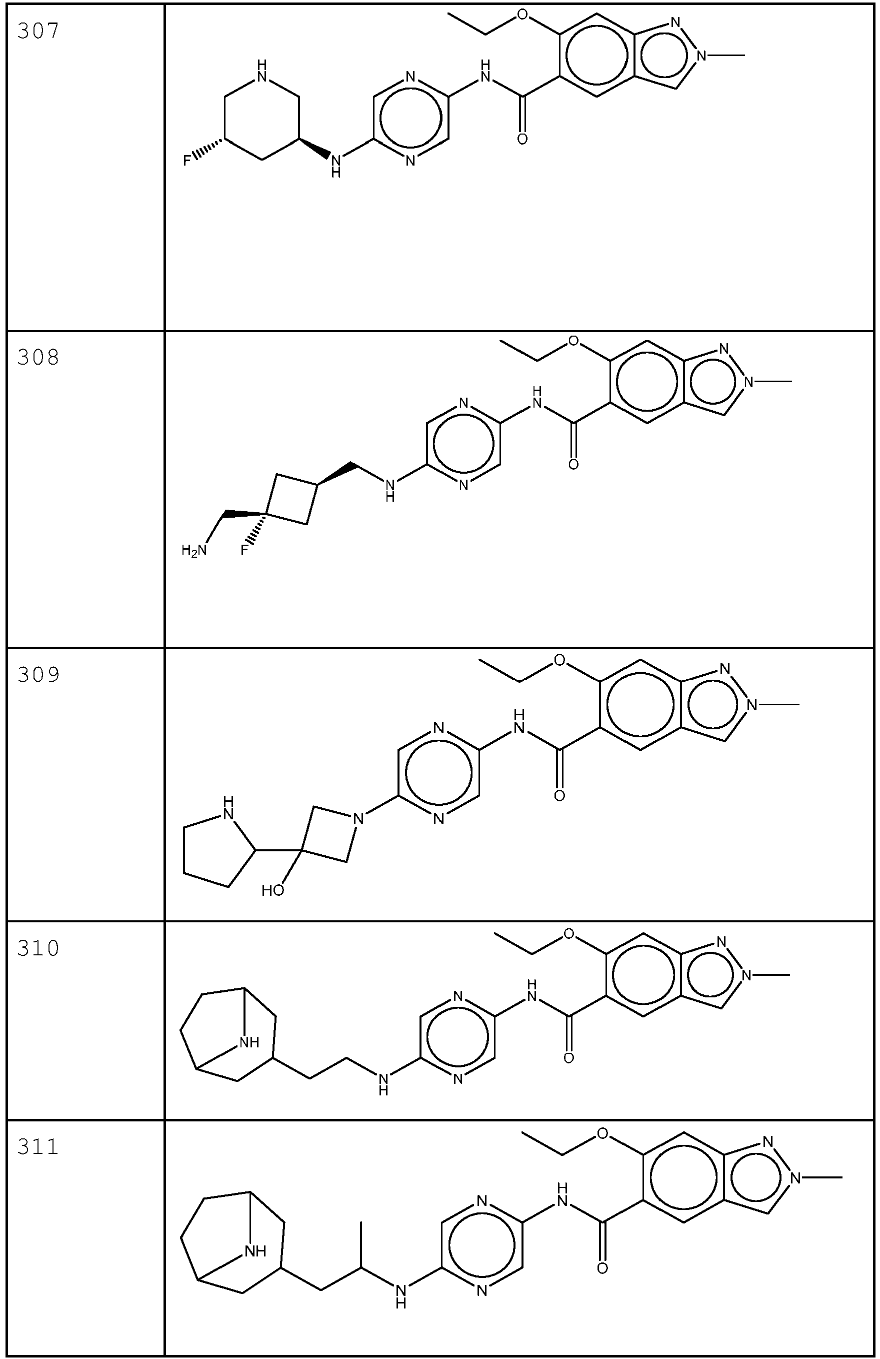

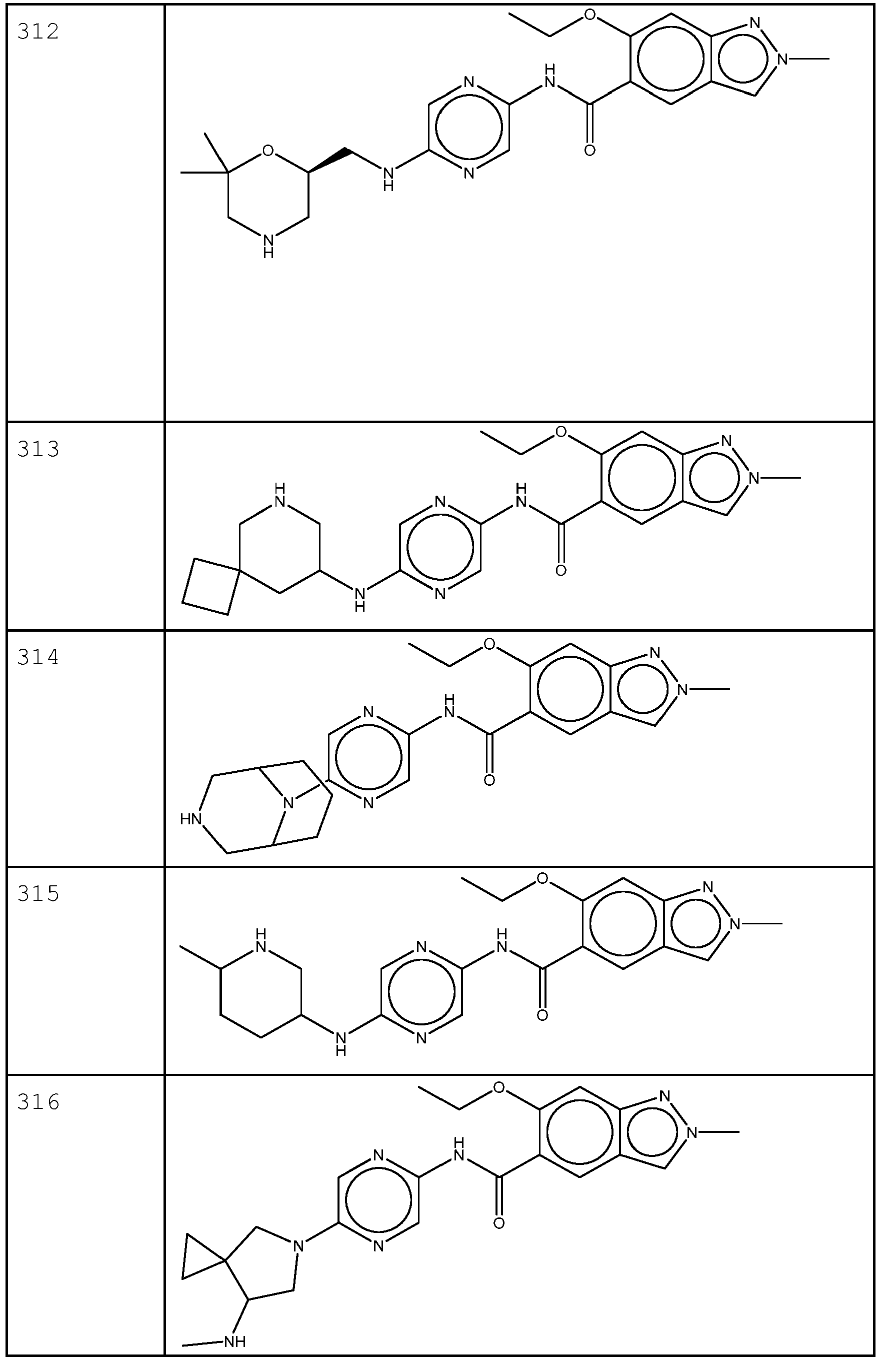

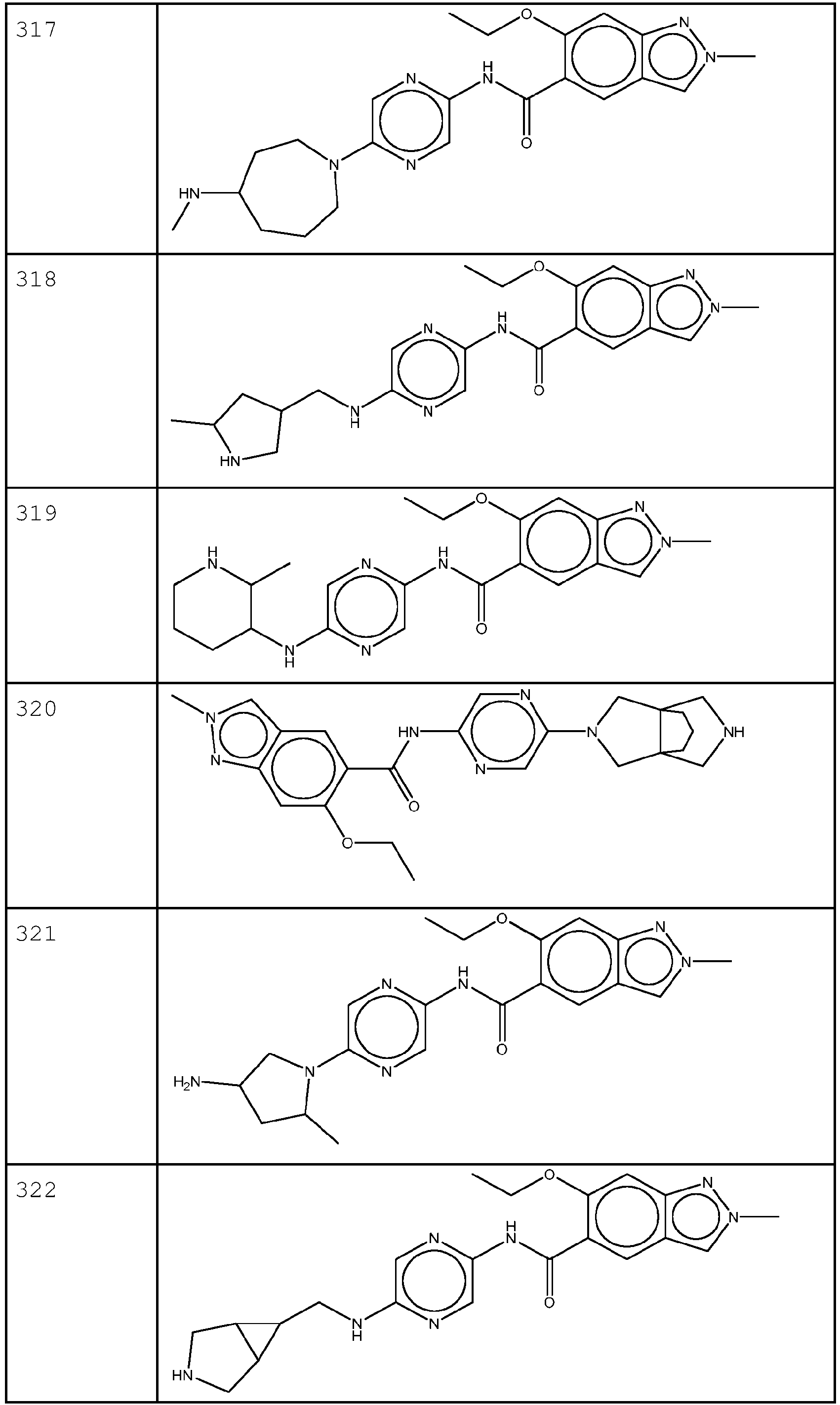

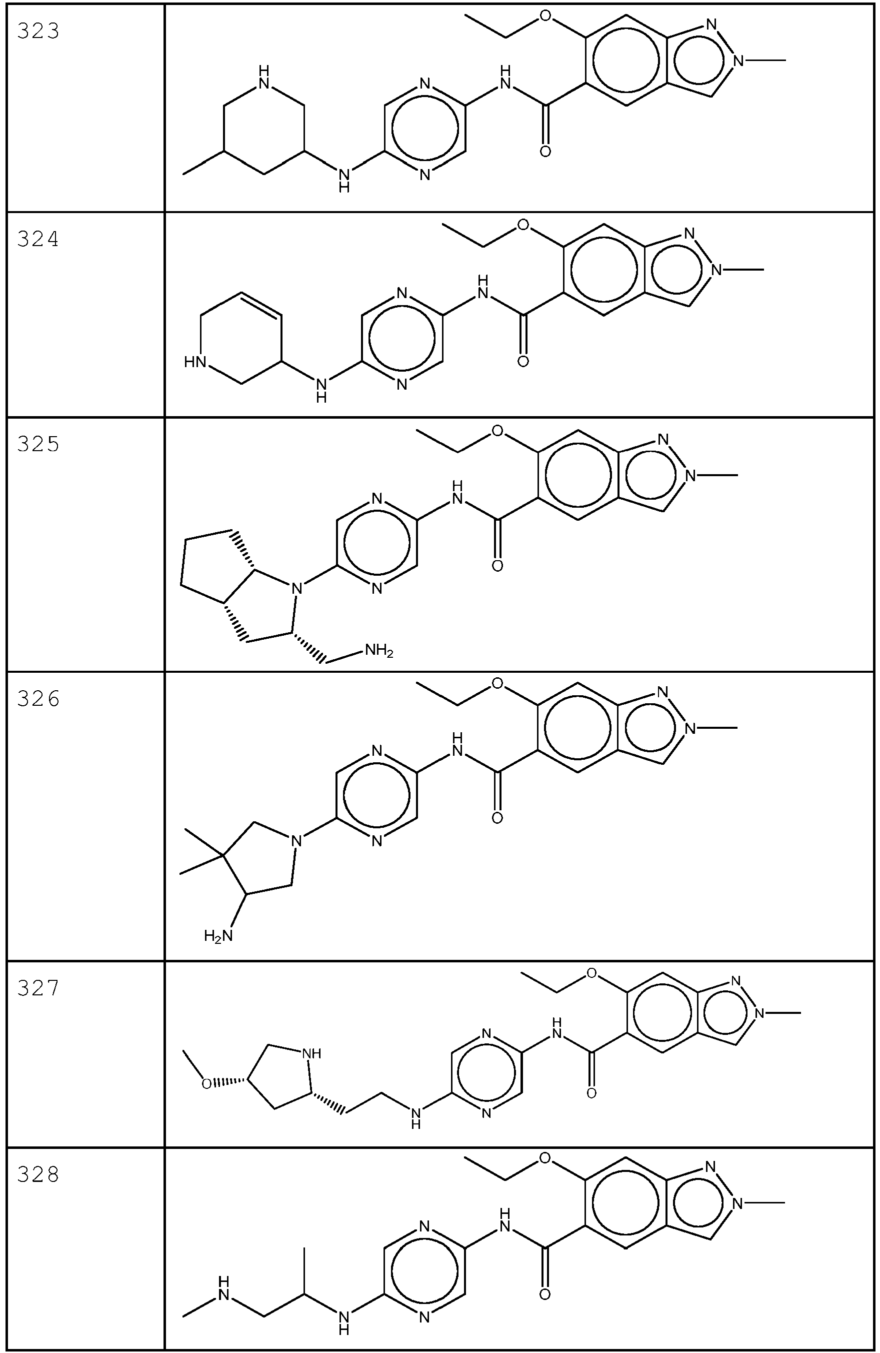

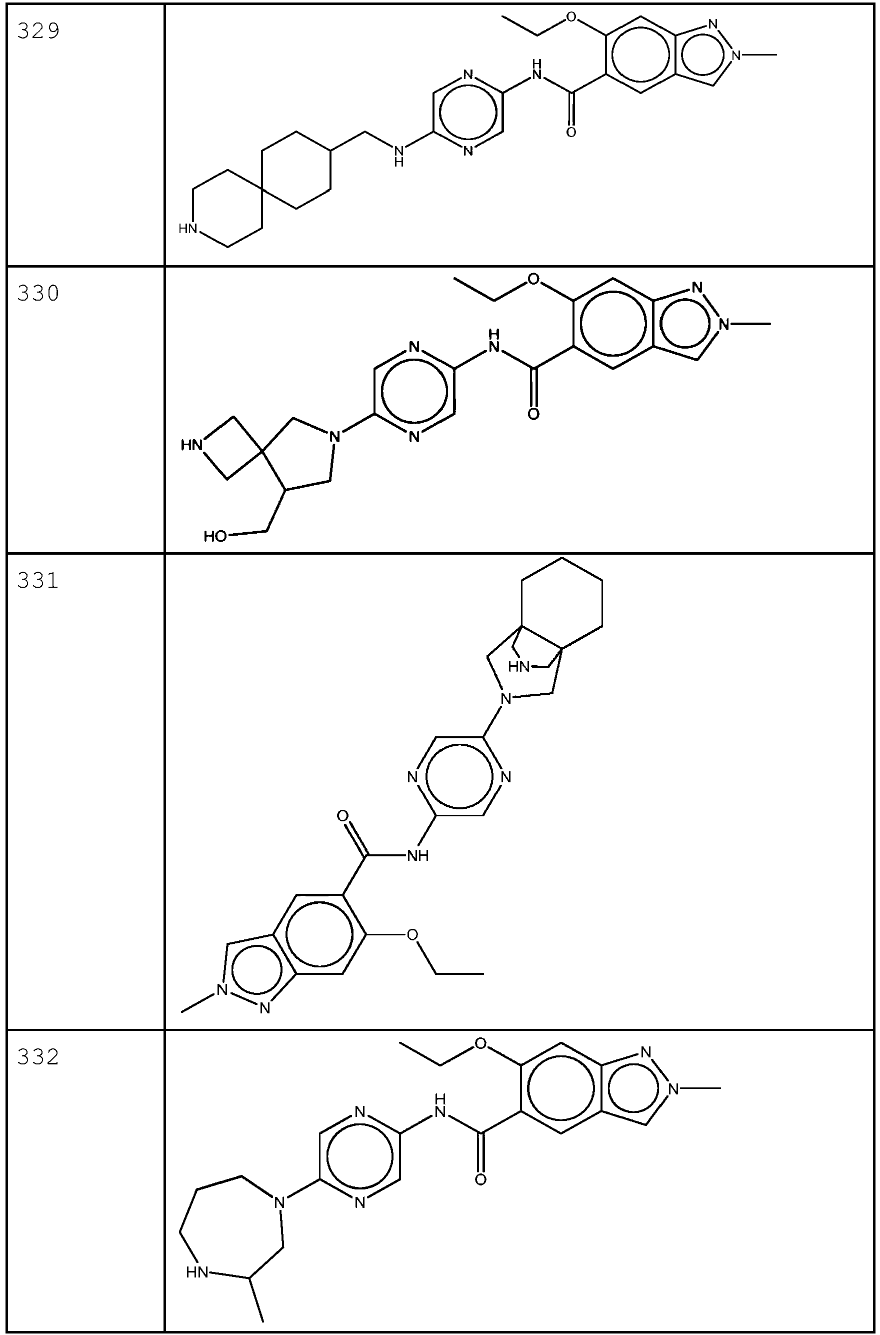

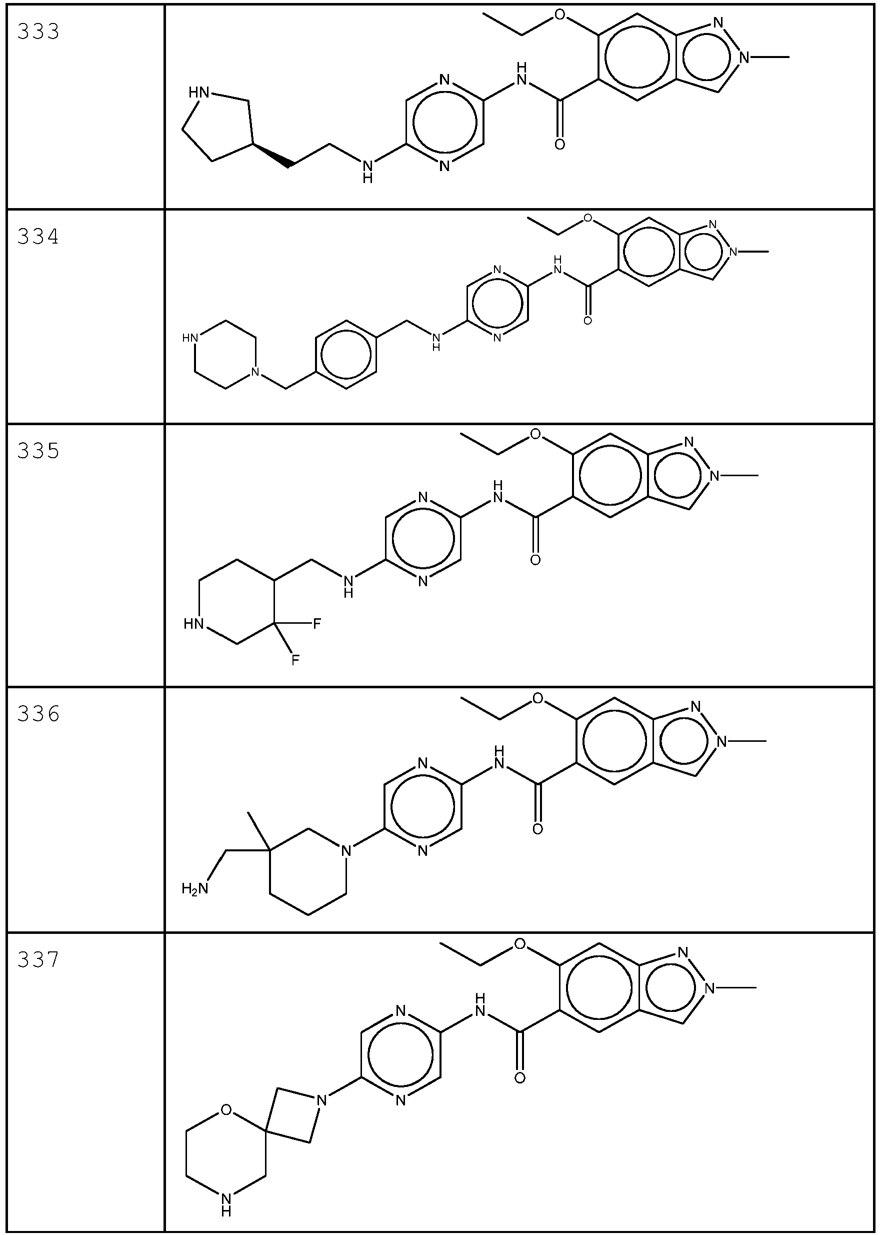

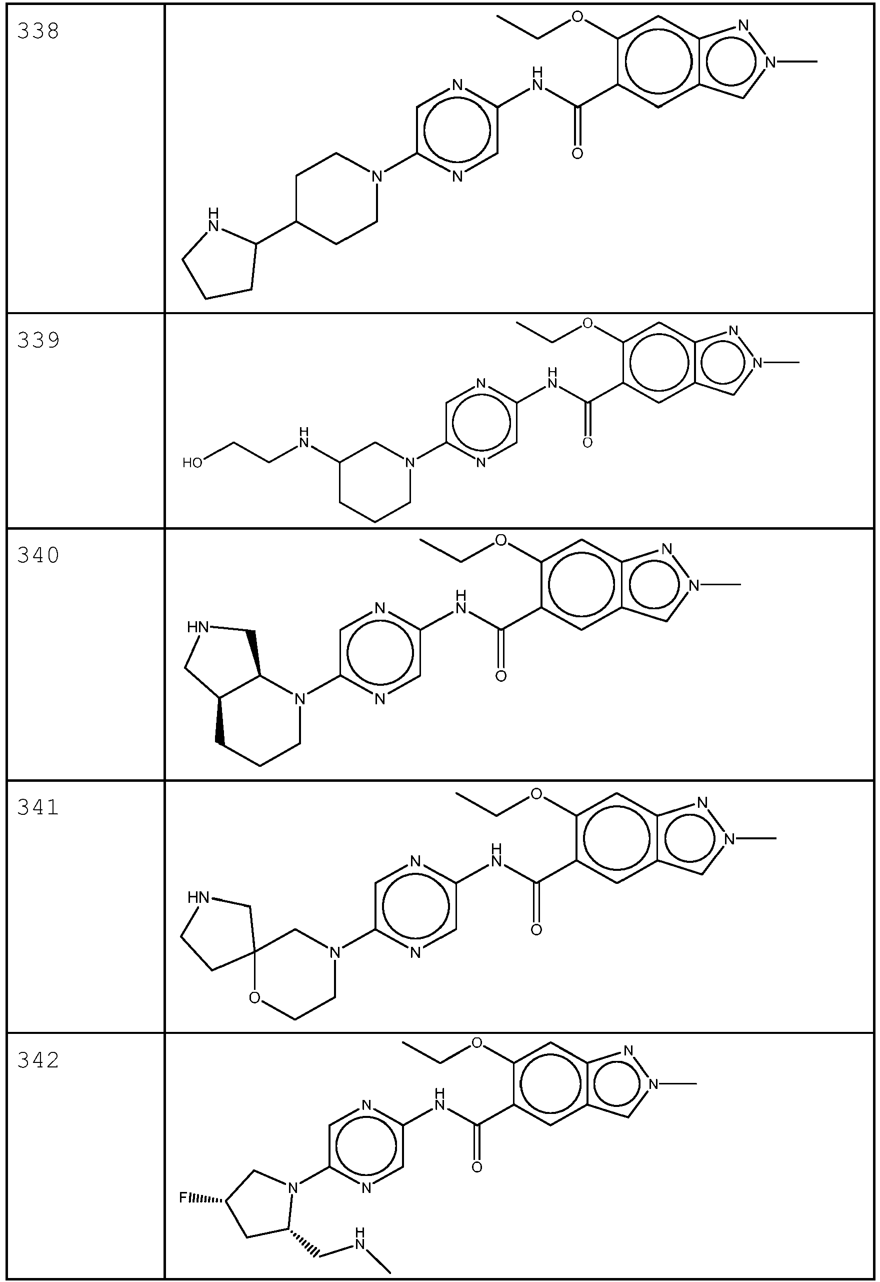

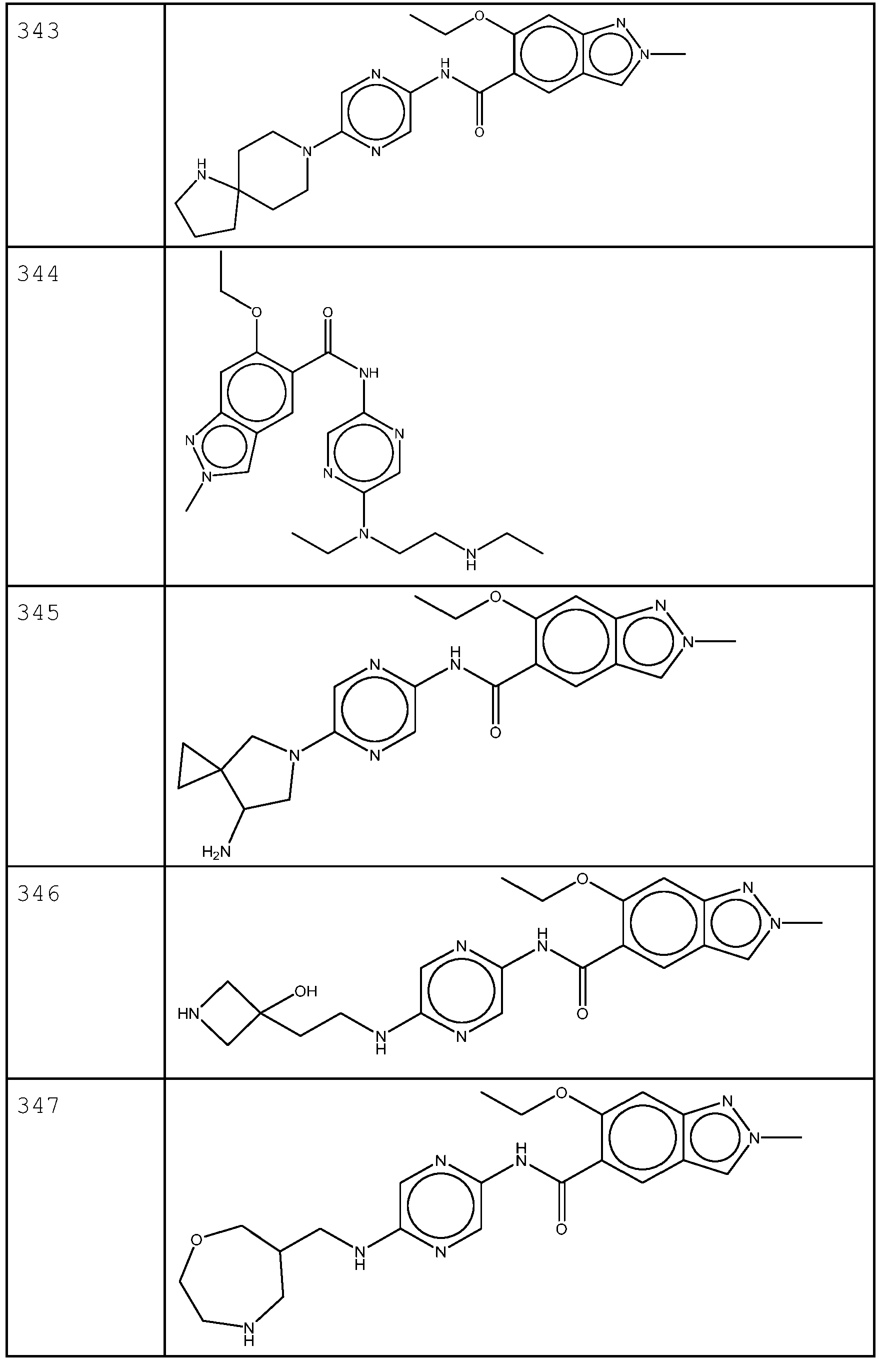

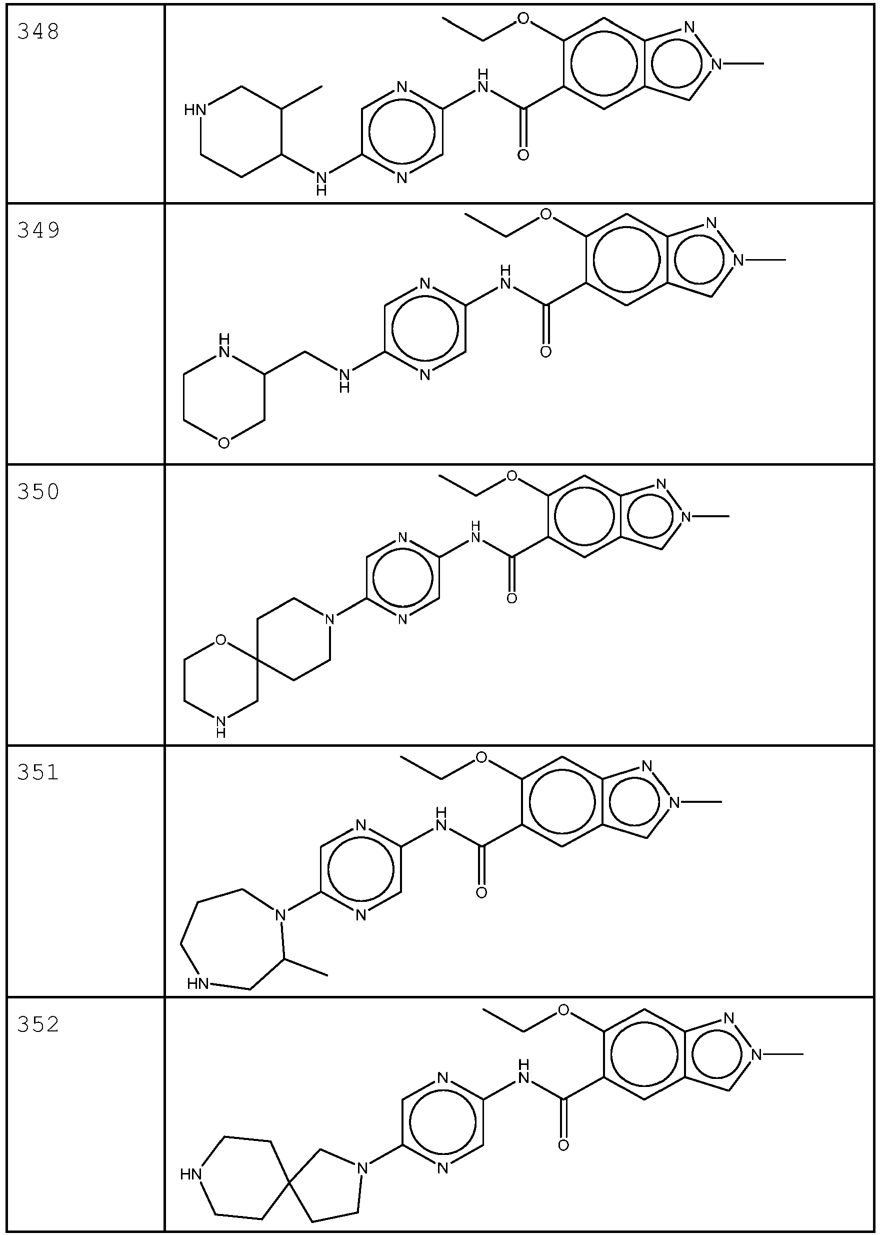

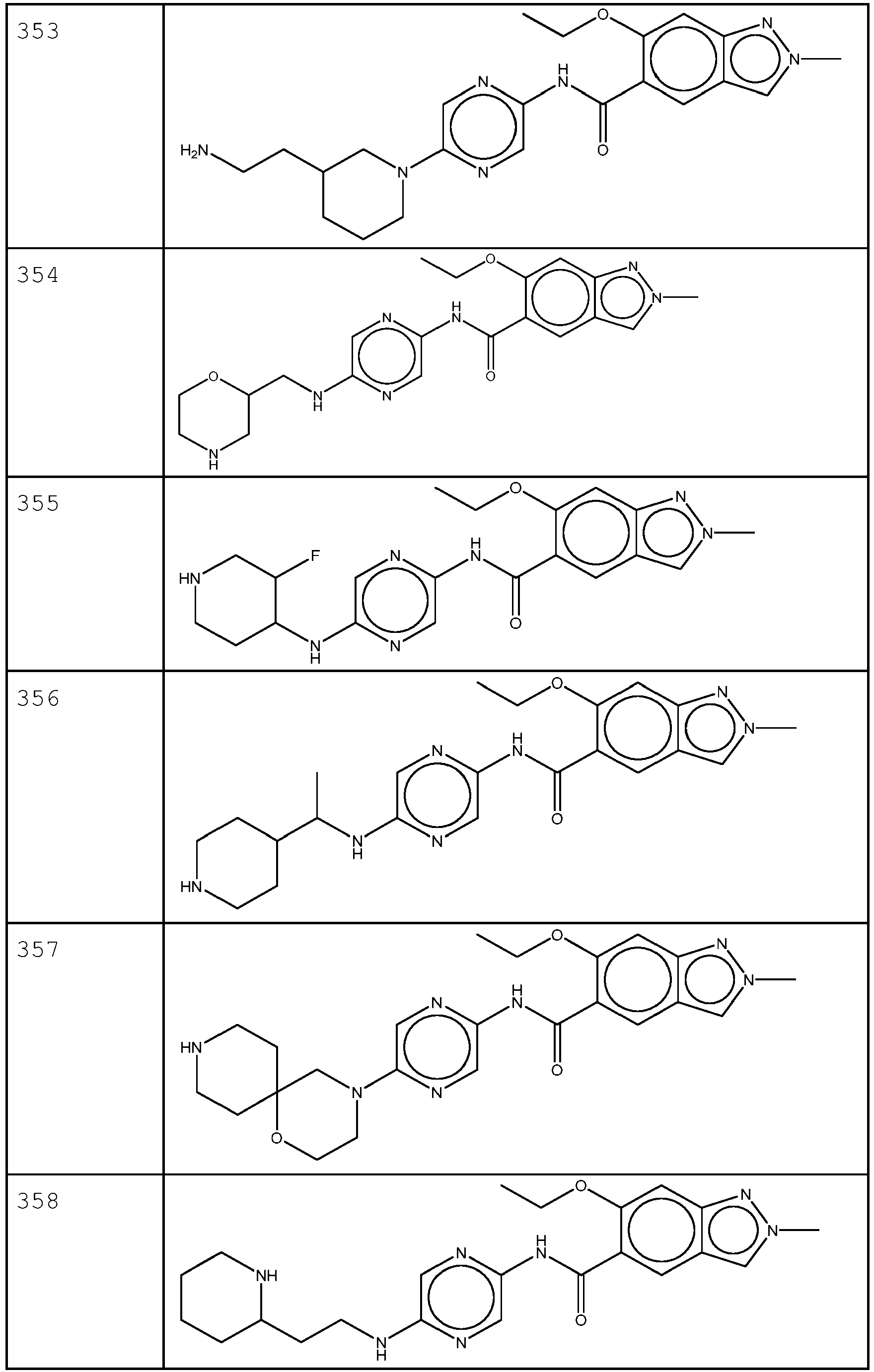

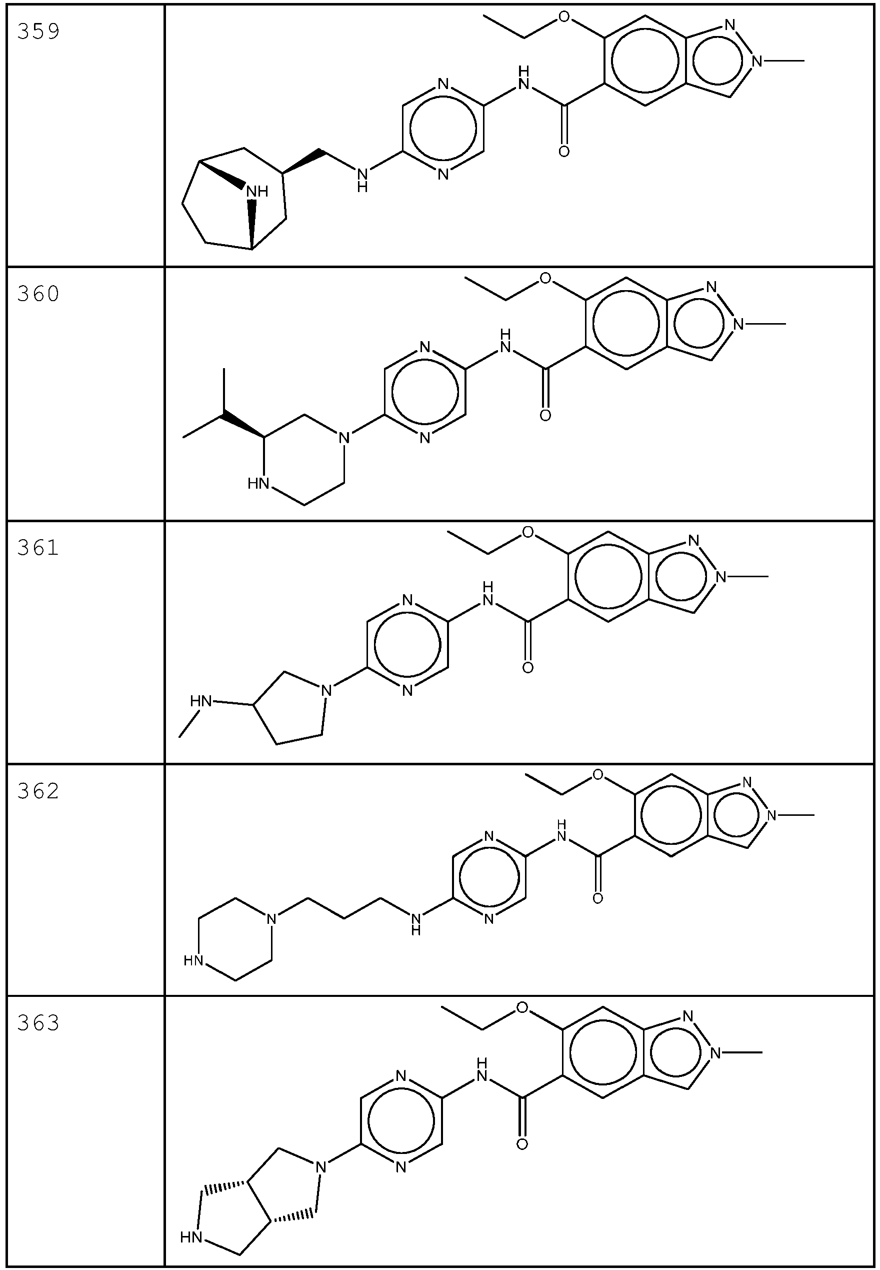

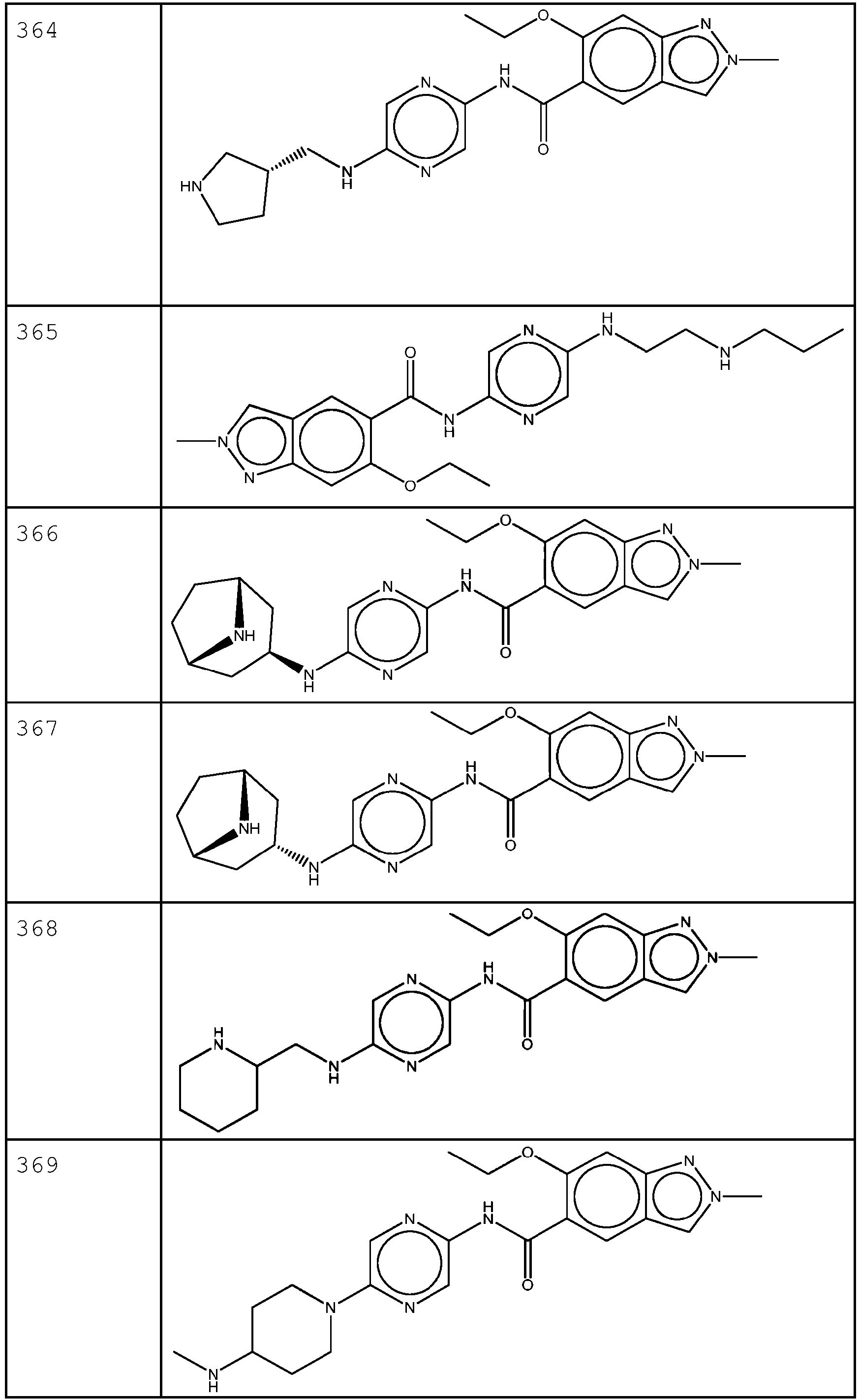

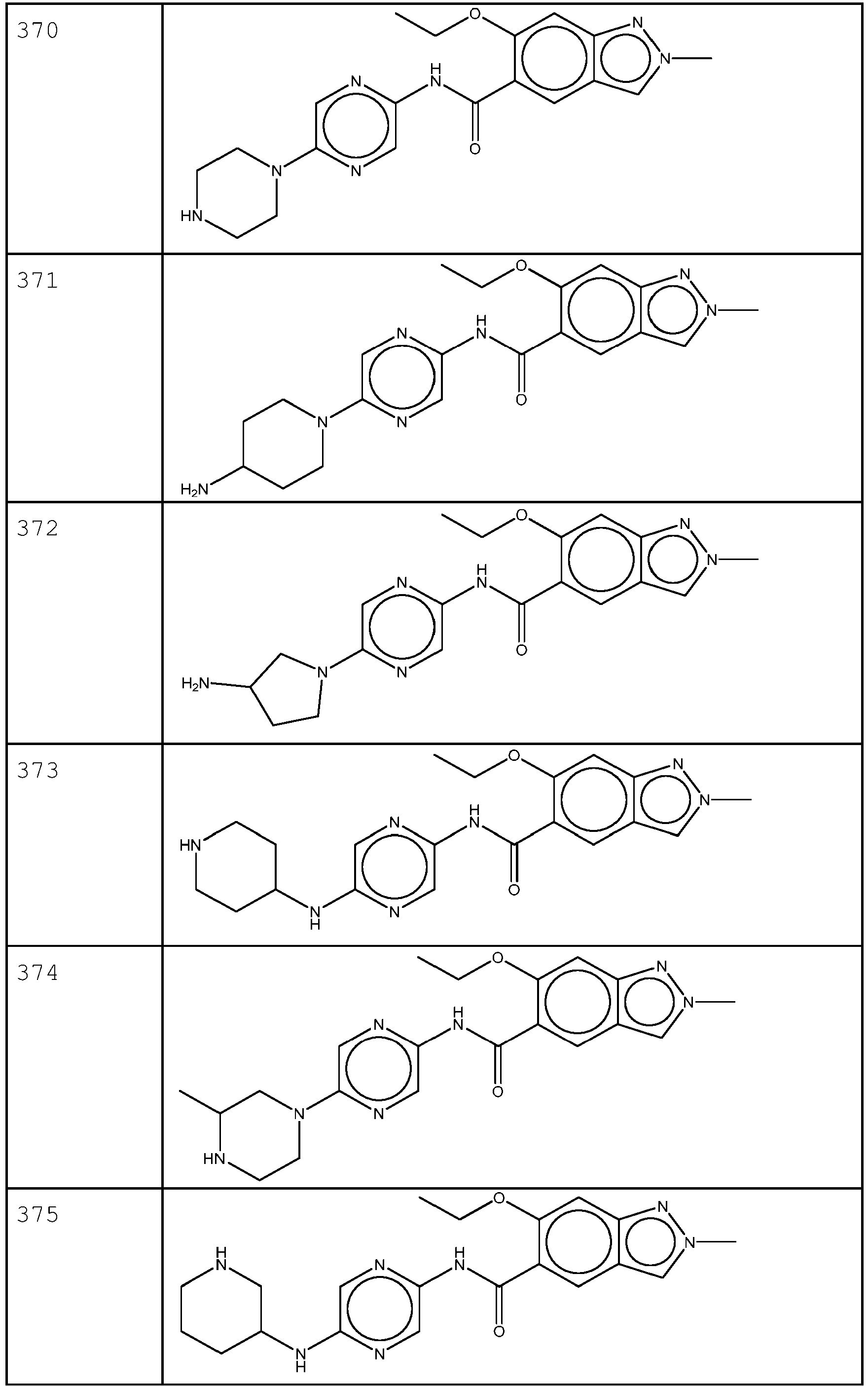

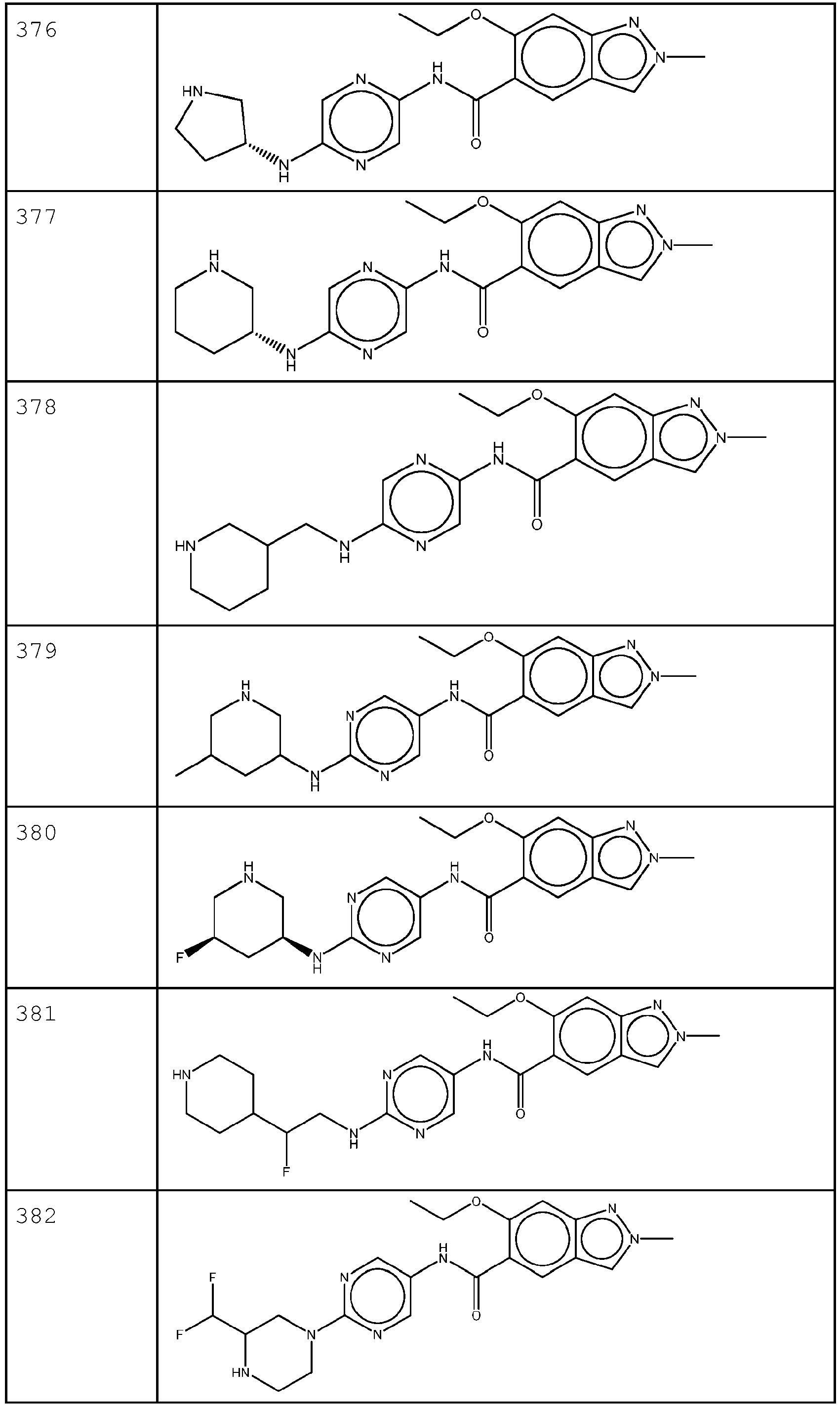

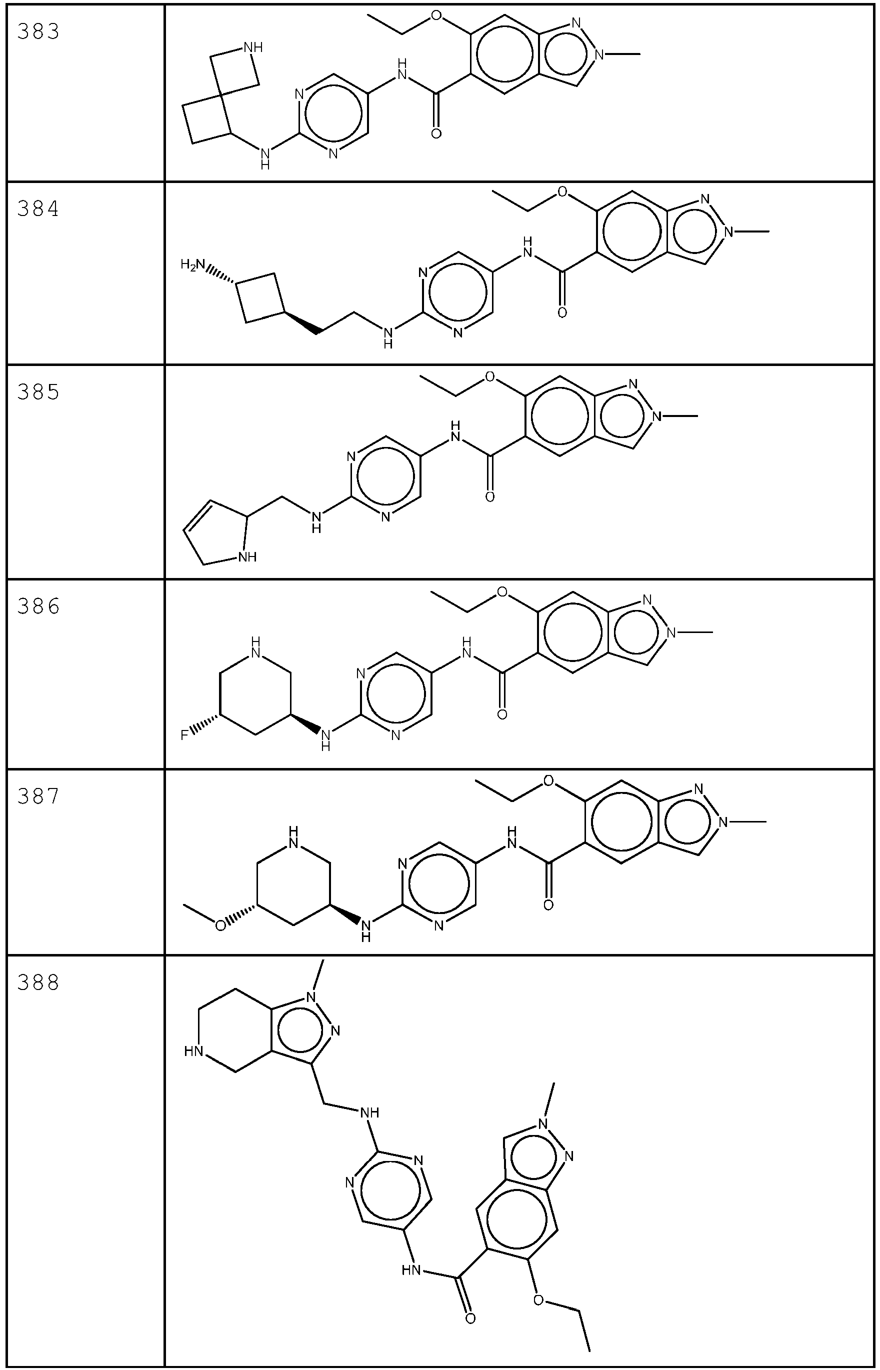

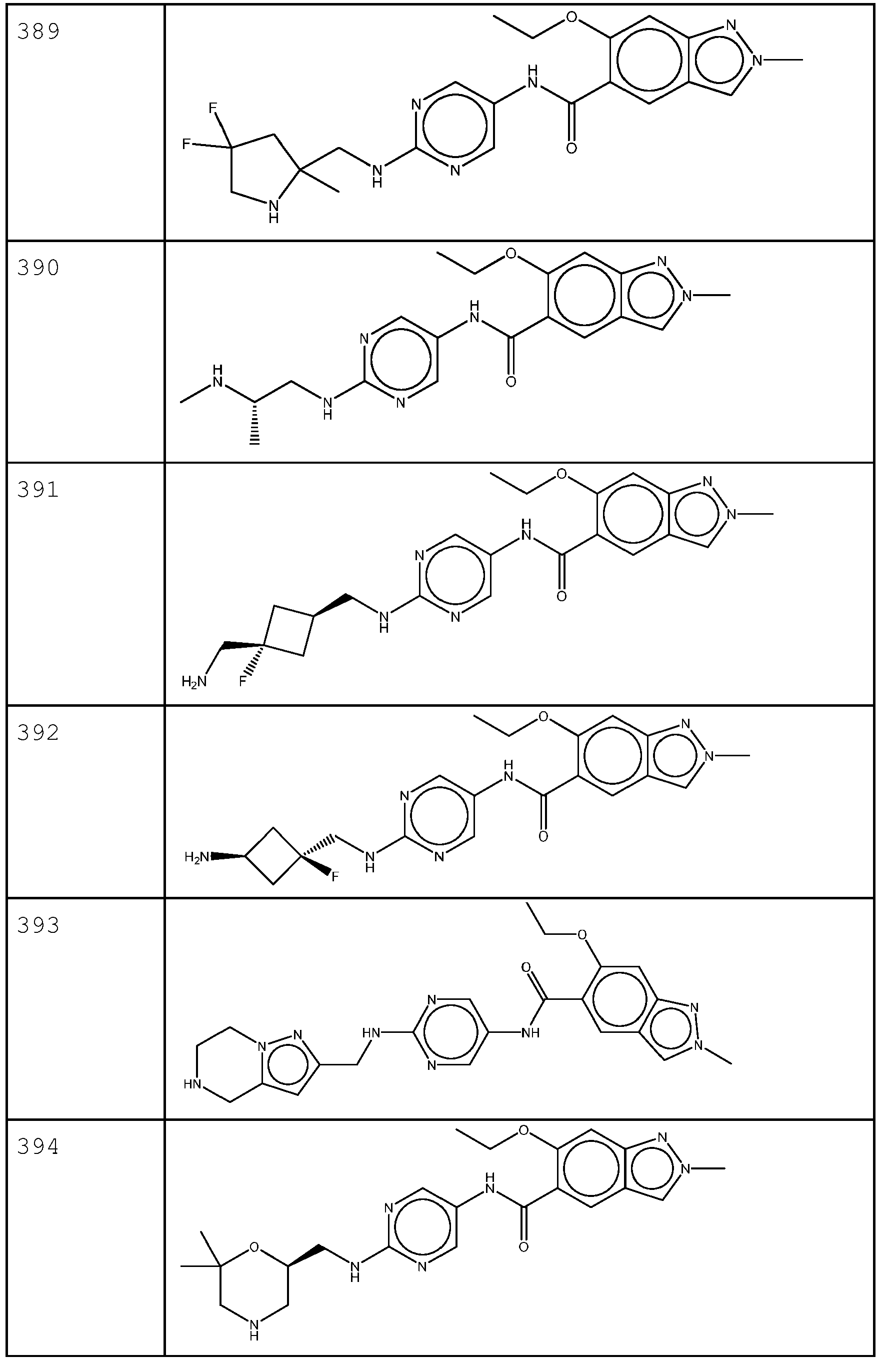

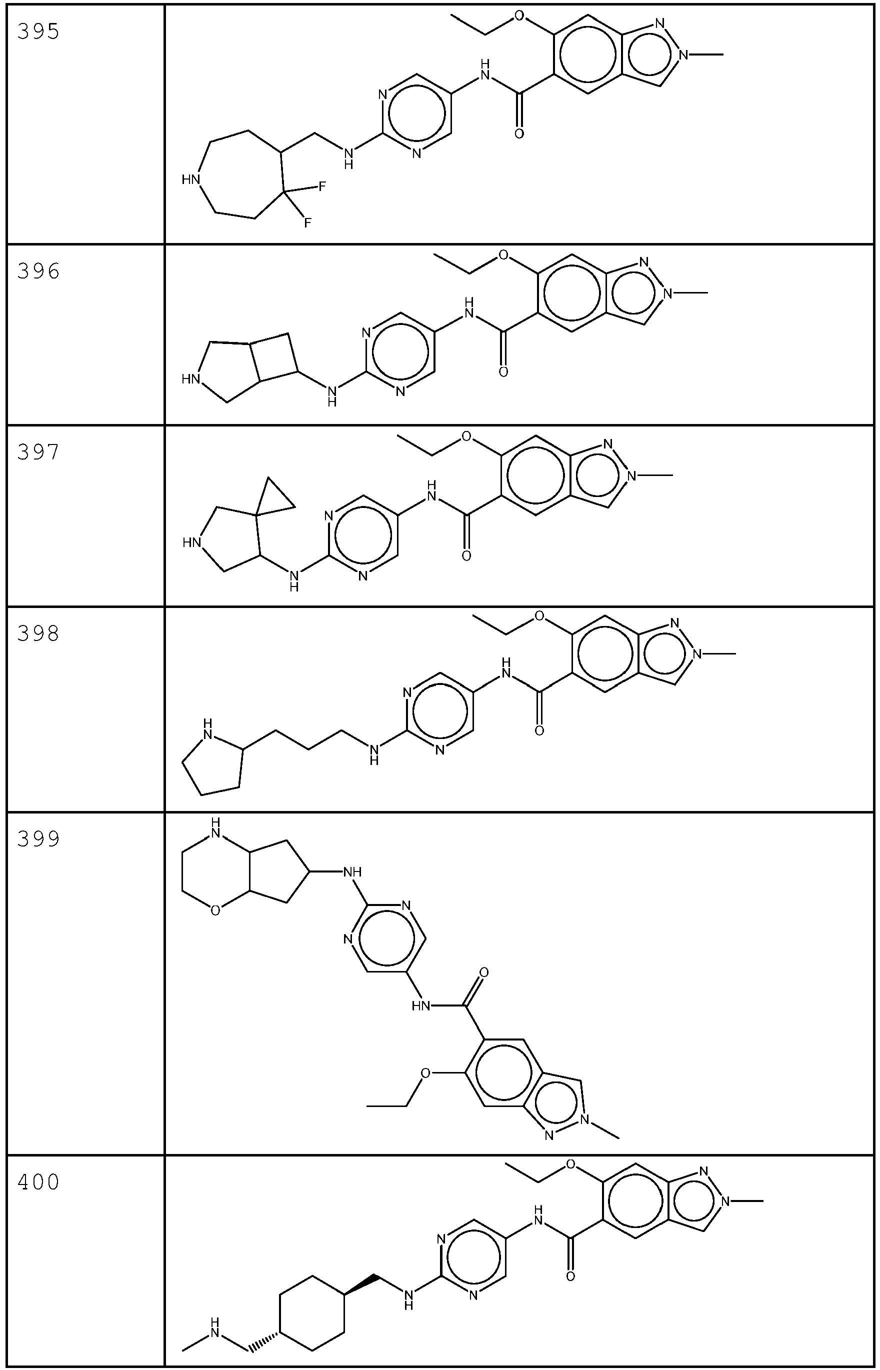

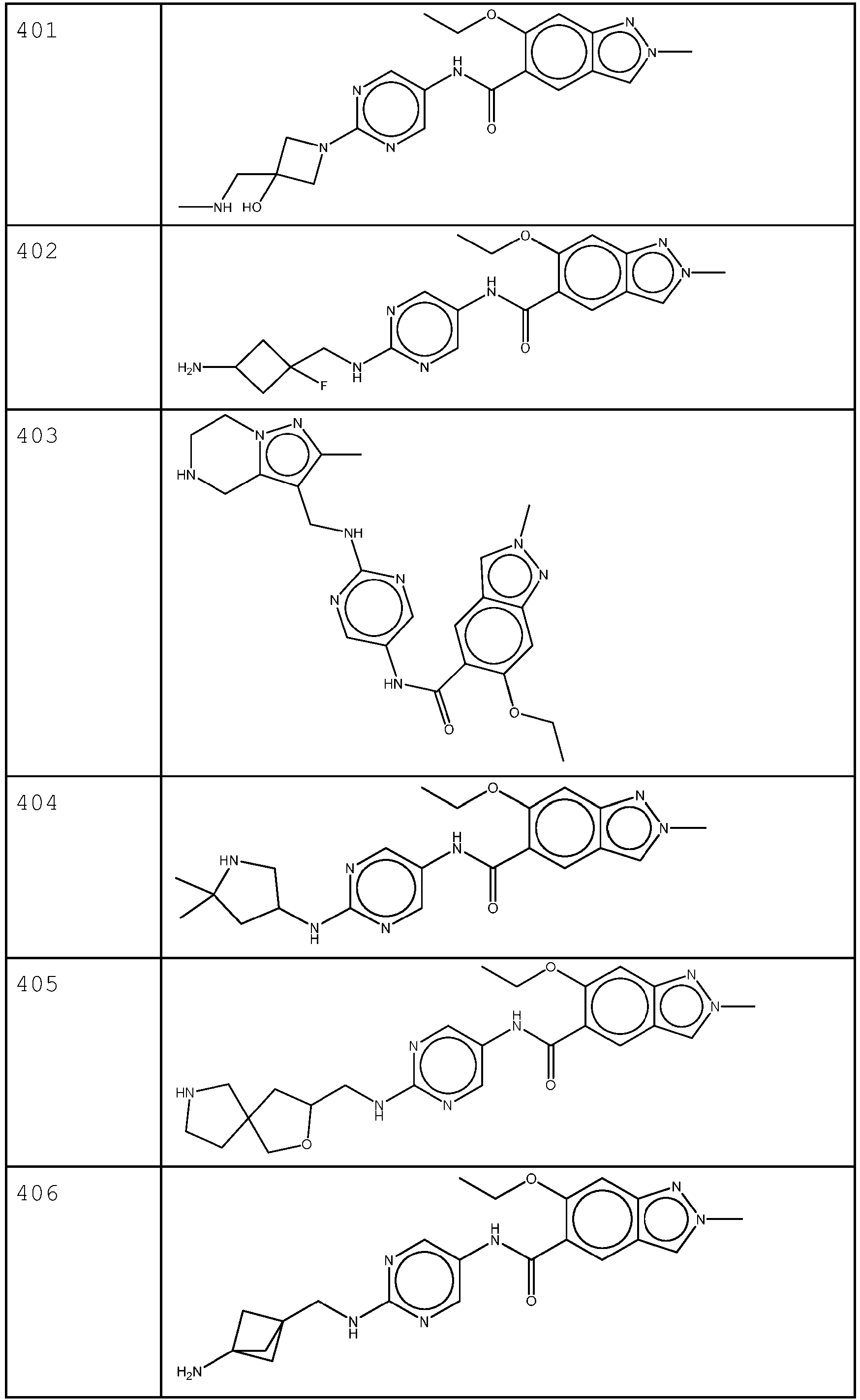

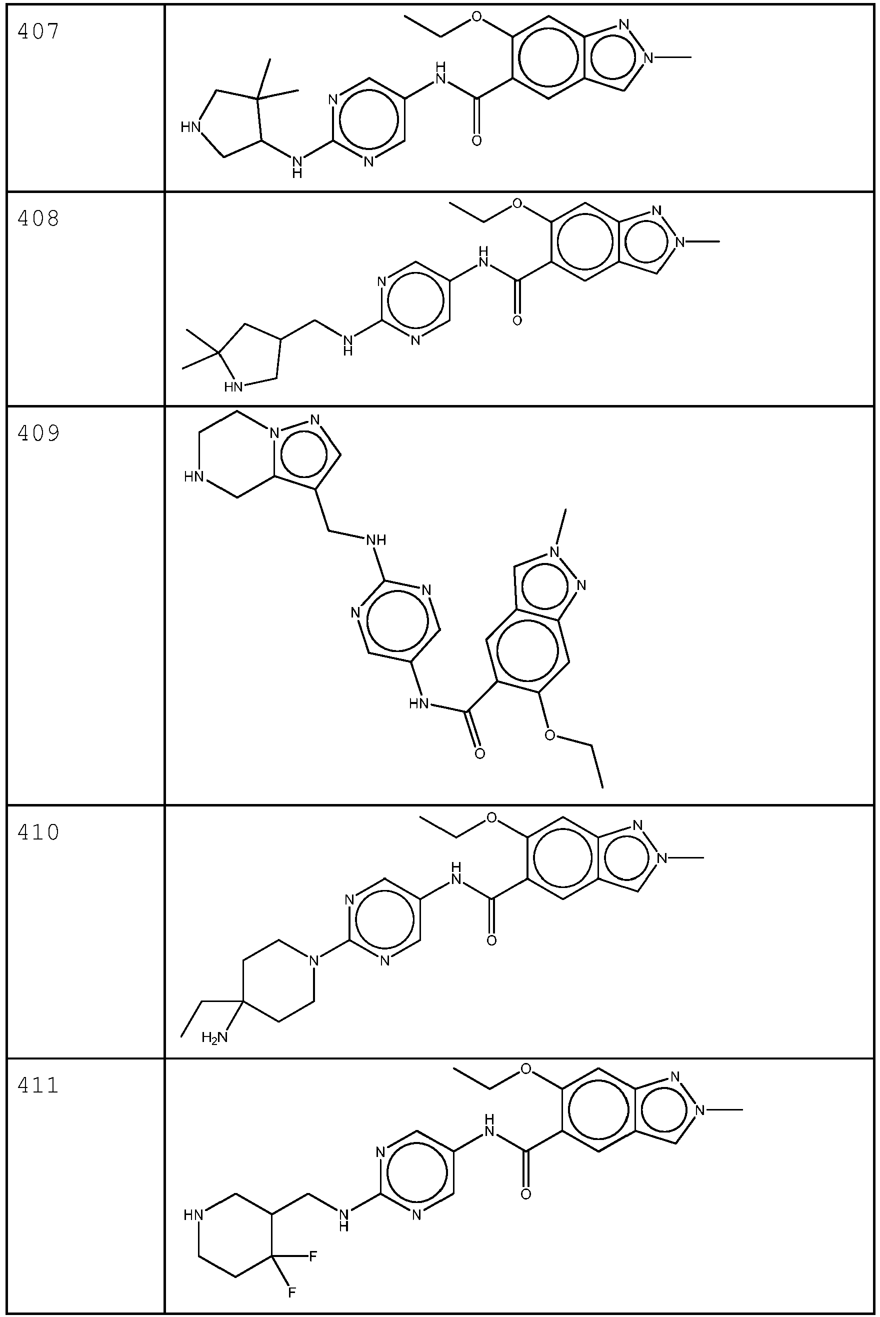

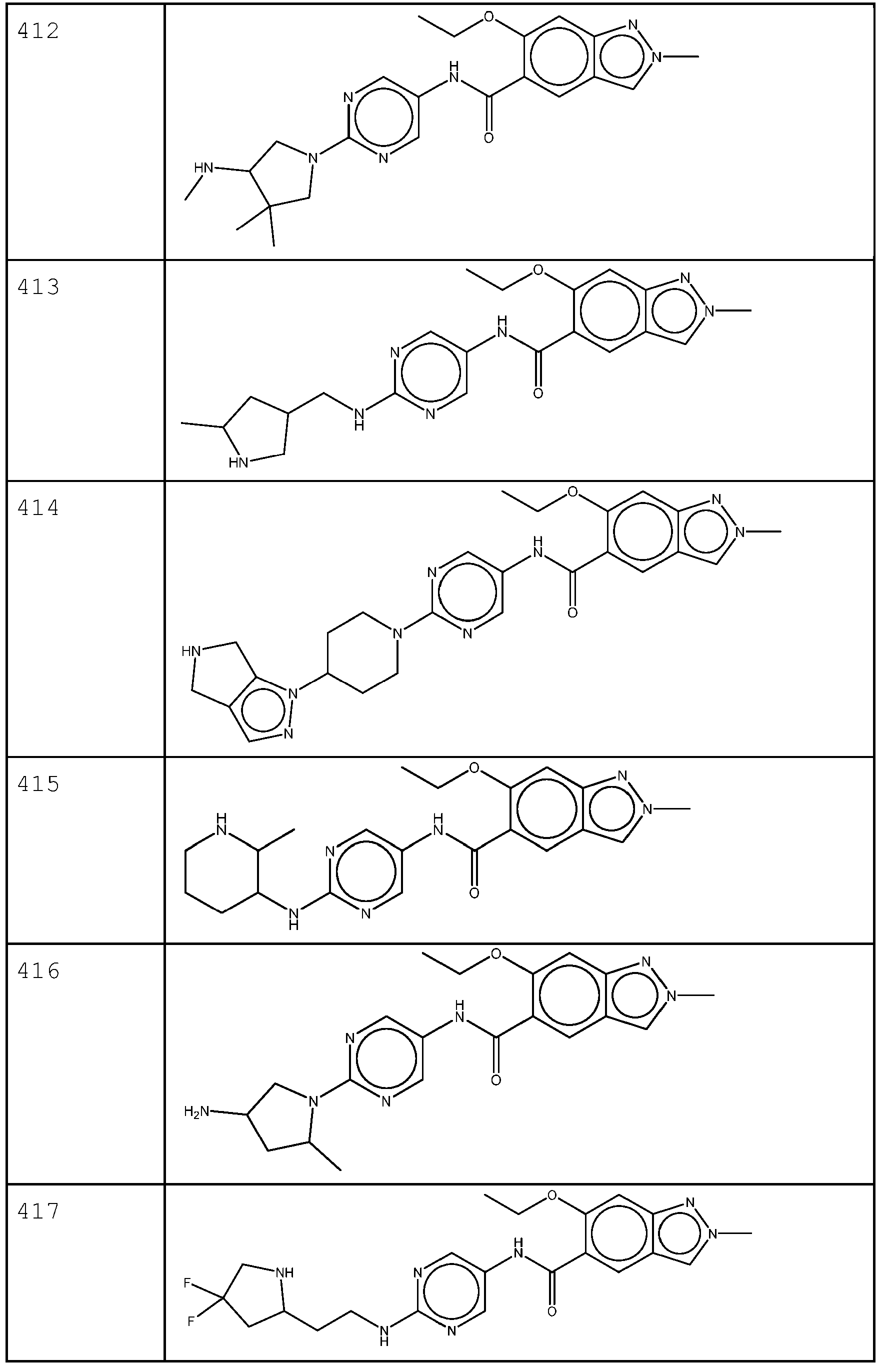

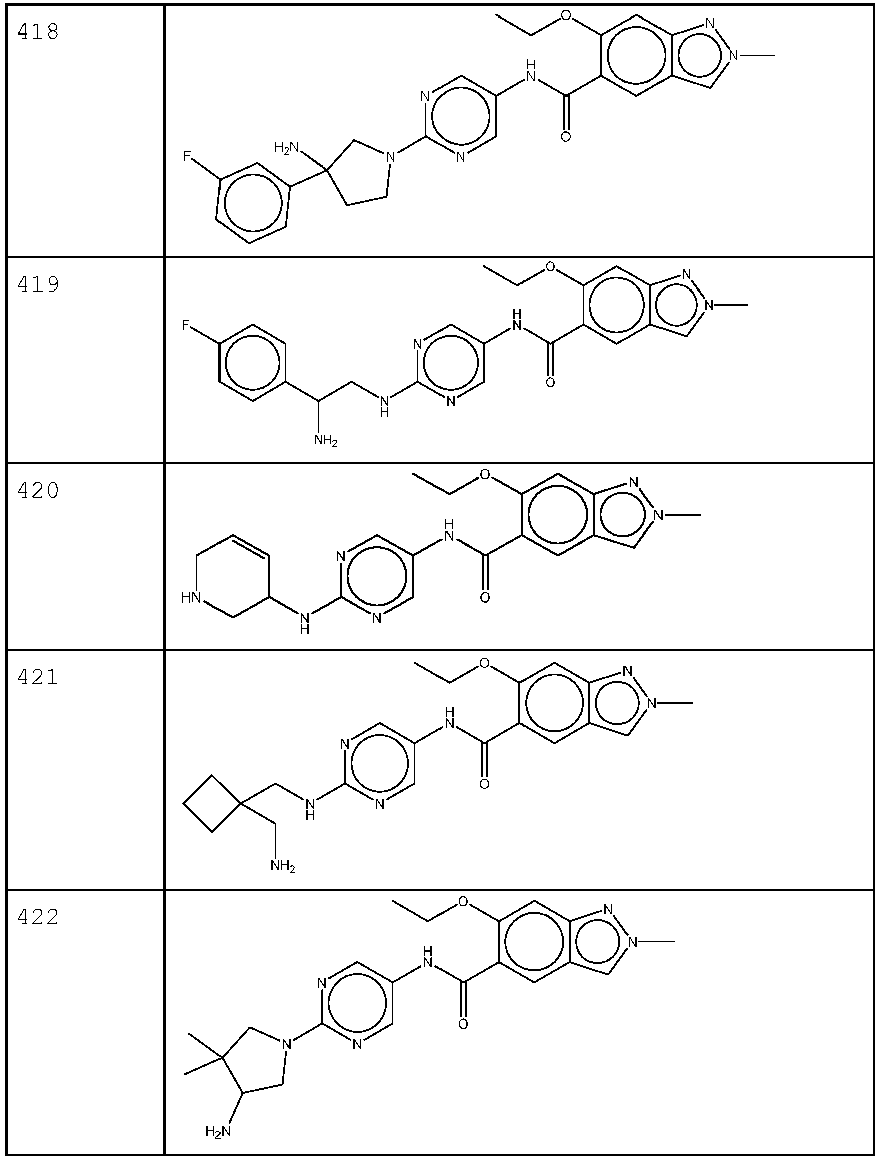

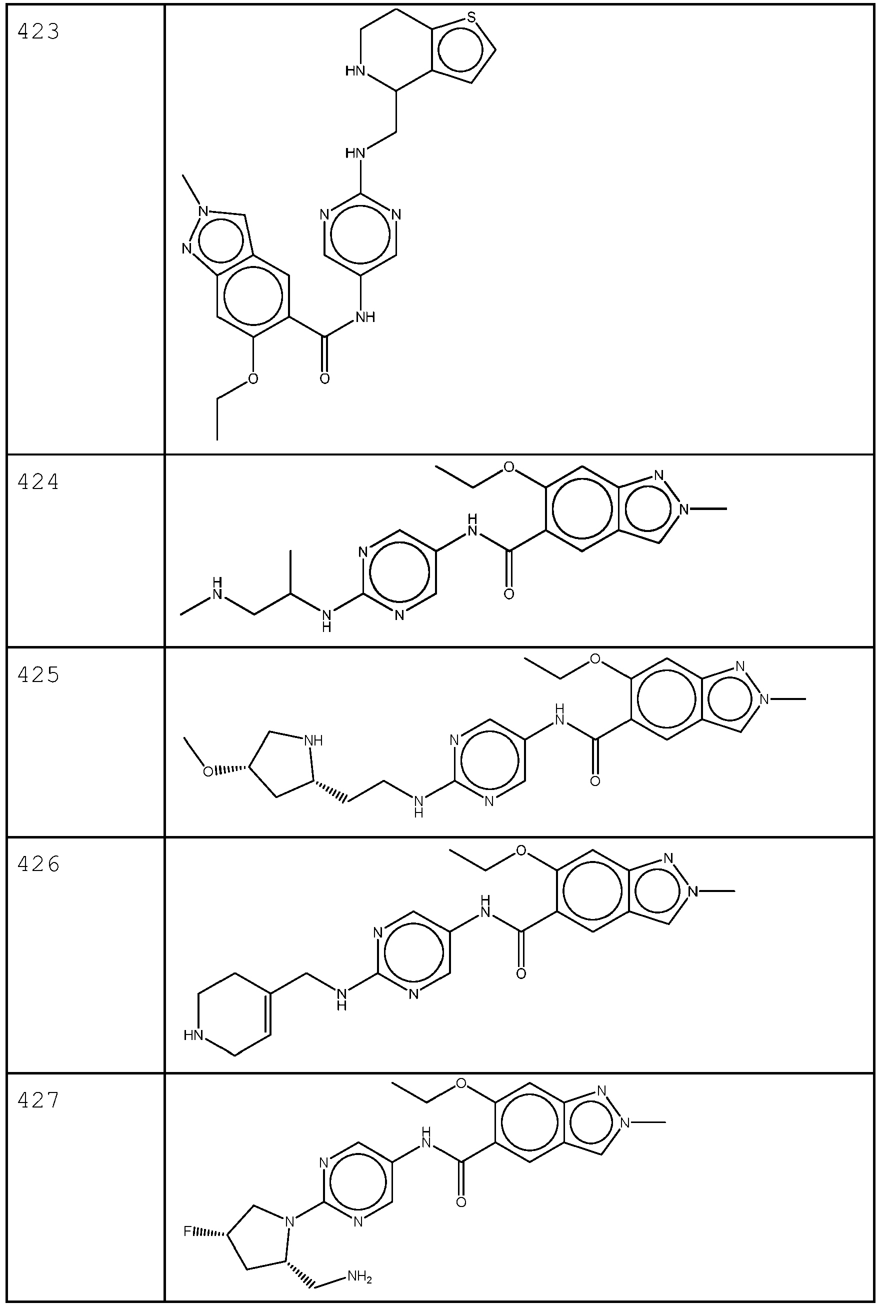

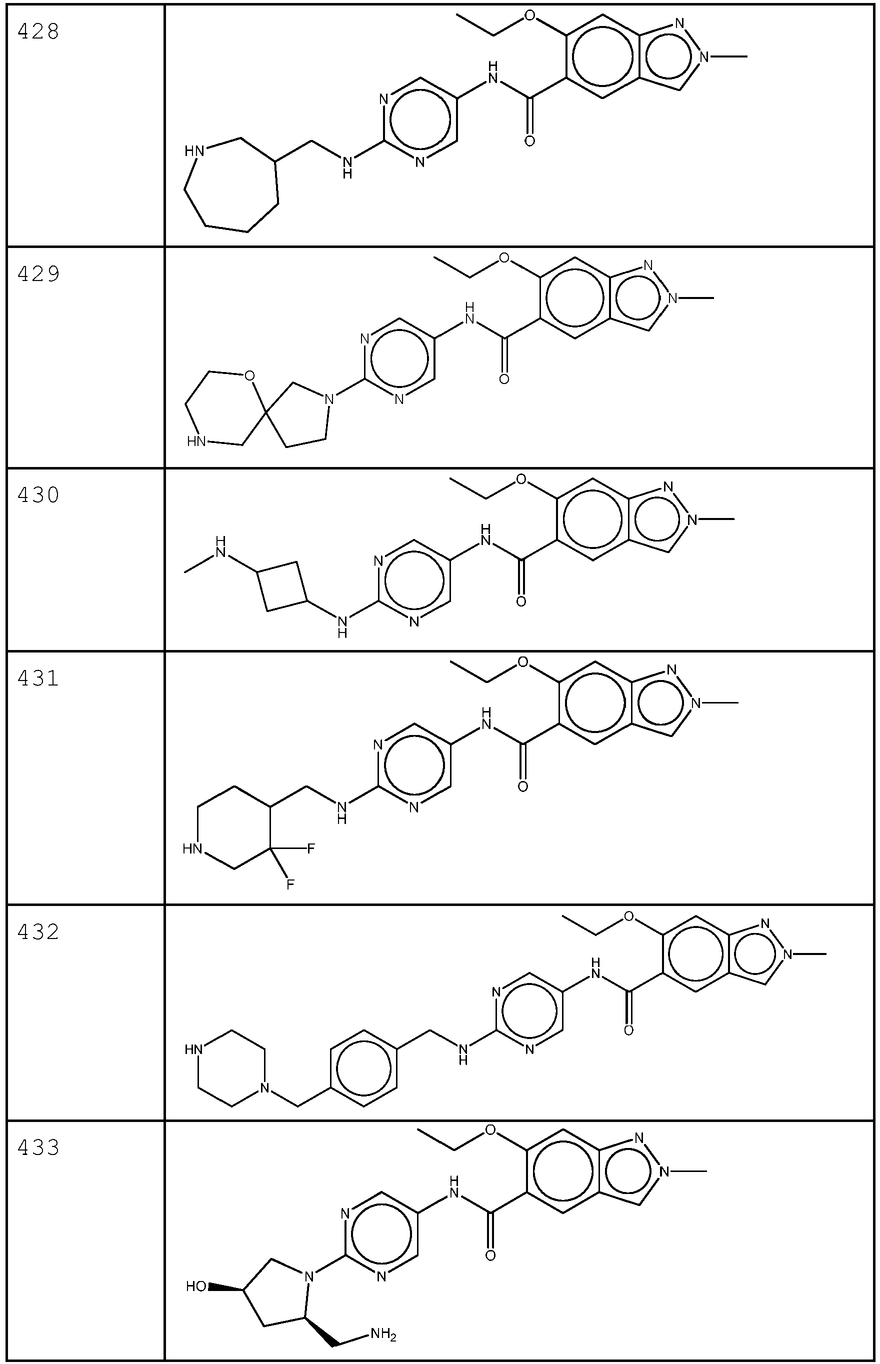

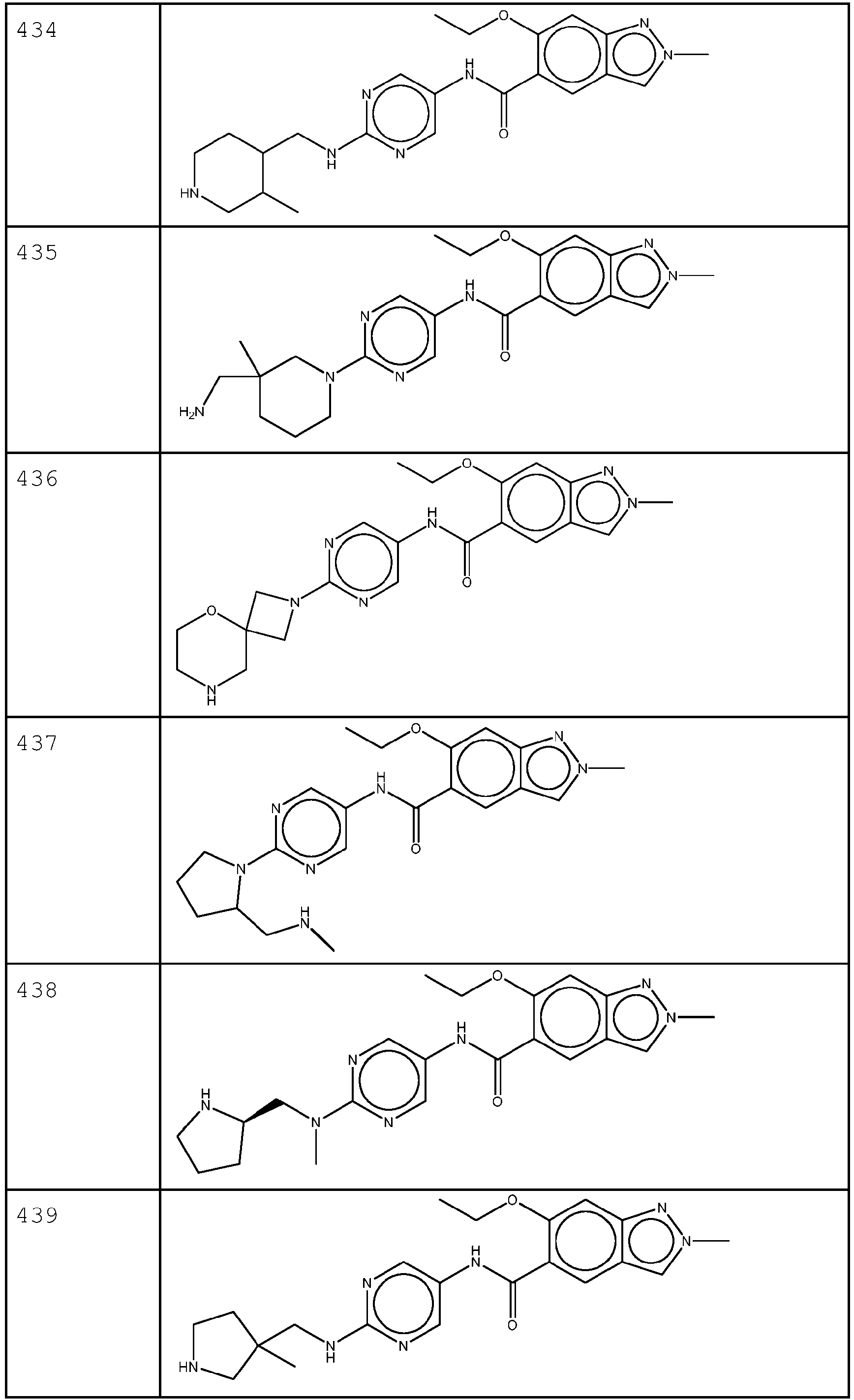

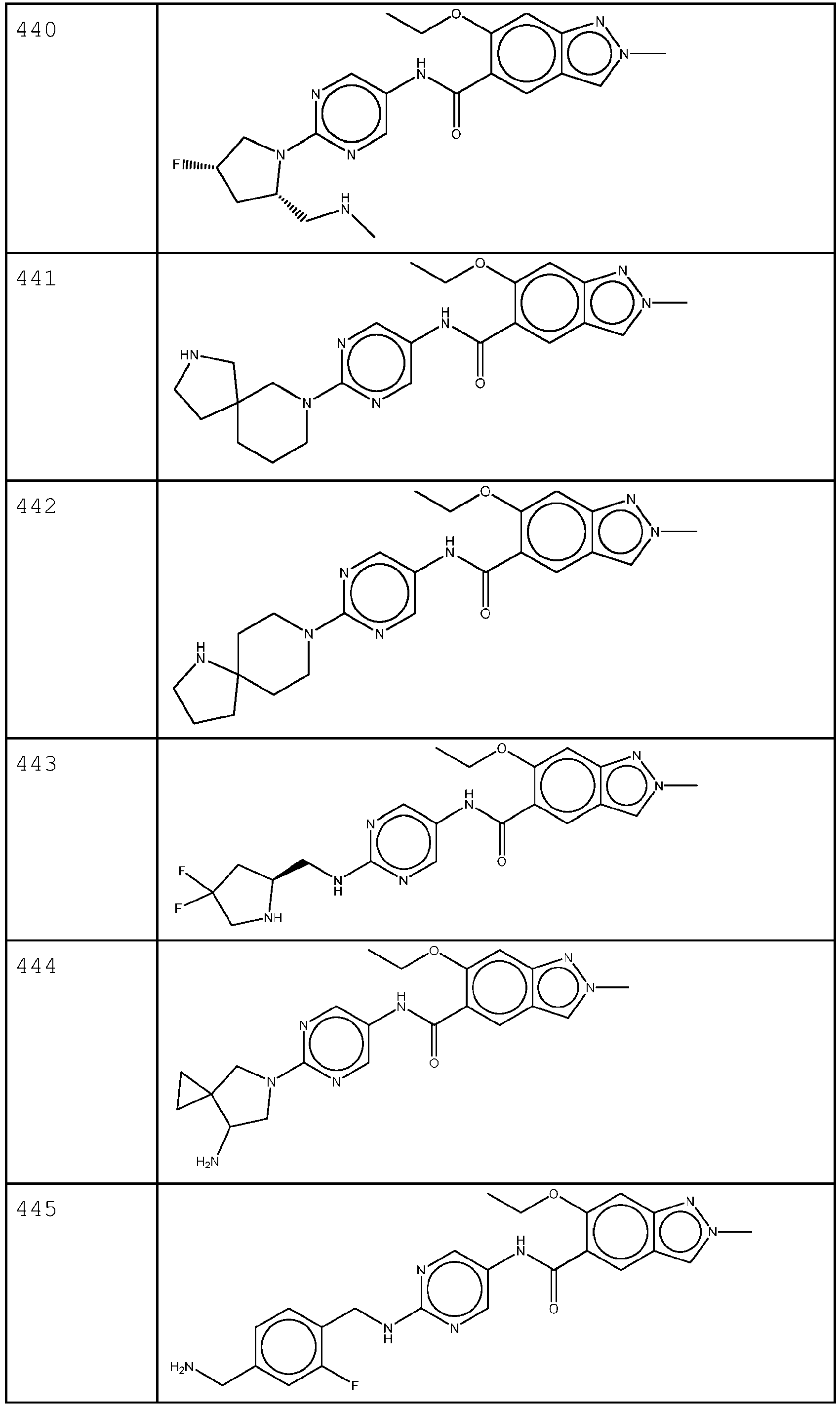

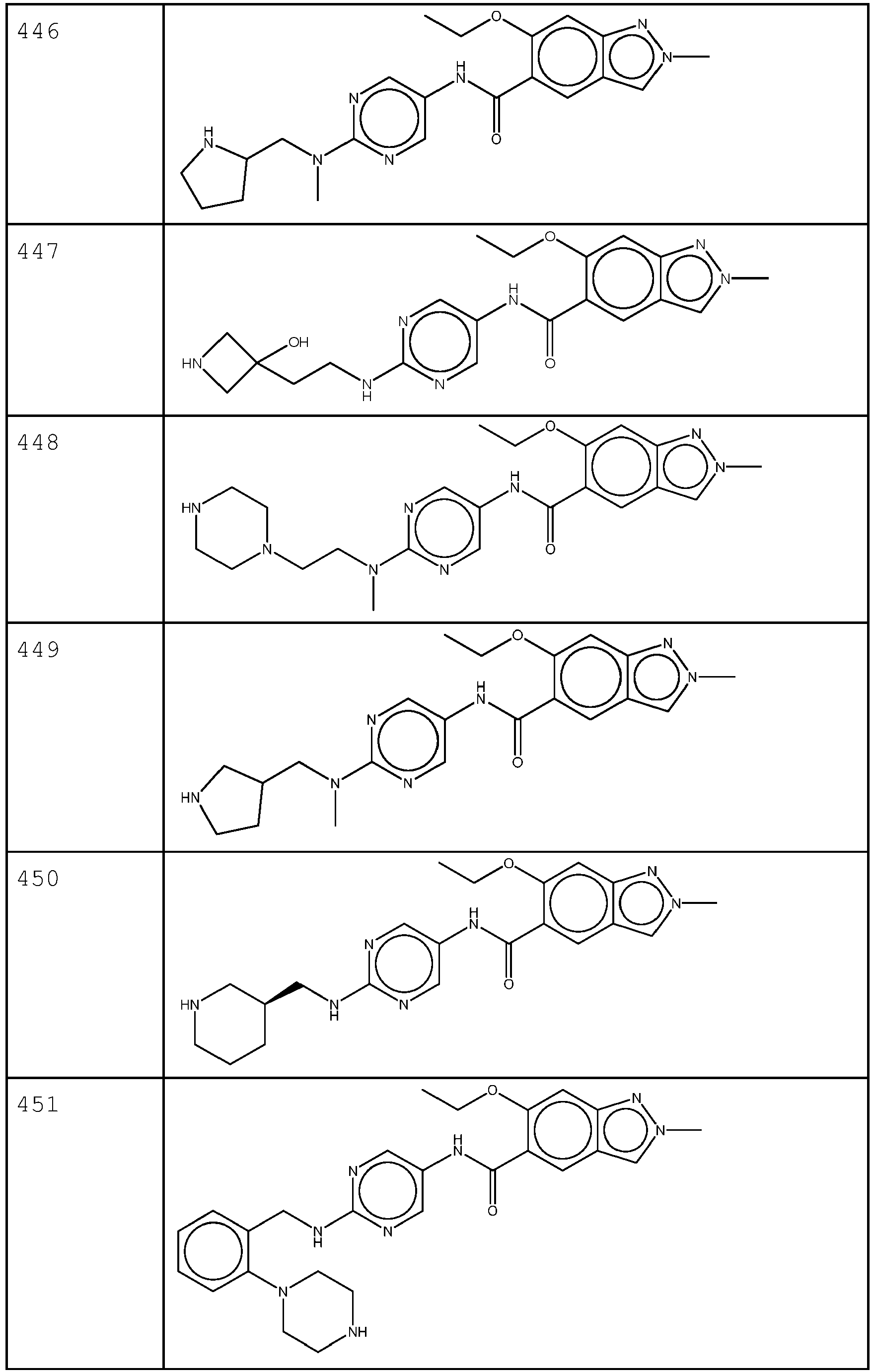

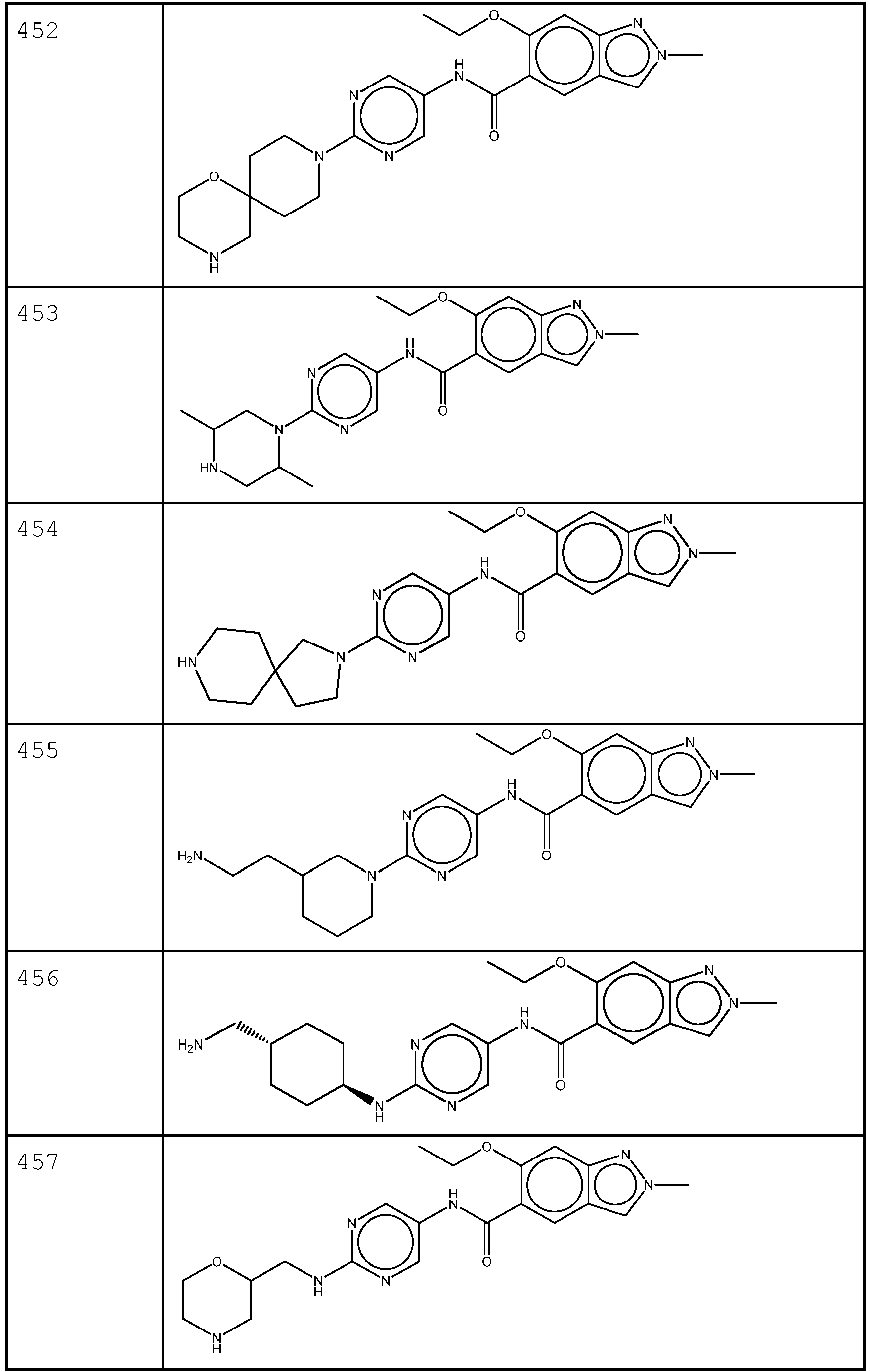

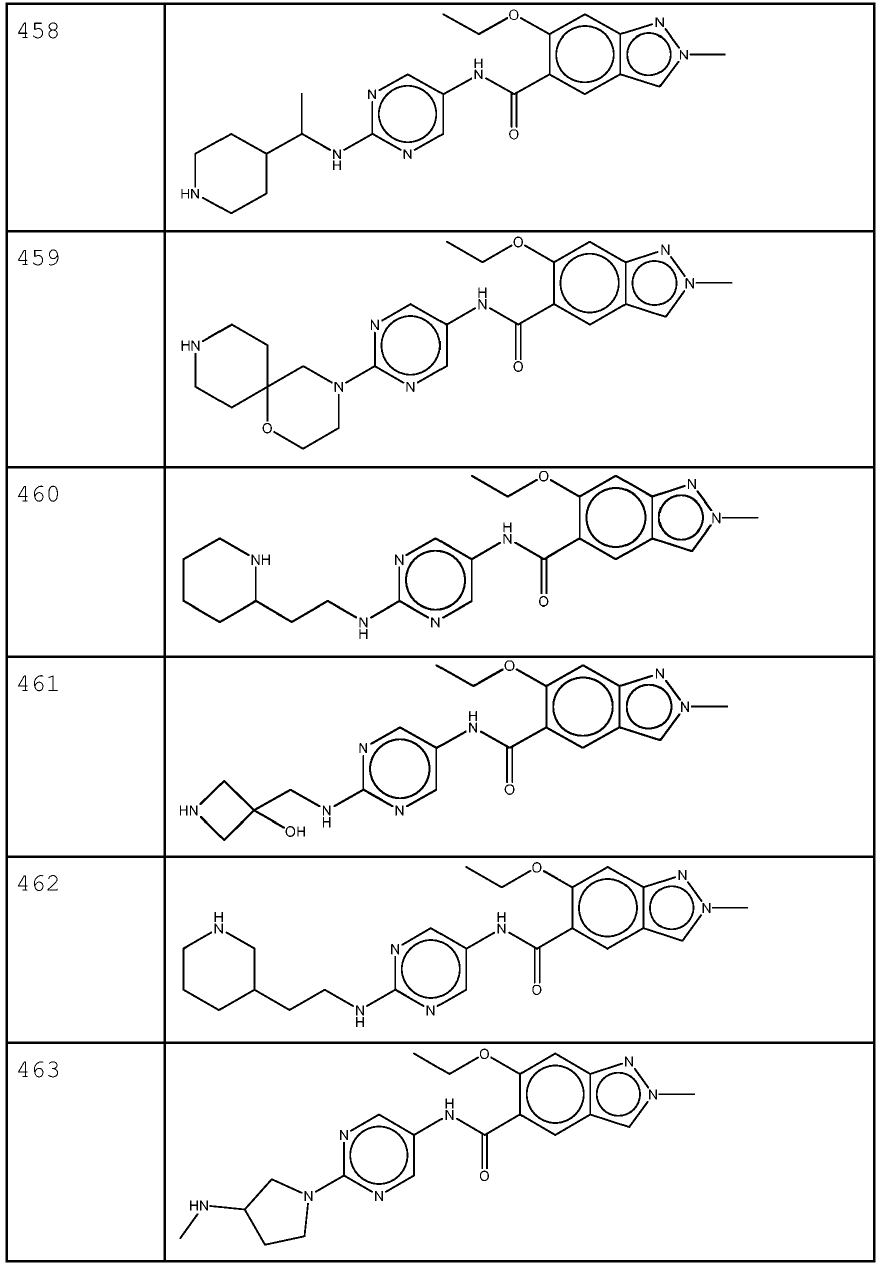

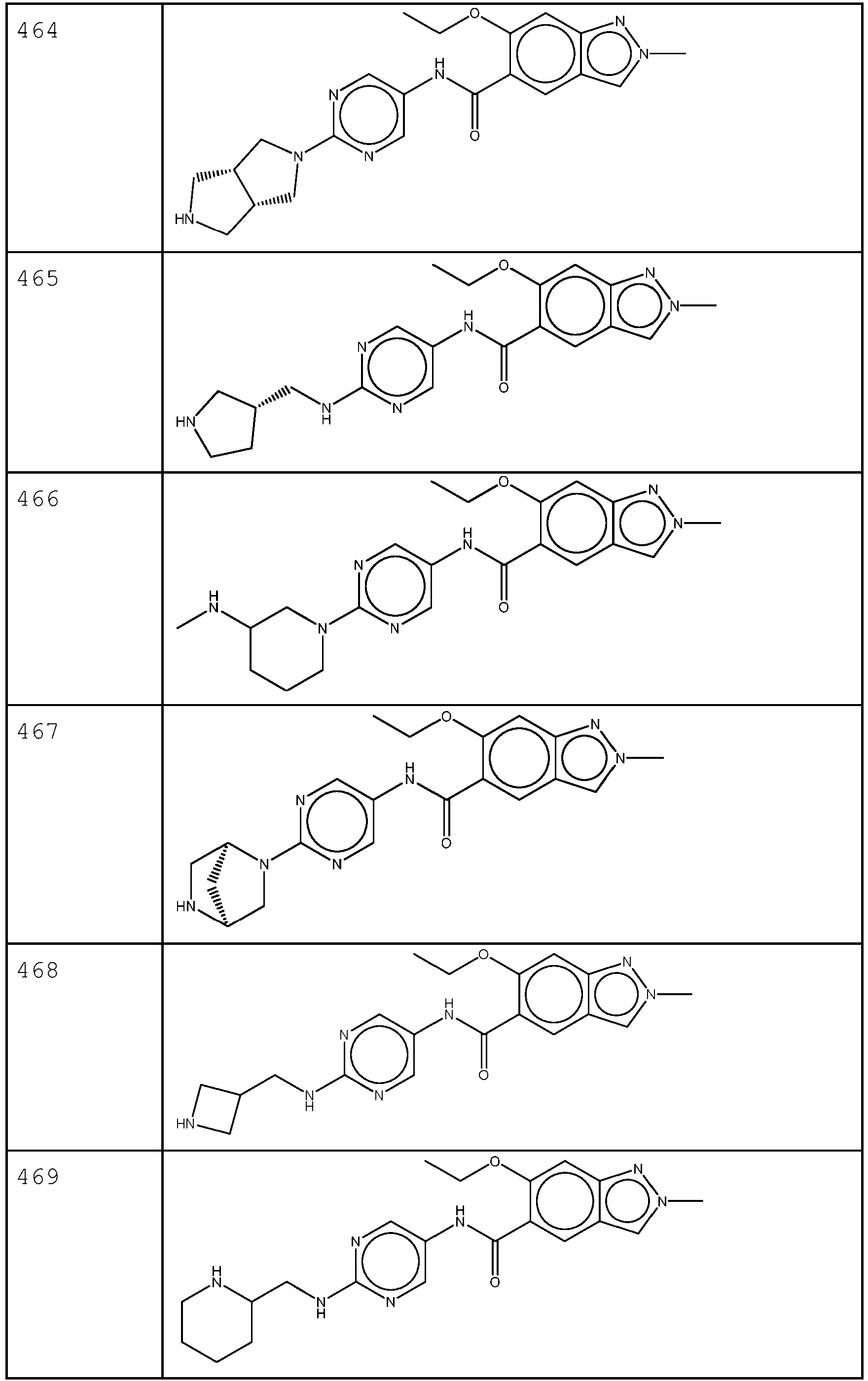

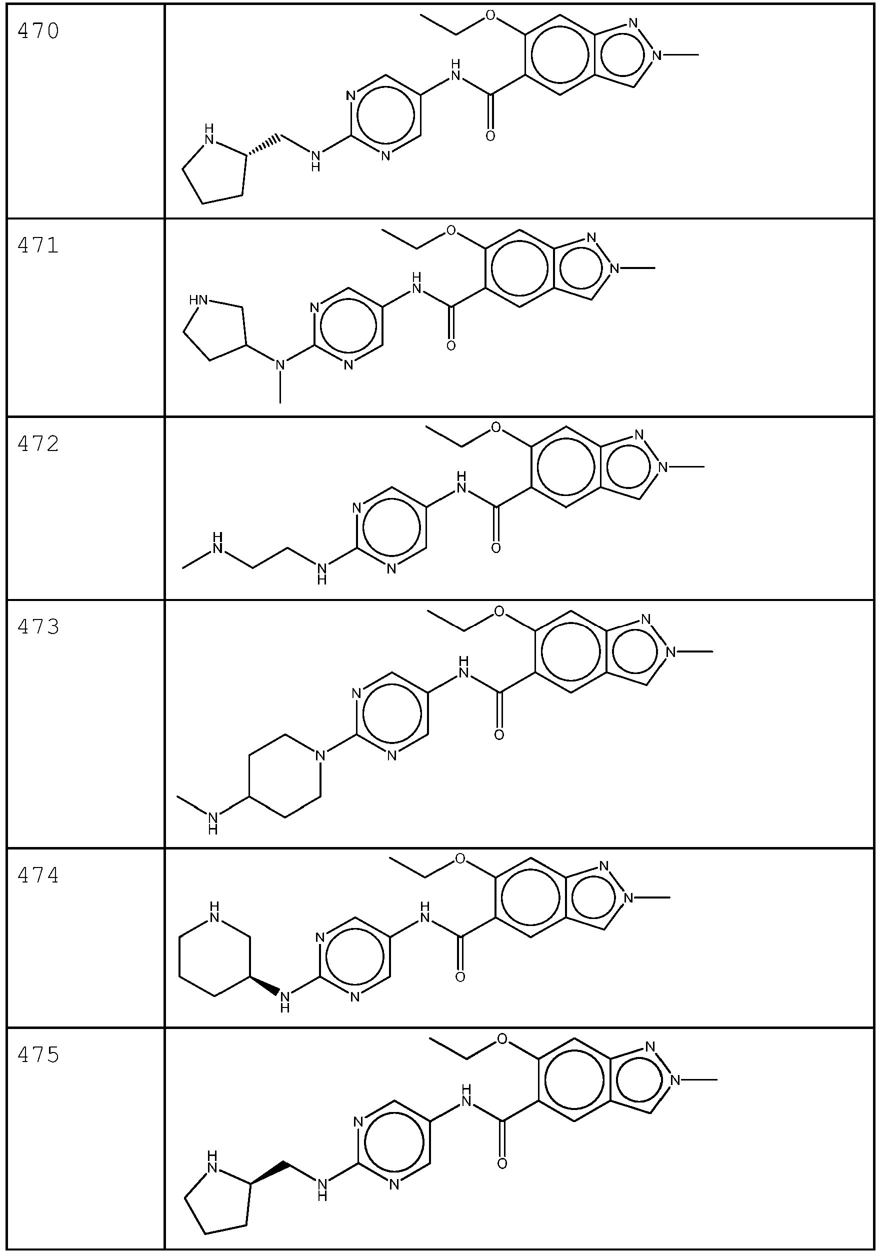

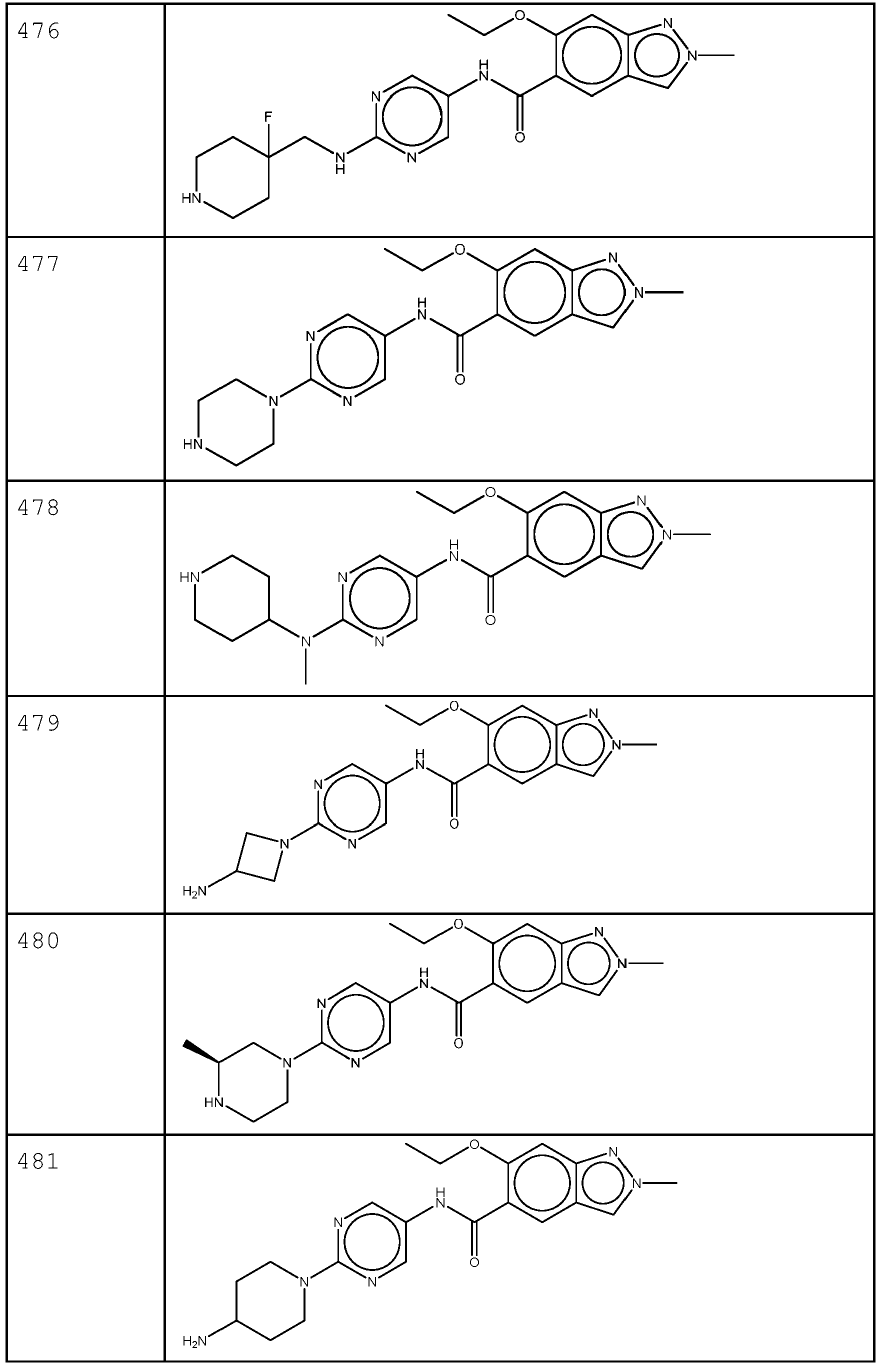

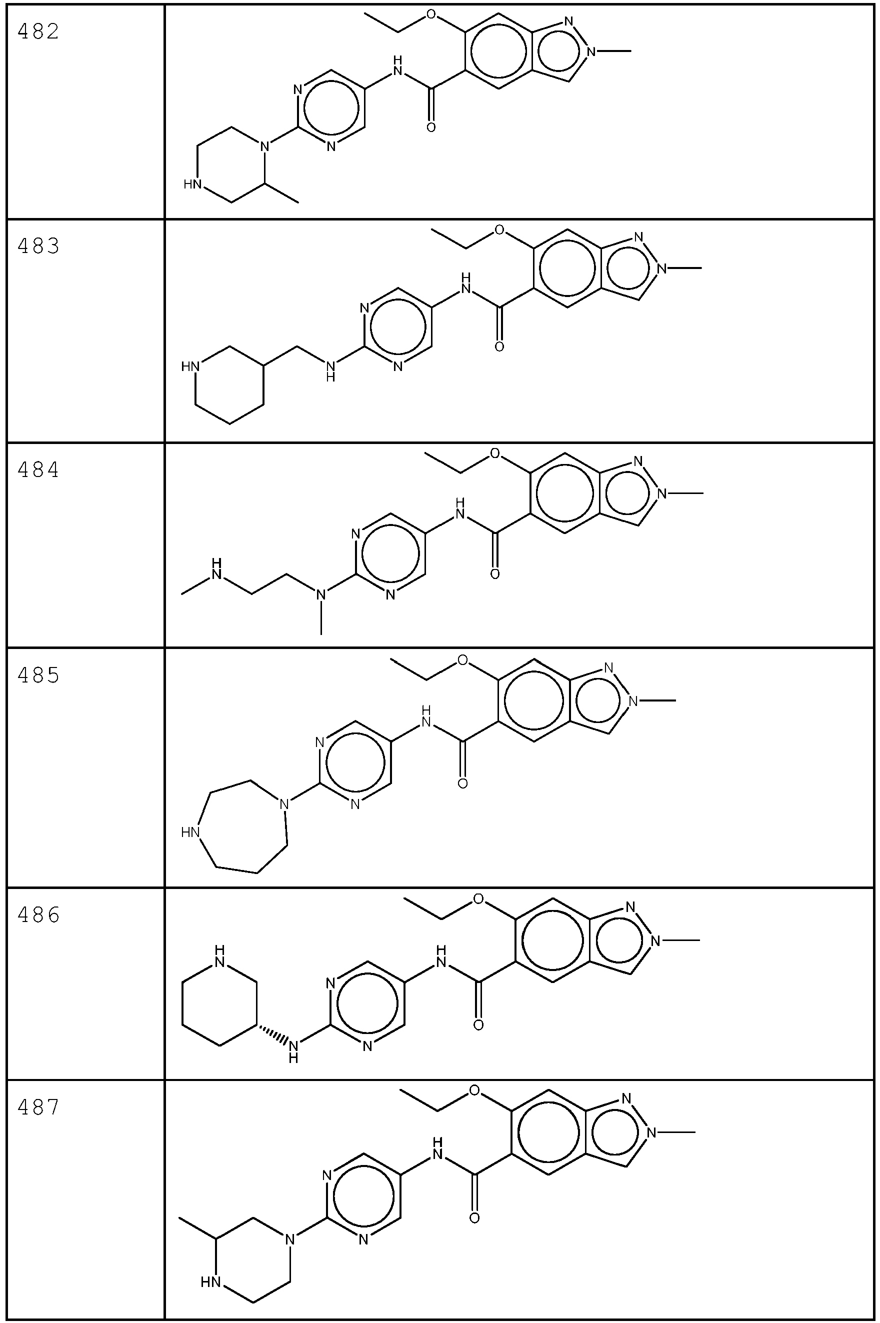

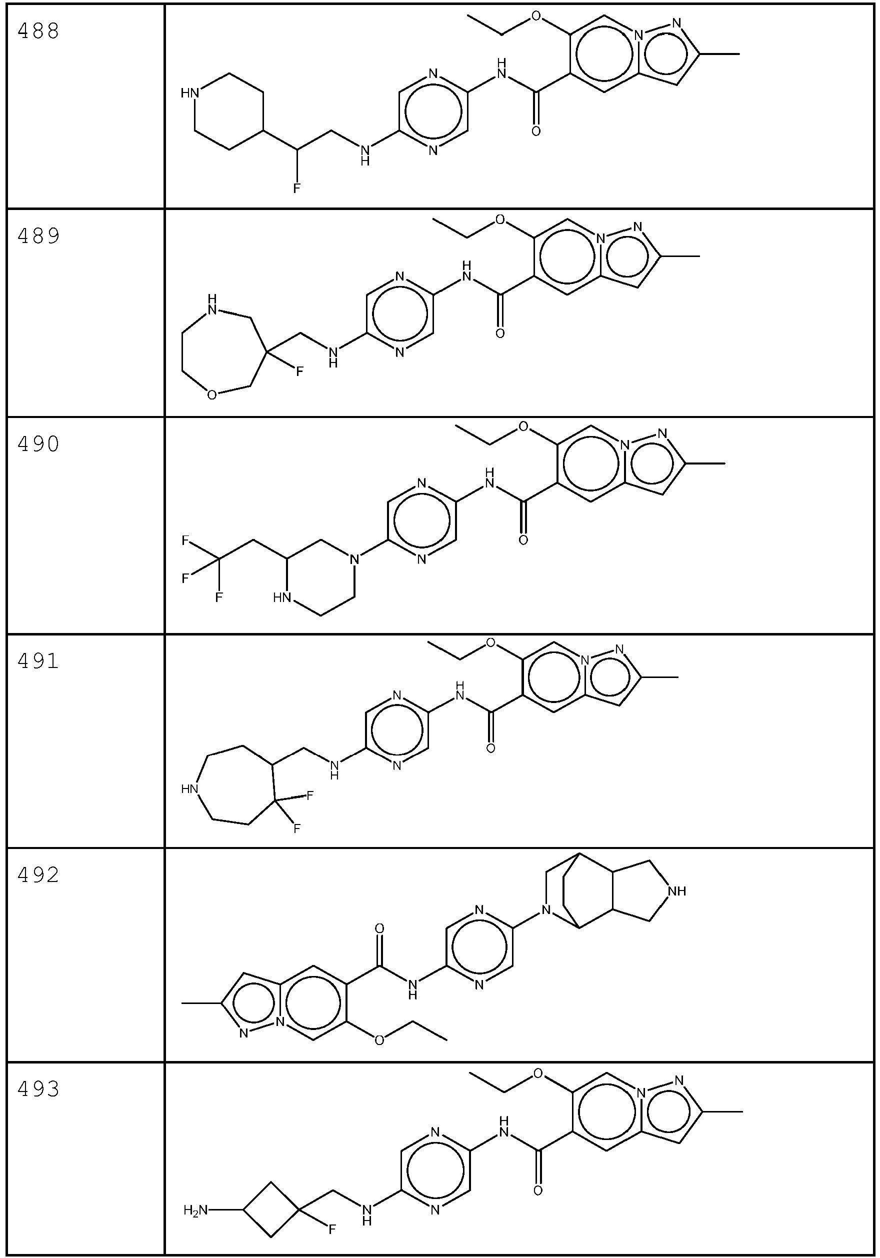

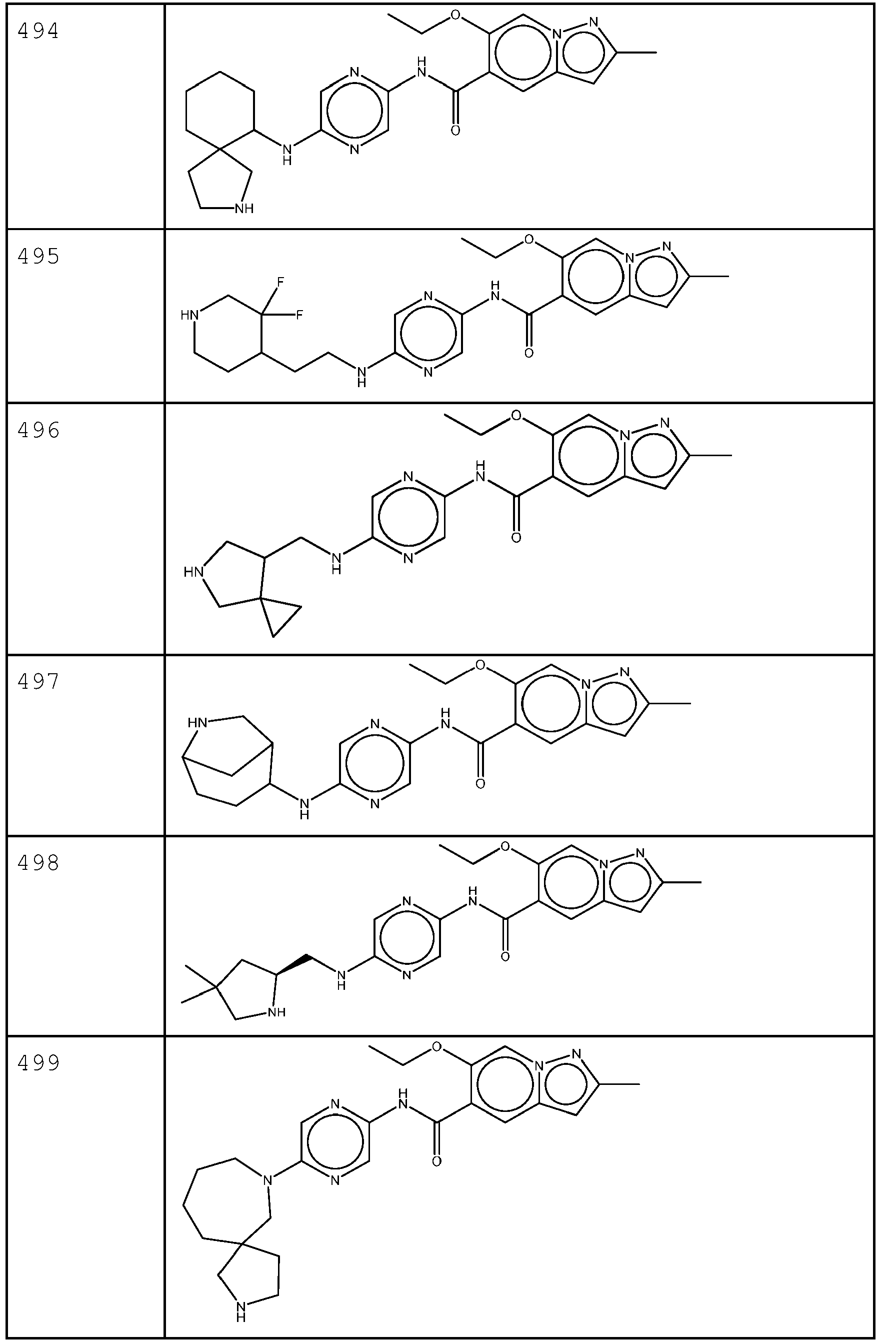

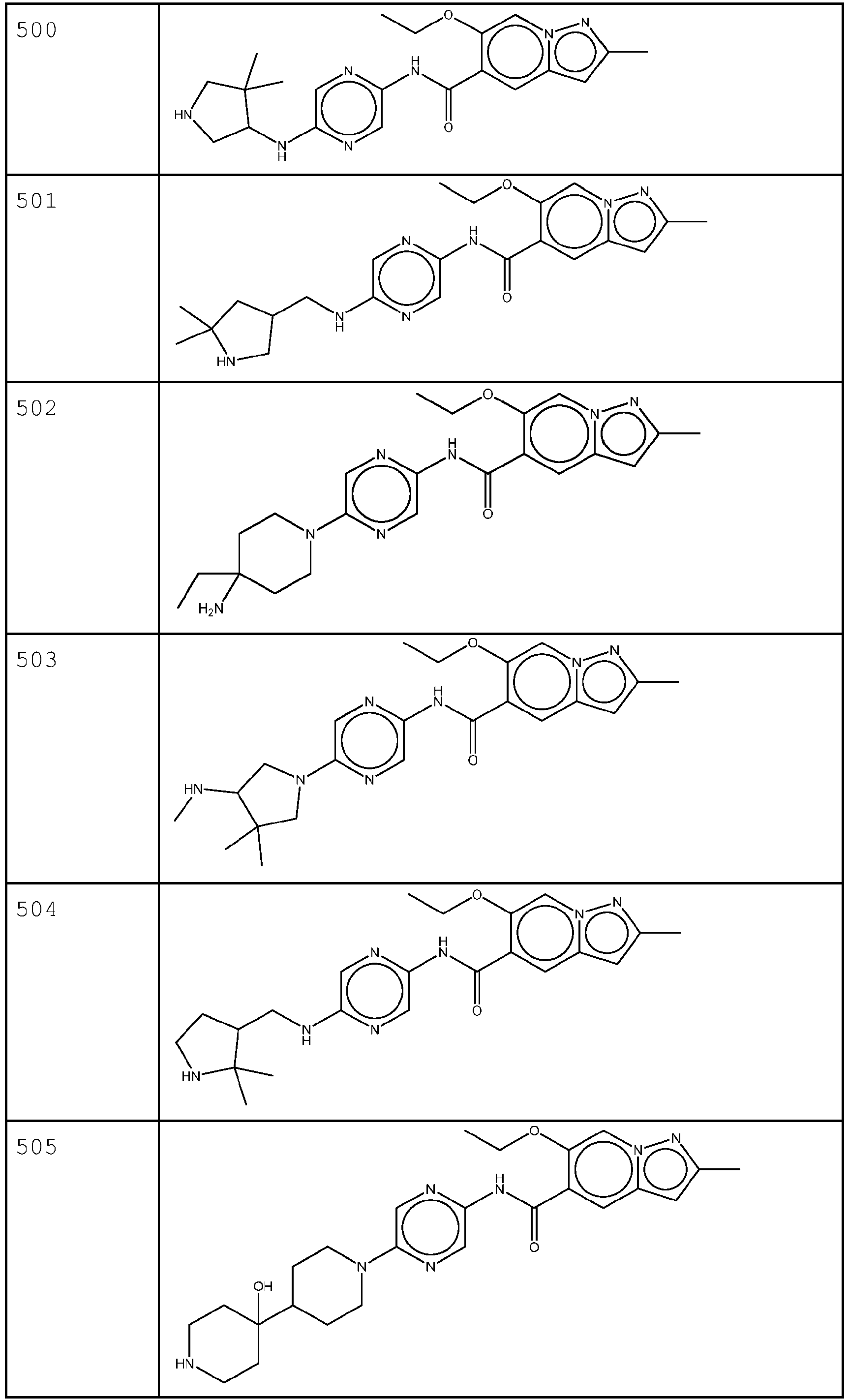

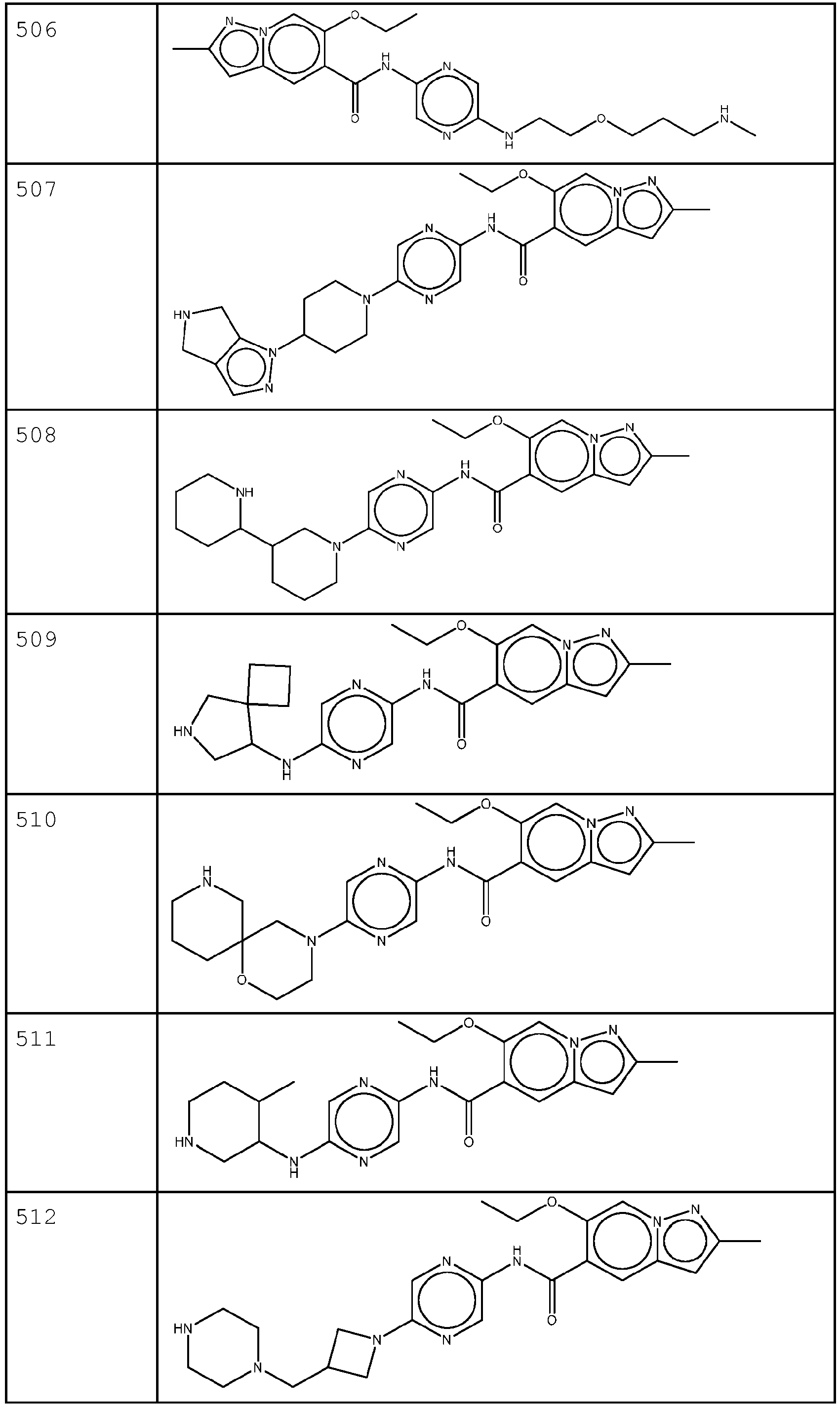

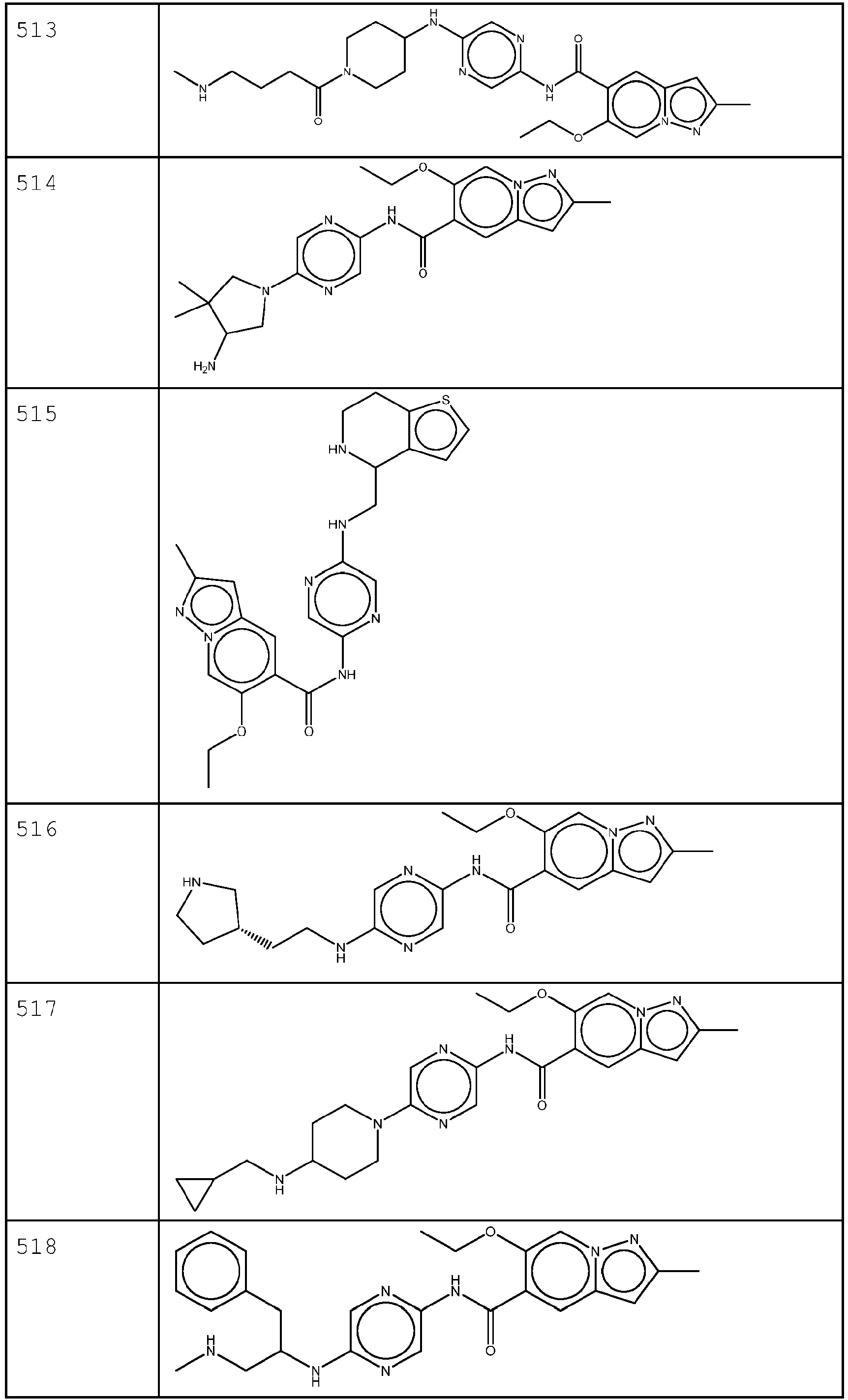

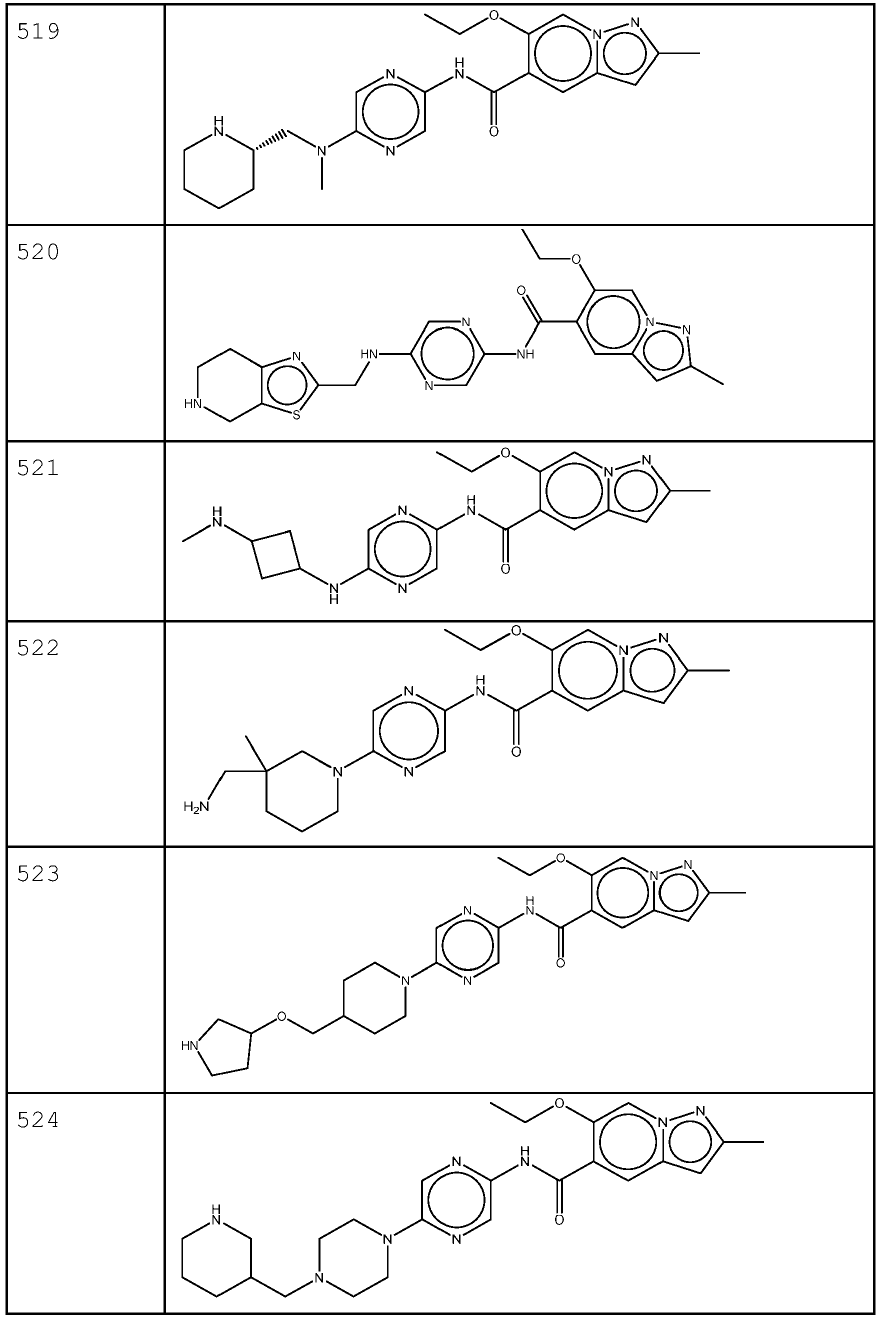

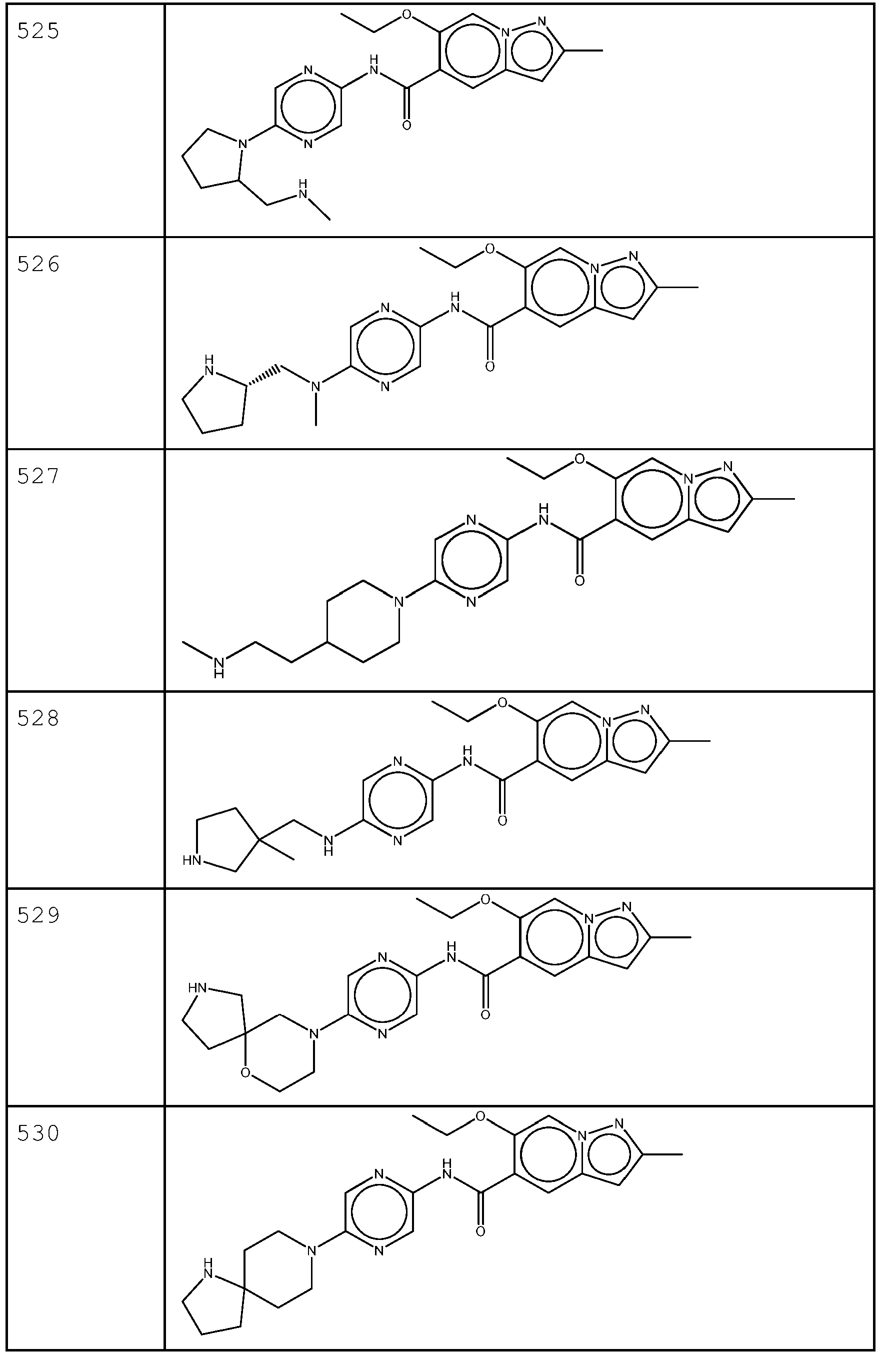

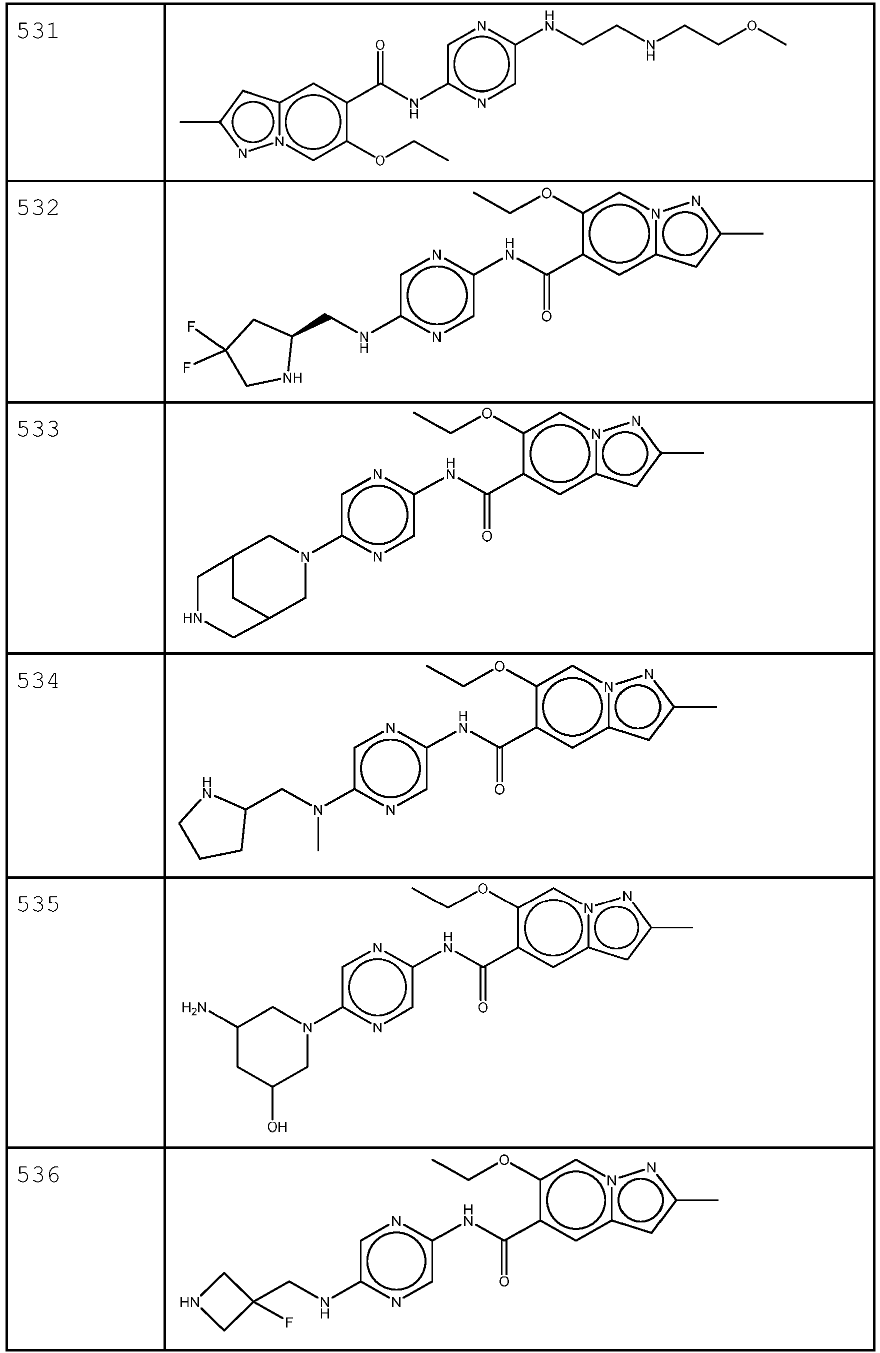

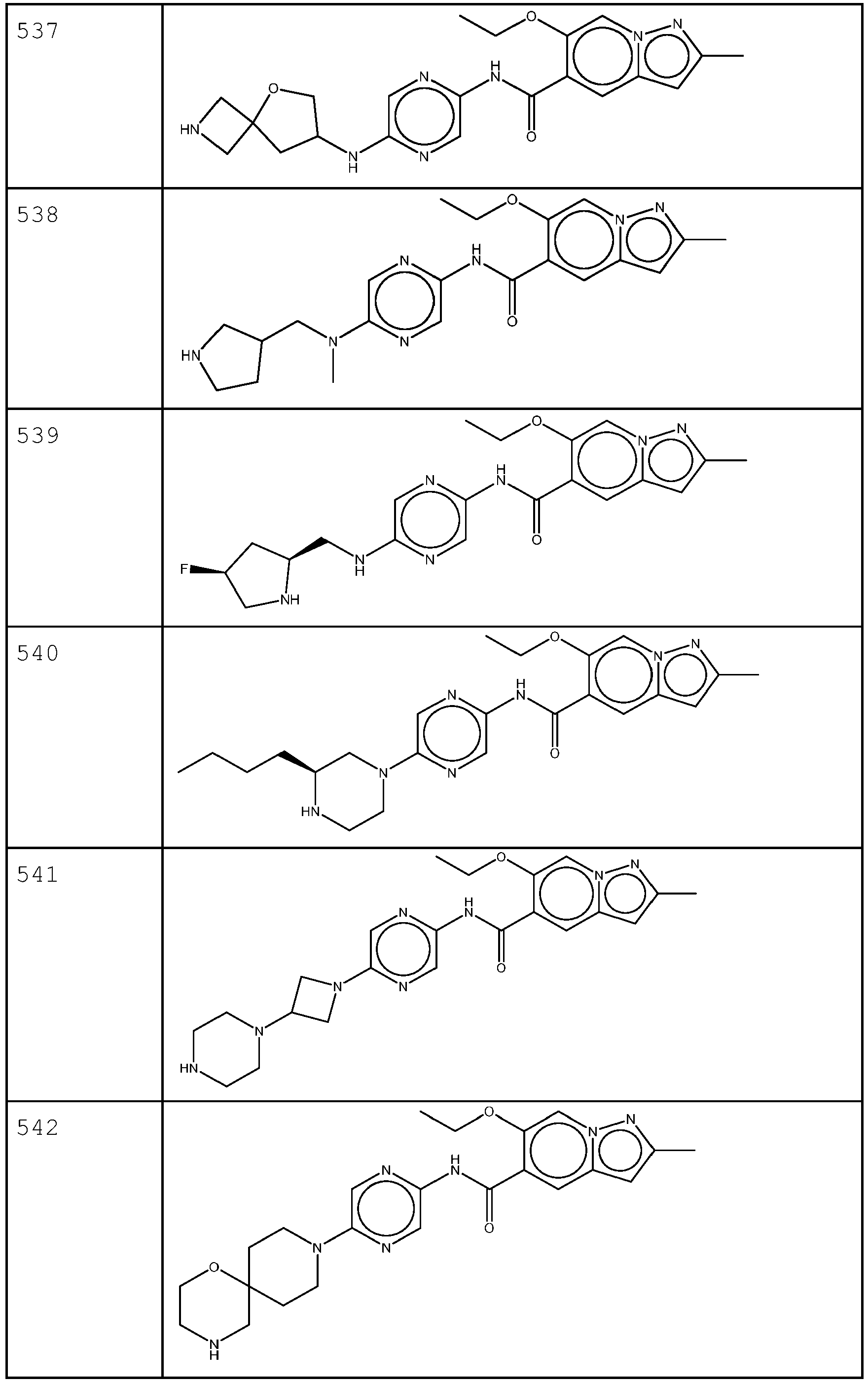

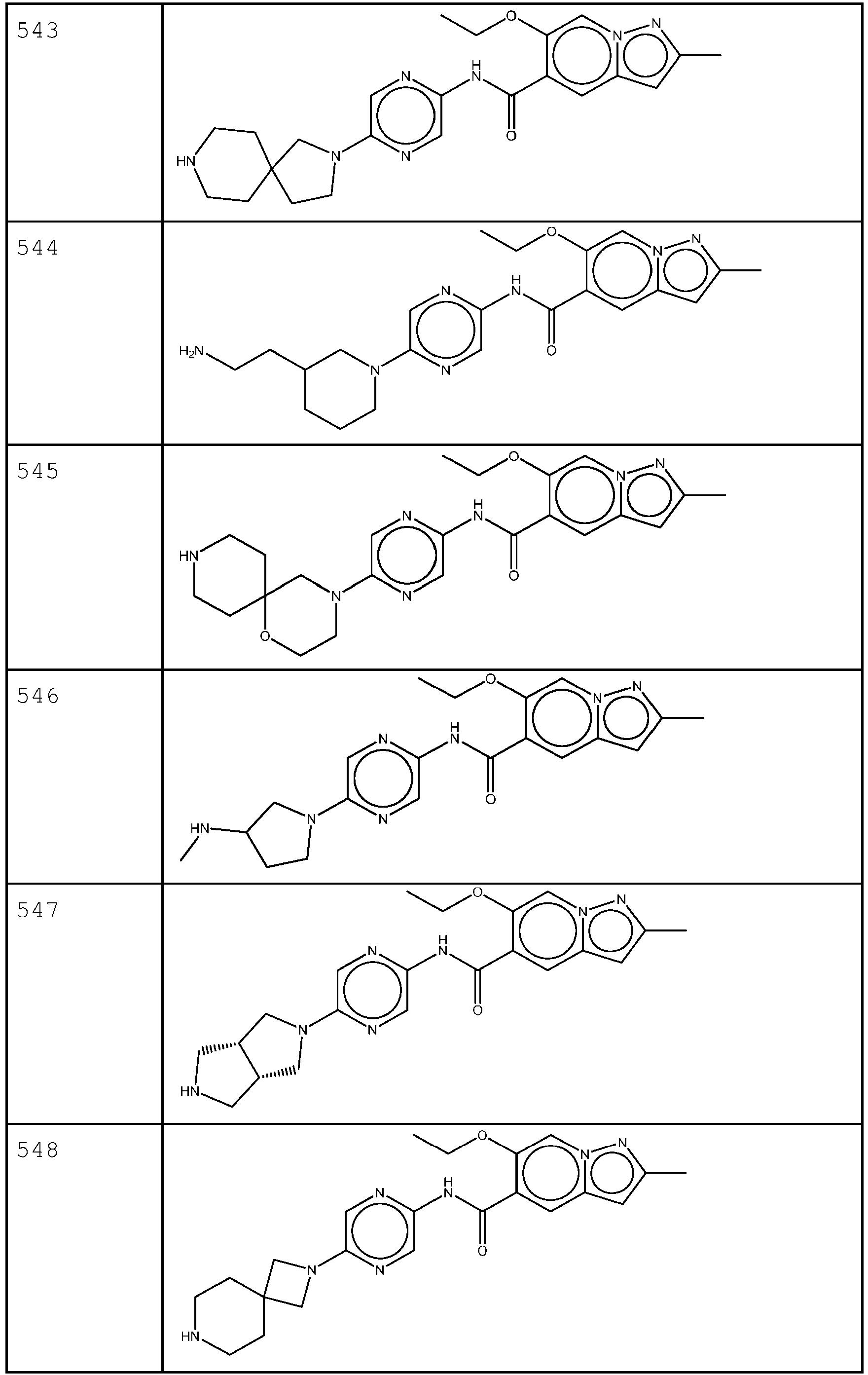

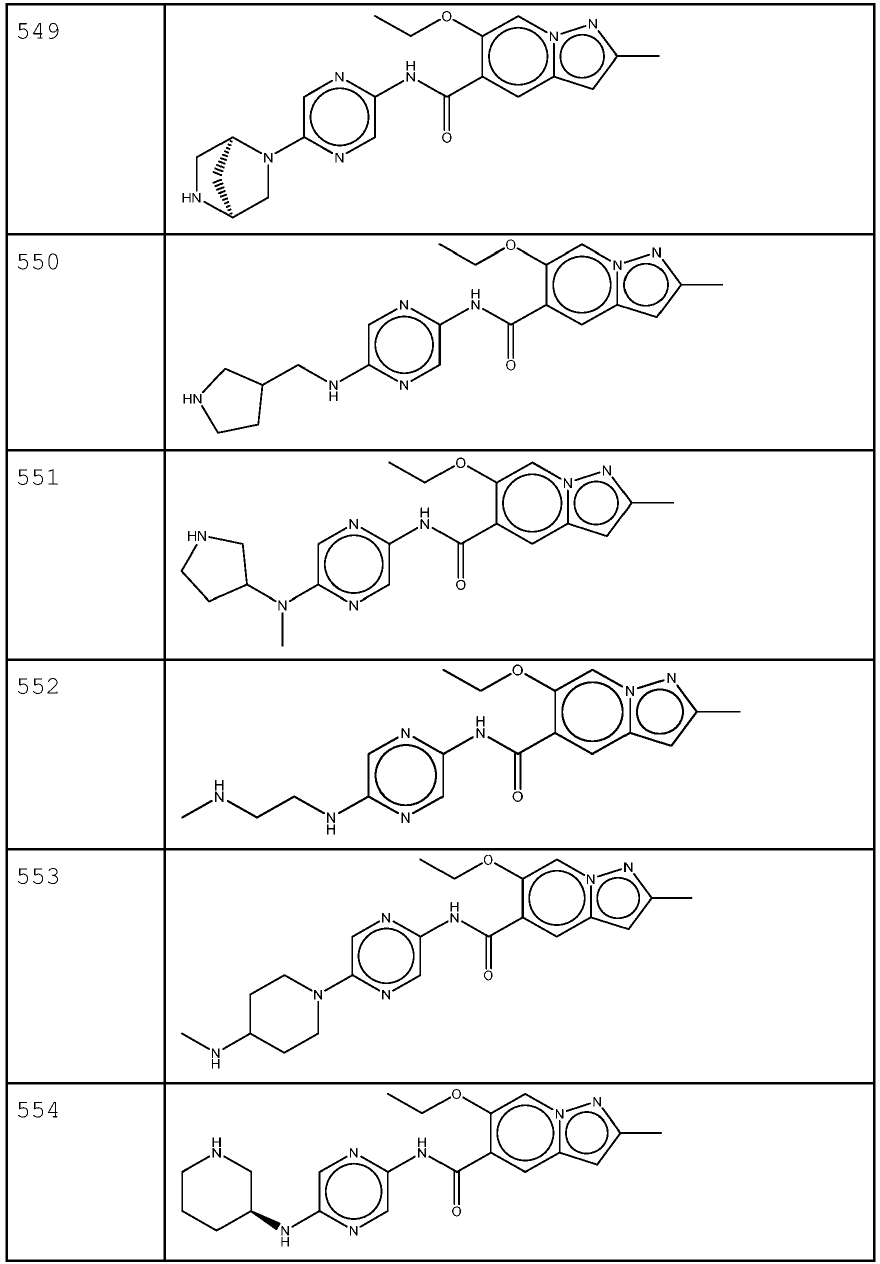

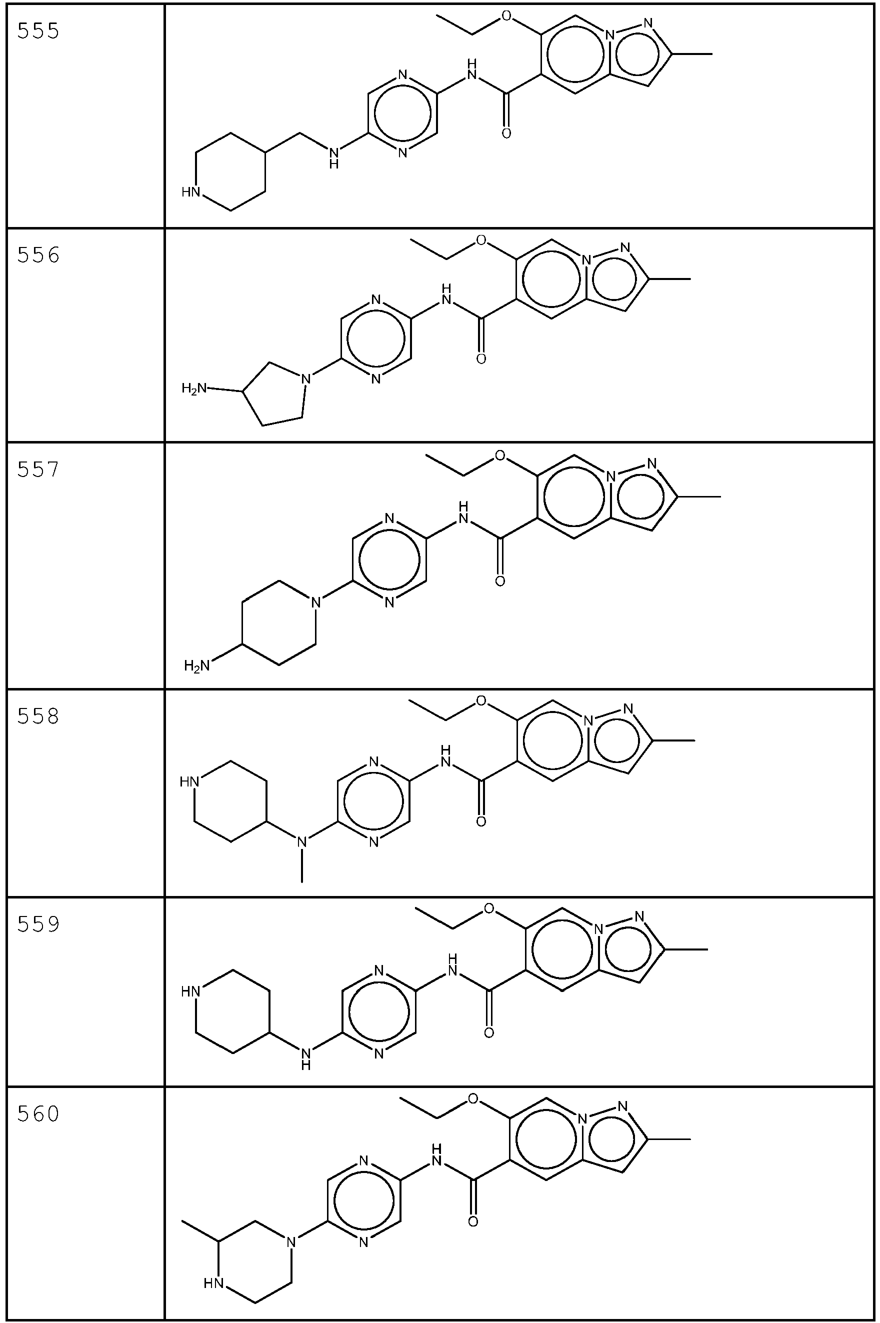

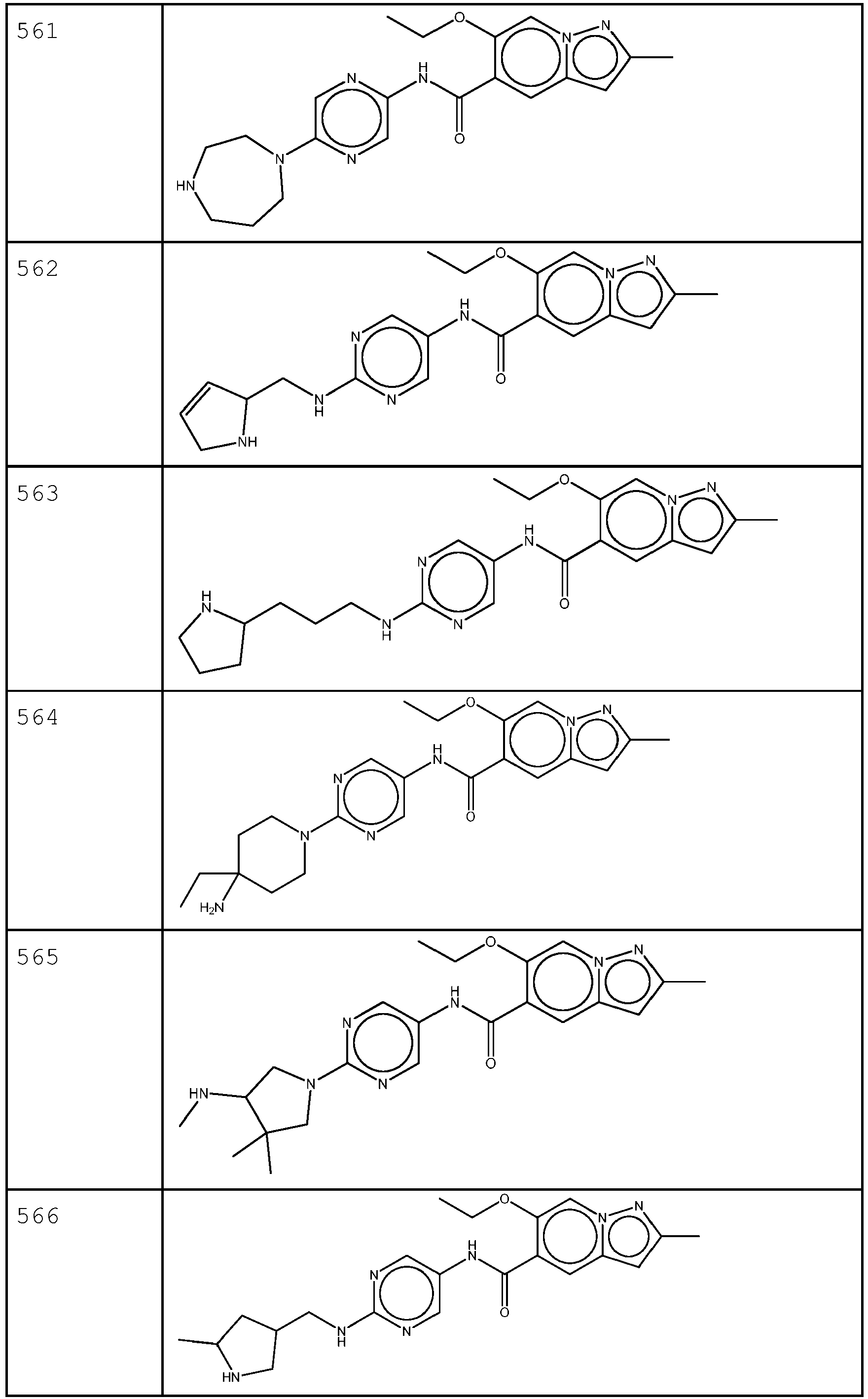

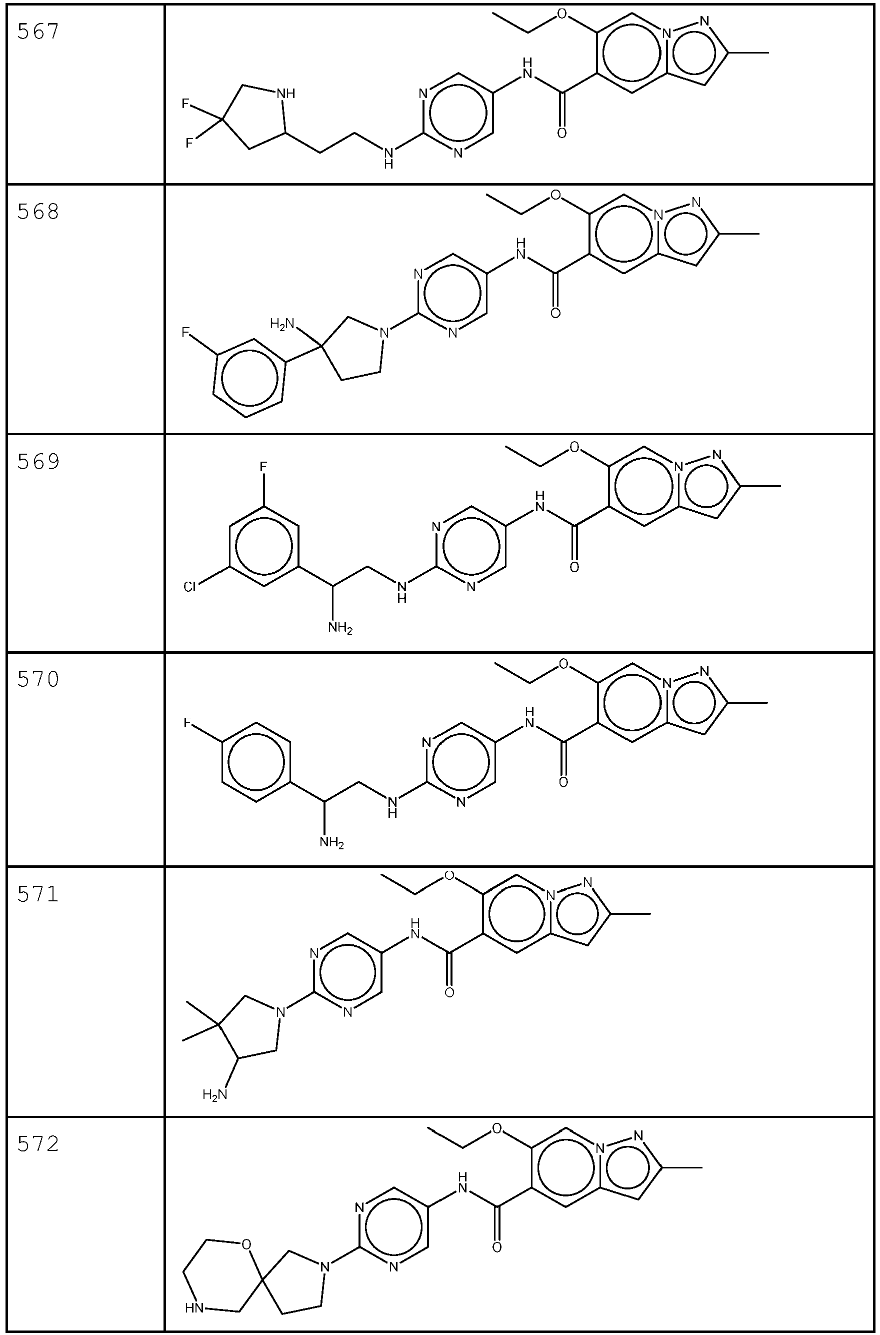

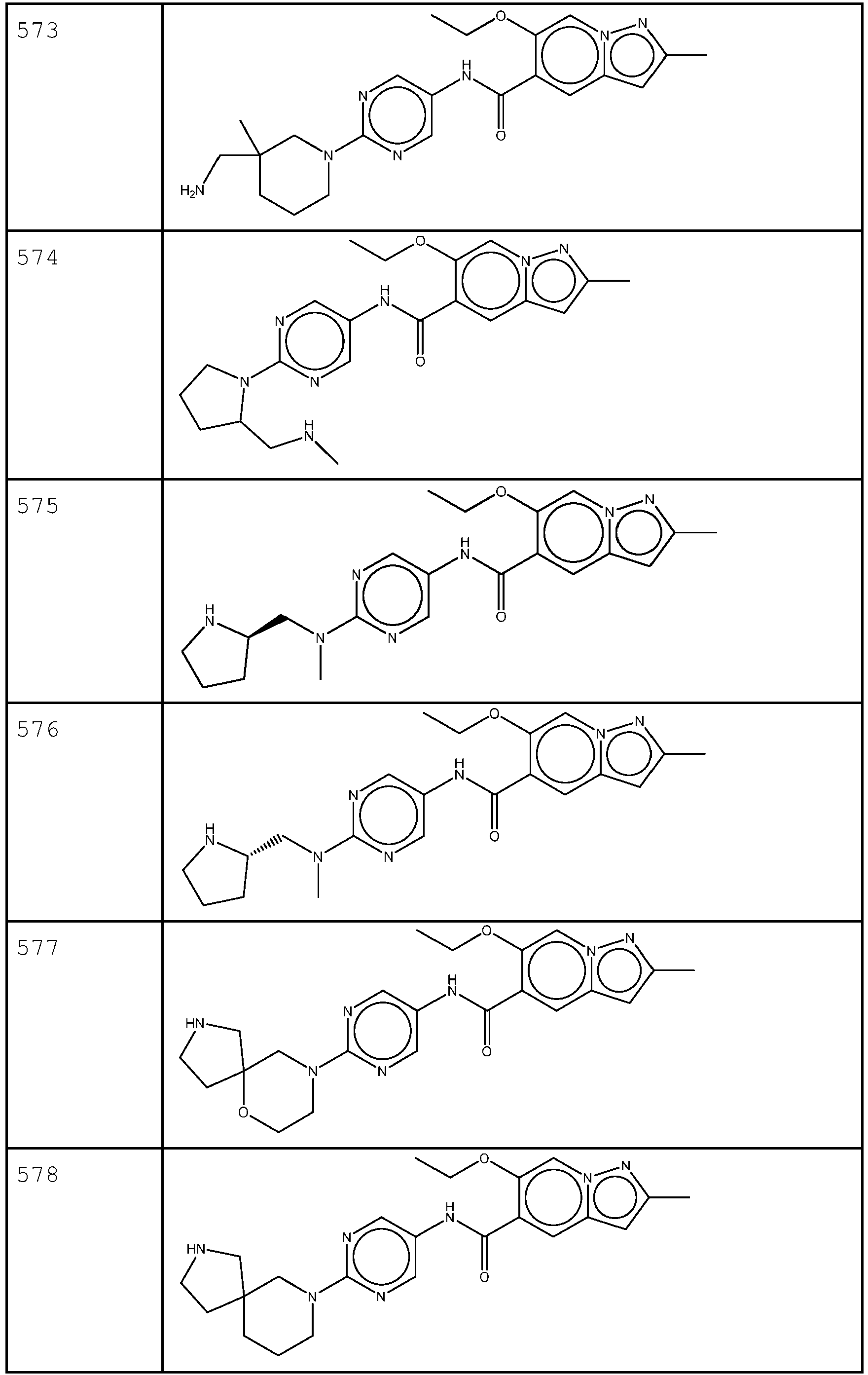

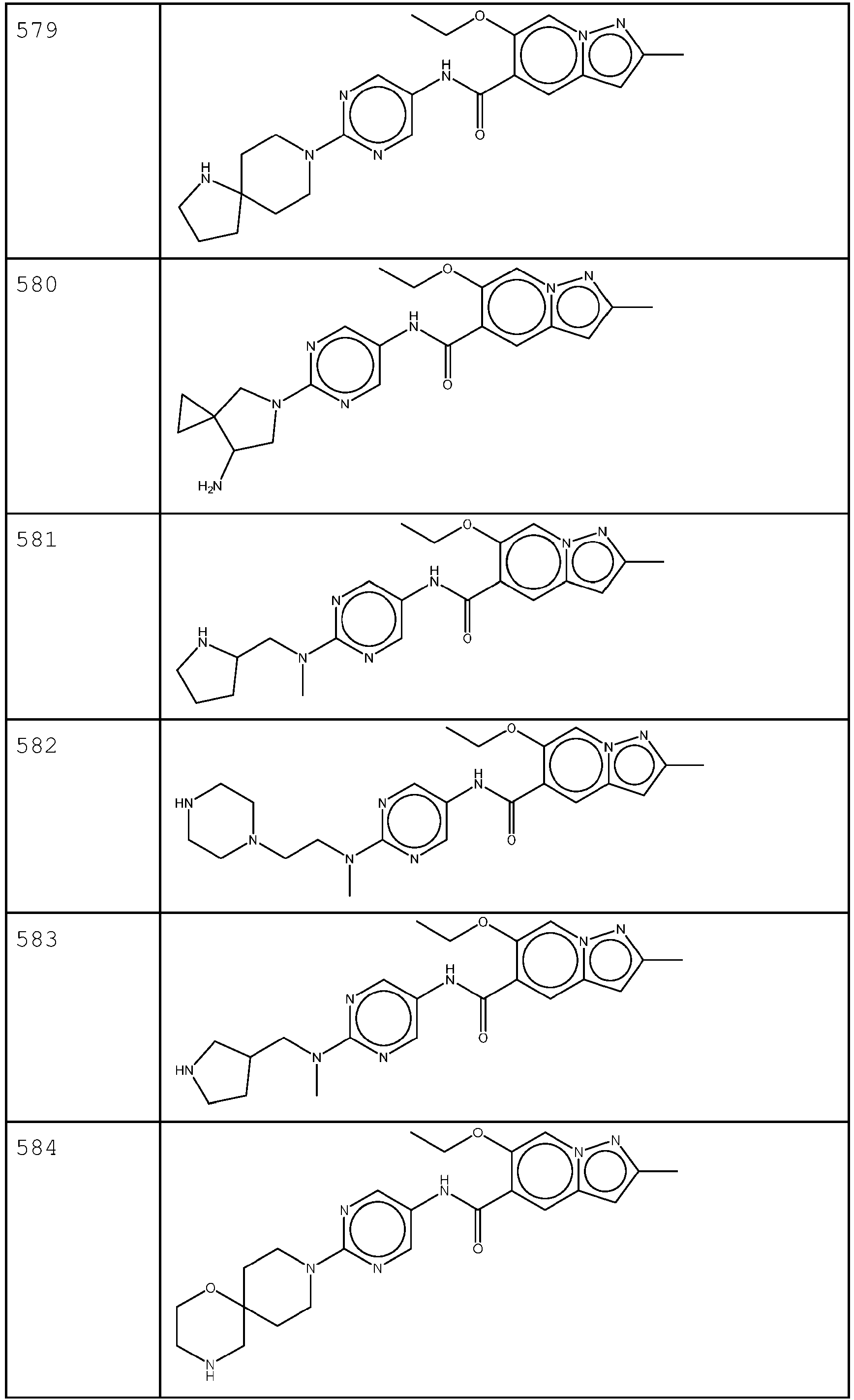

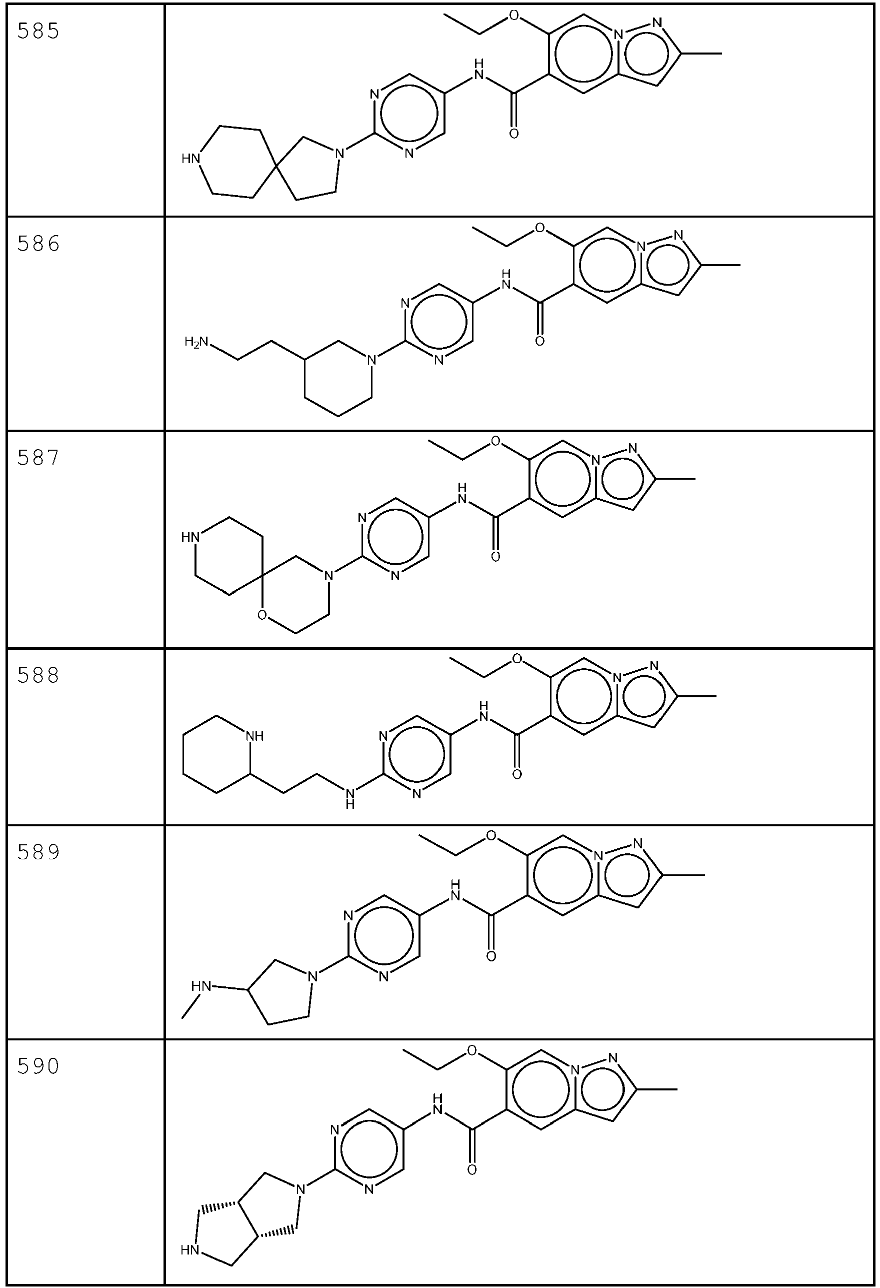

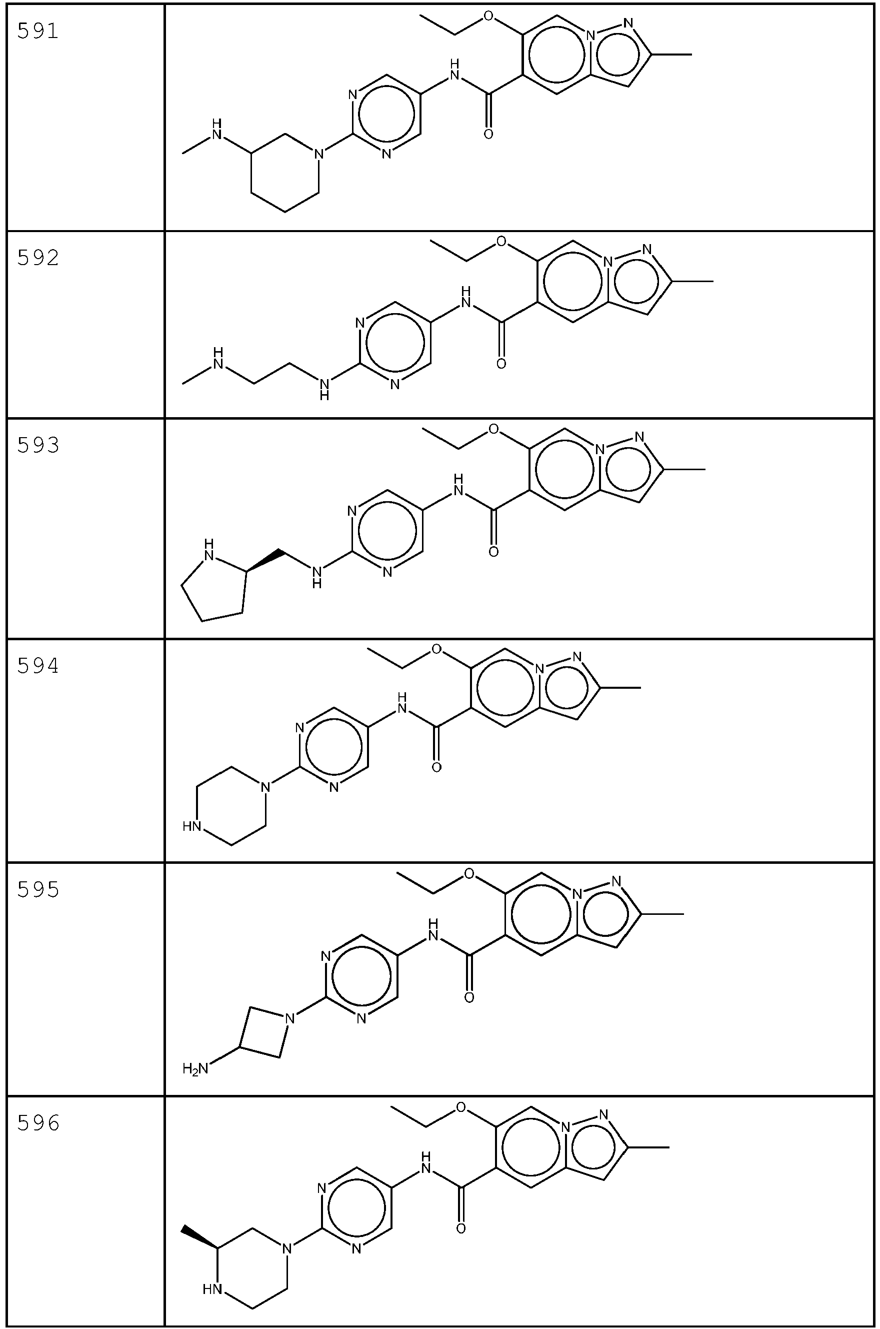

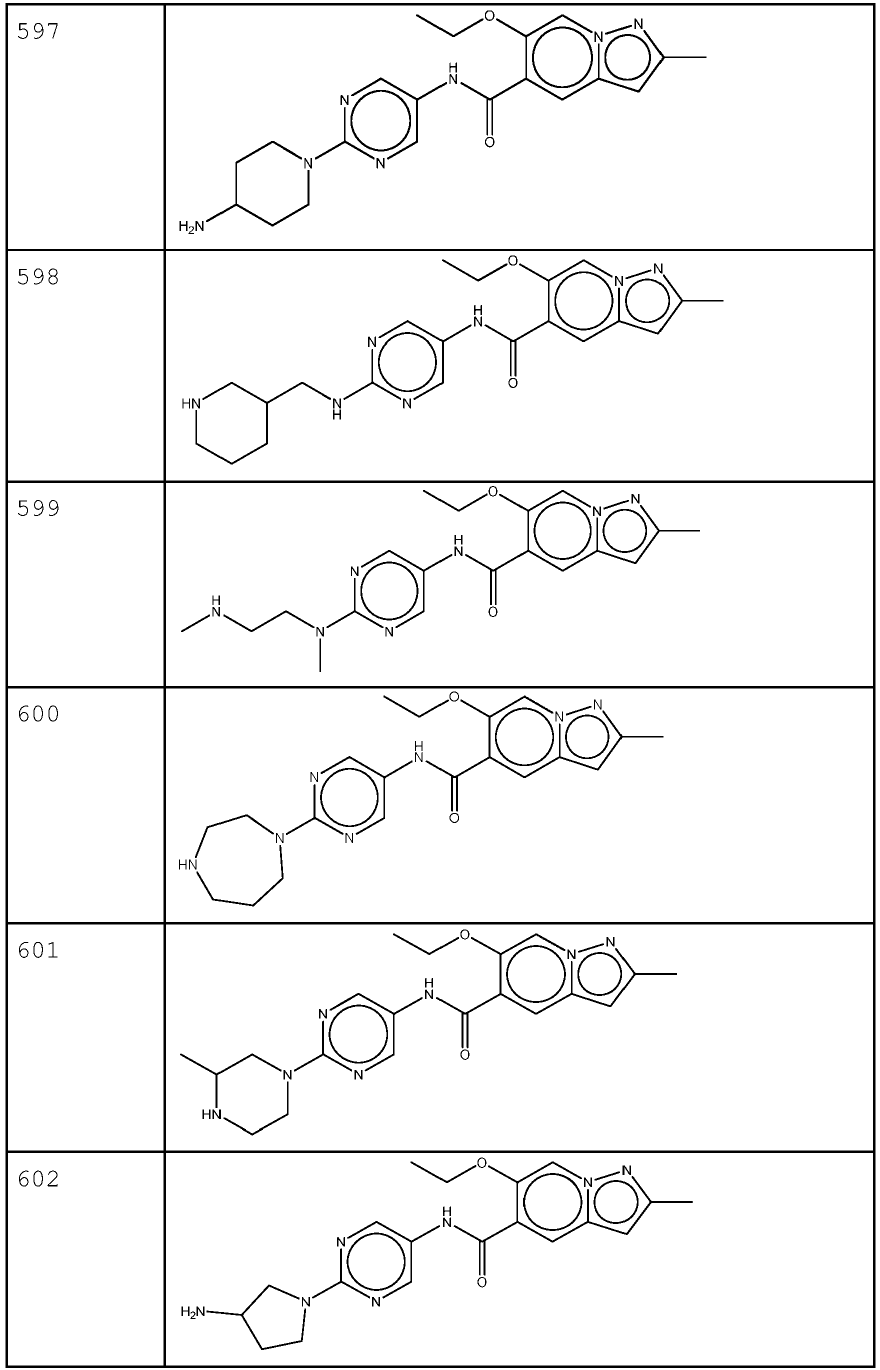

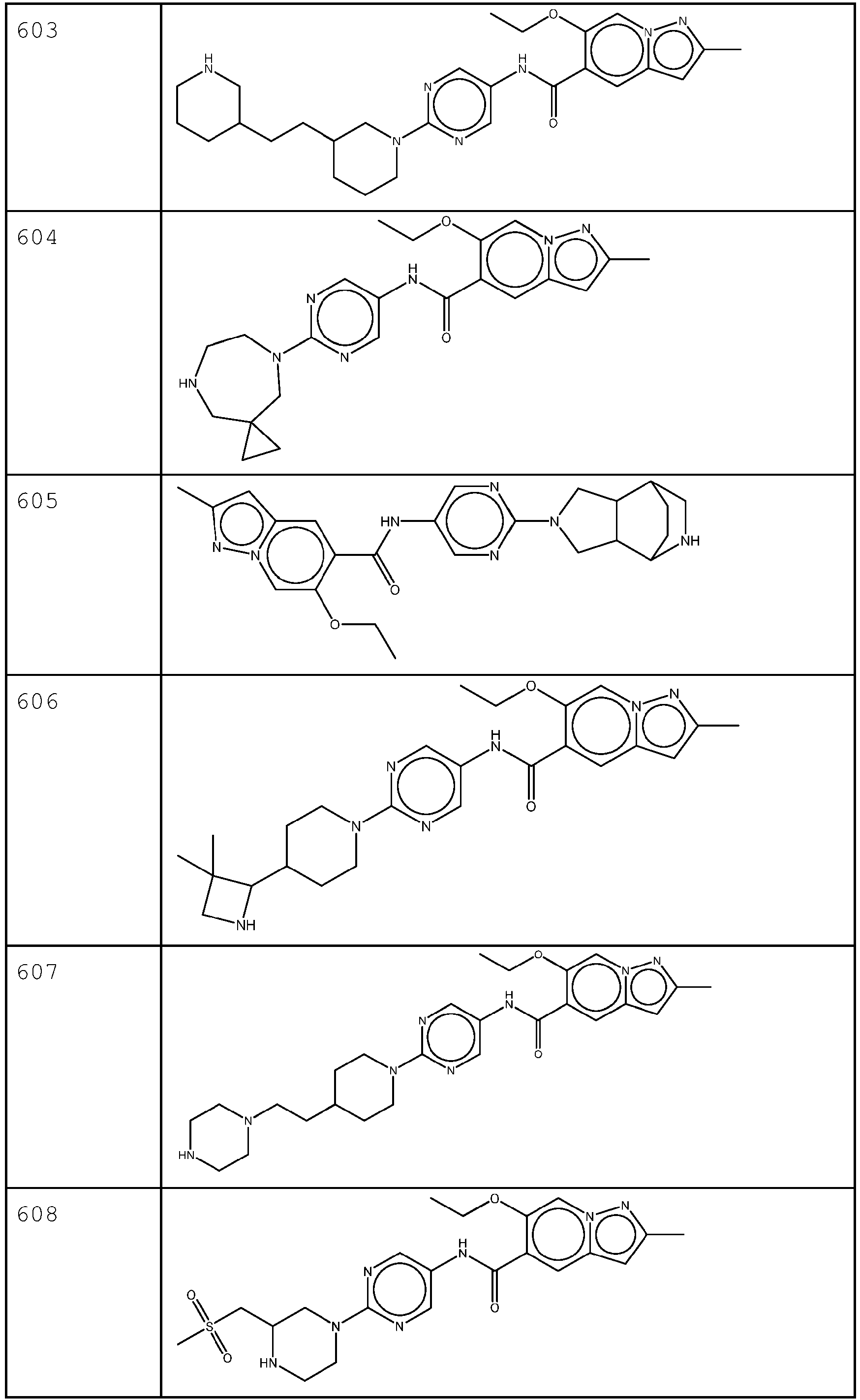

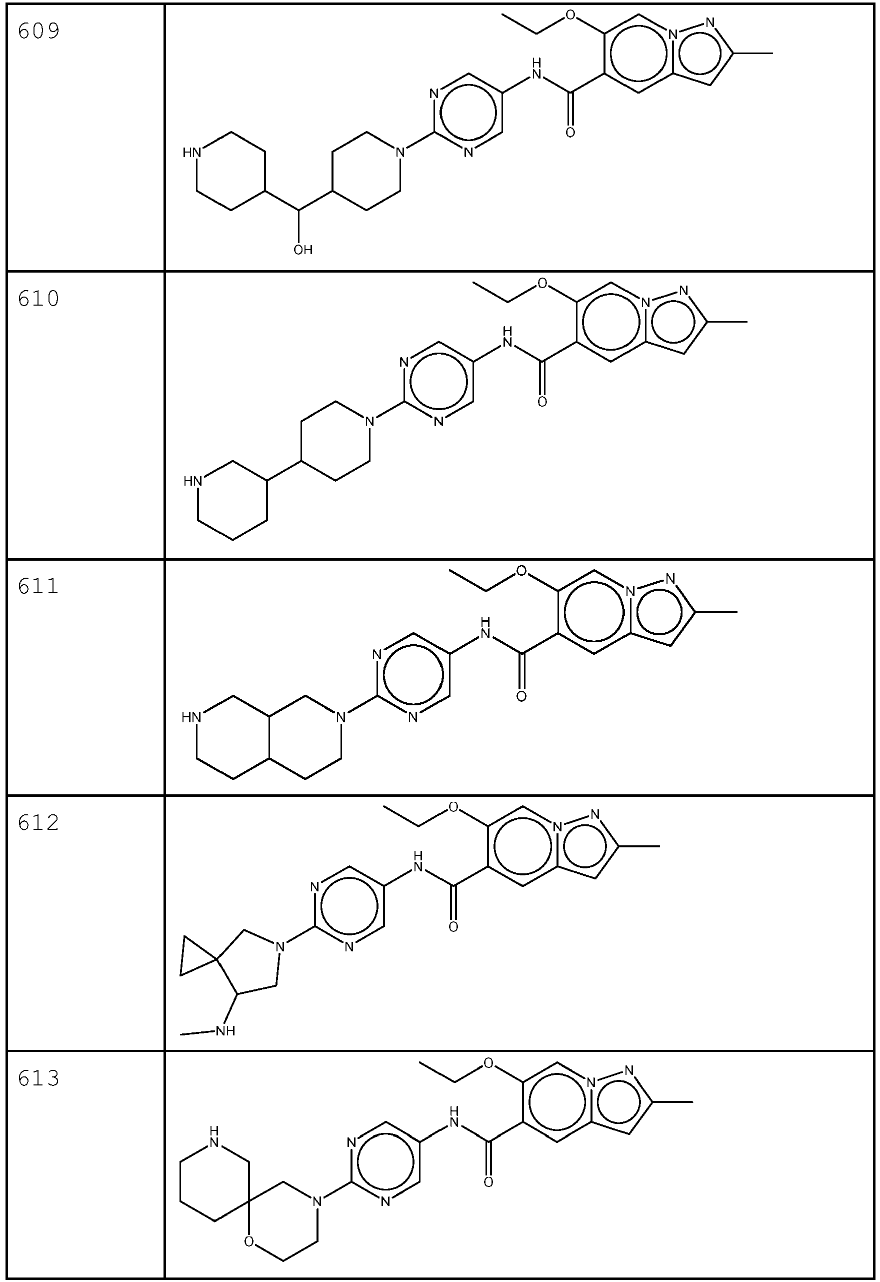

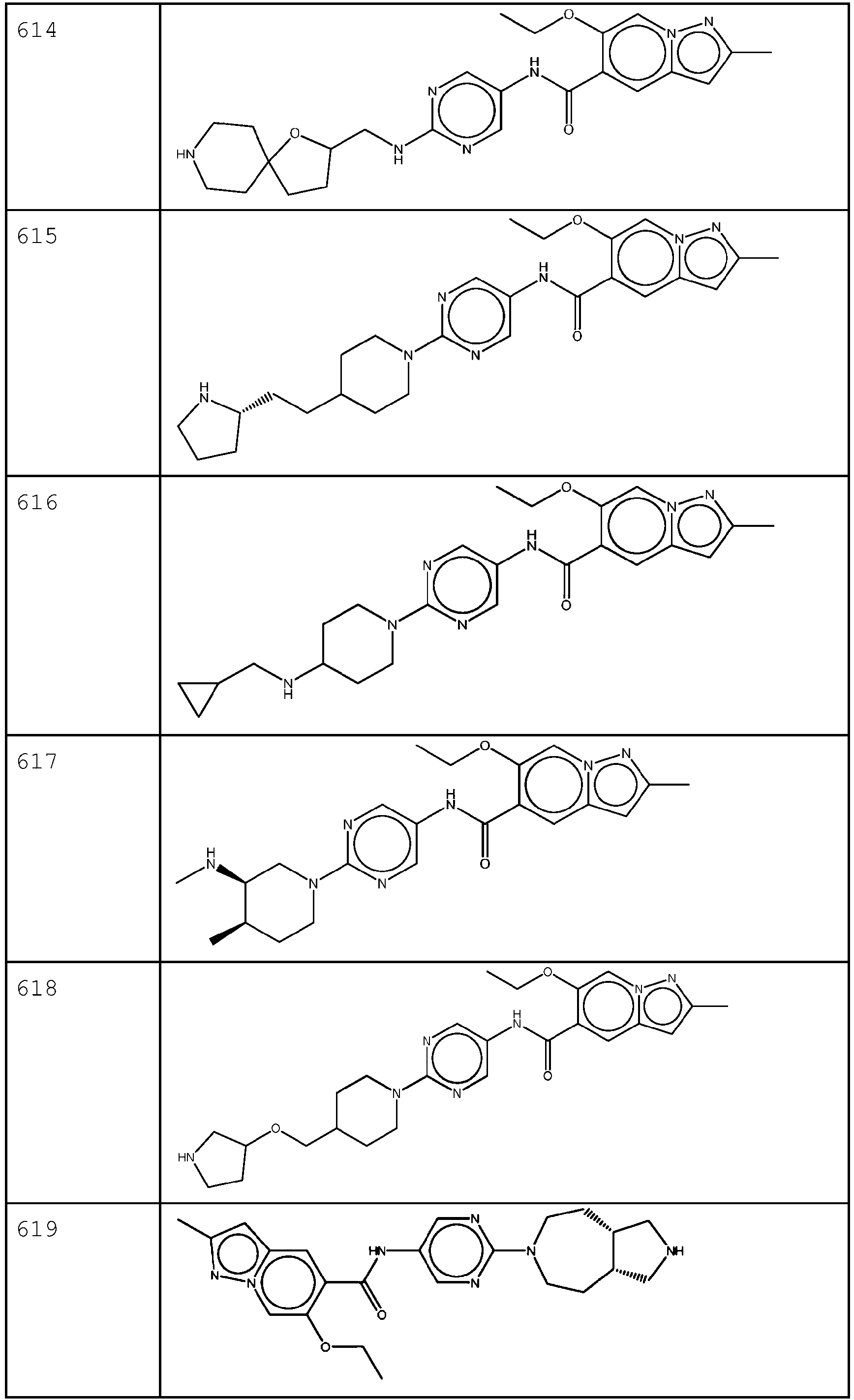

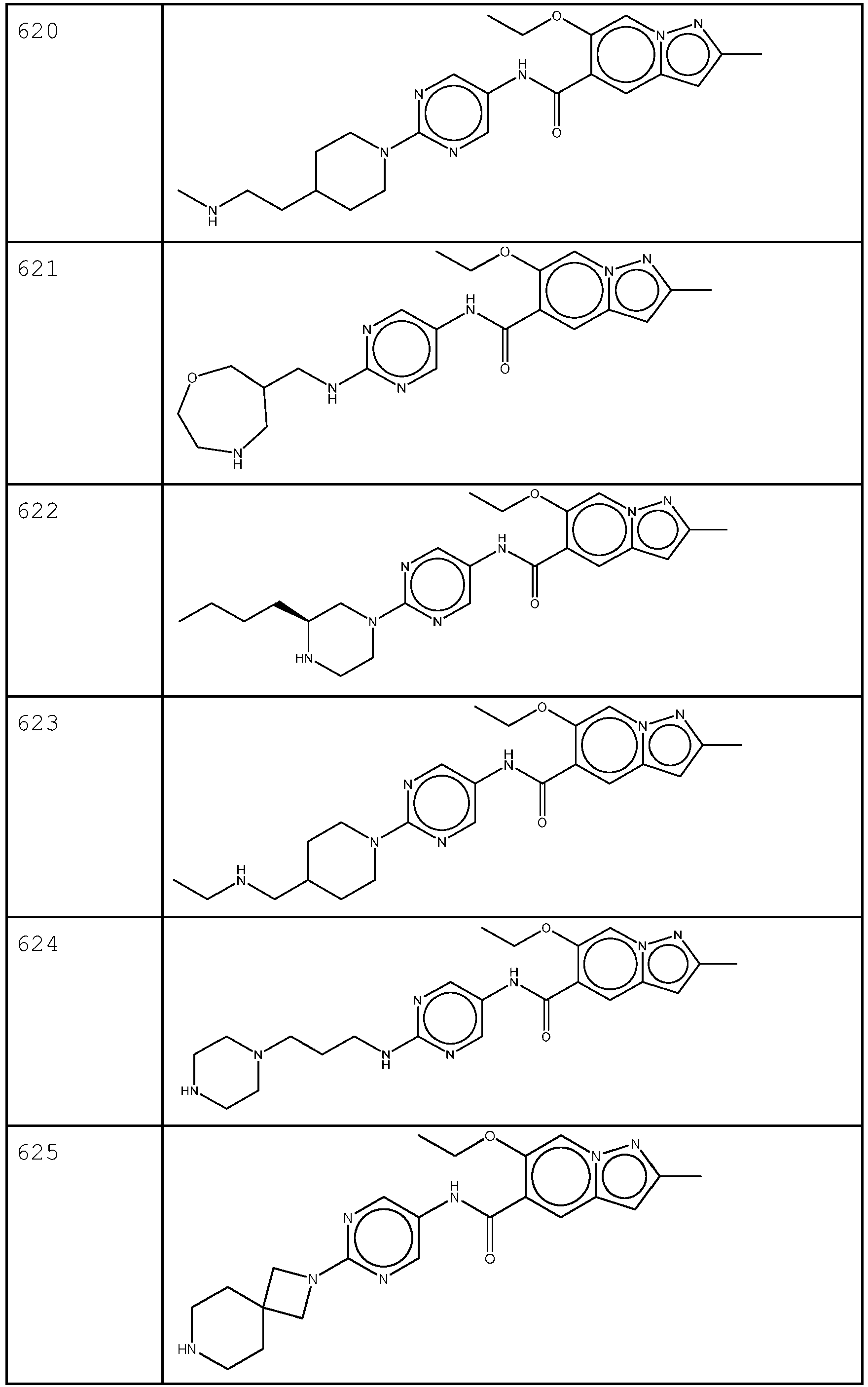

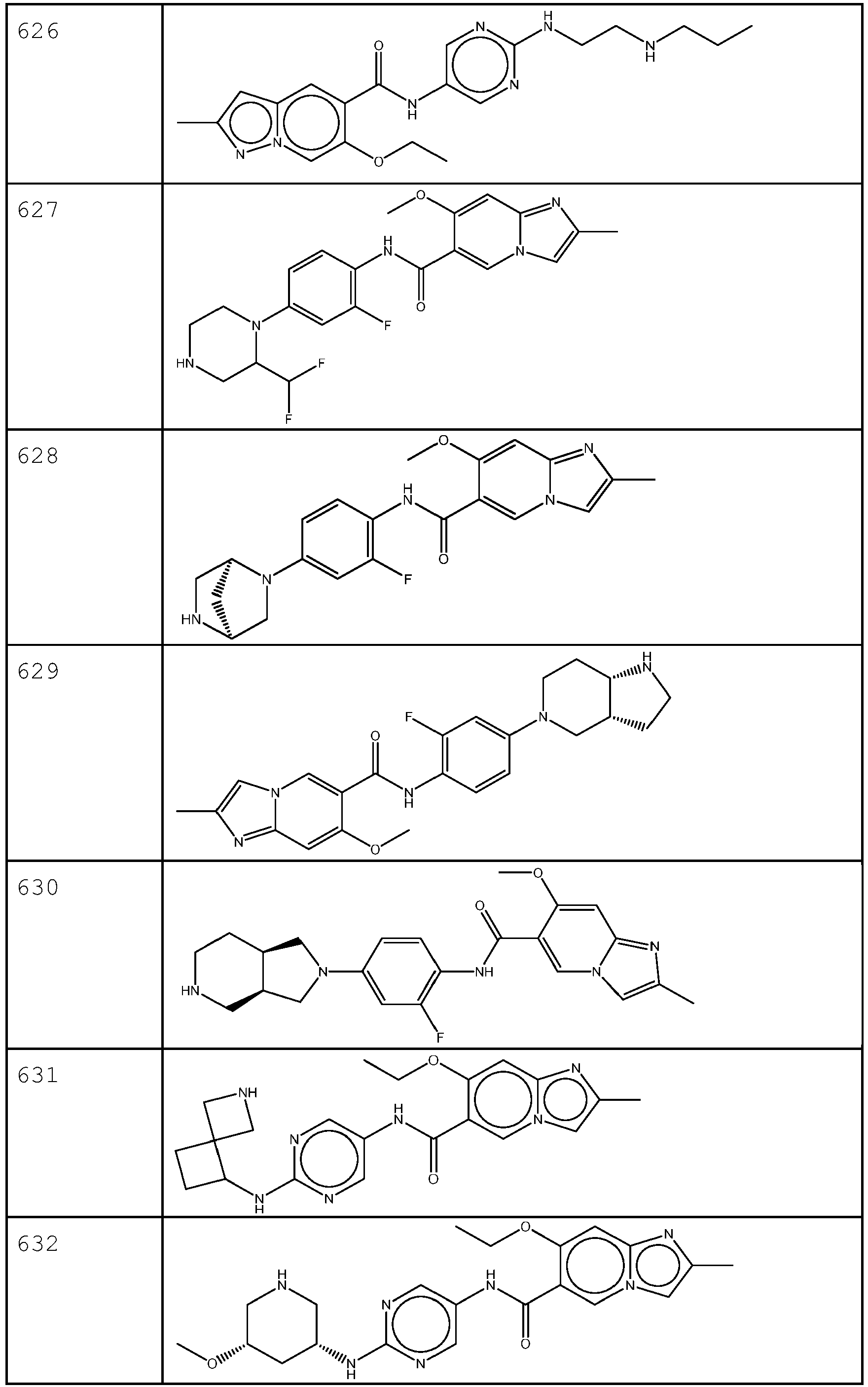

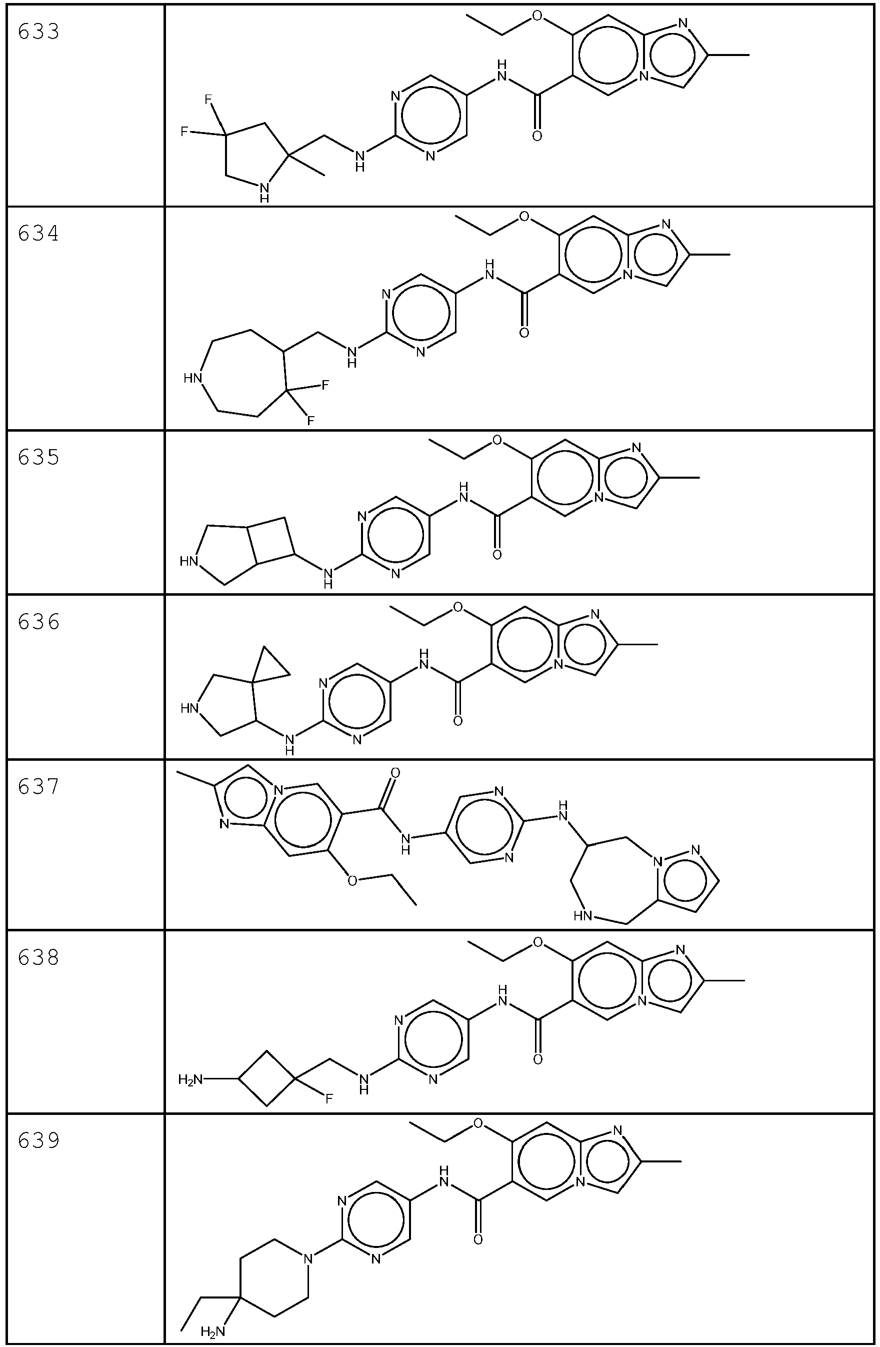

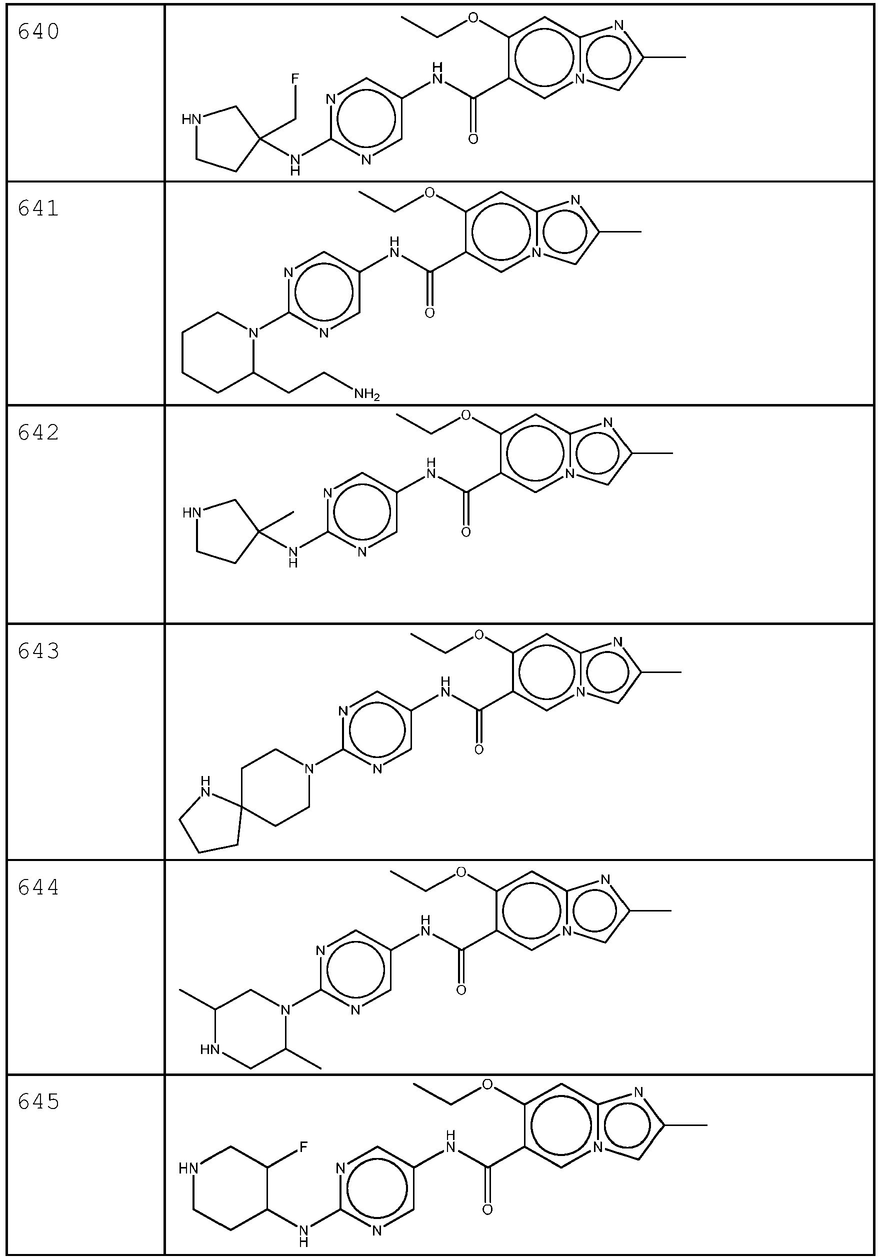

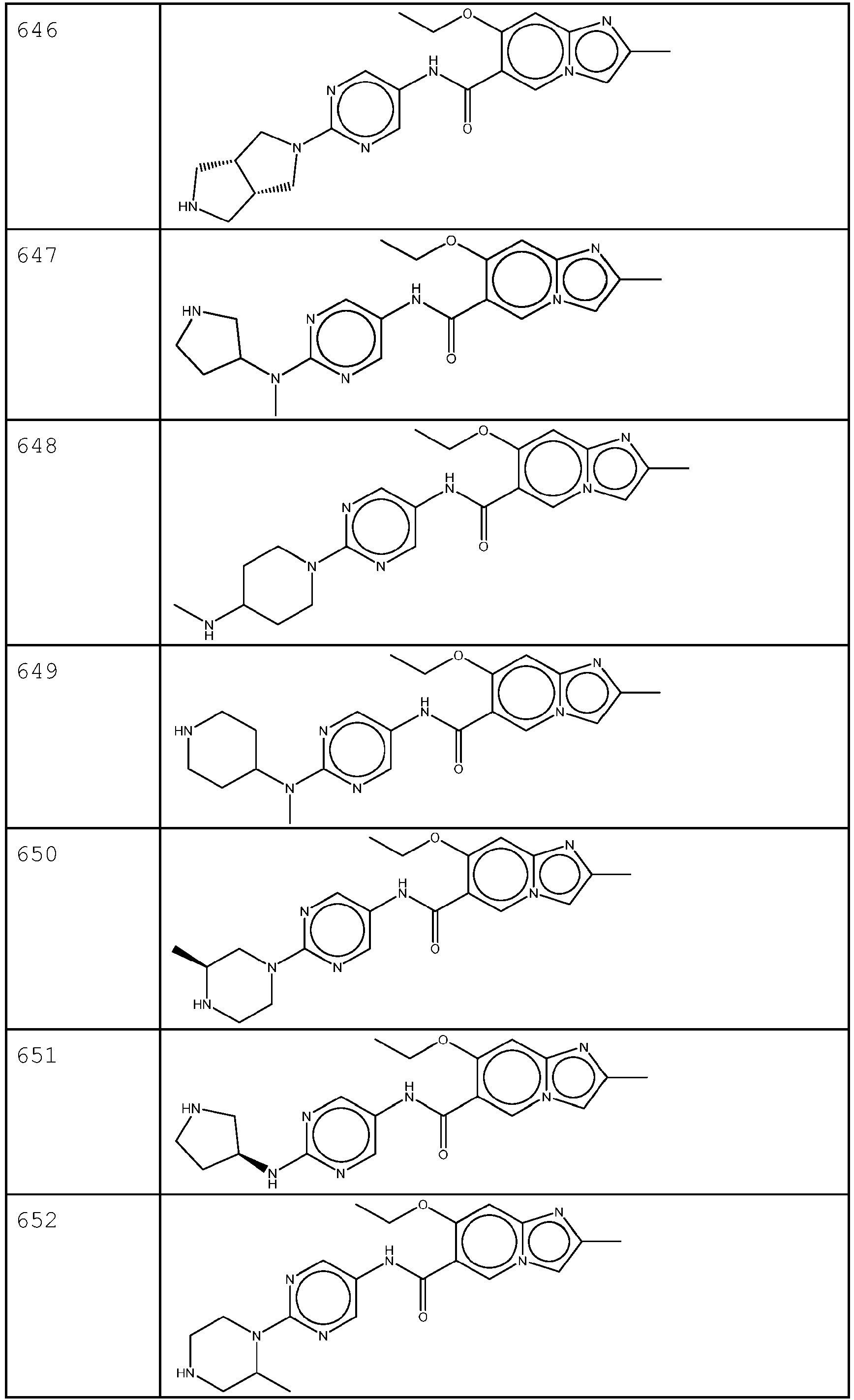

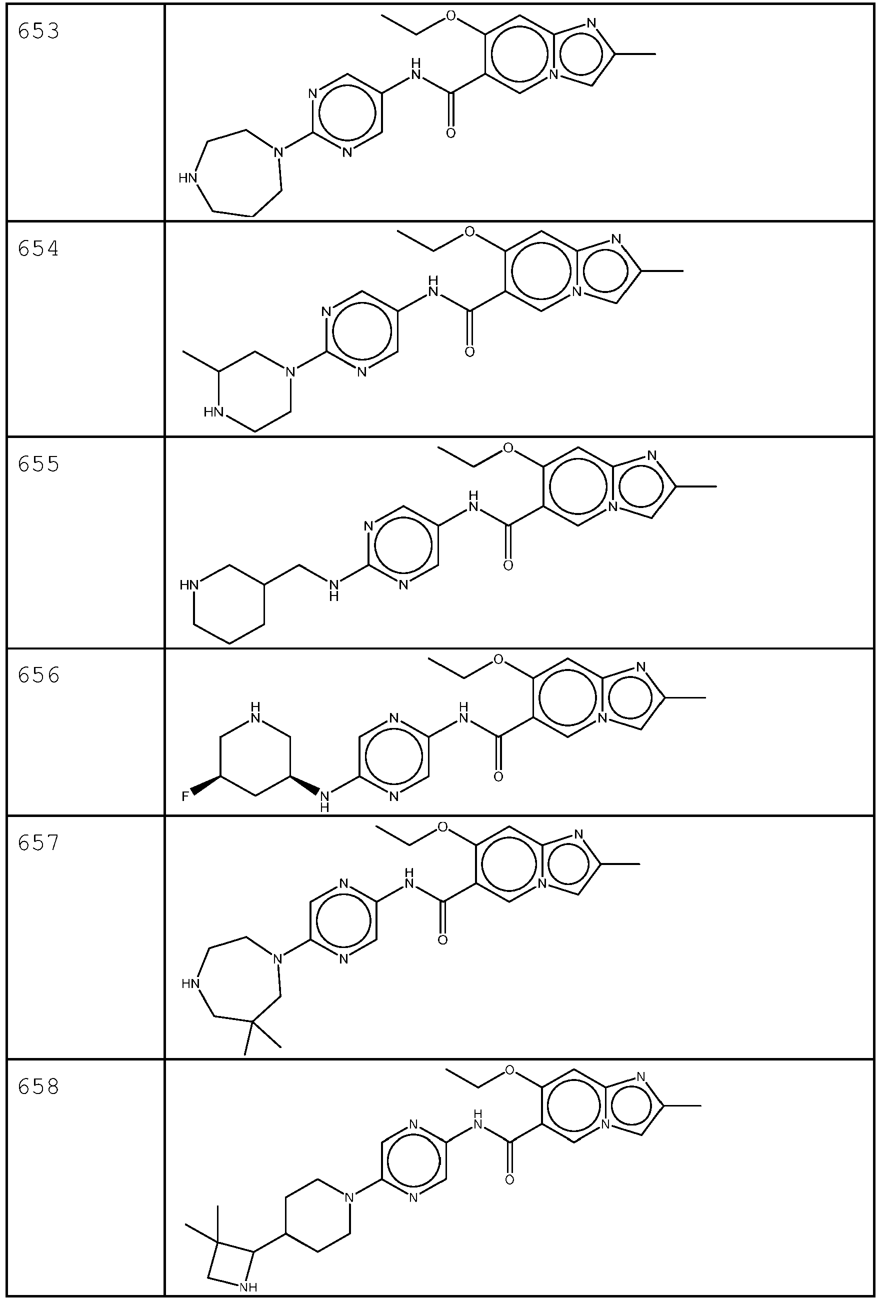

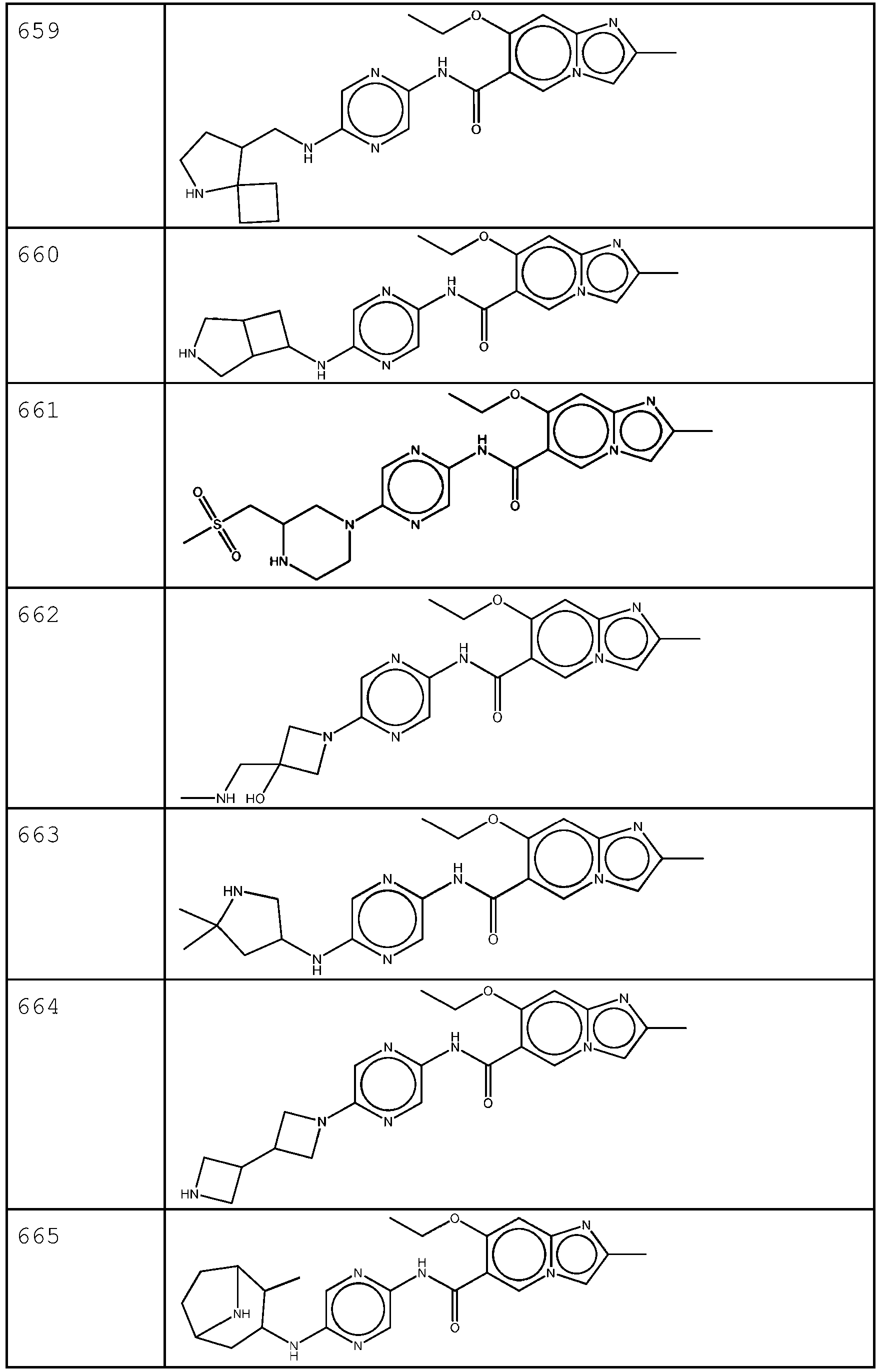

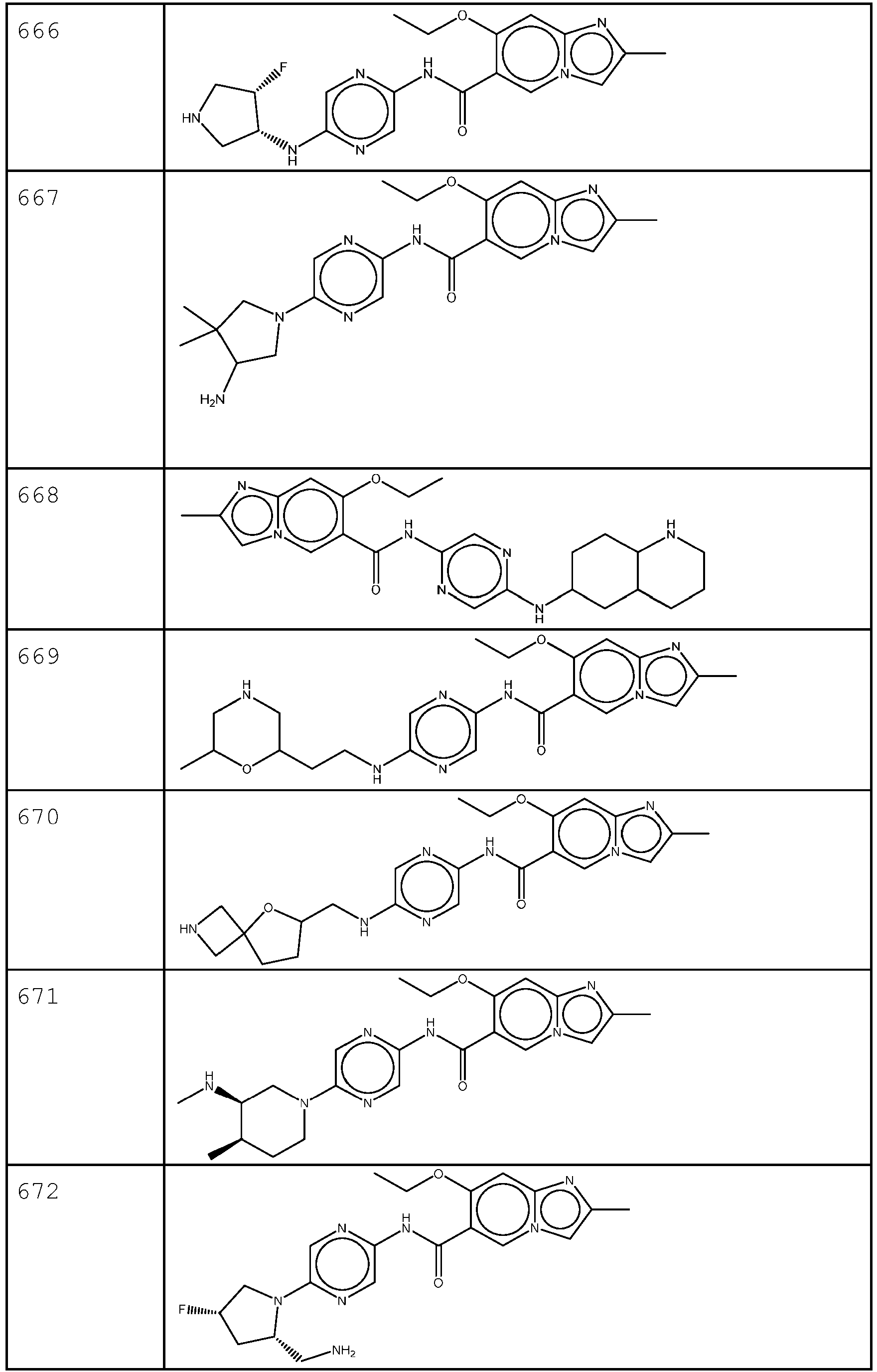

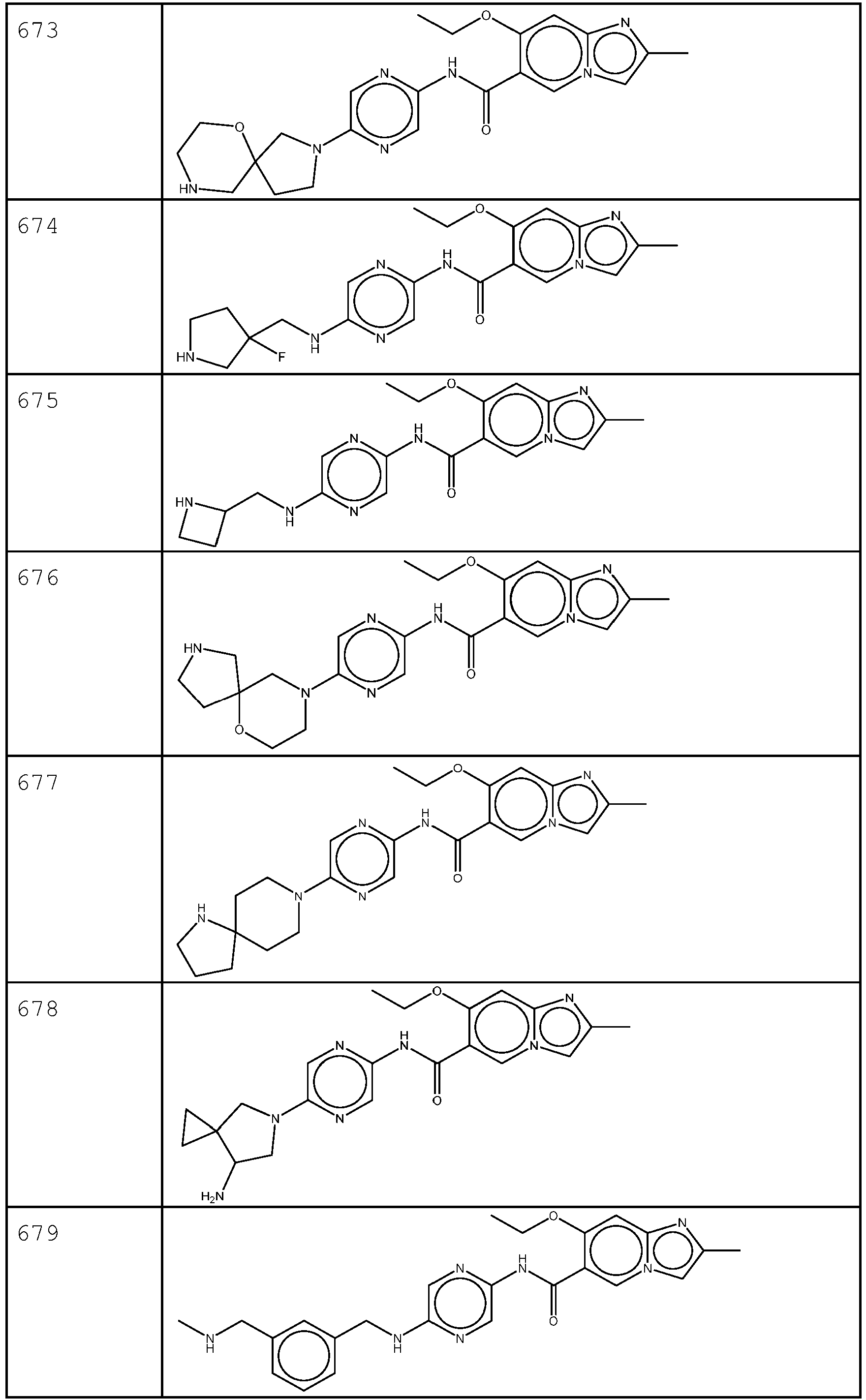

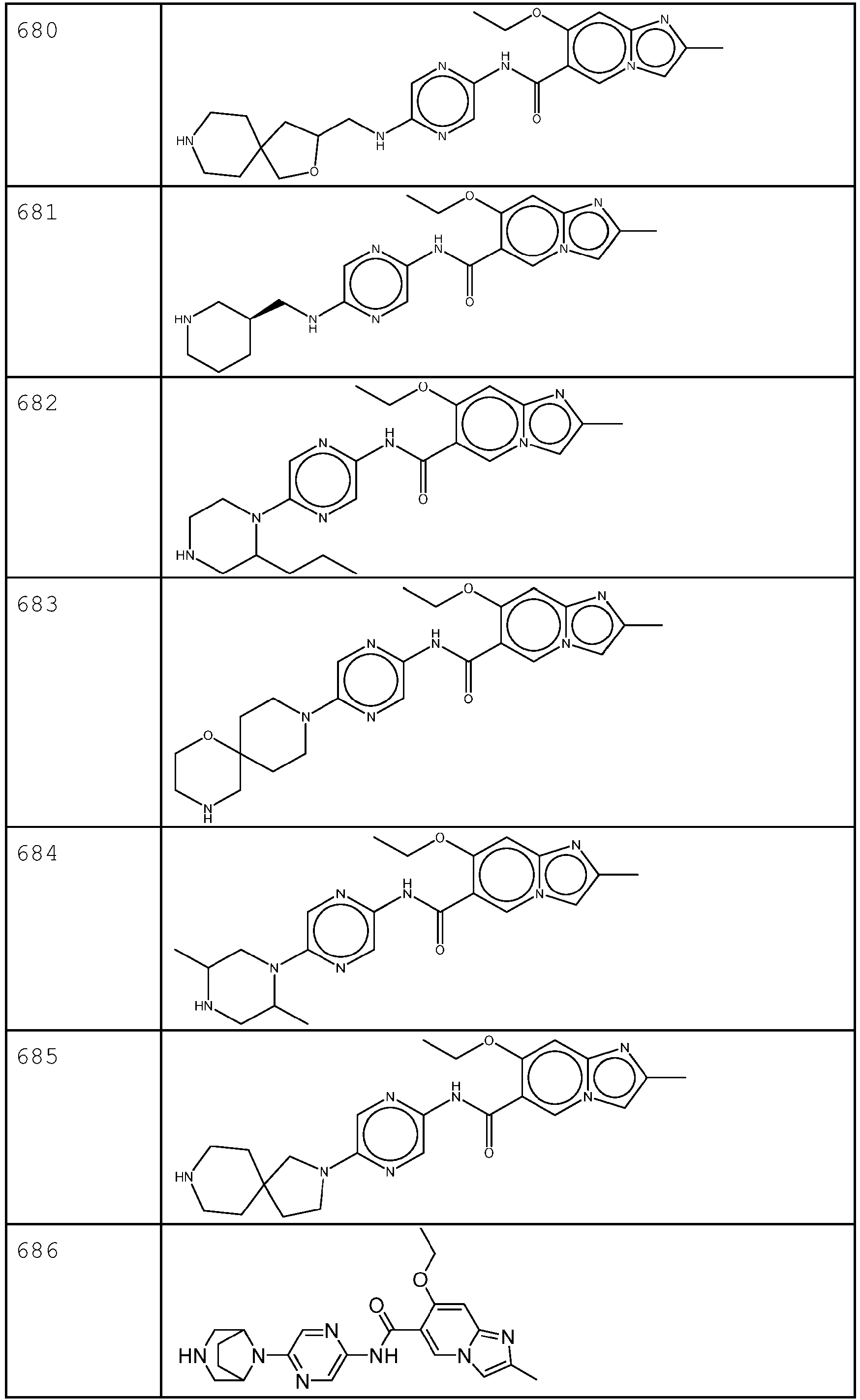

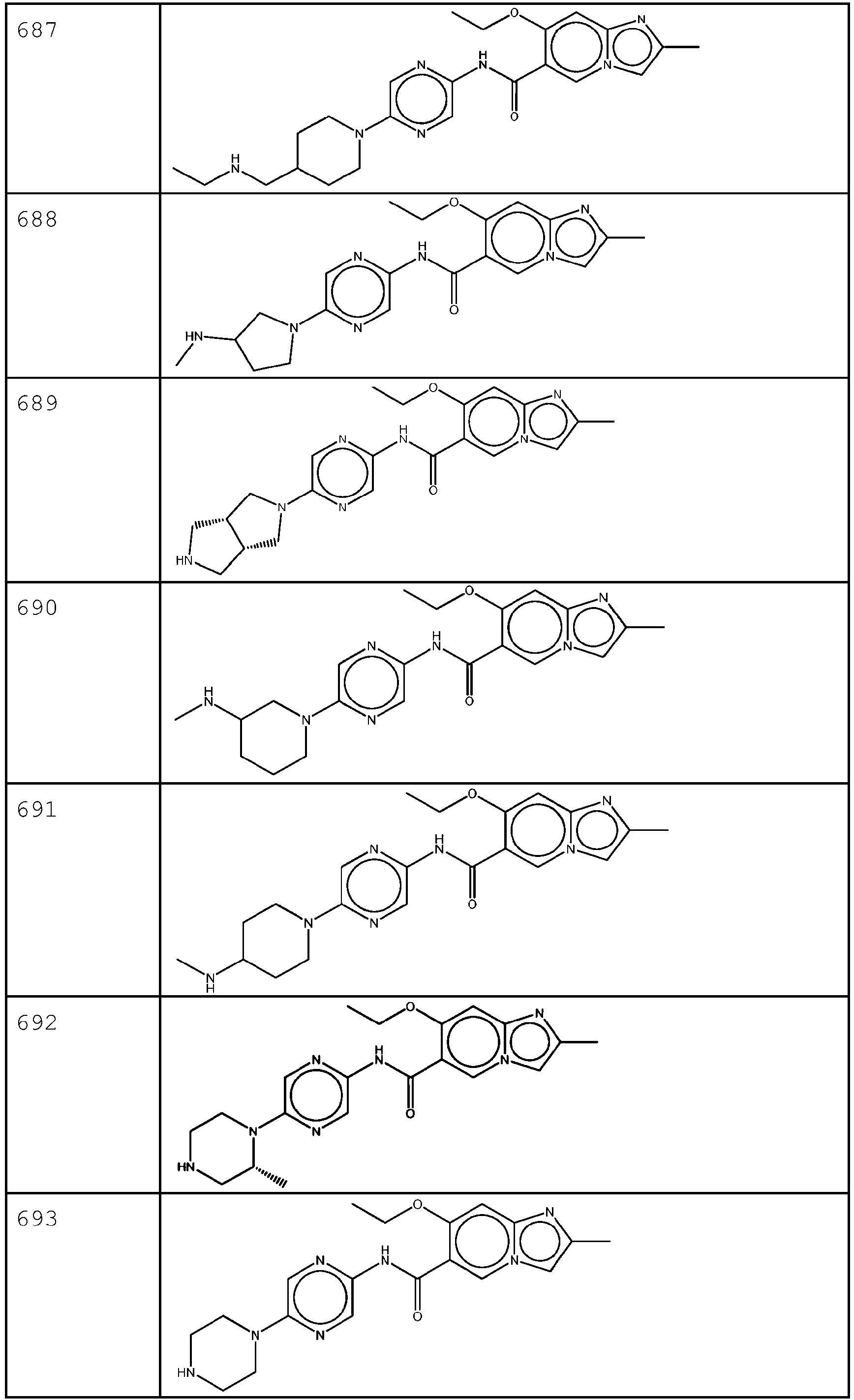

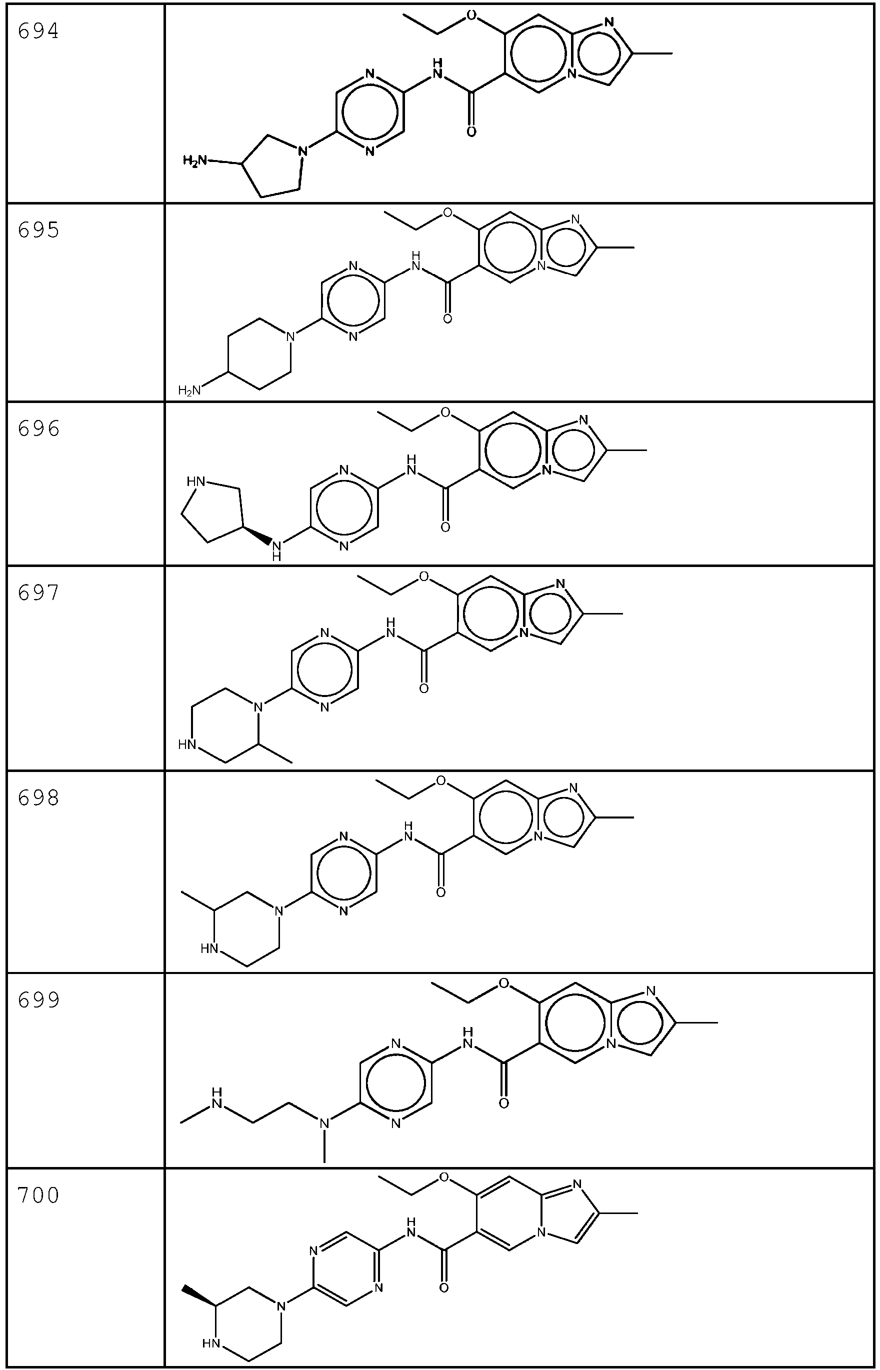

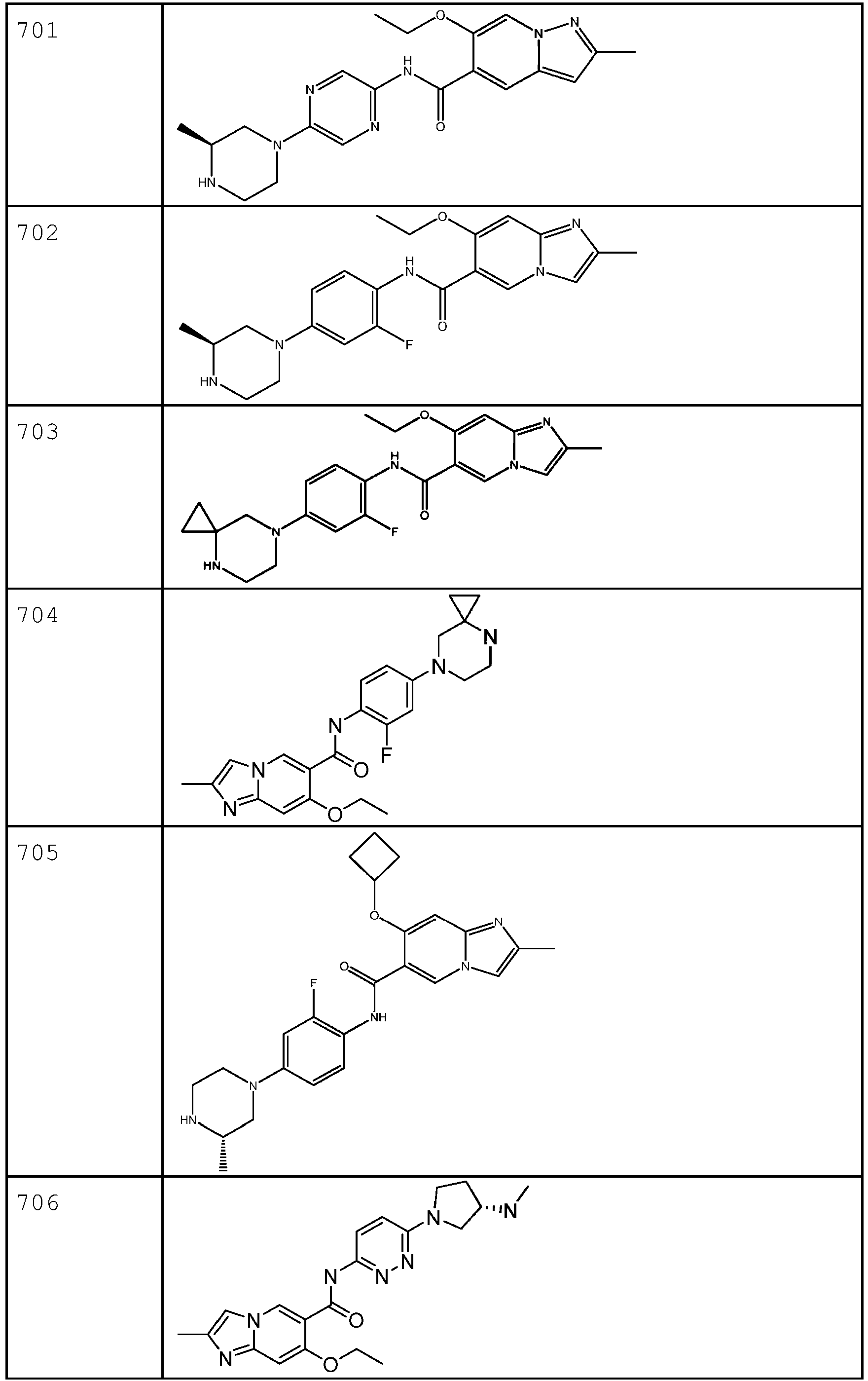

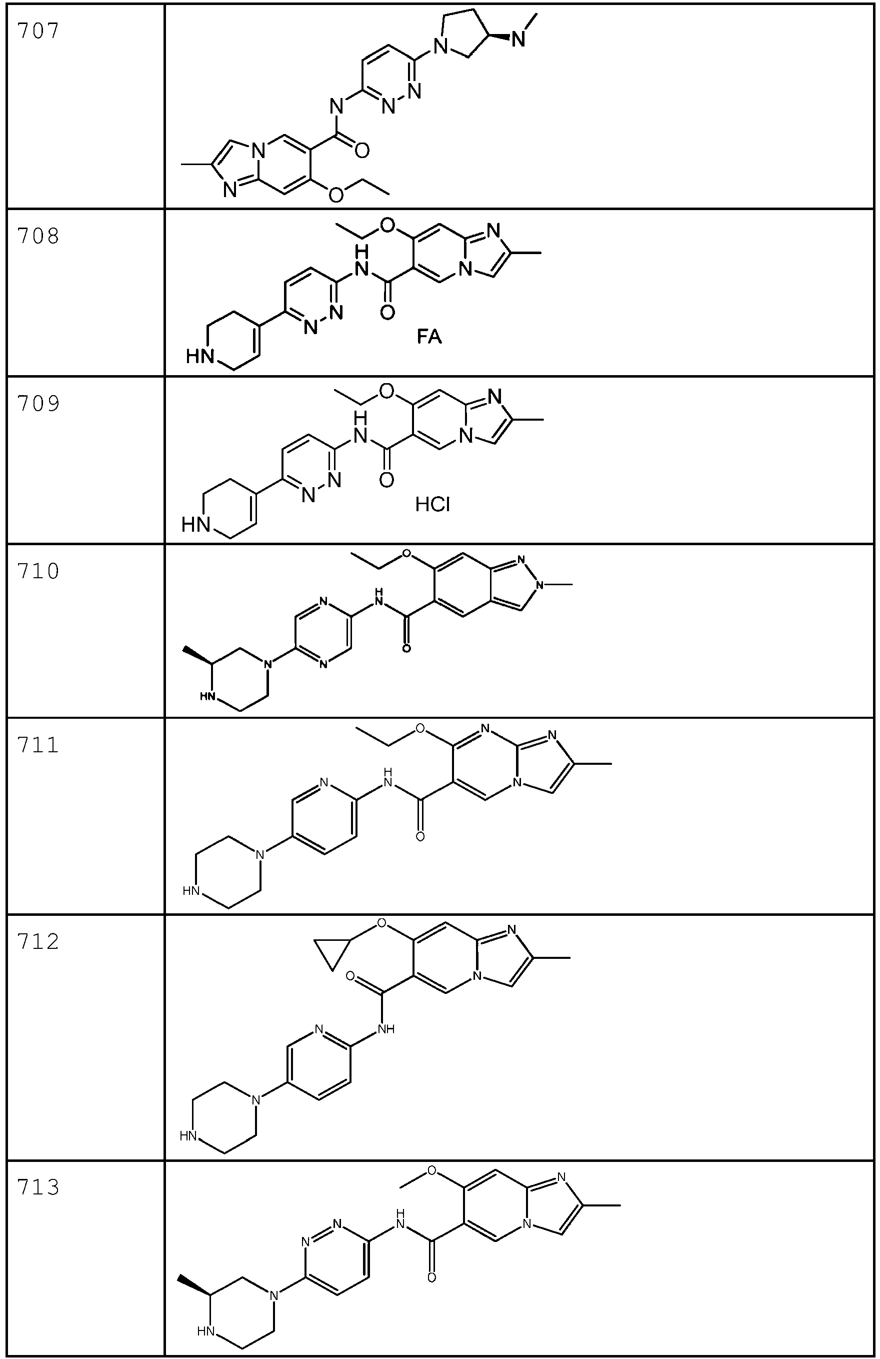

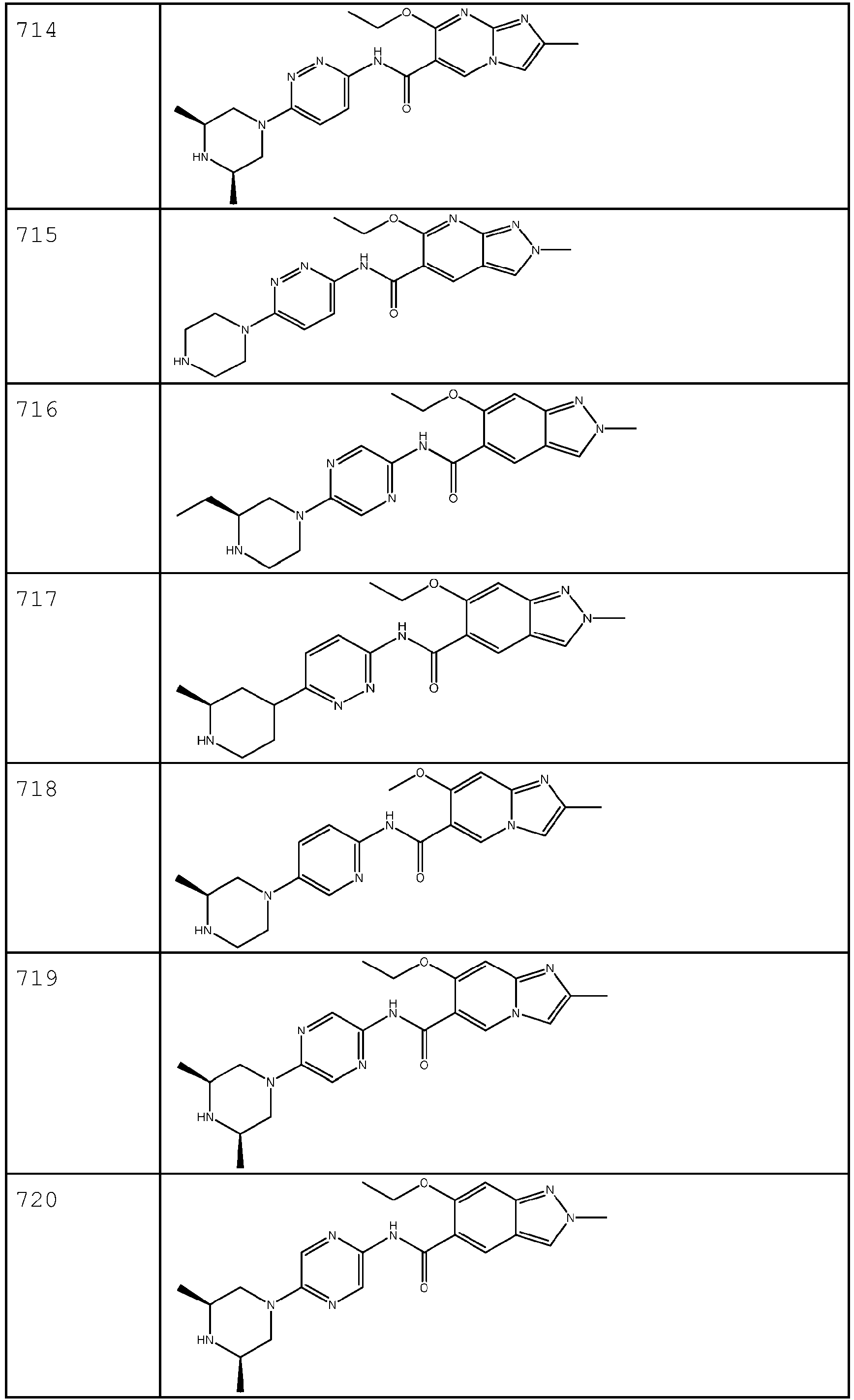

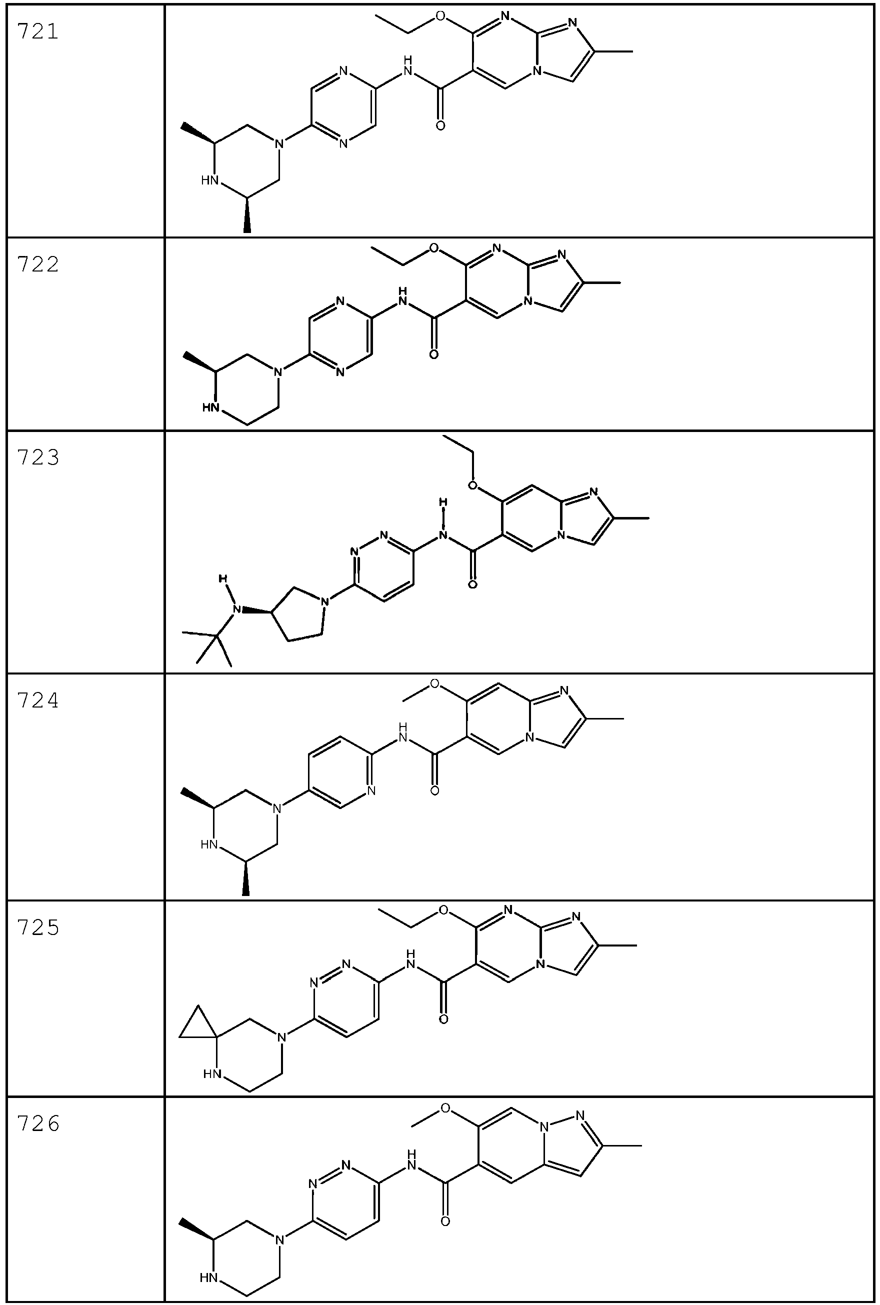

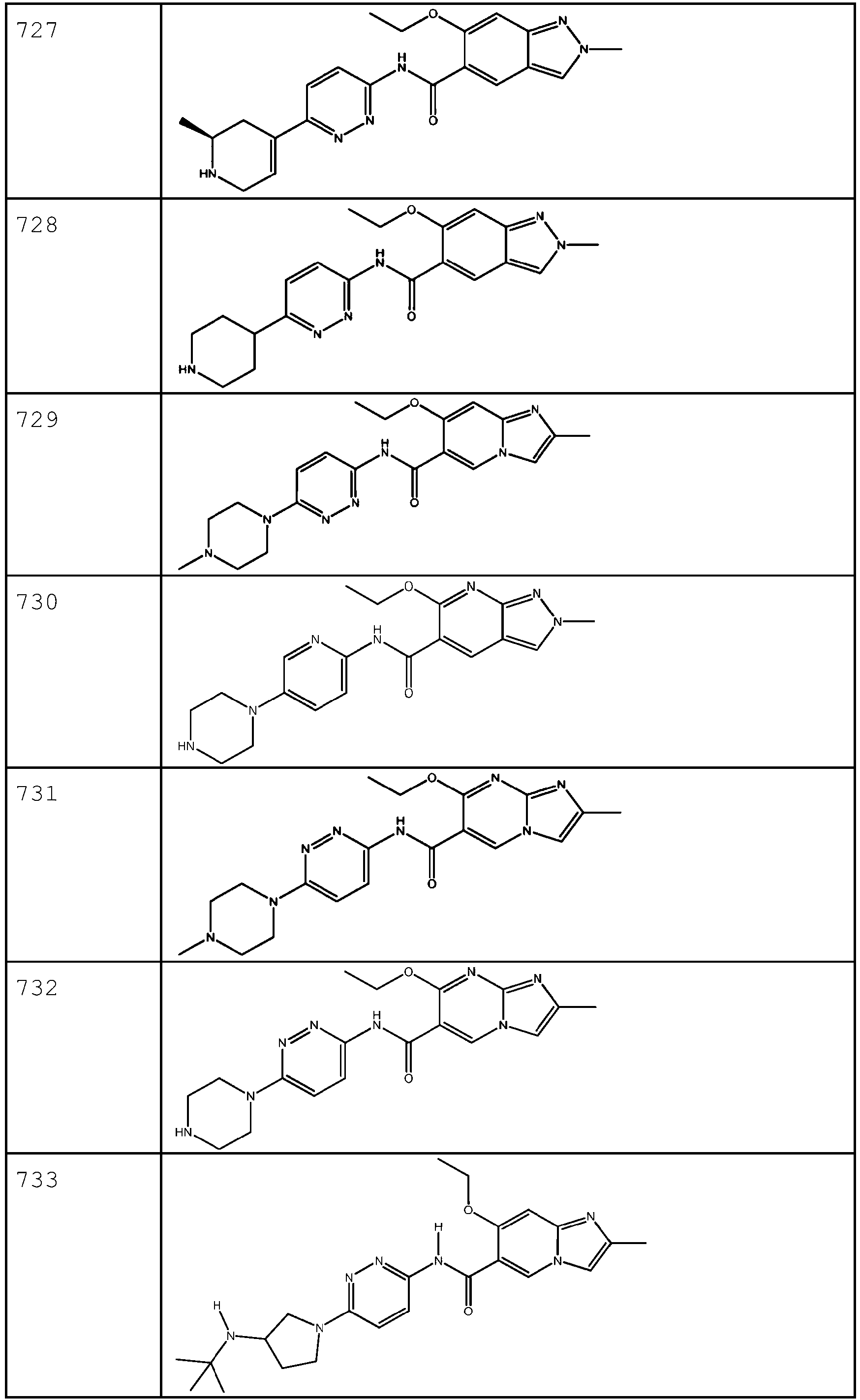

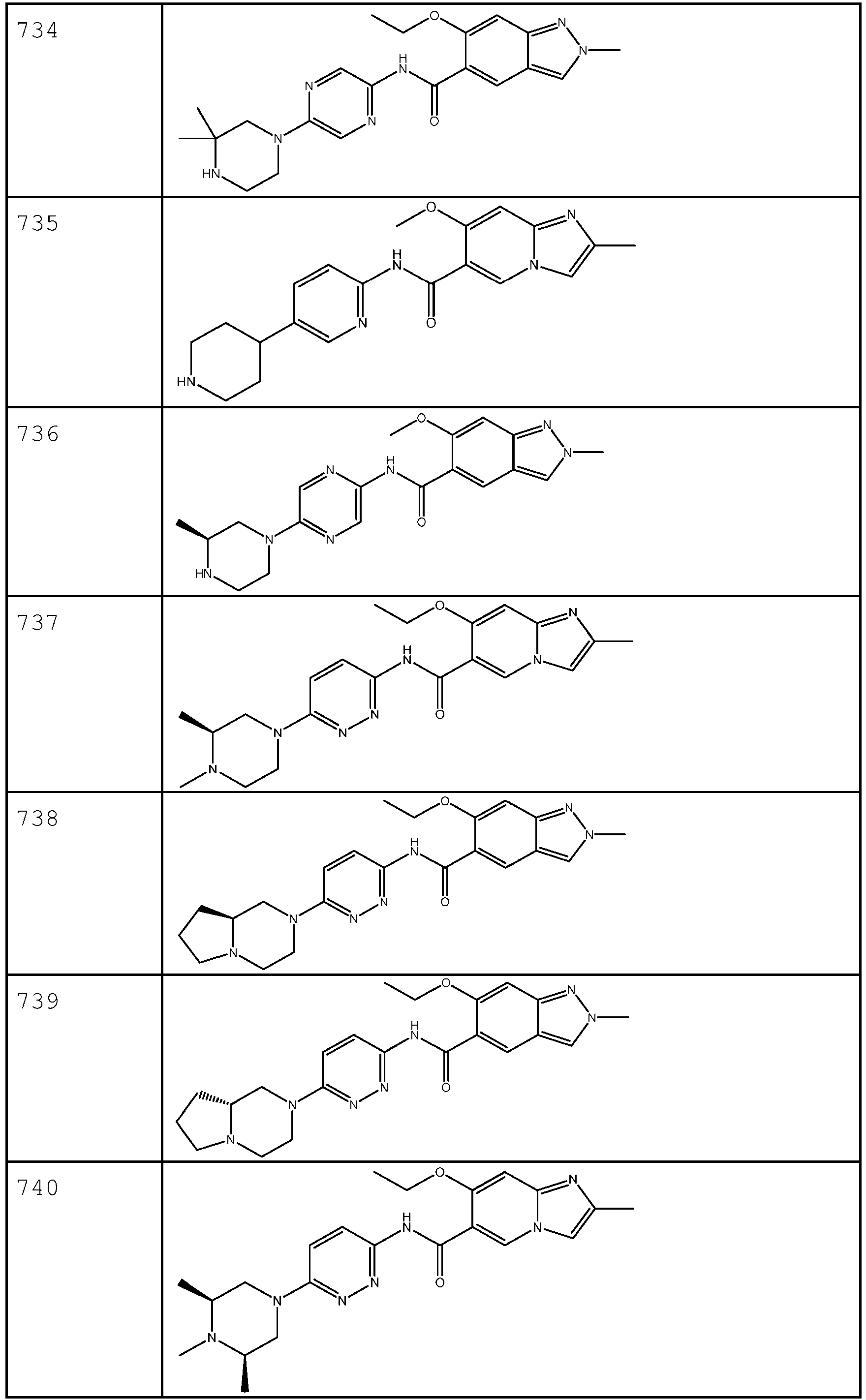

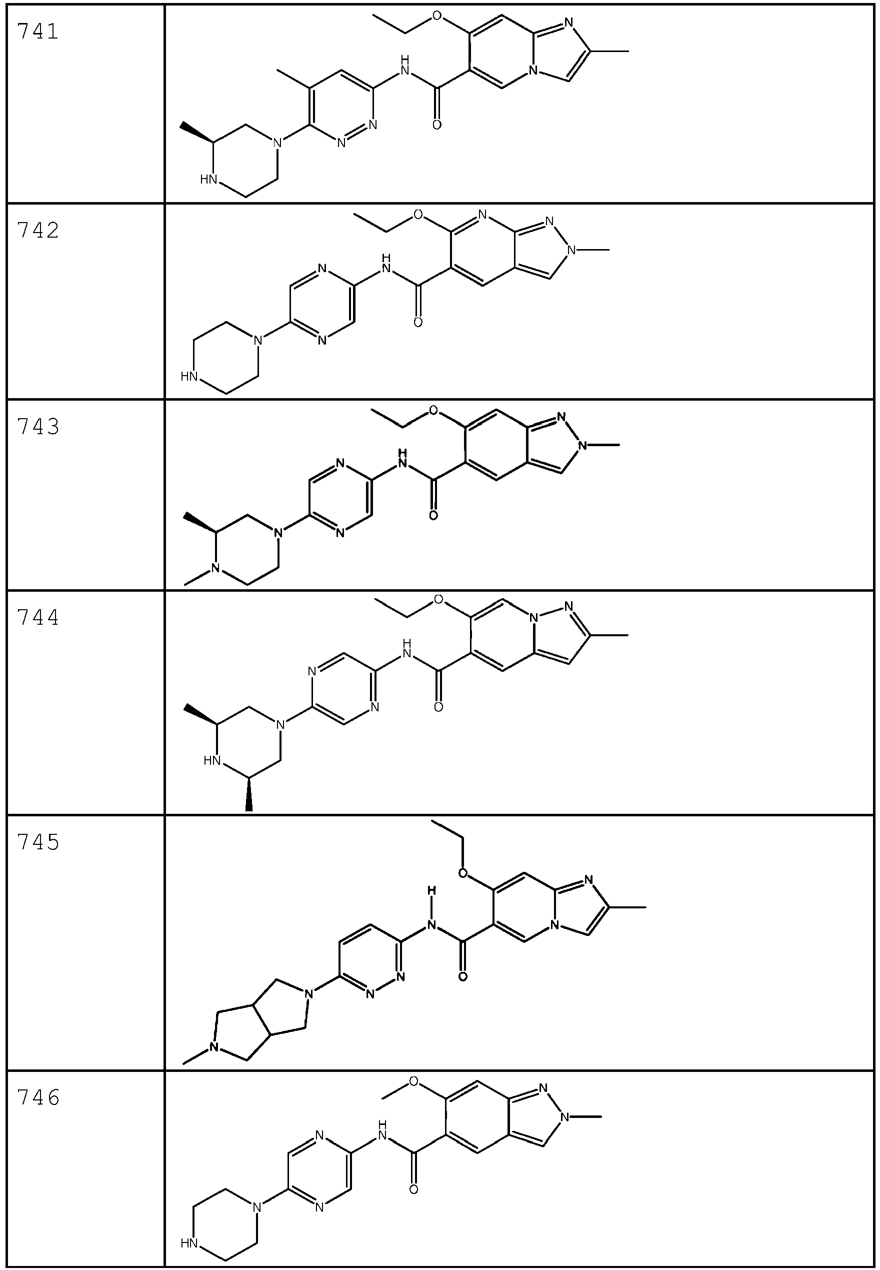

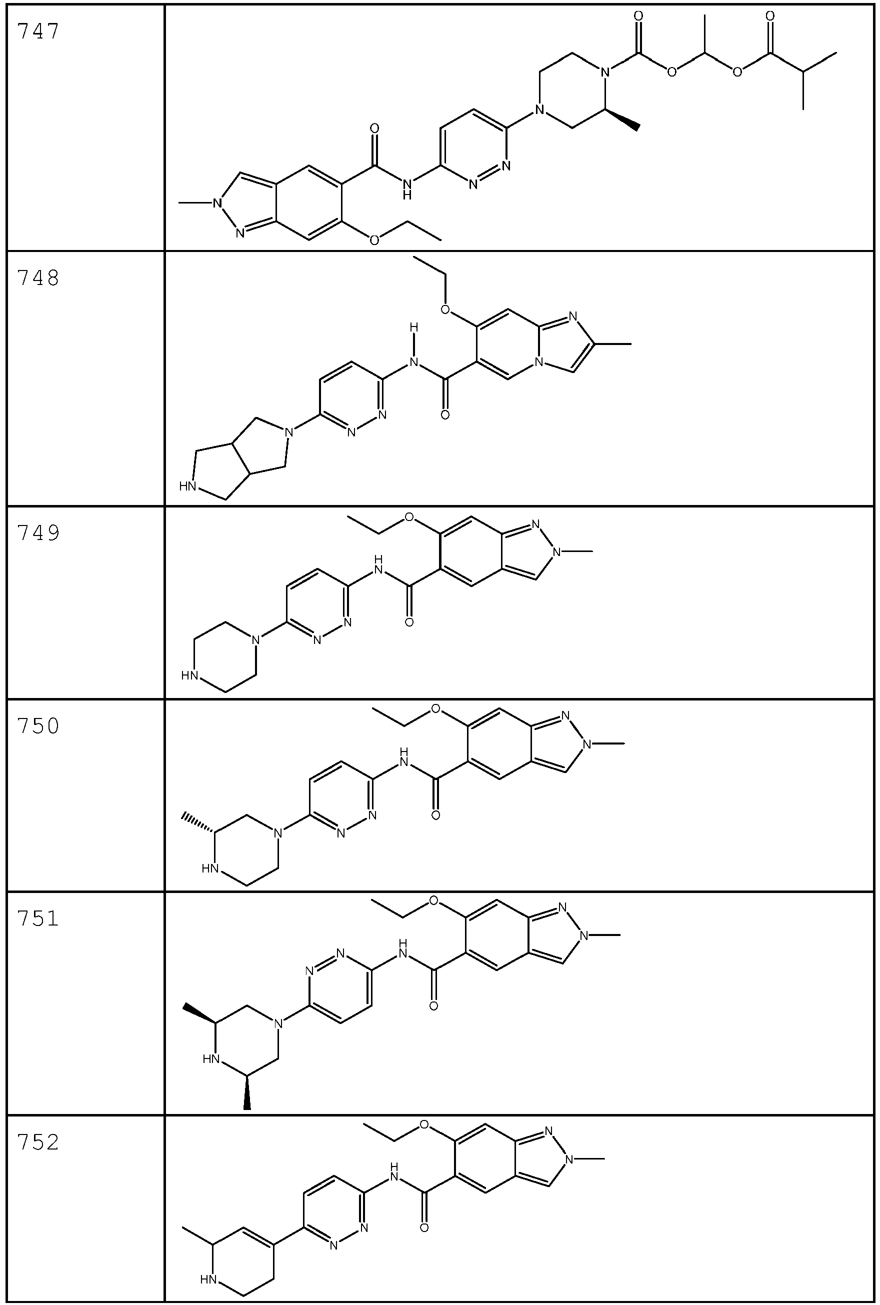

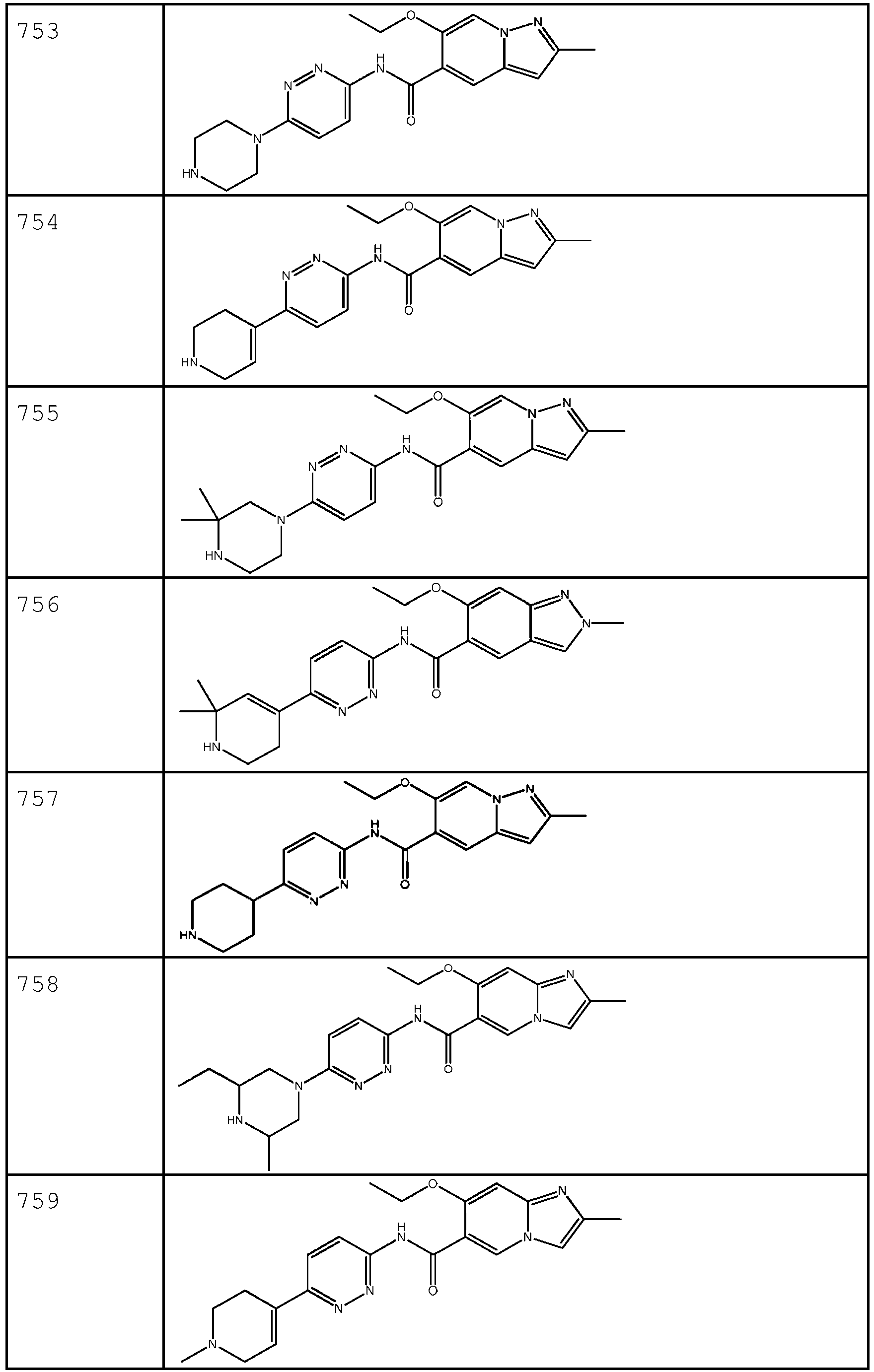

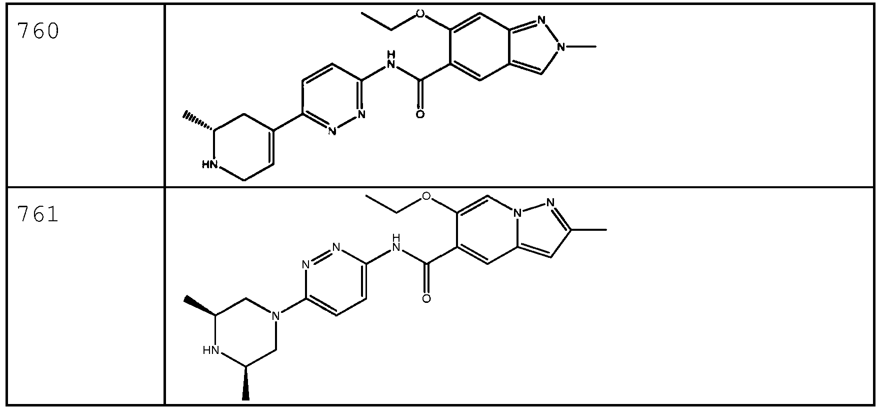

16. Composition pharmaceutique comprenant un composé selon l'une quelconque des revendications 1 à 15 ou un sel pharmaceutiquement acceptable correspondant et un ou plusieurs excipients pharmaceutiquement acceptables.

17. Composé selon l'une quelconque des revendications 1 à 15, ou sel pharmaceutiquement acceptable, ou composition pharmaceutique selon la revendication 16 destinés à être utilisés dans une méthode de traitement :
(a) d'un trouble lié à une expansion de répétitions de nucléotides ; éventuellement l'expansion de répétitions de nucléotides comprenant :
(i) une séquence nucléotidique répétée deux ou plus de deux fois, la séquence nucléotidique étant choisie dans le groupe constitué par CAG, CAG/CTG, GCG, GCN, CGG, CCG, CCCCGCCCCGCG, GCA, GGGGCC, CTG, GAA, ATTCT, TGGAA, GGCCTG, AAGGG, CCCTCT, ATTTT/ATTTC et CCCTCT ; ou
(ii) une séquence trinucléotidique répétée deux ou plus de deux fois, la séquence trinucléotidique étant choisie dans le groupe constitué par CAG, CTG, CGG et GCN ; ou
(b) d'une maladie, la maladie étant choisie dans le groupe constitué par l'atrophie dentato-rubro-pallido-luysienne, la maladie de Huntington, l'atrophie musculaire spinale et bulbaire, la SCA1 (ataxie spinocérébelleuse de type 1), la SCA2 (ataxie spinocérébelleuse de type 2), la SCA3 (ataxie spinocérébelleuse de type 3 ou maladie de Machado-Joseph),
la SCA6 (ataxie spinocérébelleuse de type 6), la SCA7 (ataxie spinocérébelleuse de type 7), la SCA12 (ataxie spinocérébelleuse de type 12), la SCA17 (ataxie spinocérébelleuse de type 17), le FRAXA (syndrome de l'X fragile), le FXTAS (syndrome tremblement/ataxie associé à l'X fragile), la FRAXE (déficience intellectuelle liée à FRAXE), le syndrome de Baratela-Scott, la FRDA (ataxie de Friedreich), la DM1 (dystrophie myotonique de type 1), la DM2 (dystrophie myotonique de type 2), la SCA8 (ataxie spinocérébelleuse de type 8), la dystrophie cornéenne endothéliale de Fuchs, la dysplasie de Desbuquois, la sclérose latérale amyotrophique, la démence fronto-temporale ; éventuellement la maladie étant :
(i) la maladie de Huntington ;
(ii) la dystrophie myotonique de type 1 ; ou
(iii) choisie dans le groupe constitué par le FRAXA (syndrome de l'X fragile), le FXTAS (syndrome tremblement/ataxie associé à l'X fragile), la FRAXE (déficience intellectuelle liée à FRAXE).
